# EUROPEAN PATENT APPLICATION

(11) **EP 2 657 352 A2**
(43) Date of publication of application: **30.10.2013**
(21) Application number: 13174143.1
(22) Date of filing: 23.10.2008
(51) Int. Cl.: C12Q 1/68

(54) **A method of diagnosing neoplasms - II**

(30) Priority: 23.10.2007 US 982115 P
(62) Divisional of application: 08842023.7
(71) Applicant: Clinical Genomics Pty Ltd, North Ryde, New South Wales 2113 (AU); Commonwealth Scientific and Industrial Research Organisation, Campbell, Australian Capital Territory 2612 (AU)
(72) Inventor: Lapointe, Lawrence C, West Pennant Hills, New South Wales 2125 (AU); Dunne, Robert, Darlington, New South Wales 2008 (AU); Young, Graeme P, Malvern, South Australia 5061 (AU); Lockett, Trevor John, Denistone, New South Wales 2114 (AU); Wilson, William J, Hornsby Heights, New South Wales 2077 (AU); Molloy, Peter Laurence, Chatswood, New South Wales 2067 (AU)
(74) Representative: Maschio, Antonio

(57) **Abstract**

The present invention relates generally to nucleic acid molecules in respect of which changes to the DNA or to the RNA or protein expression profiles are indicative of the onset, predisposition to the onset and/or progression of a neoplasm. More particularly, the present invention is directed to nucleic acid molecules in respect of which changes to the DNA or to the RNA or protein expression profiles are indicative of the onset and/or progression of a large intestine neoplasm, such as an adenoma or an adenocarcinoma. The DNA or the expression profiles of the present invention are useful in a range of applications including, but not limited to, those relating to the diagnosis and/or monitoring of colorectal neoplasms, such as colorectal adenocarcinomas. Accordingly, in a related aspect the present invention is directed to a method of screening a subject for the onset, predisposition to the onset and/or progression of a neoplasm by screening for modulation in the DNA or the RNA or protein expression profile of one or more nucleic acid molecule markers.

SEQUENCE LISTING

<110>  CLINICAL GENOMICS PTY. LTD.
       COMMONWEALTH SCIENTIFIC AND INDUSTRIAL RESEARCH
       ORGANISATION
       LaPointe, Lawrence
       Dunne, Rob
       Young, Graeme
       Lockett, Trevor
       Wilson, William
       Molloy, Peter

<120>  A method of diagnosing neoplasms - II

<130>  30637603/TDO

<150>  US 60/982115
<151>  2007-10-23

<160>  681

<170>  PatentIn version 3.4

<210>  1
<211>  544
<212>  DNA
<213>  human
<400>  1
tagtcttggg gcaggatgat tttggcctca ttactttacc acccccacac ctggaaagca      60
tatactatat tacaaaatga cattttgcca aaattattaa tataagaagc tttcagtatt     120
agtgatgtca tctgtcacta taggtcatac aatccattct taaagtactt gttatttgtt     180
tttattatta ctgtttgtct tctccccagg gttcagtccc tcaaggggcc atcctgtccc     240
accatgcagt gccccctagc ttagagcctc cctcaattcc ccctggccac caccccccac     300
tctgtgcctg accttgagga gtcttgtgtg cattgctgtg aattagctca cttggtgata     360
tgtcctatat tggctaaatt gaaacctgga attgtggggc aatctattaa tagctgcctt     420
aaagtcagta acttaccctt agggaggctg gggaaaaagg ttagattttg tattcagggg     480
ttttttgtgt actttttggg tttttaaaaa attgtttttg gaggggttta tgctcaatcc     540
atgt                                                                  544

<210>  2
<211>  530
<212>  DNA
<213>  human
<400>  2
gatagcctcc cactaggact gggaggagaa taacccaggt cttaaggacc ccaaagtcag      60
gatgttgttt gatcttctca aacatctagt tccctgcttg atgggaggat cctaatgaaa     120
tacctgaaac atatattggc atttatcaat ggctcaaatc ttcatttatc tctggcctta     180
accctggctc ctgaggctgc ggccagcaga gcccaggcca gggctctgtt cttgccacac     240
ctgcttgatc ctcagatgtg gagggaggta ggcactgcct cagtcttcat ccaaacacct     300
ttccctttgc cctgagacct cagaatcttc cctttaaccc aagaccctgc ctcttccact     360
ccacccttct ccagggaccc ttagatcaca tcactccacc cctgccaggc cccaggttag     420
gaatagtggt gggaggaagg ggaaagggct gggcctcacc gctcccagca actgaaagga     480
caacactatc tggagccacc cactgaaagg gctgcaggca tgggctgtac                530

<210>  3
<211>  416
<212>  DNA
<213>  human
<400>  3
gttggttcaa acttttggga gcacggactg tcagttctct gggaagtggt cagcgcatcc      60
tgcagggctt ctcctcctct gtcttttgga gaaccagggc tcttctcagg ggctctaggg     120
actgccaggc tgtttcagcc aggaaggcca aaatcaagag tgagatgtag aaagttgtaa     180
aatagaaaaa gtggagttgg tgaatcggtt gttctttcct cacatttgga tgattgtcat     240

aaggttttta gcatgttcct ccttttcttc accctcccct ttgttcttct attaatcaag 300
agaaacttca aagttaatgg gatggtcgga tctcacaggc tgagaactcg ttcacctcca 360
agcatttcat gaaaaagctg cttcttatta atcatacaaa ctctcaccat gatgtg 416

<210> 4
<211> 511
<212> DNA
<213> human
<400> 4
aacactgcat aacccgtttc tttgaggagt gtgaccccaa caaggataag cacatcaccc 60
tgaaggagtg gggccactgc tttggaatta aagaagagga catagatgaa aatctcttgt 120
tttgaacgaa gattttaaag aactcaactt tccagcatcc tcctctgttc taaccacttc 180
agaaatatat gcagctgtga tacttgtaga tttatattta gcaaaatgtt agcatgtatg 240
acaagacaat gagagtaatt gcttgacaac aacctatgca ccaggtattt aacattaact 300
ttggaaacaa aaatgtacaa ttaagtaaag tcaacatatg caaaatactg tacattgtga 360
acagaagttt aattcatagt aatttcactc tctgcattga cttatgagat aattaatgat 420
taaactatta atgataaaaa taatgcattt gtattgttca taatatcatg tgcacttcaa 480
gaaaatggaa tgctactctt ttgtggttta c 511

<210> 5
<211> 456
<212> DNA
<213> human
<400> 5
ggccgacaag aagaaggtgc tggacaagtg tcaagaggtc atctcgtggc tggacgccaa 60
caccttggcc gagaaggacg agtttgagca caagaggaag gagctggagc aggtgtgtaa 120
ccccatcatc agcggactgt accagggtgc cggtggtccc gggcctgggg gcttcggggc 180
tcagggtccc aagggagggt ctgggtcagg ccccaccatt gaggaggtag attaggggcc 240
tttccaagat tgctgttttt gttttggagc ttcaagactt tgcatttcct agtatttctg 300
tttgtcagtt ctcaatttcc tgtgtttgca atgttgaaat tttttggtga agtactgaac 360
ttgctttttt tccggtttct acatgcagag atgaatttat actgccatct tacgactatt 420
tcttcttttt aatacactta actcaggcca tttttt 456

<210> 6
<211> 524
<212> DNA
<213> human
<400> 6
tcctgtcacc cattttgaag agtggcagaa cttgaagttc aacttcctct gtaaatatcc 60
aagtataaag cccaggaact tctagaataa cccagatgcg ctttaatttt ttttaatatg 120
ttttgatcac agaacttcta gaataaccca gatgctcttt catattcttt taatacatct 180
tgatcacagc tgggggaaaa aaagcttttt aattctgtac cttcctagta gataagtgaa 240
gagcagggaa agagaccttt aaatattttg ctataaaaaa atttgtgata agtttctatc 300
aaaatgggga gattgcagaa aaggcttccc ttggctccca aggaggtgta gcaggtgtga 360
gcaatattag tgccatgtgc ctttcacaca gggtttgcat ttatcagtct gttttccgat 420
gatgtgtaca tgaaagagta caccatgtga agagaagaga gaatgattga aaatgtttta 480
gtatagaact cttcttgcag tgggttgcta ttttctagat ttta 524

<210> 7
<211> 562
<212> DNA
<213> human
<400> 7
gtttgtagac tctctgacca aggccacctg tgccccccag catggggccc cgggtcctgg 60
gcctgctgac gccagcaagg tggtggccaa gggcctgggg ctgagcaagg cctacgtagg 120
ccagaagagc agcttcacag tagactgcag caaagcaggc aacaacatgc tgctggtggg 180
ggttcatggc ccaaggaccc cctgcgagga gatcctggtg aagcacgtgg gcagccggct 240
ctacagcgtg tcctacctgc tcaaggacaa gggggagtac acactggtgg tcaaatgggg 300
gcacgagcac atcccaggca gcccctaccg cgttgtggtg ccctgagtct ggggcccgtg 360
ccagccggca gcccccaagc ctgccccgct acccaagcag ccccgccctc ttcccctcaa 420
ccccggccca ggccgccctg gccgcccgcc tgtcactgca gctgcccctg ccctgtgccg 480
tgctgcgctc acctgcctcc ccagccagcc gctgacctct cggctttcac ttgggcagag 540
ggagccattt ggtggcgctg ct 562

<210>  8
<211>  521
<212>  DNA
<213>  human
<400>  8
cctcacccag attgaaactc tcttcaagtc taaggactat gagttcatgt ggaaccctca      60
cctgggctac atcctcacct gcccatccaa cctgggcacc gggctgcggg caggtgtcga     120
tatcaagctg cccaacctgg gcaagcatga gaagttctcg gaggtgctta agcggctgcg     180
acttcagaag cgaggcacag gcggtgtgga cacggctgcg gtgggcgggg tcttcgacgt     240
ctccaacgct gaccgcctgg gcttctcaga ggtggagctg gtgcagatgg tggtggacgg     300
agtgaagctg ctcatcgaga tggaacagcg gctggagcag ggccaggcca tcgacgacct     360
catgcctgcc cagaaatgaa gcccggccca cacccgacac cagccctgct gcttcctaac     420
ttattgcctg cagtgcccac catgcacccc tcgatgttgc cgtctggcga gcccttagcc     480
ttgctgtaag gaaggcttcc gtcacccttg gtagagttta t                         521

<210>  9
<211>  381
<212>  DNA
<213>  human
<400>  9
ctctcttagc tcagttactc aattcatacg tagtattttt taaaataatt ttatatctgt      60
gtaccacccc atatatttca tattactgtt tcacatgtac agctttctac ttctttgtaa     120
gaacaccaac caaccaaggt ttaagtgatt aataggcttg agcaccgggt ggcagatgtt     180
ctatgcagtg tggttcaagt ttctttgacc gcacttatat gcattgctaa tatggaattt     240
aagataccat acacagtctc tcatggacct atctctattg tagaattatg acttatgtct     300
tacttggcaa atttttctga atgtgacctt tttttgctga tttgctgggt ttgggattaa     360
ctagcattat tttgccacct t                                               381

<210>  10
<211>  523
<212>  DNA
<213>  human
<400>  10
aagagttacg agttgcctga tgggcaagtg atcaccatcg gaaatgaacg tttccgctgc      60
ccagagaccc tgttccagcc atccttcatc gggatggagt ctgctggcat ccatgaaacc     120
acctacaaca gcatcatgaa gtgtgatatt gacatcagga aggacctcta tgctaacaat     180
gtcctatcag ggggcaccac tatgtaccct ggcattgccg accgaatgca gaaggagatc     240
acggccctag cacccagcac catgaagatc aagatcattg cccctccgga gcgcaaatac     300
tctgtctgga tcggtggctc catcctggcc tctctgtcca ccttccagca gatgtggatc     360
agcaaacagg aatacgatga agccgggcct tccattgtcc accgcaaatg cttctaaaac     420
actttcctgc tcctctctgt ctctagcaca caactgtgaa tgtcctgtgg aattatgcct     480
tcagttcttt tccaaatcat tcctagccaa agctctgact cgt                       523

<210>  11
<211>  513
<212>  DNA
<213>  human
<400>  11
gacgaccagc caggatatgc tctcaatcat ggagaaattg gaattcttcg atttttctta      60
tgaccttaac ctgtgtgggc tgacagagga cccagatctt caggtttctg cgatgcagca     120
ccagacagtg ctggaactga cagagactgg ggtggaggcg gctgcagcct ccgccatctc     180
tgtggcccgc accctgctgg tctttgaagt gcagcagccc ttcctcttcg tgctctggga     240
ccagcagcac aagttccctg tcttcatggg gcgagtatat gaccccaggg cctgagacct     300
gcaggatcag gttagggcga gcgctacctc tccagcctca gctctcagtt gcagccctgc     360
tgctgcctgc ctggacttgc ccctgccacc tcctgcctca ggtgtccgct atccaccaaa     420
agggctcctg agggtctggg caagggacct gcttctatta gcccttctcc atggccctgc     480
catgctctcc aaaccacttt ttgcagcttt ctc                                  513

<210>  12
<211>  360
<212>  DNA
<213>  human

<400> 12
gcaataactc tgggaggggc tcgagagggc tggtccttat ttatttaact tcacccgagt 60
tcctctgggt ttctaagcag ttatggtgat gacttagcgt caagacattt gctgaactca 120
gcacattcgg gaccaatata tagtgggtac atcaagtcca tctgacaaaa tggggcagaa 180
gagaaaggac tcagtgtgtg atccggtttc tttttgctcg cccctgtttt ttgtagaatc 240
tcttcatgct tgacatacct accagtatta ttcccgacga cacatataca tatgagaata 300
taccttattt atttttgtgt aggtgtctgc cttcacaaat gtcattgtct actcctagaa 360

<210> 13
<211> 453
<212> DNA
<213> human
<400> 13
gcatcctcaa acatggcgcc aaggataaac acgactaggc catccccagc cccctgacac 60
ccagcccccg ccagtcaccc ctccccgcac acacccgtcc ataccagctc cctgcccatg 120
accctcgctc agggatcccc ctttgagggt tagggtccca gttcccagtg gaagaaacag 180
gccaggagag tgcgtgccga gctgaggcag atgttcccac agtgacccca gagccctggg 240
ctatagtctc tgacccctcc aaggaaagac caccttctgg ggacatgggc tggagggcag 300
gacctagagg caccaaggga accgcattcc ggggctgttc cccgaggagg aagggaagcc 360
tctgtgtgcc ccccaggagg aagaggccct gagtcctggg atcagacacc ccttcacgtg 420
tatcccacac aaatgcaagc tcaccaaggt ccc 453

<210> 14
<211> 436
<212> DNA
<213> human
<400> 14
tatagctctc tttagctcaa ccactctgtc catccagcca atggatgtcc ttccctgtac 60
ccaattcaag cttattttag ggaagccttg aaactaccat gtatctggct ctagctgagt 120
tattgaggat tgagccagtg caacgttaaa ctcagtgcac ttacatttga tttaaatgat 180
ggttttatct gttgtgtgaa gtggttcacc cttgaggacc aggagcctcc atatcctgac 240
tgaaaacctt ttctgagact tagagtaaca gtacttttgg ttccttgagt tctcctgtct 300
ccagatacct aaatgacctt gacttttctg ccttgtgaat tcgtagtcca atcagctgaa 360
attaaatcac ttgggaggga cgcatagaag gagctctagg aacacagtgc cagtgcagaa 420
gtttctccag gtggcc 436

<210> 15
<211> 525
<212> DNA
<213> human
<400> 15
ctcagagcca cccctaaaga gatcctttga tattttcaac gcagccctgc tttgggctgc 60
cctggtgctg ccacacttca ggctcttctc ctttcacaac cttctgtggc tcacagaacc 120
cttggagcca atggagactg tctcaagagg gcactggtgg cccgacagcc tggcacaggg 180
cagtgggaca gggcatggcc aggtggccac tccagacccc tggcttttca ctgctggctg 240
ccttagaacc tttcttacat tagcagtttg ctttgtatgc actttgtttt tttctttggg 300
tcttgttttt tttttccact tagaaattgc atttcctgac agaaggactc aggttgtctg 360
aagtcactgc acagtgcatc tcagcccaca tagtgatggt tcccctgttc actctactta 420
gcatgtccct accgagtctc ttctccactg gatggaggaa aaccaagccg tggcttcccg 480
ctcagccctc cctgcccctc ccttcaacca ttccccatgg gaaat 525

<210> 16
<211> 398
<212> DNA
<213> human
<400> 16
ctcctggact caatcatggc ttgtggtctg gtcgccagca acctgaatct caaacctgga 60
gagtgccttc gagtgcgagg cgaggtggct cctgacgcta agagcttcgt gctgaacctg 120
ggcaaagaca gcaacaacct gtgcctgcac ttcaaccctc gcttcaacgc ccacggcgac 180
gccaacacca tcgtgtgcaa cagcaaggac ggcggggcct gggggaccga gcagcgggag 240
gctgtctttc ccttccagcc tggaagtgtt gcagaggtgt gcatcacctt cgaccaggcc 300
aacctgaccg tcaagctgcc agatggatac gaattcaagt tccccaaccg cctcaacctg 360
gaggccatca actacatggc agctgacggt gacttcaa 398

<210> 17
<211> 489
<212> DNA
<213> human
<400> 17
ggcagcattc aagtccgatg gttcctgaat ggacaggagg aaacagctgg ggtcgtgtcc 60
accaacctga tccgtaatgg agactggacc atccagatcc tggtgatgct ggaaatgacc 120
ccccagcagg gagacgtcta catctgccaa gtggagcaca ccagcctgga tagttccgtc 180
accgtggagt ggaaggcaca gtctgattct gcccagagta agacattgac gggagctggg 240
ggcttcgtgc tggggctcat catctgtgga gtgggactct tcatgcacag gaggagcaag 300
aaagttcaac gaggatctgc ataaacaggg ttcctgacct caccgaaaag actaatgtgc 360
cttagaacaa gcatttgctg tgttttgtta acacctggtt ccaggacaga ccctcagctt 420
cccaagagga tactgctgcc aagaagttgc tctgaagtca gtttctatcg ttctgctctt 480
tgattcaaa 489

<210> 18
<211> 459
<212> DNA
<213> human
<400> 18
agaggcggga tactttcagc tttccatgta actgtatgca taaagccaat gtagtccagt 60
ttctaagatc atgttccaag ctaactgaat cccacttcaa tacacactca tgaactcctg 120
atggaacaat aacaggccca agcctgtggt atgatgtgca cacttgctag actcagaaaa 180
aatactactc tcataaatgg gtgggagtat tttggtgaca acctactttg cttggctgag 240
tgaaggaatg atattcatat attcatttat tccatggaca tttagttagt gctttttata 300
taccaggcat gatgctgagt gacactcttg tgtatatttc caaatttttg tatagtcgct 360
gcacatattt gaaatcatat attaagactt tccaaagatg aggtccctgg tttttcatgg 420
caacttgatc agtaaggatt tcacctctgt ttgtaacta 459

<210> 19
<211> 493
<212> DNA
<213> human
<400> 19
gactttttgg aatagccctg tctagggcaa actgtggccc ccaggagaca ctacccttcc 60
atgccccaga cctctgtctt gcatgtgaca attgacaatc tggactaccc caagatggca 120
cccaagtgtt tggcttctgg ctacctaagg ttaacatgtc actagagtat ttttatgaga 180
gacaaacatt ataaaaatct gatggcaaaa gcaaaacaaa atggaaagta ggggaggtgg 240
atgtgacaac aacttccaaa ttggctcttt ggaggcgaga ggaaggggag aacttggaga 300
atagtttttg ctttgggggt agaggcttct tagattctcc cagcatccgc ctttcccttt 360
agccagtctg ctgtcctgaa acccagaagt gatggagaga aaccaacaag agatctcgaa 420
ccctgtctag aaggaatgta tttgttgcta aatttcgtag cactgtttac agttttcctc 480
catgttattt atg 493

<210> 20
<211> 300
<212> DNA
<213> human
<400> 20
gccacgattg gagaatactt tgcttcatag tattggagca catgttactg cttcattttg 60
gagcttgtgg agttgatgac tttctgtttt ctgtttgtaa attatttgct aagcatattt 120
tctctaggct tttttccttt tggggttcta cagtcgtaaa agagataata agattagttg 180
gacagtttaa agcttttatt cgtcctttga caaaagtaaa tgggagggca ttccatccct 240
tcctgaaggg ggacactcca tgagtgtctg tgagaggcag ctatctgcac tctaaactgc 300

<210> 21
<211> 511
<212> DNA
<213> human
<400> 21
ttaggtcaag ttcatagttt ctgtaattgg cttttgaatc aaagaatagg gagacaatct 60
aaaaaatatc ttaggttgga gatgacagaa atatgattga tttgaagtgg aaaaagaaat 120

tctgttaatg ttaattaaag taaaattatt ccctgaattg tttgatattg tcacctagca 180
gatatgtatt acttttctgc aatgttatta ttggcttgca ctttgtgagt atctatgtaa 240
aaatatatat gtatataaaa tatatattgc ataggacaga cttaggagtt ttgtttagag 300
cagttaacat ctgaagtgtc taatgcatta acttttgtaa ggtactgaat acttaatatg 360
tgggaaaccc ttttgcgtgg tccttaggct tacaatgtgc actgaatcgt ttcatgtaag 420
aatccaaagt ggacaccatt aacaggtctt tgaaatatgc atgtacttta tattttctat 480
atttgtaact ttgcatgttc ttgttttgtt a 511

<210> 22
<211> 299
<212> DNA
<213> human
<400> 22
caccaaatta cctaggctga ggttagagag attggccagc aaaaactgtg ggaagatgaa 60
ctttgtcatt atgatttcat tatcacatga ttatagaagg ctgtcttagt gcaaaaaaca 120
tacttacatt tcagacatat ccaaagggaa tactcacatt ttgttaagaa gttgaactat 180
gactggagta aaccatgtat tcccttatct tttacttttt ttctgtgaca tttatgtctc 240
atgtaatttg cattactctg gtggattgtt ctagtactgt attgggcttc ttcgttaat 299

<210> 23
<211> 280
<212> DNA
<213> human
<400> 23
accagctcta ggtccaattg ttctgctcta actgatacct caaccttggg gccagcatct 60
cccactgcct ccaaatatta gtaactatga ctgacgtccc cagaagtttc tgggtctacc 120
acactcccca accccccact cctacttcct gaagggccct cccaaggcta catccccacc 180
ccacagttct ccctgagaga gatcaacctc cctgagatca accaaggcag atgtgacagc 240
aagggccacg gaccccatgg caggggtggc gtcttcatga 280

<210> 24
<211> 428
<212> DNA
<213> human

<220>
<221> misc_feature
<222> (110)..(110)
<223> n is a, c, g, or t
<220>
<221> misc_feature
<222> (143)..(143)
<223> n is a, c, g, or t
<220>
<221> misc_feature
<222> (146)..(146)
<223> n is a, c, g, or t
<220>
<221> misc_feature
<222> (166)..(166)
<223> n is a, c, g, or t
<220>
<221> misc_feature
<222> (179)..(179)
<223> n is a, c, g, or t

<400> 24
tgtggatgtt tccaagcctg acctcacggc tgccctgcgt gacgtacgtc agcaatatga 60
aagtgtggct gccaagaacc tgcaggaggc agaagaatgg tacaaatccn agtttgctga 120
cctctctgag gctgccaacc ggnacnatga cgccctgcgc caggcnaagc aggagtccnc 180
tgagtaccgg agacaggtgc agtccctcac ctgtgaagtg gatgccctta aaggaaccaa 240
tgagtccctg gaacgccaga tgcgtgaaat ggaagagaac tttgccgttg aagctgctaa 300
ctaccaagac actattggcc gcctgcagga tgagattcag aatatgaagg aggaaatggc 360

tcgtcacctt cgtgaatacc aagacctgct caatgttaag atggcccttg acattgagat 420
tgccacct 428

<210> 25
<211> 362
<212> DNA
<213> human
<400> 25
gatccagaaa tacttaacac gtgaatattt tgctaaaaaa gcatatataa ctattttaaa 60
tatccattta tcttttgtat atctaagact catcctgatt tttactatca cacatgaata 120
aaggcctttg tatctttctt tctctaatgt tgtatcatac tcttctaaaa cttgagtggc 180
tgtcttaaaa gatataaggg gaaagataat attgtctgtc tctatattgc ttagtaagta 240
tttccatagt caatgatggt ttaataggta aaccaaaccc tataaacctg acctccttta 300
tggttaatac tattaagcaa gaatgcagta cagaattgga tacagtacgg atttgtccaa 360
at 362

<210> 26
<211> 456
<212> DNA
<213> human
<400> 26
ctccagcagt tctctcgaat actttgaatg ttgtgtaaca gttagccact gctggtgttt 60
atgtgaacat tcctatcaat ccaaattccc tctggagttt catgttatgc ctgttgcagg 120
caaatgtaaa gtctagaaaa taatgcaaat gtcacggcta ctctatatac ttttgcttgg 180
ttcatttttt ttccctttta gttaagcatg actttagatg ggaagcctgt gtatcgtgga 240
gaaacaagag accaactttt tcattccctg cccccaattt cccagactag atttcaagct 300
aattttcttt ttctgaagcc tctaacaaat gatctagttc agaaggaagc aaaatccctt 360
aatctatgtg caccgttggg accaatgcct taattaaaga atttaaaaaa gttgtaatag 420
agaatatttt tggcattcct ctcaatgttg tgtgtt 456

<210> 27
<211> 421
<212> DNA
<213> human
<400> 27
caagtgcagc gatggggagc gggcccgggc ggagctcaat gacaaagtcc acaagctgca 60
gaatgaagtt gagagcgtca cagggatgct taacgaggcc gaggggaagg ccattaagct 120
ggccaaggac gtggcgtccc tcagttccca gctccaggac acccaggagc tgcttcaaga 180
agaaacccgg cagaagctca acgtgtctac gaagctgcgc cagctggagg aggagcggaa 240
cagcctgcaa gaccagctgg acgaggagat ggaggccaag cagaacctgg agcgccacat 300
ctccactctc aacatccagc tctccgactc gaagaagaag ctgcaggact ttgccagcac 360
cgtggaagct ctggaagagg ggaagaagag gttccagaag gagatcgaga acctcaccca 420
g 421

<210> 28
<211> 563
<212> DNA
<213> human

<220>
<221> misc_feature
<222> (297)..(298)
<223> n is a, c, g, or t

<400> 28
tcaagtccaa gttcaagtcc accatcgcgg cgctggaggc caagattgca cagctggagg 60
agcaggtcga gcaggaggcc agagagaaac aggcggccac caagtcgctg aagcagaaag 120
acaagaagct gaaggaaatc ttgctgcagg tggaggacga gcgcaagatg gccgagcagt 180
acaaggagca ggcagagaaa ggcaatgcca gggtcaagca gctcaagagg cagctggagg 240
aggcagagga ggagtcccag cgcatcaacg ccaaccgcag gaagctgcag cgggagnngg 300
atgaggccac ggagagcaac gaggccatgg gccgcgaggt gaacgcactc aagagcaagc 360
tcagaggccc ccccccacag gaaacttcgc agtgatgcac caggcgagga aacgagacct 420
ctttcgttcc ttctagaagg tctggaggac gtagagttat tgaaaatgca gatggttctg 480

aggaggaaac ggacactcga gacgcagact tcaatggaac caaggccagt gaataagcaa 540
ctttctacag ttttgcacca cgg 563

<210> 29
<211> 539
<212> DNA
<213> human
<400> 29
tgagccagga agaacgctcc agcccaggac ttcgaggctg caatgagcta taattgcatc 60
attgcactcc agcctgggca acagagaccc tgtctcaacc accaccacca ccaccacccc 120
tactacccct gtattcaagg taaaaattga agtttgtatg atgtaagaga tgagaaaaac 180
ccaacaggaa acacagacac atcctccagt tctatcaatg gattgtgcag acactgagtt 240
tttagaaaaa catatccacg gtaaccggtc cctggcaatt ctgtttacat gaaatgggga 300
gaaagtcacc gaaatgggtg ccgccggccc ccactcccaa ttcattccct aacctgcaaa 360
cctttccaac ttctcacgtc aggcctttga gaattctttc cccctctcct ggtttccaca 420
cctcagacac gcacagttca ccaagtgcct tctgtagtca catgaattga aaaggagacg 480
ctgctcccac ggaggggagc aggaatgctg cactgtttac accctgactg tgcttaaaa 539

<210> 30
<211> 202
<212> DNA
<213> human
<400> 30
tgaagtgcaa caaggccatc acatctggag gaatcactta ccaggatcag ccctggcatg 60
ccgattgctt tgtgtgtgtt acctgctcta agaagctggc tgggcagcgt ttcaccgctg 120
tggaggacca gtattactgc gtggattgct acaagaactt tgtggccaag aagtgtgctg 180
gatgcaagaa ccccatcact gg 202

<210> 31
<211> 357
<212> DNA
<213> human
<400> 31
cagggctgtc atcaacatgg atatgacatt tcacaacagt gactagttga atcccttgta 60
acgtagtagt tgtctgctct ttgtccatgt gttaatgagg actgcaaagt cccttctgtt 120
gtgattccca ggacttttcc tcaagaggaa atctggattt ccacctaccg cttacctgaa 180
atgcaggatc acctacttac tgtattctac attattatat gacatagtat aatgagacaa 240
tatcaaaagt aaacatgtaa tgacaataca tactaacatt cttgtaggag tggttagaga 300
agctgatgcc tcatttctac attctgtcat tagctattat catctaacgt ttcagtg 357

<210> 32
<211> 445
<212> DNA
<213> human
<400> 32
cttgagacca ggagacgtat ccagcaagcg gaacctctgg gaaaagcaat ctgtggataa 60
ggtcacttcc cccactaagg tttgagacag ttccagaaag aacccaagct caagacgcag 120
gacgagctca gttgtagagg gctaattcgc tctgttttgt atttatgttg atttactaaa 180
ttgggttcat tatcttttat ttttcaatat cccagtaaac ccatgtatat tatcactata 240
tttaataatc acagtctaga gatgttcatg gtaaaagtac tgcctttgca caggagcctg 300
tttctaaaga aacccatgct gtgaaataga gacttttcta ctgatcatca taactctgta 360
tctgagcagt gataccaacc acatctgaag tcaacagaag atccaagttt aaaattgcct 420
gcggaatgtg tgcagtatct agaaa 445

<210> 33
<211> 496
<212> DNA
<213> human
<400> 33
tgttgtttct gcactttata ataaagcatg gaagaaatta tcttagtagg caattgtaac 60
actttttgaa agtaacccat ttcagatttg aaatactgca ataatggttg tctttaaaaa 120
aaaaaagaat gtactgttaa ggtattactt tttttcatgc tgatgattca tatctaaatt 180
acattattat gttagctgac agtggtactg attttttagg ttggttgttt tgtggatttc 240

tttagtagtg atagtagcct gaaccacatt ttagataact caattatgta tgtatgtgca 300
tacacatata caaacacact aatggtagaa tgctttttta tgtgctagac tattatattt 360
agtagtatgt cattgtaact agccaatatc acagcttttg aaaaattaaa aaatcatcat 420
cactataata tttcatattt gccaacagaa acatggcaga taggtatcaa tatgttttca 480

atgcctgatg acctat 496

<210> 34
<211> 535
<212> DNA
<213> human
<400> 34
ttttaccaaa gcatcaatac aaccagccca accatcggtc cacacctggg catttggtga 60
gaatcaaagc tgaccatgga tccctggggc caacggcaac agcatgggcc tcacctcctc 120
tgtgatttct ttctttgcac caaagacatc agtctccaac atgtttctgt tttgttgttt 180
gattcagcaa aaatctccca gtgacaacat cgcaatagtt ttttacttct cttaggtggc 240
tctgggatgg gagaggggta ggatgtacag gggtagtttg ttttagaacc agccgtattt 300
tacatgaagc tgtataatta attgtcatta tttttgttag caaagattaa atgtgtcatt 360
ggaagccatc ccttttttta catttcatac aacagaaacc agaaaagcaa tactgtttcc 420
attttaagga tatgattaat attattaata taataatgat gatgatgatg atgaaaacta 480
aggatttttc aagagatctt tctttccaaa acatttctgg acagtacctg attgt 535

<210> 35
<211> 436
<212> DNA
<213> human
<400> 35
   gagtggacta ttaaatgtgc ctaaatgaat tttgcagtaa ctggtattct tgggttttcc 60
   tacttaatac acagtaattc agaacttgta ttctattatg agtttagcag tcttttggag 120
   tgaccagcaa ctttgatgtt tgcactaaga ttttatttgg aatgcaagag aggttgaaag 180
   aggattcagt agtacacata caactaattt atttgaacta tatgttgaag acatctacca 240
   gtttctccaa atgccttttt taaaactcat cacagaagat tggtgaaaat gctgagtatg 300
   acacttttct tcttgcatgc atgtcagcta cataaacagt tttgtacaat gaaaattact 360
   aatttgtttg acattccatg ttaaactacg gtcatgttca gcttcattgc atgtaatgta 420
   gacctagtcc atcaga 436

<210> 36
<211> 534
<212> DNA
<213> human
<400> 36
tgctgtgtga accgtcgtgt gagtgtggta tgcctgatca cagatggatt ttgttataag 60
catcaatgtg acacttgcag gacactacaa cgtgggacat tgtttgtttc ttccatattt 120
ggaagataaa tttatgtgta gacttttttg taagatacgg ttaataacta aaatttattg 180
aaatggtctt gcaatgactc gtattcagat gcctaaagaa agcattgctg ctacaaatat 240
ttctattttt agaaagggtt tttatggacc aatgccccag ttgtcagtca gagccgttgg 300
tgtttttcat tgtttaaaat gtcacctgta aaatgggcat tatttatgtt tttttttttg 360
cattcctgat aattgtatgt attgtataaa gaacgtctgt acattgggtt ataacactag 420
tatatttaaa cttacaggct tatttgtaat gtaaaccacc attttaatgt actgtaatta 480
acatggttat aatacgtaca atccttccct catcccatca cacaactttt tttg 534

<210> 37
<211> 551
<212> DNA
<213> human
<400> 37
ccatccagaa tctagcgctg cgcaacacag gaatggagac gcccacaggc gtgtgcgccg 60
cactggcggc ggcaggtgtg cagccccaca gcctagacct cagccacaac tcgctgcgcg 120
ccaccgtaaa ccctagcgct ccgagatgca tgtggtccag cgccctgaac tccctcaatc 180
tgtcgttcgc tgggctggaa caggtgccta aaggactgcc agccaagctc agagtgctcg 240
atctcagctg caacagactg aacagggcgc cgcagcctga cgagctgccc gaggtggata 300
acctgacact ggacgggaat cccttcctgg tccctggaac tgccctcccc cacgagggct 360
caatgaactc cggcgtggtc ccagcctgtg cacgttcgac cctgtcggtg ggggtgtcgg 420

gaaccctggt gctgctccaa ggggcccggg gctttgccta agatccaaga cagaataatg 480
aatggactca aactgccttg gcttcagggg agtcccgtca ggacgttgag gacttttcga 540
ccaattcaac c 551

<210> 38
<211> 305
<212> DNA
<213> human
<400> 38
caaatgatgt gcaaaacctt ttactggttg catggaaatc agccaagttt tataatcctt 60
aaatcttaat gttcctcaaa gcttggatta aatacatatg gatgttactc tcttgcacca 120
aattatcttg atacattcaa atttgtctgg taaaaaaata ggtggtagat attgaggcca 180
agaatattgc aaaatacatg aagcttcatg cacttaaaga agtattttta gaataagaat 240
ttgcatactt acctagtgaa acttttctag aattattttt cactctaagt catgtatgtt 300
tctct 305

<210> 39
<211> 456
<212> DNA
<213> human
<400> 39
tcagcagtat agggaccttc cgcacaagct ctgtgttaag attgacaata atagtggggc 60
cattttcatt ttagtctttt ctaagagtca accacaggca tttaagtcag ccaaagaata 120
ttgttacctt aaagcactat tttatttata gatatatcta gtgcatctac atctctatac 180
tgtacactca cccataattc aaacaattac accatggtat aaagtgggca tttaatatgt 240
aaagattcaa agtttgtctt tattactata tgtaaattag acattaatcc actaaactgg 300
tcttcttcaa gagagctaag tatacactat ctggtgaaac ttggattctt tcctataaaa 360
gtgggaccaa gcaatgatga tcttctgtgg tgcttaagga aacttactag agctccacta 420
acagtctcat aaggaggcag ccatcataac cattga 456

<210> 40
<211> 476
<212> DNA
<213> human
<400> 40
gtgccaatcc tttgaatgtt ccacggaaac actggtggac agattctagt gctgagaaga 60
aacacgtttg gtttggagag tccatggatg gtggttttca gtttagctac ggcaatcctg 120
aacttcctga agatgtcctt gatgtgcagc tggcattcct tcgacttctc tccagccgag 180
cttcccagaa catcacatat cactgcaaaa atagcattgc atacatggat caggccagtg 240
gaaatgtaaa gaaggccctg aagctgatgg ggtcaaatga aggtgaattc aaggctgaag 300
gaaatagcaa attcacctac acagttctgg aggatggttg cacgaaacac actggggaat 360
ggagcaaaac agtctttgaa tatcgaacac gcaaggctgt gagactacct attgtagata 420
ttgcacccta tgacattggt ggtcctgatc aagaatttgg tgtggacgtt ggccct 476

<210> 41
<211> 376
<212> DNA
<213> human
<400> 41
cctggttctg gaatcctcag ttcaaggtta tcctacgcag agagccaggt accaatgggt 60
gcgctgcaat ccagacagta attctgcaaa ctgccttgaa gaaaaaggac caatgttcga 120
actacttcca ggtgaatcca acaagatccc ccgtctgagg actgaccttt ttccaaagac 180
gagaatccag gacttgaatc gtatcttccc actttctgag gactactctg gatcaggctt 240
cggctccggc tccggctctg gatcaggatc tgggagtggc ttcctaacgg aaatggaaca 300
ggattaccaa ctagtagacg aaagtgatgc tttccatgac aaccttaggt ctcttgacag 360
gaatctgccc tcagac 376

<210> 42
<211> 482
<212> DNA
<213> human
<400> 42
tatgctttaa tgctgttatc tatcttattg ttcttgaaaa tacctgcatt ttttggtatc 60

atgttcaacc aacatcatta tgaaattaat tagattccca tggccataaa atggctttaa 120
agaatatata tatattttta aagtagcttg agaagcaaat tggcaggtaa tatttcatac 180
ctaaattaag actctgactt ggattgtgaa ttataatgat atgccccttt tcttataaaa 240
acaaaaaaaa aataatgaaa cacagtgaat ttgtagagtg ggggtatttg acatatttta 300
cagggtggag tgtactatat actattacct ttgaatgtgt ttgcagagct agtggatgtg 360
tttgtctaca agtatgattg ctgttacata acaccccaaa ttaactccca aattaaaaca 420
cagttgtgct gtcaatacct catactgctt tacctttttt tcctggatat ctgtgtattt 480
tc 482

<210> 43
<211> 570
<212> DNA
<213> human
<400> 43
gttgagtcgt catcactttt cagtgatggg agagtagatg gtgaaattta ttagttaata 60
tatcccagaa attagaaacc ttaatatgtg gacgtaatct ccacagtcaa agaaggatgg 120
cacctaaacc accagtgccc aaagtctgtg tgatgaactt tctcttcata ctttttttca 180
cagttggctg gatgaaattt tctagacttt ctgttggtgt atcccccccc tgtatagtta 240
ggatagcatt tttgatttat gcatggaaac ctgaaaaaaa gtttacaagt gtatatcaga 300
aaagggaagt tgtgcctttt atagctatta ctgtctggtt ttaacaattt cctttatatt 360
tagtgaacta cgcttgctca tttttcttta cataattttt tattcaagtt attgtacagc 420
tgtttaagat gggcagctag ttcgtagctt tcccaaataa actctaaaca ttaatcaatc 480
atctgtgtga aaatgggttg gtgcttctaa cctgatggca cttagctatc agaagaccac 540
aaaaattgac tcaaatctcc agtattcttg 570

<210> 44
<211> 508
<212> DNA
<213> human
<400> 44
tctcctacat ccgcattgct gataccaata tcaccagcat tcctcaaggt cttcctcctt 60
cccttacgga attacatctt gatggcaaca aaatcagcag agttgatgca gctagcctga 120
aaggactgaa taatttggct aagttgggat tgagtttcaa cagcatctct gctgttgaca 180
atggctctct ggccaacacg cctcatctga gggagcttca cttggacaac aacaagctta 240
ccagagtacc tggtgggctg gcagagcata agtacatcca ggttgtctac cttcataaca 300
acaatatctc tgtagttgga tcaagtgact tctgcccacc tggacacaac accaaaaagg 360
cttcttattc gggtgtgagt cttttcagca acccggtcca gtactgggag atacagccat 420
ccaccttcag atgtgtctac gtgcgctctg ccattcaact cggaaactat aagtaattct 480
caagaaagcc ctcattttta taacctgg 508

<210> 45
<211> 499
<212> DNA
<213> human
<400> 45
gtatcaggct tcaattccat tatgttttaa tgttgtctct gaagatgact tgtgattttt 60
ttttcttttt tttaaaccat gaagagccgt ttgacagagc atgctctgcg ttgttggttt 120
caccagcttc tgccctcaca tgcacaggga tttaacaaca aaaatataac tacaacttcc 180
cttgtagtct cttatataag tagagtcctt ggtactctgc cctcctgtca gtagtggcag 240
gatctattgg catattcggg agcttcttag agggatgagg ttctttgaac acagtgaaaa 300
tttaaattag taactttttt gcaagcagtt tattgactgt tattgctaag aagaagtaag 360
aaagaaaaag cctgttggca atcttggtta tttctttaag atttctggca gtgtgggatg 420
gatgaatgaa gtggaatgtg aactttgggc aagttaaatg ggacagcctt ccatgttcat 480
ttgtctacct cttaactga 499

<210> 46
<211> 453
<212> DNA
<213> human
<400> 46
gttgtgccta ccactggctg gcacaccagg gcaatgattt ccctgcagaa ggaaggaaag 60
aatgtttcac ccttgcatcc ttcttggaga agctacagcc tgtgctcagt tgagtggttc 120
acactcagac tttggcttta tggttttcct tcctccttgt ctttgccctg accttgatca 180

acaggggtga aaagaaccac cctgaggttt ccatgcctct cccattttag tggtagcatt 240
ttgtgtcttt actccaccct tcaccctagt tccaccaagg ttcacacacc agatgtaact 300
gtttttcagc tgagttgtat ggattaactt cagtccactg taaatacacc tgggatgggg 360
tggggttggg gttgtttagg gagaagcagc cagacttgct ttgtgaactg aatgtatttt 420
tatgcaattt tgagtggcct ttcaacccta aga 453

<210> 47
<211> 594
<212> DNA
<213> human

<220>
<221> misc_feature
<222> (42)..(42)
<223> n is a, c, g, or t

<400> 47
tatcagtagc tcgtgttatc tttttatcaa ctgcttccca gngtcctaaa acaatagaaa 60
ttttggattg aaaagttcag cataaggagt ttgagtcagt aaaggatggg ataaaggagt 120
cgagatgatt caatgaaaag tatcacaaaa aagagattga tcaacaagag aaataaaaaa 180
gcccaagagg aagtggtagg ggaaggaatt taagaacagc aataagtaaa actcttaagt 240
aactccaaaa agaaaatggt acattttgcc aaagaccact tatacttgag aacatggaag 300
aatttgcctg atactctctt tggggaaaag agtctctcct cttttcctca aaccccagta 360
cactcagcct ctctgcccca ccttctcctg actttgtcct cacttgcttc tgcagtacat 420
tggaacctga attgaaagaa agtcttcctt gaataattgg agtttgtctt gagaggcaaa 480
tatagcccca agaatcacaa gattcgagga ccatgtaggt cttttacgta gcccaaatcc 540
ataaattagt ctcacttttt gtatttatcg tttcatatta aaccctctat atca 594

<210> 48
<211> 335
<212> DNA
<213> human
<400> 48
taacacttgg ctcttggtac ctgtgggtta gcatcaagtt ctccccaggg tagaattcaa 60
tcagagctcc agtttgcatt tggatgtgta aattacagta atcccatttc ccaaacctaa 120
aatctgtttt tctcatcaga ctctgagtaa ctggttgctg tgtcataact tcatagatgc 180
aggaggctca ggtgatctgt ttgaggagag caccctaggc agcctgcagg gaataacata 240
ctggccgttc tgacctgttg ccagcagata cacaggacat ggatgaaatt cccgtttcct 300
ctagtttctt cctgtagtac tcctctttta gatcc 335

<210> 49
<211> 464
<212> DNA
<213> human
<400> 49
cagtcactct aaatggacac cacatgaacc tctgtttaga atacctacgt atgtatgcat 60
tggtttgctt gtttcttgac agtacatttt tagatctggc cttttcttaa caaaatctgt 120
gcaaaagatg caggtggatg tccctaggtc tgttttcaaa gaactttttc caagtgcttg 180
ttttatttat taagtgtcta cctggtaaat gttttttttg taaactctga gtggactgta 240
tcatttgcta ttctaaacca ttttacactt aagttaaaat agtttctctt cagctgtaaa 300
taacaggata cagaattaac aagagaaaat gtctaacttt ttaagaaaaa ccttattttc 360
ttcggttttt gaaaaacata atggaaataa aacaggatat tgacataata gcacaaaatg 420
acactcttct aaaactaaat gggcacaaga gaattttcct ggga 464

<210> 50
<211> 499
<212> DNA
<213> human
<400> 50
gtcaaaacct aattgcaatt ctgttaaatc taagtaattt ttagacagtg tttcaccgta 60
ttatttagga tgtgaaatgc catttctttc actgattaca ccatatacag gaaacaggta 120
aaacagtgaa aactttattg tgctggttga tgccaacttg gttgaaaagc tctctgcaga 180

agaagtgatc tagactgaca gaagtgttgc taattacaag ttgtgttctc atgacgtaat 240
tagaaagtaa cttctcaaag tacaactttt atgaaaaaaa taagctgtta aaaaaaggaa 300
atcgtaggtt aatttaattg ggaaaatggg caattgacag agaccatttt cctaacacat 360
atatgtgcta gtactttaac tttttaaaat tttacttcta cgttttgtaa tataaaaatt 420
tctattttaa gtttagaatg ttatacgtac cgaaagtatg cagccaaatc gatcagatca 480
aaccatttta cctggagtt 499

<210> 51
<211> 174
<212> DNA
<213> human
<400> 51
agctgtgagc cttggctgtt ggcagtgagt gagcctggct cttgtgctgg atggagccca 60
ggcgggagcg gtggccctgt ccctcccacc tctgtgacct gaggcctacg ctttggctct 120
ggagatagcc ccagagcagg gtgttgggat actgcagggc caggactgag cccc 174

<210> 52
<211> 531
<212> DNA
<213> human
<400> 52
ctgttagctc ctcactgtgg taaatgccac acacctttaa gtagataagc agacgatagt 60
tatctgttct tttgacttaa tctcatttgg tttgattttc cctctactaa ggctttccta 120
ccttcttcag gctgcctaag acatgtaagc gaaacacttc aataattgtc catgaggaga 180
aaaaaagcat tgtcatgcat gaaggaaact gaacttgagg tggcctcctt gcttgttaca 240
tacctgggta tgtgtaggca gtttagtgca tctttgcctc tcagttgaaa cctgtataac 300
cctgttacaa agctgtgttg ttgcttcttg tgaaggccat gatattttgt tttttcccca 360
attaattgct attgtgttat tttactaact tctctctgta ttttttcttg cattgacatt 420
atagacattg aggacctcat ccaaacaatt taaaaatgag tgtgaagggg gaacaagtca 480
aaatattttt aaaagatctt caaaaataat gcctctgtct agcatgccaa c 531

<210> 53
<211> 467
<212> DNA
<213> human
<400> 53
tgtcagagtt ttcaacggtg cttatagctg ccagctggat tccaaacagg taccccattg 60
tctctgagct aatgtttata tttttccatt caggcaccga aatagttaat atttaaaata 120
agtcttcaaa agaaaacata agagattatt gagttcttgg gactggatcc tttatttcat 180
aagttcagat catcttaaat gaaaatgcca tgattatctg cagttaagta gatgacagct 240
attctacatc agacttgatt tttgtcagct aattacataa ttggtaagct ataattgaaa 300
ccttatggct taaaattcct taactccttt ttgattcatg tttgtagtca tgttgtcaac 360
agaggcaaag ttaagcttga tgatggttaa aatcggtttg atagcaccat gggacatttt 420
tctaacaaaa ataaatgcat gaagagacat agccttttag ttttgct 467

<210> 54
<211> 445
<212> DNA
<213> human
<400> 54
aagagctatg agctgccaga tgggcaggtt atcaccattg gcaatgagcg cttccgctgc 60
cctgagaccc tcttccagcc ttcctttatt ggcatggagt ccgctggaat tcatgagaca 120
acctacaatt ccatcatgaa gtgtgacatt gacatccgta aggacttata tgccaacaat 180
gtcctctctg ggggcaccac catgtaccct ggcattgctg acaggatgca gaaggagatc 240
acagccctgg cccccagcac catgaagatc aagattattg ctcccccaga gcggaagtac 300
tcagtctgga tcgggggctc tatcctggcc tctctctcca ccttccagca gatgtggatc 360
agcaagcctg agtatgatga ggcagggccc tccattgtcc acaggaagtg cttctaaagt 420
cagaacaggt tctccaagga tcccc 445

<210> 55
<211> 525
<212> DNA
<213> human

<400>  55
caactgccct tgaccggaag catgagtatc atcttcttcc tgcccctgaa agtgacccag      60
aatttgacct tgatagagga gagcctcacc tccgagttca ttcatgacat agaccgagaa     120
ctgaagaccg tgcaggcggt cctcactgtc cccaagctga agctgagtta cgaaggcgaa     180
gtcaccaagt ccctgcagga gatgaagctg caatccttgt ttgattcacc agactttagc     240
aagatcacag gcaaacccat caagctgact caggtggaac accgggctgg ctttgagtgg     300
aacgaggatg gggcgggaac cacccccagc ccagggctgc agcctgccca cctcaccttc     360
ccgctggact atcaccttaa ccagcctttc atcttcgtac tgagggacac agacacaggg     420
gcccttctct tcattggcaa gattctggac cccaggggcc cctaatatcc cagtttaata     480
ttccaatacc ctagaagaaa acccgaggga cagcagattc cacag                     525

<210>  56
<211>  444
<212>  DNA
<213>  human
<400>  56
ctgacctgca ggacgaaacc atgaagagcc tgatccttct tgccatcctg gccgccttag      60
cggtagtaac tttgtgttat gaatcacatg aaagcatgga atcttatgaa cttaatccct     120
tcattaacag gagaaatgca aataccttca tatcccctca gcagagatgg agagctaaag     180
tccaagagag gatccgagaa cgctctaagc ctgtccacga gctcaatagg gaagcctgtg     240
atgactacag actttgcgaa cgctacgcca tggtttatgg atacaatgct gcctataatc     300
gctacttcag gaagcgccga ggggccaaat gagactgagg gaagaaaaaa aatctctttt     360
tttctggagg ctggcacctg attttgtatc cccctgtagc agcattactg aaatacatag     420
gcttatatac aatgcttctt tcct                                            444

<210>  57
<211>  444
<212>  DNA
<213>  human
<400>  57
gaagccctgg aaaatcgcct gagatacaga tgaagattag aaatcgcgac acatttgtag      60
tcattgtatc acggattaca atgaacgcag tgcagagccc caaagctcag gctattgtta     120
aatcaataat gttgtgaagt aaaacaatca gtactgagaa acctggtttg ccacagaaca     180
aagacaagaa gtatacacta acttgtataa atttatctag gaaaaaaatc cttcagaatt     240
ctaagatgaa tttaccaggt gagaatgaat aagctatgca aggtattttg taatatactg     300
tggacacaac ttgcttctgc ctcatcctgc cttagtgtgc aatctcattt gactatacga     360
taaagtttgc acagtcttac ttctgtagaa cactggccat aggaaatgct gtttttttgt     420
actggacttt accttgatat atgt                                            444

<210>  58
<211>  450
<212>  DNA
<213>  human
<400>  58
gctggtatca gaacagcttc cctcactgtg tacagaacgc aagaagggaa taggtggtct      60
gaacgtggtg tctcactctg aaaagcagga atgtaagatg atgaaagaga caatgtaata     120
ctgttggtcc aaaagcattt aaaatcaata gatctgggat tatgtggcct taggtagctg     180
gttgtacatc tttccctaaa tcgatccatg ttaccacata gtagttttag tttaggattc     240
agtaacagtg aagtgtttac tatgtgcaag ggtattgaag ttcttatgac cacagatcat     300
cagtactgtt gtctcatgta atgctaaaac tgaaatggtc cgtgtttgca ttgttaaaaa     360
tgatgtgtga aatagaatga gtgctatggt gttgaaaact gcagtgtccg ttatgagtgc     420
caaaaatctg tcttgaaggc agctacactt                                      450

<210>  59
<211>  474
<212>  DNA
<213>  human
<400>  59
aacctgaaaa catcccagcc aagaactggt ataggagctc caaggacaag aaacacgtct      60
ggctaggaga aactatcaat gctggcagcc agtttgaata taatgtagaa ggagtgactt     120
ccaaggaaat ggctacccaa cttgccttca tgcgcctgct ggccaactat gcctctcaga     180
acatcaccta ccactgcaag aacagcattg catacatgga tgaggagact ggcaacctga     240
aaaaggctgt cattctacag ggctctaatg atgttgaact tgttgctgag ggcaacagca     300

ggttcactta cactgttctt gtagatggct gctctaaaaa gacaaatgaa tggggaaaga 360
caatcattga atacaaaaca aataagccat cacgcctgcc cttccttgat attgcacctt 420
tggacatcgg tggtgctgac caggaattct ttgtggacat tggcccagtc tgtt 474

<210> 60
<211> 421
<212> DNA
<213> human
<400> 60
gagccattgg aagactgtcc tctatggcaa tgatctcagg gctcagtggc aggaaatcct 60
caacagggtc accaaccagc ccgctcaatg cagaaaaact agaatctgaa gatgtgtccc 120
aagctttcct tgaggctgtt gctgaggaaa agcctcatgt aaaaccctat ttctctaaga 180
ccattcgcga tttagaagtt gtggagggaa gtgctgctag atttgactgc aagattgaag 240
gatacccaga ccccgaggtt gtctggttca aagatgacca gtcaatcagg gagtcccgcc 300
acttccagat agactacgat gaggacggga actgctcttt aattattagt gatgtttgcg 360
gggatgacga tgccaagtac acctgcaagg ctgtcaacag tcttggagaa gccacctgca 420
c 421

<210> 61
<211> 502
<212> DNA
<213> human
<400> 61
ttatcaagtg tcaagatcag caagtgtcct taagtcaaat aggttttttt ttgttggtgg 60
ttgtgcttgc tttccttttt tagaaagttc tagaaaatag gaaaacgaaa aatttcattg 120
agatgagtag tgcatttaat tattttttaa aaaacttttt aagtacttga attttatatc 180
aggaaaacaa agttgttgag ccttgcttct tccgttttgc cctttgtctc gctccttatt 240
cttttttggg gggagggtta tttgcttttt tatcttcctg gcataatttc cattttattc 300
ttctgagtgt ctatgttaac ttccctctat cccgcttata aaaaaattct ccaacaaaaa 360
tacttgttga cttgatgttt tatcacttct ctaagtaagg ttgaaatatc cttattgtag 420
ctactgtttt taatgtaaag gttaaacttg aaaagaaatt cttaatcacg gtgccaaaat 480
tcattttcta acaccatgtg tt 502

<210> 62
<211> 541
<212> DNA
<213> human

<220>
<221> misc_feature
<222> (163)..(164)
<223> n is a, c, g, or t
<400> 62
agattctaac ggtctgttct gtttcaaggc actctagatt ccattggtcc aagattccgg 60
atcctaagca tctaagttat aagactctca cactcagttg tgactaacta gacaccaaag 120
ttctaataat ttctaatgtt ggacaccttt aggttctttg ctnnattctg cctctctagg 180
accatggtta agagtccaag aatccacatt tctaaaatct tatagttcta ggcactgtag 240
ttctaagact caaatgttct aagtttctaa gattctaaag gtccacaggt ctagactatt 300
aggtgcaatt tcaaggttct aaccctatac tgtagtattc tttggggtgc ccctctcctt 360
cttagctatc attgcttcct cctccccaac tgtgggggtg tgcccccttc aagcctgtgc 420
aatgcattag ggatgcctcc tttccgcagg ggatggacga tctcccacct ttcgggccat 480
gttgcccccg tgagccaatc cctcaccttc tgagtacaga gtgtggactc tggtgcctcc 540
a 541

<210> 63
<211> 496
<212> DNA
<213> human
<400> 63
ggctctggag gtgggcagca atctgtcaat caccttgtta aagagattga tatgctgctg 60
aaagaatatt tactctctgg agacatatct gaagctgaac attgccttaa ggaactggaa 120
gtacctcatt ttcaccatga gcttgtatat gaagctatta taatggtttt agagtcaact 180

ggagaaagta catttaagat gattttggat ttattaaagt ccctttggaa gtcttctacc 240
attactgtag accaaatgaa aagaggttat gagagaattt acaatgaaat tccggacatt 300
aatctggatg tcccacattc atactctgtg ctggagcggt ttgtagaaga atgttttcag 360
gctggaataa tttggaaaca actcagagat ctttgtcctt caaggggcag aaagcgtttt 420
gtaagcgaag gagatggagg tcgtcttaaa ccagagagct actgaatata agaactcttg 480
cagtcttaga tgttat 496

<210> 64
<211> 507
<212> DNA
<213> human
<400> 64
gtaagattcc tcctaacttt cacagtcgat gacaagattg tctttttatc tgatattttg 60
aagggtatat tgctttgaag taagtctcaa taaggcaata tattttaggg catctttctt 120
cttatctctg acagtgttct taaaattatt tgaatatcat aagagccttg gtgtctgtcc 180
taattccttt ctcactcacc gatgctgaat acccagttga atcaaactgt caacctacca 240
aaaacgatat tgtggcttat gggtattgct gtctcattct tggtatattc ttgtgttaac 300
tgcccattgg cctgaaaata ctcattgtaa gcctgaaaaa aaaaatcttt cccactgttt 360
tttctgcttg ttgtaagaat caaatgaaat aatgtatgtg aaagcacctt gtaaactgta 420
acctatcaat gtaaaatgtt aaggtgtgtt gttatttcat taattacttc tttgtttaga 480
atggaatttc ctatgcacta ctgtagc 507

<210> 65
<211> 450
<212> DNA
<213> human
<400> 65
tacacttttt tttgccaact gacttaacaa cattgctgtc aggtggaaat ttcaagcact 60
tttgcacatt tagttcagtg tttgttgaga atccatggct taacccactt gttttgctat 120
ttttttcttt gcttttaatt ttccccatct gattttatct ctgcgtttca gtgacctacc 180
ttaaaacaac acacgagaag agttaaactg ggttcatttt aatgatcaat ttacctgcat 240
ataaaattta tttttaatca agctgatctt aatgtatata atcattctat ttgctttatt 300
atcggtgcag gtaggtcatt aacaccactt cttttcatct gtaccacacc ctggtgaaac 360
ctttgaagac ataaaaaaaa cctgtctgag atgttctttc taccaatcta tatgtctttc 420
ggttatcaag tgtttctgca tggtaatgtc 450

<210> 66
<211> 549
<212> DNA
<213> human

<220>
<221> misc_feature
<222> (76)..(76)
<223> n is a, c, g, or t

<400> 66
aaactactaa ccactgcaag ctcttgtcaa attttagttt aattggcatt gcttgttttt 60
tgaaactgaa attacntgag tttcattttt tctttgaatt tatagggttt agatttctga 120
aagcagcatg aatatatcac ctaacatcct gacaataaat tccatccgtt gttttttttg 180
tttgtttgtt ttttcttttc ctttaagtaa gctctttatt catcttatgg tgcagcaatt 240
ttaaaatttg aaatatttta aattgttttt gaactttttg tgtaaaatat atcagatctc 300
aacattgttg gtttcttttg tttttcattt tgtacaactt tcttgaattt agaaattaca 360
tctttgcagt tctgttaggt gctctgtaat taacctgact tatatgtgaa caattttcat 420
gagacagtca tttttaacta atgcagtgat tctttctcac tactatctgt attgtggaat 480
gcacaaaatt gtgtaggtgc tgaatgctgt aaggagttta ggttgtatga attctacaac 540
cctataata 549

<210> 67
<211> 489
<212> DNA
<213> human
<400> 67

gagatactat ttttgctgag caaccagtgt gtttcaggat gatacaaaga aaaatataga 60
atagaaataa gtgcaggctt ggaatcagct acaaatccta aagatggggt gtgtgtggat 120
gtgtgtgtgt gtgtgtgtac accattgtgt gtttgtaaaa tgtgtatgtt catgagtaag 180
ggtgtgtgtg tgtgtgtatt aaaattccag agtgaccgtg gcacttgggt gtacaggtaa 240
ttcctccaga gctgtttgct ggcttcagga gtggagtgag aatttctttt ttatgaaaag 300
ggatataaag gcaccgagct gatgcagtat ttgtaatatt aagttgacct aacaaggtat 360
ttgcatgagt cacaattaca aagttttgag cggttttgta atttgacatt taggaaagtc 420
tcctatttat tctcatactt tacattcatg cttagtatac tatagaggat gccagcttta 480
atctttctg 489

<210> 68
<211> 543
<212> DNA
<213> human
<400> 68
gctttcaatt gatggactac tctatttttt gcaaatttgt aaactttgct tctccaaata 60
caagtactag gttgtccatt tatggtacct atttggtgct agtaaatttt caaactagat 120
ttataaatgc actgtaatat gtacacaact tagaaaccaa attacaagta ttcagttcca 180
atacttcatt aatttcaatc aaccaaagtt agttcagtag cttatctcag ttatgagtat 240
aatacattac atgtaaatta agtgtgtgta tactgtaatc gtgctatttt ttatcattga 300
aacatttata aactagaata ataatgccct taatgtgagg gtttgtaatg gtgcttatta 360
agaccaaaga cttgttaaat gtatacacca agtggtaatg aaatttcgtg actggcccac 420
acgtgcatag aggtctggga ggaccaggaa acagcctcag tggccagagg atcaccagtg 480
catccttcat cacagcatgt gcaatatgcc aagattaccc tcggtcattc ctgtcaacaa 540
ggg 543

<210> 69
<211> 500
<212> DNA
<213> human
<400> 69
gcgtgagtgt gtgagcgctt ctgcagcctc ggcctaggtc acgttggccc tcaaagcgag 60
ccgttgaatt ggaaactgct tctagaaact ctggctcagc ctgtctcggg ctgacccttt 120
tctgatcgtc tcggcccctc tgattgttcc cgatggtctc tctccctctg tcttttctcc 180
tccgcctgtg tccatctgac cgttttcact tgtctccttt ctgactgtcc ctgccaatgc 240
tccagctgtc gtctgactct gggttcgttg gggacatgag attttatttt ttgtgagtga 300
gactgaggga tcgtagattt ttacaatctg tatctttgac aattctgggt gcgagtgtga 360
gagtgtgagc agggcttgct cctgccaacc acaattcaat gaatccccga cccccctacc 420
ccatgctgta cttgtggttc tctttttgta ttttgcatct gaccccgggg ggctgggaca 480
gattggcaat gggccgtccc 500

<210> 70
<211> 482
<212> DNA
<213> human
<400> 70
agaaagaggc gctgctcact gttttcctgc ttcagttttt ctcttatagt accatcacta 60
taatcaacga acttctcttc tccacccaga gatggctttt ccaacacatt ttaattaaag 120
gaactgagta cattaccctg atgtctaaat ggaccaaaga tctgagatcc attgtgatta 180
tatctgtatc aggtcagcag aagaaggaac tgagcagttg aactctgagt tcatcaattc 240
taatatttgg aaattatcta caatggaatc ttccctctgt tctctgataa cctacttgct 300
tactcaatgc ctttaagcca agtcaccctg ttgcctatgg gaggaggtgg aaggatttgg 360
caagctcaac cacatgctat ttagttagca tcagttgtca ccaacagtct ttctgcaaag 420
ggcaggagag ctttggggga aaggaaaagg cttaccaggc tgctatggtc aactcttcag 480
aa 482

<210> 71
<211> 508
<212> DNA
<213> human
<400> 71
agccctggag aagacgaaag ccaacatcaa gtgggtgaag gagaacaagg aggtggtgct 60
ccagtggttc acagaaaaca gcaaatagtc cccagccctt gaagtcaccc ggccccgatg 120

caaggtgccc acatgtgtcc atcccagcgg ctggtgcagg gcctccattc ctggagcccg 180
aggcaccagt gtcctcccct caaggacaaa gtctccagcc cacgttctct ctgcctgtga 240
gccagtctag ttcctgatga cccaggctgc ctgagcacct cccagcccct gcccctcatg 300
ccaaccccgc cctaggcctg gcatggcacc tgtcgcccag tgccctgggg ctgatctcag 360
ggaagcccag ctccagggcc agatgagcag aagctctcga tggacaatga acggccttgc 420
tgggggccgc cctgtaccct ctttcacctt tccctaaaga ccctaaatct gaggaatcaa 480
cagggcagca gatctgtata ttttttttc 508

<210> 72
<211> 460
<212> DNA
<213> human
<400> 72
attatctggt cttttcctgt tgtgcaaaaa tgactcattg ctccgaatgt caaaaacaaa 60
tgcgacaaac aatggcactt catcatttaa agtaatgttg ccaagagaaa aaatttcctg 120
ggagggaggt ttcccacaag ccaaatctcc taagcctcaa atgctagcac tttttggcag 180
ttggatagga aatgagacat tctttggcag ccaaaataag agaggccgat ggtgaaactt 240
tttgagacac cctatggcct tcttgtcaaa accttcactg gagctcaaga aaagcatttc 300
tgttgtgtta tttgcagtgc agatgatgtc tgtgtaacaa cataatggtt attcaccttt 360
ttttgatttt gatttttgct gtgttatcaa aaacttgaat actgtgagaa gaagtgaatt 420
ttcagttgac gaatcagcat cttgttccca tggtgataac 460

<210> 73
<211> 551
<212> DNA
<213> human
<400> 73
cttcgaccac gtgatcacca acatgaacaa caattatgag ccccgcagtg gcaagttcac 60
ctgcaaggtg cccggtctct actacttcac ctaccacgcc agctctcgag ggaacctgtg 120
cgtgaacctc atgcgtggcc gggagcgtgc acagaaggtg gtcaccttct gtgactatgc 180
ctacaacacc ttccaggtca ccaccggtgg catggtcctc aagctggagc agggggagaa 240
cgtcttcctg caggccaccg acaagaactc actactgggc atggagggtg ccaacagcat 300
cttttccggg ttcctgctct ttccagatat ggaggcctga cctgtgggct gcttcacatc 360
caccccggct ccccctgcca gcaacgctca ctctaccccc aacaccaccc cttgcccagc 420
caatggacac agtagggctt ggtgaatgct gctgagtgaa tgagtaaata aactcttcaa 480
ggccaaggaa cagtggtcta attcaactct gtgtcccagc actggcacac cagaagtgcc 540
atgctcagaa a 551

<210> 74
<211> 542
<212> DNA
<213> human
<400> 74
tgatgagctt tcctttgatc cggacgacgt aatcactgac attgagatgg tggacgaggg 60
ctggtggcgg ggacgttgcc atggccactt tggactcttc cctgcaaatt atgtcaagct 120
tctggagtga ctagagctca ctgtctactg caactgtgat ttcccatgtc caaagtggct 180
ctgctccacc ccctccctat tcctgatgca aatgtctaac cagatgagtt tctggacaga 240
cttccctctc ctgcttcatt aagggcttgg ggcagagaca gcatggggaa ggaggtcccc 300
ttccccaaga gtcctctcta tcctggatga gctcatgaac atttctcttg tgttcctgac 360
tccttcccaa tgaacacctc tctgccaccc caagctctgc tctcctcctc tgtgagctct 420
gggcttccca gtttgtttac ccgggaaagt acgtctagat tgtgtggttt gcctcattgt 480
gctatttgcc cactttcctt ccctgaagaa atatctgtga accttctttc tgttcagtcc 540
ta 542

<210> 75
<211> 436
<212> DNA
<213> human
<400> 75
gctggaatac ttactcttgt cgggagattg aaccactaaa atgttagagc agaattcatt 60
atgctgtggt cacaggggtg tcttgtctga gaacaaatac aattcagtct tctctttggg 120
gttttagtat gtgtcaaaca taggactgga agtttgcccc tgttcttttt tcttttgaaa 180
gaacatcagt tcatgcctga ggcatgagtg actgtgcatt tgagatagtt ttccctattc 240

tgtggataca gtcccagagt tttcagggag tacacaggta gattagtttg aagcattgac 300
cttttattta ttccttattt ctctttcatc aaaacaaaac agcagctgtg ggaggagaaa 360
tgagagggct taaatgaaat ttaaaataag ctatattata caaatactat ctctgtattg 420
ttctgaccct ggtaaa 436

<210> 76
<211> 379
<212> DNA
<213> human

<220>
<221> misc_feature
<222> (170)..(170)
<223> n is a, c, g, or t

<400> 76
aatgcgaagg ctaagtgtca ccccctttct ctgcctctgg ctgggccttg ctaagggcca 60
aggaaagaaa gacatttttt agggggcagc cagtccaaat gccaaaagaa gaccagttct 120
tgccctgatt gtatgaaatt tgacattttg gcactttttt tttttttttn ggccaatcag 180
attttctatg ttctaaggac atggctgctg tagaatagca cagacgtgga tgataaatta 240
tccccagaag cagcatgaca gaatgcctcg gggagcactt ggaagggaaa ttgcagttct 300
gttgaaatag aggaaaatcc cttggtaaag acacagcctg ttaggctcgt gtgggcctcc 360
agtatgttca ccaggggaa 379

<210> 77
<211> 451
<212> DNA
<213> human
<400> 77
aacgatgtca catgcgtgtt cgaccccgtg cacaccatct cccacaccgt catctcgctg 60
cctaccttcc gcgagttcac ccgccctgaa gagatcatct tcctccgggc catcacgcca 120
ccgcatcctg ccagccaggc taacatcatc ttcgacatca cggaagggaa cctgcgggac 180
tcttttgaca tcatcaagcg ttacatggac ggcatgaccg tgggtgtcgt gcgccaggtg 240
cggcccatcg tgggcccatt tcatgccgtc ctgaagctgg agatgaacta tgtggtcggg 300
ggcgtggttt cccaccgaaa tgttgtcaac gtccgcatct tcgtctctga gtactggttc 360
tgagggctgg tctgccgcac agccgcaggt gcacctccag gccaaatcat tgctgccagt 420
gactgtggtc tgtacttgtt tataccctca g 451

<210> 78
<211> 500
<212> DNA
<213> human

<220>
<221> misc_feature
<222> (410)..(411)
<223> n is a, c, g, or t

<400> 78
gacagacctg agaccaatct gggtagaagc aaaaagttga accttttaaa gtgctgaaca 60
caaatccaaa ttcgaatggt tcaagcagcc gtgaaatcgc tcttcataaa gtgggcttaa 120
ttctctagtt taagttcttt tgatggaatg aattaattaa tgtgtcaggt ggcttatttg 180
tggatgccat gattgatgat gttcatttta agctcttacc tatagtacaa gtacatgatg 240
ctactgaata tttttccact tggaaactgt gagctggttg ttgcattaaa acacacatac 300
aaacaaaatc aaaaacactg cggactttca ctcaagctgg tctttcttcc ccagtgtaag 360
gcaatcctgc ctactaacaa caccaacaac aaaacactcc atctgtgaan ntgacgcagt 420
taagggggct aggcagggca tttgtgccaa ctaagaatca ccagataccc accataagta 480
cctatcgcag ttttgaagtc 500

<210> 79
<211> 480
<212> DNA
<213> human

<400> 79
acctcgcaac atcaacatct atacttacga tgatatggaa gtgaagcaaa tcaacaaacg 60
tgcctctggc caggcttttg agctgatctt gaagccacca tctcctatct cagaagcccc 120
acgaacttta gcttctccaa agaagaaaga cctgtccctg gaggagatcc agaagaaact 180
ggaggctgca ggggaaagaa gaaagtctca ggaggcccag gtgctgaaac aattggcaga 240
gaagagggaa cacgagcgag aagtccttca gaaggctttg gaggagaaca acaacttcag 300
caagatggcg gaggaaaagc tgatcctgaa aatggaacaa attaaggaaa accgtgaggc 360
taatctagct gctattattg aacgtctgca ggaaaaggag aggcatgctg cggaggtgcg 420
caggaacaag gaactccagg ttgaactgtc tggctgaagc aagggagggt ctggcacgcc 480

<210> 80
<211> 510
<212> DNA
<213> human
<400> 80
atggcctctg tgaataatgt aactccagtt acacggtgac ttttaatagc atacagtgat 60
ttgatgaaag gacgtcaaac aatgtggcga tgtcgtggaa agttatcttt cccgctcttt 120
gctgtggtca ttgtgtcttg cagaaaggat ggccctgatg cagcagcagc gccagctgta 180
ataaaaaata attcacacta tcagactagc aaggcactag aactggaaaa gaccacagaa 240
aacaaagaat ccaacccttt catcttacag gtgaacaaac tgtgatgatg cacatgtatg 300
tgttttgtaa gctgtgagca ccgtaacaaa atgtaaattt gccattatta ggaaagtgct 360
ggtggcagtg aagaagcacc caggccactt gactcccagt ctggtgccct gtctacacca 420
gacaacacag gagctgggtc agattcccct cagctgctta acaaagttcc tcgaacagaa 480
agtgcttaca aagctgcctt ctcggatact 510

<210> 81
<211> 560
<212> DNA
<213> human
<400> 81
agctgccttc tcggatactg aaaggtcgag ttttctgaac tgcactgatt ttattgcagt 60
tgaaaaaccc aaagctattc caaagatttc aagctgttct gagacatctt ctgatggctt 120
tacttcctga gaggcaatgt ttttacttta tgcataattc attgttgcca aggaataaag 180
tgaagaaaca gcaccttttt aatatatagg tctctctgga agagacctaa atttagaaag 240
agaaaactgt gacaattttc atattctcat tcttaaaaaa cactaatctt aactaacaaa 300
agttcttttg agaataagtt acacacaatg gccacagcag tttgtcttta atagtatagt 360
gcctatactc atgtaatcgg ttactcacta ctgcctttaa aaaaaaccag catatttatt 420
gaaaacatga gacaggatta tagtgcctta accgatatat tttgtgactt aaaaaataca 480
tttaaaactg ctcttctgct ctagtaccat gcttagtgca aatgattatt tctatgtaca 540
actgatgctt gttcttattt 560

<210> 82
<211> 561
<212> DNA
<213> human
<400> 82
ttcagttgct aatgaggctc ctccgctctg gacacaaccc ttttatagat taatttctct 60
gccaattaac ttgtcatttt cagtacatat tttactattc cacaccaacc ataattacaa 120
caagggattt ttcttatgca ctcctatgca tgtgaataac atgtggtgta attctgcttc 180
ttacagaagt attactgaag gtattatttc caatattatt tggtttatta tgcggatctt 240
ttttatatat gcagtcccat cccttctgtg ccactcaatg ccatccagac atggtttttc 300
cctccagggg cctttctctc cagagggcac ttcggctgcc tctgcttcct ctcattcgag 360
gcccggctct tgctgacaga ataggttccg ttctgggcgg tggttctcga gcctgccatt 420
caaaaccaaa gcaaattgga gcatttctca caacatggta ttgaagttcc tttttgttct 480
caaaagttgt gaccgtgtta aattgtactc ccttagtcct gtaaggtatg ttaagtgaat 540
cgcagttacg ctgtactttt a 561

<210> 83
<211> 458
<212> DNA
<213> human
<400> 83
agaaaatttg ccaatctttc ctactttcta tttttatgat gacaatcaaa gccggcctga 60

gaaacactat ttgtgacttt ttaaacgatt agtgatgtcc ttaaaatgtg gtctgccaat 120
ctgtacaaaa tggtcctatt tttgtgaaga gggacataag ataaaatgat gttatacatc 180
aatatgtata tatgtatttc tatatagact tggagaatac tgccaaaaca tttatgacaa 240
gctgtatcac tgccttcgtt tatatttttt taactgtgat aatccccaca ggcacattaa 300
ctgttgcact tttgaatgtc caaaatttat attttagaaa taataaaaag aaagatactt 360
acatgttccc aaaacaatgg tgtggtgaat gtgtgagaaa aactaacttg atagggtcta 420
ccaatacaaa atgtattacg aatgcccctg ttcatgtt 458

<210> 84
<211> 543
<212> DNA
<213> human
<400> 84
ccaggactac agaataccat cccctggtac cgtgtagttg ccgaagtcca gatctgccat 60
ggcaaaacgg aggctgtggg ccaggtccac atcttcttcc aggatgggat ggtgacgttg 120
actccaaaca agggtgtgtg ggtgaatggt ctccgagtgg atctcccagc tgagaagtta 180
gcatctgtgt ccgtgagtcg tacacctgat ggctccctgc tagtccgcca gaaggcaggg 240
gtccaggtgt ggcttggagc caatgggaag gtggctgtga ttgtcagcaa tgaccatgct 300
gggaaactgt gtggggcctg tggaaacttt gacggggacc agaccaatga ttggcatgac 360
tcccaggaga agccagcgat ggagaaatgg agagcgcagg acttctcccc atgttatggc 420
tgatcagtca tccaccagga acgaagattt cctgaagaag acctggtccc tctggaggtt 480
gcggtggctg aaggatgcat catgtgctcc taccctgctc taccgctttt ctgggtcaca 540
gag 543

<210> 85
<211> 401
<212> DNA
<213> human
<400> 85
aaagacaagc attgggtcag acccataaac cacctcccaa aggctgtcat ttcattgcac 60
tggaattttg ctttatcaga agcaaggaag taagggagtc attgccttgg gcctgggaat 120
ctaagtggga gacaatatta atttggatcc gattaattgg agattactaa ctgtggacaa 180
aagtttatct ttgcacaatc aataaaaatg gcattttttt agtaaattaa gagcataaac 240
aatattgcta gaggtggcat gtttagtcta ccaaaaacaa tacttttcag gcactttaga 300
aatatccttt tagaagcagc gagtgcatgg gctaattatc atcaatcttt atgtatttgt 360
taaagaaaca tctacaggat ctttattggt gaccttttgt a 401

<210> 86
<211> 454
<212> DNA
<213> human
<400> 86
gtccttcaca tcaccatttt gagacctcag cttggcactc aggtgctgaa gggtaatatg 60
gactcagcct tgcaaatagc cagtgctagt tctgacccaa ccacagagga tgctgacatc 120
atttgtatta tgttccaagg ctactacaga gaaggctgcc tgctatgtat ttgcaaggct 180
gatttatggt cagaatttcc ctctgatatg tctagggtgt gatttaggtc agtagactgt 240
gattcttagc aaaaaatgaa cagtgataag tatactgggg gcaaaatcag aatggaatgc 300
tctggtctat ataaccacat ttctgagcct ttgagactgt tcctgagcct tcagcactaa 360
cctatgaggg tgagctggtc ccctctatat atacatcata cttaacttta ctaagtaatc 420
tcacagcatt tgccaagtct cccaatatcc aatt 454

<210> 87
<211> 492
<212> DNA
<213> human
<400> 87
atgtgtcact gattttttag ctcaaaatca tcactgttaa tttccagtca ccccaaatat 60
ggttaaaaga tttttttttt taatcatgaa gagaaaatta gtagcatttc tttctctccc 120
cattatttat tggttttcct cactaatctt ttttttttta gtccaaaagc caaaaatatt 180
tatcttggtt ttacatttta atttccattc ttaattgtaa tttttttctt taaataagga 240
aaccaatata atctcatgta taaaaactta aatattttac aagttacata tagcatcatt 300
ctaaaataag aatttttttt gttttctgtc tgcttttttc ttatgtctct tgttgagttt 360
tatattttca gtggttattt ttgcttgtgt tagatcatta ttaaaatata tccaatgtcc 420

ctttgatact tgtgctctgc tgagaatgta cagtttgcat taaacatccc aggtctcatc 480
cttcaggaat tt 492

<210> 88
<211> 444
<212> DNA
<213> human
<400> 88
gaagcgcctg gcagtgtacc aggccggggc ccgcgagggc gccgagcgcg gcctcagcgc 60
catccgcgag cgcctggggc ccctggtgga acagggccgc gtgcgggccg ccactgtggg 120
ctccctggcc ggccagccgc tacaggagcg ggcccaggcc tggggcgagc ggctgcgcgc 180
gcggatggag gagatgggca gccggacccg cgaccgcctg gacgaggtga aggagcaggt 240
ggcggaggtg cgcgccaagc tggaggagca ggcccagcag atacgcctgc aggccgaggc 300
cttccaggcc cgcctcaaga gctggttcga gcccctggtg gaagacatgc agcgccagtg 360
ggccgggctg gtggagaagg tgcaggctgc cgtgggcacc agcgccgccc ctgtgcccag 420
cgacaatcac tgaacgccga agcc 444

<210> 89
<211> 500
<212> DNA
<213> human
<400> 89
actgtgatat aggtactctg atttaaaact ttggacatcc tgtgatctgt tttaaagttg 60
gggggtggga aatttagctg actagggaca aacatgtaaa cctattttcc tatgaaaaaa 120
gttttaaatg tcccacttga ataacgtaat tcttcatagt ttttttaatc tatggataaa 180
tggaaaccta attatttgta atgaattatt tagacagttc taagccctgt cttctgggag 240
ttatcaattt taaagagaac ttttgtgcaa ttcaaatgaa gtttttataa gtaattgaaa 300
atgacaacac aataacactt tctgtataaa agtatatatt ttatgtgatt tattcctact 360
aaatgaaagt gcactactgc ctcatgtaaa gactcttgca cgcagagcct ttaagtgact 420
aaggaacaac atagatagtg agcatagtcc ccacctccac ccctcacaat ttatttgaat 480
acttcaattg tgcctctcaa 500

<210> 90
<211> 444
<212> DNA
<213> human
<400> 90
ttcagacagt tatatcctcc ttttaaacca ttgttgttga gtgtaagatg tccttcatgt 60
tttcttataa agtcagtgtt tagaaatgtt accctttcta agttatatac agatcaaatg 120
cttttttctt tcacgtacat ccatcatttg caactgctgt tcgtacacag aaacaggact 180
gctcaaatga tcctatttgt attttctgat gctatcagac tctaatgttt ttttccctaa 240
aatattattg ccatcatgct ttaggaattt ttatattttt acacaatcat attttagtat 300
ggtgtctgtt tatgtaactc tgacttgctg gaaaagttga aactccaaat aatctgaaac 360
tagaaaagaa atagcacata attactacct tccccttggc ggctctcctc ccccaacccc 420
caccccacaa ttttatgact tcca 444

<210> 91
<211> 435
<212> DNA
<213> human
<400> 91
caacgtctaa ctggacttcc caagataaat ggtaccagcg tcctcttaaa agatgcctta 60
atccattcct tgaggacaga ccttagttga aatgatagca gaatgtgctt ctctctggca 120
gctggccttc tgcttctgag ttgcacatta atcagattag cctgattctc ttcagtgaat 180
tttgataatg gcttccagac tctttgcgtt ggagacgcct gttaggatct tcaagtccca 240
tcatagaaaa ttgaaacaca gagttgttct gctgatagtt ttggggatac gtccatcttt 300
ttaagggatt gctttcatct aattctggca ggacctcacc aaaagatcca gcctcatacc 360
tacatcagac aaaatatcgc cgttgttcct tctgtactaa agtattgtgt tttgctttgg 420
aaacacccac tcact 435

<210> 92
<211> 486
<212> DNA

<213>  human
<400>  92
gccagacgct ggggccatag tgagtgtggg cacaaggaag acgctgcagt gaattgcaca      60
gatatttcag tgcagaaaac cccacaaaaa gccacaacag gtcgctcatc ccgtcagtca     120
tcctttattg cagtcgggat ccttggggtt gttctgttgg ccattttcgt cgcattattc     180
ttcttgacta aaaagcgaag acagagacag cggcttgcag tttcctcaag aggagagaac     240
ttagtccacc aaattcaata ccgggagatg aattcttgcc tgaatgcaga tgatctggac     300
ctaatgaatt cctcaggagg ccattctgag ccacactgaa aaggaaaatg ggaatttata     360
acccagtgag ttcagccttt aagatacctt gatgaagacc tggactattg aatggagcag     420
aaattcacct ctctcactga ctattacagt tgcattttta tggagttctt cttctcctag     480
gattcc                                                                486

<210>  93
<211>  522
<212>  DNA
<213>  human
<400>  93
ttttcattgt tcattgatat gctcagtatg ctgccacata agatgaattt aattatattc      60
aaccaaagca atatactctt acatgatttc taggccccat gacccagtgt ctagagacat     120
taattctaac cagttgtttg cttttaaatg agtgatttca ttttgggaaa caggtttcaa     180
atgaatatat atacatgggt aaaattactc tgtgctagtg tagtcttact agagaatgtt     240
tatggtccca cttgtatatg aaaatgtggt tagaatgtta attggataat gtatatataa     300
gaagttaaag tatgtaaagt ataacttcag ccacattttt agaacactgt ttaacatttt     360
tgcaaaacct tcttgtagga aaagagagct ctctacatga agatgacttg ttttatattt     420
cagattttat tttaaaagcc atgtctgtta aacaagaaaa aacacaaaag aactccagat     480
tcctggttca tcattctgta ttcttactca ctttttcaag tt                        522

<210>  94
<211>  465
<212>  DNA
<213>  human
<400>  94
atcctcattc ttatactgct tttcgtggcc actttggaca agtcctggtg gactctccct      60
gggaaagagt ccctgaatct ctggtacgac tgcacgtgga acaacgacac caaaacatgg     120
gcctgcagta atgtcagcga gaatggctgg ctgaaggcgg tgcaggtcct catggtgctc     180
tccctcattc tctgctgtct ctccttcatc ctgttcatgt tccagctcta caccatgcga     240
cgaggaggtc tcttctatgc caccggcctc tgccagcttt gcaccagcgt ggcggtgttt     300
actggcgcct tgatctatgc cattcacgcc gaggagatcc tggagaagca cccgcgaggg     360
ggcagcttcg gatactgctt cgccctggcc tgggtggcct tccccctcgc cctggtcagc     420
ggcatcatct acatccacct acggaagcgg gagtgagcgc cccgc                     465

<210>  95
<211>  560
<212>  DNA
<213>  human
<400>  95
caagtatcgg ggctctgcta tcaaggtgca aagtccttcg tggatgcaac ctcaaccata      60
tattctacag caccctggtg ccgtgttaac tccctcaatg gagcacacca tgtcactaca     120
gcccgcatca atgatcagcc ctctggccca gcagatgagt catctgtcac taggcagcac     180
cggaacatac atgcctgcaa cgtcagctat gcaaggagcc tacttgccac agtatgcaca     240
tatgcagacg acagcggttc ctgttgagga ggcaagtggt caacagcagg tggctgtcga     300
gacgtctaat gaccattctc catatacctt tcaacctaat aagtaactgt gagatgtaca     360
gaaaggtgtt cttacatgaa gaagggtgtg aaggctgaac aatcatggat ttttctgatc     420
aattgtgctt taggaaatta ttgacagttt tgcacaggtt cttgaaaacg ttatttataa     480
tgaaatcaac taaaactatt tttgctataa gttctataag gtgcataaaa cccttaaatt     540
catctagtag ctgttccccc                                                 560

<210>  96
<211>  442
<212>  DNA
<213>  human
<400>  96
caccttgggt ctgtgtcact tgtaggtttc tctgcctcca ggttgcctca acagcaggag      60

gcacagcagt ttcaccatct ttgaggtgag ggtggggtgc cccagctagg aagcaagatc 120
gctgtgctag gtctgaccaa aaccagaggg cagtctagtc ctgggggtaa agccctcaga 180
tcccagggta cactcttctc cattccctcc acccacttgc ctgtcacccc agtcacctaa 240
gcaatcactg ggcccagagg agaggagaca gacacacact ggctcctgga cctaaagggt 300
atgagctgga gctaaggcca gctagagctt ccactgtcag ccctcactgt cagccccact 360
gcacccccct gtgcctgctg ggcactgggc actagctaga tgctttaggt tgcttcagct 420
gatccttcaa ctctgtgagg tg 442

<210> 97
<211> 484
<212> DNA
<213> human
<400> 97
tatgtgacgc tggacctttt ctttacccaa ggatttttaa aactcagatt taaaacaagg 60
ggttacttta catcctacta agaagtttaa gtaagtaagt ttcattctaa aatcagaggt 120
aaatagagtg cataaataat tttgttttaa tctttttgtt tttcttttag acacattagc 180
tctggagtga gtctgtcata atatttgaac aaaaattgag agctttattg ctgcatttta 240
agcataatta atttggacat tatttcgtgt tgtgttcttt ataaccaccg agtattaaac 300
tgtaaatcat aatgtaactg aagcataaac atcacatggc atgttttgtc attgttttca 360
ggtactgagt tcttacttga gtatcataat atattgtgtt ttaacaccaa cactgtaaca 420
tttacgaatt atttttttaa acttcagttt tactgcattt tcacaacata tcagacttca 480
ccaa 484

<210> 98
<211> 509
<212> DNA
<213> human
<400> 98
tgtgtaatac gccaaccagt caagttgtgt tttggccaga gatttagata tgtccaattt 60
cctggctcat ttcattgtgc tctatgggta cgtataaaaa gcaagaattc tgtttcctag 120
gcaaacattg caactcaggg ctaaagtcat ccagtgaaac ttttagagcc agaagtaact 180
ttgtcccagt cctacaatgt gaaaagagtg aatagttgcc tctttttagc cattttcatg 240
gctggtacat attcgtacgc attacttttc agaatcaata cgcactttca gatattctta 300
tttttattct cttaagtctt tattaacttt ggagagagaa atgatgcatc tttttatttt 360
aaatgaagta gatcaacatg gtggaacaaa atgataaaga acagaaaaca tttcaatata 420
ttactaataa ctttttccaa tataaatcct aaaattccta taacatagta ttttacagtt 480
ttatgaagct ttctattgtg acttttatg 509

<210> 99
<211> 453
<212> DNA
<213> human

<220>
<221> misc_feature
<222> (250)..(250)
<223> n is a, c, g, or t
<400> 99
aacactcaca tacaagttca tactttaaaa gaggaaagct acttaacaat gacaaatatt 60
tcacaataat aatttttact tatataccat ctttcaactg aacatttcag ttcttccaag 120
agcttcttag agtagtatat tttgggggca gtcaaggaat aaactacagt gtaaacatat 180
cccagatgaa aactgctgta tggaaaaatg acagaaagta actgattgac actgttgatt 240
cacagttcan gcctcctatc tgggaaagac atttctttcc tctgctcact ttaagaactt 300
ttaccgactc caaaaatctc aggaattaaa cttttaacag ttacagcaat aaagaatagt 360
tagtactcca aaaatattat atttaagatg ctcaacaaga aaaaaatgca aatgtaatat 420
ttttttcaaa ttacttcttt attgacttgt cca 453

<210> 100
<211> 461
<212> DNA
<213> human
<400> 100
gatttccaaa tcagtcgttt tctcaaaaaa atatcgtata agtgactcat cctgtctgct 60

aactccagac ctcccagctt gaagccaaat ctttccatgt gagattgata tggatttcct 120
agaagtactg gaatgttgtc atatcttgcc ctattttaat tctgctatag aaaacaattg 180
ccttcacttt taaggagtaa tttgaatatt aataactctg gtctagattt tcatataatg 240
tattaaagac aaagtagtga acatcaatga acatctgata gagataaact gtaatcaggc 300
ataagcttgt ttgtatgttc tggcagtgac taatcagtaa atgatgtcgg tttgcccagt 360
atcacttatc ttctgtattt ttcctctgtc gtgtaaatag tataaccttt tcatttatgg 420
acaatttttt ggactagtag ccttcaatat acattctgct t 461

<210> 101
<211> 470
<212> DNA
<213> human
<400> 101
acgcacacaa ctactacaat tccgcctagg tccacaaggc cttcactgtt ttccccccaa 60
gggaggctgc tgctgctctt ggctggagcc agccagggcc agccgacccc ctctccctgc 120
atggcatcct ccagcccctg tagaactcaa cctctacagg gttagagttt ggagagagca 180
gactggcggg gggcccattg gggggaaggg gaccctccgc tctgtagtgc tacagggtcc 240
aacatagaac agggtgtccc caacagcgcc caaaggacgc actgagcaac gctattccag 300
ctgtcccccc actccctcac aagtgggtac ccccaggacc agaagctccc ccagcaaagc 360
ccccagagcc caggctcggc ctgcccccac cccattcccg cagtgggagc aaactgcatg 420
cccagagacc cagcggacac acgcggtttg gtttgcagcg actggcatac 470

<210> 102
<211> 476
<212> DNA
<213> human
<400> 102
ccaaatttca tttcagccac ttctgcagga tccctactgc caacctggaa tggagacttt 60
tatctacttc tctctctctg aagatgtcaa atcgtggttt agatcaaata tatttcaagc 120
tataaaagca ggaggttatc tgtgcagggg gctggcatca tgtatttagg ggcaagtaat 180
aatggaatgc tactaagata ctccatattc ttccccgaat cacacagaca gtttctgaca 240
ggcgcaactc ctccattttc ctcccgcagg tgagaaccct gtggagatga gtcagtgcca 300
tgactgagaa ggaaccgacc cctagttgag agcaccttgc agttccccga gaactttctg 360
attcacagtc tcattttgac agcatgaaat gtcctcttga agcatagctt tttaaatatc 420
tttttccttc tactcctccc tctgactcta agaattctct cttctggaat cgcttg 476

<210> 103
<211> 501
<212> DNA
<213> human
<400> 103
gtgtgatgct tttgtaggta cctggaaact tgtctccagt gaaaactttg atgattatat 60
gaaagaagta ggagtgggct ttgccaccag gaaagtggct ggcatggcca aacctaacat 120
gatcatcagt gtgaatgggg atgtgatcac cattaaatct gaaagtacct ttaaaaatac 180
tgagatttcc ttcatactgg gccaggaatt tgacgaagtc actgcagatg acaggaaagt 240
caagagcacc ataaccttag atgggggtgt cctggtacat gtgcagaaat gggatggaaa 300
atcaaccacc ataaagagaa aacgagagga tgataaactg gtggtggaat gcgtcatgaa 360
aggcgtcact tccacgagag tttatgagag agcataagcc aagggacgtt gacctggact 420
gaagttcgca ttgaactcta caacattctg tgggatatat tgttcaaaaa gatattgttg 480
ttttccctga tttagcaagc a 501

<210> 104
<211> 462
<212> DNA
<213> human
<400> 104
ggtccccaca gactcagaga gaacccacca tggtgctgtc tcctgccgac aagaccaacg 60
tcaaggccgc ctggggtaag gtcggcgcgc acgctggcga gtatggtgcg gaggccctgg 120
agaggatgtt cctgtccttc cccaccacca agacctactt cccgcacttc gacctgagcc 180
acggctctgc ccaggttaag ggccacggca agaaggtggc cgacgccctg accaacgccg 240
tggcgcacgt ggacgacatg cccaacgcgc tgtccgccct gagcgacctg cacgcgcaca 300
agcttcgggt ggacccggtc aacttcaagc tcctaagcca ctgcctgctg gtgaccctgg 360
ccgcccacct ccccgccgag ttcacccctg cggtgcacgc ctccctggac aagttcctgg 420

cttctgtgag caccgtgctg acctccaaat accgttaagc tg 462

<210> 105
<211> 520
<212> DNA
<213> human
<400> 105
gcccaggctg acttacacat tgaggatgga gactccatcc gcttcgggcg cttcgcgttg 60
gagaccaggg ccagccctgg ccacacccca ggctgtgtca ccttcgtcct gaatgaccac 120
agcatggcct tcactggaga tgccctgttg atccgtgggt gtgggcggac agacttccag 180
caaggctgtg ccaagacctt gtaccactcg gtccatgaaa agatcttcac acttccagga 240
gactgtctga tctaccctgc tcacgattac catgggttca cagtgtccac cgtggaggag 300

gagaggactc tgaaccctcg gctcaccctc agctgtgagg agtttgtcaa aatcatgggc 360
aacctgaact tgcctaaacc tcagcagata gactttgctg ttccagccaa catgcgctgt 420
ggggtgcaga cacccactgc ctgatctcac ttctgtcaga tgctcccatc cactattaat 480
gcactaggtg ggaggagagg gcggcaatga cactgcacct 520

<210> 106
<211> 515
<212> DNA
<213> human

<220>
<221> misc_feature
<222> (89)..(89)
<223> n is a, c, g, or t
<220>
<221> misc_feature
<222> (110)..(110)
<223> n is a, c, g, or t
<220>
<221> misc_feature
<222> (227)..(227)
<223> n is a, c, g, or t
<220>
<221> misc_feature
<222> (250)..(250)
<223> n is a, c, g, or t
<220>
<221> misc_feature
<222> (276)..(276)
<223> n is a, c, g, or t

<400> 106
aattgccaca ttttcttatg gcattaaaaa ttttacaaaa acataatttt aatggctata 60
ttatattcca tttaatggat gcaactcang tttatttaac cattcccatn gttgttaact 120
atttaggttg tttctaattt tcattattat aaagttgcag aaatttggtg tacataaaac 180
tgtctccata taattgatta ttaggatata ttcccatgaa ggattcnttt ttttaaaaaa 240
atgtgaaatn tcatcttgta cttacacctt tcatgnaaag ggatttcctg cttttgtact 300
gcatgggtgg cagttgtgag gaaaagccag tcaaatgacc tttttacaaa agaaatgcag 360
tggtcacttc agttgagagt gactttttaa tacaacaaga tcaactagaa gaattcaact 420
gtctcaagaa tcaaggtacc ccaatatatc tcgcaattcc aaactttgtt tgagggactc 480
gttatccagc tcttggtagc cacacctgca atgta 515

<210> 107
<211> 532
<212> DNA
<213> human
<400> 107
aagccttaca gttatcctgc aagggacagg aaggtctgat ttgcaggatt tttagagcat 60
taaaataact atcaggcaga agaatctttc ttctcgccta ggatttcagc catgcgcgcg 120

ctctctctct ttctctctct tttcctctct ctccctcttt ctagcctggg gcttgaattt 180
gcatgtctaa ttcatttact caccatattt gaattggcct gaacagatgt aaatcgggaa 240
ggatgggaaa aactgcagtc atcaacaatg attaatcagc tgttgcaggc agtgtcttaa 300
ggagactggt aggaggaggc atggaaacca aaaggccgtg tgtttagaag cctaattgtc 360
acatcaagca tcattgtccc catgcaacaa ccaccacctt atacatcact tcctgtttta 420
agcagctcta aaacatagac tgaagattta tttttaatat gttgacttta tttctgagca 480
aagcatcggt catgtgtgta ttttttcata gtcccacctt ggagcattta tg 532

<210> 108
<211> 415
<212> DNA
<213> human
<400> 108
gaaggcggct gatgagagcg agagaggaat gaaggtcatc gaaaaccggg ccatgaagga 60
tgaggagaag atggaactgc aggagatgca gctgaaggag gccaagcaca tcgctgagga 120
ttcagaccgc aaatatgaag aggtggccag gaagctggtg atcctggaag gagagctgga 180
gcgctcggag gagagggctg aggtggccga gagccgagcc agacagctgg aggaggaact 240
tcgaaccatg gaccaggccc tcaagtccct gatggcctca gaggaggagt attccaccaa 300
agaagataaa tatgaagagg agatcaaact gttggaggag aagctgaagg aggctgagac 360
ccgagcagag tttgccgaga ggtctgtggc aaagttggag aaaaccatcg atgac 415

<210> 109
<211> 366
<212> DNA
<213> human
<400> 109
catgggggga cttgaaccct gcagcaggct cctgctcctg cctctcctgc tggctgtaag 60
tggtctccgt cctgtccagg cccaggccca gagcgattgc agttgctcta cggtgagccc 120
gggcgtgctg gcagggatcg tgatgggaga cctggtgctg acagtgctca ttgccctggc 180
cgtgtacttc ctgggccggc tggtccctcg ggggcgaggg gctgcggagg cagcgacccg 240
gaaacagcgt atcactgaga ccgagtcgcc ttatcaggag ctccagggtc agaggtcgga 300
tgtctacagc gacctcaaca cacagaggcc gtattacaaa tgagcccgaa tcatgacagt 360
cagcaa 366

<210> 110
<211> 480
<212> DNA
<213> human
<400> 110
tccccagcag tggctcggtg agccatgtgc acctatggcc cagccactgc agcacaggag 60
gctccgtgag ccttggttcc tccccgaaaa cccccagcat tacgatcccc caagtgtcct 120
ggaccctggc ctaaagaatc ccacccccac ttcatgccca ctgccgatgc ccaatccagg 180
cccggtgagg ccaaggtttc ccagtgagcc tctgcgcctc tccactgttt catgagccca 240
aacaccctcc tggcacaacg ctctaccctg cagcttggag aactccgctg gatgggagtc 300
tcatgcaaga cttcactgca gcctttcaca ggactctgca gatagtgcct ctgcaaacta 360
aggagtgact aggtgggttg gggaccccct caggattgtt tctcggcacc agtgcctcag 420
tgctgcaatt gagggctaaa tcccaagtgt ctcttgactg gctcaagaat tagggcccca 480

<210> 111
<211> 475
<212> DNA
<213> human
<400> 111
cagaccggca gtcatcatgg cagtttcagc gttcaaacag caatagctca agtgtgataa 60
ctactgagga taataaaatc cacattcact taggaagtcc ttacatgcaa gctgtagcca 120
gcccttcagc accactgcag gataaccgaa ctcaaggctt aattaacggg gcactaaaca 180
aaacaaccaa taaagtcacc agcagtatta ctatcacacc aacagccaca cctcttcctc 240
gacaatcaca aattacagta agtaatatat ataactgacc acgctcaccc tcatccagtc 300
catactgata tttttgcaag gaactcaatc cttttttaat catccctcca tatcccccaa 360
gactgactga actcgtactt tgggaaggtt tgtgcatgaa ctatacaaga gtatctgaaa 420
ctaactgttg cctgcatagt catatcgagt gtgcacttac tgtatatctt ttcat 475

<210> 112

<211>  344
<212>  DNA
<213>  human

<220>
<221>  misc_feature
<222>  (173)..(173)
<223>  n is a, c, g, or t
<400>  112
gcgtgttttc ctcttgatcg ggaactcctg cttctccttg cctcgaaatg gaccccaact     60
gctcctgctc gcctgttggc tcctgtgcct gtgccggctc ctgcaaatgc aaagagtgca    120
aatgcacctc ctgcaagaag agctgctgct cctgctgccc tgtgggctgt gcnaagtgtg    180
cccagggctg catctgcaaa gggacgtcag acaagtgcag ctgctgtgcc tgatgccagg    240
acagctgtgc tctcagatgt aaatagagca acctatataa acctggattt tttttttttt    300
ttttttgtac aaccctgacc cgtttgctac atcttttttt ctat                      344

<210>  113
<211>  318
<212>  DNA
<213>  human
<400>  113
ttccacatca gtaactgccc tggggtttgt gctgtacaaa tacaagctcc tgccacggtc    60
ttgaagttct gttcttatgc tctctgctca ctggttttca ataccaccaa gaggaaaata    120
ttgacaagtt taaaggctgt gtcattgggc catgtttaag tgtactggat ttaactacct    180
ttggcttaat tccaatcatt gttaaagtaa aaacaattca aagaatcacc taattaattt    240
cagtaagatc aagctccatc ttatttgtca gtgtagatca actcatgtta attgatagaa    300
taaagccttg tgatcact                                                  318

<210>  114
<211>  411
<212>  DNA
<213>  human
<400>  114
ctttgcgggc acagagactg ccacaaagtg gagcggctac atggaagggg cagttgaggc     60
tggagaacga gcagctaggg aggtcttaaa tggtctcggg aaggtgaccg agaaagacat    120
ctgggtacaa gaacctgaat caaaggacgt tccagcggta gaaatcaccc acaccttctg    180
ggaaaggaac ctgccctctg tttctggcct gctgaagatc attggatttt ccacatcagt    240
aactgccctg gggtttgtgc tgtacaaata caagctcctg ccacggtctt gaagttctgt    300
tcttatgctc tctgctcact ggttttcaat accaccaaga ggaaaatatt gacaagttta    360
aaggctgtgt cattgggcca tgtttaagtg tactggattt aactaccttt g             411

<210>  115
<211>  454
<212>  DNA
<213>  human
<400>  115
taattgcttg ttttctagcc tggcaagata ttttcataaa agagggataa caatgctgat     60
tactaccttt taaaatattt tagataaatg cacagcacca cagcaccaca tctaagcatt    120
agtgatgggt agctgatgtc agcttcatgt ggattttaag cactctagaa acaatgaagc    180
ttcttggcat attttaagga gctcccaaaa tgtgttacct attaaattgt aactcagcaa    240
gtagaagacc atttgaaaag tcaggtacaa atttcctcaa gtggcataaa aatgtagtca    300
gttttctctt ttaccagttt ttatttccac tccaattatt tagaacttta tttgtacatg    360
tgcagaagaa taaggcagct gagaatcttg tttcccccaa gagagtttta caggctgagt    420
gttgcaaatg tgttctttgt cctgttatat gtat                                454

<210>  116
<211>  482
<212>  DNA
<213>  human
<400>  116
gcactgccat gtatgaaagt ctctttatga tgtttgtttt tttgtcattt ttgttcttta     60
catcaagaaa ttttatgttt aaatatgcgg agaatgtata ttgcctctgc tcctatcagg    120
gttgctaaac cctggtacat cgtatataaa atgtattaaa actggggttt gttaccagtt    180

gctgtacttt gtatatagaa tttttataaa ttgtatgctt cagaaataat ttatttttaa 240
aaagaaatta aaagttttaa actcacatcc atattacacc tttcccccct gaaatgtata 300
gaatccattt gtcatcagga atcaaaaccc acagtccatt gtgaagtgtg ctatatttag 360
aacagtctta aaatgtacag tgtattttat agaattgaag ttaacattct tattttcaag 420
agaatttatg gacgttgtag aaatgtacaa atgcatttcc aaactgcctt aaacgttgta 480
tt 482

<210> 117
<211> 422
<212> DNA
<213> human
<400> 117
gggactgtct gtcaaaagac tctgtatatc ttttgtggat gagttttgtg agagaacaga 60
gagaccattg tacctggcac aagggctctt catgaaaagg gagacttact gggaggtgca 120
agacagtggc atttctcctc tcctcttgct gctcagcaca gccctggatt gcagccccga 180
ggctgagacc agacaaagcc cgggaggcag aaagatgctc caagaaccaa cactatcaat 240
gtctttgcaa atcctcacag gattcctgtg ggtccagctt tggaactggg aaacctttct 300
tcggatccgc actcattcca ctgatgccag ctgcccctga aggatgccag tactgtggtg 360
tgtgagtctc agcagccgcc cacacgctcc taactctgct gcatggcaga tgcctaggtg 420
ga 422

<210> 118
<211> 357
<212> DNA
<213> human
<400> 118
attaatcagc cccagagtgc tttaaaaaat tctcttaaat aaaaataata gactcgctag 60
tcagtaaaga tatttgaata tgtatcgtgc cccctccggt gtctttgatc aggatgacat 120
gtgccatttt tcagaggacg tgcagacagg ctggcattct agattacttt tcttactctg 180
aaacatggcc tgtttgggag tgcgggattc aaaggtggtc ccaccgctgc ccctactgca 240
aatggcagtt ttaatcttat cttttggctt ctgcagatgg ttgcaattga tccttaacca 300
ataatggtca gtcctcatct ctgtcctgct tcataggtgc caccttgtgt gtttaaa 357

<210> 119
<211> 328
<212> DNA
<213> human
<400> 119
   ctacgcgtgt ccttgggcgg agaagggagg tgactccggc ggaagaggac aaggcagaat 60
   gcaggccctt cgggtgtccc aggcgctgat ccgctccttc agctccaccg cccggaaccg 120
   ctttcagaac cgagtgcgcg agaaacagaa gctcttccag gaggacaatg acatcccgtt 180
   gtacctgaag ggcggcatcg ttgacaacat cctgtaccga gtgacaatga cgctgtgtct 240
   gggcggcact gtctacagct tgtactccct tggctgggcc tccttcccca ggaattaaga 300
   ccaagaagcc tggggggcct gagagact 328

<210> 120
<211> 417
<212> DNA
<213> human
<400> 120
tggtgacaca aacagccttt tcccaggtac ttggtacctg gagcgagtgg acgagcagca 60
tcgccgaaag tatgcccggc gtcccgtcta aaggtgttct gcagatccat ggaaagcttc 120
ctgggaaacg tatgctagca gagcttctcc ccgtgaatca tatttttaag atcccactct 180
tagctggtaa atgaatttga atcgacatag tagccccata agcatcagcc ctgtagagtg 240
aggagccatc tctagcgggc ccttcattcc tctccatgct gcaatcactg tcctgggctt 300
atggtgccta tggactaggg gtcctttgtg aaagagcaag atggagcaat ggagagaaga 360
cctcttcctg aatcactgga ctccagaaat gtgcatgcag atcagctgtt gccttca 417

<210> 121
<211> 533
<212> DNA
<213> human
<400> 121

ttgatgccag catttgcatc ccgggctcca tcacattcat gcccaatgga tgctgcaaga 60
cctgcacccc tcgcaatgag accagggtgc cctgctccac cgtccccgtc accacggagg 120
tttcgtacgc cggctgcacc aagaccgtcc tcatgaatca ttgctccggg tcctgcggga 180
catttgtcat gtactcggcc aaggcccagg ccctggacca cagctgctcc tgctgcaaag 240
aggagaaaac cagccagcgt gaggtggtcc tgagctgccc caatggcggc tcgctgacac 300
acacctacac ccacatcgag agctgccagt gccaggacac cgtctgcggg ctccccaccg 360
gcacctcccg ccgggcccgg cgctccccta ggcatctggg gagcgggtga gcggggtggg 420
cacagccccc ttcactgccc tcgacagctt tacctccccc ggaccctctg agcctcctaa 480
gctcggcttc ctctcttcag atatttattg tctgagtctt tgttcagtcc ttg 533

<210> 122
<211> 417
<212> DNA
<213> human
<400> 122
cttgcttata tcatgtgctg ccgacgggaa ggcgtggaaa agagaaacat gcaaacacca 60
gacatccaac tggtccatca cagtgctatt cagaaatcta ccaaggagct tcgagacatg 120
tccaagaata gagagatagc atggcccctg tcaacgcttc ctgtgttcca ccctgtgact 180
ggggaaatca tacctccttt acacacagac aactatgata gcacaaacat gccattgatg 240
caaacgcagc agaacttgcc acatcagact cagattcccc aacagcagac tacaggtaaa 300
tggtatccct gaagaaagaa aactgactga agcaatgaat ttataatcag acaatatagc 360
agttacatca catttctttt ctcttccaat aatgcatgag cttttctggc atatgtt 417

<210> 123
<211> 542
<212> DNA
<213> human
<400> 123
cctcccggga ggacagcctt gaggcgggcc tgcccctcca ggtccgaggc taccccgagg 60
agaagaaaga ggaggagggc agcgcaaacc gcagaccaga ggaccaggag ctggagagcc 120
tgtcggccat tgaagcagag ctggagaaag tggcccacca gctgcaggca ctacggcggg 180
gctgagacac cggctggcag ggctggcccc agggcaccct gtggccctgg ctctgctgtc 240
cccttggcag gtcctggcca gatggcccgg acgctgcttc cggtagggag gcagcctcca 300
gcctgcccaa gcccaggcca ccctatcgcc ccctacgcgc cttgtctcct actcctgact 360
cctacctgcc ctggaacatc ctttgcaggg cagccccaca actttaaaca ttgacgattc 420
cttctctgaa cacaggcagc tttctagaag tttcccttcc tccatcctat ccactgggca 480
caactgcaat aacttctgac cttttggtga aagctgagaa ctcctgactg taacatattc 540
tg 542

<210> 124
<211> 537
<212> DNA
<213> human
<400> 124
gcaggccata ctggttccat tgttctgtat aatactgaat aaataaattt acttttacat 60
gatcgtataa gtttctagat aagataaaca aattctgttt aaattttttt aataaaaatc 120
ttaaaacact ttttttctaa cctagactga gaaattcatg tttacttttc taggtgtatg 180
atactttgta aagttgatac tttcctaaga atttaacatg tcatattttt gaaatagatt 240
taagtgtgct tcttattgct aaaaatacta aatgtcatgg gtcatagtat ctgatatcaa 300
tatcgttgat aacatatcca caggtaacac catgatgtag gcataaatgg aaaacaaaaa 360
ccctactatt tcaaatatat tgtacttttt tatttctgta agccaactgt gtgccatttt 420
cactggactt ttaaatctag actttagtga tgtctacatt gtaaatgatc ttttgtggat 480
atttgtcact tggtttcaga aagttcacaa atgtagcaac agctcacatg actgagt 537

<210> 125
<211> 312
<212> DNA
<213> human
<400> 125
tcccttctcg cttgggaact ctagtctcgc ctcgggttgc aatggacccc aactgctcct 60
gtgccgctgg tgtctcctgc acctgcgcca gctcctgcaa gtgcaaagag tgcaaatgca 120
cctcctgcaa gaagagctgc tgctcctgct gccctgtggg ctgtgccaag tgtgcccaag 180
gctgcatctg caaaggggca tcggagaagt gcagctgctg cgcctgatgt cgggacagcc 240

ctgctcccaa gtacaaatag agtgacccgt aaaatctagg attttttgtt ttttgctaca 300

atcttgaccc ct 312

<210> 126
<211> 393
<212> DNA
<213> human
<400> 126
aaaggctggc atcttatggc tcatcaaagg cggctgtgac catgttctca tcagttatga 60
gactggagct ttccaagtgg ggaattaaag ttgcttccat ccaacctgga ggcttcctaa 120
caaatatcgc aggcaccagt gacaagtggg aaaagctgga gaaggacatt ctggaccacc 180
tccccgctga ggtacaggaa gactacggcc aggactacat cttagcacag cggaatttcc 240
tcctattgat caactcgtta gccagcaagg acttctctcc ggtgctgcgg gacatccagc 300
atgctatctt ggcgaagagc ccttttgcct attacacgcc agggaaaggc gcttacttgt 360
ggatctgcct tgctcactat ttgcctattg gca 393

<210> 127
<211> 460
<212> DNA
<213> human
<400> 127
ggtgtcgaac tatatgcctt gtatgatcag ttttatgtgg ctaatgagtt tctcaaatat 60
cgtttacacg ttggtaacta taatggcaca gctggagatg cattacgttt caacaaacat 120
tacaaccacg atctgaagtt tttcaccact ccagataaag acaatgatcg atatccttct 180
gggaactgtg ggctgtacta cagttcaggc tggtggtttg atgcatgtct ttctgcaaac 240
ttaaatggca aatattatca ccaaaaatac agaggtgtcc gtaatgggat tttctggggt 300
acctggcctg gtgtaagtga ggcacaccct ggtggctaca agtcctcctt caaagaggct 360
aagatgatga tcagacccaa gcactttaag ccataaatca ctctgttcat tcctccaggt 420
attcgttatc taatagggca attaattcct tcagcacttt 460

<210> 128
<211> 525
<212> DNA
<213> human
<400> 128
ccaggagggg ctgtccaatc acattcaggc atgcgaatga gctgggccct gggtgaggtg 60
ggggtctggc ctagtgggga ggggcctggc ctgggtgggg cagggcctgg cctggtccag 120
gcttgggctc cattcccatc actgctgtcc ctcctgaggt ctggattggg gatggggaca 180
aagaaatagc aagagatgag aaacaacaga aacttttttc tctaaaggac tggttaaatc 240
aattctgata cagccttaca atacaatagt atgcagctaa aaaataattg tatgtcttta 300
tatactaata tgtaataatc ttcaggtgaa aaaggcaagc cacagaaatg tgtatagcgc 360
acttcccatt tgtgtttcag aaaggagtag aatataaaca cataattgct tatgtatgcc 420
tattcagaat aaatgggtaa cactgattac ttttgggagg ggaaccagta ggttgaggac 480
aggagaggga agggtcttaa cacttacacc cttttgtaca ttttg 525

<210> 129
<211> 515
<212> DNA
<213> human
<400> 129
aggagcccag gaacgacgtg gtcttccagc ccatttccgg ggaagacgtg cgcgatgtga 60
cagccctgaa cgtgagcacg ctgaaggctt acttcagatg cgatggctac aagggctacg 120
acctggtcta cagcccccag agcggcttca cctgcgtgtc cccgtgcagt aggggctact 180
gtgaccatgg aggccagtgc cagcacctgc ccagtgggcc ccgctgcagc tgtgtgtcct 240
tctccatcta cacggcctgg ggcgagcact gtgagcacct gagcatgaaa ctcgacgcgt 300
tcttcggcat cttctttggg gccctgggcg gcctcttgct gctgggggtc gggacgttcg 360
tggtcctgcg cttctggggt tgctccgggg ccaggttctc ctatttcctg aactcagctg 420
aggccttgcc ttgaaggggc agctgtggcc taggctacct caagactcac ctcatcctta 480
ccgcacattt aaggcgccat tgcttttggg agact 515

<210> 130
<211> 339

<212>  DNA
<213>  human
<400>  130
agcagcttat tgtttctctg aaagtgtgtg tagttttact ttcctaagga attaccaaga      60
atatccttta aaatttaaaa ggatggcaag ttgcatcaga aagctttatt ttgagatgta     120
aaaagattcc caaacgtggt tacattagcc attcatgtat gtcagaagtg cagaattggg     180
gcacttaatg gtcaccttgt aacagttttg tgtaactccc agtgatgctg tacacatatt     240
tgaagggtct ttctcaaaga aatattaagc atgttttgtt gctcagtgtt tttgtgaatt     300
gcttggttgt aattaaattc tgagcctgat attgatatg                            339

<210>  131
<211>  377
<212>  DNA
<213>  human
<400>  131
gagaacgggt acattcaccc ggtcaacctg acgtggccct ttatggtggc cgggaaaccc      60
gagagtgacc tgaaagaagt ggtgaccgcg agccgcttat gtggaaccac cgcgtcctga     120
ccttggaggt gcgagtctgg gaaaggcgcg ctcccggggg gagcgggccc cgggaaggcg     180
acccctgccc tcagtgctct ctgtctctgc ttccccctcg caatgctcct ctctctgtcc     240
caccccgcga gaacacttta caacgacgag gagattcgtt tccaaaccag aggagatcaa     300
ttgtacttac aaagattccc atctatttaa ctttattaac ttctaccgtg aatgactctg     360
caagccttgc tggtcca                                                    377

<210>  132
<211>  252
<212>  DNA
<213>  human
<400>  132
ttctctcgac cacttaaaac ttcagacttc ctgtcctgct ggtatcatgg agaaagtcca      60
atacctcact cgctcagcta taagaagagc ctcaaccatt gaaatgcctc aacaagcacg     120
tcaaaagcta cagaatctat ttatcaattt ctgtctcatc ttaatatgtc tcttgctgat     180
ctgtatcatc gtgatgcttc tctgaagttc tgctacaacc tctagatctg cagcttgcca     240
catcagctta aa                                                         252

<210>  133
<211>  191
<212>  DNA
<213>  human
<400>  133
tggcccatct attacatcta cagctgaccc ttgaacatgg gggttagggg agctgacaat      60
tcgtgggtcc gcaaaatctt aactacctaa tagcctacta ttgaccataa accttactga     120
taacataaac agtaaattaa cacatatttt gcgtgttata tgtattatac actatattcc     180
tacaataaag t                                                          191

<210>  134
<211>  371
<212>  DNA
<213>  human
<400>  134
aaatctgttc taagacatat gatcaacaga tgagaactgg tggttaatat gtgacagtga      60
gattagtcat atcactaata tactaacaac agaatctaat cttcatttaa ggcactgtag     120
tgaattatct gagctagagt tacctagctt accatactat atctttggaa tcatgaaacc     180
ttaagacttc agaatgattt tgcaggttgt cttccattcc agcctaacat ccaatgcagg     240
caaggaaaat aaaagatttc cagtgacaga aaaatatatt atctcaagta ttttttaaaa     300
atatatgaat tctctctcca aatattaact aattattaga ttatattttg aaatgaactt     360
gttggcccat c                                                          371

<210>  135
<211>  441
<212>  DNA
<213>  human
<400>  135
ggacaaagag cgctatgtct ccctggtgat gcctgtggcc ctgttcaacc tgattgacac      60

ttttcccttg agaaaagaag agatggtcct acaagccgaa atgagccaga cctgtaacac 120
ctgacatgat ggttcctctc ttggcaattc ctcttcattg tctacatagt gacatgcaca 180
cgggaaagcc ttaaaaatat ccttgatgta cagattttat ttgtaatttt aaaagtctat 240
tttattatga gctttctttg cacttaaaaa ttagcatgct gctttttgta cttggaagtg 300
tttcaaaaaa ttatatgacc atatttactc tttctaacct ttctttactc catcatggct 360
ggttgatttg tagagaaatt agaacccata accatacaca ggctatcaac atgttattca 420
atgtgacacc taactctttt c 441

<210> 136
<211> 402
<212> DNA
<213> human

<220>
<221> misc_feature
<222> (353)..(353)
<223> n is a, c, g, or t

<400> 136
tactcatgcc tttttgttta ggataaatag gtaagcacaa agagctcttc aaaatcagaa 60
aaaacaatag gagtccttcc ttgtcttttc tgtgatctct gtccttgttt ctgagacttt 120
ctctaccatt aagctctatt ttagctttca gttattctag tttgtttccc atggaatctg 180
tcctaaactg gtgtttttgt cagtgacagt cttgccagtc agcaatttct aacagcattt 240
taaatgagtt tgatgtacag taaatattga tgacaatgac agcttttaac tcttcaagtc 300
acctaaagct attatgcagg aggatttaga agtcacattc ataaaaccca agngctatgg 360
gtgtattatt catgatagct ggcccacagg tcatgaattg ag 402

<210> 137
<211> 174
<212> DNA
<213> human
<400> 137
agaagctcac caagtcaact aaacagcccc gaggacttac atcaccttct cagctcttga 60
cctcagaaag tatccaaacc aaggaggaga aacctgtggg tggcttgtcc tccagtgaca 120
caatggatta ccgacagtga ctaagggcag catgtttaac ccaggtgaag atct 174

<210> 138
<211> 371
<212> DNA
<213> human
<400> 138
tttctggcct tcacccagac gaagaccttc cacgaggcca gcgaggactg catctcgcgc 60
gggggcaccc tgagcacccc tcagactggc tcggagaacg acgccctgta tgagtacctg 120
cgccagagcg tgggcaacga ggccgagatc tggctgggcc tcaacgacat ggcggccgag 180
ggcacctggg tggacatgac cggcgcccgc atcgcctaca agaactggga gactgagatc 240
accgcgcaac ccgatggcgg caagaccgag aactgcgcgg tcctgtcagg cgcggccaac 300
ggcaagtggt tcgacaagcg ctgccgcgat cagctgccct acatctgcca gttcgggatc 360
gtgtagccgg c 371

<210> 139
<211> 406
<212> DNA
<213> human
<400> 139
gcagactggg agttgctagc aaacaaatgg cttacttaca aaagcagctt ttagttcaga 60
cttagttttt ataaaatgag aattctgact tacttaacca ggtttgggat ggagatggtc 120
tgcatcagct ttttgtatta acaaagttac tggctctttg tgtgtctcca ggtaactttg 180
cttgattaaa cagcaaagcc atattctaaa ttcactgttg aatgcctgtc ccagtccaaa 240
ttgtctgtct gctcttattt ttgtaccata ttgctcttaa aaatcttggt ttggtacagt 300
tcataattca ccaaaaagtt catataattt aaagaaacac taaattagtt taaaatgaag 360
caatttatat ctttatgcaa aaacatatgt ctgtctttgc aaagga 406

<210> 140

<211>  388
<212>  DNA
<213>  human
<400>  140
gcacccagcc ttttaccatg tgggtttctt tagtgtctta aaagcgtcca taagccacca      60
ttctgtggaa ccaaggcccc ctccacgcaa acaccctccc tcctggggac ctctggagcc     120
tcagccagaa gtaccattag gtttaatttt aatttgtttt gctggagaaa catcaggttt     180
gtaggagact gagttgttag caggtgtgct tagctcttga tagtgaacgt gtaccttggg     240
aactggctca cccacctgct aatagcacca tcgtcactat taagcagaca tttcagttgg     300
tagaatccat gtagaagtca tggacttttc tgggaaatga cttttctggg aaatgacagt     360
ttctttgaca tattttcttt gcccactt                                        388

<210>  141
<211>  317
<212>  DNA
<213>  human
<400>  141
cgccatcacc gagcgcttga tgtgcgcgga gagcaatcgc cgggacagct gcaagggtga      60
ctccgggggc ccgctggtgt gcgggggcgt gctcgagggc gtggtcacct cgggctcgcg     120
cgtttgcggc aaccgcaaga agcccgggat ctacacccgc gtggcgagct atgcggcctg     180
gatcgacagc gtcctggcct agggtgccgg ggcctgaagg tcagggtcac ccaagcaaca     240
aagtcccgag caatgaagtc atccactcct gcatctggtt ggtctttatt gagcacctac     300
tatatgcaga aggggag                                                    317

<210>  142
<211>  317
<212>  DNA
<213>  human
<400>  142
gggccacgcc aggaatattc agaaaataat gagaactaca ttgaagtgcc attgattttt      60
gatcctgtca caagagagga tttgcacatg gattttaaat gtgttgtcca taataccctg     120
agttttcaga cactacgcac cacagtcaag gaagcctcct ccacgttctc ctggggcatt     180
gtgctggccc cactttcact ggccttcttg gttttggggg gaatatggat gcacagacgg     240
tgcaaacaca gaactggaaa agcagatggt ctgactgtgc tatggcctca tcatcaagac     300
tttcaatcct atcccaa                                                    317

<210>  143
<211>  456
<212>  DNA
<213>  human
<400>  143
gctgctctgg ttctcatgac ggcagatgca gcgaagaggc tcaatgttac accactggca      60
agaatagtag catttgctga cgctgctgta gaacctattg attttccaat tgctcctgta     120
tatgctgcat ctatggttct taaagatgtg ggattgaaaa aagaagatat tgcaatgtgg     180
gaagtaaatg aagcctttag tctggttgta ctagcaaaca ttaaaatgtt ggagattgat     240
ccccaaaaag tgaatatcaa tggaggagct gtttctctgg gacatccaat tgggatgtct     300
ggagccagga ttgttggtca tttgactcat gccttgaagc aaggagaata cggtcttgcc     360
agtatttgca atggaggagg aggtgcttct gccatgctaa ttcagaagct gtagacaacc     420
tctgctattt aaggagacaa ccctatgtga ccagaa                               456

<210>  144
<211>  507
<212>  DNA
<213>  human
<400>  144
ggaaagcagg attccatcgc tggaacaatt acatgatgga ctggaaaaat caatttaacg      60
attacactag caagaaagaa agttgtgtgg gtctctaatt aatagattta ccctttatag     120
aacatatttt cctttagatc aaggcaaaaa tatcaggagc ttttttacac acctactaaa     180
aaagttatta tgtagctgaa acaaaaatgc cagaaggata atattgattc ctcacatctt     240
taacttagta ttttacctag catttcaaaa cccaaatggc tagaacatgt ttaattaaat     300
ttcacaatat aaagttctac agttaattat gtgcatatta aaacaatggc ctggttcaat     360
ttctttcttt ccttaataaa tttaagtttt ttccccccaa aattatcagt gctctgcttt     420
tagtcacgtg tattttcatt accactcgta aaaaggtatc ttttttaaat gaattaaata     480

ttgaaacact gtacaccata gtttaca 507

<210> 145
<211> 489
<212> DNA
<213> human
<400> 145
gagggtgatg ccatctaacc ctgcccctgt ccaccccggg tgggtgaaac tcactgagca 60
gccaagactg ttgcccgagg actcactgta tggtgccctc tccaaagggt cgggagggta 120
gctctccagg ccagagcttg tgtccttcaa cagagaggcc agcggcaact ggtccgttac 180
tggccaaggg ctctgaagaa tcaacggtgc tggtacagga tacaggaata aattgtatct 240
tcacctggtt cctaccctcg tccctacctg tcctgatcct ggtcctgaag acccctcgga 300
acaccctctc ctggtggcag gccacttccc tcccagtgcc agtctccatc caccccagag 360
aggaacaggc gggtgggcca tgtggttttc tccttcctgg ccttggctgg cctctggggc 420
aggggtggtg gagagatgga agggcatcag gtgtagggac cctgccaagt ggcacctgat 480
ttactctag 489

<210> 146
<211> 480
<212> DNA
<213> human
<400> 146
ttaatgtgcc aaggagccgt tttctgcctg tcaaaaccac atcagatctc ttgctggtga 60
tgtcaaacct ctatagtctt aatgcaggat ctctgacaat gagtgaaaag cgggaatttc 120
ctacagtgcc cttggttaaa ttaggcagtt cttttacgaa ggttcaagat tatctaagaa 180
gatttgaaag tataccagat atgcttgaat tggatcacct cacagtttca ggagatgtga 240
catttggaaa aaatgtttca ttaaagggaa cggttatcat cattgcaaat catggtgaca 300
gaattgatat cccacctgga gcagtattag agaacaagat agtgtctgga aaccttcgca 360
tcttggacca ctgaaatgaa aaatactgtg gacacttaaa taatgggcta gtttcttaca 420
atgaaatgtt ctctaggatt taggcactaa aaggtacttt actatgttac tgtaccctgc 480

<210> 147
<211> 529
<212> DNA
<213> human
<400> 147
caagaatgat gggcactacc gtggagatcc caactggttt atgaagaaag cgcaggagca 60
taagagggaa ttcacagaga gccagctgca ggagggaaag catgtcattg gccttcagat 120
gggcagcaac agaggggcct cccaggccgg catgacaggc tacggacgac ctcggcagat 180
catcagttag agcggagagg gctagccctg agcccggcgc tcccccagct ccttggctgc 240
agccatcccg cttagcctgc ctcacccaca cccgtgtggt accttcagcc ctggccaagc 300
tttgaggctc tgtcactgag caatggtaac tgcacctggg cagctcctcc ctgtgccccc 360
agcctcagcc caacttctta cccgaaagca tcactgcctt ggcccctccc tcccggcggc 420
ccccatcacc tctactgtct cctccctggg ctaagcaggg gagaagcggg ctgggggtag 480
cctggatgtg ggcgaagtcc actgtcctcc ttggcggcaa aagcccatt 529

<210> 148
<211> 525
<212> DNA
<213> human
<400> 148
tcaaagactt cgtgattatc cccctgcaca ccaccccaga gacatccgtt aaggagatcg 60
atgagttggt tgaggtctac acggacgtga aacaccgctg gaaggcggag aatttcattt 120
tcatgggtga cttcaatgcc ggctgcagct acgtccccaa gaaggcctgg aagaacatcc 180
gcttgaggac tgaccccagg tttgtttggc tgatcgggga ccaagaggac accacggtga 240
agaagagcac caactgtgca tatgacagga ttgtgcttag aggacaagaa atcgtcagtt 300
ctgttgttcc caagtcaaac agtgtttttg acttccagaa agcttacaag ctgactgaag 360
aggaggccct ggatgtcagc gaccactttc cagttgaatt taaactacag tcttcaaggg 420
ccttcaccaa cagcaaaaaa tctgtcactc taaggaagaa aacaaagagc aaacgctcct 480
agacccaagg gtctcatctt attaaccatt tcttgcctct aaata 525

<210> 149
<211> 559

<212>  DNA
<213>  human
<400>  149
aacgaggagc acatgaccct gctgaagatg attttgataa aatgctgtga tatctctaac      60
gaggtccgtc caatggaagt cgcagagcct tgggtggact gtttattaga ggaatatttt     120
atgcagagcg accgtgagaa gtcagaaggc cttcctgtgg caccgttcat ggaccgagac     180
aaagtgacca aggccacagc ccagattggg ttcatcaagt ttgtcctgat cccaatgttt     240
gaaacagtga ccaagctctt ccccatggtt gaggagatca tgctgcagcc actttgggaa     300
tcccgagatc gctacgagga gctgaagcgg atagatgacg ccatgaaaga gttacagaag     360
aagactgaca gcttgacgtc tggggccacc gagaagtcca gagagagaag cagagatgtg     420
aaaaacagtg aaggagactg tgcctgagga aagcgggggg cgtggctgca gttctggacg     480
ggctggccga gctgcgcggg atccttgtgc agggaagagc tgccctgggc acctggcacc     540
acaagaccat gttttctaa                                                  559

<210>  150
<211>  542
<212>  DNA
<213>  human
<400>  150
tgacgcaaaa taccaccttg gcgcctacac gggagacgac gtccgcatcg tccgtgacga      60
catgctgtgt gccgggaaca cccggaggga ctcatgccag ggcgactccg gagggcccct     120
ggtgtgcaag gtgaatggca cctggctgca ggcgggcgtg gtcagctggg gcgagggctg     180
tgcccagccc aaccggcctg gcatctacac ccgtgtcacc tactacttgg actggatcca     240
ccactatgtc cccaaaaagc cgtgagtcag gcctgggttg gccacctggg tcactggagg     300
accaacccct gctgtccaaa acaccactgc ttcctaccca ggtggcgact gccccccaca     360
ccttccctgc cccgtcctga gtgccccttc ctgtcctaag ccccctgctc tcttctgagc     420
cccttcccct gtcctgagga cccttcccta tcctgagccc ccttccctgt cctaagcctg     480
acgcctgcac cgggccctcc agccctcccc tgcccagata gctggtggtg ggcgctaatc     540
ct                                                                    542

<210>  151
<211>  220
<212>  DNA
<213>  human
<400>  151
gaagagggag ctctattgcc accatgagtt tctccggcaa gtaccaactg cagagccagg      60
aaaactttga agccttcatg aaggcaatcg gtctgccgga agagctcatc cagaagggga     120
aggatatcaa gggggtgtcg gaaatcgtgc agaatgggaa gcacttcaag ttcaccatca     180
ccgctgggtc caaagtgatc caaaacgaat tcacggtggg                           220

<210>  152
<211>  524
<212>  DNA
<213>  human
<400>  152
ccttctctgg tctccttgag atgatcgtag acacagggat gattcccacc caaacccacg      60
tattcattca gtgagttaaa cacgaattga tttaaagtga acacacacaa gggagcttgc     120
ttgcagatgg tctgagttct tgtgtcctgg taattcctct ccaggccaga ataattggca     180
tgtctcctca acccacatgg ggttcctggt tgttcctgca tcccgatacc tcagccctgg     240
ccctgcccag cccatttggg ctctggtttt ctggtggggc tgtcctgctg ccctcccaca     300
gcctccttct gtttgtcgag catttcttct actcttgaga gctcaggcag cgttagggct     360
gcttaggtct catggaccag tggctggtct cacccaactg cagtttacta ttgctatctt     420
ttctggatga tcagaaaaat aattccataa atctattgtc tacttgcgat tttttaaaaa     480
atgtatattt ttatatatat tgttaaatcc tttgcttcat tcca                      524

<210>  153
<211>  486
<212>  DNA
<213>  human
<400>  153
tctcccaacc tctactgtaa actttctggt ccgagaacga gccgaacaca gcgcgacgca      60
gggactagga cggcccggtg accgcgcgga ttcaggattg cggggacgca gaaaggttaa     120
ggcactttta aaaactatag caaggctcct gtttatttat tctactttct ttccctaata     180

atcaaaacac cgcgtaggct cctccgttta tcagtattaa tggtgtaact ttgttggcaa 240
tatttgccgt gtagaatttt ttttagatat ccattgtaaa tttgaaacaa agaccgatct 300
gtgtaaaaac aaatttccat atgttttata taaatatata tataatatga aggactaccc 360
tccttttttt ttttttgtat tttggctgct agagtgcagc atttgtgaca cgtatttgaa 420
atttgaaatt tccttctgca ctgtataaaa ggaccatttg aggatgtttt gccttttgtg 480
tatttt 486

<210> 154
<211> 560
<212> DNA
<213> human
<400> 154
gagccccatt cacaaatttt gacccctcta ctctccttcc ttcatccctg gatttctgga 60
cctaccctgg ctctctgact catcctcctc tttatgagag tgtaacttgg atcatctgta 120
aggagagcat cagtgtcagc tcagagcagc tggcacaatt ccgcagcctt ctatcaaatg 180
ttgaaggtga taacgctgtc cccatgcagc acaacaaccg cccaacccaa cctctgaagg 240
gcagaacagt gagagcttca ttttgatgat tctgagaaga aacttgtcct tcctcaagaa 300
cacagccctg cttctgacat aatccagtta aaataataat ttttaagaaa taaatttatt 360
tcaatattag caagacagca tgccttcaaa tcaatctgta aaactaagaa acttaaattt 420
tagttcttac tgcttaattc aaataataat tagtaagcta gcaaatagta atctgtaagc 480
ataagcttat cttaaattca agtttagttt gaggaattct ttaaaattac aactaagtga 540
tttgtatgtc tatttttttc 560

<210> 155
<211> 521
<212> DNA
<213> human
<400> 155
ggcagctttt ttatcagcct tgctttggat aggacctcca aggactaagc ctccagcccc 60
atgtgtgacc cttgtcatct ctctgcccca cataattatg ttactttgct atgtgctcct 120
aatgtatcta gtgtgtcctg tgacaacact catcacactt cattgtaaat cacttgtttt 180
attgactgtc tttcctatag actgtaagct ccatgagggc aggcacatgt tgttctcatt 240
gaccgtgctg gccccagtgc ctagatgcat ggctggcaca ttgttggcac tcaacaatgg 300
ttgaatgaat aaaacaataa atgaatgaat aactaagata tagaaactct catttatatt 360
gcagattgaa tatatatgat gaaattctta tgttgaatat gttagaatca aatactcatt 420
tttcattaga tacagtagtg tcatcactct tttaagatct tgttaaagat ttcaaataaa 480
ggtacttctg gcgagccagg ctgcacagca tttgctttcc t 521

<210> 156
<211> 268
<212> DNA
<213> human
<400> 156
tatttgaata tgtatcgtgc cccctccgga gtctttgatc aggatgacat gtgccatttt 60
tcagaggacg tgcagacagg ctggcattct agattacttt tcttactctg aaacatggcc 120
tgtttgggag tgcgggattc aaaggtggtc ccacggctgc ccctactgca aatggcagtt 180
ttaatcttat cttttggctt ctgcagatgg ttgcattgat ccttaaccaa taatggtcag 240
tcctcatctc tgtcctgact tcataggt 268

<210> 157
<211> 529
<212> DNA
<213> human
<400> 157
ctttctatat tgttatcagt ccaggaaaca ggtaaacaga tgtaattaga gacattggct 60
ctttgtttag gcctaatctt tctttttact tttttttttc ttttttcttt ttttttaaag 120
atcatgaatt tgtgacttag ttctgccctt tggagaacaa aagaaagcag tcttccatca 180
aatcacctta aaatgcacgg ctaaactatt cagagttaac actccagaat tgttaaatta 240
caagtactat gctttaatgc ttctttcatc ttactagtat ggcctataaa aaaaataata 300
ccacttgatg ggtgaaggct ttggcaatag aaagaagaat agaattcagg ttttatgtta 360
ttcctctgtg ttcacttcgc cttgctcttg aaagtgcagt atttttctac atcatgtcga 420
gaatgattca atgtaaatat ttttcatttt atcatgtata tcctatacac acatctcctt 480
catcatcata tatgaagttt attttgagaa gtctacattg cttacattt 529

<210> 158
<211> 494
<212> DNA
<213> human
<400> 158
taacaatcca tgatgctgtt ttgcatattt tgatgaagaa agattacagt acttcaaagt 60
ttaatcccag tcaggaaaaa gatggaaaaa ttgattttac cataaataca aatggaggat 120
tacgtaatcg ggtatatgag gtgccagttg aaacaaaatt ctaatcaaca tataattcag 180
aaggatcttc atctgactat gacataaaaa caactttata cccagaaagt tattgataag 240
ttcatacatt gtacgaagag tatttttgac agaatatgtt tcaaactttg gaacaagatg 300
gttctagcat ggcatatttt tcacatatct agtatgaaat tatataagta ttctaaattt 360
tatatcttgt agctttatca aagggtgaaa attattttgt tcatacatat ttttgtagca 420
ctgacagatt tccatcctag tcactacctt catgcatagg tttagcagta tagtggcgcc 480
actgttttga atct 494

<210> 159
<211> 469
<212> DNA
<213> human
<400> 159
tcatcaggct ccagttattc tccatctccc agctcagctt tttctgtctg taagcctgat 60
tttcaggaag gctctttcct agtgatggag atgaccacca tcagctccag gcttctatcc 120
tgctaaccca gtaacccagt gggaagagat ttacttattc caataattcc aagtggagag 180
tgtcattgac ccgtttgggg tctcatctct acttctaggg gaatgaaaca ctttgagtgg 240
ccaggcctgt gtcatgtgct aattcctaga gccagggaaa taaggtctga ggattcagga 300
tggggtgaaa ggtggttgct taaaggaaaa tgaaatacaa ttagcagaat aaggggaaac 360
gagtggtctg ctctgctcgg gcaaaacaag agatgcccat tactgtgagg gacccttgaa 420
gtctggactc ttaaatgggt ttttgctgat ttcctgggtg catgctagg 469

<210> 160
<211> 269
<212> DNA
<213> human
<400> 160
accagcaaca acttcaatcc ggtggagaac aaagatattg tggttttaac ctgtcaacct 60
gagactcaga acacaaccta cctgtggtgg gtaaacaatc agagcctcct ggtcagtccc 120
aggctgctgc tctccactga caacaggacc ctcgttctac tcagcgccac aaagaatgac 180
ataggaccct atgaatgtga aatacagaac ccagtgggtg ccagccgcag tgacccagtc 240
accctgaatg tccgctatga gtcagtaca 269

<210> 161
<211> 513
<212> DNA
<213> human
<400> 161
atattagtta ccctggtgtg ctgtattctc taaaaccttt aaatgtttgc atgcagccat 60
tcgtcaaatg tcaaatattc tctctttggc tggaatgaca aaaactcaaa taaatgtatg 120
attaggagga catcataacc tatgaatgat ggaagtccaa aatgatggta actgacagta 180
gtgttaatgc cttatgttta gtcaaactct catttaggtg acagcctggt gactccagaa 240
tggagccagt catgctaaat gccatatact cacactgaaa catgaggaag caggtagatc 300
ccagaacaga caaaactttc ctaaaaacat gagagtccag gctgtctgag tcagcacagt 360
aagaaagtcc tttctgcttt aactcttaga aaaaagtaat atgaagtatt ctgaaattaa 420
ccaatcagtt tatttaaatc aatttattta tattcttctg ttcctggatt cccattttac 480
aaaacccact gttctactgt tgtattgccc agt 513

<210> 162
<211> 355
<212> DNA
<213> human
<400> 162
cccaggaccc tgaagacgaa attgcggtgc tggcctttct ggtggaggct ggaacccagg 60
tgaacgaggg cttccagcca ctggtggagg cactgtctaa tatccccaaa cctgagatga 120

gcactacgat ggcagagagc agcctgttgg acctgctccc caaggaggag aaactgaggc 180
actacttccg ctacctgggc tcactcacca caccgacctg cgatgagaag gtcgtctgga 240
ctgtgttccg ggagcccatt cagcttcaca gagaacagat cctggcattc tctcagaagc 300
tgtactacga caaggaacag acagtgagca tgaaggacaa tgtcaggccc ctgca 355

<210> 163
<211> 283
<212> DNA
<213> human
<400> 163
accacaccga cctgcgatga gaaggtcgtc tggactgtgt tccgggagcc cattcagctt 60
cacagagaac agatcctggc attctctcag aagctgtact acgacaagga acagacagtg 120
agcatgaagg acaatgtcag gcccctgcag cagctggggc agcgcacggt gataaagtcc 180
ggggccccgg gtcggccgct gccctgggcc ctgcctgccc tgctgggccc catgctggcc 240
tgcctgctgg ccggcttcct gcgatgatgg ctcacttctg cac 283

<210> 164
<211> 337
<212> DNA
<213> human
<400> 164
actgactgga cgcacgtgga aaggagctat ttttggaggc tttaagagta aagaatctgt 60
cccgaaactt gtggctgact ttatggctaa gaagttttca ctggatgcat taataacaaa 120
tattttacct tttgaaaaaa taaatgaagg atttgacctg cttcgctctg gaaagagtat 180
ccgtaccgtc ctgacgtttt gaaacaatac agatgccttc ccttgtagca gttttcagcc 240
tcctctaccc tacatgatct ggagcaacag ctaggaaata tcattaattc tgctcttcag 300
agatgttaaa aataaattac acgtgggagc tttccaa 337

<210> 165
<211> 498
<212> DNA
<213> human
<400> 165
gttggtcact gttatttgcc tactgctgga agaaggacaa ccgctggcaa ggtagcgttc 60
cccaatctga atacctgcag gctcccacat gagggagagg gcagactcag gtgggaagat 120
gtgccatgcg taaggcatca acgtgtatct gtgggatctt cgttgccttc agtaatcagg 180
gtgtgaaaaa agcagacaag ttgtgtgtgt gtgtgtgtgt ctaagaaaac ttgtgtgctt 240
ttcaaaaagg cagtgctaag cacaagattt caagaaagcc tcttcttgtt gcctagctga 300
gtgggagagt cattttcccc agacactaca tttggataca ggtgccaaag aacattatta 360
aggaattatt tagaaacaat gtgtctagtt taagaaagtg gttttcagta ttgtgacaat 420
acaacgtttt tacaaggttg ttttctacca ccatatttta aagatatttt tatgaccgtg 480
tatactcaca ctttgctt 498

<210> 166
<211> 489
<212> DNA
<213> human
<400> 166
aaagcccttc atctaatatt tgttgctatt gccaattttt caatgaaatg acctaaaaac 60
aacaaaaaaa aataacctat acggtagttg ctttaggggg tggggggatg ctatctgtta 120
gtgcttaaaa gggggtaaat gcttgccgct ttagaggtgg atggtgctca taaaaggccc 180
cagtcggggg tatttaaaaa ggactgaaca gaaatcctta gctagtagaa tggcagcacg 240
ctgtaaaatt attactgtat tgtgtactgg ctataagatg tagacacctt tcagtaagcc 300
aatcatttgt aaccattcta gcagtgtcat attaggttaa taaggctgct gtgttttaaa 360
gggcattttt atttgggttt tggtgaaatt ctttaatttg ttgattatat tcacataaaa 420
tcagcattca ttgacacata gctctaatga catatgtatg aaaaaccata cactggatga 480
cctagtcga 489

<210> 167
<211> 556
<212> DNA
<213> human
<400> 167

ctgatggttc tttctctgat gagatgaaca ccattcttga taatcttgcc gccagggact 60
ttataaactg gttgattcag accaaaatca ctgacaggaa ataactatat cactattcaa 120
gatcatcttc acaacatcac ctgctagcca cgtgggatgt ttgaaatgtt aagtcctgta 180
aatttaagag gtgtattctg aggccacatt gctttgcatg ccaataaata aattttcttt 240
tagtgttgtg tagccaaaaa ttacaaatgg aataaagttt tatcaaaata ttgctaaaat 300
atcagcttta aaatatgaaa gtgctagatt ctgttatttt cttcttattt tggatgaagt 360
accccaacct gtttacattt agcgataaaa ttatttttct atgatataat ttgtaaatgt 420
aaattattcc gatctgacat atctgcatta taataatagg agaatagaag aactggtagc 480
cacagtggtg aaattggaaa gagaactttc ttcctgaaac ctttgtctta aaaatactca 540
gctttcaatg tatcaa 556

<210> 168
<211> 244
<212> DNA
<213> human
<400> 168
gccggctcct gcaagtgcaa aaagtgcaaa tgcacctcct gcaagaagag ctgctgctcc 60
tgttgccccc tgggctgtgc caagtgtgcc cagggctgca tctgcaaagg ggcgtcagag 120
aagtgcagct gctgtgcctg atgtcgggac agccctgctg tcagatgaaa acagaatgac 180
acgtaaaatc cgaggttttt tttttctaca actccgactc atttgctaca ttcctttttt 240
tctg 244

<210> 169
<211> 557
<212> DNA
<213> human
<400> 169
ttccctgctg gaggatccca agattaagga gattgctgca aagcacaaaa aaaccgcagc 60
ccaggttctg atccgtttcc atatccagag gaatgtgatt gtcatcccca agtctgtgac 120
accagcacgc attgttgaga acattcaggt ctttgacttt aaattgagtg atgaggagat 180
ggcaaccata ctcagcttca acagaaactg gagggcctgt aacgtgttgc aatcctctca 240
tttggaagac tatcccttca atgcagaata ttgaggttga atctcctggt gagattatac 300
aggagattct ctttcttcgc tgaagtgtga ctacctccac tcatgtccca ttttagccaa 360
gcttatttaa gatcacagtg aacttagtcc tgttatagac gagaatcgag gtgctgtttt 420
agacatttat ttctgtatgt tcaactagga tcagaatatc acagaaaagc atggcttgaa 480
taaggaaatg acaatttttt ccacttatct gatcagaaca aatgtttatt aagcatcaga 540
aactctgcca acactga 557

<210> 170
<211> 268
<212> DNA
<213> human
<400> 170
taagtccagt agtagcaaag ccccaaatca aagccagcaa gaccacagtc acaggagata 60
aggactctgt gaacctgacc tgctccacaa atgacactgg aatctccatc cgttggttct 120
tcaaaaacca gagtctcccg tcctcggaga ggatgaagct gtcccagggc aacaccaccc 180
tcagcataaa ccctgtcaag agggaggatg ctgggacgta ttggtgtgag gtcttcaacc 240
caatcagtaa gaaccaaagc gaccccat 268

<210> 171
<211> 459
<212> DNA
<213> human
<400> 171
tgagcctggg gttctggtgt tagaatattt ttaagtaggc tttactgaga gaaactaaat 60
attggcatac gttatcagca acttcccctg ttcaatagta tgggaaaaat aagatgactg 120
ggaaaaagac acacccacac cgtagaacat atattaatct actggcgaat gggaaaggag 180
accattttct tagaaagcaa ataaacttga tttttttaaa tctaaaattt acattaatga 240
gtgcaaaata acacataaaa tgaaaattca cacatcacat ttttctggaa aacagacgga 300
ttttacttct ggagacatgg catacggtta ctgacttatg agctaccaaa actaaattct 360
ttctctgcta ttaactggct agaagacatt catctatttt tcaaatgttc tttcaaaaca 420
tttttataag taatgtttgt atctatttca tgctttact 459

<210> 172
<211> 482
<212> DNA
<213> human
<400> 172
atttctttgg cagttttcgt gctaatgttt ttgctaagga agataagctc tgaaccatta 60
aaggacgagt ttaaaaacac aggatcaggt ctcctgggca tggctaacat tgacctggaa 120
aagagcagga ctggtgatga aattattctt ccgagaggcc tcgagtacac ggtggaagaa 180
tgcacctgtg aagactgcat caagagcaaa ccgaaggtcg actctgacca ttgctttcca 240
ctcccagcta tggaggaagg cgcaaccatt cttgtcacca cgaaaacgaa tgactattgc 300
aagagcctgc cagctgcttt gagtgctacg gagatagaga aatcaatttc tgctaggtaa 360
ttaaccattt cgactcgagc agtgccactt taaaaatctt ttgtcagaat agatgatgtg 420
tcagatctct ttaggatgac tgtatttttc agttgccgat acagcttttt gtcctctaac 480
tg 482

<210> 173
<211> 519
<212> DNA
<213> human
<400> 173
gaagacacta ccaacatgat attacgtatt gatctgacca cacacaatgt tactctagaa 60
gaaccaatag aaatcaactg gtcatgaaga tcaccatcaa ttttagttgt caatgggaaa 120
aaacaccagg atttaagttt cacagcactt acaattttcc ctcttcactt ggttcttgta 180
ctctacaaaa tatagctttc ataacatcga aaagttattt tgtagcgtac atcaatgata 240
atgctaattt tattatagta atgtgacttg gattcaattt taaggcatat ttaacaaaat 300
ttgaatagcc ctatttatcc ttgttaagta tcagctacaa ttgtaaacta gttactaaac 360
atgtatgtaa atagctaaga tataatttaa acgtgatttt taaattaaat aaaattttta 420
tgtaattata tatactatat ttttctcaat gtttagcaga tttaagatat gtaacaacaa 480
ttatttgaag atttaattac ttcttagtat gtgcattta 519

<210> 174
<211> 227
<212> DNA
<213> human
<400> 174
attgcaattt cttaggtaac cttatattta caataaatga agattaccct caaatgctag 60
aagctgtcta ggtccgtccg gtgtgtcaga tttcctcaga ttagatgtgc caataaccaa 120
gtttattcag taaacaactt gtacttgttt catctggttt attactctca cccataaaca 180
gtaatgactc tctgaccctc tggaaatatg taatgcttcc aatcttg 227

<210> 175
<211> 478
<212> DNA
<213> human
<400> 175
ttttggacct gcggtggtgg ccaaccactg gaacttccac tggatctact ggctgggccc 60
actcctggct ggcctgcttg ttggactgct cattaggtgc ttcattggag atgggaagac 120
ccgcctcatc ctgaaggctc ggtgagcaga gctcgtggga ttcctgctgc tccaggtgtc 180
ctcagctcac ctgtcccaga ctgaggacag gggagttcct gcatttcctg ccagggcaga 240
ggcccagagg agcgaccccc tgcttccact gcttgggcct gctttctcag atagactgac 300
tgctgaggag gctctaggtt cttggaattc ctttgtgctc atcagagacc ccagcctggg 360
gaacacgctg cccgcactgc ccagagagca gtgcaaacac cacaacacga gcgtgtttct 420
tgagaggaat gtccccgagt tggacaagga ggctgtttct gcacatcagc tcatttcc 478

<210> 176
<211> 436
<212> DNA
<213> human
<400> 176
gaggggtcac cgtgcaggat ggaaatttct ccttttctct ggagtcagtg aagaagctca 60
aagacctcca ggagccccag gagcccaggg ttgggaaact caggaacttt gcacccatcc 120
ctggtgaacc tgtggttccc atcctctgta gcaacccgaa ctttccagaa gaactcaagc 180
ctctctgcaa ggagcccaat gcccaggaga tacttcagag gctggaggaa atcgctgagg 240

acccgggcac atgtgaaatc tgtgcctacg ctgcctgtac cggatgctag gggggcttgc 300
ccactgcctg cctcccctcc gcagcaggga agctcttttc tcctgcagaa agggccaccc 360
atgatactcc actcccagca gctcaaccta ccctggtcca gtcgggagga gcagcccggg 420
gaggaactgg gtgact 436

<210> 177
<211> 234
<212> DNA
<213> human
<400> 177
tcgtctgctt cacaagctat cgctatggtg ttcgtgcgca ggccgtggcc cgccttgacc 60
acagtgcttc tggccctgct cgtctgccta ggggcgctgg tcgacgccta ccccatcaaa 120
cccgaggctc ccggcgaaga cgcctcgccg gaggagctga accgctacta cgcctccctg 180
cgccactacc tcaacctggt cacccggcag cggtatggga aaagagacgg cccg 234

<210> 178
<211> 287
<212> DNA
<213> human
<400> 178
tatttgaata tgtatcgtgc cccctccggt gtctttgatc aggatgacat gtgccatttt 60
tcagaggacg tgcagacagg ctggcattct agattacttt tcttactctg aaacatggcc 120
tgtttgggag tgcgggattc aaaggtggtc ccaccgctgc ccctactgca aatggcagtt 180
ttaatcttat cttttggctt ctgcagatgg ttgcaattga tccttaacca ataatggtca 240
gtcctcatct ctgtcctgct tcataggtgc caccttgtgt gtttaaa 287

<210> 179
<211> 536
<212> DNA
<213> human
<400> 179
tgacgcaaaa taccaccttg gcgcctacac gggagacgac gtccgcatcg tccgtgacga 60
catgctgtgt gccgggaaca cccggaggga ctcatgccag ggcgactccg gagggcccct 120
ggtgtgcaag gtgaatggca cctggctgca ggcgggcgtg gtcagctggg gcgagggctg 180
tgcccagccc aaccggcctg gcatctacac ccgtgtcacc tactacttgg actggatcca 240
ccactatgtc cccaaaaagc cgtgagtcag gcctgggttg gccacctggg tcactggagg 300
accaacccct gctgtccaaa acaccactgc ttcctaccca ggtggcgact gccccccaca 360
ccttccctgc cccgtcctga gtgccccttc ctgtcctaag ccccctgctc tcttctgagc 420
cccttcccct gtcctgagga cccttcccca tcctgagccc ccttccctgt cctaagcctg 480
acgcctgcac cgggccctcc ggccctcccc tgcccaggca gctggtggtg ggcgct 536

<210> 180
<211> 472
<212> DNA
<213> human
<400> 180
atgatcacaa aatgttgcct gttttgtttc cgaaagcttg ccaaaacagg aaagaagaag 60
aaaagggatt agttatatca aaagcctgaa gtggaatgac caaaagatgg gactcctcct 120
ttattccagc atggagggtt ttaaatggag gatttccttt ttcctgcgac aaaacgtctt 180
ttcacaactt accctgttaa gtcaaaattt attttccagg aatttaatat gtactttagt 240
tggaattatt ctatgtcaat gatttttaag ctatgaaaaa taataatata aaaccttatg 300
ggcttatatt gaaatttatt attctaatcc aaaagttacc ccacacaaaa gttactgagc 360
ttccttatgt ttcacacatt gtatttgaac acaaaacatt aacaactcca ctcatagtat 420
caacattgtt ttgcaaatac tcagaatatt ttggcttcat tttgagcaga at 472

<210> 181
<211> 334
<212> DNA
<213> human
<400> 181
caagtatcgg ggctctgcta tcaaggtgca aagtccttcg tggatgcaac ctcaaccata 60
tattctacag caccctggtg ccgtgttaac tccctcaatg gagcacacca tgtcactaca 120
gcccgcatca atgatcagcc ctctggccca gcagatgagt catctgtcac taggcagcac 180

cggaacatac atgcctgcaa cgtcagctat gcaaggagcc tacttgccac agtatgcaca 240
tatgcagacg acagcggttc ctgttgagga ggcaagtggt caacagcagg tggctgtcga 300
gacgtctaat gaccattctc catatacctt tcaa 334

<210> 182
<211> 475
<212> DNA
<213> human
<400> 182
tggactggtg tcgagccaag actcggggcc tacgagcacg tcgacatcca gaacttctcc 60
tccagctgga gtgatgggat ggccttctgt gccctggtgc acaacttctt ccctgaggcc 120
ttcgactatg ggcagcttag ccctcagaac cgacgccaga acttcgaggt ggccttctca 180
tctgcggaga tgctggtgga ctatgtgccc ctggtggagg tggacgacat gatgatcatg 240
ggcaagaagc ctgaccccaa gtgtgtcttc acctatgtgc agtcgctcta caaccacctg 300
cgacgccacg aactggcctc gcgcggcaag aatgtctagc ctgcccgccc gcatggccag 360

ccagtggcaa ctgccgcccc cactctccgg gcaccgtctc ctgcctgtgc gtccgcccac 420
cgctgccctg tctgttgcga caccctcccc cccacataca cacgcagcgt tttga 475

<210> 183
<211> 561
<212> DNA
<213> human
<400> 183
ggatgtgata gcattcctgc tggcctgcgt ggcaactgtg atatttatca tcacaaaatt 60
ttgcctgttt tgtttccgaa agcttgccaa aacaggaaag aagaagaaaa gagattagtt 120
atatcaaaag cctgaagtgg aatgactgaa agatgggact cctcctttat ttcagcatgg 180
agggttttaa atggaggatt tcctttttcc tgtgacaaaa catcttttca caacttacct 240
tgttaagaca aaatttattt tccagggatt taatacgtac tttagttgga attattctat 300
gtcaatgatt tttaagctat gaaaaataca atggggggaa ggatagcatt tggagatata 360
cctaatgtta aatgacgagt tactggatgc agcacgcaac atggcacatg tgtatacata 420
tgtagctaac ccttcgttgt gcacatgtac cctaaaactt aaagtataat ttaaaaaaag 480
caaaaaaaaa aaataccaac tctttttttt aaaccaggaa ggaaaatgtg aacatggaaa 540
caacttctag tattggatct g 561

<210> 184
<211> 512
<212> DNA
<213> human
<400> 184
gccccggagt gcagctgcgg ggacccgctg ctcgaccccg gcctgccgga gcccgaggcc 60
ccgccccctg cgggtcccga accgcttacc ctcatccctg ggcctgtcga gcccttcagc 120
atcgtgacca tgcccgggcc ccggggtccg gcgccaccct ggctgcccag ccctattggc 180
gaggaggagg agaatctggc ctgagatctt agagcccagc cccctaagga cagggaacca 240
ggtccctgca cggcacccac gcaggtgtcc cggtctgcat aagcctcgta tgcctttgta 300
aagtccacct acacttttga ccagctctcg ctgcccgcat gtgtttggcg ctgtgctagg 360
ggcgggagtt cttccagact cttggaccag cccgccctga ccaccagctc tacttcccaa 420
cccccactgc ctgagaggtc tctatcagtg tcctgcctga attctttcct tcaagtgaag 480
atgtgactga ctacctcctc gagttgtcat ga 512

<210> 185
<211> 450
<212> DNA
<213> human
<400> 185
gaacccaggg agcgcgatgg gctgcagggc tgcgtcaggg ctcctgccag gagtggccgt 60
ggtcctcctg ctgctgctgc agagcacaca gtcagtctac atccagtacc aaggcttccg 120
ggtccagctg gaatccatga agaagctgag tgacctggag gcacagtggg cacccagccc 180
ccgcctgcag gcccagagcc tcctgcccgc cgtgtgccac caccctgctc tgcctcagga 240
ccttcagcct gtctgcgcct cgcaggaggc ttccagcatc ttcaagaccc tgaggaccat 300
cgctaacgac gactgtgagc tgtgtgtgaa cgttgcgtgt accggctgcc tctgagatag 360
ccctgggtac cctgagccca ccagggacac ctcgcccttc agcccaccac cctggcaggc 420
ttccatcccc gtccatgctc aagatgggtc 450

<210>  186
<211>  474
<212>  DNA
<213>  human
<400>  186
ttattcttag cgtcactggt ctggctttca gaattaacat acaaggttgc cacacctagt      60
tctgcccagc tttatgtctt ttattccagt attccaccaa agtttgtttt cctgcattcc     120
agttctcaag tcttaagata aagattgtac ttgacagttt agtatatcca taaaactatt     180
tgaggtggtt aaggttcttg ggttcatttt ccttaatact ttgctgaata ttgtagattg     240
taggcaatga aaaagtctac taaattagga aaaccttgaa taattaggta tcctaggtaa     300
gagcccctaa acatcaagca atctgtgagt ctgtaaagaa ataaatattt tttggattat     360
tcttatctaa ttccacccct gttggaagat gatttctttg ttctttgcaa ctatggaagc     420
tgtgaaaatc atcacaagtg cctctgaaag cgagtgttag gttggttaga gggt           474

<210>  187
<211>  569
<212>  DNA
<213>  human
<400>  187
gtgacggtgc tgaagaaggc cctggatgaa gagacgcggt cccatgaggc tcaggtccag      60
gagatgaggc agaaacacgc acaggcggtg gaggagctca cagagcagct tgagcagttc     120
aagagggcca aggcgaacct agacaagaat aagcagacgc tggagaaaga gaacgcagac     180
ctggccgggg agctgcgggt cctgggccag gccaagcagg aggtggaaca taagaagaag     240
aagctggagg cgcaggtgca gacactgagt ttttagaaaa acatatccac ggtaaccggt     300
ccctggcaat tctgtttaca tgaaatgggg agaaagtcac cgaaatgggt gccgccggcc     360
cccactccca attcattccc taacctgcaa acctttccaa cttctcacgt caggcctttg     420
agaattcttt ccccctctcc tggtttccac acctcagaca cgcacagttc accaagtgcc     480
ttctgtagtc acatgaattg aaaaggagac gctgctccca cggaggggag caggaatgct     540
gcactgttta caccctgact gtgcttaaa                                       569

<210>  188
<211>  422
<212>  DNA
<213>  human
<400>  188
tagtggccgt gaggagcatc ttcagtaaga aggaaggttc tgacagacaa tggaactacg      60
cctgcatgcc cacgccacag agcctcgggg aacccacgga gtgctggtgg gaggagatca     120
acagggctgg catggaatgg taccagacgt gctccaacaa tgggctggtg gcaggattcc     180
agagccgcta cttcgagtca gtgctggatc gggagtggca gttttactgt tgtcgctaca     240
gcaagaggtg cccatattcc tgctggctaa caacagaata tccaggtcac tatggtgagg     300
aaatggacat gatttcctac aattatgatt actatatccg aggagcaaca accactttct     360
ctgcagtgga aagggatcgc cagtggaagt tcataatgtg ccggatgact gaatacgact     420
gt                                                                    422

<210>  189
<211>  183
<212>  DNA
<213>  human
<400>  189
aatgtcatag acactgattt catcgacgag gaagttctta tgtccttggt gatagaaatg      60
ggtttggacc gcatcaagga gctgcccgaa ctctggctgg ggcaaaacga gtttgatttt     120
atgacggact tcgtgtgcaa acagcagccc agcagagtga gctgttgact cgatcgaaac     180
ccc                                                                   183

<210>  190
<211>  434
<212>  DNA
<213>  human

<220>
<221>  misc_feature
<222>  (158)..(158)

<223>  n is a, c, g, or t

<400>  190
ccccccaatt gctgccaata gtggataaca tgtatcactc actgccaaaa atagaaagtg      60
accatgaaaa ataaattcgc tggggaaggg ggctccatgc tggtgtggcc aaggctgaga     120
gctctctctt ctctgttaca aaacgagata agcaagtntt agaattgcct taaggccaca     180
ctggcatctc cctgaccttc tccagggaca gaagcaggag taagtttctc atcccatggg     240
cgaccagggc catctcctcc caccagtggc ccccactcac agggagctgg caatgcccta     300
cctgcctgtt ctccagatgg agaaacaggc tctgagattt cacaggtctt gcccaaagtc     360
attgatttta atgattaaaa agaataaaca cagtgtttcc tgagtagcag tgattgttat     420
gccttgctat ttta                                                       434

<210>  191
<211>  463
<212>  DNA
<213>  human
<400>  191
gatgtattaa cctacctgtg aatcatatgt tgtaggaaaa gctgttccca tgtctaacag      60
gacttgaatt caaagcatgt caagtggata gtagatctgt ggcgatatga gagggatgca     120
gtgcctttcc ccattcattc ctgatggaat tgttatacta ggttaacatt tgtaattttt     180
ttctagttgt aatgtgtatg tctggtaaat aggtattata ttttggcctt acaataccgt     240
aacaatgttt gtcattttga aatacttaat gccaagtaac aatgcatgct ttggaaattt     300

ggaagatggt tttattcttt gagaagcaaa tatgtttgca ttaaatgctt tgattgttca     360
tatcaagaaa ttgattgaac gttctcaaac cctgtttacg gtacttggta agagggagcc     420
ggtttgggag agaccattgc atcgctgtcc aagtgtttct tgt                       463

<210>  192
<211>  468
<212>  DNA
<213>  human
<400>  192
agggcaagtg ttcccaaatt gacgccacct aataatcatc accacaccgg gagcagatct      60
gaaggcacac tttgattttt ttaaggataa gaaccacaga acactgggta gtagctaatg     120
aaattgagaa gggaaatctt agcatgcctc caaaaattca catccaatgc atactttgtt     180
caaatttaag gttactcagg cattgatctt ttcagtgttt tttcacttag ctatgtggat     240
tagctagaat gcacaccaaa aagatacttg agctgtatat atatatgtgt gtgtgtgtgt     300
gtgtgtgtgt gtgtgtgcat gtatgtgcac atgtgtctgt gtgatatttg gtatgtgtat     360
ttgtatgtac tgttattcaa aatatattta atacctttgg aaaatcttgg gcaagatgac     420
ctactagttt tccttgaaaa aaagttgctt tgttattaat attgtgct                  468

<210>  193
<211>  531
<212>  DNA
<213>  human
<400>  193
ccgagccgag cttactgtga gtgtggagat gttatcccac catgtaaagt cgcctgcgca      60
ggggagggct gcccatctcc ccaacccagt cacagagaga taggaaacgg catttgagtg     120
ggtgtccagg gccccgtaga gagacattta agatggtgta tgacagagca ttggccttga     180
ccaaatgtta aatcctctgt gtgtatttca taagttatta caggtataaa agtgatgacc     240
tatcatgagg aaatgaaagt ggctgatttg ctggtaggat tttgtacagt ttagagaagc     300
gattatttat tgtgaaactg ttctccactc caactccttt atgtggatct gttcaaagta     360
gtcactgtat atacgtatag agaggtagat aggtaggtag attttaaatt gcattctgaa     420
tacaaactca tactccttag agcttgaatt acatttttaa aatgcatatg tgctgtttgg     480
caccgtggca agatggtatc agagagaaac ccatcaattg ctcaaatact c              531

<210>  194
<211>  392
<212>  DNA
<213>  human
<400>  194
ggagctgtca ccatgacggg ggaacttgag gttaagaaca tggacatgaa gccggggtca      60
accctgaaga tcacaggcag catcgccgat ggcactgatg gctttgtaat taatctgggc     120

caggggacag acaagctgaa cctgcatttc aaccctcgct tcagcgaatc caccattgtc 180
tgcaactcat tggacggcag caactggggg caagaacaac gggaagatca cctgtgcttc 240
agcccagggt cagaggtcaa gttcacagtg acctttgaga gtgacaaatt caaggtgaag 300
ctgccagatg ggcacgagct gacttttccc aacaggctgg gtcacagcca cctgagctac 360
ctgagcgtaa ggggcgggtt caacatgtcc tc 392

<210> 195
<211> 233
<212> DNA
<213> human
<400> 195
gcgtgttttc ctcttgatcg ggaactcctg cttctccttg cctcgaaatg gaccccaact 60
gctcctgctc gcctgttggc tcctgtgcct gtgccggctc ctgcaaatgc aaagagtgca 120
aatgcacctc ctgcaagaag agctgctgct cctgctgccc tgtcggctgt gccaagtgtg 180
cccagggctg catctgcaaa gggacgtcag acaagtgcag ctgctgtgcc tga 233

<210> 196
<211> 578
<212> DNA
<213> human
<400> 196
cccgaccatc gaatcttgcg aacaacacga tacttgttaa gtggctaccc caaaacgatc 60
tgcttggtca cccgatgacc cgtgccttta tcacccatgc tggttcccat ggtgtttatg 120
aaagcatatg caatggcgtt cccatggtga tgatgccctt gtttggtgat cagatggaca 180
atgcaaagcg catggagact aagggagctg gagtgaccct gaatgttctg gaaatgactt 240
ctgaagattt agaaaatgct ctaaaagcag tcatcaatga caaaagttac aaggagaaca 300
tcatgcgcct ctccagcctt cacaaggacc gcccggtgga gccgctggac ctggccgtgt 360
tctgggtgga gtttgtgatg aggcacaagg gcgcgccaca cctgcgcccc gcagcccacg 420
acctcacctg gtaccagtac cattccttgg acgtgattgg tttcctcttg gccgtcgtgc 480
tgacagtggc cttcatcacc tttaaatgtt gtgcttatgg ctaccggaaa tgcttgggga 540
aaaaagggcg agttaagaaa gcccacaaat ccaagacc 578

<210> 197
<211> 509
<212> DNA
<213> human
<400> 197
   tgcagaggcc tatgttttca ctcctaacat gatctgtgct ggaggagaga agggcatgga 60
   tagctgtaaa ggggacagtg gtggggcctt tgctgtacag gatcccaatg acaagaccaa 120
   attctacgca gctggcctgg tgtcctgggg gccccagtgt gggacctatg ggctctacac 180
   acgggtaaag aactatgttg actggataat gaagactatg caggaaaata gcaccccccg 240
   tgaggactaa tccagataca tcccaccagc ctctccaagg gtggtgacca atgcattacc 300
   ttctgttcct tatgatattc tcattatttc atcatgactg aaagaagaca cgagcgaatg 360
   atttaaatag aacttgattg ttgagacgcc ttgctagagg tagagtttga tcatagaatt 420
   gtgctggtca tacatttgtg gtctgactcc ttggggtcct ttccccggag tacctattgt 480
   agataacact atgggtgggg cactccttt 509

<210> 198
<211> 507
<212> DNA
<213> human
<400> 198
gggcctaacc agccttggga gtattgactg gtcccttacc tcttatggct aagtctttga 60
ctgtgttcat ttaccaagtt gacccagttt gtcttttagg ttaagtaaga ctcgagagta 120
aaggcaagga ggggggccag cctctgaatg cggccacgga tgccttgctg ctgcaaccct 180
ttccccagct gtccactgaa acgtgaagtc ctgttttgaa tgccaaaccc accattcact 240
ggtgctgact acatagaatg gggttgagag aagatcagtt tgggcttcac agtgtcattt 300
gaaaacgttt tttgttttgt tttgtaatta ttgtggaaaa ctttcaagtg aacagaagga 360
tggtgtccta ctgtggatga gggatgaaca aggggatggc tttgatccaa tggagcctgg 420
gaggtgtgcc cagaaagctt gtctgtagcg ggttttgtga gagtgaacac tttccacttt 480
ttgacacctt atcctgatgt atggttc 507

<210> 199

<211>  510
<212>  DNA
<213>  human
<400>  199
agatgtgttt agaacctctt gtttaaaaat aatagactgc ttatcataaa atcacatctc      60
acacatttga ggcagtggtc aaacaggtaa agcctatgat gtgtgtcatt ttaaagtgtc     120
ggaatttagc ctctgaatac cttctccatt gggggaaaga tattcttgga accactcatg     180
acatatctta gaaggtcatt gacaatgtat aaactaattg ttggtttgat atttatgtaa     240
atatcagttt accatgcttt aattttgcac attcgtacta tagggagcct attggttctc     300
tattagtctt gtgggttttc tgtttgaaaa ggagtcatgg catctgttta catttacctt     360
atcaaaccta gaatgtgtat atttataaat gtatgtcttc attgctaggt actaatttgc     420
agatgtcttt acatatttca atacagaaac tataacattc aatagtgtgc tgtcaaagtg     480
tgcttagctc acctggatat acctacattg                                      510

<210>  200
<211>  524
<212>  DNA
<213>  human

<220>
<221>  misc_feature
<222>  (314)..(320)
<223>  n is a, c, g, or t

<400>  200
gaagggagtg ttgctcccag tccagaggcc tgattctgtt cggactgggt tctcaagaca      60
cgaccaggtt ctcaagacac gagtcccctt gttcctcccc attaaagggg gtttgtcaga     120
agcaagaaca gcccctctcc ccagtcacag cctgaaggga ggccccgaga gcttcctcct     180
tccccccacc tgctccttac cttctctgcc ctgcttttta gaactgcagt tcattgtttt     240
aagggattgg gggagggagc ctggggacac aaacctttta tacaatacaa agctttgctt     300
tttttttttt tttnnnnnnn ccttttccct ttctcggttc tcttctctcc tctgaatggc     360
tgaagacccc tctgccgagg gaggttgggg attgtgggac aaggtccctt ggtgctgatg     420
gcctgaaggg gcctgagctg tgggcagatg cagttttctg tgggcttggg gaacctctca     480
cgttgctgtg tcctggtgag cagcccgacc aataaacctg cttt                      524

<210>  201
<211>  406
<212>  DNA
<213>  human
<400>  201
ggctgcctgc ggatgaagga ccagtgtgac aagtgccggg agatcttgtc tgtggactgt      60
tccaccaaca acccctccca ggctaagctg cggcgggagc tcgacgaatc cctccaggtc     120
gctgagaggt tgaccaggaa atataacgag ctgctaaagt cctaccagtg gaagatgctc     180
aacacctcct ccttgctgga gcagctgaac gagcagttta actgggtgtc ccggctggca     240
aacctcacgc aaggcgaaga ccagtactat ctgcgggtca ccacggtggc ttcccacact     300
tctgactcgg acgttccttc cggtgtcact gaggtggtcg tgaagctctt tgactctgat     360
cccatcactg tgacggtccc tgtagaagtc tccaggaaga acccta                    406

<210>  202
<211>  286
<212>  DNA
<213>  human
<400>  202
agcagctgaa cgagcagttt aactgggtgt cccggctggc aaacctcacg caaggcgaag      60
accagtacta tctgcgggtc accacggtgg cttcccacac ttctgactcg gacgttcctt     120
ccggtgtcac tgaggtggtc gtgaagctct ttgactctga tcccatcact gtgacggtcc     180
ctgtagaagt ctccaggaag aaccctaaat ttatggagac cgtggcggag aaagcgctgc     240
aggaataccg caaaaagcac cgggaggagt gagatgtgga tgttgc                    286

<210>  203
<211>  101
<212>  DNA
<213>  human

<400>  203
cgatcaccaa tgtacctcca gaggtaactg tgctcacgaa cagccctgtg gaactgagag      60
agcccaacgt cctcatctgt ttcatagaca agttcacccc a                          101

<210>  204
<211>  530
<212>  DNA
<213>  human

<220>
<221>  misc_feature
<222>  (115)..(115)
<223>  n is a, c, g, or t
<220>
<221>  misc_feature
<222>  (117)..(117)
<223>  n is a, c, g, or t
<220>
<221>  misc_feature
<222>  (121)..(123)
<223>  n is a, c, g, or t

<400>  204
gatataccat gtattcacac tttaaaaaat acacatagca gagtatcgga aagtatgtac      60
caaaacattg atctggctac cactgggagt caggatcatg agttcatctt cttcntnctt     120
nnnactgctt ttccaaacat tctccagtaa gcaggtacta catttataat ggaaggaatt     180
ttttaaaaaa ttttaagctg tacactttca agtaagatct gaattctaat gctggcttgt     240
gcctcttact atgtggtttg gtcattatag ataatgccca gtttcagttt ccccatcttt     300
gaaaaggaga taatatgtta tctcttggga gtagttctga atatgaagtg tgttgataca     360
agaagcactg acaatgtttc tgtttcttaa atttaaaact ggcctggttt gcctttttta     420
tcaagagagc ttaacagata aaaaatgaaa ttagtctatt ttctacttgc cagcagagta     480
tctgtcttat tttaggatgc agtgtgaaac ttaccattca actgaacaac                 530

<210>  205
<211>  480
<212>  DNA
<213>  human

<220>
<221>  misc_feature
<222>  (431)..(431)
<223>  n is a, c, g, or t
<400>  205
gaaggctgga ttctatctac ataagtcctt tcaattccac cagggccaga gcagctccac      60
cactgtgcac ttagccatga tggcaacaga aaccaagaga cacaattacg caggtattta     120
gaagcagagg gacaaccaga aggcccttaa ctatcaccag tgcatcacat ctgcacactc     180
tcttctccat tccctagcag gaacttctag ctcatttaac agataaagaa actgaggccc     240
acggtttcag ctagacaatg atttggccag gcctagtaac caaggccctg tctctggcta     300
ctccctggac cacgaggctg attcctctca tttccagctt ctcagtttct gcctgggcaa     360
tggccagggg ccaggagtgg ggagagttgt gatggagggg agaggggtca cacccacccc     420
ctgcctggtt ntaggctgct gcacaccaag gccctgcatc tgtctgctct gcatatatgt     480

<210>  206
<211>  546
<212>  DNA
<213>  human
<400>  206
agacagctgt ttagggtctt ctcccctcac ccatgctttc atcatcccct ccgcacagcc      60
tccccgtcca ggccttctaa ccacacctac ccagggctgc cgcattcctg cactcagaag     120
tctgcagcgg tgcctcacaa acttgattgt gcataaaaat cactggggat cttgttaata     180
cagattctaa ctcaatagat ctgggagatc ctgcatttct aacaagctcc caggtaaggc     240
ggaggctgct ggtgtgagga ccatgctgtg agcagcaggg cgagagtgcc cagggctgat     300
atatattgga aatatcaccc ctgaagccat cgctggcccc cacctcctgt ggactgatgc     360

cccagggatt cccaccccac ttctgcaacc ccaggtatcc ttcattatcc accccatccc 420
agactcccac cccagggatt gcccgtgaag actttggcct agcaaattgt gttggttatg 480
tgagtgttgt tttaatcaga gatgtacatg attgccaatc tgcatttctt accagtgtga 540
ccacac 546

<210> 207
<211> 562
<212> DNA
<213> human
<400> 207
atcatcagat cgactcttat acgagtaata tgcctgctat ttgaagtgta attgagaagg 60
aaaattttag cgtgctcact gacctgcctg tagccccagt gacagctagg atgtgcattc 120
tccagccatc aagagactga gtcaagttgt tccttaagtc agaacagcag actcagctct 180
gacattctga ttcgaatgac actgttcagg aatcggaatc ctgtcgatta gactggacag 240
cttgtggcaa gtgaatttgc ctgtaacaag ccagattttt taaaatttat attgtaaata 300
ttgtgtgtgt gtgtgtgtgt gtatatatat atatatatgt acagttatct aagttaattt 360
aaagttgttt gtgccttttt atttttgttt ttaatgcttt gatatttcaa tgttagcctc 420
aatttctgaa caccataggt agaatgtaaa gcttgtctga tcgttcaaag catgaaatgg 480
atacttatat ggaaattctg ctcagataga atgacagtcc gtcaaaacag attgtttgca 540
aaggggaggc atcagtgtct tg 562

<210> 208
<211> 553
<212> DNA
<213> human
<400> 208
tacaccacaa tggctgagca cttcctgacg ttgctggtag tgcctgccat caagaaagat 60
tatggttccc aggaagactt cactcaagtg tggaacacca ccatgaaagg gctcaagtgc 120
tgtggcttca ccaactatac ggattttgag gactcaccct acttcaaaga gaacagtgcc 180
tttcccccat tctgttgcaa tgacaacgtc accaacacag ccaatgaaac ctgcaccaag 240
caaaaggctc acgaccaaaa agtagagggt tgcttcaatc agcttttgta tgacatccga 300
actaatgcag tcaccgtggg tggtgtggca gctggaattg ggggcctcga gctggctgcc 360
atgattgttt ccatgtatct gtactgcaat ctacaataag tccacttctg cctctgccac 420
tactgctgcc acatgggaaa ctgtgaagag gcaccctggg caagcagcag tgattggggg 480
aggggacagg atctaacaat gtcacttggg ccagaatgga cctggccttt ctgctcccag 540
acttgggggc tag 553

<210> 209
<211> 220
<212> DNA
<213> human

<220>
<221> misc_feature
<222> (132)..(132)
<223> n is a, c, g, or t
<220>
<221> misc_feature
<222> (135)..(135)
<223> n is a, c, g, or t
<220>
<221> misc_feature
<222> (159)..(159)
<223> n is a, c, g, or t
<220>
<221> misc_feature
<222> (162)..(162)
<223> n is a, c, g, or t
<220>
<221> misc_feature
<222> (172)..(172)
<223> n is a, c, g, or t
<220>

<221>  misc_feature
<222>  (174)..(174)
<223>  n is a, c, g, or t
<220>
<221>  misc_feature
<222>  (180)..(180)
<223>  n is a, c, g, or t
<400>  209
gcagttatgg gcaaccctaa ggtgaaggct catggcaaga aagtgctcgg tgcctttagt      60
gatggcctgg ctcacctgga caacctcaag ggcacctttg ccacactgag tgagctgcac     120
tgtgacaagc tncangtgga tcctgagaac ttcaggctnc tnggcaacgt gntngtctgn     180
gtgctggccc atcactttgg caaagaattc accccaccag                           220

<210>  210
<211>  417
<212>  DNA
<213>  human
<400>  210
tctctgggct ccaggctgag gacgaggctg attattactg ctgctcatat gcaggtagtt      60
acactgtggt tttcggcgga gggaccaaac tgaccgtcct aggtcagccc aaggctgccc     120
cctcggtcac tctgttcccg ccctcctctg aggagcttca agccaacaag gccacactgg     180
tgtgtctcat aagtgacttc tacccgggag ccgtgacagt ggcctggaag gcagatagca     240
gccccgtcaa ggcgggagtg gagaccacca caccctccaa acaaagcaac aacaagtacg     300
cggccagcag ctatctgagc ctgacgcctg agcagtggaa gtcccacaga agctacagct     360
gccaggtcac gcatgaaggg agcaccgtgg agaagacagt ggcccctaca gaatgtt        417

<210>  211
<211>  532
<212>  DNA
<213>  human
<400>  211
tttggtgcag ctgctagtca gtccctgact gacattgcca agtattcaat aggcagactg      60
cggcctcact tcttggatgt ttgtgatcca gattggtcaa aaatcaactg cagcgatggt     120
tacattgaat actacatatg tcgagggaat gcagaaagag ttaaggaagg caggttgtcc     180
ttctattcag gccactcttc gttttccatg tactgcatgc tgtttgtggc actttatctt     240
caagccagga tgaagggaga ctgggcaaga ctcttacgcc ccacactgca atttggtctt     300
gttgccgtat ccatttatgt gggcctttct cgagtttctg attataaaca ccactggagc     360
gatgtgttga ctggactcat tcagggagct ctggttgcaa tattagttgc tgtatatgta     420
tcggatttct tcaaagaaag aacttctttt aaagaaagaa aagaggagga ctctcataca     480
actctgcatg aaacaccaac aactgggaat cactatccga gcaatcacca gc             532

<210>  212
<211>  361
<212>  DNA
<213>  human
<400>  212
gcacgccatc aatgccatcg tgcgcagccc gcgtggcggg gcccggaggc acgcagagct      60
gtccttcgtg ttcctcacgg acggcgtcac gggcaacgac agtctgcacg agtcggcgca     120
ctccatgcgc aacgagaacg tggtacccac cgtcctggcc ttgggcagcg acgtggacat     180
ggacgtgctc accacgctca gcctgggtga ccgcgccgcc gtgttccacg agaaggacta     240
tgacagcctg gcgcaacccg gcttcttcga ccgcttcatc cgctggatct gctagcgccg     300
ccgcccgggc cccgcagtcg agggtcgtga gcccaccccg tccatggtgc taagcgggcc     360
c                                                                     361

<210>  213
<211>  498
<212>  DNA
<213>  human
<400>  213
tgtttcctca tagctagttc tcaagctgca tgtaagattt taacggaaag agaaaatagg      60
cctggacctg aaggtctcaa atatgttgag aagaaagtat gaactataag gaacttgaga     120
tgtagattta ttttgcagga aatacgagga aaataggaaa gaagtgtttg ccgcatcaag     180
tgtaaagaat gatactgagg atttttacat tttatgaaat gaaataatgg catttacaaa     240

atgaaaaatg tagtttcaca actaagtttt gttaacagag actgcatgct ttgcttatag 300
ttcttaattt tggttttgac attcatttaa ttttttccat gttaaatatg tagtttaatt 360
atttactcaa aataaacatt gttcatgctt ttaggccttt gggggaattg atttttatcc 420
acaggtagaa aatggtcttt gcacacacta cacttatttc aaatatacaa tgtgctcccg 480
aactttcgca ttagtctt 498

<210> 214
<211> 491
<212> DNA
<213> human
<400> 214
gaaatatcac tgacactcca gactaaagca gagtctaggt ttctgcaatt ttgttacagt 60
aatttgtaaa tagctttagt aaactcacct tgcatggtag attaataaga tgacttactg 120
tacatgaatt acacaataat gagatctggt ggctatttcc acattttgaa aaggattcag 180
ttatttactg acagtggtga gcatcctttt taaaataatg ttctgatact taaacattag 240
agagcagtat ctttaaatga attattaaca ctttggaata cttacatttt ctgttatttt 300
tgattgcctg ataaccagtt tcaatgatga aaatgaaaac aagtgctgaa gatgaaatgg 360
aagagaaccg ttttaatctg gattttgttt tgtcacacct ggaaaatact ttgcaaatat 420
gttctaaatt gaaaacaatt tttttatgat cacatggttc actaccaaat gaccctcaaa 480
taagccagat g 491

<210> 215
<211> 389
<212> DNA
<213> human
<400> 215
gatagtcgga atagagccgc cccaactcag atcctacaac acgcaagttc cttcttgaac 60
cctggtgcct cctaccctat ggccctgaat ggtgcactgg tttaattgtg ttggtgtcgg 120
cccctcacaa atgcagccaa gtcatgtaat tagtcatctg gaacaaagac taaaaacagc 180
agagaattgc gggttctacc cagtcagaag atcacaccat ggagactttc tactagagga 240
cttgaaagag aactgagggg ccacaaaata aacttcacct tccattaagt gttcaagcat 300
gtctgcaaat taggagggag ttagaaacag tctttttcat cctttgtgat gaagcctgaa 360
attgtgccgt gttgccttat atgaatatg 389

<210> 216
<211> 515
<212> DNA
<213> human
<400> 216
tgtctccccg ctatctaaaa aagtataaga acaagcagat aacagcgaga atcttggagg 60
cccatcagaa tgtagctcag atgagtctaa ttgaagccaa gatgagattt attcaagctt 120
ggcagtcact acctgaattt ggcatcactc acttcattgc aaggttccaa gggggcaaaa 180
aagaagaact tattggaatt gcatacaaca gactgattcg gatggatgcc agcactggag 240
atgcaattaa aacatggcgt ttcagcaaca tgaaacagtg gaatgtcaac tgggaaatca 300
aaatggtcac cgtagagttt gcagatgaag tacgattgtc cttcatttgt actgaagtag 360
attgcaaagt ggttcatgaa ttcattggtg gctacatatt tctctcaaca cgtgcaaaag 420
accaaaacga gagtttagat gaagagatgt tctacaaact taccagtggt tgggtgtgaa 480
tagaaatact gtttaatgaa actccacggc cataa 515

<210> 217
<211> 536
<212> DNA
<213> human

<220>
<221> misc_feature
<222> (26)..(26)
<223> n is a, c, g, or t
<220>
<221> misc_feature
<222> (64)..(64)
<223> n is a, c, g, or t
<220>

<221>  misc_feature
<222>  (124)..(124)
<223>  n is a, c, g, or t
<400>  217
aaaatgctat tagtccgtcg tgcttnattt gtttttgtcc ttgaataagc atgttatgta      60
tatngtctcg tgtttttatt tttacaccat attgtattac acttttagta ttcaccagca     120
taancactgt ctgcctaaaa tatgcaactc tttgcattac aatatgaagt aaagttctat     180
gaagtatgca ttttgtgtaa ctaatgtaaa aacacaaatt ttataaaatt gtacagtttt     240
ttaaaaacta ctcacaacta gcagatggct taaatgtagc aatctctgcg ttaattaaat     300
gcctttaaga gatataatta acgtgcagtt ttaatatcta ctaaattaag aatgacttca     360
ttatgatcat gatttgccac aatgtcctta actctaatgc ctggactggc catgttctag     420
tctgttgcgc tgttacaatc tgtattggtg ctagtcagaa aattcctagc tcacatagcc     480
caaaagggtg cgagggagag gtggattacc agtattgttc aataatccat ggttca         536

<210>  218
<211>  514
<212>  DNA
<213>  human
<400>  218
gaccagttct tcggagagca cctgttggag tctgatcttt tcccgacgtc tacttccctg      60
agtcccttct accttcggcc accctccttc ctgcgggcac ccagctggtt tgacactgga     120
ctctcagaga tgcgcctgga gaaggacagg ttctctgtca acctggatgt gaagcacttc     180
tccccagagg aactcaaagt taaggtgttg ggagatgtga ttgaggtgca tggaaaacat     240
gaagagcgcc aggatgaaca tggtttcatc tccagggagt tccacaggaa ataccggatc     300
ccagctgatg tagaccctct caccattact tcatccctgt catctgatgg ggtcctcact     360
gtgaatggac caaggaaaca ggtctctggc cctgagcgca ccattcccat cacccgtgaa     420
aagaagcctg ctgtcaccgc agcccccaag aaaaagatgc cctttcttga attgcatttt     480
ttaaaacaag aaagtttccc caccagtgaa tgaa                                 514

<210>  219
<211>  293
<212>  DNA
<213>  human
<400>  219
tgaatcttcg ggtgtttccc tttagctaag cacagatcta ccttggtgat ttggaccctg      60
gttgctttgt gtctagtttt ctagaccctt catctcttac ttgatagact tactaataaa     120
atgtgaagac tagaccaatt gtcatgcttg acacaactgc tgtggctggt tggtgctttg     180
tttatggtag tagtttttct gtaacacaga atataggata agaaataaga ataaagtacc     240
ttgactttgt tcacagcatg tagggtgatg agcactcaca attgttgact aaa            293

<210>  220
<211>  398
<212>  DNA
<213>  human
<400>  220
ctgtgtatcc ttcaaagacc cagcccctgc agcaccataa cctcctggtc tgttctgtga      60
gtggtttcta tccaggcagc attgaagtca ggtggttccg gaatggccag gaagagaaga     120
ctggggtggt gtccacaggc ctgatccaca atggagactg gaccttccag accctggtga     180
tgctggaaac agttcctcgg agtggagagg tttacacctg ccaagtggag cacccaagcg     240
tgacaagccc tctcacagtg gaatggagag cacggtctga atctgcacag agcaagatgc     300
tgagtggagt cgggggcttt gtgctgggcc tgctcttcct tggggccggg ctgttcatct     360
acttcaggaa tcagaaagga cactctggac ttcagcca                             398

<210>  221
<211>  396
<212>  DNA
<213>  human

<220>
<221>  misc_feature
<222>  (148)..(149)
<223>  n is a, c, g, or t
<220>

<221>  misc_feature
<222>  (151)..(154)
<223>  n is a, c, g, or t
<220>
<221>  misc_feature
<222>  (156)..(156)
<223>  n is a, c, g, or t
<400>  221
agtcaaatgc caaacactag ctctgtatta atccccatca ttactggtaa agcctcattt      60
gaatgtgtga attcaataca ggctatgtaa aatttttact aatgtcatta ttttgaaaaa     120
ataaatttaa aaatacattc aaaattanna nnnnanacaa gcttaattgt taatattccc     180
taaacacaat tttatgaagg gagaagacat tggtttgttg acaataacag tacatctttt     240
caagttctca gctatttctt ctacctctcc ctatcttaca tttgagtatg gtaacttatg     300
tcatctatgt tgaatgtaag cttataaagc acaaagcata catttcctga ctggtctaga     360
gaactgatgt ttcaatttac ccctctgcta aataaa                               396

<210>  222
<211>  396
<212>  DNA
<213>  human
<400>  222
ggcaaactgc ttaatcttgt ggattttgta gatggtttca aatgactgaa ctgcattcag      60
atttacgagt gaaaggaaaa attgcattag ttggttgcat gaactttgaa gggcagatat     120
tactgcacaa actgccatct cgcttcattt ttttaactat gcatttgagt acagactaat     180
ttttaaaata tgctaaactg gaagattaaa cagatgtggc ccaaactgtt ctggatcagg     240
aaagtcatac tgttcacttt caagttggct gtcccccccg ccgccccccc ccacccccat     300
atgtacagat gataataggg tgtggaatgt cgtcagtggc aaacatttca cagattattt     360
tgtttctgtc ttcaacattt ttgacactgt gctaat                               396

<210>  223
<211>  479
<212>  DNA
<213>  human
<400>  223
ctcctccatg agtctgacat ctcggaaact gagcagctgc cggacgcctg ggtcaggaat      60
ccaagacccc acctcttaag gactggttcc tcagaaagca ccctcaggga aaaaggtgaa     120
aacattacat ccgtggattc tcctgccaca accgcattgg aagaaaaggc tgccgcaaca     180
tctcagcgag gagtgaagga cccatgtccc aggaaccgcg ctgcgccacc tgcactcacc     240
cccctcacat tctcttaagc acccggtggc cctccgaggc ctggcggaat ggtggtgccc     300
acggggttgg gcaagggctc accaggacct caacgggcaa agttgtgcac actaaaatat     360
caaatcaagg tgcttggttt taaagtaaat gtttttctaa agaaagctgt gttcttctgt     420
tgacccagac gaatagggca cagccctgta actgcacgtg ccttctgtca ttgggaatg      479

<210>  224
<211>  259
<212>  DNA
<213>  human
<400>  224
caccatcacg tgcctggtga cgggcttctc tcccgcggac gtcttcgtgc agtggatgca      60
gagggggcag cccttgtccc cggagaagta tgtgaccagc gccccaatgc ctgagcccca     120
ggccccaggc cggtacttcg cccacagcat cctgaccgtg tccgaagagg aatggaacac     180
gggggagacc tacacctgcg tggtggccca tgaggccctg cccaacaggg tcaccgagag     240
gaccgtggac aagtccacc                                                  259

<210>  225
<211>  340
<212>  DNA
<213>  human

<220>
<221>  misc_feature
<222>  (208)..(208)
<223>  n is a, c, g, or t

<220>
<221>  misc_feature
<222>  (262)..(262)
<223>  n is a, c, g, or t

<400>  225
ttctttttta tattgtcctc cacctccatc attttcaata aaagataggg cttttgctcc      60
cttgttcttg gagggaccat tattacatct ctgaactacc tttgtatcca acatgtttta     120
aatccttaaa tgaattgctt tctcccaaaa aaagcacaat ataaagaaac acaagattta     180
attatttttc tacttggggg gaaaaaangt cctcatgtag aagcacccac ttttgcaatg     240
ttgttctaag ctatctatct anctctcagc ccatgataaa gttccttaag ctggtgattc     300
ctaatcaagg acaagccacc ctagtgtctc atgtttgtat                           340

<210>  226
<211>  505
<212>  DNA
<213>  human
<400>  226
ggaagcacaa tttccacctt attttttgaa ctttggcagt ttcaatgtct gtctctgttg      60
cttcggggca taagctgatc accgtctagt tgggaaagta accctacagg gtttgtaggg     120
acatgatcag catcctgatt tgaaccctga aatgttgtgt agacaccctc ttgggtccaa     180
tgaggtagtt ggttgaagta gcaagatgtt ggcttttctg gatttttttt gccatgggtt     240
cttcactgac cttggacttt ggcatgattc ttagtcatac ttgaacttgt ctcattccac     300
ctcttctcag agcaactctt cctttgggaa aagagttctt cagatcatag accaaaaaag     360
tcataccttc gaggtggtag cagtagattc caggaggaga agggtacttg ctaggtatcc     420
tgggtcagtg gcggtgcaaa ctggtttcct cagctgcctg tccttctgtg tgcttatgtc     480
tcttgtgaca attgttttcc tccct                                           505

<210>  227
<211>  445
<212>  DNA
<213>  human
<400>  227
agagaaccca ccatggtgct gtctcctgcc gacaagacca acgtcaaggc cgcctggggt      60
aaggtcggcg cgcacgctgg cgagtatggt gcggaggccc tggagaggat gttcctgtcc     120
ttccccacca ccaagaccta cttcccgcac ttcgacctga gccacggctc tgcccaggtt     180
aagggccacg gcaagaaggt ggccgacgcg ctgaccaacg ccgtggcgca cgtggacgac     240
atgcccaacg cgctgtccgc cctgagcgac ctgcacgcgc acaagcttcg ggtggacccg     300
gtcaacttca agctcctaag ccactgcctg ctggtgaccc tggccgccca cctccccgcc     360
gagttcaccc ctgcggtgca cgcctccctg gacaagttcc tggcttctgt gagcaccgtg     420
ctgacctcca aataccgtta agctg                                           445

<210>  228
<211>  499
<212>  DNA
<213>  human
<400>  228
tggaggaatt tcacaagcac ccgcccgtgc agtgggcctt ccaggagacc agtgtggaga      60
gcgccgtgga cacgcccttc ccagctggaa tatttgtgag gctggaattt aagctgcagc     120
agacaagctg ccggaagagg gactggaaga aacccgagtg caaagtcagg cccaatggga     180
ggaaacggaa atgcctggcc tgcatcaaac tgggctctga ggacaaagtt ctgggccggt     240
tggtccactg ccccatagag acccaagttc tgcgggaggc tgaggagcac caggagaccc     300
agtgcctcag ggtgcagcgg gctggtgagg acccccacag cttctacttc cctggacagt     360
tcgccttctc caaggccctg ccccgcagct aagccagcac tgagctgcgt ggtgcctcca     420
ggaccgctgc cggtggtaac cagtggaaga ccccagcccc cagggagagg accccgttct     480
atccccagcc atgataata                                                  499

<210>  229
<211>  465
<212>  DNA
<213>  human
<400>  229
tttgtcagga aaccttggcc tctgctaagg tgtatttggt ccttgagaag tgggagcacc      60

ctacagggac actatcactc atgctggtgg cattgtttac agctagaaag ctgcactggt 120
gctaatgccc cttggggaaa tggggctgtg aggaggagga ttataactta ggcctagcct 180
cttttaacag cctctgaaat ttatcttttc ttctatgggg tctataaatg tatcttataa 240
taaaaaggaa ggacaggagg aagacaggca aatgtacttc tcacccagtc ttctacacag 300
atggaatctc tttggggcta agagaaaggt tttattctat attgcttacc tgatctcatg 360
ttaggcctaa gaggctttct ccaggaggat tagcttggag ttctctatac tcaggtacct 420
ctttcagggt tttctaaccc tgacacggac tgtgcatact ttccc 465

<210> 230
<211> 526
<212> DNA
<213> human
<400> 230
actggatgcg ttaataaccc atgttttacc ttttgaaaaa ataaatgaag gatttgacct 60
gcttcactct gggaaaagta tccgtaccgt cctgacgttt tgaggcaata gagatgcctt 120
cccctgtagc agtcttcagc ctcctctacc ctacaagatc tggagcaaca gctaggaaat 180
atcattaatt cagctcttca gagatgttat caataaatta cacatggggg ctttccaaag 240
aaatggaaat tgatgggaaa ttatttttca ggaaaattta aaattcaagt gagaagtaaa 300
taaagtgttg aacatcagct ggggaattga agccaacaaa ccttccttct taaccattct 360
actgtgtcac ctttgccatt gaggaaaaat attcctgtga cttcttgcat ttttggtatc 420
ttcataatct ttagtcatcg aatcccagtg gaggggaccc ttttacttgc cctgaacata 480
cacatgctgg gccattgtga ttgaagtctt ctaactctgt ctcagt 526

<210> 231
<211> 555
<212> DNA
<213> human
<400> 231
gcagatttct tgcttcatat gacaaagcct caattactaa ttgtaaaaac tgaactattc 60
ccagaatcat gttcaaaaaa tctgtaattt ttgctgatcg aaagtgcttc attgactaaa 120
cagtattagt ttgtggctat aaatgattat ttagatgatg actgaaaatg tgtataaagt 180
aattaaaagt aatatggtgg ctttaagtgt agagatggga tggcaaatgc tgtgaatgca 240
gaatgtaaaa ttggtaacta agaaatggca caaacacctt aagcaatata ttttcctagt 300
agatatatat atacacatac atatatacac atatacaaat gtatattttt gcaaaattgt 360
tttcaatcta gaacttttct attaactacc atgtcttaaa atcaagtcta taatcctagc 420
attagtttaa tattttgaat atgtaaacac ctgtgttaat gctttgttaa tgcttttccc 480
actctcattt gttaatgctt tcccactctc gggaaggatt tgcattttga gctttatctc 540
taaatgtgac atgca 555

<210> 232
<211> 453
<212> DNA
<213> human
<400> 232
ggatgccgct ctgtgacaaa tgtggcagtg gcatagtcgg tgctgtggtg aaggcgcggg 60
ataagtaccg gcaccctgag tgcttcgtgt gtgccgactg caacctcaac ctcaagcaaa 120
agggctactt cttcatagaa ggggagctgt actgcgaaac ccacgcaaga gcccgcacaa 180
aacccccaag aggctatgac acggtcactc tgtatcccaa agcttaagtc tctgcaggcg 240
tggcacacgc acgcacccac ccacgcgcac ttacacgaga agacattcat ggctttgggc 300
agaaggattg tgcagattgt caactccaaa tctaaagtca aggctttaga cctttatcct 360
attgtttatt gaggaaaagg aatgggaggc aaatgcctgc tatgtgaaaa aaacatacac 420
ttagctatgt tttgcaactc tttttggggc tag 453

<210> 233
<211> 396
<212> DNA
<213> human
<400> 233
ttcctgaagc tcttcggctg acagcccgct cggctcgccc tctcccccgg aggccgcgcc 60
ctcccggaaa agccgggtcc tccagacccc gaggccttgc tctcagagcg ggaggcccca 120
cccactggag agccccgccc ctaaggtact atgagttctc aggggtcaag ttcagaaacg 180
gcccagccag acctaaaccc acacgcccac aaagtggatt gcacacagac aagaactccc 240
gtgcgggcct ccactctatt cccacccttg agggagcccc cttactgggg gagggtcctt 300

gcaattccag cgaatcggag gccaggccag gacgtccttg ctccctgcac cctcactgtt 360
ctgtgcactt tttctaccta cataaacaca cgcatt 396

<210> 234
<211> 501
<212> DNA
<213> human
<400> 234
gcagggctag ttattccgat ttcttgcaca attatttagc tttttgtaag ttcaacatgt 60
aaattttaaa gacataaata tagagagact tatgtgtttg aatataaatg atatatatgg 120
attagcatgt acctgtatat tattaaacat gcaatgaact gactggtaag tgacgtctaa 180
ttgtatggct agcaatgtaa tttattcaga ctgtattttt gtacagagca gtgcactcta 240
acctatgcct ctgtgtcctc tttaatgcct aaagctgtgc ctagaaattt catctgtctt 300
aaaagtaaaa tatacttcat gctgtttatg ctattagttt ctgtactgct attctatatt 360
tattattttt aaatatatga catgtttact acttaaacat gaattcatgg tatcctggtt 420
atttttttta agtcatctgg gggaaaacct gtttatcact ccagtgattt tgagtttgca 480
gtttcacaat cagttcttca t 501

<210> 235
<211> 94
<212> DNA
<213> human
<400> 235
caggcctttt ccactttgag ggaggtgctt cgaagaatgt tgcccacacc taagtgttag 60
aagcctatgt ccgttcatcc ctgagaggtc tgaa 94

<210> 236
<211> 508
<212> DNA
<213> human
<400> 236
tggcggactc catgtttgtg atccctgcac tccaagtagc acattttcag tgttcccggg 60
cccctgtgta cttctacgag ttccagcatc agcccagctg gctcaagaac atcaggccac 120
cgcacatgaa ggcagaccat ggtgatgagc ttccttttgt tttcagaagt ttctttgggg 180
gcaactacat taaattcact gaggaagagg agcagctaag caggaagatg atgaagtact 240
gggccaactt tgcgagaaat gggaacccga atggcgaggg tctgccacac tggccgctgt 300
tcgaccagga ggagcaatac ctgcagctga acctacagcc tgcggtgggc cgggctctga 360
aggcccacag gctccagttc tggaagaagg cgctgcccca aaagatccag gagctcgagg 420
agcctgaaga gagacacaca gagctgtagc tccctgtgcc ggggaggagg gggtgggttc 480
gctgacaggc gagggtcagc ctgctgtg 508

<210> 237
<211> 475
<212> DNA
<213> human
<400> 237
gagagctcgc tttgagtgac tgggttttgt gattgcctct gaagcctatg tatgccatgg 60
aggcactaac aaactctgag gtttccgaaa tcagaagcga aaaaatcagt gaataaacca 120
tcatcttgcc actaccccct cctgaagcca cagcagggtt tcaggttcca atcagaactg 180
ttggcaaggt gacatttcca tgcataaatg cgatccacag aaggtcctgg tggtatttgt 240
aactttttgc aaggcatttt tttatatata tttttgtgca catttttttt tacgtttctt 300
tagaaaacaa atgtatttca aaatatattt atagtcgaac aattcatata tttgaagtgg 360
agccatatga atgtcagtag tttatacttc tctattatct caaactactg gcaatttgta 420
aagaaatata tatgatatat aaatgtgatt gcagcttttc aatgttagcc acagt 475

<210> 238
<211> 491
<212> DNA
<213> human
<400> 238
tgtggtttct gtagcaacac ttctcatgac catctgtttt gtgtttatga tgattttttc 60
aggtctgttg gtcaatctca caaccattgc atcttggctg tcatggcttc agtacttcag 120
cattccacga tatggattta cggctttgca gcataatgaa tttttgggac aaaacttctg 180

cccaggactc aatgcaacag gaaacaatcc ttgtaactat gcaacatgta ctggcgaaga 240
atatttggta aagcagggca tcgatctctc accctggggc ttgtggaaga atcacgtggc 300
cttggcttgt atgattgtta ttttcctcac aattgcctac ctgaaattgt tatttcttaa 360
aaaatattct taaatttccc cttaattcag tatgatttat cctcacataa aaaagaagca 420
ctttgattga agtattcaat caagtttttt tgttgttttc tgttcccttg ccatcacact 480
gttgcacagc a 491

<210> 239
<211> 400
<212> DNA
<213> human
<400> 239
gaacacgcag agagtttccc tagatatact cctgcctcca ggtgctggga cacacctttg 60
caaaatgctg tgggaagcag gagctgggga gctgtgttaa gtcaaagtag aaaccctcca 120
gtgtttggtg ttgtgtagag aataggacat agggtaaaga ggccaagctg cctgtagtta 180
gtagagaaga atggatgtgg ttcttcttgt gtatttattt gtatcataaa cacttggaac 240
aacaaagacc ataagcatca tttagcagtt gtagccattt tctagttaac tcatgtaaac 300
aagtaagagt aacataacag tattaccctt tcactgttct cacaggacat gtacctaatt 360
atggtactta tttatgtagt cactgtattt ctggattttt 400

<210> 240
<211> 467
<212> DNA
<213> human

<220>
<221> misc_feature
<222> (84)..(85)
<223> n is a, c, g, or t
<220>
<221> misc_feature
<222> (136)..(137)
<223> n is a, c, g, or t

<400> 240
ctccagcccc agagctgaaa acaccaagtg cctatttgag ggtgtctgtc tggagactta 60
gagtttgtca tgtgtgtgtg tgtnnttggt taatgtgggt ttatgggttt tctttctttt 120
tttttttttt tttttnnagt ctacattagg gggaagtgag cgcctcccat gtgcagacag 180
tgtgtcttta tagatttttc taaggctttc cccaatgatg tcggtaattt ctgatgtttc 240
tgaagttccc aggactcaca cacccgttcc catctcactt gcccacccag tgtgacaacc 300
ctcggtgtgg atataccccc gtggactcat ggctcttccc cacccccact ttctataaat 360
gtaggcctag aatacgcttc tctgttgcaa aactcagcta agttcctgct tccaccttga 420
tgttgaaata tcttatgtaa gagggcaggg gatgtcgtga agatggc 467

<210> 241
<211> 352
<212> DNA
<213> human
<400> 241
ggaatgacac ttacttacga cccaactaca gctgctatac agaacggatt ttatccttca 60
ccatacagta ttgctacaaa ccgaatgatc actcaaactt ctattacacc ctatattgca 120
tctcctgtat ctgcctacca ggtgcaaagt ccttcgtgga tgcaacctca accatatatt 180
ctacagcacc ctggtgccgt gttaactccc tcaatggagc acaccatgtc actacagccc 240
gcatcaatga tcagccctct ggcccagcag atgagtcatc tgtcactagg cagcaccgga 300
acatacatgc ctgcaacgtc agctatgcaa ggagcctact tgccacagta tg 352

<210> 242
<211> 520
<212> DNA
<213> human
<400> 242
ggaacgaaac cagcgtccta ttccagcgcc tctacgggcc ccaggccctc ctcttctccc 60
tcttctggcc caccttcctg ctgaccggtg gcctcctcat tatcgccatg gtgaagagca 120

accagtacct gtccatcctg gcggcccaga agtagagcca tccatccatg ccataccact 180
tgtcagggca caggggactg gctgggcccc cagggctgct ccccacttgc agcacaatgc 240
cttctccacc tgccctccca ctcttccagt ccaatccacg ctgtcttctg ttgcaggact 300
aacctttgag aaatcctttt gtgaagtcat tgcctgctca agaatgtaca gtggctcccc 360
aatgccttgg agccataagg ccagccagtt ctagctctct attacctgtc cccactcaac 420
tgactcatac ctgtttccgg ctgcatcact atgtgcccca cagagaacga tgatcgtcac 480
ctctgtgcct gagttctccc tgttgtctca aagcggtacc 520

<210> 243
<211> 538
<212> DNA
<213> human
<400> 243
ccggtcagca ggatcatcgt gcacccacag ttctacatca tccagactgg agcggatatc 60
gccctgctgg agctggagga gcccgtgaac atctccagcc gcgtccacac ggtcatgctg 120
ccccctgcct cggagacctt cccccggggg atgccgtgct gggtcactgg ctggggcgat 180
gtggacaatg atgagcccct cccaccgcca tttcccctga agcaggtgaa ggtccccata 240
atggaaaacc acatttgtga cgcaaaatac caccttggcg cctacacggg agacgacgtc 300
cgcatcatcc gtgacgacat gctgtgtgcc gggaacaccc ggagggactc atgccagggc 360
gactctggag ggcccctggt gtgcaaggtg aatggcacct ggctacaggc gggcgtggtc 420
agctgggacg agggctgtgc ccagcccaac cggcctggca tctacacccg tgtcacctac 480
tacttggact ggatccacca ctatgtcccc aaaaagccgt gagtcaggcc tggggtgt 538

<210> 244
<211> 489
<212> DNA
<213> human
<400> 244
ggccaaatca ccgacctgaa ggcggaaatt cacgggggca gtctcattaa tctgacttgg 60
acagctcctg gggatgatta tgaccatgga acagctcaca agtatatcat tcgaataagt 120
acaagtattc ttgatctcag agacaagttc aatgaatctc ttcaagtgaa tactactgct 180
ctcatcccaa aggaagccaa ctctgaggaa gtctttttgt ttaaaccaga aaacattact 240
tttgaaaatg gcacagatct tttcattgct attcaggctg ttgataaggt cgatctgaaa 300
tcagaaatat ccaacattgc acgagtatct ttgtttattc ctccacagac tccgccagag 360
acacctagtc ctgatgaaac gtctgctcct tgtcctaata ttcatatcaa cagcaccatt 420
cctggcattc acattttaaa aattatgtgg aagtggatag gagaactgca gctgtcaata 480
gcctagggc 489

<210> 245
<211> 461
<212> DNA
<213> human
<400> 245
accccttcag cgactagaga gctacaggag aatcaccagt ggcaaatgtc cccagaaagc 60
tgtgatcttc aagaccaaac tggccaagga tatctgtgcc gaccccaaga agaagtgggt 120
gcaggattcc atgaagtatc tggaccaaaa atctccaact ccaaagccat aaataatcac 180
catttttgaa accaaaccag agcctgagtg ttgcctaatt tgttttccct tcttacaatg 240
cattctgagg taacctcatt atcagtccaa agggcatggg ttttattata tatatatata 300
tttttttttt aaaaaaaaac gtattgcatt taatttattg aggctttaaa acttatcctc 360
catgaatatc agttattttt aaactgtaaa gctttgtgca gattctttac cccctgggag 420
ccccaattcg atcccctgtc acgtgtgggc aatgttcccc c 461

<210> 246
<211> 534
<212> DNA
<213> human
<400> 246
tgtgaagctt tacgcgcaca cggacaaaat gcccaaactg gagcccttgc aaaaacacgg 60
cttgtggcat tggcatactt gcccttacag gtggagtatc ttcgtcacac atctaaatga 120
gaaatcagtg acaacaagtc tttgaaatgg tgctatggat ttaccattcc ttattatcac 180
taatcatcta aacaactcac tggaaatcca attaacaatt ttacaacata agatagaatg 240
gagacctgaa taattctgtg taatataaat ggtttataac tgcttttgta cctagctagg 300
ctgctattat tactataatg agtaaatcat aaagccttca tcactcccac atttttctta 360

cggtcggagc atcagaacaa gcgtctagac tccttgggac cgtgagttcc tagagcttgg 420
ctgggtctag gctgttctgt gcctccaagg actgtctggc aatgacttgt attggccacc 480
aactgtagat gtatatatgg tgcccttctg atgctaagac tccagacctt ttgt 534

<210> 247
<211> 560
<212> DNA
<213> human
<400> 247
tctactgcgt gacttgccat gagaccaagt ttgccaagca ttgcgtgaag tgcaacaagg 60
ccatcacatc tggaggaatc acttaccagg atcagccctg gcatgccgat tgctttgtgt 120
gtgttacctg ctctaagaag ctggctgggc agcgtttcac cgctgtggag gaccagtatt 180
actgcgtgga ttgctacaag aactttgtgg ccaagaagtg tgctggatgc aagaacccca 240
tcactgggaa aaggactgtg tcaagagtga gccgcccagt ctctaaagct aggaagcccc 300
cagtgtgcca cgggaaacgc ttgcctctca ccctgtttcc cagcgccaac ctccggggca 360
ggcatccggg tggagagagg acttgtccct cgtgggtggt ggttctttat agaaaaaatc 420
gaagcttagc agctcctcgt ggcccgggtt tggtaaaggc tccagtgtgg tggcctatga 480
aggacaatcc tggcacgact actgcttcca ctgcaaaaaa tgctccgtga atctggccaa 540
caagcgcttt gttttccacc 560

<210> 248
<211> 445
<212> DNA
<213> human
<400> 248
gcataggaga taaaaccccc actgagatgc tctcatgcct cagctggacc caccgtgtag 60
acacacgaca tgcaagagtt gcagcggctg ctccaactca ctgctcaccc tctctgtgag 120
cagaaagaac cctactgaca tgcatggtta acttcctcat cagaactctg cccttccctt 180
ctgttctttt gtgctttcaa ataactaaca cgaacttcca gaaaattaac atttgaactt 240
agctgtaatt ctaaactgac ctttccccgt actaacgttt ggtttccccg tgtggcatgc 300
ttttctgagc cttcctactt taaagcatgg aacatgcagg tgatttggga agtgtagaaa 360
gacctgagaa aacgagcctg tttcagagga acatcgtcac aacgaatact tctggaagct 420
taacaaaact aaccctgctg tcctt 445

<210> 249
<211> 185
<212> DNA
<213> human
<400> 249
atttcgtttt gcttttggtt gcctgaatgt tgtcaccaag tgaaaaaatt atttaactat 60
atgtaaaatt tctcttttaa aaaaaagttt tactgatgtt aaacgttctc agtgccaatg 120
tcagactgtg ctcctccctc tcctgaacct ctaccctcac cctgagctgt cttgttgaaa 180
acagt 185

<210> 250
<211> 551
<212> DNA
<213> human
<400> 250
agccctttgc tctggaggaa gttctccagc ttcagctcaa ctcacagctt ctccaagcat 60
caccctggga gtttcctgag ggttttctca taaatgaggg ctgcacattg cctgttctgc 120
ttcgaagtat tcaataccgc tcagtatttt aaatgaagtg attctaagat ttggtttggg 180
atcaatagga aagcatatgc agccaaccaa gatgcaaatg ttttgaaatg atatgaccaa 240
aattttaagt aggaaagtca cccaaacact tctgctttca cttaagtgtc tggcccgcaa 300
tactgtagga acaagcatga tcttgttact gtgatatttt aaatatccac agtactcact 360
ttttccaaat gatcctagta attgcctaga aatatctttc tcttacctgt tatttatcaa 420
tttttcccag tatttttata cggaaaaaat tgtattgaaa acacttagta tgcagttgat 480
aagaggaatt tggtataatt atggtgggtg attatttttt atactgtatg tgccaaagct 540
ttactactgt g 551

<210> 251
<211> 518
<212> DNA

<213>  human
<400>  251
gtgccatagt gcaggcttgg ggagctttaa gcctcagtta tataacccac gaaaaacaga      60
gcctcctaga tgtaacattc ctgatcaagg tacaattctt taaaattcac taatgattga     120
ggtccatatt tagtggtact ctgaaattgg tcactttcct attacacgga gtgtgctaaa     180
actaaaaagc attttgaaac atacagaatg ttctattgtc attgggaaat ttttctttct     240
aacccagtgg aggttagaaa gaagttatat tctggtagca aattaacttt acatcctttt     300
tcctacttgt tatggttgtt tggaccgata agtgtgctta atcctgaggc aaagtagtga     360
atatgtttta tatgttatga agaaaagaat tgttgtaagt ttttgattct actcttatat     420
gctggactgc attcacacat ggcatgaaat aagtcaggtt ctttacaaat ggtattttga     480
tagatactgg attgtgtttg tgccatattt gtgccatt                             518

<210>  252
<211>  419
<212>  DNA
<213>  human
<400>  252
gtcaggactg ttatggccct gttgtgctga gttagttgga acagaattca agctccctgc      60
actagtccac ctgccccact gcttcttcgc ttctctcttg gaaagtccag tctctcctcg     120
gcttgcaatg gaccccaact gctcctgcgc cgctggtgtc tcctgcacct gcgctggttc     180
ctgcaagtgc aaagagtgca aatgcacctc ctgcaagaag agctgctgct cctgctgccc     240
cgtgggctgt agcaagtgtg cccagggctg tgtttgcaaa ggggcgtcag agaagtgcag     300
ctgctgcgac tgatgccagg acaacctttc tcccagatgt aaacagagag acatgtacaa     360
acctggattt tttttttata ccaccttgac ccatttgcta cattcctttt cctgtgaaa      419

<210>  253
<211>  506
<212>  DNA
<213>  human
<400>  253
caggaaggac cttgctttgg accctacaca cttcggctct ctggacactt gcgacacctc      60
aaggtgttct ctgtagctca atctgcaaac atgccaggcc tcagggatcc tctgctgggt     120
gcctccttgc cttgggacca tggccacccc agagccatcc gatcgatgga tgggatgcac     180
tctcagacca agcagcagga attcaaagct gcttgctgta actgtgtgag attgtgaagt     240
ggtctgaatt ctggaatcac aaaccaagcc atgctggtgg gccattaatg gttggaaaac     300
actttcatcc ggggctttgc cagagcgtgc tttcaagtgt cctggaaagt ctgctgcttc     360
tccaagcttt cagacaagaa tgtgcactct ctgcttaggt tttgcttggg aaactcaact     420
tctttcctct ggagacgggg catctccctc tgatttcctt ctgctatgac aaaaccttta     480
atctgcacct tacaactcgg ggacaa                                          506

<210>  254
<211>  565
<212>  DNA
<213>  human
<400>  254
ttaccaatga caaccctgag tgccgccttg tgaaagaaac ccggatttgt gaggtgcggc      60
cttgtggaca gccagtgtac agcagcctga aaaagggcaa gaaatgcagc aagaccaaga     120
aatcccccga accagtcagg tttacttacg ctggatgttt gagtgtgaag aaataccggc     180
ccaagtactg cggttcctgc gtggacggcc gatgctgcac gccccagctg accaggacat     240
gtgaagatgc ggttccgctg cgaagatggg gagacatttt ccaagaacgt catgatgatc     300
cagtcctcaa atgcaactac aactgcccgc atgccaatga agcagcgttt cccttctaca     360
ggctgttcaa tgacattcac aaatttaggg actaaatgct acctgggttt ccagggcaca     420
cctagacaaa caagggagaa gagtgtcaga atcagaatca tggagaaaat gggcgggggt     480
ggtgtgggtg atgggactca ttgtagaaag gaagccttgc tcattcttga ggagcattaa     540
ggtatttcga aactgccaag ggtgc                                           565

<210>  255
<211>  413
<212>  DNA
<213>  human
<400>  255
aaattgtgga gttagcctcc tgtggagtta gcctcctgtg gtaaaggaat tgaagaaaat      60
ataacacctt acaccctttt tcatcttgac attaaaagtt ctggctaact ttggaatcca     120

ttagagaaaa atccttgtca ccagattcat tacaattcaa atcgaagagt tgtgaactgt 180
tatcccattg aaaagaccga gccttgtatg tatgttatgg atacataaaa tgcacgcaag 240
ccattatctc tccatgggaa gctaagttat aaaaataggt gcttggtgta caaaactttt 300
tatatcaaaa ggctttgcac atttctatat gagtgggttt actggtaaat tatgttattt 360
tttacaacta atttgtactt ctcagaatgt ttgtcatatg cttcttgcaa tgc 413

<210> 256
<211> 253
<212> DNA
<213> human
<400> 256
aaggcaagag gatgcatctt tcttcctggt gtacaagcct ttaaagactt ctgctgctga 60
tatgcctctt ggatgcacac tttgtgtgta catagttacc tttaactcag tggttatcta 120
atagctctaa actcattaaa aaaactccaa gccttccacc aaaacagtgc cccacctgta 180
tacattttta ttaaaaaaat gtaatgctta tgtataaaca tgtatgtaat atgctttcta 240
tgaatgatgt ttg 253

<210> 257
<211> 445
<212> DNA
<213> human
<400> 257
gaaggagacg gtctggcggc ttgaagaatt tggacgattt gccagctttg aggctcaagg 60
tgcattggcc aacatagctg tggacaaagc caacttggaa atcatgacaa agcgctccaa 120
ctatactccg atcaccaatg acaagttcac cccaccagtg gtcaatgtca cgtggcttcg 180
aaatggaaaa cctgtcacca caggagtgtc agagacagtc ttcctgccca gggaagacca 240
ccttttccgc aagttccact atctcccctt cctgccctca actgaggacg tttacgactg 300
cagggtggag cactggggct tggatgagcc tcttctcaag cactgggagt ttgatgctcc 360
aagccctctc ccagagacta cagagaacgt ggtgtgtgcc ctgggcctga ctgtgggtct 420
ggtgggcatc attattggga ccatc 445

<210> 258
<211> 370
<212> DNA
<213> human
<400> 258
aatattggat atcaactgct tgtaaaggtg ctcctccttt ttcttgtcat tgctggtcaa 60
gattactaat atttgggaag gctttaaaga cgcatgttat ggtgctaatg tactttcact 120
tttaaactct agatcagaat tgttgacttg cattcagaac ataaatgcac aaaatctgta 180
catgtctccc atcagaaaga ttcattggca tgccacaggg gattctcctc cttcatcctg 240
taaaggtcaa caataaaaac caaattatgg ggctgctttt gtcacactag catagagaat 300
gtgttgaaat ttaactttgt aagcttgtat gtggttgttg atcttttttt tccttacaga 360
cacccataat 370

<210> 259
<211> 449
<212> DNA
<213> human
<400> 259
atctcatacc cgcaaatttt aaccttgtca acctctgggg tattagtatg ccctgacctg 60
agtgaattca cccgtgacaa aactgacgtg aagattcaat ggtacaagga ttctcttctt 120
ttggataaag acaatgagaa atttctaagt gtgaggggga ccactcactt actcgtacac 180
gatgtggccc tggaagatgc tggctattac cgctgtgtcc tgacatttgc ccatgaaggc 240
cagcaataca acatcactag gagtattgag ctacgcatca agaaaaaaaa agaagagacc 300
attcctgtga tcatttcccc cctcaagacc atatcagctt ctctggggtc aagactgaca 360
atcccgtgta aggtgtttct gggaaccggc acacccttaa ccaccatgct gtggtggacg 420
gccaatgaca cccacataga gagcgccta 449

<210> 260
<211> 435
<212> DNA
<213> human
<400> 260

agctcgaaag agtttgcaac cccatcatca gcaaacttta ccaaggtggt cctggcggcg 60
gcagcggcgg cggcggttca ggagcctccg ggggacccac catcgaagaa gtggactaag 120
cttgcactca agtcagcgta aacctctttg cctttctctc tctctctttt tttttgtttg 180
tttctttgaa atgtccttgt gccaagtacg agatctattg ttggaagtct ttggtatatg 240
caaatgaaag gagaggtgca acaacttagt ttaattataa aagttccaaa gtttgttttt 300
taaaaacatt attcgaggtt tctctttaat gcattttgcg tgtttgctga cttgagcatt 360
tttgattagt tcgtgcatgg agatttgttt gagatgagaa accttaagtt tgcacacctg 420
ttctgtagaa gcttg 435

<210> 261
<211> 476
<212> DNA
<213> human
<400> 261
gctttttttg ttgtcagcta tcttaagaat cattaaatac acctgctttg ggtaaaactc 60
tttgcaagca gtaattaaca ctagtaacag tgaaagcaca agatttccaa atcagtcgtt 120
ttctcaaaaa aatatcgtat aagtgactca tcctgtctgc taactccaga cctcccagct 180
tgaagccaaa tctttccatg tgagattgat atggatttcc tagaagtact ggaatgttgt 240
catatcttgc cctattttaa ttctgctata gaaaacaatt gccttcactt ttaaggagta 300
atttgaatat taataactct ggtctagatt ttcatataat gtattaaaga caaagtagtg 360
aacatcaatg aacatctgat agagataaac tgtaatcagg cataagcttg tttgtatgtt 420
ctggcagtga ctaatcagta aatgatgtcg gtttgcccag tatcacttat cttctg 476

<210> 262
<211> 541
<212> DNA
<213> human
<400> 262
cgtgaacggc aaagtggcgc tggtgaccgg cgcggctcag ggcataggca gagcctttgc 60
agaggcgctg ctgcttaagg gcgccaaggt agcgctggtg gattggaatc ttgaagcagg 120
tgtacagtgt aaagctgccc tggatgagca atttgaacct cagaagactc tgttcatcca 180
gtgcgatgtg gctgaccagc aacaactgag agacactttt agaaaagttg tagaccactt 240
tggaagactg gacattttgg tcaataatgc tggagtgaat aatgagaaaa actgggaaaa 300
aactctgcaa attaatttgg tttctgttat cagtggaacc tatcttggtt tggattacat 360
gagtaagcaa aatggaggtg aaggcggcat cattatcaat atgtcatctt tagcagccca 420
ccattgattg ccaatggatt gataacactc attgaagatg atgctttaaa tggtgctatt 480
atgaagatca caacttctaa gggaattcat tttcaagact atgatacaac tccatttcaa 540
g 541

<210> 263
<211> 421
<212> DNA
<213> human
<400> 263
gagtattaca ttggccttgg gggacagaaa ggaggaagtt ctgacttttc agggctacct 60
tatttctact aaggacccag agcaggcctg tccatgccat tccttcgcac agatgaaact 120
gagctgggac tggaaaggac agcccttgac ctgggttctg ggtataattt gcacttttga 180
gactggtagc taaccatctt atgagtgcca atgtgtcatt tagtaaaact taaatagaaa 240
caaggtcctt caaatgttcc tttggccaaa agctgaaggg agttactgag aaaatagtta 300
acaattactg tcaggtgtca tcactgttca aaaggtaagc acatttagaa ttttgttctt 360
gacagttaac tgactaatct tacttccaca aaatatgtga atttgctgct tctgagaggc 420
a 421

<210> 264
<211> 281
<212> DNA
<213> human
<400> 264
ggactggtga agccttcgga gaccctgtcc ctcacctgca gtgtctctgg tggctccatc 60
agtagttact actggagctg gatccggcag cccccaggga agggactgga gtggattggg 120
tatatcaatt acagtgggag caccaactat aacccctccc tcaagagtcg agtcaccata 180
tcagtagaca cgtccaagaa ccagttctcc ctgaagctga gctctgtgac cgctgcggac 240
acggccgtgt attactgtgc gagaacgatt tcggggggtc g 281

<210> 265
<211> 293
<212> DNA
<213> human
<400> 265
aatagtgatg acgcagcttc cagccaccct gtctgtgtct ccaggggaaa gagccaccct 60
ctcctgcagg gccagtcaga gtgttcgcag caacttagcc tggtaccagc aaaaacctgg 120
ccaggctccc aggctcctca tctacggtgc atccaccagg gccaatggta tcccagccag 180
gttcagtggc agtgggtctg ggacagagtt cactctcacc atcagcggcc tgcagtctga 240
cgattttgca gtttattact gtcaacacta taataactgg cctccgtgga cgt 293

<210> 266
<211> 284
<212> DNA
<213> human
<400> 266
aattgtgttg acgcagtctc caggcaccct gtctttgtct ccaggggaaa gagccaccct 60
ctcctgcagg gccagtcaga gtattagcag cagtttctta gcctggtacc agcagaaagt 120
tggccaggct cccaggctcc tcatctatgg tgcatccagc agggccactg gcatcccaga 180
caggttcagt ggcagtgggt ctgggacaga cttcactctc accatcagca gactggagcc 240
tgaagatttt gcagtgtatt actgtcagca atatggtagc tcgc 284

<210> 267
<211> 263
<212> DNA
<213> human
<400> 267
tcctccctgt ctgcatctgt aggagacaga gtcaccatca cttgccgggc aagtcagggc 60
attagaaatg atttagcctg gtatcagcag aaaccaggga aagcccctaa gcgcctgatc 120
tatgctgcat ccagtttgca aagtggggtc ccatcaaggt tcagcggcag tggatctggg 180
acagaattca ctctcacaat cagcagcctg cagcctgaag attttgcaac ttattactgt 240
ctacagcata atagttaccc ttg 263

<210> 268
<211> 458
<212> DNA
<213> human
<400> 268
gcagttgagt cgtcatcact tttcagtgat gggagagtag atggtgaaat ttattagtta 60
atatatccca gaaattagaa accttaatat gtggacgtaa tctccacagt caaagaagga 120
tggcacctaa accaccagtg cccaaagtct gtgtgatgaa ctttctcttc atactttttt 180
tcacagttgg ctggatgaaa ttttctagac tttctgttgg tgtatccccc ccctgtatag 240
ttaggatagc atttttgatt tatgcatgga aacctgaaaa aaagtttaca agtgtatatc 300
agaaaaggga agttgtgcct tttatagcta ttactgtctg gttttaacaa tttcctttat 360
atttagtgaa ctacgcttgc tcattttttc ttacataatt ttttattcaa gttattgtac 420
agctgtttaa gatgggcagc tagttcgtag ctttccca 458

<210> 269
<211> 494
<212> DNA
<213> human
<400> 269
tgaggagaag tctgccgtta ctgccctgtg gggcaaggtg aacgtggatg aagttggtgg 60
tgaggccctg ggcaggctgc tggtggtcta cccttggacc cagaggttct ttgagtcctt 120
tggggatctg tccactcctg atgctgttat gggcaaccct aaggtgaagg ctcatggcaa 180
gaaagtgctc ggtgccttta gtgatggcct ggctcacctg gacaacctca agggcacctt 240
tgccacactg agtgagctgc actgtgacaa gctgcacgtg gatcctgaga acttcaggct 300
cctgggcaac gtgctggtct gtgtgccggc ccatcacttt ggcaaagaat tcacccaacc 360
agtgcaggct gcctatcaga aagtggtggc tggtgtggct aatgccctgg cccacaagta 420
tcactaagct cgctttcttg ctgtccaatt tctattaaag gttcctttgt tccctaagtc 480
caactactaa actg 494

<210>  270
<211>  445
<212>  DNA
<213>  human
<400>  270
agagaaccca ccatggtgct gtctcctgac gacaagacca acgtcaaggc cgcctggggt      60
aaggtcggcg cgcacgctgg cgagtatggt gcggaggccc tggagaggat gttcctgtcc     120
ttccccacca ccaagaccta cttcccgcac ttcgacctga gccacggctc tgcccaggtt     180
aagggccacg gcaagaaggt ggccgacgcg ctgaccaacg ccgtggcgca cgtggacgac     240
atgcccaacg cgctgtccgc cctgagcgac ctgcacgcgc acaagcttcg ggtggacccg     300
gtcaacttca agctcctaag ccactgcctg ctggtgaccc tggccgccca cctccccgcc     360
gagttcaccc ctgcggtgca cgcctccctg gacaagttcc tggcttctgt gagcaccgtg     420
ctgacctcca aataccgtta agctg                                           445

<210>  271
<211>  485
<212>  DNA
<213>  human
<400>  271
gggagtttcc tgagggtttt ctcataaatg agggctgcac attgcctgtt ctgcttcgaa      60
gtattcaata ccgctcagta ttttaaatga agtgattcta agatttggtt tgggatcaat     120
aggaaagcat atgcagccaa ccaagatgca aatgttttga aatgatatga ccaaaatttt     180
aagtaggaaa gtcacccaaa cacttctgct ttcacttaag tgtctggccc gcaatactgt     240
aggaacaagc atgatcttgt tactgtgata ttttaaatat ccacagtact cactttttcc     300
aaatgatcct agtaattgcc tagaaatatc tttctcttac ctgttattta tcaatttttc     360
ccagtatttt tatacggaaa aaattgtatt gaaaacactt agtatgcagt tgataagagg     420
aatttggtat aattatggtg ggtgattatt ttttatactg tatgtgccaa agctttacta     480
ctgtg                                                                 485

<210>  272
<211>  435
<212>  DNA
<213>  human
<400>  272
tcctgccgac aagaccaacg tcaaggccgc ctggggtaag gtcggcgcgc acgctggcga      60
gtatggtgcg gaggccctgg agaggatgtt cctgtccttc cccaccacca agacctactt     120
cccgcacttc gacctgagcc acggctctgc ccaggttaag ggccacggca agaaggtggc     180
cgacgcgctg accaacgccg tggcgcacgt ggacgacatg cccaacgcgc tgtccgccct     240
gagcgacctg cacgcgcaca agcttcgggt ggacccggtc aacttcaagc tcctaagcca     300
ctgcctgctg gtgaccctgg ccgcccacct ccccgccgag ttcacccctg cggtgcacgc     360
ctccctggac aagttcctgg cttctgtgag caccgtgctg acctccaaat accgttaagc     420
tggagcctcg gtggc                                                      435

<210>  273
<211>  583
<212>  DNA
<213>  human
<400>  273
tacgcagact ccgtgaaggg ccgattcacc atctccagag acaattccaa gaacacgctg      60
tatcttcaaa tgaacagcct gagagccgag gacacggccg tattactgtg caagagagac     120
ttgtgtgatt ggggccaagg taccctggtc accgtgtcga gaggtggcgg tggctcgggc     180
ggtggtgggt cgggtggcgg cggatcttct gagctgactc aggaccctgc tgtgtctgtg     240
gccttgggac agacagtcag gatcacatgc caaggagaca gcctcagaag ctattatgca     300
agctggtacc agcagaagcc aggacaggcc cctgtacttg tcatctatgg taaaaacaac     360
cggccctcag ggatcccaga ccgattctct ggctccagct caggaaacac agcttccttg     420
accatcactg gggctcaggc ggaagatgag gctgactatt actgtaactc ccgggacagc     480
agtggtaacc atgtggtatt cggcggaggg accaagctga ccgtcctagg tgcggccgca     540
gaacaaaaac tcatctcaga agaggatctg aatggggccg cat                       583

<210>  274
<211>  484
<212>  DNA
<213>  human

<400>  274
ttgcacaagt ttcctgggct ggaccgtttc aacagagagg cttatttgac tttatgctag      60
aagatgaggc ttctgggata ggcccagaag ttcctgatga ccgcgacttc gagccctccc     120
taggcccagt gtgccccttc cgctgtcaat gccatcttcg agtggtccag tgttctgatt     180
tgggtctgga caaagtgcca aaggatcttc cccctgacac aactctgcta gacctgcaaa     240
acaacaaaat aaccgaaatc aaagatggag actttaagaa cctgaagaac cttcacgttg     300
tctaccttca taacaacaat atctctgtag ttggatcaag tgacttctgc ccacctggac     360
acaacaccaa aaaggcttct tattcgggtg tgagtctttt cagcaacccg gtccagtact     420
gggagataca gccatccacc ttcagatgtg tctacgtgcg ctctgccatt caactcggaa     480
acta                                                                  484

<210>  275
<211>  502
<212>  DNA
<213>  human
<400>  275
tgtccataca gagtgtgcat tccctggcag gggctcctgc tcacagcctc gcttttaacc      60
ttctggaacc tgccaaacag tgcccagacc aatattgatg gtgtgccgtt caatgtcgca     120
gaagggaagg aggtccttct agtagtccat aatgagtccc agaatcttta tggctacaac     180
tggtacaaag ggcaaagggt gcatgccaac tatcgaatta taggatatgt aaaaaatata     240
agtcaagaaa atgccccagg gcccgcacac aacggtcgag agacaatata ccccaatgga     300
accctgctga tccagaacgt cacccacaat gacgcaggaa tctataccct acacgttata     360
aaagaaaatc ttgtgaatga agaagtaacc agacaattct acgtattcta tgagtcagta     420
caagcaagtt cacctgacct ctcagctggg accgctgtca gcatcatgat tggagtactg     480
gctgggatgg ctctgatata gc                                              502

<210>  276
<211>  510
<212>  DNA
<213>  human
<400>  276
tgcacagtac tcctggctta tcaatggaac attccagcaa agcacacaag agctctttat      60
ccctaacatc actgtgaata atagtggatc ctatacctgc cacgccaata actcagtcac     120
tggctgcaac aggaccacag tcaagacgat catagtcact gagagacaga atctcaccat     180
gttgcccggg ctggactcga actcctgggc tcaagcaatc ctcccatctg tttcccaaag     240
tgctgagatt acagataatg ctctaccaca agaaaatggc ctctcacctg gggccattgc     300
tggcattgtg attggagtag tggccctggt tgctctgata gcagtagccc tggcatgttt     360
tctgcatttc gggaagaccg gcagggcaag cgaccagcgt gatctcacag agcacaaacc     420
ctcagtgtcc aaccacactc aggaccactc caatgaccca cctaacaaga tgaatgaagt     480
tacttattct accctgaact ttgaagccca                                      510

<210>  277
<211>  244
<212>  DNA
<213>  human
<400>  277
tcctccttcc cttacggaat tacatcttga tggcaacaaa atcagcagag ttgatgcagc      60
tagcctgaaa ggactgaata atttggctaa gttgggattg agtttcaaca gcatctctgc     120
tgttgacaat ggctctctgg ccaacacgcc tcatctgagg gagcttcact tggacaacaa     180
caagcttacc agagtacctg gtgggctggc agagcataag tacatccagg ttgtctacct     240
tcat                                                                  244

<210>  278
<211>  198
<212>  DNA
<213>  human
<400>  278
tcacaccgta gcccacatgg atagcacagt tgtcagacaa gattccttca gattccgagt      60
tgcctaccgg ttgttttcgt tgttgttgtt gttgtttttc tttttctttt tttttttgaa     120
gacagcaata accacagtac atattactgt agttctctat agttttacat acattcatac     180
cataactctg ttctctcc                                                   198

<210>  279

<211>  430
<212>  DNA
<213>  human

<220>
<221>  misc_feature
<222>  (42)..(42)
<223>  n is a, c, g, or t
<220>
<221>  misc_feature
<222>  (93)..(94)
<223>  n is a, c, g, or t
<220>
<221>  misc_feature
<222>  (107)..(107)
<223>  n is a, c, g, or t
<220>
<221>  misc_feature
<222>  (121)..(121)
<223>  n is a, c, g, or t
<220>
<221>  misc_feature
<222>  (125)..(125)
<223>  n is a, c, g, or t
<220>
<221>  misc_feature
<222>  (144)..(144)
<223>  n is a, c, g, or t
<220>
<221>  misc_feature
<222>  (155)..(155)
<223>  n is a, c, g, or t
<220>
<221>  misc_feature
<222>  (163)..(163)
<223>  n is a, c, g, or t
<220>
<221>  misc_feature
<222>  (167)..(167)
<223>  n is a, c, g, or t
<220>
<221>  misc_feature
<222>  (174)..(174)
<223>  n is a, c, g, or t
<220>
<221>  misc_feature
<222>  (184)..(184)
<223>  n is a, c, g, or t
<220>
<221>  misc_feature
<222>  (188)..(188)
<223>  n is a, c, g, or t
<220>
<221>  misc_feature
<222>  (193)..(193)
<223>  n is a, c, g, or t
<220>
<221>  misc_feature
<222>  (199)..(199)
<223>  n is a, c, g, or t
<220>
<221>  misc_feature
<222>  (254)..(254)

<223>  n is a, c, g, or t
<220>
<221>  misc_feature
<222>  (270)..(270)
<223>  n is a, c, g, or t
<220>
<221>  misc_feature
<222>  (361)..(361)
<223>  n is a, c, g, or t
<220>
<221>  misc_feature
<222>  (364)..(364)
<223>  n is a, c, g, or t
<220>
<221>  misc_feature
<222>  (377)..(377)
<223>  n is a, c, g, or t

<400>  279
cactttccaa tgccacagac aactcacatt gttcaactcc cnttctcggg gtgggacaga      60
cgagacaaca gcacacaggc agccagccgt ggnnccagag gctcganggg gctcaggggc     120
ntcanggcac ccgtccccac acgnagggcc ccgtngggtg ggncctnggc cctngctttc     180
tacngccnaa tgnttatgnc cagctccatg ttctcccaaa taccgttgat gtgaattatt     240
ttaaaggcaa aacngtgctc tttattttan aaaacactga taatcacact gcggtaggtc     300
attcttttgc cacatcccta tagaccactg ggtttggcaa aactcaggca gaagtggaga     360
nccnttctag acatcantgt cagccttgct acttgaaggt acaccccata gggtcggagg     420
tgctgtcccc                                                            430

<210>  280
<211>  135
<212>  DNA
<213>  human
<400>  280
gaggcaaatg gatctcgata tttcagatgg gcttttgatg cactgttgcc aaggaaggct      60
ttttctgatt ttttgacaaa tgaatttttg cacactttca ttggtgtctt tcggcaactt     120
acacacattg aaaat                                                      135

<210>  281
<211>  260
<212>  DNA
<213>  human

<220>
<221>  misc_feature
<222>  (45)..(45)
<223>  n is a, c, g, or t
<220>
<221>  misc_feature
<222>  (48)..(48)
<223>  n is a, c, g, or t
<400>  281
gaggacttag gagagatctg aactccagct gccctacaaa ctccntcnca gcttttcttc      60
tcacttcatg tgaaaactac tccagtggct gactgaattg ctgacccttc aagctctgtc     120
cttatccatt acctcaaagc agtcattcct tagtaaagtt tccaacaaat agaaattaat     180
gacactttgg tagcactaat atggagatta tcctttcatt gagcctttta tcctctgttc     240
tcctttgaag aacccctcac                                                 260

<210>  282
<211>  131
<212>  DNA
<213>  human

<220>

<221>  misc_feature
<222>  (85)..(85)
<223>  n is a, c, g, or t

<400>  282
gcactgggag gcccaagagc caatccagat gcctgagaca acggagactg tgctctgtgc      60
cctgggcctg gtgctgggcc tagtnggcat catcgtgggc accgtcctca tcataaagtc     120
tctgcgttct g                                                          131

<210>  283
<211>  466
<212>  DNA
<213>  human
<400>  283
tgcagaagtc aaaccctcat gacaaagtag gcacaagtct acaataagct aaatcagaat      60
ttacaaatac aagtgtccca ggtagcattg actcccgtca ttggagtgaa atggatcaaa     120
gtttgaatta aggcctatgg taaggtaaca ttgctttgtt gtacttttga acaagagctc     180
ctcctgatca ctattacata tttttctaga aaatctaaag ttcagaagag aatgtatcac     240
tgctgacttt tattccaata tttggatgga gtaagtttta gggtagaatt ttgttcagtt     300
tggatttaat cttttgaaaa gtaaattcct tgtttactgg tttgactata attctctgtt     360
atctttacga ggtaaaactg caagctgact agcatgttct gtgaatctgc cattcctaaa     420
aattttataa acacttgata cttttcactg ataatggatc gctcca                    466

<210>  284
<211>  496
<212>  DNA
<213>  human

<220>
<221>  misc_feature
<222>  (304)..(304)
<223>  n is a, c, g, or t
<220>
<221>  misc_feature
<222>  (307)..(307)
<223>  n is a, c, g, or t

<400>  284
tcacagcggg caggacggac cccgcccacg acgtgcgggt ggcggtggtg cagtacagcg      60
gcacgggcca gcagcgccca gagcgggcgt cgctgcagtt cctgcagaac tacacggccc     120
tggccagtgc cgtcgatgcc atggacttta tcaacgacgc caccgacgtc aacgatgccc     180
tgggctatgt gacccgcttc taccgcgagg cctcgtccgg cgctgccaag aagaggctgc     240
tgctcttctc agatggcaac tcgcagggcg ccacgcccgc tgccatcgag aaggccgtgc     300
aggnagncca gcgggcaggc atcgagatct tcgtggtggt cgtgggccgc caggtgaatg     360
agccccacat ccgcgtcctg gtcaccggca agacggccga gtacgacgtg gcctacggcg     420
agagccacct gttccgtgtc cccagctacc aggccctgct ccgcggtgtc ttccaccaga     480
cagtctccag gaaggt                                                     496

<210>  285
<211>  541
<212>  DNA
<213>  human

<220>
<221>  misc_feature
<222>  (54)..(54)
<223>  n is a, c, g, or t
<220>
<221>  misc_feature
<222>  (91)..(91)
<223>  n is a, c, g, or t
<220>
<221>  misc_feature

<222>  (99)..(99)
<223>  n is a, c, g, or t
<220>
<221>  misc_feature
<222>  (106)..(106)
<223>  n is a, c, g, or t
<220>
<221>  misc_feature
<222>  (110)..(110)
<223>  n is a, c, g, or t
<220>
<221>  misc_feature
<222>  (136)..(136)
<223>  n is a, c, g, or t
<220>
<221>  misc_feature
<222>  (151)..(151)
<223>  n is a, c, g, or t

<400>  285
gaatttcacc tgtaaacctg agtcgtacag aaagctgcct ggtatatcca aaangctttt      60
tattcctcct gctcatattg tgattctgcc ntttggggna cttttncttn aaaccttcag     120
ttatgatttt tttttncata cacttattgg naactctgct tgatttttgc ctcttccagt     180
cttcctgaca ctttaattac caacctgtta cctactttga ctttttgcat ttaaaacaga     240
cactggcatg gatatagttt tacttttaaa ctgtgtacat aactgaaaat gtgctatact     300
gcatactttt taaatgtaaa gatattttta tctttatatg aagaaaatca cttaggaaat     360
ggctttgtga ttcaatctgt aaactgtgta ttccaagaca tgtctgttct acatagatgc     420
ttagtccctc atgcaaatca attactggtc caaaagattg ctgaaatttt atatgcttac     480
tgatatattt tacaattttt tatcatgcat gtcctgtaaa ggttacaagc ctgcacaata     540
a                                                                     541

<210>  286
<211>  557
<212>  DNA
<213>  human
<400>  286
tcttcccctt gagcttcaga gaggagagtt ggcatggtta aatctgaatg gttacctcac      60
tgctgaaaac ccagaggggc gtggcacact cgcttgtgtg gaaaagcctc taaatgcatc     120
ccttcctttc tttcctgctt cctttgcctt acaattgaag cagcccgtgg taccatcaca     180
gtatgcagag acttcctcac ctttcatatc tagggaccac ccccgatgca ttggtgaggg     240
tgggcactta taaatgcctg ctattgttaa gccattccag cctcttcctc tgaatagacc     300
agacgccctt tcacttagtt cagtgccagt ccttttgcct tcccaaccct gctgttaggc     360
ctgctgttcc ctttgctctt gattaggaga gatggaagga gatgagctcc cataactgaa     420
ttggcctttg gttcatgttt tctccccata tgtatatatg ccatatgtga atatgccata     480
tatatgtgcc aacaaatcta tctacgttgt tcttttcaaa ttagcacgca gataggaatt     540
ttgagtttct tcttctt                                                    557

<210>  287
<211>  418
<212>  DNA
<213>  human
<400>  287
tatgtaaact ttaacttcca ctttgtataa atttttaagt gtcagactat ccattttaca      60
cttgctttat ttttcattac ctgtagcttt gggcagattt gcaacagcaa attaatgtgt     120
aaaattggat tattactaca aaaccgttta gtcatatcta tctaatcaga tcttcttttg     180
ggaggatttg atgtaagtta ctgacaagcc tcagcaaacc caaagatgtt aacagtattt     240
taagaagttg ctgcagattc ctttggccac tgtatttgtt aatttcttgc aatttgaagg     300
tacgagtaga ggtttaaaga aaaatcagtt tttgttctta aaaatgcatt taagttgtaa     360
acgtcttttt aagcctttga agtgcctctg attctatgta acttgttgca gactggtg       418

<210>  288
<211>  199
<212>  DNA

<213>  human
<400>  288
cgccggctcc tgcaaatgca aagagtgcaa atgcacctcc tgcaagaaaa gctgctgctc      60
ctgctgccct gtgggctgtg ccaagtgtgc ccagggctgc atctgcaaag gggcgtcgga     120
caagtgcagc tgctgcgcct gatgctggga cagccccgct cccagatgta aagaacgcga     180
cttccacaaa cctggattt                                                  199

<210>  289
<211>  500
<212>  DNA
<213>  human
<400>  289
caactgtttt tgcgacttta taggcaggta aattttgcta ttactattga atacaaatga      60
caattcattt atgaccactc aaacagcgtt agtaaccatt tagtgacaaa ggattaaaac     120
atccatctgg atgttaattt tgaagatgta aattatatgt tgtttaaatt tttccaggca     180
tctgaaaacc ttatctgcta gacaatgtaa gattcacaca gagttatctg ggattctgat     240
tttttaaata gtacatatca ttaaaccatt ttctctaaat gtaagaagag cagaaaaaat     300
cttataagat tatcagattt ttctaatgac acagaaatgt aagaaaaaaa tccctttata     360
ttgaaaaaag atgcagtcaa agtcttttca gacatgccca aactttgaga atttcttcaa     420
ccatctaatg ctataaagat ttttgttctt cctgttcaca accagttgta taacagaaat     480
actagctact gttttccttc                                                 500

<210>  290
<211>  546
<212>  DNA
<213>  human
<400>  290
gatgggtcgt gtgatgagat gcatttaagg ccgatagtga tagatgtttt ttttatttct      60
tgaacacagg ctttgtctga atgatgttct tttatctctt gaacacaagc tttgaatgat     120
aactacaggt tttaagtgct gttacattaa taccataatg tgatgtgtta gaaacaaagg     180
gatatttcaa aggtagatat ttgaaaattc tctagtctca atatgtatgt gtattgaata     240
tactctaaaa ataaatgtgc aatttgctag taggacaatg cagtgactga ctagcattag     300
gtatgtttct tttatatcct agctatgtcc cactttcttc taagtgcaat cctttcatgt     360
tcacttgctg ttttacccca tctactctaa cttcatttgg aaggcttgtc tagagtatag     420
catgtatttt tacctttgca gtgaattgca tgtgctaatt gtaaccacag ctatttttat     480
gttgacataa ctccaaatgt tatattaaat gttctattat atattagctc taatccctta     540
agtaaa                                                                546

<210>  291
<211>  419
<212>  DNA
<213>  human

<220>
<221>  misc_feature
<222>  (249)..(249)
<223>  n is a, c, g, or t
<400>  291
   acagtgttct ctaatgttac agatgagatg cgcattgcca aagaggagat tttggaccca      60
   gtgcagcaaa tcatgaagtt taaatcttta gatgacgtga tcaaaagagc aaacaatact     120
   ttctatggct tatcagcagg agtgtttacc aaagacattg ataaagccat aacaatctcc     180
   tctgctctgc aggcaggaac agtgtgggtg aattgctatg gcgtggtaag tgcccagtgc     240
   ccctttggnt gggattcaag atgtctggaa atggaagaga actgggagag tacggtttcc     300
   atgaatatac agaggtcaaa acagtcacag tgaaaatctc tcagaagaac tcataaagaa     360
   aatacaagag tggagagaag ctcttcaata gctaagcatc tccttacagt cactaatat      419

<210>  292
<211>  538
<212>  DNA
<213>  human
<400>  292
caaggtccca ctctaggtga tagacaggga ccccttctac tgaacctttg aggaaaggag      60
gaaggaagaa atgcgtttag atcttggatg cagacctttc aaagggttaa atgtaaccat     120

atggatcaac cacatgcaca tccttactac agaatccgtc ctttcatttc aacttatagc 180
aagctatgat ttttatatat aaatattata taaataatgt ataaaacatt aaaagttaac 240
tatgtaagat attatttctg aaacaattta gctatatcca ctatgattat aaactgtgtc 300
tcgacctgtg ttatttacat tagctgctta aaaaagcatt gagttaattt ttttaaatat 360
caactaaaat atcatagttc tgtggtagac attgttttat aatgaaataa ctgcaactag 420
agaaaactgt ataaaaacat taaattgtca gtatttttgt aaggttccat tttgtaaaga 480
gaataatatt caaagacttt tgtagcatac aaagtgaaaa cttgtatctg cgaaacta 538

<210> 293
<211> 431
<212> DNA
<213> human

<220>
<221> misc_feature
<222> (131)..(131)
<223> n is a, c, g, or t

<400> 293
aagcagccta gggatgagca tttctttgaa agcaattagg ttattcacct ggtattaaaa 60
ctatttactg ttaaaaaatc tgtgacttca tgaagttgat ttttaaaggc agcatcaaaa 120
actgaaaagg naagggaaaa aataggcagc ttctctgcac ttgtttggag ctccccaaaa 180
caggagccat ggagaagtgg catcaagacc gggctgccct ttcgagaaca ccctgtggca 240
gttcagagac acgcttttcc tacactgcat gcagcccctc tttccagcac tggaaagaag 300
tggtcttgag cccagctgag aagcacttca cactcctctc tcttgttctg aatggtgttt 360
gtgtcagtct gcagctgtgt atggtattat gtcttataat cctgcatcac ttctatccta 420
tccagtcata t 431

<210> 294
<211> 445
<212> DNA
<213> human
<400> 294
gggtagatgg gagtgtcgct tgtatgttat cgtacagctg acatgtattt ttgtctattc 60
tttattatct tagtttcatg ctatgtatgt accataacca acctattgcc tatgagaaac 120
atgtaagata atgtatttac agccattgtt acaagtttat aatgtatttt tctatcttgt 180
tttatatgta tgttatataa cattcaaaaa gaattttttt cttgattgag aaaaggatac 240
aaaatgcaaa atccacaatt ttgataactg aaaattgcca attgttttgc agtactttat 300
tatattgggt gtcttgtctt ttttgggctt ttagttagct aatgaatgaa tacattagac 360
acttttgggt tttagttggg attttacata gcttgcattt taattctttg gttctttgct 420
gtttctatta acccacagca ttatt 445

<210> 295
<211> 394
<212> DNA
<213> human
<400> 295
gaaggttgtc atgggctgtt ccttgggggt ttttatcctg ctcaccgtgg agataagcct 60
gcggcttgtc taaccagcgc agcgcaaagg tctcaatgcc ttttggtaac atccgtcatt 120
gcagaagaaa gtttacacga cgtcaaaaag tgacgttcat gctaagtgtt tttccagaaa 180
tattggtttc atgtttctta ttggctctgc ctcctgtgct tatatcatcc aaaaactttt 240
taaaaaggtc cagaattcta ttttaacctg atgttgagca cctttaaaac gttcgtatgt 300
gtgttgcact aattctaaac tttggaggca ttttgctgtg tgaggccgat cgccactgta 360
aaggtcctag agttgcctgt ttgtctctgg agat 394

<210> 296
<211> 179
<212> DNA
<213> human
<400> 296
   gagatttacc atgtatcagt gcctggcttt ttgttataaa gctttgtttg tctagtgctc 60
   ttttgctata aaatagactg tagtacaccc tagtaggaaa aaaaaaaaac taaatttaaa 120
   aataaaaaat atatttggct tatttttcgc aggagcaatc cttttatacc atgaatatt 179

<210>  297
<211>  540
<212>  DNA
<213>  human

<220>
<221>  misc_feature
<222>  (59)..(59)
<223>  n is a, c, g, or t
<220>
<221>  misc_feature
<222>  (103)..(103)
<223>  n is a, c, g, or t

<400>  297
gtttatacaa ctgtcagtcc acccagtggt gcaactggtt ctgattcagt cttccgatnc      60
ctttttattt ttcacttttt cctatttctg aatttttttt ttnatttgtg atcttgattt     120
tgatgagggg ttggggagtg gggagggagt cgaaccaaga cttggagtta agaggatttt     180
catcttttgc atccaacagg cagaatatga tctgtgtcca aaagtgaact tgagtcagga     240
atgaatcaat ttcagcataa acaagcacaa aaatttagtc tgctggctga ctggaagcaa     300
aaaagtcaag atggaatatg atgaattcca acacaatggg gcaccaaggc ctttaggcct     360
ctctttttat tttgctttgg ttttgtttgt ttttctttag agacatgctc tttctcatgg     420
gacttgaagt ggactcatct ttgtgcagtg ctggttttgc catactcatt tcaagtatta     480
tagacatatg taatggtgaa aatatatgaa ctgtggcctt tttcattctt gttacttgtg     540

<210>  298
<211>  480
<212>  DNA
<213>  human
<400>  298
gaagtgactg ttgtaccatg gttgtgcaca tgcttcagaa tcctatggaa gagaatattc      60
ctacttgcag tacatcaaag gaatggatgg tggaccctac tattcatgtt ttgagacata     120
aatgttcact ttaaagcaat tgcataatag ataaaaacct gaactttcat tggatttttg     180
ttaattttcc tcattttgaa ttatgtgcac taccatagct acatcagttt gatacagtat     240
tgaaaaatta tcagttatat tttgctgttt atgatctatt tgtagattag gattaaaatg     300
gatttaatcc atttttaagg ctgtgtgaat ttttctaaac aagaaccatt tgcaatatgg     360
atttcttaga gattaaacca attataactt attagcagtc gcgagcacat gttcatatag     420
tcaatgtaaa aatacactaa tgagtatttg gtaaatccca gtaggctttt accattagca     480

<210>  299
<211>  124
<212>  DNA
<213>  human
<400>  299
tccctcactt ctcttaagcc aagaagtttg ctttccctag ctgcagtgta gatggctctt      60
gtttttgttt ttttgtttta atcatttggc attcacatgt ggctgttaat atgtgcttgt     120
tttt                                                                  124

<210>  300
<211>  547
<212>  DNA
<213>  human

<220>
<221>  misc_feature
<222>  (59)..(59)
<223>  n is a, c, g, or t
<220>
<221>  misc_feature
<222>  (308)..(308)
<223>  n is a, c, g, or t

<400>  300
tggtgggagg gctctcaatc agcagggccc caggagggaa gaagaagtgg ggcaaagcnt      60
ggcctcgccg ctcgggagct ttgccatctg agccacgcct cctccaggcc atgctccttg     120
aacttggaaa tgtcaaccgg agcccttaca ccagccctcc agcatctaat agacttgaat     180
ctactctaaa cgaatattta atccaacctc actacattgt agctcagtcc aacgactaac     240
cctgaaatgg gggtgttcca gccttcagcg agatggccaa gcggtcccct gggggctgtg     300
gcagcggnct tatccttctc tgttgccaac cttgccgtcc gacctcctcc gcccccatgc     360
ggtgaccccg tccgtgtctg tgtctgtcca tacgtgtgag tccagctaaa aagacaaaac     420
agaacccgtg ggcccagctc ggaaggtgcg tggagaaggc tccgacgtct ccgaagtgca     480
gcccttggga tggcattccg ttgtgtgcct tattcctgga gaatctgtat acggctcgcc     540
tatagaa                                                               547

<210>  301
<211>  350
<212>  DNA
<213>  human

<220>
<221>  misc_feature
<222>  (90)..(90)
<223>  n is a, c, g, or t
<220>
<221>  misc_feature
<222>  (124)..(124)
<223>  n is a, c, g, or t
<220>
<221>  misc_feature
<222>  (143)..(143)
<223>  n is a, c, g, or t
<220>
<221>  misc_feature
<222>  (150)..(150)
<223>  n is a, c, g, or t
<220>
<221>  misc_feature
<222>  (157)..(158)
<223>  n is a, c, g, or t
<220>
<221>  misc_feature
<222>  (162)..(163)
<223>  n is a, c, g, or t
<220>
<221>  misc_feature
<222>  (168)..(168)
<223>  n is a, c, g, or t
<220>
<221>  misc_feature
<222>  (223)..(223)
<223>  n is a, c, g, or t

<400>  301
cttctccaag catcaccctg ggagtttcct gagggttttc tcataaatga gggctgcaca      60
ttgcctgttc tgcttcgaag tattcaatan ccgctcagta ttttaaatga agtgattcta     120
agantttggt ttgggatcaa tanggaaagn catatgnnca gnnccaancc aagatgcaaa     180
tgttttgaaa tgatatgacc aaaattttaa gtaggaaagt cancccaaac acttctgctt     240
tcacttaagt gtctggcccg caatactgta ggaacaagca tgatcttgtt actgtgatat     300
tttaaatatc cacagtactc actttttcca aatgatccta gtaattgcct                 350

<210>  302
<211>  414
<212>  DNA
<213>  human

<220>
<221>  misc_feature
<222>  (157)..(157)
<223>  n is a, c, g, or t

<400>  302
gtatcaaaat cttccaatta tcatgctcac ctgaaagagg tatgctctct taggaataca      60
gtttctagca ttaaacaaat aaacaagggg agaaaataaa actcaaggag tgaaaatcag     120
gaggtgtaat aaaatgttcc tcgcattccc ccccgcnttt tttttttttt ttgactttgc     180
cttggagagc cagagcttcc gcattttctt tactattctt tttaaaaaaa gtttcactgt     240
gtagagaaca tatatgcata aacataggtc aattatatgt ctccattaga aaaataataa     300
ttggaaaaca tgttctagaa ctagttacaa aaataattta aggtgaaatc tctaatattt     360
ataaaagtag caaaataaat gcataattaa aatatatttg gacataacag actt           414

<210>  303
<211>  426
<212>  DNA
<213>  human

<220>
<221>  misc_feature
<222>  (324)..(324)
<223>  n is a, c, g, or t
<400>  303
caggaggaca cagcattatt ctagtgctgt actgttccgt acggcagcca ctacccacat      60
gtaacttttt aagatttaaa tttaaattag ttaacattca aaacgcagct ccccaatcac     120
actagcaaca tttcaagtgc ttgagagcca tgcatgatta gtggttaccc tattgaatag     180
gtcagaagta gaatcttttc atcatcacag aaagttctat tggacagtgc tcttctagat     240
catcataaga ctacagagca cttttcaaag ctcatgcatg ttcatcatgt tagtgtcgta     300
ttttgagctg gggttttgag actnccctta gagatagaga aacagaccca agaaatgtgc     360
tcaattgcaa tgggccacat acctagatct ccagatgtca tttcccctct cttattttaa     420
gttatg                                                                426

<210>  304
<211>  295
<212>  DNA
<213>  human

<220>
<221>  misc_feature
<222>  (114)..(114)
<223>  n is a, c, g, or t

<220>
<221>  misc_feature
<222>  (224)..(224)
<223>  n is a, c, g, or t

<400>  304
accaatgagg ttcctgaggt cacagtgttt tccaagtctc ccgtgacact gggtcagccc      60
aacaccctca tctgtcttgt ggacaacatc tttcctcctg tggtcaacat cacntggctg     120
agcaatgggc actcagtcac agaaggtgtt tctgagacca gcttcctctc caagagtgat     180
cattccttct tcaagatcag ttacctcacc ttcctccctt ctgntgatga gatttatgac     240
tgcaaggtgg agcactgggg cctggatgag cctcttctga aacactggga gcctg          295

<210>  305
<211>  537
<212>  DNA
<213>  human

<220>
<221>  misc_feature
<222>  (368)..(368)

<223>  n is a, c, g, or t

<400>  305
cacatggttc tagcctggac ctggctgggc tccatgagaa tgagttgcct ccaccctgtc      60
ccaacagctg acagccagga gccactctcc cagctgcagg cctttgtggt ccatcttgtc     120
ctgcttcctc actgtggacc cctgtctggg ccaccctagt gtgctaagct gagcagtgca     180
gtgtgaacag ggcccatggt gtattctagg ccacagccca gcactcctct gggctgctct     240
caaaccatgt cccatcttca gcatccctcc caccaactta ctcccctgtg gtgagtaccg     300
tggaacccca gcccacctca ctatcatact cagcttcccc tgatggccca tcccagcccc     360
tgaagctnta tgccaagaac acagctaccg cacaccaccc tgaaacagcc acagccaagg     420
taggcatgca tatgaggtct tccccatacc ctctgggtgt tgagaggttt agccacatga     480
gggagcagag gacaatctct gcagggctgg gagtgggtag ggactgaagg tctcaat        537

<210>  306
<211>  466
<212>  DNA
<213>  human
<400>  306
tcagtgtctg ggctcatgac ttgtaaatgg aagctgatgt gaacaggtaa ttaatattat      60
gacccacttc tatttacttt gggaaatatc ttggatctta attatcatct gcaagtttca     120
agaagtattc tgccaaaagt atttacaagt atggactcat gagctattgt tggttgctaa     180
atgtgaatca cgcgggagtg agtgtgccct tcacactgtg acattgtgac attgtgacaa     240
gctccatgtc ctttaaaatc agtcactctg cacacaagag aaatcaactt cgtggttgga     300
tggggccgga acacaaccag tctttttgta tttattgtta ctgagacaaa acagtactca     360
ctgagtgttt ttcagtttcc tactggtggt tttgatattg tttgtttaag atgtatattt     420
agaatgacat catctaagaa gctgattttg ctaaactcct gttccc                    466

<210>  307
<211>  474
<212>  DNA
<213>  human
<400>  307
gaactcatgt gatttaccct tttcaacttt ttggaaaacg atttaattta ttctaattag      60
attaacccta ttaatctatg gattgggtat caaaatgaat gccagtccag atgtgcctag     120
acacgaaatt ggagctgagg actctcacga tatgcaagtt catccaacgt gaagatacca     180
taagcttttt ctctgaacca gagaaatgaa agtcagttta agaggctgat agatcttggc     240
cctgttaagg catccacttc acagttctga aggctgagtc agccccactc cacagttagg     300
ccaagaatta gattttaaaa cttcatctgt ctgtcccagt taactgttaa ataaggcctc     360
atcctccact gaagagtatg gattgaagga ttgtgaacta tgtttagtgt gattgtgaac     420
ttggtgccta atgttccatg tctgaagttt gccccagtgc tacacgttgg agta           474

<210>  308
<211>  388
<212>  DNA
<213>  human

<220>
<221>  misc_feature
<222>  (218)..(218)
<223>  n is a, c, g, or t

<400>  308
gatgcaattg gttctcctgc attgagattt gatttaacag tgttatgtta acatttatac      60
ttgccttgga ctgtagaaca gaacttaaat gggaatgtat tagttttaca actacaatca     120
agtcatttta cctttaccca gtttttaata taaaacttaa attttgaaat tcactgtgtg     180
actaatagca tgatgctctg cagttttatt aagaaatnag cctaaccata caactctcat     240
ttccttagta agccaaatta ggattaactt ctataaacag tgttgggaac aatgtttaac     300
attttgtgcc aatttgttcc tgtattcatg tatgtaagtt acagatctga ctcttcattt     360
ttaagttcct tgttacatca tggtcatt                                        388

<210>  309
<211>  379
<212>  DNA

<213>  human
<400>  309
cctatcctac ttgcccatat gagcacggct ccccatggcc acatactcct gcaaagcttt      60
tatgctgctt cgcttttctc taaacagatc tgatattgct gctcctgtgg ttttctcaaa     120
attaactttg ccgtggtttt taaaaaggaa tcaaaatgca ttgttgcatt aagctttttc     180
aataaaggaa aattacggaa ggaaaatagg caacaccagc aaattatatg tggacaggtt     240
ctaaactcta tatatacata tatatatata tatctatata tctatatacg taatcatcta     300
gttctgtcat cttactgaaa ggaataacac ttctaaagat caccatttct gagaagttct     360
tggaaatctt tatgtctaa                                                  379

<210>  310
<211>  519
<212>  DNA
<213>  human

<220>
<221>  misc_feature
<222>  (130)..(130)
<223>  n is a, c, g, or t
<220>
<221>  misc_feature
<222>  (144)..(144)
<223>  n is a, c, g, or t
<220>
<221>  misc_feature
<222>  (167)..(167)
<223>  n is a, c, g, or t
<220>
<221>  misc_feature
<222>  (169)..(169)
<223>  n is a, c, g, or t
<220>
<221>  misc_feature
<222>  (268)..(268)
<223>  n is a, c, g, or t
<220>
<221>  misc_feature
<222>  (349)..(349)
<223>  n is a, c, g, or t
<220>
<221>  misc_feature
<222>  (358)..(358)
<223>  n is a, c, g, or t
<220>
<221>  misc_feature
<222>  (478)..(478)
<223>  n is a, c, g, or t

<400>  310
caccgcgcag agctcagggg gtggtgcgcc cggcccttct gcggcgcaca gcccagccca      60
ggaacgcggg cggtgcggac tcagcgggcc gggtgcaggc gcggagctgg gcctctgcgc     120
ccggcccgan ctccgtctat aaanagagca gccagttgca gggctcnant ctgctttcca     180
actgcctgac tgcttgttcg tctcactggt gtgagctcca gcatcccctt tgctcgaaat     240
ggaccccaac tgctcttgcg ccactggntg gctcctgcac gtgcgccggc tcctgcaagt     300
gcaaagagtg caaatgcacc tcctgcaaga agagctgctg ttcctgctnc cccgtggnct     360
gtgccaagtg tgcccagggc tgcgtctgca aaggggcatc ggagaagtgc agctgctgtg     420
cctgatgtgg gaacagctct tctcccagat gtaaatagaa caacctgcac aacctggnat     480
ttttttaaaa atacaacact gagccatttg ctgcatttc                            519

<210>  311
<211>  468
<212>  DNA
<213>  human

<400>  311
tactgatgct tctgatactg gatgtttagc ttcttactgc aaaaacataa gtaaaacagt      60
caactttacc atttccgtat tctccataga ttgaagaaat ttataccaca tatcgcatat     120
gaccatcttt ccatcaaatc aatgtagaga taatgtaaac tgaaaaaaaa tctgcaagat     180
aatgtaactg aatgttttaa aaacagaact tgtcacttta tataaaagaa tagtatgctc     240
tatttcctga atggatgtgg aaatgaaagc tagcgcacct gcactttgaa ttcttgcttc     300
ttttttatta ctgttatgat tttgcttttt acagatgttg gacgattttt tcttctgatt     360
gttgaattca taatcatggt ctcatttcct ttgcttcttt ggaatatttc tttcaacaca     420
ttcctttatt ttattataca ttgtgtcctt tttttagcta ttgctgct                  468

<210>  312
<211>  147
<212>  DNA
<213>  human
<400>  312
gacaggaggg tgtccacata tgttaacatc agttggatct cctatagaag tttctgctgc      60
tctctttcct tctccctgag ctggtaactg caatgccaac ttcctgggcc tttctgacta     120
gtatcacact tctaataaaa tccacaa                                         147

<210>  313
<211>  560
<212>  DNA
<213>  human

<220>
<221>  misc_feature
<222>  (175)..(177)
<223>  n is a, c, g, or t
<220>
<221>  misc_feature
<222>  (179)..(181)
<223>  n is a, c, g, or t
<220>
<221>  misc_feature
<222>  (190)..(190)
<223>  n is a, c, g, or t
<220>
<221>  misc_feature
<222>  (422)..(422)
<223>  n is a, c, g, or t
<220>
<221>  misc_feature
<222>  (426)..(426)
<223>  n is a, c, g, or t
<220>
<221>  misc_feature
<222>  (429)..(430)
<223>  n is a, c, g, or t

<400>  313
gctccacatg agccatgcat gcttagcaat ccaagtgcag agctctttgc tccaggagtg      60
aggagactgg gaggtgaaat ggggaaatgg aagggtttgg aggcagagct gaaaacaggg     120
ttggaaggat ttcctgaatt agaagacaaa cgttagcata cccagtaagg aaaannngnn     180
naggggccan ggggaacccg tgaggatcac tctcaaatga gattaaaaac aaggaagcag     240
agaatggtca gagaatggga ttcagattgg gaacttgtgg ggatgagagt gaccaggttg     300
aactgggaag tggaaaaagg agtttgagtc actggcacct agaagcctgc ccacgattcc     360
taggaaggct ggcagacacc ctggaaccct ggggagctac tggcaaactc tcctggattg     420
gncctnatnn ttttggtggg aaaggctgcc ctggggatca actttccttc tgtgtgtggc     480
tcaggagttc ttctgcagag atggcgctat ctttcctcct cctgtgatgt cctgctccca     540
accatttgta ctcttcatta                                                 560

<210>  314
<211>  530

<212>  DNA
<213>  human
<400>  314
aagtgtgcat aatttcattt aacgttaaag aaatagatcc aattcctttc ttgcaaccaa      60
aaataaataa aatacgttgc ctcaatataa ggtttgggct attctgtgtt tctatagaag     120
caatctgttt ttggtaaaat gtacttttaa ggatccagtc atctgaagta ttttatgtag     180
agttagagat ttcacaatat tgactataca tatatttaaa atataaatta tccagctgat     240
gtttgaattt gtcttacttt cctggccacc tcgttgtcct attttataag ctggggagtt     300
aactagctta acaaaagatg cttagctttt gtaaaagaac aagtgtttca ttttacaaag     360
acactccaaa tgatagttac ttgattttct cgagaccttt aactatggtg atgaataaca     420
ggacttgctt tcaagcctta ataaatgtaa aatgcctttt aatgaagata cagctgagtg     480
ttttcctcat gaatctgaac caattaccaa tttgtgttcc agtcttgatt                530

<210>  315
<211>  417
<212>  DNA
<213>  human
<400>  315
gaaaccaagg tcaggaggcc gttgcagaca taaatctcgg cgactcggcc ccgtctcctg      60
agggtcctgc tggtgaccgg cctggacctt ggccctacag ccctggaggc cgctgctgac     120
cagcactgac cccgacctca gagagtactc gcaggggcgc tggctgcact caagaccctc     180
gagattaacg gtgctaaccc cgtctgctcc tccctcccgc agagactggg gcctggactg     240
gacatgagag ccccttggtg ccacagaggg ctgtgtctta ctagaaacaa cgcaaacctc     300
tccttcctca gaatagtgat gtgttcgacg ttttatcaaa ggcccccttt ctatgttcat     360
gttagttttg ctccttctgt gtttttttct gaaccatatc catgttgctg acttttc        417

<210>  316
<211>  544
<212>  DNA
<213>  human
<400>  316
cacaggcagg tgactactcc atgcgcgtgg acctgcgggc tggggacgag gctgtgttcg      60
cccagtacga ctccttccac gtagactcgg ctgcggagta ctaccgcctc cacttggagg     120
gctaccacgg caccgcaggg gactccatga gctaccacag cggcagtgtc ttctctgccc     180
gtgatcggga ccccaacagc ttgctcatct cctgcgctgt ctcctaccga ggggcctggt     240
ggtacaggaa ctgccactac gccaacctca acgggctcta cgggagcaca gtggaccatc     300
agggagtgag ctggtaccac tggaagggct tcgagttctc ggtgcccttc acggaaatga     360
agctgagacc aagaaacttt cgctccccag cggggggagg ctgagctgct gcccacctct     420
ctcgcacccc agtatgactg ccgagcactg aggggtcgcc ccgagagaag agccagggtc     480
cttcaccacc cagccgctgg aggaagcctt ctctgccagc gatctcgcag cactgtgttt     540
acag                                                                  544

<210>  317
<211>  437
<212>  DNA
<213>  human
<400>  317
ccaatggcga gggtctgcca cactggccgc tgttcgacca ggaggagcaa tacctgcagc      60
tgaacctaca gcctgcggtg ggccgggctc tgaaggccca caggctccag ttctggaaga     120
aggcgctgcc ccaaaagatc caggagctcg aggagcctga agagagacac acagagctgt     180
agctccctgt gccggggagg agggggtggg ttcgctgaca ggcgagggtc agcctgctgt     240
gcccacacac acccactaag gagaaagaag ttgattcctt cattcacttc gccattcatt     300
catacttccg tccatccatt cagaaagcat ttattaagaa tttactcagg catgatggcc     360
catacttgta atcccagcta ttgggaagga tgagatggga ggatggcttg aggccagagg     420
tttgagaccg accagcc                                                    437

<210>  318
<211>  557
<212>  DNA
<213>  human

<220>
<221>  misc_feature

<222>  (464)..(464)
<223>  n is a, c, g, or t

<400>  318
gggagagtcc atctggaagc tggagtatat cctgacccag acctacgaca ttgaagattt      60
gcagccggaa agtttatatg gattagctaa acaatttaca atcctagaca gtaagcagtt     120
tataaaatac tacaattact tctttgtgag ttatgacagc agtgtaacat gtgataagac     180
atgtaaggcc tttcagattt gtgcaattat gaatcttgat aatatttcct atgcagattg     240
cctcaaacag ctttatataa agcacaatta ctagtatttc acagtttttg ctaatagaaa     300
atgctgattc tgattctgag atcaatttgt gggaatttta cataaatctt tgttaattac     360
tgagtgggca agtagacttc ctgtctttgc tttctttttt tttttctttt tgatgcctta     420
atgtagatat ctttatcatt ctgaattgta ttatatattt aaantgctca ttaatagaat     480
gatggatgta aattggatgt aaatattcag tttatataat tatatctaat ttgtaccctt     540
gttgaaattg tcattta                                                    557

<210>  319
<211>  336
<212>  DNA
<213>  human

<220>
<221>  misc_feature
<222>  (150)..(150)
<223>  n is a, c, g, or t
<220>
<221>  misc_feature
<222>  (154)..(154)
<223>  n is a, c, g, or t
<400>  319
cttcgcttct ctcttggaaa gtccagtctc tcctcggctt gcaatggacc ccaactgctc      60
ctgcgccgct ggtgtctcct gcacctgcgc tggttcctgc aagtgcaaag agtgcaaatg     120
cacctcctgc aagaagagct gctgctcctn gctnccccgt gggctgtagc aagtgtgccc     180
agggctgtgt ttgcaaaggg gcgtcagaga agtgcagctg ctgcgactga tgccaggaca     240
acctttctcc cagatgtaaa cagagagaca tgtacaaacc tggatttttt ttttatacca     300
ccttgaccca tttgctacat tccttttcct gtgaaa                              336

<210>  320
<211>  208
<212>  DNA
<213>  human

<220>
<221>  misc_feature
<222>  (85)..(85)
<223>  n is a, c, g, or t
<220>
<221>  misc_feature
<222>  (100)..(100)
<223>  n is a, c, g, or t
<220>
<221>  misc_feature
<222>  (102)..(102)
<223>  n is a, c, g, or t
<220>
<221>  misc_feature
<222>  (119)..(123)
<223>  n is a, c, g, or t
<220>
<221>  misc_feature
<222>  (125)..(126)
<223>  n is a, c, g, or t
<220>
<221>  misc_feature

<222>  (128)..(128)
<223>  n is a, c, g, or t
<220>
<221>  misc_feature
<222>  (148)..(148)
<223>  n is a, c, g, or t

<400>  320
cctggggctg agcaaggcct acgtaggcca gaagagcagc ttcacagtag actgcagcaa      60
agcaggcaac aacatgctgc tggtnggggt tcatggcccn angaccccct gcgaggagnn     120
nnngnngnag cacgtgggca gccggctnta cagcgtgtcc tacctgctca aggacaaggg     180
ggagtacaca ctggtggtca aatggggg                                         208

<210>  321
<211>  358
<212>  DNA
<213>  human

<220>
<221>  misc_feature
<222>  (146)..(146)
<223>  n is a, c, g, or t
<220>
<221>  misc_feature
<222>  (180)..(181)
<223>  n is a, c, g, or t
<400>  321
gaaactgtat gggtagcttt tttgtttgtt ttttgttttg tttttgtttt tgtttttgtt      60
tttagttgta ggtcgcagcg gggaaatttt ttgcgactgt acacatagct gcagcattaa     120
aaacttaaaa aaattgttaa aaaaanaaaa aaagggaaaa catttcaaaa aaaaaaaaan     180
ngataaacag ttacaccttg ttttcaatgt gtggctgagt gcctcgattt tttcatgttt     240
ttggtgtatt tctgatttgt agaagtgtcc aaacaggttg tgtgctggag ttccttcaag     300
acaaaaacaa acccagcttg gtcaaggcca ttacctgttt cccatctgta gttattcg       358

<210>  322
<211>  344
<212>  DNA
<213>  human
<400>  322
gctgctgccg cggggaagca ggaactggcc aagtacttct tggcagagct gctgtctgaa      60
cccaaccaga cggagaatga tgccctggaa cctgaagatc tgtcccaggc tgctgagcag     120
gatgaaatga ggcttgagct gcagagatct gctaactcaa acccggctat ggcaccccga     180
gaacgcaaag ctggctgcaa gaatttcttc tggaagactt tcacatcctg ttagctttct     240
taactagtat tgtccatatc agacctctga tccctcgccc ccacacccca tctctcttcc     300
ctaatcctcc aagtcttcag cgagaccctt gcattagaaa ctga                       344

<210>  323
<211>  486
<212>  DNA
<213>  human

<220>
<221>  misc_feature
<222>  (103)..(103)
<223>  n is a, c, g, or t
<400>  323
acaggaaatt gctacttacc gccgccttct ggaaggagaa gacgtaaaaa ctacagaata      60
tcagttaagc accctggaag agagagatat aaagaaaacc agnaagatta agacagtcgt     120
gcaagaagta gtggatggca aggtcgtgtc atctgaagtc aaagaggtgg aagaaaatat     180
ctaaatagct accagaagga gatgctgctg aggttttgaa agaaatttgg ctataatctt     240
atctttgctc cctgcaagaa atcagccata agaaagcact attaatactc tgcagtgatt     300
agaaggggtg gggtggcggg aatcctattt atcagactct gtaattgaat ataaatgttt     360
tactcagagg agctgcaaat tgcctgcaaa aatgaaatcc agtgagcact agaatattta     420

aaacatcatt actgccatct ttatcatgaa gcacatcaat tacaagctgt agaccaccta 480
atatca 486

<210> 324
<211> 299
<212> DNA
<213> human
<400> 324
gagaaagcca ggcaggtagc cagggggact agcccctgtg gagactgggg ggcttgaaat 60
tgtccccgtg gtctcttact ttcctttccc cagcccaggg tggacttaga aagcaggggc 120
tacaagaggg aatccccgaa ggtgctggag gtgggagcag gagattgaga aggagagaaa 180
gtgggtgaga tgctggagaa gagaggagag gagagaggca gagagcggtc tcaggctggt 240
gggaggggcg cccacctccc cacgccctcc cctcccctgc tgcaggggct ctggagaga 299

<210> 325
<211> 419
<212> DNA
<213> human
<400> 325
atgagccttc atacatggac tgagagtcag agcttgaaga aaagcttatt tattttcccc 60
aacctccccc aggtgcagtg tgacattatt ttattataac atccacaaag agattatttt 120
taaataattt aaagcataat atttcttaaa aagtatttaa ttatatttaa gttgttgatg 180
ttttaactct atctgtcata catcctagtg aatgtaaaat gcaaaatcct ggtgatgtgt 240
tttttgtttt tgttttcctg tgagctcaac taagttcacg gcaaaatgtc attgttctcc 300
ctcctacctg tctgtagtgt tgtggggtcc tcccatggat catcaaggtg aaacactttg 360
gtattctttg gcaatcagtg ctcctgtaag tcaaatgtgt gctttgtact gctgttgtt 419

<210> 326
<211> 445
<212> DNA
<213> human

<220>
<221> misc_feature
<222> (42)..(42)
<223> n is a, c, g, or t
<220>
<221> misc_feature
<222> (51)..(51)
<223> n is a, c, g, or t
<220>
<221> misc_feature
<222> (197)..(197)
<223> n is a, c, g, or t
<220>
<221> misc_feature
<222> (212)..(212)
<223> n is a, c, g, or t
<220>
<221> misc_feature
<222> (220)..(220)
<223> n is a, c, g, or t
<220>
<221> misc_feature
<222> (275)..(275)
<223> n is a, c, g, or t
<220>
<221> misc_feature
<222> (288)..(288)
<223> n is a, c, g, or t
<220>
<221> misc_feature
<222> (293)..(293)

<223>  n is a, c, g, or t
<220>
<221>  misc_feature
<222>  (300)..(300)
<223>  n is a, c, g, or t
<220>
<221>  misc_feature
<222>  (306)..(308)
<223>  n is a, c, g, or t
<220>
<221>  misc_feature
<222>  (336)..(336)
<223>  n is a, c, g, or t
<220>
<221>  misc_feature
<222>  (359)..(359)
<223>  n is a, c, g, or t

<400>  326
atgcctacag gtatgcgtga ttgtgtgtgt gtgcatgggt gnacagccac ntgtctacct      60
atgtgtcttt ctgggaatgt gtaccatctg tgtgcctgca gctgtgtagt gctggacagt     120
gacaaccctt tctctccagt tctccactcc aatgataata gttcacttac acctaaaccc     180
aaaggaaaaa ccagctntag gtccaattgt tntgctctan actgatacct caaccttggg     240
gccagcatct cccactgcct ccaaatatta gtaantatga ctgacgtncc canaagtttn     300
tgggtnnnac cacactcccc aaccccccac tcctanttcc tgaagggccc tcccaaggnt     360
acatccccac cccacagttc tccctgagag agatcaacct ccctgagatc aaccaaggca     420
gatgtgacag caagggccac ggacc                                           445

<210>  327
<211>  524
<212>  DNA
<213>  human

<220>
<221>  misc_feature
<222>  (40)..(40)
<223>  n is a, c, g, or t
<220>
<221>  misc_feature
<222>  (44)..(44)
<223>  n is a, c, g, or t
<220>
<221>  misc_feature
<222>  (48)..(49)
<223>  n is a, c, g, or t
<220>
<221>  misc_feature
<222>  (52)..(56)
<223>  n is a, c, g, or t
<220>
<221>  misc_feature
<222>  (60)..(60)
<223>  n is a, c, g, or t
<220>
<221>  misc_feature
<222>  (98)..(98)
<223>  n is a, c, g, or t

<400>  327
gcctctggca atgccattca ggccaggtct tcctcctatn gtgnaganna tnnnnntggn      60
ggtggaaagc gattctctca ttctggcaac cagttggnac ggccccatca ccgccctccg     120
ggtccgagtc aacacatact acatcgtagg tcttcaggtg cgctatggca aggtgtggag     180
cgactatgtg ggtggtcgca acggagacct ggaggagatc tttctgcacc ctggggaatc     240

agtgatccag gtttctggga agtacaagtg gtacctgaag aagctggtat ttgtgacaga 300
caagggccgc tatctgtctt ttgggaaaga cagtggcaca agtttcaatg ccgtcccctt 360
gcaccccaac accgtgctcc gcttcatcag tggccggtct ggttctctca tcgatgccat 420
tggcctgcac tgggatgttt accccactag ctgcagcaga tgctgagcct cctctccttg 480
gcaggggcac tgtgatgagg agtaagaact cccttatcac taac 524

<210> 328
<211> 335
<212> DNA
<213> human
<400> 328
acaaggccca ggctggggcc agggccagag gggaaggccc tggattctca ctcatgtgag 60
atcttgaatc tctttctttg ttctgtttgt ttagttagta tcatctggta aaatagttaa 120
aaaacaacaa aaaactctgt atctgtttct agcatgtgct gcattgactc tattaatcac 180
atttcaaatt caccctacat tcctctcctc ttcactagcc tctctgaagg tgtcctggcc 240
agccctggag aagcactggt gtctgcagca cccctcagtt cctgtgcctc agcccacagg 300
ccactgtgat aatggtctgt ttagcacttc tgtat 335

<210> 329
<211> 244
<212> DNA
<213> human

<220>
<221> misc_feature
<222> (74)..(74)
<223> n is a, c, g, or t
<220>
<221> misc_feature
<222> (93)..(93)
<223> n is a, c, g, or t
<220>
<221> misc_feature
<222> (164)..(169)
<223> n is a, c, g, or t
<220>
<221> misc_feature
<222> (171)..(172)
<223> n is a, c, g, or t
<220>
<221> misc_feature
<222> (176)..(176)
<223> n is a, c, g, or t
<220>
<221> misc_feature
<222> (180)..(181)
<223> n is a, c, g, or t

<400> 329
tcaacttcaa gctcctaagc cactgcctgc tggtgaccct ggccgcccac ctccccgccg 60
agttcaaccc ctgnggtgca cgcctccctg ganaagttcc tggcttctgt gagcaccgtg 120
ctgacctcca aataccgtta agctggagcc tcggtagccg ttcnnnnnnc nngctnggcn 180
ntccaacggg ccctcctccc ctccttgcac cggcccttcc tggtctttga ataaagtctg 240
agtg 244

<210> 330
<211> 543
<212> DNA
<213> human

<220>
<221> misc_feature
<222> (324)..(324)

<223>  n is a, c, g, or t
<400>  330
cttcctcgga ggcagcattg ttaagggagg ccctgtgcaa gtgctggagg acgaggaact      60
aaagtcccag ccagagcccc tagtggtcaa gggaaaacgg gtggctggag gccctcagat     120
gatccagctc agcctggatg ggaagcgcct ctacatcacc acgtcgctgt acagtgcctg     180
ggacaagcag ttttaccctg atctcatcag ggaaggctct gtgatgctgc aggttgatgt     240
agacacagta aaaggagggc tgaagttgaa ccccaacttc ctggtggact tcgggaagga     300
gccccttggc ccagcccttg cccnatgagc tccgctaccc tgggggcgat tgtagctctg     360
acatctggat ttgaaggctc caccctcatc acccacactc cctattttgg gccctcactt      420
ccttggggac ctggcttcat tctgctctct cttggcaccc gacccttggc agcatgtacc     480
acacagccaa gctgagactg tggcaatgtg ttgagtcata tacatttact gaccactgtt     540
gct                                                                   543

<210>  331
<211>  507
<212>  DNA
<213>  human
<400>  331
ccatcggtgc ggactccaag gaggtgcact ataagaaccg cttctggcat gacacctgct      60
tccgctgtgc caagtgcctt caccccttgg ccaatgagac ctttgtggcc aaggacaaca     120
agatcctgtg caacaagtgc accactcggg aggactcccc caagtgcaag gggtgcttca     180
aggccattgt ggcaggagat caaaacgtgg agtacaaggg gaccgtctgg cacaaagact     240
gcttcacctg tagtaactgc aagcaagtca tcgggactgg aagcttcttc cctaaagggg     300
aggacttcta ctgcgtgact tgccatgaga ccaagtttgc caagcattgc gtgaagtgca     360
acaagggttt ggtaaaggct ccagtgtggt ggcctatgaa ggacaatcct ggcacgacta     420
ctgcttccac tgcaaaaaat gctccgtgaa tctggccaac aagcgctttg ttttccacca     480
ggagcaagtg tattgtcccg actgtgc                                         507

<210>  332
<211>  492
<212>  DNA
<213>  human
<400>  332
ttgtcaaggg gctttgcatt caaactgctt ttccagggct atactcagaa gaaagataaa      60
agtgtgatct aagaaaaagt gatggtttta ggaaagtgaa aatatttttg tttttgtatt     120
tgaagaagaa tgatgcattt tgacaagaaa tcatatatgt atggatatat tttaataagt     180
atttgagtac agactttgag gtttcatcaa tataaataaa agagcagaaa aatatgtctt     240
ggttttcatt tgcttaccaa aaaaacaaca acaaaaaaag ttgtcctttg agaacttcac     300
ctgctcctat gtgggtacct gagtcaaaat tgtcattttt gttctgtgaa aaataaattt     360
ccttcttgta ccatttctgt ttagttttac taaaatctgt aaatactgta tttttctgtt     420
tattccaaat ttgatgaaac tgacaatcca atttgaaagt ttgtgtcgac gtctgtctag     480
cttaaatgaa tg                                                         492

<210>  333
<211>  251
<212>  DNA
<213>  human
<400>  333
tcataagtga cttctacccg ggagccgtga cagtggcctg gaaggcagat agcagccccg      60
tcaaggcggg agtggagacc accacaccct ccaaacaaag caacaacaag tacgcggcca     120
gcagctatct gagcctgacg cctgagcagt ggaagtccca cagaagctac agctgccagg     180
tcacgcatga agggagcacc gtggagaaga cagtggcccc tacagaatgt tcataggttc     240
tcaaccctca c                                                          251

<210>  334
<211>  519
<212>  DNA
<213>  human

<220>
<221>  misc_feature
<222>  (118)..(118)
<223>  n is a, c, g, or t

<220>
<221>  misc_feature
<222>  (125)..(125)
<223>  n is a, c, g, or t
<220>
<221>  misc_feature
<222>  (133)..(133)
<223>  n is a, c, g, or t
<220>
<221>  misc_feature
<222>  (136)..(136)
<223>  n is a, c, g, or t

<400>  334
gagtatacat cggtgcaggc ttcctggatg acagttgggt gatatgtgtc atgtggccta      60
aaagcctcca tgtcatttga cctacgaatt ctatctttgg gaatttatcc taagaaanta     120
cttanggatt tanttngtga taagatgttc atcccagcat tgcaatggag aaaaatggga     180
agcaatggtt tggttgggaa tttattcctt ttctgctgta acgaaagttt gcaatagggg     240
attgcttaag taaattattg tatctccatc cagatggtgg agtaccgcgc agacattaaa     300
agtcatgtaa aagaacatct gactgaaaga aaaatgctcc ttgaatatta aaaggttgta     360
aaaatagtgc atgttatgtg atttcaattt tgttttttaa aatatgggtg tatgcttgta     420
tacgtagagc agataaaaaa gacggaaggc atactaaaaa atgttgagtg gttatctttg     480
tatggtggaa caaagtcact gtaattttca tctttggtt                            519

<210>  335
<211>  492
<212>  DNA
<213>  human

<220>
<221>  misc_feature
<222>  (254)..(254)
<223>  n is a, c, g, or t
<220>
<221>  misc_feature
<222>  (315)..(315)
<223>  n is a, c, g, or t
<220>
<221>  misc_feature
<222>  (388)..(388)
<223>  n is a, c, g, or t

<400>  335
tggggctgag caaggcctac gtaggccaga agagcagctt cacagtagac tgcagcaaag      60
caggcaacaa catgctgctg gtgggggttc atggcccaag gaccccctgc gaggagatcc     120
tggtgaagca cgtgggcagc cggctctaca gcgtgtccta cctgctcaag gacaaggggg     180
agtacacact ggtggtcaaa tggggggacg agcacatccc aggcagcccc taccgcgttg     240
tggtgccctg agtntggggc ccgtgccagc cggcagcccc caagcctgcc ccgctaccca     300
agcagccccg ccctnttccc ctcaaccccg gcccaggccg ccctggccgc ccgcctgtca     360
ctgcagccgc ccctgccctg tgccgtgntg cgctcacctg cctccccagc cagccgctga     420
cctctcggct ttcacttggg cagagggagc catttggtgg cgctgcttgt cttctttggt     480
tctgggaggg gt                                                         492

<210>  336
<211>  386
<212>  DNA
<213>  human

<220>
<221>  misc_feature
<222>  (30)..(30)
<223>  n is a, c, g, or t
<220>

<221>  misc_feature
<222>  (117)..(117)
<223>  n is a, c, g, or t
<220>
<221>  misc_feature
<222>  (123)..(124)
<223>  n is a, c, g, or t
<220>
<221>  misc_feature
<222>  (221)..(221)
<223>  n is a, c, g, or t
<220>
<221>  misc_feature
<222>  (331)..(331)
<223>  n is a, c, g, or t
<220>
<221>  misc_feature
<222>  (335)..(335)
<223>  n is a, c, g, or t
<220>
<221>  misc_feature
<222>  (338)..(338)
<223>  n is a, c, g, or t
<220>
<221>  misc_feature
<222>  (344)..(344)
<223>  n is a, c, g, or t
<220>
<221>  misc_feature
<222>  (354)..(354)
<223>  n is a, c, g, or t
<220>
<221>  misc_feature
<222>  (359)..(359)
<223>  n is a, c, g, or t

<400>  336
aatgctctgg gtctctggat ccagtggggn atattgtgat gactcagtct ccactctccc     60
tgcccgtcac ccctggagag ccggcctcca tctcctgcag gtctagtcag agcctcntgc    120
atnntaatgg atacaactat ttggattggt acctgcagaa gccagggcag tctccacagc    180
tcctgatcta tttgggttct aatcgggcct ccggggtccc ngacaggttc agtggcagtg    240
gatcaggcac agattttaca ctgaaaatca gcagagtgga ggctgaggat gttggggttt    300
attactgcat gcaagctcta caaactcctc ngacnttngg ccangggacc aagntggana    360
tcaaacgaac tgtggctgca ccatct                                          386

<210>  337
<211>  295
<212>  DNA
<213>  human

<220>
<221>  misc_feature
<222>  (136)..(136)
<223>  n is a, c, g, or t
<220>
<221>  misc_feature
<222>  (221)..(221)
<223>  n is a, c, g, or t

<400>  337
gcaagatggt gttgcagacc caggtcttca tttctctgtt gctctggatc tctggtgcct     60
acggggacat cgtgatgacc cagtctccag actccctggc tgtgtctctg ggcgagaggg    120
ccaccatcaa ctgcangtcc agccagagtg ttttatacag ctccaacaat aagaactact    180

tagcttggta ccagcagaaa ccaggacagc ctcctaagct nctcatttac tgggcatcta    240
cccgggaatc cggggtccct gaccgattca gtggcagcgg gtctgggaca gattt         295

<210>  338
<211>  470
<212>  DNA
<213>  human

<220>
<221>  misc_feature
<222>  (92)..(92)
<223>  n is a, c, g, or t
<220>
<221>  misc_feature
<222>  (126)..(126)
<223>  n is a, c, g, or t
<220>
<221>  misc_feature
<222>  (148)..(149)
<223>  n is a, c, g, or t
<220>
<221>  misc_feature
<222>  (166)..(167)
<223>  n is a, c, g, or t
<220>
<221>  misc_feature
<222>  (170)..(170)
<223>  n is a, c, g, or t
<220>
<221>  misc_feature
<222>  (182)..(182)
<223>  n is a, c, g, or t
<220>
<221>  misc_feature
<222>  (239)..(239)
<223>  n is a, c, g, or t
<220>
<221>  misc_feature
<222>  (269)..(269)
<223>  n is a, c, g, or t
<220>
<221>  misc_feature
<222>  (289)..(289)
<223>  n is a, c, g, or t
<220>
<221>  misc_feature
<222>  (291)..(293)
<223>  n is a, c, g, or t
<220>
<221>  misc_feature
<222>  (317)..(318)
<223>  n is a, c, g, or t
<220>
<221>  misc_feature
<222>  (320)..(320)
<223>  n is a, c, g, or t
<220>
<221>  misc_feature
<222>  (327)..(327)
<223>  n is a, c, g, or t
<220>
<221>  misc_feature
<222>  (329)..(330)

<223>  n is a, c, g, or t

<400>  338
gaggtgcagc tgttggagtc tgggggaggc ttggtacagc ctggggggtc cctgagactc      60
tcctgtgcag cctctggatt cacctttagc anctatgcca tgagctgggt ccgccaggct     120
ccaggnaagg ggctggagtg ggtctcannt attagtggta gtggtnntan cacatactac     180
gnagactccg tgaagggccg gttcaccatc tccagagaca attccaagaa cacgctgtnt     240
ctgcaaatga acagcctgag agccgaggnc acggccgtat attactgtnc nnnagaaaat     300
ggtgtcagca gttggannt n ccaaganann accccaacct atgttgacta ctggggccag     360
gaaccctggt caccgtctcc tcagggagtg catccgcccc aacccttttc cccctcgtct     420
cctgtgagaa ttccccgtcg gatacgaagc agcgtggccg ttggctgctc                470

<210>  339
<211>  473
<212>  DNA
<213>  human
<400>  339
cgtcagtcat cctttattgc agtcgggatc cttggggttg ttctgttggc cattttcgtc      60
gcattattct tcttgactaa aaagcgaaga cagagacagc ggcttgcagt ttcctcaaga     120
ggagagaact tagtccacca aattcaatac cgggagatga attcttgcct gaatgcagat     180
gatctggacc taatgaattc ctcagaaaat tcccatgagt cagctgattt cagtgctgct     240
gaactaattt ctgtgtctaa atttcttcct atttctggaa tggaaaagga ggccattctg     300
agccacactg aaaaggaaaa tgggaattta taacccagtg agttcagcct ttaagatacc     360
ttgatgaaga cctggactat tgaatggagc agaaattcac ctctctcact gactattaca     420
gttgcatttt tatggagttc ttcttctcct aggattccta agactgctgc tga            473

<210>  340
<211>  123
<212>  DNA
<213>  human
<400>  340
ggggaatgga gcaaaacagt ctttgaatat cgaacacgca aggctgtgag actacctatt      60
gtagatattg caccctatga cattggtggt cctgatcaag aatttggtgt ggacgttggc     120
cct                                                                   123

<210>  341
<211>  290
<212>  DNA
<213>  human
<400>  341
cttgctctgt tgcagattgg cagatgccgc ctccctatgt ggtgctggac ttgccgcagg      60
agaccctgga ggaggagacc cccggcgcca acctgtggcc caccaccatc accttcctca     120
ccctcttcct gctgagcctg ttctatagca cagcactgac cgtgaccagc gtccggggcc     180
catctggcaa cagggagggc ccccagtact gagcgggagc cggcaaggca cagggaggaa     240
gtgtggagga acctcttgga gaagccagct atgcttgcca gaactcagcc                290

<210>  342
<211>  276
<212>  DNA
<213>  human

<220>
<221>  misc_feature
<222>  (32)..(35)
<223>  n is a, c, g, or t
<220>
<221>  misc_feature
<222>  (203)..(203)
<223>  n is a, c, g, or t
<220>
<221>  misc_feature
<222>  (240)..(240)
<223>  n is a, c, g, or t

<400>  342
gcataaatta atcagcccca gagtgcttta annnnttctc ttaaataccg gtgtctttga      60
tcaggatgac atgtgccatt tttcagagga cgtgcagaca ggctggcatt ctagattact     120
tttcttactc tgaaacatgg cctgtttggg agtgcgggat tcaaaggtgg tcccaccgct     180
gcccctactg caaatggcag ttntaatctt atcttttggc ttctgcagat ggttgcaatn     240
gatccttaac caataatggt cagtcctcat ctctgt                               276

<210>  343
<211>  314
<212>  DNA
<213>  human

<220>
<221>  misc_feature
<222>  (150)..(150)
<223>  n is a, c, g, or t
<220>
<221>  misc_feature
<222>  (161)..(161)
<223>  n is a, c, g, or t
<220>
<221>  misc_feature
<222>  (185)..(185)
<223>  n is a, c, g, or t
<400>  343
ctactctggc tccgaggtgc cagatgtgac atccagatga cccagtctcc atcctccctg      60
tctgcatctg taggagacag agtcaccatc acttgccggg caagtcagag cattagcagc     120
tatttaaatt ggtatcagca gaaaccaggn aaagccccta ngctcctgat ctatgctgca     180
tccantttgc aaagtggggt cccatcaagg ttcagtggca gtggatctgg gacagatttc     240
actctcacca tcagcagtct gcaacctgaa gattttgcaa cttactactg tcaacagagt     300
tacagtaccc ctca                                                       314

<210>  344
<211>  448
<212>  DNA
<213>  human

<220>
<221>  misc_feature
<222>  (28)..(28)
<223>  n is a, c, g, or t
<220>
<221>  misc_feature
<222>  (40)..(40)
<223>  n is a, c, g, or t
<220>
<221>  misc_feature
<222>  (49)..(50)
<223>  n is a, c, g, or t
<220>
<221>  misc_feature
<222>  (86)..(87)
<223>  n is a, c, g, or t
<220>
<221>  misc_feature
<222>  (99)..(99)
<223>  n is a, c, g, or t
<220>
<221>  misc_feature
<222>  (121)..(121)
<223>  n is a, c, g, or t
<220>

<221>  misc_feature
<222>  (139)..(139)
<223>  n is a, c, g, or t
<220>
<221>  misc_feature
<222>  (168)..(168)
<223>  n is a, c, g, or t
<220>
<221>  misc_feature
<222>  (180)..(180)
<223>  n is a, c, g, or t
<220>
<221>  misc_feature
<222>  (212)..(212)
<223>  n is a, c, g, or t
<220>
<221>  misc_feature
<222>  (226)..(226)
<223>  n is a, c, g, or t
<220>
<221>  misc_feature
<222>  (232)..(232)
<223>  n is a, c, g, or t
<220>
<221>  misc_feature
<222>  (236)..(236)
<223>  n is a, c, g, or t
<220>
<221>  misc_feature
<222>  (248)..(248)
<223>  n is a, c, g, or t
<220>
<221>  misc_feature
<222>  (255)..(255)
<223>  n is a, c, g, or t
<220>
<221>  misc_feature
<222>  (266)..(266)
<223>  n is a, c, g, or t
<220>
<221>  misc_feature
<222>  (314)..(314)
<223>  n is a, c, g, or t
<220>
<221>  misc_feature
<222>  (369)..(369)
<223>  n is a, c, g, or t
<220>
<221>  misc_feature
<222>  (371)..(371)
<223>  n is a, c, g, or t
<220>
<221>  misc_feature
<222>  (398)..(398)
<223>  n is a, c, g, or t

<400>  344
gccgagtact ggaacagcca gaaggacntc ctggaagacn agcgggccnn ggtggacacc      60
tactgcagac acaactacgg ggttgnngag agcttcacng tgcagcggcg agtccatcct     120
naggtgactg tgtatcctnc aaagacccag cccctgcagc accacaanct cctggtctgn     180
tctgtgagtg gtttctatcc aggcagcatt gnaagtcagg tggttncgga anggcnagga     240
agagaagnct ggggntggtg tccacnggcc tgatccagaa tggagactgg accttccaga     300
ccctggtgat gctngaaaca gttcctcgga gtggagaggt ttacacctgc caagtggagc     360

acccaagcnt nacgagccct ctcacagtgg aatggagngc acggtctgaa tctgcacaga    420
gcaagatgct gagtggagtc gggggctt                                        448

<210>  345
<211>  550
<212>  DNA
<213>  human

<220>
<221>  misc_feature
<222>  (30)..(30)
<223>  n is a, c, g, or t
<220>
<221>  misc_feature
<222>  (42)..(42)
<223>  n is a, c, g, or t
<220>
<221>  misc_feature
<222>  (49)..(49)
<223>  n is a, c, g, or t
<220>
<221>  misc_feature
<222>  (58)..(58)
<223>  n is a, c, g, or t
<220>
<221>  misc_feature
<222>  (116)..(116)
<223>  n is a, c, g, or t
<220>
<221>  misc_feature
<222>  (119)..(119)
<223>  n is a, c, g, or t
<220>
<221>  misc_feature
<222>  (175)..(176)
<223>  n is a, c, g, or t
<220>
<221>  misc_feature
<222>  (178)..(178)
<223>  n is a, c, g, or t
<220>
<221>  misc_feature
<222>  (180)..(181)
<223>  n is a, c, g, or t
<220>
<221>  misc_feature
<222>  (183)..(183)
<223>  n is a, c, g, or t
<220>
<221>  misc_feature
<222>  (188)..(188)
<223>  n is a, c, g, or t
<220>
<221>  misc_feature
<222>  (225)..(225)
<223>  n is a, c, g, or t
<220>
<221>  misc_feature
<222>  (246)..(246)
<223>  n is a, c, g, or t
<220>
<221>  misc_feature
<222>  (248)..(248)

<223>  n is a, c, g, or t
<220>
<221>  misc_feature
<222>  (258)..(258)
<223>  n is a, c, g, or t
<220>
<221>  misc_feature
<222>  (268)..(268)
<223>  n is a, c, g, or t
<220>
<221>  misc_feature
<222>  (274)..(274)
<223>  n is a, c, g, or t
<220>
<221>  misc_feature
<222>  (285)..(287)
<223>  n is a, c, g, or t
<220>
<221>  misc_feature
<222>  (289)..(289)
<223>  n is a, c, g, or t
<220>
<221>  misc_feature
<222>  (299)..(299)
<223>  n is a, c, g, or t
<220>
<221>  misc_feature
<222>  (336)..(336)
<223>  n is a, c, g, or t
<220>
<221>  misc_feature
<222>  (364)..(364)
<223>  n is a, c, g, or t

<400>  345
aagatcctgg agtttgtggg gcgctccctn ccagaggaga cngtggacnt catggttnag      60
cacacgtcgt tcaaggagat gaagaagaac cctatgacca actacaccac cgtccnccng     120
gagttcatgg accacagcat ctcccccttc atgaggaaag gcatggctgg ggacnngngn     180
ngnccacntt caccgtggcg cagaatgagc gcttcgatgc ggacntatgc ggagaagatg     240
gcaggncngc agcctcangc ttccgctntg agcngtgaga ggggnnncnt ggagtcacng     300
cagagggagt gtgcgaatca aacctgacca agcggntcaa gaataaaata tgaattgagg     360
gccngggacg gtaggtcatg tctgtaatcc cagcaatttg gaggctgagg tgggaggatc     420
atttgagccc aggagttcga gaccaacctg ggcaacatag tgagattctg ttaaaaaaat     480
aaaataaaat aaaaccaatt tttaaaaaga gaataaaata tgattgtggg ccaggcagag     540
tggctcatgc                                                            550

<210>  346
<211>  496
<212>  DNA
<213>  human

<220>
<221>  misc_feature
<222>  (150)..(151)
<223>  n is a, c, g, or t
<220>
<221>  misc_feature
<222>  (345)..(345)
<223>  n is a, c, g, or t
<220>
<221>  misc_feature
<222>  (347)..(348)
<223>  n is a, c, g, or t

<220>
<221>  misc_feature
<222>  (366)..(366)
<223>  n is a, c, g, or t
<220>
<221>  misc_feature
<222>  (405)..(405)
<223>  n is a, c, g, or t
<220>
<221>  misc_feature
<222>  (428)..(428)
<223>  n is a, c, g, or t

<400>  346
ccggtcagca ggatcatcgt gcacccacag ttctacatca tccagactgg agcggatatc      60
gccctgctgg agctggagga gcccgtgaac atctccagcc gcgtccacac ggtcatgctg     120
ccccctgcct cggagacctt ccccccgggn ntgccgtgct gggtcactgg ctggggcgat     180
gtggacaatg atgagcccct cccaccgcca tttcccctga agcaggtgaa ggtccccata     240
atggaaaacc acatttgtga cgcaaaatac caccttggcg cctacacggg agacgacgtc     300
cgcatcatcc gtgacgacat gctgtgtgcc gggaacaccc ggagngnntc atgccagggc     360
gactcnggag ggcccctggt gtgcaaggtg aatggcacct ggctncaggc gggcgtggtc     420
agctgggncg agggctgtgc ccagcccaac cggcctggca tctacacccg tgtcacctac     480
tacttggact ggatcc                                                     496

<210>  347
<211>  519
<212>  DNA
<213>  human

<220>
<221>  misc_feature
<222>  (326)..(326)
<223>  n is a, c, g, or t
<220>
<221>  misc_feature
<222>  (374)..(376)
<223>  n is a, c, g, or t
<220>
<221>  misc_feature
<222>  (398)..(401)
<223>  n is a, c, g, or t

<400>  347
gaaccacctc aatgcaaaga ttctacggga aaatgtgggc cccctccacc tattgacaat      60
ggggacatta cttcattccc gttgtcagta tatgctccag cttcatcagt tgagtaccaa     120
tgccagaact tgtatcaact tgagggtaac aagcgaataa catgtagaaa tggacaatgg     180
tcagaaccac caaaatgctt acatccgtgt gtaatatccc gagaaattat ggaaaattat     240
aacatagcat taaggtggac agccaaacag aagctttatt tgagaacagg tgaatcagct     300
gaatttgtgt gtaaacgggg atatcngtct ttcatcacgt tctcacacat tgcgaacaac     360
atgttgggat gggnnnctgg agtatccaac ttgtgcannn ngatagaatc aatcataaaa     420
tgcacacctt tattcagaac tttagtatta aatcagttct taatttcatt tttaagtatt     480
gttttactcc tttttattca tacgtaaaat tttggatta                            519

<210>  348
<211>  516
<212>  DNA
<213>  human

<220>
<221>  misc_feature
<222>  (161)..(162)
<223>  n is a, c, g, or t
<220>

<221>  misc_feature
<222>  (164)..(167)
<223>  n is a, c, g, or t
<220>
<221>  misc_feature
<222>  (170)..(170)
<223>  n is a, c, g, or t
<220>
<221>  misc_feature
<222>  (174)..(176)
<223>  n is a, c, g, or t
<220>
<221>  misc_feature
<222>  (179)..(179)
<223>  n is a, c, g, or t
<220>
<221>  misc_feature
<222>  (256)..(256)
<223>  n is a, c, g, or t
<220>
<221>  misc_feature
<222>  (364)..(370)
<223>  n is a, c, g, or t
<220>
<221>  misc_feature
<222>  (372)..(376)
<223>  n is a, c, g, or t
<220>
<221>  misc_feature
<222>  (465)..(465)
<223>  n is a, c, g, or t

<400>  348
tcttccaatt tattatctgc accaattatt ttgggcactt ctcctgtgta tgtaaacttt      60
ggagtttagg tctacgtcat ttatataaga gcctgtagac ctgagtggat ggacactgcc     120
tcttagaact agaacttaga actttatctt gaaaatgtac nncnnnngcn gaannnccnc     180
acagagtatg tgtcaggtag gaaccaaatt tatgattgtt atattcaaac tggttttctg     240
ttccttgaaa atggtntttt ttctgatcaa agcctattta ggagttatta tgttcttgag     300
tttattccat gtgtgtgaat attttgaatg aatccactaa taagatatgc atgtatgtct     360
gganmnnnnn cnnnnnagaa taaatattta agtagacttt aacaagtaaa tggttgtagg     420
catcattaag tcttaaatag ccttttcaaa gatattagtg ttctngtttt aattattcag     480
ataattcctt taattctcca ctcaaatctg acttta                               516

<210>  349
<211>  507
<212>  DNA
<213>  human
<400>  349
gattcctgtg ggtccagctt tggaactggg aaacctttct tcggatccgc actcattcca      60
ctgatgccag ctgcccctga aggatgccag tactgtggtg tgtgagtctc agcagccgcc     120
cacacgctcc taactctgct gcatggcaga tgcctaggtg gaaatagcaa aaacaaggcc     180
cgggctgggg ccagggccag aggggaaggc cctggattct cactcatgtg agatcttgaa     240
tctctttctt tgttctgttt gtttagttag tatcatctgg taaaatagtt aaaaaacaac     300
aaaaaactct gtatctgttt ctagcatgtg ctgcattgac tctattaatc acatttcaaa     360
ttcaccctac attcctctcc tcttcactag cctctctgaa ggtgtcctgg ccagccctgg     420
agaagcactg gtgtctgcag cacccctcag ttcctgtgcc tcagcccaca ggccactgtg     480
ataatggtct gtttagcact tctgtat                                         507

<210>  350
<211>  275
<212>  DNA
<213>  human

<220>
<221>  misc_feature
<222>  (129)..(129)
<223>  n is a, c, g, or t
<220>
<221>  misc_feature
<222>  (187)..(187)
<223>  n is a, c, g, or t
<220>
<221>  misc_feature
<222>  (191)..(191)
<223>  n is a, c, g, or t

<400>  350
ctctggctcc caggtgccag atgtgccatc cagttgaccc agtctccatc ctccctgtct      60
gcatctgtag gagacagagt caccatcact tgccgggcaa gtcagggcat tagcagtgct     120
ttagcctgnt atcagcagaa accagggaaa gctcctaagc tcctgatcta tgatgcctcc     180
agtttgnaaa ntggggtccc atcaaggttc agcggcagtg gatctgggac agatttcact     240
ctcaccatca gcagcctgca gcctgaagat tttgc                                275

<210>  351
<211>  339
<212>  DNA
<213>  human
<400>  351
ccaactgcct gactgcttgt tggcctcacc ggtgggagct ccagcatctc ctttgctcga      60
aatggacccc aactgctcct gtgccactgg tggctcctgc atgtgcgccg gctcctgcaa     120
gtgcaaagag tgcaaatgca cctcctgcaa gaagagctgc tgctcctgct gccccgtggg     180
ctgtgccaag tgtgcccagg gctgcatctg caaaggggcg ttggagaagt gcaactgcta     240
tgcctgacgt ggggacagct ctgctcccag atgtaaatag agtaacctgc acaaacctgg     300
attttttaaa aaatacaaca ctgagccatt tgcttcatt                             339

<210>  352
<211>  259
<212>  DNA
<213>  human
<400>  352
tcctccctgt ctgcatctgt aggagacaga gtcaccatca cttgccgggt gagtcagggc      60
attagcagtt atttaaattg gtatcggcag aaaccaggga aagttcctaa gctcctgatc     120
tatagtgcat ccaatttgca atctggagtc ccatctcggt tcagtggcag tggatctggg     180
acagatttca ctctcactat cagcagcctg cagcctgaag atgttgcaac ttattacggt     240
caacggactt acaatgccc                                                  259

<210>  353
<211>  539
<212>  DNA
<213>  human
<400>  353
tggaggaagt tctccagctt cagctcaact cacagcttct ccaagcatca ccctgggagt      60
ttcctgaggg ttttctcata aatgagggct gcacattgcc tgttctgctt cgaagtattc     120
aataccgctc agtattttaa atgaagtgat tctaagattt ggtttgggat caataggaaa     180
gcatatgcag ccaaccaaga tgcaaatgtt ttgaaatgat atgaccaaaa ttttaagtag     240
gaaagtcacc caaacacttc tgctttcact taagtgtctg gcccgcaata ctgtaggaac     300
aagcatgatc ttgttactgt gatattttaa atatccacag tactcacttt ttccaaatga     360
tcctagtaat tgcctagaaa tatctttctc ttacctgtta tttatcaatt tttcccagta     420
tttttatacg gaaaaaattg tattgaaaac acttagtatg cagttgataa gaggaatttg     480
gtataattat ggtgggtgat tattttttat actgtatgtg ccaaagcttt actactgtg      539

<210>  354
<211>  358
<212>  DNA
<213>  human
<400>  354

ccgccatttc ctctgaagca ggtgaaggtc cccataatgg aaaaccacat ttgtgacgca 60
aaataccacc ttggcgccta cacgggagac gacgtccgca tcgtccgtga cgacatgctg 120
tgtgccggga acacccggag ggactcatgc cagggcgact ccggagggcc cctggtgtgc 180
aaggtgaatg gcacctggct gcaggcgggc gtggtcagct ggggcgaggg ctgtgcccag 240
cccaaccggc ctggcatcta cacccgtgtc acctactact tggactggat ccaccactat 300
gtccccaaaa agccgtgagt caggcctggg ttggccacct gggtcactgg aggaccaa 358

<210> 355
<211> 273
<212> DNA
<213> human
<400> 355
agtcgggccc aggactggtg aagccttcgg agaccctgtc cctcacctgc actgtctctg 60
gtggctccat cagtagttac tactggagct ggatccggca gcccgccggg aagggactgg 120
agtggattgg gcgtatctat accagtggga gcaccaacta caacccctcc ctcaagagtc 180
gagtcaccat gtcagtagac acgtccaaga accagttctc cctgaagctg agctctgtga 240
ccgccgcgga cacggccgtg tattactgtg cga 273

<210> 356
<211> 162
<212> DNA
<213> human

<220>
<221> misc_feature
<222> (114)..(114)
<223> n is a, c, g, or t
<220>
<221> misc_feature
<222> (119)..(119)
<223> n is a, c, g, or t

<400> 356
agtgggtctc agctattagt gggagtgcag gtaccacata ctacgcagac tccgtgaagg 60
gccggttcac cacctccaga gacaattcca agaacacgct gtatctgcaa atgnacagnc 120
tgagagccga ggacacggcc gtatattact gtgcgaaatc cc 162

<210> 357
<211> 288
<212> DNA
<213> human
<400> 357
tcctccctgt ctgcatctgt aggagacaga gtcaccatca cttgccaggc gagtcaggac 60
attagtaatt ttttaacttg gtatcagaag aagccaggga aagcccctaa agtcctgatc 120
tacgatgcat ccaatttgga aacaggggtc ccatcaaggt tcagtggaag tggttctggg 180
acagatttta ctttcaccat cgccagcctg cagcctgacg attttgcaac atattactgt 240
caacaatata atgatcaccc cctcactttc ggccctggga ccaaagtg 288

<210> 358
<211> 356
<212> DNA
<213> human
<400> 358
atgaaactga ttacaacagg ctgtaagaat caaagtcaac tgacatctat gctacatatt 60
attatatagt ttgtactgag ctattgaagt cccattaact taaagtatat gttttcaaat 120
tgccattgct actattgctt gtcggtgtat tttattttat tgtttttgac tttggaagag 180
atgaactgtg tatttaactt aagctattgc tcttaaaacc agggatcaga atatatttgt 240
aagttaaatc attggtgcta ataataaatg tggattttgt attaaaatat atagaagcaa 300
tttctgttta catgtccttg ctacttttaa aaacttgcat ttattcctca gatttt 356

<210> 359
<211> 247
<212> DNA

<213>  human
<400>  359
catctcctgc actggaacca gcagtgacgt tggtgcttat aactatgtct cctggtacca     60
acagcaccca ggcaaagccc ccaaactcat gatttatgag gtcagtaatc ggccctcagg    120
ggtttctaat cgcttctctg gctccaagtc tggcaacacg gcctccctga ccatctctgg    180
gctccaggct gacgacgagg gtgattacta ctgcatctca tatacaagta gcaaccctct    240
cgtggtt                                                              247

<210>  360
<211>  548
<212>  DNA
<213>  human
<400>  360
tcaagtggga agagcgctgt tcaaggacca cctgagcgtg acctctgtgg ctgctacagc     60
gtgttccagt gtcctgccgg gctgtgccga gccatggaac catggggaga ccttcacttg    120
cactgctgcc caccccgagt tgaagacccc actaaccgcc aacatcacaa aatccggaaa    180
cacattccgg cccgaggtcc acctgctgcc gccgccgtcg gaggagctgg ccctgaacga    240
gctggtgacg ctgacgtgcc tggcacgtgg cttcagccca aggatgtgct ggttcgctgg    300
ctgcaggggt cacaggagct gccccgcgag aagtacctga cttgggcatc ccggcaggag    360
cccagccagg gcaccaccac cttcgctgtg accagcatac tgcgcgtggc agccgaggac    420
tggaagaagg gggacacctt ctcctgcatg gtgggccacg aggccctgcc gctggccttc    480
acacagaaga ccatcgaccg cttggcgggt aaacccaccc atgtcaatgt gtctgttgtc    540
atggcgga                                                             548

<210>  361
<211>  257
<212>  DNA
<213>  human
<400>  361
gctgtcagga ttcttcaagg agaatgaata ctgggaatca agacaggact ataccttatc     60
cataggcgca ggtgcacagg gggaggccat aaagatcaaa catgcatgga tgggtcctca    120
cgcagacaca cccacagaag gacactagcc tggcgcgcgt gcacacacac acacacacac    180
acgagttcat aatgtggtga tggccctaag ttaagcaaaa tgcttctgca cacaaaactc    240
tctggtttac ttcaaat                                                   257

<210>  362
<211>  277
<212>  DNA
<213>  human
<400>  362
gcacctgagc atgaaactcg acgcgttctt cggcatcttc tttggggccc tgggcggcct     60
cttgctgctg ggggtcggga cgttcgtggt cctgcgcttc tggggttgct ccggggccag    120
gttctcctat ttcctgaact cagctgaggc cttgccttga aggggcagct gtggcctagg    180
ctacctcaag actcacctca tccttaccgc acatttaagg cgccattgct tttgggagac    240
tggaaaaggg aaggtgactg aaggctgtca ggattct                             277

<210>  363
<211>  140
<212>  DNA
<213>  human

<220>
<221>  misc_feature
<222>  (33)..(33)
<223>  n is a, c, g, or t
<220>
<221>  misc_feature
<222>  (44)..(44)
<223>  n is a, c, g, or t
<220>
<221>  misc_feature
<222>  (64)..(65)
<223>  n is a, c, g, or t

<220>
<221>  misc_feature
<222>  (99)..(100)
<223>  n is a, c, g, or t

<400>  363
catctcctgc actggaacca gcagtgacgt tgntggttat aacnatgtct cctggtacca      60
acannaccca ggcaaagccc ccaaactcat gatttatgnn gtcagtaatc ggccctcagg     120
ggtttctaat cgcttctctg                                                 140

<210>  364
<211>  113
<212>  DNA
<213>  human
<400>  364
gcgctggttc ctgcaagtgc aaagagtgca aatgcacctc ctgcaagaag agctgctgct      60
cctgctgccc cgtgggctgt agcaagtgtg cccagggctg tgtttgcaaa ggg            113

<210>  365
<211>  266
<212>  DNA
<213>  human
<400>  365
aggacagagg gtcaccatct cttgctctgg aagcagctcc aacattggga agaattatgt      60
atcctggtac cagcaactcc caggaacagc ccccaaactc ctcatctata aaaataataa     120
gcgaccctca gggattcctg accgattctc tggctccaag tctggcacgt cagccaccct     180
gggcatcacc agactccaga ctggggacga ggccgattat tactgcggaa catgggatag     240
cagcctgagt ggtggccggg ggatat                                          266

<210>  366
<211>  434
<212>  DNA
<213>  human
<400>  366
tgaggagaag tctgccgtta ctgccctgtg gggcaaggtg aacgtggatg aagttggtgg      60
tgaggccctg ggcaggctgc tggtggtcta cccttggacc cagaggttct ttgagtcctt     120
tggggatctg tccactcctg atgctgttat gggcaaccct aaggtgaagg ctcatggcaa     180
gaaagtgctc ggttctttga gtcctttggg gatctgtcca ctcctgatgc tgttatgggc     240
aaccctaagg tgaaggctca tggcaagaaa gtgctcggtg cctttagtga tggcctggct     300
cacctggaca acctcaaggg cacctttgcc acactgagtg agctgcactg tgacaagctg     360
cacgtggatc ctgagaactt caggctcctg ggcaacgtgc tggtctgtgt gctggcccat     420
cactttggca aaga                                                       434

<210>  367
<211>  273
<212>  DNA
<213>  human
<400>  367
cagtgtccgg atctcctgga cagtcagtca ccatctcctg caccggaacc agcagtgatg      60
ttggtggtta taactatgtc tcctggtacc aacagcaccc aggcaaagcc cccacactca     120
tgatttatga tgtcattaag cggccctcag gggtccctga tcgcttctct ggctccaaat     180
ctggcaacac ggcctccctg accatctctg ggctccaggc tgaggatgag gctgattatt     240
attgcagctc aaatgcaggc agtttttatg tct                                  273

<210>  368
<211>  233
<212>  DNA
<213>  human
<400>  368
tcctgcactg ggagcagctc ctacatcggg gcaggttatg atgtacactg gtaccggcaa      60
cttcccggca cagcccccaa actcctcatc tatggtaaca ccgatcggcc ctcaggggtc     120
cctgaccgat tctctggctc caagtctggc atctcagcct ccctggccat cactgggctc     180
caggctgagg atgaggctga ttattactgc aagtcctatg acagcagcct gag            233

<210> 369
<211> 256
<212> DNA
<213> human
<400> 369
gatgtgacat ccaggtgacc cagtctccat cttccctgtc tgcgtctgta ggagacagag 60
tcaccatcac ctgccgggca agtcagggca ttagcaatgg gttatcctgg tatcagcaga 120
aaccagggca agcccctacg ctcctgatct atgctgcatc cagtttgcag tcgggggtcc 180
catctcggtt cagtggcagt ggatctggga cagatttcac tctcaccatc agcagcctgc 240
agcctgaaga tgttgc 256

<210> 370
<211> 434
<212> DNA
<213> human
<400> 370
ggtccccaca gactcagaga gaacccacca tggtgctgtc tcctgccgac aagaccaacg 60
tcaaggccgc ctggggtaag gtcggcgcgc acgctggcga gtatggtgcg gaggccctgg 120
agaggcactt cgacctgagc cacggctctg cccaagttaa gggccacggc aagaaggtgg 180
ccgacgcgct gaccaacgcc gtggcgcacg tggacgacat gcccaacgcg ctgtccgccc 240
tgagcgacct gcacgcgcac aagcttcggg tggacccggt caacttcaag ctaagccact 300
gcctgctggt gaccctggcc gcccacctcc ccgccgagtt cacccctgcg gtgcacgctt 360
ccctggacaa gttcctggct tctgtgagca ccgtgctgac ctccaaatac cgttaagctg 420
gagcctcggt agcc 434

<210> 371
<211> 270
<212> DNA
<213> human
<400> 371
gctgccaggt gccagatgtg acatccagat gacccagtct ccatcctccc tgtctgcatc 60
tgtaggagac agagtcacca tcacttgccg ggcgagtcag ggcattagca ataatttaaa 120
ttggtatcag cagaaaccag ggaaaactcc taagctcctg atctatgctg cacccagtct 180
gcaaagtggg attccctctc ggttcagtga cagtggatct ggggcagatt acactctcac 240
catccgcagc ctgcagcctg aagattttgc 270

<210> 372
<211> 319
<212> DNA
<213> human
<400> 372
agtcgctcca tttatcgctt gagatctcca gccttaccgc ggctcgaaat ggaccccaac 60
tgctcctgca ccactggtgt ctcctgcgcc tgcaccggct cctgcaagtg caaagagtgc 120
aaatgcacct cctgcaagaa gagctgctgc tcctgctgcc ccgtgggctg tgccaagtgt 180
gcccacggct gtgtctgcaa agggacgttg gagaactgca gctgctgtgc ctgatgtggg 240
aacagctctt ctcccagatg ttaatagaac aagctgcaca acctggattt tttttcaata 300
cgatactgag ccatttgct 319

<210> 373
<211> 434
<212> DNA
<213> human
<400> 373
cctccagacc tccttgaaat acaatattct cccagaaaag gaagagttcc cctttgcttt 60
aggagtgcag actctgcctc aaacttgtga tgaacccaaa gcccacacca gcttccaaat 120
ctccctaagt gtcagttaca cagggagccg ctctgcctcc aacatggcga tcgttgatgt 180
gaagatggtc tctggcttca ttcccctgaa gccaacagtg aaaatgcttg aaagatctaa 240
ccatgtgagc cggacagaag tcagcagcaa ccatgtcttg atttaccttg ataaggtgtc 300
aaatcagaca ctgagcttgt tcttcacggt tctgcaagat gtcccagtaa gagatctcaa 360
accagccata gtgaaagtct atgattacta cgagacggat gagtttgcaa tcgctgagta 420
caatgctcct tgca 434

<210>  374
<211>  500
<212>  DNA
<213>  human

<220>
<221>  misc_feature
<222>  (77)..(77)
<223>  n is a, c, g, or t
<220>
<221>  misc_feature
<222>  (181)..(181)
<223>  n is a, c, g, or t
<220>
<221>  misc_feature
<222>  (207)..(207)
<223>  n is a, c, g, or t

<400>  374
ctattaccat tctaactttc ataaaaagtt gggatcaaga agcagctgat ttcctgccag      60
ggcttatatt agggggntga ttcttaaagg acattaggat tggtgctcag aaatggttaa     120
tcatgctgtg tgctagccag ggccagctgg taccttcttt gccatgagca ttcaagggac     180
ngctaacctt tattgacaat ctatatngca aaagtcagga aagaggttgt gagctgattg     240
gattaaagac ctggcacttc agtaactcag cacgcttcca cttcactcaa cttaagagag     300
ttcattgaca gtgttaggat gtgaaggctg ggaaacactt attttgcttc aagagttcca     360
cttggctctc ccaaataggt acctcaaaaa ctgttagcaa gcggcatttg gatgtcttga     420
caggggcttt gcagggattt ttagggtttt ttccacattg tccacattaa tggttggcat     480
gattgtgctt gcaggccaag                                                  500

<210>  375
<211>  542
<212>  DNA
<213>  human
<400>  375
gaatatgacg ttaccttgca gactaaaagg ttgaaggccc gaaactaact tttagctaac      60
aataagggct gtgccccaat ggaaactgag ttcattttct gagaaaggtt tggatgactg     120
aaatatttcc tctacagtca aggactttgg catgcggtgg ctgaaactga gcttttttgt     180
gtgggctcca gttctcactg ttctgcaatg ctcatggcaa gttgaatggt gagctagctt     240
ataaattaaa gagctctgaa ctgtattcag accgactggg tatctagctt actgttttaa     300
catcattgtt gaaaccagac cctgtagtcc agtggtgctg ccctgttgtg caaactgctc     360
ctttttctcg tgtttttgta aagagcttcc atctgggctg gacccagttc ttgcacatac     420
aagacaccgc tgcagtcagc taggaccttt ccgccatgta ttctattctg tagtaaagca     480
tttccatcaa caatgcctaa ttgtatctgt tatttttggt ttaacacaca ctgattcata     540
ct                                                                     542

<210>  376
<211>  541
<212>  DNA
<213>  human
<400>  376
ggtctacgcc tattacaacc tggaggaaag ctgtacccgg ttctaccatc cggaaaagga      60
ggatggaaag ctgaacaagc tctgccgtga tgaactgtgc cgctgtgctg aggagaattg     120
cttcatacaa aagtcggatg acaaggtcac cctggaagaa cggctggaca aggcctgtga     180
gccaggagtg gactatgtgt acaagacccg actggtcaag gttcagctgt ccaatgactt     240
tgacgagtac atcatggcca ttgagcagac catcaagtca ggctcggatg aggtgcaggt     300
tggacagcag cgcacgttca tcagccccat caagtgcaga gaagccctga agctggagga     360
gaagaaacac tacctcatgt ggggtctctc ctccgatttc tggggagaga agcccaacct     420
cagctacatc atcgggaagg acacttgggt ggagcactgg cctgaggagg acgaatgcca     480
agacgaagag aaccagaaac aatgccagga cctcggcgcc ttcaccgaga gcatggttgt     540
c                                                                      541

<210>  377
<211>  485

<212>  DNA
<213>  human
<400>  377
cccctgcata tcttctcagc aataactcca tgggctctgg gaccctaccc cttccaacct     60
tccctgcttc tgagacttca atctacagcc cagctcatcc agatgcagac tacagtccct    120
gcaattgggt ctctggcagg caatagttga aggactcctg ttccgttggg gccagcacac    180
cgggatggat ggagggagag cagaggcctt tgcttctctg cctacgtccc cttagatggg    240
cagcagaggc aactcccgca tcctttgctc tgcctgtcgg tggtcagagc ggtgagcgag    300
gtgggttgga gactcagcag gctccgtgca gcccttggga acagtgagag gttgaaggtc    360
ataacgagag tgggaactca acccagatcc cgcccctcct gtcctctgtg ttcccgcgga    420
aaccaaccaa accgtgcgct gtgacccatt gctgttctct gtatcgtgat ctatcctcaa    480
caaca                                                                485

<210>  378
<211>  512
<212>  DNA
<213>  human
<400>  378
ctcttttact actgaccttt cacagagaaa aaatatttcc cttgaaaaaa actgggcttg     60
tcattttttc ccttgtagct ttaagcagag acataagtgc cttgcattac acatagtaaa    120
ctttctttaa aaaaaaaaaa aaaagatttt ggagactacc agggtaagat tccaacttgt    180
ccaaaagctt tctggcctta catattttat tataaaaatt ctcaagtctg gtaatcttct    240
atgtcagagc tagtgatttc aaaaggtttc acaattcccc aagacaaaag tgattttcgt    300
tcattataat aaggttaagt gatatgtgat tcataacaat tttgatgtga agaaggaaag    360
gacatcattg acttaataat agtatcagtc ggtgcaacag ttggcaacat gtgccttcac    420
actttaccat aaagagacgg gtttgagggt ttgccttcta aagtctgcaa cttcaagaaa    480
aaaaatcgac actgtggatt gactttcccg gt                                  512

<210>  379
<211>  520
<212>  DNA
<213>  human
<400>  379
aactttgtat agcccatgta cctaccttgt atagaaaaat aattttaaaa atttgaatgg     60
aagggggtaa aggaagtcat gaagtttttt tgcattttta tttaaatgaa ggaattccaa    120
ataactcacc tacagatttt tagcacaaaa atagccattg taaagtgtta aaatttacga    180
taagtattct attggggagg aaaggtaact ctgatctcag ttacagtttt tttttccttt    240
ttaatttcat tattttgggt ttttggtttt tgcagtccta tttatctgca gtcgtattaa    300
gtcctattgc tagaataggt tactacaaaa aaggttatat tctgaaagaa aaataactga    360
cattatatat aaccaattaa tttaaagtat tgccatttaa attacacact gagagcatgt    420
cctatgcaga catagatttt tctgttcatt tatttttctt cattgcagtg gattgatttg    480
ataaatagat gtgttgaatt actacatttg ctgtacatat                          520

<210>  380
<211>  448
<212>  DNA
<213>  human
<400>  380
ggagctagcc ttgttttttg catctttctc actcccatac atttatatta tatccccact     60
aaatttcttg ttcctcattc ttcaaatgtg ggccagttgt ggctcaaatc ctctatattt    120
ttagccaatg gcaatcaaat tctttcagct cctttgtttc atacggaact ccagatcctg    180
agtaatcctt ttagagcccg aagagtcaaa accctcaatg ttccctcctg ctctcctgcc    240
ccatgtcaac aaatttcagg ctaaggatgc cccagaccca gggctctaac cttgtatgcg    300
ggcaggccca gggagcaggc agcagtgttc ttcccctcag agtgacttgg ggagggagaa    360
ataggaggag acgtccagct ctgtcctctc ttcctcactc ctcccttcag tgtcctgagg    420
aacaggactt tctccacatt gttttgta                                       448

<210>  381
<211>  547
<212>  DNA
<213>  human

<220>

<221>  misc_feature
<222>  (159)..(159)
<223>  n is a, c, g, or t
<220>
<221>  misc_feature
<222>  (240)..(240)
<223>  n is a, c, g, or t
<220>
<221>  misc_feature
<222>  (299)..(299)
<223>  n is a, c, g, or t
<220>
<221>  misc_feature
<222>  (312)..(312)
<223>  n is a, c, g, or t

<400>  381
gcgggtcttg ctggctaaaa tgcccaggta aagggttggt tggacacagc gcttagtgca      60
cgctgtcatc atggacatca taatcagttg tgaaaaacac gcgaacctat gacacttctt     120
attccacact gaatgtgaaa ttgcatgttc agatgtttna ctacgaggcc tggctcacag     180
gaagtgttca gtaaaagtat gcactgttag attactgata acgcggatag atttttgttn     240
accataaatt gttccagatt tatattaatg gaaggaagtg tgcatttatt agctattant     300
caactttaca angcaaacat cttatttctc atctttaaac atgtcgacca gtttaattga     360
aaagtattct gagactgcaa aatggggtgt taaaaaatac tgcagttacg gagctgtgta     420
aaccagtttc tcattgcata agatacagat gtaaattgca tggagaggtt gatatgcacc     480
tgtacagtaa ttcactcccc catttcactt ctttgtcaga gaatagttct tgttcatact     540
gagtgtt                                                               547

<210>  382
<211>  425
<212>  DNA
<213>  human
<400>  382
   gcatccgagg ggagccgccg gatgtggaag aagactcggc tttcctgcag ccatttagtg      60
   ccgccccatg ctaggttatt tgacattgtg cagtgtagag ttgccttaaa gtgcgtgatc     120
   tgccagtgct ttcttcaagt cacccttgcc ccgattcctc atgtttgcgc tccccagggt    180
   tgctcaagtg gaaattttgt cagctgttta gccttttcgt acttggcgtg atgtcaactt     240
   cacttctaat ctgcaaaagc agaagctgtt tcctagttta cctcgcgtgt gtttacctat     300
   atggagtagc tcgcagagat cacagaaatg cttgcagcct aaggcagggt tttcagaccg     360
   tgggtcccag cccatttagt aaaatgggaa atcaattagc aagtggtcac cagcattaca     420
   cagca                                                                 425

<210>  383
<211>  551
<212>  DNA
<213>  human
<400>  383
tatacagttt aataagcctc ttgcaagtta cttgttctct cacctgaggt atttttttcc      60
tccccacctt gcccctgttc ctcccttcct cttctccctt tgcaagagga aatatttaac     120
atatttgggt ccaacttcaa taatgtaata attaatacat taaaagcatt taacttcctt     180
tctagaaaaa tgcacaggct aaggcataga caaaacaaag agaaatgctg agaaatttgc     240
cactggagac aagcaatctg aataaatatt tgccaaaagt tctttttatg tcatatagtg     300
tcaggatttg aaggagctat tttttttaat gttgcaacta gcaactcatc ttcggaagac     360
acagccagga gaatgaagta gaagtgaaag gtttataaat ccatttgtaa gcatttatcc     420
catatatttt aaattcaaga aaaattgtgt ttatctttag aattttgtat tcaatacttt     480
atgtactatg tgactcatgc ttctggataa ataaagcacc aaatatgtat ctgtaaccac     540
aatcacacat a                                                          551

<210>  384
<211>  468
<212>  DNA
<213>  human
<400>  384

ggcttgatgt agactggctt gctttgattt ttagtgaagg gaatgtacgt aaaacaaaat 60
agggcttggc tggtcaaagg agacaagcag gatggatgga tggatggatg aatagataga 120
tggtgtttgc atgtaaattg cagagaaaac aaaaccaaag ctgattggaa acaattaatt 180
gtgggtgtct gagggggaag gtcgcagctt tgggcagctt tgagaagcgg tacaagagct 240
ctgtgcctgt gtgtccagcc ctggagccag ccagtgcatt tattttaagc tcttagaagc 300
aactccttgg cccaggaatg cgtgacccct gagatgggtc cacgcatctc tctacacgtc 360
cttctctccg tgggatactg gactcgtgcc tctgcgccca ttctcttctc acgcatatcc 420
atgagcttta atttcacttt ctgatcacgg tacgtccata aagccagt 468

<210> 385
<211> 355
<212> DNA
<213> human
<400> 385
ggagatgact taagttggca gcagtaatct tcttttagga gcttgtacca cagtcttgca 60
cataagtgca gatttgcccc aagtaaagag aatttcctca acactaactt cacggggata 120
atcaccacgt aactaccctt aaagcatatc actagccaaa gaggggaata tctgttcttc 180
ttactgtgcc tatattaaga ctagtacaaa tgtggtgtgt cttccaactt tcattgaaaa 240
tgccatatct ataccatatt ttattcgagt cactgatgat gtaatgatat attttttcat 300
tattatagta gaatattttt atggcaagag atttgtggtc ttgatcatac ctatt 355

<210> 386
<211> 551
<212> DNA
<213> human
<400> 386
gaagctgaca caccgtatgt tgttagagtc ttttatctgg tcaggggaaa caaaatcttg 60
acccagctga acatgtcttc ctgagtcagt gcctgaatct ttatttttta aattgaatgt 120
tccttaaagg ttaacatttc taaagcaata ttaagaaaga ctttaaatgt tattttggaa 180
gacttacgat gcatgtatac aaacgaatag cagataatga tgactagttc acacataaag 240
tccttttaag gagaaaatct aaaatgaaaa gtggataaac agaacattta taagtgatca 300
gttaatgcct aagagtgaaa gtagttctat tgacattcct caagatattt aatatcaact 360
gcattatgta ttatgtctgc ttaaatcatt taaaaacggc aaagaattat atagactatg 420
aggtaccttg ctgtgtagga ggatgaaagg ggagttgata gtctcataaa actaatttgg 480
cttcaagttt catgaatctg taactagaat ttaattttca ccccaataat gttctatata 540
gcctttgcta a 551

<210> 387
<211> 433
<212> DNA
<213> human
<400> 387
gttcaaaata tttccaagcc tgagtattgt ctattggtat agatttttag aaatcaataa 60
ttgattattt atttgcactt attacaatgc ctgaaaaagt gcaccacatg gatgttaagt 120
agaaattcaa gaaagtaaga tgtcttcagc aactcagtaa aaccttacgc caccttttgg 180
tttgtaagag gttttttata catttcaaac aggttgcaca aaagttaaaa taatggggtc 240
ttttataaat ccaaagtact gtgaaaacat tttacatatt ttttaaatct tctgactaat 300
gctaaaacgt aatctaatta aatttcatac agttactgca gtaagcatta ggaagtgaat 360
atgatataca aaatagttta taaagactct atagtttcta taatttattt tactggcaaa 420
tgtcatgcaa caa 433

<210> 388
<211> 493
<212> DNA
<213> human
<400> 388
ggcagcccac aagtttctcg tggggagatg gaggcagagc ccagggtagg ggacagagct 60
gctggggcct ttccttgcct gggaatctgt cccaggaaga gcttccccac tcccatcccc 120
caaattggaa aaaccgtaca ttcaagcctg tttggccctg aaattcttaa gaatctggtt 180
aagaattaac tcactaatgt caaaagtcaa aacctcctag gggttgtcct gggagtcagg 240
ttcacgggta cagaagatga atctcagatg tcactcaacc tgagccgtca ttctctgtgg 300
cagggctgcc ctgggtttct cttactcaat ccctggagtg taagcatttg gattgtgtca 360
cagattacct ttttaccttt tctttctttt tttttctttt tttcaatatc agtgcccaca 420

ccttactgag tattgagttt tagagctttc gcttgatgtg cttgaccaag agacttcttt 480
tgtatccttt tct 493

<210> 389
<211> 473
<212> DNA
<213> human
<400> 389
tcaagtgcgt tctttagacc aatgcattgc gtttctttcc ctgcttttga gatagtagga 60
agagttcttg gtggtgtccc cccccttcaa ttcttcagtt gtatagtagt tatagggaag 120
atatgggtgt ttttctttat tattactttt ttttttctgc aggtcagtaa aaggatttaa 180
gttgcactga caaaaatacc aaaataaaag tgtattttta agttcccatt tgaaattgct 240
ggcgctgctg gccggatgca tttttgagtt tgtattagtt gataaattaa cagtaataac 300
aagattgtat gaaccgcatg gtgcttgcag ttttaaatat tgtggatatt tgtcctgcat 360
cagaaacgag ctttggtttt tacagattca actgtgttga aatcaaacct gccgcaacag 420
aaattgtttt tatttcatgt aaaataaggg atcaatttca aaccctgctt atg 473

<210> 390
<211> 495
<212> DNA
<213> human
<400> 390
cctcctgcct ctggatttta ggtgttgatt tctggatcac gggataccac ttcctgtcca 60
caccccgacc aggggctaga aaatttgttt gagattttta tatcatcttg tcaaattgct 120
tcagttgtaa atgtgaaaaa tgggctgggg aaaggaggtg gtgtccctaa ttgttttact 180
tgttaacttg ttcttgtgcc cctgggcact tggcctttgt ctgctctcag tgtcttccct 240
ttgacatggg aaaggagttg tggccaaaat ccccatcttc ttgcacctca acgtctgtgg 300
ctcagggctg gggtggcaga gggaggcctt caccttatat ctgtgttgtt atccagggct 360
ccagacttcc tcctctgcct gccccactgc accctctccc ccttatctat ctccttctcg 420
gctccccagc ccagtcttgg cttcttgtcc cctcctgggg tcatccctcc actctgactc 480
tgactatggc agcag 495

<210> 391
<211> 482
<212> DNA
<213> human
<400> 391
ggcatgtgtg actgtttcag cgactgcgga gtctgtctct gtggcacatt ttgtttcccg 60
tgccttgggt gtcaagttgc agctgatatg aatgaatgct gtctgtgtgg aacaagcgtc 120
gcaatgagga ctctctacag gacccgatat ggcatccctg gatctatttg tgatgactat 180
atggcaactc tttgctgtcc tcattgtact ctttgccaaa tcaagagaga tatcaacaga 240
aggagagcca tgcgtacttt ctaaaaactg atggtgaaaa gctcttaccg aagcaacaaa 300
attcagcaga cacctcttca gcttgagttc ttcaccatct tttgcaactg aaatatgatg 360
gatatgctta agtacaactg atggcatgaa aaaaatcaaa tttttgattt attataaatg 420
aatgttgtcc ctgaacttag ctaaatggtg caacttagtt tctccttgct ttcatattat 480
cg 482

<210> 392
<211> 566
<212> DNA
<213> human

<220>
<221> misc_feature
<222> (124)..(124)
<223> n is a, c, g, or t

<400> 392
agagaatgcc cttctcctta ttgtaaccct gtctggatcc tatcctccta cctccaaagc 60
ttcccacggc ctttctagcc tggctatgtc ctaataatat cccactggga gaaaggagtt 120
ttgncaaagt gcaaggacct aaaacatctc atcagtatcc agtggtaaaa aggcctcctg 180
gctgtctgag gctaggtggg ttgaaagcca aggagtcact gagaccaagg ctttctctac 240
tgattccgca gctcagaccc tttcttcagc tctgaaagag aaacacgtat cccacctgac 300

atgtccttct gagcccggta agagcaaaag aatggcagaa aagtttagcc cctgaaagcc 360
atggagattc tcataacttg agacctaatc tctgtaaagc taaaataaag aaatagaaca 420
aggctgagga tacgacagta cactgtcagc agggactgta aacacagaca gggtcaaagt 480
gttttctctg aacacattga gttggaatca ctgtttagaa cacacacact tactttttct 540
ggtctctacc actgctgata ttttct 566

<210> 393
<211> 489
<212> DNA
<213> human
<400> 393
attaccttat catgtcttag agcccgtctt tatgtttaaa actaatttct taaaataaag 60
ccttcagtaa atgttcatta ccaacttgat aaatgctact cataagagct ggtttggggc 120
tatagcatat gctttttttt ttttaattat tacctgattt aaaaatctct gtaaaaacgt 180
gtagtgtttc ataaaatctg taactcgcat tttaatgatc cgctattata agcttttaat 240
agcatgaaaa ttgttaggct atataacatt gccacttcaa ctctaaggaa tatttttgag 300
atatcccttt ggaagacctt gcttggaaga gcctggacac taacaattct acaccaaatt 360
gtctcttcaa atacgtatgg actggataac tctgagaaac acatctagta taactgaata 420
agcagagcat caaattaaac agacagaaac cgaaagctct atataaatgc tcagagttct 480
ttatgtatt 489

<210> 394
<211> 506
<212> DNA
<213> human
<400> 394
atggggctgg ggacttgaat tggatgcttc aaaaccatca cctgttggcc aacaagtttg 60
acccaaaggt agatgataat gctcttcagt gcttagaaga atacctacgt tataaggcca 120
tctatgggac tgaactttga gacacactat gagagcgttg ctacctgtgg ggcaagagca 180
tgtacaaaca tgctcagaac ttgctgggac agtgtgggtg ggagaccagg gctttgcaat 240
tcgtggcatc ctttaggata agagggctgc tattagattg tgggtaagta gatcttttgc 300
cttgcaaatt gctgcctggg tgaatgctgc ttgttctctc acccctaacc ctagtagttc 360
ctccactaac tttctcacta agtgagaatg agaactgctg tgatagggag agtgaaggag 420
ggatatgtgg tagagcactt gatttcagtt gaatgcctgc tggtagcttt tccattctgt 480
ggagctgccg ttcctaataa ttccag 506

<210> 395
<211> 315
<212> DNA
<213> human
<400> 395
ggaattcttc tcttacttca ataaaatggg ttttaacata actttaaatt cagttaaata 60
tacaatattg aatacctata gttgactttg ggatggggac tttttcaagt cattaagagt 120
gtttgtttaa ggtgatctca ttgatggtag ttctcagccg tctcaaaaac tgcaagctaa 180
tcagtcagac attctttaat gaccccaatt ttttcacttt aattgttacc atgttttcta 240
tttttactga tttttgctaa agcatgtaag agtgaattta ttatagcagt aatcttgtgt 300
ttctcctgat gtgca 315

<210> 396
<211> 331
<212> DNA
<213> human
<400> 396
atcaacacat ttagcttggc gttttattca ttccatcacc cttactgtaa ctactatggc 60
aactcaaata attgtcatgg gactatgtcc atcttaatgg gtctggatgg catggtgctc 120
ctcttaagtg tgctggaatt ctgcattgct gtgtccctct ctgcctttgg atgtaaagtg 180
ctctgttgta cccctggtgg ggttgtgtta attctgccat cacattctca catggcagaa 240
acagcatctc ccacaccact taatgaggtt tgaggccacc caaagatcaa cagacaaatg 300
ctccagaaat ctatgctgac tgtgacacaa g 331

<210> 397
<211> 449
<212> DNA

<213>  human
<400>  397
catctctcag gaggtgggct gtccaccaaa atgagcattc agggctgcgt ggcccaacct      60
tccagcttct tgttgaacca caccagacaa atcgggatct tctctgcgcg tgagaagcgt     120
gatgtgcagc ctcctgcctc tcagcatgag ggaggtgggg ctgagggcct ggagtctctc     180
acttgggggg tggggctggc actggcccca gcgctgtggt ggggagtggt ttgcccttcc     240
tgctaactct attaccccca cgattcttca ccgctgctga ccacccacac tcaacctccc     300
tctgacctca taacctaatg gccttggaca ccagattctt tcccattctg tccatgaatc     360
atcttcccca cacacaatca ttcatatcta ctcacctaac agcaacactg gggagagcct     420
ggagcatccg gacttgccct atgggagag                                       449

<210>  398
<211>  483
<212>  DNA
<213>  human
<400>  398
tccttgtcca caagcactat ggcccccggc tcaagtgctg ctctggcaaa gctccggagc      60
cccagcccca aggctttgac aaccaggcgt tcctccctga ccacaaggcc aactgggcgc     120
ccgtccccag ccccacgcac gaccccaagc ccgcggaggc accgatgccc gcagagcccg     180
caccccccgg ccctgcctcc ccaggcggtg cccctgagcc ccccgcagcg gcccgagctg     240
gcggaagccc cacggcggtg aggtccatcc tgaccaagga gcggcggcca gagggcgggt     300
acaaggctgt ctggtttggc gaggacatcg ggacggaggc agacgtggtc gttctcaacg     360
cgcccaccct ggacgtggat ggcgccagtg actccggcag cggcgatgag ggcgagggcg     420
cggggagggg tggggtcccc tacgatgcgc ccggtggtga tgactcctac atctaagtgg     480
ccc                                                                   483

<210>  399
<211>  241
<212>  DNA
<213>  human
<400>  399
accatcgctg gtggtatccc agggtccctg ctcaagtttt ctttgaaaag gagggctgga      60
atggtacatc acataggcaa gtcctgccct gtatttaggc tttgcctgct tggtgtgatg     120
taagggaaat tgaaagactt gcccattcaa aatgatcttt accgtggcct gccccatgct     180
tatggtcccc agcatttaca gtaacttgtg aatgttaagt atcatctctt atctaaatat     240
t                                                                     241

<210>  400
<211>  470
<212>  DNA
<213>  human
<400>  400
gaatactcat aattcttatc tctataatca aaagtataat ttactgtaga aaaataaaga      60
gatgcttgtt ctgaaagtaa gatcagtgaa ctgcttttca gtctcaatct ttgagaattg     120
taaattcatc aaataattgc ttacatagta aaaatttaag gtattagaaa acctgcataa     180
caaatagtat tatatattaa atattttgat atgtaaagct ctacacaaag ctaaatatag     240
tgtaataatg tttacactaa taagcaaata tgttaatctt ctcatttttt tactgtcata     300
taatcttagt gatatgccta ttaatagttt taaataaata aattggctca tctggctttt     360
tgaaaatttt gaaattctta cagatgttga ttaggtatat ctacaaatta atttcaattt     420
taaaatgatg atataaaaat aaatataagt atttttcttg tgtatgtata                470

<210>  401
<211>  488
<212>  DNA
<213>  human
<400>  401
agtaactttg tttatccctc aagcaaatcc tgatgacatt gatcctactc ctactcctac      60
tcctactcct gataaaagtc ataattctgg agttaatatt tctacgctgg tattgtctgt     120
gattgggtct gttgtaattg ttaactttat tttaagtacc accatttgaa ccttaacgaa     180
gaaaaaaatc ttcaagtaga cctagaagag agttttaaaa aacaaaacaa tgtaagtaaa     240
ggatatttct gaatcttaaa attcatccca tgtgtgatca taaactcata aaaataattt     300
taagatgtcg gaaaaggata ctttgattaa ataaaaacac tcatggatat gtaaaaactg     360
tcaagattaa aatttaatag tttcatttat ttgttatttt atttgtaaga aatagtgatg     420

aacaaagatc ctttttcata ctgatacctg gttgtatatt atttgatgca acagttttct 480
gaaatgat 488

<210> 402
<211> 407
<212> DNA
<213> human
<400> 402
ttccacttct attccacgga gaaaaaaatt gatttgtgtc acagaagttt ttatggaaac 60
tagcaccatg tctacagaaa ctgaaccatt tgttgaaaat aaagcagcat tcaagaatga 120

agctgctggg tttggaggtg tccccacggc tctgctagtg cttgctctcc tcttctttgg 180
tgctgcagct ggtcttggat tttgctatgt caaaaggtat gtgaaggcct tcccttttac 240
aaacaagaat cagcagaagg aaatgatcga aaccaaagta gtaaaggagg agaaggccaa 300
tgatagcaac cctaatgagg aatcaaagaa aactgataaa aacccagaag agtccaagag 360
tccaagcaaa actaccgtgc gatgcctgga agctgaagtt tagatga 407

<210> 403
<211> 120
<212> DNA
<213> human
<400> 403
ctcaactcgg ccatcacata tcgaattacc aaccactcac acttccggat ggagggagag 60
gttgtgctga ccaccaccac actggcacag gcgggagcct tctacgcaga ggttgaggcc 120

<210> 404
<211> 321
<212> DNA
<213> human
<400> 404
tccattacca agagctcatg ccacccggtt cctgcatgcc agaggagccc aagccaaaga 60
ggggaagacg atcgtggccc cggaaaagga ccgccaccca cacttgtgat tacgcgggct 120
gcggcaaaac ctacacaaag agttcccatc tcaaggcaca cctgcgaacc cacacaggtg 180
agaaacctta ccactgtgac tgggacggct gtggatggaa attcgcccgc tcagatgaac 240
tgaccaggca ctaccgtaaa cacacggggc accgcccgtt ccagtgccaa aaatgcgacc 300
gagcattttc caggtcggac c 321

<210> 405
<211> 531
<212> DNA
<213> human
<400> 405
aaaacccatt ctttcagtta catctactaa gagctcgtag ttcaataaca ttccaaagaa 60
cgtagtttgg aaaacactgg cctatcggct gtaataggaa gggtctaaag aaaattattt 120
gctgagtcct cataatcaat tggctataat cacaatttaa taatttatca atgcaaacag 180
tacattaaaa actgtagcac aatctataat ttttaccttc atatgcagag aaattaagaa 240
tttacatctt ttacaaatat gtgtatctct gaaataatga aatatattga tgggtgaaat 300
aatatttctg gtattttctt cattataaca cgtggaatgg gacagtggct agaggtatgg 360
atggagcaag gtcaaatatg agctgatgga gctgggcgct aaataagggt ataatatgct 420
acttctgtgt atgttgtgta tggtatagta tgctatttct gtgtatgttc aaattgttta 480
tgtatataat aaagttttaa aaatttgtat gtacttgaca attccttaga a 531

<210> 406
<211> 533
<212> DNA
<213> human
<400> 406
agaggagaca ctttggcgtt cttcaagcag aactgaggct gcgaaaaatc caagtctcta 60
cagagacact gatgaagttg aaagggtaat tgtttttcca tgccaaatga ggaaatcaaa 120
ttaatgagtt gacaaacttt tcctgagatg ttatttcatc tacatttagt taaacaactt 180
agaatgatat ctagaaaata tttattttca gccaggaact ttagcaaact gtggcaatga 240
tcactgggaa tgaaaacgta taatgttcta caatttttgt tatcacattg gatgactttg 300
aatatagtag tgacacttgg gaccaaataa attattttat atgactacta gaacaaagtt 360

ttagtaagct gtctgctaat agagtcagaa ttcttccaga ttgtttccag ctgtaacagg 420
tattgaattt ccaccgtgct cctatgtaac agctgggtgg tgtaaaaaat gaacttcaaa 480
ttatggagtg gaagaagcgt aatgttaata tcttgtaaat tcgtattccc tat 533

<210> 407
<211> 394
<212> DNA
<213> human
<400> 407
cattttccag gatctcagtg tgagtatcat tgatgcctgg gatataacaa ttgcatatgg 60
cacaaataat gtacacccac ctcaacatgt agtcggaaat cagattaata tattattaaa 120
ctatatttgt taaataacac aaaagtctga aattcattca cttaagtaaa aaaatttatt 180
gactgtctac tagcaggcca gatgctgtgt ttggctctga attcccaact agcaagagca 240
gagaatctac attatggctg atccataagc caccaattca gctaaatgag atgtttctaa 300
tctgggcttc cacttaaaaa taaaactaaa aatactcatg aaaagagcct acccttcttg 360
atcagagcca tacccctctt caccaatggg aagt 394

<210> 408
<211> 478
<212> DNA
<213> human
<400> 408
ggtattttag tctagtttta tatgaacggt tgtatcaggg taaccaactc gatttgggat 60
gaatcttagg gcaccaaaga ctaagacagt atctttaaga ttgctaggga aaagggccct 120
atgtgtcagg cctctgagcc caagccaagc atcgcatccc ctgtgatttg cacgtataca 180
tccagatggc ctaaagtaac tgaagatcca caaaagaagt aaaaatagcc ttaactgatg 240
acattccacc attgtgattt gttcctgccc caccctaact gatcaatgta ctttgtaatc 300
tcccccaccc ttaagaaggt actttgtaat cttccccacc cttaagaagg ttctttgtaa 360
ttctccccac ccttgagaat gtactttgtg agatccaccc tgcccacaaa acattgctct 420
taacttcacc gcctaaccca aaacctataa gaactaatga taatccatca cccttcgc 478

<210> 409
<211> 487
<212> DNA
<213> human
<400> 409
gctggccaag actactgggc cgtgctttct ggaaaaggca tttcagccac gctgatgatc 60
ttctccctct tggagttctt cgtagcttgt gccacagccc attttgccaa ccaagcaaac 120
accacaacca atatgtctgt cctggttatt ccaaatatgt atgaaagcaa ccctgtgaca 180
ccagcgtctt cttcagctcc tcccagatgc aacaactact cagctaatgc ccctaaatag 240
taaaagaaaa aggggtatca gtctaatctc atggagaaaa actacttgca aaaacttctt 300
aagaagatgt cttttattgt ctacaatgat ttctagtctt taaaaactgt gtttgagatt 360
tgtttttagg ttggtcgcta atgatggctg tatctccctt cactgtctct tcctacatta 420
ccactactac atgctggcaa aggtgaagga tcagaggact gaaaaatgat tctgcaactc 480
tcttaaa 487

<210> 410
<211> 313
<212> DNA
<213> human
<400> 410
agcccaccag caggagcttg gagtttgggg agtggggatg agtccgtcaa gcacaactgt 60
tctctgagtg gaaccaaaga agcaaggagc taggaccccc agtcctgccc cccaggagca 120
caagcagggt cccctcagtc aaggcagtgg gatgggcggc tgaggaacgg ggcaggcaag 180
gtcactgctc agtcacgtcc acgggggacg agccgtgggt tctgctgagt aggtggagct 240
cattgctttc tccaagcttg gaactgtttt gaaagataac acagagggaa agggagagcc 300
acctggtact tgt 313

<210> 411
<211> 325
<212> DNA
<213> human
<400> 411

gccactatct tgaagaaggt gcacagtgcc ctgctcctct ttcctatgtc cccagaattc 60
tcttagggtt ctcagatgcc atgactttca aggagagagt acggaaccac atcatgcact 120
tggaggaaca tttattttgc cagtattttt ccaaaaatgc cctagaaata gcctctgaaa 180
ttctccaaac acctgtcaca gcatatgatc tctacagcca cacatcaatt tggttgttgc 240
gaacagactt tgttttggac tatcccaaac ccgtgatgcc caatatgatc ttcattggtg 300
gtatcaactg ccatcaggga aagcc 325

<210> 412
<211> 284
<212> DNA
<213> human
<400> 412
ctttatcatc cccacaaaca ttttgaaact ggaatatttg tcttcagaaa atggaaacaa 60
gactataaat gataagccct gtccctagca ccacctctcc tgtgtgtgga atagaggccc 120
ctcgtgctac caacacttac cctgtgttta aaaagatctt gtaccaagcc aacggcgttc 180
ctggctctcc tgcccacagg atgaacattt tcggcttcct taggagtttt gccctaccgt 240
attccaaagc gtgtgctggt ttctcatatt gtctgtaggc tcac 284

<210> 413
<211> 539
<212> DNA
<213> human
<400> 413
gtgaggctca gtcagaacct ccaccctccc ccacaccaaa gacaggggca gcgtagtatt 60
caaaccagta ttgtggtggg gaataattgt atacatgtaa attatcaagc cctatgagtg 120
gaagaatttt ttcaaattat ttttgtctct ctatatattg atttatatta tgtataacta 180
tctctttata taaactatat ataattatat atatataact atataattat atatatataa 240
ctatatatat aactatatat atgtatcccc tagtattgga tcatgaagag ctcttcatgc 300
attctttgca aaggaggtta taaagttacg ccctcagaac atttataact ataagaatgt 360

gccagttaaa gtgctcaaca ggaaatatga cagtttaaaa gcattgtaaa actcacatag 420
cttacttctc tctctaaagt gcaacaagga tgaatagaat gggccaaggt atgacaatta 480
atggttctgc atgacctagc cactgctggg ggttttcttc tataacgttg tccttgtga 539

<210> 414
<211> 511
<212> DNA
<213> human
<400> 414
gggacttaac atttcacgtt gtatcttact tgcagtgaat gcaagggtta cttttctctg 60
gggacctccc ccatcaccca ggttcctact ctgggctccc gattcccatg gctcccaaac 120
catgccgcat ggtttggtta atgaaaccca gtagctaacc ccactgtgct tccacatgcc 180
tggcctaaaa tgggtgatat acaggtctta tatccccata tggaatttat ccatcaacca 240
cataaaaaca aacagtgcct tctgccctct gcccagatgt gtccagcacg ttctcaaagt 300
ttccacatta gcactcccta aggacgctgg gagcctgtca gtttatgatc tgacctaggt 360
cccccctttc ttctgtcccc tgtttttaag tccggatttt tacagaagga actgtctcca 420
gacagctcat caaggaacca agcaaaggcc agatagcctg acagataggc tagtggtaat 480
tgtgtatatg ggcgggacgt gtgtgtcatt a 511

<210> 415
<211> 316
<212> DNA
<213> human
<400> 415
cctctgtcct caaatgtcca aaatgttgga ggacctctgt tcatatccca cgcctgggct 60
cttgccagca gtggagttac tgtagaggga tgtcccaagc ttgttttcca atcagtgtta 120
agctgtttga aactctcctg tgtctgtgtt ttgtttgtgc gtgtgtgtga gagcacatca 180
gtgtgtgcag gctgtgtttc cccatttctc tcctcccttc agacccatca ttgagaacaa 240
atgtaagaaa tcccttccca ccaccctccc tgcctcccag gccctctgcg ggggaaacaa 300
gatcacccag catcct 316

<210> 416
<211> 149

<212>  DNA
<213>  human

<220>
<221>  misc_feature
<222>  (55)..(55)
<223>  n is a, c, g, or t

<400>  416
atattttgta tcatcgtgcc tatagccgct gccaccgtgt ataaatcctg gtgtntgctc     60
cttatcctgg acatgaatgt attgtacact gacgcgtccc cactcctgta cagctgcttt    120
gtttctttgc aatgcattgt atggcttta                                      149

<210>  417
<211>  523
<212>  DNA
<213>  human

<220>
<221>  misc_feature
<222>  (29)..(29)
<223>  n is a, c, g, or t

<400>  417
atccgacttg aatattcctg gacttacana atgccaaggg ggtgactgga agttgtggat     60
atcagggtat aaattatatc cgtgagttgg gggagggaag accagaattc ccttgaattg    120
tgtattgatg caatataagc ataaaagatc accttgtatt ctctttacct tctaaaagcc    180
attattatga tgttagaaga agaggaagaa attcaggtac agaaaacatg tttaaatagc    240
ctaaatgatg gtgcttggtg agtcttggtt ctaaaggtac caaacaagga agccaaagtt    300
ttcaaactgc tgcatacttt gacaaggaaa atctatattt gtcttccgat caacatttat    360
gacctaagtc aggtaatata cctggtttac ttctttagca tttttatgca gacagtctgt    420
tatgcactgt ggtttcagat gtgcaataat ttgtacaatg gtttattccc aagtatgcct    480
taagcagaac aaatgtgttt ttctatatag ttccttgcct taa                      523

<210>  418
<211>  576
<212>  DNA
<213>  human
<400>  418
ccaatcctga aggtactccc tgtttgctgc agaatgtcag atattttgga tgttgcataa     60
gagtcctatt tgccccagtt aattcaactt ttgtctgcct gttttgtgga ctggctggct    120
ctgttagaac tctgtccaaa aagtgcatgg aatataactt gtaaagcttc ccacaattga    180
caatatatat gcatgtgttt aaaccaaatc cagaaagctt aaacaataga gctgcataat    240
agtatttatt aaagaatcac aactgtaaac atgagaataa cttaaggatt ctagtttagt    300
tttttgtaat tgcaaattat atttttgctg ctgatatatt agaataattt ttaaatgtca    360
tcttgaaata gaaatatgta ttttaagcac tcacgcaaag gtaaatgaac acgttttaaa    420
tgtgtgtgtt gctaattttt tccataagaa ttgtaaacat tgaactgaac aaattaccta    480
taatggattt ggttaatgac ttatgagcaa gctggtttgg ccagacagta tacccaaact    540
tttatataat atacagaagg ctatcacact tgtgaa                              576

<210>  419
<211>  323
<212>  DNA
<213>  human
<400>  419
tcgactctgc tgctcatggg aagaacagaa ttgctcctgc atgcaactaa ttcaataaaa     60
ctgtcttgtg agctgatcgc ttggagggtc ctctttttat gttgagttgc tgcttcccgg    120
catgccttca ttttgctatg gggggcaggc aggggggatg gaaaataagt agaaacaaaa    180
aagcagtggc taagatggta tagggactgt cataccagtg aagaataaaa gggtgaagaa    240
taaaagggat atgatgacaa ggttgatcca cttcaagaat tgcttgcttt caggaagaga    300
gatgtgtttc aacaagccaa cta                                            323

<210>  420

<211>  448
<212>  DNA
<213>  human
<400>  420
aataacgcaa atggcttcct ctttcctttt tttggaccat ctcagtcttt atttgtgtaa      60
ttcattttga ggaaaaaaca actccatgta tttattcaag tgcattaaag tctacaatgg     120
aaaaaaagca gtgaagcatt agatgctggt aaaagctaga ggagacacaa tgagcttagt     180
acctccaatt tcctttcttt cctaccatgt aaccctgctt tgggaatatg gatgtaaaga     240
agtaacttgt gtctcatgaa aatcagtaca atcacacaag gaggatgaaa cgccggaaca     300
aaaatgaggt gtgtagaaca gggtcccaca ggtttgggga cattgagatc acttgtcttg     360
tggtggggag gctgctgagg ggtagcaggt ccatctccag cagctggtcc aacagtcgta     420
tcctggtgaa tgtctgttca gctcttct                                        448

<210>  421
<211>  477
<212>  DNA
<213>  human
<400>  421
tcaaatggaa cctgccctct aaagcacttt ctttccttta cttgcgtggt ttcatgtaag      60
ctgtgctgtt tagaaacaac atctcagact ttacaaagaa atgacaaaga aggcaattgc     120
actttttaag ggatatcgac aagcagtttc tgttttctaa aggacaaaat acagagtgtg     180
tgtcattttt aattagattc tttcccctgc tgagttggaa attccagtgc agcactgatt     240
gaccacagtt gccaatctaa aagcacaaag acagaagtaa agctttatgc taattttatt     300
tcaatatgat agaaaattta tcttggtatg tcctttttta gataactcca gcaggaaact     360
gtaactgcta tgtctttagg aaaacgtaga agaaagaaca ttattattct ttaattccta     420
caaggtactt gaaaacctta agtgaaaaag atttctatct ttttatcttg gcgcatt        477

<210>  422
<211>  577
<212>  DNA
<213>  human
<400>  422
tgccactaat tcattcacac taaggtgtaa atgattgata ataggaatga gttacctctt      60
cccacagaca tttgttttta agtatgacag agcagggcct taatcccaag ggaaaaggtt     120
atggaactgg agggggtgag ctttctgggt agaaggagac ttcctgaatt tccttaaaac     180
ccagtaagag taagacctgt tgttttggaa ggtctgctcc accatctaag agcactgttt     240
tttttttttt gttgttgttg ttgttttacg gtctctgagg gaatatagta aaaatgcata     300
tgcacgtgca atttgcacgg cagcatttca ccgattgtgg actgtattgg ctaatgtgtt     360
tcctggtctt tagatgcaaa ccattaataa cactatctta tctcatagtt ttttcagggg     420
tgcttcttga ttagtaggga attttgaaca cctctttaaa tacagctaga aaataaaacc     480
aatttgtaaa gccacatttg catatgatgc cagcctcacg catttgtata tctccagaaa     540
ttcaggtatg cctcaccaat ttgcccgtct ttaataa                              577

<210>  423
<211>  544
<212>  DNA
<213>  human

<220>
<221>  misc_feature
<222>  (46)..(46)
<223>  n is a, c, g, or t
<220>
<221>  misc_feature
<222>  (64)..(64)
<223>  n is a, c, g, or t
<220>
<221>  misc_feature
<222>  (66)..(66)
<223>  n is a, c, g, or t
<220>
<221>  misc_feature
<222>  (97)..(97)

<223>  n is a, c, g, or t

<400>  423
taaactaacc cttacattcc atgtgatgtg atgtaggctt ataagntgct aaaatctata      60
ggtntnggaa gtgaaagatt ctattttttc ttttttngta cataatggga atttcattcc     120
agattatatt ttatttacat attaatttca cagaatatta atatttctta acttcttaaa     180
gcatgctagc gttttatgta tatgtacaca tatattcaga cagggtaatt ttatctgctg     240
cctaacattg tactaaaata ttgctttatc tgtttttaat tacaaaatgc taatgatttc     300
ttaaattata gtttaaagac aattggccca ggaagcaaat cccctgcctt tagtatgaac     360
cactataagt aaccttacaa atagagttaa tccaagacaa tattaacaaa ctgtgctttg     420
tctttaataa aagggatagg attaacaaac atattgatgg cataacctat tcagctatgt     480
ccttattttt gcaataatgt aacctcaaat attggattgt tgaaccaaca atggctgtgt     540
taaa                                                                 544

<210>  424
<211>  512
<212>  DNA
<213>  human

<220>
<221>  misc_feature
<222>  (79)..(79)
<223>  n is a, c, g, or t

<400>  424
tttatgatga ctcagtggtg ccagagtaaa gtttctaaaa taacattcct ctcacttgta      60
ccccactaaa agtattagnc tacacattac attgaagtta aacacaaaat tatcagtgtt     120
ttagaaacat gagtccggac tgtgtaagta aaagtacaaa cattatttcc accataaagt     180
atgtattgaa atcaagttgt ctctgtgtac agaatacata cttattccca tttttaagca     240
tttgcttctg ttttccctac ctagaatgtc agatgttttt cagttatctc cccatttgtc     300
aaagttgacc tcaagataac atttttcatt aaagcatctg agatctaaga acacaattat     360
tattctaaca atgattatta gctcattcac ttattttgat aactaatgat cacagctatt     420
atactacttt ctcgttattt tgtgtgcatg cctcatttcc ctgacttaaa cctcactgag     480
agcgcaaaat gcagctttat actttttact tt                                  512

<210>  425
<211>  517
<212>  DNA
<213>  human
<400>  425
gataacctac atcaaccgag ataccaaaat catcctggag accaagagca agaccattta      60
caagctgaac ggtgtgtccg aaagggacct gaagaaatcg gtgctgtggc tcaaagacag     120
cttgcagtgc acctgtgagg agatgaacga catcaacgcg ccctatctgg tcatggacaa     180
gaaacagggt ggggagctgg tgatcacctc ggtgaagcgg tggcagaagg ggcagagaga     240
gttcaagcgc atctcccgca gcatccgcaa gctgcagtgc tagtcccggc atcctgatgg     300
ctccgacagg cctgctccag agcacggctg accatttctg ctccgggatc tcagctcccg     360
ttccccaagc acactcctag ctgctccagt ctcagcctgg gcagcttccc cctgcctttt     420
gcacgtttgc atccccagca tttcctgagt tataaggcca caggagtgga tagctgtttt     480
cacctaaagg aaaagcccac ccgaatcttg tagaaat                             517

<210>  426
<211>  441
<212>  DNA
<213>  human
<400>  426
ggtgcaactg tgacttgggt ctggttggtt gttgtttgtt gttttgagtc agctgatttt      60
cacttcccac tgaggttgtc ataacatgca aattgcttca attttctctg tggcccaaac     120
ttgtgggtca caaaccctgt tgagataaag ctggctgtta tctcaacatc ttcatcagct     180
ccagactgag actcagtgtc taagtcttac aacaattcat cattttatac cttcaatggg     240
aacttaaact gttacatgta tcacattcca gctacaatac ttccatttat tagaagcaca     300
ttaaccattt ctatagcatg atttcttcaa gtaaaaggca aaagatataa attttataat     360
tgacttgagt actttaagcc ttgtttaaaa catttcttac ttaacttttg caaattaaac     420
ccattgtagc ttacctgtaa t                                              441

<210>  427
<211>  377
<212>  DNA
<213>  human
<400>  427
agggtacgag aacttgccaa tgggaaattc atccgagtgg cactggcaga gaaggatagg      60
agtggaatgc ccacacagtg accaacagaa ctggtctgcg tgcataacca gctgccaccc     120
tcaggcctgg gccccagagc tcagggcacc cagtgtctta aggaaccatt tggaggacag     180
tctgagagca ggaacttcaa gctgtgattc tatctcggct cagacttttg gttggaaaaa     240
gatcttcatg gccccaaatc ccctgagaca tgccttgtag aatgattttg tgatgttgtg     300
atgcttgtgg agcatcgcgt aaggcttctt gcttatttaa actgtgcaag gtaaaaatca     360
agcctttgga gccacag                                                    377

<210>  428
<211>  521
<212>  DNA
<213>  human

<220>
<221>  misc_feature
<222>  (96)..(96)
<223>  n is a, c, g, or t
<220>
<221>  misc_feature
<222>  (102)..(102)
<223>  n is a, c, g, or t
<220>
<221>  misc_feature
<222>  (354)..(354)
<223>  n is a, c, g, or t

<400>  428
gagtccaaat gtcatcagtg ctcattttga gataccctgc tatcgatggt cgctacaaac      60
caggaaatac tcaagttatt atgtgtatac attggnttta gntttatgaa acaatttacc     120
ttcatgatct catagttaaa attgtaataa atttaggaat ataaaggatc aatatgggaa     180
gcaaaatttc taaaggcagt ttctgttgtt ttaattagta tttgtgtagt tcaaaccagg     240
aaggatttga ctatcattag attttgctta actttatgaa agctaaaata ttctctgtta     300
taaaggggca actccatctg gtcctatagc atctttacta ctgatttttt tttngtttaa     360
tttgaaaatg caaagaattg ttaaatgttc ttaaatgttc tcactacaaa aaaagaaaaa     420
agataactac gtgaggtgat ggatatgtta attagctgga ttgtggtaat cattttggaa      480
tgtatatgta tatcaaaaca tgtagtacac cctaaatata t                         521

<210>  429
<211>  423
<212>  DNA
<213>  human
<400>  429
agatacattt ttacccacca taaatgttac aatatctgaa tatgctttgt caaactatcc      60
ctttatgcaa tcgtcttcat attgttttta tgattctaat caagctgtat gtagagactg     120
aatgtgaagt caagtctgag cacaaaaaga taatgcacga tgagattgcc taccatttta     180
taggatattt actatgtatt tatacgttaa gacctctatg aatgaatgta tcagagaatg     240
tctttgtaac tgtttaattc aatctgtaat aaaaatctaa ctaactaact catttatttc     300
tattaaaaag gtattgtcct ttaggcgggg aatgggaatc cttgctgcac tgttgcagtc     360
attctgaaag gacctttccc tgtacttacc tttcaacatg cttcaatctt atcaacgcta     420
cat                                                                   423

<210>  430
<211>  465
<212>  DNA
<213>  human
<400>  430
gaaaaccgat gtgatccttg atcgaaaaaa aaatccagaa ccttgggaaa ctgtggaccc      60

tactgtacct caaaagctta taacaatcaa ccaacaatgg aaacccattg aagagttgca 120
aaatgtccaa agggtgacca aatgacgagc cctcgcctct ttcttctgaa gagtactcta 180
taaatctagt ggaaacattt ctgcacaaac tagattctgg acaccagtgt gcggaaatgc 240
ttctgctaca tttttagggt ttgtctacat tttttgggct ctggataagg aattaaagga 300
gtgcagcaat aactgcactg tctaaaagtt tgtgcttatt ttcttgtaaa tttgaatatt 360
gcatattgaa atttttgttt atgatctatg aatgtttttc ttaaaattta caaagctttg 420
taaattagat tttctttaat aaaatgccat ttgtgcaaga tttct 465

<210> 431
<211> 548
<212> DNA
<213> human
<400> 431
aattgtgttg tgatgctact cactttgatt gcaatgatga tgtccaaggt aagctattaa 60
aaggcaggtt acttccaaat cgcactgaag gaaaaggtta agaataatac atgatcacag 120
aaatgcatac cactgtctgt aaacccaaca aaattcactg ttctcttttg gatttattta 180
gcctgatgta tttttaattc aatttttatg gtgatgggca aatcattctt ggtaaatgta 240
aatcaaacat gattgattta aaacttcatg gaatttgtag aaaattatgg acatttttgg 300
tgagaaagaa caatagtcaa aactcacatg gatagagtgt gtttgttttt tgccaaaaat 360
gccccagacc ttttcccaaa cctcaaaaac gtcttggaaa aattgtaaaa gtttgataac 420
agaaacatct ttaggatatt tttgtctgac gtattttgct tctagtatgt gcctactgtg 480
atttttttca tgtggaaaat gcaaaatttg taacaaaatg gttatatgga acatgcctat 540
taaacgaa 548

<210> 432
<211> 494
<212> DNA
<213> human
<400> 432
ggcatctggc acgtgcccaa taagtccccc atgcagcact ggagaaacag ctccctgctg 60
aggtaccgca cggacactgg cttcctccag acactgggac ataatctgtt tggcatctac 120
cagaaatatc cagtgaaata tggagaagga aagtgttgga ctgacaacgg cccggtgatc 180
cctgtggtct atgatttggg cgacgcccag aaaacagcat cttattactc accctatggc 240
cagcgggaat tcactgcggg atttgttcag ttcagggtat ttaataacga gagagcagcc 300
aacgccttgt gtgctggaat gagggtcacc ggatgtaaca ctgagcacca ctgcattggt 360
ggaggaggat actttccaga ggccagtccc cagcagtgtg gagatttttc tggttttgat 420
tggagtggat atggaactca tgttggttac agcagcagcc gtgagataac tgaggcagct 480
gtgcttctat tcta 494

<210> 433
<211> 443
<212> DNA
<213> human
<400> 433
gagaagcacc tgttccaact aagactaaag tggccgttga tgagaataaa gccaaagaat 60
tccttggcag cctgaagcgc cagaagcggc agctgtggga ccggactcgg cccgaggtgc 120
agcagtggta ccagcagttt ctctacatgg gctttgacga agcgaaattt gaagatgaca 180
tcacctattg gcttaacaga gatcgaaatg gacatgaata ctatggcgat tactaccaac 240
gtcactatga tgaagactct gcaattggtc cccggagccc ctacggcttt aggcatggag 300
ccagcgtcaa ctacgatgac tactaaccat gacttgccac acgctgtaca agaagcaaat 360
agcgattctc ttcatgtatc tcctaatgcc ttacactact tggtttctga tttgctctat 420
ttcagcagat ctttctacct act 443

<210> 434
<211> 507
<212> DNA
<213> human
<400> 434
agctgccggg caacgtgttg tgtaagtgaa catctgggag gtaaacacta cacgtgaaga 60
gtggtgaaag ggaacattga ttactgaagt gccctggaga gggaaagcac tggtcaacat 120
cacatggaca aatttcattg ttttctaaag atggcctgga agtagtcttt gccactgctt 180
cctccacaaa cagctcttca taacatgggc tgcatgaaat caaagcaaac tttcccattt 240
cctaccatat atgaaggtga gaagcagcat gagagtgaag aaccctttat gccagaagag 300

agatgtctac ctaggatggc ttctccagtt aatgtcaaag aggaagtgaa ggaacctcca 360
gggaccaata ttgtgatctt ggaatatgca caccgcctgt ctcaggatat cttgtgtgat 420
gccttgcagc aatgggcatg caataacatc aagtaccatg acattccata cattgagagt 480
gaggggcctt gaggctgtag gatgaca 507

<210> 435
<211> 392
<212> DNA
<213> human
<400> 435
   ggggctatac tccatccaaa tatgcagtgg aaggtttcaa tgacagctta agacgggaca 60
   tgaaagcttt tggtgtgcac gtctcatgca ttgaacgtct agacaaactg aaaggcaata 120
   aatcctatgt gaacatggac ctctctccgg tggtagagtg catggaccac gctctaacaa 180
   gtctcttccc taagactcat tatgccgctg gaaaagatgc caaaattttc tggatacctc 240
   tgtctcacat gccagcagct ttgcaagact ttttattgtt gaaacagaaa gcagagctgg 300
   ctaatcccaa ggcagtgtga ctcagctaac cacaaatgtc tcctccaggc tatgaaattg 360
   gccgatttca agaacacatc tccttttcaa cc 392

<210> 436
<211> 512
<212> DNA
<213> human
<400> 436
   ggggctatac tccatccaaa tatgcagtgg aaggtttcaa tgacagctta agacgggacc 60
   tgaaagcttt tggtgtgcac gtctcatgca ttgaaccagg attgttcaaa acaaacttgg 120
   cagatccagt aaaggtaatt gaaaaaaaac tcgccatttg ggagcagctg tctccagaca 180
   tcaaacaaca atatggagaa ggttacattg aaaaaagtct agacaaactg aaaggcaata 240
   aatcctatgt gaacatggac ctctctccgg tggtagagtg catggaccac gctctaacaa 300
   gtctcttccc taagactcat tatgccgctg gaaaagatgc caaaattttc tggatacctc 360
   tgtctcacat gccagcagct ttgcaagact ttttattgtt gaaacagaaa gcagagctgg 420
   ctaatcccaa ggcagtgtga ctcagctaac cacaaatgtc tcctccaggc tatgaaattg 480
   gccgatttca agaacacatc tccttttcaa cc 512

<210> 437
<211> 262
<212> DNA
<213> human
<400> 437
tatctgcggc cccaggacag aaggtcgcca tctcctgctc tggaagcagc tccaacattg 60
ggaataatta tgtatcctgg taccaacaac tcccaggaac agcccccaaa ctcctcattt 120
atgacaataa taagcgaccc tcagggattc ctggccgatt ctctggctcc aagtctggca 180
cgtcagccac cctgggcatc accggactcc agactgggga cgaggccgat tattactgcg 240
gaacatggga tagcagcctg ag 262

<210> 438
<211> 544
<212> DNA
<213> human
<400> 438
ttttatttca ggcttccagc tgtccctgtg agttatcctg gacatttcga tggtttttgg 60
taaggccaaa ctctgataag caaaacagag aatactgacg gtatacttaa ccatatgtgt 120
aactgatact tggcaccatg gaatttttca ttgagttatt tcctcattct tttaaaaaat 180
aagggactat aaatcagtta tttagtatct tttgtttttg tagctgattc cttaactttc 240
ttgtatgcct ctagtaattt cagagattaa atattgcttt aaactgtgat actttgattt 300
gctagattga caaaactgat actaatataa ttaagttcat ctttgaaata catctttgtg 360
cgtagagcca aaaaaagaga taaaattaat aatagttcac ttgttatttg agattaattt 420
ggcatttgaa atgatcattt tattttacaa tcatttataa tgaatcaatg ttccagttag 480
ctttaaaagg tatacggtgc taattagtaa aatattgaag gcaatatttt actgctagct 540
tgca 544

<210> 439
<211> 380
<212> DNA

<213>  human
<400>  439
taatcctgac attagctgac ttgctagtga gcttgcttta aaaatctaca ctcttgcatt     60
cttaggcata caggggaaat gttgaaaagg aaggtggaaa accaagaatt tagtttgcca    120
atgattgcct ctgattcttg taagtttgag ttccacaagg gctaatttat tcccctttta    180
cttgggtttt ggggtggtgg aaagcgggaa atttgggtga tttgttgatt ggcaatgagg    240
ataaaatgtt aatacttttt tggggactta acaactttat cctattctac aagtcagtaa    300
aggaacaatt ggtactcacc tcagtgctgc actcaactat ggaaagaggc agagtttgct    360
tgcccaattg ccaaactaaa                                                380

<210>  440
<211>  529
<212>  DNA
<213>  human
<400>  440
attagtgtca ttagtgtgtt ggaagagaaa tactattcag taagcttcgc caaagaaaag     60
tgagtcaaag ttaatgtgtg tgtgcattta tatgtaggca gctcgtagac cacattttag    120
ccagcaactg gtaacaaaga gcttagtttt ccttgtttga atgctgtaga tctgtaccta    180
gtacccctcc catctactga tttgtttgtt tttgtaacca aacacatttt cagatagaag    240
gagccttaaa aaaaaaaaaa tcacattgag taacttcagt atgaatgaat gagagtgtgt    300
ggagctaccc ctcaccctcc acccctttgt gctttttatt cccgaatttt cccagtctct    360
taaacagaaa aatgactgat ataattatct tttggaaact gagccttaat tttttttaga    420
gggggaaata agttttcccc aactcacaca gcataagcaa tgtttgacag caatataatg    480
ccgttgtaaa ctactgagag tattgtatct gttctggtaa ccatgtaca                529

<210>  441
<211>  389
<212>  DNA
<213>  human

<220>
<221>  misc_feature
<222>  (74)..(74)
<223>  n is a, c, g, or t
<220>
<221>  misc_feature
<222>  (181)..(181)
<223>  n is a, c, g, or t

<400>  441
ttgtttttga catcagctgt aatcattcct gtgctgtgtt ttttattacc cttggtaggt     60
attagacttg cacntttttt aaaaaaaggt ttctgcatcg tggaagcatt tgacccagag    120
tggaacgcgt ggcctatgca ggtggattcc ttcaggtctt tcctttggtt ctttgagcat    180
nctttgcttt cattcgtctc ccgtctttgg ttctccagtt caaattattg caaagtaaag    240
gatctttgag taggttcggt ctgaaaggtg tggcctttat atttgatcca cacacgttgg    300
tcttttaacc gtgctgagca gaaaacaaaa caggttaaga agagccgggt ggcagctgac    360
agaggaagcc gctcaaatac cttcacaat                                      389

<210>  442
<211>  450
<212>  DNA
<213>  human
<400>  442
ggtcagatgg tcaacttttt tcagtattat ttatagttgg cacttgattg cagttctgtg     60
aggcttgagc attcatacac ctcacctgcc ttggcaagcc tattttagtg atatggcagc    120
acggatataa cactatgcat taaaagcact ttttgtaata agtttaatat cctaaaagga    180
atgccaatta agttttgtta actgtgtcat caacttatcc tagtacctca gtgttcattc    240
ctgttacctg catatcttct taaaagaaat agctgttatt aatgcctttt tgttttccat    300
tgagtgtaca ctactgaata agtgtaggag ttttatgttt accatgtgag tcctgcaaca    360
ctaaagatat tttgaatatc agtcatgatg gcaatttctg tataaaagag ccttaaatgg    420
aacattgttt tgagatcaaa ctccccaccc                                     450

<210>  443

<211>  496
<212>  DNA
<213>  human
<400>  443
gatatctggc catgggtaac ctcattgtaa ctatcatcag aatgggcaga gatgatcttg      60
aagtgtcaca tacactaaag tccaaacact atgtcagatg ggggtaaaat ccattaaaga     120
acaggaaaaa ataattataa gatgataagc aaatgtttca gcccaatgtc aacccagtta     180
aaaaaaaaat taatgctgtg taaaatggtt gaattagttt gcaaactata taaagacata     240
tgcagtaaaa agtctgttaa tgcacatcct gtgggaatgg agtgttctaa ccaattgcct     300
tttcttgtta tctgagctct cctatattat catactcaga taaccaaatt aaaagaatta     360
gaatatgatt tttaatacac ttaacattaa actcttctaa ctttcttctt tctgtgataa     420
ttcagaagat agttatggat cttcaatgcc tctgagtcat tgttataaaa aatcagttat     480
cactatacca tgctat                                                     496

<210>  444
<211>  538
<212>  DNA
<213>  human
<400>  444
gacctaatac atttcctctg tgtgtgtgtg taacattcca aatacttttt ttttcttttc      60
cactgtttgt aaggtgcaac aatttaatat ttttaaggga ctttttaaga gttccttaag     120
aaccaattta aaattacttc agtgcaatcc tacacagtat caacattaga attttgatat     180
tagtcttatg ttatcttcca ttctattttt atctgctttt tgctgctagt ttcaaactgc     240
cagtattttt ccttttgctt ttaaaatagt tacaatattt ttcatgatag ccacagtatt     300
gccacagttt attataataa agggttttta tttgatttag cgcattcaaa gcttttttct     360
atcacttttg tgttcagaat ataacctttg tgtgcgtgta tgttgtgtgt gtgcatgtgt     420
ggcgtatatg tgtgttacag gttaatgcct tcttggaatt gtgttaatgt tctcttggtt     480
tattatgcca tcagaatggt aaatgagaac actacaactg tagtcagctc acaatttt       538

<210>  445
<211>  474
<212>  DNA
<213>  human
<400>  445
gagtgagtcc cagttacatc aaacagtgac ttccagttat tccccagtaa gtctgagtgg      60
ttccttcaag ctgggtgtct ttccagcctt tgccagtcta gccccagcag ggcaccgtgt     120
atgaatgcag tttggtgctg ttttagagta tgcctgctcc ccagccccct gcctggaacc     180
ctctgagcaa cttgctctga cctataatgt cttaggtgca acacggaccc caccagagct     240
cttggatacc cccctagatc catgtggctt tatgtgaggg gactgaatgc agacacacca     300
tagccccctt ctactacttt ccctctcgcc ctgccaccta gttccacatg gaaccaacaa     360
gttgagtgca tccctgttgg gtgttttgtg ttgagactgg ctgaaatgag gagactttga     420
ccatgtgacg tgtcaacaga ctcaaggaga caaccacctc aactgggtca tgtg           474

<210>  446
<211>  414
<212>  DNA
<213>  human
<400>  446
ccccagtctc acctacgaaa tatgaagagc aaaagctgat tttgcttact tgctaaactg      60
ttgggaaagc tctgtagagc atggttccag tgaggccaag attgaaattt gatactaaaa     120
aggccaccta gctttttgca gataacaaac aagaaagcta ttccaagact cagatgatgc     180
cagctgtctc ccacgtgtgt attatggttc accaggggga actggcaaaa gtgtgtgtgg     240
ggaggggaag ggtgtgtgag tggttctgag caaataacta cagggtgccc attaccactc     300

aagaagacac ttcacgtatt cttgtatcaa attcaataat cttaaacaat ttgtgtagaa     360
gtccacagac atctttcaac caccttttag gctgcatatg gattgccaag tcag           414

<210>  447
<211>  524
<212>  DNA
<213>  human
<400>  447
tcatgcgtca tccctaaaat aataagatac atgggatcaa atagcccttg ccttttcaac      60

acaaatcagt tggaaaatta tggtttgagt cctgttgctg ccatggcttc tgtttctcag 120
aaatgagtgt gtatgaacat accaatctat gtaataggct accttttttt gtcttctttg 180
gaactttgta cacaaaccaa gacaatatca gggtgacagg tgaatgaact taaattctca 240
gtcttgtcta ttcaccaaaa aagtatactg cctgtttttt ctttaattat tcaaggttga 300
tgacttttag gaacatgttt tatactgtat tttttaatta aagcaagtgc cttgatgtaa 360
ttccatgtaa atcattgctt aaccctctta tgggatgagg atgagttatt aatgtattgc 420
agcctactgg aaaggagggg gagttggtta atagcagata cttttcttct agaagcttat 480
gttttatgct gtttattatg taagatcctg tatgtgtgtt gaga 524

<210> 448
<211> 426
<212> DNA
<213> human
<400> 448
ataatgatcc attcgagttc tgtgatcctt attgttctta attgtgtttc tctacgtatt 60
gttacagatg agccatacgt ttctttgtat caatgtagac atgacttcag atacctctga 120
ggacctaccc agcagtctag gaccctgggc caagtgctgg gactatggta ctaaatccag 180
tagatgggct gtgtagcaac tctcccaggg aacacactag ggtacttagg gaggtgcttt 240
gtggagcatg ttgaagcttt gagatctgag caggaggcag tgatgtccct ggtctattca 300
gggaaagatt tcagtgtgaa atggtaaaca tccaattgac aggatttaga ttttgcttag 360
tttttctgct tttaatgttt tctatccccc atctcagtgt tttctttatc catcccagtg 420
atgcct 426

<210> 449
<211> 470
<212> DNA
<213> human
<400> 449
ataattttac atgatcctca atatcaactc cagtttaaaa agtgttattt ttaaaacatt 60
tgaaaccaag tactgtttaa tttcaatcag aagatgcaaa tacatacttt gatctatgtt 120
tgattttgct aataatattt gaaggagatt gcctaccaag gacaaaacaa taaatttaaa 180
aatcaaacga tttctccata cgctcatagt cacatatgga attttgagaa aataaagcat 240
gctgtcttta ggaattttta tacttctttg tctttcttcc ttaatatttg cttctagctg 300
ctcttggcaa tgatgaattg ttatgtatgc attaatgttt tgcagcccaa aagttgttca 360
catttttcct atataagatc tgtggagtgt gtgtttcaaa gagagaacta cagaaatgtt 420
aaagcaggaa aacctgaatg tgatgtgcac attttcatcc cacatggaca 470

<210> 450
<211> 404
<212> DNA
<213> human
<400> 450
catttctgtc acatgcactt agttgacatt acatctacat atattagctt tttcctacat 60
gagccatcta tttacttagt aaccagtgtt cttaatgaag tatttagtct tgggtttctt 120
gtaaaatttc tctgcattcc ttagacagtg tactatacat gaaatattct tgttgaccta 180
gtaatttata ttattccatt taattcttaa acctatggcc tttttattga gcacactctt 240
aaatcattat ttggcttgta aacattcatc tgaattgtgg ctacaatcct ctttaaataa 300
tctaggaaaa aagaaagata aagcttacat tttcacagtt ttggctctta aacacattcc 360
acaaatgcca ttaagaattt attttgtttt aggccagtca tggt 404

<210> 451
<211> 522
<212> DNA
<213> human

<220>
<221> misc_feature
<222> (388)..(388)
<223> n is a, c, g, or t

<400> 451
gagcttctag aaatttcact tgcaagttta tttttgcttc ctgtgttact gccattccta 60
tttacagtat atttgagtga atgattatat ttttaaaaag ttacatgggg cttttttggt 120

tgtcctaaac ttacaaacat tccactcatt ctgtttgtaa ctgtgattat aatttttgtg 180
ataatttctg gcctgattga aggaaatttg agaggtctgc atttatatat tttaaataga 240
tttgataggt ttttaaattg ctttttttca taaggtattt ataaagttat ttggggttgt 300
ctgggattgt gtgaaagaaa attagaacca cgctgtattt acatttacct tggtagttta 360
tttgtggatg gcagttttct gtagtttngg ggactgtggt agctcttgga ttgttttgca 420
aattacagct gaaatctgtg tcatggatta aactggctta tgtggctaga ataggaagag 480
agaaaaaatg aaatggttgt ttactaattt tatactccca tt 522

<210> 452
<211> 500
<212> DNA
<213> human

<220>
<221> misc_feature
<222> (299)..(299)
<223> n is a, c, g, or t
<220>
<221> misc_feature
<222> (456)..(456)
<223> n is a, c, g, or t

<400> 452
ttgtgtgtaa tttcatggtg gcctagtgtt gtggtgcttc tggtaatggt aatagaagct 60
caactatttt tttgtggatt tcagttttta tcatcagaag tcctagacag tgacatttct 120
taatggtggg agtccagctc atgcatttct gattatacaa aacagtttgc agtaggttat 180
ttgtcatttc agttttttac tgaaatttga gctaaacatt tttacatgta aatacttgta 240
tttaccaaag atttaaatca gttgattaat taattaactc aaatactgtg aactatctnt 300
aaaacactag aaaaaagaaa tgttagtatc tcaattacac caactgtgca aatgaacttt 360
gataaaatag aaataatcta cattggcctt tgtgaaatct ggggaagagc tttaggattc 420
tagtagatgg atactgaata ctcaggccca cttaanttat taatgtatac attgtgtttt 480
tgtctttatg ctatgtacag 500

<210> 453
<211> 512
<212> DNA
<213> human

<220>
<221> misc_feature
<222> (447)..(448)
<223> n is a, c, g, or t
<220>
<221> misc_feature
<222> (450)..(455)
<223> n is a, c, g, or t

<400> 453
ggcgtgcaat tttggtctgc gccacataac cattctgaag cttttaggcg ttggagagga 60
agttggggga gtgttagaac tgttcccaat taatgggagc tctgttgttg agcgagaaga 120
cgtaccagcc catttggtga aagacattcg taactatttt caagtgagcc cggagtactt 180
ctccatgctt ctagtcggaa aagacggaaa tgtcaaatcc tggtatcctt ccccaatgtg 240
gtccatggtg attgtgtacg atttaattga ttcgatgcaa cttcggagac aggaaatggc 300
gattcagcag tcactgggga tgcgctgccc agaagatgag tatgcaggct atggttacca 360
tagttaccac caaggatacc aggatggtta ccaggatgac taccgtcatc atgagagtta 420
tcaccatgga tacccttact gagcagnnan nnnnnacctt agactcagcc agtttcctct 480
gcagctgcta aaactacatg tggccagctc ca 512

<210> 454
<211> 565
<212> DNA
<213> human
<400> 454

aactgtgtac ttgtctggtc agctgtgtat gatcagttat ctacctcaga gtctattttc 60
ttttgtgctg ggacaggttg ctggccctcc ctgtttccac agaccaaatc ctcctagctc 120
aggagctagg gctaagcagt tatttctttc aagtattttt tagttcttaa attttatgct 180
tgtatttgat gatagatgtc agtgacattt catagtttca aaagtccttg ctgctctgag 240
aagtgtagat tctagtgaaa attacatagt cataagagaa atgtgttttt gtttttgttt 300
ttgtttcatt tttttaaagt tgtggtatta ttggttctat gctccctgga atattactgc 360
tttgtgaaag tccagactga acgcagcacc ctctgtgtac ctagtacagt tataaacctg 420
ggtctctcac tacttgatat ttttgcatta gttaagacag aaatttgata gctcggttag 480
aggggagggg aaatctgctg ctagaaatgt ctgaactaag tgccatactc gtctgggtaa 540
gatttgggaa acataacctc tgtac 565

<210> 455
<211> 575
<212> DNA
<213> human
<400> 455
gatgagagca aaggggtcgc accatatgga aatgttgaaa actattgtaa agtagtatta 60
tgaagtagct tttgtgtcat tcatgtcgat gacatgaaag tgaagtaaat ttattctatg 120
taaattcaca ctaaaaccag tacagtacca taagtagaat acatgtaaga atcacctagt 180
cttcactata ttgagtaaat ataacatgct aattttacaa ttaatgaaac taaactttta 240
aacatctcca ttatatctac atccttttga aggtatttat catagttgcc aattttaatt 300
ttaggattga ctttctcttt ctgaatgact tcataaagtt tggtgtgaat tttgaagact 360
tgggttacta atgattgtat ctttgctagt caacaactta tgaaatatac tcaatgcgtc 420
tgatgtgtca ttaagtgcag aaataactaa gacacaaata acctttgcaa accttcaagc 480
tgtgtaatat tccaatgttg tttttttctt tgtatatata cttatatcac gtaggatgta 540
aaaccagtat gaccttgtct agtctccaaa cttaa 575

<210> 456
<211> 411
<212> DNA
<213> human

<220>
<221> misc_feature
<222> (179)..(179)
<223> n is a, c, g, or t
<220>
<221> misc_feature
<222> (343)..(343)
<223> n is a, c, g, or t

<400> 456
gccacacgtc caaaaccaat caggtcaact cgggcggtgt gctgctgagg ttgcaggtgg 60
gcgaggaggt gtggctggct gtcaatgact actacgacat ggtgggcatc cagggctctg 120
acagcgtctt ctccggcttc ctgctcttcc ccgactaggg cgggcagatg cgctcgagnc 180
ccacgggcct tccacctccc tcagcttcct gcatggaccc accttactgg ccagtctgca 240
tccttgccta gaccattctc cccaccagat ggacttctcc tccagggagc ccaccctgac 300
ccacccccac tgcaccccct ccccatgggt tctctccttc ctntgaactt ctttaggagt 360
cactgcttgt gtggttcctg ggacacttaa ccaatgcctt ctggtactgc c 411

<210> 457
<211> 550
<212> DNA
<213> human
<400> 457
gtatgtttgt aagtgcttct gcacgaatgt ttatacatga ctgtttccat agtacttatg 60
tttttaaaaa tattcagtca tttcctacta taatcctcat gtatccatgt aactgactca 120
aaaatacttc agccacagaa agctaaaact gagcaaatct cattcttctt ttccatcccc 180
tttgcatgtg gctggcattt agtaatgatt aataatatgg ccagctgaat aacagaggtt 240
tgagacacaa ttctttctca aaggagtcag ctaagctggg tctacttatg gacaaacatc 300
taaatgtgtg gaagtatctg atatttgaca atggtaaatt tccacttagc tagctagcat 360
tgtcagactt caatctcctc atggctctgg ccgtcctgtt ttaagcatga taattgttgg 420
ccacatctca catagttctc attgagtgag ttcataaata aacagggttt tttttttttt 480

ttaaagagca gccaagcaca aagtgtgact ttgttgacat tttatgtgac tttgtcatat 540
gttcctaacc 550

<210> 458
<211> 353
<212> DNA
<213> human
<400> 458
agtgtcaggt gccttgcagg aaaataatct gagtcccaag ctagcctgtg ctcatccaca 60
atcacaatga acatgtcaag gaagaatttg cagagactca agggaagcac aatgggataa 120
ggtaatcact ttcagtgaaa aactgttttc ttgaaaacag gcttggacac aattgaaagc 180
tggcttcctg caaacacacc aagagtctgt aatctagcct atccattata tgtcctttat 240
tattcatgat atcctattct tctaccttgt tgcctggtaa ctttttctga ggactgagtt 300
tctgcagcga tgtggtgcac tcttcctgtg atgaggaaac atctgggccc cct 353

<210> 459
<211> 528
<212> DNA
<213> human
<400> 459
ctggacaccc ggttaccaaa gtcagcaaag aagatgcggt aatcgccgcc tgatctccac 60
atggtgaaca caacactccc accaacacct ccttgactgg tcggtcttca gcaccggggg 120
tgggcaggca ggtgttctgt gttgacgaga attgcacagg ctaaacacaa acacggaacc 180
agagtgagaa cacctcactc acggcagccc aggctgctcc ctaccaggtg acggagcgcg 240
ccggggctgt gggtgccagg ggctgagtgc tagggactcg tcatgagtgg ggatccccac 300
gttcctgtca ctgctgtcaa acagaaggta aacagtctta tgaatgtatt tccttaggaa 360
aacttgtaaa aacttttatt aggatatcta tttaatactg aactttggcc tactttgtga 420
tagactataa acaaattgag gaaatcacta tttctcactt ctgtattttc tcaaaaataa 480
ttttgttaca gagttcaata tactgtgtac cattgatctt ctattgtg 528

<210> 460
<211> 469
<212> DNA
<213> human

<220>
<221> misc_feature
<222> (99)..(99)
<223> n is a, c, g, or t

<400> 460
gaaactgcgg atcccaaact gttccctttt tcatttcttg aaatgttacc actacagaca 60
tttttttaag gtgaataaac agttgtgatg tgctgtacnt aaaatcatgt ttaatcgtat 120
aaggaaacat ttcaatacac ttatacagga agaaaactat agatgaagta catgtgtgtg 180
attcagtctg attcacagaa ttctgagagt aatatggaat aaaacaactc cacttagatg 240
ataactgaag catttcctgc cttgtgaaaa tttggatttt aaattgctgt tagaatggga 300
aatttggaca ctttatatca ttgtataatt tcagaattta gtttctgtat cttttggaaa 360
acatgattat agcaaaaaca tagaaaataa tctattacta aaacaccata aatgtaaaac 420
tagtatgctt ggctgttaac tctaaagatg ttacttatgt ctgtttttta 469

<210> 461
<211> 457
<212> DNA
<213> human

<220>
<221> misc_feature
<222> (151)..(151)
<223> n is a, c, g, or t
<220>
<221> misc_feature
<222> (155)..(155)
<223> n is a, c, g, or t

<400> 461
tgagggtgag cttactcagg gcccccagag gaagccctca gcctctgccc tccccccaca 60
cagggcggga gcccaggcct gttcctggca gctgtggctg cagctgtgct cctgctccct 120
cctggaatgt gcgacaagcc caaatgttcc ngggnaggcg gccggggcag ggggcttaga 180
agtgctaata tggttctgtg ttttgcctga aacgatacca ggttcccctg aatagcaact 240
ttacaaggtc catgtgggag ggaccaaccc agatgccctg ctgagtgtcc ctgaaaccat 300
ggcagctcca tctgtcaaga tggcaggggc cggagtgagg gggctgctgg cttaacagca 360
ggcatctggg caggccagtc ctcaaagcag ctcctgaagg tctgtgttgc actgtcacca 420
gtctcaagct atgcctctaa tttcaccagg gatattg 457

<210> 462
<211> 139
<212> DNA
<213> human
<400> 462
gtgtgatgca tgtgagcgtc tctggcacac acacttggac atacagttct gtgtgcgctc 60
attcttatta caggagtgag caaaggaagc atttaccccg atggttacct agaccacgat 120
tatttggatt gggggagg 139

<210> 463
<211> 430
<212> DNA
<213> human
<400> 463
atccctgtat tcatggcttg actttgtgac tgctctacac tgcatgtctg acattgcaga 60
gtgagctatg ttgaggtaaa ctggttggtt gtcattattt tgcaatcagc ctggtctctc 120
ccatgaagat gtcgtgtgca taagcacaat catcactgat tagaagatca cagcagaata 180
cccttggatt agagagaagt tcgtaccttg catttctctg aattctagtc tctcataagc 240
actgctttgc tggatgattt tcactgcttt gtgttaatga ctttgagcga tctctcacat 300
gatggggttc tttagtacat ggtaacagcc atgtcatctt acacacctag cattgtgaat 360
gctgtagtga catcctttat aggcacctta cagctcaaaa cttttgtttc atttcatgcc 420
ttacttatca 430

<210> 464
<211> 486
<212> DNA
<213> human
<400> 464
agtgccattt attctagttt atcatgtttt gcatgtttga aagtatgaat gtgctctttc 60
ctaaaacatg gcaaatgaat agatgtagag aataacaata ttacttacaa gatgaaatga 120
ttagattaga agtgtccctt tattaaactt tgtcagcctg actgggtaca attcttttgt 180
taatttgcag tgtggtttgt atacacgtat acgtgttatc aataataaga ttttgcaact 240
ggatgacaca agattttact tgaacagtga aggacaaaaa tcatgattgt ggaagatatt 300
tttaaaatct gattttgcag cgatcacttt taaaccctgt agtgatgtaa gactaaaata 360
taattgctaa gattttgttg gttaatgtaa agatatgact tttctgcact gtactctctt 420
cataggattg taaaggtgtt ctaatccaat tgcatgatgt agtaagcctc ttaaatatgt 480
gtgtta 486

<210> 465
<211> 550
<212> DNA
<213> human
<400> 465
tcctatgtct tctttcttaa atccagttgc tgattttgta aaatacagtt gtgataaagc 60
agcattacgg gggggaaaaa gctatattcc aactggtgtt aaatgtattc aacaaaatct 120
tacatcatac agtatttatt tcttaattaa tagaacttca gtgatatact tggtagatat 180
ctcaagcctt ttgtctttta cacaatggtg ctctatccta ttgttttctt ttcaaagaag 240
catctgaaca cttgcatttc tattttccta tccaaaggca tccacatcta agtgtgtttt 300
taaagttgat taaaattatt tttctgttaa agcattctga aagtgtttgt ctttacctag 360
aatgatttgt acacactcgt ggtcaactga acatgaatgt cagtagtagt ctaattatgg 420
gaagggtaaa cgtgttagat taaggctctt aaagctctaa accatataaa ctatggactt 480
gtatcatgat ttaactgttc ttagatcttt cttacacagt gattcattcc tctatttgta 540

cagtggcttt                                                          550

<210>  466
<211>  493
<212>  DNA
<213>  human
<400>  466
tatcaaaggt ttgccagcca ataaagtgca tcccaagtat acaggggaga aagctagact      60
cctacagggt cctagagttt aagtaatttt tttgttatta atataggtaa taatttttct     120
aatttttatt ttttggttcc aaatgtaaag ctccttgtgt ttacctctgt ttatgtcatt     180
cttgacatgt ttatctaaat tatgtgtgct ctgtgacagg tgaaatgtaa atctgggatc     240
catagtcaag atatcataag gacctacttc ccagcctacc tttcttcctc tacctgataa     300
tgataatact caaaataaca acattcaaag gaaacacaaa gaaatcctgc tttcacatct      360
cctatttctt gggctcctta ataactactg atggtttgtt catgaaaaaa aatttttaaa     420
tcaaaagatt gtacttggcc ctgagttgaa aaaatttcaa aaatcaaaag tttgtacttg     480
gccctgagtt gaa                                                        493

<210>  467
<211>  461
<212>  DNA
<213>  human
<400>  467
cagaaacatg accctcgctg gtcttgggtc cacatatcat tggactctgg gggacacaaa      60
gatgcctgtg acactttggt gttgccgagt tagtcaacaa ttattctggg aaaaagcaga     120
attgaattct tctctagatg tcctaccagg gttggccaag ggccacaaag caggctaata     180
aattcccaca ggatccagac accaggcaaa attgctctaa gaagccagtt actgtcatcc     240
ctctatggtt ctagaaaaaa tagtacaaaa atgacaggtc atcctatgag cgtcatgcca     300
atgaaacccc atcttctgga gaagcccttg aatcagaatt atcttttttc ttgatgtcgt      360
cagatgcagc cagtttctta atttttttaa aaactgtatg tttctgtggt atgtatattt     420
gtacacctaa ctacctggca cttggaaatc acagcactac t                         461

<210>  468
<211>  448
<212>  DNA
<213>  human

<220>
<221>  misc_feature
<222>  (28)..(28)
<223>  n is a, c, g, or t

<400>  468
ttatcctgcg acaacacttt cagcaganta gcctctcctt atctcacaga tcacaagcac      60
ccctagatag tgtgattctg tcagatagca tttatgcaaa aatctatgaa gttaaaagat     120
cgtagaagcc aaatgaaatg tacatatcta ctgactgatg acaaggaaat ttcattagga      180
agaaggtaaa gaaacatcgt tgagtagcct accttgattt ctgtcaagtt cataaccagc     240
ttcatatttt aaaggcttca ggtttgaaat taagtcaact gcatgcagct ttgctgataa     300
atgaataatt ctctttgatg ccatttatga gaaaagactt caatatctgt tgcctgtcat     360
atttaagaaa aattactgtt tctactctct gtatctgatt ttaaaagaaa aaactattca     420
tacctggctt ccaggtaatt gactttga                                        448

<210>  469
<211>  427
<212>  DNA
<213>  human
<400>  469
gaactgagtc aatatggcaa ggtgtatgtg atctgtggga gttatgccat ttaacatagg      60
aagtgcatgg gactttccct ctctgcactc cagctcttac tgtaccatta gaagatgcag     120
aattctgttg gtgtgcaaaa agtatagcct tacattcaag cagaatggat ctgaagaaag     180
cagcaatatc tgttactaga gaacattccc atgtgtttaa actcttcact tcttagatgc     240
atttaaattc ttaatgcaaa tgacgtagca atttgaaaac ttctccgtat tacttgtgtt     300
taaaatgtct tgctttaaat acaaaacaaa tggtaaaggg gattatcttt tgtttagatg     360
gttaaatatt atttttgcct tagatagctt tgtaataatt tttctccaga cagttcaaca     420

cttttga                                                              427

<210>  470
<211>  567
<212>  DNA
<213>  human

<220>
<221>  misc_feature
<222>  (246)..(246)
<223>  n is a, c, g, or t

<400>  470
   tactgactga ctacaggggc tgattgtgaa gcacgaggaa ccccatgtgt gtggagactg      60
   tagggtgaga gcacacaatt attagcatca tttctgagtg atctcacaga ttttttttct      120
   tgtgtttgtt ttgctttttg acaactgctt ctcccacgtt ccttgcaatt ctattctctc     180
   accttcactt tactatttgt attcgatgga ccaggataat tcaggcaagg ttaccttgta     240
   aacttnaatt ggccacacac catgttgtca cccagctggc tatgaagtga ataatggtac     300
   tgaaagtaaa cctgaagacc tttctcagat ctattttaag tctgagtctg accaaccatg     360
   gaaaatattc gacatgaatt aatgtagaga actataaagc atttatgaca gctccaagaa     420
   aaatcatcta ctctatgcag gagatatgtt tagagacctc tcagaaaaac ttgcctggtt     480
   tgagggtaca cagtaccatt ttaatcttct gaaaatatct gtattcctgc tctttttctg     540
   ctgtcactgt caatctgcta tattttt                                          567

<210>  471
<211>  506
<212>  DNA
<213>  human
<400>  471
ttcctgacgg gaatgttgtg ctataatgaa tctgcataac gcttgggatt ctaggaggaa      60
ggaaggttcc atggacatgt aagtacagca tattcccctc agtcttctag gagggcagag     120
tgaatcccag aactggtaag attgggaatc tgagcattgc cactttaatc ttagaatatt     180
tatcattttg acacatcctg ttttttagag aggaaaacaa acacagtttc tgcattggta      240
gtgtaaagca taccttgtta ggaacgtgtt ttgtaagaca catttgggtt gtcattctag     300
agcatgtcaa actttgtact tcaaaatata tttagtatga ttgttagtgg taacatatat     360
caaggctttg aattaactgt tttatttaat tttcacaaga agcacttatt ttagccatag     420
gaaaaccaat ctgagctaca aatagttctt taaaataagc ccaggttatt tagctattct     480
agaaagtgcc gacttctttc aagaag                                          506

<210>  472
<211>  488
<212>  DNA
<213>  human
<400>  472
ttaaatattc attccattac atctagactc accaagaact acatgttatg atgttaagtt      60
gaagttgaaa catgatgttt tgcattaaat ttaagatatg caaatttatg tagagaaaat     120
aaatgttata taccctataa tctttcacct aattagtatt taattatatg gatttgtttt     180
atattataaa agatgttttg atttgtcttt ttgatattga caaaattgtt tggatatcct     240
tatgttctca agtctgtatc tgcctcccct gccttatttc ttatgttttg ccacagttaa     300
cccattgtgc ttctttgtaa tcaaacagtt tgtgggagaa tgggcttatt gaatgtctaa     360
aaaataagtt taaagtgttt gttaccctaa gttttttaca tttttaaact ctaattacat      420
atgtgaatgt tattactctc agtgaattgt tattgtttgc aaaaatgcac tgggcagtaa     480
cattttgt                                                              488

<210>  473
<211>  426
<212>  DNA
<213>  human

<220>
<221>  misc_feature
<222>  (262)..(262)
<223>  n is a, c, g, or t

<220>
<221>  misc_feature
<222>  (271)..(271)
<223>  n is a, c, g, or t

<400>  473
gccggactgg aaccgtctga gcggcctaac ccgcgcccgg gtagagacct gcgggggatg      60
acagctgaac cgcctaaagg aggtgaaggc tttcgtcacg caggacattc cattctatca     120
caacctggtg atgaaacacc tccctggggc cgaccctgag ctcgtgctgc tgggccgccg     180
ctacgaggaa ctagagcgca tcccactcag tgaaatgacc cgcgaagaga tcaatgcgct     240
agtgcaggag ctcggcttct ancgcaaggc ngcgcccgac gcgcaggtgc cccccgagta     300
cgtgtgggcg cccgcgaagc ccccagagga aacttcggac cacgctgacc tgtaggtccg     360
ggggcgcggc ggagctggga cctacctgcc tgagtcctgg agacagaatg aagcgctcag     420
catccc                                                                426

<210>  474
<211>  563
<212>  DNA
<213>  human

<220>
<221>  misc_feature
<222>  (35)..(35)
<223>  n is a, c, g, or t
<220>
<221>  misc_feature
<222>  (55)..(55)
<223>  n is a, c, g, or t
<220>
<221>  misc_feature
<222>  (96)..(96)
<223>  n is a, c, g, or t

<400>  474
aatggcttct atgatcagaa ctgggaaaac agtgnatctt atggtggaag aggtnctcag      60
caagtgtaca gtatttacct tcctttgtct tacatnggct ttttaaattt tccattaatt     120
tcaacataat tatgggaaca agtgtacaga agaatttttt ttttaagata tgtgagaact     180
tttcatagat gaacttttta acaaatgttt tcatttacag gaaattgcaa agaaaattct     240
caagtgatag tctttttttt taagtgtttc gtaagacaaa aattgaataa tgttttttga     300
agttctggca agattgaagt ctgatattgc agtaatgata tttattaaaa acccataact     360
accaggaata atgatacctc ccaccccttg attcccataa cataaaagtg ctacttgaga     420
gtgggggaga atggcatggt aggctacttt tcagggcctt gacaagtaca tcacccagtg     480
gtatcctaca tacttctttc aagatcttca accatgaggt aaaagagcca agttcaaaga     540
accctagcac aaatttgctt tgg                                             563

<210>  475
<211>  474
<212>  DNA
<213>  human

<220>
<221>  misc_feature
<222>  (48)..(48)
<223>  n is a, c, g, or t
<220>
<221>  misc_feature
<222>  (55)..(55)
<223>  n is a, c, g, or t
<400>  475
ggaacctatc aatttgagtg gactttttct ttagtagtac accatttngg ttgtngtagt      60
ttcaaagtct ttctgaagca gatatattgg gattggagcg gggtggggaa aactgtcact     120
cctttcagag gaaaagggga ggagcatgga gaaaaacaaa aattaaagga cttaaagaat     180
ggctatacag tgttgagtgt tgaggatatt aaacatgtta tttttcaaac gtatgtaata     240

tatattaaat ttataaagca aatttatgtt gtgatcttgc ctgaacaaat tatattttaa 300
tgaaaaaact ttctattaat agttcacgca agagaaaaca ctttcaacat agtcgaaggc 360
ttcaagatct aagtgtatca gacttaggga aaaagtggca caaccttcga tttaaaattc 420
tagtctttaa aatgagtttg taaataatta gctattacgt tctattaagt tgtt 474

<210> 476
<211> 322
<212> DNA
<213> human
<400> 476
agggatctct ctacaagagc ccctgcccct ctgttggagg cacagtttta gaataaggag 60
gaggagggag aagagaaaat gtaaaggagg gagatctttc ccaggccgca ccatttctgt 120
cactcacatg gacccaagat aaaagaatgg ccaaaccctc acaacccctg atgtttgaag 180
agttccaagt tgaagggaaa caaagaagtg tttgatggtg ccagagaggg gctgctctcc 240
agaaagctaa aatttaattt cttttttcct ctgagttctg tacttcaacc agcctacaag 300
ctggcacttg ctaacaaatc ag 322

<210> 477
<211> 409
<212> DNA
<213> human
<400> 477
ggtaattatg gaaactcctc aaagaggaga aagtaatttt tttccagaca tttttctcat 60
tctgtgtctt tcacacacta gtttccatag ttcgagaatt ctgtttttta ccattgggct 120
gtgaatgttc acaatatcag tcctgttgaa ttcctatgag gtaatcacaa tgtgtatatg 180
ttcattttct aggtatgata aaagaatgta tggcttttta ttctgtggaa gtaaaatcct 240
gaacgtttac aacttttcct taacttgtaa ataaaaaatt gtaagttttt tcttttttta 300
cagaaaactt agcttgtgta attctgttag tttcagattt ctctcctgtt tttgcaaatt 360
gtgggaaaga ttgacaatgc aaatgtgtca aagacatact gttgggtgc 409

<210> 478
<211> 544
<212> DNA
<213> human
<400> 478
acatgggttt atacaagttc ctcttgagaa ggcaaaaaga ccaccatgtg tgagagctct 60
ttgacttggc caataggggc ctatcttaat gcacttgttt ggacacattt ctgatcttat 120
ttgtaaaggc tgcaaaagga gaggatgaaa tgctgtaaaa gtaggaaatg aagtggaagc 180
tggaagaaaa tgtaattggt ggtacagcta tgggccagat ggtggagggg agggtgggga 240
cccctgccgg caagcagagt gtcacagctg gctttcctca cttgggaaaa gggtactgcc 300
ggtctagcag cctcctctgt actcagccag gacacccagc gcgtgggacc tgtttgtgtc 360
tgttttgctt ccttgggaac ggcacagtca ctcaccctgc catttgcgga aatgacctgg 420
tgcactttga ctgttaagca atgcgttatt gctgtagtca aggttagtgc aagcaaggaa 480
acattcccag taaggtattt gtttccattt tctgtctgtg cttctgtcag aaacttgcta 540
ggac 544

<210> 479
<211> 479
<212> DNA
<213> human
<400> 479
caaacgtcct ggactgcaca gacctcccac tccagaccat ccaggcctgg ttcccaagac 60
ccgatccttc ccctgcaacc agacagtcta caactgcccc ctccagccca ttttctgccg 120
tgaaacccca gccagccaca ccagactctg gaaccctttt tcgactgccc caactcttgg 180
acaccaggcc aactagaaca cccaacacca aactgtacag actctcccac cccaacctcc 240
ccagactctg cacggatgtc ctaggccccc tccccaactc taaccagacc ccatccccct 300
aagtcccttt gtcttgaccc ccaagtcttc aaccagatat cctcggcaac ccacctccca 360
ccctcctcct cttctccttc aagacccaac tgagcacccg ctctgattcc ccacagcctt 420
tctccctgcc accactccct tagtctttcc caggcttact ctcccaataa atgtgctag 479

<210> 480
<211> 493
<212> DNA

<213>  human
<400>  480
ggtggagttg gtggtcttaa tttggagatg caggggcaac ctgtgaccct ttgaggcaag      60
agccctgcac ccagctgtcc cgtgcagccg tgggcagggg ctgcacacgg aggggcaggc     120
gggccagttc agggtccgtg ccaggccctc ctcagtgccc tgtgaaggcc tcctgtcctc     180
cgtgcggctg ggcaccagca ccagggagtt tctatggcaa ccttagtgat tattaaggaa     240
cactgtcagt tttatgaaca tatgctcaaa tgaaattcta ctttaggagg aaaggattgg     300
aacagcatgt cacaaggctg ttaattaaca gagagacctt attggatgga gatcacatct     360
gttaaataga atacctcaac tctacgttgt tttcttggag ataaataata gtttcaagtt     420
tttgtttgtt tgttttacct aattacctga aagcaaatac caaaggctga tgtctgtata     480
tggggcaaag ggt                                                        493

<210>  481
<211>  483
<212>  DNA
<213>  human
<400>  481
atttttccat cttattcaag tcagagcact tttttttaaa tagcaataca acaggcaaga      60
gaatactcaa aaatatttaa aactgtattg ataccaagag tatgttttaa atattttcta     120
ataaatactt gagcggtttt tgtctggcag gcttccaaat ttgccaaaat taagcgttca     180
gtattttcaa cacatacgct ttttactggt ttatactgaa ctatctgatg agaattcctg     240
tgttcccaaa gcaactgatg tttacaggtc ttgtgtttct cctcctcctt tctaaggatg     300
agggaatcca caacagactt tctctagaaa acactaatga tggacaactt tttggtgtca     360
tcaatgagtt ggctactacc ttgatgtaaa aatttgtaag gaaaattttc accatttcga     420
gtgtcaagtg tatttttaac tgtctggttt gtacttttat gacttttgta ctaccaaagc     480
gga                                                                   483

<210>  482
<211>  408
<212>  DNA
<213>  human
<400>  482
agtgatctct ggaagcgcta aagctaaaat ttctgttctt gaaacacttc agctttgcaa      60
ctaaaatatt acagattaat aataaattaa accaaccaat gataaacact actcagtcca     120
ccaacaacaa acgtgtttga attcacctta ccaatattaa tcccagcgtg tgtaaaacag     180
aacagtaact ctatgtgacc ccagataaca ttttgtaaca ttgtgcttcc ttgtagtttg     240
taatgtgagt tcaatcagta tttatgttga aatttctaac attaaatcta gtctctatcc     300
tgttaattta atttttaaat gctttatcca tttgtgcaaa ggtaaacgca gattgtatct     360
tttttaatgg tacggcataa aaagtaaccc tcaagtgaag tgtctcta                 408

<210>  483
<211>  489
<212>  DNA
<213>  human
<400>  483
tgacgagacc tcttcaaaaa cccacagtaa aactcccctc cctccagttg gccaccagtc      60
tgccaccaaa catgaacaaa ttctgctgct aatcggtttc ccttgtgatc tggttcctga     120
ggtcttcgga tctgtgcaat gaattattta ttgttttatt aaaccgacag tggtgtccca     180
gagaggaacc ataaataaaa tggaaatctg gtgctgtgat aaagtaataa ctagcattaa     240
tgagacctgg ttttcctttc agaaagtcca gtatacctgt aacaaaggtt aaagcaattt     300
atatttaatt tgcattctga tgttaacatt taaacagcaa ttctaacaaa aatgcatcga     360
gtctaattct tacctctatc aaaaaacaac tgtataaatt tatgaccaac attaaaacaa     420
aaacaaaatg taaattttct ctttagaaat gattaactgg aaatgagtga gacagcacct     480
tgtgggttt                                                             489

<210>  484
<211>  460
<212>  DNA
<213>  human

<220>
<221>  misc_feature
<222>  (101)..(101)

<223>  n is a, c, g, or t
<220>
<221>  misc_feature
<222>  (129)..(129)
<223>  n is a, c, g, or t
<220>
<221>  misc_feature
<222>  (131)..(133)
<223>  n is a, c, g, or t

<400>  484
tgctcgcatt gatcatccta atcatcttat tcagcctatc ccagagaaaa cggcacaggg      60
aacagtatga tgtgcctcaa gagtggcgaa aggaaggcac ncctggcatc ttccagaaga     120
cggccatcng nnnagaccag aatctgaggg agagcagatt cggccttgag aacgcctaca     180
acaacttccg gcccaccctg gagactgttg actctggcac agagctccac atccagaggc     240
cggagatggt agcatccact gtgtgagcca acgggggcct cccaccctca tctagctctg     300
ttcaggagag ctgcaaacac agagcccacc acaagcctcc ggggcgggtc aagaggagac     360
cgaagtcagg ccctgaagcc ggtcctgctc tgagctgaca gacttggcca gtcccctgcc     420
tgtgctcctg ctggggaagg ctgggggctg taagcctctc                           460

<210>  485
<211>  560
<212>  DNA
<213>  human

<220>
<221>  misc_feature
<222>  (144)..(144)
<223>  n is a, c, g, or t

<400>  485
gctgattcta attgtgtgta ggtcttgagg attaagcaca caaatttcac aaacttctgt      60
ttgagtaaac aaactcagcc ttctgtaaat atacatgcaa gtttggaaac agtaatactg     120
tacctataaa tatatgctgt ctgntttgtg tacagtatgt aaaaactcct tttctgccac     180
actaaaaatg caagccattt atgggaatcc taaaactagt attgaactaa aactttgcta     240
atgatcttta ttagaggatc gtccaacttt tcacttacct tgggttttct tttcaattca     300
ctcttacact agtctgctta tttccagctg tttattttat tgagtcctga atttaaaaaa     360
aaaatatttt gattcatttt gtaaatacaa gctgtacaaa aaagagagat ttaatgttgt     420
cttttaaata ctccaatttt cattctaata tgaatgttgt tatattgtac ttagaaactg     480
tacctttaat attacattac ctttattaaa agtgcattga acacatcaat tttagatgtg     540
ctttatgtac tgttatccta                                                 560

<210>  486
<211>  212
<212>  DNA
<213>  human
<400>  486
taagcattca gtgagctgcc aattttgatt ttgtgttgct ctttacccaa attatttttt      60
ctttgttttt ctttttttgg gggaggaggg gaaaaaagca gcaatactgt gtttggaaat     120
tatactctgt atctggtttt cctgtgtatg ttaaccactt aaatgttatt atcctgcttt     180
ggttttagag tgattgtgag gcattcaatg ca                                   212

<210>  487
<211>  407
<212>  DNA
<213>  human

<220>
<221>  misc_feature
<222>  (75)..(75)
<223>  n is a, c, g, or t

<400>  487

agtgcctaaa tcttgtttac ctatcacttt aaaaaaataa ttgaagtgta agctaaataa 60
aatgcttgga gtttngcctg ggctagtgag agttggtgca aattcttgtg tgtgtttgca 120
taggaaggtg agatgaccat ctactaaaga ggaagtagct aaatacagat ctgtgggtgt 180
ttttaaaaaa actcaaccta tctggtgttt tattttaatg gataaaaatg taatttttct 240
aaggtagcaa cttatttcca aattaatata gatgaaaaat agataccaat tagactaaat 300
tgaaagcttt ttgttctata tttgcatagc ctttgaaata tttcttagtg cctaggaggt 360
ctggggattc ctctttcgtg gtggtcacta accttacttg atgcaga 407

<210> 488
<211> 553
<212> DNA
<213> human

<220>
<221> misc_feature
<222> (105)..(105)
<223> n is a, c, g, or t
<220>
<221> misc_feature
<222> (136)..(136)
<223> n is a, c, g, or t
<220>
<221> misc_feature
<222> (142)..(142)
<223> n is a, c, g, or t
<220>
<221> misc_feature
<222> (155)..(156)
<223> n is a, c, g, or t
<220>
<221> misc_feature
<222> (481)..(481)
<223> n is a, c, g, or t

<400> 488
aacaccaatt atgcaaacta cttttttttc cagcagaaag ggagctgaca tgatcaaatc 60
catgttttca aatgaactga aaaaggcatc cagcaccact tatancacat ttatttcagt 120
tccaggctga cagccntggg gnatctagca ggatnnataa ttgtcatctg gtgagagttg 180
ggactttgct caattttaat accaatctct cctgattgta attacctcca ctactttcat 240
gatcccccta caatattttt ttaaatgata tatttattca ctgaatcaaa tgtcattaat 300
gagttatctt ctgtggagga tgactgttct ctttgttaat gttcacaatc aagatcttgg 360
gctgagaaga ggcccttcac ccaaggagtt tgaagtatca cagcgtgtgg gaaggtggga 420
accaggatac ccattcattt ccaaccgaga cacagagaag tgagtcacag aatttgagcc 480
ngctctcttg actgcccagc cagagacact gatttctgta acctcttcac ttgatcctgc 540
ctcttaagca tta 553

<210> 489
<211> 421
<212> DNA
<213> human
<400> 489
tcatttctag agtacctgag ccaaacaaat acacaacgga agctgcagct gtatcatcac 60
tagcaatttg ctcatcatta tttactacct ttgaacctaa ggtttcctgc ctatgctttt 120
gaaagcaaaa atcagtctcc tttgcatgaa aaagagcctt agatttttaa acatgttagt 180
taccagaatg ctaaaatacc agttgattac ccaaattatt ttggaaatct atccataatg 240
gaagtctaca acaaacacat aaaacagatt acactaagag ctgagaaatt caaaggaact 300
gaagattctg agagataaac tgttcaagtc ttagcaatga tactgcactt ctctttgaca 360
ggttctgggc ttaagttaga ggccctactg gttccaaacc atattccact gactttgcaa 420
g 421

<210> 490
<211> 505
<212> DNA

<213>  human
<400>  490
cctctgagtg ttttgtgggt tctgggtgtt ttgtacattt tagccaagct aaccacttgt     60
ctgcaagtac tgactttcct atgaattctt tgaagattat tgagtcagaa aggaaaaata    120
tagccccaaa ttcccaggct tttaatgcat tacattaact gcctattgaa atgagaagtt    180
cttcacaaac ttgtataccc actaacaaga ttgcacataa acatgcatta aagtatatac    240
taagaaaccc tctgtccaac ggctcatgca tatgaagtcc gaacatggga gtttgccaat    300
tgcattcatc aagtcgtttt gcggagtcag atccctgatg gaagagctca caggctctgc    360
cttccaagtc ctgggttcct aactggtgac cttagcctgg ggtctgtggg gagaccaacc    420
ctggcttcca agaaaaccac attccatgga ctatcagaaa tagacacaga tttgggtgac    480
aaagctggct ctgtatttgc atttt                                          505

<210>  491
<211>  497
<212>  DNA
<213>  human

<220>
<221>  misc_feature
<222>  (48)..(48)
<223>  n is a, c, g, or t
<220>
<221>  misc_feature
<222>  (84)..(84)
<223>  n is a, c, g, or t
<220>
<221>  misc_feature
<222>  (86)..(87)
<223>  n is a, c, g, or t
<220>
<221>  misc_feature
<222>  (116)..(116)
<223>  n is a, c, g, or t
<220>
<221>  misc_feature
<222>  (348)..(348)
<223>  n is a, c, g, or t

<400>  491
gcaagcgcgt gctgagcaag ctgcagtctc catcgcgggc ccgcgggnca gggggcagtc     60
ccggggggct gcagaagcgg cacncnngcg tcaccgtcaa gtatgaccgg cggganctgc    120
agcggcggct ggacgtggag aagtggatcg acgggcgcct ggaggagctg taccgcggca    180
tggaggcaga catgcccgat gagatcaaca ttgatgaatt gttggagtta gagagtgaag    240
aggagagaag ccggaaaatc cagggactcc tgaagtcatg tgggaaacct gtcgaggact    300
tcatccagga gctgctggca aagcttcaag gcctccacag gcagcccngc ctccgccagc    360
caagcccctc ccacgacggc agcctcagcc ccctccagga ccgggcccgg actgctcacc    420
cctgaccctc ttgcactctc cctgcccccc ggacgccgcc cagcttgctt gtgtataagt    480
tgtatttaat ggttctg                                                   497

<210>  492
<211>  540
<212>  DNA
<213>  human

<220>
<221>  misc_feature
<222>  (380)..(380)
<223>  n is a, c, g, or t

<400>  492
cactgttcca tggtcagcaa gtcatatttc ataatgtgga ttttccaaaa taattattga     60
atacagctat tctatggcta cttttagtgt ttttgtggta tgtggtgtgg gagtgtttat    120
ggaattacca gtatcttaaa ttttcaaagg aaccttggaa gtctatcact ctaaatgaaa    180

gtctgtcact ctacatgaat tatgtgctca aatttgacca actcagttta agacacaaaa 240
cagtaatttg aagaaggaaa aatgaagaga gtttctagtt taatgggtta aatttttgtt 300
gttgcaatag taagtttagt cttcttataa tatttctaaa tgaaaaatca taggtatttg 360
ttaccatgtg tgaagattan tttgttaaaa gcaaaagtgg tcgtgtgata tgctaaatgt 420
taattactga ttttatatgt ttaaatcacg ccaaacaaat tatgtctgtg ccatccaggg 480
tctgttgtta atctttttct gagtacttgg attgggataa agggcttgta ctatgcactt 540

<210> 493
<211> 569
<212> DNA
<213> human

<220>
<221> misc_feature
<222> (79)..(79)
<223> n is a, c, g, or t
<220>
<221> misc_feature
<222> (286)..(286)
<223> n is a, c, g, or t

<400> 493
acacttatag tctactgccc atgtaaggat cagctccggc taagaggcca aagatgggtg 60
acatcgttat gctctgccnt ttattttttc tttcttaccc acttagcttc ctaattggag 120
gaaggaggcg tggtaaaggt atatgaagac tatggtttaa ttagaccaga aaacactgtc 180
ataatctctg gggtcatcag aatgtccagt tttgtctttg ggccaagata agggcagtgg 240
gatttatgat gtgttgttta tagtctgaaa ctactctggt gatcanccag ggtcagtttc 300
tttaatgatg gtttccaact ggcctaatac attaagtaag actggctgat aacatgacca 360
gacagacata aagaccctgt tgggaatgac attgaactct caaagtcaag atttcttaca 420
caaatctatc agctggagaa aatgaaggca gtgtggtata tgtgtgcaaa taaggacatt 480
atgaagctta aatatggaat gtctcttgga cccccgatgt catctgtatt ctctttttct 540
tcttgtacta tatcctttgc ctgtaaata 569

<210> 494
<211> 326
<212> DNA
<213> human
<400> 494
tcccagtgcg aactctgctg tgagtgtgtg cggggaggcg cgcccgcgct gagtcggcgg 60
cgggtagcca ctccatgccc ttgtccgatg gtttgcaact ccgattttgc acaccgctcc 120
accgtgcccc ccagcgcaca cccattcaca ctcacgccaa cactctcgct gaacactttt 180
ataattgtta ggcgtggccg ttgggacttt gggcgcagcg cggctgctac tgcgtctgga 240
ggattgatat ttatttttgc attgcgatgg ctgaaggcat ttatttaacg atctttttac 300
ctggatatgt ctgtgaggct cctgaa 326

<210> 495
<211> 548
<212> DNA
<213> human
<400> 495
gtgaagtacc atagaccata ttgtctagat gggacaaaat aataaatttt gactaagaac 60
ttactgttta gagagaaaaa atagtaaata atgttcaaaa tgagaagaca ataactgaca 120
agtctgtgat ttttgggtta gatgagacca cttttgattt ttgaggaata aaaggagtta 180
gttttttggt cctgatgtgt aaaatgattc atggggtttc acatacagac aagtagtttt 240
cttttcagca aatatttaac atatctctca atatattttt caccagtttt tggtaaatat 300
gcattgctat aggtatacca agtttttttt gttatgttta gtaactaatt tcattttttc 360
tgtatatgta aatatagcta ttggaccctg cattgaaaac cttcttagta ctaacaacac 420
tccttttatt attaccttca tagttacttt aaatgacata tttatttcga gttttacttc 480
tggctgagtt gcaaagtcta tgagaatgtt acctcctgtc tcagaagctt gttagttatt 540
gtgccacc 548

<210> 496
<211> 476

<212>  DNA
<213>  human
<400>  496
acatctgcta gaaccttttg ccttaactat tcaccaatat atgctaatat tcataaatat      60
ggattgactg tttacaaaca ttagaatctt gtcttggttc cattttgatg gctaatattt     120
gttatcttaa ttaagactat ttctgaggtc atgattactt gaaaatattg actaaaactg     180
ggtccttaga aattccaggt ggagctgatt tacctatgac tgaggggaaa aaaaaatcaa     240
attttactga taatagtaat gctccaaatg aattaatgac acatctgttc aataaataaa     300
gagcttaaat atacaaaaca taagaaatct gggcaacaaa acttgtggtc tttacttttg     360
aatagctacc caagaaaagg ttttaaaggt aaaagttatg agtaatgtca tcacaataag     420
ctcttgttta aaattctttt cttttatgta taattaggtt tatgtttcat gtcttt         476

<210>  497
<211>  414
<212>  DNA
<213>  human
<400>  497
atcccatggg caataaagcg cattcaatgt gtttataagc caaacagtca ctttgtttaa      60
gcaaacacaa gtacaaagta aaatagaacc acaaaataat gaactgcatg ttcataacat     120
acaaaaatcg ccgcctactc agtaggtaac tacaacattc caactcctga atatatttat     180
aaatttacat tttcagttaa aaaaatagac ttttgagagt tcagattttg ttttagattt     240
tgttttctta cattctggag aaccgaagct cagctcagcc ctcttcctta ttttgctccc     300
aaagcctccc ccaaatcatc actccctgcc ccccttaagg ctagaggtga gcatgtccct     360
cacaattgca catgtcaagc catcagcaag gcgcatcaca caaaaggcac caag           414

<210>  498
<211>  309
<212>  DNA
<213>  human

<220>
<221>  misc_feature
<222>  (61)..(61)
<223>  n is a, c, g, or t

<400>  498
cagtgagtcg aggttgcgct actgcactcc agcctggaca acagagggag actctgtctc      60
naaaaaaaaa aaaacctaca gctgttcaag gaccagctga caggtcaagt gtggcctttt     120
ctggtctttg aacacatcat agaaagtgac aaatgctgca aagccatgaa gaacatgaac     180
tataaacggg tagactaact gcccagctta gacacttatc tatgccacaa aacagctgaa     240
tttgtcacat ttatatattg caatatggga agtattgaga tcaaaacagg attccattga     300
cctaattat                                                             309

<210>  499
<211>  233
<212>  DNA
<213>  human
<400>  499
gccatcacag ttgcgattcc atgagtagct gctttatgac tgctttttgt actatctgga      60
tgtgcccaga gttacttctg tacaagctct gtatctatgt ccgttgagaa cattatttta     120
acaagaagaa caccaacagt agcatgaaat ataatactgt tttataattc taaagctgct     180
gttaatttat gaagtacata ataatctaat gtaaactgca gaagtcagag caa            233

<210>  500
<211>  420
<212>  DNA
<213>  human

<220>
<221>  misc_feature
<222>  (71)..(71)
<223>  n is a, c, g, or t

<400>  500
gcaaccattg gggaatgacc ttttcatttc agaagtggat gaggaaggtg gtgtgagcat      60
caggtatatt nctggaccat ttcaagtgct ggtgagaaga aaggaactct ttgcctgaac     120
tgggcttggt tttccaagtg ctgctttgga aatgaagacc cagagatgca gagcttatgg     180
tagttcataa atcttcatgt tctattatct ttcatctgcc aataaagttc attttcaata     240
atgtccacca ttgctgtgcc cagaataacc acaggcaaac atcaaaacaa tacgcataag     300
ttagacaaga ttaaatcttg tctgatatct gcacaaacag atatgcacca tgttggaaac     360
atgtgttttc ctagtcccat ccaggcttcc cacaagaaag ccatgatgtg ggtctaaacc     420
<210>  501
<211>  390
<212>  DNA
<213>  human

<220>
<221>  misc_feature
<222>  (96)..(96)
<223>  n is a, c, g, or t

<400>  501
attgtttccc atagcagaat gtcaatattc acagtacatt tctgtaaaga gcaaaccaat      60
ataatgtttt gagtgttgaa aaaaattcca gatttntgaa gaattagaca actcttcatc     120
taccttattt ctagttcaca cagttatctc aaattccact gaaactaatg ggatactgtc     180
ttgtgtagat gccagttgag tttataatgt gacctagtaa agctgtcttt tttgttgtgt     240
tgtatgagtg tcggatcatg cttttaggaa tacttttatt aaaatggtgt gcattcatgc     300
aaaaggccaa ctggcttttg tgaacaatag atcttttctc ccctttattt tgttctcttg     360
acacttttgt gaaaattacc tagcctgata                                      390

<210>  502
<211>  460
<212>  DNA
<213>  human

<220>
<221>  misc_feature
<222>  (258)..(258)
<223>  n is a, c, g, or t

<400>  502
aaagccttcc ctaaaaagag agcatgccag tcatagagac actaatttgg cacttttcct      60
ccttcttagg tttaaatgtt ataccaatgc atgtgtttga aatatccatt aagatgattt     120
aaaatttgtc ttgtattttg agtttcttaa atgcgtggat tcttgttcat ttatctctga     180
atatgtctct tttatatttt ggcacaatta tacacattgg aaagggccaa cctattaggg     240
ctcaagtatg tatatgcnaa aaaaaaaagt tatatcaaac aggcacagtt attacaacta     300
gagagaaatt ccagaaatat ttgttttttt aagagaaagt aaattttcac attagatttc     360
tattcaaagt actaatatct acatggtcca cacttttcta ttttaaaaaa ttgtgttctc     420
ttgttaaata gattaacatt tccacttctg tttatcacaa                           460

<210>  503
<211>  514
<212>  DNA
<213>  human

<220>
<221>  misc_feature
<222>  (285)..(285)
<223>  n is a, c, g, or t
<220>
<221>  misc_feature
<222>  (469)..(469)
<223>  n is a, c, g, or t

<400>  503
atctttccat attgaggaca gcctctgcat ataaaaatgt tcattgggag aaaaattatt      60

tttaaaacta aatttttata gtagtcctcc attactggca attaaggagc aacccacaaa 120
atgtcattat gtggttcttt gattaggcat aaaaagtatt gcaaacagct tttctattcc 180
tttggaatat gaatcttttt aatgtctaaa gttaacctgc aacattttct agtaggacaa 240
gtcaatgtaa attaggtaat ttttgcctgt tttatacctt ctgtngcatt ttgttcaaat 300
gaaaacattt tttaaaacat gattgaattt ttacttctca gataattggt ttgagtgtgg 360
aatttctttt ttaacacttt gatatgtaag ttagtgtaaa ttaatacttt tataacacta 420
ctaggcttta tatttttatg gaatgtttta aaatttgaaa tttttgaant gttaatttag 480
cagtacttta tcattatgtg ccaatactga caag 514

<210> 504
<211> 445
<212> DNA
<213> human
<400> 504
tttactccag tcattcgatg ttgctgagat ttacatatga ctcttgtcaa catctcatct 60
tttgacccaa tcttattcat ttaataagag gtctcattca tttgcatgga aaaatgctca 120
ttgtatattg caaagtgaaa ataacgagtt gcaaaacagt gtatacatat atgtgtgtat 180
atatgtacac tttatttgta catttctatg tgacataatg caaaggaaag tgtctgattt 240
tattatacac caaaggttaa cagtgaatct ctgtgtgatc tctttttttt tctttttgcc 300
tatctgcatc ttctcacttg ccaaaaaatg aatatatgtt tatgtgtgta tattacttgt 360
gtcacaaaaa accctaaagt agacagtaaa agaacttgtc aatcgccttt ggaaggcaat 420
gaaacactta ataaactctc aataa 445

<210> 505
<211> 514
<212> DNA
<213> human

<220>
<221> misc_feature
<222> (26)..(26)
<223> n is a, c, g, or t
<220>
<221> misc_feature
<222> (128)..(128)
<223> n is a, c, g, or t

<400> 505
agccaagaac tgaccggggc cagggnctgc catggtctcc ttgcctgctc caaggcacag 60
gatacagtgg gaatcttgag actctttggc catttcccat ggctcagact aagctccaag 120
cccttcanga gttccaaggg aacacttgaa ccatggacaa gactctctca agatggcaaa 180
tggctaattg aggttctgaa gttcttcagt acattgctgt aggtcctgag gccagggatt 240
tttaattaaa tggggtgatg ggtggccaat accacaattc ctgctgaaaa acactcttcc 300
agtccaaaag cttcttgata cagaaaaaag agcctggatt tacagaaaca tatagatctg 360
gtttgaattc cagatcgagt ttacagttgt gaaatcttga aggtattact taacttcact 420
acagattgtc tagaagacct ttctaggagt tatctgattc tagaagggtc tatacttgtc 480
cttgtcttta agctatttga caactctacg tgtt 514

<210> 506
<211> 432
<212> DNA
<213> human

<220>
<221> misc_feature
<222> (197)..(197)
<223> n is a, c, g, or t
<220>
<221> misc_feature
<222> (268)..(268)
<223> n is a, c, g, or t
<220>
<221> misc_feature

<222>  (357)..(357)
<223>  n is a, c, g, or t
<220>
<221>  misc_feature
<222>  (364)..(365)
<223>  n is a, c, g, or t

<400>  506
gggctgttcc agagattcga tcctcttaag gcattatcag tgagcaaatg tgaaggaaat      60
ggtgtctgga agaaagttct ggttcacatg ccttgtagct aagtctttct gcaaacaacc     120
tcccttcccc ccgtcgagtc atttggtgac tttgatgggg ggatttctgg ttatgtcaag     180
gctctggaga caggaanggc ctttggccgc cttgggtagt tgacctgcct tttctgactc     240
cgggacgagc cagtcctagg ctgcctcngg gagcacttga ggtatcccgc aggccatgag     300
gacccactgg gcagctcctg gacagcctct tggctccagc ccccacccga aagtggnaca     360
ctgnntccgc cctggccacc tggggactgg cactgtggtg cacagtggcc caatgtggcc     420
aacggaagtt tt                                                         432

<210>  507
<211>  225
<212>  DNA
<213>  human

<220>
<221>  misc_feature
<222>  (35)..(36)
<223>  n is a, c, g, or t
<220>
<221>  misc_feature
<222>  (38)..(43)
<223>  n is a, c, g, or t

<400>  507
cacaggtgta gcactcccaa agcaagactc caganngnnn nnnacctcat gcctggcacc      60
tgaggtaccc agcagcctcc tgtctcccct ttcagccttc acagcagtga gctgcaatgt     120
tggagggctt catctcgggc tgcaaggacc ctgggaaagt tccagaactc cacgtccttg     180
tctcaattgt gccatcaact ttcagagcta tcatgagcca acctc                     225

<210>  508
<211>  224
<212>  DNA
<213>  human

<220>
<221>  misc_feature
<222>  (59)..(59)
<223>  n is a, c, g, or t

<400>  508
caccatgacc ggtcacagct acaaatccag agaccatcaa tcctgctaga gtgcagggng      60
gcaagcaccc aagggtggct gaccaagact gcagagtctc ctccatcttc aggtccattc     120
agcctcctgg catttaacta ccagcatcca gtggtcccca aggaatccct tcctagcctc     180
ctgacatgag tctgctggaa agagcatcca aacaaacaag taat                      224

<210>  509
<211>  177
<212>  DNA
<213>  human
<400>  509
ggttagattc cgttttgact gcttggtatc tcattgtaag catttcccag ggacactaca      60
cagtctttat aattatgatc actacatctt ttggataact taggcttttg cactattttg     120
tcacgatgcc tcaaactttt ctatcattgt ctgtaattac tcctataatc ttttatt        177

<210>  510

<211>  498
<212>  DNA
<213>  human
<400>  510
aatatctcaa gatttctagg cattttccta tgtctaatag taggatgttc tctctgaatt      60
tatttattac aaaaataaca ttacaacaat aaagcaaaat aatatttaaa agaaagaaaa     120
aacaaacagt cgcaatacca gaacaccatt gtagtcgttt gtgtatgtgt gacttcttta     180
ctgtgttcat gcagagacaa gctttgtaat acctttgtaa ctactgtaca ggtgcgtggt     240
tagattccgt tttgactgct tggtatctca ttgtaagcat ttcccaggga cactacacag     300
tctttataat tatgatcact acatcttttg gataacttag gcttttgcac tattttgtca     360
cgatgcctca aacttttcta tcattgtctg taattactcc tataatcttt tattataaaa     420
tattgaaaca aagtcacatc actaaaaaca ctgtgagtat aatttattag taagtgtaga     480
ctgtgtatca ttagcaaa                                                   498

<210>  511
<211>  453
<212>  DNA
<213>  human

<220>
<221>  misc_feature
<222>  (348)..(348)
<223>  n is a, c, g, or t

<400>  511
ggagctgctt caagaagaaa cccggcagaa gctcaacgtg tctacgaagc tgcgccagct      60
ggaggaggag cggaacagcc tgcaagacca gctggacgag gagatggagg ccaagcagaa     120
cctggagcgc cacatctcca ctctcaacat ccaggtgcct gccccgtgtc cttgcttcct     180
tcatgggtcc tctcaacttc tctgcgctga gatcccccgc aggcagatcg cggtggagtg     240
ttggtgcgat ggtgcttgac cccccagctt cccctgctat tgggtttctc caacgaggag     300
acatggtctt cgcttctcag agtctgtggg gccagggaca ggggccantc atggtccccc     360
tctcacccta ccctggacgc tgtccttgta gctctccgac tcgaagaaga agctgcagga     420
ctttgccagc accgtggaag ctctggaaga ggg                                  453

<210>  512
<211>  447
<212>  DNA
<213>  human

<220>
<221>  misc_feature
<222>  (49)..(49)
<223>  n is a, c, g, or t
<220>
<221>  misc_feature
<222>  (153)..(153)
<223>  n is a, c, g, or t
<220>
<221>  misc_feature
<222>  (177)..(177)
<223>  n is a, c, g, or t

<400>  512
tgttaccttt cagttacatg cctttgatcc taaaattctc tacttttgnt gccttatcag      60
ttctttgcaa tctgcctgtg gttatcagca cttaaagcac aattttgaag gggaaaaaaa     120
tgataatcac cttagtccca aagaaataat ttntgtcaaa ctgccttatt agtattnaaa     180
acagacacac tgaatgaagt agcatgatac gcatatatcc tactcagtat cattggcctt     240
ttatcaaatg gggaaactat acttttgtat tacatagttt tagaaatcga aagttagaga     300
ctctttataa gtaatgtcaa ggaacagtaa tttaaaaaca aagttctaac aaatatattg     360
tttgcttaat cacaatgccc tcaacttgta tttgaataac taaataggac atgtcttcct     420
tggagctgtg ggcattagtt cagaagc                                         447

<210>  513

<211>  469
<212>  DNA
<213>  human
<400>  513
gtttacacct atactgcata atccaacaat tttaatttca gttgaagaca tgttactaat      60
ataactatta ttaaaagagt agaggatgtg taattaacca tatcttctaa aacatggtta     120
ctaaaagaat atgtaacatc aatattgacc ttggtttctt acacaagtgt tgctaactca     180
atagtgaagg agacactatt aaattttctg aacccatgag agatactaga gatggggagt     240
ggaaagtgtt tggttcaggg atatctgaag aacagaaggg cagagatttc ttaagtgacg     300
cctcatctac aagctggaaa ttcctaaaaa caagtagaaa gcttataaac aacaggtgat     360
acactcacct cactggtttt agtaaattac caatacagaa agtatcccta gtcttaaaaa     420
caagtggaaa atttgaactg attagtcata ttcctttgat tacactgtt                 469

<210>  514
<211>  236
<212>  DNA
<213>  human

<220>
<221>  misc_feature
<222>  (135)..(135)
<223>  n is a, c, g, or t

<400>  514
cagtcagagt ggacaaacct ctgtgggagg ctctactgca ctgagatgca gctcttccga      60
gggggctcct aagccagtgt acaactgggt gcgtcttgga acttttccta caccttctcc     120
tggcagcatg gttcnagatg aggtgtctgg ccagctcatt ctcaccaacc tctccctgac     180
ctcctcgggc acctaccgct gtgtggccac caaccagatg ggcagtgcat cctgtg         236

<210>  515
<211>  488
<212>  DNA
<213>  human
<400>  515
gcctcctcag acactctcaa gaggatgggg agatgacatc acttgggtac aaacttatga      60
agaaggtctc ttttatgctc aaaaaagtaa gaagccatta atggttattc atcacctgga     120
ggattgtcaa tactctcaag cactaaagaa agtatttgcc caaaatgaag aaatacaaga     180
aatggctcag aataagttca tcatgctaaa ccttatgcat gaaaccactg ataagaattt     240
atcacctgat gggcaatatg tgcctagaat catgtttgta gacccttctt taacagttag     300
agctgacata gctggaagat actctaacag attgtacaca tatgagcctc gggatttacc     360
cctattgata gaaaacatga agaaagcatt aagacttatt cagtcagagc tataagagat     420
gatagaaaaa agccttcact tcaaagaagt caaatttcat gaagaaaacc tctggcacat     480
tgacaaat                                                              488

<210>  516
<211>  452
<212>  DNA
<213>  human

<220>
<221>  misc_feature
<222>  (34)..(34)
<223>  n is a, c, g, or t

<400>  516
aaggttatag ccacattcaa cttgaatggt cccnaatgtt aaatttacca tgaatactgt      60
gtcttacaat gctgagctct cgactagact aaccagaata ggaacacagt ctcaaaggat     120
accataagac aaacagtatt tagacatata aaaaactaca gagatgataa cttgaagaat     180
aaataacgtg aaaaactact atattcttga atgggagtag aatgtaagct ctttgagggc     240
agggactttg actgtcttgt tcattgctat atgataatgc ctggcccaac agcaggcatt     300
ctgtagtgtc tgttgaacaa acatctaaaa tctggcaaag tgaacaatgg ttcctgagga     360
taagtattta aataacaaaa ttaactttca ctacaaaagc tgccaactta cctttatctt     420
ctggtgctat cccctgagct ttcttaagat ca                                   452

<210>  517
<211>  399
<212>  DNA
<213>  human

<220>
<221>  misc_feature
<222>  (119)..(119)
<223>  n is a, c, g, or t

<400>  517
gaatgcatga tctcaagcct taactactat aatctttttc tgcccctcag aaattgaata      60
acctaaccaa gatgccttta ggggatgccc taagtaaatg taatttcaga tttcagggnt     120
tttttttttt tcctctctaa gtgttccttc cctttcttct cctgctctcc atcatgttat     180
ggagaccagt gaggaaccag tgttaacttg gtgacaatgt gacagctggt gctttatcta     240
agctccgttt tctatttctt gggaatgctt tattgtggaa actgcttcag atacttaaat     300
tgaatcataa cttgcttctg taaattgcgt aaagacaaca aactgatttt agtttgaaaa     360
gtttatcttt tacttgtaaa ccttgtttgc cagttacct                            399

<210>  518
<211>  529
<212>  DNA
<213>  human

<220>
<221>  misc_feature
<222>  (60)..(64)
<223>  n is a, c, g, or t
<220>
<221>  misc_feature
<222>  (68)..(70)
<223>  n is a, c, g, or t
<220>
<221>  misc_feature
<222>  (74)..(83)
<223>  n is a, c, g, or t
<220>
<221>  misc_feature
<222>  (86)..(87)
<223>  n is a, c, g, or t
<220>
<221>  misc_feature
<222>  (89)..(90)
<223>  n is a, c, g, or t
<220>
<221>  misc_feature
<222>  (92)..(92)
<223>  n is a, c, g, or t
<220>
<221>  misc_feature
<222>  (95)..(95)
<223>  n is a, c, g, or t
<220>
<221>  misc_feature
<222>  (99)..(99)
<223>  n is a, c, g, or t
<220>
<221>  misc_feature
<222>  (101)..(103)
<223>  n is a, c, g, or t
<220>
<221>  misc_feature

<222>  (111)..(112)
<223>  n is a, c, g, or t
<220>
<221>  misc_feature
<222>  (115)..(115)
<223>  n is a, c, g, or t
<220>
<221>  misc_feature
<222>  (121)..(121)
<223>  n is a, c, g, or t
<220>
<221>  misc_feature
<222>  (124)..(124)
<223>  n is a, c, g, or t
<220>
<221>  misc_feature
<222>  (127)..(127)
<223>  n is a, c, g, or t
<220>
<221>  misc_feature
<222>  (129)..(129)
<223>  n is a, c, g, or t
<220>
<221>  misc_feature
<222>  (432)..(432)
<223>  n is a, c, g, or t

<400>  518
agccccaggt gtatttctga gtgtcaacag tgttaatttc aagcatgcta ataaaatgtn      60
nnnncggnnn atannnnnnn nnnaannann cnttnaaant nnnggtttcc nntgntaaaa     120
ncanccncnt ttgcttttgt tagctgtaat attttttgtc atttagataa gacctggttt     180
ggctctcaat aaaagatgaa gacagtagct ctgtacaggg atatatctat attagtcttc     240
atctgatgaa tgaagaaatt ttctcatatt atgttcaaga aagtatttac ttcctaaaaa     300
tagaattccc gattctgtct attttggttg aataccagaa caaatctttc cgttgcaatc     360
ccagtaaaac gaaagaaaag gaatatctta cagactgttc atattagatg tatgtagact     420
gttaatttgc antttcccca tatttcctgc ctatcttacc cagataactt tctttgaagg     480
taaaagctgt gcaaaaggca tgagactcag gcctactctt tgtttaaat                529

<210>  519
<211>  281
<212>  DNA
<213>  human

<220>
<221>  misc_feature
<222>  (31)..(31)
<223>  n is a, c, g, or t

<400>  519
aatcttgtga gttgtgacct aggtgaaggt ntaatgataa catctggagg aagcatcatt      60
tgttcagcta cctagggttt actttcagac ttttcctaca gtttaaagca cttgcgttca     120
gtgtggtacg atctgtttgt aatgttaatc attgactatt gttctgctac ttggatgttg     180
atagtgaagc agagaacaat ttatcatcgt ttattatgag tcactatgca cgcaactatg     240
cttaacatgg cgaaatgtat taaacactcg tccaactatt t                        281

<210>  520
<211>  352
<212>  DNA
<213>  human
<400>  520
aaagggtggc atttgcgtca cgtggaccag ggacagtgct gaaatcagca gtgctcagaa      60
acaatttaac atgttgaaac gacaatattc taaaatactg atgaatcttg catcaatata     120
attattgggt ttttttcttt tttcctgctg tataactcct tgccatgcaa actctcaaga     180

ggccaatata ttcctggcca tgtttgaatg agcctcttaa aataaactta gagccatgca 240
aatgccagca gcttaatgga tttcatggaa tgaaataccg tgattaactc atagctacat 300
atcattgcat aaatgggatt tatctttttt ctcacttatt tttgcggtga aa 352

<210> 521
<211> 245
<212> DNA
<213> human
<400> 521
agagccatgc aaatgccagc agcttaatgg atttcatgga atgaaatacc gtgattaact 60
catagctaca tatcattgca taaatgggat ttatcttttt tctcacttat ttttgcggtg 120
aaagtcgagg gcatgcaaga gtttctcttc cagaagccaa gaggagaaca aaggtcctaa 180
tgctgtacta ttccaccctt tggacgcctc atccaggacg cagaggactc taggtttaac 240
atttt 245

<210> 522
<211> 495
<212> DNA
<213> human
<400> 522
gacccagagt cacctaagag aactcttcca acttaaatga ctagggtgtg taatcagttg 60
cttcttattc tgtgtctcag atttcctagt ccatgacatt taatagtgag tatagagtag 120
gatattctat acattttagt atttggccct tggaaacaaa gttaaatacc atgtcactca 180
gaagactctc aaagtttgtg tataaatggc gaagtatgca ctatgagaga ttctaatcca 240
ttcacatcat tcttgagttg tgcaaataca cttggctttg atttcacttg gtgatctaaa 300
tttgtttatt taagctgcaa gaaagttaca ttaatttgga atgtgtcatc acttggaccc 360
agtatatcag atttttattg agtaaaatgt taaaaataat aagtgtaact cagattctgg 420
atgttcgagt ttacaataca ttgcctgtaa taaaagctga ttctgaagtg catttttctt 480
gacttacttt ggcag 495

<210> 523
<211> 374
<212> DNA
<213> human
<400> 523
atgcacgtac aaatttctta acctgttatc aatgtctgag ctacataatt atctttctag 60
ttggagtttg ttttaggtgt gtaccaactg acatttcagt ttttctgttt gaagtccaat 120
gtattagtga ctctgtggct gctctcttca cctgcccctt gtggcctgtc tacaattcta 180
aatggatttt gaactcaatg tcgtcgcttc tggtttcctg catataccaa tagcattacc 240
tatgactttt tttttcctga gctattttca ctgagctgag ctaatgaact aaaactgagt 300
tatgtttaat atttgtatca aatacataaa aggaatactg ctttttcctt ttgtggctca 360
aaggtagctg catt 374

<210> 524
<211> 451
<212> DNA
<213> human

<220>
<221> misc_feature
<222> (271)..(271)
<223> n is a, c, g, or t

<400> 524
ggaatctttt ccaataacca gctaaagatt tgcactgaaa tacaacttgt atgccttttg 60
catttttaaa gcctgcttcc tggatttaag cagagtgata gtgttcaaag agccagttca 120
gcctgtaaca tatttgaaaa agatatgtct gcactttgag gtcccttttg aatgccattc 180
actagacctc tcaagcattt tgtttcattg ctacatccaa gcgcctcaca agtccacaat 240
gcgggacagc atcaaaagct caagactttg naaaaagctt gtgggcttgc actgggggag 300
ggaagggaac aaaatttgtg tacttctttg tttaatttag aaataaggca tccaagagat 360
gccattattt tctgtgtttc aattgttgtg cctttgagtt aaactgcatt tttgtctttt 420
ggttgaaatc tgaaatgtac tgtcccaata t 451

<210> 525
<211> 360
<212> DNA
<213> human
<400> 525
ctccttatct gttctagttc cgaagcagtt tcactcgaag ttgtgcagtc ctggttgcag 60
ctttccgcat ctgccttcgt ttcgtgtaga ttgacgcgtt tctttgtaat ttcagtgttt 120
ctgacaagat ttaaaaaaaa aaaaaaggaa aaaaaaagaa aaaatgaatt tactgctgca 180
ggtttttttc tctctccatg tgtcactaag tgaagtttgt gccttctata gcaaagagaa 240
tattttttac atcctactaa cagtagattt ttttgtagtg aacatttttt gtatttttat 300
ttataagtct cataagaaaa atagcaatgt tcagttgtat accttgaatc tgcagttaga 360

<210> 526
<211> 541
<212> DNA
<213> human
<400> 526
aataggtctt gcaggtttcc ttttgaattt taagtatcat aaatattttt taagtaaata 60
atacggggtg tcagtaatat ctgcagaatg aatgcagtct ttcatgctaa tgagttagtc 120
tggaaaaata aagtcttatt ttctatgttt tattcataga aatggagtat taatttttaa 180
tattttcacc atatgtgata acaaaggatc tttcatgaat gtccaagggt aagtcagtat 240
taattaatgc tgtattacaa ggcaatgcta ccttctttat tccccctttg aactaccttt 300
gaagtcacta tgagcacatg gatagaaatt taactttttt ttgtaaagca agcttaaaat 360
gtttatgtat acatacccag caacttttat aaatgtgtta aacaatttta ctgattttta 420
taataaatat tttggtaaga ttttgaataa tatgaattca ggcagatata ctaaactgct 480
tttatttact tgtttagaaa attgtatata tatgtttgtg tatcctaaca gctgctatga 540
a 541

<210> 527
<211> 484
<212> DNA
<213> human

<220>
<221> misc_feature
<222> (70)..(70)
<223> n is a, c, g, or t

<400> 527
gagaacatca gatctaatag agcatagtga tactatttaa tttaaccaaa gtctctagtg 60
aatatttcan ctttgaatgt aaactaacaa ataaacctga ccaccaagga gattgtttgc 120
ccagagtttc aaagcacatt gtctacaaat ggaaattgaa ataatttata aaatattgac 180
gttactatgt tttttaaaaa gttcctaatt ttttcactaa atggaggaaa ctattagttt 240
tattgttaaa tatggtagat attaatattc ctcttagatg accagtgatt ccaattgtcc 300
cagtttgaaa taagtaccct gtgagtatga gataaattag tgacaatcag aacaagtttc 360
agtatcagat gttcaagagg aagttgctat tgcattgatt ttaatatttg tacataaaca 420
ctgatttttt tgagcattat tttgtatttg ttgtacttta atacctggtg tacagttcca 480
gaaa 484

<210> 528
<211> 399
<212> DNA
<213> human

<220>
<221> misc_feature
<222> (119)..(120)
<223> n is a, c, g, or t
<220>
<221> misc_feature
<222> (145)..(145)
<223> n is a, c, g, or t

<400>  528
gaaggatgct gaggacctag atgagaagtt acctgcaaaa ggcaaaaggg ttacttagtg      60
tcagaaccaa ggcaatgact tctctctccc agatctccta gctactggtc ctgggccann     120
tttttttttt taaataatcc caacntttct ttaaaagaca agcatttcag taagctagtt     180
attttcatgg gttgctcatc catttttttc agtgatctaa aaatgtaggg agatggctac     240
tactgaagtt gtctgtctac ttgggataat agcaaattaa ttgaagacaa tgggaaagta     300
agttataaaa aatactggga aatctgtttc tcttctgagc aagcattcag ggcaggtata     360
aacatcaaac atagtgacat tgtcaaaacc tcttccatt                            399

<210>  529
<211>  420
<212>  DNA
<213>  human

<220>
<221>  misc_feature
<222>  (78)..(78)
<223>  n is a, c, g, or t
<220>
<221>  misc_feature
<222>  (161)..(161)
<223>  n is a, c, g, or t
<220>
<221>  misc_feature
<222>  (163)..(165)
<223>  n is a, c, g, or t

<400>  529
ggaaagagaa acgtggctca agtgctggct caccttctag ctgtgtggcc ctgggcaggt      60
tactgaggct cttccaanct cacttttcac atgtaaaatg gcatttctaa aagtacctac     120
cctacctcaa aggaattctg aaaaaagtaa gtgaatttgt nannnaagca aagcactcac     180
aatatgcctg acacacaaac tgcttaataa atgttagtaa tttttaaaac ctcctcataa     240
gcggcttaga aattattcat gtttggtaca tatttattgc aagtcaagca gatccaactt     300
ttttgaaagc tggtggtcta ggagtatagt tttaattata tttgtaaata ctcaaaagta     360
ttagatgcca gtctaattta aatgcagtct gactgtagcg agtaactgac ctgggcaatg     420

<210>  530
<211>  378
<212>  DNA
<213>  human
<400>  530
gggactcagt tcttagagaa gtcttttggt cttagagttc atcaagcaga gaaggggaag      60
gacatggtcc aggaaaataa atcagcacac aaagagatgg ggtcttgagg gtgcatagta     120
cattctggac agccagttac ctgggatgag ttgggaggga ggagaataag gacaaaagac     180
catctgggca aaaatcacga aggggtatgt gtgtcatgta aaggtgtgcc atgatagtta     240
ttcatattgc tattgtaata ttaatatata gtaattaact acacatgaca cagctttaca     300
tgaccttaag tagttatcaa cattaccata atagtaatat taataactac aataagagcc     360
attattattc acttgagg                                                   378

<210>  531
<211>  360
<212>  DNA
<213>  human
<400>  531
gttagcaagt gccagcttgt aggctggttg aagtacagaa ctcagaggaa aaaagaaatt      60
aaattttagc tttctggaga gcagcccctc tctggcacca tcaaacactt ctttgtttcc     120
cttcaacttg gaactcttca aacatcaggg gttgtgaggg tttggccatt cttttatctt     180
gggtccatgt gagtgacaga aatggtgcgg cctgggaaag atctccctcc tttacatttt     240
ctcttctccc tcctcctcct tattctaaaa ctgtgcctcc aacagagggg caggggctct     300
tgtagagaga tccctggccc aggacaggag atgccaaatc taatttatct cactgagggc     360

<210>  532
<211>  502

<212>  DNA
<213>  human

<220>
<221>  misc_feature
<222>  (371)..(371)
<223>  n is a, c, g, or t

<400>  532
tttcctacca atttcacatt ttgcagaaac ttgttcacat ttccaacaat atcagaatta     60
gaaaacagtt cagataacaa gaaagattaa aaattaggga aattctgata tcaccataaa    120
gcactatttt acatttagag attacattta agataaagtc atcatacaca aaaacaataa    180
atatttataa ctttctctat aaggtccgca tatactgtat atattgaaac aatctgaatg    240
actagtagat ttcatatgac cattgttatt tccactttct ccaatacttg tattttatgc    300
tacatgtaat gaagttggac ctttttatta tttagtaatt cctatatgtt cctatacttt    360
tcattttcaa natgattgct ctattgtttc atgttgtttc tagcaatata tctccatgag    420
atatgcactt tgtttcatat tgaaaagtat aaaatttatc tttcaattcc tgtgtgtgta    480
tcctatggtt atctgtatgt at                                             502

<210>  533
<211>  258
<212>  DNA
<213>  human
<400>  533
tcccaacccg gttctggcaa agaatccagt tatcccttcc atgaagacgc acataactct     60
cttacttggt ctttccatta gggacaacat aagtcttgtt ttacatcaaa taaaaacaat    120
gttaaaaagt gtgtgaacct taaaaatgga agtctactag tttacatacc tacttcagag    180
gacatggaaa tgaccatggg cctgcatttc agggaccaaa gcaaattagg cctggcctaa    240
aatacatcag accttttg                                                  258

<210>  534
<211>  460
<212>  DNA
<213>  human
<400>  534
gttgacccca actttggaac tcagatcata ggatctccca gaggtttcag gtgttcctaa     60
gaagcttcta aaggaagttt tggaagaaag actaataggt gtaaacatat aaacatatat    120
attatatata gctatatata tttaaagaga tctctacata tatatatata taaatacaac    180
tactgtactc tctgcatctg tacaaagtat gagttaaact gcccaggtag tgtgagatac    240
gtaatgcatg aatgtgaata tcctgaaatc tgccttctta gcaacgtgcc gcgtgtactg    300
gagagaggac agttgctcct gggagttctc tcaatgggca aatgtacaag ttgatgccgt    360
cttatccacc aacaagccat taaggttggt ttcctgctga gacttttgta ccggaagcga    420
gaccggcagc actgggggtg tccatatgcc acttatgagg                          460

<210>  535
<211>  493
<212>  DNA
<213>  human

<220>
<221>  misc_feature
<222>  (280)..(280)
<223>  n is a, c, g, or t

<400>  535
ggccaaggca catttgactc ctgagatgaa ttttttgtgg tcataatcaa atacttagtt     60
gtttttgatg ccccaaaata aagtgagaat ggtaatttgc caggaattct tcataacagt    120
atcttacaaa aaacgtgttg ctctcttcac agtattatgt gtaaagtcat tgtttaaagc    180
acgaatgttc cctctggggt acttgttaaa gctaaattta ttttgcttcc ctccacttag    240
aagtgctgca cactttacag cagcttcctt tctttccatn gcactgccta gttaacagaa    300
gtcttataaa aatttaaaaa gacacatttc ttacaaaaaa gagttgaatg aggtaaaatg    360
gcattagatg gctctatatt ttttaaagct atgtaattgt tcagcgtcac ttttctaagt    420
acttatacat atctaaacat gtcttcatgg tttatatttt cacttatata tgctgggctg    480

gattaagctt tgt                                                          493

<210>  536
<211>  486
<212>  DNA
<213>  human

<220>
<221>  misc_feature
<222>  (28)..(28)
<223>  n is a, c, g, or t
<220>
<221>  misc_feature
<222>  (31)..(31)
<223>  n is a, c, g, or t
<220>
<221>  misc_feature
<222>  (39)..(39)
<223>  n is a, c, g, or t
<220>
<221>  misc_feature
<222>  (118)..(149)
<223>  n is a, c, g, or t

<400>  536
ggggcagagg gactatgttg tgagcctngc naaagaagnt tgtgtgggga ctgtgggcag      60
tgaatgcgtt gggaacaata tggaaaactg ggagctgcct tggaatctac agggccgnnn     120
nnnnnnnnnn nnnnnnnnnn nnnnnnnnng gctgaagaaa agaagaatgg tattcatgca     180
gttcccattt tacagatgaa aacaagagga ctttttctgt gaagtcaaga aagtggttac     240
aatggtactt tcagcctgtc cgaattatgt attgcccctc ccctttttat taataacatt      300
gaagtgtgat gggacaacca ctgaagccgt ctgttgaaac ctgctgggac tttttagcca     360
ttctcttcaa cataaagaat gggtgttttt ggagggggtg agaggaatgg ggaaatgttg     420
tcaaagagta caatgtttta gttgagacag gaggaatata ttttgttgag atctacagca     480
cagcat                                                                486

<210>  537
<211>  299
<212>  DNA
<213>  human
<400>  537
gatagcgaat gcactcaggg tcagcagtgg agtttaaaaa ttgtctcttt tcaacttatt      60
taaatgacag cacctgagaa gaggaaccgt tttacactgg atgtttctca tgtagaacaa     120
gaaatctttc tggaattgat gtttacatgt ctgttgttgg tcatctctcc tgtgtcttaa     180
atactttaat gttggaagag catagtgttt gggctagtgg gtttctgaca gcccatggga     240
atgccctgaa actactgtat ctgatgtttg ttttcgatga ggttccatgt tttgttttc      299

<210>  538
<211>  367
<212>  DNA
<213>  human

<220>
<221>  misc_feature
<222>  (27)..(27)
<223>  n is a, c, g, or t
<220>
<221>  misc_feature
<222>  (32)..(32)
<223>  n is a, c, g, or t

<400>  538
gcagctgtgg tggtcattat aagcatngtc cntcctggga agaagcatcc aggcaagcag      60
aaaagaaaag atgcagccac cagaaaaaga aactccagaa gtcctgcatt tggatgaggc     120

caaggatcac aacagcctaa acaacctaag agaaactttg ctctcagaaa agccaaactt     180
ggcccaggtg gaacttgagt taaaagagag agatgtgctg tcagttttcc ttccggatgt     240
accagaaact gagagctagt gagggttcag agaagcccca tcctaagcca gacacatgat     300
gtgggctcag ctcagtggcc tgaaacctct caggttttag agtctctccc aagaagccgc     360
ttttttc                                                               367

<210>  539
<211>  394
<212>  DNA
<213>  human

<220>
<221>  misc_feature
<222>  (54)..(54)
<223>  n is a, c, g, or t
<220>
<221>  misc_feature
<222>  (58)..(59)
<223>  n is a, c, g, or t
<220>
<221>  misc_feature
<222>  (61)..(61)
<223>  n is a, c, g, or t
<220>
<221>  misc_feature
<222>  (65)..(65)
<223>  n is a, c, g, or t
<220>
<221>  misc_feature
<222>  (68)..(69)
<223>  n is a, c, g, or t
<220>
<221>  misc_feature
<222>  (71)..(72)
<223>  n is a, c, g, or t
<220>
<221>  misc_feature
<222>  (74)..(74)
<223>  n is a, c, g, or t
<220>
<221>  misc_feature
<222>  (76)..(76)
<223>  n is a, c, g, or t
<220>
<221>  misc_feature
<222>  (78)..(81)
<223>  n is a, c, g, or t
<220>
<221>  misc_feature
<222>  (83)..(84)
<223>  n is a, c, g, or t
<220>
<221>  misc_feature
<222>  (95)..(95)
<223>  n is a, c, g, or t
<220>
<221>  misc_feature
<222>  (115)..(115)
<223>  n is a, c, g, or t
<220>
<221>  misc_feature
<222>  (127)..(127)
<223>  n is a, c, g, or t

<220>
<221>  misc_feature
<222>  (129)..(129)
<223>  n is a, c, g, or t
<220>
<221>  misc_feature
<222>  (219)..(219)
<223>  n is a, c, g, or t

<400>  539
tactagttgt tttacactct cttttcttat tcttagggct tttgtgtatg tctnactnnt      60
ntttnaanna nntncncnnn nanncagacc ttaantttta tattttttta aagtngctaa     120
catagcngna ggcacttaag catttagtca atgatattgg tagaaatagt aaaatacatc     180
ctttaaatat atatctaagc atatatttta aaaggagcna aaataaaacc aaagtgttag     240
taaatttgga tttattagat attttagaaa aataatagaa ttctgaagtt ttaaaaatgt     300
cagtaattaa tttattttca ttttcagaaa tatatgcatg cagttatgtt ttatttgatt     360
gttgacttag gctatgtctg tatacagtaa ccaa                                 394

<210>  540
<211>  452
<212>  DNA
<213>  human

<220>
<221>  misc_feature
<222>  (72)..(72)
<223>  n is a, c, g, or t
<220>
<221>  misc_feature
<222>  (86)..(86)
<223>  n is a, c, g, or t
<220>
<221>  misc_feature
<222>  (93)..(93)
<223>  n is a, c, g, or t
<220>
<221>  misc_feature
<222>  (96)..(96)
<223>  n is a, c, g, or t
<220>
<221>  misc_feature
<222>  (98)..(98)
<223>  n is a, c, g, or t
<220>
<221>  misc_feature
<222>  (107)..(108)
<223>  n is a, c, g, or t
<220>
<221>  misc_feature
<222>  (111)..(111)
<223>  n is a, c, g, or t
<220>
<221>  misc_feature
<222>  (121)..(121)
<223>  n is a, c, g, or t
<220>
<221>  misc_feature
<222>  (124)..(124)
<223>  n is a, c, g, or t
<220>
<221>  misc_feature
<222>  (130)..(130)
<223>  n is a, c, g, or t

<220>
<221>  misc_feature
<222>  (264)..(266)
<223>  n is a, c, g, or t
<220>
<221>  misc_feature
<222>  (269)..(269)
<223>  n is a, c, g, or t
<220>
<221>  misc_feature
<222>  (273)..(274)
<223>  n is a, c, g, or t
<220>
<221>  misc_feature
<222>  (277)..(278)
<223>  n is a, c, g, or t
<220>
<221>  misc_feature
<222>  (281)..(284)
<223>  n is a, c, g, or t
<220>
<221>  misc_feature
<222>  (328)..(328)
<223>  n is a, c, g, or t
<220>
<221>  misc_feature
<222>  (362)..(362)
<223>  n is a, c, g, or t
<220>
<221>  misc_feature
<222>  (373)..(373)
<223>  n is a, c, g, or t
<220>
<221>  misc_feature
<222>  (379)..(379)
<223>  n is a, c, g, or t

<400>  540
gagtaggttc ggtctgaaag gtgtgggcct ttatatttga tccacacacg ttggtctttt      60
aaccgtgctg ancagaaaac aaaacnggtt aanaanancc gggtggnnag ntgacagagg     120
naanccgctn aaataccttc acaataaata gtggcaatat atatatagtt taagaaggct     180
ccccatttgg catcgtttaa tttaaatgtt atgttataag cacagctctc ttctccaatt     240
ttcatcctgc aagcaactca aaannnttna aannaanntt nnnntgtagt tattttcaaa     300
tctttgcttg ataagtatta agaaatantg gacttgctgc cgtaatttaa agctctgttg     360
antttgtttc cgnttggant tttgggggag gggagcactg tgtttatgct ggaatatgaa     420
gtctgagacc ttccggtgct gggaacacac aa                                   452

<210>  541
<211>  457
<212>  DNA
<213>  human

<220>
<221>  misc_feature
<222>  (364)..(366)
<223>  n is a, c, g, or t
<220>
<221>  misc_feature
<222>  (369)..(369)
<223>  n is a, c, g, or t
<220>
<221>  misc_feature
<222>  (379)..(379)

<223>  n is a, c, g, or t
<220>
<221>  misc_feature
<222>  (382)..(383)
<223>  n is a, c, g, or t
<220>
<221>  misc_feature
<222>  (398)..(398)
<223>  n is a, c, g, or t
<220>
<221>  misc_feature
<222>  (407)..(407)
<223>  n is a, c, g, or t

<400>  541
ggaacctata aatgtagttt catctgtcgt caattactgc agaaaattat gtgtatccac      60
aacctagtta ttttaaaaat tatgttgact aaatacaaag tttgttttct aaaatgtaaa     120
tatttgccac aatgtaaagc aaatcttagc tatattttaa atcataaata acatgttcaa     180
gatacttaac aatttattta aaatctaaga ttgctctaac gtctagtgaa aaaaatattt     240
ttaaaatttc agccaaataa tgcattttat ttataaaaat acagacagaa aattagggag     300
aaacctctag ttttgccaat agaaaatgct tcttccattg aataaaagtt atttcaaatt     360
gaannngtnc ctttcacang tnnatgatta aatctgantt cttaatnata tatcctatgc     420
tgattttccc aaaacatgac ccatagtatt aaataca                              457

<210>  542
<211>  344
<212>  DNA
<213>  human
<400>  542
tctttttgag tgatagtgcc attgaatgag cagtatggaa acagtgttac ttgatatttt      60
gagctttctc aggtttatct aaatcagtgg tagcttaaca aaacccagac taattgtgtg     120
taattgtatt tttaataaaa ggaaagtaca tttcctataa tagcatagta ctgtttgcat     180
gtaagagtat gcaaaacctt gtgtgtgtgt gtgtgtgtgt gtgtgtgtgt gtgtcttagt     240
gtgtgtaagg catggcagcc aactttgtat ctgctatttt tagtacgagc agagcttcat     300
aattgtggtc actagaactg tacttaccat ggacagttaa aact                      344

<210>  543
<211>  414
<212>  DNA
<213>  human
<400>  543
tctctcagtt taaacatctt aacagagaat ttgttactgt ttattaaaag aattgcattt      60
ggaagcaaga aaacaaccgg atttgcaatc atgtgaagaa acaataatgc ctttattatc     120
aagtgttaag cacaattctt ataacaaact gtgataaatt ctttttgttt ttttttcctt     180
tgccccatta ttttcttatg aacaaaccaa aaattcatgg tgagcagttg cagtgttggc     240
tgatatatct tttatgtaca gggaatttga aaaggacagt ggattcattt agaagtgtaa     300
ctggtgctgt gattatagca atacatttgt tagttgtact tcatcttttt catgctagct     360
ttttaaatgt ttagttttcc tcttgtcatg gtcagctgct gaatttactt gaag           414

<210>  544
<211>  545
<212>  DNA
<213>  human
<400>  544
agattctggc cacggctggg aacgttgtca ccctgaccat catccccagt gtgatctacg      60
agcacatggt caaaaagttg cctccagtcc tgctccacca caccatggac cactccatcc     120
cagatgcctg aagccactgc agggcagggc aggcaggggg ggcttcccgc cctcctgcag     180
caaagggcaa ccaccctcgg atgatgggtt gcagccggcc tgctgcttaa ggtgggggct     240
gccatgaggg gggcgtgtcc aggagggtga ccatgggatg gcttatacac acaggcctcc     300
ttggagcctc agactccaag ctaggctgag gctcaggcag ggcccacagg cagccgattc     360
tcttgtgctg atttaaatgc tggacacgga ggcaggctgt ttaaacgctg cttaaagtcg     420
caactgggcc cctttcaaga aattttgctc taccaggaaa acagttacac attttaagag     480
aacagagcta cgttctttgt gagagctttt tccttgcctt gacttgctct ttgtcacaga     540

ctgca                                                                 545

<210>  545
<211>  248
<212>  DNA
<213>  human
<400>  545
gtcaccatct cttgttctgg aagcagctcc aacatcggaa gtaattatgt atactggtac      60
cagcagctcc caggaacggc ccccaaactc ctcatctata ggaataatca gcggccctca     120
ggggtccctg accgattctc tggctccaag tctggcacct cagcctccct ggccatcagt     180
gggctccggt ccgaggatga ggctgattat tactgtgcag catgggatga cagattgagt     240
ggttttat                                                              248

<210>  546
<211>  329
<212>  DNA
<213>  human

<220>
<221>  misc_feature
<222>  (73)..(73)
<223>  n is a, c, g, or t
<220>
<221>  misc_feature
<222>  (298)..(298)
<223>  n is a, c, g, or t

<400>  546
gtttactctg agattctgta gtcctaatat tgtatcattg tgctgtctgc aaaacaactt      60
gaatctattt tgnttgcatc ttttgttaca tgtaacgcag ctgtacttta tgttctttgc     120
aactgtttcc attatgagaa cgctgtgcta tttacaaggt tacatttttc ttggccaggc     180
gaggtggtca tgcctgtaat cccagcactt tgggaggcca aggtgggcgg atcacttgag     240
gtaaagagtt gagaccagcc tggctagcat ggcgaaaccc agtctctact aaaaatanca     300
aaaattagcc gggtgaaatt agccgggcg                                       329

<210>  547
<211>  488
<212>  DNA
<213>  human
<400>  547
ggtgttttaa ggttagcaca tgcacaaagg aacgagttgg tttaaaaaga taaatcactg      60
caaagaatga aattggctta ttcacatcaa aactagataa gatgctaaaa aaaaaagata     120
tgaaacagaa ctaacctata gtttcctgaa atcagtacag tttaatttat aagaagctag     180
aaagtaatgc accttgattg ttttaggaat gatttatgtg ttgcaatttt aatttattta     240
aagcatgtct actgtgtttg tcctaagaga aatatttcaa caaaacgtgc tctgtgttta     300
agatatgttt aggcagtagt tagcaactct gaaagtagaa actggaaatg tttattgtga     360
ggcttgttgc agaatttcca ttttgtgagt tactacttag tttcatgtca gcctaaaatt     420
gtaaattccc tgtagatctt caccccattg tggtgtcatc aatgaatcca aagcaggtgc     480
cattattt                                                              488

<210>  548
<211>  373
<212>  DNA
<213>  human

<220>
<221>  misc_feature
<222>  (67)..(67)
<223>  n is a, c, g, or t
<220>
<221>  misc_feature
<222>  (74)..(74)
<223>  n is a, c, g, or t

<400>  548
gcagggtcat aatcacacag cagtgcctta tatagttgcc ataagacttc agtgcagtac      60
aacatanttt tacngctaca tatcagggca tattctatat ggtgtatttg tgttagaata     120
acacattaaa tgtctttaaa cataaaaata agaatgtttg catgtttcag ttttcaagaa     180
ccaaatgagt aattagctat agattccact ggccttaaac atacaattaa gtgtatacat     240
gatatagtgc acacacaaaa gccaccttta attattgaaa taacctgtat tctttttgga     300
aatcatttaa gtttggtatt gaagtactat attttttgtg catcaatgta tttttctatt     360
tacaagccta tgt                                                        373

<210>  549
<211>  498
<212>  DNA
<213>  human
<400>  549
ggagatcgtg ttctgctttt tacatgagga gcagaactgg gccatacacg tgttcaagaa      60
ctaggggagc tacctggtag caagtgagtg cagacccacc tcaccttggg ggaatctcaa     120
actcataggc ctcagataca cgatcacctg tcatatcagg tgagcactgg cctgcttggg     180
gagagacctg ggcccctcca ggtgtaggaa cagcaacact cctggctgac aactaagcca     240
atatggccct aggtcattct tgcttccaat atgcttgcca ctccttaaat gtcctaatga     300
tgagaaactc tctttctgac caattgctat gtttacataa cacgcatgta ctcatgcatc     360
ccttgccaga gcccatatat gtatgcatat ataaacatag cactttttac tacatagctc     420
agcacattgc aaggtttgca tttaagttaa aaaaaaaaag aaaaaaaacc taagggtgaa     480
acgatgccac tttgacgc                                                   498

<210>  550
<211>  551
<212>  DNA
<213>  human

<220>
<221>  misc_feature
<222>  (77)..(77)
<223>  n is a, c, g, or t
<220>
<221>  misc_feature
<222>  (228)..(228)
<223>  n is a, c, g, or t
<220>
<221>  misc_feature
<222>  (340)..(340)
<223>  n is a, c, g, or t
<220>
<221>  misc_feature
<222>  (342)..(346)
<223>  n is a, c, g, or t
<220>
<221>  misc_feature
<222>  (349)..(353)
<223>  n is a, c, g, or t
<220>
<221>  misc_feature
<222>  (355)..(356)
<223>  n is a, c, g, or t
<220>
<221>  misc_feature
<222>  (358)..(359)
<223>  n is a, c, g, or t
<220>
<221>  misc_feature
<222>  (361)..(361)
<223>  n is a, c, g, or t

<400>  550
tgactttcat acacacccag tacatctcaa aggatgctaa gggacatttt ctgccagtag      60
agttctcccc ctttttnggt gacagcaata ttattatgtt cacatctaac tccagagctt     120
acttcctgtg gtgccaatgt atttgttgca atttactaca tttttatatg agcctattta     180
taggtgccat taaactcagg tctttcaaat gaaagagttt ctagcccnct tagggaaaaa     240
gataattgtt tagaaaacca taaaatcaat ggtaggaaaa gttggaactg gttacctgga     300
tgccatggtt ctctgttaaa taaagtaaga gaccaggtgn annnnnaann nnnannanng     360
ntattttcag catgctaata aatgtctttc cggttatata tctatctaaa attaaccttt     420
aaaatattgg tttccttgat aaaagcacca ctttggcttt tgttagctgt aatattttgt     480
catttagatt aagacctggt taggctctca ataaaagatg aagacagtag ctctgtacag     540
cggataatat c                                                          551

<210>  551
<211>  537
<212>  DNA
<213>  human

<220>
<221>  misc_feature
<222>  (109)..(109)
<223>  n is a, c, g, or t

<400>  551
gctagttgca ttatactggg tcatgaaaaa ttatcccttg aaatagatat gaaacatgtt      60
acttcatttc tggtttaaat aacttgtgga atctttccta atgacaacnt gatattaagg     120
gaaactaaag aaaatgttat tgtggatccc acagtactat attacactgt ttttttttgt      180
ttgttttgtt agtttttttt tatttaaagc aaacctcaaa cattattggg tatcaattac      240
cacctggttg tattaaaata gtaacttatc aatgccatgt aaaaattaat tccattttcg     300
aagccacctg gcagacaggt ttagctgttt catcagcagc ctaatatata ctgttaaatt     360
tgttaaggat ttcactttga aggatacatg caaaacatat agttactatt ttcatgagtc     420
ctgcttctag ctccattgtg gaatacagaa aattaaatat acctgttaag ttcgtatcta     480
aacctaagac attaccaagg tttgtacaaa ttctactacc tgacatttat tccaaga        537

<210>  552
<211>  532
<212>  DNA
<213>  human

<220>
<221>  misc_feature
<222>  (69)..(69)
<223>  n is a, c, g, or t
<220>
<221>  misc_feature
<222>  (131)..(131)
<223>  n is a, c, g, or t
<220>
<221>  misc_feature
<222>  (283)..(283)
<223>  n is a, c, g, or t
<220>
<221>  misc_feature
<222>  (412)..(412)
<223>  n is a, c, g, or t

<400>  552
ctcccctgaa gaacgtgtcc agcaacgcag agtgccctgc ttgttatgaa tctaatggaa      60
cttcctgtng tgggaagccc tggaaatgct atgaagaaga acagtgtgtc tttctagttg     120
cagaacttaa naatgacatt gagtctaaga gtctcgtgct gaaaggctgt tccaacgtca     180
gtaacgccac ctgtcagttc ctgtctggtg aaaacaagac tcttggagga gtcatctttc     240
gaaagtttga gtgtgcaaat gtaaacagct taacccccac gtntgcacca accacttccc     300
acaacgtggg ctccaaagct tccctctacc tcttggccct tgccagcctc cttcttcggg     360
gactgctgcc ctgaggtcct ggggctgcac tttgcccagc accccatttc tncttctctg     420

aggtccagag caccccctgc ggtgctgaca ccctctttcc ctgctctgcc ccgtttaact 480
gcccagtaag tgggagtcac aggtctccag gcaatgccga cagctgcctt gt 532

<210> 553
<211> 521
<212> DNA
<213> human

<220>
<221> misc_feature
<222> (37)..(37)
<223> n is a, c, g, or t

<400> 553
tttgtccata gaggcttcaa gatagatagg taagagncca gtagtgttca taagaagcca 60
atagagagca ggagccactt tatcaggtgg caggtgtcct gggcctccct gctggctagt 120
cccaagcggt ggtgttgcca ggatgtcttg gaggtgataa tgggacacac agaggcactg 180
agtctccata ggttaaaatg ccaccaaaac tggcctttgc ctaatatccc tcattgacta 240
tttagcattt aatttattta ttttcctgac atttctgcaa gctttgtatt tatatttcca 300
ctttatagat gaggaaattt gaggctctta gaggtaaaat gacttgccca ggtcacacag 360
gaagtggcag agacaagctt tttaaataag aaaaaattaa taaaatataa tatgagagta 420
acttaaaata ttaataaacc acaattttaa attaattaac cgtgataacc aacattaata 480
aaagttaaga taccaaaaca ctggtgtcta attctttcaa c 521

<210> 554
<211> 410
<212> DNA
<213> human

<220>
<221> misc_feature
<222> (45)..(45)
<223> n is a, c, g, or t

<400> 554
gttctgttgt ttatccatga agcctatata tttctagtgc aaaangccca gtgggtctca 60
aaacattctt ggaggggttt gacacactgc tattagtagt gactcataat tccacgattc 120
cctgggatac agagaaggca ggggggtagt actgtttgaa caaaaatctt cccaagaccc 180
tggcaaagag atcttcctta ggaatcagtg gcatatgcgt ttatgttctt taacagggaa 240
agtaaaagtg agcgcacttt tttgaagctc ataaatactc atgtgactgt aaatttagaa 300
atgttaatta attgggccta atgtttgatc tggaaaagta cttagcagtc accaggaatt 360
tgccttgaac tgactcgaac cacaaatcag aaccgtattt ccacaattta 410

<210> 555
<211> 296
<212> DNA
<213> human

<220>
<221> misc_feature
<222> (123)..(123)
<223> n is a, c, g, or t

<400> 555
gcactgttaa aatccggcta ggccaagatt tcccaatgtc ttgaagccaa tggctaggaa 60
aacagctgag cctgggactg gactttgtca gtaacctctc ttccctaggg cacgggagga 120
gtngggtgaa taggacctct tggcttgagg accatgattg tgaatacaga catttaattc 180
agaatgacac ctacattgat gaattttcat tgtacaagtg tttttccaga tcaaacacaa 240
aacccatgta attccttgat aaattttcaa tgcagggcag ataaactgtt gctaac 296

<210> 556
<211> 432
<212> DNA

<213>  human

<220>
<221>  misc_feature
<222>  (26)..(26)
<223>  n is a, c, g, or t
<220>
<221>  misc_feature
<222>  (332)..(332)
<223>  n is a, c, g, or t

<400>  556
gtgatcttgg actgtcaatt cccctncctg tgatccattt tactgcaaac ataagggttg      60
cagtaaaggg ttgtctcacg tcttctgctt taaaagccta taaatatatg acctgaaaac     120
tccagttaca taaaggatct gcagctatct aaggcttggt tttcttactg tcatatgata     180
cctgggtcta atgaactctg ctgagatcac ctcaagtttc tgcggttggt aaagagaaca     240
agggaagaac aaacatccct tttattgctc caaatggtga tttaatccct acatggtgct     300
gggtggacaa tgtgtcactg tcacatgcct tnactgtata aatccaacct tctgccagag     360
agaatctgtg gttctggcca tggagggagg atagtggaaa tgatatagtt ggactggtgc     420
ttgatgtcac ta                                                         432

<210>  557
<211>  383
<212>  DNA
<213>  human

<220>
<221>  misc_feature
<222>  (42)..(43)
<223>  n is a, c, g, or t
<220>
<221>  misc_feature
<222>  (85)..(85)
<223>  n is a, c, g, or t
<220>
<221>  misc_feature
<222>  (93)..(93)
<223>  n is a, c, g, or t
<220>
<221>  misc_feature
<222>  (99)..(99)
<223>  n is a, c, g, or t
<220>
<221>  misc_feature
<222>  (106)..(106)
<223>  n is a, c, g, or t

<400>  557
atgtgattat acttctcttt gacttgtcag cttagcttta gnngatacac tctggtgccc      60
aactattatt gtatcagtga acttnccact ttncttttnc cttttnctct caatttttgt     120
tgtatcattc ctaccttgtg aggacatata atatttacat tctgttgtca tcctcacatt     180
tcttagttcc acagtttaaa tgtatttgaa actcaaaaca ttcccattaa tctcttggtc     240
agctgaaatt aatgatttaa tagtttcctt aaaaaagact catggaacaa tttccctaaa     300
tttttgccat gtcaaatatg tttatctgta gcctttacac agtaaaaaca atttggctag     360
ataatacaat tctcagttca tat                                             383

<210>  558
<211>  391
<212>  DNA
<213>  human
<400>  558
agggcacttc tcaacattaa ttttggaatt attcaccatt taaaaattgt cactgctctt      60
tttcattact gtagataact gagagttagc tgttctagga aaaggccatg tttcagttag     120

tgctatgact tcttttttcg attaaggttg aagcagtgtt ctcttagtag cctgaacact 180
tctcaaagcg acttatcact agcggcttta atattgagct tctatatcgt tgtgtccacc 240
tcacttttta aggctgttca tacctggcaa tattcactta ctctgctatt tttaaaaact 300
attttaaagt tttaagcaca atgttgactc ttctgtaatt tcctaaaatt atattgtttt 360
cctacagaca gctcaacttt ccttgatatg t 391

<210> 559
<211> 301
<212> DNA
<213> human
<400> 559
ctcattattc ctttacatgc agaatagagg catttatgca aattgaactg cagtttttca 60
gcatatacac aatgtcttgt gcaacagaaa aacatgttgg ggaaatattc ctcagtggag 120
agtcgttctc atgctgacgg ggagaacgaa agtgacaggg gtttcctcat aagttttgta 180
tgaaatatct ctacaaacct caattagttc tactctacac tttcactatc atcaacactg 240
agactatcct gtctcaccta caaatgtgga aactttacat tgttcgattt ttcagcagac 300
t 301

<210> 560
<211> 463
<212> DNA
<213> human

<220>
<221> misc_feature
<222> (226)..(226)
<223> n is a, c, g, or t

<400> 560
ataaatgacc agactttttc taagaaaaat gttgctttaa tgcatttcat gaatttttac 60
tcttatatca ttgcttgcta gtaatagcaa atctgctttt ctgcatctgc tttgcgtagc 120
tattgtaagg ctttgaacta atgtatgtat ttattgcttg aacttctgtg cataccttat 180
aaagcataat gtctgacaat ttaaatggct catgtattct tgcttntatc ataagctgat 240
tatggggact atgatctttt gtatacagca aattttaaac tgtagcacaa acatctgttt 300
atgtattggt ggaatatacc tgttttattt atcttttttg aggtaaacta atttttgata 360
cttttcatta ctgtgtacta tgttcatact ttgaattctc tgacgttaga agtcatggtt 420
gagaattgta acagctgtta ttcgttctgt attcatggct ttc 463

<210> 561
<211> 541
<212> DNA
<213> human
<400> 561
aaaagcgagg tggccatgtt atgctggtgg ttgccagggc ctccaaccac tgtgccactg 60
acttgctgtg tgaccctggg caagtcactt aactataagg tgcctcagtt ttccttctgt 120
taaaatgggg ataataatac tgacctacct caaagggcag ttttgaggca tgactaatgc 180
tttttagaaa gcattttggg atccttcagc acaggaattc tcaagacctg agtatttttt 240
ataataggaa tgtccaccat gaacttgata cgtccgtgtg tcccagatgc tgtcattagt 300
ctatatggtt ctccaagaaa ctgaatgaat ccattggaga agcggtggat aactagccag 360
acaaaatttg agaatacata aacaacgcat tgccacggaa acatacagag gatgcctttt 420
ctgtgattgg gtgggatttt ttcccttttt atgtgggata tagtagttac ttgtgacaag 480
aataattttg gaataatttc tattaatatc aactctgaag ctaattgtac taatctgaga 540
t 541

<210> 562
<211> 419
<212> DNA
<213> human
<400> 562
gagtcggaga tgatgcagca cacacacaat tccccagccc agtgatgctt gtgttgacca 60
gatgttcctg agtctggagc aagcacccag gccagaataa cagagctttc ttagttggtg 120
aagacttaaa catctgcctg aggtcaggag gcaatttgcc tgccttgtac aaaagctcag 180
gtgaaagact gagatgaatg tctttcctct ccctgcctcc caccagactt cctcctggaa 240

aacgctttgg tagatttggc caggagcttt cttttatgta aattggataa atacacacac 300
catacactat ccacagatat agccaagtag atttgggtag aggatactat ttccagaata 360
gtgtttagct cacctagggg gatatgtttg tatacacatt tgcatatacc cacatgggg 419

<210> 563
<211> 303
<212> DNA
<213> human
<400> 563
ggaatgtcac tggtgcccag gtgtacttga gctgtgaggt catcggaatc ccgacacctg 60
tcctcatctg gaacaaggta aaaaggggtc actatggagt tcaaaggaca gaactcctgc 120
ctggtgaccg ggacaacctg gccattcaga cccggggtgg cccagaaaag catgaagtaa 180
ctggctgggt gctggtatct cctctaagta aggaagatgc tggagaatat gagtgccatg 240
catccaattc ccaaggacag gcttcagcat cagcaaaaat tacagtggtt gatgccttac 300
atg 303

<210> 564
<211> 192
<212> DNA
<213> human
<400> 564
aggtgaaggt gccgagctat aaacctccag aatattatta gtctgcatgg ttaaaagtag 60
tcatggataa ctacattacc tgttcttgcc taataagttt cttttaatcc aatccactaa 120
cactttagtt atattcactg gttttacaca gagaaataca aaataaagat cacacatcaa 180
gactatctac aa 192

<210> 565
<211> 449
<212> DNA
<213> human
<400> 565
ctcaaagact ctcccgtgga tgacggatga ggactctggg ctgctggaat aggacactca 60
agacttttga ctgccatttt gtttgttcag tggagactcc ctggccaaca gaatccttct 120
tgatagtttg caggcaaaac aaatgtaatg ttgcagatcc gcaggcagaa gctctgccct 180
tctgtatcct atgtatgcag tgtgcttttt cttgccagct tgggccattc ttgcttagac 240
agtcagcatt tgtctcctcc tttaactgag tcatcatctt agtccaacta atgcagtcga 300
tacaatgcgt agatagaaga agccccacgg gagccaggat gggactggtc gtgtttgtgc 360
ttttctccaa gtcagcaccc aaaggtcaat gcacagagac cccgggtggg tgagcgctgg 420
cttctcaaac ggccgaagtt gcctctttt 449

<210> 566
<211> 479
<212> DNA
<213> human
<400> 566
tctctctttc tgtgtgtgtg tgtgtgtgtg tgtgtgtgtg tgtgtgtgtc ttgtgcttcc 60
tcagaccttt ctcgcttctg agcttggtgg cctgttccct ccatctctcc gaacctggct 120
tcgcctgtcc ctttcactcc acaccctctg gccttctgcc ttgagctggg actgctttct 180
gtctgtccgg cctgcaccca gcccctgccc acaaaacccc agggacagcg gtctccccag 240
cctgccctgc tcaggccttg cccccaaacc tgtactgtcc cggaggaggt tgggaggtgg 300
aggcccagca tcccgcgcag atgacaccat caaccgccag agtcccagac accggttttc 360
ctagaagccc ctcaccccca ctggcccact ggtggctagg tctcccctta tccttctggt 420
ccagcgcaag gaggggctgc ttctgaggtc ggtggctgtc tttccattaa agaaacacc 479

<210> 567
<211> 537
<212> DNA
<213> human
<400> 567
ccaccgctgg ctgggaggag tcggagactg agacctacac agaggtggtg acagagtttg 60
ggaccgaggt ggagcccgag tttgggacca aggtggagcc cgagtttgag acccagttgg 120
agcctgagtt cgagacccag ctggaacccg agtttgagga agaggaggag gaggagaaag 180
aggaggagat agccactggc caggcattcc ccttcacaac agtagagacc tacacagtga 240

actttgggga cttctgagat cagcgtccta ccaagacccc agcccaactc aagctacagc 300
agcagcactt cccaagcctg ctgaccacag tcacatcacc catcagcaca tggaaggccc 360
ctggtatgga cactgaaagg aagggctggt cctgcccctt tgagggggtg caaacatgac 420
tgggacctaa gagccagagg ctgtgtagag gctcctgctc cacctgccag tctcgtaaga 480
gatggggttg ctgcagtgtt ggagtagggg cagagggagg gagccaaggt cactcca 537

<210> 568
<211> 180
<212> DNA
<213> human
<400> 568
ctactacatg attggtgagc agaagttctc cagcctcccc ctgggccggg aggcagtaga 60
ggctgctgtg aaagaggctg gctacacaat cgaatggttt gaggtgatct cgcaaagtta 120
ttcttccacc atggccaaca acgaaggact tttctccctg gtggcgagga agctgagcag 180

<210> 569
<211> 368
<212> DNA
<213> human
<400> 569
tcgtcactga ctactcagac cagaacctgc aggagctgga gaagtggctg aagaaagagc 60
cagaggcctt tgactggtcc ccagtggtga cctatgtgtg tgatcttgaa gggaacagag 120
tcaagggtcc agagaaggag gagaagttga gacaggcggt caagcaggtg ctgaagtgtg 180
atgtgactca gagccagcca ctgggggccg tccccttacc cccggctgac tgcgtgctca 240
gcacactgtg tctggatgcc gcctgcccag acctccccac ctactgcagg gcgctcagga 300
acctcggcag cctactgaag ccagggggct tcctggtgat catggatgcg ctcaagagca 360
gctactac 368

<210> 570
<211> 473
<212> DNA
<213> human
<400> 570
tggcctacat ggaccagcag actggcaacc tcaagaaggc cctgctcctc cagggctcca 60
acgagatcga gatccgcgcc gagggcaaca gccgcttcac ctacagcgtc actgtcgatg 120
gctgcacgag tcacaccgga gcctggggca agacagtgat tgaatacaaa accaccaaga 180
cctcccgcct gcccatcatc gatgtggccc ccttggacgt tggtgcccca gaccaggaat 240
tcggcttcga cgttggccct gtctgcttcc tgtaaactcc ctccatccca acctggctcc 300
ctcccaccca accaactttc cccccaaccc ggaaacagac aagcaaccca aactgaaccc 360
cctcaaaagc caaaaaatgg gagacaattt cacatggact ttggaaaata tttttttcct 420
ttgcattcat ctctcaaact tagtttttat ctttgaccaa ccgaacatga cca 473

<210> 571
<211> 472
<212> DNA
<213> human

<220>
<221> misc_feature
<222> (149)..(149)
<223> n is a, c, g, or t
<220>
<221> misc_feature
<222> (153)..(153)
<223> n is a, c, g, or t
<220>
<221> misc_feature
<222> (428)..(428)
<223> n is a, c, g, or t

<400> 571
gctccccatt tttataccaa aggtgctaca tctatgtgat gggtggggtg gggagggaat 60
cactggtgct atagaaattg agatgccccc ccaggccagc aaatgttcct ttttgttcaa 120

agtctatttt tattccttga tattttttnt ttnttttttt ttttttttgt ggatggggac 180
ttgtgaattt ttctaaaggt gctatttaac atgggaggag agcgtgtgcg gctccagccc 240
agcccgctgc tcactttcca ccctctctcc acctgcctct ggcttctcag gcctctgctc 300
tccgacctct ctcctctgaa accctcctcc acagctgcag cccatcctcc cggctccctc 360
ctagtctgtc ctgcgtcctc tgtccccggg tttcagagac aacttcccaa agcacaaagc 420
agtttttncc cctaggggtg ggaggaagca aaagactctg tacctatttt gt 472

<210> 572
<211> 356
<212> DNA
<213> human
<400> 572
actttcccat gagtgtgatc cacattgtta ggtgctgacc tagacagaga tgaactgagg 60
tccttgtttt gttttgttca taatacaaag gtgctaatta atagtatttc agatacttga 120
agaatgttga tggtgctaga agaatttgag aagaaatact cctgtattga gttgtatcgt 180
gtggtgtatt ttttaaaaaa tttgatttag cattcatatt ttccatctta ttcccaatta 240
aaagtatgca gattatttgc ccaaagttgt cctcttcttc agattcagca tttgttcttt 300
gccagtctca ttttcatctt cttccatggt tccacagaag ctttgtttct tgggca 356

<210> 573
<211> 503
<212> DNA
<213> human
<400> 573
gaacaacgcc tgtggcattg ccaacctggc cagcttcccc aagatgtgac tccagccagc 60
caaatccatc ctgctcttcc atttcttcca cgatggtgca gtgtaacgat gcactttgga 120
agggagttgg tgtgctattt ttgaagcaga tgtggtgata ctgagattgt ctgttcagtt 180
tccccatttg tttgtgcttc aaatgatcct tcctactttg cttctctcca cccatgacct 240
ttttcactgt ggccatcagg actttccctg acagctgtgt actcttaggc taagagatgt 300
gactacagcc tgcccctgac tgtgttgtcc cagggctgat gctgtacagg tacaggctgg 360
agattttcac ataggttaga ttctcattca cgggactagt tagctttaag caccctagag 420
gactagggta atctgacttc tcacttccta agttcccttc tatatcctca aggtagaaat 480
gtctatgttt tctactccaa ttc 503

<210> 574
<211> 364
<212> DNA
<213> human
<400> 574
gtacccagtt aaattttcat ttcagataaa caacaaataa ttttttagta taagtacatt 60
attgtttatc tgaaagtttt aattgaacta acaatcctag tttgatactc ccagtcttgt 120
cattgccagc tgtgttggta gtgctgtgtt gaattacgga ataatgagtt agaactatta 180
aaacagccaa aactccacag tcaatattag taatttcttg ctggttgaaa cttgtttatt 240
atgtacaaat agattcttat aatattattt aaatgactgc atttttaaat acaaggcttt 300
atatttttaa ctttaagatg tttttatgtg ctctccaaat tttttttact gtttctgatt 360
gtat 364

<210> 575
<211> 557
<212> DNA
<213> human
<400> 575
atccgaattc tccatatatt cactaatcaa agacactatt ttcatactag attcctgaga 60
caaatactca ctgaagggct tgtttaaaaa taaattgtgt tttggtctgt tcttgtagat 120
aatgcccttc tattttaggt agaagctctg gaatcccttt attgtgctgt tgctcttatc 180
tgcaaggtgg caagcagttc ttttcagcag attttgccca ctattcctct gagctgaagt 240
tctttgcata gatttggctt aagcttgaat tagatccctg caaaggcttg ctctgtgatg 300
tcagatgtaa ttgtaaatgt cagtaatcac ttcatgaatg ctaaatgaga atgtaagtat 360
ttttaaatgt gtgtatttca aatttgtttg actaattctg gaattacaag atttctatgc 420
aggatttacc ttcatcctgt gcatgtttcc caaactgtga ggagggaagg ctcagagatc 480
gagcttctcc tctgagttct aacaaaatgg tgctttgagg gtcagccttt aggaaggtgc 540
agctttgttg tcctttg 557

<210> 576
<211> 168
<212> DNA
<213> human
<400> 576
ggcatcatgt tgaccgagct ggagaaagcc ttgaactcta tcatcgacgt ctaccacaag 60
tactccctga taaaggggaa tttccatgcc gtctacaggg atgacctgaa gaaattgcta 120
gagaccgagt gtcctcagta tatcaggaaa aagggtgcag acgtctgg 168

<210> 577
<211> 557
<212> DNA
<213> human
<400> 577
gccagtcttg accgggatga ggcccacaga caggttgtca tcagcttgtc ccattcaagc 60
caccgagctc accacagaca cagtggagcc gcgctcttct ccagtgacac gtggacaaat 120
gcgggctcat cagccccccc agagagggtc aggccgaacc ccatttctcc tcctcttagg 180
tcattttcag caaacttgaa tatctagacc tctcttccaa tgaaaccctc cagtctatta 240
tagtcacata gataatggtg ccacgtgttt tctgatttgg tgagctcaga cttggtgctt 300
ccctctccac aacccccacc ccttgttttt caagatacta ttattatatt ttcacagact 360
tttgaagcac aaatttattg gcatttaata ttggacatct gggcccttgg aagtacaaat 420
ctaaggaaaa accaacccac tgtgtaagtg actcatcttc ctgttgttcc aattctgtgg 480
gtttttgatt caacggtgct ataaccaggg tcctgggtga cagggcgctc actgagcacc 540
atgtgtcatc acagaca 557

<210> 578
<211> 546
<212> DNA
<213> human
<400> 578
caggaaatag tcactcatcc cactccacat aaggggttta gtaagagaag tctgtctgtc 60
tgatgatgga tagggggcaa atctttttcc cctttctgtt aatagtcatc acatttctat 120
gccaaacagg aacgatccat aactttagtc ttaatgtaca cattgcattt tgataaaatt 180
aattttgttg tttcctttga ggttgatcgt tgtgttgttt tgctgcactt tttacttttt 240
tgcgtgtgga gctgtattcc cgagacaacg aagcgttggg atacttcatt aaatgtagcg 300
actgtcaaca gcgtgcaggt tttctgtttc tgtgttgtgg ggtcaaccgt acaatggtgt 360
gggaatgacg atgatgtgaa tatttagaat gtaccatatt ttttgtaaat tatttatgtt 420
tttctaaaca aatttatcgt ataggttgat gaaacgtcat gtgttttgcc aaagactgta 480
aatatttatt tatgtgttca catggtcaaa atttcaccac tgaaaccctg cacttagcta 540
gaacct 546

<210> 579
<211> 361
<212> DNA
<213> human

<220>
<221> misc_feature
<222> (75)..(75)
<223> n is a, c, g, or t

<400> 579
cccacctgga gctgaatcac atgacctagc tgcaccccag cgcctgggcc cgccccacgc 60
tctgtccaca cccangcgcc ccgggagcgg ggccatgcct ccagcccccc agctcgcccg 120
acccatcctg ttcgtgaata ggtctcaggg gttgggggag ggactgccag atttggacac 180
tatatttttt tctaaattca acttgaagat gtgtatttcc cctgaccttc aaaaaatgtt 240
ccaaggtaag cctcgtaaag gtcatcccac catcaccaaa gcctccgttt ttaacaacct 300
ccaacacgat ccatttagag gccaaatgtc attctgcagg tgccttcccg atggattaaa 360
g 361

<210> 580
<211> 549
<212> DNA

<213>  human
<400>  580
ctggaggaca gcacctgtga cttcggcaac ctcaagcgct atgcatgcac ctctcatacc    60
cagggcctga gcccaggctg ctatgacacc tacaatgcgg acatcgactg ccagtggatc   120
gacataaccg acgtgcagcc tgggaactac atcctcaagg tgcacgtgaa cccaaagtat   180
attgttttgg agtctgactt caccaacaac gtggtgagat gcaacattca ctacacaggt   240
cgctacgttt ctgcaacaaa ctgcaaaatt gtccaatcct gatctccggg agggacagat   300
ggccaatctc tccccttcca aagcaggccc tgctccccgg gcagcctccc gccgaggggc   360
ccagccccca acccacaggc agggaggggc atccctccct gccggcctca gggagcgaac   420
gtggatgaaa accacaggga ttccggatgc cagaccccat tttatacttc acttttctct   480
acagtgttgt tttgttgttg ttggttttta tttttatac tttggccata ccacagagct   540
agattgccc                                                          549

<210>  581
<211>  539
<212>  DNA
<213>  human
<400>  581
tgccagcgac tgtctcagac tgggcaggga ggctttggca tgacttaaga ggaagggcag    60
tcttgggacc cgctatgcag gtcctggcaa acctggctgc cctgtctcat ccctgtccct   120
cagggtagca ccatggcagg actgggggaa ctggagtgtc cttgctgtat ccctgttgtg   180
aggttccttc caggggctgg cactgaagca agggtgctgg ggccccatgg ccttcagccc   240
tggctgagca actgggctgt agggcagggc cacttcctga ggtcaggtct tggtaggtgc   300
ctgcatctgt ctgccttctg gctgacaatc ctggaaatct gttctccaga atccaggcca   360
aaaagttcac agtcaaatgg ggaggggtat tcttcatgca ggagacccca ggccctggag   420
gctgcaacat acctcaatcc tgtcccaggc cggatcctcc tgaagccctt ttcgcagcac   480
tgctatcctc caaagccatt gtaaatgtgt gtacagtgtg tataaacctt cttcttctt    539

<210>  582
<211>  512
<212>  DNA
<213>  human
<400>  582
gagtacaggt gccagacaaa cctctccacc ctcagtgacc cggtgcagct agaagtccat    60
atcggctggc tgttgctcca ggcccctcgg tgggtgttca aggaggaaga ccctattcac   120
ctgaggtgtc acagctggaa gaacactgct ctgcataagg tcacatattt acagaatggc   180
aaagacagga agtattttca tcataattct gacttccaca ttccaaaagc cacactcaaa   240
gatagcggct cctacttctg cagggggctt gttgggagta aaaatgtgtc ttcagagact   300
gtgaacatca ccatcactca aggtttggca gtgtcaacca tctcatcatt ctctccacct   360
gggtaccaag tctctttctg cttggtgatg gtactccttt ttgcagtgga cacaggacta   420
tatttctctg tgaagacaaa catttgaagc tcaacaagag actggaagga ccataaactt   480
aaatggagaa aggaccctca agacaaatga cc                                512

<210>  583
<211>  291
<212>  DNA
<213>  human
<400>  583
aaccagccag tcccaagaag aacattaaaa ctaggagtgc ccagaagaga acaaacccga    60
aaagagtgtg agctaactag tttccaaagc ggagacttcc gacttcctta caggatgagg   120
ctgggcattg cctgggacag cctatgtaag gccatgtgcc ccttgcccta acaactcact   180
gcagtgctct tcatagacac atcttgcagc atttttctta aggctatgct tcagtttttc   240
tttgtaagcc atcacaagcc atagtggtag gtttgccctt tggtacagaa g           291

<210>  584
<211>  507
<212>  DNA
<213>  human
<400>  584
gaaaatgtac cttggtgcca ccaacccatt ttgtgccaca tgcaagtttt gaataaggat    60
gtatggaaaa caacgctgca tatacaggta ccatttagga aataccgatg cctttgtggg   120
ggcagaatca cagacaaaag ctttgaaaat cataaagata taagttggtg tggctaagat   180
ggaaacaggg ctgattcttg attcccaatt ctcaactctc cttttcctat ttgaatttct   240

ttggtgctgt agaaaacaaa aaaagaaaaa tatatattca taaaaaatat ggtgctcatt 300
ctcatccatc caggatgtac taaaacagtg tgtttaataa attgtaatta ttttgtgtac 360
agttctatac tgttatctgt gtccatttcc aaaacttgca cgtgtccctg aattccgctg 420
actctaattt atgaggatgc cgaactctga tggcaataat atatgtatta tgaaaatgaa 480
gttatgattt ccgatgaccc taagtcc 507

<210> 585
<211> 386
<212> DNA
<213> human
<400> 585
gaagaactgt ctattttctc agtcattttt aacctctaga gtcactgata cacagaatat 60
aatcttattt atacctcagt ttgcatattt ttttactatt tagaatgtag ccctttttgt 120
actgatataa tttagttcca caaatggtgg gtacaaaaag tcaagtttgt ggcttatgga 180
ttcatatagg ccagagttgc aaagatcttt tccagagtat gcaactctga cgttgatccc 240
agagagcagc ttcagtgaca aacatatcct ttcaagacag aaagagacag gagacatgag 300
tctttgccgg aggaaaagca gctcaagaac acatgtgcag tcactggtgt caccctggat 360
aggcaaggga taactcttct aacaca 386

<210> 586
<211> 541
<212> DNA
<213> human
<400> 586
tgccgtgctc ccaaaacatt ttaaatgaaa gtattggcat tcaaaaagac agcagacaaa 60
atgaaagaaa atgagagcag aaagtaagca tttccagcct atctaatttc tttagttttc 120
tatttgcctc cagtgcagtc catttcctaa tgtataccag cctactgtac tatttaaaat 180
gctcaatttc agcaccgatg gccatgtaaa taagatgatt taatgttgat tttaatcctg 240
tatataaaat aaaaagtcac aatgagtttg ggcatattta atgatgatta tggagcctta 300
gaggtcttta atcattggtt cggctgcttt tatgtagttt aggctggaaa tggtttcact 360
tgctctttga ctgtcagcaa gactgaagat ggcttttcct ggacagctag aaaacacaaa 420
atcttgtagg tcattgcacc tatctcagcc ataggtgcag tttgcttcta catgatgcta 480
aaggctgcga atgggatcct gatggaacta aggactccaa tgtcgaactc ttctttgctg 540
c 541

<210> 587
<211> 524
<212> DNA
<213> human
<400> 587
ggggaactga gggcaagggg atatagtgat ggggctcaga tggactggga ggagggggag 60
ggtgatgcat taattaatgg cttcgttaat taatgtcatg ttgcttgtcg ctttctcagt 120
gtgtgtgtgt ggtccatgcc cactgctggt gccagggtgg gtgtccatgt gcacccggcc 180
tggatgccag ctgtgtcctt cgggggcgtg cgtgtaactg tagtgtagtc aggtgctcaa 240
tggagaatat aaacatatac agaaaaatat atattttaag tttaaaaaac agaaaaacag 300
acaaaacaat ccccatcagg tagctgtcta acccccagct gggtctaatc cttctcatta 360
cccacccgac ctggctgccc ctcaccttgg gctgggggac tgggggccca tttccttttc 420
tctgcccttt ttttgttgtt ctattttgta cagacaagtt ggaaaaacaa cagcgacaaa 480
aaagtcaaga aactttgtaa aatatcgtgt gtgtgattcc ttgt 524

<210> 588
<211> 502
<212> DNA
<213> human
<400> 588
cccgaccggt gggcatttgt gaggcccatg gttgagaaat gaataatttc ccaattagga 60
agtgtaagca gctgaggtct cttgagggag cttagccaat gtgggagcag cggtttgggg 120
agcagagaca ctaacgactt cagggcaggg ctctgatatt ccatgaatgt atcaggaaat 180
atatatgtgt gtgtatgttt gcacacttgt tgtgtgggct gtgagtgtaa gtgtgagtaa 240
gagctggtgt ctgattgtta agtctaaata tttccttaaa ctgtgtggac tgtgatgcca 300
cacagagtgg tctttctgga gaggttatag gtcactcctg gggcctcttg ggtcccccac 360
gtgacagtgc ctgggaatgt acttattctg cagcatgacc tgtgaccagc actgtctcag 420
tttcactttc acatagatgt ccctttcttg gccagttatc ccttcctttt agcctagttc 480

atccaatcct cactgggtgg gg 502

<210> 589
<211> 555
<212> DNA
<213> human
<400> 589
caccacctac ctatgatgcc gtggtacaga tggagtacct tgacatggtg gtgaatgaaa 60
cactcagatt attcccagtt gctattagac ttgagaggac ttgcaagaaa gatgttgaaa 120
tcaatggggt attcattccc aaagggtcaa tggtggtgat tccaacttat gctcttcacc 180
atgacccaaa gtactggaca gagcctgagg agttccgccc tgaaaggttc agtaagaaga 240
aggacagcat agatccttac atatacacac cctttggaac tggacccaga aactgcattg 300
gcatgaggtt tgctctcatg aacatgaaac ttgctctaat cagagtcctt cagaacttct 360
ccttcaaacc ttgtaaagaa acacagatcc ccttgaaatt agacacgcaa ggacttcttc 420
aaccagaaaa acccattgtt ctaaaggtgg attcaagaga tggaacccta agtggagaat 480
gagttattct aaggacttct actttggtct tcaagaaagc tgtgccccag aacaccagag 540
atttcaactt agtca 555

<210> 590
<211> 540
<212> DNA
<213> human
<400> 590
gaaaatcgat gcagccattt ctgataagga aaagaacaaa acatatttct ttgtagagga 60
caaatactgg agatttgatg agaagagaaa ttccatggag ccaggctttc ccaagcaaat 120
agctgaagac tttccaggga ttgactcaaa gattgatgct gtttttgaag aatttgggtt 180
cttttatttc tttactggat cttcacagtt ggagtttgac ccaaatgcaa agaaagtgac 240
acacactttg aagagtaaca gctggcttaa ttgttgaaag agatatgtag aaggcacaat 300
atgggcactt taaatgaagc taataattct tcacctaagt ctctgtgaat tgaaatgttc 360
gttttctcct gcctgtgctg tgactcgagt cacactcaag ggaacttgag cgtgaatctg 420
tatcttgccg gtcattttta tgttattaca gggcattcaa atggctgctg cttagcttgg 480
caccttgtca catagagtga tctttcccaa gagaagggga agcactcgtg tgcaacagac 540

<210> 591
<211> 511
<212> DNA
<213> human
<400> 591
tccacacacg gccaggcctg tttatctaca ctgctgccca ctcctctctc cagctccaca 60
tgctgtacct ggatcattct gaagcaaatt ccgagcatta catcattttg tccataaata 120
tttctaacat ccttaaatat acaatcggaa ttcaagcatc tcccattgtc ccacaaatgt 180
ttggctgttt ttgtagttgg attgtttgta ttaggattca agcaaggccc atatattgca 240
tttatttgaa atgtctgtaa gtctctttcc atctacagag tttagcacat ttgaacgttg 300
ctggttgaaa tcccgaggtg tcatttgaca tggttctctg aacttatctt tcctataaaa 360
tggtagttag atctggaggt ctgattttgt ggcaaaaata cttcctaggt ggtgctgggt 420
acttcttgtt gcatcctgtc aggaggcaga taatgctggt gcctctctat tggtaatgtt 480
aagactgctg ggtgggtttg gagttcttgg c 511

<210> 592
<211> 428
<212> DNA
<213> human
<400> 592
gaacatccaa gtctttcttc ttttttaagt tgtcaaagaa gcttccacaa aattagaaag 60
gacaacagtt ctgagctgta atttcgcctt aaactctgga cactctatat gtagtgcatt 120
tttaaacttg aaatatataa tattcagcca gcttaaaccc atacaatgta tgtacaatac 180
aatgtacaat tatgtctctt gagcatcaat cttgttactg ctgattcttg taaatctttt 240
tgcttctact ttcatcttaa actaatacgt gccagatata actgtcttgt ttcagtgaga 300
gacgccctat ttctatgtca tttttaatgt atctatttgt acaattttaa agttcttatt 360
ttagtataca tataaatatc agtattctga catgtaagaa aatgttacgg catcacactt 420
atatttta 428

<210> 593

<211>  541
<212>  DNA
<213>  human
<400>  593
aatctcttct agagcacctg ctatccccaa cttctagacc tgctccaaac tagtgactag      60
gatagaattt gatcccctaa ctcactgtct gcggtgctca ttgctgctaa cagcattgcc     120
tgtgctctcc tctcaggggc agcatgctaa cggggcgacg tcctaatcca actgggagaa     180
gcctcagtgg tggaattcca ggcactgtga ctgtcaagct ggcaagggcc aggattgggg     240
gaatggagct ggggcttagc tgggaggtgg tctgaagcag acagggaatg ggagaggagg     300
atgggaagta gacagtggct ggtatggctc tgaggctccc tggggcctgc tcaagctcct     360
cctgctcctt gctgttttct gatgatttgg gggcttggga gtccctttgt cctcatctga     420
gactgaaatg tggggatcca ggatggcttc cttcctctta cccttcctcc ctcagcctgc     480
aacctctatc ctggaacctg tcctcccttt ctccccaact atgcatctgt tgtctgctcc     540
t                                                                     541

<210>  594
<211>  502
<212>  DNA
<213>  human
<400>  594
aagaagctaa gcctgatctc ctgtaaaaac atcatttccc tgatggacac cagcggcaat      60
gggaagctgg agtttgatga attcaaagtg ttctgggaca agctgaagca gtggattaac     120
cttttccttc ggtttgatgc tgacaagtcc ggcaccatgt ctacctatga actacggact     180
gcactgaaag ctgcaggctt tcagctgagc agccacctcc tgcagctgat tgtgctcagg     240
tatgcggatg aggagctcca gctggacttc gatgacttcc tcaactgcct ggtccggctg     300
gagaatgcga gccgggtgtt ccaggctctc agtacaaaga acaaggagtt cattcatctc     360
aatataaatg agttcatcca tttgacaatg aacatctgag gctgccttgt agagatgcag     420
cctgcccagc tgaatcttgg cttctggacc ttgaccttca gaacttctct tggtgtggaa     480
ccattacgcc cagggttcac tc                                              502

<210>  595
<211>  542
<212>  DNA
<213>  human
<400>  595
actcccaaga gcaaatccac attcctcttg agttctgcag cttctgtgta aatagggcag      60
ctgtcgtcta tgccgtagaa tcacatgatc tgaggaccat tcatggaagc tgctaaatag     120
cctagtctgg ggagtcttcc ataaagtttt gcatggagca aacaaacagg attaaactag     180
gtttggttcc ttcagccctc taaaagcata gggcttagcc tgcaggcttc cttgggcttt     240
ctctgtgtgt gtagttttgt aaacactata gcatctgtta agatccagtg tccatggaaa     300
cattcccaca tgccgtgact ctggactata tcagtttttg gaaagcaggg ttcctctgcc     360
tgctaacaag cccacgtgga ccagtctgaa tgtctttcct ttacacctat gtttttaagt     420
agtcaaactt caagaaacaa tctaaacaag tttctgttgc atatgtgttt gtgaacttgt     480
atttgtattt agtaggcttc tatattgcat ttaacttgtt tttgtaactc ctgattcttc     540
ct                                                                    542

<210>  596
<211>  473
<212>  DNA
<213>  human
<400>  596
gaagcctcaa gttccagtgc agagatccta cttctctgag tcagctgacc ccctccccgc      60
aatccctcaa accttgagga gaagtgggga ccccacccct catcaggagt tccagtgctg     120
catgcgatta tctacccacg tccacgcggc cacctcaccc tctccgcaca cctctggctg     180
tctttttgta ctttttgttc cagagctgct tctgtctggt ttatttaggt tttatccttc     240
cttttctttg agagttcgtg aagagggaag ccaggattgg ggacctgatg gagagtgaga     300
gcatgtgagg ggtagtggga tggtggggta ccagccactg gaggggtcat ccttgcccat     360
cgggaccaga aacctgggag agacttggat gaggagtggt tgggctgtgc ctgggcctag     420
cacggacatg gtctgtcctg acagcactcc tcggcaggca tggctggtgc ctg            473

<210>  597
<211>  477
<212>  DNA

<213>  human
<400>  597
aaagcagcgc tagtggaggg ttggagaagg aggtaaggat gagggttcat catccctccc      60
tgcctaagga agctaaaagc atggccctgc tgcccctccc tgcctccacc cacagtggag     120
agggctacaa aggaggacaa gaccctctca ggctgtccca agctcccaag agcttccaga     180
gctctgaccc acagcctcca agtcaggtgg ggtggagtcc cagagctgca cagggtttgg     240
cccaagtttc taagggaggc acttcctccc ctcgcccatc agtgccagcc cctgctggct     300
ggtgcctgag cccctcagac agccccctgc cccgcaggcc tgccttctca gggacttctg     360
cggggcctga ggcaagccat ggagtgagac ccaggagccg gacacttctc aggaaatggc     420
ttttcccaac ccccagcccc cacccggtgg ttcttcctgt tctgtgactg tgtatag        477

<210>  598
<211>  432
<212>  DNA
<213>  human
<400>  598
ttcagccagt cgccaagaat catgaaagtc gccagtggca gcaccgccac cgccgccgcg      60
ggccccacgt gcgcgctgaa ggccggcaag acagcgagcg gtgcgggcga ggtggtgcgc     120
tgtctgtctg agcagagcgt ggccatctcg cgctgccggg gcgccggggc gcgcctgcct     180
gccctgctgg acgagcagca ggtaaacgtg ctgctctacg acatgaacgg ctgttactca     240
cgcctcaagg agctggtgcc caccctgccc cagaaccgca aggtgagcaa ggtggagatt     300
ctccagcacg tcatcgacta catcagggac cttcagttgg agctgaactc ggaatccgaa     360
gttggaaccc ccgggggccg agggctgccg gtccgggctc cgctcagcac cctcaacggc     420
gagatcagcg cc                                                         432

<210>  599
<211>  382
<212>  DNA
<213>  human
<400>  599
gattccctgc atcaactaag aaaagcctgt tttctttatt tcaaacttgg tggcgaatgt      60
gttgcgggtc ctgttgggct gctttctgta ttgtctccta accctctagt tttaattgga     120
cacttctttg ctgttgcaat ctatgccgtg tatttttgct ttaagtcaga accttggatt     180
acaaaacctc gagcccttct cagtagtggt gctgtattgt acaaagcgtg ttctgtaata     240
tttcctctaa tttactcaga aatgaagtat atggttcatt aagcttaaag gggaaccatt     300
tgtgaatgaa tatttggaac ttaccaagtc ctaagagact tttggaagag gatatatata     360
gcatagtacc ataccactta ta                                              382

<210>  600
<211>  269
<212>  DNA
<213>  human
<400>  600
gctgtgggga tagtgaggca tcgcaatgta agactcggga ttagtacaca cttgttgatg      60
attaatggaa atgtttacag atccccaagc ctggcaaggg aatttcttca actccctgcc     120
ccctagccct ccttatcaaa ggacaccatt ttggcaagct ctatcaccaa ggagccaaac     180
atcctacaag acacagtgac catactaatt ataccccctg caaagccagc ttgaaacctt     240
cacttaggaa cgtaatcgtg tcccctatc                                       269

<210>  601
<211>  473
<212>  DNA
<213>  human
<400>  601
tcaccaatgc catcaaggat gcactcgctg caacgtagcc ctctgttctg cacacagcac      60
gggggccaag gatgccccgt ccccctctgg ctccagctgg ccgggagcct gatcacctgc     120
cctgctgagt cccaggctga gcctcagtct ccctcccttg gggcctatgc agaggtccac      180
aacacacaga tttgagctca gccctggtgg gcagagaggt agggatgggg ctgtggggat     240
agtgaggcat cgcaatgtaa gactcgggat tagtacacac ttgttgatga ttaatggaaa     300
tgtttacaga tccccaagcc tggcaaggga atttcttcaa ctccctgccc cctagccctc     360
cttatcaaag gacaccattt tggcaagctc tatcaccaag gagccaaaca tcctacaaga     420
cacagtgacc atactaatta taccccctgc aaagccagct tgaaaccttc act            473

<210> 602
<211> 329
<212> DNA
<213> human
<400> 602
tccaacctcc ttcaaattca gtcaccactg ttatattacc ttctccagga accctccagt 60
ggggaaggct gcgatattag atttccttgt atgcaaagtt tttgttgaaa gctgtgctca 120
gaggaggtga gaggagagga aggagaaaac tgcatcataa ctttacagaa ttgaatctag 180
agtcttcccc gaaaagccca gaaacttctc tgcagtatct ggcttgtcca tctggtctaa 240
ggtggctgct tcttccccag ccatgagtca gtttgtgccc atgaataata cacgacctgt 300
tatttccatg actgctttac tgtatttttt 329

<210> 603
<211> 529
<212> DNA
<213> human
<400> 603
gaatggtgca tacaaggcca tccccgttgc ccaggacctg aacgcgcctt ctgattggga 60
cagccgtggg aaggacagtt atgaaacgag tcagctggat gaccagagtg ctgaaaccca 120
cagccacaag cagtccagat tatataagcg gaaagctaat gatgagagca atgagcattc 180
cgatgtgatt gatagtcagg aactttccaa agtcagccgt gaattccaca gccatgaatt 240
tcacagccat gaagatatgc tggttgtaga ccccaaaagt aaggaagaag ataaacacct 300
gaaatttcgt atttctcatg aattagatag tgcatcttct gaggtcaatt aaaaggagaa 360
aaaatacaat ttctcacttt gcatttagtc aaaagaaaaa atgctttata gcaaaatgaa 420
agagaacatg aaatgcttct ttctcagttt attggttgaa tgtgtatcta tttgagtctg 480
gaaataactg atgtgtttga taattagttt agtttgtggc ttcatggaa 529

<210> 604
<211> 536
<212> DNA
<213> human
<400> 604
acagctttcc aaggtgacaa actcctctat gcagtgtatc gaaagctggg tgtttatgaa 60
gttgaagacc agattacagc tgtcagaaaa ttcatagaaa tgggtttcat tgatgaaaaa 120
agaatagcca tatggggctg gtcctatgga ggatacgttt catcactggc ccttgcatct 180
ggaactggtc ttttcaaatg tggtatagca gtggctccag tctccagctg ggaatattac 240
gcgtctgtct acacagagag attcatgggt ctcccaacaa aggatgataa tcttgagcac 300
tataagaatt caactgtgat ggcaagagca gaatatttca gaaatgtaga ctatcttctc 360
atccacggaa cagcagatga taatgtgcac tttcagaact cagcacagat tgctaaagct 420
ctggttaatg cacaagtgga tttccaggca atgtggtact ctgaccagaa ccacggctta 480
tccggcctgt ccacgaacca cttatacacc cacatgaccc acttcctaaa gcagtg 536

<210> 605
<211> 470
<212> DNA
<213> human
<400> 605
tcttttgtcc tccttagcac aatgtaaaaa agaatagtaa tatcagaaca ggaaggagga 60
atggcttgct ggggagccca tccaggacac tgggagcaca tagagattca cccatgtttg 120
ttgaacttag agtcattctc atgcttttct ttataattca cacatatatg cagagaagat 180
atgttcttgt taacattgta tacaacatag ccccaaatat agtaagatct atactagata 240
atcctagatg aaatgttaga gatgctatat gatacaactg tggccatgac tgaggaaagg 300
agctcacgcc cagagactgg gctgctctcc cggaggccaa acccaagaag gtctggcaaa 360
gtcaggctca gggagactct gccctgctgc agacctcggt gtggacacac gctgcataga 420
gctctccttg aaaacagagg ggtctcaaga cattctgcct acctattagc 470

<210> 606
<211> 441
<212> DNA
<213> human
<400> 606
aaaggagcag tcgcacagac ctttcctcat gctgcaggcc cggcagtctg aagaccaccc 60
tcatcgccgg cgtcggcggg gcttggagtg tgatggcaag gtcaacatct gctgtaagaa 120

acagttcttt gtcagtttca aggacatcgg ctggaatgac tggatcattg ctccctctgg 180
ctatcatgcc aactactgcg agggtgagtg cccgagccat atagcaggca cgtccgggtc 240
ctcactgtcc ttccactcaa cagtcatcaa ccactaccgc atgcggggcc atagcccctt 300
tgccaacctc aaatcgtgct gtgtgcccac caagctgaga cccatgtcca tgttgtacta 360
tgatgatggt caaaacatca tcaaaaagga cattcagaac atgatcgtgg aggagtgtgg 420
gtgctcatag agttgcccag c 441

<210> 607
<211> 561
<212> DNA
<213> human
<400> 607
acaagcatcc tgtctcacga agaacaaatg tttgttaatc gtgtgggcca tgattatcag 60
tggataggcc tcaatgacaa gatgtttgag catgacttcc gttggactga tggcagcaca 120
ctgcaatacg agaattggag acccaaccag ccagacagct tcttttctgc tggagaagac 180
tgtgttgtaa tcatttggca tgagaatggc cagtggaatg atgttccctg caattaccat 240
ctcacctata cgtgcaagaa aggaacagtt gcttgcggcc agccccctgt tgtagaaaat 300
gccaagacct ttggaaagat gaaacctcgt tatgaaatca actccctgat tagataccac 360
tgcaaagatg gtttcattca acgtcacctt ccaactatcc ggtgcttagg aaatggaaga 420
tgggctatac ctaaaattac ctgcatgaac ccatctgcat accaaaggac ttattctatg 480
aaatacttta aaaattcctc atcagcaaag gacaattcaa taaatacatc caaacatgat 540
catcgttgga gccggaggtg g 561

<210> 608
<211> 557
<212> DNA
<213> human
<400> 608
tggtgatgtc tgctactatg ccagccggaa cgacaagtcc tactggctct ctaccactgc 60
gccgctgccc atgatgcccg tggccgagga cgagatcaag ccctacatca gccgctgttc 120
tgtgtgtgag gccccggcca tcgccatcgc ggtccacagt caggatgtct ccatcccaca 180
ctgcccagct gggtggcgga gtttgtggat cggatattcc ttcctcatgc acacggcggc 240
gggagacgaa ggcggtggcc aatcactggt gtcaccgggc agctgtctag aggacttccg 300
cgccacacca ttcatcgaat gcaatggagg ccgcggcacc tgccactact acgccaacaa 360
gtacagcttc tggctgacca ccattcccga gcagagcttc cagggctcgc cctccgccga 420
cacgctcaag gccggcctca tccgcacaca catcagccgc tgccaggtgt gcatgaagaa 480
cctgtgagcc ggcgcgtgcc aggaagggcc attttggtgc ttattcttaa cttattacct 540
caggtgccaa cccaaaa 557

<210> 609
<211> 436
<212> DNA
<213> human
<400> 609
gaaagactgt gctgtccttt aacataggtt tttaaagact aggatattga atgtgaaaca 60
tccgttttca ttgttcactt ctaaaccaaa aattatgtgt tgccaaaacc aaacccaggt 120
tcatgaatat ggtgtctatt atagtgaaac atgtactttg agcttattgt ttttattctg 180
tattaaatat tttcagggtt ttaaacacta atcacaaact gaatgacttg acttcaaaag 240
caacaacctt aaaggccgtc atttcattag tattcctcat tctgcatcct ggcttgaaaa 300
acagctctgt tgaatcacag tatcagtatt ttcacacgta agcacattcg ggccatttcc 360
gtggtttctc atgagctgtg ttcacagacc tcagcagggc atcgcatgga ccgcaggagg 420
gcagattcgg accact 436

<210> 610
<211> 429
<212> DNA
<213> human
<400> 610
tcggctactc ttttgtgatg cacaccagcg ctggtgcaga aggctctggc caagccctgg 60
cgtcccccgg ctcctgcctg gaggagttta gaagtgcgcc attcatcgag tgtcacggcc 120
gtgggacctg caattactac gcaaacgctt acagcttttg gctcgccacc atagagagga 180
gcgagatgtt caagaagcct acgccgtcca ccttgaaggc aggggagctg cgcacgcacg 240
tcagccgctg ccaagtctgt atgagaagaa cataatgaag cctgactcag ctaatgtcac 300

aacatggtgc tacttcttct tctttttgtt aacagcaacg aaccctagaa atatatcctg 360
tgtacctcac tgtccaatat gaaaaccgta aagtgcctta taggaatttg cgtaactaac 420
acaccctgc 429

<210> 611
<211> 517
<212> DNA
<213> human

<220>
<221> misc_feature
<222> (181)..(181)
<223> n is a, c, g, or t

<400> 611
ggaaaacacc tcatttgacc ttgccagctg accttcaaac cctgcatttg aaccgaccaa 60
cattaagtcc agagagtaaa cttgaatgga ataacgacat tccagaagtt aatcatttga 120
attctgaaca ctggagaaaa accgaaaaat ggacggggca tgaagagact aatcatctgg 180
naaaccgatt tcagtggcga tggcatgaca gagctagagc tcgggcccag ccccaggctg 240
cagcccattc gcaggcaccc gaaagaactt ccccagtatg gtggtcctgg aaaggacatt 300
tttgaagatc aactatatct tcctgtgcat tccgatggaa tttcagttca tcagatgttc 360
accatggcca ccgcagaaca ccgaagtaat tccagcatag cggggaagat gttgaccaag 420
gtggagaaga atcacgaaaa ggagaagtca cagcacctag aaggcagcgc ctcctcttca 480
ctctcctctg attagatgaa actgttacct tacccta 517

<210> 612
<211> 397
<212> DNA
<213> human
<400> 612
aatatccttg ttgtgtatta ggtttttaaa taccagctaa aggattacct cactgagtca 60
tcagtaccct cctattcagc tccccaagat gatgtgtttt tgcttaccct aagagaggtt 120
ttcttcttat ttttagataa ttcaagtgct tagataaatt atgttttctt taagtgttta 180
tggtaaactc ttttaaagaa aatttaatat gttatagctg aatctttttg gtaactttaa 240
atctttatca tagactctgt acatatgttc aaattagctg cttgcctgat gtgtgtatca 300
tcggtgggat gacagaacaa acatatttat gatcatgaat aatgtgcttt gtaaaaagat 360
ttcaagttat taggaagcat actctgtttt ttaatca 397

<210> 613
<211> 518
<212> DNA
<213> human
<400> 613
gtgtgcacac ggagactcat cgttataatt tactatctgc caagagtaga aagaaaggct 60
ggggatattt gggttggctt ggttttgatt ttttgcttgt ttgtttgttt tgtactaaaa 120
cagtattatc ttttgaatat cgtagggaca taagtatata catgttatcc aatcaagatg 180
gctagaatgg tgcctttctg agtgtctaaa acttgacacc cctggtaaat ctttcaacac 240
acttccactg cctgcgtaat gaagttttga ttcattttta accactggaa tttttcaatg 300
ccgtcatttt cagttagatg attttgcact ttgagattaa aatgccatgt ctatttgatt 360
agtcttattt ttttattttt acaggcttat cagtctcact gttggctgtc attgtgacaa 420
agtcaaataa acccccaagg acgacacaca gtatggatca catattgttt gacattaagc 480
ttttgccaga aaatgttgca tgtgttttac ctcgactt 518

<210> 614
<211> 523
<212> DNA
<213> human
<400> 614
gaaaggcgat ctcttcactg tgaaaagttg cccgggtgca gcgccttttc cttctaccat 60
gggaaatgca ggctgggccc ttggggtgag cctgcggggc tctggtgctg tccccgaccc 120
ccaccaccac cagaatgcag ttccagctta ggaagccaca aacaagccac ccaggaggaa 180
caaaacaccg ccagcgtgga ttttccaaat ttccctggaa agtaagtctc gctcttgcca 240
aagaaaagtc tggcttggag agtctctgga gcccaggatg ccagcatgtg ccaatgactg 300

tcaccttcat ctcttcaaaa gaaaagccat agccgaggac tgtcccgcga cccccgtgga 360
ctgcgtctag gtcatgtgat tctgttttca tttctcatcc catccaattt gtccttttct 420
cctgtcattt tcttcctctg tggtcccttc aaagttgtta taatttgtac tgaacttcaa 480
aatgtgtccc gttctcccca gaccactcta gccacagtat att 523

<210> 615
<211> 542
<212> DNA
<213> human

<220>
<221> misc_feature
<222> (56)..(56)
<223> n is a, c, g, or t
<220>
<221> misc_feature
<222> (63)..(63)
<223> n is a, c, g, or t
<220>
<221> misc_feature
<222> (102)..(102)
<223> n is a, c, g, or t

<400> 615
attgaaaagc aggtaccagt gccccttttc agacagtttt tgattcgctc tagacntttt 60
ttntttttta atagggaggg aaaaaatttg ataattttct tntttctaca tgcacttaag 120
actaaaacac aggtttggat taattttatt tgcttccttt ttccgctttt cttcccgcag 180
agcctgatgg gagaatgtcc agggcaggga aaccacattt tttgtaggtg ataactcaat 240
gaaaattggt gcttattttt tacacttctc tcttgtggct ctcttgtggt gctatctgtt 300
ttaaggtctc cttgaaggcg cactggggtc cctggccatg cctcgttctc cctgctttct 360
ttatcctgtt attgcctcca cagtctgttg ccaaggactc taagatcaat gcacgtcact 420
ttcctttcca ctgggcagga tagccaagca cactccctcc tgcgctctcc cgccccggtg 480
cgtccactcc cgagggctgt tatgaggact gggttgtgcc tacttgattt gaaaacacac 540
ac 542

<210> 616
<211> 559
<212> DNA
<213> human

<220>
<221> misc_feature
<222> (36)..(36)
<223> n is a, c, g, or t
<220>
<221> misc_feature
<222> (51)..(51)
<223> n is a, c, g, or t
<220>
<221> misc_feature
<222> (56)..(56)
<223> n is a, c, g, or t

<400> 616
acttctcagc aaataaatct cccttaagta ggaaanctag atttcatatt ngcttncttt 60
gaattaacag caactttcca caggtaaatc tgttcttgca aagatgtgag cagaatagtt 120
aaaaataata tttttatgtt tcatggttct aaatggaagc cataaatgca gtaaatacta 180
tctgttgttt aactacttta atcgtcattt tttacatttt caagtttatt aggttaagaa 240
aaacaggcca gccttggaag gcagctacta cagaaaactg cagttttgcg ttaaagataa 300
agtagtattt tcagctccct gaaaaaccat tcctgctgaa actgctgtag aaattgtgaa 360
gctgcatgag tggagagtat tgaatctgtg gttatagtag ttttctcagg tttgtttatc 420
ttgatgtttg atgcactgtg ttttatagtt attaaaattg agtaatatta tttctatgca 480
gtgttatgtg tcattggcct tttgtgaatg tgcatgtttt aaactgcaaa ttttaaacat 540

tttgtcctct aattgttat 559

<210> 617
<211> 462
<212> DNA
<213> human
<400> 617
gaatgagtat tcctatacga cccagataga ctacaacccc aaggaccgcc tgctctatgc 60
ctgggacaat ggccaccagg tcacttacca tgtcatcttt gcctactgac acccttgtcc 120
ccacaagcag aagcacagag gggtcactag caccttgtgt gtatgtgtgt gcgcgcacgt 180
gtgtgtaggt gggtatgtgt tgtttaaaaa tatatattat tttgtataat attgcaaatg 240
taaaatgaca atttgggtct atttttttat atggattgta gatcaatcca tacgtgtatg 300
tgctggtctc atcctcccca gtttatattt ttgtgcaaat gaacttctcc ttttgaccag 360
taaccacctt ccttcaagcc ttcagcccct ccagctccaa gtctcagatc tcgaccattg 420
aaaaggtttc ttcatctggg tcttgcagga ggcaggcaac ac 462

<210> 618
<211> 503
<212> DNA
<213> human

<220>
<221> misc_feature
<222> (419)..(419)
<223> n is a, c, g, or t

<400> 618
gtgctcggcc tgatggagac tgtgtgcagc cccttcacgg ccgccagacg tctgcgggac 60
caggaggcag ccgtggcgga gctgcaggcc gccctggagc gacaggctct ccagaagcaa 120
gccctgcagg agaaaggcaa gcagcaggac acggtcctcg gcggccgggc cctgtccaac 180
cggcagcacg cctcctagga actgtgggag accagcggag tgggagggag acgcagtaga 240
cagagacaga ccgagaagga agggagagac agagggggcg cgcgcacagg agcctgactc 300
cgctgggaga gtgcaggagc acgtgctgtt ttttatttgg acttaacttc agagaaaccg 360
ctgacatcta gaactgacct accacaagca tccaccaaag gagtttggga ttgagtttnt 420
gctgctgtgc agcactgcat tgtcatgaca tttccaacac tgtgtgaatt atctaaatgc 480
gtctaccatt ttgcactagg gag 503

<210> 619
<211> 568
<212> DNA
<213> human

<220>
<221> misc_feature
<222> (82)..(82)
<223> n is a, c, g, or t
<220>
<221> misc_feature
<222> (251)..(251)
<223> n is a, c, g, or t
<220>
<221> misc_feature
<222> (393)..(393)
<223> n is a, c, g, or t
<220>
<221> misc_feature
<222> (407)..(407)
<223> n is a, c, g, or t
<220>
<221> misc_feature
<222> (412)..(412)
<223> n is a, c, g, or t

<400>  619
cacagcctcc aagtcaggtg gggtggagtc ccagagctgc acagggtttg gcccaagttt      60
ctaagggagg cacttcctcc cntcgcccat cagtgccagc ccctgctggc tggtgcctga      120
gcccctcaga cagccccctg ccccgcaggc ctgccttctc agggacttct gcggggcctg      180
aggcaagcca tggagtgaga cccaggagcc ggacacttct caggaaatgg cttttcccaa      240
cccccagccc nccacccggt ggttcttcct gttctgtgac tgtgtatagt gccaccacag      300
cttatggcat ctcattgagg acaaagaaaa ctgcacaata aaaccaagcc tctggaatct      360
gtcctcgtgt ccacctggcc ttcgctcctc cancagtgcc tgcctgnccc cncttcgctg      420
gggtctccac gggtgaggct ggggaacgcc acctcttcct cttccctgac ttctccccaa      480
ccacttagta gcaacgctac cccaggggct aatgactgca cactgggctt cttttcagaa      540
tgaccctaac gagacacatt tgcccaaa                                          568

<210>  620
<211>  248
<212>  DNA
<213>  human

<220>
<221>  misc_feature
<222>  (45)..(45)
<223>  n is a, c, g, or t
<400>  620
cacaaaaccc cagggacagc ggtctcccca gcctgccctg ctcangcctt gcccccaaac      60
ctgtactgtc ccggaggagg ttgggaggtg gaggcccagc atcccgcgca gatgacaccg      120
gttttcctag aagcccctca cccccactgg cccactggtg gctaggtctc cccttatcct      180
tctggtccag cgcaaggagg ggctgcttct gaggtcggtg gctgtctttc cattaaagaa      240
acaccgtg                                                                248

<210>  621
<211>  261
<212>  DNA
<213>  human
<400>  621
gaatgaagaa aagtcgcctc aacgacaaac aaaagcaccg actagatttc cttcagctga      60
tgattgactc ccagaattcg aaagaaactg agtcccacaa agctctgtct gatctggagc      120
tcgcagccca gtcaataatc ttcatttttg ctggctatga aaccaccagc agtgttcttt      180
ccttcacttt atatgaactg gccactcacc ctgatgtcca gcagaaactg caaaaggaga      240
ttgatgcagt tttgcccaat a                                                 261

<210>  622
<211>  245
<212>  DNA
<213>  human
<400>  622
ggtgagggga tgacccctgg agatgaaggg aagaggtgaa gccttagcaa aaatgcctcc      60
tcaccactcc ccaggagaat ttttataaaa agcataatca ctgattcctt cactgacata      120
atgtaggaag cctctgagga gaaaaacaaa gggagaaaca tagagaacgg ttgctactgg      180
cagaagcata agatctttgt acaatattgc tggccctggt tcacctgttt actgttatca      240
caata                                                                   245

<210>  623
<211>  273
<212>  DNA
<213>  human
<400>  623
acttaggtaa ttgtagggcg aggattataa atgaaatttg caaaatcact tagcagcaac      60
tgaagacaat tatcaaccac gtggagaaaa tcaaaccgag cagggctgtg tgaaacatgg      120
ttgtaatatg cgactgcgaa cactgaactc tacgccactc cacaaatgat gttttcaggt      180
gtcatggact gttgccacca tgtattcatc cagagttctt aaagtttaaa gttgcacatg      240
attgtataag catgctttct ttgagtttta aat                                    273

<210>  624
<211>  212

<212>  DNA
<213>  human

<220>
<221>  misc_feature
<222>  (43)..(43)
<223>  n is a, c, g, or t
<220>
<221>  misc_feature
<222>  (121)..(121)
<223>  n is a, c, g, or t

<400>  624
tctcacctat acgtgcaaga aaggaacagt tgcttgcggc cancccctgt tgtagaaaat     60
gccaagacct ttggaaagat gaaacctcgt tatgaaatca actccctgat tagataccac    120
ntgcaaagat ggtttcattc aacgtcacct tccaactatc cggtgcttag gaaatggaag    180
atgggctata cctaaaatta cctgcatgaa cc                                  212

<210>  625
<211>  157
<212>  DNA
<213>  human
<400>  625
agggcctaag ggtgacagag gtgatgctgg tcccaaaggt gctgatggct ctcctggcaa     60
agatggcgtc cgtggtctga ccggccccat tggtcctcct ggccctgctg gtgcccctgg    120
tgacaagggg gaccccattc ccgaggagct ttatgag                             157

<210>  626
<211>  481
<212>  DNA
<213>  human
<400>  626
aacattgtaa tggccatcgc tggaaacaag tgcgacctct cagatattag ggaggttccc     60
ctgaaggatg ctaaggaata cgctgaatcc ataggtgcca tcgtggttga gacaagtgca    120
aaaaatgcta ttaatatcga agagctcttt caaggaatca gccgccagat cccacccttg    180
gacccccatg aaaatggaaa caatggaaca atcaaagttg agaagccaac catgcaagcc    240
agccgccggt gctgttgacc caagggcgtg gtccacggta cttgaagaag ccagagccca    300
catcctgtgc actgctgaag gaccctacgc tcggtggcct ggcacctcac tttgagaaga    360
gtgagcacac tggctttgca tcctggaagg cctgcagggg gcggggcagg aaatgtacct    420
gaaaaggatt ttagaaaacc ctgggaaacc caccacacca ccacaaaatg gcctttagtg    480
t                                                                    481

<210>  627
<211>  399
<212>  DNA
<213>  human
<400>  627
gccacacctt cgcgaaacct gtggtggccc accagtccta acgggacagg acagagagac     60
agagcagccc tgcactgttt tccctccacc acagccatcc tgtccctcat tggctctgtg    120
ctttccacta tacacagtca ccgtcccaat gagaaacaag aaggagcacc ctccacatgg    180
actcccacct gcaagtggac agcgacattc agtcctgcac tgctcacctg ggtttactga    240
tgactcctgg ctgccccacc atcctctctg atctgtgaga aacagctaag ctgctgtgac    300
ttccctttag gacaatgttg tgtaaatctt tgaaggacac accgaagacc tttatactgt    360
gatcttttac ccctttcact cttggctttc ttatgttgc                           399

<210>  628
<211>  548
<212>  DNA
<213>  human
<400>  628
ctgccccgag ctcgaagaag aggctgagtg cgtccctgat aactgcgtct aagaccagag     60
ccccgcagcc cctggggccc ccggagccat ggggtgtcgg gggctcctgt gcaggctcat    120
gctgcaggcg gccgaggcac agggggtttc gcgctgctcc tgaccgcggt gaggccgcgc    180

cgaccatctc tgcactgaag ggccctctgg tggccggcac gggcattggg aaacagcctc 240
ctcctttccc aaccttgctt cttaggggcc cccgtgtccc gtctgctctc agcctcctcc 300
tcctgcagga taaagtcatc cccaaggctc cagctactct aaattatggt ctccttataa 360
gttattgctg ctccaggaga ttgtccttca tcgtccaggg gcctggctcc cacgtggttg 420
cagatacctc agacctggtg ctctaggctg tgctgagccc actctcccga gggcgcatcc 480
aagcgggggc cacttgagaa gtgaataaat ggggcggttt cggaagcgtc agtgtttcca 540
tgttatgg 548

<210> 629
<211> 524
<212> DNA
<213> human
<400> 629
gaagttgcaa cattcgtttg ataggaattc cagaaaagga gagttatgag aatagggcag 60
aggacataat taaagaaata attgatgaaa actttgcaga actaaagaaa ggttcaagtc 120
ttgagattgt cagtgcttgt cgagtaccta gtaaaattga tgaaaagaga ctgactccta 180
gacacatctt ggtgaaattt tggaattcta gtgataaaga gaaaataata agggcttcta 240
gagagagaag agaaattacc taccaaggaa caagaatcag gttgacagca gacttatcac 300
tggacacact ggatgctaga agtaaatgga gcaatgtctt caaagttctg ctggaaaaag 360
gctttaatcc tagaatccta tatccagcca aaatggcatt tgattttagg ggcaaaacaa 420
aggtatttct tagtattgaa gaatttagag attatgtttt gcatatgccc accttgagag 480
aattactggg gaataatata ccttagcacg ccagggtgac taca 524

<210> 630
<211> 488
<212> DNA
<213> human
<400> 630
gagtggttca tccatactct cattccctcg cctccccttg tggacggggg tcttgccttt 60
tcaattcctg tgttttggtg tcttccctta tctgctaccc tgaatcacct gtcctggtct 120
tgctgtgtga tgggaacatg cttgtaaact gcgtaacaaa tctactttgt gtatgtgtct 180
gtttatgggg gtggtttatt atttttgctg gtccctagac cactttgtat gaccgtttgc 240
agtctgagca ggccaggggc tgacagctaa tgtcaggacc ctcagcggtg gagcctgctg 300
gggggaccca gctgctcttg gacaagtggc tgagctccta tctggcctcc tctttttttt 360
ttttttcaag taatttgtgt gtatttctaa ctgattgtat tgaaaaaatt cctagtattt 420
cagtaaaaat gcctgttgtg agatgaacct cctgtaactt ctatctgttc ttttttgagg 480
ctcaggga 488

<210> 631
<211> 460
<212> DNA
<213> human
<400> 631
tggaattaga ccatttggcc tttgaacttt cataggaaaa atgacccaac atttcttagc 60
atgagctacc tcatctctag aagctgggat ggacttacta ttcttgttta tattttagat 120
actgaaaggt gctatgcttc tgttattatt ccaagactgg agataggcag ggctaaaaag 180
gtattattat ttttccttta atgatggtgc taaaattctt cctataaaat tccttaaaaa 240
taaagatggt ttaatcacta ccattgtgaa aacataactg ttagacttcc cgtttctgaa 300
agaaagagca tcgttccaat gcttgttcac tgttcctctg tcatactgta tctggaatgc 360
tttgtaatac ttgcatgctt cttagaccag aacatgtagg tccccttgtg tctcaatact 420
ttttttttct taattgcatt tgttggctct attttaattt 460

<210> 632
<211> 446
<212> DNA
<213> human
<400> 632
tagattccgg tatatcgttc ttcaagacac ttgctctaag cggaatggaa atgtgggcaa 60
gactgtcttt gaatatagaa cacagaatgt ggcacgcttg cccatcatag atcttgctcc 120
tgtggatgtt ggcggcacag accaggaatt cggcgttgaa attgggccag tttgttttgt 180
gtaaagtaag ccaagacaca tcgacaatga gcaccaccat caatgaccac cgccattcac 240
aagaactttg actgtttgaa gttgatcctg agactcttga agtaatggct gatcctgcat 300
cagcattgta tatatggtct taagtgcctg gcctccttat ccttcagaat atttatttta 360

cttacaatcc tcaagtttta attgatttta aatatttttc aatacaacag tttaggttta     420
agatgaccaa tgacaatgac caccttt                                          446

<210>  633
<211>  552
<212>  DNA
<213>  human

<220>
<221>  misc_feature
<222>  (63)..(63)
<223>  n is a, c, g, or t
<220>
<221>  misc_feature
<222>  (72)..(72)
<223>  n is a, c, g, or t
<220>
<221>  misc_feature
<222>  (74)..(74)
<223>  n is a, c, g, or t
<220>
<221>  misc_feature
<222>  (85)..(85)
<223>  n is a, c, g, or t
<220>
<221>  misc_feature
<222>  (138)..(138)
<223>  n is a, c, g, or t

<400>  633
tttcagcacc gatggccatg taaataagat gatttaatgt tgattttaat cctgtatata      60
aantaaaaag tncncaatga gtttngggca tatttaatga tgattatgga gccttagagg     120
tctttaatca ttggttcngg ctgcttttat gtagtttagg ctggaaatgg tttcacttgc     180
tctttgactg tcagcaagac tgaagatggc ttttcctgga cagctagaaa acacaaaatc     240
ttgtaggtca ttgcacctat ctcagccata ggtgcagttt gcttctacat gatgctaaag     300
gctgcgaatg ggatcctgat ggaactaagg actccaatgt cgaactcttc tttgctgcat     360
tcctttttct tcacttacaa gaaaggcctg aatggaggac ttttctgtaa ccaggaacat     420
tttttagggg tcaaagtgct aataattaac tcaaccaggt ctacttttta atggctttca     480
taacactaac tcataaggtt accgatcaat gcatttcata cggatataga cctagggctc     540
tggagggtgg gg                                                          552

<210>  634
<211>  440
<212>  DNA
<213>  human

<220>
<221>  misc_feature
<222>  (165)..(165)
<223>  n is a, c, g, or t

<400>  634
ctggcctacg tgttcaattt tctatgaaca aaggctttag tccttgaccc agggctaaag      60
tggtctgtcc aagctgttgt tggtagaggg agtatgataa aatgtttaaa tctcatttgg     120
ttaccttgag tcctggaaca cgcagtaact gtcatgctat agtcntcatc tgtatttggc     180
tgggaataca aatgaagatt gtggtgtatt caagcagtag ggtttttgct tttgtttttg     240
ttttagtgcc aacaaaactt ttttttgtct gactacatta aagataagac tgactatatt     300
tatacaacag aaactttgta atagattttt tcagctttgt gaaatcgaat tttttttcat     360
cagggctggt tggatttcct ttttaccctg taatccaagc gttaatagtt tgttagaaga     420
tgggttattg catgtcactt                                                  440

<210>  635
<211>  360

<212>  DNA
<213>  human
<400>  635
gaagatcaag gatgttctca acagtctaga gtacagtccc tctcctataa gcaagaagct      60
ctcgtgtgct agtgtcaaaa gccaaggcag accgtcctcc tgccctgctg ggatggctgt     120
cactggctgt gcttgtggct atggctgtgg ttcgtgggat gttcagctgg aaaccacctg     180
ccactgccag tgcagtgtgg tggactggac cactgcccgc tgctgccacc tgacctgaca     240
gggaggaggc tgagaactca gttttgtgac catgacagta atgaaaccag ggtcccaacc     300
aagaaatcta actcaaacgt cccacttcat ttgttccatt cctgattctt gggtaataaa     360

<210>  636
<211>  490
<212>  DNA
<213>  human
<400>  636
ggagctcaga gatctaagct gctttccatc ttttctccca gccccaggac actgactctg      60
tacaggatgg ggccgtcctc ttgcctcctt ctcatcctaa tcccccttct ccagctgatc     120
aacccgggga gtactcagtg ttccttagac tccgttatgg ataagaagat caaggatgtt     180
ctcaacagtc tagagtacag tccctctcct ataagcaaga agctctcgtg tgctagtgtc     240
aaaagccaag gcagaccgtc ctcctgccct gctgggatgg ctgtcactgg ctgtgcttgt     300
ggctatggct gtggttcgtg ggatgttcag ctggaaacca cctgccactg ccagtgcagt     360
gtggtggact ggaccactgc ccgctgctgc cacctgacct gacagggagg aggctgagaa     420
ctcagttttg tgaccatgac agtaatgaaa ccagggtccc aaccaagaaa tctaactcaa     480
acgtcccact                                                            490

<210>  637
<211>  577
<212>  DNA
<213>  human
<400>  637
gcctgagaaa gcaagcacgc actctcagtc aacatgacag attctggagg ataaccagca      60
ggagcagaga taacttcagg aagtccattt ttgcccctgc ttttgctttg gattatacct     120
caccagctgc acaaaatgca ttttttcgta tcaaaaagtc accactaacc ctcccccaga     180
agctcacaaa ggaaaacgga gagagcgagc gagagagatt tccttggaaa tttctcccaa     240
gggcgaaagt cattggaatt tttaaatcat aggggaaaag cagtcctgtt ctaaatcctc     300
ttattctttt ggtttgtcac aaagaaggaa ctaagaagca ggacagaggc aacgtggaga     360
ggctgaaaac agtgcagaga cgtttgacaa tgagtcagta gcacaaaaga gatgacattt     420
acctagcact ataaaccctg gttgcctctg aagaaactgc cttcattgta tatatgtgac     480
tatttacatg taatcaacat gggaactttt aggggaacct aataagaaat cccaattttc     540
aggagtggtg gtgtcaataa acgctctgtg gccagtg                              577

<210>  638
<211>  351
<212>  DNA
<213>  human
<400>  638
ggaacccaga gctgctgtgt atttcgagcg ggcagtttat cttttgctat acttattttc      60
aattcaatta caccacgatt caaataattc ccctcctaaa accaaaaagg agggaaacgt     120
caactccatt gcaattactt atcttcctct tctatctctg ttatacgccg gggcatagaa     180
tgctcgtata catctcttta acaaccacaa accttaagcc atgtagatga agttagtgca     240
tcaacgggat acagttccat attgccttaa acctccttgt tttagacaca ctaacattta     300
taccaaattg cagattattc tgcagagagg gaattgcatg tttgtgttgt a              351

<210>  639
<211>  506
<212>  DNA
<213>  human
<400>  639
aattaatatt catcgcactt cttctgtgga aggactttgt gaaggaattg gtgctggatt      60
agtggatgtt gctatctggg ttggcacttg ttcagattac ccaaaaggag atgcttctac     120
tggatggaat tcagtttctc gcatcattat tgaagaacta ccaaaataaa tgctttaatt     180
ttcatttgct acctcttttt ttattatgcc ttggaatggt tcacttaaat gacattttaa     240
ataagtttat gtatacatct gaatgaaaag caaagctaaa tatgtttaca gaccaaagtg     300

tgatttcaca ctgtttttaa atctagcatt attcattttg cttcaatcaa aagtggtttc 360
aatatttttt ttagttggtt agaatacttt cttcatagtc acattctctc aacctataat 420
ttggaatatt gttgtggtct tttgtttttt ctcttagtat agcattttta aaaaaatata 480
aaagctacca atctttgtac aatttg 506

<210> 640
<211> 360
<212> DNA
<213> human

<220>
<221> misc_feature
<222> (128)..(128)
<223> n is a, c, g, or t
<220>
<221> misc_feature
<222> (190)..(190)
<223> n is a, c, g, or t
<220>
<221> misc_feature
<222> (206)..(206)
<223> n is a, c, g, or t
<400> 640
aaatgactgg atggtcgctg ctttttaagt ttcaaattga cattccagac aagcggtgcc 60
tgagcccgtg cctgtcttca gatcttcaca gcacagttcc tgggaaggtg gagccaccag 120
cctctccntg aataactggg agatgaaaca ggaagctcta tgacacactt gatcgaatat 180
gacagacacn gaaaatcacg actcancccc ctccagcacc tctacctgtt gcccgccgat 240
cacagccgga atgcagctga aagattccct ggggcctggt tccaaccgcc cactgtggac 300
tctgaggcct ctgcatttgc gggtggtctg cctgtgatat tttggtcatg ggctggtctg 360

<210> 641
<211> 427
<212> DNA
<213> human
<400> 641
gaagaggagc aacatctatg ccaaatactg tgcattctac aatggtgcta atctcagacc 60
taaatgatac tccatttaat ttaaaaaaga gttttaaata attatctatg tgcctgtatt 120
tccctttgga gtgctgcaca acatgttaac atattagtgt aaaagcagat gaaacaacca 180
cgtgttctaa agtctaggga ttgtgctata atccctattt agttcaaaat taaccagaat 240
tcttccatgt gaaatggacc aaactcatat tattgttatg taaatacaga gttttaatgc 300
agtatgacat cccacagggg aaaagaatgt ctgtagtggg tgactgttat caaatatttt 360
atagaataca atgaacggtg aacagactgg taacttgttt gagttcccat gacagatttg 420
agacttg 427

<210> 642
<211> 574
<212> DNA
<213> human

<220>
<221> misc_feature
<222> (83)..(83)
<223> n is a, c, g, or t
<220>
<221> misc_feature
<222> (94)..(94)
<223> n is a, c, g, or t
<220>
<221> misc_feature
<222> (103)..(103)
<223> n is a, c, g, or t
<220>
<221> misc_feature

<222>  (134)..(138)
<223>  n is a, c, g, or t
<220>
<221>  misc_feature
<222>  (140)..(141)
<223>  n is a, c, g, or t
<220>
<221>  misc_feature
<222>  (143)..(143)
<223>  n is a, c, g, or t
<220>
<221>  misc_feature
<222>  (145)..(146)
<223>  n is a, c, g, or t
<220>
<221>  misc_feature
<222>  (169)..(169)
<223>  n is a, c, g, or t
<220>
<221>  misc_feature
<222>  (171)..(172)
<223>  n is a, c, g, or t
<220>
<221>  misc_feature
<222>  (174)..(177)
<223>  n is a, c, g, or t
<220>
<221>  misc_feature
<222>  (179)..(179)
<223>  n is a, c, g, or t
<220>
<221>  misc_feature
<222>  (181)..(181)
<223>  n is a, c, g, or t
<220>
<221>  misc_feature
<222>  (183)..(184)
<223>  n is a, c, g, or t
<220>
<221>  misc_feature
<222>  (186)..(193)
<223>  n is a, c, g, or t
<220>
<221>  misc_feature
<222>  (196)..(196)
<223>  n is a, c, g, or t
<220>
<221>  misc_feature
<222>  (198)..(203)
<223>  n is a, c, g, or t
<220>
<221>  misc_feature
<222>  (206)..(207)
<223>  n is a, c, g, or t

<400>  642
tgaaagtggg catctctgca ggcacctgta ctgccatcct gctcaccgtc ttgacctgct      60
acttttggaa aaagaatcaa aanctagagt acangtactc cangctggtg atgaatgcta     120
ctctcaagga ctgnnnnntn ncngnngctg acagctgcgc catcatggna nncnnnnang     180
nanngnnnnn nnncancnnn nnncanntgc gccatcatgg aaggcgagga tgtagaggac     240
gacctcatct ttaccagcaa gaagtcactc tttgggaaga tcaaatcatt tacctccaag     300
aggactcctg atggatttga ctcagtgccg ctgaagacat cctcaggagg cccagacatg     360
gacctgtgag aggcactgcc tgcctcacct gcctcctcac cttgcatagc acctttgcaa     420

gcctgcggcg atttgggtgc cagcatcctg caacacccac tgctggaaat ctcttcattg 480
tggccttatc agatgtttga atttcagatc tttttttata gagtacccaa accctccttt 540
ctgcttgcct caaacctgcc aaatataccc acac 574

<210> 643
<211> 485
<212> DNA
<213> human
<400> 643
aaacgacgca aatctctgag ctggggacca cttggagaac cggcttagta acagtcctga 60
tcttcgcaag ccagcttctt ctgcatctga ggggctcctg gcgcccagag gaggcagaca 120
gatgtcttct agctgagttt ctaaccgcat gatgagactc agaccttccg ctgcactaga 180
aaatctgcaa cagtgtccct gagtcacttc tccttagtgg gcagactcgt gttagatttg 240
tggaacccag ctctctgatt tactcctttt ggaaaaccca tggaatttca tgtataaggc 300
tttcatttgt attttaaggt ttttctgttt gttttgagta tatacatggt gctcaatagc 360
aacatcttag cagatgaagc agtttatgat tccactccct cctgtatgac aggtagccac 420
tatactgaat caaggtgctg aactcaaatc acaaaattct ggcttaccga tacaacaacc 480
aatac 485

<210> 644
<211> 487
<212> DNA
<213> human

<220>
<221> misc_feature
<222> (296)..(296)
<223> n is a, c, g, or t

<400> 644
tataaggtaa ctctttagtc ctccatttag cacattttaa atcctccaaa gaataagtat 60
catgtgatta ttttagcttt acaaaaaaaa agttgaatgg cgttttattt tcatggccta 120
taagcaggta ccttagtagg gcagatatag gaaaaacaaa ttagagcaaa acaaatcctc 180
tacaaatcca aggcaggaaa agtggtggca gagtgactca ttctcctgtc cctcccatca 240
ggtcaaatca ggaggctgca gtgaatgcct gttctttgaa tgtgtagcag ttgttncctg 300
taactcttta aaacttggct ataggctgtt tagcacagta cagattaaag atacagttac 360
gtaaacagca aagtaatttt atagtgcttc atccatttat catgctttgg tttgctaatt 420
ttttcacata cctttttcta tcacagtctg ttgcttttgt acacatttct catattgggg 480
ttcgaca 487

<210> 645
<211> 520
<212> DNA
<213> human
<400> 645
ggtgccagga aggtcacaga tggacactgg ccattctggt catctcagtc tggaactcag 60
tcccacttct tggcctggac aatgaacagg attcagtttt gctgttaact ttgcttctct 120
actttttttt gtttgtttgt aatagcacat cccagagaca tcagaaacca gcaactgatt 180
cagtgtgatt tccagacttt ttaggcatga aattcggaca cttcagtatt tccaggaata 240
gcatatgcac gctgttcttg cttcatggaa tgctacatgc tttctgtttt tctcattttg 300
gatttctcca aaactaactg aatttaagct tcaggtccct ttgtatgcag tagaaaggaa 360
ttattaaaaa caccaccaaa gaaaataaat atatcctact tgaaatttac tctatggact 420
tacccactgc tagaataaat gtatcaaatc ttatttgtaa attctcaatt ttgatatata 480
tatgtatata tgcatataca tatccacact tgtctgcaag 520

<210> 646
<211> 516
<212> DNA
<213> human
<400> 646
ttaccctcta tttaaatgct ttgaaaaaca gtgcattgac aatgggttga tatttttctt 60
taaaagaaaa atataattat gaaagccaag ataatctgaa gcctgtttta ttttaaaact 120
ttttatgttc tgtggttgat gttgtttgtt tgtttgtttc tattttgttg gttttttact 180

ttgttttttg ttttgttttg ttttgttttg catactacat gcagttcttt aaccaatgtc 240
tgtttggcta atgtaattaa agttgttaat ttatatgagt gcatttcaac tatgtcaatg 300
gtttcttaat atttattgtg tagaagtact ggtaattttt ttatttacaa tatgtttaaa 360
gagataacag tttgatatgt tttcatgtgt ttatagcaga agttatttat ttctatggca 420
ttccagcgga tattttggtg tttgcgaggc atgcagtcaa tattttgtac agttagtgga 480
cagtattcag caacgcctga tagcttcttt ggcctt 516

<210> 647
<211> 390
<212> DNA
<213> human
<400> 647
ccacacacca ggcactaatc acctggtgag gatttggcat atccaccaaa aaatgcatcc 60
gatttaacca acatctccac cagcgctacg gactcctccc aattctgaca tctcttgcag 120
acaatactat gctctctaca cactgtttag aaatggaaag gtgatctgca ctgtatcttg 180
ggtttgttgg ctatgcttcc tttgatgaca tatattatac agtatatata tacatatatt 240
ttttttgtta gagttctagc cattttattt ctccgcaggg tcctttctca gacattactg 300
catgctgtat atggcgttag ctgtgtgttg atcttctaaa agatgataga gtttactggt 360
aattgtgtaa tcagctcctg cctttttatt 390

<210> 648
<211> 505
<212> DNA
<213> human

<220>
<221> misc_feature
<222> (39)..(39)
<223> n is a, c, g, or t

<400> 648
caggaaactc ttctctgact tggggagttc ctacgcgana caactgggct tccgggacag 60
ctgggtcttc ataggagcca aagacctcag gggtaaaagc ccctttgagc agttcttaaa 120
gaacagccca gacacaaaca aatacgaggg atggccagag ctgctggaga tggagggctg 180
catgcccccg aagccatttt agggtggctg tggctcttcc tcagccaggg gcctgaagaa 240
gctcctgcct gacttaggag tcagagcccg gcaggggctg aggaggagga gcagggggtg 300
ctgcgtggaa ggtgctgcag gtccttgcac gctgtgtcgc gcctctcctc ctcggaaaca 360
gaaccctccc acagcacatc ctacccggaa gaccagcctc agagggtcct tctggaacca 420
gctgtctgtg gagagaatgg ggtgctttcg tcagggactg ctgacggctg gtcctgagga 480
aggacaaact gcccagactt gagcc 505

<210> 649
<211> 331
<212> DNA
<213> human

<220>
<221> misc_feature
<222> (73)..(74)
<223> n is a, c, g, or t
<220>
<221> misc_feature
<222> (76)..(76)
<223> n is a, c, g, or t

<400> 649
cacttgatag actgtaagca cctgcttaac tttgtgtccc aaatatttag tgtgtatata 60
tatatatata tanncncaca cacacacata tatattcaac aaataaagca aaatataaca 120
tgcatttcac atttgtcttt tccctgttac gattttaata gcagaactgt atgacaagtt 180
taggtgatcc tagcatatgt taaattcaaa ttaatgtaaa acagattaac aacaacaaag 240
aaactgtcta tttgagtgaa gtcatgcttt ctattataat aacttggctt cggttatcca 300
tcaaatgcac acttatactg ttatctgatt g 331

<210>  650
<211>  582
<212>  DNA
<213>  human

<220>
<221>  misc_feature
<222>  (179)..(180)
<223>  n is a, c, g, or t
<220>
<221>  misc_feature
<222>  (218)..(218)
<223>  n is a, c, g, or t
<220>
<221>  misc_feature
<222>  (226)..(227)
<223>  n is a, c, g, or t

<400>  650
gccaaaaggc ctggcttctg ataggaaact ggtaagaaac tcttcatgaa aacacatcac      60
taatattcgc tattactctc ctggtctgaa gtcagctttt ctgaaccatt aaggtatttc     120
atcacaagtt atattttata atatcagttt aagaggcttt tattcatgtg aacaccagnn     180
ccctttcagg ggcatggtct ttttgaaaaa aaaaaaanaa aaaaannaac agttttagcc     240
acatatcaga tatttctata tctaattatc ctttatggct aacattctgc ctccattgtt     300
aaggtataat tgttcctgaa tttaaaggtg gtttggcctc taatttaatt ctgattcaga     360
ctctcctgtc aggactcaag aaaatttaat taattaccaa ggattaagtc ttctggttaa     420
ggtttctggg aaaaaaaaat agcaaagatg ttgatttctt ggaatccttt tacaggttca     480
taacagaaaa atcttcattc cctgtaggca tttaattaaa cctagttgag aagtgtgtgg     540
gattcctcaa ttatgaacaa aacacgtata ttggctttct tt                        582

<210>  651
<211>  433
<212>  DNA
<213>  human
<400>  651
gacatgattg tctataatct cgctagcctt gtactgtgtg tgcatagcaa ttacagggaa      60
gtaatctagc tcctgactat tatgttgaac tatgtcgctg ctttttacaa acttgtcttg     120
atccaaagca gtcacaatga taaccctgca tatctgggaa tcataagtca actatgtatc     180
tctgtgtgtg tatatatatg tatgtatgta tctattttca aactgtgatt taatatttaa     240
atattcctac tgccattttt gtgactgaaa aactacacat gaggaaacgt cttagaattt     300
tccaatagag gaaaaataac acttgggcaa tctgtcatgt ttcacaacag ttctcatttt     360
tctcatgatt tgtgtagcgt ggaatgtgtt tgctcaatgt gaagggtttt cattgctcaa     420
tttctctgtg taa                                                        433

<210>  652
<211>  361
<212>  DNA
<213>  human

<220>
<221>  misc_feature
<222>  (71)..(71)
<223>  n is a, c, g, or t
<220>
<221>  misc_feature
<222>  (295)..(304)
<223>  n is a, c, g, or t

<400>  652
ggttccggct aacacatttt ctaagtcgcc agtgctgctt acagtttgaa tacatgaaaa      60
tcctgtttct nagatgtttg cgcacgtgct tattaggaaa tgagtctgta tggaaatctc     120
accacagata atggttaacg aaccgggtcg acatcacaaa ggagggtgga gactcttttt     180
actaacttga atgagacaaa agcagtggtg tcagtttata atcctgatgc atttcagtaa     240

taatgtagaa aaacattatt ttaaaaaagt tccaacacac agccatgagg agccnnnnnn 300
nnnntcagtt ttgaaagagg tgcataataa aactactaac cagaggagtc tatgccattt 360
t 361

<210> 653
<211> 415
<212> DNA
<213> human

<220>
<221> misc_feature
<222> (42)..(42)
<223> n is a, c, g, or t
<220>
<221> misc_feature
<222> (95)..(95)
<223> n is a, c, g, or t
<220>
<221> misc_feature
<222> (353)..(353)
<223> n is a, c, g, or t

<400> 653
gagtttcaac tttaaatgtt cactatgtca tttagtgtcc anctttacgg ataggttgac 60
tatctaaata ggcattttta gtcattaaaa aaaantctag tcaccaggag gatccctata 120
actcaaaata acttgtttgt aaaagaaaat ttgtttactt acccattagt aagttcctgc 180
atattcatta taagatggca aatcaaactt ttctaggatg aagacagctt atttttaagt 240
tgtatagtct tagttggttt agggtctcaa ttttaattaa taaaatactt ggtttttatt 300
tgcttgtcct tttgaattcc tgttttaata attttaaaat gagcacaaag aangttgaag 360
ttcagattaa tctcttctga atgatgtttt tttcctctgt gatgagttgt ttctg 415

<210> 654
<211> 364
<212> DNA
<213> human

<220>
<221> misc_feature
<222> (282)..(282)
<223> n is a, c, g, or t
<220>
<221> misc_feature
<222> (294)..(294)
<223> n is a, c, g, or t
<220>
<221> misc_feature
<222> (336)..(337)
<223> n is a, c, g, or t

<400> 654
tgctgtagtc actggtgttc ctcacccacc agctgtaact cagtttgtgt gaggtacagc 60
cacagaagat gtcatgtact gtatattacc tggtgatagt tgcttttcac cccccgagtt 120
cagtttctag gagccaatga aacttcccct cacctcctca tctttccaag ttgttctttg 180
aattgaggag tttgaaggca taaacagtta cttggggatt tgcgaaaatc ctacttagtt 240
actgcgttta cagttctttg gcccagtctc tgacccttcc cnagtatttg tgcnatgatt 300
gtgtttactg ctggattttt gaaggttttt tttttnnaag aaagtgccat ttcattattt 360
gatt 364

<210> 655
<211> 436
<212> DNA
<213> human
<400> 655

gggatcactg ggagaagcca tggcattatc ttcaggcaat ttagtctgtc ccaaataaaa 60
taaatccttg catgtaaatc attcaagggt tatagtaata tttcatatac tgaaaagtgt 120
ctcataggag tcctcttgca catctaaaaa ggctgaacat ttaagtatcc cgaattttct 180
tgaattgctt tccctataga ttaattacaa ttggatttca tcatttaaaa accatacttg 240
tatatgtagt tataatatgt aaggaataca ttgtttataa ccagtatgta cttcaaaaat 300
gtgtattgtc aaacatacct aactttcttg caataaatgc aaaagaaact ggaacttgac 360
aattataaat agtaatagtg aagaaaaaat agaaaggttg caattatata ggccatgggt 420
ggctcaaaac tttgaa 436

<210> 656
<211> 427
<212> DNA
<213> human

<220>
<221> misc_feature
<222> (45)..(51)
<223> n is a, c, g, or t
<220>
<221> misc_feature
<222> (402)..(402)
<223> n is a, c, g, or t

<400> 656
gattcctgga ttgatgtctt atctacaggc ttctttttaa aaatnnnnnn ngattggctg 60
tgagtttacc aggattatag ttgaggctaa aggacagctc ctcaggaaag cccctgttct 120
acatctacgg tcacatgctg gaccttgagt tgtcactcag agaaaagagt gccatctacc 180
gaaactccac agtttccatt gtgaatggct tctttggtgc agagttccaa aaattatgta 240
gcccagctct ttaattttgt aacatctaat gatatcaccg ccttgaagtg attaaagtag 300
attgcttaaa gaattaaagc tttaaagatg aaagatgtta ttgcttttgc tggacatgag 360
gaacagttgt aaagtttcca ggtctacaat aactttctgg anccctctca gtgaactgtt 420
tcttgta 427

<210> 657
<211> 559
<212> DNA
<213> human
<400> 657
gaagtccatc ctttggtcca aagcatctgg aagaggaaga agagaggaat gagaaagaag 60
gaagtgatgc aaaacatctc caaagaagtc ttttggaaca ggaaaatcat tcaccactca 120
cagggtcaaa tatgaaatac aaaaccacga accaatcaac agaattttta tccttccaag 180
atgccagctc attgtacaga aacattttag aaaaagaaag ggaacttcag caactgggaa 240
tcacagaata cctaaggaaa aacattgctc agctccagcc tgatatggag gcacattatc 300
ctggagccca cgaagagctg aagttaatgg aaacattaat gtactcacgt ccaaggaagg 360
tattagtgga acagacaaaa aatgagtatt ttgaacttaa agctaattta catgctgaac 420
ctgactattt agaagtcctg gagcagcaaa catagatgga gagtttgagg gctttcgcag 480
aaatgctgtg attctgtttt aagtccatac cttgtaaata agtgccttac gtgagtgtgt 540
catcaatcag aacctaagc 559

<210> 658
<211> 368
<212> DNA
<213> human
<400> 658
gctgctcaag cgcctgcaga acaacgacac gtgcagcatg ccaggcctca cgtgcttcac 60
ccacgacaac cagcactggc agacggcgcc tttctggaca ctggggcctt tctgtgcctg 120
caccagcgcc aacaataaca cgtactggtg catgaggacc atcaatgaga ctcacaattt 180
cctcttctgt gaatttgcaa ctggcttcct agagtacttt gatctcaaca cagaccccta 240
ccagctgatg aatgcagtga acacactgga cagggatgtc ctcaaccagc tacacgtaca 300
gctcatggag ctgaggagct gcaagggtta caagcagtgt aacccccgga ctcgaaacat 360
ggacctgg 368

<210> 659

<211>  436
<212>  DNA
<213>  human

<220>
<221>  misc_feature
<222>  (300)..(300)
<223>  n is a, c, g, or t

<400>  659
acaatcggct aaccttgaca tttcttttta ccttcatatg ccactatctc ggtagttcaa      60
aaaaatttag ttcttgataa attgccttga agtttacctt gtgctggaga gccttatgat     120
aactccaaag actttcttac ggtataatac atgttgttta ggattgtgtt tcttagtcac     180
tgaagataat aaatattaaa atggatgttt tcatcagaaa attttcatgt tttcctttaa     240
ggtaacataa ttgtaagaat tgtttaataa aatactcagg aaattctaaa ggtttctccn     300
aatacctaaa catttctgaa catcagtatt gcagttgtgg aagagcagaa ggaggataca     360
tttgtttgtg ttgctcccca aaattccacc ttgcatttgc atcacaaact tccctcaatt     420
gaggcagttt tctttg                                                     436

<210>  660
<211>  431
<212>  DNA
<213>  human

<220>
<221>  misc_feature
<222>  (311)..(311)
<223>  n is a, c, g, or t
<220>
<221>  misc_feature
<222>  (391)..(391)
<223>  n is a, c, g, or t

<400>  660
cagtgctgct gtgaactaaa gtatgtcatt tatgctcaaa gtttaattct tcttcttggg      60
atattttaaa aatgctactg agattctgct gtaaatatga ctagagaata tattgggttt     120
gctttatttc ataggcttaa ttctttgtaa atctgaatga ccataataga aatacatttc     180
ttgtggcaag taattcacag ttgtaaagta aataggaaaa attattttat ttttattgat     240
gtacattgat agatgccata aatcagtagc aaaaggcact tctaaaggta agtggtttaa     300
gttgcctcaa nagagggaca atgtagcttt attttacaag aaggcatagt tagatttcta     360
tgaaatattt attctgtaca gttttatata nttttggttc acaaaagtaa ttattcttgg     420
gtgcctttca a                                                          431

<210>  661
<211>  322
<212>  DNA
<213>  human

<220>
<221>  misc_feature
<222>  (37)..(38)
<223>  n is a, c, g, or t
<400>  661
aaagtatatt gtgctagctt gtctaagaat aaacttnnat actgttgggg gagggctgca      60
cctgtcaaga taacctgtca atgtagtagg aaaacaggag gggacagtaa cagaaaagca     120
cgggaaaaga tggcaaggtt agttaaaata gaaaagtgct cagttcctca tacctgtaat     180
cccagcagtt tagggggcca aggaaggtgg gtcacttgag cccaagagtt caaggccatc     240
ctgggcaatg tggcgaaagt gtctacaaaa aaatacaaaa agaggaagaa atgatatttc     300
acaagtttgt atcatttgtc at                                              322

<210>  662
<211>  282
<212>  DNA

<213>  human

<220>
<221>  misc_feature
<222>  (43)..(43)
<223>  n is a, c, g, or t
<220>
<221>  misc_feature
<222>  (50)..(50)
<223>  n is a, c, g, or t
<220>
<221>  misc_feature
<222>  (99)..(99)
<223>  n is a, c, g, or t
<220>
<221>  misc_feature
<222>  (115)..(115)
<223>  n is a, c, g, or t

<400>  662
cagtccaaaa tccatagaca cagacagtag aaaggtggtt atncaagggn ctggtaggag      60
agggagttag tatttagtgg tatagagttt tagtgtttnc cgggtaaaaa tgttnctaga     120
gatctgttgc aaccatgtga atatacttaa cactaccgta ctgtacactg aaaaaatggt     180
taagaggata aatttaaatg gttaagagga aaaatggtta agaggataat gttgttttgt     240
taccacagta gtaactttta aaaaccctca tgtatgttct tt                        282

<210>  663
<211>  398
<212>  DNA
<213>  human

<220>
<221>  misc_feature
<222>  (80)..(80)
<223>  n is a, c, g, or t
<220>
<221>  misc_feature
<222>  (82)..(83)
<223>  n is a, c, g, or t
<220>
<221>  misc_feature
<222>  (85)..(85)
<223>  n is a, c, g, or t
<220>
<221>  misc_feature
<222>  (87)..(87)
<223>  n is a, c, g, or t
<220>
<221>  misc_feature
<222>  (97)..(97)
<223>  n is a, c, g, or t

<400>  663
caacccattt tgtgccacat gcaagttttg aataaggatg gtatagaaaa caacgctgca      60
tatacaggta ccatttaggn annancngat gcctttntgg gggcagaatc acatggcaaa     120
agctttgaaa atcataaaga tataagttgg tgtggctaag atggaaacag ggctgattct     180
tgattcccaa ttctcaactc tccttttcct atttgaattt ctttggtgct gtagaaaaca     240
aaaaaagaaa aatatatatt cataaaaaat atggtgctca ttctcatcca tccaggatgt     300
actaaaacag tgtgtttaat aaattgtaat tattttgtgt acagttctat actgttatct     360
gtgtccattt ccaaaacttg cacgtgtccc tgaattcc                            398

<210>  664
<211>  22

<212>  DNA
<213>  synthetic
<400>  664
tcgtttttcg cgtttagttg tc                                                22

<210>  665
<211>  17
<212>  DNA
<213>  synthetic
<400>  665
gcgcaccgaa aaaaccg                                                      17

<210>  666
<211>  24
<212>  DNA
<213>  synthetic
<400>  666
cggttacggt tagtattcgg tttc                                              24

<210>  667
<211>  23
<212>  DNA
<213>  synthetic
<400>  667
aaaatacgac gataaaaaac gcg                                               23

<210>  668
<211>  23
<212>  DNA
<213>  synthetic
<400>  668
cgagggtttt ttagcgattt gtc                                               23

<210>  669
<211>  17
<212>  DNA
<213>  synthetic
<400>  669
aaacgaaccg ctccccg                                                      17

<210>  670
<211>  21
<212>  DNA
<213>  synthetic
<400>  670
ttcggcgttt tcgtttttta c                                                 21

<210>  671
<211>  21
<212>  DNA
<213>  synthetic
<400>  671
   ccccttaaca acataatcgc g                                                21

<210>  672
<211>  23
<212>  DNA
<213>  synthetic
<400>  672
gatggtgcgt tcggtattta tgt                                               23

<210>  673
<211>  19

<212>  DNA
<213>  synthetic
<400>  673
gcttacaccc gcccgacta                                                    19

<210>  674
<211>  28
<212>  DNA
<213>  synthetic
<400>  674
gttttgtgat tggttaggtt attaaagt                                          28

<210>  675
<211>  25
<212>  DNA
<213>  synthetic
<400>  675
ccrtcacctt ctacaccaaa aaata                                             25

<210>  676
<211>  30
<212>  DNA
<213>  synthetic
<400>  676
tgtggattta tttttataag gatattgaat                                        30

<210>  677
<211>  32
<212>  DNA
<213>  synthetic
<400>  677
ataactcatt aaaaactcac atctaaacac ta                                     32

<210>  678
<211>  28
<212>  DNA
<213>  synthetic
<400>  678
ttaagaaaga gagtaaggag gaagagtt                                          28

<210>  679
<211>  24
<212>  DNA
<213>  synthetic
<400>  679
aaaatcccaa ttaaaaaaca acca                                              24

<210>  680
<211>  33
<212>  DNA
<213>  synthetic
<400>  680
caaaataata aatccctcta ctactattat caa                                   33

<210>  681
<211>  30
<212>  DNA
<213>  synthetic
<400>  681
ggaggaaagg aattagttta gaagttagtt                                        30

## Description

### FIELD OF THE INVENTION

The present invention relates generally to nucleic acid molecules in respect of which changes to the DNA or to the RNA or protein expression profiles are indicative of the onset, predisposition to the onset and/or progression of a neoplasm. More particularly, the present invention is directed to nucleic acid molecules in respect of which changes to the DNA or to the RNA or protein expression profiles are indicative of the onset and/or progression of a large intestine neoplasm, such as an adenoma or an adenocarcinoma. The DNA or the expression profiles of the present invention are useful in a range of applications including, but not limited to, those relating to the diagnosis and/or monitoring of colorectal neoplasms, such as colorectal adenocarcinomas. Accordingly, in a related aspect the present invention is directed to a method of screening a subject for the onset, predisposition to the onset and/or progression of a neoplasm by screening for modulation in the DNA or the RNA or protein expression profile of one or more nucleic acid molecule markers.

### BACKGROUND OF THE INVENTION

Bibliographic details of the publications referred to by author in this specification are collected alphabetically at the end of the description.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Adenomas are benign tumours, or neoplasms, of epithelial origin which are derived from glandular tissue or exhibit clearly defined glandular structures. Some adenomas show recognisable tissue elements, such as fibrous tissue (fibroadenomas) and epithelial structure, while others, such as bronchial adenomas, produce active compounds that might give rise to clinical syndromes.

Adenomas may progress to become an invasive neoplasm and are then termed adenocarcinomas. Accordingly, adenocarcinomas are defined as malignant epithelial tumours arising from glandular structures, which are constituent parts of many organs of the body. The term adenocarcinoma is also applied to tumours showing a glandular growth pattern. These tumours may be sub-classified according to the substances that they produce, for example mucus secreting and serous adenocarcinomas, or to the microscopic arrangement of their cells into patterns, for example papillary and follicular adenocarcinomas. These carcinomas may be solid or cystic (cystadenocarcinomas). Each organ may produce tumours showing a variety of histological types, for example the ovary may produce both mucinous and cystadenocarcinoma.

Adenomas in different organs behave differently. In general, the overall chance of carcinoma being present within an adenoma (i.e. a focus of cancer having developed within a benign lesion) is approximately 5%. However, this is related to size of an adenoma. For instance, in the large bowel (colon and rectum specifically) occurrence of a cancer within an adenoma is rare in adenomas of less than 1 centimetre. Such a development is estimated at 40 to 50% in adenomas which are greater than 4 centimetres and show certain histopathological change such as villous change, or high grade dysplasia. Adenomas with higher degrees of dysplasia have a higher incidence of carcinoma. In any given colorectal adenoma, the predictors of the presence of cancer now or the future occurrence of cancer in the organ include size (especially greater than 9mm) degree of change from tubular to villous morphology, presence of high grade dysplasia and the morphological change described as "serrated adenoma". In any given individual, the additional features of increasing age, familial occurrence of colorectal adenoma or cancer, male gender or multiplicity of adenomas, predict a future increased risk for cancer in the organ - so-called risk factors for cancer. Except for the presence of adenomas and its size, none of these is objectively defined and all those other than number and size are subject to observer error and to confusion as to precise definition of the feature in question. Because such factors can be difficult to assess and define, their value as predictors of current or future risk for cancer is imprecise.

Once a sporadic adenoma has developed, the chance of a new adenoma occurring is approximately 30% within 26 months.

Colorectal adenomas represent a class of adenomas which are exhibiting an increasing incidence, particularly in more affluent countries. The causes of adenoma, and of progression to adenocarcinoma, are still the subject of intensive research. To date it has been speculated that in addition to genetic predisposition, environmental factors (such as diet) play a role in the development of this condition. Most studies indicate that the relevant environmental factors relate to high dietary fat, low fibre, low vegetable intake, smoking, obesity, physical inactivity and high refined carbohydrates.

Colonic adenomas are localised areas of dysplastic epithelium which initially involve just one or several crypts and may not protrude from the surface, but with increased growth in size, usually resulting from an imbalance in proliferation and/or apoptosis, they may protrude. Adenomas can be classified in several ways. One is by their gross appearance and the major descriptors include degrees of protrusion: flat sessile (i.e. protruding but without a distinct stalk) or pedunculated (i.e. having a stalk). Other gross descriptors include actual size in the largest dimension and actual number in the colon/rectum. While small adenomas (less than say 5 or 10 millimetres) exhibit a smooth tan surface, pedunculated and especially larger adenomas tend to have a cobblestone or lobulated red-brown surface. Larger sessile adenomas may exhibit a more delicate villous surface. Another set of descriptors include the histopathological classification; the prime descriptors of clinical value include degree of dysplasia (low or high), whether or not a focus of invasive cancer is present, degree of change from tubular gland formation to villous gland formation (hence classification is tubular, villous or tubulovillous), presence of admixed hyperplastic change and of so-called "serrated" adenomas and its subgroups. Adenomas can be situated at any site in the colon and/or rectum although they tend to be more common in the rectum and distal colon. All of these descriptors, with the exception of number and size, are relatively subjective and subject to interobserver disagreement.

The various descriptive features of adenomas are of value not just to ascertain the neoplastic status of any given adenomas when detected, but also to predict a person's future risk of developing colorectal adenomas or cancer. Those features of an adenoma or number of adenomas in an individual that point to an increased future risk for cancer or recurrence of new adenomas include: size of the largest adenoma (especially 10mm or larger), degree of villous change (especially at least 25% such change and particularly 100% such change), high grade dysplasia, number (3 or more of any size or histological status) or presence of serrated adenoma features. None except size or number is objective and all are relatively subjective and subject to interobserver disagreement. These predictors of risk for future neoplasia (hence "risk") are vital in practice because they are used to determine the rate and need for and frequency of future colonoscopic surveillance. More accurate risk classification might thus reduce workload of colonoscopy, make it more cost-effective and reduce the risk of complications from unnecessary procedures.

Adenomas are generally asymptomatic, therefore rendering difficult their diagnosis and treatment at a stage prior to when they might develop invasive characteristics and so became cancer. It is technically impossible to predict the presence or absence of carcinoma based on the gross appearance of adenomas, although larger adenomas are more likely to show a region of malignant change than are smaller adenomas. Sessile adenomas exhibit a higher incidence of malignancy than pedunculated adenomas of the same size. Some adenomas result in blood loss which might be observed or detectable in the stools; while sometimes visible by eye, it is often, when it occurs, microscopic or "occult". Larger adenomas tend to bleed more than smaller adenomas. However, since blood in the stool, whether overt or occult, can also be indicative of non-adenomatous conditions, the accurate diagnosis of adenoma is rendered difficult without the application of highly invasive procedures such as colonoscopy combined with tissue acquisition by either removal (i.e. polypectomy) or biopsy and subsequent histopathological analysis.

Accordingly, there is an on-going need to elucidate the causes of adenoma and to develop more informative diagnostic protocols or aids to diagnosis that enable one to direct colonoscopy at people more likely to have adenomas. These adenomas may be high risk, advanced or neither of these, in particular protocols which will enable the rapid, routine and accurate diagnosis of adenoma. Furthermore, it can be difficult after colonoscopy to be certain that all adenomas have been removed, especially in a person who has had multiple adenomas. An accurate screening test may minimise the need to undertake an early second colonoscopy to ensure that the colon has been cleared of neoplasms. Accordingly, the identification of molecular markers for adenomas would provide means for understanding the cause of adenomas and cancer, improving diagnosis of adenomas including development of useful screening tests, elucidating the histological stage of an adenoma, characterising a patient's future risk for colorectal neoplasia on the basis of the molecular state of an adenoma and facilitating treatment of adenomas.

To date, research has focused on the identification of gene mutations which lead to the development of colorectal neoplasms. In work leading up to the present invention, however, it has been determined that changes in the DNA or the RNA or protein expression profiles of genes which are also expressed in healthy individuals are indicative of the development of neoplasms of the large intestine, such as adenomas and adenocarcinomas. It has been further determined that in relation to neoplasms of the large intestine, diagnosis can be made based on screening for one or more of a panel of these differentially expressed genes. In a related aspect, it has still further been determined that to the extent that neoplastic tissue has been identified either by the method of the invention or by some other method, the present invention provides still further means of characterising that tissue as an adenoma or a cancer. In yet another aspect, it has been determined that a proportion of these genes are characterised by gene expression which occurs in the context of non-neoplastic tissue but not in the context of neoplastic tissue, thereby facilitating the development of qualitative analyses which do not require a relative analysis to be performed against a non-neoplastic or normal control reference level. Accordingly, the inventors have identified a panel of genes which facilitate the diagnosis of adenocarcinoma and adenoma development and/or the monitoring of conditions characterised by the development of these types of neoplasms.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

The subject specification contains amino acid and nucleotide sequence information prepared using the programme PatentIn Version 3.4, presented herein after the bibliography. Each amino acid and nucleotide sequence is identified in the sequence listing by the numeric indicator <210> followed by the sequence identifier (eg. <210>1, <210>2, etc). The length, type of sequence (amino acid, DNA, etc.) and source organism for each sequence is indicated by information provided in the numeric indicator fields <211>m <212> and <213>, respectively. Amino acid and nucleotide sequences referred to in the specification are identified by the indicator SEQ ID NO: followed by the sequence identifier (eg. SEQ ID NO:I, SEQ ID NO: 2, etc). The sequence identifier referred to in the specification correlates to the information provided in numeric indicator field <400> in the sequence listing, which is followed by the sequence identifier (eg. <400>1, <400>2, etc). That is SEQ ID NO: 1 as detailed in the specification correlates to the sequence indicated as <400>1 in the sequence listing.

One aspect of the present invention is directed to a method of screening for the onset or predisposition to the onset of a large intestine neoplasm in an individual, said method comprising assessing the level of expression of one or more genes or transcripts selected from:

| **(i)** | the gene, genes or transcripts detected by Affymetrix probeset IDs: | | | |
|---|---|---|---|---|
| | 200600_at | 208399_s_at | 217546_at | 203296_s_at |
| | 200621_at | 208450_at | 217757_at | 203343_at |
| | 200795_at | 208581_x_at | 217762_s_at | 203474_at |
| | 200799_at | 208747_s_at | 217764_s_at | 203638_s_at |
| | 200845_s_at | 208763_s_at | 217767_at | 203963_at |
| | 200859_x_at | 208788_at | 217897_at | 204018_x_at |
| | 200897_s_at | 208789_at | 217967_s_at | 204034_at |
| | 200974_at | 208791_at | 218087_s_at | 204036_at |
| | 200986_at | 208792_s_at | 218162_at | 204130_at |
| | 201041_s_at | 208894_at | 218224_at | 204388_s_at |
| | 201058_s_at | 209047_at | 218312_s_at | 204389_at |
| | 201061_s_at | 209074_s_at | 218353_at | 204508_s_at |
| | 201069_at | 209101_at | 218418_s_at | 204532_x_at |
| | 201105_at | 209116_x_at | 218468_s_at | 204607_at |
| | 201137_s_at | 209138_x_at | 218469_at | 204673_at |
| | 201141_at | 209147_s_at | 218559_s_at | 204818_at |
| | 201150_s_at | 209156_s_at | 218756_s_at | 204895_x_at |
| | 201289_at | 209167_at | 219014_at | 204897_at |
| | 201300_s_at | 209170_s_at | 219087_at | 205112_at |
| | 201324_at | 209191_at | 219508_at | 205259_at |
| | 201348_at | 209209_s_at | 219607_s_at | 205403_at |
| | 201426_s_at | 209210_s_at | 219669_at | 205480_s_at |
| | 201427_s_at | 209283_at | 219799_s_at | 205554_s_at |
| | 201438_at | 209301_at | 220026_at | 205593_s_at |
| | 201496_x_at | 209312_x_at | 220037_s_at | 205892_s_at |
| | 201497_x_at | 209335_at | 220376_at | 205929_at |
| | 201539_s_at | 209357_at | 220834_at | 206000_at |
| | 201540_at | 209373_at | 221541_at | 206094_x_at |
| | 201616_s_at | 209436_at | 221667_s_at | 206262_at |
| | 201617_x_at | 209457_at | 221747_at | 206377_at |
| | 201645_at | 209496_at | 221748_s_at | 206385_s_at |
| | 201667_at | 209498_at | 222043_at | 206664_at |
| | 201739_at | 209612_s_at | 222162_s_at | 207126_x_at |
| | 201743_at | 209613_s_at | 222453_at | 207245_at |
| | 201744_s_at | 209621_s_at | 222513_s_at | 207390_s_at |
| | 201842_s_at | 209651_at | 222717_at | 207392_x_at |
| | 201852_x_at | 209656_s_at | 222722_at | 207432_at |
| | 201858_s_at | 209667 at | 223121_s_at | 207761_s_at |
| | 201859_at | 209668_x_at | 223122_s_at | 208596_s_at |
| | 201865_x_at | 209687_at | 223235_s_at | 208920_at |
| | 201893_x_at | 209735_at | 223343_at | 209114_at |
| | 201920_at | 209763_at | 223395_at | 209374_s_at |
| | 201957_at | 209868_s_at | 223551_at | 209458_x_at |
| | 202007_at | 209948_at | 223623_at | 209791_at |
| | 202037_s_at | 210084_x_at | 223952_x_at | 210107_at |
| | 202069_s_at | 227099_s_at | 224009_x_at | 210524_x_at |
| | 202133_at | 210133_at | 224352_s_at | 210735_s_at |
| | 202222_s_at | 210139_s_at | 224412_s_at | 211372_s_at |
| | 202242_at | 210298_x_at | 224480_s_at | 211538_s_at |
| | 202274_at | 210299_s_at | 224560_at | 211549_s_at |
| | 202283_at | 210302_s_at | 224663_s_at | 211637_x_at |
| | 202291_s_at | 210495_x_at | 224694_at | 211699_x_at |
| | 202388_at | 210517_s_at | 224823_at | 211745_x_at |
| | 202555_s_at | 210764_s_at | 224836_at | 212224_at |
| | 202620_s_at | 210809_s_at | 224840_at | 212592_at |
| | 202686_s_at | 210946_at | 224959_at | 212741_at |
| | 202746_at | 210982_s_at | 224963_at | 212814_at |
| | 202760_s_at | 211161_s_at | 224964_s_at | 213317_at |
| | 202766_s_at | 211548_s_at | 225207_at | 213451_x_at |
| | 202888_s_at | 211596_s_at | 225242_s_at | 213629_x_at |
| | 202920_at | 211643_x_at | 225269_s_at | 213921_at |
| | 202953_at | 211644_x_at | 225275_at | 213953_at |
| | 202957_at | 211645_x_at | 225353_s_at | 214164_x_at |
| | 202992_at | 211671_s_at | 225381_at | 214433_s_at |
| | 202994_s_at | 211696_x_at | 225442 at | 214598_at |
| | 202995_s_at | 211719_x_at | 225575_at | 214916_x_at |
| | 203000_at | 211798_x_at | 225602_at | 215125_s_at |
| | 203001_s_at | 211813_x_at | 225604_s_at | 215299_x_at |
| | 203066_at | 211848_s_at | 225626_at | 215867_x_at |
| | 203131_at | 211889_x_at | 225688_s_at | 216336_x_at |
| | 203305_at | 211896_s_at | 225710_at | 216491_x_at |
| | 203382_s_at | 211959_at | 225720_at | 216510_x_at |
| | 203477_at | 211964_at | 225721_at | 217022_s_at |
| | 203645_s_at | 211985_s_at | 225782_at | 217109_at |
| | 203680_at | 211990_at | 225894_at | 217110_s_at |
| | 203729_at | 211991_s_at | 225895_at | 217165_x_at |
| | 203748_x_at | 212077_at | 226001_at | 217232_x_at |
| | 203766_s_at | 212091_s_at | 226051_at | 217414_x_at |
| | 203881_s_at | 212097_at | 226084_at | 218541_s_at |
| | 203908_at | 212136_at | 226103_at | 218546_at |
| | 203913_s_at | 212158_at | 226303_at | 219059_s_at |
| | 203914_x_at | 212185_x_at | 226304_at | 219543_at |
| | 203951_at | 212192_at | 226333_at | 219796_s_at |
| | 203980_at | 212195_at | 226430_at | 219948_x_at |
| | 204069_at | 212230_at | 226492_at | 220075_s_at |
| | 204083_s_at | 212233_at | 226682_at | 220266_s_at |
| | 204122_at | 212265_at | 226694_at | 220468_at |
| | 204135_at | 212288_at | 226818_at | 220645_at |
| | 204326_x_at | 212386_at | 226834_at | 220812_s_at |
| | 204438_at | 212387_at | 226841_at | 221004_s_at |
| | 204457_s_at | 212397_at | 227006_at | 221305_s_at |
| | 204570_at | 212414_s_at | 227061_at | 221584_s_at |
| | 204688_at | 212419_at | 227235_at | 221841_s_at |
| | 204697_s_at | 212464_s_at | 227265_at | 221896_s_at |
| | 204719_at | 212667_at | 227404_s_at | 223484_at |
| | 204745_x_at | 212671_s_at | 227529_s_at | 223597_at |
| | 204834_at | 212713_at | 227561_at | 223754_at |
| | 204894_s_at | 212730_at | 227623_at | 224342_x_at |
| | 204931_at | 212764_at | 227662_at | 224989_at |
| | 204938_s_at | 212859_x_at | 227705_at | 224990_at |
| | 204939_s_at | 212956_at | 227727_at | 225458_at |
| | 204940_at | 213068_at | 227826_s_at | 225728_at |
| | 204955_at | 213071_at | 227827_at | 226147_s_at |
| | 205097_at | 213428_s_at | 228202_at | 226302_at |
| | 205200_at | 213509_x_at | 228504_at | 226594_at |
| | 205267_at | 213624_at | 228507_at | 226654_at |
| | 205382_s_at | 213746_s_at | 228640_at | 226811_at |
| | 205412_at | 213891_s_at | 228706_s_at | 227052_at |
| | 205433_at | 214027_x_at | 228707_at | 227522_at |
| | 205464_at | 214038_at | 228750_at | 227682_at |
| | 205547_s_at | 214091_s_at | 228766_at | 227725_at |
| | 205683_x_at | 214142_at | 228846_at | 227735_s_at |
| | 205935_at | 214414_x_at | 228854_at | 227736_at |
| | 205950_s_at | 214505_s_at | 228885_at | 228133_s_at |
| | 206134_at | 214677_x_at | 229530_at | 228195_at |
| | 206143_at | 214696_at | 229839_at | 228232_s_at |
| | 206149_at | 214752_x_at | 230087_at | 228241_at |
| | 206198_s_at | 214768_x_at | 230264_s_at | 228469_at |
| | 206199_at | 214777_at | 230788_at | 228961_at |
| | 206208_at | 215049_x_at | 230830_at | 229070_at |
| | 206209_s_at | 215076_s_at | 231120_x_at | 229254_at |
| | 206422_at | 215118_s_at | 231579_s_at | 229659_s_at |
| | 206461_x_at | 215176_x_at | 231773_at | 229831_at |
| | 206561_s_at | 215193_x_at | 234764_x_at | 230595_at |
| | 206576_s_at | 215382_x_at | 234987_at | 231925_at |
| | 206637_at | 215388_s_at | 236300_at | 231975_s_at |
| | 206641_at | 215657_at | 236313_at | 233565_s_at |
| | 206710_s_at | 216207_x_at | 242317_at | 235146_at |
| | 206784_at | 216401_x_at | 200884_at | 235766_x_at |
| | 207003_at | 216442_x_at | 201495_x_at | 235849_at |
| | 207080_s_at | 216474_x_at | 202266_at | 238143_at |
| | 207134_x_at | 216576_x_at | 202350_s_at | 238750_at |
| | 207266_x_at | 216834_at | 202731_at | 238751_at |
| | 207502_at | 216984_x_at | 202741_at | 239272_at |
| | 207961_x_at | 217148_x_at | 202742_s_at | 241994_at |
| | 207977_s_at | 217179_x_at | 202768_at | 242447_at |
| | 207980_s_at | 217235_x_at | 202838_at | 242601_at |
| | 208131_s_at | 217258_x_at | 203058_s_at | 243278_at; and/or |
| | 208370_s_at | 217378_x_at | 203060_s_at | |
| | 208383_s_at | 217480_x_at | 203240_at | |
| **(ii)** | CLCA4 | VIM | CA12 | AP1S2 |
| | ZG16 | SMPDL3A | FKBP5 | EMP3 |
| | CA2 | P2RY14 | HSPB8 | MMP28 |
| | CA1 | CHGA | TPSB2 | UGT2A3 |
| | MS4A12 | C15orf48 | FGL2 | RGS5 |
| | AQP8 | COL3A1 | C1QB | PTGIS |
| | SLC4A4 | CYR61 | ANGPTL1 | DUSP5 |
| | CEACAM7 | TRPM6 | MEP1A | MFAP4 |
| | TAGLN | OSTbeta | GUCY1A3 | UGT1A6 |
| | GUCA1B | IGLV1-44 | UGDH | PRKAR2B |
| | GCG | VSIG2 | DUSP1 | HHLA2 |
| | ADH1B | IGHM | C2orf40 | LOC652128 |
| | UGT2B17 | LRRC19 | PLN | C3 |
| | ADAMDEC1 | CD163 | UGT2B15 | ATP2B4 |
| | MT1M | CEACAM1 | PDLIM3 | HBA1 |
| | AKR1B10 | TIMP2 | TP53INP2 | TCF21 |
| | FN1 | ENTPD5 | ATP8B1 | PPID |
| | MGP | DDR2 | ANK3 | PPAP2B |
| | CXCL12 | CHRDL1 | CTGF | SPON1 |
| | PDK4 | SRGN | MUCDHL | PHLDB2 |
| | CA4 | PDE9A | SDPR | RARRES2 |
| | PYY | PMP22 | COL14A1 | ETHE1 |
| | IGHA1 | FLNA | DSCR1 | MMP2 |
| | TPM2 | STMN2 | CITED2 | SRI |
| | C6orf105 | MYL9 | MT1H | CNTN3 |
| | HPGD | SEMA6D | NEXN | RGS2 |
| | ADH1C | PADI2 | MUC2 | COL6A1 |
| | CLCA1 | SEPP1 | NID1 | FBN1 |
| | FABP1 | TGFB1I1 | HBB | MXD1 |
| | ENAM | SFRP2 | GCNT2 | PLCE1 |
| | CFD | UGT1A3 | C20orf118 | KCNMB1 |
| | GUCA2B | MS4A7 | SLC20A1 | CALM1 |
| | FBLN1 | ALDH1A1 | CD14 | HLA-DPB1 |
| | LOC63928 | SGK | KCTD12 | SMOC2 |
| | ABCA8 | CFL2 | RBMS1 | LOC285382 |
| | POSTN | C1S | PTRF | CLIC5 |
| | DCN | SELENBP1 | TSPAN1 | APOE |
| | ITLN1 | MT1E | UGT1A9 | SERPINF1 |
| | COL6A2 | ADAMTS1 | COX7A1 | PPP1R12B |
| | FCGBP | ITM2A | MUC12 | HSPB6 |
| | SLC26A2 | POU2AF1 | PDCD4 | FNBP1 |
| | PGM5 | FAM55D | CAV1 | C4orf34 |
| | DMN | C6orf204 | FAM46C | SORBS2 |
| | GPNMB | AKAP12 | LRIG1 | GPA33 |
| | IGFBP5 | TUBB6 | HLA-DPA1 | GALNAC4S- |
| | CLEC3B | LGALS2 | C1orf115 | 6ST |
| | LOC253012 | KIAA0828 | HBA2 | CFHR1 |
| | DPT | MGC14376 | EDIL3 | MGC13057 |
| | PCK1 | PPP1R14A | DES | C10orf56 |
| | CNN1 | MUC4 | MT2A | SULT1A1 |
| | HSD17B2 | PKIB | KCNMA1 | TTRAP |
| | PLAC8 | PIGR | GAS1 | CCL28 |
| | TMEM47 | ASPN | TBC1D9 | IDH3A |
| | OGN | A2M | C7 | EDG2 |
| | CALD1 | LOC25845 | P2RY1 | UGT1A8 |
| | ACTG2 | LGALS1 | NR3C1 | RAB27A |
| | MGC4172 | BCHE | STOM | ANTXR1 |
| | MAB21L2 | ST6GALNAC1 | CKB | EMP1 |
| | RPL24 | GJA1 | CLU | CSRP1 |
| | ABCG2 | SCNN1B | SLC26A3 | PLEKHC1 |
| | CCDC80 | FABP4 | SDC2 | LOC572558 |
| | UGT1A1 | F13A1 | SST | FOXP2 |
| | MRC1 | CD36 | HLA-DRA | HSPA2 |
| | HSD11B2 | SPARCL1 | TSC22D3 | ATP1A2 |
| | ANPEP | ZCWPW2 | IL6ST | TNXB |
| | MATN2 | TNC | C1QC | FUCA1 |
| | PRNP | MT1A | MT1X | MRGPRF |
| | ABI3BP | LOC652745 | AOC3 | HIGD1A |
| | HLA-C | MALL | PPAP2A | MFSD4 |
| | NDE1 | GNG2 | ZSCAN18 | AXL |
| | SRPX | DNASE1L3 | IVD | AQP1 |
| | WWTR1 | EGR1 | SFRP1 | MAP1B |
| | HMGCS2 | CMBL | COL4A2 | PALLD |
| | LOC646627 | GCNT3 | GPM6B | MPEG1 |
| | KRT20 | SERPING1 | EPB41L3 | KLHL5 |
| | KLF4 | MEIS1 | MAOA | TCEAL7 |
| | FHL1 | EDN3 | DMD | FILIP1L |
| | ARL14 | MSN | MSRB3 | IQGAP2 |
| | LUM | MT1G | PLOD2 | PRDX6 |
| | SORBS1 | TPSAB1 | C9orF19 | RAB31 |
| | METTL7A | GPX3 | MIER3 | LOC96610 |
| | FAM129A | CDKN2B | XDH | FGFR2 |
| | SCARA5 | FOSB | CLDN23 | PAPSS2 |
| | SI | HSPA1A | SGCE | XLKD1 |
| | ACTA2 | CYBRD1 | FOXF2 | SMTN |
| | CD177 | PTGER4 | AGR3 | C8orf4 |
| | C10orf99 | MAG1 | IGLJ3 | SDCBP2 |
| | COL15A1 | BEST2 | QKI | CCL11 |
| | NR3C2 | HLA-DQA1 | LOC399959 | ELOVL5 |
| | DHRS9 | PRIMA1 | ANKRD25 | FOXF1 |
| | LMOD1 | MT1F | CRISPLD2 | RELL1 |
| | EFEMP1 | MAFB | ANK2 | PNMA1 |
| | GREM1 | FAM107A | LOC283666 | LOC339562 |
| | IL1R2 | PRKACB | CRYAB | PALM2- |
| | LOC387763 | SELM | ACAT1 | AKAP2 |
| | TIMP3 | TYROBP | IGL@ | PAG1 |
| | MYLK | TNS1 | PBLD | HCLS1 |
| | CLDN8 | MYH11 | CCL8 | RGS1 |
| | RDX | ITM2C | LIFR | FXYD6 |
| | TSPAN7 | CES2 | HLA-DRB1 | OLFML3 |
| | TNFRSF17 | MS4A4A | UGP2 | COL6A3 |
| | SYNPO2 | PDGFRA | IGKV1D-13 | |

in a biological sample from said individual wherein a lower level of expression of the genes or transcripts of group (i) and/or group (ii) relative to control levels is indicative of a neoplastic large intestine cell or a cell predisposed to the onset of a neoplastic state.

Another aspect of the present invention provides a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs: 220026_at; and/or
**(ii)** CLCA4
in a biological sample from said individual wherein a lower level of expression of the gene or transcripts of group (i) and/or group (ii) relative to control levels is indicative of a neoplastic large intestine cell or a cell predisposed to the onset of a neoplastic state.

In yet another aspect there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs: 214142_at; and/or
**(ii)** ZG16
in a biological sample from said individual wherein a lower level of expression of the genes or transcripts of group (i) and/or group (ii) relative to control levels is indicative of a neoplastic large intestine cell or a cell predisposed to the onset of a neoplastic state.

In still another aspect there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs: 209301_at 205950_s_at; and/or
**(ii)** CA2 CA1
in a biological sample from said individual wherein a lower level of expression of the genes or transcripts of group (i) and/or group (ii) relative to control levels is indicative of a neoplastic large intestine cell or a cell predisposed to the onset of a neoplastic state.

In still yet another aspect there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs: 220834_at; and/or
**(ii)** MS4A12
in a biological sample from said individual wherein a lower level of expression of the genes or transcripts of group (i) and/or group (ii) relative to control levels is indicative of a neoplastic large intestine cell or a cell predisposed to the onset of a neoplastic state.

In yet still another aspect there is provided a method of screening for the onset or predisposition to the onset of a large intestine neoplasm in an individual, said method comprising assessing the level of expression of one or more genes or transcripts selected from:
**(i)** the gene, genes or transcripts detected by Affymetrix probeset IDs: 206784_at; and/or
**(ii)** AQP8
in a biological sample from said individual wherein a lower level of expression of the genes or transcripts of group (i) and/or group (ii) relative to control levels is indicative of a neoplastic large intestine cell or a cell predisposed to the onset of a neoplastic state.

In a further aspect there is provided a method of screening for the onset or predisposition to the onset of a large intestine neoplasm in an individual, said method comprising assessing the level of expression of one or more genes or transcripts selected from:
the gene, genes or transcripts detected by Affymetrix probeset IDs:
   **(i)** 203908_at, 206198_s_at, 205547_s_at, 207003_at, 206422_at, 209613_s_at, 207245_at; and/or
   **(ii)** SLC4A4, CEACAM7, TAGLN, GUCA1B, GCG, ADH1B, UGT2B 17,
in a biological sample from said individual wherein a lower level of expression of the genes or transcripts of group (i) and/or group (ii) relative to control levels is indicative of a neoplastic large intestine cell or a cell predisposed to the onset of a neoplastic state.

In another further aspect there is provided a method of screening for the onset or predisposition to the onset of a large intestine neoplasm in an individual, said method comprising assessing the level of expression of one or more genes or transcripts selected from:
the gene, genes or transcripts detected by Affymetrix probeset IDs:
   **(i)** 203908_at, 206198_s_at, 205547_s_at, 207003_at, 206422_at, 209613_s_at, 207245_at; and/or
   **(ii)** SLC4A4, CEACAM7, TAGLN, GUCA1B, GCG, ADH1B, UGT2B 17,
in a biological sample from said individual wherein a lower level of expression of the genes or transcripts of group (i) and/or group (ii) relative to control levels is indicative of a neoplastic large intestine cell or a cell predisposed to the onset of a neoplastic state.

In still another further aspect there is provided a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | | |
|---|---|---|---|---|
| | 225207_at | 211548_s_at | 205382_s_at | 204083_s_at |
| | 206208_at | 206262_at | 207502_at | 212592_at |
| | 207080_s_at | 210107_at | 202995_s_at | 204719_at |
| | 215118_s_at | 205892_s_at | 206149_at | 229070_at; and/or |
| **(ii)** | PDK4 | HPGD | CFD | TPM2 |
| | CA4 | ADH1C | GUCA2B | ENAM |
| | PYY | CLCA1 | FBLN1 | ABCA8 |
| | IGHA1 | FABP1 | LOC63928 | C6orf105 |

in a biological sample from said individual wherein a lower level of expression of the genes or transcripts of group (i) and/or group (ii) relative to control levels is indicative of a neoplastic large intestine cell or a cell predisposed to the onset of a neoplastic state.

In yet still yet another further aspect there is provided a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | | |
|---|---|---|---|---|
| | 210809_s_at | 201617_x_at | 202133_at | 217967_s_at |
| | 201893_x_at | 202274_at | 204607_at | 229839_at |
| | 223597_at | 218756_s_at | 238143_at | 206664_at |
| | 209156_s_at | 210302_s_at | 213953_at | 200974_at |
| | 203240_at | 228885_at | 220266_s_at | 219669_at |
| | 224963_at | 209735_at | 210299_s_at | 227736_at |
| | 226303_at | 228504_at | 220468_at | 203477_at |
| | 212730_at | 225242_s_at | 201744_s_at | 205259_at |
| | 201141_at | 215125_s_at | 218087_s_at | 203951_at |
| | 211959_at | 204438_at | 207761_s_at | 204818_at |
| | 205200_at | 204130_at | 223395_at | 219014_at |
| | 242601_at | 202888_s_at | 214768_x_at | 209656_s_at |
| | 213068_at | 202350_s_at | 228133_s_at | 222722_at; and/or |
| | 208383_s_at | 201300_s_at | 204955_at | |
| **(ii)** | POSTN | OGN | WWTR1 | FAM129A |
| | DCN | CALD1 | HMGCS2 | SCARA5 |
| | ITLN1 | ACTG2 | LOC646627 | SI |
| | COL6A2 | MGC4172 | KRT20 | ACTA2 |
| | FCGBP | MAB21L2 | KLF4 | CD177 |
| | SLC26A2 | RPL24 | FHL1 | C10orf99 |
| | PGM5 | ABCG2 | ARL14 | COL15A1 |
| | DMN | CCDC80 | LUM | NR3C2 |
| | GPNMB | UGT1A1 | SORBS1 | CNN1 |
| | IGFBP5 | MRC1 | METTL7A | HSD17B2 |
| | CLEC3B | HSD11B2 | PRNP | PLAC8 |
| | LOC253012 | ANPEP | ABI3BP | TMEM47 |
| | DPT | MATN2 | HLA-C | SRPX |
| | PCK1 | NDE1 | | |

in a biological sample from said individual wherein a lower level of expression of the genes or transcripts of group (i) and/or group (ii) relative to control levels is indicative of a neoplastic large intestine cell or a cell predisposed to the onset of a neoplastic state.

In a related aspect the present invention is directed to a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | | |
|---|---|---|---|---|
| | 200600_at | 203680_at | 211959_at | 215076_s_at |
| | 200665_s_at | 203729_at | 211964_at | 215193_x_at |
| | 200799_at | 203748_x_at | 211985_s_at | 219087_at |
| | 200845_s_at | 204069_at | 211990_at | 219607_s_at |
| | 200859_x_at | 204122_at | 211991_s_at | 221541_at |
| | 200897_s_at | 204135_at | 212077_at | 222043_at |
| | 200974_at | 204438_at | 212091_s_at | 222453_at |
| | 200986_at | 204457_s_at | 212136_at | 222513_s_at |
| | 201041_s_at | 204570_at | 215382_x_at | 223121_s_at |
| | 201061_s_at | 204688_at | 215388_s_at | 223122_s_at |
| | 201069_at | 205412_at | 216442_x_at | 223235_s_at |
| | 201105_at | 205683_x_at | 216474_x_at | 223343_at |
| | 201137_s_at | 205935_at | 216834_at | 224560_at |
| | 201141_at | 207134_x_at | 217480_x_at | 224694_at |
| | 201150_s_at | 207266_x_at | 217757_at | 224840_at |
| | 201289_at | 208131_s_at | 217762_s_at | 224964_s_at |
| | 201300_s_at | 208370_s_at | 217764_s_at | 225242_s_at |
| | 201426_s_at | 208747_s_at | 217767_at | 225269_s_at |
| | 201438_at | 208788_at | 217897_at | 225353_s_at |
| | 201616_s_at | 208789_at | 218162_at | 225381_at |
| | 201617_x_at | 208894_at | 218224_at | 225442_at |
| | 201645_at | 209047_at | 218312_s_at | 225602_at |
| | 201667_at | 209101_at | 218353_at | 225604_s_at |
| | 201743_at | 209138_x_at | 218418_s_at | 225626_at |
| | 201744_s_at | 209147_s_at | 218468_s_at | 225688_s_at |
| | 201842_s_at | 209156_s_at | 218469_at | 225710_at |
| | 201852_x_at | 209191_at | 218559_s_at | 226001_at |
| | 201858_s_at | 209209_s_at | 212158_at | 226051_at |
| | 201859_at | 209210_s_at | 212185_x_at | 226084_at |
| | 201865_x_at | 209312_x_at | 212195_at | 226103_at |
| | 201893_x_at | 209335_at | 212230_at | 226430_at |
| | 201920_at | 209436_at | 212233_at | 226682_at |
| | 202007_at | 209457_at | 212265_at | 226694_at |
| | 202069_s_at | 209496_at | 212386_at | 226818_at |
| | 202133_at | 209621_s_at | 212387_at | 226834_at |
| | 202283_at | 209651_at | 212397_at | 226841_at |
| | 202291_s_at | 209656_s_at | 212414_s_at | 227061_at |
| | 202403_s_at | 209868_s_at | 212419_at | 227099_s_at |
| | 202620_s_at | 210084_x_at | 212464_s_at | 227235_at |
| | 202686_s_at | 210133_at | 212667_at | 227404_s_at |
| | 202760_s_at | 210139_s_at | 212671_s_at | 227529_s_at |
| | 202766_s_at | 210495_x_at | 212713_at | 227561_at |
| | 202953_at | 210517_s_at | 212764_at | 227623_at |
| | 202957_at | 210764_s_at | 212956_at | 227705_at |
| | 202994_s_at | 210809_s_at | 213428_s_at | 227727_at |
| | 202995_s_at | 210982_s_at | 213509_x_at | 228507_at |
| | 203066_at | 211161_s_at | 213746_s_at | 228750_at |
| | 203131_at | 211596_s_at | 213891_s_at | 228846_at |
| | 203305_at | 211671_s_at | 214038_at | 229530_at |
| | 203382_s_at | 211719_x_at | 214677_x_at | 230264_s_at |
| | 203477_at | 211813_x_at | 214752_x_at | 231579_s_at |
| | 203645_s_at | 211896_s_at | 215049_x_at | 234987_at; and/or |
| **(ii)** | A2M | CYBRD1 | LOC283666 | PTGIS |
| | ACAT1 | CYR61 | LOC339562 | PTRF |
| | ACTA2 | DCN | LOC387763 | QKI |
| | AKAP12 | DDR2 | LOC399959 | RAB31 |
| | ANKRD25 | DSCR1 | LRIG1 | RARRES2 |
| | ANTXR1 | DUSP1 | LUM | RBMS1 |
| | AP1S2 | DUSP5 | MAFB | RDX |
| | APOE | EFEMP1 | MAP1B | RELL1 |
| | AQP1 | EGR1 | MEIS1 | RGS1 |
| | ASPN | ELOVL5 | MFAP4 | RGS5 |
| | ATP2B4 | EMP3 | MGP | SDC2 |
| | AXL | F13A1 | MMP2 | SELM |
| | C10orf56 | FBLN1 | MPEG1 | SEPT6 |
| | C1QB | FBN1 | MRC1 | SERPINF1 |
| | C1QC | FILIP1L | MRGPRF | SERPING1 |
| | C1S | FKBP5 | MS4A4A | SFRP2 |
| | C20orf118 | FLNA | MS4A7 | SGCE |
| | C3 | FN1 | MSN | SLC20A1 |
| | C9orf19 | FOXF1 | MT2A | SMOC2 |
| | CALD1 | FXYD6 | MXD1 | SORBS1 |
| | CALM1 | GALNAC4S-6ST | NEXN | SPARC |
| | CCDC80 | GAS1 | NID1 | SPON1 |
| | CCL11 | GJA1 | NR3C1 | SRGN |
| | CCL8 | GNG2 | OLFML3 | STOM |
| | CD14 | GPNMB | PAG1 | TBC1D9 |
| | CD163 | GREM1 | PALLD | TCEAL7 |
| | CES2 | GUCY1A3 | PALM2-AKAP2 | TGFB1I1 |
| | CFHR1 | HCLS1 | PDGFRA | TIMP2 |
| | CLU | HLA-DPA1 | PDLIM3 | TIMP3 |
| | COL14A1 | HLA-DPB1 | PHLDB2 | TMEM47 |
| | COL15A1 | HLA-DQA1 | PLEKHC1 | TNC |
| | COL1A2 | HLA-DRA | PLOD2 | TPSAB1 |
| | COL3A1 | HLA-DRB1 | PMP22 | TPSB2 |
| | COL4A2 | HSPA1A | PNMA1 | TUBB6 |
| | COL6A1 | IDH3A | POSTN | TYROBP |
| | COL6A2 | IGFBP5 | PPAP2A | VIM |
| | COL6A3 | IGL@ | PPAP2B | WWTR1 |
| | COX7A1 | IGLJ3 | PRDX6 | ZSCAN18 |
| | CRISPLD2 | IL6ST | PRKAR2B | |
| | CTGF | KLHL5 | PRNP | |
| | | LGALS1 | | |

in a biological sample from said individual wherein a lower level of expression of the genes or transcripts of group (i) and/or group (ii) relative to control levels is indicative of an adenoma cell or a cell predisposed to the onset of an adenoma state.

In another aspect of the present invention there is provided a method of screening for the onset or predisposition to the onset of a large intestine neoplasm in an individual, said method comprising screening the level of expression of one or more genes or transcripts selected from:

| **(i)** | the gene or genes detected by Affymetrix probeset IDs: | | | |
|---|---|---|---|---|
| | 200884_at | 206262_at | 215867_x_at | 227522_at |
| | 201495_x_at | 206377_at | 216336_x_at | 227682_at |
| | 202266_at | 206385_s_at | 216491_x_at | 227725_at |
| | 202350_s_at | 206664_at | 216510_x_at | 227735_s_at |
| | 202731_at | 207126_x_at | 217022_s_at | 227736_at |
| | 202741_at | 207245_at | 217109_at | 228133_s_at |
| | 202742_s_at | 207390_s_at | 217110_s_at | 228195_at |
| | 202768_at | 207392_x_at | 217165_x_at | 228232_s_at |
| | 202838_at | 207432_at | 217232_x_at | 228241_at |
| | 203058_s_at | 207761_s_at | 217414_x_at | 228469_at |
| | 203060_s_at | 208596_s_at | 218541_s_at | 228961_at |
| | 203240_at | 208920_at | 218546_at | 229070_at |
| | 203296_s_at | 209114_at | 219059_s_at | 229254_at |
| | 203343_at | 209374_s_at | 219543_at | 229659_s_at |
| | 203474_at | 209458_x_at | 219796_s_at | 229831_at |
| | 203638_s_at | 209791_at | 219948_x_at | 230595_at |
| | 203963_at | 210107_at | 220075_s_at | 231925_at |
| | 204018_x_at | 210524_x_at | 220266_s_at | 231975_s_at |
| | 204034_at | 210735_s_at | 220468_at | 233565_s_at |
| | 204036_at | 211372_s_at | 220645_at | 235146_at |
| | 204130_at | 211538_s_at | 220812_s_at | 235766_x_at |
| | 204388_s_at | 211549_s_at | 221004_s_at | 235849_at |
| | 204389_at | 211637_x_at | 221305_s_at | 238143_at |
| | 204508_s_at | 211699_x_at | 221584_s_at | 238750_at |
| | 204532_x_at | 211745_x_at | 221841_s_at | 238751_at |
| | 204607_at | 212224_at | 221896_s_at | 239272_at |
| | 204673_at | 212592_at | 223484_at | 241994_at |
| | 204818_at | 212741_at | 223597_at | 242447_at |
| | 204895_x_at | 212814_at | 223754_at | 242601_at |
| | 204897_at | 213317_at | 224342_x_at | 243278_at; and/or |
| | 205112_at | 213451_x_at | 224989_at | |
| | 205259_at | 213629_x_at | 224990_at | |
| | 205403_at | 213921_at | 225458_at | |
| | 205480_s_at | 213953_at | 225728_at | |
| | 205554_s_at | 214164_x_at | 226147_s_at | |
| | 205593_s_at | 214433_s_at | 226302_at | |
| | 205892_s_at | 214598_at | 226594_at | |
| | 205929_at | 214916_x_at | 226654_at | |
| | 206000_at | 215125_s_at | 226811_at | |
| | 206094_x_at | 215299_x_at | 227052_at | |
| | | | | |
| **(ii)** | ADH1C | FOSB | LOC96610 | SDCBP2 |
| | AGR3 | FOXF2 | MAOA | SELENBP1 |
| | ALDH1A1 | FOXP2 | MATN2 | SI |
| | ANK3 | FUCA1 | MEP1A | SMTN |
| | ARL14 | GPA33 | METTL7A | SORBS2 |
| | ATP1A2 | HBA1 | MFSD4 | SRI |
| | ATP8B1 | HBA2 | MGC13057 | SST |
| | BEST2 | HBB | MIER3 | ST6GALNAC1 |
| | C10orf99 | HHLA2 | MMP28 | SULT1A1 |
| | C15orf48 | HIGD1A | MT1A | TNXB |
| | C1orf115 | HMGCS2 | MT1F | TSPAN1 |
| | C4orf34 | HPGD | MT1M | TTRAP |
| | C6orf105 | HSD11B2 | MUC12 | UGDH |
| | C8orf4 | HSD17B2 | MUC2 | UGP2 |
| | CA12 | HSPA2 | MUC4 | UGT1A1 |
| | CCL28 | IGHA1 | MUCDHL | UGT1A3 |
| | CKB | IGHM | MYH11 | UGT1A6 |
| | CLCA1 | IL1R2 | NDE1 | UGT1A8 |
| | CLDN8 | IL8 | NR3C2 | UGT1A9 |
| | CLIC5 | IQGAP2 | P2RY1 | UGT2A3 |
| | CMBL | ITLN1 | PADI2 | UGT2B15 |
| | CNTN3 | ITM2C | PAPSS2 | UGT2B17 |
| | DNASE1L3 | KCNMA1 | PBLD | VSIG2 |
| | EDG2 | KIAA0828 | PDCD4 | XDH |
| | ENAM | KLF4 | PDE9A | XLKD1 |
| | ENTPD5 | KRT20 | PIGR | ZCWPW2 |
| | ETHE1 | LOC253012 | PLCE1 | |
| | FABP1 | LOC25845 | PPID | |
| | FAM46C | LOC285382 | PRKACB | |
| | FAM55D | LOC572558 | PTGER4 | |
| | FCGBP | LOC646627 | RAB27A | |
| | FGFR2 | LOC652128 | SCARA5 | |

in a biological sample from said individual wherein a lower level of expression of the genes or transcripts of group (i) and/or group (ii) relative to control levels is indicative of a cancer cell or a cell predisposed to the onset of a cancerous state.

In still another aspect there is provided a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | | |
|---|---|---|---|---|
| | 202920_at | 209613_s_at | 228504_at | 214598_at |
| | 203881_s_at | 220037_s_at | 228706_s_at | 219948_x_at |
| | 204719_at | 220376_at | 228766_at | 220812_s_at |
| | 204931_at | 222717_at | 228854_at | 221305_s_at |
| | 204940_at | 224412_s_at | 228885_at | 229831_at |
| | 205433_at | 225381_at | 230788_at | 231925_at |
| | 206637_at | 225575_at | 231120_x_at | 235146_at |
| | 207080_s_at | 227529_s_at | 231773_at | 238751_at |
| | 207980_s_at | 227623_at | 203296_s_at | 243278_at; and/or |
| | 209170_s_at | 227705_at | 206664_at | |
| | 209209_s_at | 227827_at | 211549_s_at | |
| **(ii)** | ADH1B | CD36 | P2RY1 | PLEKHC1 |
| | SORBS2 | BCHE | ANK2 | LRRC19 |
| | PYY | TCEAL7 | XLKD1 | LIFR |
| | ABCA8 | ANGPTL1 | LOC399959 | ATP1A2 |
| | RPL24 | DMD | AKAP12 | HPGD |
| | SI | GCNT2 | UGT2A3 | GPM6B |
| | CLDN8 | SDPR | HHLA2 | UGT1A8 |
| | P2RY14 | PKIB | SORBS2 | FOXP2 |
| | PLN | CITED2 | CLDN23 | |
| | TRPM6 | TCF21 | CNTN3 | |

in a biological sample from said individual wherein a level of expression of the genes or transcripts of group (i) and/or group (ii) which is not substantially above background levels is indicative of a neoplastic cell or a cell predisposed to the onset of a neoplastic state.

In a further aspect there is provided a method of screening for the onset or predisposition to the onset of a large intestine neoplasm in an individual, said method comprising screening the level of expression of one or more genes or transcripts selected from:
(i) the gene, genes or transcripts detected by Affymetrix probeset IDs: 209209_s_at, 225381_at, 227529_s_at, 227623_at, 227705_at; and/or
(ii) AKAP12, LOC399959, PLEKHC1, TCEAL7,
in a biological sample from said individual wherein a lower level of expression of the genes or transcripts of group (i) and/or group (ii) which is not substantially above background levels is indicative of an adenoma cell or a cell predisposed to the onset of an adenoma state.

In yet still another further aspect there is provided a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | | |
|---|---|---|---|---|
| | 203296_s_at | 214598_at | 221305_s_at | 235146_at |
| | 206664_at | 219948_x_at | 229831_at | 238751_at |
| | 211549_s_at | 220812_s_at | 231925_at | 243278_at; and/or |
| **(ii)** | ATP1A2 | FOXP2 | P2RY1 | UGT1A8 |
| | CLDN8 | HHLA2 | SI | UGT2A3 |
| | CNTN3 | HPGD | SORBS2 | |

in a biological sample from said individual wherein a lower level of expression of the genes or transcripts of group (i) and/or group (ii) which is not substantially above background levels is indicative of a cancer cell or a cell predisposed to the onset of a cancerous state.

In another further aspect there is provided a method of characterising a neoplastic cell or cellular population, which cell or cellular population is derived from the large intestine of an individual, said method comprising assessing the level of expression of one or more genes or transcripts selected from:

| **(i)** | the gene, genes or transcripts detected by Affymetrix probeset IDs: | | | |
|---|---|---|---|---|
| | 200600_at | 202766_s_at | 210495_x_at | 218469_at |
| | 200665_s_at | 202859_x_at | 210511_s_at | 218559_s_at |
| | 200832_s_at | 202878_s_at | 210764_s_at | 218638_s_at |
| | 200974_at | 202917_s_at | 210809_s_at | 219087_at |
| | 200986_at | 202998_s_at | 211161_s_at | 221011_s_at |
| | 201058_s_at | 203083_at | 211571_s_at | 221729_at |
| | 201069_at | 203325_s_at | 211719_x_at | 221730_at |
| | 201105_at | 203382_s_at | 211813_x_at | 221731_x_at |
| | 201141_at | 203477_at | 211896_s_at | 37892_at |
| | 201147_s_at | 203570_at | 211959_at | 223122_s_at |
| | 201150_s_at | 203645_s_at | 211964_at | 223235_s_at |
| | 201162_at | 203878_s_at | 211966_at | 224560_at |
| | 201163_s_at | 204006_s_at | 211980_at | 224694_at |
| | 201185_at | 204051_s_at | 211981_at | 224724_at |
| | 201261_x_at | 204122_at | 212077_at | 225664_at |
| | 201289_at | 204320_at | 212344_at | 225681_at |
| | 201426_s_at | 204475_at | 212353_at | 225710_at |
| | 201438_at | 204620_s_at | 212354_at | 225799_at |
| | 201616_s_at | 205479_s_at | 212464_s_at | 226237_at |
| | 201645_at | 205547_s_at | 212488_at | 226311_at |
| | 201667_at | 205828_at | 212489_at | 226694_at |
| | 201744_s_at | 207173_x_at | 212667_at | 226777_at |
| | 201792_at | 207191_s_at | 213125_at | 226930_at |
| | 201842_s_at | 208747_s_at | 213428_s_at | 227099_s_at |
| | 201852_x_at | 208782_at | 213524_s_at | 227140_at |
| | 201859_at | 208788_at | 213869_x_at | 227566_at |
| | 201893_x_at | 208850_s_at | 213905_x_at | 229218_at |
| | 202237_at | 208851_s_at | 214247_s_at | 229802_at |
| | 202238_s_at | 209101_at | 215049_x_at | 231579_s_at |
| | 202283_at | 209156_s_at | 215076_s_at | 231766_s_at |
| | 202291_s_at | 209218_at | 215646_s_at | 231879_at |
| | 202310_s_at | 209395_at | 216442_x_at | 232458_at |
| | 202311_s_at | 209396_s_at | 217430_x_at | 233555_s_at |
| | 202403_s_at | 209596_at | 217762_s_at | 234994_at; and/or |
| | 202404_s_at | 209875_s_at | 217763_s_at | |
| | 202450_s_at | 209955_s_at | 217764_s_at | |
| | 202620_s_at | 210095_s_at | 218468_s_at | |
| **(ii)** | COL1A2 | INHBA | FAP | IGFBP3 |
| | CTHRC1 | COL6A2 | VIM | SERPINF1 |
| | FN1 | ANTXR1 | TIMP2 | ISLR |
| | POSTN | GPNMB | SCD | HNT |
| | SPP1 | BGN | TIMP3 | COL5A1 |
| | MMP1 | TAGLN | AEBP1 | OLFML2B |
| | SPARC | COL4A1 | GJA1 | KIAA1913 |
| | LUM | RAB31 | NNMT | PALM2-AKAP2 |
| | GREM1 | LGALS1 | COL1A1 | SERPING1 |
| | IL8 | ELOVL5 | SULF2 | TYROBP |
| | IGFBP5 | MGP | COL6A1 | ACTA2 |
| | SFRP2 | MMP2 | SPON2 | COL3A1 |
| | SULF1 | LOXL2 | CTSK | PLOD2 |
| | ASPN | MYL9 | MXRA5 | MMP11 |
| | COL6A3 | DCN | C1S | CD163 |
| | COL8A1 | CALD1 | DKK3 | FCGR3B |
| | COL12A1 | FBN1 | SRGN | PLAU |
| | COL5A2 | MMP3 | LBH | MAFB |
| | CDH11 | IGFBP7 | CTGF | LOC541471 |
| | THBS2 | FSTL1 | TNC | LOC387763 |
| | COL15A1 | COL4A2 | G0S2 | CHI3L1 |
| | COL11A1 | VCAN | SQLE | THY1 |
| | S100A8 | SMOC2 | EFEMP1 | LOXL1 |
| | FNDC1 | HTRA1 | APOE | CD93 |
| | SFRP4 | CYR61 | MSN | |

in said cell or cellular population wherein a lower level of expression of the genes of group (i) and/or group (ii) relative to a gastrointestinal cancer control level is indicative of an adenoma cell or a cell predisposed to the onset of an adenoma state.

In still another aspect there is provided a method of characterising a neoplastic cell or cellular population, which cell or cellular population is derived from the large intestine of an individual, said method comprising assessing the level of expression of one or more genes or transcripts selected from:

| **(i)** | the gene, genes or transcripts detected by Affymetrix probeset IDs: | | |
|---|---|---|---|
| | 200884_at | 214234_s_at | 226248_s_at |
| | 203240_at | 214235_at | 226302_at |
| | 203963_at | 214433_s_at | 227676_at |
| | 204508_s_at | 215125_s_at | 227719_at |
| | 204607_at | 215867_x_at | 227725_at |
| | 204811_s_at | 217109_at | 228232_s_at |
| | 204895_x_at | 217110_s_at | 229070_at |
| | 204897_at | 218211_s_at | 231832_at |
| | 205259_at | 219543_at | 232176_at |
| | 205765_at | 219955_at | 232481_s_at |
| | 205927_s_at | 221841_s_at | 235976_at |
| | 208063_s_at | 221874_at | 236894_at |
| | 208937_s_at | 223969_s_at | 237521_x_at |
| | 210107_at | 223970_at | 242601_at; and/or |
| | 213106_at | | |
| **(ii)** | CLCA1 | CTSE | ATP8B1 |
| | FCGBP | C6orf105 | CACNA2D2 |
| | HMGCS2 | CKB | KLF4 |
| | RETNLB | ATP8A1 | CYP3A5P2 |
| | L1TD1 | MUC4 | CAPN9 |
| | SLITRK6 | UGT1A1 | NR3C2 |
| | VSIG2 | SELENBP1 | PBLD |
| | LOC253012 | PTGER4 | CA12 |
| | ST6GALNAC1 | MLPH | WDR51B |
| | ID1 | KIAA1324 | FAM3D |
| | CYP3A5 | | |

in a biological sample from said individual wherein a lower level of expression of the genes or transcripts of group (i) and/or group (ii) relative to a gastrointestinal adenoma control level is indicative of a cancer or a cell predisposed to the onset of a cancerous state.

In yet another aspect there is provided a method of characterising a neoplastic cell or cellular population, which cell or cellular population is derived from the large intestine of an individual, said method comprising assessing the level of expression of one or more genes or transcripts selected from:
(i) the gene or genes detected by Affymetrix probeset IDs: 202404_s_at, 212464_s_at, 210809_s_at, 225681_at;and/or
(ii) COL1A2, FN 1, POSTN, CTHRC1
in a biological sample from said individual wherein a lower level of expression of the genes or transcripts of group (i) and/or group (ii) relative to a gastrointestinal cancer control level is indicative of an adenoma cell or a cell predisposed to the onset of an adenoma state.

In yet still another aspect, there is provided a method of characterising a neoplastic cell or cellular population, which cell or cellular population is derived from the large intestine of an individual, said method comprising assessing the level of expression of one or more genes selected from:
(i) the gene, genes or transcripts detected by Affymetrix probeset IDs: 209875_s_at 227140_at 204475_at; and/or
(ii) SPP1 MMP1
in a biological sample from said individual wherein a lower level of expression of the genes or transcripts of group (i) and/or group (ii) relative to a gastrointestinal cancer control level is indicative of an adenoma cell or a cell predisposed to the onset of an adenoma state.

In still yet another aspect the present invention is directed to a method of characterising a cell or cellular population, which cell or cellular population is derived from the large intestine of an individual, said method comprising assessing the level of expression of one or more genes or transcripts selected from:

| **(i)** | the gene, genes or transcripts detected by Affymetrix probeset IDs: | | | |
|---|---|---|---|---|
| | 200665_s_at | 219087_at | 37892_at | 201141_at |
| | 201744_s_at | 226237_at | 202917_s_at | 213905_x_at |
| | 218468_s_at | 225664_at | 226930_at | 205547_s_at |
| | 202859_x_at | 221730_at | 204051_s_at | 201438_at; and/or |
| | 211959_at | 207173_x_at | 210511_s_at | |
| | 223122_s_at | 203083_at | 209156_s_at | |
| | 212353_at | 203477_at | 224694_at | |
| **(ii)** | SPARC | ASPN | COL15A1 | ANTXR1 |
| | LUM | COL6A3 | COL11A1 | GPNMB |
| | GREM1 | COL8A1 | S100A8 | BGN |
| | IL8 | COL12A1 | FNDC1 | TAGLN |
| | IGFBP5 | COL5A2 | SFRP4 | |
| | SFRP2 | CDH11 | INHBA | |
| | SULF1 | THBS2 | COL6A2 | |

in a biological sample from said individual wherein a lower level of expression of the genes or transcripts of group (i) and/or (ii) relative to a gastrointestinal cancer control level is indicative of an adenoma or a cell predisposed to the onset of an adenoma state.

In yet another aspect the present invention is directed to a method of characterising a cell or cellular population, which cell or cellular population is derived from the large intestine of an individual, said method comprising assessing the level of expression of one or more genes or transcripts selected from:
(i) the gene, genes or transcripts detected by Affymetrix probeset IDs: 210107_at; and/or
(ii) CLCA1
in a biological sample from said individual wherein a lower level of expression of the genes or transcripts of group (i) and/or (ii) relative to a gastrointestinal adenoma control level is indicative of a cancer or a cell predisposed to the onset of a cancerous state.

In still yet another aspect the present invention is directed to a method of characterising a cell or cellular population, which cell or cellular population is derived from the large intestine of an individual, said method comprising assessing the level of expression of one or more genes or transcripts selected from:

| **(i)** | the gene, genes or transcripts detected by Affymetrix probeset IDs: | | |
|---|---|---|---|
| | 203240_at | 219955_at | 242601_at |
| | 204607_at | 232481_s_at | 227725_at |
| | 223969_s_at | 228232_s_at; and/or | |
| **(ii)** | FCGBP | L1TD1 | LOC253012 |
| | HMGCS2 | SLITRK6 | ST6GALNAC1 |
| | RETNLB | VSIG2 | |

in a biological sample from said individual wherein a lower level of expression of the genes or transcripts of group (i) and/or (ii) relative to a gastrointestinal adenoma control level is indicative of a cancer or a cell predisposed to the onset of a cancerous state.

A further aspect of the present invention provides a method of characterising a neoplastic cell or cellular population, which cell or cellular population is derived from the large intestine of an individual, said method comprising assessing the level of expression of one or more genes or transcripts selected from:

| **(i)** | the gene or genes detected by Affymetrix probeset IDs: | | |
|---|---|---|---|
| | 235976_at | 236894_at | 237521; and/or |
| **(ii)** | SLITRK6 | L1TD1 | |

in a biological sample from said individual wherein expression of the genes or transcripts of group (i) and/or (ii) at a level which is not substantially greater than background neoplastic tissue levels is indicative of a cancer or a cell predisposed to the onset of a cancerous state.

A related aspect of the present invention provides a molecular array, which array comprises a plurality of:
(i) nucleic acid molecules comprising a nucleotide sequence corresponding to any one or more of the neoplastic marker genes hereinbefore described or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (iv) is indicative of the neoplastic state of a cell or cellular subpopulation derived from the large intestine.

### BRIEF DESCRIPTION OF THE DRAWINGS

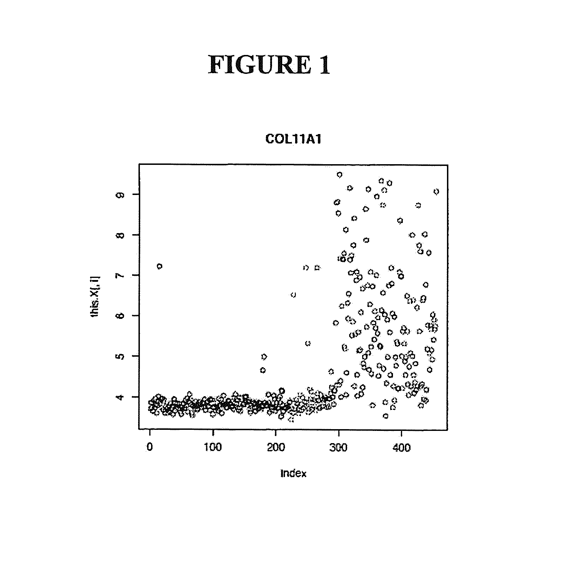

**Figure 1** is a graphical representation of alcohol dehydrogenase IB (class I), beta polypeptide.

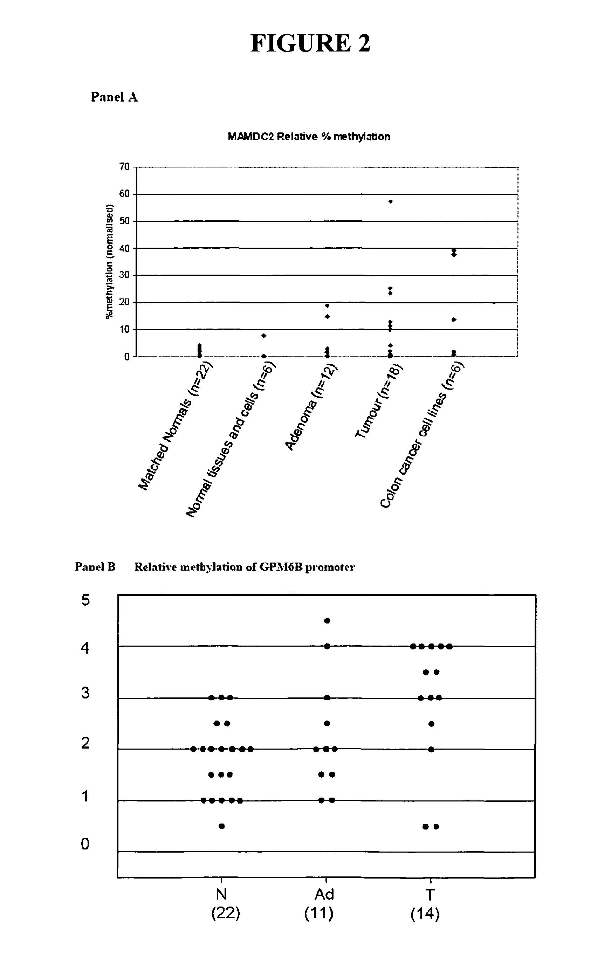

**Figure 2** is a graphical representation of the methylation of MAMDC2 and GPM6B in normal and neoplastic tissues and cell lines. Panel A shows the methylation level of the MAMDC2 gene as assessed by methylation specific PCR, using amplification of the CAGE gene to normalise for input DNA levels. Each point represents an individual tissue sample or cell line. Samples included DNAs from 18 colorectal cancer tissues, 12 colorectal adenomas, 22 matched normal colorectal tissues, 6 other normal tissues and a cell line and 6 colon cancer cell lines. Panel B shows the relative level of methylation of the GPM6B gene assessed by a COBRA assay. Levels of methylation were scored between 0 (no restriction enzyme digestion) and 5 (complete restriction enzyme digestion). Each point represents a single tissue sample. Samples included 14 colorectal cancer tissues, 11 colorectal adenomas and 22 matched normal tissues.

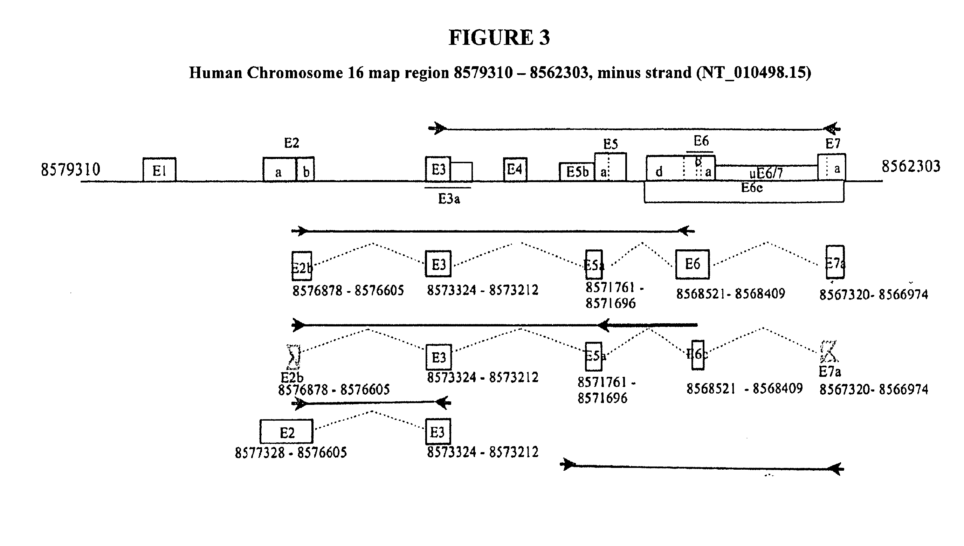

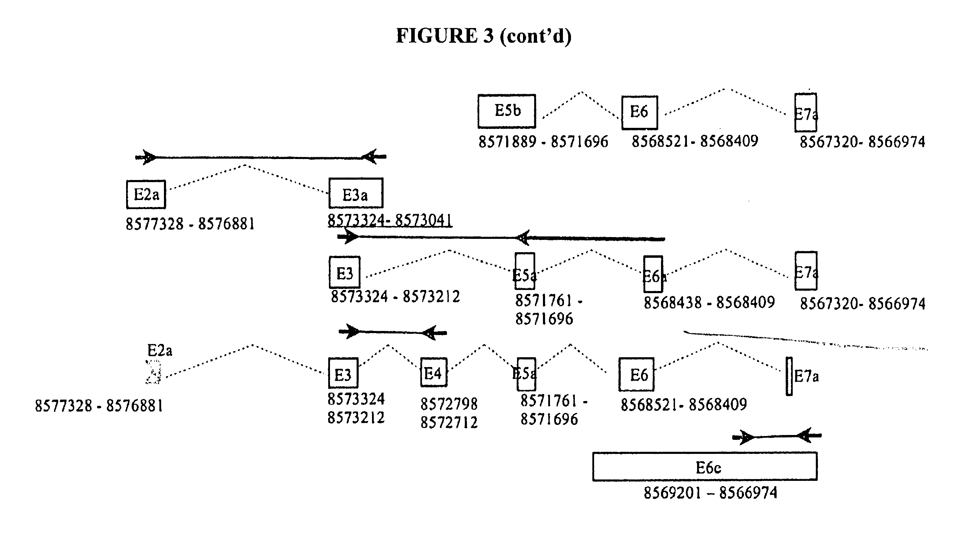

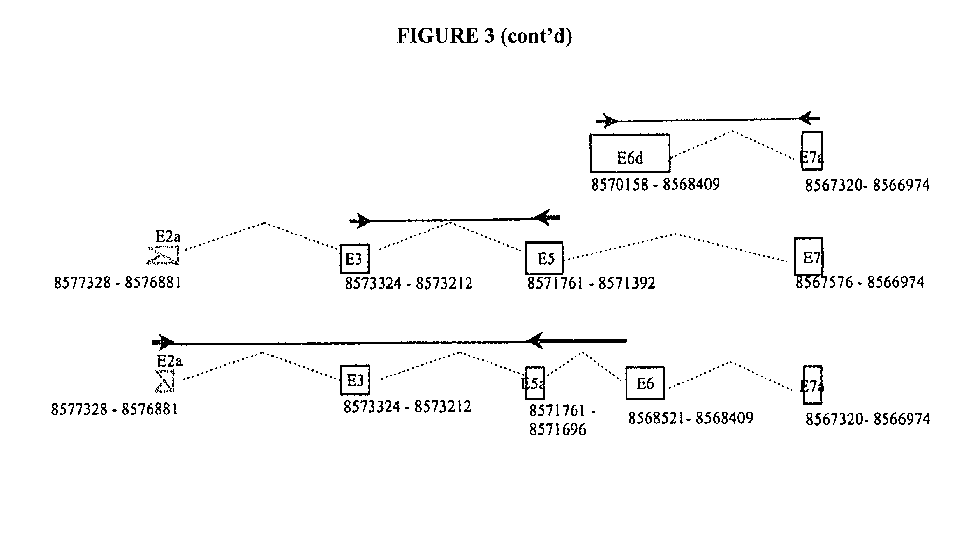

**Figure 3** is a schematic representation of predicted RNA variants derived from hCG_1815491. cDNA clones derived from map region 8579310 to 8562303 on human chromosome 16 were used to locate exon sequences. Arrows: Oligo nucleotide primer sets were designed to allow measurement of individual RNA variants by PCR. Primers covering splice junctions are shown as spanning intron sequences which is not included in the actual oligonucleotide primer sequence.

### DETAILED DESCRIPTION OF THE INVENTION

The present invention is predicated, in part, on the elucidation of gene expression profiles which characterise large intestine cellular populations in terms of their neoplastic state and, more particularly, whether they are malignant or pre-malignant. This finding has now facilitated the development of routine means of screening for the onset or predisposition to the onset of a large intestine neoplasm or characterising cellular populations derived from the large intestine based on screening for downregulation of the expression of these molecules, relative to control expression patterns and levels. To this end, in addition to assessing expression levels of the subject genes relative to normal or non-neoplastic levels, it has been determined that a proportion of these genes are not expressed in the diseased state, thereby facilitating the development of a simple qualitative test based on requiring assessment only relative to test background levels.

In accordance with the present invention, it has been determined that the genes detailed above are modulated, in terms of differential changes to their levels of expression, depending on whether the cell expressing that gene is neoplastic or not. It should be understood that reference to a gene "expression product" or "expression of a gene" is a reference to either a transcription product (such as primary RNA or mRNA) or a translation product such as protein. In this regard, one can assess changes to the level of expression of a gene either by screening for changes to the level of expression product which is produced (i.e. RNA or protein), changes to the chromatin proteins with which the gene is associated, for example the presence of histone H3 methylated on lysine at amino acid position number 9 or 27 (repressive modifications) or changes to the DNA itself which acts to downregulate expression, such as changes to the methylation of the DNA. These genes and their gene expression products, whether they be RNA transcripts, changes to the DNA which act to downregulate expression or encoded proteins, are collectively referred to as "neoplastic markers".

Accordingly, one aspect of the present invention is directed to a method of screening for the onset or predisposition to the onset of a large intestine neoplasm in an individual, said method comprising assessing the level of expression of one or more genes or transcripts selected from:

| **(i)** | the gene, genes or transcripts detected by Affymetrix probeset IDs: | | | |
|---|---|---|---|---|
| | 200600_at | 208399_s_at | 217546_at | 203296_s_at |
| | 200621_at | 208450_at | 217757_at | 203343_at |
| | 200795_at | 208581_x_at | 217762_s_at | 203474_at |
| | 200799_at | 208747_s_at | 217764_s_at | 203638_s_at |
| | 200845_s_at | 208763_s_at | 217767_at | 203963_at |
| | 200859_x_at | 208788_at | 217897_at | 204018_x_at |
| | 200897_s_at | 208789_at | 217967_s_at | 204034_at |
| | 200974_at | 208791_at | 218087_s_at | 204036_at |
| | 200986_at | 208792_s_at | 218162_at | 204130_at |
| | 201041_s_at | 208894_at | 218224_at | 204388_s_at |
| | 201058_s_at | 209047_at | 218312_s_at | 204389_at |
| | 201061_s_at | 209074_s_at | 218353_at | 204508_s_at |
| | 201069 at | 209101_at | 218418_s_at | 204532_x_at |
| | 201105_at | 209116_x_at | 218468_s_at | 204607_at |
| | 201137_s_at | 209138_x_at | 218469_at | 204673_at |
| | 201141 at | 209147_s_at | 218559_s_at | 204818_at |
| | 201150_s_at | 209156_s_at | 218756_s_at | 204895_x_at |
| | 201289_at | 209167_at | 219014_at | 204897_at |
| | 201300_s_at | 209170_s_at | 219087_at | 205112_at |
| | 201324_at | 209191_at | 219508_at | 205259_at |
| | 201348_at | 209209_s_at | 219607_s_at | 205403_at |
| | 201426_s_at | 209210_s_at | 219669_at | 205480_s_at |
| | 201427_s_at | 209283_at | 219799_s_at | 205554_s_at |
| | 201438_at | 209301_at | 220026_at | 205593_s_at |
| | 201496_x_at | 209312_x_at | 220037_s_at | 205892_s_at |
| | 201497_x_at | 209335_at | 220376_at | 205929_at |
| | 201539_s_at | 209357_at | 220834_at | 206000_at |
| | 201540_at | 209373_at | 221541_at | 206094_x_at |
| | 201616_s_at | 209436_at | 221667_s_at | 206262_at |
| | 201617_x_at | 209457_at | 221747_at | 206377_at |
| | 201645_at | 209496_at | 221748_s_at | 206385_s_at |
| | 201667_at | 209498_at | 222043_at | 206664_at |
| | 201739_at | 209612_s_at | 222162_s_at | 207126_x_at |
| | 201743 at | 209613_s_at | 222453_at | 207245_at |
| | 201744_s_at | 209621_s_at | 222513_s_at | 207390_s_at |
| | 201842_s_at | 209651_at | 222717_at | 207392_x_at |
| | 201852_x_at | 209656_s_at | 222722_at | 207432_at |
| | 201858_s_at | 209667_at | 223121_s_at | 207761_s_at |
| | 201859_at | 209668_x_at | 223122_s_at | 208596_s_at |
| | 201865_x_at | 209687_at | 223235_s_at | 208920_at |
| | 201893_x_at | 209735_at | 223343_at | 209114_at |
| | 201920_at | 209763_at | 223395_at | 209374_s_at |
| | 201957_at | 209868_s_at | 223551_at | 209458_x_at |
| | 202007_at | 209948_at | 223623_at | 209791_at |
| | 202037_s_at | 210084_x_at | 223952_x_at | 210107_at |
| | 202069_s_at | 227099_s_at | 224009_x_at | 210524_x_at |
| | 202133_at | 210133_at | 224352_s_at | 210735_s_at |
| | 202222_s_at | 210139_s_at | 224412_s_at | 211372_s_at |
| | 202242_at | 210298_x_at | 224480_s_at | 211538_s_at |
| | 202274_at | 210299_s_at | 224560_at | 211549_s_at |
| | 202283_at | 210302_s_at | 224663_s_at | 211637_x_at |
| | 202291_s_at | 210495_x_at | 224694_at | 211699_x_at |
| | 202388_at | 210517_s_at | 224823_at | 211745_x_at |
| | 202555_s_at | 210764_s_at | 224836_at | 212224_at |
| | 202620_s_at | 210809_s_at | 224840_at | 212592_at |
| | 202686_s_at | 210946_at | 224959_at | 212741_at |
| | 202746_at | 210982_s_at | 224963_at | 212814_at |
| | 202760_s_at | 211161_s_at | 224964_s_at | 213317_at |
| | 202766_s_at | 211548_s_at | 225207_at | 213451_x_at |
| | 202888_s_at | 211596_s_at | 225242_s_at | 213629_x_at |
| | 202920_at | 211643_x_at | 225269_s_at | 213921_at |
| | 202953_at | 211644_x_at | 225275_at | 213953_at |
| | 202957_at | 211645_x_at | 225353_s_at | 214164_x_at |
| | 202992_at | 211671_s_at | 225381_at | 214433_s_at |
| | 202994_s_at | 211696_x_at | 225442_at | 214598_at |
| | 202995_s_at | 211719_x_at | 225575_at | 214916_x_at |
| | 203000_at | 211798_x_at | 225602_at | 215125_s_at |
| | 203001_s_at | 211813_x_at | 225604_s_at | 215299_x_at |
| | 203066_at | 211848_s_at | 225626_at | 215867_x_at |
| | 203131_at | 211889_x_at | 225688_s_at | 216336_x_at |
| | 203305_at | 211896_s_at | 225710_at | 216491_x_at |
| | 203382_s_at | 211959_at | 225720_at | 216510_x_at |
| | 203477_at | 211964_at | 225721_at | 217022_s_at |
| | 203645_s_at | 211985_s_at | 225782_at | 217109_at |
| | 203680_at | 211990_at | 225894_at | 217110_s_at |
| | 203729_at | 211991_s_at | 225895_at | 217165_x_at |
| | 203748_x_at | 212077_at | 226001_at | 217232_x_at |
| | 203766_s_at | 212091_s_at | 226051_at | 217414_x_at |
| | 203881_s_at | 212097_at | 226084_at | 218541_s_at |
| | 203908_at | 212136_at | 226103_at | 218546_at |
| | 203913_s_at | 212158_at | 226303_at | 219059_s_at |
| | 203914_x_at | 212185_x_at | 226304_at | 219543_at |
| | 203951_at | 212192_at | 226333_at | 219796_s_at |
| | 203980_at | 212195_at | 226430_at | 219948_x_at |
| | 204069_at | 212230_at | 226492_at | 220075_s_at |
| | 204083_s_at | 212233_at | 226682_at | 220266_s_at |
| | 204122_at | 212265_at | 226694_at | 220468_at |
| | 204135_at | 212288_at | 226818_at | 220645_at |
| | 204326_x_at | 212386_at | 226834_at | 220812_s_at |
| | 204438_at | 212387_at | 226841_at | 221004_s_at |
| | 204457_s_at | 212397_at | 227006_at | 221305_s_at |
| | 204570_at | 212414_s_at | 227061_at | 221584_s_at |
| | 204688_at | 212419_at | 227235_at | 221841_s_at |
| | 204697_s_at | 212464_s_at | 227265_at | 221896_s_at |
| | 204719_at | 212667_at | 227404_s_at | 223484_at |
| | 204745_x_at | 212671_s_at | 227529_s_at | 223597_at |
| | 204834_at | 212713_at | 227561_at | 223754_at |
| | 204894_s_at | 212730_at | 227623_at | 224342_x_at |
| | 204931_at | 212764_at | 227662_at | 224989_at |
| | 204938_s_at | 212859_x_at | 227705_at | 224990_at |
| | 204939_s_at | 212956_at | 227727_at | 225458_at |
| | 204940_at | 213068_at | 227826_s_at | 225728_at |
| | 204955_at | 213071_at | 227827_at | 226147_s_at |
| | 205097_at | 213428_s_at | 228202_at | 226302_at |
| | 205200_at | 213509_x_at | 228504_at | 226594_at |
| | 205267_at | 213624_at | 228507_at | 226654_at |
| | 205382_s_at | 213746_s_at | 228640_at | 226811_at |
| | 205412_at | 213891_s_at | 228706_s_at | 227052_at |
| | 205433_at | 214027_x_at | 228707_at | 227522_at |
| | 205464_at | 214038_at | 228750_at | 227682_at |
| | 205547_s_at | 214091_s_at | 228766_at | 227725_at |
| | 205683_x_at | 214142_at | 228846_at | 227735_s_at |
| | 205935_at | 214414_x_at | 228854_at | 227736_at |
| | 205950_s_at | 2t4505_s_at | 228885_at | 228133_s_at |
| | 206134_at | 214677_x_at | 229530_at | 228195_at |
| | 206143_at | 214696_at | 229839_at | 228232_s_at |
| | 206149_at | 214752_x_at | 230087_at | 228241_at |
| | 206198_s_at | 214768_x_at | 230264_s_at | 228469_at |
| | 206199_at | 214777_at | 230788_at | 228961_at |
| | 206208_at | 215049_x_at | 230830_at | 229070_at |
| | 206209_s_at | 215076_s_at | 231120_x_at | 229254_at |
| | 206422_at | 215118_s_at | 231579_s_at | 229659_s_at |
| | 206461_x_at | 215176_x_at | 231773_at | 229831_at |
| | 206561_s_at | 215193_x_at | 234764_x_at | 230595_at |
| | 206576_s_at | 215382_x_at | 234987_at | 231925_at |
| | 206637_at | 215388_s_at | 236300_at | 231975_s_at |
| | 206641_at | 215657_at | 236313_at | 233565_s_at |
| | 206710_s_at | 216207_x_at | 242317_at | 235146_at |
| | 206784_at | 216401_x_at | 200884_at | 235766_x_at |
| | 207003_at | 216442_x_at | 201495_x_at | 235849_at |
| | 207080_s_at | 216474_x_at | 202266_at | 238143_at |
| | 207134_x_at | 216576_x_at | 202350_s_at | 238750_at |
| | 207266_x_at | 216834_at | 202731_at | 238751_at |
| | 207502_at | 216984_x_at | 202741_at | 239272_at |
| | 207961_x_at | 217148_x_at | 202742_s_at | 241994_at |
| | 207977_s_at | 217179_x_at | 202768_at | 242447_at |
| | 207980_s_at | 217235_x_at | 202838_at | 242601_at |
| | 208131_s_at | 217258_x_at | 203058_s_at | 243278_at; and/or |
| | 208370_s_at | 217378_x_at | 203060_s_at | |
| | 208383_s_at | 217480_x_at | 203240_at | |
| **(ii)** | CLCA4 | VIM | CA12 | AP1S2 |
| | ZG16 | SMPDL3A | FKBP5 | EMP3 |
| | CA2 | P2RY14 | HSPB8 | MMP28 |
| | CA1 | CHGA | TPSB2 | UGT2A3 |
| | MS4A12 | C15orf48 | FGL2 | RGS5 |
| | AQP8 | COL3A1 | C1QB | PTGIS |
| | SLC4A4 | CYR61 | ANGPTL1 | DUSP5 |
| | CEACAM7 | TRPM6 | MEP1A | MFAP4 |
| | TAGLN | OSTbeta | GUCY1A3 | UGT1A6 |
| | GUCA1B | IGLV1-44 | UGDH | PRKAR2B |
| | GCG | VSIG2 | DUSP1 | HHLA2 |
| | ADH1B | IGHM | C2orf40 | LOC652128 |
| | UGT2B17 | LRRC19 | PLN | C3 |
| | ADAMDEC1 | CD163 | UGT2B15 | ATP2B4 |
| | MT1M | CEACAM1 | PDLIM3 | HBA1 |
| | AKR1B10 | TIMP2 | TP53INP2 | TCF21 |
| | FN1 | ENTPD5 | ATP8B1 | PPID |
| | MGP | DDR2 | ANK3 | PPAP2B |
| | CXCL12 | CHRDL1 | CTGF | SPON1 |
| | PDK4 | SRGN | MUCDHL | PHLDB2 |
| | CA4 | PDE9A | SDPR | RARRES2 |
| | PYY | PMP22 | COL14A1 | ETHE1 |
| | IGHA1 | FLNA | DSCR1 | MMP2 |
| | TPM2 | STMN2 | CITED2 | SRI |
| | C6orf105 | MYL9 | MT1H | CNTN3 |
| | HPGD | SEMA6D | NEXN | RGS2 |
| | ADH1C | PADI2 | MUC2 | COL6A1 |
| | CLCA1 | SEPP1 | NID1 | FBN1 |
| | FABP1 | TGFB1I1 | HBB | MXD1 |
| | ENAM | SFRP2 | GCNT2 | PLCE1 |
| | CFD | UGT1A3 | C20orf118 | KCNMB1 |
| | GUCA2B | MS4A7 | SLC20A1 | CALM1 |
| | FBLN1 | ALDH1A1 | CD14 | HLA-DPB1 |
| | LOC63928 | SGK | KCTD12 | SMOC2 |
| | ABCA8 | CFL2 | RBMS1 | LOC285382 |
| | POSTN | C1S | PTRF | CLIC5 |
| | DCN | SELENBP1 | TSPAN1 | APOE |
| | ITLN1 | MT1E | UGT1A9 | SERPINF1 |
| | COL6A2 | ADAMTS1 | COX7A1 | PPP1R12B |
| | FCGBP | ITM2A | MUC12 | HSPB6 |
| | SLC26A2 | POU2AF1 | PDCD4 | FNBP1 |
| | PGM5 | FAM55D | CAV1 | C4orf34 |
| | DMN | C6orf204 | FAM46C | SORBS2 |
| | GPNMB | AKAP12 | LRIG1 | GPA33 |
| | IGFBP5 | TUBB6 | HLA-DPA1 | GALNAC4S- |
| | CLEC3B | LGALS2 | C1orf115 | 6ST |
| | LOC253012 | KIAA0828 | HBA2 | CFHR1 |
| | DPT | MGC14376 | EDIL3 | MGC13057 |
| | PCK1 | PPP1R14A | DES | C10orf56 |
| | CNN1 | MUC4 | MT2A | SULT1A1 |
| | HSD17B2 | PKIB | KCNMA1 | TTRAP |
| | PLAC8 | PIGR | GAS 1 | CCL28 |
| | TMEM47 | ASPN | TBC1D9 | IDH3A |
| | OGN | A2M | C7 | EDG2 |
| | CALD1 | LOC25845 | P2RY1 | UGT1A8 |
| | ACTG2 | LGALS1 | NR3C1 | RAB27A |
| | MGC4172 | BCHE | STOM | ANTXR1 |
| | MAB21L2 | ST6GALNAC1 | CKB | EMP1 |
| | RPL24 | GJA1 | CLU | CSRP1 |
| | ABCG2 | SCNN1B | SLC26A3 | PLEKHC1 |
| | CCDC80 | FABP4 | SDC2 | LOC572558 |
| | UGT1A1 | F13A1 | SST | FOXP2 |
| | MRC1 | CD36 | HLA-DRA | HSPA2 |
| | HSD11B2 | SPARCL1 | TSC22D3 | ATP1A2 |
| | ANPEP | ZCWPW2 | IL6ST | TNXB |
| | MATN2 | TNC | C1QC | FUCA1 |
| | PRNP | MT1A | MT1X | MRGPRF |
| | ABI3BP | LOC652745 | AOC3 | HIGD1A |
| | HLA-C | MALL | PPAP2A | MFSD4 |
| | NDE1 | GNG2 | ZSCAN18 | AXL |
| | SRPX | DNASE1L3 | IVD | AQP1 |
| | WWTR1 | EGR1 | SFRP1 | MAP1B |
| | HMGCS2 | CMBL | COL4A2 | PALLD |
| | LOC646627 | GCNT3 | GPM6B | MPEG1 |
| | KRT20 | SERPING1 | EPB41L3 | KLHL5 |
| | KLF4 | MEIS1 | MAOA | TCEAL7 |
| | FHL1 | EDN3 | DMD | FILIP1L |
| | ARL14 | MSN | MSRB3 | IQGAP2 |
| | LUM | MT1G | PLOD2 | PRDX6 |
| | SORBS1 | TPSAB1 | C9orf19 | RAB31 |
| | METTL7A | GPX3 | MIER3 | LOC96610 |
| | FAM129A | CDKN2B | XDH | FGFR2 |
| | SCARA5 | FOSB | CLDN23 | PAPSS2 |
| | SI | HSPA1A | SGCE | XLKD1 |
| | ACTA2 | CYBRD1 | FOXF2 | SMTN |
| | CD177 | PTGER4 | AGR3 | C8orf4 |
| | C10orf99 | MAG1 | IGLJ3 | SDCBP2 |
| | COL15A1 | BEST2 | QKI | CCL11 |
| | NR3C2 | HLA-DQA1 | LOC399959 | ELOVL5 |
| | DHRS9 | PRIMA1 | ANKRD25 | FOXF1 |
| | LMOD1 | MT1F | CRISPLD2 | RELL1 |
| | EFEMP1 | MAFB | ANK2 | PNMA1 |
| | GREM1 | FAM107A | LOC283666 | LOC339562 |
| | IL1R2 | PRKACB | CRYAB | PALM2- |
| | LOC387763 | SELM | ACAT1 | AKAP2 |
| | TIMP3 | TYROBP | IGL@ | PAG1 |
| | MYLK | TNS1 | PBLD | HCLS1 |
| | CLDN8 | MYH11 | CCL8 | RGS1 |
| | RDX | ITM2C | LIFR | FXYD6 |
| | TSPAN7 | CES2 | HLA-DRB1 | OLFML3 |
| | TNFRSF17 | MS4A4A | UGP2 | COL6A3 |
| | SYNPO2 | PDGFRA | IGKV1D-13 | |

in a biological sample from said individual wherein a lower level of expression of the genes or transcripts of group (i) and/or group (ii) relative to control levels is indicative of a neoplastic large intestine cell or a cell predisposed to the onset of a neoplastic state.

In one embodiment, said expression is assessed by screening for DNA changes which impact on methylation, in particular hypermethylation. In another embodiment expression is assessed by the association of DNA with chromatin proteins carrying repressive modifications, for example, methylation of lysines 9 or 27 of the histone H3.

Reference to "large intestine" should be understood as a reference to a cell derived from one of the six anatomical regions of the large intestine, which regions commence after the terminal region of the ileum, these being:
(i) the cecum;
(ii) the ascending colon;
(iii) the transverse colon;
(iv) the descending colon;
(v) the sigmoid colon; and
(vi) the rectum.

Reference to "neoplasm" should be understood as a reference to a lesion, tumour or other encapsulated or unencapsulated mass or other form of growth which comprises neoplastic cells. A "neoplastic cell" should be understood as a reference to a cell exhibiting abnormal growth. The term "growth" should be understood in its broadest sense and includes reference to proliferation. In this regard, an example of abnormal cell growth is the uncontrolled proliferation of a cell. Another example is failed apoptosis in a cell, thus prolonging its usual life span. The neoplastic cell may be a benign cell or a malignant cell. In a preferred embodiment, the subject neoplasm is an adenoma or an adenocarcinoma. Without limiting the present invention to any one theory or mode of action, an adenoma is generally a benign tumour of epithelial origin which is either derived from epithelial tissue or exhibits clearly defined epithelial structures. These structures may take on a glandular appearance. It can comprise a malignant cell population within the adenoma, such as occurs with the progression of a benign adenoma to a malignant adenocarcinoma.

Preferably, said neoplastic cell is an adenoma or adenocarcinoma and even more preferably a colorectal adenoma or adenocarcinoma.

Each of the genes and transcripts detailed in sub-paragraphs (i) and (ii), above, would be well known to the person of skill in the art, as would their encoded proteins. The identification of the expression products of these genes and transcripts as markers of neoplasia occurred by virtue of differential expression analysis using Affymetrix HGU133Aor HGU133B gene chips. To this end, each gene chip is characterised by approximately 45,000 probe sets which detect the RNA transcribed from the genome. On average, approximately 11 probe pairs detect overlapping or consecutive regions of the RNA transcript. In general, the genes from which the RNA transcripts described herein are identifiable by the Affymetrix probesets are well known and characterised genes. However, to the extent that some of the probesets detect RNA transcripts which are not yet defined, these transcripts are indicated as "the gene, genes or transcripts detected by Affymetrix probe x". In some cases a number of genes may be detectable by a single probeset. It should be understood, however, that this is not intended as a limitation as to how the expression level of the subject gene or transcript can be detected. In the first instance, it would be understood that the subject gene transcript is also detectable by other probesets which would be present on the Affymetrix gene chip. The reference to a single probeset is merely included as an identifier of the gene transcript of interest. In terms of actually screening for the transcript, however, one may utilise a probe or probeset directed to any region of the transcript and not just to the 3- terminal 600bp transcript region to which the Affymetrix probes are often directed.

Reference to each of the genes and transcripts detailed above and their transcribed and translated expression products should therefore be understood as a reference to all forms of these molecules and to fragments or variants thereof. As would be appreciated by the person of skill in the art, some genes are known to exhibit allelic variation between individuals. Accordingly, the present invention should be understood to extend to such variants which, in terms of the present diagnostic applications, achieve the same outcome despite the fact that minor genetic variants between the actual nucleic acid sequences may exist between individuals or that within one individual there may exist 2 or more splice variants of the subject gene. The present invention should therefore be understood to extend to all forms of RNA (eg mRNA, primary RNA transcript, miRNA, etc), cDNA and peptide isoforms which arise from alternative splicing or any other mutation, polymorphic or allelic variation. It should also be understood to include reference to any subunit polypeptides such as precursor forms which may be generated, whether existing as a monomer, multimer, fusion protein or other complex.

To this end, in terms of the genes encompassed by the present invention, means for determining the existence of such variants, and characterising same, are described in Example 6. To the extent that the genes of the present invention are described by reference to an Affymetrix probeset, Table 6 provides details of the nucleic acid sequence to which each probe set is directed. Based on this information, the skilled person could, as a matter of routine procedure, identify the gene in respect of which that sequence forms part. A typical protocol for doing this is also outlined in Example 6.

It should be understood that the "individual" who is the subject of testing may be any human or non-human mammal. Examples of non-human mammals includes primates, livestock animals (e.g. horses, cattle, sheep, pigs, donkeys), laboratory test animals (e.g. mice, rats, rabbits, guinea pigs), companion animals (e.g. dogs, cats) and captive wild animals (e.g. deer, foxes). Preferably the mammal is a human.

The method of the present invention is predicated on the comparison of the level of the neoplastic markers of a biological sample with the control levels of these markers. The "control level" may be either a "normal level", which is the level of marker expressed by a corresponding large intestine cell or cellular population which is not neoplastic.

The normal (or "non-neoplastic") level may be determined using tissues derived from the same individual who is the subject of testing. However, it would be appreciated that this may be quite invasive for the individual concerned and it is therefore likely to be more convenient to analyse the test results relative to a standard result which reflects individual or collective results obtained from individuals other than the patient in issue. This latter form of analysis is in fact the preferred method of analysis since it enables the design of kits which require the collection and analysis of a single biological sample, being a test sample of interest. The standard results which provide the normal level may be calculated by any suitable means which would be well known to the person of skill in the art. For example, a population of normal tissues can be assessed in terms of the level of the neoplastic markers of the present invention, thereby providing a standard value or range of values against which all future test samples are analysed. It should also be understood that the normal level may be determined from the subjects of a specific cohort and for use with respect to test samples derived from that cohort. Accordingly, there may be determined a number of standard values or ranges which correspond to cohorts which differ in respect of characteristics such as age, gender, ethnicity or health status. Said "normal level" may be a discrete level or a range of levels. A decrease in the expression level of the subject genes relative to normal levels is indicative of the tissue being neoplastic.

Without limiting the present invention to any one theory or mode of action, although each of the genes or transcripts hereinbefore described is differentially expressed, either singly or in combination, as between neoplastic versus non-neoplastic cells of the large intestine, and is therefore diagnostic of the existence of a large intestine neoplasm, the expression of some of these genes was found to exhibit particularly significant levels of sensitivity, specificity and positive and negative predictive value. Accordingly, in a preferred embodiment one would screen for and assess the expression level of one or more of these genes. To this end, and without limiting the present invention to any one theory or mode of action, the following markers were determined to be expressed in neoplastic tissue at a level of 3-11 fold less than non-neoplastic tissue, when assessed by virtue of the method exemplified herein:

| **Fold Decrease** | **Gene, genes or transcripts detected by Affymetrix Probe No:** | **Gene** |
|---|---|---|
| 11 | 220026_at | CLCA4 |
| 10 | 214142_at | ZG16 |
| 9 | 209301_at | CA1 |
| | 205950_s_at | CA2 |
| 8 | 220834_at | MS4A12 |
| 7 | 206784_at | AQP8 |
| 6 | 203908_at | SLC4A4 |
| | 206198_s_at | CEACAM7 |
| | 205547_s_at | TAGLN |
| | 207003_at | GUCA1B |
| | 206422_at | GCG |
| | 209613_s_at | ADH1B |
| | 207245_at | UGT2B17 |
| 5 | 206134_at | ADAMDEC1 |
| | 217546_at | MT1M |
| | 206561_s_at | AKR1B10 |
| | 211719_x_at | FN1 |
| | 202291_s_at | MGP |
| | 209687_at | CXCL12 |
| 4 | 225207_at | PDK4 |
| | 206208_at | CA4 |
| | 207080_s_at | PYY |
| | 215118_s_at | IGHA1 |
| | 204083_s_at | TPM2 |
| | 229070_at | C6orf105 |
| | 211548_s_at | HPGD |
| | 206262_at | ADH1C |
| | 210107_at | CLCA1 |
| | 205892_s_at | FABP1 |
| | 212592_at | ENAM |
| | 205382_s_at | CFD |
| | 207502_at | GUCA2B |
| | 202995_s_at | FBLN1 |
| | 206149_at | LOC63928 |
| | 204719_at | ABCA8 |
| 3 | 210809_s_at | POSTN |
| | 201893_x_at | DCN |
| | 223597_at | ITLN1 |
| | 209156_s_at | COL6A2 |
| | 203240_at | FCGBP |
| | 224963_at | SLC26A2 |
| | 226303_at | PGM5 |
| | 212730_at | DMN |
| | 201141_at | GPNMB |
| | 211959_at | IGFBP5 |
| | 205200_at | CLEC3B |
| | 242601_at | LOC253012 |
| | 213068_at | DPT |
| | 208383_s_at | PCK1 |
| | 203951_at | CNN1 |
| | 204818_at | HSD17B2 |
| | 219014_at | PLAC8 |
| | 209656_s_at | TMEM47 |
| | 222722_at | OGN |
| | 201617_x_at | CALD1 |
| | 202274_at | ACTG2 |
| | 218756_s_at | MGC4172 |
| | 210302_s_at | MAB21L2 |
| | 228885_at | RPL24 |
| | 209735_at | ABCG2 |
| | 228504_at | CCDC80 |
| | 225242_s_at | UGT1A1 |
| | 215125_s_at | MRC1 |
| | 204438_at | HSD11B2 |
| | 204130_at | ANPEP |
| | 202888_s_at | MATN2 |
| | 202350_s_at | PRNP |
| | 201300_s_at | ABI3BP |
| | 223395_at | HLA-C |
| | 214768_x_at | NDE1 |
| | 228133_s_at | SRPX |
| | 204955_at | WWTR1 |
| | 202133_at | HMGCS2 |
| | 204607 at | LOC646627 |
| | 238143_at | KRT20 |
| | 213953_at | KLF4 |
| | 220266_s_at | FHL1 |
| | 210299_s_at | ARL14 |
| | 220468_at | LUM |
| | 201744_s_at | SORBS1 |
| | 218087_s_at | METTL7A |
| | 207761_s_at | FAM129A |
| | 217967_s_at | SCARA5 |
| | 229839_at | SI |
| | 206664 at | ACTA2 |
| | 200974_at | CD177 |
| | 219669_at | C10orf99 |
| | 227736_at | COL15A1 |
| | 203477_at | NR3C2 |
| | 205259_at | |

There is therefore more particularly provided a method of screening for the onset or predisposition to the onset of a large intestine neoplasm in an individual, said method comprising assessing the level of expression of one or more genes or transcripts selected from:
(i) the gene, genes or transcripts detected by Affymetrix probeset IDs: 220026_at; and/or
(ii) CLCA4
in a biological sample from said individual wherein a lower level of expression of the gene or transcripts of group (i) and/or group (ii) relative to control levels is indicative of a neoplastic large intestine cell or a cell predisposed to the onset of a neoplastic state.

### Preferably, said control level is a non-neoplastic level.

In another embodiment, there is provided a method of screening for the onset or predisposition to the onset of a large intestine neoplasm in an individual, said method comprising assessing the level of expression of one or more genes or transcripts selected from:
(i) the gene, genes or transcripts detected by Affymetrix probeset IDs: 214142_at; and/or
(ii) ZG16
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In yet another embodiment there is provided a method of screening for the onset or predisposition to the onset of a large intestine neoplasm in an individual, said method comprising assessing the level of expression of one or more genes or transcripts selected from:

| **(i)** | the gene, genes or transcripts detected by Affymetrix probeset IDs: | |
|---|---|---|
| | 209301_at | 205950_s_at; and/or |
| **(ii)** | CA2 | CA1 |

in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In still yet another preferred embodiment, there is provided a method of screening for the onset or predisposition to the onset of a large intestine neoplasm in an individual, said method comprising assessing the level of expression of one or more genes or transcripts selected from:
(i) the gene, genes or transcripts detected by Affymetrix probeset IDs: 220834_at; and/or
(ii) MS4A12
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In yet still another preferred embodiment, there is provided a method of screening for the onset or predisposition to the onset of a large intestine neoplasm in an individual, said method comprising assessing the level of expression of one or more genes or transcripts selected from:
(i) the gene, genes or transcripts detected by Affymetrix probeset IDs:
   206784_at; and/or
   (ii) AQP8
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In a further embodiment, there is provided a method of screening for the onset or predisposition to the onset of a large intestine neoplasm in an individual, said method comprising assessing the level of expression of one or more genes or transcripts selected from:
(i) (i) the gene, genes or transcripts detected by Affymetrix probeset IDs: 203908_at, 206198_s_at, 205547_s_at, 207003_at, 206422_at, 209613_s_at, 207245_at; and/or
(ii) SLC4A4, CEACAM7, TAGLN, GUCA1B, GCG, ADH1B, UGT2B 17
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In another further embodiment, there is provided a method of screening for the onset or predisposition to the onset of a large intestine neoplasm in an individual, said method comprising assessing the level of expression of one or more genes or transcripts selected from:
(i) the gene, genes or transcripts detected by Affymetrix probeset IDs:
   206134_at, 217546_at, 206561_s_at, 2117t9_x_at, 202291_s_at, 209687_at; and/or
(ii) ADAMDEC1, MT1M, AKR1B10, FN1, MGP, CXCL12
in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In still another further embodiment, there is provided a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | |
|---|---|---|---|
| | 225207_at | 211548_s_at | 205382_s_at |
| | 206208_at | 206262_at | 207502_at |
| | 207080_s_at | 210107_at | 202995_s_at |
| | 215118_s_at | 205892_s_at | 206149_at |
| | 204083_s_at | 212592_at | 204719_at |
| | 229070_at; and/or | | |
| **(ii)** | PDK4 | HPGD | CFD |
| | CA4 | ADH1C | GUCA2B |
| | PYY | CLCA1 | FBLN1 |
| | IGHA1 | FABP1 | LOC63928 |
| | TPM2 | ENAM | ABCA8 |
| | C6orf105 | | |

in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

In yet still yet another further embodiment, there is provided a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | |
|---|---|---|---|
| | 210809_s_at | 201617_x_at | 202133_at |
| | 201893_x_at | 202274_at | 204607_at |
| | 223597_at | 218756_s_at | 238143_at |
| | 209156_s_at | 210302_s_at | 213953_at |
| | 203240_at | 228885_at | 220266_s_at |
| | 224963_at | 209735_at | 210299_s_at |
| | 226303_at | 228504_at | 220468_at |
| | 212730_at | 225242_s_at | 201744_s_at |
| | 201141_at | 215125_s_at | 218087_s_at |
| | 211959_at | 204438_at | 207761_s_at |
| | 205200_at | 204130_at | 217967_s_at |
| | 242601_at | 202888_s_at | 229839_at |
| | 213068_at | 202350_s_at | 206664_at |
| | 208383_s_at | 201300_s_at | 200974_at |
| | 203951_at | 223395_at | 219669_at |
| | 204818_at | 214768_x_at | 227736_at |
| | 219014_at | 228133_s_at | 203477_at |
| | 209656_s_at | 204955_at | 205259_at |
| | 222722_at; and/or | | |
| **(ii)** | POSTN | OGN | WWTR1 |
| | DCN | CALD1 | HMGCS2 |
| | ITLN1 | ACTG2 | LOC646627 |
| | COL6A2 | MGC4172 | KRT20 |
| | FCGBP | MAB21L2 | KLF4 |
| | SLC26A2 | RPL24 | FHL1 |
| | PGM5 | ABCG2 | ARL14 |
| | DMN | CCDC80 | LUM |
| | GPNMB | UGT1A1 | SORBS1 |
| | IGFBP5 | MRC1 | METTL7A |
| | CLEC3B | HSD11B2 | FAM129A |
| | LOC253012 | ANPEP | SCARA5 |
| | DPT | MATN2 | SI |
| | PCK1 | PRNP | ACTA2 |
| | CNN1 | ABI3BP | CD177 |
| | HSD17B2 | HLA-C | C10orf99 |
| | PLAC8 | NDE1 | COL15A1 |
| | TMEM47 | SRPX | NR3C2 |

in a biological sample from said individual wherein a lower level of expression of the genes or transcripts of group (i) and/or group (ii) relative to control levels is indicative of a neoplastic large intestine cell or a cell predisposed to the onset of a neoplastic state.

Preferably, said control level is a non-neoplastic level.

According to these aspects of the present invention, said large intestine tissue is preferably colorectal tissue.

In one embodiment, said expression is assessed by screening for DNA changes which impact on methylation, in particular hypermethylation. In another embodiment, expression is assessed by the association of DNA with chromatin proteins carrying repressive modifications, for example, methylation of lysines 9 or 27 of histone H3.

The detection method of the present invention can be performed on any suitable biological sample. To this end, reference to a "biological sample" should be understood as a reference to any sample of biological material derived from an animal such as, but not limited to, cellular material, biofluids (eg. blood), faeces, tissue specimens (such as biopsy specimens), surgical specimens or fluid which has been introduced into the body of an animal and subsequently removed (such as, for example, the solution retrieved from an enema wash). The biological sample which is tested according to the method of the present invention may be tested directly or may require some form of treatment prior to testing. For example, a biopsy or surgical sample may require homogenisation prior to testing or it may require sectioning for *in situ* testing of the qualitative expression levels of individual genes. Alternatively, a cell sample may require permeabilisation prior to testing. Further, to the extent that the biological sample is not in liquid form, (if such form is required for testing) it may require the addition of a reagent, such as a buffer, to mobilise the sample.

To the extent that the neoplastic marker gene expression product is present in a biological sample, the biological sample may be directly tested or else all or some of the nucleic acid material present in the biological sample may be isolated prior to testing. In yet another example, the sample may be partially purified or otherwise enriched prior to analysis. For example, to the extent that a biological sample comprises a very diverse cell population, it may be desirable to enrich for a sub-population of particular interest. It is within the scope of the present invention for the target cell population or molecules derived therefrom to be pretreated prior to testing, for example, inactivation of live virus or being run on a gel. It should also be understood that the biological sample may be freshly harvested or it may have been stored (for example by freezing) prior to testing or otherwise treated prior to testing (such as by undergoing culturing).

The choice of what type of sample is most suitable for testing in accordance with the method disclosed herein will be dependent on the nature of the situation. Preferably, said sample is a faecal (stool) sample, enema wash, surgical resection, tissue or blood specimen.

In a related aspect, it has been determined that certain of the markers hereinbefore defined are more indicative of adenoma development versus cancer development or vice versa. This is an extremely valuable finding since it enables one to more specifically characterise the likely nature of a neoplasm which is detected by virtue of the method of the present invention.

Accordingly, in a related aspect the present invention is directed to a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | | |
|---|---|---|---|---|
| | 200600_at | 203680_at | 211959_at | 218469_at |
| | 200665_s_at | 203729_at | 211964_at | 218559_s_at |
| | 200799_at | 203748_x_at | 211985_s_at | 219087_at |
| | 200845_s_at | 204069_at | 211990_at | 219607_s_at |
| | 200859_x_at | 204122_at | 211991_s_at | 221541_at |
| | 200897_s_at | 204135_at | 212077_at | 222043_at |
| | 200974_at | 204438_at | 212091_s_at | 222453_at |
| | 200986_at | 204457_s_at | 212136_at | 222513_s_at |
| | 201041_s_at | 204570_at | 212158_at | 223121_s_at |
| | 201061_s_at | 204688_at | 212185_x_at | 223122_s_at |
| | 201069_at | 205412_at | 212195_at | 223235_s_at |
| | 201105_at | 205683_x_at | 212230_at | 223343_at |
| | 201137_s_at | 205935_at | 212233_at | 224560_at |
| | 201141_at | 207134_x at | 212265_at | 224694_at |
| | 201150_s_at | 207266_x_at | 212386_at | 224840_at |
| | 201289_at | 208131_s_at | 212387_at | 224964_s_at |
| | 201300_s_at | 208370_s_at | 212397_at | 225242_s_at |
| | 201426_s_at | 208747_s_at | 212414_s_at | 225269_s_at |
| | 201438_at | 208788_at | 212419_at | 225353_s_at |
| | 201616_s_at | 208789_at | 212464_s_at | 225381_at |
| | 201617_x_at | 208894_at | 212667_at | 225442_at |
| | 201645_at | 209047_at | 212671_s_at | 225602_at |
| | 201667_at | 209101_at | 212713_at | 225604_s_at |
| | 201743_at | 209138_x_at | 212764_at | 225626_at |
| | 201744_s_at | 209147_s_at | 212956_at | 225688_s_at |
| | 201842_s_at | 209156_s_at | 213428_s_at | 225710_at |
| | 201852_x_at | 209191_at | 213509_x_at | 226001_at |
| | 201858_s_at | 209209_s_at | 213746_s_at | 226051_at |
| | 201859_at | 209210_s_at | 213891_s_at | 226084_at |
| | 201865_x_at | 209312_x_at | 214038_at | 226103_at |
| | 201893_x_at | 209335_at | 214677_x_at | 226430_at |
| | 201920_at | 209436 at | 214752_x_at | 226682_at |
| | 202007 at | 209457_at | 215049_x_at | 226694_at |
| | 202069_s_at | 209496_at | 215076_s_at | 226818_at |
| | 202133_at | 209621_s_at | 215193_x_at | 226834_at |
| | 202283_at | 209651_at | 215382_x_at | 226841_at |
| | 202291_s_at | 209656_s_at | 215388_s_at | 227061_at |
| | 202403_s_at | 209868_s_at | 216442_x_at | 227099_s_at |
| | 202620_s_at | 210084_x_at | 216474_x_at | 227235_at |
| | 202686_s_at | 210133_at | 216834_at | 227404_s_at |
| | 202760_s_at | 210139_s_at | 217480_x_at | 227529_s_at |
| | 202766_s_at | 210495_x_at | 217757_at | 227561_at |
| | 202953_at | 210517_s at | 217762_s_at | 227623_at |
| | 202957_at | 210764_s_at | 217764_s_at | 227705_at |
| | 202994_s_at | 210809_s_at | 217767_at | 227727_at |
| | 202995_s_at | 210982_s_at | 217897_at | 228507_at |
| | 203066_at | 211161_s_at | 218162_at | 228750_at |
| | 203131_at | 211596_s_at | 218224_at | 228846_at |
| | 203305_at | 211671_s_at | 218312_s_at | 229530_at |
| | 203382_s_at | 211719_x_at | 218353_at | 230264_s_at |
| | 203477_at | 211813_x_at | 218418_s_at | 231579_s_at |
| | 203645_s_at | 211896_s_at | 218468_s_at | 234987_at; and/or |
| **(ii)** | A2M | CYBRD1 | LGALS1 | PRKAR2B |
| | ACAT1 | CYR61 | LOC283666 | PRNP |
| | ACTA2 | DCN | LOC339562 | PTGIS |
| | AKAP12 | DDR2 | LOC387763 | PTRF |
| | ANKRD25 | DSCR1 | LOC399959 | QKI |
| | ANTXR1 | DUSP1 | LRIG1 | RAB31 |
| | AP1S2 | DUSP5 | LUM | RARRES2 |
| | APOE | EFEMP1 | MAFB | RBMS1 |
| | AQP1 | EGR1 | MAP1B | RDX |
| | ASPN | ELOVL5 | MEIS1 | RELL1 |
| | ATP2B4 | EMP3 | MFAP4 | RGS1 |
| | AXL | F13A1 | MGP | RGS5 |
| | C10orf56 | FBLN1 | MMP2 | SDC2 |
| | C1QB | FBN1 | MPEG1 | SELM |
| | C1QC | FILIP1L | MRC1 | SEPT6 |
| | C1S | FKBP5 | MRGPRF | SERPINF1 |
| | C20orf118 | FLNA | MS4A4A | SERPING1 |
| | C3 | FN1 | MS4A7 | SFRP2 |
| | C9orf19 | FOXF1 | MSN | SGCE |
| | CALD1 | FXYD6 | MT2A | SLC20A1 |
| | CALM1 | GALNAC4S-6ST | MXD1 | SMOC2 |
| | CCDC80 | GAS1 | NEXN | SORBS1 |
| | CCL11 | GJA1 | NID1 | SPARC |
| | CCL8 | GNG2 | NR3C1 | SPON1 |
| | CD14 | GPNMB | OLFML3 | SRGN |
| | CD163 | GREM1 | PAG1 | STOM |
| | CES2 | GUCY1A3 | PALLD | TBC1D9 |
| | CFHR1 | HCLS1 | PALM2- | TCEAL7 |
| | CLU | HLA-DPA1 | AKAP2 | TGFB1I1 |
| | COL14A1 | HLA-DPB1 | PDGFRA | TIMP2 |
| | COL15A1 | HLA-DQA1 | PDLIM3 | TIMP3 |
| | COL1A2 | HLA-DRA | PHLDB2 | TMEM47 |
| | COL3A1 | HLA-DRB1 | PLEKHC1 | TNC |
| | COL4A2 | HSPA1A | PLOD2 | TPSAB1 |
| | COL6A1 | IDH3A | PMP22 | TPSB2 |
| | COL6A2 | IGFBP5 | PNMA1 | TUBB6 |
| | COL6A3 | IGL@ | POSTN | TYROBP |
| | COX7A1 | IGLJ3 | PPAP2A | VIM |
| | CRISPLD2 | IL6ST | PPAP2B | WWTR1 |
| | CTGF | KLHL5 | PRDX6 | ZSCAN18 |

in a biological sample from said individual wherein a lower level of expression of the genes or transcripts of group (i) and/or group (ii) relative to control levels is indicative of an adenoma cell or a cell predisposed to the onset of an adenoma state.

In another preferred embodiment of this aspect of the present invention there is provided a method of screening for the onset or predisposition to the onset of a large intestine neoplasm in an individual, said method comprising screening the level of expression of one or more genes or transcripts selected from:

| **(i)** | the gene or genes detected by Affymetrix probeset IDs: | | | |
|---|---|---|---|---|
| | 200884_at | 206000_at | 214598_at | 226147_s_at |
| | 201495_x_at | 206094_x_at | 214916_x_at | 226302_at |
| | 202266_at | 206262_at | 215125_s_at | 226594_at |
| | 202350_s_at | 206377_at | 215299_x_at | 226654_at |
| | 202731_at | 206385_s_at | 215867_x_at | 226811_at |
| | 202741_at | 206664_at | 216336_x_at | 227052_at |
| | 202742_s_at | 207126_x_at | 216491_x_at | 227522_at |
| | 202768_at | 207245_at | 216510_x_at | 227682_at |
| | 202838_at | 207390_s_at | 217022_s_at | 227725_at |
| | 203058_s_at | 207392_x_at | 217109_at | 227735_s_at |
| | 203060_s_at | 207432_at | 217110_s_at | 227736_at |
| | 203240_at | 207761_s_at | 217165_x_at | 228133_s_at |
| | 203296_s_at | 208596_s_at | 217232_x_at | 228195_at |
| | 203343_at | 208920_at | 217414_x_at | 228232_s_at |
| | 203474_at | 209114_at | 218541_s_at | 228241_at |
| | 203638_s_at | 209374_s_at | 218546_at | 228469_at |
| | 203963_at | 209458_x_at | 219059_s_at | 228961_at |
| | 204018_x_at | 209791_at | 219543_at | 229070_at |
| | 204034_at | 210107_at | 219796_s_at | 229254_at |
| | 204036_at | 210524_x_at | 219948_x_at | 229659_s_at |
| | 204130_at | 210735_s_at | 220075_s_at | 229831_at |
| | 204388_s_at | 211372_s_at | 220266_s_at | 230595_at |
| | 204389_at | 211538_s_at | 220468_at | 231925_at |
| | 204508_s_at | 211549_s_at | 220645_at | 231975_s_at |
| | 204532_x_at | 211637_x_at | 220812_s_at | 233565_s_at |
| | 204607_at | 211699_x_at | 221004_s_at | 235146_at |
| | 204673_at | 211745_x_at | 221305_s_at | 235766_x_at |
| | 204818_at | 212224_at | 221584_s_at | 235849_at |
| | 204895_x_at | 212592_at | 221841_s_at | 238143_at |
| | 204897_at | 212741 at | 221896_s_at | 238750_at |
| | 205112_at | 212814_at | 223484_at | 238751_at |
| | 205259_at | 213317_at | 223597_at | 239272_at |
| | 205403_at | 213451_x_at | 223754_at | 241994_at |
| | 205480_s_at | 213629_x_at | 224342_x_at | 242447_at |
| | 205554_s_at | 213921_at | 224989_at | 242601_at |
| | 205593_s_at | 213953_at | 224990_at | 243278_at; and/or |
| | 205892_s_at | 214164_x_at | 225458_at | |
| | 205929_at | 214433_s_at | 225728_at | |
| | | | | |
| **(ii)** | ADH1C | FGFR2 | LOC646627 | PTGER4 |
| | AGR3 | FOSB | LOC652128 | RAB27A |
| | ALDH1A1 | FOXF2 | LOC96610 | SCARA5 |
| | ANK3 | FOXP2 | MAOA | SDCBP2 |
| | ARL14 | FUCA1 | MATN2 | SELENBP1 |
| | ATP1A2 | GPA33 | MEP1A | SI |
| | ATP8B1 | HBA1 | METTL7A | SMTN |
| | BEST2 | HBA2 | MFSD4 | SORBS2 |
| | C10orf99 | HBB | MGC13057 | SRI |
| | C15orf48 | HHLA2 | MIER3 | SST |
| | C1orf115 | HIGD1A | MMP28 | ST6GALNAC1 |
| | C4orf34 | HMGCS2 | MT1A | SULT1A1 |
| | C6orf105 | HPGD | MT1F | TNXB |
| | C8orf4 | HSD11B2 | MT1M | TSPAN1 |
| | CA12 | HSD17B2 | MUC12 | TTRAP |
| | CCL28 | HSPA2 | MUC2 | UGDH |
| | CKB | IGHA1 | MUC4 | UGP2 |
| | CLCA1 | IGHM | MUCDHL | UGT1A1 |
| | CLDN8 | IL1R2 | MYH11 | UGT1A3 |
| | CLIC5 | IL8 | NDE1 | UGT1A6 |
| | CMBL | IQGAP2 | NR3C2 | UGT1A8 |
| | CNTN3 | ITLN1 | P2RY1 | UGT1A9 |
| | DNASE1L3 | ITM2C | PADI2 | UGT2A3 |
| | EDG2 | KCNMA1 | PAPSS2 | UGT2B15 |
| | ENAM | KIAA0828 | PBLD | UGT2B17 |
| | ENTPD5 | KLF4 | PDCD4 | VSIG2 |
| | ETHE1 | KRT20 | PDE9A | XDH |
| | FABP1 | LOC253012 | PIGR | XLKD1 |
| | FAM46C | LOC25845 | PLCE1 | ZCWPW2 |
| | FAM55D | LOC285382 | PPID | |
| | FCGBP | LOC572558 | PRKACB | |

in a biological sample from said individual wherein a lower level of expression of the genes or transcripts of group (i) and/or group (ii) relative to control levels is indicative of a cancer cell or a cell predisposed to the onset of a cancerous state.

According to these aspects, said control levels are preferably non-neoplastic levels and said large intestine tissue is colorectal tissue. Even more preferably, said biological sample is a stool sample or blood sample.

In one embodiment, said expression is assessed by screening for DNA changes which impact on methylation, in particular hypermethylation. In another embodiment, expression is assessed by the association of DNA with chromatin proteins carrying repressive modifications, for example, methylation of lysines 9 or 27 of histone H3.

In a related aspect, it has been determined that a subpopulation of the markers of the present invention are not only expressed at levels lower than normal levels, their expression pattern is uniquely characterised by the fact that expression levels above that of background control levels are not detectable in neoplastic tissue. This determination has therefore enabled the development of qualitative screening systems which are simply designed to detect marker expression relative to a control background level. In accordance with this aspect of the present invention, said "control level" is therefore the "background level".

According to this aspect, there is therefore provided a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | | |
|---|---|---|---|---|
| | 202920_at | 209613_s_at | 228504_at | 214598_at |
| | 203881_s_at | 220037_s_at | 228706_s_at | 219948_x_at |
| | 204719_at | 220376_at | 228766_at | 220812_s_at |
| | 204931_at | 222717_at | 228854_at | 221305_s_at |
| | 204940_at | 224412_s_at | 228885_at | 229831_at |
| | 205433_at | 225381_at | 230788_at | 231925_at |
| | 206637_at | 225575_at | 231120_x_at | 235146_at |
| | 207080_s_at | 227529_s_at | 231773_at | 238751_at |
| | 207980_s_at | 227623_at | 203296_s_at | 243278_at; and/or |
| | 209170_s_at | 227705_at | 206664_at | |
| | 209209_s_at | 227827 at | 211549_s_at | |
| **(ii)** | ADH1B | CD36 | P2RY1 | PLEKHC1 |
| | SORBS2 | BCHE | ANK2 | LRRC19 |
| | PYY | TCEAL7 | XLKD1 | LIFR |
| | ABCA8 | ANGPTL1 | LOC399959 | ATP1A2 |
| | RPL24 | DMD | AKAP12 | HPGD |
| | SI | GCNT2 | UGT2A3 | GPM6B |
| | CLDN8 | SDPR | HHLA2 | UGT1A8 |
| | P2RY14 | PKIB | SORBS2 | FOXP2 |
| | PLN | CITED2 | CLDN23 | |
| | TRPM6 | TCF21 | CNTN3 | |

in a biological sample from said individual wherein a level of expression of the genes or transcripts of group (i) and/or group (ii) which is not substantially above background levels is indicative of a neoplastic cell or a cell predisposed to the onset of a neoplastic state.

In a most preferred embodiment, said genes or transcripts are selected from:
**(i)** the gene, genes or transcripts detected by Affymetrix probeset IDs: 209613_s_at, 227827_at, 204719_at, 228504_at, 228885_at, 206664_at, 207080_s_at; and/or
**(ii)** ADH1B, SORBS2, PYY, ABCA8, RPL24, SI

Preferably, said neoplasm is an adenoma or an adenocarcinoma and said gastrointestinal tissue is colorectal tissue.

In yet another embodiment, it has been determined that a further subpopulation of these markers are more characteristic of adenoma development, while others are more characteristic of cancer development. Accordingly, there is provided a convenient means of qualitatively obtaining indicative information in relation to the characteristics of the subject neoplasm.

According to this embodiment there is provided a method of screening for the onset or predisposition to the onset of a large intestine neoplasm in an individual, said method comprising screening the level of expression of one or more genes or transcripts selected from:
**(i)** the gene, genes or transcripts detected by Affymetrix probeset IDs: 209209_s_at, 225381_at, 227529_s_at, 227623_at, 227705_at; and/or
**(ii)** AKAP12, LOC399959, PLEKHC1, TCEAL7
in a biological sample from said individual wherein a lower level of expression of the genes or transcripts of group (i) and/or group (ii) which is not substantially above background levels is indicative of an adenoma cell or a cell predisposed to the onset of an adenoma state.

In yet still another preferred embodiment there is provided a method of screening for the onset or predisposition to the onset of a large intestine neoplasm in an individual, said method comprising screening the level of expression of one or more genes or transcripts selected from:

| **(i)** | the gene, genes or transcripts detected by Affymetrix probeset IDs: | | |
|---|---|---|---|
| | 203296_s_at | 219948_x_at | 231925_at |
| | 206664_at | 220812_s_at | 235146_at |
| | 211549_s_at | 221305_s_at | 238751_at |
| | 214598_at | 229831_at | 243278_at; and/or |
| | | | |
| **(ii)** | ATP1A2 | HHLA2 | SORBS2 |
| | CLDN8 | HPGD | UGT1A8 |
| | CNTN3 | P2RY1 | UGT2A3 |
| | FOXP2 | SI | |

in a biological sample from said individual wherein a lower level of expression of the genes or transcripts of group (i) and/or group (ii) which is not substantially above background levels is indicative of a cancer cell or a cell predisposed to the onset of a cancerous state.

Preferably, said large intestine tissue is colorectal tissue.

More preferably, said biological sample is a stool sample or a blood sample.

In one embodiment, said expression is assessed by screening for DNA changes which impact on methylation, in particular hypermethylation. In another embodiment, expression is assessed by the association of DNA with chromatin proteins carrying repressive modifications, for example, methylation of lysines 9 or 27 of histone H3.

As detailed hereinbefore, the present invention is designed to screen for a neoplastic cell or cellular population, which is located in the large intestine. Accordingly, reference to "cell or cellular population" should be understood as a reference to an individual cell or a group of cells. Said group of cells may be a diffuse population of cells, a cell suspension, an encapsulated population of cells or a population of cells which take the form of tissue.

Reference to "expression" should be understood as a reference to the transcription and/or translation of a nucleic acid molecule. In this regard, the present invention is exemplified with respect to screening for neoplastic marker expression products taking the form of RNA transcripts (eg primary RNA or mRNA). Reference to "RNA" should be understood to encompass reference to any form of RNA, such as primary RNA or mRNA. Without limiting the present invention in any way, the modulation of gene transcription leading to increased or decreased RNA synthesis will also correlate with the translation of some of these RNA transcripts (such as mRNA) to produce a protein product. Accordingly, the present invention also extends to detection methodology which is directed to screening for modulated levels or patterns of the neoplastic marker protein products as an indicator of the neoplastic state of a cell or cellular population. Although one method is to screen for mRNA transcripts and/or the corresponding protein product, it should be understood that the present invention is not limited in this regard and extends to screening for any other form of neoplastic marker expression product such as, for example, a primary RNA transcript.

In terms of screening for the downregulation of expression of a marker it would also be well known to the person of skill in the art that changes which are detectable at the DNA level are indicative of changes to gene expression activity and therefore changes to expression product levels. Such changes include but are not limited to, changes to DNA methylation. Accordingly, reference herein to "screening the level of expression" and comparison of these "levels of expression" to control "levels of expression" should be understood as a reference to assessing DNA factors which are related to transcription, such as gene/DNA methylation patterns.

It would also be known to a person skilled in the art that changes in the structure of chromatin are indicative of changes in gene expression. Silencing of gene expression is often associated with modification of chromatin proteins, methylation of lysines at either or both positions 9 and 27 of histone H3 being well studied examples, while active chromatin is marked by acetylation of lysine 9 of histone H3. Thus association of gene sequences with chromatin carrying repressive or active modifications can be used to make an assessment of the expression level of a gene.

It is well within the skill of the person of skill in the art to determine the most appropriate screening method for any given situation. To this end, the genes which are known to encode an expression product which is either secreted by the cell or membrane bound is detailed in the table below. It would be appreciated that screening for neoplastic markers which are secreted or membrane bound may provide particular advantages in terms of the design of a diagnostic screening product.

| The gene or genes detected by Affymetrix probe Nos: | | | |
|---|---|---|---|
| 200600_at | 205593_s_at | 212185_x_at | 224480_s_at |
| 200845_s_at | 205765_at | 212192_at | 224663_s_at |
| 200859_x_at | 205892_s_at | 212224_at | 224694_at |
| 200884_at | 205927_s_at | 212230_at | 224823_at |
| 200897_s_at | 205929_at | 212233_at | 224836_at |
| 200974_at | 205935_at | 212265_at | 224840_at |
| 201041_s_at | 205935_at | 212288_at | 224959_at |
| 201058_s_at | 205950_s_at | 212386_at | 224963_at |
| 201061_s_at | 206000_at | 212387_at | 224964_s_at |
| 201069_at | 206094_x_at | 212397_at | 224989_at |
| 201105_at | 206143_at | 212414_s_at | 224990_at |
| 201137_s_at | 206149_at | 212419_at | 225207_at |
| 201300_s_at | 206198_s_at | 212671_s_at | 225242_s_at |
| 201324_at | 206199_at | 212730_at | 225269_s_at |
| 201426_s_at | 206208_at | 212741_at | 225381_at |
| 201539_s_at | 206209_s_at | 212764_at | 225442_at |
| 201540_at | 206262_at | 212814 at | 225458_at |
| 201616_s_at | 206377_at | 212859_x_at | 225575_at |
| 201617_x_at | 206385_s_at | 212956_at | 225602_at |
| 201667_at | 206461_x_at | 213106_at | 225604_s_at |
| 201739_at | 206561_s_at | 213317_at | 225626_at |
| 201743_at | 206576_s_at | 213509_x_at | 225710_at |
| 201865_x_at | 206637_at | 213629_x_at | 225720_at |
| 201920_at | 206664_at | 213746_s_at | 225721_at |
| 201957_at | 206710_s_at | 213891_s_at | 225782_at |
| 202007_at | 206784_at | 213953_at | 225894_at |
| 202069_s_at | 207126_x_at | 214027_x_at | 225895_at |
| 202133_at | 207245_at | 214234_s_at | 226001_at |
| 202242_at | 207266_x_at | 214235_at | 226051_at |
| 202266_at | 207390_s_at | 214414_x_at | 226084_at |
| 202274_at | 207392_x_at | 214433_s_at | 226103_at |
| 202388_at | 207432_at | 214505_s_at | 226147_s_at |
| 202555_s_at | 207761_s_at | 214598_at | 226248_s_at |
| 202620_s_at | 207980_s_at | 214677_x_at | 226302_at |
| 202686_s_at | 208063_s_at | 214696_at | 226303_at |
| 202731_at | 208131_s_at | 214752_x_at | 226304_at |
| 202741_at | 208370_s_at | 214768_x_at | 226333_at |
| 202742_s_at | 208383_s_at | 214777_at | 226430_at |
| 202746_at | 208450_at | 215049_x_at | 226594_at |
| 202760_s_at | 208581_x_at | 215118_s_at | 226654_at |
| 202768_at | 208596_s_at | 215125_s_at | 226682_at |
| 202888_s_at | 208763_s_at | 215176_x_at | 226694_at |
| 202920_at | 208788_at | 215193_x_at | 226811_at |
| 202957_at | 208789_at | 215299_x_at | 226818_at |
| 202992_at | 208920_at | 215657_at | 226834_at |
| 202994_s_at | 208937_s_at | 216207_x_at | 226841_at |
| 202995_s_at | 209047_at | 216336_x_at | 227006_at |
| 203000_at | 209074_s_at | 216401_x_at | 227052_at |
| 203001_s_at | 209114_at | 216491_x_at | 227061_at |
| 203058_s_at | 209116_x_at | 216576_x_at | 227099_s_at |
| 203060_s_at | 209138_x_at | 216834_at | 227235_at |
| 203066_at | 209147_s_at | 216984_x_at | 227404_s_at |
| 203131_at | 209156_s_at | 217022_s_at | 227522_at |
| 203240_at | 209167_at | 217148 x at | 227529_s_at |
| 203305_at | 209170_s_at | 217165_x_at | 227561_at |
| 203343_at | 209191_at | 217232_x_at | 227623_at |
| 203382_s_at | 209209_s_at | 217235_x_at | 227662_at |
| 203474_at | 209210_s_at | 217378_x_at | 227682_at |
| 203638_s_at | 209283_at | 217414_x_at | 227705_at |
| 203645_s_at | 209301_at | 217480_x_at | 227719_at |
| 203680_at | 209312_x_at | 217546_at | 227725_at |
| 203729_at | 209357_at | 217762_s_at | 227727_at |
| 203748_x_at | 209373_at | 217764_s_at | 227735_s_at |
| 203766_s_at | 209374_s_at | 217897_at | 227736_at |
| 203908_at | 209457_at | 217967_s_at | 228202_at |
| 203913_s_at | 209458_x_at | 218087_s_at | 228232_s_at |
| 203914_x_at | 209498_at | 218211_s_at | 228469_at |
| 203951_at | 209612_s_at | 218224_at | 228504_at |
| 203980_at | 209613_s_at | 218312_s_at | 228507_at |
| 204018_x_at | 209621_s_at | 218353_at | 228640_at |
| 204034_at | 209651_at | 218418_s_at | 228766_at |
| 204036_at | 209656_s_at | 218546_at | 228846_at |
| 204069_at | 209667_at | 218559_s_at | 228854_at |
| 204083_s_at | 209668_x_at | 219014_at | 228961_at |
| 204122_at | 209868_s_at | 219059_s_at | 229070_at |
| 204130_at | 209948_at | 219508_at | 229254_at |
| 204135_at | 210107_at | 219543_at | 229530_at |
| 204326_x_at | 210139_s_at | 219607_s_at | 229659_s_at |
| 204388_s_at | 210298_x_at | 219796_s_at | 229831_at |
| 204389_at | 210299_s_at | 219948_x_at | 229839_at |
| 204438_at | 210302_s_at | 219955_at | 230087_at |
| 204457_s_at | 210517_s_at | 220026_at | 230264_s_at |
| 204532_x_at | 210524_x_at | 220037_s_at | 230595_at |
| 204570_at | 210524_x_at | 220075_s_at | 230788_at |
| 204607_at | 210946_at | 220266_s_at | 230830_at |
| 204688_at | 211372_s_at | 220376_at | 231120_x_at |
| 204697_s_at | 211538_s_at | 220468_at | 231832_at |
| 204719_at | 211548_s_at | 220812_s_at | 231925_at |
| 204745_x_at | 211549_s_at | 220834_at | 231975_s_at |
| 204818_at | 211596_s_at | 221004_s_at | 232176_at |
| 204894_s_at | 211637_x_at | 221305_s_at | 232481_s_at |
| 204897_at | 211643_x_at | 221667_s_at | 233565_s_at |
| 204931_at | 211645_x_at | 221747 at | 234987_at |
| 204938_s_at | 211671_s_at | 221748_s_at | 235146_at |
| 204939_s_at | 211696_x_at | 221841_s_at | 235766_x_at |
| 204940_at | 211699 x_at | 221874_at | 235849_at |
| 204955_at | 211745_x_at | 221896_s_at | 235976_at |
| 205097_at | 211798_x_at | 222513_s_at | 236300_at |
| 205112_at | 211848_s_at | 222717_at | 236313_at |
| 205259_at | 211889_x_at | 223235_s_at | 236894_at |
| 205267_at | 211964_at | 223343_at | 237521_x_at |
| 205403_at | 211985_s_at | 223395_at | 238750_at |
| 205412_at | 211990_at | 223484_at | 241994_at |
| 205433_at | 211991_s_at | 223551_at | 242317_at |
| 205464_at | 212077_at | 223597_at | 242447_at |
| 205480_s_at | 212097_at | 223623_at | 242601_at |
| 205547_s_at | 212136_at | 224352_s_at | 243278_at |
| 205554_s_at | 212158_at | 224412_s_at | |
| ABCA8 | EGR1 | LOC25845 | PPID |
| ABI3BP | ELOVL5 | LOC283666 | PPP1R12B |
| ACAT1 | EMP1 | LOC285382 | PPP1R14A |
| ACTA2 | EMP3 | LOC339562 | PRDX6 |
| ACTG2 | ENTPD5 | LOC387763 | PRIMA1 |
| ADH1B | EPB41L3 | LOC399959 | PRKACB |
| ADH1C | ETHE1 | LOC572558 | PRKAR2B |
| AKAP12 | F13A1 | LOC63928 | PRNP |
| AKR1B10 | FABP1 | LOC652128 | PTGER4 |
| ALDH1A1 | FABP4 | LOC652745 | PTGIS |
| ANK2 | FAM107A | LRIG1 | PTRF |
| ANK3 | FAM129A | LRRC19 | QKI |
| ANKRD25 | FAM46C | MAB21L2 | RAB27A |
| ANPEP | FBLN1 | MAFB | RAB31 |
| ANTXR1 | FCGBP | MAG1 | RBMS1 |
| AOC3 | FGFR2 | MALL | RDX |
| AP1S2 | FHL1 | MAOA | RELL1 |
| APOE | FILIP1L | MAP1B | RGS1 |
| AQP1 | FKBP5 | MEIS1 | RGS2 |
| AQP8 | FLNA | MEP1A | RGS5 |
| ARL14 | FNBP1 | METTL7A | SCARA5 |
| ATP2B4 | FOSB | MFSD4 | SCNN1B |
| ATP8A1 | FOXF1 | MGC14376 | SDC2 |
| AXL | FOXF2 | MIER3 | SDCBP2 |
| BCHE | FOXP2 | MLPH | SDPR |
| BEST2 | FXYD6 | MMP2 | SELENBP1 |
| C10orf56 | GALNAC4S-6ST | MPEG1 | SELM |
| C10orf99 | | MPEG1 | SGCE |
| C15orf48 | GAS1 | MRC1 | SGK |
| C1orf115 | GCNT2 | MRGPRF | SI |
| C20orf118 | GCNT3 | MS4A12 | SLC20A1 |
| C2orf40 | GJA1 | MS4A4A | SLC26A2 |
| C4orF34 | GNG2 | MS4A7 | SLC26A3 |
| C6orf105 | GPA33 | MSN | SLC4A4 |
| C6orf204 | GPM6B | MSRB3 | SLITRK6 |
| C7 | GUCY1A3 | MT1A | SMOC2 |
| C9orf19 | HBA1 | MT1E | SMTN |
| CA1 | HBA2 | MT1F | SORBS 1 |
| CA2 | HBB | MT1G | SRI |
| CA4 | HCLS1 | MT1H | SRPX |
| CALD1 | HHLA2 | MT1M | ST6GALNAC1 |
| CALM1 | HIGD1A | MT1X | STMN2 |
| CAPN9 | HLA-C | MT2A | STOM |
| CAV1 | HLA-DPA1 | MUC12 | SULT1A1 |
| CCDC80 | HLA-DPB1 | MUCDHL | SYNPO2 |
| CCL28 | HLA-DQA1 | MXD1 | TAGLN |
| CD14 | HLA-DRB1 | MYL9 | TBC1D9 |
| CD163 | HMGCS2 | MYLK | TCEAL7 |
| CD36 | HPGD | NEXN | TCF21 |
| CDKN2B | HSD11B2 | NID1 | TGFB1I1 |
| CEACAM1 | HSD17B2 | NR3C1 | TMEM47 |
| CEACAM7 | HSPA2 | NR3C2 | TNS1 |
| CES2 | HSPB6 | OSTbeta | TP53INP2 |
| CFL2 | HSPB8 | P2RY1 | TPM2 |
| CHGA | ID1 | P2RY14 | TRPM6 |
| CITED2 | IDH3A | PAG1 | TSC22D3 |
| CKB | IGHA1 | PALLD | TSPAN1 |
| CLCA1 | IGHM | PALM2- | TSPAN7 |
| CLCA4 | IGKV1D-13 | AKAP2 | TTRAP |
| CLDN8 | IGL@ | PAPSS2 | TUBB6 |
| CLIC5 | IGLJ3 | PBLD | TYROBP |
| CMBL | IL1R2 | PCK1 | UGDH |
| CNN1 | IQGAP2 | PDCD4 | UGP2 |
| CNTN3 | ITLN1 | PDE9A | UGT1A1 |
| COL4A2 | ITM2A | PDGFRA | UGT1A3 |
| COL6A2 | ITM2C | PDK4 | UGT1A6 |
| COX7A1 | KCNMA1 | PDLIM3 | UGT1A8 |
| CRYAB | KCNMB1 | PGM5 | UGT1A9 |
| CTSE | KCTD12 | PIGR | UGT2A3 |
| CYP3A5 | KIAA0828 | PKIB | UGT2B15 |
| CYP3A5P2 | KIAA1324 | PLAC8 | UGT2B17 |
| DDR2 | KLF4 | PLCE1 | VIM |
| DES | KLHL5 | PLEKHC1 | VSIG2 |
| DMN | KRT20 | PLN | WDR51B |
| DNASE1L3 | L1TD1 | PLOD2 | WWTR1 |
| DSCR1 | LGALS1 | PMP22 | XDH |
| DUSP1 | LGALS2 | PNMA1 | XLKD1 |
| DUSP5 | LIFR | POU2AF1 | ZSCAN18 |
| EDG2 | LMOD1 | PPAP2A | LOC253012 |
| PPAP2B | | | |

Reference to "nucleic acid molecule" should be understood as a reference to both deoxyribonucleic acid molecules and ribonucleic acid molecules and fragments thereof. The present invention therefore extends to both directly screening for mRNA levels in a biological sample or screening for the complementary cDNA which has been reverse-transcribed from an mRNA population of interest. It is well within the skill of the person of skill in the art to design methodology directed to screening for either DNA or RNA. As detailed above, the method of the present invention also extends to screening for the protein product translated from the subject mRNA or the genomic DNA itself.

In one preferred embodiment, the level of gene expression is measured by reference to genes which encode a protein product and, more particularly, said level of expression is measured at the protein level. Accordingly, to the extent that the present invention is directed to screening for markers which are detailed in the preceding table, said screening is preferably directed to the encoded protein.

In another particularly preferred embodiment, said gene expression is assessed by analysing genomic DNA methylation. In another embodiment, expression is assessed by the association of DNA with chromatin proteins carrying repressive modifications, for example, methylation of lysines 9 or 27 of histone H3.

As detailed hereinbefore, it should be understood that although the present invention is exemplified with respect to the detection of expressed nucleic acid molecules (e.g. mRNA), it also encompasses methods of detection based on screening for the protein product of the subject genes. The present invention should also be understood to encompass methods of detection based on identifying both proteins and/or nucleic acid molecules in one or more biological samples. This may be of particular significance to the extent that some of the neoplastic markers of interest may correspond to genes or gene fragments which do not encode a protein product. Accordingly, to the extent that this occurs it would not be possible to test for a protein and the subject marker would have to be assessed on the basis of transcription expression profiles or changes to genomic DNA.

The term "protein" should be understood to encompass peptides, polypeptides and proteins (including protein fragments). The protein may be glycosylated or unglycosylated and/or may contain a range of other molecules fused, linked, bound or otherwise associated to the protein such as amino acids, lipids, carbohydrates or other peptides, polypeptides or proteins. Reference herein to a "protein" includes a protein comprising a sequence of amino acids as well as a protein associated with other molecules such as amino acids, lipids, carbohydrates or other peptides, polypeptides or proteins.

The proteins encoded by the neoplastic markers of the present invention may be in multimeric form meaning that two or more molecules are associated together. Where the same protein molecules are associated together, the complex is a homomultimer. An example of a homomultimer is a homodimer. Where at least one marker protein is associated with at least one non-marker protein, then the complex is a heteromultimer such as a heterodimer.

Reference to a "fragment" should be understood as a reference to a portion of the subject nucleic acid molecule or protein. This is particularly relevant with respect to screening for modulated RNA levels in stool samples since the subject RNA is likely to have been degraded or otherwise fragmented due to the environment of the gut. One may therefore actually be detecting fragments of the subject RNA molecule, which fragments are identified by virtue of the use of a suitably specific probe.

Reference to the "onset" of a neoplasm, such as adenoma or adenocarcinoma, should be understood as a reference to one or more cells of that individual exhibiting dysplasia. In this regard, the adenoma or adenocarcinoma may be well developed in that a mass of dysplastic cells has developed. Alternatively, the adenoma or adenocarcinoma may be at a very early stage in that only relatively few abnormal cell divisions have occurred at the time of diagnosis. The present invention also extends to the assessment of an individual's predisposition to the development of a neoplasm, such as an adenoma or adenocarcinoma. Without limiting the present invention in any way, changed levels of the neoplastic markers may be indicative of that individual's predisposition to developing a neoplasia, such as the future development of an adenoma or adenocarcinoma or another adenoma or adenocarcinoma.

In yet another related aspect of the present invention, markers have been identified which enable the characterisation of neoplastic tissue of the large intestine in terms of whether it is an adenoma or a cancer. This development now provides a simple yet accurate means of characterising tissue using means other than the traditional methods which are currently utilised.

According to this aspect of the present invention, there is provided a method of characterising a neoplastic cell or cellular population, which cell or cellular population is derived from the large intestine of an individual, said method comprising assessing the level of expression of one or more genes or transcripts selected from:

| **(i)** | the gene, genes or transcripts detected by Affymetrix probeset IDs: | | | |
|---|---|---|---|---|
| | 200600_at | 202859_x_at | 210764_s_at | 219087_at |
| | 200665_s_at | 202878_s_at | 210809_s_at | 221011_s_at |
| | 200832_s_at | 202917_s_at | 211161_s_at | 221729_at |
| | 200974_at | 202998_s_at | 211571_s_at | 221730_at |
| | 200986_at | 203083_at | 211719_x_at | 221731_x_at |
| | 201058_s_at | 203325_s_at | 211813_x_at | 37892_at |
| | 201069_at | 203382_s_at | 211896_s_at | 223122_s_at |
| | 201105_at | 203477_at | 211959_at | 223235_s_at |
| | 201141_at | 203570_at | 211964_at | 224560_at |
| | 201147_s_at | 203645_s_at | 211966_at | 224694_at |
| | 201150_s_at | 203878_s_at | 211980_at | 224724_at |
| | 201162_at | 204006_s_at | 211981_at | 225664_at |
| | 201163_s_at | 204051_s_at | 212077_at | 225681_at |
| | 201185_at | 204122_at | 212344_at | 225710_at |
| | 201261_x_at | 204320_at | 212353_at | 225799_at |
| | 201289_at | 204475_at | 212354_at | 226237_at |
| | 201426_s_at | 204620_s_at | 212464_s_at | 226311_at |
| | 201438_at | 205479_s_at | 212488_at | 226694_at |
| | 201616_s_at | 205547_s_at | 212489_at | 226777_at |
| | 201645_at | 205828_at | 212667_at | 226930_at |
| | 201667_at | 207173_x_at | 213125_at | 227099_s_at |
| | 201744_s_at | 207191_s_at | 213428_s_at | 227140_at |
| | 201792_at | 208747_s_at | 213524_s_at | 227566_at |
| | 201842_s_at | 208782_at | 213869_x_at | 229218_at |
| | 201852_x_at | 208788_at | 213905_x_at | 229802_at |
| | 201859_at | 208850_s_at | 214247_s_at | 231579_s_at |
| | 201893_x_at | 208851_s_at | 215049_x_at | 231766_s_at |
| | 202237_at | 209101_at | 215076_s_at | 231879_at |
| | 202238_s_at | 209156_s_at | 215646_s_at | 232458_at |
| | 202283_at | 209218_at | 216442_x_at | 233555_s_at |
| | 202291_s_at | 209395_at | 217430_x_at | 234994_at; and/or |
| | 202310_s_at | 209396_s_at | 217762_s_at | |
| | 202311_s_at | 209596_at | 217763_s_at | |
| | 202403_s_at | 209875_s_at | 217764_s_at | |
| | 202404_s_at | 209955_s_at | 218468_s_at | |
| | 202450_s_at | 210095_s_at | 218469_at | |
| | 202620_s_at | 210495_x_at | 218559_s_at | |
| | 202766_s_at | 210511_s_at | 218638_s_at | |
| **(ii)** | COL1A2 | INHBA | FAP | IGFBP3 |
| | CTHRC1 | COL6A2 | VIM | SERPINF1 |
| | FN1 | ANTXR1 | TIMP2 | ISLR |
| | POSTN | GPNMB | SCD | HNT |
| | SPP1 | BGN | TIMP3 | COL5A1 |
| | MMP1 | TAGLN | AEBP1 | OLFML2B |
| | SPARC | COL4A1 | GJA1 | KIAA1913 |
| | LUM | RAB31 | NNMT | PALM2-AKAP2 |
| | GREM1 | LGALS1 | COL1A1 | SERPING1 |
| | IL8 | ELOVL5 | SULF2 | TYROBP |
| | IGFBP5 | MGP | COL6A1 | ACTA2 |
| | SFRP2 | MMP2 | SPON2 | COL3A1 |
| | SULF1 | LOXL2 | CTSK | PLOD2 |
| | ASPN | MYL9 | MXRA5 | MMP11 |
| | COL6A3 | DCN | C1S | CD163 |
| | COL8A1 | CALD1 | DKK3 | FCGR3B |
| | COL12A1 | FBN1 | SRGN | PLAU |
| | COL5A2 | MMP3 | LBH | MAFB |
| | CDH11 | IGFBP7 | CTGF | LOC541471 |
| | THBS2 | FSTL1 | TNC | LOC387763 |
| | COL15A1 | COL4A2 | G0S2 | CHI3L1 |
| | COL11A1 | VCAN | SQLE | THY1 |
| | S100A8 | SMOC2 | EFEMP1 | LOXL1 |
| | FNDC1 | HTRA1 | APOE | CD93 |
| | SFRP4 | CYR61 | MSN | |

in said cell or cellular population wherein a lower level of expression of the genes of group (i) and/or group (ii) relative to a gastrointestinal cancer control level is indicative of an adenoma cell or a cell predisposed to the onset of an adenoma state.

In another aspect there is provided a method of characterising a neoplastic cell or cellular population, which cell or cellular population is derived from the large intestine of an individual, said method comprising assessing the level of expression of one or more genes or transcripts selected from:

| **(i)** | the gene, genes or transcripts detected by Affymetrix probeset IDs: | | | |
|---|---|---|---|---|
| | 200884_at | 214234_s_at | 226248_s_at | |
| | 203240_at | 214235_at | 226302_at | |
| | 203963_at | 214433_s_at | 227676_at | |
| | 204508_s_at | 215125_s_at | 227719_at | |
| | 204607_at | 215867_x_at | 227725_at | |
| | 204811_s_at | 217109_at | 228232_s_at | |
| | 204895_x_at | 217110_s_at | 229070_at | |
| | 204897_at | 218211_s_at | 231832_at | |
| | 205259_at | 219543_at | 232176_at | |
| | 205765_at | 219955_at | 232481_s_at | |
| | 205927_s_at | 221841_s_at | 235976_at | |
| | 208063_s_at | 221874_at | 236894_at | |
| | 208937_s_at | 223969_s_at | 237521_x_at | |
| | 210107_at | 223970_at | 242601_at; and/or | |
| | 213106_at | | | |
| **(ii)** | CLCA1 | ST6GALNAC1 | UGT1A1 | CYP3A5P2 |
| | FCGBP | ID1 | SELENBP1 | CAPN9 |
| | HMGCS2 | CYP3A5 | PTGER4 | NR3C2 |
| | RETNLB | CTSE | MLPH | PBLD |
| | L1TD1 | C6orf105 | KIAA1324 | CA12 |
| | SLITRK6 | CKB | ATP8B1 | WDR51B |
| | VSIG2 | ATP8A1 | CACNA2D2 | FAM3D |
| | LOC253012 | MUC4 | KLF4 | |

in a biological sample from said individual wherein a lower level of expression of the genes or transcripts of group (i) and/or group (ii) relative to a gastrointestinal adenoma control level is indicative of a cancer or a cell predisposed to the onset of a cancerous state.

Preferably, said gastrointestinal tissue is colorectal tissue.

In one embodiment, said expression is assessed by screening for DNA changes which impact on methylation, in particular hypermethylation. In another embodiment, expression is assessed by the association of DNA with chromatin proteins carrying repressive modifications, for example, methylation of lysines 9 or 27 of histone H3.

Reference to an "adenoma control level" or "cancer control level" should be understood as a reference to the level of said gene expression in a population of adenoma or cancer gastrointestinal cells, respectively. As discussed hereinbefore in relation to "normal levels", the subject level may be a discrete level or a range of levels. Accordingly, the definition of "adenoma control level" or "cancer control level" should be understood to have a corresponding definition to "normal level", albeit in the context of the expression of genes by a neoplastic population of large intestine cells.

In terms of this aspect of the present invention, the subject analysis is performed on a population of neoplastic cells. These cells may be derived in any manner, such as sloughed off neoplastic cells which have been collected via an enema wash or from a gastrointestinal sample, such as a stool sample. Alternatively, the subject cells may have been obtained via a biopsy or other surgical technique.

Without limiting this aspect of the invention in any way, several of the markers of this aspect of the present invention have been determined to be expressed at particularly significant levels below those of neoplastic cells. For example, decreased expression levels of 3 to 9 fold have been observed in respect of the following markers which are indicative of gastrointestinal adenomas, when assessed by the method herein exemplified.

| **Fold Decrease** | **Gene, genes or transcripts detected by Affymetrix Probe No:** | **Gene** |
|---|---|---|
| 9 | 202404_s_at | COL1A2 |
| 8 | 225681_at | CTHRC1 |
| 7 | 212464_s_at | FN1 |
| | 210809_s_at | POSTN |
| 6 | 209875_s_at | SPP1 |
| 5 | 221740_at | MMP1 |
| | 204475 at | |
| 4 | 200665_s_at | SPARC |
| | 201744_s_at | LUM |
| | 218468_s_at | GREM1 |
| | 202859_x_at | IL8 |
| | 211959_at | IGFBP5 |
| 3 | 223122_s_at | SFRP2 |
| | 212353_at | SULF1 |
| | 219087_at | ASPN |
| | 201438_at | COL6A3 |
| | 226237_at | COL8A1 |
| | 225664_at | COL12A1 |
| | 221730_at | COL5A2 |
| | 207173_x_at | CDH11 |
| | 203083_at | THBS2 |
| | 203477_at | COL15A1 |
| | 37892_at | COL11A1 |
| | 202917_s_at | S100A8 |
| | 226930_at | FNDC1 |
| | 204051_s_at | SFRP4 |
| | 210511_s_at | INHBA |
| | 209156_s_at | COL6A2 |
| | 224694_at | ANTXR1 |
| | 201141_at | GPNMB |
| | 213905_x_at | BGN |
| | 205547_s_at | TAGLN |

In another example, decreased expression levels of between 3 to 5 fold have been observed in respect of the following markers which are indicative of gastrointestinal cancers, when assessed by the method herein exemplified.

| **Fold Decrease** | **Gene, genes or transcripts detected by Affymetrix Probe No:** | **Gene** |
|---|---|---|
| 5 | 210107_at | CLCA1 |
| 3 | 203240_at | FCGBP |
| | 204607 at | HMGCS2 |
| | 223969_s_at | RETNLB |
| | 219955_at | L1TD1 |
| | 232481_s_at | SLITRK6 |
| | 228232_s_at | VSIG2 |
| | 242601_at | LOC253012 |
| | 227725_at | ST6GALNAC1 |

According to this embodiment, there is therefore provided a method of characterising a neoplastic cell or cellular population, which cell or cellular population is derived from the large intestine of an individual, said method comprising assessing the level of expression of one or more genes or transcripts selected from:
(i) the gene or genes detected by Affymetrix probeset IDs: 202404_s_at, 212464_s_at, 210809_s_a, 225681_at;and/or
(ii) COLIA2, CTHRC1, FN1, POSTN
   in a biological sample from said individual wherein a lower level of expression of the genes or transcripts of group (i) and/or group (ii) relative to a gastrointestinal cancer control level is indicative of an adenoma cell or a cell predisposed to the onset of an adenoma state.

In another embodiment, there is provided a method of characterising a neoplastic cell or cellular population, which cell or cellular population is derived from the large intestine of an individual, said method comprising assessing the level of expression of one or more genes selected from:

| **(i)** | the gene, genes or transcripts detected by Affymetrix probeset IDs: | | |
|---|---|---|---|
| | 209875_s_at | 227140_at | 204475_at; and/or |
| **(ii)** | SPP1 | MMP1 | |

in a biological sample from said individual wherein a lower level of expression of the genes or transcripts of group (i) and/or group (ii) relative to a gastrointestinal cancer control level is indicative of an adenoma cell or a cell predisposed to the onset of an adenoma state.

Preferably, said gastrointestinal tissue is colorectal tissue.

Still more preferably, said biological sample is a tissue sample.

In another preferred embodiment the present invention is directed to a method of characterising a cell or cellular population, which cell or cellular population is derived from the large intestine of an individual, said method comprising assessing the level of expression of one or more genes or transcripts selected from:

| **(i)** | the gene, genes or transcripts detected by Affymetrix probeset IDs: | | | |
|---|---|---|---|---|
| | 200665_s_at | 212353_at | 203083_at | 210511_s_at |
| | 201744_s_at | 219087_at | 203477_at | 209156_s_at |
| | 218468_s_at | 226237_at | 37892_at | 201438_at; and/or |
| | 202859_x_at | 225664_at | 202917_s_at | |
| | 211959_at | 221730_at | 226930_at | |
| | 223122_s_at | 207173_x_at | 204051_s_at | |
| **(ii)** | SPARC | ASPN | COL15A1 | ANTXR1 |
| | LUM | COL6A3 | COL11A1 | GPNMB |
| | GREM1 | COL8A1 | S100A8 | BGN |
| | IL8 | COL12A1 | FNDC1 | TAGLN |
| | IGFBP5 | COL5A2 | SFRP4 | |
| | SFRP2 | CDH11 | INHBA | |
| | SULF1 | THBS2 | COL6A2 | |

in a biological sample from said individual wherein a lower level of expression of the genes or transcripts of group (i) and/or (ii) relative to a gastrointestinal cancer control level is indicative of an adenoma or a cell predisposed to the onset of an adenoma state.

In yet another preferred embodiment the present invention is directed to a method of characterising a cell or cellular population, which cell or cellular population is derived from the large intestine of an individual, said method comprising assessing the level of expression of one or more genes or transcripts selected from:
(i) the gene, genes or transcripts detected by Affymetrix probeset IDs: 210107_at; and/or
(ii) CLCA1
in a biological sample from said individual wherein a lower level of expression of the genes or transcripts of group (i) and/or (ii) relative to a gastrointestinal adenoma control level is indicative of a cancer or a cell predisposed to the onset of a cancerous state.

In still yet another preferred embodiment the present invention is directed to a method of characterising a cell or cellular population, which cell or cellular population is derived from the large intestine of an individual, said method comprising assessing the level of expression of one or more genes or transcripts selected from:
(i) the gene, genes or transcripts detected by Affymetrix probeset IDs: 203240_at, 204607_at, 223969_s_at, 219955_at, 232481_s_at, 242601_at, 227725_at, 228232_s_at; and/or
(ii) FCGBP, HMGCS2, RETNLB, L1TD1, SLITRK6, VSIG2, LOC253012, ST6GALNAC1
in a biological sample from said individual wherein a lower level of expression of the genes or transcripts of group (i) and/or (ii) relative to a gastrointestinal adenoma control level is indicative of a cancer or a cell predisposed to the onset of a cancerous state.

Preferably, said gastrointestinal tissue is colorectal tissue.

Even more preferably, said biological sample is a tissue sample.

In still another related aspect it has been determined that a subset of the markers of this aspect of the present invention are useful as qualitative markers of neoplastic tissue characterisation in that these markers, if not detectable at levels substantially above background levels in neoplastic tissue are indicative of cancerous tissue.

According to this aspect, the present invention provides a method of characterising a neoplastic cell or cellular population, which cell or cellular population is derived from the large intestine of an individual, said method comprising assessing the level of expression of one or more genes or transcripts selected from:

| **(i)** | the gene or genes detected by Affymetrix probeset IDs: | | |
|---|---|---|---|
| | 235976_at | 236894_at | 237521; and/or |
| **(ii)** | SLITRK6 | L1TD1 | |

in a biological sample from said individual wherein expression of the genes or transcripts of group (i) and/or (ii) at a level which is not substantially greater than background neoplastic tissue levels is indicative of a cancer or a cell predisposed to the onset of a cancerous state.

Preferably, said gastrointestinal tissue is colorectal tissue.

Still more preferably, said biological sample is a tissue sample.

In a most preferred embodiment, the methods of the present invention are preferably directed to screening for proteins encoded by the markers of the present invention or changes to DNA methylation of genomic DNA. In another embodiment, expression is assessed by the association of DNA with chromatin proteins carrying repressive modifications, for example, methylation of lysines 9 or 27 of histone H3.

Although the preferred method is to detect the expression product or DNA changes of the neoplastic markers for the purpose of diagnosing neoplasia development or predisposition thereto, the detection of converse changes in the levels of said markers may be desired under certain circumstances, for example, to monitor the effectiveness of therapeutic or prophylactic treatment directed to modulating a neoplastic condition, such as adenoma or adenocarcinoma development. For example, where reduced expression of the subject markers indicates that an individual has developed a condition characterised by adenoma or adenocarcinoma development, for example, screening for an increase in the levels of these markers subsequently to the onset of a therapeutic regime may be utilised to indicate reversal or other form of improvement of the subject individual's condition.

The method of the present invention is therefore useful as a one off test or as an on-going monitor of those individuals thought to be at risk of neoplasia development or as a monitor of the effectiveness of therapeutic or prophylactic treatment regimes directed to inhibiting or otherwise slowing neoplasia development. In these situations, mapping the modulation of neoplastic marker expression levels in any one or more classes of biological samples is a valuable indicator of the status of an individual or the effectiveness of a therapeutic or prophylactic regime which is currently in use. Accordingly, the method of the present invention should be understood to extend to monitoring for increases or decreases in marker expression levels in an individual relative to their normal level (as hereinbefore defined), background control levels, cancer levels, adenoma levels or relative to one or more earlier marker expression levels determined from a biological sample of said individual.

Means of assessing the subject expressed neoplasm markers in a biological sample can be achieved by any suitable method, which would be well known to the person of skill in the art. To this end, it would be appreciated that to the extent that one is examining either a homogeneous cellular population (such as a tumour biopsy or a cellular population which has been enriched from a heterogeneous starting population) or a tissue section, one may utilise a wide range of techniques such as *in situ* hybridisation, assessment of expression profiles by microassays, immunoassays and the like (hereinafter described in more detail) to detect the absence of or downregulation of the level of expression of one or more markers of interest. However, to the extent that one is screening a heterogenous cellular population or a bodily fluid in which heterogeneous populations of cells are found, such as a blood sample, the absence of or reduction in level of expression of a particular marker may be undetectable due to the inherent expression of the marker by non-neoplastic cells which are present in the sample. That is, a decrease in the level of expression of a subgroup of cells may not be detectable. In this situation, a more appropriate mechanism of detecting a reduction in a neoplastic subpopulation of the expression levels of one or more markers of the present invention is via indirect means, such as the detection of epigenetic changes.

Without limiting the present invention to any one theory or mode of action, during development gene expression is regulated by processes that alter the availability of genes for expression in different cell lineages without any alteration in gene sequence, and these states can be inherited through a cell division - a process called epigenetic inheritance. Epigenetic inheritance is determined by a combination of DNA methylation (modification of cytosine to give 5-methyl cytosine, 5meC) and by modifications of the histone chromosomal proteins that package DNA. Thus methylation of DNA at CpG sites and modifications such as deacetylation of histone H3 on lysine 9, and methylation on lysine 9 or 27 are associated with inactive chromatin, while the converse state of a lack of DNA methylation, acetylation of lysine 9 of histone H3 is associated with open chromatin and active gene expression. In cancer, this epigenetic regulation of gene expression is frequently found to be disrupted (Esteller & Herman, 2000; Jones & Baylin, 2002). Genes such as tumour suppressor or metastasis suppressor genes are often found to be silenced by DNA methylation, while other genes may be hypomethylated and inappropriately expressed. Thus, among genes that show a decrease or loss of expression in cancer, this is often characterised by methylation of the promoter or regulatory region of the gene.

A variety of methods are available for detection of aberrantly methylated DNA of a specific gene, even in the presence of a large excess of normal DNA (Clark 2007). Thus, loss of expression of a gene which can be difficult to detect at the protein or RNA level except by immunohistochemistry can often be detected in tumour samples and in bodily fluids of cancer patients by the presence of hypermethylated DNA of the gene's promoter. Similarly DNA hypomethylation may be used for the detection of certain genes whose expression is elevated in cancer. Epigenetic alterations and chromatin changes in cancer are also evident in the altered association of modified histones with specific genes (Esteller, 2007); for example repressed genes are often found associated with histone H3 that is deacetylated and methylated on lysine 9. The use of antibodies targeted to altered histones allows for the isolation of DNA associated with particular chromatin states and its potential use in cancer diagnosis.

Other methods of detecting changes to gene expression levels, particularly where the subject biological sample is not contaminated with high numbers of non-neoplastic cells, include but are not limited to:
(i) *In vivo* detection.
   Molecular Imaging may be used following administration of imaging probes or reagents capable of disclosing altered expression of the markers in the intestinal tissues.
   Molecular imaging (Moore et al., BBA, 1402:239-249, 1988; Weissleder et al., Nature Medicine 6:351-355, 2000) is the *in vivo* imaging of molecular expression that correlates with the macro-features currently visualized using "classical" diagnostic imaging techniques such as X-Ray, computed tomography (CT), MRI, Positron Emission Tomography (PET) or endoscopy.
(ii) Detection of downregulation of RNA expression in the cells by Fluorescent *In Situ* Hybridization (FISH), or in extracts from the cells by technologies such as Quantitative Reverse Transcriptase Polymerase Chain Reaction (QRTPCR) or Flow cytometric qualification of competitive RT-PCR products (Wedemeyer et al., Clinical Chemistry 48:9 1398-1405, 2002).
(iii) Assessment of expression profiles of RNA, for example by array technologies (Alon et al., Proc. Natl. Acad Sci. USA: 96, 6745-6750, June 1999).

A "microarray" is a linear or multi-dimensional array of preferably discrete regions, each having a defined area, formed on the surface of a solid support. The density of the discrete regions on a microarray is determined by the total numbers of target polynucleotides to be detected on the surface of a single solid phase support. As used herein, a DNA microarray is an array of oligonucleotide probes placed onto a chip or other surfaces used to amplify or clone target polynucleotides. Since the position of each particular group of probes in the array is known, the identities of the target polynucleotides can be determined based on their binding to a particular position in the microarray.

Recent developments in DNA microarray technology make it possible to conduct a large scale assay of a plurality of target nucleic acid molecules on a single solid phase support. U.S. Pat. No. 5,837,832 (Chee et al.*)* and related patent applications describe immobilizing an array of oligonucleotide probes for hybridization and detection of specific nucleic acid sequences in a sample. Target polynucleotides of interest isolated from a tissue of interest are hybridized to the DNA chip and the specific sequences detected based on the target polynucleotides' preference and degree of hybridization at discrete probe locations. One important use of arrays is in the analysis of differential gene expression, where the profile of expression of genes in different cells or tissues, often a tissue of interest and a control tissue, is compared and any differences in gene expression among the respective tissues are identified. Such information is useful for the identification of the types of genes expressed in a particular tissue type and diagnosis of conditions based on the expression profile.

In one example, RNA from the sample of interest is subjected to reverse transcription to obtain labelled cDNA. See U.S. Pat. No. 6,410,229 (Lockhart et al.*)* The cDNA is then hybridized to oligonucleotides or cDNAs of known sequence arrayed on a chip or other surface in a known order. In another example, the RNA is isolated from a biological sample and hybridised to a chip on which are anchored cDNA probes. The location of the oligonucleotide to which the labelled cDNA hybridizes provides sequence information on the cDNA, while the amount of labelled hybridized RNA or cDNA provides an estimate of the relative representation of the RNA or cDNA of interest. See Schena, et al. Science 270:467-470 (1995). For example, use of a cDNA microarray to analyze gene expression patterns in human cancer is described by DeRisi, et al. (Nature Genetics 14:457-460 (1996)).

In a preferred embodiment, nucleic acid probes corresponding to the subject nucleic acids are made. The nucleic acid probes attached to the biochip are designed to be substantially complementary to the nucleic acids of the biological sample such that specific hybridization of the target sequence and the probes of the present invention occurs. This complementarity need not be perfect, in that there may be any number of base pair mismatches that will interfere with hybridization between the target sequence and the single stranded nucleic acids of the present invention. It is expected that the overall homology of the genes at the nucleotide level probably will be about 40% or greater, probably about 60% or greater, and even more probably about 80% or greater; and in addition that there will be corresponding contiguous sequences of about 8-12 nucleotides or longer. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions.

A nucleic acid probe is generally single stranded but can be partly single and partly double stranded. The strandedness of the probe is dictated by the structure, composition, and properties of the target sequence. In general, the oligonucleotide probes range from about 6, 8, 10, 12, 15, 20, 30 to about 100 bases long, with from about 10 to about 80 bases being preferred, and from about 15 to about 40 bases being particularly preferred. That is, generally entire genes are rarely used as probes. In some embodiments, much longer nucleic acids can be used, up to hundreds of bases. The probes are sufficiently specific to hybridize to a complementary template sequence under conditions known by those of skill in the art. The number of mismatches between the probe's sequences and their complementary template (target) sequences to which they hybridize during hybridization generally do not exceed 15%, usually do not exceed 10% and preferably do not exceed 5%, as-determined by BLAST (default settings).

Oligonucleotide probes can include the naturally-occurring heterocyclic bases normally found in nucleic acids (uracil, cytosine, thymine, adenine and guanine), as well as modified bases and base analogues. Any modified base or base analogue compatible with hybridization of the probe to a target sequence is useful in the practice of the invention. The sugar or glycoside portion of the probe can comprise deoxyribose, ribose, and/or modified forms of these sugars, such as, for example, 2'-O-alkyl ribose. In a preferred embodiment, the sugar moiety is 2'-deoxyribose; however, any sugar moiety that is compatible with the ability of the probe to hybridize to a target sequence can be used.

In one embodiment, the nucleoside units of the probe are linked by a phosphodiester backbone, as is well known in the art. In additional embodiments, internucleotide linkages can include any linkage known to one of skill in the art that is compatible with specific hybridization of the probe including, but not limited to phosphorothioate, methylphosphonate, sulfamate (e.g., U.S. Pat. No. 5,470,967) and polyamide (i.e., peptide nucleic acids). Peptide nucleic acids are described in Nielsen et al. (1991) Science 254: 1497-1500, U.S. Pat. No. 5,714,331, and Nielsen (1999) Curr. Opin. Biotechnol. 10:71-75.

In certain embodiments, the probe can be a chimeric molecule; i.e., can comprise more than one type of base or sugar subunit, and/or the linkages can be of more than one type within the same primer. The probe can comprise a moiety to facilitate hybridization to its target sequence, as are known in the art, for example, intercalators and/or minor groove binders. Variations of the bases, sugars, and internucleoside backbone, as well as the presence of any pendant group on the probe, will be compatible with the ability of the probe to bind, in a sequence-specific fashion, with its target sequence. A large number of structural modifications, are possible within these bounds. Advantageously, the probes according to the present invention may have structural characteristics such that they allow the signal amplification, such structural characteristics being, for example, branched DNA probes as those described by Urdea et al. (Nucleic Acids Symp. Ser., 24:197-200 (1991)) or in the European Patent No. EP-0225,807. Moreover, synthetic methods for preparing the various heterocyclic bases, sugars, nucleosides and nucleotides that form the probe, and preparation of oligonucleotides of specific predetermined sequence, are well-developed and known in the art. A preferred method for oligonucleotide synthesis incorporates the teaching of U.S. Pat. No. 5,419,966.

Multiple probes may be designed for a particular target nucleic acid to account for polymorphism and/or secondary structure in the target nucleic acid, redundancy of data and the like. In some embodiments, where more than one probe per sequence is used, either overlapping probes or probes to different sections of a single target gene are used. That is, two, three, four or more probes, are used to build in a redundancy for a particular target. The probes can be overlapping (i.e. have some sequence in common), or are specific for distinct sequences of a gene. When multiple target polynucleotides are to be detected according to the present invention, each probe or probe group corresponding to a particular target polynucleotide is situated in a discrete area of the microarray.

Probes may be in solution, such as in wells or on the surface of a micro-array, or attached to a solid support. Examples of solid support materials that can be used include a plastic, a ceramic, a metal, a resin, a gel and a membrane. Useful types of solid supports include plates, beads, magnetic material, microbeads, hybridization chips, membranes, crystals, ceramics and self-assembling monolayers. One example comprises a two-dimensional or three-dimensional matrix, such as a gel or hybridization chip with multiple probe binding sites (Pevzner et al., J. Biomol. Struc. & Dyn. 9:399-410, 1991; Maskos and Southern, Nuc. Acids Res. 20:1679-84, 1992). Hybridization chips can be used to construct very large probe arrays that are subsequently hybridized with a target nucleic acid. Analysis of the hybridization pattern of the

chip can assist in the identification of the target nucleotide sequence. Patterns can be manually or computer analyzed, but it is clear that positional sequencing by hybridization lends itself to computer analysis and automation. In another example, one may use an Affymetrix chip on a solid phase structural support in combination with a fluorescent bead based approach. In yet another example, one may utilise a cDNA microarray. In this regard, the oligonucleotides described by Lockkart *et al* (i.e. Affymetrix synthesis probes *in situ* on the solid phase) are particularly preferred, that is, photolithography.

As will be appreciated by those in the art, nucleic acids can be attached or immobilized to a solid support in a wide variety of ways. By "immobilized" herein is meant the association or binding between the nucleic acid probe and the solid support is sufficient to be stable under the conditions of binding, washing, analysis, and removal. The binding can be covalent or non-covalent. By "non-covalent binding" and grammatical equivalents herein is meant one or more of either electrostatic, hydrophilic, and hydrophobic interactions. Included in non-covalent binding is the covalent attachment of a molecule, such as streptavidin, to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.

Nucleic acid probes may be attached to the solid support by covalent binding such as by conjugation with a coupling agent or by covalent or non-covalent binding such as electrostatic interactions, hydrogen bonds or antibody-antigen coupling, or by combinations thereof. Typical coupling agents include biotin/avidin, biotin/streptavidin, Staphylococcus aureus protein A/IgG antibody F_{c} fragment, and streptavidin/protein A chimeras (T. Sano and C. R. Cantor, Bio/Technology 9:1378-81 (1991)), or derivatives or combinations of these agents. Nucleic acids may be attached to the solid support by a photocleavable bond, an electrostatic bond, a disulfide bond, a peptide bond, a diester bond or a combination of these sorts of bonds. The array may also be attached to the solid support by a selectively releasable bond such as 4,4'-dimethoxytrityl or its derivative. Derivatives which have been found to be useful include 3 or 4 [bis-(4-methoxyphenyl)]-methyl-benzoic acid, N-succinimidyl-3 or 4 [bis-(4-methoxyphenyl)]-methyl-benzoic acid, N-succinimidyl-3 or 4 [bis-(4-methoxyphenyl)]-hydroxymethyl-benzoic acid, N-succinimidyl-3 or 4 [bis-(4-methoxyphenyl)]-chloromethyl-benzoic acid, and salts of these acids.

In general, the probes are attached to the biochip in a wide variety of ways, as will be appreciated by those in the art. As described herein, the nucleic acids can either be synthesized first, with subsequent attachment to the biochip, or can be directly synthesized on the biochip.

The biochip comprises a suitable solid substrate. By "substrate" or "solid support" or other grammatical equivalents herein is meant any material that can be modified to contain discrete individual sites appropriate for the attachment or association of the nucleic acid probes and is amenable to at least one detection method. The solid phase support of the present invention can be of any solid materials and structures suitable for supporting nucleotide hybridization and synthesis. Preferably, the solid phase support comprises at least one substantially rigid surface on which the primers can be immobilized and the reverse transcriptase reaction performed. The substrates with which the polynucleotide microarray elements are stably associated and may be fabricated from a variety of materials, including plastics, ceramics, metals, acrylamide, cellulose, nitrocellulose, glass, polystyrene, polyethylene vinyl acetate, polypropylene, polymethacrylate, polyethylene, polyethylene oxide, polysilicates, polycarbonates, Teflon, fluorocarbons, nylon, silicon rubber, polyanhydrides, polyglycolic acid, polylactic acid, polyorthoesters, polypropylfumerate, collagen, glycosaminoglycans, and polyamino acids. Substrates may be two-dimensional or three-dimensional in form, such as gels, membranes, thin films, glasses, plates, cylinders, beads, magnetic beads, optical fibers, woven fibers, etc. A preferred form of array is a three-dimensional array. A preferred three-dimensional array is a collection of tagged beads. Each tagged bead has different primers attached to it. Tags are detectable by signalling means such as color (Luminex, Illumina) and electromagnetic field (Pharmaseq) and signals on tagged beads can even be remotely detected (e.g., using optical fibers). The size of the solid support can be any of the standard microarray sizes, useful for DNA microarray technology, and the size may be tailored to fit the particular machine being used to conduct a reaction of the invention. In general, the substrates allow optical detection and do not appreciably fluoresce.

In one embodiment, the surface of the biochip and the probe may be derivatized with chemical functional groups for subsequent attachment of the two. Thus, for example, the biochip is derivatized with a chemical functional group including, but not limited to, amino groups, carboxy groups, oxo groups and thiol groups, with amino groups being particularly preferred. Using these functional groups, the probes can be attached using functional groups on the probes. For example, nucleic acids containing amino groups can be attached to surfaces comprising amino groups, for example using linkers as are known in the art; for example, homo-or hetero-bifunctional linkers as are well known. In addition, in some cases, additional linkers, such as alkyl groups (including substituted and heteroalkyl groups) may be used.

In this embodiment, the oligonucleotides are synthesized as is known in the art, and then attached to the surface of the solid support. As will be appreciated by those skilled in the art, either the 5' or 3' terminus may be attached to the solid support, or attachment may be via an internal nucleoside. In an additional embodiment, the immobilization to the solid support may be very strong, yet non-covalent. For example, biotinylated oligonucleotides can be made, which bind to surfaces covalently coated with streptavidin, resulting in attachment.

The arrays may be produced according to any convenient methodology, such as preforming the polynucleotide microarray elements and then stably associating them with the surface. Alternatively, the oligonucleotides may be synthesized on the surface, as is known in the art. A number of different array configurations and methods for their production are known to those of skill in the art and disclosed in WO 95/25116 and WO 95/35505 (photolithographic techniques), U.S. Pat. No. 5,445,934 (in situ synthesis by photolithography), U.S. Pat. No. 5,384,261 (in situ synthesis by mechanically directed flow paths); and U.S. Pat. No. 5,700,637 (synthesis by spotting, printing or coupling); the disclosure of which are herein incorporated in their entirety by reference. Another method for coupling DNA to beads uses specific ligands attached to the end of the DNA to link to ligand-binding molecules attached to a bead. Possible ligand-binding partner pairs include biotin-avidin/streptavidin, or various antibody/antigen pairs such as digoxygenin-antidigoxygenin antibody (Smith et al., Science 258:1122-1126 (1992)). Covalent chemical attachment of DNA to the support can be accomplished by using standard coupling agents to link the 5'-phosphate on the DNA to coated microspheres through a phosphoamidate bond. Methods for immobilization of oligonucleotides to solid-state substrates are well established. See Pease et al., Proc. Natl. Acad. Sci. USA 91(11):5022-5026 (1994). A preferred method of attaching oligonucleotides to solid-state substrates is described by Guo et al., Nucleic Acids Res. 22:5456-5465 (1994). Immobilization can be accomplished either by in situ DNA synthesis (Maskos and Southern, *supra)* or by covalent attachment of chemically synthesized oligonucleotides (Guo *et al., supra)* in combination with robotic arraying technologies.

In addition to the solid-phase technology represented by biochip arrays, gene expression can also be quantified using liquid-phase arrays. One such system is kinetic polymerase chain reaction (PCR). Kinetic PCR allows for the simultaneous amplification and quantification of specific nucleic acid sequences. The specificity is derived from synthetic oligonucleotide primers designed to preferentially adhere to single-stranded nucleic acid sequences bracketing the target site. This pair of oligonucleotide primers form specific, non-covalently bound complexes on each strand of the target sequence. These complexes facilitate in vitro transcription of double-stranded DNA in opposite orientations. Temperature cycling of the reaction mixture creates a continuous cycle of primer binding, transcription, and re-melting of the nucleic acid to individual strands. The result is an exponential increase of the target dsDNA product. This product can be quantified in real time either through the use of an intercalating dye or a sequence specific probe. SYBR(r) Green 1, is an example of an intercalating dye, that preferentially binds to dsDNA resulting in a concomitant increase in the fluorescent signal. Sequence specific probes, such as used with TaqMan technology, consist of a fluorochrome and a quenching molecule covalently bound to opposite ends of an oligonucleotide. The probe is designed to selectively bind the target DNA sequence between the two primers. When the DNA strands are synthesized during the PCR reaction, the fluorochrome is cleaved from the probe by the exonuclease activity of the polymerase resulting in signal dequenching. The probe signalling method can be more specific than the intercalating dye method, but in each case, signal strength is proportional to the dsDNA product produced. Each type of quantification method can be used in multi-well liquid phase arrays with each well representing primers and/or probes specific to nucleic acid sequences of interest. When used with messenger RNA preparations of tissues or cell lines, an array of probe/primer reactions can simultaneously quantify the expression of multiple gene products of interest. See Germer et al., Genome Res. 10:258-266 (2000); Heid et al., Genome Res. 6:986-994 (1996).

### (iv) Measurement of altered neoplastic marker protein levels in cell extracts, for example by immunoassay.

Testing for proteinaceous neoplastic marker expression product in a biological sample can be performed by any one of a number of suitable methods which are well known to those skilled in the art. Examples of suitable methods include, but are not limited to, antibody screening of tissue sections, biopsy specimens or bodily fluid samples.

To the extent that antibody based methods of diagnosis are used, the presence of the marker protein may be determined in a number of ways such as by Western blotting, ELISA or flow cytometry procedures. These, of course, include both single-site and two-site or "sandwich" assays of the non-competitive types, as well as in the traditional competitive binding assays. These assays also include direct binding of a labelled antibody to a target.

Sandwich assays are a useful and commonly used assay. A number of variations of the sandwich assay technique exist, and all are intended to be encompassed by the present invention. Briefly, in a typical forward assay, an unlabelled antibody is immobilized on a solid substrate and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample. Variations on the forward assay include a simultaneous assay, in which both sample and labelled antibody are added simultaneously to the bound antibody. These techniques are well known to those skilled in the art, including any minor variations as will be readily apparent.

In the typical forward sandwich assay, a first antibody having specificity for the marker or antigenic parts thereof, is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking, covalently binding or physically adsorbing, the polymer-antibody complex is washed in preparation for the test sample. An aliquot of the sample to be tested is then added to the solid phase complex and incubated for a period of time sufficient (e.g. 2-40 minutes) and under suitable conditions (e.g. 25°C) to allow binding of any subunit present in the antibody. Following the incubation period, the antibody subunit solid phase is washed and dried and incubated with a second antibody specific for a portion of the antigen. The second antibody is linked to a reporter molecule which is used to indicate the binding of the second antibody to the antigen.

An alternative method involves immobilizing the target molecules in the biological sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody hapten complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample. "Reporter molecule" also extends to use of cell agglutination or inhibition of agglutination such as red blood cells on latex beads, and the like.

Alternately, fluorescent compounds, such as fluorecein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope. As in the EIA, the fluorescent labelled antibody is allowed to bind to the first antibody-hapten complex. After washing off the unbound reagent, the remaining tertiary complex is then exposed to the light of the appropriate wavelength the fluorescence observed indicates the presence of the hapten of interest. Immunofluorescence and EIA techniques are both very well established in the art and are particularly preferred for the present method. However, other reporter molecules, such as radioisotope, chemiluminescent or bioluminescent molecules, may also be employed.
(v) Determining altered expression of protein neoplastic markers on the cell surface, for example by immunohistochemistry.
(vi) Determining altered protein expression based on any suitable functional test, enzymatic test or
immunological test in addition to those detailed in points (iv) and (v) above.

A person of ordinary skill in the art could determine, as a matter of routine procedure, the appropriateness of applying a given method to a particular type of biological sample.

Without limiting the present invention in any way, and as detailed above, gene expression levels can be measured by a variety of methods known in the art. For example, gene transcription or translation products can be measured. Gene transcription products, i.e., RNA, can be measured, for example, by hybridization assays, run-off assays., Northern blots, or other methods known in the art. Hybridization assays generally involve the use of oligonucleotide probes that hybridize to the single-stranded RNA transcription products. Thus, the oligonucleotide probes are complementary to the transcribed RNA expression product. Typically, a sequence-specific probe can be directed to hybridize to RNA or cDNA. A "nucleic acid probe", as used herein, can be a DNA probe or an RNA probe that hybridizes to a complementary sequence. One of skill in the art would know how to design such a probe such that sequence specific hybridization will occur. One of skill in the art will further know how to quantify the amount of sequence specific hybridization as a measure of the amount of gene expression for the gene was transcribed to produce the specific RNA.

The hybridization sample is maintained under conditions that are sufficient to allow specific hybridization of the nucleic acid probe to a specific gene expression product. "Specific hybridization", as used herein, indicates near exact hybridization (e.g., with few if any mismatches). Specific hybridization can be performed under high stringency conditions or moderate stringency conditions. In one embodiment, the hybridization conditions for specific hybridization are high stringency. For example, certain high stringency conditions can be used to distinguish perfectly complementary nucleic acids from those of less complementarity. "High stringency conditions", "moderate stringency conditions" and "low stringency conditions" for nucleic acid hybridizations are explained on pages 2.10.1-2.10.16 and pages 6.3.1-6.3.6 in Current Protocols in Molecular Biology (Ausubel, F. et al., "Current Protocols in Molecular Biology", John Wiley & Sons, (1998), the entire teachings of which are incorporated by reference herein). The exact conditions that determine the stringency of hybridization depend not only on ionic strength (e.g., 0.2.times.SSC, 0.1.times.SSC), temperature (e.g., room temperature, 42°C., 68°C.) and the concentration of destabilizing agents such as formamide or denaturing agents such as SDS, but also on factors such as the length of the nucleic acid sequence, base composition, percent mismatch between hybridizing sequences and the frequency of occurrence of subsets of that sequence within other non-identical sequences. Thus, equivalent conditions can be determined by varying one or more of these parameters while maintaining a similar degree of identity or similarity between the two nucleic acid molecules. Typically, conditions are used such that sequences at least about 60%, at least about 70%, at least about 80%, at least about 90% or at least about 95% or more identical to each other remain hybridized to one another. By varying hybridization conditions from a level of stringency at which no hybridization occurs to a level at which hybridization is first observed, conditions that will allow a given sequence to hybridize (e.g., selectively) with the most complementary sequences in the sample can be determined.

Exemplary conditions that describe the determination of wash conditions for moderate or low stringency conditions are described in Kraus, M. and Aaronson, S., 1991. Methods Enzymol., 200:546-556; and in, Ausubel et al., Current Protocols in Molecular Biology, John Wiley & Sons, (1998). Washing is the step in which conditions are usually set so as to determine a minimum level of complementarity of the hybrids. Generally, starting from the lowest temperature at which only homologous hybridization occurs, each °C. by which the final wash temperature is reduced (holding SSC concentration constant) allows an increase by 1% in the maximum mismatch percentage among the sequences that hybridize. Generally, doubling the concentration of SSC results in an increase in Tₘ of about 17°C. Using these guidelines, the wash temperature can be determined empirically for high, moderate or low stringency, depending on the level of mismatch sought. For example, a low stringency wash can comprise washing in a solution containing 0.2.times.SSC/0.1% SDS for 10 minutes at room temperature; a moderate stringency wash can comprise washing in a pre-warmed solution (42°C) solution containing 0.2.times.SSC/0.1% SDS for 15 minutes at 42°C.; and a high stringency wash can comprise washing in pre-warmed (68°C.) solution containing 0.1.times.SSC/0.1% SDS for 15 minutes at 68°C. Furthermore, washes can be performed repeatedly or sequentially to obtain a desired result as known in the art. Equivalent conditions can be determined by varying one or more of the parameters given as an example, as known in the art, while maintaining a similar degree of complementarity between the target nucleic acid molecule and the primer or probe used (e.g., the sequence to be hybridized).

A related aspect of the present invention provides a molecular array, which array comprises a plurality of:
(i) nucleic acid molecules comprising a nucleotide sequence corresponding to any one or more of the neoplastic marker genes hereinbefore described or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof

wherein the level of expression of said marker genes of (i) or proteins of (iv) is indicative of the neoplastic state of a cell or cellular subpopulation derived from the large intestine.

Preferably, said percent identity is at least 85%, 90%, 91%, 92%, 93%, 94%, 95%, 96%, 97%, 98% or 99%.

Low stringency includes and encompasses from at least about 1% v/v to at least about 15% v/v formamide and from at least about 1M to at least about 2M salt for hybridisation, and at least about 1M to at least about 2M salt for washing conditions. Alternative stringency conditions may be applied where necessary, such as medium stringency, which includes and encompasses from at least about 16% v/v at least about 30% v/v formamide and from at least about 0.5M to at least about 0.9M salt for hybridisation, and at least about 0.5M to at least about 0.9M salt for washing conditions, or high stringency, which includes and encompasses from at least about 31 % v/v to at least about 50% v/v formamide and from at least about 0.01M to at least about 0.15M salt for hybridisation, and at least about 0.01M to at least about 0.15M salt for washing conditions. In general, washing is carried out at Tₘ = 69.3 + 0.41 (G + C) % [19] = -12°C. However, the Tₘ of a duplex DNA decreases by 1°C with every increase of 1% in the number of mismatched based pairs (Bonner et al (1973) J. Mol. Biol. 81:123).

Preferably, the subject probes are designed to bind to the nucleic acid or protein to which they are directed with a level of specificity which minimises the incidence of non-specific reactivity. However, it would be appreciated that it may not be possible to eliminate all potential cross-reactivity or non-specific reactivity, this being an inherent limitation of any probe based system.

In terms of the probes which are used to detect the subject proteins, they may take any suitable form including antibodies and aptamers.

A library or array of nucleic acid or protein probes provides rich and highly valuable information. Further, two or more arrays or profiles (information obtained from use of an array) of such sequences are useful tools for comparing a test set of results with a reference, such as another sample or stored calibrator. In using an array, individual probes typically are immobilized at separate locations and allowed to react for binding reactions. Primers associated with assembled sets of markers are useful for either preparing libraries of sequences or directly detecting markers from other biological samples.

A library (or array, when referring to physically separated nucleic acids corresponding to at least some sequences in a library) of gene markers exhibits highly desirable properties. These properties are associated with specific conditions, and may be characterized as regulatory profiles. A profile, as termed here refers to a set of members that provides diagnostic information of the tissue from which the markers were originally derived. A profile in many instances comprises a series of spots on an array made from deposited sequences.

A characteristic patient profile is generally prepared by use of an array. An array profile may be compared with one or more other array profiles or other reference profiles. The comparative results can provide rich information pertaining to disease states, developmental state, receptiveness to therapy and other information about the patient.

Another aspect of the present invention provides a diagnostic kit for assaying biological samples comprising an agent for detecting one or more neoplastic marker reagents useful for facilitating the detection by the agent in the first compartment. Further means may also be included, for example, to receive a biological sample. The agent may be any suitable detecting molecule.

The present invention is further described by the following non-limiting examples:

### EXAMPLE 1

### Methods and Materials

### Affymetrix GeneChip data

Gene expression profiling data and accompanying clinical data was purchased from GeneLogic Inc (Gaithersburg, MD USA). For each tissue analysed, oligonucleotide microarray data for 44,928 probesets (Affymetrix HGU133A & HGU133B, combined), experimental and clinical descriptors, and digitally archived microscopy images of histological preparations were recieved. A quality control analysis was performed to remove arrays not meeting essential quality control measures as defined by the manufacturer.

Transcript expression levels were calculated by both Microarray Suite (MAS) 5.0 (Affymetrix) and the Robust Multichip Average (RMA) normalization techniques (Affymetrix. GeneChip expression data analysis fundamentals. Affymetrix, Santa Clara, CA USA, 2001; Hubbell et al. Bioinformatics, 18:1585-1592, 2002; Irizarry et al. Nucleic Acid Research, 31, 2003)MAS normalized data was used for performing standard quality control routines and the final data set was normalized with RMA for all subsequent analyses.

### Univariate differential expression

Differentially expressed gene transcripts were identified using a moderated t-test implemented in the limma library downloaded from the Bioconductor repository for R. (G. K. Smyth. Statistical Applications in Genetics and Molecular Biology, 3(1):Article 3, 2004; G K Smyth. Bioinformatics and Computational Biology Solutions using R and Bioconductor. Springer, New York, 2005). Significance estimates (p-values) were corrected to adjust for multiple hypothesis testing using the Bonferonni correction.

### Tissue specific expression patterns

To construct a filter for hypothetically 'turned off' gene expression the mean expression level for all 44,928 probesets across the full range of 454 tissues was first estimated. To estimate an expression on/off threshold, the 44,928 mean values were ranked and the expression value equivalent to the 30th percentile across the dataset calculated. This arbitrary threshold was chosen because it was theorized that the majority of transcripts (and presumably more than 30%) in a given specimen should be transcriptionally silenced. Thus this threshold represents a conservative upper bound for what is estimated as non-specific, or background, signal.

### Gene symbol annotations

To map Affymetrix probeset names to official gene symbols the annotation metadata available from Bioconductor was used. Hgu133plus2 library version 1.16.0, which was assembled using Entrez Gene data downloaded on 15 March 2007, was used.

### Estimates of performance characteristics

Diagnostic utility for each table of markers shown herein was estimated including: sensitivity, specificity, positive predictive value, negative predictive value, likelihood ratio positive, likelihood ratio negative. These estimates were calculated in the same data used to discover the markers and will therefore potentially overestimate the performance characteristics in future tissue samples. To improve the generalisabilty of the estimates a modified jackknife resampling technique was used to calculate a less biased value for each characteristic.

### Results

A range of univariate statistical tests were applied on Affymetrix oligonucleotide microarray data to reveal human genes that could be used to discriminate colorectal neoplastic tissues from non-neoplastic tissues. There were further identified a number of gene transcripts that appear to be useful for differentiating colorectal adenomas from colorectal carcinoma. Also identified were a subset of these transcripts that may have particular diagnostic utility due to the protein products being either secreted or displayed on the cell surface of epithelial cells. Finally, there were identified a further subset of transcripts expressed specifically in neoplastic tissues and at low- or near-background levels in non-neoplastic tissues.

### Genes differentially expressed in neoplastic tissues

From a total GeneChip set of 44,928 probesets it was determined that over 11,000 probesets were differentially expressed by moderated t-test using the limma package in BioConductor (G. K. Smyth, 2004 *supra)* employing conservative (Bonferroni) multiple test correction. When this list was further filtered to include only those probesets demonstrating a 2-fold or greater mean expression change between the neoplastic and non-neoplastic tissues, 560 probesets were found to be expressed *lower* in neoplasias relative to normal tissues.

These 560 probesets were annotated using the most recent metadata and annotation packages available for the chips. The 560 underexpressed probesets were mapped to 434 gene symbols.

| Δ-expression | ProbeSet ID | Gene Symbol Maps |
|---|---|---|
| DOWN | 560 | 434 |

### Hypothetical markers specific for colorectal neoplasia

While differential gene expression patterns are useful for diagnostic purposes this project also seeks to identify diagnostic proteins shed into the lumen of the gut by neoplastic colorectal epithelia. To discover candidate proteins, the list of differentially expressed transcripts was filtered with a selection criteria aimed at identifying markers specifically turned off in colorectal neoplasia tissues. To identify 'off' genes the filter criteria were designed to find genes with i) neoplastic expression levels below a theoretical on/off threshold and ii) normal signals at least 2-fold higher. The expression profile of an example transcript that is 'turned-off' in neoplastic tissues is shown in Figure 1.

### EXAMPLE 2

### PROBESETS ELEVATED IN NON-NEOPLASIA RELATIVE TO NEOPLASTIC TISSUES

Differential expression analysis was applied to identify down-regulated probesets in Affymetrix gene chip data measuring RNA concentration in 454 colorectal tissues including 161 adenocarcinoma specimens, 29 adenoma specimens, 42 colitis specimens and 222 non-diseased tissues. Using conservative corrections for multiple hypothesis testing and a 2-fold absolute fold change cut-off it was determined that 560 probesets exhibit a decreased expression level in neoplastic tissues relative to non-neoplastic controls. 560 of these probesets have been mapped to 434 putative gene symbols based on transcript nucleotide sequence.

### Validation/Hypothesis testing

RNA expression levels of these candidates were measured in independently derived clinical specimens. 526 probesets were hybridised to RNA extracts from 68 clinical specimens comprising 19 adenomas, 19 adenocarcinomas, and 30 non-diseased controls using a custom-designed 'Adenoma Gene Chip'. Thirty-four (34) probesets were not tested as they were not included on the custom design. It was confirmed that 459 of 526 of the target probesets (or directly related probesets with the same gene locus target) were likewise differentially expressed (P < 0.05) in these independently-derived tissues. The results of differential expression analysis of these 459 probesets is shown in Table 1.

The 372 of the 434 unique gene loci to which the 560 probesets are understood to hybridise were further tested. The remaining 62 gene symbols were not represented in the validation data. It was observed that 328 of 372 gene symbols were represented in the validation data by at least one differentially expressed probeset and many symbols included multiple probesets against regions across the putative locus. A complete list of probesets that bind to target loci is shown in Table 2.

### Conclusion

The candidate probesets and symbols shown in Tables 1 and 2. respectively, are differentially expressed lower in neoplastic colorectal tissues compared to non-neoplastic controls.

### EXAMPLE 3

### PROBESETS DEMONSTRATING A NON-NEOPLASIA SPECIFIC PROFILE

During analysis of the discovery data, a novel expression profile was observed between neoplastic and non-neoplastic phenotypes. It was hypothesized that a subset of quantitatively differentially expressed probesets are furthermore qualitatively differentially expressed. Such probesets show no evidence of a gene expression activity in neoplastic tissues, i.e. these probesets appear to be expressed above background levels in non-neoplastic tissues only. This observation and the resulting hypothesis are based on two principles:
1. That the majority of human transcripts that are present on a genome-wide GeneChip (e.g.
   U133) will not likely be expressed in the colorectal mucosa; and
2. That microarray binding intensity for such'off probesets (to labeled cRNA) will reflect technical assay background, i.e. non-specific oligonucleotide binding.

To generate a list of non-neoplasia specific probesets the neoplastic intensity of differentially expressed probesets were compared with a hypothetical background signal threshold from across all probesets on the chip. We note that, by design, all probesets in the candidate pool from which the 'on' transcripts are chosen are at least two fold over-expressed in the non-diseased tissues relative to diseased tissues. Combined, these criteria yield the subset of differentially expressed transcript species that are specifically expressed in non-neoplastic tissues.

This analysis demonstrated that 42 probesets corresponding to approximately 41 gene loci exhibit a non-neoplasia specific transcription expression profile.

### Validation/Hypothesis Testing

The custom gene chip design precludes testing the non-neoplasia-specific probesets using the same principles as used for discovery. In particular, the custom gene chip (by design) does not contain a large pool of probesets anticipated to hybridise to hypothetically 'off'/'non-transcribed' gene transcripts. This is because the custom gene chip design is heavily biased toward differentially expressed transcripts in colorectal neoplastic tissues.

The usual differential expression testing (limma) was therefore applied to these candidate probesets for specifically expressed in non-neoplastic tissues. Of the 37 (of 42) probesets on the custom gene chip, 33 probesets (or probesets which bind to the same locus) were differentially expressed between the 38 neoplastic tissues (adenoma & cancer) and non-neoplastic controls. The results of these validation experiments is shown in Table 3.

It was further aimed to test all probesets which are known to hybridise to the gene loci to which the probesets claimed herein. Of the 41 putative gene loci targeted by the probesets, 33 were present in the validation data. All thirty-three (33) of these 33 (100%) gene symbols demonstrated at least one hybridising probeset which was differentially expressed in the neoplastic tissues. Results for these experiments, including all probesets that bind to each target locus in a differentially expressed manner are shown in Table 4.

### EXAMPLE 4

### MATERIALS AND METHODS FOR EXAMPLES 2 AND 3

Gene expression profiling data measured in 454 colorectal tissue specimens including neoplastic, normal and non-neoplastic disease controls was purchased from GeneLogic Inc (Gaithersburg, MD USA). For each tissue specimen Affymetrix (Santa Clara, CA USA) oligonucleotide microarray data totalling 44,928 probesets (HGU133A & HGU133B, combined), experimental and clinical descriptors, and digitally archived microscopy images of histological preparations was received. Prior to applying discovery methods to these data, extensive quality control methods, including statistical exploration, review of clinical records for consistency and histopathology audit of a random sample of arrays was carried out. Microarrays that did not meet acceptable quality criteria were removed from the analysis.

### Hypothesis testing

Candidate transcription biomarkers were tested using a custom oligonucleotide microarray of 25-mer oligonucleotide probesets designed to hybridise to candidate RNA transcripts identified during discovery. Differential expression hypotheses were tested using RNA extracts derived from independently collected clinical samples comprising 30 normal colorectal tissues, 19 colorectal adenoma tissues, and 19 colorectal adenocarcinoma tissues. Each RNA extract was confirmed to meet strict quality control criteria.

### Colorectal tissue specimens

All tissues used for hypothesis testing were obtained from a tertiary referral hospital tissue bank in metropolitan Adelaide, Australia (Repatriation General Hospital and Flinders Medical Centre). Access to the tissue bank for this research was approved by the Research and Ethics Committee of the Repatriation General Hospital and the Ethics Committee of Flinders Medical Centre. Informed patient consent was received for each tissue studied.

Following surgical resection, specimens were placed in a sterile receptacle and collected from theatre. The time from operative resection to collection from theatre was variable but not more than 30 minutes. Samples, approximately 125mm3 (5x5x5mm) in size, were taken from the macroscopically normal tissue as far from pathology as possible, defined both by colonic region as well as by distance either proximal or distal to the pathology. Tissues were placed in cryovials, then immediately immersed in liquid nitrogen and stored at -150°C until processing.

### RNA extraction

RNA extractions were performed using Trizol(R)reagent (Invitrogen, Carlsbad, CA, USA) as per manufacturer's instructions. Each sample was homogenised in 300µL of Trizol reagent using a modified Dremel drill and sterilised disposable pestles. Additional 200µL of Trizol reagent was added to the homogenate and samples were incubated at RT for 10 minutes. 100µL of chloroform was then added, samples were shaken vortexed for 15 seconds, and incubated at RT for 3 further minutes. The aqueous phase containing target RNA was obtained by centrifugation at 12,000 rpm for 15 min, 40°C. RNA was then precipitated by incubating samples at RT for 10 min with 250µL of isopropanol. Purified RNA precipitate was collected by centrifugation at 12,000 rpm for 10 minutes, 40°C and supernatants were discarded. Pellets were then washed with 1mL 75% ethanol, followed by vortexing and centrifugation at 7,500g for 8 min, 40°C. Finally, pellets were air-dried for 5 min and resuspended in 80µL of RNase free water. To improve subsequent solubility samples were incubated at 55°C for 10 min. RNA was quantified by measuring the optical density at A260/280 nm. RNA quality was assessed by electrophoresis on a 1.2% agarose formaldehyde gel.

### Gene Chip processing

To test hypotheses related to biomarker candidates for colorectal neoplasia RNA extracts were assayed using a custom GeneChip designed by us in collaboration with Affymertix (Santa Clara, CA USA). These custom GeneChips were processed using the standard Affymetrix protocol developed for the HU Gene ST 1.0 array described in (Affy:WTAssay).

### Statistical software and data processing

The R statistics environment R and BioConductor libraries (BioConductor, www.bioconductor.org) (BIOC) was used for most analyses. To map probeset IDs to gene symbol on the Custom GeneChip hgu133plus2 library version 2.2.0 was used which was assembled using Entrez Gene data downloaded on Apr 18 12:30:55 2008 (BIOC).

*Hypothesis testing of* differentially *expressed biomarkers* To assess differential expression between tissue classes the Student's t test for equal means between two samples or the robust variant provided by the limma library (Smyth)(limma) was used. To mitigate the impact of false discovery due to multiple hypothesis testing, a Bonferroni adjustment to P values in the discovery process (MHT:Bonf) was applied. For hypotheses testing the slightly less conservative multiple hypothesis testing correction of Benjamini & Hochberg, which aims to control the false discovery rate of solutions(MHT:BH), was applied.

### Discovery of tissue-specific gene expression patterns

Discovery methods using gene expression data often yield numerous candidates, many of which are not suitable for commercial products because they involve subtle gene expression differences that would be difficult to detect in laboratory practice. Pepe *et al.* note that the 'ideal' biomarker is detectable in tumor tissue but not detectable (at all) in non-tumour tissue (Pepe:biomarker:development.) To bias the discovery toward candidates that meet this criteria, an analysis method was developed that aims to enrich the candidates for biomarkers whose qualitative absence or presence measurement is diagnostic for the phenotype of interest. This method attempts to select candidates that show a prototypical 'turned-on' or'turned-off pattern relative to an estimate of the background/noise expression across the chip. It is theorized that such RNA transcripts are more likely to correlate with downstream translated proteins with diagnostic potential or to predict upstream genomic changes (e.g. methylation status) that could be used diagnostically. This focus on qualitative rather than quantitative outcomes may simplify the product development process for such biomarkers.

The method is based on the assumption that the pool of extracted RNA species in any given tissue (e.g. colorectal mucosae) will specifically bind to a relatively small subset of the full set of probesets on a GeneChip designed to measure the whole genome. On this assumption, it is estimated that most probesets on a full human gene chip will not exhibit specific, high-intensity signals.

This observation is utilised to approximate the background or'non-specific binding' across the chip by choosing a theoretical level equal to the value of e.g. lowest 25% quantile of the ranked mean values. This quantile can be arbitrarily set to some level below which there is made a reasonable assumption that the signals do not represent above-background RNA binding. Finally, this background estimate is used as a threshold to estimate the 'OFF' probesets in an experiment for, say, the non-neoplastic tissue specimens.

Conversely, it is further hypothesized that probesets which are 1) expressed above this theoretical threshold level and 2) at differentially higher levels in the tumour specimens may be a tumour specific candidate biomarker. It is noted that in this case the concept of 'fold-change' thresholds can also be conveniently applied to further emphasize the concept of absolute expression increases in a putatively 'ON' probeset.

Given the assumption of low background binding for a sizeable fraction of the measured probesets, this method was only used in the large GeneLogic data and discovery. To construct a filter for hypothetically 'turned on' biomarker in the GeneLogic discovery data, the mean expression level for all 44,928 probesets across the full range of 454 tissues was estimated. The 44,928 mean values were then ranked and the expression value equivalent to the 25th percentile across the dataset calculated. This arbitrary threshold was chosen because the majority of transcripts (and presumably more than 25%) in a given specimen should exhibit low concentration which effectively transcriptional silence. Thus this threshold represents a conservative upper bound for what is estimated is non-specific, or background, expression.

### EXAMPLE 5

### DNA METHYLATION DATA

Assays were developed for detection of methylation in the promoter regions the eight down-regulated genes in Table 5. Methods for bisulphite treatment of DNA and assays for determination of DNA methylation levels, including MSP and COBRA, are described in Clark *et al..* (2006).

Five MSP assays used the primer pairs shown in TABLE 7. A control PCR for unbiased amplification of the CAGE gene was used to determine the quantity of input DNA to provide a reference for quantification of the level of methylation of each gene. For PCRs, 25 µL reactions in Biorad iQ SyBr Green Super Mix contained 5ng of bisulphite-treated DNAs (1 ng for cell line assays and 6 ng for clinical specimens) and 200nM of forward and reverse primers. PCR cycling conditions were:
95.0°C for 2 min
Followed by 50 cycles of: 95.0°C/15sec Temp°C/30sec 72.0°C/30sec

Where "Temp" is the re-annealing temperature optimised for each gene as shown in Table yy.

For the DF gene, 3 preliminary cycles were done using a 95.0°C melting temperature, followed by 50 cycles with a lower, 84.0°C melting temperature (to reduce nonspecific amplification).

A standard curve was generated using DNA methylated with M.SssI methylase (100% methylated) and DNA that had been in vitro amplified using Phi29 DNA polymerase (0% methylation).

COBRA assays were developed for three genes as shown in TABLE 8. PCRs were setup as above with cycling conditions: 95.0°C for 2 min

Followed by 50 cycles of:
95.0°C/15sec
Temp°C/30sec
72.0°C/30sec

After PCR, 10 µL of PCR product was digested with the appropriate enzyme (TABLE 8), digestion products analysed by gel electrophoresis and methylation levels determined semiquantitatively.

The methylation state of the eight genes was determined in four colorectal cancer cell lines, Caco2, HCT116, HT29 and SW480 as well as normal blood DNA and the normal lung fibroblast cell line, MRC5. The level of methylation in summarised in Table 5. The promoter regions of all eight genes show strong methylation in 2 or 3 of the four colorectal cancer cell lines tested. All showed a lack or low level of methylation in DNA from normal blood DNA and the fibroblast cell line MRC5, except for methylation of DF in MRC5.

For two of these genes, MAMDC2 and GPM6B analysis has been extended to a set of 12 adenoma, 18 cancer and 22 matched normal tissue samples (Figure 2, A and B).

For MAMDC2 quantitative analysis demonstrated that 2 of 12 adenomas and 6 of 18 cancer samples showed elevated methylation compared with the highest level observed in normal tissue samples. Methylation levels of the GPM6B gene were determined by semiquantitative COBRA assays, scored on a scale of 0 to 5 based on visual inspection of restriction digestions. A clear trend toward increasing promoter methylation in progression from normal to adenoma to cancer was evident (Figure 2, panel B).

These data demonstrate for a number of examples of the down-regulated genes that such downregulation in colorectal cancer cell lines and primary neoplasia tissue may be associated with DNA methylation and that assays of DNA methylation can be used to discriminate cancer and normal tissue.

### EXAMPLE 6

### Determine Gene Identity of a Nucleic Acid Sequence of Interest which is Define by an Affymetrix Probeset

### BLAST the sequence of interest using online available Basic Local Alignment Search Tools [BLAST]. e.g. NCBI/BLAST

(http://blast.ncbi.nlm.nih.gov/Blast.cgi)
(a) Select "Human" in BLAST ASSEMBLED GENOMES on the web page http://blast.ncbi.nim.nih.gov/Blast.cgi
(b) Leave the default settings, i.e.:
   - Database: Genome (all assemblies)
   - Program: megaBLAST: compare highly related nucleotide sequences
   - Optional parameters: Expect: 0.01, Filter: default, Descriptions: 100, Alignments: 100
(c) Copy/Paste Sequence into the "BLAST" window
(d) Click "Begin Search"
(e) Click "View Report"

### Assessment of the Open BLAST Search Results

Multiple significant sequence alignments may be identified when "blasting" the sequence.

### Identify gene nomenclature of the identified sequence match

(a) Click the link to one of the identified hits
(b) The new page will schematically depict the position of the hit on one chromosome. It will be apparent which gene is hit.
(c) Retrieve the "hit" sequence clicking on the link
(d) Do a search for the gene in the provided "search" window. This provides the gene nucleotide coordinates for the gene.

### Determine promiscuity of Sequence

(a) Open the NCBI/BLAST tool, (http://blast.ncbi.nlm.nih.gov/Blast.cgi)
(b) Click on "nucleotide Blast" under "basic BLAST"
(c) Copy/paste the sequence of interest into the "Query Sequence" window
(d) Click "Blast".

### Assessment of the nBLAST Search Results of the Sequence

(a) The nBLAST exercise with the Sequence may result in multiple Blast hits of which some accession entry numbers are listed in "Description".
(b) These hits should be reviewed.

### Determine location of the Sequence in the Gene

The Ensembl database is an online database, which produces and maintains automatic annotation selected eukaryotic genomes (www.ensembl.orq/index.html)

### Identify location of the Sequence in the Gene

(a) Set "Search" to Homo Sapiens, Type "the gene name" in the provided Search Field Ensemble.org/index.html)
(b) Click "Go"
(c) Click the "vega protein_coding Gene: OTTHUMG000000144184" link to get an annotation report
(d) Click on "Gene DAS Report" to retrieve information regarding Alternative splice site database: Type "the gene name" in search field
- Click on "the gene entry"
- Scroll down to "evidences"
- Review alternative splice sites
- Click "Confirmed intron/exons" to get a list of coordinates for the exons & introns.

### Alternative splicing and/or transcription

The AceView Database provides curated and non-redundant sequence representation of all public mRNA sequences. The database is available through NCBI:

### http://www.ncbi.nlm.nih.gov/IEB/Research/Acembly/

### Further investigation of the Gene mRNA transcripts

(a) Type "the gene name" into the provided "search" field
(b) Click "Go"
(c) The following information is available from the resulting entry in AceView:
   - The number of cDNA clones from which the gene is constructed (ie originated-from experimental work involving isolation of mRNA)
   - The mRNAs predicted to be produced by the gene
   - The existence of non-overlapping alternative exons and validated alternative polyadenylation sites
   - The existence of truncations
   - The possibility of regulated alternate expression
   - Introns recorded as participating in alternatively splicing of the gene
(d) Classic splice site motives

### Application of Method to LOC643911/hCG_1815491

### Materials and Methods

### Extraction of RNA

RNA extractions were performed using Trizol(R) reagent (Invitrogen, Carlsbad, CA, USA) as per manufacturer's instructions. Each sample was homogenised in 300µL of Trizol reagent using a modified dremel drill and sterilised disposable pestles. Additional 200µL of Trizol reagent was added to the homogenate and samples were incubated at RT for 10 minutes. 100µL of chloroform was then added, samples were shaken vortexed for 15 seconds, and incubated at RT for 3 further minutes. The aqueous phase containg target RNA was obtained by centrifugation at 12,000 rpm for 15 min, 40° C. RNA was then precipitated by incubating samples at RT for 10 min with 250µL of isopropanol. Purified RNA precipitate was collected by centrifugation at 12,000 rpm for 10 minutes, 40° C and supernatants were discarded. Pellets were then washed with 1mL 75% ethanol, followed by vortexing and centrifugation at 7,500g for 8 min, 40° C . Finally, pellets were air-dried for 5 min and resuspended in 80µL of RNase free water. To improve subsequent solubility samples were incubated at 55° C for 10 min. RNA was quantified by measuring the optical density at A260/280 nm. RNA quality was assessed by electrophoresis on a 1.2% agarose formaldehyde gel.

### Gene Chip processing

RNA samples to analyze on Human Exon 1.0 ST GeneChips were processed using the Affymetrix WT target labeling and control kit (part# 900652) following the protocol described in (Affymetrix 2007 P/N 701880 Rev.4). Briefly: First cycle cDNA was synthesized from 100ng ribosomal reduced RNA using random hexamer primers tagged with T7 promoter sequence and SuperScript II (Invitrogen, Carlsbad CA), this was followed by DNA Polymerase I synthesis of the second strand cDNA. Anti-sense cRNA was then synthesized using T7 polymerase. Second cycle sense cDNA was then synthesised using SuperScript II, dNTP+ dUTP, and random hexamers to produce sense strand cDNA incorporating uracil. This single stranded uracil containing cDNA was then fragmented using a combination of uracil DNA glycosylase (UDG) and apurinic/ apyrimidinic endonuclease1 (APE 1). Finally the DNA was biotin labelled using terminal deoxynucleotidyl transferase (TdT) and the Affymetrix proprietary DNA Labeling reagent. Hybridization to the arrays was carried out at 45°C for 16-18hours.

Washing and staining of the hybridized GeneChips was carried out using the Affymetrix Fluidics Station 450 and scanned with the Affymetrix Scanner 3000 following recommended protocols.

### SYBR green based Quantitative Real Time-PCR

Quantitative real time polymerase chain reaction was performed on RNA isolated from clinical samples for the amplification and detection of the various hCG_1815491 transcripts.

Firstly cDNA was synthesized from 2ug of total RNA using the Applied Biosystems High Capacity Reverse transcription Kit (P/N 4368814). After synthesis the reaction was diluted 1:2 with water to obtain a final volume of 40ul and 1ul of this diluted cDNA used in subsequent PCR reactions.

PCR was performed in a 25ul volume using 12.5ul Promega 2x PCR master mix (P/N M7502), 1.5ul 5uM forward primer, 1.5ul 5uM reverse primer, 7.875ul water, 0.625ul of a 1:3000 dilution of 10,000x stock of SYBR green 1 pure dye (Invitrogen P/N S7567), and 1ul of cDNA.

Cycling conditions for amplification were 95° for 2minutes x1 cycle, 95° for 15 seconds and 60° for 1 minute x40 cycles. The amplification reactions were performed in a Corbett Research Rotor-Gene RG3000 or a Roche LightCycler480 real-time PCR machine. When the Roche LightCycler480 real-time PCR machine was used for amplification the reaction volume was reduced to 10ul and performed in a 384 well plate but the relative ratios between all the components remained the same. Final results were calculated using the ΔΔCt method with the expression levels of the various hCG_1815491 transcripts being calculated relative to the expression level of the endogenous house keeping gene HPRT.

### End-point PCR

End point PCR was performed on RNA isolated from clinical samples for the various hCG_1815491 transcripts. Conditions were identical to those described for the SYBR green assay above but with the SYBR green dye being replaced with water. The amplification reactions were performed in a MJ Research PTC-200 thermal cycler. 2.5µl of the amplified products were analysed on 2% agarose E-gel (Invitrogen) along with a 100-base pair DNA Ladder Marker.

### Results

The nucleotide structure and expression levels of transcripts related to hCG_1815491 was analysed based on the identification of diagnostic utility of Affymetrix probesets 238021_s_at and 238022_at from the gene chip analysis.

The gene hCG_1815491 is currently represented in NCBI as a single RefSeq sequence, XM_93911. The RefSeq sequence of hCG_1815491 is based on 89 GenBank accessions from 83 cDNA clones. Prior to March 2006, these clones were predicted to represent two overlapping genes, LOC388279 and LOC650242 (the latter also known as LOC643911). In March 2006, the human genome database was filtered against clone rearrangements, co-aligned with the genome and clustered in a minimal non-redundant way. As a result, LOC388272 and LOC650242 were merged into one gene named hCG_1815491 (earlier references to hCG_1815491 are: LOC388279, LOC643911, LOC650242, XM_944116, AF275804, XM_373688).

It has been determined that the Ref Sequence, which is defined by the genomic coordinates 8579310 to 8562303 on human chromosome 16 as defined by the NCBI contig reference NT_010498.15 | Hs16_10655, NCBI 36 March 2006 genome encompasses hCG_1815491. The 10 predicted RNA variants derived from this gene have been aligned with the genomic nucleotide sequence residing in the map region 8579310 to 8562303. This alignment analysis revealed the existence of at least 6 exons of which several are alternatively spliced. The identified exons are in contrast to the just 4 exons specified in the NCBI hCG_1815491 RefSeq XM_93911. Two additional putative exons were also identified in the Ref Sequence by examination of included probesets on Affymetrix Genechip HuGene Exon 1.0 that target nucleotide sequences embedded in the Ref Sequence. The identified and expanded exon-intron structure of hCG_1815491 have been used to design specific oligonucleotide primers, which allowed measurement of the expression of RNA variants generated from the Ref Sequence by using PCR-based methodology (Figure 4)

**TABLES**
The probeset designations include both HG-133plus2 probeset IDs and Human Gene 1.0ST array probe ids. The latter can be conveniently mapped to Transcript Cluster ID using the Human Gene 1.0ST probe tab file provided by Affymetrix
(http://www.affymetrix.com/Auth/analysis/downloads/na22/wtgene/HuGene-1_0-stv1.probe.tab.zip). Using publicly available software such as NetAffx (provided by Affymetrix), the Transcript Cluster ID may be further mapped to gene symbol, chromosomal location, etc.

**Table 1**
Probesets demonstrated to be expressed higher in non-neoplastic tissues relative to neoplastic controls. TargetPS: Affymetrix HG-U133plus2 probeset id; Symbol: putative gene symbol corresponding to target probeset id - multiple symbol names indicate the possibility of probeset hybridisation to multiple gene targets; Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of non-neoplasia vs. neoplasia; Sens-Spec: Estimate of diagnostic performance corresponding to the ROC curve point demonstrating equal sensitivity and specificity; CI (95): 95% confidence interval of sensitivity and specificity estimates.

**Table 2** Evidence of multiple probesets which correspond to gene symbols claimed herein exhibiting RNA concentration differences between non-neoplastic tissues and neoplastic controls. Symbol: gene symbol; ValidPs_DOWN: Affymetrix probeset IDs demonstrating statistically significant overexpression in non-neoplastic RNA extracts relative to neoplastic controls. Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of non-neoplasia vs. neoplasia; Sens-Spec: Estimate of diagnostic performance corresponding to the ROC curve point demonstrating equal sensitivity and specificity; CI (95): 95% confidence interval of sensitivity and specificity estimates.

**Table 3** Probesets which demonstrate a qualitatively (in addition to quantitative) elevated profile in non-neoplastic tissues relative to neoplastic controls. TargetPS: Affymetrix HG-U133plus2 probeset id; Symbol: putative gene symbol corresponding to target probeset id - multiple symbol names indicate the possibility of probeset hybridisation to multiple gene targets; Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of non-neoplasia vs. neoplasia; Sens-Spec: Estimate of diagnostic performance corresponding to the ROC curve point demonstrating equal sensitivity and specificity; CI (95): 95% confidence interval of sensitivity and specificity estimates.

**Table 4** Evidence of multiple probesets which correspond to gene symbols claimed herein exhibiting qualitative changes in RNA concentration in non-neoplastic tissues compared to neoplastic tissues. Symbol: gene symbol; ValidPS_DOWN: Affymetrix probeset IDs demonstrating statistically significant overexpression in neoplastic RNA extracts relative to non-neoplastic controls. Signif. FDR: Adjusted p-value for mean difference testing between RNA extracted from neoplasia and non-neoplastic tissues. Adjustment is made using Benjamini & Hochberg correction for multiple hypothesis testing (Benjamini and Hochberg, 1995); D.value50: Diagnostic effectiveness parameter estimate corresponding to the area of a receiver operator characteristic ROC. This parameter provides a convenient estimate of diagnostic utility and is described in (Saunders, 2006); FC: fold change between mean expression level of non-neoplasia vs. neoplasia; Sens-Spec: Estimate of diagnostic performance corresponding to the ROC curve point demonstrating equal sensitivity and specificity; CI (95): 95% confidence interval of sensitivity and specificity estimates.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

**TABLE 1**

| **TargetPS** | **Symbol** | .**Signif. FDR** | **D.val5** | **FC** | **Sens-Spec** | **CI (95)** |
|---|---|---|---|---|---|---|
| 230788_at | SPTLC3:GCNT2 | 2.16E-27 | 3.9512 | 13.36 | 97.6 | 94.2-99.2 |
| 207502_at | GUCA2B | 7.04E-25 | 3.6279 | 51.78 | 96.5 | 92.3-98.6 |
| 207003_at | GUCA1B | 7.32E-25 | 3.565 | 10.7 | 96.3 | 92-98.5 |
| 206208_at | CA4 | 1.73E-24 | 3.6263 | 10.41 | 96.5 | 92.4-98.6 |
| 206209_s_at | CA4 | 1.73E-24 | 3.6265 | 10.41 | 96.5 | 92.3-98.6 |
| 203908_at | LOC727995:SLC4A4:LOC730 895 | 2.23E-23 | 3.6298 | 3.58 | 96.5 | 92.4-98.6 |
| 206784_at | AQP8 | 2.98E-22 | 3.3284 | 10.82 | 95.2 | 90.3-97.9 |
| 205950_s_at | CA1 | 3.15E-22 | 3.368 | 38.27 | 95.4 | 90.6-98 |
| 209735_at | ABCG2 | 1.09E-21 | 3.2584 | 31.53 | 94.8 | 89.7-97.7 |
| 230830_at | OSTbeta | 6.23E-21 | 3.1861 | 7.15 | 94.4 | 89.2-97.5 |
| 223754_at | MGC13057 | 6.94E-21 | 3.2036 | 3.18 | 94.5 | 89.3-97.5 |
| 228195_at | MGC13057 | 6.94E-21 | 3.2023 | 3.18 | 94.5 | 89.4-97.5 |
| 228706_s_at | CLDN23 | 4.27E-20 | 3.0757 | 3.47 | 93.8 | 88.2-97.1 |
| 228707_at | CLDN23 | 4.27E-20 | 3.072 | 3.47 | 93.8 | 88.3-97.1 |
| 223551_at | PKIB | 5.94E-20 | 3.1219 | 3.28 | 94.1 | 88.6-97.3 |
| 231120_x_at | PKIB | 5.94E-20 | 3.1226 | 3.28 | 94.1 | 88.6-97.3 |
| 226492_at | SEMA6D | 6.07E-20 | 3.0898 | 4.27 | 93.9 | 88.4-97.2 |
| 220026_at | CLCA4 | 1.25E-19 | 3.1909 | 16.3 | 94.5 | 89.2-97.5 |
| 224836_at | TP53INP2 | 1.45E-19 | 3.0248 | 3.78 | 93.5 | 87.9-96.9 |
| 220834_at | MS4A12 | 2.13E-19 | 3.29 | 7.15 | 95 | 90-97.8 |
| 224412_s_at | TRPM6 | 2.25E-19 | 3.01 | 7.5 | 93.4 | 87.7-96.8 |
| 220037_s_at | XLKD1 | 3.60E-19 | 2.9412 | 7.44 | 92.9 | 87.1-96.6 |
| 219059_s_at | XLKD1 | 3.60E-19 | 2.942 | 7.44 | 92.9 | 87-96.6 |
| 209612_s_at | ADH1B:ADH1A | 4.70E-19 | 3.0183 | 4.67 | 93.4 | 87.7-96.9 |
| 209613_s_at | ADH1B:ADH1A | 4.70E-19 | 3.0147 | 4.67 | 93.4 | 87.7-96.9 |
| 200845_s_at | LOC389249:PRDX6 | 5.56E-19 | 2.8412 | 2.76 | 92.2 | 86.1-96.1 |
| 209301_at | CA2 | 5.87E-19 | 2.9662 | 9.22 | 93.1 | 87.3-96.7 |
| 208399_s_at | EDN3 | 6.42E-19 | 2.9342 | 11.21 | 92.9 | 87-96.5 |
| 228961_at | MIER3 | 9.14E-19 | 2.9183 | 3.91 | 92.8 | 86.9-96.5 |
| 231975_s_at | MIER3 | 9.14E-19 | 2.9172 | 3.91 | 92.8 | 86.8-96.4 |
| 204719_at | ABCA8 | 1.26E-18 | 2.9172 | 6.82 | 92.8 | 86.8-96.5 |
| 207761_s_at | METTL7A | 1.35E-18 | 3.1247 | 1.93 | 94.1 | 88.6-97.3 |
| 207977_s_at | DPT | 1.53E-18 | 2.8967 | 9.07 | 92.6 | 86.6-96.4 |
| 213068_at | DPT | 1.53E-18 | 2.8973 | 9.07 | 92.6 | 86.7-96.4 |
| 213071_at | DPT | 1.53E-18 | 2.8962 | 9.07 | 92.6 | 86.7-96.4 |
| 218756_s_at | MRM1:MGC4172 | 1.74E-18 | 2.9041 | 3.74 | 92.7 | 86.6-96.4 |
| 212288_at | FNBP1:C1orf85:CCT3 | 1.76E-18 | 3.0475 | 2.04 | 93.6 | 88-97 |
| 204036_at | EDG2 | 2.44E-18 | 2.8853 | 3.19 | 92.5 | 86.4-96.3 |
| 202037_s_at | SFRP1 | 2.77E-18 | 2.8647 | 15.53 | 92.4 | 86.3-96.2 |
| 206143_at | SLC26A3 | 4.18E-18 | 2.9202 | 21.4 | 92.8 | 86.9-96.5 |
| 215657_at | SLC26A3 | 4.18E-18 | 2.9226 | 21.4 | 92.8 | 86.9-96.5 |
| 231773_at | ANGPTL1 | 1.42E-17 | 2.7938 | 4.64 | 91.9 | 85.6-95.9 |
| 205480_s_at | UGP2 | 1.64E-17 | 2.7911 | 2.41 | 91.9 | 85.6-95.9 |
| 204955_at | SRPX | 1.79E-17 | 2.7753 | 3.91 | 91.7 | 85.5-95.8 |
| 222722_at | OGN | 2.25E-17 | 2.7048 | 13.67 | 91.2 | 84.8-95.4 |
| 202555_s_at | MYLK | 2.33E-17 | 2.773 | 3.99 | 91.7 | 85.4-95.8 |
| 224823_at | MYLK | 2.33E-17 | 2.7727 | 3.99 | 91.7 | 85.4-95.8 |
| 225575_at | LIFR | 2.45E-17 | 2.7823 | 6.36 | 91.8 | 85.5-95.8 |
| 214142_at | LOC653808:ZG16 | 2.51E-17 | 2.8119 | 8.14 | 92 | 85.8-95.9 |
| 206710_s_at | EPB41L3 | 2.62E-17 | 2.8298 | 2.51 | 92.1 | 85.9-96.1 |
| 205464_at | SCNN1B | 3.20E-17 | 2.7209 | 16.15 | 91.3 | 84.9-95.5 |
| 220812_s_at | HHLA2 | 3.47E-17 | 2.6717 | 10.02 | 90.9 | 84.4-95.3 |
| 203913_s_at | HPGD | 3.65E-17 | 2.8441 | 3.21 | 92.2 | 86.1-96.1 |
| 203914_x_at | HPGD | 3.65E-17 | 2.8456 | 3.21 | 92.3 | 86.1-96.1 |
| 211548_s_at | HPGD | 3.65E-17 | 2.8446 | 3.21 | 92.3 | 86.1-96.1 |
| 211549_s_at | HPGD | 3.65E-17 | 2.8466 | 3.21 | 92.3 | 86.1-96.1 |
| 206198_s_at | CEACAM7 | 3.96E-17 | 2.7882 | 10.4 | 91.8 | 85.5-95.9 |
| 206199_at | CEACAM7 | 3.96E-17 | 2.7855 | 10.4 | 91.8 | 85.6-95.8 |
| 211848_s_at | CEACAM7 | 3.96E-17 | 2.7882 | 10.4 | 91.8 | 85.6-95.8 |
| 226430_at | SMAD5:RELL1 | 4.10E-17 | 2.6968 | 2.5 | 91.1 | 84.6-95.4 |
| 202992_at | C7 | 4.40E-17 | 2.7163 | 6.67 | 91.3 | 84.9-95.5 |
| 205112_at | PLCE1 | 6.91E-17 | 2.7542 | 2.43 | 91.6 | 85.2-95.7 |
| 229839_at | SCARA5 | 1.25E-16 | 2.6897 | 2.86 | 91.1 | 84.6-95.3 |
| 235849_at | SCARA5 | 1.25E-16 | 2.6918 | 2.86 | 91.1 | 84.5-95.3 |
| 209763_at | CHRDL1 | 1.49E-16 | 2.6093 | 13.88 | 90.4 | 83.7-94.9 |
| 205259_at | NR3C2 | 2.08E-16 | 2.5812 | 3.19 | 90.2 | 83.4-94.7 |
| 202242_at | TSPAN7 | 2.13E-16 | 2.6519 | 3.68 | 90.8 | 84.1-95.1 |
| 203000_at | STMN2 | 2.25E-16 | 2.6115 | 6.13 | 90.4 | 83.7-94.9 |
| 203001_s_at | STMN2 | 2.25E-16 | 2.6141 | 6.13 | 90.4 | 83.7-94.9 |
| 209074_s_at | FAM107A | 2.52E-16 | 2.6171 | 2.92 | 90.5 | 83.8-94.9 |
| 202920_at | ANK2 | 2.59E-16 | 2.6499 | 6.76 | 90.7 | 84.1-95.1 |
| 213317_at | CLIC5 | 3.68E-16 | 2.696 | 2.37 | 91.1 | 84.6-95.4 |
| 204697_s_at | CHGA | 5.54E-16 | 2.6087 | 7.21 | 90.4 | 83.7-94.9 |
| 212814_at | KIAA0828 | 5.58E-16 | 2.5495 | 4.09 | 89.9 | 83-94.5 |
| 225275_at | EDIL3 | 6.21E-16 | 2.6252 | 2.96 | 90.5 | 83.9-95 |
| 208370_s_at | DSCR1 | 6.21E-16 | 2.6146 | 2.14 | 90.4 | 83.7-94.9 |
| 209147_s_at | PPAP2A | 6.43E-16 | 2.5961 | 2.4 | 90.3 | 83.6-94.8 |
| 210946_at | PPAP2A | 6.43E-16 | 2.5967 | 2.4 | 90.3 | 83.5-94.8 |
| 202731_at | PDCD4 | 8.67E-16 | 2.4558 | 2.6 | 89 | 82-94 |
| 219799_s_at | DHRS9:GORASP2 | 1.09E-15 | 2.7368 | 1.51 | 91.4 | 85.1-95.6 |
| 223952_x_at | DHRS9:GORASP2 | 1.09E-15 | 2.7334 | 1.51 | 91.4 | 85-95.6 |
| 224009_x_at | DHRS9:GORASP2 | 1.09E-15 | 2.7359 | 1.51 | 91.4 | 85.1-95.6 |
| 236313_at | CDKN2B | 1.34E-15 | 2.5442 | 10.09 | 89.8 | 83-94.5 |
| 231925_at | P2RY1 | 1.41E-15 | 2.5354 | 3.6 | 89.8 | 82.9-94.4 |
| 238143_at | LOC646627 | 1.58E-15 | 1.9142 | 5.02 | 83.1 | 74.8-89.4 |
| 224480_s_at | LPAAT-THETA | 2.06E-15 | 2.3867 | 2.63 | 88.4 | 81.1-93.5 |
| 212230_at | PPAP2B | 2.44E-15 | 2.5821 | 1.81 | 90.2 | 83.4-94.7 |
| 207080_s_at | PYY | 3.50E-15 | 2.4811 | 11.71 | 89.3 | 82.3-94.1 |
| 205200_at | CLEC3B | 4.65E-15 | 2.4426 | 4.46 | 88.9 | 81.8-93.8 |
| 228133_s_at | NDE1 | 4.84E-15 | 2.514 | 1.99 | 89.6 | 82.7-94.3 |
| 214038_at | CCL8 | 5.75E-15 | 2.4504 | 7.51 | 89 | 81.9-93.9 |
| 219014_at | PLAC8 | 5.76E-15 | 2.4248 | 3.87 | 88.7 | 81.6-93.7 |
| 219796_s_at | MUCDHL | 5.81E-15 | 2.4346 | 3.06 | 88.8 | 81.6-93.8 |
| 220075_s_at | MUCDHL | 5.81E-15 | 2.4354 | 3.06 | 88.8 | 81.7-93.8 |
| 215299_x_at | SULT1A1:SULT1A2 | 7.17E-15 | 2.4231 | 3.37 | 88.7 | 81.5-93.7 |
| 233565_s_at | SDCBP2 | 9.28E-15 | 2.4871 | 2.37 | 89.3 | 82.3-94.1 |
| 228885_at | RPL24:LOC731365 | 1.23E-14 | 2.5555 | 1.63 | 89.9 | 83.1-94.6 |
| 209687_at | CXCL12 | 1.54E-14 | 2.4322 | 3.79 | 88.8 | 81.7-93.8 |
| 218546_at | C1orf115 | 1.95E-14 | 2.4106 | 2.95 | 88.6 | 81.4-93.6 |
| 205097_at | SLC26A2 | 2.08E-14 | 2.4219 | 7.83 | 88.7 | 81.5-93.7 |
| 224959_at | SLC26A2 | 2.08E-14 | 2.4229 | 7.83 | 88.7 | 81.6-93.7 |
| 224963_at | SLC26A2 | 2.08E-14 | 2.4205 | 7.83 | 88.7 | 81.5-93.7 |
| 204069_at | MEIS1 | 2.49E-14 | 2.3759 | 5.44 | 88.3 | 81-93.4 |
| 223121_s_at | SFRP2 | 3.05E-14 | 2.3937 | 7.39 | 88.4 | 81.2-93.5 |
| 223122_s_at | SFRP2 | 3.05E-14 | 2.3967 | 7.39 | 88.5 | 81.2-93.5 |
| 209191_at | TUBB3:MC1R:TUBB6 | 3.55E-14 | 2.3684 | 5.9 | 88.2 | 80.9-93.3 |
| 201348_at | GPX3 | 5.68E-14 | 2.36 | 2.57 | 88.1 | 80.8-93.2 |
| 214091_s_at | GPX3 | 5.68E-14 | 2.3613 | 2.57 | 88.1 | 80.7-93.3 |
| 228766_at | CD36 | 6.27E-14 | 2.4671 | 1.65 | 89.1 | 82.1-94 |
| 221896_s_at | HIGD1A | 6.29E-14 | 2.3985 | 2.09 | 88.5 | 81.3-93.5 |
| 201865_x_at | NR3C1 | 7.65E-14 | 2.3539 | 2.51 | 88 | 80.6-93.2 |
| 211671_s_at | NR3C1 | 7.65E-14 | 2.3517 | 2.51 | 88 | 80.7-93.2 |
| 206149_at | LOC63928 | 8.09E-14 | 2.3513 | 3.2 | 88 | 80.7-93.2 |
| 228846_at | MXD1 | 8.35E-14 | 2.4074 | 2.11 | 88.6 | 81.4-93.6 |
| 225602_at | C9orf19 | 1.74E-13 | 2.3619 | 1.95 | 88.1 | 80.9-93.3 |
| 225604_s_at | C9orf19 | 1.74E-13 | 2.3646 | 1.95 | 88.1 | 80.8-93.3 |
| 201893_x_at | DCN | 2.05E-13 | 2.311 | 5.62 | 87.6 | 80.2-92.9 |
| 209335_at | DCN | 2.05E-13 | 2.3115 | 5.62 | 87.6 | 80.3-92.9 |
| 211813_x_at | DCN | 2.05E-13 | 2.3145 | 5.62 | 87.6 | 80.2-92.9 |
| 211896_s_at | DCN | 2.05E-13 | 2.3124 | 5.62 | 87.6 | 80.2-92.9 |
| 204818_at | HSD17B2 | 2.32E-13 | 2.196 | 4.76 | 86.4 | 78.7-92 |
| 204931_at | TCF21 | 2.51E-13 | 2.306 | 2.34 | 87.6 | 80.1-92.9 |
| 204438_at | MRC1 | 2.72E-13 | 2.3105 | 2.6 | 87.6 | 80.2-92.9 |
| 206262_at | ADH1A:ADH1C | 2.94E-13 | 2.3719 | 3.1 | 88.2 | 80.9-93.3 |
| 205433 at | BCHE | 3.18E-13 | 2.2514 | 6.21 | 87 | 79.5-92.5 |
| 225242_s_at | CCDC80 | 3.60E-13 | 2.2316 | 3.67 | 86.8 | 79.2-92.3 |
| 207980_s_at | CITED2 | 5.29E-13 | 2.2709 | 1.64 | 87.2 | 79.7-92.6 |
| 209357_at | CITED2 | 5.29E-13 | 2.2704 | 1.64 | 87.2 | 79.7-92.6 |
| 208383_s_at | PCK1 | 5.74E-13 | 2.3242 | 3.6 | 87.7 | 80.4-93 |
| 206385_s_at | ANK3:LOC729184:LOC7311 86 | 7.46E-13 | 2.2305 | 2.45 | 86.8 | 79.2-92.2 |
| 203305_at | F13A1 | 8.82E-13 | 2.1821 | 4.74 | 86.2 | 78.6-91.8 |
| 206134_at | ADAMDEC1 | 9.04E-13 | 2.2607 | 3.19 | 87.1 | 79.5-92.5 |
| 215118_s_at | AHNAK:IGHG1 | 9.41E-13 | 2.3277 | 1.73 | 87.8 | 80.3-93 |
| 217022_s_at | AHNAK:IGHG1 | 9.41E-13 | 2.3254 | 1.73 | 87.8 | 80.4-93 |
| 223395_at | ABI3BP | 9.47E-13 | 2.2207 | 3.62 | 86.7 | 79-92.2 |
| 225626_at | PAG1 | 1.11E-12 | 2.2136 | 2.68 | 86.6 | 78.9-92.1 |
| 213953_at | KRT20 | 1.26E-12 | 2.0964 | 5.49 | 85.3 | 77.4-91.1 |
| 226594 at | ENTPD5 | 1.43E-12 | 2.1687 | 3.01 | 86.1 | 78.4-91.8 |
| 209373_at | MALL | 2.10E-12 | 2.1597 | 3.96 | 86 | 78.3-91.7 |
| 212713_at | MFAP4 | 2.96E-12 | 2.1797 | 2.51 | 86.2 | 78.5-91.9 |
| 208920_at | SRI | 3.03E-12 | 2.0982 | 2.43 | 85.3 | 77.4-91.1 |
| 201739_at | SGK | 3.12E-12 | 2.1221 | 4.08 | 85.6 | 77.8-91.3 |
| 214696_at | MGC14376 | 3.39E-12 | 2.1045 | 2.58 | 85.4 | 77.5-91.2 |
| 204034_at | ETHE1 | 3.46E-12 | 2.2127 | 1.75 | 86.6 | 79-92.1 |
| 209667_at | CES2 | 3.51E-12 | 2.102 | 2.87 | 85.3 | 77.4-91.2 |
| 209668_x_at | CES2 | 3.51E-12 | 2.0978 | 2.87 | 85.3 | 77.4-91.1 |
| 213509_x_at | CES2 | 3.51E-12 | 2.097 | 2.87 | 85.3 | 77.4-91.1 |
| 202291_s_at | MGP:C12orf46 | 3.63E-12 | 2.1448 | 4.68 | 85.8 | 78-91.5 |
| 209167_at | GPM6B | 4.04E-12 | 2.1568 | 3.59 | 86 | 78.2-91.6 |
| 209170_s_at | GPM6B | 4.04E-12 | 2.1581 | 3.59 | 86 | 78.2-91.6 |
| 225720_at | SYNPO2 | 5.07E-12 | 2.1053 | 6.43 | 85.4 | 77.5-91.2 |
| 225721_at | SYNPO2 | 5.07E-12 | 2.1069 | 6.43 | 85.4 | 77.5-91.2 |
| 225894_at | SYNPO2 | 5.07E-12 | 2.1043 | 6.43 | 85.4 | 77.5-91.2 |
| 225895_at | SYNPO2 | 5.07E-12 | 2.1069 | 6.43 | 85.4 | 77.5-91.2 |
| 227662_at | SYNPO2 | 5.07E-12 | 2.1043 | 6.43 | 85.4 | 77.5-91.2 |
| 206422_at | GCG | 5.58E-12 | 2.1185 | 12.56 | 85.5 | 77.7-91.3 |
| 205593_s_at | PDE9A | 6.56E-12 | 2.1443 | 3.91 | 85.8 | 78-91.5 |
| 220376_at | LRRC19 | 6.75E-12 | 2.076 | 4.51 | 85 | 77.1-91 |
| 204130_at | HSD11B2 | 6.97E-12 | 2.0896 | 2.53 | 85.2 | 77.3-91.1 |
| 224964_s_at | GNG2 | 7.25E-12 | 2.1405 | 1.91 | 85.8 | 78-91.5 |
| 219508_at | GCNT3 | 8.39E-12 | 2.0843 | 3.94 | 85.1 | 77.2-91 |
| 211645_x_at | No Symbol | 9.19E-12 | 2.1536 | 1.61 | 85.9 | 78.2-91.6 |
| 212233_at | No Symbol | 9.19E-12 | 2.1541 | 1.61 | 85.9 | 78.1-91.6 |
| 212764_at | No Symbol | 9.19E-12 | 2.1541 | 1.61 | 85.9 | 78.1-91.6 |
| 214777_at | No Symbol | 9.19E-12 | 2.1543 | 1.61 | 85.9 | 78.2-91.6 |
| 217235_x_at | No Symbol | 9.19E-12 | 2.155 | 1.61 | 85.9 | 78.2-91.6 |
| 225710_at | No Symbol | 9.19E-12 | 2.1539 | 1.61 | 85.9 | 78.1-91.6 |
| 226333_at | No Symbol | 9.19E-12 | 2.1529 | 1.61 | 85.9 | 78.1-91.6 |
| 226834_at | No Symbol | 9.19E-12 | 2.154 | 1.61 | 85.9 | 78.1-91.6 |
| 227061_at | No Symbol | 9.19E-12 | 2.152 | 1.61 | 85.9 | 78.1-91.6 |
| 228504 at | No Symbol | 9.19E-12 | 2.1533 | 1.61 | 85.9 | 78.1-91.6 |
| 228507_at | No Symbol | 9.19E-12 | 2.1538 | 1.61 | 85.9 | 78.2-91.6 |
| 228640_at | No Symbol | 9.19E-12 | 2.1532 | 1.61 | 85.9 | 78.2-91.6 |
| 228854_at | No Symbol | 9.19E-12 | 2.1532 | 1.61 | 85.9 | 78.2-91.7 |
| 236300_at | No Symbol | 9.19E-12 | 2.1532 | 1.61 | 85.9 | 78.2-91.6 |
| 242317_at | No Symbol | 9.19E-12 | 2.1529 | 1.61 | 85.9 | 78.1-91.6 |
| 2t0524_x_at | No Symbol | 9.19E-12 | 2.1554 | 1.61 | 85.9 | 78.2-91.7 |
| 224989_at | No Symbol | 9.19E-12 | 2.1526 | 1.61 | 85.9 | 78.2-91.6 |
| 227052_at | No Symbol | 9.19E-12 | 2.151 | 1.61 | 85.9 | 78.1-91.6 |
| 227682_at | No Symbol | 9.19E-12 | 2.1549 | 1.61 | 85.9 | 78.2-91.6 |
| 235146_at | No Symbol | 9.19E-12 | 2.1527 | 1.61 | 85.9 | 78.2-91.7 |
| 207126_x_at | UGT1A10:UGT1A7:UGT1A8: UGT1A1:UGT1A9:UGT1A6: UGT1A5:UGT1A3:UGT1A4 | 9.98E-12 | 2.0677 | 4.56 | 84.9 | 77-90.9 |
| 206094_x_at | UGT1A10:UGT1A7:UGT1A8: UGT1A1:UGT1A9:UGT1A6: UGT1A5:UGT1A3:UGT1A4 | 1.01E-11 | 2.0693 | 4.56 | 85 | 77-90.8 |
| 208596_s_at | UGT1A10:UGT1A7:UGT1A8: UGT1A1:UGT1A9:UGT1A6: UGT1A5:UGT1A3:UGT1A4 | 1.01E-11 | 2.0662 | 4.56 | 84.9 | 76.9-90.8 |
| 221305_s_at | UGT1A10:UGT1A7:UGT1A8: UGT1A1:UGT1A9:UGT1A6: UGT1A5:UGT1A3:UGT1A4 | 1.03E-11 | 2.0663 | 4.56 | 84.9 | 77-90.9 |
| 204532_x_at | UGT1A10:UGT1A7:UGT1A8: UGT1A1:UGT1A9:UGT1A6: UGT1A5:UGT1A3:UGT1A4 | 1.18E-11 | 2.0678 | 4.56 | 84.9 | 77-90.9 |
| 215125_s_at | UGT1A10:UGT1A7:UGT1A8: UGT1A1:UGT1A9:UGT1A6: UGT1A5.UGT1A3:UGT1A4 | 1.18E-11 | 2.0682 | 4.56 | 84.9 | 77-90.9 |
| 209791_at | PAD12 | 1.46E-11 | 2.0258 | 3.98 | 84.4 | 76.4-90.5 |
| 219669_at | CD177 | 1.49E-11 | 2.0618 | 5.77 | 84.9 | 76.9-90.8 |
| 201539_s_at | FHL1 | 1.63E-11 | 2.0448 | 3.25 | 84.7 | 76.7-90.7 |
| 201540_at | FHL1 | 1.63E-11 | 2.0466 | 3.25 | 84.7 | 76.7-90.7 |
| 210298_x_at | FHL1 | 1.63E-11 | 2.0438 | 3.25 | 84.7 | 76.7-90.6 |
| 210299_s_at | FHL1 | 1.63E-11 | 2.0458 | 3.25 | 84.7 | 76.6-90.6 |
| 214505_s_at | FHL1 | 1.63E-11 | 2.0459 | 3.25 | 84.7 | 76.7-90.6 |
| 206576_s_at | CEACAM1 | 2.66E-11 | 2.0448 | 2.85 | 84.7 | 76.7-90.7 |
| 209498_at | CEACAM1 | 2.66E-11 | 2.0446 | 2.85 | 84.7 | 76.7-90.7 |
| 211889_x_at | CEACAM1 | 2.66E-11 | 2.0471 | 2.85 | 84.7 | 76.7-90.7 |
| 202994_s_at | FBLN1 | 2.82E-11 | 2.0183 | 3.55 | 84.4 | 76.3-90.4 |
| 202995_s_at | FBLN1 | 2.82E-11 | 2.0191 | 3.55 | 84.4 | 76.3-90.4 |
| 201427_s_at | SEPP1 | 2.83E-11 | 2.034 | 4.15 | 84.5 | 76.5-90.6 |
| 212956_at | TBC1D9 | 3.89E-11 | 2.1428 | 1.54 | 85.8 | 78-91.5 |
| 203963_at | CA12 | 4.32E-11 | 2.0024 | 2.65 | 84.2 | 76.1-90.2 |
| 204508_s_at | CA12 | 4.32E-11 | 2.0029 | 2.65 | 84.2 | 76.2-90.3 |
| 210735_s_at | CA12 | 4.32E-11 | 2.0016 | 2.65 | 84.2 | 76.1-90.2 |
| 214164_x_at | CA12 | 4.32E-11 | 2.0016 | 2.65 | 84.2 | 76.1-90.3 |
| 215867_x_at | CA12 | 4.32E-11 | 2.0027 | 2.65 | 84.2 | 76.1-90.3 |
| 203881_s_at | DMD | 5.01E-11 | 2.0159 | 4.74 | 84.3 | 76.2-90.4 |
| 213624_at | SMPDL3A | 5.84E-11 | 1.9409 | 3.14 | 83.4 | 75.2-89.6 |
| 226304_at | HSPB6 | 6.16E-11 | 1.9486 | 6.88 | 83.5 | 75.4-89.8 |
| 206561_s_at | LOC441282:AKR1B10:LOC3 40888 | 7.91E-11 | 1.9478 | 5.17 | 83.5 | 75.3-89.7 |
| 203343_at | UGDH | 8.72E-11 | 1.9619 | 2.4 | 83.7 | 75.6-89.9 |
| 205892_s_at | FABP1 | 9.02E-11 | 1.9694 | 6.54 | 83.8 | 75.6-89.9 |
| 206637_at | P2RY14 | 1.31E-10 | 1.9821 | 2 | 83.9 | 75.8-90.1 |
| 202266_at | TTRAP | 1.59E-10 | 1.9147 | 2.2 | 83.1 | 74.9-89.4 |
| 206000_at | LOC642840:MEP1A:LOC389 747:LOC644777 | 2.07E-10 | 1.9873 | 3.17 | 84 | 75.9-90.1 |
| 201496_x_at | MYH11 | 2.15E-10 | 1.8577 | 5.35 | 82.4 | 74-88.8 |
| 201497_x_at | MYH11 | 2.15E-10 | 1.8599 | 5.35 | 82.4 | 74.1-88.8 |
| 207961_x_at | MYH11 | 2.15E-10 | 1.859 | 5.35 | 82.4 | 74.1-88.9 |
| 201495_x_at | MYH11 | 2.15E-10 | 1.8585 | 5.35 | 82.4 | 74-88.8 |
| 204388_s_at | MAOA | 2.49E-10 | 2.0297 | 2.18 | 84.5 | 76.4-90.5 |
| 204389_at | MAOA | 2.49E-10 | 2.0256 | 2.18 | 84.4 | 76.5-90.5 |
| 212741_at | MAOA | 2.49E-10 | 2.0267 | 2.18 | 84.5 | 76.5-90.5 |
| 214598_at | CLDN8 | 2.92E-10 | 1.862 | 12.96 | 82.4 | 74.2-88.9 |
| 202838_at | FUCA1 | 4.24E-10 | 1.8831 | 2.03 | 82.7 | 74.4-89.1 |
| 217897_at | MB:FXYD6 | 6.39E-10 | 1.8109 | 2.92 | 81.7 | 73,3-88.3 |
| 220468_at | ARL14 | 7.01E-10 | 1.8242 | 3.06 | 81.9 | 73.5-88.4 |
| 201920_at | SLC20A1 | 7.65E-10 | 1.7849 | 4.04 | 81.4 | 72.9-88.1 |
| 210302_s_at | MAB21L2 | 1.05E-09 | 1.7744 | 5.2 | 81.3 | 72.8-87.9 |
| 209114_at | TSPAN1 | 1.16E-09 | 1.7896 | 2.16 | 81.5 | 73-88.1 |
| 220266_s_at | KLF4 | 1.19E-09 | 1.8515 | 2.27 | 82.3 | 73.8-88.7 |
| 221841_s_at | KLF4 | 1.19E-09 | 1.8493 | 2.27 | 82.2 | 73.9-88.8 |
| 209283_at | CRYAB | 1.47E-09 | 1.7693 | 3.91 | 81.2 | 72.7-87.8 |
| 223484_at | C15orf48 | 1.54E-09 | 1.8189 | 3.07 | 81.8 | 73.4-88.4 |
| 205412_at | ACAT1 | 1.70E-09 | 1.8575 | 1.77 | 82.3 | 73.9-88.8 |
| 202888_s_at | ANPEP | 2.04E-09 | 1.7673 | 8.18 | 81.2 | 72.6-87.8 |
| 225458_at | EXOC3 | 2.25E-09 | 1.7957 | 3.17 | 81.5 | 73.1-88.1 |
| 204834_at | FGL2 | 2.64E-09 | 1.8245 | 2.07 | 81.9 | 73.4-88.5 |
| 227265_at | FGL2 | 2.64E-09 | 1.8246 | 2.07 | 81.9 | 73.6-88.4 |
| 228469_at | PPID | 3.34E-09 | 1.8032 | 1.73 | 81.6 | 73.2-88.3 |
| 221004_s_at | ITM2C | 3.87E-09 | 1.7063 | 2.22 | 80.3 | 71.8-87.1 |
| 213921_at | SST | 4.16E-09 | 1.7652 | 6.14 | 81.1 | 72.6-87.8 |
| 230087_at | PRIMA1 | 4.74E-09 | 1.7085 | 2.75 | 80.4 | 71.8-87.2 |
| 201842_s_at | EFEMP1 | 5.80E-09 | 1.8196 | 1.69 | 81.9 | 73.4-88.4 |
| 222162_s_at | ADAMTS1 | 6.42E-09 | 1.7467 | 2.37 | 80.9 | 72.3-87.6 |
| 210517_s_at | AKAP12 | 7.41E-09 | 1.7454 | 2.83 | 80.9 | 72.3-87.6 |
| 227529_s_at | AKAP12 | 7.41E-09 | 1.7437 | 2.83 | 80.8 | 72.3-87.6 |
| 228750_at | COL14A1 | 7.60E-09 | 1.7813 | 1.67 | 81.3 | 72.9-88 |
| 219948_x_at | LOC642329:UGT2A3 | 8.64E-09 | 1.7422 | 4.89 | 80.8 | 72.2-87.5 |
| 208131_s_at | PTGIS | 9.15E-09 | 1.7212 | 3.98 | 80.5 | 71.9-87.3 |
| 207432_at | BTN2A2:BEST2 | 9.19E-09 | 1.7179 | 3.63 | 80.5 | 71.9-87.3 |
| 219607_s_at | MS4A4A:LOC643680 | 9.59E-09 | 1.8197 | 1.55 | 81.9 | 73.4-88.4 |
| 204688 at | SGCE | 1.02E-08 | 1.7438 | 2.03 | 80.8 | 72.3-87.6 |
| 207134_x_at | TPSB2 | 1.24E-08 | 1.3713 | 1.87 | 75.4 | 66.3-82.9 |
| 209156_s_at | COL6A2 | 1.30E-08 | 1.68 | 2.08 | 80 | 71.3-86.8 |
| 202741_at | PRKACB | 1.30E-08 | 1.7217 | 2.18 | 80.5 | 71.9-87.3 |
| 202742_s_at | PRKACB | 1.30E-08 | 1.721 | 2.18 | 80.5 | 71.9-87.3 |
| 200795_at | SPARC1 | 1.33E-08 | 1.6379 | 2.85 | 79.4 | 70.7-86.3 |
| 219543_at | PBLD | 1.38E-08 | 1.6981 | 2.74 | 80.2 | 71.6-87.1 |
| 225207_at | PDK4 | 1.45E-08 | 1.705 | 3.02 | 80.3 | 71.7-87.2 |
| 202222_s_at | SPEG:LOC729871:DES | 1.47E-08 | 1.7229 | 6.66 | 80.6 | 72-87.3 |
| 214027_x_at | SPEG:LOC729871:DES | 1.47E-08 | 1.7254 | 6.66 | 80.6 | 72-87.4 |
| 239272_at | MMP28 | 1.80E-08 | 1.6622 | 2.47 | 79.7 | 71-86.6 |
| 222453_at | THEM4:CYBRD1 | 1.84E-08 | 1.6934 | 2.7 | 80.1 | 71.6-87 |
| 212195_at | MAGEA4:IL6ST | 1.87E-08 | 1.764 | 1.58 | 81.1 | 72.6-87.8 |
| 203980_at | FABP4 | 2.01E-08 | 1.6762 | 5.55 | 79.9 | 71.3-86.8 |
| 211985_s_at | CALM1 | 2.12E-08 | 1.6839 | 1.72 | 80 | 71.4-86.9 |
| 221747 at | TNS1:AKAP9 | 2.21E-08 | 1.7434 | 2.01 | 80.8 | 72.2-87.5 |
| 221748_s_at | TNS1:AKAP9 | 2.21E-08 | 1.7409 | 2.01 | 80.8 | 72.3-87.5 |
| 201324_at | EMP1 | 2.59E-08 | 1.7149 | 1.95 | 80.4 | 71.9-87.3 |
| 212397_at | LOC643244:RDX | 3.06E-08 | 1.7431 | 1.9 | 80.8 | 72.3-87.6 |
| 205382_s_at | CFD | 3.06E-08 | 1.6462 | 2.81 | 79.5 | 70.8-86.4 |
| 201141_at | GPNMB | 3.11E-08 | 1.6928 | 1.99 | 80.1 | 71.5-87 |
| 205683_x_at | TPSAB1,TPSAB1 | 3.52E-08 | 1.7075 | 1.93 | 80.3 | 71.8-87.1 |
| 210084_x_at | TPSAB1,TPSAB1 | 3.52E-08 | 1.7045 | 1.93 | 80.3 | 71.8-87.1 |
| 215382_x_at | TPSAB1,TPSAB1 | 3.52E-08 | 1.7091 | 1.93 | 80.4 | 71.7-87.2 |
| 216474_x_at | TPSAB1,TPSAB1 | 3.52E-08 | 1.7069 | 1.93 | 80.3 | 71.7-87.2 |
| 227006_at | PPP1R14A | 3.54E-08 | 1.619 | 2.51 | 79.1 | 70.3-86.1 |
| 242601_at | LOC253012 | 3.59E-08 | 1.6517 | 4.46 | 79.6 | 70.8-86.5 |
| 212730_at | DMN | 4.36E-08 | 1.6046 | 4.05 | 78.9 | 70.2-86 |
| 226818_at | MPEG1 | 4.58E-08 | 1.6466 | 1.86 | 79.5 | 70.8-86.5 |
| 226841_at | MPEG1 | 4.58E-08 | 1.6494 | 1.86 | 79.5 | 70.8-86.5 |
| 203474_at | IQGAP2 | 5.06E-08 | 1.6275 | 2.51 | 79.2 | 70.6-86.2 |
| 203766_s_at | LMOD1 | 5.55E-08 | 1.58 | 2.97 | 78.5 | 69.8-85.6 |
| 214916_x_at | LOC652128:IGHG1:IGHM:IG HV4-31:LOC647189:IGHV1-69:IGHA1:IL8:EXOC7:SIX6:I GHD:IGH@:IGHG3:C12orf32 :ZCWPW2:IFI6:IGHG4:IGHA 2:IGHG2:RAC1 | 5.75E-08 | 1.6352 | 3.1 | 79.3 | 70.6-86.3 |
| 226303_at | PGM5 | 5.87E-08 | 1.6215 | 5.87 | 79.1 | 70.4-86.2 |
| 225442_at | DDR2 | 5.89E-08 | 1.562 | 2.61 | 78.3 | 69.4-85.4 |
| 227561_at | DDR2 | 5.89E-08 | 1.5604 | 2.61 | 78.2 | 69.4-85.5 |
| 202760_s_at | AKAP2:PALM2:PALM2-AKAP2 | 6.28E-08 | 1.6137 | 2.17 | 79 | 70.3-86.1 |
| 226694_at | AKAP2:PALM2:PALM2-AKAP2 | 6.28E-08 | 1.6143 | 2.17 | 79 | 70.2-86.1 |
| 204894_s_at | AOC3 | 6.43E-08 | 1.556 | 2.92 | 78.2 | 69.4-85.4 |
| 203058_s_at | PAPSS2 | 6.43E-08 | 1.6171 | 1.97 | 79.1 | 70.4-86.1 |
| 203060_s_at | PAPSS2 | 6.43E-08 | 1.6159 | 1.97 | 79 | 70.4-86.1 |
| 208763_s_at | TSC22D3 | 7.22E-08 | 1.6098 | 2.76 | 79 | 70.2-86 |
| 222717_at | SDPR | 9.62E-08 | 1.5998 | 2.21 | 78.8 | 70.1-85.9 |
| 203680_at | PRKAR2B | 1.05E-07 | 1.6759 | 2.1 | 79.9 | 71.2-86.8 |
| 201041_s_at | DUSP1 | 1.06E-07 | 1.5295 | 2.44 | 77.8 | 68.9-85 |
| 209374_s_at | LOC652128:IGHG1:IGHM:IG HV4-31:LOC647189:IGHV1-69:IGHA1:IL8:EXOC7:SIX6:I GHD:IGH@:IGHG3:C12orf32 :ZCWPW2:IFI6:IGHG4:IGHA 2:IGHG2:RAC1 | 1.12E-07 | 1-6333 | 3.1 | 79.3 | 70.6-86.3 |
| 216491_x_at | LOC652128:IGHG1:IGHM:IG HV4-31:LOC647189:IGHV1-69:IGHA1:IL8:EXOC7:SIX6:I GHD:IGH@:IGHG3:C12orf32 :ZCWPW2:IFI6:IGHG4:IGHA 2:IGHG2:RAC1 | 1.12E-07 | 1.6335 | 3.1 | 79.3 | 70.5-86.3 |
| 229831_at | CNTN3 | 1.20E-07 | 1.6157 | 3.93 | 79 | 70.2-86.1 |
| 235766_x_at | RAB27A | 1.22E-07 | 1.5984 | 1.79 | 78.8 | 70.1-85.9 |
| 229070_at | C6orf105 | 1.38E-07 | 1.6049 | 2.13 | 78.9 | 70.1-86 |
| 226654_at | MUC12 | 1.53E-07 | 1.4559 | 4.89 | 76.7 | 67.7-84.1 |
| 202686_s_at | AXL | 1.59E-07 | 1.5758 | 1.71 | 78.5 | 69.7-85.6 |
| 205403_at | IL1R2 | 1.73E-07 | 1.5237 | 2.54 | 77.7 | 68.8-84.9 |
| 211372_s_at | IL1R2 | 1.73E-07 | 1.5229 | 2.54 | 77.7 | 68.7-85 |
| 205929_at | GPA33 | 1.86E-07 | 1.7056 | 1.61 | 80.3 | 71.7-87.2 |
| 202069_s_at | IDH3A | 2.08E-07 | 1.5696 | 1.75 | 78.4 | 69.5-85.5 |
| 202350_s_at | MATN2 | 2.11E-07 | 1.506 | 2.23 | 77.4 | 68.4-84.7 |
| 212859_x_at | NUTF2:MT1E:MT1M | 2.14E-07 | 1.6442 | 2.41 | 79.4 | 70.8-86.4 |
| 217546_at | NUTF2:MT1E:MT1M | 2.14E-07 | 1.645 | 2.41 | 79.5 | 70.7-86.5 |
| 216336_x_at | NUTF2:MT1E:MT1M | 2.14E-07 | 1.645 | 2.41 | 79.5 | 70.8-86.4 |
| 221667_s_at | HSPB8 | 2.61E-07 | 1.514 | 3.85 | 77.5 | 68.6-84.8 |
| 217757_at | A2M | 2.72E-07 | 1.598 | 1.64 | 78.8 | 70.1-85.9 |
| 216510_x_at | LOC652128:IGHG1:IGHM:IG HV4-31:LOC647189:IGHV1-69:IGHA1:IL8:EXOC7:SIX6:1 GHD:IGH@:IGHG3:C12orf32 :ZCWPW2:IFI6:IGHG4:IGHA 2:IGHG2:RAC1 | 2.80E-07 | 1.6346 | 3.1 | 79.3 | 70.6-86.4 |
| 223343_at | NYD-SP21:MS4A7 | 2.92E-07 | 1.5396 | 2.27 | 77.9 | 69.1-85.1 |
| 202620_s_at | PLOD2 | 3.06E-07 | 1.5551 | 2.33 | 78.2 | 69.3-85.4 |
| 207245_at | UGT2B17 | 3.75E-07 | 1.4796 | 4.88 | 77 | 68.1-84.5 |
| 210139_s_at | PMP22 | 3.97E-07 | 1.5183 | 2.23 | 77.6 | 68.7-84.9 |
| 204938_s_at | PLN | 4.14E-07 | 1.5454 | 2.01 | 78 | 69.1-85.2 |
| 204939_s_at | PLN | 4.14E-07 | 1.5425 | 2.01 | 78 | 69.1-85.2 |
| 204940_at | PLN | 4.14E-07 | 1.5436 | 2.01 | 78 | 69.1-85.2 |
| 203951_at | CNN1 | 4.77E-07 | 1.4324 | 4.56 | 76.3 | 67.3-83.8 |
| 202746_at | ITM2A | 5.27E-07 | 1.4988 | 2.77 | 77.3 | 68.4-84.6 |
| 221584_s_at | KCNMA1 | 6.86E-07 | 1.4959 | 2.37 | 77.3 | 68.4-84.6 |
| 241994_at | XDH | 8.13E-07 | 1.4709 | 2.03 | 76.9 | 67.9-84.3 |
| 209621_s_at | PDLIM3 | 8.20E-07 | 1.4805 | 2.38 | 77 | 68-84.4 |
| 204326_x_at | LOC645652:MT1X | 9.84E-07 | 1.4427 | 4.35 | 76.5 | 67.4-83.9 |
| 208581_x_at | LOC645652:MT1X | 9.84E-07 | 1.4397 | 4.35 | 76.4 | 67.5-83.9 |
| 201616_s_at | CALD1 | 1.02E-06 | 1.4583 | 3.29 | 76.7 | 67.8-84.1 |
| 201617_x_at | CALD1 | 1.02E-06 | 1.4617 | 3.29 | 76.8 | 67.7-84.1 |
| 212077_at | CALD1 | 1.02E-06 | 1.4581 | 3.29 | 76.7 | 67.8-84.1 |
| 225782_at | MSRB3 | 1.20E-06 | 1.4747 | 2.24 | 77 | 68-84.3 |
| 209436_at | SPON1 | 1.46E-06 | 1.4849 | 1.88 | 77.1 | 68.1-84.4 |
| 223623_at | C2orf40 | 1.69E-06 | 1.416 | 2.97 | 76.1 | 67-83.5 |
| 218087_s_at | SORBS1:KIAA0894 | 2.18E-06 | 1.4259 | 2.54 | 76.2 | 67.3-83.7 |
| 222513_s_at | SORBS1:KIAA0894 | 2.18E-06 | 1.4263 | 2.54 | 76.2 | 67.2-83.7 |
| 224990_at | C4orf34 | 2.55E-06 | 1.3853 | 1.95 | 75.6 | 66.5-83.1 |
| 203131_at | FIP1L1:PDGFRA | 2.94E-06 | 1.4845 | 1.37 | 77.1 | 68.2-84.5 |
| 203638_s_at | FGFR2 | 3.55E-06 | 1.5287 | 1.31 | 77.8 | 68.9-85 |
| 201957_at | PPP1R12B:LOC731632 | 4.26E-06 | 1.3611 | 2.21 | 75.2 | 66.2-82.8 |
| 217967_s_at | FAM129A | 4.59E-06 | 1.4131 | 1.8 | 76 | 67-83.5 |
| 210809_s_at | POSTN | 4.94E-06 | 1.3954 | 2.25 | 75.7 | 66.7-83.2 |
| 226302_at | ATP8B1 | 5.73E-06 | 1.313 | 1.67 | 74.4 | 65.3-82.1 |
| 238750_at | CCL28 | 6.01E-06 | 1.4932 | 1.53 | 77.2 | 68.3-84.6 |
| 226103_at | NEXN | 6.83E-06 | 1.3666 | 2.23 | 75.3 | 66.2-82.8 |
| 209101_at | CTGF | 6.96E-06 | 1.4774 | 1.3 | 77 | 68.1-84.4 |
| 229254_at | MFSD4 | 7.19E-06 | 1.3235 | 2.12 | 74.6 | 65.4-82.3 |
| 219087_at | ASPN | 7.45E-06 | 1.3249 | 2.74 | 74.6 | 65.5-82.3 |
| 209457_at | DUSP5 | 8.60E-06 | 1.3336 | 1.91 | 74.8 | 65.7-82.4 |
| 221541_at | CRISPLD2 | 9.24E-06 | 1.3664 | 1.64 | 75.3 | 66.2-82.9 |
| 206377_at | FOXF2 | 9.50E-06 | 1.3032 | 2.08 | 74.3 | 65.1-82 |
| 207392_x_at | UGT2B11:LOC728160:UGT2 B15,UGT2B11:LOC728160:U GT2B15 | 9.73E-06 | 1.3478 | 5.72 | 75 | 65.8-82.6 |
| 202274_at | ACTG2 | 1.07E-05 | 1.4015 | 1.82 | 75.8 | 66.8-83.4 |
| 227522_at | CMBL | 1.09E-05 | 1.3588 | 1.58 | 75.2 | 66-82.8 |
| 212192 at | KCTD12 | 1.44E-05 | 1.3659 | 1.36 | 75.3 | 66.2-82.8 |
| 227727_at | MRGPRF | 1.52E-05 | 1.2965 | 2.99 | 74.2 | 65-82 |
| 234987_at | C20orf118 | 1.75E-05 | 1.4278 | 1.3 | 76.2 | 67.3-83.7 |
| 209656_s_at | TMEM47 | 1.86E-05 | 1.2983 | 2.02 | 74.2 | 65.1-82 |
| 212265_at | QKI | 1.93E-05 | 1.3443 | 1.31 | 74.9 | 65.9-82.6 |
| 228232_s_at | VSIG2 | 2.01E-05 | 1.2957 | 2.05 | 74.1 | 65-81.9 |
| 200799_at | HSPA1A,HSPA1A | 2.16E-05 | 1.272 | 2.41 | 73.8 | 64.6-81.6 |
| 218312_s_at | ZNF447 | 3.05E-05 | 1.107 | 1.39 | 71 | 61.6-79.1 |
| 230264_s_at | AP1S2 | 3.39E-05 | 1.2546 | 1.92 | 73.5 | 64.3-81.4 |
| 224840_at | FKBP5 | 3.41E-05 | 1.2371 | 1.71 | 73.2 | 63.9-81.1 |
| 209948_at | KCNMB1 | 3.74E-05 | 1.2181 | 2.66 | 72.9 | 63.6-80.8 |
| 201426_s_at | VIM | 4.15E-05 | 1.2221 | 1.86 | 72.9 | 63.7-80.8 |
| 227826_s_at | SORBS2 | 4.23E-05 | 1.3017 | 2.1 | 74.2 | 65.1-82 |
| 227827_at | SORBS2 | 4.23E-05 | 1.3015 | 2.1 | 74.2 | 65.1-82 |
| 225728_at | SORBS2 | 4.23E-05 | 1.3015 | 2.1 | 74.2 | 65.1-82 |
| 238751_at | SORBS2 | 4.23E-05 | 1.3019 | 2.1 | 74.2 | 65.1-82 |
| 228202_at | C6orf204 | 4.90E-05 | 1.2062 | 2.07 | 72.7 | 63.5-80.6 |
| 220645_at | FAM55D | 5.67E-05 | 1.1786 | 3.48 | 72.2 | 63-80.2 |
| 224560_at | TIMP2 | 6.40E-05 | 1.2304 | 1.82 | 73.1 | 63.9-80.9 |
| 231579_s_at | TIMP2 | 6.40E-05 | 1.2286 | 1.82 | 73 | 63.8-81 |
| 208788_at | ELOVL5 | 8.15E-05 | 1.1804 | 2.05 | 72.2 | 63-80.2 |
| 200974_at | ACTA2 | 1.00E-04 | 1.2299 | 1.81 | 73.1 | 63.8-81 |
| 202388_at | RGS2 | 0.0001 | 1.1604 | 1.94 | 71.9 | 62.7-80 |
| 206461_x_at | MT1A:MT1H:LOC645745:LO C727730 | 0.0001 | 1.189 | 4.55 | 72.4 | 63.2-80.4 |
| 208747_s_at | C1S:PRB1:PRB2,C1S:PRB2:P RB1 | 0.0001 | 1.2884 | 1.61 | 74 | 64.9-81.9 |
| 208791_at | CLU | 0.0001 | 1.2303 | 1.42 | 73.1 | 63.9-80.9 |
| 208792_s_at | CLU | 0.0001 | 1.2302 | 1.42 | 73.1 | 63.9-81 |
| 212185_x_at | NUTF2:LOC441019:MT2A:M T1M,NUTF2:LOC441019:MT 2A:MT1M | 0.0001 | 1.1894 | 1.89 | 72.4 | 63.1-80.3 |
| 222043_at | CLU | 0.0001 | 1.2306 | 1.42 | 73.1 | 63.9-81 |
| 227099_s_at | LOC387763 | 0.0001 | 1.1963 | 1.36 | 72.5 | 63.2-80.5 |
| 202766_s_at | FBN1 | 0.0002 | 1.1359 | 1.93 | 71.5 | 62.2-79.6 |
| 204745_x_at | NUTF2:MT1G | 0.0002 | 1.0888 | 2.66 | 70.7 | 61.3-78.9 |
| 218418_s_at | ANKRD25 | 0.0002 | 1.1139 | 1.56 | 71.1 | 61.9-79.3 |
| 201058_s_at | MYL9 | 0.0003 | 1.131 | 2.18 | 71.4 | 62.1-79.5 |
| 205547_s_at | TAGLN | 0.0003 | 1.0914 | 2.12 | 70.7 | 61.4-78.9 |
| 205935_at | FOXF1 | 0.0004 | 1.1743 | 1.28 | 72.1 | 62.9-80.1 |
| 208450_at | LGALS2 | 0.0004 | 1.0845 | 3.07 | 70.6 | 61.4-78.8 |
| 203748_x_at | RBMS1 | 0.0005 | 1.1435 | 1.55 | 71.6 | 62.3-79.7 |
| 207266_x_at | RBMS1 | 0.0005 | 1.1428 | 1.55 | 71.6 | 62.4-79.7 |
| 209868_s_at | RBMS1 | 0.0005 | 1.1437 | 1.55 | 71.6 | 62.3-79.7 |
| 225269_s_at | RBMS1 | 0.0005 | 1.1446 | 1.55 | 71.6 | 62.3-79.6 |
| 212158_at | SDC2 | 0.0006 | 1.0977 | 1.39 | 70.8 | 61.6-79.1 |
| 209209_s_at | PLEKHC1 | 0.0007 | 1.0309 | 1.82 | 69.7 | 60.3-77.9 |
| 209210_s_at | PLEKHC1 | 0.0007 | 1.0325 | 1.82 | 69.7 | 60.3-78 |
| 205554_s_at | ACTB:DNASE1L3 | 0.0007 | 1.0979 | 1.5 | 70.8 | 61.4-78.9 |
| 202283_at | SERPINF1 | 0.0009 | 1.0003 | 1.47 | 69.2 | 59.8-77.5 |
| 209496_at | RARRES2:LOC648925 | 0.0009 | 1.0318 | 1.6 | 69.7 | 60.3-77.9 |
| 224352_s_at | CFL2 | 0.0009 | 1.0586 | 1.75 | 70.2 | 60.8-78.4 |
| 224663_s_at | CFL2 | 0.0009 | 1.0575 | 1.75 | 70.2 | 60.8-78.4 |
| 214433_s_at | SELENBP1 | 0.0009 | 1.0574 | 1.7 | 70.1 | 60.8-78.3 |
| 212592_at | ENAM | 0.0011 | 1.0024 | 2.58 | 69.2 | 59.8-77.5 |
| 213629_x_at | MT1F | 0.0012 | 0.9664 | 2.2 | 68.6 | 59.2-76.9 |
| 217165_x_at | MT1F | 0.0012 | 0.9649 | 2.2 | 68.5 | 59.2-76.9 |
| 203645_s_at | CD163 | 0.0013 | 1.0231 | 1.9 | 69.6 | 60-77.9 |
| 215049_x_at | CD163 | 0.0013 | 1.0215 | 1.9 | 69.5 | 60.1-77.8 |
| 206641_at | TNFRSF17 | 0.0015 | 1.0206 | 2.08 | 69.5 | 60.1-77.8 |
| 227235_at | GUCY1A3 | 0.0015 | 1.0116 | 2.69 | 69.4 | 59.8-77.6 |
| 229530_at | GUCY1A3 | 0.0015 | 1.0115 | 2.69 | 69.3 | 60-77.6 |
| 212097_at | CAV1 | 0.0016 | 1.0199 | 1.58 | 69.5 | 60.1-77.7 |
| 200884_at | CKB | 0.0019 | 0.9758 | 2.27 | 68.7 | 59.2-77 |
| 202133_at | WWTR1 | 0.002 | 0.9917 | 1.48 | 69 | 59.5-77.4 |
| 204607_at | HMGCS2 | 0.0022 | 0.8777 | 2.67 | 67 | 57.5-75.5 |
| 218559_s_at | MAFB | 0.0034 | 0.9595 | 1.66 | 68.4 | 59-76.8 |
| 201061_s_at | STOM | 0.0035 | 1.0165 | 1.21 | 69.4 | 60-77.7 |
| 201743_at | CD14 | 0.0035 | 0.9627 | 1.58 | 68.5 | 59.1-76.9 |
| 204895_x_at | MUC4:TAF5L:LOC650855:L OC645744 | 0.0039 | 1.0448 | 1.2 | 69.9 | 60.6-78.2 |
| 217109_at | MUC4:TAF5L:LOC650855:L OC645744 | 0.0039 | 1.0458 | 1.2 | 69.9 | 60.6-78.1 |
| 217110_s_at | MUC4:TAF5L:LOC650855:L OC645744 | 0.0039 | 1.0453 | 1.2 | 69.9 | 60.5-78.2 |
| 203382_s_at | APOE | 0.0044 | 0.9388 | 1.56 | 68.1 | 58.7-76.5 |
| 206664_at | SI | 0.0054 | 0.9293 | 2.79 | 67.9 | 58.4-76.3 |
| 242447_at | LOC285382 | 0.0058 | 0.7401 | 1.26 | 64.4 | 54.9-73.2 |
| 209312_x_at | HLA-DRB1:HLA-DRB6:LOC731247:LOC73041 5:HLA-DRB4:HLA-DRB5:HLA-DRB3:LOC651845 | 0.0063 | 1.0636 | 1.51 | 70.3 | 60.9-78.5 |
| 215193_x_at | HLA-DRB1:HLA-DRB6:LOC731247:LOC73041 5:HLA-DRB4:HLA-DRB5:HLA-DRB3:LOC651845 | 0.0063 | 1.0646 | 1.51 | 70.3 | 60.9-78.5 |
| 224694_at | ANTXR1 | 0.0071 | 0.9531 | 1.29 | 68.3 | 58.8-76.7 |
| 226811_at | FAM46C | 0.0085 | 0.9163 | 1.38 | 67.7 | 58.1-76.1 |
| 201150_s_at | TIMP3 | 0.0091 | 0.9049 | 1.81 | 67.5 | 57.9-76 |
| 226682_at | LOC283666 | 0.0099 | 0.7784 | 1.22 | 65.1 | 55.7-73.8 |
| 218468_s_at | GREM1 | 0.0106 | 0.8585 | 3.03 | 66.6 | 57.2-75.2 |
| 218469_at | GREM1 | 0.0106 | 0.8579 | 3.03 | 66.6 | 57.1-75.2 |
| 203240_at | FCGBP:LOC651441:LOC652 599 | 0.0121 | 0.7868 | 1.99 | 65.3 | 55.8-73.9 |
| 211538_s_at | HSPA2 | 0.0123 | 0.8916 | 1.43 | 67.2 | 57.7-75.7 |
| 212091_s_at | COL6A1 | 0.0126 | 0.848 | 1.45 | 66.4 | 56.9-74.9 |
| 213428_s_at | COL6A1 | 0.0126 | 0.8474 | 1.45 | 66.4 | 56.9-74.9 |
| 226051_at | SELM | 0.0133 | 0.8606 | 1.32 | 66.7 | 57.1-75.1 |
| 200859_x_at | FLNA:WTAP | 0.0157 | 0.7975 | 1.59 | 65.5 | 55.9-74 |
| 213746_s_at | FLNA:WTAP | 0.0157 | 0.7959 | 1.59 | 65.5 | 56-74.2 |
| 214752_x_at | FLNA:WTAP | 0.0157 | 0.798 | 1.59 | 65.5 | 56-74.1 |
| 204570_at | COX7A1 | 0.0161 | 0.8622 | 1.32 | 66.7 | 57.2-75.2 |
| 203729_at | EMP3 | 0.0164 | 0.8043 | 1.41 | 65.6 | 56.1-74.3 |
| 223597_at | ITLN1 | 0.021 | 0.7148 | 2.54 | 64 | 54.4-72.7 |
| 227735_s_at | C10orf99 | 0.0218 | 0.7254 | 1.76 | 64.2 | 54.6-72.9 |
| 227736_at | C10orf99 | 0.0218 | 0.7259 | 1.76 | 64.2 | 54.6-72.9 |
| 200621_at | CSRP1 | 0.023 | 0.7246 | 1.39 | 64.1 | 54.6-72.9 |
| 204897_at | PTGER4:LOC730002:LOC73 0882 | 0.0237 | 0.8114 | 1.41 | 65.8 | 56.2-74.4 |
| 204083_s_at | PPIL5:TPM2 | 0.026 | 0.7531 | 1.53 | 64.7 | 55.2-73.4 |
| 211643_x_at | HLA-C:HLA-B:LOC730410:LOC652614:L OC732037 | 0.0268 | 0.9237 | 1.24 | 67.8 | 58.3-76.3 |
| 211644_x_at | HLA-C:HLA-B:LOC730410:LOC652614:L OC732037 | 0.0268 | 0.9229 | 1.24 | 67.8 | 58.3-76.2 |
| 214768_x_at | HLA-C:HLA-B:LOC730410:LOC652614:L OC732037 | 0.0268 | 0.9245 | 1.24 | 67.8 | 58.3-76.3 |
| 226147_s_at | PIGR | 0.0278 | 0.7282 | 1.59 | 64.2 | 54.6-73 |
| 229659_s_at | PIGR | 0.0278 | 0.7253 | 1.59 | 64.2 | 54.7-72.9 |
| 201300_s_at | PRNP | 0.0288 | 0.7985 | 1.24 | 65.5 | 56-74.1 |
| 210133_at | CCL11 | 0.0361 | 0.8534 | 1.32 | 66.5 | 57-75.1 |
| 225353_s_at | C1QC | 0.0391 | 0.7457 | 1.36 | 64.5 | 55-73.2 |
| 201289_at | CYR61 | 0.0425 | 0.7849 | 1.47 | 65.3 | 55.8-73.9 |
| 210764_s_at | CYR61 | 0.0425 | 0.7836 | 1.47 | 65.2 | 55.6-73.9 |
| 200986_at | SERPING1 | 0.0503 | 0.7672 | 1.44 | 64.9 | 55.4-73.6 |
| 204122_at | TYROBP:ZNF160 | 0.053 | 0.8181 | 1.17 | 65.9 | 56.4-74.5 |
| 201667_at | GJA1 | 0.0561 | 0.784 | 1.76 | 65.2 | 55.7-73.9 |
| 208789_at | PTRF | 0.0624 | 0.736 | 1.5 | 64.4 | 54.8-73.1 |
| 210107_at | CLCA1 | 0.0681 | 0.7868 | 1.61 | 65.3 | 55.7-73.9 |
| 200665_s_at | SPARC | 0.0684 | 0.6714 | 1.45 | 63.1 | 53.6-72.1 |
| 212667_at | SPARC | 0.0684 | 0.6732 | 1.45 | 63.2 | 53.6-71.9 |
| 211964_at | COL4A2 | 0.0738 | 0.7064 | 1.32 | 63.8 | 54.2-72.6 |
| 201858_s_at | PRG1 | 0.0972 | 0.5559 | 1.25 | 60.9 | 51.3-69.9 |
| 201859_at | PRG1 | 0.0972 | 0.5563 | 1.25 | 61 | 51.4-69.9 |
| 228241_at | BCMP11 | 0.102 | 0.4028 | 1.28 | 58 | 48.4-67.1 |
| 217762_s_at | RAB31 | 0.1041 | 0.8135 | 1.14 | 65.8 | 56.3-74.4 |
| 217764_s_at | RAB31 | 0.1041 | 0.8132 | 1.14 | 65.8 | 56.2-74.5 |
| 202007_at | NID1 | 0.105 | 0.7425 | 1.33 | 64.5 | 55-73.1 |
| 210495_x_at | FN1 | 0.1121 | 0.5756 | 1.73 | 61.3 | 51.8-70.2 |
| 211719_x_at | FN1 | 0.1121 | 0.5767 | 1.73 | 61.3 | 51.8-70.2 |
| 212464_s_at | FN1 | 0.1121 | 0.5765 | 1.73 | 61.3 | 51.6-70.3 |
| 216442_x_at | FN1 | 0.1121 | 0.5745 | 1.73 | 61.3 | 51.7-70.3 |
| 200600_at | MSN | 0.1499 | 0.7232 | 1.15 | 64.1 | 54.6-72.9 |
| 209138_x_at | IGL@:LOC651536:LOC73106 2:CPVL:IGLV2-14:IGLV4-3:IGLV3-25:IGLV3-21:IL8:RPL14 | 0.1551 | 0.681 | 1.26 | 63.3 | 53.8-72.2 |
| 216984_x_at | IGL@:LOC651536:LOC73106 2:CPVL:IGLV2-14:IGLV4-3:IGLV3-25:IGLV3-21:IL8:RPL14 | 0.1551 | 0.6808 | 1.26 | 63.3 | 53.8-72.2 |
| 217148_x_at | IGL@:LOC651536:LOC73106 2:CPVL:IGLV2-14:IGLV4-3:IGLV3-25:IGLV3-21:IL8:RPL14 | 0.1551 | 0.6808 | 1.26 | 63.3 | 53.9-72.1 |
| 201069_at | MMP2 | 0.1755 | 0.6214 | 1.24 | 62.2 | 52.6-71 |
| 202403_s_at | COL1A2:LOC728628 | 0.1891 | 0.6508 | 1.9 | 62.8 | 53.2-71.5 |
| 202768_at | FOSB | 0.2076 | 0.6392 | 2.17 | 62.5 | 52.9-71.5 |
| 209116_x_at | HBB | 0.2357 | 0.6432 | 1.28 | 62.6 | 52.9-71.4 |
| 211696_x_at | HBB | 0.2357 | 0.6436 | 1.28 | 62.6 | 53.1-71.4 |
| 217232_x_at | HBB | 0.2357 | 0.6444 | 1.28 | 62.6 | 53.1-71.5 |
| 205267_at | POU2AF1 | 0.2389 | 0.6573 | 1.18 | 62.9 | 53.3-71.7 |
| 201852_x_at | COL3A1 | 0.2551 | 0.5524 | 1.35 | 60.9 | 51.3-69.8 |
| 211161_s_at | COL3A1 | 0.2551 | 0.5509 | 1.35 | 60.9 | 51.3-69.9 |
| 215076_s_at | COL3A1 | 0.2551 | 0.5513 | 1.35 | 60.9 | 51.3-69.8 |
| 217378_x_at | LOC391427 | 0.2627 | 0.2759 | 1.15 | 55.5 | 45.9-64.8 |
| 223235_s_at | SMOC2 | 0.2701 | 0.656 | 1.34 | 62.9 | 53.2-71.7 |
| 204673_at | MUC5AC:LOC652741:MUC2 | 0.2802 | 0.8531 | 1.22 | 66.5 | 57-75.1 |
| 212414_s_at | Sep-06 | 0.3109 | 0.5714 | 1.21 | 61.2 | 51.7-70.2 |
| 201744_s_at | LUM | 0.3157 | 0.5425 | 1.54 | 60.7 | 51-69.7 |
| 202953_at | C1QB | 0.3668 | 0.4998 | 1.24 | 59.9 | 50.3-68.9 |
| 209651_at | TGFB1I1 | 0.4093 | 0.4932 | 1.35 | 59.7 | 50.2-68.8 |
| 212224_at | ALDH1A1 | 0.4232 | 0.3525 | 1.4 | 57 | 47.4-66.2 |
| 211596_s_at | LRIG1 | 0.4614 | 0.5988 | 1.26 | 61.8 | 52.2-70.7 |
| 218541_s_at | C8orf4 | 0.475 | 0.4838 | 1.44 | 59.6 | 50-68.5 |
| 208894 at | HLA-DRA:HLA-DQA1,HLA-DRA:HLA-DQA1,HLA-DRA:HLA-DQA1 | 0.4823 | 0.3234 | 1.29 | 56.4 | 46.8-65.6 |
| 210982_s_at | HLA-DRA:HLA-DQA1,HLA-DRA:HLA-DQA1,HLA-DRA:HLA-DQA1 | 0.4823 | 0.3228 | 1.29 | 56.4 | 46.8-65.6 |
| 227404_s_at | EGR1 | 0.4851 | 0.5387 | 1.38 | 60.6 | 51-69.6 |
| 216401_x_at | LOC652745 | 0.4864 | 0.1728 | 1.09 | 53.4 | 43.9-62.8 |
| 216207_x_at | IGKV1D-13 | 0.5177 | 0.16 | 1.1 | 53.2 | 43.6-62.6 |
| 203477_at | COL15A1 | 0.6601 | 0.5385 | 1.22 | 60.6 | 51.1-69.7 |
| 211959_at | IGFBP5 | 0.7663 | 0.3526 | 1.25 | 57 | 47.4-66.2 |
| 201105_at | LGALS1 | 0.8555 | 0.4634 | 1.16 | 59.2 | 49.6-68.2 |
| 201438_at | COL6A3 | 0.8739 | 0.4434 | 1.2 | 58.8 | 49.2-67.8 |
| 212671_s_at | HLA-DQA1:HLA-DQA2,HLA-DQA 1:LOC731682:HLA-DQA2 | 0.8836 | 0.5398 | 1.28 | 60.6 | 51.1-69.7 |
| 227725_at | ST6GALNAC1 | 0.9452 | 0.2599 | 1.15 | 55.2 | 45.6-64.5 |
| 211990_at | HLA-DPA1,HLA-DPA1 | 0.9664 | 0.417 | 1.39 | 58.3 | 48.7-67.4 |
| 211991_s_at | HLA-DPA1,HLA-DPA1 | 0.9664 | 0.4166 | 1.39 | 58.3 | 48.7-67.4 |
| 217179_x_at | IGLV1-44 | 0.9777 | 0.2852 | 1.07 | 55.7 | 46.1-65 |
| 234764_x_at | IGLV1-44 | 0.9777 | 0.2847 | 1.07 | 55.7 | 46.1-64.9 |
| 224342_x_at | IGLV1-44 | 0.9777 | 0.2848 | 1.07 | 55.7 | 46.1-65 |
| 215176_x_at | NTN2L:IGKC:IGKV1-5:GJB6:HLA-C | 0.9858 | 0.4167 | 1.43 | 58.3 | 48.6-67.3 |
| 216576_x_at | NTN2L:IGKC:IGKV1-5:GJB6:HLA-C | 0.9858 | 0.4171 | 1.43 | 58.3 | 48.6-67.4 |
| 201645_at | TNC | 0.9905 | 0.3687 | 1.09 | 57.3 | 47.7-66.6 |
| 214414_x_at | HBA1:HBA2,HBA1:HBA2 | 0.9979 | 0.4707 | 1.21 | 59.3 | 49.7-68.3 |
| 204018_x_at | HBA1:HBA2,HBA1:HBA2 | 0.9979 | 0.4711 | 1.21 | 59.3 | 49.7-68.3 |
| 209458_x_at | HBA1:HBA2,HBA1:HBA2 | 0.9979 | 0.4727 | 1.21 | 59.3 | 49.7-68.4 |
| 211699_x_at | HBA1:HBA2,HBA1:HBA2 | 0.9979 | 0.4697 | 1.21 | 59.3 | 49.7-68.3 |
| 211745_x_at | HBA1:HBA2,HBA1:HBA2 | 0.9979 | 0.4708 | 1.21 | 59.3 | 49.8-68.3 |
| 217414_x_at | HBA1:HBA2,HBA1:HBA2 | 0.9979 | 0.469 | 1.21 | 59.3 | 49.7-68.4 |

**TABLE 2**

| **Gene Symbol** | **ValidPS_DOWN** | **Signif. FDF** | **D.val5** | **FC** | **Sens-Spec** | **CI (95)** |
|---|---|---|---|---|---|---|
| GCNT2 | 935239-HuGene_st:225205- | 2.16E-27 | 3.9513 | 13.36 | 97.6 | 94.2-99.2 |
| | HuGene_st:1026280-HuGene_st:668101- | | | | | |
| | HuGene_st:1099985-HuGene_st:698568- | | | | | |
| | HuGene_st:134540-HuGene_st:697147- | | | | | |
| | HuGene_st:250092-HuGene_st:611927- | | | | | |
| | HuGene_st:972833-HuGene_st:168891- | | | | | |
| | HuGene_st:990860-HuGene_st:109287- | | | | | |
| | HuGene_st:322116-HuGene_st:231019- | | | | | |
| | HuGene_st:211020_at:959570- | | | | | |
| | HuGene_st:858764- | | | | | |
| | HuGene_st:215593_at:820195- | | | | | |
| | HuGene_st:239606_at:41059- | | | | | |
| | HuGene_st:669940 | | | | | |
| | HuGene_st:215595_x_at:230788_at | | | | | |
| GUCA2B | 276512-HuGene_st: 1006871- | 7.04E-25 | 3.6268 | 51.78 | 96.5 | 92.4-98.6 |
| | HuGene_st:948364-HuGene_st:46575- | | | | | |
| | HuGene_st:207502_at:436788- | | | | | |
| | HuGene_st:485636-HuGene_st:60132- | | | | | |
| | HuGene_st:608718-HuGene_st:779511- | | | | | |
| | HuGene_st:132431-HuGene_st:974232- | | | | | |
| | HuGene_st:425596-HuGene_st:308287- | | | | | |
| | HuGene_st:800088-HuGene_st:827119- | | | | | |
| | HuGene_st:233789-HuGene_st:623623- | | | | | |
| | HuGene_st | | | | | |
| GUCA1B | 207003_at | 7.32E-25 | 3.5625 | 10.7 | 96.3 | 91.9-98.5 |
| CA4 | 636392-HuGene_st:206209_s_at:365396- | 1.73E-24 | 3.6291 | 10.41 | 96.5 | 92.4-98.7 |
| | HuGene_st:978203-HuGene_st:987169- | | | | | |
| | HuGene_st:638557- | | | | | |
| | HuGene_st:206208_at:1033858- | | | | | |
| | HuGene_st:597808-HuGene_st:756864- | | | | | |
| | HuGene_st:326439-HuGene_st:356096- | | | | | |
| | HuGene_st:478435-HuGene_st:31642- | | | | | |
| | HuGene_st:682778-HuGene_st:1006162- | | | | | |
| | HuGene_st:673533-HuGene_st:491473- | | | | | |
| | HuGene_st:822299-HuGene_st:871453- | | | | | |
| | HuGene_st:209666-HuGene_st:465368- | | | | | |
| | HuGene_st:354896-HuGene_st | | | | | |
| SLC4A4 | 810103-HuGene_st:634869- | 2.23E-23 | 3.6301 | 3.58 | 96.5 | 92.4-98.6 |
| | HuGene_st:210739_x_at:211494_s_at:268 096-HuGene_st:70822- | | | | | |
| | | | | | | |
| | HuGene_st:940785-HuGene_st:484105- | | | | | |
| | HuGene_st:210738_s_at:203908_at:99375 -HuGene_st:238833-HuGene_st:149464- | | | | | |
| | | | | | | |
| | HuGene_st:874564-HuGene_st:161948- | | | | | |
| | HuGene_st:501640-HuGene_st:65687- | | | | | |
| | HuGene_st:886057-HuGene_st:495376- | | | | | |
| | HuGene_st:247215-HuGene_st:847550- | | | | | |
| | HuGene_st:190252-HuGene_st:244077- | | | | | |
| | HuGene_st:477911-HuGene_st:904959- | | | | | |
| | HuGene_st:218417- | | | | | |
| | HuGene_st:1554027_a_at | | | | | |
| AQP8 | 486167-HuGene_st:126752- | 2.98E-22 | 3.3311 | 10.82 | 95.2 | 90.3-97.9 |
| | HuGene_st:93102-HuGene_st:1090469- | | | | | |
| | HuGene_st:980308-HuGene st:107169- | | | | | |
| | HuGene_st:829020-HuGene_st:1053234- | | | | | |
| | HuGene_st:954102-HuGene_st:40331- | | | | | |
| | HuGene_st:206784_at:990703- | | | | | |
| | HuGene_st:459706-HuGene_st:863327- | | | | | |
| | HuGene_st:965233-HuGene_st:459009- | | | | | |
| | HuGene_st:180228-HuGene_st:944315- | | | | | |
| | HuGene_st:814773-HuGene_st:810460- | | | | | |
| | HuGene_st:228837-HuGene_st | | | | | |
| CA1 | 632246-HuGene_st:205950_s_at:382371- | 3.15E-22 | 3.3666 | 38.27 | 95.4 | 90.6-98 |
| | HuGene_st:1004598-HuGene_st:405837- | | | | | |
| | HuGene_st:551543-HuGene_st:242482- | | | | | |
| | HuGene_st:267733-HuGene_st:1074039- | | | | | |
| | HuGene_st:107073-HuGene_st:485365- | | | | | |
| | HuGene_st:254908-HuGene_st:257502- | | | | | |
| | HuGene_st:333301-HuGene_st:180359- | | | | | |
| | HuGene_st:696950-HuGene_st:588557- | | | | | |
| | HuGene_st:381399-HuGene_st:495540- | | | | | |
| | HuGene_st:384192-HuGene_st:205949_at | | | | | |
| ABCG2 | 236197-HuGene_st:10623- | 1.09E-21 | 3.2599 | 31.53 | 94.8 | 89.8-97.7 |
| | HuGene_st:1005470-HuGene_st:140860- | | | | | |
| | HuGene_st:492719-HuGene_st:417387- | | | | | |
| | HuGene_st:113831-HuGene_st:507347- | | | | | |
| | HuGene_st:944251-HuGene_st:175319- | | | | | |
| | HuGene_st:784920-HuGene_st:709551- | | | | | |
| | HuGene_st:136344-HuGene_st:689536- | | | | | |
| | HuGene_st:209735_at:167748- | | | | | |
| | HuGene_st:759590-HuGene_st:805222- | | | | | |
| | HuGene_st:945575-HuGene_st:294363- | | | | | |
| | HuGene_st:958524-HuGene_st:402982- | | | | | |
| | HuGene_st:301335-HuGene_st | | | | | |
| OSTbeta | 552746-HuGene_st:57742- | 6.23E-21 | 3.188 | 7.15 | 94.5 | 89.2-97.5 |
| | HuGene_st:461622-HuGene_st:965982- | | | | | |
| | HuGene_st:413709- | | | | | |
| | HuGene_st:230830_at:232719- | | | | | |
| | HuGene_st:165400-HuGene_st:203597- | | | | | |
| | HuGene_st:1025456-HuGene_st:625341- | | | | | |
| | HuGene_st:881755-HuGene_st:490180- | | | | | |
| | HuGene_st:113547-HuGene_st:605512- | | | | | |
| | HuGene_st:179662-HuGene_st:280309- | | | | | |
| | HuGene_st:647118-HuGene_st | | | | | |
| MGC13057 | 926449-HuGene_st:317301- | 6.94E-21 | 3.2029 | 3.18 | 94.5 | 89.3-97.5 |
| | HuGene_st:834797-HuGene_st:173388- | | | | | |
| | HuGene_st:127937-HuGene_st:182395- | | | | | |
| | HuGene_st:800172-HuGene_st:1041848- | | | | | |
| | HuGene_st:93911-HuGene_st:810617- | | | | | |
| | HuGene_st:1035428-HuGene_st:644866- | | | | | |
| | HuGene_st:343402- | | | | | |
| | HuGene_st:223754_at:228195_at:509933- | | | | | |
| | HuGene_st | | | | | |
| CLDN23 | 403960-HuGene_st:25144- | 4.27E-20 | 3.0777 | 3.47 | 93.8 | 88.2-97.1 |
| | HuGene_st:947653- | | | | | |
| | HuGene_st:228704_s_at:228706_s_at:320 375-HuGene_st:441629- | | | | | |
| | | | | | | |
| | HuGene_st:367414-HuGene_st:855269- | | | | | |
| | HuGene_st:228707_at:788659- | | | | | |
| | HuGene_st:698816-HuGene_st:95789- | | | | | |
| | HuGene_st:270197-HuGene_st:472976- | | | | | |
| | HuGene_st:280539-HuGene_st:1056334- | | | | | |
| | HuGene_st:516288-HuGene_st:579963- | | | | | |
| | HuGene_st | | | | | |
| PKIB | 866170-HuGene_st:1055812- | 5.94E-20 | 3.1232 | 3.28 | 94.1 | 88.7-97.3 |
| | HuGene_st:264946-HuGene_st:684057- | | | | | |
| | HuGene_st:124791-HuGene_st:134561- | | | | | |
| | HuGene_st:1026756-HuGene_st:468593- | | | | | |
| | HuGene_st:1045852-HuGene_st:939917- | | | | | |
| | HuGene_st:110205-HuGene_st:660721- | | | | | |
| | HuGene_st:905229- | | | | | |
| | HuGene_st:223551_at:610426-HuGene_st | | | | | |
| SEMA6D | 855766-HuGene_st:233882_s_at:543564- | 6.07E-20 | 3.092 | 4.27 | 93.9 | 88.4-97.2 |
| | HuGene_st:277987-HuGene_st:1075165- | | | | | |
| | HuGene_st:220574_at:732853- | | | | | |
| | HuGene_st:410940-HuGene_st:384488- | | | | | |
| | HuGene_st:1041056- | | | | | |
| | HuGene_st:233801_s_at:535378- | | | | | |
| | HuGene_st:477671-HuGene_st:626423- | | | | | |
| | HuGene_st:101599-HuGene_st:714098- | | | | | |
| | HuGene_st:301505-HuGene_st:53682- | | | | | |
| | HuGene_st:177281-HuGene_st:596415- | | | | | |
| | HuGene_st:244746_at:226492_at:699792- | | | | | |
| | HuGene_st:73748-HuGene_st:692437- | | | | | |
| | HuGene_st | | | | | |
| CLCA4 | 220026_at:1000955-HuGene_st:215807- | 1.25E-19 | 3.1922 | 16.3 | 94.5 | 89.2-97.5 |
| | HuGene_st:108406-HuGene_st:538359- | | | | | |
| | HuGene_st:366726-HuGene_st:727431- | | | | | |
| | HuGene_st:240891-HuGene_st:485685- | | | | | |
| | HuGene_st:205213-HuGene_st:476179- | | | | | |
| | HuGene_st:283676-HuGene_st:933390- | | | | | |
| | HuGene_st:601908-HuGene_st:844633- | | | | | |
| | HuGene_st:846667-HuGene_st:99723- | | | | | |
| | HuGene_st:376041-HuGene_st:71188- | | | | | |
| | HuGene_st:261183-HuGene_st:498330- | | | | | |
| | HuGene_st:500064-HuGene_st:785031- | | | | | |
| | HuGene_st | | | | | |
| TP53INP2 | 224836_at:294906-HuGene_st:857693- | 1.45E-19 | 3.0229 | 3.78 | 93.5 | 87.8-96.9 |
| | HuGene_st:852970-HuGene_st:594178- | | | | | |
| | HuGene_st:868173-HuGene_st:353925- | | | | | |
| | HuGene_st:212485-HuGene_st:804363- | | | | | |
| | HuGene_st:778869-HuGene_st:427072- | | | | | |
| | HuGene_st:451529-HuGene_st:808765- | | | | | |
| | HuGene_st:205314-HuGene_st:415164- | | | | | |
| | HuGene_st:1048358-HuGene_st | | | | | |
| MS4A12 | 220834_at:582400-HuGene_st:354845- | 2.13E-19 | 3.2962 | 7.15 | 95 | 90-97.8 |
| | HuGene_st:341876-HuGene_st:769180- | | | | | |
| | HuGene_st:740541-HuGene_st:436753- | | | | | |
| | HuGene_st:570027-HuGene_st:26519- | | | | | |
| | HuGene_st:956581-HuGene_st:335315- | | | | | |
| | HuGene_st:206721-HuGene_st:589444- | | | | | |
| | HuGene_st:16629-HuGene_st:113533- | | | | | |
| | HuGene_st:201614-HuGene_st:774878- | | | | | |
| | HuGene_st:786494-HuGene_st:493550- | | | | | |
| | HuGene_st:403871-HuGene_st:192686- | | | | | |
| | HuGene_st:846528-HuGene_st:249799- | | | | | |
| | HuGene_st | | | | | |
| TRPM6 | 767074-HuGene_st:695352- | 2.25E-19 | 3.0106 | 7.5 | 93.4 | 87.7-96.8 |
| | HuGene_st:411125- | | | | | |
| | HuGene_st:221102_s_at:234864_s_at:240 389_at:358229-HuGene_st:755964- | | | | | |
| | | | | | | |
| | HuGene_st:840301-HuGene_st:959234- | | | | | |
| | HuGene_st:782639-HuGene_st:833079- | | | | | |
| | HuGene_st:1066034-HuGene_st:678013- | | | | | |
| | HuGene_st:249083-HuGene_st:143934- | | | | | |
| | HuGene_st:159130-HuGene_st:486486- | | | | | |
| | HuGene_st:185057-HuGene_st:878793- | | | | | |
| | HuGene_st:133981- | | | | | |
| | HuGene_st:224412_s_at:202194- | | | | | |
| | HuGene_st | | | | | |
| XLKD1 | 520080-HuGene_st:1091117- | 3.60E-19 | 2.94 | 7.44 | 92.9 | 87.1-96.6 |
| | HuGene_st:943125-HuGene_st:444068- | | | | | |
| | HuGene_st:648558-HuGene_st:346991- | | | | | |
| | HuGene_st:1006205-HuGene_st:373107- | | | | | |
| | HuGene_st:682535-HuGene_st:1083245- | | | | | |
| | HuGene_st:863143-HuGene_st:820120- | | | | | |
| | HuGene_st:1044561- | | | | | |
| | HuGene_st:220037_s_at:541228- | | | | | |
| | HuGene_st:220256-HuGene_st:289122- | | | | | |
| | HuGene_st:219059_s_at:246683- | | | | | |
| | HuGene_st:775976-HuGene_st:207399- | | | | | |
| | HuGene_st:1052557-HuGene_st:92121- | | | | | |
| | HuGene_st | | | | | |
| ADH1B | 1078343-HuGene_st:512808- | 4.70E-19 | 3.0172 | 4.67 | 93.4 | 87.7-96.9 |
| | HuGene_st:614446-HuGene_st:910188- | | | | | |
| | HuGene_st:422504-HuGene_st:731361- | | | | | |
| | HuGene_st:209612_s_at:258079- | | | | | |
| | HuGene_st:568239-HuGene_st:879930- | | | | | |
| | HuGene_st:420417-HuGene_st:1025048- | | | | | |
| | HuGene_st:908335-HuGene_st:654633- | | | | | |
| | HuGene_st:947292-HuGene_st:1087125- | | | | | |
| | HuGene_st:1004870- | | | | | |
| | HuGene_st:209613_s_at:579636- | | | | | |
| | HuGene_st:681018-HuGene_st:822774- | | | | | |
| | HuGene_st | | | | | |
| PRDX6 | 353187-HuGene_st:520885- | 5.56E-19 | 2.8414 | 2.76 | 92.2 | 86.1-96.1 |
| | HuGene_st:919334-HuGene_st:646105- | | | | | |
| | HuGene_st:390352- | | | | | |
| | HuGene_st:200844_s_at:855125- | | | | | |
| | HuGene_st:200845_s_at:54231- | | | | | |
| | HuGene_st:612931-HuGene_st:837652- | | | | | |
| | HuGene_st:538086-HuGene_st:796167- | | | | | |
| | HuGene_st:208992-HuGene_st:39945- | | | | | |
| | HuGene_st:489353-HuGene_st:963499- | | | | | |
| | HuGene_st:376244-HuGene_st:297536- | | | | | |
| | HuGene_st:495777-HuGene_st: 1005253- | | | | | |
| | HuGene_st:833460-HuGene_st | | | | | |
| CA2 | 762141-HuGene_st:111686- | 5.87E-19 | 2.9625 | 9.22 | 93.1 | 87.2-96.7 |
| | HuGene_st:512916-HuGene_st:9132- | | | | | |
| | HuGene_st:995083- | | | | | |
| | HuGene_st:209301_at:578483- | | | | | |
| | HuGene_st:583428-HuGene_st:961832- | | | | | |
| | HuGene_st:11748-HuGene_st:1056641- | | | | | |
| | HuGene_st:545067-HuGene_st:952781- | | | | | |
| | HuGene_st:689246-HuGene_st:246103- | | | | | |
| | HuGene_st:803284-HuGene_st:828842- | | | | | |
| | HuGene_st:694989-HuGene_st:808226- | | | | | |
| | HuGene_st:199030-HuGene_st | | | | | |
| EDN3 | 314103-HuGene_st:217154_s_at:820613- | 6.42E-19 | 2.937 | 11.21 | 92.9 | 87-96.5 |
| | HuGene_st:1050996-HuGene_st:348157- | | | | | |
| | HuGene_st:338856-HuGene_st:594687- | | | | | |
| | HuGene_st:38595-HuGene_st:270770- | | | | | |
| | HuGene_st:817067-HuGene_st:766407- | | | | | |
| | HuGene_st:256284- | | | | | |
| | HuGene_st:208399_s_at:200417- | | | | | |
| | HuGene_st:496738-HuGene_st:590141- | | | | | |
| | HuGene_st:137170-HuGene_st:499250- | | | | | |
| | HuGene_st:478524-HuGene_st:720694- | | | | | |
| | HuGene_st:543565-HuGene_st | | | | | |
| MIER3 | 884917-HuGene_st:740856- | 9.14E-19 | 2.9162 | 3.91 | 92.8 | 86.8-96.5 |
| | HuGene_st:107094-HuGene_st:408774- | | | | | |
| | HuGene_st:461523-HuGene_st:153511- | | | | | |
| | HuGene_st:207042-HuGene_st:737492- | | | | | |
| | HuGene_st:43443-HuGene_st:614740- | | | | | |
| | HuGene_st:1559652_at:783045- | | | | | |
| | HuGene_st:887606-HuGene_st:996253- | | | | | |
| | HuGene_st:1041635-HuGene_st:142612- | | | | | |
| | HuGene_st:1553336_a_at:220555- | | | | | |
| | HuGene_st:975347-HuGene_st:470750- | | | | | |
| | HuGene_st:1554450_s_at:804524- | | | | | |
| | HuGene_st:265536-HuGene_st:774142- | | | | | |
| | HuGene_st:231975_s_at:34726- | | | | | |
| | HuGene_st:1554449_at:228961_at | | | | | |
| ABCA8 | 57305-HuGene_st:111752- | 1.26E-18 | 2.9165 | 6.82 | 92.8 | 86.8-96.5 |
| | HuGene_st:61357-HuGene_st:123451- | | | | | |
| | HuGene_st:740746-HuGene_st:512280- | | | | | |
| | HuGene_st:389185-HuGene_st:427721- | | | | | |
| | HuGene_st:346028-HuGene_st:224566- | | | | | |
| | HuGene_st:149653-HuGene_st:680699- | | | | | |
| | HuGene_st:76772-HuGene_st:742091- | | | | | |
| | HuGene_st:423333-HuGene_st:559944- | | | | | |
| | HuGene_st:341399- | | | | | |
| | HuGene_st:1565780_at:863400- | | | | | |
| | HuGene_st:921748-HuGene_st:623719- | | | | | |
| | HuGene_st:204719_at:123143- | | | | | |
| | HuGene_st:1077391-HuGene_st | | | | | |
| METTL7A | 278779-HuGene_st:481332- | 1.35E-18 | 3.1231 | 1.93 | 94.1 | 88.6-97.3 |
| | HuGene_st:787584-HuGene_st:989793- | | | | | |
| | HuGene_st:278513-HuGene_st:424901- | | | | | |
| | HuGene_st:551005-HuGene_st:921169- | | | | | |
| | HuGene_st:125176-HuGene_st:277465- | | | | | |
| | HuGene_st:560742- | | | | | |
| | HuGene_st:207761_s_at:367459- | | | | | |
| | HuGene_st:224782-HuGene_st:660194- | | | | | |
| | HuGene_st:266654-HuGene_st:1086300- | | | | | |
| | HuGene_st:134633- | | | | | |
| | HuGene_st:211424_x_at:273973- | | | | | |
| | HuGene_st:947408- | | | | | |
| | HuGene_st:209703_x_at:617184- | | | | | |
| | HuGene_st:864197-HuGene_st:10061- | | | | | |
| | HuGene_st | | | | | |
| DPT | 876245-HuGene_st:1036210- | 1.53E-18 | 2.8977 | 9.07 | 92.6 | 86.7-96.4 |
| | HuGene_st:214758-HuGene_st:1005554- | | | | | |
| | HuGene_st:178625-HuGene_st:701967- | | | | | |
| | HuGene_st:35076-HuGene_st:33432- | | | | | |
| | HuGene_st:897771-HuGene_st:304494- | | | | | |
| | HuGene_st:445757-HuGene_st:491297- | | | | | |
| | HuGene_st:646408-HuGene_st:842542- | | | | | |
| | HuGene_st:87795- | | | | | |
| | HuGene_st:213068_at:207977_s_at:55338 9-HuGene_st:477075- | | | | | |
| | | | | | | |
| | HuGene_st:213071_at:1046282- | | | | | |
| | HuGene_st | | | | | |
| MGC4172 | 339712-HuGene_st:1089815- | 1.74E-18 | 2.9044 | 3.74 | 92.7 | 86.7-96.4 |
| | HuGene_st:265870-HuGene_st:895908- | | | | | |
| | HuGene_st:226292-HuGene_st:322910- | | | | | |
| | HuGene_st:575887-HuGene_st:416485- | | | | | |
| | HuGene_st:1055581-HuGene_st:1088692- | | | | | |
| | HuGene_st:442194- | | | | | |
| | HuGene_st:218756_s_at:775684- | | | | | |
| | HuGene_st:773137-HuGene_st:373028- | | | | | |
| | HuGene_st:512667-HuGene_st:164027- | | | | | |
| | HuGene_st:541185-HuGene_st:593729- | | | | | |
| | HuGene_st:506796-HuGene_st | | | | | |
| FNBP1 | 728822-HuGene_st:845505- | 1.76E-18 | 3.0483 | 2.04 | 93.6 | 88-97 |
| | HuGene_st:1054016-HuGene_st:383098- | | | | | |
| | HuGene_st:230389_at:31865 | | | | | |
| | HuGene_st:981305- | | | | | |
| | HuGene_st:213940_s_at:755179- | | | | | |
| | HuGene_st:839717- | | | | | |
| | HuGene_st:230086_at:239453_at:985213- | | | | | |
| | HuGene_st:125814-HuGene_st:451336- | | | | | |
| | HuGene_st:22306-HuGene_st:540957- | | | | | |
| | HuGene_st:277486-HuGene_st:314000- | | | | | |
| | HuGene_st:212288_at:662225- | | | | | |
| | HuGene_st:524650-HuGene_st:605840- | | | | | |
| | HuGene_st:180978-HuGene_st:604436- | | | | | |
| | HuGene_st | | | | | |
| EDG2 | 816816-HuGene_st:309471- | 2.44E-18 | 2.8838 | 3.19 | 92.5 | 86.5-96.3 |
| | HuGene_st:77020-HuGene_st:289373- | | | | | |
| | HuGene_st:10014-HuGene_st:981213- | | | | | |
| | HuGene_st:204038_s_at:1035537- | | | | | |
| | HuGene_st:75833-HuGene_st:781880- | | | | | |
| | HuGene_st:494373-HuGene_st:745317- | | | | | |
| | HuGene_st:497715-HuGene_st:693528- | | | | | |
| | HuGene_st:806817-HuGene_st:812294- | | | | | |
| | HuGene_st:204036_at:241773_at:176887- | | | | | |
| | HuGene_st:441141-HuGene_st:189399- | | | | | |
| | HuGene_st:723788- | | | | | |
| | HuGene_st:204037_at:485543- | | | | | |
| | HuGene_st:1014086- | | | | | |
| | HuGene_st:232716_at | | | | | |
| SFRP1 | 722739-HuGene_st:1095144- | 2.77E-18 | 2.8631 | 15.53 | 92.4 | 86.3-96.2 |
| | HuGene_st:1039611-HuGene_st:836419- | | | | | |
| | HuGene_st:34446-HuGene_st:50826- | | | | | |
| | HuGene_st:483033- | | | | | |
| | HuGene_st:202036_s_at:988375- | | | | | |
| | HuGene_st:774922 | | | | | |
| | HuGene_st:228413_s_at:665988- | | | | | |
| | HuGene_st:302344- | | | | | |
| | HuGene_st:202035_s_at:412438- | | | | | |
| | HuGene_st:995086- | | | | | |
| | HuGene_st:202037_s_at:181713- | | | | | |
| | HuGene_st:527962-HuGene_st:880207- | | | | | |
| | HuGene_st:100260-HuGene_st | | | | | |
| SLC26A3 | 206143_at:172542-HuGene_st:768419- | 4.18E-18 | 2.9214 | 21.4 | 92.8 | 86.8-96.5 |
| | HuGene_st:820082-HuGene_st:263997- | | | | | |
| | HuGene_st:681117-HuGene_st:581666- | | | | | |
| | HuGene_st:180553-HuGene_st:356894- | | | | | |
| | HuGene_st:240715-HuGene_st:718555- | | | | | |
| | HuGene_st:517735-HuGene_st:339388- | | | | | |
| | HuGene_st:989337-HuGene_st:1511- | | | | | |
| | HuGene_st:228629-HuGene_st:354149- | | | | | |
| | HuGene_st:603695-HuGene_st:791438- | | | | | |
| | HuGene_st:154730-HuGene_st: 905529- | | | | | |
| | HuGene_st:248690-HuGene_st:431441- | | | | | |
| | HuGene_st:215657_at | | | | | |
| ANGPTL1 | 572942-HuGene_st:891312- | 1.42E-17 | 2.7929 | 4.64 | 91.9 | 85.6-95.9 |
| | HuGene_st:953040-HuGene_st:232844- | | | | | |
| | HuGene_st:145730-HuGene_st:142205- | | | | | |
| | HuGene_st:227771-HuGene_st:80584- | | | | | |
| | HuGene_st:982090-HuGene_st:999640- | | | | | |
| | HuGene_st:672931-HuGene_st:148578- | | | | | |
| | HuGene_st:224339_s_at:1046706- | | | | | |
| | HuGene_st:239183_at:155600- | | | | | |
| | HuGene_st:284674- | | | | | |
| | HuGene_st:231773_at:818064- | | | | | |
| | HuGene_st:978991-HuGene_st:728775- | | | | | |
| | HuGene_st | | | | | |
| UGP2 | 795967-HuGene_st:561220- | 1.64E-17 | 2.7904 | 2.41 | 91.9 | 85.6-95.9 |
| | HuGene_st:754258-HuGene_st:846757- | | | | | |
| | HuGene_st:868436-HuGene_st:321605- | | | | | |
| | HuGene_st:299097-HuGene_st:7536- | | | | | |
| | HuGene_st:216268-HuGene_st:925542- | | | | | |
| | HuGene_st:967807- | | | | | |
| | HuGene_st:205480_s_at:639621- | | | | | |
| | HuGene_st:177627-HuGene_st:894271- | | | | | |
| | HuGene_st:617530- | | | | | |
| | HuGene_st:231698_at:971369- | | | | | |
| | HuGene_st:132663-HuGene_st:215198- | | | | | |
| | HuGene_st:647604-HuGene_st:537579- | | | | | |
| | HuGene_st:232180_at:1056881- | | | | | |
| | HuGene_st | | | | | |
| SRPX | 195599-HuGene_st:541428- | 1.79E-17 | 2.7749 | 3.91 | 91.7 | 85.5-95.8 |
| | HuGene_st:143706-HuGene_st:728558- | | | | | |
| | HuGene_st:190938-HuGene_st:455214- | | | | | |
| | HuGene_st:522280-HuGene_st:966576- | | | | | |
| | HuGene_st:650551-HuGene_st:810331- | | | | | |
| | HuGene_st:396246-HuGene_st:133110- | | | | | |
| | HuGene_st:232310-HuGene_st:475511- | | | | | |
| | HuGene_st:225561-HuGene_st:351281- | | | | | |
| | HuGene_st:204955_at | | | | | |
| OGN | 955964-HuGene_st:775073- | 2.25E-17 | 2.7078 | 13.67 | 91.2 | 84.7-95.4 |
| | HuGene_st:678354-HuGene_st:238500- | | | | | |
| | HuGene_st:1019070-HuGene_st:614445- | | | | | |
| | HuGene_st:23784-HuGene_st:658925- | | | | | |
| | HuGene_st:757128-HuGene_st:632689- | | | | | |
| | HuGene_st:989474-HuGene_st:233177- | | | | | |
| | HuGene_st:650498-HuGene_st:950000- | | | | | |
| | HuGene_st:751146-HuGene_st:67232- | | | | | |
| | HuGene_st:997991- | | | | | |
| | HuGene_st:218730_s_at:222722_at:82148 8-HuGene_st | | | | | |
| | | | | | | |
| MYLK | 527465-HuGene_st:617817- | 2.33E-17 | 2.7729 | 3.99 | 91.7 | 85.4-95.8 |
| | HuGene_st:716971-HuGene_st:942711- | | | | | |
| | HuGene_st:230460_at:161532- | | | | | |
| | HuGene_st:767103-HuGene_st:1041083- | | | | | |
| | HuGene_st:202167-HuGene_st:738247- | | | | | |
| | HuGene_st:505966-HuGene_st:62701- | | | | | |
| | HuGene_st:776564-HuGene_st:679137- | | | | | |
| | HuGene_st:551306- | | | | | |
| | HuGene_st:202555_s_at:224823_at:98631 | | | | | |
| | 3-HuGene_st:324943-HuGene_st:218172- | | | | | |
| | HuGene_st:351524- | | | | | |
| | HuGene_st:1568770_at:280094- | | | | | |
| | HuGene_st:1017324- | | | | | |
| | HuGene_st:1563466_at | | | | | |
| LIFR | 275506-HuGene_st:323055- | 2.45E-17 | 2.7826 | 6.36 | 91.8 | 85.6-95.8 |
| | HuGene_st:444251-HuGene_st:1056178- | | | | | |
| | HuGene_st:398104-HuGene_st:917434- | | | | | |
| | HuGene_st:1044918-HuGene_st:167500- | | | | | |
| | HuGene_st:423760-HuGene_st:837336- | | | | | |
| | HuGene_st:321505-HuGene_st:918321- | | | | | |
| | HuGene_st:252278-HuGene_st:884504- | | | | | |
| | HuGene_st:124845-HuGene_st:499777- | | | | | |
| | HuGene_st:969722-HuGene_st:709439- | | | | | |
| | HuGene_st:611505- | | | | | |
| | HuGene_st:227771_at:287217- | | | | | |
| | HuGene_st:205876_at:225571_at:229185_ | | | | | |
| | at:233367_at:1093011-HuGene_st | | | | | |
| ZG16 | 287680-HuGene_st:1704- | 2.51E-17 | 2.8097 | 8.14 | 92 | 85.8-96 |
| | HuGene_st:61566-HuGene_st:964936- | | | | | |
| | HuGene_st:9737-HuGene_st:620497- | | | | | |
| | HuGene_st:785765- | | | | | |
| | HuGene_st:214142_at:323025- | | | | | |
| | HuGene_st:1093257-HuGene_st:398415-V | | | | | |
| | HuGene_st:942991-HuGene_st:871899- | | | | | |
| | HuGene_st:548837-HuGene_st:605328- | | | | | |
| | HuGene_st:231918-HuGene_st:461816- | | | | | |
| | HuGene_st:604283-HuGene_st:971003- | | | | | |
| | HuGene_st:750108-HuGene_st:927723- | | | | | |
| | HuGene_st:782940-HuGene_st:98669-V | | | | | |
| | HuGene_st | | | | | |
| EPB41L3 | 275431-HuGene_st:540074- | 2.62E-17 | 2.8312 | 2.51 | 92.2 | 86-96.1 |
| | HuGene_st:731277- | | | | | |
| | HuGene_st:211776_s_at:218157- | | | | | |
| | HuGene_st:328922-HuGene_st:384314- | | | | | |
| | HuGene_st:272486-HuGene_st:196590- | | | | | |
| | HuGene_st:294230- | | | | | |
| | HuGene_st:206710_s_at:747631- | | | | | |
| | HuGene_st:52743-HuGene_st:1089957- | | | | | |
| | HuGene_st:423337-HuGene_st:915755- | | | | | |
| | HuGene_st:623682-HuGene_st:130815- | | | | | |
| | HuGene_st:497574- | | | | | |
| | HuGene_st:212681_at:77106- | | | | | |
| | HuGene_st:874781-HuGene_st | | | | | |
| SCNN1B | 773390-HuGene_st:467376- | 3.20E-17 | 2.7219 | 16.15 | 91.3 | 84.9-95.5 |
| | HuGene_st:411583-HuGene_st:433511- | | | | | |
| | HuGene_st:142579-HuGene_st:99655- | | | | | |
| | HuGene_st:278054-HuGene_st:371417- | | | | | |
| | HuGene_st:99189-HuGene_st:491965- | | | | | |
| | HuGene_st:490957-HuGene_st:916581- | | | | | |
| | HuGene_st:285916-HuGene_st:359156- | | | | | |
| | HuGene_st:977951-HuGene_st:291765- | | | | | |
| | HuGene_st:767266-HuGene_st:206091- | | | | | |
| | HuGene_st:348267- | | | | | |
| | HuGene_st:205464_at:838576-HuGene_st | | | | | |
| HHLA2 | 371335-HuGene_st:978721- | 3.47E-17 | 2.6741 | 10.02 | 90.9 | 84.4-95.2 |
| | HuGene_st:1065567-HuGene_st:282548- | | | | | |
| | HuGene_st:240410-HuGene_st:170899- | | | | | |
| | HuGene_st:947848-HuGene_st:438234- | | | | | |
| | HuGene_st:220812_s_at:927495- | | | | | |
| | HuGene_st:351364- | | | | | |
| | HuGene_st:234673_at:993142- | | | | | |
| | HuGene_st:1009637-HuGene_st:335000- | | | | | |
| | HuGene_st:285313-HuGene_st:533646- | | | | | |
| | HuGene_st:234624_at:458597- | | | | | |
| | HuGene_st:104838-HuGene_st:26687- | | | | | |
| | HuGene_st:258409-HuGene_st:493304- | | | | | |
| | HuGene_st:378019-HuGene_st:576796- | | | | | |
| | HuGene_st | | | | | |
| HPGD | 291863-HuGene_st:375608- | 3.65E-17 | 2.8433 | 3.21 | 92.2 | 86.1-96.1 |
| | HuGene_st:793406-HuGene_st:436293- | | | | | |
| | HuGene_st:75568- | | | | | |
| | HuGene_st:211549_s_at:684728- | | | | | |
| | HuGene_st:674596-HuGene_st:527856- | | | | | |
| | HuGene_st:329920-HuGene_st:748432- | | | | | |
| | HuGene_st:259392-HuGene_st:769902- | | | | | |
| | HuGene_st:620673-HuGene_st:450707- | | | | | |
| | HuGene_st:203913_s_at:304752- | | | | | |
| | HuGene_st:447604-HuGene_st:170968- | | | | | |
| | HuGene_st:852359-HuGene_st:836377- | | | | | |
| | HuGene_st:242733_at:243846- | | | | | |
| | HuGene_st:136281- | | | | | |
| | HuGene_st:203914_x_at:211548_s_at:288 252-HuGene_st | | | | | |
| | | | | | | |
| CEACAM7 | 206199_at:105665-HuGene_st:481561- | 3.96E-17 | 2.785 | 10.4 | 91.8 | 85.5-95.8 |
| | HuGene_st:211848_s_at:574369- | | | | | |
| | HuGene_st:_104615-HuGene_st:242258- | | | | | |
| | HuGene_st:740634-HuGene_st:439249- | | | | | |
| | HuGene_st:15044-HuGene_st:424954- | | | | | |
| | HuGene_st:1002484- | | | | | |
| | HuGene_st:206198_s_at:1067871- | | | | | |
| | HuGene_st:751891-HuGene_st:36594- | | | | | |
| | HuGene_st:298155-HuGene_st:1024423- | | | | | |
| | HuGene_st:384378-HuGene_st:1093665- | | | | | |
| | HuGene_st:985998-HuGene_st:325578- | | | | | |
| | HuGene_st:596079-HuGene_st:89770- | | | | | |
| | HuGene_st:864758-HuGene_st:205004- | | | | | |
| | HuGene_st:374052-HuGene_st:133822- | | | | | |
| | HuGene_st:1093942-HuGene_st:780375- | | | | | |
| | HuGene_st:338636-HuGene_st | | | | | |
| RELL1 | 753672-HuGene_st:871189- | 4.10E-17 | 2.6983 | 2.5 | 91.1 | 84.6-95.4 |
| | HuGene_st:202462-HuGene_st:615056- | | | | | |
| | HuGene_st:639180-HuGene_st:4103- | | | | | |
| | HuGene_st:778017-HuGene_st:236549- | | | | | |
| | HuGene_st:86799-HuGene_st:908328- | | | | | |
| | HuGene_st:813998-HuGene_st:859771- | | | | | |
| | HuGene_st:254041-HuGene_st:787272- | | | | | |
| | HuGene_st:916269-HuGene_st:885786- | | | | | |
| | HuGene_st:1554714_at:645386- | | | | | |
| | HuGene_st:777480-HuGene_st:927821- | | | | | |
| | HuGene_st:226430_at | | | | | |
| C7 | 893214-HuGene_st:54339- | 4.40E-17 | 2.7158 | 6.67 | 91.3 | 84.9-95.5 |
| | HuGene_st:501090-HuGene_st:659367- | | | | | |
| | HuGene_st:536407-HuGene_st:598145- | | | | | |
| | HuGene_st:444111-HuGene_st:643669- | | | | | |
| | HuGene_st:191981-HuGene_st:832001- | | | | | |
| | HuGene_st:313065-HuGene_st:612836- | | | | | |
| | HuGene_st:673870-HuGene_st:1005162- | | | | | |
| | HuGene_st:206652-HuGene_st:1021871- | | | | | |
| | HuGene_st:489813-HuGene_st:350161- | | | | | |
| | HuGene_st:766550-HuGene_st:2377- | | | | | |
| | HuGene_st:202992_at:578639-HuGene_st | | | | | |
| PLCE1 | 1562826_at:1563103_at:699801- | 6.91E-17 | 2.7528 | 2.43 | 91.6 | 85.2-95.7 |
| | HuGene_st:353862-HuGene_st:187026- | | | | | |
| | HuGene_st:475246-HuGene_st:819995- | | | | | |
| | HuGene_st:477396-HuGene_st:920273- | | | | | |
| | HuGene_st: 1566739_at:158053- | | | | | |
| | HuGene_st:554291-HuGene_st:854670- | | | | | |
| | HuGene_st:950080-HuGene_st:1064466- | | | | | |
| | HuGene_st:1017434-HuGene_st:987146- | | | | | |
| | HuGene_st:137607-HuGene_st:833052- | | | | | |
| | HuGene_st:205112_at:987189- | | | | | |
| | HuGene_st:1092203-HuGene_st:449587- | | | | | |
| | HuGene_st:205111_s_at:1566740_at:9284 26-HuGene_st:649071-HuGene_st | | | | | |
| | | | | | | |
| SCARA5 | 734536-HuGene_st:1080104- | 1.25E-16 | 2.6912 | 2.86 | 91.1 | 84.5-95.3 |
| | HuGene_st:796100-HuGene_st:258146- | | | | | |
| | HuGene_st:670462-HuGene_st:362431- | | | | | |
| | HuGene_st:1019057-HuGene_st:287988- | | | | | |
| | HuGene_st:235849_at:222306- | | | | | |
| | HuGene_st:495319-HuGene_st:84286- | | | | | |
| | HuGene_st:47679-HuGene_st:855551- | | | | | |
| | HuGene_st:229839_at:999202- | | | | | |
| | HuGene_st:982358-HuGene_st:86142- | | | | | |
| | HuGene_st:568080-HuGene_st:109115- | | | | | |
| | HuGene_st:590235- | | | | | |
| | HuGene_st:1554705_at:800351- | | | | | |
| | HuGene_st | | | | | |
| CHRDL1 | 335657-HuGene_st:1058673- | 1.49E-16 | 2.6092 | 13.88 | 90.4 | 83.7-94.9 |
| | HuGene_st:166016-HuGene_st:825273- | | | | | |
| | HuGene_st:35456-HuGene_st:91115- | | | | | |
| | HuGene_st:303433-HuGene_st:209044- | | | | | |
| | HuGene_st:1081642-HuGene_st:135625- | | | | | |
| | HuGene_st:284037-HuGene_st:452295- | | | | | |
| | HuGene_st:313790-HuGene_st:912814- | | | | | |
| | HuGene_st:712513-HuGene_st:1078238- | | | | | |
| | HuGene_st:721877- | | | | | |
| | HuGene_st:209763_at:657006-HuGene_st | | | | | |
| NR3C2 | 748063-HuGene_st:177629- | 2.08E-16 | 2.5811 | 3.19 | 90.2 | 83.3-94.7 |
| | HuGene_st:964944-HuGene_st:346404- | | | | | |
| | HuGene_st:120283-HuGene_st:646104- | | | | | |
| | HuGene_st:731385-HuGene_st:493776- | | | | | |
| | HuGene_st:440054-HuGene_st:286582- | | | | | |
| | HuGene_st:1097927-HuGene_st:804888- | | | | | |
| | HuGene_st:234142_at:49341- | | | | | |
| | HuGene_st:370558-HuGene_st:22007- | | | | | |
| | HuGene_st:981978-HuGene_st:100953- | | | | | |
| | HuGene_st:239673_at:149241- | | | | | |
| | HuGene_st:940356-HuGene_st:628366- | | | | | |
| | HuGene_st:205259_at:1564236_at: 328672 -HuGene_st | | | | | |
| | | | | | | |
| TSPAN7 | 703402-HuGene_st:745610- | 2.13E-16 | 2.651 | 3.68 | 90.7 | 84.2-95.1 |
| | HuGene_st:1018812-HuGene_st:742943- | | | | | |
| | HuGene_st:168712- | | | | | |
| | HuGene_st:234246_at:902239- | | | | | |
| | HuGene_st:285968-HuGene_st:883914- | | | | | |
| | HuGene_st:113610-HuGene_st:236336- | | | | | |
| | HuGene_st:511310-HuGene_st:40850- | | | | | |
| | HuGene_st:346422-HuGene_st:189042- | | | | | |
| | HuGene_st:745521-HuGene_st:996022- | | | | | |
| | HuGene_st:202242_at:195303- | | | | | |
| | HuGene_st:70610-HuGene_st:31995- | | | | | |
| | HuGene_st:234245_at | | | | | |
| STMN2 | 459270-HuGene_st:611227- | 2.25E-16 | 2.6147 | 6.13 | 90.4 | 83.8-94.9 |
| | HuGene_st:295241-HuGene_st:935618- | | | | | |
| | HuGene_st:161827-HuGene_st:17520- | | | | | |
| | HuGene_st:337557-HuGene_st:16478- | | | | | |
| | HuGene_st:1015231-HuGene_st:790658- | | | | | |
| | HuGene_st:892767-HuGene_st:343589- | | | | | |
| | HuGene_st:1047491-HuGene_st:454759- | | | | | |
| | HuGene_st:9820- | | | | | |
| | HuGene_st:203001_s_at:472072- | | | | | |
| | HuGene_st:203000_at:691598- | | | | | |
| | HuGene_st:1064573-HuGene_st:811444- | | | | | |
| | HuGene_st:485134-HuGene_st | | | | | |
| FAM107A | 315531-HuGene_st:294482- | 2.52E-16 | 2.6168 | 2.92 | 90.5 | 83.8-94.9 |
| | HuGene_st:764207-HuGene_st:809908- | | | | | |
| | HuGene_st:209074_s_at:341591- | | | | | |
| | HuGene_st:948705- | | | | | |
| | HuGene_st:207547_s_at:415418- | | | | | |
| | HuGene_st:215297-HuGene_st:332641-V | | | | | |
| | HuGene_st | | | | | |
| ANK2 | 314086-HuGene_st:282874- | 2.59E-16 | 2.6501 | 6.76 | 90.7 | 84.1-95.1 |
| | HuGene_st:382431-HuGene_st:229308- | | | | | |
| | HuGene_st:779600-HuGene_st:297624- | | | | | |
| | HuGene_st:385943-HuGene_st:730140- | | | | | |
| | HuGene_st:442277-HuGene_st:699309- | | | | | |
| | HuGene_st:182816- | | | | | |
| | HuGene_st:202921_s_at:799860- | | | | | |
| | HuGene_st:868462-HuGene_st:634421- | | | | | |
| | HuGene_st:571536-HuGene_st:1050903- | | | | | |
| | HuGene_st:649509-HuGene_st:239935- | | | | | |
| | HuGene_st:202920_at | | | | | |
| CLIC5 | 217628_at:219866_at:116794- | 3.68E-16 | 2.6963 | 2.37 | 91.1 | 84.6-95.4 |
| | HuGene_st:114619-HuGene_st:895974- | | | | | |
| | HuGene_st:749788-HuGene_st:485909- | | | | | |
| | HuGene_st:326032-HuGene_st:1083118- | | | | | |
| | HuGene_st:213317_at:507492- | | | | | |
| | HuGene_st:480370-HuGene_st:160114- | | | | | |
| | HuGene_st:637800-HuGene_st:33914- | | | | | |
| | HuGene_st:1044626- | | | | | |
| | HuGene_st:243917_at:664038- | | | | | |
| | HuGene_st:867787-HuGene_st:261656- | | | | | |
| | HuGene_st:399156-HuGene_st:558641- | | | | | |
| | HuGene_st | | | | | |
| CHGA | 152790-HuGene_st:354161- | 5.54E-16 | 2.6097 | 7.21 | 90.4 | 83.6-94.9 |
| | HuGene_st:365802-HuGene_st:813261- | | | | | |
| | HuGene_st:40004-HuGene_st:504502- | | | | | |
| | HuGene_st:624128-HuGene_st:719186- | | | | | |
| | HuGene_st:916335-HuGene_st:921714- | | | | | |
| | HuGene_st:489360-HuGene_st:849455- | | | | | |
| | HuGene_st:231475-HuGene_st:294992- | | | | | |
| | HuGene_st:204697_s_at:659997- | | | | | |
| | HuGene_st:521835-HuGene_st:659283- | | | | | |
| | HuGene_st:890388-HuGene_st:786316- | | | | | |
| | HuGene_st | | | | | |
| KIAA0828 | 613787-HuGene_st:478932- | 5.58E-16 | 2.5514 | 4.09 | 89.9 | 83-94.5 |
| | HuGene_st:67325-HuGene_st:668897- | | | | | |
| | HuGene_st:160683-HuGene_st:1031978- | | | | | |
| | HuGene_st:1018444-HuGene_st:237998- | | | | | |
| | HuGene_st:892138-HuGene_st:262221- | | | | | |
| | HuGene_st:864937-HuGene_st:917541- | | | | | |
| | HuGene_st:593865-HuGene_st:55857- | | | | | |
| | HuGene_st:215672_s_at:509888- | | | | | |
| | HuGene_st:302988-HuGene_st:668914- | | | | | |
| | HuGene_st:507489-HuGene_st:212814_at | | | | | |
| EDIL3 | 436107- | 6.21E-16 | 2.6259 | 2.96 | 90.5 | 83.8-95 |
| | HuGene_st:1558643_s_at:214345_at:6687 80-HuGene_st:545521 | | | | | |
| | | | | | | |
| | HuGene_st:641822- | | | | | |
| | HuGene_st:207379_at:411378- | | | | | |
| | HuGene_st:485618-HuGene_st:576523- | | | | | |
| | HuGene_st:843058-HuGene_st:910789- | | | | | |
| | HuGene_st:165720-HuGene_st:79965- | | | | | |
| | HuGene_st:533665-HuGene_st:237605- | | | | | |
| | HuGene_st:593615-HuGene_st:396108- | | | | | |
| | HuGene_st:701416-HuGene_st:325630- | | | | | |
| | HuGene_st:912197-HuGene_st:224797- | | | | | |
| | HuGene_st:724765-HuGene_st:167229- | | | | | |
| | HuGene_st:233668_at:225275_at:16759- | | | | | |
| | HuGene_st:233875_at | | | | | |
| DSCR1 | 535011-HuGene_st:789105- | 6.21E-16 | 2.6122 | 2.14 | 90.4 | 83.7-94.9 |
| | HuGene_st:215253_s_at:838760- | | | | | |
| | HuGene_st:617570-HuGene_st:419279- | | | | | |
| | HuGene_st:1015938- | | | | | |
| | HuGene_st:208370_s_at:131478- | | | | | |
| | HuGene_st:228884-HuGene_st:685397- | | | | | |
| | HuGene_st:68677- | | | | | |
| | HuGene_st:215254_at:656173- | | | | | |
| | HuGene_st:971107-HuGene_st:1039926- | | | | | |
| | HuGene_st:206974-HuGene_st:232227- | | | | | |
| | HuGene_st:546248-HuGene_st:175597- | | | | | |
| | HuGene_st:1558181_at | | | | | |
| PPAP2A | 282276-HuGene_st:1062765- | 6.43E-16 | 2.596 | 2.4 | 90.3 | 83.6-94.8 |
| | HuGene_st:952258-HuGene_st:214647- | | | | | |
| | HuGene_st:153982-HuGene_st:678140- | | | | | |
| | HuGene_st:209147_s_at:1083041- | | | | | |
| | HuGene_st:459000-HuGene_st:505597- | | | | | |
| | HuGene_st:1035745-HuGene_st:576550- | | | | | |
| | HuGene_st:243715_at:180473- | | | | | |
| | HuGene_st:527974-HuGene_st:821572- | | | | | |
| | HuGene_st:260389-HuGene_st:1042377- | | | | | |
| | HuGene_st:138155- | | | | | |
| | HuGene_st:210946_at:243442_x_at | | | | | |
| PDCD4 | 681770-HuGene_st:1029160- | 8.67E-16 | 2.4578 | 2.6 | 89 | 82-93.9 |
| | HuGene_st:594740-HuGene_st:247013- | | | | | |
| | HuGene_st:845755-HuGene_st:813933- | | | | | |
| | HuGene_st:367555-HuGene_st:456148- | | | | | |
| | HuGene_st:295280-HuGene_st:144200- | | | | | |
| | HuGene_st:620666- | | | | | |
| | HuGene_st:212593_s_at:590149- | | | | | |
| | HuGene_st:87471-HuGene_st:189624- | | | | | |
| | HuGene_st:202730_s_at:44562- | | | | | |
| | HuGene_st:641673-HuGene_st:577621- | | | | | |
| | HuGene_st:102842- | | | | | |
| | HuGene_st:202731_at:776615- | | | | | |
| | HuGene_st:615983- | | | | | |
| | HuGene_st:212594_at:1557166_at | | | | | |
| DHRS9 | 413956-HuGene_st:371161- | 1.09E-15 | 2.7371 | 1.51 | 91.4 | 85.1-95.6 |
| | HuGene_st:224009_x_at:61832- | | | | | |
| | HuGene_st:874913-HuGene_st:321055- | | | | | |
| | HuGene_st:223952_x_at:916038- | | | | | |
| | HuGene_st:313731-HuGene_st:629747- | | | | | |
| | HuGene_st:836048-HuGene_st:369374- | | | | | |
| | HuGene_st:157673-HuGene_st:194386- | | | | | |
| | HuGene_st:1089433-HuGene_st:352973- | | | | | |
| | HuGene_st:637905-HuGene_st:1010893- | | | | | |
| | HuGene_st:219799_s_at:361862- | | | | | |
| | HuGene_st:800910-HuGene_st:_1012296- | | | | | |
| | HuGene_st | | | | | |
| CDKN2B | 252481-HuGene_st:1000015- | 1.34E-15 | 2.5433 | 10.09 | 89.8 | 82.9-94.5 |
| | HuGene_st:172198-HuGene_st:967979- | | | | | |
| | HuGene_st:675431-HuGene_st:605224- | | | | | |
| | HuGene_st:277126-HuGene_st:846115- | | | | | |
| | HuGene_st:452433-HuGene_st:332731- | | | | | |
| | HuGene_st:563208-HuGene_st:527577- | | | | | |
| | HuGene_st:207530_s_at:891201- | | | | | |
| | HuGene_st:512260 | | | | | |
| | HuGene_st:236313_at:26244- | | | | | |
| | HuGene_st:61586-HuGene_st | | | | | |
| P2RY1 | 628249-HuGene_st:113454- | 1.41E-15 | 2.5341 | 3.6 | 89.7 | 82.8-94.4 |
| | HuGene_st:627857-HuGene_st:461281- | | | | | |
| | HuGene_st:207455_at:259065- | | | | | |
| | HuGene_st:797734-HuGene_st:135788- | | | | | |
| | HuGene_st:42916-HuGene_st:315405- | | | | | |
| | HuGene_st:340050-HuGene_st:173225- | | | | | |
| | HuGene_st:919818-HuGene_st:591228- | | | | | |
| | HuGene_st:899117-HuGene_st:785070- | | | | | |
| | HuGene_st:286200-HuGene_st:231925_at | | | | | |
| LOC64662 7 | 238143_at | 1.58E-15 | 1.9131 | 5.02 | 83.1 | 74.9-89.4 |
| PPAP2B | 484735-HuGene_st:860271- | 2.44E-15 | 2.5819 | 1.81 | 90.2 | 83.4-94.7 |
| | HuGene_st:501067-HuGene_st:180731- | | | | | |
| | HuGene_st:919245- | | | | | |
| | HuGene_st:209355_s_at:322730- | | | | | |
| | HuGene_st:830995- | | | | | |
| | HuGene_st:212226_s_at:353268- | | | | | |
| | HuGene_st:989778-HuGene_st:279549- | | | | | |
| | HuGene_st:583834-HuGene_st:1035367- | | | | | |
| | HuGene_st:155537-HuGene_st:782552- | | | | | |
| | HuGene_st:867734-HuGene_st:267847- | | | | | |
| | HuGene_st:212230_at:619833- | | | | | |
| | HuGene_st:232324_x_at | | | | | |
| PYY | 656845-HuGene_st:816022- | 3.50E-15 | 2.4793 | 11.71 | 89.2 | 82.2-94 |
| | HuGene_st:633572-HuGene_st:20355- | | | | | |
| | HuGene_st:240779-HuGene_st:638358- | | | | | |
| | HuGene_st:879780- | | | | | |
| | HuGene_st:207080_s_at:211253_x_at:368 591-HuGene_st | | | | | |
| | | | | | | |
| CLEC3B | 794375-HuGene_st:894763- | 4.65E-15 | 2.4437 | 4.46 | 88.9 | 81.7-93.8 |
| | HuGene_st:152328-HuGene_st:669650- | | | | | |
| | HuGene_st:1090081-HuGene_st:251162- | | | | | |
| | HuGene_st:264101-HuGene_st:367416- | | | | | |
| | HuGene_st:984078-HuGene_st:512272- | | | | | |
| | HuGene_st:166348-HuGene_st:181414- | | | | | |
| | HuGene_st:995862-HuGene_st:603678- | | | | | |
| | HuGene_st:205200_at:56968- | | | | | |
| | HuGene_st:542483-HuGene_st | | | | | |
| NDE1 | 228133_s_at:218414_s_at:555730- | 4.84E-15 | 2.5125 | 1.99 | 89.5 | 82.6-94.3 |
| | HuGene_st:620739-HuGene_st:111363- | | | | | |
| | HuGene_st | | | | | |
| CCL8 | 402294-HuGene_st:1098932- | 5.75E-15 | 2.4512 | 7.51 | 89 | 81.9-93.9 |
| | HuGene_st:930533-HuGene_st:524795- | | | | | |
| | HuGene_st:574692-HuGene_st:314202- | | | | | |
| | HuGene_st:719831-HuGene_st:410906- | | | | | |
| | HuGene_st:643479-HuGene_st:911966- | | | | | |
| | HuGene_st:31200-HuGene_st:547634- | | | | | |
| | HuGene_st:39456-HuGene_st:527508- | | | | | |
| | HuGene_st:1057270-HuGene_st:1062460- | | | | | |
| | HuGene_st:214038_at | | | | | |
| PLAC8 | 219014_at:739479-HuGene_st:354260- | 5.76E-15 | 2.4228 | 3.87 | 88.7 | 81.5-93.7 |
| | HuGene_st:994047-HuGene_st:456534- | | | | | |
| | HuGene_st:236727_at:330382- | | | | | |
| | HuGene_st:118286-HuGene_st:940467- | | | | | |
| | HuGene_st:493579-HuGene_st:37786- | | | | | |
| | HuGene_st:776403-HuGene_st:295172- | | | | | |
| | HuGene_st:271859-HuGene_st:438305- | | | | | |
| | HuGene_st:770531-HuGene_st:982881- | | | | | |
| | HuGene_st:543535-HuGene_st:554805- | | | | | |
| | HuGene_st:667817-HuGene_st:519806- | | | | | |
| | HuGene_st:1065533-HuGene_st:345940- | | | | | |
| | HuGene_st | | | | | |
| MUCDHL | 690807-HuGene_st:11068- | 5.81E-15 | 2.4358 | 3.06 | 88.8 | 81.7-93.8 |
| | HuGene_st:955242-HuGene_st:969280- | | | | | |
| | HuGene_st:220075_s_at:625411- | | | | | |
| | HuGene_st:789310-HuGene_st:444568- | | | | | |
| | HuGene_st:589749-HuGene_st:265998- | | | | | |
| | HuGene_st:275576- | | | | | |
| | HuGene_st:219796_s_at:625643- | | | | | |
| | HuGene_st:452184-HuGene_st:692224- | | | | | |
| | HuGene_st:1009431-HuGene_st:311533- | | | | | |
| | HuGene_st:289109-HuGene_st:101091- | | | | | |
| | HuGene_st:220074_at:307121-HuGene_st | | | | | |
| SULT1A1 | 322199-HuGene_st:1021081- | 7.17E-15 | 2.422 | 3.37 | 88.7 | 81.6-93.7 |
| | HuGene_st:372215-HuGene_st:689177- | | | | | |
| | HuGene_st:215299_x_at:369102- | | | | | |
| | HuGene_st:203615_x_at:761577- | | | | | |
| | HuGene_st:744847-HuGene_st:691837- | | | | | |
| | HuGene_st:2491-HuGene_st:742631- | | | | | |
| | HuGene_st:1037597-HuGene_st:805936- | | | | | |
| | HuGene_st | | | | | |
| SDCBP2 | 233565_s_at:263537-HuGene_st:901885- | 9.28E-15 | 2.4869 | 2.37 | 89.3 | 82.3-94.1 |
| | HuGene_st:421400-HuGene_st:835589- | | | | | |
| | HuGene_st:70935-HuGene_st:462061- | | | | | |
| | HuGene_st:702702-HuGene_st:719818- | | | | | |
| | HuGene_st:766065-HuGene_st:612065- | | | | | |
| | HuGene_st:825595-HuGene_st:1004265- | | | | | |
| | HuGene_st:206488-HuGene_st:867231- | | | | | |
| | HuGene_st:858503-HuGene_st:29674- | | | | | |
| | HuGene_st:26237-HuGene_st:351356- | | | | | |
| | HuGene_st:52388-HuGene_st:192518- | | | | | |
| | HuGene_st | | | | | |
| RPL24 | 1559655_at:1559656_a_at:228885_at | 1.23E-14 | 2.5579 | 1.63 | 90 | 83.1-94.6 |
| CXCL12 | 567389-HuGene_st:886750- | 1.54E-14 | 2.4343 | 3.79 | 88.8 | 81.6-93.7 |
| | HuGene_st:815979-HuGene_st:1018943- | | | | | |
| | HuGene_st:209687_at:800403- | | | | | |
| | HuGene_st:500331-HuGene_st:369373- | | | | | |
| | HuGene_st:1050367-HuGene_st:952256- | | | | | |
| | HuGene_st:1066302-HuGene_st:448047- | | | | | |
| | HuGene_st:186655-HuGene_st:771363- | | | | | |
| | HuGene_st:103922-HuGene_st:1025591- | | | | | |
| | HuGene_st:47442-HuGene_st:624854- | | | | | |
| | HuGene_st:211406-HuGene_st:255389- | | | | | |
| | HuGene_st | | | | | |
| C1orf115 | 293810-HuGene_st:439869- | 1.95E-14 | 2.4078 | 2.95 | 88.6 | 81.4-93.6 |
| | HuGene_st:640378-HuGene_st:226391- | | | | | |
| | HuGene_st:88026- | | | | | |
| | HuGene_st:230159_at:709535- | | | | | |
| | HuGene_st:687885- | | | | | |
| | HuGene_st:218546_at:628297- | | | | | |
| | HuGene_st:695536-HuGene_st:230774- | | | | | |
| | HuGene_st:884109-HuGene_st:517531- | | | | | |
| | HuGene_st:472518-HuGene_st:8635- | | | | | |
| | HuGene_st:158841-HuGene_st | | | | | |
| SLC26A2 | 224963_at:861453-HuGene_st:175674- | 2.08E-14 | 2.4223 | 7.83 | 88.7 | 81.5-93.7 |
| | HuGene_st:658865-HuGene_st:979969- | | | | | |
| | HuGene_st:1053352- | | | | | |
| | HuGene_st:224959_at:509013- | | | | | |
| | HuGene_st:755618-HuGene_st:879968- | | | | | |
| | HuGene_st:205097_at:901728- | | | | | |
| | HuGene_st:975514-HuGene_st:407147- | | | | | |
| | HuGene_st:891478-HuGene_st:196344- | | | | | |
| | HuGene_st:1015698-HuGene_st:955403- | | | | | |
| | HuGene_st:430227-HuGene_st:1075936- | | | | | |
| | HuGene_st:1080935-HuGene_st:18346- | | | | | |
| | HuGene_st:341007-HuGene_st:471383- | | | | | |
| | HuGene_st | | | | | |
| MEIS1 | 868613-HuGene_st:1072855- | 2.49E-14 | 2.3774 | 5.44 | 88.3 | 81-93.4 |
| | HuGene_st:139075-HuGene_st:721526- | | | | | |
| | HuGene_st:1559477_s_at:381698- | | | | | |
| | HuGene_st:641395-HuGene_st:494458- | | | | | |
| | HuGene_st:183063-HuGene_st:212378- | | | | | |
| | HuGene_st:579377-HuGene_st:1006382- | | | | | |
| | HuGene_st:753093-HuGene_st:863677- | | | | | |
| | HuGene_st:517122-HuGene_st:777085- | | | | | |
| | HuGene_st:386678- | | | | | |
| | HuGene_st:204069_at:1005832- | | | | | |
| | HuGene_st:395572-HuGene_st:231941- | | | | | |
| | HuGene_st:568986-HuGene_st:108139- | | | | | |
| | HuGene_st | | | | | |
| SFRP2 | 144787-HuGene_st:337246- | 3.05E-14 | 2.3944 | 7.39 | 88.4 | 81.2-93.5 |
| | HuGene_st:97123-HuGene_st:614920- | | | | | |
| | HuGene_st:223121_s_at:236674- | | | | | |
| | HuGene_st:602699-HuGene_st:370588- | | | | | |
| | HuGene_st:794693-HuGene_st:252957- | | | | | |
| | HuGene_st:870849-HuGene_st:279759- | | | | | |
| | HuGene_st:367084-HuGene_st:691080- | | | | | |
| | HuGene_st:223122_s_at:466709- | | | | | |
| | HuGene_st | | | | | |
| TUBB6 | 209191_at:238151_at:267951- | 3.55E-14 | 2.3694 | 5.9 | 88.2 | 80.9-93.3 |
| | HuGene_st:578028-HuGene_st:862750- | | | | | |
| | HuGene_st | | | | | |
| GPX3 | 623237-HuGene_st:40650- | 5.68E-14 | 2.3613 | 2.57 | 88.1 | 80.8-93.2 |
| | HuGene_st:134873-HuGene_st:952394- | | | | | |
| | HuGene_st:644367-HuGene_st:428176- | | | | | |
| | HuGene_st:201348_at:214091_s_at:37706 0-HuGene_st:674301-HuGene_st:142325- | | | | | |
| | | | | | | |
| | HuGene_st:905944-HuGene_st:439631- | | | | | |
| | HuGene_st:1064572-HuGene_st:587834- | | | | | |
| | HuGene_st:1040504-HuGene_st:250733- | | | | | |
| | HuGene_st:199701-HuGene_st:784815- | | | | | |
| | HuGene_st:652659-HuGene_st | | | | | |
| CD36 | 392196-HuGene_st:274514- | 6.27E-14 | 2.4677 | 1.65 | 89.1 | 82.1-94 |
| | HuGene_st:477005-HuGene_st:691585- | | | | | |
| | HuGene_st:872909-HuGene_st:543050- | | | | | |
| | HuGene_st:603343-HuGene_st:514557- | | | | | |
| | HuGene_st:296850-HuGene_st:945913- | | | | | |
| | HuGene_st:495755- | | | | | |
| | HuGene_st:206488_s_at:1035854- | | | | | |
| | HuGene_st:887301-HuGene_st:836370- | | | | | |
| | HuGene_st:209555_s_at:939919- | | | | | |
| | HuGene_st:507440-HuGene_st:151788- | | | | | |
| | HuGene_st:146280-HuGene_st:360545- | | | | | |
| | HuGene_st:1051486- | | | | | |
| | HuGene_st:228766_at:512885-HuGene_st | | | | | |
| HIGD1A | 936261-HuGene_st:824654- | 6.29E-14 | 2.4011 | 2.09 | 88.5 | 81.3-93.5 |
| | HuGene_st:165981-HuGene_st:225595- | | | | | |
| | HuGene_st:290747-HuGene_st:641007- | | | | | |
| | HuGene_st:1073341-HuGene_st:761357- | | | | | |
| | HuGene_st:818902-HuGene_st:1038575- | | | | | |
| | HuGene_st:102828-HuGene_st:533707- | | | | | |
| | HuGene_st:414059- | | | | | |
| | HuGene_st:217845_x_at:852712- | | | | | |
| | HuGene_st:221896_s_at:291015- | | | | | |
| | HuGene_st:295866-HuGene_st:520869- | | | | | |
| | HuGene_st:155393-HuGene_st:460566- | | | | | |
| | HuGene_st:969731-HuGene_st:170508- | | | | | |
| | HuGene_st:744650-HuGene_st:309982- | | | | | |
| | HuGene_st:795342-HuGene_st:387733- | | | | | |
| | HuGene_st:324047-HuGene_st:239559- | | | | | |
| | HuGene_st:1055553-HuGene_st:827869- | | | | | |
| | HuGene_st:1058468-HuGene_st | | | | | |
| NR3C1 | 111450-HuGene_st:434945- | 7.65E-14 | 2.3495 | 2.51 | 88 | 80.6-93.2 |
| | HuGene_st:1054045- | | | | | |
| | HuGene_st:201866_s_at:98168- | | | | | |
| | HuGene_st:402326-HuGene_st:154154- | | | | | |
| | HuGene_st:117026-HuGene_st:1027472- | | | | | |
| | HuGene_st:517524-HuGene_st:540910- | | | | | |
| | HuGene_st:538776-HuGene_st:550290- | | | | | |
| | HuGene_st:460796- | | | | | |
| | HuGene_st:211671_s_at:216321_s_at:201 865_x_at:649431-HuGene_st:461795- | | | | | |
| | | | | | | |
| | HuGene_st | | | | | |
| LOC63928 | 206149_at:681928-HuGene_st:1004459- | 8.09E-14 | 2.352 | 3.2 | 88 | 80.6-93.2 |
| | HuGene_st:249694-HuGene_st:80283- | | | | | |
| | HuGene_st:323497-HuGene_st:650243- | | | | | |
| | HuGene_st:872231-HuGene_st:253533- | | | | | |
| | HuGene_st:270238-HuGene_st:282291- | | | | | |
| | HuGene_st:268597-HuGene_st:913922- | | | | | |
| | HuGene_st:416350-HuGene_st:992808- | | | | | |
| | HuGene_st:472127-HuGene_st:988731- | | | | | |
| | HuGene_st:243977-HuGene_st:802623- | | | | | |
| | HuGene_st:314649-HuGene_st | | | | | |
| MXD1 | 226275_at:206877_at:602969- | 8.35E-14 | 2.4077 | 2.11 | 88.6 | 81.3-93.6 |
| | HuGene_st:127172-HuGene_st:577694- | | | | | |
| | HuGene_st:53257- | | | | | |
| | HuGene_st:228846_at:669193- | | | | | |
| | HuGene_st:37027-HuGene_st:458872- | | | | | |
| | HuGene_st:518444-HuGene_st:410159- | | | | | |
| | HuGene_st:77113-HuGene_st:280760- | | | | | |
| | HuGene_st:229252-HuGene_st:318164- | | | | | |
| | HuGene_st:393130-HuGene_st:172809- | | | | | |
| | HuGene st:1020416-HuGene_st:1050501- | | | | | |
| | HuGene_st:269397-HuGene_st:68122- | | | | | |
| | HuGene_st:423400-HuGene_st:103208- | | | | | |
| | HuGene_st:936311-HuGene_st | | | | | |
| C9orf19 | 268503-HuGene_st:209268- | 1.74E-13 | 2.3659 | 1.95 | 88.2 | 80.9-93.3 |
| | HuGene_st:225604_s_at:518559- | | | | | |
| | HuGene_st:1007612-HuGene_st:499070- | | | | | |
| | HuGene_st:225602_at:291028- | | | | | |
| | HuGene_st:615472-HuGene_st:3136- | | | | | |
| | HuGene_st:926635-HuGene_st:219003- | | | | | |
| | HuGene_st:396117-HuGene_st:94077- | | | | | |
| | HuGene_st:747558-HuGene_st:226948- | | | | | |
| | HuGene_st:940376-HuGene_st:977640- | | | | | |
| | HuGene_st:316643-HuGene_st:382182- | | | | | |
| | HuGene_st:745966-HuGene_st | | | | | |
| DCN | 6640- | 2.05E-13 | 2.3127 | 5.62 | 87.6 | 80.3-92.9 |
| | HuGene_st:201893_x_at:211813_x_at:311 | | | | | |
| | 031-HuGene_st:211896_s_at:505982- | | | | | |
| | HuGene_st:219729-HuGene_st:1067446- | | | | | |
| | HuGene_st:538017-HuGene_st:13085- | | | | | |
| | HuGene_st:960643-HuGene_st:132704- | | | | | |
| | HuGene_st:253410-HuGene_st:54359- | | | | | |
| | HuGene_st:1020632-HuGene_st:973501- | | | | | |
| | HuGene_st:483405-HuGene_st:252884- | | | | | |
| | HuGene_st:274013-HuGene_st:664014- | | | | | |
| | HuGene_st:522817-HuGene_st:745324- | | | | | |
| | HuGene_st:209335_at:339470- | | | | | |
| | HuGene_st:916917-HuGene_st | | | | | |
| HSD17B2 | 281748-HuGene_st:142500- | 2.32E-13 | 2.1956 | 4.76 | 86.4 | 78.7-92 |
| | HuGene_st:602472-HuGene_st:437526- | | | | | |
| | HuGene_st:1057583-HuGene_st:999857- | | | | | |
| | HuGene_st:915850-HuGene_st:1097510- | | | | | |
| | HuGene_st:127085-HuGene_st:1088665- | | | | | |
| | HuGene_st:194898-HuGene_st:834134- | | | | | |
| | HuGene_st:360927-HuGene_st:1029542- | | | | | |
| | HuGene_st:422429-HuGene_st:573141- | | | | | |
| | HuGene_st:204818_at:918446- | | | | | |
| | HuGene_st:386936-HuGene_st:613850- | | | | | |
| | HuGene_st:489258-HuGene_st | | | | | |
| TCF21 | 804657-HuGene_st:106365- | 2.51E-13 | 2.3047 | 2.34 | 87.5 | 80.1-92.8 |
| | HuGene_st:710514-HuGene_st:299556- | | | | | |
| | HuGene_st:608149-HuGene_st:242233- | | | | | |
| | HuGene_st:655881-HuGene_st:356773- | | | | | |
| | HuGene_st:788445-HuGene_st:709897- | | | | | |
| | HuGene_st:605488-HuGene_st:652466- | | | | | |
| | HuGene_st:204931_at:830709- | | | | | |
| | HuGene_st:273418-HuGene_st:1004754- | | | | | |
| | HuGene_st:990861-HuGene_st:238739- | | | | | |
| | HuGene_st:836241-HuGene_st:110045- | | | | | |
| | HuGene_st:229529_at:1001969- | | | | | |
| | HuGene_st | | | | | |
| MRC1 | 467197-HuGene_st:758500- | 2.72E-13 | 2.3106 | 2.6 | 87.6 | 80.2-92.9 |
| | HuGene_st:600690-HuGene_st:270842- | | | | | |
| | HuGene_st:787475-HuGene_st:710055- | | | | | |
| | HuGene_st:295499-HuGene_st:757670- | | | | | |
| | HuGene_st:994062-HuGene_st:587369- | | | | | |
| | HuGene_st:224499-HuGene_st:428438- | | | | | |
| | HuGene_st:577964-HuGene_st:159156- | | | | | |
| | HuGene_st:298439-HuGene_st:1088275- | | | | | |
| | HuGene_st:715300-HuGene_st:1627- | | | | | |
| | HuGene_st:513230-HuGene_st:106268- | | | | | |
| | HuGene_st:204438_at | | | | | |
| ADH1C | 661427-HuGene_st:243544_at:634797- | 2.94E-13 | 2.3724 | 3.1 | 88.2 | 81-93.3 |
| | HuGene_st:291687-HuGene_st:972177- | | | | | |
| | HuGene_st:316339-HuGene_st:580686- | | | | | |
| | HuGene_st:206262_at:211851- | | | | | |
| | HuGene_st:641619-HuGene_st:142959- | | | | | |
| | HuGene_st:705200-HuGene_st:1063529- | | | | | |
| | HuGene_st:916148-HuGene_st:901368- | | | | | |
| | HuGene_st:43611-HuGene_st:789755- | | | | | |
| | HuGene_st:410220-HuGene_st:289785- | | | | | |
| | HuGene_st:155724-HuGene_st:289958- | | | | | |
| | HuGene_st:232595-HuGene_st | | | | | |
| BCHE | 228090-HuGene_st:752051- | 3.18E-13 | 2.2535 | 6.21 | 87 | 79.4-92.4 |
| | HuGene_st:800167-HuGene_st:221362- | | | | | |
| | HuGene_st:155900-HuGene_st:717363- | | | | | |
| | HuGene_st:536584-HuGene_st:146780- | | | | | |
| | HuGene_st:302487-HuGene_st:508472- | | | | | |
| | HuGene_st:516293-HuGene_st:968992- | | | | | |
| | HuGene_st:666625-HuGene_st:923158- | | | | | |
| | HuGene_st | | | | | |
| CCDC80 | 1072039-HuGene_st:973747- | 3.60E-13 | 2.2322 | 3.67 | 86.8 | 79.2-92.3 |
| | HuGene_st:580839-HuGene_st:583669- | | | | | |
| | HuGene_st:290210-HuGene_st:308086- | | | | | |
| | HuGene_st:679211-HuGene_st:43315- | | | | | |
| | HuGene_st:1080603-HuGene_st:852141- | | | | | |
| | HuGene_st:569200-HuGene_st:92231- | | | | | |
| | HuGene_st:535306-HuGene_st:427093- | | | | | |
| | HuGene_st: 1003098-HuGene_st:408541- | | | | | |
| | HuGene_st:135243-HuGene_st:225298- | | | | | |
| | HuGene_st:225242_s_at:225241_at:63307 9-HuGene_st:43353-HuGene_st:642388- | | | | | |
| | | | | | | |
| | HuGene_st:243864_at | | | | | |
| CITED2 | 125201-HuGene_st:410723- | 5.29E-13 | 2.2712 | 1.64 | 87.2 | 79.7-92.6 |
| | HuGene_st:463405-HuGene_st:401168- | | | | | |
| | HuGene_st:1012057-HuGene_st:235057- | | | | | |
| | HuGene_st:361772- | | | | | |
| | HuGene_st:207980_s_at:1091907- | | | | | |
| | HuGene_st:985355-HuGene_st:175990- | | | | | |
| | HuGene_st:227287_at:48433- | | | | | |
| | HuGene_st:209357_at:477746- | | | | | |
| | HuGene_st:243264-HuGene_st:904401- | | | | | |
| | HuGene_st:328536-HuGene_st:1095110- | | | | | |
| | HuGene_st:89784-HuGene_st:206734- | | | | | |
| | HuGene_st:927615-HuGene_st | | | | | |
| PCK1 | 208383_s_at:221954-HuGene_st:772877- | 5.74E-13 | 2.3236 | 3.6 | 87.7 | 80.4-93 |
| | HuGene_st:518166-HuGene_st:1098164- | | | | | |
| | HuGene_st:241175-HuGene_st:111987- | | | | | |
| | HuGene_st:395761-HuGene_st:846308- | | | | | |
| | HuGene_st:584532-HuGene_st:547720- | | | | | |
| | HuGene_st:1018765-HuGene_st:817976- | | | | | |
| | HuGene_st:454213-HuGene_st:66958- | | | | | |
| | HuGene_st:301878-HuGene_st:804419- | | | | | |
| | HuGene_st:442879-HuGene_st:211870- | | | | | |
| | HuGene_st:638998-HuGene_st:448080- | | | | | |
| | HuGene_st:948845-HuGene_st:313770- | | | | | |
| | HuGene_st | | | | | |
| ANK3 | 472262-HuGene_st:337923- | 7.46E-13 | 2.2325 | 2.45 | 86.8 | 79.2-92.3 |
| | HuGene_st:715126-HuGene_st:416201- | | | | | |
| | HuGene_st:942091-HuGene_st:364373- | | | | | |
| | HuGene_st:207950_s_at:209442_x_at:410 536-HuGene_st:206385_s_at:845022- | | | | | |
| | | | | | | |
| | HuGene_st:400240-HuGene_st:651770- | | | | | |
| | HuGene_st:29904-HuGene_st:215314_at | | | | | |
| F13A1 | 412872-HuGene_st:755197- | 8.82E-13 | 2.1855 | 4.74 | 86.3 | 78.6-91.9 |
| | HuGene_st:1023946-HuGene_st:591121- | | | | | |
| | HuGene_st:912274-HuGene_st:465239- | | | | | |
| | HuGene_st:1002989-HuGene_st:541053- | | | | | |
| | HuGene_st:1052450-HuGene_st:204339- | | | | | |
| | HuGene_st:176807-HuGene_st:58339- | | | | | |
| | HuGene_st:42656-HuGene_st:965853- | | | | | |
| | HuGene_st:1096456-HuGene_st:365069- | | | | | |
| | HuGene_st:300786-HuGene_st:739769- | | | | | |
| | HuGene_st:70605-HuGene_st:174382- | | | | | |
| | HuGene_st:203305_at:783884-HuGene_st | | | | | |
| ADAMDE C1 | 893574-HuGene_st:1087946- | 9.04E-13 | 2.2604 | 3.19 | 87.1 | 79.5-92.5 |
| | HuGene_st:453119-HuGene_st:995166- | | | | | |
| | HuGene_st:660805-HuGene_st:162940- | | | | | |
| | HuGene_st:402188-HuGene_st:782577- | | | | | |
| | HuGene_st:89831-HuGene_st:11993- | | | | | |
| | HuGene_st:1020874-HuGene_st:997719- | | | | | |
| | HuGene_st:268541-HuGene_st:41641- | | | | | |
| | HuGene_st:1004377-HuGene_st:53970- | | | | | |
| | HuGene_st:206134_at:904943- | | | | | |
| | HuGene_st:644140-HuGene_st:332803- | | | | | |
| | HuGene_st:16627-HuGene_st:1555- | | | | | |
| | HuGene_st | | | | | |
| ABI3BP | 801423-HuGene_st:1065486- | 9.47E-13 | 2.2176 | 3.62 | 86.6 | 79-92.2 |
| | HuGene_st:692353-HuGene_st:456260- | | | | | |
| | HuGene_st:686533-HuGene_st:68008- | | | | | |
| | HuGene_st:317350-HuGene_st:239950- | | | | | |
| | HuGene_st:604210-HuGene_st:20407- | | | | | |
| | HuGene_st:1099546-HuGene_st:395466- | | | | | |
| | HuGene_st:548603-HuGene_st:931079- | | | | | |
| | HuGene_st:159325-HuGene_st:251067- | | | | | |
| | HuGene_st:1056116-HuGene_st:224253- | | | | | |
| | HuGene_st:885069-HuGene_st:1075816- | | | | | |
| | HuGene_st:592859-HuGene_st | | | | | |
| PAG1 | 572231-HuGene_st:431317- | 1.11E-12 | 2.215 | 2.68 | 86.6 | 79-92.1 |
| | HuGene_st:342766-HuGene_st:1080197- | | | | | |
| | HuGene_st:225622_at:81123- | | | | | |
| | HuGene_st:859237-HuGene_st:287765- | | | | | |
| | HuGene_st:225626_at:743424- | | | | | |
| | HuGene_st:759835-HuGene_st:622195- | | | | | |
| | HuGene_st:208082-HuGene_st:768207- | | | | | |
| | HuGene_st:657358-HuGene_st:506609- | | | | | |
| | HuGene_st:226184-HuGene_st:540710- | | | | | |
| | HuGene_st:236888-HuGene_st:36408- | | | | | |
| | HuGene_st:546382-HuGene_st:498489- | | | | | |
| | HuGene_st:227354_at | | | | | |
| KRT20 | 11827-HuGene_st:50958- | 1.26E-12 | 2.0981 | 5.49 | 85.3 | 77.4-91.1 |
| | HuGene_st:684746-HuGene_st:574501- | | | | | |
| | HuGene_st:270813-HuGene_st:421213- | | | | | |
| | HuGene_st:213953_at:225474- | | | | | |
| | HuGene_st:310071-HuGene_st:356429- | | | | | |
| | HuGene_st:997077-HuGene_st:963515- | | | | | |
| | HuGene_st:944854-HuGene_st:178191- | | | | | |
| | HuGene_st:613655-HuGene_st:337174- | | | | | |
| | HuGene_st:268017-HuGene_st:81041- | | | | | |
| | HuGene_st:85506-HuGene_st:798272- | | | | | |
| | HuGene_st:925916-HuGene_st:780714- | | | | | |
| | HuGene_st:138025-HuGene_st | | | | | |
| ENTPD5 | 760608-HuGene_st:231676_s_at:831252- | 1.43E-12 | 2.1686 | 3.01 | 86.1 | 78.3-91.7 |
| | HuGene_st:497317-HuGene_st:712688- | | | | | |
| | HuGene_st:259180-HuGene_st:832617- | | | | | |
| | HuGene_st:785663- | | | | | |
| | HuGene_st:205757_at:553118- | | | | | |
| | HuGene_st:384386-HuGene_st:911183- | | | | | |
| | HuGene_st:440835-HuGene_st:150070- | | | | | |
| | HuGene_st:821276-HuGene_st:14461- | | | | | |
| | HuGene_st:520930-HuGene_st:181407- | | | | | |
| | HuGene_st:294287- | | | | | |
| | HuGene_st:226594_at:551172- | | | | | |
| | HuGene_st:1040872-HuGene_st:1048020- | | | | | |
| | HuGene_st | | | | | |
| MALL | 209373_at:628866-HuGene_st:1092892- | 2.10E-12 | 2.1585 | 3.96 | 86 | 78.2-91.7 |
| | HuGene_st:488187-HuGene_st:515617- | | | | | |
| | HuGene_st:649198-HuGene_st:991653- | | | | | |
| | HuGene_st:301852-HuGene_st:1067925- | | | | | |
| | HuGene_st:451072-HuGene_st:102723- | | | | | |
| | HuGene_st:670639-HuGene_st:848482- | | | | | |
| | HuGene_st:928502-HuGene_st:787192- | | | | | |
| | HuGene_st:916920-HuGene_st:959742- | | | | | |
| | HuGene_st:711672-HuGene_st:20252- | | | | | |
| | HuGene_st:225337-HuGene_st | | | | | |
| MFAP4 | 308095-HuGene_st:1004622- | 2.96E-12 | 2.1813 | 2.51 | 86.2 | 78.5-91.9 |
| | HuGene_st:754788- | | | | | |
| | HuGene_st:212713_at:911082- | | | | | |
| | HuGene_st:767889-HuGene_st:5569- | | | | | |
| | HuGene_st:308458-HuGene_st:699815- | | | | | |
| | HuGene_st:658908-HuGene_st:73048- | | | | | |
| | HuGene_st:515283-HuGene_st:671512- | | | | | |
| | HuGene_st:792571-HuGene_st:689375- | | | | | |
| | HuGene_st:590963-HuGene_st:821074- | | | | | |
| | HuGene_st:132543-HuGene_st:655171- | | | | | |
| | HuGene_st:341975-HuGene_st:1048103- | | | | | |
| | HuGene_st | | | | | |
| SRI | 1018107-HuGene_st:929936- | 3.03E-12 | 2.0985 | 2.43 | 85.3 | 77.4-91.1 |
| | HuGene_st:596109-HuGene_st:469173- | | | | | |
| | HuGene_st:235900-HuGene_st:303619- | | | | | |
| | HuGene_st:792372-HuGene_st:1034405- | | | | | |
| | HuGene_st:145638-HuGene_st:76828- | | | | | |
| | HuGene_st:103032-HuGene_st:788729- | | | | | |
| | HuGene_st:262807-HuGene_st:452805- | | | | | |
| | HuGene_st:208921_s_at:863794- | | | | | |
| | HuGene_st:172167-HuGene_st:309679- | | | | | |
| | HuGene_st:1058573- | | | | | |
| | HuGene_st:208920_at:235325- | | | | | |
| | HuGene_st:813384-HuGene_st | | | | | |
| SGK | 425210-HuGene_st:201739_at:137804- | 3.12E-12 | 2.1186 | 4.08 | 85.5 | 77.7-91.4 |
| | HuGene_st:182666-HuGene_st:271994- | | | | | |
| | HuGene_st:629671-HuGene_st:350855- | | | | | |
| | HuGene_st:179953-HuGene_st:1039396- | | | | | |
| | HuGene_st:664449-HuGene_st:1049246- | | | | | |
| | HuGene_st: 1100037-HuGene_st: 1094410- | | | | | |
| | HuGene_st:677142-HuGene_st:231137- | | | | | |
| | HuGene_st:393473-HuGene_st | | | | | |
| IGHM | 1001583-HuGene_st:162285- | 3.25E-12 | 2.3285 | 1.73 | 87.8 | 80.4-93 |
| | HuGene_st:1061429-HuGene_st:359832- | | | | | |
| | HuGene_st:60182-HuGene_st:1002550- | | | | | |
| | HuGene_st:311771-HuGene_st:790352- | | | | | |
| | HuGene_st:977059-HuGene_st:587401- | | | | | |
| | HuGene_st:343597-HuGene_st:25657- | | | | | |
| | HuGene_st:640259-HuGene_st:184414- | | | | | |
| | HuGene_st:336659-HuGene_st:1008406- | | | | | |
| | HuGene_st:1044209-HuGene_st:519020- | | | | | |
| | HuGene_st:427467-HuGene_st:669553- | | | | | |
| | HuGene_st:722843-HuGene_st:126162- | | | | | |
| | HuGene_st:654578-HuGene_st:146515- | | | | | |
| | HuGene_st:1100721-HuGene-st:823307- | | | | | |
| | HuGene_st:899494-HuGene_st:952685- | | | | | |
| | HuGene_st:215118_s_at:235399- | | | | | |
| | HuGene_st:802521-HuGene_st:126175- | | | | | |
| | HuGene_st:910847- | | | | | |
| | HuGene_st:212827_at:722700- | | | | | |
| | HuGene_st:173623-HuGene_st:1042953- | | | | | |
| | HuGene_st:38807-HuGene_st:408026- | | | | | |
| | HuGene_st | | | | | |
| ZCWPW2 | 1001583-HuGene_st:162285- | 3.25E-12 | 2.3264 | 1.73 | 87.8 | 80.4-93 |
| | HuGene_st:1061429-HuGene_st:359832- | | | | | |
| | HuGene_st:60182-HuGene_st:1002550- | | | | | |
| | HuGene_st:311771-HuGene_st:790352- | | | | | |
| | HuGene_st:977059-HuGene_st:587401- | | | | | |
| | HuGene_st:343597-HuGene_st:25657- | | | | | |
| | HuGene_st:640259-HuGene_st:184414- | | | | | |
| | HuGene_st:336659-HuGene_st:1008406- | | | | | |
| | HuGene_st:1044209-HuGene_st:519020- | | | | | |
| | HuGene_st:427467-HuGene_st:669553- | | | | | |
| | HuGene_st:722843-HuGene_st:126162- | | | | | |
| | HuGene_st:654578-HuGene_st:146515- | | | | | |
| | HuGene_st:1100721-HuGene_st:823307- | | | | | |
| | HuGene_st:899494-HuGene_st:952685- | | | | | |
| | HuGene_st:215118_s_at:235399- | | | | | |
| | HuGene_st:802521-HuGene_st:126175- | | | | | |
| | HuGene_st:910847- | | | | | |
| | HuGene_st:212827_at:722700- | | | | | |
| | HuGene_st:173623-HuGene_st:1042953- | | | | | |
| | HuGene_st:38807-HuGene_st:408026- | | | | | |
| | HuGene_st | | | | | |
| IL8 | 1001583-HuGene_st:162285- | 3.28E-12 | 2.3266 | 1.73 | 87.8 | 80.4-93 |
| | HuGene_st:1061429-HuGene_st:359832- | | | | | |
| | HuGene_st:60182-HuGene_st:1002550- | | | | | |
| | HuGene_st:311771-HuGene_st:790352- | | | | | |
| | HuGene_st:977059-HuGene_st:587401- | | | | | |
| | HuGene_st:343597-HuGene_st:25657- | | | | | |
| | HuGene_st:640259-HuGene_st:184414- | | | | | |
| | HuGene_st:336659-HuGene_st:1008406- | | | | | |
| | HuGene_st:1044209-HuGene_st:519020- | | | | | |
| | HuGene_st:427467-HuGene_st:669553- | | | | | |
| | HuGene_st:722843-HuGene_st:126162- | | | | | |
| | HuGene_st:654578-HuGene_st:146515- | | | | | |
| | HuGene_st:1100721-HuGene_st:823307- | | | | | |
| | HuGene_st:899494-HuGene_st:952685- | | | | | |
| | HuGene_st:215118_s_at:235399- | | | | | |
| | HuGene_st:802521-HuGene_st:126175- | | | | | |
| | HuGene_st:910847- | | | | | |
| | HuGene_st:212827_at:722700- | | | | | |
| | HuGene_st:173623-HuGene_st: 1042953- | | | | | |
| | HuGene_st:38807-HuGene_st:408026- | | | | | |
| | HuGene_st | | | | | |
| IGHA1 | 1001583-HuGene_st:162285- | 3.28E-12 | 2.3276 | 1.73 | 87.8 | 80.4-93 |
| | HuGene_st:1061429-HuGene_st:359832- | | | | | |
| | HuGene_st:60182-HuGene_st:1002550- | | | | | |
| | HuGene_st:311771-HuGene_st:790352- | | | | | |
| | HuGene_st:977059-HuGene_st:587401- | | | | | |
| | HuGene_st:343597-HuGene_st:25657- | | | | | |
| | HuGene_st:640259-HuGene_st:184414- | | | | | |
| | HuGene_st:336659-HuGene_st:1008406- | | | | | |
| | HuGene_st:235364_at:1044209- | | | | | |
| | HuGene_st:519020-HuGene_st:427467- | | | | | |
| | HuGene_st:669553-HuGene_st:722843- | | | | | |
| | HuGene_st:208763_s_at:207001_x_at:126 162-HuGene_st:654578- | | | | | |
| | | | | | | |
| | HuGene_st:146515-HuGene_st:1100721- | | | | | |
| | HuGene_st:823307-HuGene_st:899494- | | | | | |
| | HuGene_st:952685- | | | | | |
| | HuGene_st:215118_s_at:235399- | | | | | |
| | HuGene_st:802521-HuGene_st:126175- | | | | | |
| | HuGene_st:910847- | | | | | |
| | HuGene_st:212827_at:722700- | | | | | |
| | HuGene_st:173623-HuGene_st:1042953- | | | | | |
| | HuGene_st:38807-HuGene_st:408026- | | | | | |
| | HuGene_st | | | | | |
| MGC14376 | 20793-HuGene_st:228915- | 3.39E-12 | 2.1014 | 2.58 | 85.3 | 77.5-91.2 |
| | HuGene_st:630688-HuGene_st:117325- | | | | | |
| | HuGene_st:532108-HuGene_st:373670- | | | | | |
| | HuGene_st:552164-HuGene_st:1004615- | | | | | |
| | HuGene_st:527421-HuGene_st:880155- | | | | | |
| | HuGene_st:10973-HuGene_st:395595- | | | | | |
| | HuGene_st:312241-HuGene_st:644241- | | | | | |
| | HuGene_st:214696_at:234825- | | | | | |
| | HuGene_st:1002029-HuGene_st:1032648- | | | | | |
| | HuGene_st | | | | | |
| ETHE1 | 1008653-HuGene_st:507181- | 3.46E-12 | 2.2097 | 1.75 | 86.5 | 78.9-92.1 |
| | HuGene_st:435886-HuGene_st:296660- | | | | | |
| | HuGene_st:764234-HuGene_st:918966- | | | | | |
| | HuGene_st:1078682-HuGene_st:789091- | | | | | |
| | HuGene_st:100698- | | | | | |
| | HuGene_st:204034_at:301334- | | | | | |
| | HuGene_st:869871-HuGene_st:841094- | | | | | |
| | HuGene_st:612751-HuGene_st:43763- | | | | | |
| | HuGene_st:853527-HuGene_st:593768- | | | | | |
| | HuGene_st:52787-HuGene_st:555225- | | | | | |
| | HuGene_st:1028299-HuGene_st | | | | | |
| CES2 | 817786-HuGene_st:63720- | 3.51E-12 | 2.0993 | 2.87 | 85.3 | 77.4-91.1 |
| | HuGene_st:527565-HuGene_st:678893- | | | | | |
| | HuGene_st:209668_x_at:825633- | | | | | |
| | HuGene_st:336873-HuGene_st:409854- | | | | | |
| | HuGene_st:213509_x_at:134988- | | | | | |
| | HuGene_st:446296-HuGene_st:842160- | | | | | |
| | HuGene_st:827636- | | | | | |
| | HuGene_st:209667_at:882043- | | | | | |
| | HuGene_st:279172-HuGene_st:1081267- | | | | | |
| | HuGene_st:756682-HuGene_st:90632- | | | | | |
| | HuGene_st | | | | | |
| GPM6B | 599862-HuGene_st:754598- | 4.04E-12 | 2.1563 | 3.59 | 86 | 78.2-91.6 |
| | HuGene_st:503642-HuGene_st:224935- | | | | | |
| | HuGene_st:754449-HuGene_st:577489- | | | | | |
| | HuGene_st:1073242-HuGene_st:560873- | | | | | |
| | HuGene_st:1003662-HuGene_st:430217- | | | | | |
| | HuGene_st:903323-HuGene_st:231962- | | | | | |
| | HuGene_st:244945-HuGene_st:562460- | | | | | |
| | HuGene_st:583561-HuGene_st:209168_at | | | | | |
| SYNPO2 | 800385-HuGene_st:938576- | 5.07E-12 | 2.1029 | 6.43 | 85.3 | 77.5-91.2 |
| | HuGene_st:54552-HuGene_st:946854- | | | | | |
| | HuGene_st:278208-HuGene_st:170777- | | | | | |
| | HuGene_st:796143-HuGene_st:544243- | | | | | |
| | HuGene_st:469048-HuGene_st:364454- | | | | | |
| | HuGene_st:400491-HuGene_st:22204-v | | | | | |
| | HuGene_st:315299-HuGene_st:526384- | | | | | |
| | HuGene_st:841867- | | | | | |
| | HuGene_st:244108_at:2158- | | | | | |
| | HuGene_st:283531-HuGene_st:407855- | | | | | |
| | HuGene_st:225721_at:225720_at:225895_ at:225894_at:1017102- | | | | | |
| | | | | | | |
| | HuGene_st:227662_at:232119_at | | | | | |
| GCG | 1044997-HuGene_st:682227- | 5.58E-12 | 2.1165 | 12.56 | 85.5 | 77.7-91.3 |
| | HuGene_st:368693-HuGene_st:25836- | | | | | |
| | HuGene_st:228935-HuGene_st:1030502- | | | | | |
| | HuGene_st:12466-HuGene_st:1098133- | | | | | |
| | HuGene_st:994392-HuGene_st:581476-v | | | | | |
| | HuGene_st:799068-HuGene_st:350795- | | | | | |
| | HuGene_st:705389-HuGene_st:981135- | | | | | |
| | HuGene_st:736837-HuGene_st:253854- | | | | | |
| | HuGene_st:1032660-HuGene_st:273941- | | | | | |
| | HuGene_st:206422_at:940866- | | | | | |
| | HuGene_st:514731-HuGene_st | | | | | |
| MGP | 696658-HuGene_st:618386- | 6.11E-12 | 2.1428 | 4.68 | 85.8 | 78-91.5 |
| | HuGene_st:202291_s_at:195765- | | | | | |
| | HuGene_st:3486-HuGene_st:829319- | | | | | |
| | HuGene_st:731815-HuGene_st:829950- | | | | | |
| | HuGene_st:601468-HuGene_st:366689- | | | | | |
| | HuGene_st:460503-HuGene_st:712045- | | | | | |
| | HuGene_st:545937-HuGene_st:148839- | | | | | |
| | HuGene_st:704906-HuGene_st:612318- | | | | | |
| | HuGene_st:708651-HuGene_st:722064- | | | | | |
| | HuGene_st:846497-HuGene_st:1084237- | | | | | |
| | HuGene_st:854168-HuGene_st | | | | | |
| PDE9A | 779446-HuGene_st:768516- | 6.56E-12 | 2.145 | 3.91 | 85.8 | 78-91.5 |
| | HuGene_st:609563-HuGene_st:387613- | | | | | |
| | HuGene_st:594122-HuGene_st:774367- | | | | | |
| | HuGene_st:221476-HuGene_st:140410- | | | | | |
| | HuGene_st:812534-HuGene_st:581440- | | | | | |
| | HuGene_st:205593_s_at:142832- | | | | | |
| | HuGene_st:767027-HuGene_st:464973- | | | | | |
| | HuGene_st:97182-HuGene_st:128487- | | | | | |
| | HuGene_st:206471-HuGene_st:878522- | | | | | |
| | HuGene_st:237139_at:988884- | | | | | |
| | HuGene_st:237283_at | | | | | |
| LRRC19 | 177641-HuGene_st:1070020- | 6.75E-12 | 2.0763 | 4.51 | 85 | 77.1-91 |
| | HuGene_st:1055140-HuGene_st:525999- | | | | | |
| | HuGene_st:937256-HuGene_st:620791- | | | | | |
| | HuGene_st:891251-HuGene_st:707559- | | | | | |
| | HuGene_st:892056-HuGene_st:764919- | | | | | |
| | HuGene_st:382143-HuGene_st:52584- | | | | | |
| | HuGene_st:920414-HuGene_st:1028155- | | | | | |
| | HuGene_st:755055-HuGene_st:678651- | | | | | |
| | HuGene_st:1080156-HuGene_st:530282- | | | | | |
| | HuGene_st:523877-HuGene_st:335198- | | | | | |
| | HuGene_st:787709-HuGene_st:153175-v | | | | | |
| | HuGene_st:220376_at | | | | | |
| HSD11B2 | 78582-HuGene_st:653519- | 6.97E-12 | 2.0895 | 2.53 | 85.2 | 77.3-91.1 |
| | HuGene_st:630341-HuGene_st:919091- | | | | | |
| | HuGene_st:132633-HuGene_st:907106- | | | | | |
| | HuGene_st:621993-HuGene_st:1089280- | | | | | |
| | HuGene_st:34729-HuGene_st:342165- | | | | | |
| | HuGene_st:887157-HuGene_st:323862- | | | | | |
| | HuGene_st:870050- | | | | | |
| | HuGene_st:204130_at:635792- | | | | | |
| | HuGene_st:606173-HuGene_st:867938- | | | | | |
| | HuGene_st:113467-HuGene_st:806665- | | | | | |
| | HuGene_st | | | | | |
| GNG2 | 1555766_a_at:224965_at:1011730- | 7.25E-12 | 2.1437 | 1.91 | 85.8 | 78-91.5 |
| | HuGene_st:77527-HuGene_st:669616- | | | | | |
| | HuGene_st:223943_s_at:938512- | | | | | |
| | HuGene_st:450519-HuGene_st:1025129-v | | | | | |
| | HuGene_st:30927-HuGene_st:382458- | | | | | |
| | HuGene_st:330996-HuGene_st:545415- | | | | | |
| | HuGene_st:774093-HuGene_st:758152- | | | | | |
| | HuGene_st:176111-HuGene_st:743969- | | | | | |
| | HuGene_st | | | | | |
| GCNT3 | 230376_at:254999-HuGene_st:476622- | 8.39E-12 | 2.0861 | 3.94 | 85.2 | 77.2-91 |
| | HuGene_st:219508_at:452671- | | | | | |
| | HuGene_st:732602-HuGene_st:306264- | | | | | |
| | HuGene_st:696871-HuGene_st:721027- | | | | | |
| | HuGene_st:235453-HuGene_st:676136- | | | | | |
| | HuGene_st:777254-HuGene_st:506598- | | | | | |
| | HuGene_st:598423-HuGene_st:699538- | | | | | |
| | HuGene_st:220940-HuGene_st:804174- | | | | | |
| | HuGene_st:12527-HuGene_st:802770- | | | | | |
| | HuGene_st:403287-HuGene_st:351721- | | | | | |
| | HuGene_st:581784-HuGene_st:700724- | | | | | |
| | HuGene_st:398939-HuGene_st | | | | | |
| UGT1A6 | 116025-HuGene_st:6488- | 1.32E-11 | 2.07 | 4.56 | 85 | 77-90.9 |
| | HuGene_st:881135- | | | | | |
| | HuGene_st:230953_at:221304_at:221305_ | | | | | |
| | s_at:511516-HuGene_st:594963- | | | | | |
| | HuGene_st:396121-HuGene_st:123777- | | | | | |
| | HuGene_st:1016481- | | | | | |
| | HuGene_st:204532_x_at:683377- | | | | | |
| | HuGene_st:1055169-HuGene_st:1035103- | | | | | |
| | HuGene_st:1088102- | | | | | |
| | HuGene_st:207126_x_at:42874- | | | | | |
| | HuGene_st:215125_s_at:206094_x_at:208 596_s_at:97211-HuGene_st:1009861- | | | | | |
| | | | | | | |
| | HuGene_st:603368- | | | | | |
| | HuGene_st:232654_s_at:625897- | | | | | |
| | HuGene_st:998604-HuGene_st | | | | | |
| UGT1A8 | 116025-HuGene_st:6488- | 1.32E-11 | 2.0687 | 4.56 | 85 | 77-90.9 |
| | HuGene_st:881135- | | | | | |
| | HuGene_st:230953_at:221304_at:221305_ s_at:511516-HuGene_st:594963- | | | | | |
| | | | | | | |
| | HuGene_st:396121-HuGene_st:123777- | | | | | |
| | HuGene_st:1016481- | | | | | |
| | HuGene_st:204532_x_at:683377- | | | | | |
| | HuGene_st:1055169-HuGene_st:1035103- | | | | | |
| | HuGene_st:1088102- | | | | | |
| | HuGene_st:207126_x_at:42874- | | | | | |
| | HuGene_st: | | | | | |
| | | | | | | |
| | HuGene_st:603368- | | | | | |
| | HuGene_st:232654_s_at:625897- | | | | | |
| | HuGene_st:998604-HuGene_st | | | | | |
| UGT1A1 | 116025-HuGene_st:6488- | 1.32E-11 | 2.0661 | 4.56 | 84.9 | 77-90.8 |
| | HuGene_st:881135- | | | | | |
| | HuGene_st:230953_at:221304_at:221305_ s_at:511516-HuGene_st:594963- | | | | | |
| | | | | | | |
| | HuGene_st:396121-HuGene_st:123777- | | | | | |
| | HuGene_st:1016481- | | | | | |
| | HuGene_st:204532_x_at:683377- | | | | | |
| | HuGene_st:1055169-HuGene_st:1035103- | | | | | |
| | HuGene_st:1088102- | | | | | |
| | HuGene_st:207126_x_at:42874- | | | | | |
| | HuGene_st:215125_s_at:206094_x_at:208 596_s_at:97211-HuGene_st:1009861- | | | | | |
| | | | | | | |
| | HuGene_st:603368- | | | | | |
| | HuGene_st:232654_s_at:625897- | | | | | |
| | HuGene_st:998604-HuGene_st | | | | | |
| UGT1A3 | 116025-HuGene_st:6488- | 1.32E-11 | 2.07 | 4.56 | 85 | 77-90.9 |
| | HuGene_st:881135- | | | | | |
| | HuGene_st:230953_at:221304_at:221305_ s_at:511516-HuGene_st:594963- | | | | | |
| | | | | | | |
| | HuGene_st:396121-HuGene_st:123777- | | | | | |
| | HuGene_st:1016481- | | | | | |
| | HuGene_st:204532_x_at:683377- | | | | | |
| | HuGene_st:1055169-HuGene_st:1035103- | | | | | |
| | HuGene_st:1088102- | | | | | |
| | HuGene_st:207126_x_at:42874- | | | | | |
| | HuGene_st:215125_s_at:206094_x_at:208 596_s_at:97211-HuGene_st:1009861- | | | | | |
| | | | | | | |
| | HuGene_st:603368- | | | | | |
| | HuGene_st:232654_s_at:625897- | | | | | |
| | HuGene_st:998604-HuGene_st | | | | | |
| UGT1A9 | 116025-HuGene_st:6488- | 1.32E-11 | 2.0667 | 4.56 | 84.9 | 77-90.8 |
| | HuGene_st:881135- | | | | | |
| | HuGene_st:230953_at:221304_at:221305_ s_at:511516-HuGene_st:594963- | | | | | |
| | | | | | | |
| | HuGene_st:396121-HuGene_st:123777- | | | | | |
| | HuGene_st:1016481- | | | | | |
| | HuGene_st:204532_x_at:683377- | | | | | |
| | HuGene_st:1055169-HuGene_st:1035103- | | | | | |
| | HuGene_st:1088102- | | | | | |
| | HuGene_st:207126_x_at:42874- | | | | | |
| | HuGene_st:215125_s_at:206094_x_at:208 596_s_at:97211-HuGene_st:1009861- | | | | | |
| | | | | | | |
| | HuGene_st:603368- | | | | | |
| | HuGene_st:232654_s_at:625897- | | | | | |
| | HuGene_st:998604-HuGene_st | | | | | |
| PADI2 | 391522-HuGene_st:918392- | 1.46E-11 | 2.0234 | 3.98 | 84.4 | 76.4-90.5 |
| | HuGene_st:10163-HuGene_st:399093- | | | | | |
| | HuGene_st:704572- | | | | | |
| | HuGene_st:1554385_a_at:366338- | | | | | |
| | HuGene_st:440876-HuGene_st:1075540- | | | | | |
| | HuGene_st:963259-HuGene_st:994995- | | | | | |
| | HuGene_st:315189-HuGene_st:272079- | | | | | |
| | HuGene_st:243230-HuGene_st:96325- | | | | | |
| | HuGene_st:793116-HuGene_st:362093- | | | | | |
| | HuGene_st:390381-HuGene_st:690679- | | | | | |
| | HuGene_st:209791_at:173109- | | | | | |
| | HuGene_st:339627- | | | | | |
| | HuGene_st:1554384_at | | | | | |
| CD177 | 993284-HuGene_st:444696- | 1.49E-11 | 2.0611 | 5.77 | 84.9 | 76.9-90.8 |
| | HuGene_st:79609-HuGene_st:969709- | | | | | |
| | HuGene_st:369998- | | | | | |
| | HuGene_st:219669_at:572266- | | | | | |
| | HuGene_st:536281-HuGene_st:90319- | | | | | |
| | HuGene_st:699790-HuGene_st:531312- | | | | | |
| | HuGene_st:707358-HuGene_st:180891- | | | | | |
| | HuGene_st:426820-HuGene_st:1006900- | | | | | |
| | HuGene_st:638144-HuGene_st:- | | | | | |
| | HuGene_st:134551-HuGene_st:609683- | | | | | |
| | HuGene_st | | | | | |
| FHL1 | 72610-HuGene_st:539308- | 1.63E-11 | 2.0432 | 3.25 | 84.7 | 76.7-90.6 |
| | HuGene_st:951657-HuGene_st:902973- | | | | | |
| | HuGene_st:1001828-HuGene_st:286356- | | | | | |
| | HuGene_st:899294-HuGene_st:25935- | | | | | |
| | HuGene_st:214505_s_at:210298_x_at:210 299_s_at:602362-HuGene_st:1055343- | | | | | |
| | | | | | | |
| | HuGene_st:201680-HuGene_st:319870- | | | | | |
| | HuGene_st:21197-HuGene_st:36473- | | | | | |
| | HuGene_st:689087-HuGene_st:528922- | | | | | |
| | HuGene_st:201539_s_at:1022628- | | | | | |
| | HuGene_st:201540_at:437953-v | | | | | |
| | HuGene_st:746514-HuGene_st:829623- | | | | | |
| | HuGene_st | | | | | |
| CEACAM1 | 209498_at:206576_s_at:211889_x_at:864 113-HuGene_st:14136- | 2.66E-11 | 2.0448 | 2.85 | 84.7 | 76.7-90.6 |
| | | | | | | |
| | HuGene_st:361816-HuGene_st:959455- | | | | | |
| | HuGene_st:428485-HuGene_st:747162- | | | | | |
| | HuGene_st:647202-HuGene_st:472587- | | | | | |
| | HuGene_st:344791-HuGene_st:508072- | | | | | |
| | HuGene_st:762013-HuGene_st:546799- | | | | | |
| | HuGene_st:213733-HuGene_st:652484- | | | | | |
| | HuGene_st:872278- | | | | | |
| | HuGene_st:211883_x_at:309256- | | | | | |
| | HuGene_st:699246-HuGene_st:698844- | | | | | |
| | HuGene_st:677813-HuGene_st:26592- | | | | | |
| | HuGene_st:210610_at:276021- | | | | | |
| | HuGene_st:873164-HuGene_st | | | | | |
| FBLN1 | 546550-HuGene_st:562183- | 2.82E-11 | 2.0164 | 3.55 | 84.3 | 76.3-90.4 |
| | HuGene_st:202995_s_at:507438- | | | | | |
| | HuGene_st:361582-HuGene_st:134034- | | | | | |
| | HuGene_st:804953-HuGene_st:122878- | | | | | |
| | HuGene_st:331523-HuGene_st:1089938- | | | | | |
| | HuGene_st:453797-HuGene_st:106596- | | | | | |
| | HuGene_st:862139-HuGene_st:972691- | | | | | |
| | HuGene_st:201787_at:687992- | | | | | |
| | HuGene_st:478818-HuGene_st:991293- | | | | | |
| | HuGene_st:328594-HuGene_st:844984- | | | | | |
| | HuGene_st:202994_s_at:675821- | | | | | |
| | HuGene_st:207835_at:55816-HuGene_st | | | | | |
| SEPP1 | 803943-HuGene_st:717194- | 2.83E-11 | 2.0327 | 4.15 | 84.5 | 76.6-90.6 |
| | HuGene_st:113867-HuGene_st:286524- | | | | | |
| | HuGene_st:228585-HuGene_st:824292- | | | | | |
| | HuGene_st:754251-HuGene_st:150171- | | | | | |
| | HuGene_st:904197-HuGene_st:116381- | | | | | |
| | HuGene_st:267171-HuGene_st:854108- | | | | | |
| | HuGene_st:201427_s_at:231669_at:32788 4-HuGene_st:136636-HuGene_st:164967- | | | | | |
| | | | | | | |
| | HuGene_st:1037558-HuGene_st:26213- | | | | | |
| | HuGene_st:456616-HuGene_st:1000147- | | | | | |
| | HuGene_st:748769-HuGene_st:658219- | | | | | |
| | HuGene_st:242519_at:229620_at:237475_ x_at | | | | | |
| | | | | | | |
| TBC1D9 | 237091_at:730535-HuGene_st:659317- | 3.89E-11 | 2.1454 | 1.54 | 85.8 | 78-91.5 |
| | HuGene_st:68121-HuGene_st:899166- | | | | | |
| | HuGene_st:785719-HuGene_st:138865- | | | | | |
| | HuGene_st:953028-HuGene_st:20928- | | | | | |
| | HuGene_st:969812-HuGene_st:591384- | | | | | |
| | HuGene_st:953516-HuGene_st:643103- | | | | | |
| | HuGene_st:821110-HuGene_st:988068- | | | | | |
| | HuGene_st:623144-HuGene_st:381615- | | | | | |
| | HuGene_st:708956-HuGene_st:212956_at | | | | | |
| CA12 | 89078-HuGene_st:363685- | 4.32E-11 | 2.0009 | 2.65 | 84.1 | 76-90.3 |
| | HuGene_st:814108-HuGene_st:125567- | | | | | |
| | HuGene_st:226479-HuGene_st:495486- | | | | | |
| | HuGene_st:692924- | | | | | |
| | HuGene_st:203963_at:65565- | | | | | |
| | HuGene_st:963408-HuGene_st:1017708- | | | | | |
| | HuGene_st:210735_s_at:1009602- | | | | | |
| | HuGene_st:875965- | | | | | |
| | HuGene_st:215867_x_at:749017- | | | | | |
| | HuGene_st:638145- | | | | | |
| | HuGene_st:204508_s_at:214164_x_at:104 9350-HuGene_st:189118- | | | | | |
| | | | | | | |
| | HuGene_st:698714-HuGene_st:1013062- | | | | | |
| | HuGene_st:280470-HuGene_st:439438- | | | | | |
| | HuGene_st:1049334- | | | | | |
| | HuGene_st:204509_at:233388_at | | | | | |
| DMD | 945600-HuGene_st:12596- | 5.01E-11 | 2.0152 | 4.74 | 84.3 | 76.3-90.3 |
| | HuGene_st:434657-HuGene_st:676962- | | | | | |
| | HuGene_st:1020969-HuGene_st:170680- | | | | | |
| | HuGene_st:855670-HuGene_st:909848- | | | | | |
| | HuGene_st:134644-HuGene_st:887814- | | | | | |
| | HuGene_st:649184-HuGene_st:110990- | | | | | |
| | HuGene_st:914217-HuGene_st:961622- | | | | | |
| | HuGene_st:514011-HuGene_st:842792- | | | | | |
| | HuGene_st:445483- | | | | | |
| | HuGene_st:208086_s_at:987362- | | | | | |
| | HuGene_st:997308-HuGene_st | | | | | |
| SMPDL3A | 734740-HuGene_st:406928- | 5.84E-11 | 1.941 | 3.14 | 83.4 | 75.2-89.7 |
| | HuGene_st:871227-HuGene_st:30922- | | | | | |
| | HuGene_st:771860-HuGene_st:376657- | | | | | |
| | HuGene_st:539013-HuGene_st:900092- | | | | | |
| | HuGene_st:253658-HuGene_st:696711- | | | | | |
| | HuGene_st:284449-HuGene_st:324159- | | | | | |
| | HuGene_st:70962-HuGene_st:161952- | | | | | |
| | HuGene st:873946-HuGene st:588668- | | | | | |
| | HuGene_st:681531-HuGene_st:195216- | | | | | |
| | HuGene_st:213624_at:471149- | | | | | |
| | HuGene_st:1562512_at | | | | | |
| HSPB6 | 438264-HuGene_st:771601- | 6.16E-11 | 1.9492 | 6.88 | 83.5 | 75.4-89.8 |
| | HuGene_st:17884-HuGene_st:587849- | | | | | |
| | HuGene_st:923983- | | | | | |
| | HuGene_st:214767_s_at:226304_at:76350 5-HuGene_st:835176-HuGene_st:868647- | | | | | |
| | | | | | | |
| | HuGene_st:511313-HuGene_st:848921- | | | | | |
| | HuGene_st | | | | | |
| AKR1B10 | 1017995-HuGene_st:244038- | 7.91E-11 | 1.9484 | 5.17 | 83.5 | 75.3-89.7 |
| | HuGene_st:943992-HuGene_st:690334- | | | | | |
| | HuGene_st:486646-HuGene_st:234015- | | | | | |
| | HuGene_st:599681- | | | | | |
| | HuGene_st:206561_s_at:347314- | | | | | |
| | HuGene_st:827689-HuGene_st:212880- | | | | | |
| | HuGene_st:516961-HuGene_st:638468- | | | | | |
| | HuGene_st | | | | | |
| UGDH | 961600-HuGene_st:1046173- | 8.72E-11 | 1.9628 | 2.4 | 83.7 | 75.5-89.9 |
| | HuGene_st:509128-HuGene_st:428595- | | | | | |
| | HuGene_st:363704-HuGene_st:519385- | | | | | |
| | HuGene_st:319601-HuGene_st:653273- | | | | | |
| | HuGene_st:121518-HuGene_st:786461- | | | | | |
| | HuGene_st:470924-HuGene_st:530032- | | | | | |
| | HuGene_st:810299-HuGene_st:954191- | | | | | |
| | HuGene_st:203343_at:855306- | | | | | |
| | HuGene_st:1075627-HuGene_st:184297- | | | | | |
| | HuGene_st:775350-HuGene_st:170400- | | | | | |
| | HuGene_st:374055-HuGene_st:186342- | | | | | |
| | HuGene_st | | | | | |
| FABP1 | 825235-HuGene_st:208491- | 9.02E-11 | 1.968 | 6.54 | 83.7 | 75.6-89.9 |
| | HuGene_st:626063-HuGene_st:625600- | | | | | |
| | HuGene_st:909598-HuGene_st:350768- | | | | | |
| | HuGene_st:760377-HuGene_st:52477- | | | | | |
| | HuGene_st:421055- | | | | | |
| | HuGene_st:205892_s_at:231693_at:69993 2-HuGene_st:561606-HuGene_st:791463- | | | | | |
| | | | | | | |
| | HuGene_st:495764-HuGene_st:703121- | | | | | |
| | HuGene_st:1025066-HuGene_st:392859- | | | | | |
| | HuGene_st:902525-HuGene_st:81171- | | | | | |
| | HuGene_st:1073289-HuGene_st:219434- | | | | | |
| | HuGene_st:735424-HuGene_st:297976- | | | | | |
| | HuGene_st | | | | | |
| P2RY14 | 528057-HuGene_st:780310- | 1.31E-10 | 1.9821 | 2 | 83.9 | 75.8-90 |
| | HuGene_st:235178-HuGene_st:352954- | | | | | |
| | HuGene_st:699489-HuGene_st:38001- | | | | | |
| | HuGene_st:637791-HuGene_st:25606- | | | | | |
| | HuGene_st:40647-HuGene_st:896487- | | | | | |
| | HuGene_st:672149-HuGene_st:863820- | | | | | |
| | HuGene_st:352427-HuGene_st:632821- | | | | | |
| | HuGene_st:116148-HuGene_st:561792- | | | | | |
| | HuGene_st:840910-HuGene_st:296420- | | | | | |
| | HuGene_st:974643-HuGene_st:206637_at | | | | | |
| TTRAP | 369720-HuGene_st:914515- | 1.59E-10 | 1.9196 | 2.2 | 83.1 | 74.9-89.4 |
| | HuGene_st:43073-HuGene_st:85920- | | | | | |
| | HuGene_st:278231-HuGene_st:345627- | | | | | |
| | HuGene_st:358211-HuGene_st:331406- | | | | | |
| | HuGene_st:748071-HuGene_st:840774- | | | | | |
| | HuGene_st:384150-HuGene_st:528293- | | | | | |
| | HuGene_st:202266_at:784674- | | | | | |
| | HuGene_st:370526-HuGene_st:1031392- | | | | | |
| | HuGene_st:258400-HuGene_st:1084398- | | | | | |
| | HuGene_st:223012-HuGene_st:1033688- | | | | | |
| | HuGene_st:232744-HuGene_st | | | | | |
| MEP1A | 854525-HuGene_st:233471- | 2.07E-10 | 1.9887 | 3.17 | 84 | 76-90.1 |
| | HuGene_st:521357-HuGene_st:398055- | | | | | |
| | HuGene_st:479853-HuGene_st:686103- | | | | | |
| | HuGene_st:711452-HuGene_st:984096- | | | | | |
| | HuGene_st:1083396-HuGene_st:1058253- | | | | | |
| | HuGene_st:951000-HuGene_st:984505- | | | | | |
| | HuGene_st:390982-HuGene_st:289221- | | | | | |
| | HuGene_st:1044210-HuGene_st:700879- | | | | | |
| | HuGene_st:368627-HuGene_st:1081805- | | | | | |
| | HuGene_st:206000_at:54151- | | | | | |
| | HuGene_st:558849-HuGene_st:200136- | | | | | |
| | HuGene_st:519266-HuGene_st | | | | | |
| MYH11 | 628303-HuGene_st:20510- | 2.15E-10 | 1.8602 | 5.35 | 82.4 | 74-88.8 |
| | HuGene_st:77517-HuGene_st:847369- | | | | | |
| | HuGene_st:396579-HuGene_st:751338- | | | | | |
| | HuGene_st:56832- | | | | | |
| | HuGene_st:227843_at:643005- | | | | | |
| | HuGene_st:410559-HuGene_st:618203- | | | | | |
| | HuGene_st:64921-HuGene_st:1083141- | | | | | |
| | HuGene_st:724450-HuGene_st:755245- | | | | | |
| | HuGene_st:947574- | | | | | |
| | HuGene_st:201496_x_at:482335- | | | | | |
| | HuGene_st:456670-HuGene_st:974777- | | | | | |
| | HuGene_st:86441-HuGene_st:1066402- | | | | | |
| | HuGene_st:289331-HuGene_st:888083- | | | | | |
| | HuGene_st:201495_x_at:201497_x_at:207 961_x_at:1568760_at:239307_at | | | | | |
| | | | | | | |
| MAOA | 234534_at:353863-HuGene_st:1038534- | 2.49E-10 | 2.0268 | 2.18 | 84.5 | 76.5-90.5 |
| | HuGene_st:732300- | | | | | |
| | HuGene_st:204389_at:204388_s_at:21167 0-HuGene_st:360481-HuGene_st:151427- | | | | | |
| | | | | | | |
| | HuGene_st:347487-HuGene_st:791116- | | | | | |
| | HuGene_st:212741_at:1003275- | | | | | |
| | HuGene_st:24543-HuGene_st:126920- | | | | | |
| | HuGene_st:500217-HuGene_st:434659- | | | | | |
| | HuGene_st:73861-HuGene_st:382884- | | | | | |
| | HuGene_st:675946-HuGene_st:108162- | | | | | |
| | HuGene_st:119160-HuGene_st | | | | | |
| CLDN8 | 1018006-HuGene_st:190634- | 2.92E-10 | 1.8632 | 12.96 | 82.4 | 74-88.9 |
| | HuGene_st:590280-HuGene_st:186468- | | | | | |
| | HuGene_st:954438-HuGene_st:428391- | | | | | |
| | HuGene_st:480543-HuGene_st:944337- | | | | | |
| | HuGene_st:179725-HuGene_st:508584- | | | | | |
| | HuGene_st:1009114-HuGene_st:948216- | | | | | |
| | HuGene_st:658285-HuGene_st:1022600- | | | | | |
| | HuGene_st:737498-HuGene_st:470015- | | | | | |
| | HuGene_st:103315-HuGene_st:699348- | | | | | |
| | HuGene_st:89877-HuGene_st:56937- | | | | | |
| | HuGene_st:862663-HuGene_st:504945- | | | | | |
| | HuGene_st:214598_at | | | | | |
| FUCA1 | 295335-HuGene_st:550769- | 4.24E-10 | 1.8845 | 2.03 | 82.7 | 74.4-89.1 |
| | HuGene_st:674252-HuGene_st:343732- | | | | | |
| | HuGene_st:10170-HuGene_st:1012064- | | | | | |
| | HuGene_st:296930-HuGene_st:179394- | | | | | |
| | HuGene_st:587765- | | | | | |
| | HuGene_st:229137_at:142430- | | | | | |
| | HuGene_st:801038-HuGene_st:447607- | | | | | |
| | HuGene_st:66284-HuGene_st:937530- | | | | | |
| | HuGene_st:884152-HuGene_st:674608- | | | | | |
| | HuGene_st:202838_at:106313- | | | | | |
| | HuGene_st:602308-HuGene_st:887004- | | | | | |
| | HuGene_st:206251-HuGene_st:357516- | | | | | |
| | HuGene_st | | | | | |
| FXYD6 | 138547-HuGene_st:814211- | 6.39E-10 | 1.8087 | 2.92 | 81.7 | 73.3-88.3 |
| | HuGene_st:185591-HuGene_st:992039- | | | | | |
| | HuGene_st:549291-HuGene_st:921510- | | | | | |
| | HuGene_st:163300-HuGene_st:4792- | | | | | |
| | HuGene_st:369594-HuGene_st:209348- | | | | | |
| | HuGene_st:1028937-HuGene_st:77232- | | | | | |
| | HuGene_st:529384- | | | | | |
| | HuGene_st:217897_at:877189- | | | | | |
| | HuGene_st:973770-HuGene_st:1054980- | | | | | |
| | HuGene_st:240323_at:466048-HuGene_st | | | | | |
| ARL14 | 220468_at:471121-HuGene_st:690873- | 7.01E-10 | 1.8255 | 3.06 | 81.9 | 73.5-88.4 |
| | HuGene_st:468858-HuGene_st:750196- | | | | | |
| | HuGene_st:62407-HuGene_st:159605- | | | | | |
| | HuGene_st:979866-HuGene_st:1032832- | | | | | |
| | HuGene_st:446532-HuGene_st:861783- | | | | | |
| | HuGene_st:116203-HuGene_st:991610- | | | | | |
| | HuGene_st:515168-HuGene_st:1073679- | | | | | |
| | HuGene_st:439428-HuGene_st:976681- | | | | | |
| | HuGene_st:452044-HuGene_st:197209- | | | | | |
| | HuGene_st:12441-HuGene_st | | | | | |
| SLC20A1 | 335219-HuGene_st:230038- | 7.65E-10 | 1.7854 | 4.04 | 81.4 | 72.9-88.1 |
| | HuGene_st:1078924-HuGene_st:520074- | | | | | |
| | HuGene_st:769870-HuGene_st:175687- | | | | | |
| | HuGene_st:541149-HuGene_st:973433- | | | | | |
| | HuGene_st:643658-HuGene_st:220784- | | | | | |
| | HuGene_st:175599-HuGene_st:482373- | | | | | |
| | HuGene_st:1003908-HuGene_st:337454- | | | | | |
| | HuGene_st:655800-HuGene_st:321368- | | | | | |
| | HuGene_st:230494_at:178021- | | | | | |
| | HuGene_st:201920_at:448729- | | | | | |
| | HuGene_st:896684-HuGene_st:363789- | | | | | |
| | HuGene_st:130920-HuGene_st:786196- | | | | | |
| | HuGene_st | | | | | |
| MAB21L2 | 338297-HuGene_st:994188- | 1.05E-09 | 1.7739 | 5.2 | 81.2 | 72.7-87.9 |
| | HuGene_st:109548-HuGene_st:395419- | | | | | |
| | HuGene_st:922092-HuGene_st:737843- | | | | | |
| | HuGene_st:6731-HuGene_st:708698- | | | | | |
| | HuGene_st:352943-HuGene_st:485256- | | | | | |
| | HuGene_st:1023066-HuGene_st:825747- | | | | | |
| | HuGene_st:210303_at:309088- | | | | | |
| | HuGene_st:1031483-HuGene_st:441727- | | | | | |
| | HuGene_st:751700-HuGene_st:974270- | | | | | |
| | HuGene_st:210302_s_at:254681- | | | | | |
| | HuGene_st:982880-HuGene_st | | | | | |
| TSPAN1 | 833585-HuGene_st:209114_at:664359- | 1.16E-09 | 1.7875 | 2.16 | 81.4 | 73-88.1 |
| | HuGene_st:307816-HuGene_st:111939- | | | | | |
| | HuGene_st:39185-HuGene_st:496300- | | | | | |
| | HuGene_st:1038310-HuGene_st:401084- | | | | | |
| | HuGene_st:632040-HuGene_st:1076526- | | | | | |
| | HuGene_st:488552-HuGene_st:35199- | | | | | |
| | HuGene_st:611793-HuGene_st:1007567- | | | | | |
| | HuGene_st:305523-HuGene_st:1033895- | | | | | |
| | HuGene_st:145680-HuGene_st:812712- | | | | | |
| | HuGene_st:929225-HuGene_st:641156- | | | | | |
| | HuGene_st:116814-HuGene_st | | | | | |
| KLF4 | 310345-HuGene_st:421025- | 1.19E-09 | 1.8535 | 2.27 | 82.3 | 74-88.8 |
| | HuGene_st:853068-HuGene_st:1040228- | | | | | |
| | HuGene_st:1040166-HuGene_st:549197- | | | | | |
| | HuGene_st:257340-HuGene_st:95161- | | | | | |
| | HuGene_st:166058-HuGene_st:749339- | | | | | |
| | HuGene_st:212619- | | | | | |
| | HuGene_st:220266_s_at:1078952- | | | | | |
| | HuGene_st:289946-HuGene_st:824845- | | | | | |
| | HuGene_st:965293-HuGene_st:467978- | | | | | |
| | HuGene_st:287320-HuGene_st:334405- | | | | | |
| | HuGene_st:234619- | | | | | |
| | HuGene_st:221841_s_at:145954- | | | | | |
| | HuGene_st:733888-HuGene_st | | | | | |
| CRYAB | 581894-HuGene_st:42986- | 1.47E-09 | 1.768 | 3.91 | 81.2 | 72.7-87.9 |
| | HuGene_st:626903-HuGene_st:612490- | | | | | |
| | HuGene_st:1017597-HuGene_st:473983- | | | | | |
| | HuGene_st:380047-HuGene_st:25982- | | | | | |
| | HuGene_st:298978-HuGene_st:485492- | | | | | |
| | HuGene_st:413012-HuGene_st:944836- | | | | | |
| | HuGene_st:209283_at:615857- | | | | | |
| | HuGene_st:303036-HuGene_st:592729- | | | | | |
| | HuGene_st:349645-HuGene_st:226566- | | | | | |
| | HuGene_st:215779-HuGene_st:702453- | | | | | |
| | HuGene_st:95542-HuGene_st:697816- | | | | | |
| | HuGene_st | | | | | |
| C15orf48 | 661133-HuGene_st:240581- | 1.54E-09 | 1.8166 | 3.07 | 81.8 | 73.5-88.4 |
| | HuGene_st:302164-HuGene_st:262469- | | | | | |
| | HuGene_st:512593-HuGene_st:122711- | | | | | |
| | HuGene_st:269066-HuGene_st:246950- | | | | | |
| | HuGene_st:483493-HuGene_st:352750- | | | | | |
| | HuGene_st:645087-HuGene_st:775549- | | | | | |
| | HuGene_st:223484_at:521885- | | | | | |
| | HuGene_st:1004430-HuGene_st:885617- | | | | | |
| | HuGene_st:166185-HuGene_st:752633- | | | | | |
| | HuGene_st:1056973-HuGene_st:60479- | | | | | |
| | HuGene_st | | | | | |
| ACAT1 | 205412_at:877718-HuGene_st:697995- | 1.70E-09 | 1.8589 | 1.77 | 82.4 | 74-88.8 |
| | HuGene_st:244911-HuGene_st:705306- | | | | | |
| | HuGene_st:110401-HuGene_st:31594- | | | | | |
| | HuGene_st:19333-HuGene_st:499096- | | | | | |
| | HuGene_st:942019-HuGene_st:618952- | | | | | |
| | HuGene_st:933332-HuGene_st:715946- | | | | | |
| | HuGene_st:676239-HuGene_st:172044- | | | | | |
| | HuGene_st:828361-HuGene_st:321829- | | | | | |
| | HuGene_st:897946-HuGene_st:331003- | | | | | |
| | HuGene_st:1559239_s_at:814756- | | | | | |
| | HuGene_st | | | | | |
| ANPEP | 42832-HuGene_st:567567- | 2.04E-09 | 1.7687 | 8.18 | 81.2 | 72.7-87.9 |
| | HuGene_st:992744-HuGene_st:524001- | | | | | |
| | HuGene_st:229640-HuGene_st:372330- | | | | | |
| | HuGene_st:842255-HuGene_st:42234- | | | | | |
| | HuGene_st:640878-HuGene_st:702098- | | | | | |
| | HuGene_st:917110-HuGene_st:25968- | | | | | |
| | HuGene_st:850385-HuGene_st:503143- | | | | | |
| | HuGene_st:202888_s_at:429651- | | | | | |
| | HuGene_st:409574-HuGene_st:60717- | | | | | |
| | HuGene_st:998436-HuGene_st:618326- | | | | | |
| | HuGene_st:1067290-HuGene_st:333846- | | | | | |
| | HuGene_st | | | | | |
| FGL2 | 884418-HuGene_st:249194- | 2.64E-09 | 1.8254 | 2.07 | 81.9 | 73.5-88.5 |
| | HuGene_st:463016-HuGene_st:194197- | | | | | |
| | HuGene_st:789123-HuGene_st:998752- | | | | | |
| | HuGene_st:596140-HuGene_st:896312- | | | | | |
| | HuGene_st:762195-HuGene_st:909927- | | | | | |
| | | | | | | |
| | | | | | | |
| | HuGene_st:206261-HuGene_st:706108- | | | | | |
| | HuGene_st:202575-HuGene_st:148017- | | | | | |
| | HuGene_st:199906-HuGene_st:707754- | | | | | |
| | HuGene_st:204834_at | | | | | |
| PPID | 204185_x_at:204186_s_at | 3.34E-09 | 1.8025 | 1.73 | 81.6 | 73.2-88.2 |
| ITM2C | 83688-HuGene_st:398134- | 3.87E-09 | 1.7063 | 2.22 | 80.3 | 71.7-87.2 |
| | HuGene_st:972817-HuGene_st:698719- | | | | | |
| | HuGene_st:369206-HuGene_st:377066- | | | | | |
| | HuGene_st:23436-HuGene_st:593996- | | | | | |
| | HuGene_st:987556- | | | | | |
| | HuGene_st:221004_s_at:465490- | | | | | |
| | HuGene_st:776424-HuGene_st:1052550- | | | | | |
| | HuGene_st:344231-HuGene_st:1039973- | | | | | |
| | HuGene_st:127312-HuGene_st:577346- | | | | | |
| | HuGene_st:394326-HuGene_st:848902- | | | | | |
| | HuGene_st | | | | | |
| SST | 477599-HuGene_st:1101191- | 4.16E-09 | 1.7627 | 6.14 | 81.1 | 72.6-87.8 |
| | HuGene_st:474819-HuGene_st:144116- | | | | | |
| | HuGene_st:76078-HuGene_st:698582- | | | | | |
| | HuGene_st:600708-HuGene_st:98570- | | | | | |
| | HuGene_st:4754-HuGene_st:675107- | | | | | |
| | HuGene_st:213921_at:115911- | | | | | |
| | HuGene_st:235647-HuGene_st:131210- | | | | | |
| | HuGene_st:502176-HuGene_st:63243- | | | | | |
| | HuGene_st | | | | | |
| PRIMA1 | 1084922-HuGene_st:1008125- | 4.74E-09 | 1.7053 | 2.75 | 80.3 | 71.7-87.1 |
| | HuGene_st:22062-HuGene_st:182482- | | | | | |
| | HuGene_st:225291-HuGene_st:566243- | | | | | |
| | HuGene_st:50975- | | | | | |
| | HuGene_st:230087_at:787213- | | | | | |
| | HuGene_st:661160-HuGene_st:256004- | | | | | |
| | HuGene_st:6276-HuGene_st:44738- | | | | | |
| | HuGene_st | | | | | |
| EFEMP1 | 201842_s_at:1073952- | 5.80E-09 | 1.8208 | 1.69 | 81.9 | 73.4-88.4 |
| | HuGene_st:228421_s_at:520435- | | | | | |
| | HuGene_st:551581-HuGene_st:909695- | | | | | |
| | HuGene_st:539520-HuGene_st:719105- | | | | | |
| | HuGene_st:1046788-HuGene_st:728978- | | | | | |
| | HuGene_st:956186-HuGene_st:89258- | | | | | |
| | HuGene_st:155486-HuGene_st:986491- | | | | | |
| | HuGene_st:207603-HuGene_st:243191- | | | | | |
| | HuGene_st:842290-HuGene_st:757697- | | | | | |
| | HuGene_st:784694-HuGene_st:132382- | | | | | |
| | HuGene_st | | | | | |
| ADAMTS1 | 432578-HuGene_st:139364- | 6.42E-09 | 1.7467 | 2.37 | 80.9 | 72.4-87.6 |
| | HuGene_st:423315-HuGene_st:578666- | | | | | |
| | HuGene_st:419311-HuGene_st:928538- | | | | | |
| | HuGene_st:344696-HuGene_st:617885- | | | | | |
| | HuGene_st:136304-HuGene_st:75724- | | | | | |
| | HuGene_st:412640-HuGene_st:368117- | | | | | |
| | HuGene_st:97806-HuGene_st:641285- | | | | | |
| | HuGene_st:222486_s_at:711563- | | | | | |
| | HuGene_st:1006281- | | | | | |
| | HuGene_st:222162_s_at:32418- | | | | | |
| | HuGene_st | | | | | |
| AKAP12 | 212419-HuGene_st:231067_s_at:379659- | 7.41E-09 | 1.744 | 2.83 | 80.8 | 72.2-87.5 |
| | HuGene_st;1010338-HuGene_st:1075094- | | | | | |
| | HuGene_st:42401-HuGene_st:522584- | | | | | |
| | HuGene_st:480972-HuGene_st:948623- | | | | | |
| | HuGene_st:701945-HuGene_st:276784- | | | | | |
| | HuGene_st:64858- | | | | | |
| | HuGene_st:210517_s_at:874382- | | | | | |
| | HuGene_st:909976-HuGene_st:182037- | | | | | |
| | HuGene_st:417182-HuGene_st:722881- | | | | | |
| | HuGene_st | | | | | |
| COL14A1 | 216866_s_at:309808-HuGene_st:532905- | 7.60E-09 | 1.7817 | 1.67 | 81.3 | 72.9-88 |
| | HuGene_st:938881-HuGene_st:184937- | | | | | |
| | HuGene_st:332362-HuGene_st:431855- | | | | | |
| | HuGene_st:295715-HuGene_st:813208- | | | | | |
| | HuGene_st:216865_at:426456- | | | | | |
| | HuGene_st:212865_s_at:513900- | | | | | |
| | HuGene_st:1054393-HuGene_st:947013- | | | | | |
| | HuGene_st:966609-HuGene_st:929330- | | | | | |
| | HuGene_st:283165-HuGene_st:67940- | | | | | |
| | HuGene_st:655615-HuGene_st:236228- | | | | | |
| | HuGene_st | | | | | |
| UGT2A3 | 149647-HuGene_st:860083- | 8.64E-09 | 1.7414 | 4.89 | 80.8 | 72.2-87.5 |
| | HuGene_st:922544-HuGene_st:244206- | | | | | |
| | HuGene_st:503323-HuGene_st:353576- | | | | | |
| | HuGene_st:603619-HuGene_st:787458- | | | | | |
| | HuGene_st:219796-HuGene_st:333564- | | | | | |
| | HuGene_st:257402-HuGene_st:366699- | | | | | |
| | HuGene_st:461685-HuGene_st:891681- | | | | | |
| | HuGene_st:644952-HuGene_st:621618- | | | | | |
| | HuGene_st:737617-HuGene_st:88682- | | | | | |
| | HuGene_st:529761-HuGene_st:895203- | | | | | |
| | HuGene_st:658594-HuGene_st:455115- | | | | | |
| | HuGene_st | | | | | |
| PTGIS | 162553-HuGene_st:277727- | 9.15E-09 | 1.7213 | 3.98 | 80.5 | 72-87.3 |
| | HuGene_st:426160-HuGene_st:49754- | | | | | |
| | HuGene_st:491989-HuGene_st:846155- | | | | | |
| | HuGene_st:214744-HuGene_st:81045- | | | | | |
| | HuGene_st:757700-HuGene_st:713439- | | | | | |
| | HuGene_st:765856- | | | | | |
| | HuGene_st:211892_s_at:424645- | | | | | |
| | HuGene_st:805861-HuGene_st:16678- | | | | | |
| | HuGene_st:49009- | | | | | |
| | HuGene_st:210702_s_at:33677- | | | | | |
| | HuGene_st:757535-HuGene_st:1065183- | | | | | |
| | HuGene_st:949066-HuGene_st | | | | | |
| BEST2 | 242542-HuGene_st:368041- | 9.19E-09 | 1.7181 | 3.63 | 80.5 | 71.9-87.3 |
| | HuGene_st:316182-HuGene_st:417689- | | | | | |
| | HuGene_st:953359-HuGene_st:196857- | | | | | |
| | HuGene_st:2545- | | | | | |
| | HuGene_st:207432_at:371039- | | | | | |
| | HuGene_st:105864-HuGene_st:946835- | | | | | |
| | HuGene_st:371107-HuGene_st:170027- | | | | | |
| | HuGene_st:238631-HuGene_st:515622- | | | | | |
| | HuGene_st:886418-HuGene_st:396311- | | | | | |
| | HuGene_st:735639-HuGene_st:947978- | | | | | |
| | HuGene_st:882286-HuGene_st:894968- | | | | | |
| | HuGene_st | | | | | |
| MS4A4A | 651445-HuGene_st:308636- | 9.59E-09 | 1.8179 | 1.55 | 81.8 | 73.4-88.4 |
| | HuGene_st:174209-HuGene_st:486453- | | | | | |
| | HuGene_st:219607_s_at:1041327- | | | | | |
| | HuGene_st:84116-HuGene_st:161192- | | | | | |
| | HuGene_st:491070- | | | | | |
| | HuGene_st:1555728_a_at:142937- | | | | | |
| | HuGene_st:581150-HuGene_st:579786- | | | | | |
| | HuGene_st:588041- | | | | | |
| | HuGene_st:224357_s_at:886807- | | | | | |
| | HuGene_st:122093-HuGene_st | | | | | |
| SGCE | 976641-HuGene_st:204688_at:241763- | 1.02E-08 | 1.7444 | 2.03 | 80.8 | 72.3-87.6 |
| | HuGene_st:83484-HuGene_st:281261- | | | | | |
| | HuGene_st:324304-HuGene_st:683152- | | | | | |
| | H uGene_st:850448-HuGene_st:254097- | | | | | |
| | HuGene_st:992961-HuGene_st:69099- | | | | | |
| | HuGene_st:781384-HuGene_st:899200- | | | | | |
| | HuGene_st:974761-HuGene_st:417302- | | | | | |
| | HuGene_st:211350-HuGene_st:198065-v | | | | | |
| | HuGene_st | | | | | |
| TPSB2 | 207134_x_at:207741_x_at | 1.24E-08 | 1.3708 | 1.87 | 75.3 | 66.3-83 |
| COL6A2 | 919359-HuGene_st:40510- | 1.30E-08 | 1.6771 | 2.08 | 79.9 | 71.2-86.8 |
| | HuGene_st:375216-HuGene_st:1086501- | | | | | |
| | HuGene_st:160736-HuGene_st:602554- | | | | | |
| | HuGene_st:148827-HuGene_st:794188- | | | | | |
| | HuGene_st:213290_at:536399- | | | | | |
| | HuGene_st:501950-HuGene_st:404866- | | | | | |
| | HuGene_st:209156_s_at:813295- | | | | | |
| | HuGene_st | | | | | |
| PRKACB | 202741_at:795011-HuGene_st:360410- | 1.30E-08 | 1.7234 | 2.18 | 80.6 | 72-87.3 |
| | HuGene_st:970814-HuGene_st:70528- | | | | | |
| | HuGene_st:1042799- | | | | | |
| | HuGene_st:202742_s_at:964845- | | | | | |
| | HuGene_st:563484-HuGene_st:142353- | | | | | |
| | HuGene_st:597859-HuGene_st:660092- | | | | | |
| | HuGene_st:948901-HuGene_st:301751- | | | | | |
| | HuGene_st:294241-HuGene_st:694446- | | | | | |
| | HuGene_st:309819-HuGene_st:668357- | | | | | |
| | HuGene_st:589422-HuGene_st | | | | | |
| SPARCL1 | 246509-HuGene_st:300147- | 1.33E-08 | 1.634 | 2.85 | 79.3 | 70.5-86.3 |
| | HuGene_st:525246-HuGene_st:380181- | | | | | |
| | HuGene_st:257526-HuGene_st:585652- | | | | | |
| | HuGene_st:733855-HuGene_st:517768- | | | | | |
| | HuGene_st:853991-HuGene_st:854537- | | | | | |
| | HuGene_st:1023002-HuGene_st:55936- | | | | | |
| | HuGene_st:371227-HuGene_st:782475- | | | | | |
| | HuGene_st:670571-HuGene_st:1026998- | | | | | |
| | HuGene_st:894310-HuGene_st:948826- | | | | | |
| | HuGene_st:302303-HuGene_st:151789- | | | | | |
| | HuGene_st:905519- | | | | | |
| | HuGene_st:200795_at:602848-HuGene_st | | | | | |
| PBLD | 1003993-HuGene_st:308979- | 1.38E-08 | 1.6983 | 2.74 | 80.2 | 71.5-87 |
| | HuGene_st:622498-HuGene_st:840594- | | | | | |
| | HuGene_st:84006-HuGene_st:636001- | | | | | |
| | HuGene_st:529256-HuGene_st:651369- | | | | | |
| | HuGene_st:379972-HuGene_st:492235- | | | | | |
| | HuGene_st:584905-HuGene_st:222771- | | | | | |
| | HuGene_st:383791-HuGene_st:1016249- | | | | | |
| | HuGene_st:819013- | | | | | |
| | HuGene_st:1555175_a_at:115400- | | | | | |
| | HuGene_st:969544-HuGene_st:949581- | | | | | |
| | HuGene_st:897360- | | | | | |
| | HuGene_st:219543_at:548506-HuGene_st | | | | | |
| PDK4 | 806272-HuGene_st:44901- | 1.45E-08 | 1.7059 | 3.02 | 80.3 | 71.8-87.1 |
| | HuGene_st:272445-HuGene_st:650209- | | | | | |
| | HuGene_st:796256-HuGene_st:342230- | | | | | |
| | HuGene_st:648024- | | | | | |
| | HuGene_st:205960_at:25700- | | | | | |
| | HuGene_st:152414-HuGene_st:360382- | | | | | |
| | HuGene_st:471160-HuGene_st:219862- | | | | | |
| | HuGene_st:584279- | | | | | |
| | HuGene_st:225207_at:498670- | | | | | |
| | HuGene_st:405050-HuGene_st:546964- | | | | | |
| | HuGene_st:735039- | | | | | |
| | HuGene_st:1562321_at:348010- | | | | | |
| | HuGene_st:1073661-HuGene_st:578954- | | | | | |
| | HuGene_st:966364-HuGene_st:115766- | | | | | |
| | HuGene_st | | | | | |
| DES | 680953-HuGene_st:710186- | 1.47E-08 | 1.7257 | 6.66 | 80.6 | 72-87.4 |
| | HuGene_st:224168-HuGene_st:720121- | | | | | |
| | HuGene_st:632911-HuGene_st:14146- | | | | | |
| | HuGene_st:472740-HuGene_st:452392- | | | | | |
| | HuGene_st:70983-HuGene_st:626407- | | | | | |
| | HuGene_st:330060-HuGene_st:678707- | | | | | |
| | HuGene_st:202222_s_at:830447- | | | | | |
| | HuGene_st:466888-HuGene_st:832644- | | | | | |
| | HuGene_st:190691-HuGene_st:1043063- | | | | | |
| | HuGene_st:476251-HuGene_st:856127- | | | | | |
| | HuGene_st:680072-HuGene_st:1053701- | | | | | |
| | HuGene_st:490364-HuGene_st:954860- | | | | | |
| | HuGene_st:7755-HuGene_st:856708- | | | | | |
| | HuGene_st:272796-HuGene_st:377421- | | | | | |
| | HuGene_st:214027_x_at:458352- | | | | | |
| | HuGene_st:104906-HuGene_st:276614- | | | | | |
| | HuGene_st | | | | | |
| MMP28 | 578497-HuGene_st:31358- | 1.80E-08 | 1.663 | 2.47 | 79.7 | 71.1-86.7 |
| | HuGene_st:283053-HuGene_st:152786- | | | | | |
| | HuGene_st:519390- | | | | | |
| | HuGene_st:239273_s_at:599795- | | | | | |
| | HuGene_st:800954- | | | | | |
| | HuGene_st:219909_at:190189- | | | | | |
| | HuGene_st:222937_s_at:1084226- | | | | | |
| | HuGene_st:315451-HuGene_st:100436- | | | | | |
| | HuGene_st:794275-HuGene_st:256212- | | | | | |
| | HuGene_st:8583-HuGene_st:987411- | | | | | |
| | HuGene_st:239272_at:224207_x_at:21241 | | | | | |
| | 8-HuGene_st:319017-HuGene_st:604727- | | | | | |
| | HuGene_st | | | | | |
| CYBRD1 | 580256-HuGene_st:931026- | 1.84E-08 | 1.6939 | 2.7 | 80.1 | 71.6-87 |
| | HuGene_st:412808-HuGene_st:138769- | | | | | |
| | HuGene_st:199471-HuGene_st:675528- | | | | | |
| | HuGene_st:217889_s_at:570877- | | | | | |
| | HuGene_st:438204-HuGene_st:836676- | | | | | |
| | HuGene_st:251906-HuGene_st:71842- | | | | | |
| | HuGene_st:140118-HuGene_st:266609- | | | | | |
| | HuGene_st:132102-HuGene_st:774519- | | | | | |
| | HuGene_st:1068423-HuGene_st:995512- | | | | | |
| | HuGene_st:298248-HuGene_st:1099283- | | | | | |
| | HuGene_st:222189- | | | | | |
| | HuGene_st:222453_at:232459_at | | | | | |
| IL6ST | 1054392-HuGene_st:234474_x_at:953995- | 1.87E-08 | 1.7642 | 1.58 | 81.1 | 72.6-87.8 |
| | HuGene_st:234967_at:48974- | | | | | |
| | HuGene_st:325364-HuGene_st:32344- | | | | | |
| | HuGene_st:821422-HuGene_st:298043- | | | | | |
| | HuGene_st:606460-HuGene_st:1074930- | | | | | |
| | HuGene_st:44790- | | | | | |
| | HuGene_st:204864_s_at:1059165- | | | | | |
| | HuGene_st:288593-HuGene_st:39514- | | | | | |
| | HuGene_st:204863_s_at:175414- | | | | | |
| | HuGene_st:293958- | | | | | |
| | HuGene_st:212196_at:148922- | | | | | |
| | HuGene_st:211000_s_at:648367- | | | | | |
| | HuGene_st:240067- | | | | | |
| | HuGene_st:212195_at:822552- | | | | | |
| | HuGene_st:523608-HuGene_st:474929- | | | | | |
| | HuGene_st | | | | | |
| FABP4 | 547124-HuGene_st:96292- | 2.01E-08 | 1.6776 | 5.55 | 79.9 | 71.2-86.8 |
| | HuGene_st:545014-HuGene_st:22370- | | | | | |
| | HuGene_st:138079-HuGene_st:1064936- | | | | | |
| | HuGene_st:804933-HuGene_st:1087992- | | | | | |
| | HuGene_st:20784-HuGene_st:671128- | | | | | |
| | HuGene_st:328798-HuGene_st:594739- | | | | | |
| | HuGene_st:1047009-HuGene_st:825613- | | | | | |
| | HuGene_st:637074-HuGene_st:759940- | | | | | |
| | HuGene_st:754024-HuGene_st:88372- | | | | | |
| | HuGene_st | | | | | |
| CALM1 | 196751-HuGene_st:1094382- | 2.12E-08 | 1.6819 | 1.72 | 80 | 71.3-86.9 |
| | HuGene_st:211985_s_at:445194- | | | | | |
| | HuGene_st:211984_at:213688_at:213710_ s_at:444099-HuGene_st:708994- | | | | | |
| | | | | | | |
| | HuGene_st:418220- | | | | | |
| | HuGene_st:241614_at:96889- | | | | | |
| | HuGene_st:50211- | | | | | |
| | HuGene_st:200653_s_at:767359- | | | | | |
| | HuGene_st:200655_s_at:616208- | | | | | |
| | HuGene_st:1019255- | | | | | |
| | HuGene_st:209563_x_at:715468- | | | | | |
| | HuGene_st:18183-HuGene_st:544779- | | | | | |
| | HuGene_st:239705-HuGene_st:1084911- | | | | | |
| | HuGene_st:98189-HuGene_st | | | | | |
| TNS1 | 766751-HuGene_st:503077- | 2.21E-08 | 1.7412 | 2.01 | 80.8 | 72.3-87.5 |
| | HuGene_st:455229-HuGene_st:905238- | | | | | |
| | HuGene_st:129966-HuGene_st:750627- | | | | | |
| | HuGene_st:989199-HuGene_st:61598- | | | | | |
| | HuGene_st:671515-HuGene_st:129638- | | | | | |
| | HuGene_st:1088728-HuGene_st:693249- | | | | | |
| | HuGene_st:418601-HuGene_st:168879- | | | | | |
| | HuGene_st:220476-HuGene_st:23579- | | | | | |
| | HuGene_st:731710-HuGene_st:476750- | | | | | |
| | HuGene_st:295235- | | | | | |
| | HuGene_st:218863_s_at:665955- | | | | | |
| | HuGene_st:221747_at:105442- | | | | | |
| | HuGene_st:676236-HuGene_st:124848- | | | | | |
| | HuGene_st:474353- | | | | | |
| | HuGene_st:218864_at:925862- | | | | | |
| | HuGene_st:279934-HuGene_st:1008295- | | | | | |
| | HuGene_st:221246_x_at:384388- | | | | | |
| | HuGene_st:522313-HuGene_st:754212- | | | | | |
| | HuGene_st:221748_s_at:420410- | | | | | |
| | HuGene_st:250619-HuGene_st | | | | | |
| EMP1 | 201324_at:311769- | 2.59E-08 | 1.7156 | 1.95 | 80.4 | 71.9-87.3 |
| | HuGene_st:201325_s_at:506469- | | | | | |
| | HuGene_st:861706-HuGene_st:46080- | | | | | |
| | HuGene_st:236046-HuGene_st:548197- | | | | | |
| | HuGene_st:100442-HuGene_st:1096474- | | | | | |
| | HuGene_st:717809-HuGene_st:596930- | | | | | |
| | HuGene_st:468199-HuGene_st:751888- | | | | | |
| | HuGene_st:166375- | | | | | |
| | HuGene_st:213895_at:871061- | | | | | |
| | HuGene_st:164793-HuGene_st:509462- | | | | | |
| | HuGene_st:182838- | | | | | |
| | HuGene_st:1564796_at:446286- | | | | | |
| | HuGene_st | | | | | |
| RDX | 350620-HuGene_st:1005467- | 3.06E-08 | 1.7426 | 1.9 | 80.8 | 72.2-87.6 |
| | HuGene_st:284799-HuGene_st:78392- | | | | | |
| | HuGene_st:923159- | | | | | |
| | HuGene_st:204969_s_at:935366- | | | | | |
| | HuGene_st:603982-HuGene_st:418137- | | | | | |
| | HuGene_st:304555-HuGene_st:174237- | | | | | |
| | HuGene_st:140908-HuGene_st:1034349- | | | | | |
| | HuGene_st:212398_at:977130- | | | | | |
| | HuGene_st:6485-HuGene_st | | | | | |
| CFD | 396618-HuGene_st:672277- | 3.06E-08 | 1.6465 | 2.81 | 79.5 | 70.8-86.4 |
| | HuGene_st:290815-HuGene_st:304988- | | | | | |
| | HuGene_st:580394-HuGene_st:605542- | | | | | |
| | HuGene_st:698498-HuGene_st:580177- | | | | | |
| | HuGene_st:633095-HuGene_st:35339- | | | | | |
| | HuGene_st:530155-HuGene_st:584306- | | | | | |
| | HuGene_st:205382_s_at:915940- | | | | | |
| | HuGene_st:859510-HuGene_st:978939- | | | | | |
| | HuGene_st:59414-HuGene_st:557142- | | | | | |
| | HuGene_st:104453-HuGene_st | | | | | |
| GPNMB | 201141_at:25823-HuGene_st:926046- | 3.11E-08 | 1.6924 | 1.99 | 80.1 | 71.5-87 |
| | HuGene_st:724736-HuGene_st:591503- | | | | | |
| | HuGene_st:660677-HuGene_st:701912- | | | | | |
| | HuGene_st:267933-HuGene_st:799615- | | | | | |
| | HuGene_st:569803-HuGene_st:979125- | | | | | |
| | HuGene_st:38037-HuGene_st:89805- | | | | | |
| | HuGene_st:335128-HuGene_st:131027- | | | | | |
| | HuGene_st:908272-HuGene_st:950837- | | | | | |
| | HuGene_st:39441-HuGene_st:1056618- | | | | | |
| | HuGene_st:860466-HuGene_st | | | | | |
| TPSAB1 | 350712-HuGene_st:134899- | 3.52E-08 | 1.7066 | 1.93 | 80.3 | 71.7-87.2 |
| | HuGene_st:122333-HuGene_st:1098415- | | | | | |
| | HuGene_st:665622-HuGene_st:701849- | | | | | |
| | HuGene_st:535697-HuGene_st:163189- | | | | | |
| | HuGene_st:744603-HuGene_st:153098- | | | | | |
| | HuGene_st:216485_s_at:216474_x_at:205 | | | | | |
| | 683_x_at:416078-HuGene_st:162760- | | | | | |
| | HuGene_st:217023_x_at:520284- | | | | | |
| | HuGene_st:215382_x_at:210084_x_at:567 | | | | | |
| | 472-HuGene_st:519234- | | | | | |
| | HuGene_st:154829-HuGene_st:247406- | | | | | |
| | HuGene_st:331572-HuGene_st:264147- | | | | | |
| | HuGene_st:462114-HuGene_st:880961- | | | | | |
| | HuGene_st:705156-HuGene_st | | | | | |
| PPP1R14A | 374630-HuGene_st:551938- | 3.54E-08 | 1.6167 | 2.51 | 79.1 | 70.3-86.1 |
| | HuGene_st:783588-HuGene_st:44913- | | | | | |
| | HuGene_st:966135- | | | | | |
| | HuGene_st:227006_at:537429- | | | | | |
| | HuGene_st:815370-HuGene_st:1040980- | | | | | |
| | HuGene_st:76662-HuGene_st | | | | | |
| LOC25301 | 873053-HuGene_st:945097- | 3.59E-08 | 1.6527 | 4.46 | 79.6 | 70.9-86.5 |
| 2 | HuGene_st:896826-HuGene_st:363568- | | | | | |
| | HuGene_st:162938-HuGene_st:441915- | | | | | |
| | HuGene_st:1070854-HuGene_st:463072- | | | | | |
| | HuGene_st:199784-HuGene_st:304834- | | | | | |
| | HuGene_st:381523-HuGene_st:568837- | | | | | |
| | HuGene_st:39629-HuGene_st:1026726- | | | | | |
| | HuGene_st:1014415-HuGene_st:51876- | | | | | |
| | HuGene_st:749900-HuGene_st:804632- | | | | | |
| | HuGene_st | | | | | |
| DMN | 207872-HuGene_st:407332- | 4.36E-08 | 1.6041 | 4.05 | 78.9 | 70-86 |
| | HuGene_st:828944-HuGene_st:469719- | | | | | |
| | HuGene_st:166409-HuGene_st:430636- | | | | | |
| | HuGene_st:87850-HuGene_st:1061724- | | | | | |
| | HuGene_st:1040362-HuGene_st:336607- | | | | | |
| | HuGene_st:799683-HuGene_st:504888- | | | | | |
| | HuGene_st:212730_at:872910- | | | | | |
| | HuGene_st:861909-HuGene_st:406723- | | | | | |
| | HuGene_st:530704- | | | | | |
| | HuGene_st:214304_x_at | | | | | |
| MPEG1 | 226818_at:918920-HuGene_st:283447- | 4.58E-08 | 1.6464 | 1.86 | 79.5 | 70.8-86.4 |
| | HuGene_st:449729-HuGene_st:301732- | | | | | |
| | HuGene_st:915566-HuGene_st:854136- | | | | | |
| | HuGene_st:226841_at:477225- | | | | | |
| | HuGene_st:1084905-HuGene_st:104883- | | | | | |
| | HuGene_st:342839-HuGene_st:863106- | | | | | |
| | HuGene_st:504453-HuGene_st:44663- | | | | | |
| | HuGene_st:607415-HuGene_st:968224- | | | | | |
| | HuGene_st:990725-HuGene_st:735251- | | | | | |
| | HuGene_st | | | | | |
| IQGAP2 | 185028-HuGene_st:763806- | 5.06E-08 | 1.6277 | 2.51 | 79.2 | 70.5-86.2 |
| | HuGene_st:88647-HuGene_st:93358- | | | | | |
| | HuGene_st:393573-HuGene_st:743251- | | | | | |
| | HuGene_st:1010599-HuGene_st:786301- | | | | | |
| | HuGene_st:1066864-HuGene_st:231977- | | | | | |
| | HuGene_st:370908-HuGene_st:937713- | | | | | |
| | HuGene_st:201743- | | | | | |
| | HuGene_st:243728_at:899180- | | | | | |
| | HuGene_st:981926-HuGene_st:897914- | | | | | |
| | HuGene_st:1095716-HuGene_st:802231-v | | | | | |
| | HuGene_st:263364-HuGene_st:986453- | | | | | |
| | HuGene_st:203474_at | | | | | |
| LMOD1 | 611871-HuGene_st:53373- | 5.55E-08 | 1.5812 | 2.97 | 78.5 | 69.8-85.6 |
| | HuGene_st:380885-HuGene_st:958079- | | | | | |
| | HuGene_st:986515-HuGene_st:173411- | | | | | |
| | HuGene_st:616270-HuGene_st:517377- | | | | | |
| | HuGene_st:433277-HuGene_st:287901- | | | | | |
| | HuGene_st:1071756-HuGene_st:180631- | | | | | |
| | HuGene_st:52042- | | | | | |
| | HuGene_st:203766_s_at:732981- | | | | | |
| | HuGene_st:211562_s_at:999216- | | | | | |
| | HuGene_st:993253-HuGene_st:589530- | | | | | |
| | HuGene_st:675886-HuGene_st:945700- | | | | | |
| | HuGene_st | | | | | |
| PGM5 | 810480-HuGene_st:111685- | 5.87E-08 | 1.6218 | 5.87 | 79.1 | 70.4-86.2 |
| | HuGene_st:338926-HuGene_st:81611- | | | | | |
| | HuGene_st:658883-HuGene_st:803066- | | | | | |
| | HuGene_st:208491_s_at:956998- | | | | | |
| | HuGene_st:1095193-HuGene_st:1066912- | | | | | |
| | HuGene_st:1079743-HuGene_st:743259- | | | | | |
| | HuGene_st:429274-HuGene_st:259184- | | | | | |
| | HuGene_st:376761-HuGene_st:249074- | | | | | |
| | HuGene_st:226303_at | | | | | |
| DDR2 | 857174-HuGene_st:297630- | 5.89E-08 | 1.5612 | 2.61 | 78.2 | 69.5-85.4 |
| | HuGene_st:198601-HuGene_st:951531- | | | | | |
| | HuGene_st:341474-HuGene_st:1008685- | | | | | |
| | HuGene_st:1000005-HuGene_st:712988- | | | | | |
| | HuGene_st:263417-HuGene_st:511597- | | | | | |
| | HuGene_st:348176-HuGene_st:928611- | | | | | |
| | HuGene_st:318480-HuGene_st:801516- | | | | | |
| | HuGene_st:849806-HuGene_st:510631- | | | | | |
| | HuGene_st:225442_at:87448- | | | | | |
| | HuGene_st:704299-HuGene_st:227561_at | | | | | |
| PALM2-AKAP2 | 202759_s_at:202760_s_at | 6.28E-08 | 1.613 | 2.17 | 79 | 70.3-86.1 |
| AOC3 | 557248-HuGene_st:209280- | 6.43E-08 | 1.5565 | 2.92 | 78.2 | 69.3-85.4 |
| | HuGene_st:588332-HuGene_st:108003- | | | | | |
| | HuGene_st:960783-HuGene_st:407777- | | | | | |
| | HuGene_st:147438-HuGene_st:548345- | | | | | |
| | HuGene_st:721301-HuGene_st:128882- | | | | | |
| | HuGene_st:340215-HuGene_st:631235- | | | | | |
| | HuGene_st:641187-HuGene_st:117366- | | | | | |
| | HuGene_st:949088- | | | | | |
| | HuGene_st:204894_s_at | | | | | |
| PAPSS2 | 1040734-HuGene_st:893182- | 6.43E-08 | 1.6163 | 1.97 | 79 | 70.4-86.1 |
| | HuGene_st:355010-HuGene_st:808055- | | | | | |
| | HuGene_st:449559- | | | | | |
| | HuGene_st:203059_s_at:710965- | | | | | |
| | HuGene_st:891049-HuGene_st:1050455- | | | | | |
| | HuGene_st:59122- | | | | | |
| | HuGene_st:203058_s_at:203060_s_at:968 | | | | | |
| | 581-HuGene_st:932781- | | | | | |
| | HuGene_st:291649-HuGene_st:600968- | | | | | |
| | HuGene_st:413041-HuGene_st:247101- | | | | | |
| | HuGene_st:1087256- | | | | | |
| | HuGene_st:237496_at:15324- | | | | | |
| | HuGene_st:675829-HuGene_st:771003- | | | | | |
| | HuGene_st:343109-HuGene_st | | | | | |
| SDPR | 878908-HuGene_st:781527- | 9.62E-08 | 1.6006 | 2.21 | 78.8 | 70-85.9 |
| | HuGene_st:331976-HuGene_st:238150- | | | | | |
| | HuGene_st:306039-HuGene_st:535903- | | | | | |
| | HuGene_st:302361-HuGene_st:1005813- | | | | | |
| | HuGene_st:71118-HuGene_st:992629- | | | | | |
| | HuGene_st:218711_s_at:293110- | | | | | |
| | HuGene_st:779040- | | | | | |
| | HuGene_st:222717_at:970479- | | | | | |
| | HuGene_st:581654-HuGene_st | | | | | |
| DUSP1 | 308761-HuGene_st:904724- | 1.06E-07 | 1.527 | 2.44 | 77.7 | 68.8-85 |
| | HuGene_st:371253- | | | | | |
| | HuGene_st:201041_s_at:918293- | | | | | |
| | HuGene_st:636896-HuGene_st:793780- | | | | | |
| | HuGene_st:714382-HuGene_st:83208- | | | | | |
| | HuGene_st:504846-HuGene_st:1046152- | | | | | |
| | HuGene_st:936429- | | | | | |
| | HuGene_st:226578_s_at:367434- | | | | | |
| | HuGene_st:676144-HuGene_st:57046- | | | | | |
| | HuGene_st:466265-HuGene_st:775780- | | | | | |
| | HuGene_st:747888-HuGene_st:554753- | | | | | |
| | HuGene_st:101136-HuGene_st:829116- | | | | | |
| | HuGene_st:201044_x_at:571046- | | | | | |
| | HuGene_st | | | | | |
| PRKAR2B | 101664-HuGene_st:162600- | 1.09E-07 | 1.677 | 2.1 | 79.9 | 71.2-86.9 |
| | HuGene_st:426444-HuGene_st:988677- | | | | | |
| | HuGene_st:546073-HuGene_st:714756- | | | | | |
| | HuGene_st:683742-HuGene_st:422832- | | | | | |
| | HuGene_st:509601-HuGene_st:898244- | | | | | |
| | HuGene_st:904472-HuGene_st:705909- | | | | | |
| | HuGene_st:348626-HuGene_st:699010- | | | | | |
| | HuGene_st:742973-HuGene_st:283465- | | | | | |
| | HuGene_st:720328-HuGene_st:320328- | | | | | |
| | HuGene_st:210153_s_at:688661- | | | | | |
| | HuGene_st:478012-HuGene_st:373286- | | | | | |
| | HuGene_st:484605-HuGene_st:989810- | | | | | |
| | HuGene_st:280047-HuGene_st:473253- | | | | | |
| | HuGene_st:630331-HuGene_st:1003297- | | | | | |
| | HuGene_st:209397_at:203680_at | | | | | |
| CNTN3 | 267567-HuGene_st:782053- | 1.20E-07 | 1.6161 | 3.93 | 79 | 70.3-86.1 |
| | HuGene_st:550157-HuGene_st:541394- | | | | | |
| | HuGene_st:989173-HuGene_st:15899- | | | | | |
| | HuGene_st:78622-HuGene_st:339738- | | | | | |
| | HuGene_st:585282-HuGene_st:661814- | | | | | |
| | HuGene_st:360715-HuGene_st:695033- | | | | | |
| | HuGene_st:483058-HuGene_st:555394- | | | | | |
| | HuGene_st:315011-HuGene_st:905374- | | | | | |
| | HuGene_st:1067212-HuGene_st:557263- | | | | | |
| | HuGene_st:233502_at:811729- | | | | | |
| | HuGene_st:87414-HuGene_st | | | | | |
| RAB27A | 339168-HuGene_st:340479- | 1.22E-07 | 1.5976 | 1.79 | 78.8 | 70-85.9 |
| | HuGene_st:1057756-HuGene_st:99120- | | | | | |
| | HuGene_st:301308-HuGene_st:309278- | | | | | |
| | HuGene_st:641167-HuGene_st:900707- | | | | | |
| | HuGene_st:961788- | | | | | |
| | HuGene_st:210951_x_at:209515_s_at:924 | | | | | |
| | 247-HuGene_st:209514_s_at:2432- | | | | | |
| | HuGene_st:405271-HuGene st:758428- | | | | | |
| | HuGene_st:235766_x_at:391537- | | | | | |
| | HuGene_st:983411- | | | | | |
| | HuGene_st:222294_s_at | | | | | |
| C6orf105 | 669403-HuGene_st:937383- | 1.38E-07 | 1.6043 | 2.13 | 78.9 | 70.1-85.9 |
| | HuGene_st:344202- | | | | | |
| | HuGene_st:229070_at:1088682- | | | | | |
| | HuGene_st:215100_at:287254- | | | | | |
| | HuGene_st:34196-HuGene_st:701140- | | | | | |
| | HuGene_st:461427-HuGene_st:1032543- | | | | | |
| | HuGene_st:237434-HuGene_st:542250- | | | | | |
| | HuGene_st:316541-HuGene_st:179382- | | | | | |
| | HuGene_st:148807-HuGene_st:1029919- | | | | | |
| | HuGene_st:58955-HuGene_st:48477- | | | | | |
| | HuGene_st:869984-HuGene_st:667357- | | | | | |
| | HuGene_st:697774-HuGene_st:164714- | | | | | |
| | HuGene_st:105735-HuGene_st | | | | | |
| MUC12 | 412725-HuGene_st:1083259- | 1.53E-07 | 1.4566 | 4.89 | 76.7 | 67.7-84.1 |
| | HuGene_st:437412-HuGene_st:751451- | | | | | |
| | HuGene_st:498196-HuGene_st:1087628- | | | | | |
| | HuGene_st:214378-HuGene_st:61291- | | | | | |
| | HuGene_st:661525-HuGene_st:678015- | | | | | |
| | HuGene_st:468158-HuGene_st:991377- | | | | | |
| | HuGene_st:280731-HuGene_st:790312- | | | | | |
| | HuGene_st:770658-HuGene_st:56500- | | | | | |
| | HuGene_st:181935-HuGene_st:1013137- | | | | | |
| | HuGene_st:1557906_at:226654_at:155790 | | | | | |
| | 7_x_at:153751-HuGene_st:765891- | | | | | |
| | HuGene_st:946487- | | | | | |
| | HuGene_st:231814_at:42037-HuGene_st | | | | | |
| AXL | 321003-HuGene_st:546377- | 1.59E-07 | 1.5776 | 1.71 | 78.5 | 69.7-85.6 |
| | HuGene_st:429002-HuGene_st:790260- | | | | | |
| | HuGene_st:796222-HuGene_st:59559- | | | | | |
| | HuGene_st:221741-HuGene_st:669140- | | | | | |
| | HuGene_st:822162-HuGene_st:521036- | | | | | |
| | HuGene_st:202685_s_at:501383- | | | | | |
| | HuGene_st:883192-HuGene_st | | | | | |
| IL1R2 | 377084-HuGene_st:278685- | 1.73E-07 | 1.5226 | 2.54 | 77.7 | 68.7-84.9 |
| | HuGene_st:472031-HuGene_st:792041- | | | | | |
| | HuGene_st:1012886-HuGene_st:568945- | | | | | |
| | HuGene_st:80054-HuGene_st:866918- | | | | | |
| | HuGene_st:866883-HuGene_st:17623- | | | | | |
| | HuGene_st:314768-HuGene_st:326744- | | | | | |
| | HuGene_st:211372_s_at:888405- | | | | | |
| | HuGene_st:205403_at:834475- | | | | | |
| | HuGene_st:386354-HuGene_st:411355- | | | | | |
| | HuGene_st:862981-HuGene_st:70227- | | | | | |
| | HuGene_st | | | | | |
| TSC22D3 | 235364_at:1044209-HuGene_st:519020- | 1.85E-07 | 1.6337 | 3.1 | 79.3 | 70.6-86.3 |
| | HuGene_st:427467-HuGene_st:669553- | | | | | |
| | HuGene_st:722843- | | | | | |
| | HuGene_st:208763_s_at:126162- | | | | | |
| | HuGene_st:207001_x_at:654578- | | | | | |
| | HuGene_st:146515-HuGene_st:1100721- | | | | | |
| | HuGene_st:823307-HuGene_st:899494- | | | | | |
| | HuGene_st:952685-HuGene_st:235399- | | | | | |
| | HuGene_st:802521-HuGene_st:126175- | | | | | |
| | HuGene_st:722700-HuGene_st | | | | | |
| GPA33 | 169349-HuGene_st:772424- | 1.86E-07 | 1.7041 | 1.61 | 80.3 | 71.7-87.2 |
| | HuGene_st:482659-HuGene_st:1053088- | | | | | |
| | HuGene_st:221229- | | | | | |
| | HuGene_st:205929_at:21623- | | | | | |
| | HuGene_st:352510-HuGene_st:431411- | | | | | |
| | HuGene_st:507225-HuGene_st:362352- | | | | | |
| | HuGene_st:492326-HuGene_st:555302- | | | | | |
| | HuGene_st:812966-HuGene_st:207883- | | | | | |
| | HuGene_st:401745-HuGene_st:630242- | | | | | |
| | HuGene_st:212295-HuGene_st:1097025- | | | | | |
| | HuGene_st:157847-HuGene_st | | | | | |
| IDH3A | 202069_s_at:300578- | 2.08E-07 | 1.567 | 1.75 | 78.3 | 69.6-85.5 |
| | HuGene_st:202070_s_at:342767- | | | | | |
| | HuGene_st:8965-HuGene_st:1079553- | | | | | |
| | HuGene_st:221816-HuGene_st:1042110- | | | | | |
| | HuGene_st:647088-HuGene_st:670662- | | | | | |
| | HuGene_st:53656-HuGene_st:572196- | | | | | |
| | HuGene_st:607148-HuGene_st:463534- | | | | | |
| | HuGene_st:784198-HuGene_st:859733- | | | | | |
| | HuGene_st:221939-HuGene_st:606301- | | | | | |
| | HuGene_st:546210-HuGene_st:475783- | | | | | |
| | HuGene_st:1084692-HuGene_st:538386- | | | | | |
| | HuGene_st | | | | | |
| MATN2 | 902099-HuGene_st:1065921- | 2.11E-07 | 1.5084 | 2.23 | 77.5 | 68.6-84.8 |
| | HuGene_st:452682-HuGene_st:1012649- | | | | | |
| | HuGene_st:1019476-HuGene_st:88797- | | | | | |
| | HuGene_st:986521-HuGene_st:181168- | | | | | |
| | HuGene_st:102298-HuGene_st:757359- | | | | | |
| | HuGene_st:859886-HuGene_st:540941- | | | | | |
| | HuGene_st:202350_s_at:326901- | | | | | |
| | HuGene_st:781583-HuGene_st:498255- | | | | | |
| | HuGene_st:212041-HuGene_st | | | | | |
| MT2A | 723245-HuGene_st:710161- | 2.49E-07 | 1.6459 | 2.41 | 79.5 | 70.8-86.5 |
| | HuGene_st:133506-HuGene_st:90770- | | | | | |
| | HuGene_st:309654-HuGene_st:194391- | | | | | |
| | HuGene_st:692585-HuGene_st:117983- | | | | | |
| | HuGene_st:858756-HuGene_st:123483- | | | | | |
| | HuGene_st:296152- | | | | | |
| | HuGene_st:217546_at:239766- | | | | | |
| | HuGene_st:46711-HuGene_st:1057926- | | | | | |
| | HuGene_st:611729-HuGene_st:1048755- | | | | | |
| | HuGene_st:519540-HuGene_st:284543- | | | | | |
| | HuGene_st:847898-HuGene_st:648660- | | | | | |
| | HuGene_st:977125-HuGene_st:743580- | | | | | |
| | HuGene_st:327541-HuGene_st:948505- | | | | | |
| | HuGene_st:6546-HuGene_st:84521- | | | | | |
| | HuGene_st:907619-HuGene_st:939779- | | | | | |
| | HuGene_st:561550-HuGene_st:76641- | | | | | |
| | HuGene_st:1000258-HuGene_st:447330- | | | | | |
| | HuGene_st:983649-HuGene_st:613324- | | | | | |
| | HuGene_st:846448-HuGene_st:304444- | | | | | |
| | HuGene_st:212859_x_at:40069- | | | | | |
| | HuGene_st:212884-HuGene_st:575774- | | | | | |
| | HuGene_st:427914-HuGene_st:56303- | | | | | |
| | HuGene_st:213349-HuGene_st:721776- | | | | | |
| | HuGene_st:212185_x_at:739002- | | | | | |
| | HuGene_st:902404-HuGene_st:483985- | | | | | |
| | HuGene_st:91794-HuGene_st:137880- | | | | | |
| | HuGene_st:317864-HuGene_st:203291- | | | | | |
| | HuGene_st:66799-HuGene_st:160991- | | | | | |
| | HuGene_st:216336_x_at:310066- | | | | | |
| | HuGene_st:1079767-HuGene_st:781831- | | | | | |
| | HuGene_st:1095705-HuGene_st:534398- | | | | | |
| | HuGene_st:467500-HuGene_st:491335- | | | | | |
| | HuGene_st:66800-HuGene_st:485318- | | | | | |
| | HuGene_st:90966-HuGene_st:365104- | | | | | |
| | HuGene_st:79259-HuGene_st:495714- | | | | | |
| | HuGene_st:1095744-HuGene_st:102946- | | | | | |
| | HuGene_st:477858-HuGene_st:771030- | | | | | |
| | HuGene_st:675707-HuGene_st:854293- | | | | | |
| | HuGene_st:160013-HuGene_st:320514- | | | | | |
| | HuGene_st:1047433-HuGene_st:890901- | | | | | |
| | HuGene_st:230953-HuGene_st:749925- | | | | | |
| | HuGene_st:19613-HuGene_st:166551- | | | | | |
| | HuGene_st:574267-HuGene_st:623210- | | | | | |
| | HuGene_st:1006466-HuGene_st:900368- | | | | | |
| | HuGene_st:760559-HuGene_st:446223- | | | | | |
| | HuGene_st:205427-HuGene_st:983813- | | | | | |
| | HuGene_st:435034-HuGene_st:250747- | | | | | |
| | HuGene_st | | | | | |
| MT1M | 723245-HuGene_st:710161- | 2.49E-07 | 1.6419 | 2.41 | 79.4 | 70.7-86.4 |
| | HuGene_st:133506-HuGene_st:90770- | | | | | |
| | HuGene_st:309654-HuGene_st:194391- | | | | | |
| | HuGene_st:692585-HuGene_st:117983- | | | | | |
| | HuGene_st:858756-HuGene_st:123483- | | | | | |
| | HuGene_st:296152- | | | | | |
| | HuGene_st:217546_at:239766- | | | | | |
| | HuGene_st:46711-HuGene_st:1057926- | | | | | |
| | HuGene_st:611729-HuGene_st:1048755- | | | | | |
| | HuGene_st:519540-HuGene_st:284543- | | | | | |
| | HuGene_st:847898-HuGene_st:648660- | | | | | |
| | HuGene_st:977125-HuGene_st:743580- | | | | | |
| | HuGene_st:327541-HuGene_st:948505- | | | | | |
| | HuGene_st:6546-HuGene_st:84521- | | | | | |
| | HuGene_st:907619-HuGene_st:939779- | | | | | |
| | HuGene_st:561550-HuGene_st:76641- | | | | | |
| | HuGene_st:1000258-HuGene_st:447330- | | | | | |
| | HuGene_st:983649-HuGene_st:613324- | | | | | |
| | HuGene_st:846448-HuGene_st:304444- | | | | | |
| | HuGene_st:212859_x_at:40069- | | | | | |
| | HuGene_st:212884-HuGene_st:575774- | | | | | |
| | HuGene_st:427914-HuGene_st:56303- | | | | | |
| | HuGene_st:213349-HuGene_st:721776- | | | | | |
| | HuGene_st:212185_x_at:739002- | | | | | |
| | HuGene_st:902404-HuGene_st:483985- | | | | | |
| | HuGene_st:91794-HuGene_st:137880- | | | | | |
| | HuGene_st:317864-HuGene_st:203291- | | | | | |
| | HuGene_st:66799-HuGene_st:160991- | | | | | |
| | HuGene_st:216336_x_at:310066- | | | | | |
| | HuGene_st:1079767-HuGene_st:781831- | | | | | |
| | HuGene_st:1095705-HuGene_st:534398- | | | | | |
| | HuGene_st:467500-HuGene_st:491335- | | | | | |
| | HuGene_st:66800-HuGene_st:485318- | | | | | |
| | HuGene_st:90966-HuGene_st:365104- | | | | | |
| | HuGene_st:79259-HuGene_st:495714- | | | | | |
| | HuGene_st:1095744-HuGene_st:102946- | | | | | |
| | HuGene_st:477858-HuGene_st:771030- | | | | | |
| | HuGene_st:675707-HuGene_st:854293- | | | | | |
| | HuGene_st:160013-HuGene_st:320514- | | | | | |
| | HuGene_st:1047433-HuGene_st:890901- | | | | | |
| | HuGene_st:230953-HuGene_st:749925- | | | | | |
| | HuGene_st:19613-HuGene_st:166551- | | | | | |
| | HuGene_st:574267-HuGene_st:623210- | | | | | |
| | HuGene_st:1006466-HuGene_st:900368- | | | | | |
| | HuGene_st:760559-HuGene_st:446223- | | | | | |
| | HuGene_st:205427-HuGene_st:983813- | | | | | |
| | HuGene_st:435034-HuGene_st:250747- | | | | | |
| | HuGene_st | | | | | |
| IGL@ | 173623-HuGene_st:408026- | 2.50E-07 | 1.6337 | 3.1 | 79.3 | 70.7-86.3 |
| | HuGene_st:38807-HuGene_st | | | | | |
| HSPB8 | 688528-HuGene_st:311091- | 2.61E-07 | 1.5121 | 3.85 | 77.5 | 68.7-84.8 |
| | HuGene_st:415722-HuGene_st:56376- | | | | | |
| | HuGene_st:79205-HuGene_st:499751- | | | | | |
| | HuGene_st:214231-HuGene_st:225808- | | | | | |
| | HuGene_st:174359-HuGene_st:1083550- | | | | | |
| | HuGene_st:221667_s_at:662744- | | | | | |
| | HuGene_st:346589-HuGene_st | | | | | |
| A2M | 897456-HuGene_st:217757_at:164005- | 2.72E-07 | 1.5968 | 1.64 | 78.8 | 69.9-85.9 |
| | HuGene_st:848096-HuGene_st:244657- | | | | | |
| | HuGene_st:317437-HuGene_st:330214- | | | | | |
| | HuGene_st:814658-HuGene_st:1100520- | | | | | |
| | HuGene_st:451567-HuGene_st:559737- | | | | | |
| | HuGene_st:600508-HuGene_st:730036- | | | | | |
| | HuGene_st:385395-HuGene_st:833129- | | | | | |
| | HuGene_st:101038-HuGene_st:195639- | | | | | |
| | HuGene_st:371583-HuGene_st:808161- | | | | | |
| | HuGene_st:893977- | | | | | |
| | HuGene_st:1558450_at:167145- | | | | | |
| | HuGene_st:660806-HuGene_st | | | | | |
| MS4A7 | 803709-HuGene_st:372940- | 2.92E-07 | 1.5398 | 2.27 | 77.9 | 69.1-85.2 |
| | HuGene_st:558657-HuGene_st:758990- | | | | | |
| | HuGene_st:279234-HuGene_st:716878- | | | | | |
| | HuGene_st:228770-HuGene_st:77553- | | | | | |
| | HuGene_st:212226-HuGene_st:148024- | | | | | |
| | HuGene_st:727350- | | | | | |
| | HuGene_st:223344_s_at:223343_at:22435 | | | | | |
| | 8_s_at:932003-HuGene_st:584291- | | | | | |
| | HuGene_st:16857-HuGene_st:751363- | | | | | |
| | HuGene_st:120594-HuGene_st:66810- | | | | | |
| | HuGene_st:473741-HuGene_st | | | | | |
| PLOD2 | 366970-HuGene_st:202619_s_at:557917- | 3.06E-07 | 1.5569 | 2.33 | 78.2 | 69.3-85.4 |
| | HuGene_st:371377-HuGene_st:606385- | | | | | |
| | HuGene_st:54079-HuGene_st:976248- | | | | | |
| | HuGene_st:572307-HuGene_st:716158- | | | | | |
| | HuGene_st:754684-HuGene_st:255595- | | | | | |
| | HuGene_st:17343-HuGene_st:124945- | | | | | |
| | HuGene_st:770320-HuGene_st:704827- | | | | | |
| | HuGene_st:202620_s_at:309852- | | | | | |
| | HuGene_st:509273-HuGene_st:703772- | | | | | |
| | HuGene_st:1082989-HuGene_st:550147- | | | | | |
| | HuGene_st:740582-HuGene_st:6711- | | | | | |
| | HuGene_st | | | | | |
| UGT2B17 | 357315-HuGene_st:493733- | 3.75E-07 | 1.4818 | 4.88 | 77.1 | 68.2-84.5 |
| | HuGene_st:353886- | | | | | |
| | HuGene_st:207245_at:59042- | | | | | |
| | HuGene_st:1025125-HuGene_st | | | | | |
| PMP22 | 1086412-HuGene_st:139615- | 3.97E-07 | 1.5173 | 2.23 | 77.6 | 68.8-84.9 |
| | HuGene_st:864327- | | | | | |
| | HuGene_st:1565637_at:493430- | | | | | |
| | HuGene_st:640953-HuGene_st:309874- | | | | | |
| | HuGene_st:1072798-HuGene_st:761816- | | | | | |
| | HuGene_st:136620-HuGene_st:441104- | | | | | |
| | HuGene_st:97712-HuGene_st:800086- | | | | | |
| | HuGene_st:352501-HuGene_st:363233- | | | | | |
| | HuGene_st:210139_s_at:48107- | | | | | |
| | HuGene_st:338800-HuGene_st:532682- | | | | | |
| | HuGene_st:988715-HuGene_st:853970- | | | | | |
| | HuGene_st | | | | | |
| PLN | 204938_s_at:418214-HuGene_st:769837- | 4.14E-07 | 1.5464 | 2.01 | 78 | 69.2-85.2 |
| | HuGene_st:603329-HuGene_st:1048096- | | | | | |
| | HuGene_st:944510-HuGene_st:430402- | | | | | |
| | HuGene_st:86633-HuGene_st:795093- | | | | | |
| | HuGene_st:377757-HuGene_st:770145- | | | | | |
| | HuGene_st:920628-HuGene_st:594048- | | | | | |
| | HuGene_st:800583- | | | | | |
| | HuGene_st:204939_s_at:556723- | | | | | |
| | HuGene_st:818339-HuGene_st:175524- | | | | | |
| | HuGene_st:498924-HuGene_st:204940_at | | | | | |
| CNN1 | 337187-HuGene_st:787686- | 4.77E-07 | 1.4318 | 4.56 | 76.3 | 67.2-83.8 |
| | HuGene_st:532503-HuGene_st:41547- | | | | | |
| | HuGene_st:236587-HuGene_st:809046- | | | | | |
| | HuGene_st:674638-HuGene_st:1055006- | | | | | |
| | HuGene_st:617683-HuGene_st:769218- | | | | | |
| | HuGene_st:373491-HuGene_st:609613- | | | | | |
| | HuGene_st:402708-HuGene_st:871546- | | | | | |
| | HuGene_st:203951_at:880804- | | | | | |
| | HuGene_st:1091224-HuGene_st:911860- | | | | | |
| | HuGene_st:1090431-HuGene_st | | | | | |
| ITM2A | 391687-HuGene_st:1059695- | 5.27E-07 | 1.4958 | 2.77 | 77.3 | 68.3-84.6 |
| | HuGene_st:12009-HuGene_st:677746- | | | | | |
| | HuGene_st:258271-HuGene_st:676659- | | | | | |
| | HuGene_st:496836-HuGene_st:452608- | | | | | |
| | HuGene_st:1059257-HuGene_st:47720- | | | | | |
| | HuGene_st:105265-HuGene_st:657686- | | | | | |
| | HuGene_st:68702-HuGene_st:115530- | | | | | |
| | HuGene_st:202747_s_at:202746_at | | | | | |
| KCNMA1 | 554163-HuGene_st:716361- | 6.86E-07 | 1.497 | 2.37 | 77.3 | 68.4-84.6 |
| | HuGene_st:403072-HuGene_st:63347- | | | | | |
| | HuGene_st:587079-HuGene_st:256038- | | | | | |
| | HuGene_st:172619-HuGene_st:918468- | | | | | |
| | HuGene_st:94420-HuGene_st:399013- | | | | | |
| | HuGene_st:414900-HuGene_st:307320- | | | | | |
| | HuGene_st:906761-HuGene_st:115093- | | | | | |
| | HuGene_st:221583_s_at:745071- | | | | | |
| | HuGene_st:915037-HuGene_st:343951- | | | | | |
| | HuGene_st:1569763_at:446685- | | | | | |
| | HuGene_st:652192-HuGene_st | | | | | |
| XDH | 919455-HuGene_st:105269- | 8.13E-07 | 1.4727 | 2.03 | 76.9 | 67.9-84.3 |
| | HuGene_st:106986-HuGene_st:972900- | | | | | |
| | HuGene_st:210301_at:716491- | | | | | |
| | HuGene_st:667307-HuGene_st:790507- | | | | | |
| | HuGene_st:800140-HuGene_st:328962- | | | | | |
| | HuGene_st:355792-HuGene_st:707858- | | | | | |
| | HuGene_st:731429-HuGene_st:403069- | | | | | |
| | HuGene_st:215338-HuGene_st:101916- | | | | | |
| | HuGene_st:48855-HuGene_st:895641- | | | | | |
| | HuGene_st:68212-HuGene_st:287011- | | | | | |
| | HuGene_st:1082168- | | | | | |
| | HuGene_st:241994_at:709200-HuGene_st | | | | | |
| PDLIM3 | 659306-HuGene_st:9491- | 8.20E-07 | 1.4799 | 2.38 | 77 | 68.2-84.4 |
| | HuGene_st:787864-HuGene_st:482305- | | | | | |
| | HuGene_st:810161-HuGene_st:1037974- | | | | | |
| | HuGene_st:353413-HuGene_st:731754- | | | | | |
| | HuGene_st:733690- | | | | | |
| | HuGene_st:209621_s_at:394037- | | | | | |
| | HuGene_st:957822-HuGene_st:67576- | | | | | |
| | HuGene_st:369461-HuGene_st:768470- | | | | | |
| | HuGene_st:489488-HuGene_st:711969- | | | | | |
| | HuGene_st:1569564_at:1077054- | | | | | |
| | HuGene_st:210170_at:238592_at | | | | | |
| MT1X | 788358-HuGene_st:20578- | 9.84E-07 | 1.4411 | 4.35 | 76.4 | 67.5-83.9 |
| | HuGene_st:92311-HuGene_st:501820- | | | | | |
| | HuGene_st:208581_x_at:73687- | | | | | |
| | HuGene_st:905260-HuGene_st:1087816- | | | | | |
| | HuGene_st:204326_x_at:96216- | | | | | |
| | HuGene_st:764373-HuGene_st:103649- | | | | | |
| | HuGene_st:40373-HuGene_st:198342- | | | | | |
| | HuGene_st | | | | | |
| CALD1 | 796040-HuGene_st:839187- | 1.02E-06 | 1.4584 | 3.29 | 76.7 | 67.7-84.1 |
| | HuGene_st:412659-HuGene_st:1023569- | | | | | |
| | HuGene_st:450656- | | | | | |
| | HuGene_st:212077_at:201616_s_at:81643 | | | | | |
| | 9-HuGene_st:165931- | | | | | |
| | HuGene_st:201617_x_at:576686- | | | | | |
| | HuGene_st:1094139- | | | | | |
| | HuGene_st:201615_x_at:519079- | | | | | |
| | HuGene_st:558226-HuGene_st:755661- | | | | | |
| | HuGene_st:243084_at:318906- | | | | | |
| | HuGene_st:688034-HuGene_st:215199_at | | | | | |
| MSRB3 | 483115-HuGene_st:439736- | 1.20E-06 | 1.4754 | 2.24 | 77 | 68-84.4 |
| | HuGene_st:1096860-HuGene_st:644381- | | | | | |
| | HuGene_st:225790_at:306630- | | | | | |
| | HuGene_st:1073255-HuGene_st:121556- | | | | | |
| | HuGene_st:126154-HuGene_st:72517- | | | | | |
| | HuGene_st:480208-HuGene_st:331986- | | | | | |
| | HuGene_st:294511-HuGene_st:204701- | | | | | |
| | HuGene_st:1554127_s_at:42731- | | | | | |
| | HuGene_st:675396-HuGene_st:174560- | | | | | |
| | HuGene_st:1566481_at | | | | | |
| SPON1 | 209437_s_at:209436_at:892181- | 1.46E-06 | 1.4862 | 1.88 | 77.1 | 68.2-84.5 |
| | HuGene_st:960315-HuGene_st:377887- | | | | | |
| | HuGene_st:253667-HuGene_st:48054- | | | | | |
| | HuGene_st:1008441-HuGene_st:979120- | | | | | |
| | HuGene_st:213994_s_at:811254- | | | | | |
| | HuGene_st:928583-HuGene_st:405680- | | | | | |
| | HuGene_st:963535-HuGene_st:836638- | | | | | |
| | HuGene_st:1033364-HuGene_st:965345- | | | | | |
| | HuGene_st:936102-HuGene_st:555640- | | | | | |
| | HuGene_st:22053-HuGene_st:234367- | | | | | |
| | HuGene_st:224488_s_at:327210- | | | | | |
| | HuGene_st:481375- | | | | | |
| | HuGene_st:213993_at:1013182- | | | | | |
| | HuGene_st | | | | | |
| C2orf40 | 1011921-HuGene_st:304065- | 1.69E-06 | 1.4159 | 2.97 | 76.1 | 67-83.6 |
| | HuGene_st:441303-HuGene_st:1001854- | | | | | |
| | HuGene_st:999754-HuGene_st:893886- | | | | | |
| | HuGene_st:951843-HuGene_st:375926- | | | | | |
| | HuGene_st | | | | | |
| SORBS1 | 1041567-HuGene_st:83451- | 2.18E-06 | 1.4269 | 2.54 | 76.2 | 67.2-83.7 |
| | HuGene_st:29118- | | | | | |
| | HuGene_st:222513_s_at:617821- | | | | | |
| | HuGene_st:56685-HuGene_st:280855- | | | | | |
| | HuGene_st:67922-HuGene_st:1028489- | | | | | |
| | HuGene_st:611267-HuGene_st:931477- | | | | | |
| | HuGene_st:66752-HuGene_st:618734- | | | | | |
| | HuGene_st:624287- | | | | | |
| | HuGene_st:211819_s_at:581936- | | | | | |
| | HuGene_st:218087_s_at:1039879- | | | | | |
| | HuGene_st:45815-HuGene_st:186556- | | | | | |
| | HuGene_st:628496-HuGene_st:242736_at | | | | | |
| C4orf34 | 576095-HuGene_st:1061109- | 2.55E-06 | 1.3851 | 1.95 | 75.6 | 66.5-83.1 |
| | HuGene_st:698934-HuGene_st:634224- | | | | | |
| | HuGene_st:854845-HuGene_st:18233- | | | | | |
| | HuGene_st:275684-HuGene_st:784949- | | | | | |
| | HuGene_st:346086-HuGene_st:91468- | | | | | |
| | HuGene_st:936558-HuGene_st:163564- | | | | | |
| | HuGene_st:614699-HuGene_st:428090- | | | | | |
| | HuGene_st:340671-HuGene_st:90795- | | | | | |
| | HuGene_st:823553-HuGene_st:431110- | | | | | |
| | HuGene_st:719502-HuGene_st:850208- | | | | | |
| | HuGene_st:224990_at:542653-HuGene_st | | | | | |
| PDGFRA | 782752-HuGene_st:789846- | 2.94E-06 | 1.4819 | 1.37 | 77.1 | 68.1-84.4 |
| | HuGene_st:116091-HuGene_st:860506- | | | | | |
| | HuGene_st:537532-HuGene_st:112478- | | | | | |
| | HuGene_st:97681-HuGene_st:514113- | | | | | |
| | HuGene_st:561375-HuGene_st:115277- | | | | | |
| | HuGene_st:240764-HuGene_st:578952- | | | | | |
| | HuGene_st:723105-HuGene_st:230638- | | | | | |
| | HuGene_st:544163-HuGene_st:50957- | | | | | |
| | HuGene_st:468599-HuGene_st:328136- | | | | | |
| | HuGene_st:346391- | | | | | |
| | HuGene st:203131_at:853019-HuGene st | | | | | |
| FGFR2 | 476373-HuGene_st:92078- | 3.55E-06 | 1.527 | 1.31 | 77.7 | 68.9-85 |
| | HuGene_st:969020-HuGene_st:1022426- | | | | | |
| | HuGene_st:203639_s_at:24514- | | | | | |
| | HuGene_st:614945- | | | | | |
| | HuGene_st:211398_at:1097953- | | | | | |
| | HuGene_st:117962-HuGene_st:777874- | | | | | |
| | HuGene_st:829764-HuGene_st:868500- | | | | | |
| | HuGene_st:211401_s_at:403546- | | | | | |
| | HuGene_st:850416 | | | | | |
| | HuGene_st:208234_x_at:537762- | | | | | |
| | HuGene_st:132866-HuGene_st:426700- | | | | | |
| | HuGene_st:208228_s_at:831045- | | | | | |
| | HuGene_st:203638_s_at:240913_at:23084 2_at | | | | | |
| PPP1R12B | 23543-HuGene_st:654289- | 4.26E-06 | 1.3592 | 2.21 | 75.2 | 66.1-82.8 |
| | HuGene_st:947526-HuGene_st:714709- | | | | | |
| | HuGene_st:544138- | | | | | |
| | HuGene_st:1557553_at:227411- | | | | | |
| | HuGene_st:201958_s_at:1559911_at:7483 8- | | | | | |
| | HuGene_st:201957_at:233700_at:67323- | | | | | |
| | HuGene_st:767707- | | | | | |
| | HuGene_st:224270_at:50561- | | | | | |
| | HuGene_st:233359-HuGene_st:565179- | | | | | |
| | HuGene_st:317428-HuGene_st:442074- | | | | | |
| | HuGene_st:591561-HuGene_st | | | | | |
| FAM129A | 148319-HuGene_st:19042- | 4.59E-06 | 1.4144 | 1.8 | 76 | 67-83.5 |
| | HuGene_st:1075897-HuGene_st:49110- | | | | | |
| | HuGene_st:245230-HuGene_st:154650- | | | | | |
| | HuGene_st:203917-HuGene_st:503184- | | | | | |
| | HuGene_st:373453-HuGene_st:432126- | | | | | |
| | HuGene_st:786874-HuGene_st:648379- | | | | | |
| | HuGene_st:1033222- | | | | | |
| | HuGene_st:217966_s_at:574775- | | | | | |
| | HuGene_st:217967_s_at:156986- | | | | | |
| | HuGene_st:9160-HuGene_st:212626- | | | | | |
| | HuGene_st:935377-HuGene_st:921695- | | | | | |
| | HuGene_st:1053262-HuGene_st | | | | | |
| POSTN | 210809_s_at:297712- | 4.94E-06 | 1.3944 | 2.25 | 75.7 | 66.7-83.3 |
| | HuGene_st:1555778_a_at:608435- | | | | | |
| | HuGene_st:743877-HuGene_st:753556- | | | | | |
| | HuGene_st:388659-HuGene_st:954838- | | | | | |
| | HuGene_st:724846-HuGene_st:588010- | | | | | |
| | HuGene_st:713459-HuGene_st:713707- | | | | | |
| | HuGene_st:106590-HuGene_st:538299- | | | | | |
| | HuGene_st:649547-HuGene_st:13993- | | | | | |
| | HuGene_st:417058-HuGene_st:636479- | | | | | |
| | HuGene_st:228481_at:874100- | | | | | |
| | HuGene_st:776445-HuGene_st:779754- | | | | | |
| | HuGene_st | | | | | |
| ATP8B1 | 1088077-HuGene_st:699379- | 5.73E-06 | 1.3113 | 1.67 | 74.4 | 65.3-82.1 |
| | HuGene_st:81949-HuGene_st:120481- | | | | | |
| | HuGene_st:1006809-HuGene_st:960479- | | | | | |
| | HuGene_st:1033226-HuGene_st:854521- | | | | | |
| | HuGene_st:258436-HuGene_st:224444- | | | | | |
| | HuGene_st:958640-HuGene_st:670830- | | | | | |
| | HuGene_st:734950-HuGene_st:61644- | | | | | |
| | HuGene_st:354636-HuGene_st:308159- | | | | | |
| | HuGene_st:645299-HuGene_st:306422- | | | | | |
| | HuGene_st:223980-HuGene_st:772656- | | | | | |
| | HuGene_st:226302_at:745109- | | | | | |
| | HuGene_st:1011843-HuGene_st | | | | | |
| CCL28 | 224240_s_at:155321-HuGene_st:780816- | 6.01E-06 | 1.494 | 1.53 | 77.2 | 68.4-84.6 |
| | HuGene_st:224027_at:356805- | | | | | |
| | HuGene_st:120679-HuGene_st:525328- | | | | | |
| | HuGene_st:623557- | | | | | |
| | HuGene_st:238750_at:519318- | | | | | |
| | HuGene_st:956859-HuGene_st:251110- | | | | | |
| | HuGene_st:331562-HuGene_st:224868- | | | | | |
| | HuGene_st:28476-HuGene_st:153501- | | | | | |
| | HuGene_st:242217-HuGene_st:821675- | | | | | |
| | HuGene_st:451844-HuGene_st:513548- | | | | | |
| | HuGene_st:773827-HuGene_st:398738- | | | | | |
| | HuGene_st | | | | | |
| NEXN | 184663-HuGene_st:720053- | 6.83E-06 | 1.363 | 2.23 | 75.2 | 66.1-82.9 |
| | HuGene_st:539444-HuGene_st:311368- | | | | | |
| | HuGene_st:260384-HuGene_st:56754- | | | | | |
| | HuGene_st:1040436-HuGene_st:1064626- | | | | | |
| | HuGene_st:292849-HuGene_st:453309- | | | | | |
| | HuGene_st:804402-HuGene_st:500301- | | | | | |
| | HuGene_st:248505-HuGene_st:402104- | | | | | |
| | HuGene_st:1552309_a_at:941387- | | | | | |
| | HuGene_st:709246-HuGene_st:226103_at | | | | | |
| CTGF | NA | 6.96E-06 | 1.4786 | 1.3 | 77 | 68.1-84.4 |
| MFSD4 | 15050-HuGene_st:785959- | 7.19E-06 | 1.3233 | 2.12 | 74.6 | 65.5-82.3 |
| | HuGene_st:884375-HuGene_st:75016- | | | | | |
| | HuGene_st:235772-HuGene_st:185211- | | | | | |
| | HuGene_st:539224-HuGene_st:871027- | | | | | |
| | HuGene_st:855473-HuGene_st:728207- | | | | | |
| | HuGene_st:242782-HuGene_st:711626- | | | | | |
| | HuGene_st:484086- | | | | | |
| | HuGene_st:242372_s_at:238862_at:22925 | | | | | |
| | 4_at:656926-HuGene_st:767273- | | | | | |
| | HuGene_st:184295-HuGene_st:697889- | | | | | |
| | HuGene_st:1566569_at:708165- | | | | | |
| | HuGene_st | | | | | |
| ASPN | 787638-HuGene_st:567513- | 7.45E-06 | 1.3261 | 2.74 | 74.6 | 65.5-82.3 |
| | HuGene_st:499513-HuGene_st:546047- | | | | | |
| | HuGene_st:1055545-HuGene_st:720939- | | | | | |
| | HuGene_st:835521-HuGene_st:640448- | | | | | |
| | HuGene_st:673620-HuGene_st:821057- | | | | | |
| | HuGene_st:248224-HuGene_st:447994- | | | | | |
| | HuGene_st:407869-HuGene_st:510872- | | | | | |
| | HuGene_st:766560-HuGene_st | | | | | |
| DUSP5 | 25091-HuGene_st:352326- | 8.60E-06 | 1.334 | 1.91 | 74.8 | 65.6-82.4 |
| | HuGene_st:119170-HuGene_st:292093- | | | | | |
| | HuGene_st:209457_at:899442- | | | | | |
| | HuGene_st:171695-HuGene_st:331265- | | | | | |
| | HuGene_st:991652-HuGene_st:242476- | | | | | |
| | HuGene_st:430239-HuGene_st:1070934- | | | | | |
| | HuGene_st:555959-HuGene_st | | | | | |
| CRISPLD2 | 314440-HuGene_st:395147- | 9.24E-06 | 1.3669 | 1.64 | 75.3 | 66.2-82.9 |
| | HuGene_st:429916-HuGene_st:763823- | | | | | |
| | HuGene_st:729858- | | | | | |
| | HuGene_st:1555809_at:909486- | | | | | |
| | HuGene_st:239091-HuGene_st:418565- | | | | | |
| | HuGene_st:949339-HuGene_st:658945- | | | | | |
| | HuGene_st:276173-HuGene_st:96079- | | | | | |
| | HuGene_st | | | | | |
| FOXF2 | 589126-HuGene_st:856761- | 9.50E-06 | 1.3047 | 2.08 | 74.3 | 65.2-82.1 |
| | HuGene_st:145881-HuGene_st:915587- | | | | | |
| | HuGene_st:1088669-HuGene_st:81821- | | | | | |
| | HuGene_st:206377_at:575658- | | | | | |
| | HuGene_st:494266-HuGene_st:813114- | | | | | |
| | HuGene_st:613721-HuGene_st | | | | | |
| UGT2B15 | 500573-HuGene_st:352298- | 9.73E-06 | 1.3469 | 5.72 | 75 | 65.9-82.6 |
| | HuGene_st:770027- | | | | | |
| | HuGene_st:207392_x_at:31342- | | | | | |
| | HuGene_st:829850- | | | | | |
| | HuGene_st:217175_at:57112- | | | | | |
| | HuGene_st:1062564-HuGene_st:951114- | | | | | |
| | HuGene_st:844819-HuGene_st:841767- | | | | | |
| | HuGene_st:57590-HuGene_st:504268- | | | | | |
| | HuGene_st:27324-HuGene_st:340207- | | | | | |
| | HuGene_st:102754-HuGene_st:1055412- | | | | | |
| | HuGene_st | | | | | |
| ACTG2 | 667818-HuGene_st:500390- | 1.07E-05 | 1.3992 | 1.82 | 75.8 | 66.8-83.4 |
| | HuGene_st:979342-HuGene_st:560814- | | | | | |
| | HuGene_st:801310-HuGene_st:552015- | | | | | |
| | HuGene_st:972136-HuGene_st:617230- | | | | | |
| | HuGene_st:409424-HuGene_st:15155- | | | | | |
| | HuGene_st:416391-HuGene_st:800450- | | | | | |
| | HuGene_st:902303-HuGene_st:647647- | | | | | |
| | HuGene_st:123624- | | | | | |
| | HuGene_st:202274_at:586898- | | | | | |
| | HuGene_st:431150-HuGene_st:247321- | | | | | |
| | HuGene_st:735136-HuGene_st:986398- | | | | | |
| | HuGene_st:261974- | | | | | |
| | HuGene_st:241148_at:575416-HuGene_st | | | | | |
| CMBL | 157156-HuGene_st:305010- | 1.09E-05 | 1.3583 | 1.58 | 75.1 | 66.1-82.8 |
| | HuGene_st:227522_at:553921- | | | | | |
| | HuGene_st:529224-HuGene_st:1032780- | | | | | |
| | HuGene_st:204886-HuGene_st:623086- | | | | | |
| | HuGene_st:451862-HuGene_st:537678- | | | | | |
| | HuGene_st:918595-HuGene_st:178105- | | | | | |
| | HuGene_st:417861- | | | | | |
| | HuGene_st:230995_at:986212- | | | | | |
| | HuGene_st:405624-HuGene_st:424469- | | | | | |
| | HuGene_st:836099-HuGene_st:665079- | | | | | |
| | HuGene_st:234981_x_at | | | | | |
| KCTD12 | 509699-HuGene_st:77199- | 1.44E-05 | 1.3636 | 1.36 | 75.2 | 66.2-82.8 |
| | HuGene_st:212188_at:229632- | | | | | |
| | HuGene_st:502611-HuGene_st:931430- | | | | | |
| | HuGene_st:908582-HuGene_st:769157- | | | | | |
| | HuGene_st:312044- | | | | | |
| | HuGene_st:212192_at:43415- | | | | | |
| | HuGene_st:412026-HuGene_st:247270- | | | | | |
| | HuGene_st:770750-HuGene_st:827060- | | | | | |
| | HuGene_st:714432-HuGene_st | | | | | |
| MRGPRF | 717973-HuGene_st:226058- | 1.52E-05 | 1.2986 | 2.99 | 74.2 | 65.1-82 |
| | HuGene_st:541082-HuGene_st:60861- | | | | | |
| | HuGene_st:1024643-HuGene_st:354954- | | | | | |
| | HuGene_st:1099895-HuGene_st:672489- | | | | | |
| | HuGene_st:489062-HuGene_st:435421- | | | | | |
| | HuGene_st:227727_at:99658-HuGene_st | | | | | |
| C20orf118 | 235964_x_at:234987_at:235529_x_at:174 | 1.75E-05 | 1.4284 | 1.3 | 76.2 | 67.3-83.8 |
| | 500-HuGene_st:168513- | | | | | |
| | HuGene_st:312879-HuGene_st:57294- | | | | | |
| | HuGene_st:748530-HuGene_st:24012- | | | | | |
| | HuGene_st:219008-HuGene_st:471274- | | | | | |
| | HuGene_st:1086281-HuGene_st:740018- | | | | | |
| | HuGene_st:233829_at | | | | | |
| TMEM47 | 20487-HuGene_st:587019- | 1.86E-05 | 1.301 | 2.02 | 74.2 | 65.1-82 |
| | HuGene_st:458329-HuGene_st:944333- | | | | | |
| | HuGene_st:292459-HuGene_st:436914- | | | | | |
| | HuGene_st:632184- | | | | | |
| | HuGene_st:209655_s_at:885991- | | | | | |
| | HuGene_st:33524-HuGene_st:528167- | | | | | |
| | HuGene_st:555631-HuGene_st:595266- | | | | | |
| | HuGene_st:503408-HuGene_st:296998- | | | | | |
| | HuGene_st:672458-HuGene_st:64704- | | | | | |
| | HuGene_st:285813-HuGene_st:865055- | | | | | |
| | HuGene_st:734911-HuGene_st | | | | | |
| QKI | 214543_x_at:212262_at:696914- | 1.93E-05 | 1.3452 | 1.31 | 74.9 | 65.8-82.6 |
| | HuGene_st:363144-HuGene_st:820387- | | | | | |
| | HuGene_st:646624-HuGene_st:979853- | | | | | |
| | HuGene_st:658669-HuGene_st:748520- | | | | | |
| | HuGene_st:656477-HuGene_st:296134- | | | | | |
| | HuGene_st:1555154_a_at:212636_at:2344 | | | | | |
| | 92_at:462894-HuGene_st:1055543- | | | | | |
| | HuGene_st | | | | | |
| VSIG2 | 985113-HuGene_st:555767- | 2.01E-05 | 1.2959 | 2.05 | 74.1 | 65.1-81.9 |
| | HuGene_st:391438-HuGene_st:1093892- | | | | | |
| | HuGene_st:675062-HuGene_st:788466- | | | | | |
| | HuGene_st:826720-HuGene_st:997596- | | | | | |
| | HuGene_st:1069591- | | | | | |
| | HuGene_st:228232_s_at:223925- | | | | | |
| | HuGene_st:637781-HuGene_st:265221- | | | | | |
| | HuGene_st:652010-HuGene_st:265186- | | | | | |
| | HuGene_st:343187-HuGene_st:1095607- | | | | | |
| | HuGene_st | | | | | |
| HSPA1A | 979726-HuGene_st:1069731- | 2.16E-05 | 1.2699 | 2.41 | 73.7 | 64.6-81.5 |
| | HuGene_st:50191-HuGene_st:799087- | | | | | |
| | HuGene_st:200799_at:539596- | | | | | |
| | HuGene_st:150764 | | | | | |
| | HuGene_st:200800_s_at:492579- | | | | | |
| | HuGene_st | | | | | |
| AP1S2 | 230413_s_at:230264_s_at:99873- | 3.39E-05 | 1.2572 | 1.92 | 73.5 | 64.3-81.3 |
| | HuGene_st:57979-HuGene_st:985324- | | | | | |
| | HuGene_st:203300_x_at:84828- | | | | | |
| | HuGene_st:910415-HuGene_st | | | | | |
| FKBP5 | 204560_at:224856_at:246403- | 3.41E-05 | 1.2369 | 1.71 | 73.2 | 64-81 |
| | HuGene_st:513199-HuGene_st:69253- | | | | | |
| | HuGene_st:642461-HuGene_st:467598- | | | | | |
| | HuGene_st:1038165-HuGene_st:512075- | | | | | |
| | HuGene_st:224840_at:797941- | | | | | |
| | HuGene_st:633285-HuGene_st:511644- | | | | | |
| | HuGene_st:469540-HuGene_st:209829- | | | | | |
| | HuGene_st:800516-HuGene_st:247764- | | | | | |
| | HuGene_st:468711-HuGene_st:112353- | | | | | |
| | HuGene_st:746630-HuGene_st:337035- | | | | | |
| | HuGene_st:1014328-HuGene_st:292916- | | | | | |
| | HuGene_st | | | | | |
| KCNMB1 | 17348-HuGene_st:414025- | 3.74E-05 | 1.2189 | 2.66 | 72.9 | 63.7-80.8 |
| | HuGene_st:792493-HuGene_st:131717- | | | | | |
| | HuGene_st:569127-HuGene_st:17962- | | | | | |
| | HuGene_st:504462-HuGene_st:758432- | | | | | |
| | HuGene_st:209948_at:772102- | | | | | |
| | HuGene_st:518039-HuGene_st | | | | | |
| VIM | 564251-HuGene_st:201426_s_at:234475- | 4.15E-05 | 1.2225 | 1.86 | 72.9 | 63.7-80.9 |
| | HuGene_st:319318-HuGene_st:339807- | | | | | |
| | HuGene_st:527110-HuGene_st:994975- | | | | | |
| | HuGene_st:837477-HuGene_st:302722- | | | | | |
| | HuGene_st:516025-HuGene_st:1079757- | | | | | |
| | HuGene_st:398387-HuGene_st:155888- | | | | | |
| | HuGene_st:139661-HuGene_st:192324- | | | | | |
| | HuGene_st:1093618-HuGene_st:364983- | | | | | |
| | HuGene_st:436158-HuGene_st:410777- | | | | | |
| | HuGene_st:1555938_x_at | | | | | |
| SORBS2 | 238751_at:805920-HuGene_st | 4.23E-05 | 1.3007 | 2.1 | 74.2 | 65.1-82 |
| C6orf204 | 486932-HuGene_st:573436- | 4.90E-05 | 1.2042 | 2.07 | 72.6 | 63.5-80.6 |
| | HuGene_st:400510- | | | | | |
| | HuGene_st:228202_at:123307- | | | | | |
| | HuGene_st:181714-HuGene_st:206020- | | | | | |
| | HuGene_st:949623-HuGene_st:814579- | | | | | |
| | HuGene_st:515524-HuGene_st:514175- | | | | | |
| | HuGene_st | | | | | |
| FAM55D | 633727-HuGene_st:966568- | 5.67E-05 | 1.1792 | 3.48 | 72.2 | 62.9-80.2 |
| | HuGene_st:625133-HuGene_st:858568- | | | | | |
| | HuGene_st:220645_at:201856- | | | | | |
| | HuGene_st:1013940-HuGene_st:316604- | | | | | |
| | HuGene_st:450702-HuGene_st:1028406- | | | | | |
| | HuGene_st:1090862-HuGene_st:741930- | | | | | |
| | HuGene_st:981211-HuGene_st:970069- | | | | | |
| | HuGene_st:259706-HuGene_st:291282- | | | | | |
| | HuGene_st:56493-HuGene_st:888970- | | | | | |
| | HuGene_st:821065-HuGene_st:53354- | | | | | |
| | HuGene_st:752570-HuGene_st:539086- | | | | | |
| | HuGene_st:204142-HuGene_st | | | | | |
| TIMP2 | 1087828-HuGene_st:231579_s_at:436546- | 6.40E-05 | 1.2298 | 1.82 | 73.1 | 63.8-80.9 |
| | HuGene_st:799495-HuGene_st:563754- | | | | | |
| | HuGene_st:914626-HuGene_st:526357- | | | | | |
| | HuGene_st:203167_at:760416- | | | | | |
| | HuGene_st:651774-HuGene_st:965142- | | | | | |
| | HuGene_st:224540-HuGene_st:690107- | | | | | |
| | HuGene_st:224560_at:319958- | | | | | |
| | HuGene_st:437652-HuGene_st:43390- | | | | | |
| | HuGene_st:727465-HuGene_st:310783- | | | | | |
| | HuGene_st:750028-HuGene_st:957648- | | | | | |
| | HuGene_st:106688-HuGene_st:289430- | | | | | |
| | HuGene_st:740790-HuGene_st | | | | | |
| ELOVL5 | NA | 8.15E-05 | 1.1814 | 2.05 | 72.3 | 63-80.3 |
| ACTA2 | 215787_at:743686- | 1.00E-04 | 1.2318 | 1.81 | 73.1 | 63.9-80.9 |
| | HuGene_st:200974_at:1070983- | | | | | |
| | HuGene_st:120087-HuGene_st:980364- | | | | | |
| | HuGene_st:529704-HuGene_st:855050- | | | | | |
| | HuGene_st:530590-HuGene_st:692252- | | | | | |
| | HuGene_st:404109-HuGene_st:630797- | | | | | |
| | HuGene_st:670805-HuGene_st:293590- | | | | | |
| | HuGene_st:757648-HuGene_st:445454- | | | | | |
| | HuGene_st:479447-HuGene_st:548186- | | | | | |
| | HuGene_st:237159-HuGene_st:90357- | | | | | |
| | HuGene_st:313690-HuGene_st:183075- | | | | | |
| | HuGene_st:785232-HuGene_st | | | | | |
| C1S | 208747_s_at:233042_at:620011- | 0.0001 | 1.2896 | 1.61 | 74 | 64.8-81.9 |
| | HuGene_st:1555229_a_at:245571- | | | | | |
| | HuGene_st:426716-HuGene_st:866948- | | | | | |
| | HuGene_st:705447-HuGene_st:947234- | | | | | |
| | HuGene_st:682561-HuGene_st:332123- | | | | | |
| | HuGene_st | | | | | |
| LOC38776 3 | 1062323-HuGene_st | 0.0001 | 1.1943 | 1.36 | 72.5 | 63.2-80.5 |
| CLU | 222043_at:939366-HuGene_st:832450- | 0.0001 | 1.2285 | 1.42 | 73 | 63.8-81 |
| | HuGene_st:208792_s_at:125360- | | | | | |
| | HuGene_st:216446 | | | | | |
| | HuGene_st:208791_at:1054768- | | | | | |
| | HuGene_st:1034873-HuGene_st:1056536- | | | | | |
| | HuGene_st:787017-HuGene_st:1060096- | | | | | |
| | HuGene_st:591687-HuGene_st:1014914- | | | | | |
| | HuGene_st:213483-HuGene_st:459630- | | | | | |
| | HuGene_st:1036064-HuGene_st:972503- | | | | | |
| | HuGene_st:492827-HuGene_st:485686- | | | | | |
| | HuGene_st | | | | | |
| MT1H | 197989-HuGene_st:589780- | 0.0001 | 1.1885 | 4.55 | 72.4 | 63.1-80.4 |
| | HuGene_st:800631-HuGene_st:854292- | | | | | |
| | HuGene_st:644419-HuGene_st:851562- | | | | | |
| | HuGene_st:590830-HuGene_st:727551- | | | | | |
| | HuGene_st:51225- | | | | | |
| | HuGene_st:206461_x_at:1099945- | | | | | |
| | HuGene_st:334778-HuGene_st:6545- | | | | | |
| | HuGene_st:451479-HuGene_st:621584- | | | | | |
| | HuGene_st:885933-HuGene_st:73199- | | | | | |
| | HuGene_st:882631-HuGene_st | | | | | |
| RGS2 | 331021-HuGene_st:202388_at:895469- | 0.0001 | 1.1617 | 1.94 | 71.9 | 62.6-79.9 |
| | HuGene_st:962521-HuGene_st:843420- | | | | | |
| | HuGene_st:767847-HuGene_st:1022759- | | | | | |
| | HuGene_st:507303-HuGene_st:121934- | | | | | |
| | HuGene_st:1094262-HuGene_st:1067241- | | | | | |
| | HuGene_st:111860-HuGene_st:661918- | | | | | |
| | HuGene_st | | | | | |
| FBN1 | 192231-HuGene_st:794561- | 0.0002 | 1.1368 | 1.93 | 71.5 | 62.2-79.6 |
| | HuGene_st:1094040-HuGene_st:427611- | | | | | |
| | HuGene_st:394963-HuGene_st:828273- | | | | | |
| | HuGene_st:202765_s_at:162818- | | | | | |
| | HuGene_st:169932-HuGene_st:192029- | | | | | |
| | HuGene_st:288955-HuGene_st:600623- | | | | | |
| | HuGene_st:941525-HuGene_st:971683- | | | | | |
| | HuGene_st:82155-HuGene_st:359130- | | | | | |
| | HuGene_st:404656-HuGene_st:921008- | | | | | |
| | HuGene_st:999573-HuGene_st:112593- | | | | | |
| | HuGene_st | | | | | |
| ANKRD25 | 808074-HuGene_st:932564- | 0.0002 | 1.1162 | 1.56 | 71.2 | 61.9-79.3 |
| | HuGene_st:143731-HuGene_st:292383- | | | | | |
| | HuGene_st:393569-HuGene_st:201770- | | | | | |
| | HuGene_st:973796-HuGene_st:824703- | | | | | |
| | HuGene_st:109143-HuGene_st:132685- | | | | | |
| | HuGene_st:698165- | | | | | |
| | HuGene_st:218418_s_at | | | | | |
| MT1G | 226585-HuGene_st:495514-v | 0.0002 | 1.0893 | 2.66 | 70.7 | 61.3-78.8 |
| | HuGene_st:159768-HuGene_st:186270- | | | | | |
| | HuGene_st:855342-HuGene_st:449379- | | | | | |
| | HuGene_st:1100995-HuGene_st:617733- | | | | | |
| | HuGene_st:914660-HuGene_st:884883- | | | | | |
| | HuGene_st:903179-HuGene_st:311881- | | | | | |
| | HuGene_st:977536- | | | | | |
| | HuGene_st:210472_at:1061010- | | | | | |
| | HuGene_st:415267-HuGene_st:1048754- | | | | | |
| | HuGene_st:1011704-HuGene_st:59546- | | | | | |
| | HuGene_st:917923- | | | | | |
| | HuGene_st:204745_x_at | | | | | |
| MYL9 | 201058_s_at:543240-HuGene_st:923797- | 0.0003 | 1.1288 | 2.18 | 71.4 | 62-79.4 |
| | HuGene_st:173220-HuGene_st:874422- | | | | | |
| | HuGene_st:925187-HuGene_st:979054- | | | | | |
| | HuGene_st:676360-HuGene_st:117460- | | | | | |
| | HuGene_st:1092848-HuGene_st:608837- | | | | | |
| | HuGene_st:404703-HuGene_st:50995- | | | | | |
| | HuGene_st:206106-HuGene_st:995028- | | | | | |
| | HuGene_st | | | | | |
| TAGLN | 279405-HuGene_st:205547_s_at:695669- | 0.0003 | 1.0901 | 2.12 | 70.7 | 61.4-78.8 |
| | HuGene_st:902958-HuGene_st:304704- | | | | | |
| | HuGene_st:741240-HuGene_st:898711- | | | | | |
| | HuGene_st:1555724_s_at:26929- | | | | | |
| | HuGene_st:66114-HuGene_st:505391- | | | | | |
| | HuGene_st:613243-HuGene_st:73428- | | | | | |
| | HuGene_st:983072-HuGene_st:543865- | | | | | |
| | HuGene_st:179049-HuGene_st:458482- | | | | | |
| | HuGene_st:823959-HuGene_st:931316- | | | | | |
| | HuGene_st:556028-HuGene_st:565900- | | | | | |
| | HuGene_st:278667- | | | | | |
| | HuGene_st:226523_at:232248_at | | | | | |
| FOXF1 | 21663-HuGene_st:832719- | 0.0004 | 1.1745 | 1.28 | 72.1 | 62.9-80.1 |
| | HuGene_st:174216-HuGene_st:337755- | | | | | |
| | HuGene_st:775422-HuGene_st:205935_at | | | | | |
| LGALS2 | 204754-HuGene_st:455546- | 0.0004 | 1.0851 | 3.07 | 70.6 | 61.2-78.8 |
| | HuGene_st:428640-HuGene_st:228729- | | | | | |
| | HuGene_st:450048-HuGene_st:1087923- | | | | | |
| | HuGene_st:616130-HuGene_st:818575- | | | | | |
| | HuGene_st:699850-HuGene_st:820908- | | | | | |
| | HuGene_st:850596-HuGene_st:751645- | | | | | |
| | HuGene_st:1028381-HuGene_st:667147- | | | | | |
| | HuGene_st:245361-HuGene_st:754620- | | | | | |
| | HuGene_st | | | | | |
| SDC2 | 1019857-HuGene_st:730583- | 0.0006 | 1.099 | 1.39 | 70.9 | 61.5-79 |
| | HuGene_st:33355-HuGene_st:717018- | | | | | |
| | HuGene_st:212158_at:272847- | | | | | |
| | HuGene_st:458585- | | | | | |
| | HuGene_st:212154_at:446134- | | | | | |
| | HuGene_st:1096317-HuGene_st:815650- | | | | | |
| | HuGene_st:590116-HuGene_st:367740- | | | | | |
| | HuGene_st:1074315-HuGene_st | | | | | |
| DNASE1L 3 | 867148-HuGene_st:588394- | 0.0007 | 1.0972 | 1.5 | 70.8 | 61.6-79 |
| | HuGene_st:205554_s_at:616846- | | | | | |
| | HuGene_st:58544-HuGene_st:277847- | | | | | |
| | HuGene_st:18171-HuGene_st:429485- | | | | | |
| | HuGene_st:960168-HuGene_st:981221- | | | | | |
| | HuGene_st:23306-HuGene_st:991254- | | | | | |
| | HuGene_st | | | | | |
| PLEKHC1 | 831786-HuGene_st:88016- | 0.0007 | 1.0318 | 1.82 | 69.7 | 60.3-78 |
| | HuGene_st:1087035-HuGene_st:530331- | | | | | |
| | HuGene_st:209209_s_at:184398- | | | | | |
| | HuGene_st:904923-HuGene_st:193550- | | | | | |
| | HuGene_st:468805-HuGene_st:104495- | | | | | |
| | HuGene_st:604317-HuGene_st:501652- | | | | | |
| | HuGene_st:118096-HuGene_st:321532- | | | | | |
| | HuGene_st:675257-HuGene_st:79496- | | | | | |
| | HuGene_st:590867-HuGene_st:147248- | | | | | |
| | HuGene_st:627402-HuGene_st:377514- | | | | | |
| | HuGene_st | | | | | |
| SERPINF1 | 546682-HuGene_st:814007- | 0.0009 | 1.0012 | 1.47 | 69.2 | 59.8-77.4 |
| | HuGene_st:202283_at:898654- | | | | | |
| | HuGene_st:884794-HuGene_st:264195- | | | | | |
| | HuGene_st:787378-HuGene_st:1036417- | | | | | |
| | HuGene_st | | | | | |
| RARRES2 | 1098138-HuGene_st:515302- | 0.0009 | 1.0325 | 1.6 | 69.7 | 60.3-78 |
| | HuGene_st:849018- | | | | | |
| | HuGene_st:209496_at:741143- | | | | | |
| | HuGene_st:903829-HuGene_st:305131- | | | | | |
| | HuGene_st:715557-HuGene_st:373579- | | | | | |
| | HuGene_st:177871-HuGene_st:106318- | | | | | |
| | HuGene_st:662795-HuGene_st:38090- | | | | | |
| | HuGene_st | | | | | |
| CFL2 | 545956-HuGene_st:833414- | 0.0009 | 1.0592 | 1.75 | 70.2 | 60.8-78.4 |
| | HuGene_st:214239-HuGene_st:192253- | | | | | |
| | HuGene_st:224663_s_at:694174- | | | | | |
| | HuGene_st:233496_s_at:992798- | | | | | |
| | HuGene_st:328584-HuGene_st:950857- | | | | | |
| | HuGene_st:612593-HuGene_st:95686- | | | | | |
| | HuGene_st:177218-HuGene_st:451953- | | | | | |
| | HuGene_st:800232-HuGene_st:575557- | | | | | |
| | HuGene_st:794701-HuGene_st | | | | | |
| RBMS1 | 375424-HuGene_st:87825- | 0.0009 | 1.1417 | 1.55 | 71.6 | 62.3-79.7 |
| | H uGene_st:1046943- | | | | | |
| | HuGene_st:209868_s_at:207266_x_at:225 | | | | | |
| | 269_s_at:238185_at | | | | | |
| SELENBP1 | 914892-HuGene_st:582091- | 0.0009 | 1.0563 | 1.7 | 70.1 | 60.7-78.4 |
| | HuGene_st:643114-HuGene_st:532506- | | | | | |
| | HuGene_st:823159-HuGene_st:171447- | | | | | |
| | HuGene_st:189960-HuGene_st:75763- | | | | | |
| | HuGene_st:834358-HuGene_st:245623- | | | | | |
| | HuGene_st:214433_s_at:233267_at:56418 | | | | | |
| | 6-HuGene_st:637748-HuGene_st:200643- | | | | | |
| | HuGene_st:781415-HuGene_st:208204- | | | | | |
| | HuGene_st:730504-HuGene_st:1008117- | | | | | |
| | HuGene_st:801694-HuGene_st:433986- | | | | | |
| | HuGene_st:183676-HuGene_st:634656- | | | | | |
| | HuGene_st:1087258-HuGene_st | | | | | |
| ENAM | 212592_at | 0.0011 | 1.0044 | 2.58 | 69.2 | 59.9-77.5 |
| MT1F | 987574-HuGene_st:213629_x_at:171840- | 0.0012 | 0.9654 | 2.2 | 68.5 | 59.1-76.9 |
| | HuGene_st:217165_x_at:867979- | | | | | |
| | HuGene_st:354284-HuGene_st:424055- | | | | | |
| | HuGene_st:998184-HuGene_st:616693- | | | | | |
| | HuGene_st:459005-HuGene_st:221141- | | | | | |
| | HuGene_st:338518-HuGene_st:951452- | | | | | |
| | HuGene_st:915306-HuGene_st:506126- | | | | | |
| | HuGene_st:845635-HuGene_st:1048483- | | | | | |
| | HuGene_st:1094672-HuGene_st:576368- | | | | | |
| | HuGene_st:414800-HuGene_st | | | | | |
| CD163 | 620892-HuGene_st:215049_x_at:32161- | 0.0013 | 1.0234 | 1.9 | 69.6 | 60.2-77.9 |
| | HuGene_st:867013-HuGene_st:899841- | | | | | |
| | HuGene_st:662901-HuGene_st:97726- | | | | | |
| | HuGene_st:180883-HuGene_st:706228- | | | | | |
| | HuGene_st:849505-HuGene_st:217692- | | | | | |
| | HuGene_st:566183-HuGene_st:154492- | | | | | |
| | HuGene_st:737668- | | | | | |
| | HuGene_st:203645_s_at:948100- | | | | | |
| | HuGene_st:900552-HuGene_st:288236- | | | | | |
| | HuGene_st | | | | | |
| GUCY1A3 | 560981-HuGene_st:1088572- | 0.0015 | 1.0136 | 2.69 | 69.4 | 60-77.7 |
| | HuGene_st:692186-HuGene_st:648114- | | | | | |
| | HuGene_st:783434- | | | | | |
| | H uGene_st:221942_s_at:1026405- | | | | | |
| | HuGene_st:256104-HuGene_st:965063- | | | | | |
| | HuGene_st:889044-HuGene_st:752296- | | | | | |
| | HuGene_st:239580_at:185668- | | | | | |
| | HuGene_st:120195-HuGene_st:324658- | | | | | |
| | HuGene_st:229530_at | | | | | |
| TNFRSF17 | 1062688-HuGene_st:38682- | 0.0015 | 1.0199 | 2.08 | 69.5 | 60.1-77.7 |
| | HuGene_st:558270-HuGene_st:631025- | | | | | |
| | HuGene_st:24518-HuGene_st:240754- | | | | | |
| | HuGene_st:193693-HuGene_st:255074- | | | | | |
| | HuGene_st:493368-HuGene_st:191710- | | | | | |
| | HuGene_st:610232-HuGene_st:544680- | | | | | |
| | HuGene_st:205576-HuGene_st:738267- | | | | | |
| | HuGene_st:746498-HuGene_st | | | | | |
| CAV1 | 793412-HuGene_st:1007317- | 0.0016 | 1.0215 | 1.58 | 69.5 | 60.1-77.8 |
| | HuGene_st:212097_at:203065_s_at:81283 | | | | | |
| | 2-HuGene_st:505037-HuGene_st:819580- | | | | | |
| | HuGene_st:348883-HuGene_st:659416- | | | | | |
| | HuGene_st:698493-HuGene_st:861877- | | | | | |
| | HuGene_st:518081-HuGene_st:35773- | | | | | |
| | HuGene_st | | | | | |
| CKB | 718720-HuGene_st:769422- | 0.0019 | 0.9741 | 2.27 | 68.7 | 59.2-77 |
| | HuGene_st:581051-HuGene_st:549987- | | | | | |
| | HuGene_st:24890-HuGene_st:1041238- | | | | | |
| | HuGene_st:16603-HuGene_st:480007- | | | | | |
| | HuGene_st:435665-HuGene_st:25470- | | | | | |
| | HuGene_st:40899-HuGene_st:896708- | | | | | |
| | HuGene_st:373774-HuGene_st:963331- | | | | | |
| | HuGene_st:23457-HuGene_st:766369- | | | | | |
| | HuGene_st:200884_at:405417- | | | | | |
| | HuGene_st:888623-HuGene_st:396007- | | | | | |
| | HuGene_st | | | | | |
| WWTR1 | 202132_at:249804-HuGene_st:735408- | 0.002 | 0.9904 | 1.48 | 69 | 59.6-77.3 |
| | HuGene_st:925151-HuGene_st:213564- | | | | | |
| | HuGene_st:561456-HuGene_st:125734- | | | | | |
| | HuGene_st:367743-HuGene_st:153369- | | | | | |
| | HuGene_st:628871-HuGene_st:942493- | | | | | |
| | HuGene_st:949013- | | | | | |
| | HuGene_st:202134_s_at:426662- | | | | | |
| | HuGene_st:586249- | | | | | |
| | HuGene_st:228629_s_at:506281- | | | | | |
| | HuGene_st | | | | | |
| HMGCS2 | 729816-HuGene_st:658722- | 0.0022 | 0.8767 | 2.67 | 66.9 | 57.5-75.5 |
| | HuGene_st:253113-HuGene_st:900260- | | | | | |
| | HuGene_st:253100-HuGene_st:283650- | | | | | |
| | HuGene_st:624250-HuGene_st:439478- | | | | | |
| | HuGene_st:407398-HuGene_st:171889- | | | | | |
| | HuGene_st:397691-HuGene_st:448587- | | | | | |
| | HuGene_st:593619-HuGene_st:371076- | | | | | |
| | HuGene_st:228587-HuGene_st:888308- | | | | | |
| | HuGene_st:538023- | | | | | |
| | HuGene_st:204607_at:43162- | | | | | |
| | HuGene_st:865416-HuGene_st:619083- | | | | | |
| | HuGene_st:789982-HuGene_st:616916- | | | | | |
| | HuGene_st | | | | | |
| MAFB | 876871-HuGene_st:600037- | 0.0034 | 0.9563 | 1.66 | 68.4 | 59-76.8 |
| | HuGene_st:177002-HuGene_st:3125- | | | | | |
| | HuGene_st:1057503-HuGene_st:274807- | | | | | |
| | HuGene_st | | | | | |
| STOM | 201060_x_at:669723-HuGene_st:1099166- | 0.0035 | 1.0176 | 1.21 | 69.5 | 60.1-77.7 |
| | HuGene_st:201061_s_at:22513- | | | | | |
| | HuGene_st:430393-HuGene_st:486266- | | | | | |
| | HuGene_st:47888-HuGene_st | | | | | |
| CD14 | 327635-HuGene_st:201743_at:822666- | 0.0035 | 0.9646 | 1.58 | 68.5 | 59-76.9 |
| | HuGene_st:999890-HuGene_st:753790- | | | | | |
| | HuGene_st:1080129-HuGene_st:532035- | | | | | |
| | HuGene_st:1087885-HuGene_st:528527- | | | | | |
| | HuGene_st:851691-HuGene_st:905038- | | | | | |
| | HuGene_st:437599-HuGene_st:31858- | | | | | |
| | HuGene_st | | | | | |
| MUC4 | 1061780-HuGene_st:43545- | 0.0039 | 1.0448 | 1.2 | 69.9 | 60.5- |
| | HuGene_st:217110_s_at:47402- | | | | | 78.2 |
| | HuGene_st:1042572-HuGene_st:548777- | | | | | |
| | HuGene_st:612341-HuGene_st:711231- | | | | | |
| | HuGene_st:217109_at:278229- | | | | | |
| | HuGene_st:536580- | | | | | |
| | HuGene_st:235055_x_at:743233- | | | | | |
| | HuGene_st:223515- | | | | | |
| | HuGene_st:204895_x_at:670488- | | | | | |
| | HuGene_st:106413-HuGene_st:35359- | | | | | |
| | HuGene_st:344313-HuGene_st:284609- | | | | | |
| | HuGene_st:882714-HuGene_st:512794- | | | | | |
| | HuGene_st | | | | | |
| APOE | 520118-HuGene_st:886930- | 0.0044 | 0.9406 | 1.56 | 68.1 | 58.7- |
| | HuGene_st:771679-HuGene_st:944600- | | | | | 76.6 |
| | HuGene_st:169546-HuGene_st:483757- | | | | | |
| | HuGene_st:16650-HuGene_st:511913- | | | | | |
| | HuGene_st:1099818-HuGene_st:33219- | | | | | |
| | HuGene_st:663513-HuGene_st | | | | | |
| SI | 9605-HuGene_st:514814- | 0.0054 | 0.9299 | 2.79 | 67.9 | 58.5-76.4 |
| | HuGene_st:809314-HuGene_st:576613- | | | | | |
| | HuGene_st:45502-HuGene_st:369676- | | | | | |
| | HuGene_st:438636-HuGene_st:267204- | | | | | |
| | HuGene_st:326716-HuGene_st:897825- | | | | | |
| | HuGene_st:377666-HuGene_st:519273- | | | | | |
| | HuGene_st:325741-HuGene_st:245381- | | | | | |
| | HuGene_st:108368-HuGene_st:464198- | | | | | |
| | HuGene_st:679193-HuGene_st:168180- | | | | | |
| | HuGene_st | | | | | |
| LOC28538 2 | 242447_at | 0.0058 | 0.7422 | 1.26 | 64.5 | 55-73.2 |
| HLA-DRB1 | 971813-HuGene_st | 0.0063 | 1.0668 | 1.51 | 70.3 | 60.9-78.6 |
| ANTXR1 | 241549_at:232956_at | 0.0071 | 0.9507 | 1.29 | 68.3 | 58.8-76.7 |
| FAM46C | 197110-HuGene_st:220306_at:110466- | 0.0085 | 0.9172 | 1.38 | 67.7 | 58.2-76.1 |
| | HuGene_st:988119-HuGene_st:1098424- | | | | | |
| | HuGene_st:560529-HuGene_st:387944- | | | | | |
| | HuGene_st:38645-HuGene_st:221689- | | | | | |
| | HuGene_st:322082-HuGene_st | | | | | |
| TIMP3 | 767021- | 0.0091 | 0.9064 | 1.81 | 67.5 | 58-76 |
| | HuGene_st:201150_s_at:201148_s_at:228 | | | | | |
| | 752-HuGene_st:241221- | | | | | |
| | HuGene_st:201147_s_at:125730- | | | | | |
| | HuGene_st | | | | | |
| LOC28366 6 | 235567_at:236266_at:1562682_at:240951 _at | 0.0099 | 0.7796 | 1.22 | 65.2 | 55.6-73.8 |
| GREM1 | 569365-HuGene_st:407017- | 0.0106 | 0.8579 | 3.03 | 66.6 | 57-75.1 |
| | HuGene_st:218468_s_at:292333- | | | | | |
| | HuGene_st:330742-HuGene_st:546941- | | | | | |
| | HuGene_st:556201-HuGene_st:484435- | | | | | |
| | HuGene_st:326978-HuGene_st:144073- | | | | | |
| | HuGene_st:411208-HuGene_st:1081939- | | | | | |
| | HuGene_st:345681-HuGene_st:2445- | | | | | |
| | HuGene_st:864620-HuGene_st | | | | | |
| FCGBP | 22847-HuGene_st:203240_at:338164- | 0.0121 | 0.7889 | 1.99 | 65.3 | 55.7-74 |
| | HuGene_st:516715-HuGene_st:88809- | | | | | |
| | HuGene_st:948682-HuGene_st:311904- | | | | | |
| | HuGene_st:997435-HuGene_st:841000- | | | | | |
| | HuGene_st:508230-HuGene_st:226428- | | | | | |
| | HuGene_st:634071-HuGene_st:948120- | | | | | |
| | HuGene_st:1079875-HuGene_st:71797- | | | | | |
| | HuGene_st:781083-HuGene_st:426005- | | | | | |
| | HuGene_st:27264-HuGene_st:16108- | | | | | |
| | HuGene_st:63672-HuGene_st:1085208- | | | | | |
| | HuGene_st:708701-HuGene_st | | | | | |
| HSPA2 | 831351-HuGene_st:270747- | 0.0123 | 0.8908 | 1.43 | 67.2 | 57.7-75.7 |
| | HuGene_st:720913-HuGene_st:660812- | | | | | |
| | HuGene_st:939000-HuGene_st:920196- | | | | | |
| | HuGene_st | | | | | |
| COL6A1 | 138366-HuGene_st:528191- | 0.0126 | 0.8452 | 1.45 | 66.4 | 56.9-75 |
| | HuGene_st:884210-HuGene_st:57954- | | | | | |
| | HuGene_st:737256-HuGene_st:858575- | | | | | |
| | H uGene_st:212937_s_at:214200_s_at:190 | | | | | |
| | 064-HuGene_st:216904_at | | | | | |
| SELM | NA | 0.0133 | 0.8596 | 1.32 | 66.6 | 57.2-75.2 |
| FLNA | 296693-HuGene_st:685285- | 0.0157 | 0.7967 | 1.59 | 65.5 | 55.9-74.1 |
| | HuGene_st:704065-HuGene_st:445061- | | | | | |
| | HuGene_st:11735-HuGene_st:1066792- | | | | | |
| | HuGene_st:214752_x_at:213746_s_at:871 | | | | | |
| | 038-HuGene_st:396236- | | | | | |
| | HuGene_st:200859_x_at:786282- | | | | | |
| | HuGene_st:136680-HuGene_st:50466- | | | | | |
| | HuGene_st:955232-HuGene_st:588605- | | | | | |
| | HuGene_st:40025-HuGene_st:1002306- | | | | | |
| | HuGene_st:833338-HuGene_st:86435- | | | | | |
| | HuGene_st:172457-HuGene_st | | | | | |
| COX7A1 | 386303-HuGene_st:354428-HuGene_st | 0.0161 | 0.8613 | 1.32 | 66.7 | 57.2-75.3 |
| EMP3 | 736604-HuGene_st:155271- | 0.0164 | 0.8053 | 1.41 | 65.6 | 56.1-74.2 |
| | HuGene_st:584704-HuGene_st | | | | | |
| ITLN1 | 389198-HuGene_st:496499- | 0.021 | 0.7137 | 2.54 | 63.9 | 54.4-72.7 |
| | HuGene_st:48547-HuGene_st:552029- | | | | | |
| | HuGene_st:126777- | | | | | |
| | HuGene_st:223597_at:281671- | | | | | |
| | HuGene_st:45052-HuGene_st:573120- | | | | | |
| | HuGene_st:137624-HuGene_st:948945- | | | | | |
| | HuGene_st:527196-HuGene_st:358648- | | | | | |
| | HuGene_st:45640-HuGene_st:512550- | | | | | |
| | HuGene_st:839317-HuGene_st:634036- | | | | | |
| | HuGene_st:101083-HuGene_st:857541- | | | | | |
| | HuGene_st:404069-HuGene_st | | | | | |
| C10orf99 | 227735_s_at:563767-HuGene_st:963471- | 0.0218 | 0.724 | 1.76 | 64.1 | 54.7-72.9 |
| | HuGene_st:965947-HuGene_st:1081062- | | | | | |
| | HuGene_st:354432-HuGene_st:602661- | | | | | |
| | HuGene_st:227736_at:1009594- | | | | | |
| | HuGene_st:969063-HuGene_st:77052- | | | | | |
| | HuGene_st:483367-HuGene_st:50662- | | | | | |
| | HuGene_st:738457-HuGene_st:69019- | | | | | |
| | HuGene_st:215415-HuGene_st:1003926- | | | | | |
| | HuGene_st:676577-HuGene_st:676855- | | | | | |
| | HuGene_st:225297-HuGene_st:560897- | | | | | |
| | HuGene_st:886384-HuGene_st:303754- | | | | | |
| | HuGene_st | | | | | |
| CSRP1 | 453033-HuGene_st:673637- | 0.023 | 0.7234 | 1.39 | 64.1 | 54.6-72.9 |
| | HuGene_st:607761-HuGene_st:582630- | | | | | |
| | HuGene_st:654799-HuGene_st:258474- | | | | | |
| | HuGene_st:200621_at:1028504- | | | | | |
| | HuGene_st:802909-HuGene_st:339914- | | | | | |
| | HuGene_st:533385-HuGene_st:256461- | | | | | |
| | HuGene_st:1043113-HuGene_st:72101- | | | | | |
| | HuGene_st:105671-HuGene_st:339691- | | | | | |
| | HuGene_st:108781-HuGene_st:133369- | | | | | |
| | HuGene_st:614016-HuGene_st:629648- | | | | | |
| | HuGene_st:81550-HuGene_st | | | | | |
| PTGER4 | 450915-HuGene_st:1067155- | 0.0237 | 0.8101 | 1.41 | 65.7 | 56.2-74.4 |
| | HuGene_st:812085-HuGene_st:1943- | | | | | |
| | HuGene_st:109314- | | | | | |
| | HuGene_st:204897_at:252442- | | | | | |
| | HuGene_st:800125-HuGene_st:965220- | | | | | |
| | HuGene_st:446764- | | | | | |
| | HuGene_st:204896_s_at:23686- | | | | | |
| | HuGene_st:548266-HuGene_st:392964- | | | | | |
| | HuGene_st:986807-HuGene_st:619166- | | | | | |
| | HuGene st | | | | | |
| TPM2 | 828216-HuGene_st:373854- | 0.026 | 0.7539 | 1.53 | 64.7 | 55.2-73.4 |
| | HuGene_st:981165-HuGene_st:191586- | | | | | |
| | HuGene_st:445912-HuGene_st:446500- | | | | | |
| | HuGene_st:922931-HuGene_st:336603- | | | | | |
| | HuGene_st:272114-HuGene_st:375166- | | | | | |
| | HuGene_st:8388- | | | | | |
| | HuGene_st:204083_s_at:544548- | | | | | |
| | HuGene_st:945443-HuGene_st:628440- | | | | | |
| | HuGene_st:763118-HuGene_st:538951- | | | | | |
| | HuGene st | | | | | |
| PIGR | 748831-HuGene_st:226147_s_at:166398- | 0.0278 | 0.7282 | 1.59 | 64.2 | 54.7-73 |
| | HuGene_st:593192- | | | | | |
| | HuGene_st:204213_at:557727- | | | | | |
| | HuGene_st:229659_s_at:242106- | | | | | |
| | HuGene_st:146237-HuGene_st:883137- | | | | | |
| | HuGene_st:691362-HuGene_st:201991- | | | | | |
| | HuGene_st:1045753-HuGene_st:589879- | | | | | |
| | HuGene_st:1017162-HuGene_st:325979- | | | | | |
| | HuGene_st:105903-HuGene_st:1063932- | | | | | |
| | HuGene_st:207526-HuGene_st:566905- | | | | | |
| | HuGene_st | | | | | |
| HLA-C | 754900-HuGene_st | 0.0279 | 0.9253 | 1.24 | 67.8 | 58.3-76.2 |
| PRNP | 178399-HuGene_st:201300_s_at:386578- | 0.0288 | 0.798 | 1.24 | 65.5 | 56-74.2 |
| | HuGene_st:783439-HuGene_st | | | | | |
| CCL11 | 495934-HuGene_st | 0.0361 | 0.8547 | 1.32 | 66.5 | 57.1-75 |
| C1QC | 137801-HuGene_st:851445-HuGene_st | 0.0391 | 0.7457 | 1.36 | 64.5 | 54.9-73.3 |
| CYR61 | 736150-HuGene_st:385534- | 0.0425 | 0.7875 | 1.47 | 65.3 | 55.7-73.9 |
| | HuGene_st:48239-HuGene_st:242630- | | | | | |
| | HuGene_st | | | | | |
| SERPING1 | NA | 0.0503 | 0.7659 | 1.44 | 64.9 | 55.4-73.6 |
| TYROBP | NA | 0.053 | 0.8176 | 1.17 | 65.9 | 56.3-74.5 |
| GJA1 | NA | 0.0561 | 0.7839 | 1.76 | 65.2 | 55.6-73.9 |
| PTRF | NA | 0.0624 | 0.7354 | 1.5 | 64.3 | 54.8-73.1 |
| CLCA1 | NA | 0.0681 | 0.7855 | 1.61 | 65.3 | 55.8-74 |
| SPARC | NA | 0.0684 | 0.6702 | 1.45 | 63.1 | 53.5-71.9 |
| COL4A2 | NA | 0.0738 | 0.7081 | 1.32 | 63.8 | 54.3-72.5 |
| RAB31 | NA | 0.1041 | 0.8159 | 1.14 | 65.8 | 56.3-74.4 |
| NID1 | NA | 0.105 | 0.7428 | 1.33 | 64.5 | 54.9-73.2 |
| FN1 | NA | 0.1121 | 0.5769 | 1.73 | 61.3 | 51.7-70.2 |
| MSN | NA | 0.1499 | 0.7215 | 1.15 | 64.1 | 54.6-72.9 |
| MMP2 | NA | 0.1755 | 0.6219 | 1.24 | 62.2 | 52.5-71.2 |
| COL1A2 | NA | 0.1891 | 0.65 | 1.9 | 62.7 | 53.2-71.6 |
| FOSB | NA | 0.2076 | 0.6389 | 2.17 | 62.5 | 52.9-71.4 |
| HBB | NA | 0.2357 | 0.6434 | 1.28 | 62.6 | 53-71.5 |
| POU2AF1 | NA | 0.2389 | 0.6564 | 1.18 | 62.9 | 53.3-71.7 |
| COL3A1 | NA | 0.2551 | 0.5538 | 1.35 | 60.9 | 51.3-69.9 |
| SMOC2 | NA | 0.2701 | 0.6533 | 1.34 | 62.8 | 53.2-71.7 |
| MUC2 | NA | 0.2802 | 0.8523 | 1.22 | 66.5 | 57-75 |
| Sep-06 | NA | 0.3109 | 0.5713 | 1.21 | 61.2 | 51.7-70.2 |
| LUM | NA | 0.3157 | 0.5442 | 1.54 | 60.7 | 51.1-69.7 |
| C1QB | NA | 0.3668 | 0.5005 | 1.24 | 59.9 | 50.4-69 |
| TGFB1I1 | NA | 0.4093 | 0.4951 | 1.35 | 59.8 | 50.1-68.8 |
| ALDH1A1 | NA | 0.4232 | 0.354 | 1.4 | 57 | 47.4-66.2 |
| LRIG1 | NA | 0.4614 | 0.5998 | 1.26 | 61.8 | 52.2-70.7 |
| C8orf4 | NA | 0.475 | 0.483 | 1.44 | 59.5 | 49.9-68.5 |
| EGR1 | NA | 0.4851 | 0.5374 | 1.38 | 60.6 | 51-69.6 |
| LOC65274 5 | NA | 0.4864 | 0.1722 | 1.09 | 53.4 | 43.8-62.8 |
| IGKV1D-13 | NA | 0.5177 | 0.1597 | 1.1 | 53.2 | 43.7-62.5 |
| COL15A1 | NA | 0.6601 | 0.54 | 1.22 | 60.6 | 51-69.6 |
| IGFBP5 | NA | 0.7663 | 0.3504 | 1.25 | 57 | 47.4-66.3 |
| LGALS1 | NA | 0.8555 | 0.4626 | 1.16 | 59.1 | 49.5-68.3 |
| COL6A3 | NA | 0.8739 | 0.4412 | 1.2 | 58.7 | 49.1-67.9 |
| HLA-DQA1 | NA | 0.9084 | 0.5417 | 1.28 | 60.7 | 51.1-69.6 |
| HLA-DRA | NA | 0.9084 | 0.5391 | 1.28 | 60.6 | 51-69.6 |
| ST6GALN AC1 | NA | 0.9452 | 0.2594 | 1.15 | 55.2 | 45.6-64.5 |
| HLA-DPA1 | NA | 0.9664 | 0.4166 | 1.39 | 58.3 | 48.7-67.4 |
| IGLV1-44 | NA | 0.9777 | 0.2865 | 1.07 | 55.7 | 46.1-64.9 |
| TNC | NA | 0.9905 | 0.3682 | 1.09 | 57.3 | 47.7-66.5 |
| HBA1 | NA | 0.9979 | 0.47 | 1.21 | 59.3 | 49.6-68.4 |
| HBA2 | NA | 0.9979 | 0.4705 | 1.21 | 59.3 | 49.6-68.3 |

**TABLE 3**

| **TargetPS** | **Symbol** | **Signif. FDR** | **D.val5** | **FC** | **Sens-Spec** | **CI (95)** |
|---|---|---|---|---|---|---|
| 230788_at | SPTLC3:GCNT2 | 2.1587E-27 | 3.9562 | 13.36 | 97.6 | 94.2-99.2 |
| 228706_s_at | CLDN23 | 4.2694E-20 | 3.0727 | 3.47 | 93.8 | 88.3-97.1 |
| 231120_x_at | PKIB | 5.9397E-20 | 3.1191 | 3.28 | 94.1 | 88.6-97.3 |
| 224412_s_at | TRPM6 | 2.2514E-19 | 3.0115 | 7.5 | 93.4 | 87.7-96.8 |
| 220037_s_at | XLKD1 | 3.5991E-19 | 2.9405 | 7.44 | 92.9 | 87-96.5 |
| 209613_s_at | ADH1B:ADH1A | 4.6957E-19 | 3.0158 | 4.67 | 93.4 | 87.7-96.9 |
| 204719_at | ABCA8 | 1.2559E-18 | 2.9171 | 6.82 | 92.8 | 86.8-96.5 |
| 231773_at | ANGPTL1 | 1.4172E-17 | 2.7946 | 4.64 | 91.9 | 85.6-95.9 |
| 225575_at | LIFR | 2.4532E-17 | 2.7797 | 6.36 | 91.8 | 85.5-95.8 |
| 220812_s_at | HHLA2 | 3.4739E-17 | 2.6718 | 10.02 | 90.9 | 84.4-95.2 |
| 211549_s_at | HPGD | 3.6502E-17 | 2.8451 | 3.21 | 92.3 | 86.1-96.1 |
| 202920_at | ANK2 | 2.5934E-16 | 2.6488 | 6.76 | 90.7 | 84.2-95.1 |
| 231925_at | P2RY1 | 1.4091E-15 | 2.5329 | 3.6 | 89.7 | 82.8-94.4 |
| 207080_s_at | PYY | 3.5022E-15 | 2.4783 | 11.71 | 89.2 | 82.2-94 |
| 228885_at | RPL24:LOC731365 | 1.2312E-14 | 2.5575 | 1.63 | 90 | 83.1-94.6 |
| 228766_at | CD36 | 6.2677E-14 | 2.4687 | 1.65 | 89.1 | 82.1-94 |
| 204931_at | TCF21 | 2.5147E-13. | 2.3036 | 2.34 | 87.5 | 80.1-92.8 |
| 205433_at | BCHE | 3.1756E-13 | 2.2538 | 6.21 | 87 | 79.5-92.5 |
| 207980_s_at | CITED2 | 5.2905E-13 | 2.2709 | 1.64 | 87.2 | 79.7-92.6 |
| 209170_s_at | GPM6B | 4.0429E-12 | 2.1588 | 3.59 | 86 | 78.1-91.6 |
| 220376_at | LRRC19 | 6.7548E-12 | 2.0763 | 4.51 | 85 | 77.1-90.9 |
| 228504_at | No Symbol | 9.1926E-12 | 2.152 | 1.61 | 85.9 | 78.2-91.6 |
| 228854_at | No Symbol | 9.1926E-12 | 2.1538 | 1.61 | 85.9 | 78.2-91.6 |
| 235146_at | No Symbol | 9.1926E-12 | 2.1537 | 1.61 | 85.9 | 78.2-91.6 |
| 221305_s_at | UGT1A10:UGT1A7:UGT1A8:UGT1 A1:UGT1A9:UGT1A6:UGT1A5;UG T1A3:UGT1A4 | 1.0319E-11 | 2.0677 | 4.56 | 84.9 | 77.1-90.9 |
| 203881_s_at | DMD | 5.0063E-11 | 2.0137 | 4.74 | 84.3 | 76.3-90.4 |
| 206637_at | P2RY14 | 1.3104E-10 | 1.9836 | 2 | 83.9 | 75.8-90.1 |
| 214598_at | CLDN8 | 2.9225E-10 | 1.8624 | 12.96 | 82.4 | 74.1-88.9 |
| 227529_s_at | AKAP12 | 7.4077E-09 | 1.7434 | 2.83 | 80.8 | 72.3-87.5 |
| 219948_x_at | LOC642329:UGT2A3 | 8.638E-09 | 1.741 | 4.89 | 80.8 | 72.3-87.6 |
| 222717_at | SDPR | 9.6207E-08 | 1.5993 | 2.21 | 78.8 | 70.1-85.9 |
| 229831_at | CNTN3 | 1.1953E-07 | 1.6145 | 3.93 | 79 | 70.3-86.1 |
| 204940_at | PLN | 4.1416E-07 | 1.5439 | 2.01 | 78 | 69.2-85.2 |
| 227827_at | SORBS2 | 4.2323E-05 | 1.3016 | 2.1 | 74.2 | 65.1-81.9 |
| 238751_at | SORBS2 | 4.2323E-05 | 1.3015 | 2.1 | 74.2 | 65.1-82 |
| 209209_s_at | PLEKHC1 | 0.0007 | 1.0304 | 1.82 | 69.7 | 60.3-78 |
| 206664_at | SI | 0.0054 | 0.9301 | 2.79 | 67.9 | 58.4-76.3 |

**TABLE 4**

| **Gene Symbol** | **ValidPS_DOWN** | **Signif. FDR** | **D.val 5** | **FC** | **Sens-Spec** | **CI (95)** |
|---|---|---|---|---|---|---|
| ANK2 | 314086-HuGene_st:282874-HuGene_st:382431- | 2.59E-16 | 2.6515 | 6.76 | 90.8 | 84.1-95.1 |
| | HuGene_st:229308-HuGene_st:779600- | | | | | |
| | HuGene_st:297624-HuGene_st:385943- | | | | | |
| | HuGene_st:730140-HuGene_st:442277- | | | | | |
| | HuGene_st:699309-HuGene_st:182816- | | | | | |
| | HuGene_st:202921_s_at:799860- | | | | | |
| | HuGene_st:868462-HuGene_st:634421- | | | | | |
| | HuGene_st:571536-HuGene_st:1050903- | | | | | |
| | HuGene_st:649509-HuGene_st:239935- | | | | | |
| | HuGene_st:202920_at | | | | | |
| DMD | 945600-HuGene_st:12596-HuGene_st:434657- | 5.01E-11 | 2.0162 | 4.74 | 84.3 | 76.3-90.4 |
| | HuGene_st:676962-HuGene_st:1020969- | | | | | |
| | HuGene_st:170680-HuGene_st:855670- | | | | | |
| | HuGene_st:909848-HuGene_st:134644- | | | | | |
| | HuGene_st:887814-HuGene_st:649184- | | | | | |
| | HuGene_st:110990-HuGene_st:914217- | | | | | |
| | HuGene_st:961622-HuGene_st:514011- | | | | | |
| | HuGene_st:842792-HuGene_st:445483- | | | | | |
| | HuGene_st:208086_s_at:987362- | | | | | |
| | HuGene_st:997308-HuGene_st | | | | | |
| ABCA8 | 57305-HuGene_st:111752-HuGene_st:61357- | 1.26E-18 | 2.9182 | 6.82 | 92.8 | 86.8-96.5 |
| | HuGene_st:123451-HuGene_st:740746- | | | | | |
| | HuGene_st:512280-HuGene_st:389185- | | | | | |
| | HuGene_st:427721-HuGene_st:346028- | | | | | |
| | HuGene_st:224566-HuGene_st:149653- | | | | | |
| | HuGene_st:680699-HuGene_st:76772- | | | | | |
| | HuGene_st:742091-HuGene_st:423333- | | | | | |
| | HuGene_st:559944-HuGene_st:341399- | | | | | |
| | HuGene_st:1565780_at:863400- | | | | | |
| | HuGene_st:921748-HuGene_st:623719- | | | | | |
| | HuGene_st:204719_at:123143- | | | | | |
| | HuGene_st:1077391-HuGene_st | | | | | |
| TCF21 | 804657-HuGene_st:106365-HuGene_st:710514- | 2.51E-13 | 2.3037 | 2.34 | 87.5 | 80.1-92.9 |
| | HuGene_st:299556-HuGene_st:608149- | | | | | |
| | HuGene_st:242233-HuGene_st:655881- | | | | | |
| | HuGene_st:356773-HuGene_st:788445- | | | | | |
| | HuGene_st:709897-HuGene_st:605488- | | | | | |
| | HuGene_st:652466-HuGene_st:204931_at:830709- | | | | | |
| | HuGene_st:273418-HuGene_st:1004754- | | | | | |
| | HuGene_st:990861-HuGene_st:238739- | | | | | |
| | HuGene_st:836241-HuGene_st:110045- | | | | | |
| | HuGene_st:229529_at:1001969-HuGene_st | | | | | |
| PLN | 204938_s_at:418214-HuGene_st:769837- | 4.14E-07 | 1.5399 | 2.01 | 77.9 | 69.2-85.2 |
| | HuGene_st:603329-HuGene_st:1048096- | | | | | |
| | HuGene_st:944510-HuGene_st:430402- | | | | | |
| | HuGene_st:86633-HuGene_st:795093- | | | | | |
| | HuGene_st:377757-HuGene_st:770145- | | | | | |
| | HuGene_st:920628-HuGene_st:594048- | | | | | |
| | HuGene_st:800583- | | | | | |
| | HuGene_st:204939_s_at:556723- | | | | | |
| | HuGene_st:818339-HuGene_st:175524- | | | | | |
| | HuGene_st:498924-HuGene_st:204940_at | | | | | |
| BCHE | 228090-HuGene_st:752051-HuGene_st:800167- | 3.18E-13 | 2.2544 | 6.21 | 87 | 79.4-92.5 |
| | HuGene_st:221362-HuGene_st:155900- | | | | | |
| | HuGene_st:717363-HuGene_st:536584- | | | | | |
| | HuGene_st:146780-HuGene_st:302487- | | | | | |
| | HuGene_st:508472-HuGene_st:516293- | | | | | |
| | HuGene_st:968992-HuGene_st:666625- | | | | | |
| | HuGene_st:923158-HuGene_st | | | | | |
| P2RY14 | 528057-HuGene_st:780310-HuGene_st:235178- | 1.31E-10 | 1.9805 | 2 | 83.9 | 75.9-90 |
| | HuGene_st:352954-HuGene_st:699489- | | | | | |
| | HuGene_st:38001-HuGene_st:637791- | | | | | |
| | HuGene_st:25606-HuGene_st:40647- | | | | | |
| | HuGene_st:896487-HuGene_st:672149- | | | | | |
| | HuGene_st:863820-HuGene_st:352427- | | | | | |
| | HuGene_st:632821-HuGene_st:116148- | | | | | |
| | HuGene_st:561792-HuGene_st:840910- | | | | | |
| | HuGene_st:296420-HuGene_st:974643- | | | | | |
| | HuGene_st:206637_at | | | | | |
| PYY | 656845-HuGene_st:816022-HuGene_st:633572- | 3.50E-15 | 2.4828 | 11.71 | 89.3 | 82.2-94 |
| | HuGene_st:20355-HuGene_st:240779- | | | | | |
| | HuGene_st:638358-HuGene_st:879780- | | | | | |
| | HuGene_st:207080_s_at:211253_x_at:368591- | | | | | |
| | HuGene_st | | | | | |
| CITED2 | 125201-HuGene_st:410723-HuGene_st:463405- | 5.29E-13 | 2.269 | 1.64 | 87.2 | 79.7-92.6 |
| | HuGene_st:401168-HuGene_st:1012057- | | | | | |
| | HuGene_st:235057-HuGene_st:361772- | | | | | |
| | HuGene_st:207980_s_at:1091907- | | | | | |
| | HuGene_st:985355-HuGene_st:175990- | | | | | |
| | HuGene_st:227287_at:48433- | | | | | |
| | HuGene_st:209357_at:477746-HuGene_st:243264- | | | | | |
| | HuGene_st:904401-HuGene_st:328536 | | | | | |
| | HuGene_st:1095110-HuGene_st:89784- | | | | | |
| | HuGene_st:206734-HuGene_st:927615-HuGene_st | | | | | |
| GPM6B | 599862-HuGene_st:754598-HuGene_st:503642- | 4.04E-12 | 2.1547 | 3.59 | 85.9 | 78.2-91.7 |
| | HuGene_st:224935-HuGene_st:754449- | | | | | |
| | HuGene_st:577489-HuGene_st:1073242- | | | | | |
| | HuGene_st:560873-HuGene_st:1003662- | | | | | |
| | HuGene_st:430217-HuGene_st:903323- | | | | | |
| | HuGene_st:231962-HuGene_st:244945- | | | | | |
| | HuGene_st:562460-HuGene_st:583561- | | | | | |
| | HuGene_st:209168_at | | | | | |
| PLEKH | 831786-HuGene_st:88016-HuGene_st:1087035- | 7.00E-04 | 1.0307 | 1.82 | 69.7 | 60.4-78 |
| C1 | HuGene_st:530331- | | | | | |
| | HuGene_st:209209_s_at:184398- | | | | | |
| | HuGene_st:904923-HuGene_st:193550- | | | | | |
| | HuGene_st:468805-HuGene_st:104495- | | | | | |
| | HuGene_st:604317-HuGene_st:501652- | | | | | |
| | HuGene_st:118096-HuGene_st:321532- | | | | | |
| | HuGene_st:675257-HuGene_st:79496- | | | | | |
| | HuGene_st:590867-HuGene_st:147248- | | | | | |
| | HuGene_st:627402-HuGene_st:377514-HuGene_st | | | | | |
| ADH1B | 1078343-HuGene_st:512808-HuGene_st:614446- | 4.70E-19 | 3.0174 | 4.67 | 93.4 | 87.7-96.9 |
| | HuGene_st:910188-HuGene_st:422504- | | | | | |
| | HuGene_st:731361- | | | | | |
| | HuGene_st:209612_s_at:258079- | | | | | |
| | HuGene_st:568239-HuGene_st:879930- | | | | | |
| | HuGene_st:420417-HuGene_st:1025048- | | | | | |
| | HuGene_st:908335-HuGene_st:654633- | | | | | |
| | HuGene_st:947292-HuGene_st:1087125- | | | | | |
| | HuGene_st:1004870- | | | | | |
| | HuGene_st:209613_s_at:579636- | | | | | |
| | HuGene_st:681018-HuGene_st:822774-HuGene_st | | | | | |
| XLKD1 | 520080-HuGene_st:1091117-HuGene_st:943125- | 3.60E-19 | 2.9461 | 7.44 | 93 | 87.1-96.6 |
| | HuGene_st:444068-HuGene_st:648558- | | | | | |
| | HuGene_st:346991-HuGene_st:1006205- | | | | | |
| | HuGene_st:373107-HuGene_st:682535- | | | | | |
| | HuGene_st:1083245-HuGene_st:863143- | | | | | |
| | HuGene_st:820120-HuGene_st:1044561- | | | | | |
| | HuGene_st:220037_s_at:541228- | | | | | |
| | HuGene_st:220256-HuGene_st:289122- | | | | | |
| | HuGene_st:219059_s_at:246683- | | | | | |
| | HuGene_st:775976-HuGene_st:207399- | | | | | |
| | HuGene_st:1052557-HuGene_st:92121-HuGene_st | | | | | |
| LRRC19 | 177641-HuGene_st:1070020-HuGene_st:1055140- | 6.75E-12 | 2.0779 | 4.51 | 85.1 | 77.1-91 |
| | HuGene_st:525999-HuGene_st:937256- | | | | | |
| | HuGene_st:620791-HuGene_st:891251- | | | | | |
| | HuGene_st:707559-HuGene_st:892056- | | | | | |
| | HuGene_st:764919-HuGene_st:382143- | | | | | |
| | HuGene_st:52584-HuGene_st:920414- | | | | | |
| | HuGene_st:1028155-HuGene_st:755055- | | | | | |
| | HuGene_st:678651-HuGene_st:1080156- | | | | | |
| | HuGene_st:530282-HuGene_st:523877- | | | | | |
| | HuGene_st:335198-HuGene_st:787709- | | | | | |
| | HuGene_st:153175-HuGene_st:220376_at | | | | | |
| SDPR | 878908-HuGene_st:781527-HuGene_st:331976- | 9.62E-08 | 1.6036 | 2.21 | 78.9 | 70-85.9 |
| | HuGene_st:238150-HuGene_st:306039- | | | | | |
| | HuGene_st:535903-HuGene_st:302361- | | | | | |
| | HuGene_st:1005813-HuGene_st:71118- | | | | | |
| | HuGene_st:992629- | | | | | |
| | HuGene_st:218711_s_at:293110- | | | | | |
| | HuGene_st:779040-HuGene_st:222717_at:970479- | | | | | |
| | HuGene_st:581654-HuGene_st | | | | | |
| TRPM6 | 767074-HuGene_st:695352-HuGene_st:411125- | 2.25E-19 | 3.0111 | 7.5 | 93.4 | 87.7-96.8 |
| | HuGene_st:221102_s_at:234864_s_at:240389_at:3 | | | | | |
| | 58229-HuGene_st:755964-HuGene_st:840301 | | | | | |
| | HuGene_st:959234-HuGene_st:782639- | | | | | |
| | HuGene_st:833079-HuGene_st:1066034- | | | | | |
| | HuGene_st:678013-HuGene_st:249083- | | | | | |
| | HuGene_st:143934-HuGene_st:159130- | | | | | |
| | HuGene_st:486486-HuGene_st:185057- | | | | | |
| | HuGene_st:878793-HuGene_st:133981- | | | | | |
| | HuGene_st:224412_s_at:202194-HuGene_st | | | | | |
| LIFR | 275506-HuGene_st:323055-HuGene_st:444251- | 2.45E-17 | 2.7814 | 6.36 | 91.8 | 85.5-95.8 |
| | HuGene_st:1056178-HuGene_st:398104- | | | | | |
| | HuGene_st:917434-HuGene_st:1044918- | | | | | |
| | HuGene_st:167500-HuGene_st:423760- | | | | | |
| | HuGene_st:837336-HuGene_st:321505- | | | | | |
| | HuGene_st:918321-HuGene_st:252278- | | | | | |
| | HuGene_st:884504-HuGene_st:124845- | | | | | |
| | HuGene_st:499777-HuGene_st:969722- | | | | | |
| | HuGene_st:709439-HuGene_st:611505- | | | | | |
| | HuGene_st:227771_at:287217- | | | | | |
| | HuGene_st:205876_at:225571_at:229185_at:23336 | | | | | |
| | 7_at:1093011-HuGene_st | | | | | |
| AKAP12 | 212419-HuGene_st:231067_s_at:379659- | 7.41E-09 | 1.7456 | 2.83 | 80.9 | 72.4-87.6 |
| | HuGene_st:1010338-HuGene_st:1075094- | | | | | |
| | HuGene_st:42401-HuGene_st:522584- | | | | | |
| | HuGene_st:480972-HuGene_st:948623- | | | | | |
| | HuGene_st:701945-HuGene_st:276784- | | | | | |
| | HuGene_st:64858- | | | | | |
| | HuGene_st:210517_s_at:874382- | | | | | |
| | HuGene_st:909976-HuGene_st:182037- | | | | | |
| | HuGene_st:417182-HuGene_st:722881-HuGene_st | | | | | |
| CLDN23 | 403960-HuGene_st:25144-HuGene_st:947653- | 4.27E-20 | 3.0748 | 3.47 | 93.8 | 88.2-97.1 |
| | HuGene_st:228704_s_at:228706_s_at:320375- | | | | | |
| | HuGene_st:441629-HuGene_st:367414- | | | | | |
| | HuGene_st:855269-HuGene_st:228707_at:788659- | | | | | |
| | HuGene_st:698816-HuGene_st:95789- | | | | | |
| | HuGene_st:270197-HuGene_st:472976- | | | | | |
| | HuGene_st:280539-HuGene_st:1056334- | | | | | |
| | HuGene_st:516288-HuGene_st:579963-HuGene_st | | | | | |
| CD36 | 392196-HuGene_st:274514-HuGene_st:477005- | 6.27E-14 | 2.4696 | 1.65 | 89.2 | 82.1-94 |
| | HuGene_st:691585-HuGene_st:872909- | | | | | |
| | HuGene_st:543050-HuGene_st:603343- | | | | | |
| | HuGene_st:514557-HuGene_st:296850- | | | | | |
| | HuGene_st:945913-HuGene_st:495755- | | | | | |
| | HuGene_st:206488_s_at:1035854- | | | | | |
| | HuGene_st:887301-HuGene_st:836370- | | | | | |
| | HuGene_st:209555_s_at:939919- | | | | | |
| | HuGene_st:507440-HuGene_st:151788- | | | | | |
| | HuGene_st:146280-HuGene_st:360545- | | | | | |
| | HuGene_st:1051486- | | | | | |
| | HuGene_st:228766_at:512885-HuGene_st | | | | | |
| RPL24 | 1559655_at:1559656_a_at:228885_at | 1.23E-14 | 2.5568 | 1.63 | 89.9 | 83.1-94.6 |
| GCNT2 | 935239-HuGene_st:225205-HuGene_st:1026280- | 2.16E-27 | 3.9536 | 13.36 | 97.6 | 94.1-99.2 |
| | HuGene_st:668101-HuGene_st:1099985- | | | | | |
| | HuGene_st:698568-HuGene_st:134540- | | | | | |
| | HuGene_st:697147-HuGene_st:250092- | | | | | |
| | HuGene_st:611927-HuGene_st:972833- | | | | | |
| | HuGene_st:168891-HuGene_st:990860- | | | | | |
| | HuGene_st:109287-HuGene_st:322116- | | | | | |
| | HuGene_st:231019-HuGene_st:211020_at:959570- | | | | | |
| | HuGene_st:858764-HuGene_st:215593_at:820195- | | | | | |
| | HuGene_st:239606_at:41059-HuGene_st:669940- | | | | | |
| | HuGene_st:215595 x_at:230788_at | | | | | |
| PKIB | 866170-HuGene_st:1055812-HuGene_st:264946- | 5.94E-20 | 3.1204 | 3.28 | 94.1 | 88.6-97.3 |
| | HuGene_st:684057-HuGene_st:124791- | | | | | |
| | HuGene_st:134561-HuGene_st:1026756- | | | | | |
| | HuGene_st:468593-HuGene_st:1045852- | | | | | |
| | HuGene_st:939917-HuGene_st:110205- | | | | | |
| | HuGene_st:660721-HuGene_st:905229- | | | | | |
| | HuGene_st:223551_at:610426-HuGene_st | | | | | |
| ANGPT | 572942-HuGene_st:891312-HuGene_st:953040- | 1.42E-17 | 2.7964 | 4.64 | 91.9 | 85.6-95.9 |
| L1 | HuGene_st:232844-HuGene_st:145730- | | | | | |
| | HuGene_st:142205-HuGene_st:227771- | | | | | |
| | HuGene_st:80584-HuGene_st:982090- | | | | | |
| | HuGene_st:999640-HuGene_st:672931- | | | | | |
| | HuGene_st:148578- | | | | | |
| | HuGene_st:224339_s_at:1046706- | | | | | |
| | HuGene_st:239183_at:155600-HuGene_st:284674- | | | | | |
| | HuGene_st:231773_at:818064-HuGene_st:978991- | | | | | |
| | HuGene_st:728775-HuGene_st | | | | | |
| SI | 9605-HuGene_st:514814-HuGene_st:809314- | 5.40E-03 | 0.9299 | 2.79 | 67.9. | 58.4-76.4 |
| | HuGene_st:576613-HuGene_st:45502- | | | | | |
| | HuGene_st:369676-HuGene_st:438636- | | | | | |
| | HuGene_st:267204-HuGene_st:326716- | | | | | |
| | HuGene_st:897825-HuGene_st:377666- | | | | | |
| | HuGene_st:519273-HuGene_st:325741- | | | | | |
| | HuGene_st:245381-HuGene_st:108368- | | | | | |
| | HuGene_st:464198-HuGene_st:679193- | | | | | |
| | HuGene_st:168180-HuGene_st | | | | | |
| HPGD | 291863-HuGene_st:375608-HuGene_st:793406- | 3.65E-17 | 2.8462 | 3.21 | 92.3 | 86.2-96.1 |
| | HuGene_st:436293-HuGene_st:75568- | | | | | |
| | HuGene_st:211549_s_at:684728- | | | | | |
| | HuGene_st:674596-HuGene_st:527856- | | | | | |
| | HuGene_st:329920-HuGene_st:748432- | | | | | |
| | HuGene_st:259392-HuGene_st:769902- | | | | | |
| | HuGene_st:620673-HuGene_st:450707- | | | | | |
| | HuGene_st:203913_s_at:304752- | | | | | |
| | HuGene_st:447604-HuGene_st:170968- | | | | | |
| | HuGene_st:852359-HuGene_st:836377- | | | | | |
| | HuGene_st:242733_at:243846-HuGene_st:136281- | | | | | |
| | HuGene_st:203914_x_at:211548_s_at:288252- | | | | | |
| | HuGene_st | | | | | |
| CLDN8 | 1018006-HuGene_st:190634-HuGene_st:590280- | 2.92E-10 | 1.8639 | 12.96 | 82.4 | 74.1-88.9 |
| | HuGene_st:186468-HuGene_st:954438- | | | | | |
| | HuGene_st:428391-HuGene_st:480543- | | | | | |
| | HuGene_st:944337-HuGene_st:179725- | | | | | |
| | HuGene_st:508584-HuGene_st:1009114- | | | | | |
| | HuGene_st:948216-HuGene_st:658285- | | | | | |
| | HuGene_st:1022600-HuGene_st:737498- | | | | | |
| | HuGene_st:470015-HuGene_st:103315- | | | | | |
| | HuGene_st:699348-HuGene_st:89877- | | | | | |
| | HuGene_st:56937-HuGene_st:862663- | | | | | |
| | HuGene_st:504945-HuGene_st:214598_at | | | | | |
| UGT2A3 | 149647-HuGene_st:860083-HuGene_st:922544- | 8.64E-09 | 1.7422 | 4.89 | 80.8 | 72.3-87.5 |
| | HuGene_st:244206-HuGene_st:503323- | | | | | |
| | HuGene_st:353576-HuGene_st:603619- | | | | | |
| | HuGene_st:787458-HuGene_st:219796- | | | | | |
| | HuGene_st:333564-HuGene_st:257402- | | | | | |
| | HuGene_st:366699-HuGene_st:461685- | | | | | |
| | HuGene_st:891681-HuGene_st:644952- | | | | | |
| | HuGene_st:621618-HuGene_st:737617- | | | | | |
| | HuGene_st:88682-HuGene_st:529761- | | | | | |
| | HuGene_st:895203-HuGene_st:658594- | | | | | |
| | HuGene_st:455115-HuGene_st | | | | | |
| HHLA2 | 371335-HuGene_st:978721-HuGene_st:1065567- | 3.47E-17 | 2.6743 | 10.02 | 90.9 | 84.3-95.2 |
| | HuGene_st:282548-HuGene_st:240410- | | | | | |
| | HuGene_st:170899-HuGene_st:947848- | | | | | |
| | HuGene_st:438234- | | | | | |
| | HuGene_st:220812_s_at:927495- | | | | | |
| | HuGene_st:351364-HuGene_st:234673_at:993142- | | | | | |
| | HuGene_st:1009637-HuGene_st:335000- | | | | | |
| | HuGene_st:285313-HuGene_st:533646- | | | | | |
| | HuGene_st:234624_at:458597-HuGene_st:104838- | | | | | |
| | HuGene_st:26687-HuGene_st:258409- | | | | | |
| | HuGene_st:493304-HuGene_st:378019- | | | | | |
| | HuGene_st:576796-HuGene_st | | | | | |
| UGT1A8 | 116025-HuGene_st:6488-HuGene_st:881135- | 1.32E-11 | 2.0703 | 4.56 | 85 | 77.1-90.9 |
| | HuGene_st:230953_at:221304_at:221305_s_at:511 | | | | | |
| | 516-HuGene_st:594963-HuGene_st:396121 | | | | | |
| | HuGene_st:123777-HuGene_st:1016481- | | | | | |
| | HuGene_st:204532_x_at:683377- | | | | | |
| | HuGene_st:1055169-HuGene_st:1035103- | | | | | |
| | HuGene_st:1088102- | | | | | |
| | HuGene_st:207126_x_at:42874- | | | | | |
| | HuGene_st:215125_s_at:206094_x_at:208596_s_at:97211-HuGene_st:1009861-HuGene_st:603368- | | | | | |
| | HuGene_st:232654_s_at:625897- | | | | | |
| | HuGene_st:998604-HuGene_st | | | | | |
| CNTN3 | 267567-HuGene_st:782053-HuGene_st:550157- | 1.20E-07 | 1.6155 | 3.93 | 79 | 70.3-86.1 |
| | HuGene_st:541394-HuGene_st:989173- | | | | | |
| | HuGene_st:15899-HuGene_st:78622- | | | | | |
| | HuGene_st:339738-HuGene_st:585282- | | | | | |
| | HuGene_st:661814-HuGene_st:360715- | | | | | |
| | HuGene_st:695033-HuGene_st:483058- | | | | | |
| | HuGene_st:555394-HuGene_st:315011- | | | | | |
| | HuGene_st:905374-HuGene_st:1067212- | | | | | |
| | HuGene_st:557263-HuGene_st:233502_at:811729- | | | | | |
| | HuGene_st:87414-HuGene_st | | | | | |
| P2RY1 | S28249-HuGene_st:113454-HuGene_st:627857- | 1.41E-15 | 2.5326 | 3.6 | 89.7 | 82.8-94.4 |
| | HuGene_st:461281-HuGene_st:207455_at:259065- | | | | | |
| | HuGene_st:797734-HuGene_st:135788- | | | | | |
| | HuGene_st:42916-HuGene_st:315405- | | | | | |
| | HuGene_st:340050-HuGene_st:173225- | | | | | |
| | HuGene_st:919818-HuGene_st:591228- | | | | | |
| | HuGene_st:899117-HuGene_st:785070- | | | | | |
| | HuGene_st:286200-HuGene_st:231925_at | | | | | |
| SORBS2 | 238751_at:805920-HuGene_st | 4.23E-05 | 1.3026 | 2.1 | 74.3 | 65.1-82 |

**TABLE 5**

| **Gene** | **Normal Blood** | **MRC5** | **CaCo2** | **HCT116** | **HT29** | **SW480** |
|---|---|---|---|---|---|---|
| **CXCL12** | Unmeth | low | unmeth | meth | meth | unmeth |
| **DF** | Low | meth | meth | meth | unmeth | meth |
| **MAMDC2** | Unmeth | unmeth | meth | unmeth | unmeth | meth |
| **CA4** | unmeth | unmeth | meth | meth | meth | unmeth |
| **MT1M** | low | low | low | meth | low | meth |
| **P2RY14** | unmeth | Low | Meth | Meth | Unmeth | Meth |
| **GPM6B** | Low | Low | Low | Meth | Meth | Meth |
| **ADAMDEC1** | low | Unmeth | Meth | Meth | unmeth | meth |

**TABLE 6**

| | Probe Set ID | Target Sequence |
|---|---|---|
| **1** | 200600 _at | tagtcttggggcaggatgattttggcctcattactttaccacccccacacctggaaagcatatactatattacaaaatgacattttgcca aaattattaatataagaagctttcagtattagtgatgtcatctgtcactataggtcatacaatccattcttaaagtacttgttatttgtttttatt attactgtttgtcttctccccagggttcagtccctcaaggggccatcctgtcccaccatgcagtgcccccctagcttagagcctccctcaa ttccccctggccaccacccccccactctgtgcctgaccttgaggagtcttgtgtgcattgctgtgaattagctcacttggtgatatgtcctat attggctaaattgaaacctggaattgtggggcaatctattaatagctgccttaaagtcagtaacttacccttaggaggctgggggaa aaggttagatttgtattcaggggtttttgtgtactttttgggtttttaaaaaattgtttttggaggggtttatgctcaatccatgt |
| **2** | 200621 _at | gatagcctcccactaggactgggaggagaataacccaggtcttaaggaccccaaagtcaggatgttgtttgatcttctcaaacatct agttccctgcttgatgggaggatcctaatgaaatacctgaaacatatattggcatttatcaatggctcaaatcttcatttatctctggcctt aaccctggctcctgaggctgcggccagcagagcccaggccagggctctgttcttgccacacctgcttgatcctcagatgtggagg gaggtaggcactgcctcagtcttcatccaaacacctttccctttgccctgagacctcagaatcttccctttaacccaagaccctgcctct tccactccacccttctccagggacccttagatcacatcactccacccctgccaggccccaggttaggaatagtggtgggaggaag gggaaaggctgggcctcaccgctcccagcaactgaaaggacaacactatctggagccacccactgaaaggctgcaggcat gggctgtac |
| **3** | 200665 _s_at | gttggttcaaacttttgggagcacggactgtcagttctctgggaagtggtcagcgcatcctgcagggcttctcctcctctgtcttttggag aaccagggctcttctcaggggctctagggactgccaggctgtttcagccaggaaggccaaaatcaagagtgagatgtagaaagt tgtaaaatagaaaaagtggagttggtgaatcggttgttctttcctcacatttggatgattgtcataaggtttttagcatgttcctccttttcttc accctcccctttgttcttctattaatcaagagaaacttcaaagttaatgggatggtcggatctcacaggctgagaactcgttcacctcca agcatttcatgaaaaagctgcttcttattaatcatacaaactctcaccatgatgtg |
| **4** | 200795 _at | aacactgcataacccgtttctttgaggagtgtgaccccaacaaggataagcacatcaccctgaaggagtggggccactgctttgg aattaaagaagaggacatagatgaaaatctcttgttttgaacgaagattttaaagaactcaactttccagcatcctcctctgttctaac cacttcagaaatatatgcagctgtgatacttgtagatttatatttagcaaaatgttagcatgtatgacaagacaatgagagtaattgctt gacaacaacctatgcaccaggtatttaacattaactttggaaacaaaaatgtacaattaagtaaagtcaacatatgcaaaatactgt acattgtgaacagaagtttaattcatagtaatttcactctctgcattgacttatgagataattaatgattaaactattaatgataaaaataa tgcatttgtattgttcataatatcatgtgcacttcaagaaaatggaatgctactcttttgtggtttac |
| **5** | 200799 _at | ggccgacaagaagaaggtgctggacaagtgtcaagaggtcatctcgtggctggacgccaacaccttggccgagaaggacgag tttgagcacaagaggaaggagctggagcaggtgtgtaaccccatcatcagcggactgtaccagggtgccggtggtcccggcct gggggcttcggggctcagggtcccaagggagggtctgggtcaggccccaccattgaggaggtagattaggggcctttccaagatt gctgtttttgttttggagcttcaagactttgcatttcctagtatttctgtttgtcagttctcaatttcctgtgtttgcaatgttgaaatttttttggtgaa gtactgaacttgcttttttttccggtttctacatgcagagatgaatttatactgccatcttacgactatttcttcttttaatacacttaactcagg ccattttttt |
| **6** | 200845 _s_at | tcctgtcacccattttgaagagtggcagaacttgaagttcaacttcctctgtaaatatccaagtataaagcccaggaacttctagaata acccagatgcgctttaatttttttaatatgtttgatcacagaacttctagaataacccagatgctctttcatattcttttaatacatcttgatc acagctgggggaaaaaaagctttttaattctgtaccttcctagtagataagtgaagagcagggaaagagacctttaaatattttgcta taaaaaaatttgtgataagtttctatcaaaatggggagattgcagaaaaggcttcccttggctcccaaggaggtgtagcaggtgtga gcaatattagtgccatgtgcctttcacacagggtttgcatttatcagtctgttttccgatgatgtgtacatgaaagagtacaccatgtgaa gagaagagagaatgattgaaaatgttttagtatagaactcttcttgcagtgggttgctatttctagattta |
| **7** | 200859 _x_at | gtttgtagactctctgaccaaggccacctgtgccccccagcatggggccccgggtcctgggcctgctgacgccagcaaggtggtg gccaagggcctggggctgagcaaggcctacgtaggccagaagagcagcttcacagtagactgcagcaaagcaggcaacaa catgctgctggtgggggttcatggcccaaggacccccctgcgaggagatcctggtgaagcacgtgggcagccggctctacagcgt gtcctacctgctcaaggacaagggggagtacacactggtggtcaaatggggggcacgagcacatcccaggcagcccctaccgc gttgtggtgccctgagtctggggcccgtgccagccggcagcccccaagcctgccccgctacccaagcagccccgccctcttcccc tcaaccccggcccaggccgccctggccgcccgcctgtcactgcagctgcccctgccctgtgccgtgctgcgctcacctgcctcccc agccagccgctgacctctcggctttcacttgggcagagggagccatttggtggcgctgct |
| **8** | 200884 _at | cctcacccagattgaaactctcttcaagtctaaggactatgagttcatgtggaaccctcacctgggctacatcctcacctgcccatcc aacctgggcaccgggctgcgggcaggtgtcgatatcaagctgcccaacctgggcaagcatgagaagttctcggaggtgcttaag cggctgcgacttcagaagcgaggcacaggcggtgtggacacggctgcggtgggcggggtcttcgacgtctccaacgctgaccg cctgggcttctcagaggtggagctggtgcagatggtggtggacggagtgaagctgctcatcgagatggaacagcggctggagca gggccaggccatcgacgacctcatgcctgcccagaaatgaagcccggcccacacccgacaccagccctgctgcttcctaactta ttgcctgcagtgcccaccatgcacccctcgatgttgccgtctggcgagcccttagccttgctgtaaggaaggcttccgtcacccttggt agagtttat |
| **9** | 200897 _s_at | ctctcttagctcagttactcaattcatacgtagtattttttaaaataattttatatctgtgtaccaccccatatatttcatattactgtttcacatgt acagctttctacttctttgtaagaacaccaaccaaccaaggtttaagtgattaataggcttgagcaccgggtggcagatgttctatgca gtgtggttcaagtttctttgaccgcacttatatgcattgctaatatggaatttaagataccatacacagtctctcatggacctatctctattgt agaattatgacttatgtcttacttggcaaatttttctgaatgtgaccttttttttgctgattgctgggtttgggattaactagcattattttgccac ctt |
| **10** | 200974 at | aagagttacgagttgcctgatgggcaagtgatcaccatcggaaatgaacgtttccgctgcccagagaccctgttccagccatccttc atcggatggagtctgctggcatccatgaaaccacctacaacagcatcatgaagtgtgatattgacatcaggaaggacctctatgc |
| | | taacaatgtcctatcagggggcaccactatgtaccctggcattgccgaccgaatgcagaaggagatcacggccctagcacccag caccatgaagatcaagatcattgcccctccggagcgcaaatactctgtctggatcggtggctccatcctggcctctctgtccaccttc cagcagatgtggatcagcaaacaggaatacgatgaagccgggccttccattgtccaccgcaaatgcttctaaaacactttcctgct cctctctgtctctagcacacaactgtgaatgtcctgtggaattatgccttcagttcttttccaaatcattcctagccaaagctctgactcgt |
| **11** | 200986 _at | gacgaccagccaggatatgctctcaatcatggagaaattggaattcttcgatttttcttatgaccttaacctgtgtgggctgacagagg acccagatcttcaggtttctgcgatgcagcaccagacagtgctggaactgacagagactggggtggaggcggctgcagcctccg ccatctctgtggcccgcaccctgctggtctttgaagtgcagcagcccttcctcttcgtgctctgggaccagcagcacaagttccctgtc ttcatggggcgagtatatgaccccagggcctgagacctgcaggatcaggttagggcgagcgctacctctccagcctcagctctca gttgcagccctgctgctgcctgcctggacttgcccctgccacctcctgcctcaggtgtccgctatccaccaaaagggctcctgagggt ctgggcaagggacctgcttctattagcccttctccatggccctgccatgctctccaaaccactttttgcagctttctc |
| **12** | 201041 _s_at | gcaataactctgggaggggctcgagaggctggtccttatttatttaacttcacccgagttcctctgggtttctaagcagttatggtgat gacttagcgtcaagacatttgctgaactcagcacattcgggaccaatatatagtgggtacatcaagtccatctgacaaaatggggc agaagagaaaggactcagtgtgtgatccggtttctttttgctcgcccctgtttttgtagaatctcttcatgcttgacatacctaccagtatt attcccgacgacacatatacatatgagaatataccttatttatttttgtgtaggtgtctgccttcacaaatgtcattgtctactcctagaa |
| **13** | 201058 _s_at | gcatcctcaaacatggcgccaaggataaacacgactaggccatccccagcccccctgacacccagcccccgccagtcacccctc cccgcacacacccgtccataccagctccctgcccatgaccctcgctcagggatccccctttgaggttagggtcccagttcccagt ggaagaaacaggccaggagagtgcgtgccgagctgaggcagatgttcccacagtgaccccagagccctgggctatagtctctg acccctccaaggaaagaccaccttctggggacatgggctggagggcaggacctagaggcaccaagggaaccgcattccggg gctgttccccgaggaggaagggaagcctctgtgtgccccccaggaggaagaggccctgagtcctgggatcagacaccccttca cgtgtatcccacacaaatgcaagctcaccaaggtccc |
| **14** | 201061 _s_at | tatagctctctttagctcaaccactctgtccatccagccaatggatgtccttccctgtacccaattcaagcttattttagggaagccttga aactaccatgtatctggctctagctgagttattgaggattgagccagtgcaacgttaaactcagtgcacttacatttgatttaaatgatg gttttatctgttgtgtgaagtggttcacccttgaggaccaggagcctccatatcctgactgaaaaccttttctgagacttagagtaacagt acttttggttccttgagttctcctgtctccagatacctaaatgaccttgacttttctgccttgtgaattcgtagtccaatcagctgaaattaaa tcacttgggagggacgcatagaaggagctctaggaacacagtgccagtgcagaagtttctccaggtggcc |
| **15** | 201069 _at | ctcagagccacccctaaagagatcctttgatattttcaacgcagccctgctttgggctgccctggtgctgccacacttcaggctcttctc ctttcacaaccttctgtggctcacagaacccttggagccaatggagactgtctcaagagggcactggtggcccgacagcctggca cagggcagtgggacagggcatggccaggtggccactccagacccctggcttttcactgctggctgccttagaacctttcttacatta gcagtttgctttgtatgcactttgtttttttcttgggtcttgtttttttttccacttagaaattgcatttcctgacagaaggactcaggttgtctga agtcactgcacagtgcatctcagcccacatagtgatggttcccctgttcactctacttagcatgtccctaccgagtctcttctccactgg atggaggaaaaccaagccgtggcttcccgctcagccctccctgcccctcccttcaaccattccccatgggaaat |
| **16** | 201105 _at | ctcctggactcaatcatggcttgtggtctggtcgccagcaacctgaatctcaaacctggagagtgccttcgagtgcgaggcgaggt ggctcctgacgctaagagcttcgtgctgaacctgggcaaagacagcaacaacctgtgcctgcacttcaaccctcgcttcaacgcc cacggcgacgccaacaccatcgtgtgcaacagcaaggacggcggggcctggggggaccgagcagcgggaggctgtctttccct tccagcctggaagtgttgcagaggtgtgcatcaccttcgaccaggccaacctgaccgtcaagctgccagatggatacgaattcaa gttccccaaccgcctcaacctggaggccatcaactacatggcagctgacggtgacttcaa |
| **17** | 201137 _s_at | ggcagcattcaagtccgatggttcctgaatggacaggaggaaacagctggggtcgtgtccaccaacctgatccgtaatggagact ggaccatccagatcctggtgatgctggaaatgacccccagcagggagacgtctacatctgccaagtggagcacaccagcctg gatagttccgtcaccgtggagtggaaggcacagtctgattctgcccagagtaagacattgacgggagctgggggcttcgtgctgg ggctcatcatctgtggagtgggactcttcatgcacaggaggagcaagaaagttcaacgaggatctgcataaacagggttcctgac ctcaccgaaaagactaatgtgccttagaacaagcatttgctgtgttttgttaacacctggttccaggacagaccctcagcttcccaag aggatactgctgccaagaagttgctctgaagtcagtttctatcgttctgctctttgattcaaa |
| **18** | 201141 _at | agaggcgggatactttcagctttccatgtaactgtatgcataaagccaatgtagtccagtttctaagatcatgttccaagctaactgaa tcccacttcaatacacactcatgaactcctgatggaacaataacaggcccaagcctgtggtatgatgtgcacacttgctagactcag aaaaaatactactctcataaatgggtgggagtattttggtgacaacctactttgcttggctgagtgaaggaatgatattcatatattcatt tattccatggacatttagttagtgctttttatataccaggcatgatgctgagtgacactcttgtgtatatttccaaatttttgtatagtcgctgc acatatttgaaatcatatattaagactttccaaagatgaggtccctggtttttcatggcaacttgatcagtaaggatttcacctctgtttgta acta |
| **19** | 201150 _s_at | gactttttggaatagccctgtctagggcaaactgtggcccccaggagacactacccttccatgccccagacctctgtcttgcatgtga caattgacaatctggactaccccaagatggcacccaagtgtttggcttctggctacctaaggttaacatgtcactagagtatttttatga gagacaaacattataaaaatctgatggcaaaagcaaaacaaaatggaaagtaggggaggtggatgtgacaacaacttccaaa ttggctctttggaggcgagaggaaggggagaacttggagaatagtttttgctttgggggtagaggcttcttagattctcccagcatccg cctttccctttagccagtctgctgtcctgaaacccagaagtgatggagagaaaccaacaagagatctcgaaccctgtctagaagg aatgtatttgttgctaaatttcgtagcactgtttacagttttcctccatgttatttatg |
| **20** | 201289 _at | gccacgattggagaatactttgcttcatagtattggagcacatgttactgcttcattttggagcttgtggagttgatgactttctgttttctgttt gtaaattatttgctaagcatatttctctaggcttttttcctttggggttctacagtcgtaaaagagataataagattagttggacagtttaa agcttttattcgtcctttgacaaaagtaaatggagggcattccatcccttcctgaaggggacactccatgagtgtctgtgagaggc |
| | | agqctatctgcactctaaactgc |
| **21** | 201300 _s_at | ttaggtcaagttcatagtttctgtaattggcttttgaatcaaagaatagggagacaatctaaaaaatatcttaggttggagatgacaga aatatgattgatttgaagtggaaaaagaaattctgttaatgttaattaaagtaaaattattccctgaattgtttgatattgtcacctagcag atatgtattacttttctgcaatgttattattggcttgcactttgtgagtatctatgtaaaaatatatatgtatataaaatatatattgcatagg ac agacttaggagttttgtttagagcagttaacatctgaagtgtctaatgcattaacttttgtaaggtactgaatacttaatatgtgggaaac ccttttgcgtggtccttaggcttacaatgtgcactgaatcgtttcatgtaagaatccaaagtggacaccattaacaggtctttgaaatat gcatgtactttatatttctatatttgtaactttgcatgttcttgttttgtta |
| **22** | 201324 _at | caccaaattacctaggctgaggttagagagattggccagcaaaaactgtgggaagatgaactttgtcattatgatttcattatcacat gattatagaaggctgtcttagtgcaaaaaacatacttacatttcagacatatccaaaggaatactcacatttgttaagaagttgaac tatgactggagtaaaccatgtattcccttatcttttactttttttctgtgacatttatgtctcatgtaatttgcattactctggtggattgttctagta ctgtattgggcttcttcgttaat |
| **23** | 201348 _at | accagctctaggtccaattgttctgctctaactgatacctcaaccttggggccagcatctcccactgcctccaaatattagtaactatg actgacgtccccagaagtttctgggtctaccacactccccaacccccactcctacttcctgaagggccctcccaaggctacatccc caccccacagttctccctgagagagatcaacctccctgagatcaaccaaggcagatgtgacagcaagggccacggaccccatg gcaggggtggcgtcttcatga |
| **24** | 201426 _s_at | tgtggatgtttccaagcctgacctcacggctgccctgcgtgacgtacgtcagcaatatgaaagtgtggctgccaagaacctgcagg aggcagaagaatggtacaaatccnagtttgctgacctctctgaggctgccaaccggnacnatgacgccctgcgccaggcnaag caggagtccnctgagtaccggagacaggtgcagtccctcacctgtgaagtggatgcccttaaaggaaccaatgagtccctggaa cgccagatgcgtgaaatggaagagaactttgccgttgaagctgctaactaccaagacactattggccgcctgcaggatgagattc agaatatgaaggaggaaatggctcgtcaccttcgtgaataccaagacctgctcaatgttaagatggcccttgacattgagattgcc acct |
| **25** | 201427 _s_at | gatccagaaatacttaacacgtgaatattttgctaaaaagcatatataactattttaaatatccatttatcttttgtatatctaagactcat cctgatttttactatcacacatgaataaaggcctttgtatctttctttctctaatgttgtatcatactcttctaaacttgagtggctgtcttaaa agatataaggggaaagataatattgtctgtctctatattgcttagtaagtatttccatagtcaatgatggtttaataggtaaaccaaacc ctataaacctgacctcctttatggttaatactattaagcaagaatgcagtacagaattggatacagtacggatttgtccaaat |
| **26** | 201438 _at | ctccagcagttctctcgaatactttgaatgttgtgtaacagttagccactgctggtgtttatgtgaacattcctatcaatccaaattccctct ggagtttcatgttatgcctgttgcaggcaaatgtaaagtctagaaaataatgcaaatgtcacggctactctatatactttgcttggttcat ttttttccctttagttaagcatgactttagatgggaagcctgtgtatcgtggagaaacaagagaccaactttttcattccctgcccccaa tttcccagactagatttcaagctaattttctttttctgaagcctctaacaaatgatctagttcagaaggaagcaaaatcccttaatctatgt gcaccgttgggaccaatgccttaattaaagaatttaaaaagttgtaatagagaatattttggcattcctctcaatgttgtgtgtt |
| **27** | 201495 _x_at | caagtgcagcgatggggagcgggcccgggcggagctcaatgacaaagtccacaagctgcagaatgaagttgagagcgtcac agggatgcttaacgaggccgaggggaaggccattaagctggccaaggacgtggcgtccctcagttcccagctccaggacaccc aggagctgcttcaagaagaaacccggcagaagctcaacgtgtctacgaagctgcgccagctggaggaggagcggaacagcc tgcaagaccagctggacgaggagatggaggccaagcagaacctggagcgccacatctccactctcaacatccagctctccga ctcgaagaagaagctgcaggactttgccagcaccgtggaagctctggaagaggggaagaagaggttccagaaggagatcga gaacctcacccag |
| **28** | 201496 _x_at | tcaagtccaagttcaagtccaccatcgcggcgctggaggccaagattgcacagctggaggagcaggtcgagcaggaggccag agagaaacaggcggccaccaagtcgctgaagcagaaagacaagaagctgaaggaaatcttgctgcaggtggaggacgagc gcaagatggccgagcagtacaaggagcaggcagagaaaggcaatgccagggtcaagcagctcaagaggcagctggagga ggcagaggaggagtcccagcgcatcaacgccaaccgcaggaagctgcagcgggagnnggatgaggccacggagagcaa cgaggccatgggccgcgaggtgaacgcactcaagagcaagctcagagggcccccccacaggaaacttcgcagtgatgcac caggcgaggaaacgagacctctttcgttccttctagaaggtctggaggacgtagagttattgaaaatgcagatggttctgaggagg aaacggacactcgagacgcagacttcaatggaaccaaggccagtgaataagcaactttctacagttttgcaccacgg |
| **29** | 201497 _x_at | tgagccaggaagaacgctccagcccaggacttcgaggctgcaatgagctataattgcatcattgcactccagcctgggcaacag agaccctgtctcaaccaccaccaccaccaccacccctactacccctgtattcaaggtaaaaattgaagtttgtatgatgtaagagat gagaaaaacccaacaggaaacacagacacatcctccagttctatcaatggattgtgcagacactgagtttttagaaaaacatatc cacggtaaccggtccctggcaattctgtttacatgaaatggggagaaagtcaccgaaatgggtgccgccggcccccactcccaat tcattccctaacctgcaaacctttccaacttctcacgtcaggcctttgagaattctttccccctctcctggtttccacacctcagacacgc acagttcaccaagtgccttctgtagtcacatgaattgaaaaggagacgctgctcccacggaggggagcaggaatgctgcactgttt acaccctgactgtgcttaaaa |
| **30** | 201539 _s_at | tgaagtgcaacaaggccatcacatctggaggaatcacttaccaggatcagccctggcatgccgattgctttgtgtgtgttacctgctc taagaagctggctgggcagcgtttcaccgctgtggaggaccagtattactgcgtggattgctacaagaactttgtggccaagaagt gtgctggatgcaagaaccccatcactgg |
| **31** | 201540 _at | caggctgtcatcaacatggatatgacatttcacaacagtgactagttgaatcccttgtaacgtagtagttgtctgctctttgtccatgtgt taatgaggactgcaaagtcccttctgttgtgattcccaggacttttcctcaagaggaaatctggatttccacctaccgcttacctgaaat gcaggatcacctacttactgtattctacattattatatgacatagtataatgagacaatatcaaaagtaaacatgtaatgacaatacat actaacattcttgtaggagtggttagagaagctgatgcctcatttctacattctgtcattagctattatcatctaacgtttcagtg |
| **32** | 201616 _s_at | cttgagaccaggagacgtatccagcaagcggaacctctgggaaaagcaatctgtggataaggtcacttcccccactaaggtttga gacagttccagaaagaacccaagctcaagacgcaggacgagctcagttgtagagggctaattcgctctgttttgtatttatgttgattt actaaattgggttcattatcttttatttttcaatatcccagtaaacccatgtatattatcactatatttaataatcacagtctagagatgttcat ggtaaaagtactgcctttgcacaggagcctgtttctaaagaaacccatgctgtgaaatagagacttttctactgatcatcataactctgt atctgagcagtgataccaaccacatctgaagtcaacagaagatccaagtttaaaattgcctgcggaatgtgtgcagtatctagaaa |
| **33** | 201617 _x_at | tgttgtttctgcactttataataaagcatggaagaaattatcttagtaggcaattgtaacactttttgaaagtaacccatttcagatttgaa atactgcaataatggttgtctttaaaaaaaaaaagaatgtactgttaaggtattactttttttcatgctgatgattcatatctaaattacatta ttatgttagctgacagtggtactgatttttttaggttggttgttttgtggatttctttagtagtgatagtagcctgaaccacattttagataactca attatgtatgtatgtgcatacacatatacaaacacactaatggtagaatgctttttttatgtgctagactattatatttagtagtatgtcattgt aactagccaatatcacagcttttgaaaaattaaaaaatcatcatcactataatatttcatatttgccaacagaaacatggcagatagg tatcaatatgttttcaatgcctgatgacctat |
| **34** | 201645 _at | ttttaccaaagcatcaatacaaccagcccaaccatcggtccacacctgggcatttggtgagaatcaaagctgaccatggatccctg gggccaacggcaacagcatgggcctcacctcctctgtgatttctttcttgcaccaaagacatcagtctccaacatgtttctgttttgttgt ttgattcagcaaaaatctcccagtgacaacatcgcaatagtttttacttctcttaggtggctctgggatgggagaggggtaggatgta caggggtagtttgttttagaaccagccgtattttacatgaagctgtataattaattgtcattatttttgttagcaaagattaaatgtgtcattg gaagccatcccttttttacatttcatacaacagaaaccagaaaagcaatactgtttccattttaaggatatgattaatattattaatataa taatgatgatgatgatgatgaaaactaaggatttttcaagagatctttcttccaaaacatttctggacagtacctgattgt |
| **35** | 201667 _at | gagtggactattaaatgtgcctaaatgaattttgcagtaactggtattcttgggttttcctacttaatacacagtaattcagaacttgtattct attatgagtttagcagtcttttggagtgaccagcaactttgatgtttgcactaagattttatttggaatgcaagagaggttgaaagaggat tcagtagtacacatacaactaatttatttgaactatatgttgaagacatctaccagtttctccaaatgcctttttaaaactcatcacagaa gattggtgaaaatgctgagtatgacacttttcttcttgcatgcatgtcagctacataaacagttttgtacaatgaaaattactaatttgtttg acattccatgttaaactacggtcatgttcagcttcattgcatgtaatgtagacctagtccatcaga |
| **36** | 201739 _at | tgctgtgtgaaccgtcgtgtgagtgtggtatgcctgatcacagatggatttgttataagcatcaatgtgacacttgcaggacactaca acgtgggacattgtttgtttcttccatatttggaagataaatttatgtgtagactttttgtaagatacggttaataactaaaatttattgaaat ggtcttgcaatgactcgtattcagatgcctaaagaaagcattgctgctacaaatatttctatttttagaaagggtttttatggaccaatgc cccagttgtcagtcagagccgttggtgtttttcattgtttaaaatgtcacctgtaaaatgggcattatttatgtttttttttttgcattcctgataat tgtatgtattgtataaagaacgtctgtacattgggttataacactagtatatttaaacttacaggcttatttgtaatgtaaaccaccatttta atgtactgtaattaacatggttataatacgtacaatccttccctcatcccatcacacaactttttttg |
| **37** | 201743 _at | ccatccagaatctagcgctgcgcaacacaggaatggagacgcccacaggcgtgtgcgccgcactggcggcggcaggtgtgca gccccacagcctagacctcagccacaactcgctgcgcgccaccgtaaaccctagcgctccgagatgcatgtggtccagcgccct gaactccctcaatctgtcgttcgctgggctggaacaggtgcctaaaggactgccagccaagctcagagtgctcgatctcagctgca acagactgaacagggcgccgcagcctgacgagctgcccgaggtggataacctgacactggacgggaatcccttcctggtccct ggaactgccctcccccacgagggctcaatgaactccggcgtggtcccagcctgtgcacgttcgaccctgtcggtgggggtgtcgg gaaccctggtgctgctccaagggcccggggctttgcctaagatccaagacagaataatgaatggactcaaactgccttggcttc aggggagtcccgtcaggacgttgaggacttttcgaccaattcaacc |
| **38** | 201744 _s_at | caaatgatgtgcaaaaccttttactggttgcatggaaatcagccaagttttataatccttaaatcttaatgttcctcaaagcttggattaa atacatatggatgttactctcttgcaccaaattatcttgatacattcaaatttgtctggtaaaaaaataggtggtagatattgaggccaa gaatattgcaaaatacatgaagcttcatgcacttaaagaagta tttttagaataagaatttgcatacttacctagtgaaacttttctaga attatttttcactctaagtcatgtatgtttctct |
| **39** | 201842 _s_at | tcagcagtatagggaccttccgcacaagctctgtgttaagattgacaataatagtggggccattttcatttagtcttttctaagagtcaa ccacaggcatttaagtcagccaaagaatattgttaccttaaagcactatttatttatagatatatctagtgcatctacatctctatactgt acactcacccataattcaaacaattacaccatggtataaagtgggcatttaatatgtaaagattcaaagtttgtctttattactatatgta aattagacattaatccactaaactggtcttcttcaagagagctaagtatacactatctggtgaaacttggattctttcctataaaagtgg gaccaagcaatgatgatcttctgtggtgcttaaggaaacttactagagctccactaacagtctcataaggaggcagccatcataac cattga |
| **40** | 201852 _x_at | gtgccaatcctttgaatgttccacggaaacactggtggacagattctagtgctgagaagaaacacgtttggtttggagagtccatgg atggtggttttcagtttagctacggcaatcctgaacttcctgaagatgtccttgatgtgcagctggcattccttcgacttctctccagccga gcttcccagaacatcacatatcactgcaaaaatagcattgcatacatggatcaggccagtggaaatgtaaagaaggccctgaag ctgatggggtcaaatgaaggtgaattcaaggctgaaggaaatagcaaattcacctacacagttctggaggatggttgcacgaaac acactggggaatggagcaaaacagtctttgaatatcgaacacgcaaggctgtgagactacctattgtagatattgcaccctatgac attggtggtcctgatcaagaatttggtgtggacgttggccct |
| **41** | 201858 _s_at | cctggttctggaatcctcagttcaaggttatcctacgcagagagccaggtaccaatgggtgcgctgcaatccagacagtaattctgc aaactgccttgaagaaaaaggaccaatgttcgaactacttccaggtgaatccaacaagatcccccgtctgaggactgaccttttttcc aaagacgagaatccaggacttgaatcgtatcttcccactttctgaggactactctggatcaggcttcggctccggctccggctctgga tcaggatctgggagtggcttcctaacggaaatggaacaggattaccaactagtagacgaaagtgatgctttccatgacaaccttag gtctcttgacaggaatctgccctcagac |
| **42** | 201859 _at | tatgctttaatgctgttatctatcttattgttcttgaaaatacctgcattttttggtatcatgttcaaccaacatcattatgaaattaattagattc ccatggccataaaatggctttaaagaatatatatatatttttaaagtagcttgagaagcaaattggcaggtaatatttcatacctaaatt aagactctgacttggattgtgaattataatgatatgccccttttcttataaaaacaaaaaaaaaaataatgaaacacagtgaatttgtag agtgggggtatttgacatattttacagggtggagtgtactatatactattacctttgaatgtgtttgcagagctagtggatgtgtttgtctac aagtatgattgctgttacataacaccccaaattaactcccaaattaaaacacagttgtgctgtcaatacctcatactgctttaccttttttt cctggatatctgtgtattttc |
| **43** | 201865 _x_at | gttgagtcgtcatcacttttcagtgatgggagagtagatggtgaaatttattagttaatatatcccagaaattagaaaccttaatatgtgg acgtaatctccacagtcaaagaaggatggcacctaaaccaccagtgcccaaagtctgtgtgatgaactttctcttcatactttttttcac agttggctggatgaaatttctagactttctgttggtgtatcccccccctgtatagttaggatagcattttgatttatgcatggaaacctga aaaaaagtttacaagtgtatatcagaaaagggaagttgtgccttttatagctattactgtctggttttaacaatttcctttatatttagtgaa ctacgcttgctcatttttttcttacataatttttttattcaagttattgtacagctgtttaagatgggcagctagttcgtagctttcccaaataaact ctaaacattaatcaatcatctgtgtgaaaatgggttggtgcttctaacctgatggcacttagctatcagaagaccacaaaaattgact caaatctccagtattcttg |
| **44** | 201893 _x_at | tctcctacatccgcattgctgataccaatatcaccagcattcctcaaggtcttcctccttcccttacggaattacatcttgatggcaacaa aatcagcagagttgatgcagctagcctgaaaggactgaataatttggctaagttgggattgagtttcaacagcatctctgctgttgac aatggctctctggccaacacgcctcatctgagggagcttcacttggacaacaacaagcttaccagagtacctggtgggctggcag agcataagtacatccaggttgtctaccttcataacaacaatatctctgtagttggatcaagtgacttctgcccacctggacacaacac caaaaaggcttcttattcgggtgtgagtcttttcagcaacccggtccagtactgggagatacagccatccaccttcagatgtgtctac gtgcgctctgccattcaactcggaaactataagtaattctcaagaaagccctcatttttataacctgg |
| **45** | 201920 _at | gtatcaggcttcaattccattatgttttaatgttgtctctgaagatgacttgtgattttttttcttttttttaaaccatgaagagccgtttgacaga gcatgctctgcgttgttggtttcaccagcttctgccctcacatgcacagggatttaacaacaaaaatataactacaacttcccttgtagt ctcttatataagtagagtccttggtactctgccctcctgtcagtagtggcaggatctattggcatattcgggagcttcttagagggatga ggttctttgaacacagtgaaaatttaaattagtaactttttttgcaagcagtttattgactgttattgctaagaagaagtaagaaagaaaa agcctgttggcaatcttggttatttctttaagatttctggcagtgtgggatggatgaatgaagtggaatgtgaactttgggcaagttaaat gggacagccttccatgttcatttgtctacctcttaactga |
| **46** | 201957 _at | gttgtgcctaccactggctggcacaccagggcaatgatttccctgcagaaggaaggaaagaatgtttcacccttgcatccttcttgg agaagctacagcctgtgctcagttgagtggttcacactcagactttggctttatggttttccttcctccttgtctttgccctgaccttgatcaa caggggtgaaaagaaccaccctgaggtttccatgcctctcccattttagtggtagcattttgtgtctttactccacccttcaccctagttc caccaaggttcacacaccagatgtaactgtttttcagctgagttgtatggattaacttcagtccactgtaaatacacctgggatggggt ggggttggggttgtttagggagaagcagccagacttgctttgtgaactgaatgtattttatgcaattttgagtggcctttcaaccctaag a |
| **47** | 202007 _at | tatcagtagctcgtgttatctttttatcaactgcttcccagngtcctaaaacaatagaaattttggattgaaaagttcagcataaggagttt gagtcagtaaaggatgggataaaggagtcgagatgattcaatgaaaagtatcacaaaaagagattgatcaacaagagaaat aaaaaagcccaagaggaagtggtaggggaaggaatttaagaacagcaataagtaaaactcttaagtaactccaaaaagaaa atggtacattttgccaaagaccacttatacttgagaacatggaagaatttgcctgatactctcttggggaaaagagtctctcctcttttc ctcaaaccccagtacactcagcctctctgccccaccttctcctgactttgtcctcacttgcttctgcagtacattggaacctgaattgaa agaaagtcttccttgaataattggagtttgtcttgagaggcaaatatagccccaagaatcacaagattcgaggaccatgtaggtctttt acgtagcccaaatccataaattagtctcactttttgtatttatcgtttcatattaaaccctctatatca |
| **48** | 202037 _s_at | taacacttggctcttggtacctgtgggttagcatcaagttctccccagggtagaattcaatcagagctccagtttgcatttggatgtgtaa attacagtaatcccatttcccaaacctaaaatctgtttttctcatcagactctgagtaactggttgctgtgtcataacttcatagatgcagg aggctcaggtgatctgtttgaggagagcaccctaggcagcctgcagggaataacatactggccgttctgacctgttgccagcagat acacaggacatggatgaaattcccgtttcctctagtttcttcctgtagtactcctcttttagatcc |
| **49** | 202069 _s_at | cagtcactctaaatggacaccacatgaacctctgtttagaatacctacgtatgtatgcattggtttgcttgtttcttgacagtacatttttag atctggcctttcttaacaaaatctgtgcaaaagatgcaggtggatgtccctaggtctgtttcaaagaactttttccaagtgcttgttttatt tattaagtgtctacctggtaaatgtttttttgtaaactctgagtggactgtatcatttgctattctaaaccatttacacttaagttaaaatagt ttctcttcagctgtaaataacaggatacagaattaacaagagaaaatgtctaactttttaagaaaaaccttatttcttcggtttttgaaa aacataatggaaataaaacaggatattgacataatagcacaaaatgacactcttctaaaactaaatgggcacaagagaattttcct ggga |
| **50** | 202133 _at | gtcaaaacctaattgcaattctgttaaatctaagtaattttagacagtgtttcaccgtattatttaggatgtgaaatgccatttctttcactg attacaccatatacaggaaacaggtaaaacagtgaaaactttattgtgctggttgatgccaacttggttgaaaagctctctgcagaa gaagtgatctagactgacagaagtgttgctaattacaagttgtgttctcatgacgtaattagaaagtaacttctcaaagtacaactttta tgaaaaaaataagctgttaaaaaaggaaatcgtaggttaatttaattgggaaaatgggcaattgacagagaccattttcctaaca catatatgtgctagtactttaactttttaaaatttacttctacgttttgtaatataaaaatttctattttaagtttagaatgttatacgtaccgaa agtatgcagccaaatcgatcagatcaaaccattttacctggagtt |
| **51** | 202222 _s_at | agctgtgagccttggctgttggcagtgagtgagcctggctcttgtgctggatggagcccaggcgggagcggtggccctgtccctccc acctctgtgacctgaggcctacgctttggctctggagatagccccagagcagggtgttgggatactgcagggccaggactgagcc cc |
| **52** | 202242 _at | ctgttagctcctcactgtggtaaatgccacacacctttaagtagataagcagacgatagttatctgttcttttgacttaatctcatttggtttg attttccctctactaaggctttcctaccttcttcaggctgcctaagacatgtaagcgaaacacttcaataattgtccatgaggagaaaa aaagcattgtcatgcatgaaggaaactgaacttgaggtggcctccttgcttgttacatacctgggtatgtgtaggcagtttagtgcatct ttgcctctcagttgaaacctgtataaccctgttacaaagctgtgttgttgcttcttgtgaaggccatgatatttgtttttccccaattaattgc tattgtgttattttactaacttctctctgtatttttcttgcattgacattatagacattgaggacctcatccaaacaatttaaaaatgagtgtga aggggaacaagtcaaaatattttaaaagatcttcaaaaataatgcctctgtctagcatgccaac |
| **53** | 202266 _at | tgtcagagttttcaacggtgcttatagctgccagctggattccaaacaggtaccccattgtctctgagctaatgtttatatttttccattcag gcaccgaaatagttaatatttaaaataagtcttcaaaagaaaacataagagattattgagttcttgggactggatcctttatttcataag ttcagatcatcttaaatgaaaatgccatgattatctgcagttaagtagatgacagctattctacatcagacttgatttttgtcagctaatta cataattggtaagctataattgaaaccttatggcttaaaattccttaactcctttttgattcatgtttgtagtcatgttgtcaacagaggcaa agttaagcttgatgatggttaaaatcggtttgatagcaccatgggacatttttctaacaaaaataaatgcatgaagagacatagcctttt tagttttgct |
| **54** | 202274 _at | aagagctatgagctgccagatgggcaggttatcaccattggcaatgagcgcttccgctgccctgagaccctcttccagccttccttta ttggcatggagtccgctggaattcatgagacaacctacaattccatcatgaagtgtgacattgacatccgtaaggacttatatgccaa caatgtcctctctggggggcaccaccatgtaccctggcattgctgacaggatgcagaaggagatcacagccctggcccccagcac catgaagatcaagattattgctcccccagagcggaagtactcagtctggatcgggggctctatcctggcctctctctccaccttccag cagatgtggatcagcaagcctgagtatgatgaggcagggccctccattgtccacaggaagtgcttctaaagtcagaacaggttctc caaggatcccc |
| **55** | 202283 _at | caactgcccttgaccggaagcatgagtatcatcttcttcctgcccctgaaagtgacccagaatttgaccttgatagaggagagcctc acctccgagttcattcatgacatagaccgagaactgaagaccgtgcaggcggtcctcactgtccccaagctgaagctgagttacg aaggcgaagtcaccaagtccctgcaggagatgaagctgcaatccttgtttgattcaccagactttagcaagatcacaggcaaacc catcaagctgactcaggtggaacaccgggctggctttgagtggaacgaggatggggcgggaaccacccccagcccagggctg cagcctgcccacctcaccttcccgctggactatcaccttaaccagcctttcatcttcgtactgagggacacagacacaggggccctt ctcttcattggcaagattctggaccccaggggcccctaatatcccagtttaatattccaataccctagaagaaaacccgagggaca gcagattccacag |
| **56** | 202291 _s_at | ctgacctgcaggacgaaaccatgaagagcctgatccttcttgccatcctggccgccttagcggtagtaactttgtgttatgaatcacat gaaagcatggaatcttatgaacttaatcccttcattaacaggagaaatgcaaataccttcatatcccctcagcagagatggagagct aaagtccaagagaggatccgagaacgctctaagcctgtccacgagctcaatagggaagcctgtgatgactacagactttgcgaa cgctacgccatggtttatggatacaatgctgcctataatcgctacttcaggaagcgccgaggggccaaatgagactgagggaaga aaaaaatctcttttttctggaggctggcacctgatttgtatccccctgtagcagcattactgaaatacataggcttatatacaatgctt ctttcct |
| **57** | 202350 _s_at | gaagccctggaaaatcgcctgagatacagatgaagattagaaatcgcgacacatttgtagtcattgtatcacggattacaatgaac gcagtgcagagccccaaagctcaggctattgttaaatcaataatgttgtgaagtaaaacaatcagtactgagaaacctggtttgcc acagaacaaagacaagaagtatacactaacttgtataaatttatctaggaaaaaaatccttcagaattctaagatgaatttaccag gtgagaatgaataagctatgcaaggtattttgtaatatactgtggacacaacttgcttctgcctcatcctgccttagtgtgcaatctcattt gactatacgataaagtttgcacagtcttacttctgtagaacactggccataggaaatgctgtttttttgtactggactttaccttgatatatg t |
| **58** | 202388 _at | gctggtatcagaacagcttccctcactgtgtacagaacgcaagaagggaataggtggtctgaacgtggtgtctcactctgaaaagc aggaatgtaagatgatgaaagagacaatgtaatactgttggtccaaaagcatttaaaatcaatagatctgggattatgtggccttag gtagctggttgtacatctttccctaaatcgatccatgttaccacatagtagttttagtttaggattcagtaacagtgaagtgtttactatgtg caagggtattgaagttcttatgaccacagatcatcagtactgttgtctcatgtaatgctaaaactgaaatggtccgtgtttgcattgttaa aaatgatgtgtgaaatagaatgagtgctatggtgttgaaaactgcagtgtccgttatgagtgccaaaaatctgtcttgaaggcagcta cactt |
| **59** | 202403 _s_at | aacctgaaaacatcccagccaagaactggtataggagctccaaggacaagaaacacgtctggctaggagaaactatcaatgct ggcagccagtttgaatataatgtagaaggagtgacttccaaggaaatggctacccaacttgccttcatgcgcctgctggccaactat gcctctcagaacatcacctaccactgcaagaacagcattgcatacatggatgaggagactggcaacctgaaaaaggctgtcatt ctacagggctctaatgatgttgaacttgttgctgagggcaacagcaggttcacttacactgttcttgtagatggctgctctaaaagac aaatgaatggggaaagacaatcattgaatacaaaacaaataagccatcacgcctgcccttccttgatattgcacctttggacatcg gtggtgctgaccaggaattctttgtggacattggcccagtctgtt |
| **60** | 202555 _s_at | gagccattggaagactgtcctctatggcaatgatctcagggctcagtggcaggaaatcctcaacagggtcaccaaccagcccgct caatgcagaaaaactagaatctgaagatgtgtcccaagctttccttgaggctgttgctgaggaaaagcctcatgtaaaaccctatttc tctaagaccattcgcgatttagaagttgtggagggaagtgctgctagatttgactgcaagattgaaggatacccagaccccgaggtt gtctggttcaaagatgaccagtcaatcagggagtcccgccacttccagatagactacgatgaggacgggaactgctctttaattatt agtgatgtttgcggggatgacgatgccaagtacacctgcaaggctgtcaacagtcttggagaagccacctgcac |
| **61** | 202620 _s_at | ttatcaagtgtcaagatcagcaagtgtccttaagtcaaataggtttttttttgttggtggttgtgcttgctttcctttttttagaaagttctagaaa ataggaaaacgaaaaatttcattgagatgagtagtgcatttaattatttttaaaaaacttttaagtacttgaattttatatcaggaaaac aaagttgttgagccttgcttcttccgttttgccctttgtctcgctccttattctttttgggggagggttatttgctttttatcttcctggcataattt |
| | | ccattttattcttctgagtgtctatgttaacttccctctatcccgcttataaaaaaattctccaacaaaaatacttgttgacttgatgttttatca cttctctaagtaaggttgaaatatccttattgtagctactgtttttaatgtaaaggttaaacttgaaaagaaattcttaatcacggtgccaa aattcattttctaacaccatgtgtt |
| **62** | 202686 _s_at | agattctaacggtctgttctgtttcaaggcactctagattccattggtccaagattccggatcctaagcatctaagttataagactctcac actcagttgtgactaactagacaccaaagttctaataatttctaatgttggacacctttaggttctttgctnnattctgcctctctaggacc atggttaagagtccaagaatccacatttctaaaatcttatagttctaggcactgtagttctaagactcaaatgttctaagtttctaagattc taaaggtccacaggtctagactattaggtgcaatttcaaggttctaaccctatactgtagtattctttggggtgcccctctccttcttagct atcattgcttcctcctccccaactgtgggggtgtgccccttcaagcctgtgcaatgcattagggatgcctcctttccgcaggggatgg acgatctcccacctttcgggccatgttgcccccgtgagccaatccctcaccttctgagtacagagtgtggactctggtgcctcca |
| **63** | 202731 _at | ggctctggaggtgggcagcaatctgtcaatcaccttgttaaagagattgatatgctgctgaaagaatatttactctctggagacatatc tgaagctgaacattgccttaaggaactggaagtacctcattttcaccatgagcttgtatatgaagctattataatggttttagagtcaact ggagaaagtacatttaagatgattttggatttattaaagtccctttggaagtcttctaccattactgtagaccaaatgaaaagaggttat gagagaatttacaatgaaattccggacattaatctggatgtcccacattcatactctgtgctggagcggtttgtagaagaatgttttcag gctggaataatttggaaacaactcagagatctttgtccttcaaggggcagaaagcgttttgtaagcgaaggagatggaggtcgtctt aaaccagagagctactgaatataagaactcttgcagtcttagatgttat |
| **64** | 202741 _at | gtaagattcctcctaactttcacagtcgatgacaagattgtctttttatctgatattttgaagggtatattgctttgaagtaagtctcaataag gcaatatatttagggcatctttcttcttatctctgacagtgttcttaaaattatttgaatatcataagagccttggtgtctgtcctaattcctttc tcactcaccgatgctgaatacccagttgaatcaaactgtcaacctaccaaaaacgatattgtggcttatgggtattgctgtctcattctt ggtatattcttgtgttaactgcccattggcctgaaaatactcattgtaagcctgaaaaaaaaatctttcccactgtttttctgcttgttgta agaatcaaatgaaataatgtatgtgaaagcaccttgtaaactgtaacctatcaatgtaaaatgttaaggtgtgttgttatttcattaatta cttctttgtttagaatggaatttcctatgcactactgtagc |
| **65** | 202742 _s_at | tacactttttttgccaactgacttaacaacattgctgtcaggtggaaatttcaagcacttttgcacatttagttcagtgtttgttgagaatcc atggcttaacccacttgtttgctattttttctttgcttttaattttccccatctgatttatctctgcgtttcagtgacctaccttaaaacaacaca cgagaagagttaaactgggttcattttaatgatcaatttacctgcatataaaatttatttttaatcaagctgatcttaatgtatataatcattc tatttgctttattatcggtgcaggtaggtcattaacaccacttcttttcatctgtaccacaccctggtgaaacctttgaagacataaaaaa aacctgtctgagatgttctttctaccaatctatatgtctttcggttatcaagtgtttctgcatggtaatgtc |
| **66** | 202746 _at | aaactactaaccactgcaagctcttgtcaaattttagtttaattggcattgcttgtttttgaaactgaaattacntgagtttcatttttctttga atttatagggtttagatttctgaaagcagcatgaatatatcacctaacatcctgacaataaattccatccgttgtttttttgtttgtttgtttttc ttttcctttaagtaagctctttattcatcttatggtgcagcaattttaaaatttgaaatatttaaattgtttttgaacttttgtgtaaaatatatca gatctcaacattgttggtttcttttgtttttcattttgtacaactttcttgaatttagaaattacatctttgcagttctgttaggtgctctgtaattaac ctgacttatatgtgaacaattttcatgagacagtcatttttaactaatgcagtgattctttctcactactatctgtattgtggaatgcacaaa attgtgtaggtgctgaatgctgtaaggagtttaggttgtatgaattctacaaccctataata |
| **67** | 202760 _s_at | gagatactatttttgctgagcaaccagtgtgtttcaggatgatacaaagaaaaatatagaatagaaataagtgcaggcttggaatca gctacaaatcctaaagatggggtgtgtgtggatgtgtgtgtgtgtgtgtacaccattgtgtgtttgtaaaatgtgtatgttcatgagtaa gggtgtgtgtgtgtgtgtattaaaattccagagtgaccgtggcacttgggtgtacaggtaattcctccagagctgtttgctggcttcagg agtggagtgagaatttctttttatgaaaagggatataaaggcaccgagctgatgcagtatttgtaatattaagttgacctaacaaggt atttgcatgagtcacaattacaaagttttgagcggttttgtaatttgacatttaggaaagtctcctatttattctcatactttacattcatgctta gtatactatagaggatgccagctttaatctttctg |
| **68** | 202766 _s_at | gctttcaattgatggactactctatttttgcaaattgtaaactttgcttctccaaatacaagtactaggttgtccatttatggtacctatttgg tgctagtaaatttcaaactagatttataaatgcactgtaatatgtacacaacttagaaaccaaattacaagtattcagttccaatacttc attaatttcaatcaaccaaagttagttcagtagcttatctcagttatgagtataatacattacatgtaaattaagtgtgtgtatactgtaatc gtgctattttttatcattgaaacatttataaactagaataataatgcccttaatgtgagggtttgtaatggtgcttattaagaccaaagactt gttaaatgtatacaccaagtggtaatgaaatttcgtgactggcccacacgtgcatagaggtctgggaggaccaggaaacagcctc agtggccagaggatcaccagtgcatccttcatcacagcatgtgcaatatgccaagattaccctcggtcattcctgtcaacaaggg |
| **69** | 202768 _at | gcgtgagtgtgtgagcgcttctgcagcctcggcctaggtcacgttggccctcaaagcgagccgttgaattggaaactgcttctagaa actctggctcagcctgtctcgggctgacccttttctgatcgtctcggcccctctgattgttcccgatggtctctctccctctgtcttttctcctc cgcctgtgtccatctgaccgttttcacttgtctcctttctgactgtccctgccaatgctccagctgtcgtctgactctgggttcgttggggac atgagattttatttttgtgagtgagactgagggatcgtagatttttacaatctgtatctttgacaattctgggtgcgagtgtgagagtgtga gcagggcttgctcctgccaaccacaattcaatgaatccccgacccccctaccccatgctgtacttgtggttctcttttgtattttgcatct gaccccgggggctgggacagattggcaatgggccgtccc |
| **70** | 202838 _at | agaaagaggcgctgctcactgttttcctgcttcagtttttctcttatagtaccatcactataatcaacgaacttctcttctccacccagaga tggcttttccaacacatttaattaaaggaactgagtacattaccctgatgtctaaatggaccaaagatctgagatccattgtgattatat ctgtatcaggtcagcagaagaaggaactgagcagttgaactctgagttcatcaattctaatatttggaaattatctacaatggaatctt ccctctgttctctgataacctacttgcttactcaatgcctttaagccaagtcaccctgttgcctatgggaggaggtggaaggatttggca agctcaaccacatgctatttagttagcatcagttgtcaccaacagtctttctgcaaagggcaggagagcttgggggaaaggaaaa ggcttaccaggctgctatggtcaactcttcagaa |
| **71** | 202888 _s_at | agccctggagaagacgaaagccaacatcaagtgggtgaaggagaacaaggaggtggtgctccagtggttcacagaaaacag caaatagtccccagcccttgaagtcacccggccccgatgcaaggtgcccacatgtgtccatcccagcggctggtgcaggcctc cattcctggagcccgaggcaccagtgtcctcccctcaaggacaaagtctccagcccacgttctctctgcctgtgagccagtctagtt cctgatgacccaggctgcctgagcacctcccagcccctgcccctcatgccaaccccgccctaggcctggcatggcacctgtcgcc cagtgccctggggctgatctcagggaagcccagctccagggccagatgagcagaagctctcgatggacaatgaacggccttgct gggggccgccctgtaccctctttcacctttccctaaagaccctaaatctgaggaatcaacagggcagcagatctgtatattttttc |
| **72** | 202920 _at | attatctggtcttttcctgttgtgcaaaaatgactcattgctccgaatgtcaaaaacaaatgcgacaaacaatggcacttcatcatttaa agtaatgttgccaagagaaaaaatttcctgggagggaggtttcccacaagccaaatctcctaagcctcaaatgctagcactttttgg cagttggataggaaatgagacattctttggcagccaaaataagagaggccgatggtgaaactttttgagacaccctatggccttctt gtcaaaaccttcactggagctcaagaaaagcatttctgttgtgttatttgcagtgcagatgatgtctgtgtaacaacataatggttattca cctttttttgattttgatttttgctgtgttatcaaaaacttgaatactgtgagaagaagtgaattttcagttgacgaatcagcatcttgttcccat ggtgataac |
| **73** | 202953 _at | cttcgaccacgtgatcaccaacatgaacaacaattatgagccccgcagtggcaagttcacctgcaaggtgcccggtctctactact tcacctaccacgccagctctcgagggaacctgtgcgtgaacctcatgcgtggccgggagcgtgcacagaaggtggtcaccttctg tgactatgcctacaacaccttccaggtcaccaccggtggcatggtcctcaagctggagcagggggagaacgtcttcctgcaggcc accgacaagaactcactactgggcatggagggtgccaacagcatcttttccgggttcctgctctttccagatatggaggcctgacct gtgggctgcttcacatccaccccggctcccctgccagcaacgctcactctacccccaacaccaccccttgcccagccaatggac acagtagggcttggtgaatgctgctgagtgaatgagtaaataaactcttcaaggccaaggaacagtggtctaattcaactctgtgtc ccagcactggcacaccagaagtgccatgctcagaaa |
| **74** | 202957 _at | tgatgagctttcctttgatccggacgacgtaatcactgacattgagatggtggacgagggctggtggcggggacgttgccatggcc actttggactcttccctgcaaattatgtcaagcttctggagtgactagagctcactgtctactgcaactgtgatttcccatgtccaaagtg gctctgctccacccccctccctattcctgatgcaaatgtctaaccagatgagtttctggacagacttccctctcctgcttcattaagggctt ggggcagagacagcatggggaaggaggtccccttccccaagagtcctctctatcctggatgagctcatgaacatttctcttgtgttcc tgactccttcccaatgaacacctctctgccaccccaagctctgctctcctcctctgtgagctctgggcttcccagtttgtttacccgggaa agtacgtctagattgtgtggtttgcctcattgtgctattgcccactttccttccctgaagaaatatctgtgaaccttctttctgttcagtccta |
| **75** | 202992 _at | gctggaatacttactcttgtcgggagattgaaccactaaaatgttagagcagaattcattatgctgtggtcacaggggtgtcttgtctga gaacaaatacaattcagtcttctctttggggttttagtatgtgtcaaacataggactggaagtttgcccctgttctttttcttttgaaagaac atcagttcatgcctgaggcatgagtgactgtgcatttgagatagttttccctattctgtggatacagtcccagagttttcagggagtaca caggtagattagtttgaagcattgaccttttatttattccttatttctctttcatcaaaacaaaacagcagctgtgggaggagaaatgaga gggcttaaatgaaatttaaaataagctatattatacaaatactatctctgtattgttctgaccctggtaaa |
| **76** | 202994 _s_at | aatgcgaaggctaagtgtcacccccttctctgcctctggctgggccttgctaagggccaaggaaagaaagacatttttaggggggc agccagtccaaatgccaaaagaagaccagttcttgccctgattgtatgaaatttgacattttggcactttttttttttttttnggccaatcag attttctatgttctaaggacatggctgctgtagaatagcacagacgtggatgataaattatccccagaagcagcatgacagaatgcc tcggggagcacttggaagggaaattgcagttctgttgaaatagaggaaaatcccttggtaaagacacagcctgttaggctcgtgtg ggcctccagtatgttcaccaggggaa |
| **77** | 202995 _s_at | aacgatgtcacatgcgtgttcgaccccgtgcacaccatctcccacaccgtcatctcgctgcctaccttccgcgagttcacccgccctg aagagatcatcttcctccgggccatcacgccaccgcatcctgccagccaggctaacatcatcttcgacatcacggaaggaacct gcgggactcttttgacatcatcaagcgttacatggacggcatgaccgtgggtgtcgtgcgccaggtgcggcccatcgtgggcccatt tcatgccgtcctgaagctggagatgaactatgtggtcgggggcgtggtttcccaccgaaatgttgtcaacgtccgcatcttcgtctctg agtactggttctgagggctggtctgccgcacagccgcaggtgcacctccaggccaaatcattgctgccagtgactgtggtctgtactt gtttataccctcag |
| **78** | 203000 _at | gacagacctgagaccaatctgggtagaagcaaaaagttgaacctttttaaagtgctgaacacaaatccaaattcgaatggttcaag cagccgtgaaatcgctcttcataaagtgggcttaattctctagtttaagttcttttgatggaatgaattaattaatgtgtcaggtggcttattt gtggatgccatgattgatgatgttcattttaagctcttacctatagtacaagtacatgatgctactgaatatttttccacttggaaactgtga gctggttgttgcattaaaacacacatacaaacaaaatcaaaaacactgcggactttcactcaagctggtctttcttccccagtgtaag gcaatcctgcctactaacaacaccaacaacaaaacactccatctgtgaanntgacgcagttaagggggctaggcagggcatttg tgccaactaagaatcaccagatacccaccataagtacctatcgcagttttgaagtc |
| **79** | 203001 _s_at | acctcgcaacatcaacatctatacttacgatgatatggaagtgaagcaaatcaacaaacgtgcctctggccaggcttttgagctgat cttgaagccaccatctcctatctcagaagccccacgaactttagcttctccaaagaagaaagacctgtccctggaggagatccag aagaaactggaggctgcaggggaaagaagaaagtctcaggaggcccaggtgctgaaacaattggcagagaagagggaac acgagcgagaagtccttcagaaggctttggaggagaacaacaacttcagcaagatggcggaggaaaagctgatcctgaaaat ggaacaaattaaggaaaaccgtgaggctaatctagctgctattattgaacgtctgcaggaaaaggagaggcatgctgcggaggt gcgcaggaacaaggaactccaggttgaactgtctggctgaagcaagggagggtctggcacgcc |
| **80** | 203058 _s_at | atggcctctgtgaataatgtaactccagttacacggtgacttttaatagcatacagtgatttgatgaaaggacgtcaaacaatgtggc gatgtcgtggaaagttatctttcccgctctttgctgtggtcattgtgtcttgcagaaaggatggccctgatgcagcagcagcgccagct gtaataaaaaataattcacactatcagactagcaaggcactagaactggaaaagaccacagaaaacaaagaatccaacccttt catcttacaggtgaacaaactgtgatgatgcacatgtatgtgtttgtaagctgtgagcaccgtaacaaaatgtaaatttgccattatta |
| | | ggaaagtgctggtggcagtgaagaagcacccaggccacttgactcccagtctggtgccctgtctacaccagacaacacaggag ctgggtcagattcccctcagctgcttaacaaagttcctcgaacagaaagtgcttacaaagctgccttctcggatact |
| **81** | 203060 _s_at | agctgccttctcggatactgaaaggtcgagttttctgaactgcactgattttattgcagttgaaaaacccaaagctattccaaagatttc aagctgttctgagacatcttctgatggctttacttcctgagaggcaatgtttttactttatgcataattcattgttgccaaggaataaagtga agaaacagcaccttttttaatatataggtctctctggaagagacctaaatttagaaagagaaaactgtgacaatttcatattctcattctt aaaaaacactaatcttaactaacaaaagttcttttgagaataagttacacacaatggccacagcagtttgtctttaatagtatagtgcc tatactcatgtaatcggttactcactactgcctttaaaaaaaaccagcatatttattgaaacatgagacaggattatagtgccttaac cgatatattttgtgacttaaaaaatacatttaaaactgctcttctgctctagtaccatgcttagtgcaaatgattatttctatgtacaactgat gcttgttcttattt |
| **82** | 203066 _at | ttcagttgctaatgaggctcctccgctctggacacaacccttttatagattaatttctctgccaattaacttgtcattttcagtacatattttac tattccacaccaaccataattacaacaagggattttcttatgcactcctatgcatgtgaataacatgtggtgtaattctgcttcttacaga agtattactgaaggtattatttccaatattatttggtttattatgcggatctttttatatatgcagtcccatcccttctgtgccactcaatgccat ccagacatggtttttccctccaggggcctttctctccagagggcacttcggctgcctctgcttcctctcattcgaggcccggctcttgctg acagaataggttccgttctgggcggtggttctcgagcctgccattcaaaaccaaagcaaattggagcatttctcacaacatggtattg aagttcctttttgttctcaaaagttgtgaccgtgttaaattgtactcccttagtcctgtaaggtatgttaagtgaatcgcagttacgctgtact ttta |
| **83** | 203131 _at | agaaaatttgccaatctttcctactttctatttttatgatgacaatcaaagccggcctgagaaacactatttgtgactttttaaacgattagt gatgtccttaaaatgtggtctgccaatctgtacaaaatggtcctatttttgtgaagagggacataagataaaatgatgttatacatcaat atgtatatatgtatttctatatagacttggagaatactgccaaaacatttatgacaagctgtatcactgccttcgtttatattttttaactgtg ataatccccacaggcacattaactgttgcacttttgaatgtccaaaatttatattttagaaataataaaaagaaagatacttacatgttc ccaaaacaatggtgtggtgaatgtgtgagaaaaactaacttgatagggtctaccaatacaaaatgtattacgaatgcccctgttcat gtt |
| **84** | 203240 _at | ccaggactacagaataccatcccctggtaccgtgtagttgccgaagtccagatctgccatggcaaaacggaggctgtgggccag gtccacatcttcttccaggatggatggtgacgttgactccaaacaagggtgtgtgggtgaatggtctccgagtggatctcccagctg agaagttagcatctgtgtccgtgagtcgtacacctgatggctccctgctagtccgccagaaggcaggggtccaggtgtggcttgga gccaatgggaaggtggctgtgattgtcagcaatgaccatgctgggaaactgtgtggggcctgtggaaactttgacggggaccaga ccaatgattggcatgactcccaggagaagccagcgatggagaaatggagagcgcaggacttctccccatgttatggctgatcagt catccaccaggaacgaagatttcctgaagaagacctggtccctctggaggttgcggtggctgaaggatgcatcatgtgctcctacc ctgctctaccgcttttctgggtcacagag |
| **85** | 203296 _s_at | aaagacaagcattgggtcagacccataaaccacctcccaaaggctgtcatttcattgcactggaattttgctttatcagaagcaagg aagtaagggagtcattgccttgggcctgggaatctaagtgggagacaatattaatttggatccgattaattggagattactaactgtg gacaaaagtttatctttgcacaatcaataaaaatggcatttttttagtaaattaagagcataaacaatattgctagaggtggcatgttta gtctaccaaaaacaatacttttcaggcactttagaaatatccttttagaagcagcgagtgcatgggctaattatcatcaatctttatgtat ttgttaaagaaacatctacaggatctttattggtgaccttttgta |
| **86** | 203305 _at | gtccttcacatcaccatttgagacctcagcttggcactcaggtgctgaagggtaatatggactcagccttgcaaatagccagtgcta gttctgacccaaccacagaggatgctgacatcatttgtattatgttccaaggctactacagagaaggctgcctgctatgtatttgcaag gctgatttatggtcagaatttccctctgatatgtctagggtgtgatttaggtcagtagactgtgattcttagcaaaaaatgaacagtgata agtatactgggggcaaaatcagaatggaatgctctggtctatataaccacatttctgagcctttgagactgttcctgagccttcagcac taacctatgagggtgagctggtcccctctatatatacatcatacttaactttactaagtaatctcacagcatttgccaagtctcccaatat ccaatt |
| **87** | 203343 _at | atgtgtcactgatttttttagctcaaaatcatcactgttaatttccagtcaccccaaatatggttaaaagattttttttttaatcatgaagaga aaattagtagcatttctttctctccccattatttattggtttcctcactaatcttttttttttagtccaaaagccaaaaatatttatcttggttttac attttaatttccattcttaattgtaatttttttctttaaataaggaaaccaatataatctcatgtataaaaacttaaatatttttacaagttacatat agcatcattctaaaataagaattttttttgttttctgtctgctttttttcttatgtctcttgttgagttttatattttcagtggttatttttgcttgtgttagat cattattaaaatatatccaatgtccctttgatacttgtgctctgctgagaatgtacagtttgcattaaacatcccaggtctcatccttcagg aattt |
| **88** | 203382 _s_at | gaagcgcctggcagtgtaccaggccggggcccgcgagggcgccgagcgcggcctcagcgccatccgcgagcgcctggggc ccctggtggaacagggccgcgtgcgggccgccactgtgggctccctggccggccagccgctacaggagcgggcccaggcctg ggcgagcggctgcgcgcgcggatggaggagatgggcagccggacccgcgaccgcctggacgaggtgaaggagcaggtg gcggaggtgcgcgccaagctggaggagcaggcccagcagatacgcctgcaggccgaggccttccaggcccgcctcaagag ctggttcgagcccctggtggaagacatgcagcgccagtgggccgggctggtggagaaggtgcaggctgccgtgggcaccagc gccgcccctgtgcccagcgacaatcactgaacgccgaagcc |
| **89** | 203474 _at | actgtgatataggtactctgatttaaaactttggacatcctgtgatctgttttaaagttgggggtgggaaatttagctgactagggaca aacatgtaaacctatttcctatgaaaaagttttaaatgtcccacttgaataacgtaattcttcatagtttttttaatctatggataaatgg aaacctaattatttgtaatgaattatttagacagttctaagccctgtcttctgggagttatcaattttaaagagaacttttgtgcaattcaaa tgaagtttttataagtaattgaaaatgacaacacaataacactttctgtataaaagtatatattttatgtgatttattcctactaaatgaaag tgcactactgcctcatgtaaagactcttgcacgcagagcctttaagtgactaaggaacaacatagatagtgagcatagtccccacc |
| | | tccacccctcacaatttatttgaatacttcaattgtgcctctcaa |
| **90** | 203477 _at | ttcagacagttatatcctcctttaaaccattgttgttgagtgtaagatgtccttcatgttttcttataaagtcagtgtttagaaatgttaccctttt ctaagttatatacagatcaaatgctttttcttcacgtacatccatcattgcaactgctgttcgtacacagaaacaggactgctcaaat gatcctatttgtattttctgatgctatcagactctaatgtttttttccctaaaatattattgccatcatgctttaggaatttttatatttttacacaat catattttagtatggtgtctgtttatgtaactctgacttgctggaaaagttgaaactccaaataatctgaaactagaaaagaaatagca cataattactaccttccccttggcggctctcctcccccaacccccaccccacaattttatgacttcca |
| **91** | 203638 _s_at | caacgtctaactggacttcccaagataaatggtaccagcgtcctcttaaaagatgccttaatccattccttgaggacagaccttagtt gaaatgatagcagaatgtgcttctctctggcagctggccttctgcttctgagttgcacattaatcagattagcctgattctcttcagtgaat tttgataatggcttccagactctttgcgttggagacgcctgttaggatcttcaagtcccatcatagaaaattgaaacacagagttgttct gctgatagttttggggatacgtccatctttttaagggattgctttcatctaattctggcaggacctcaccaaaagatccagcctcatacct acatcagacaaaatatcgccgttgttccttctgtactaaagtattgtgttttgctttggaaacacccactcact |
| **92** | 203645 _s_at | gccagacgctggggccatagtgagtgtgggcacaaggaagacgctgcagtgaattgcacagatatttcagtgcagaaacccc acaaaaagccacaacaggtcgctcatcccgtcagtcatcctttattgcagtcggatccttggggttgttctgttggccattttcgtcgc attattcttcttgactaaaaagcgaagacagagacagcggcttgcagtttcctcaagaggagagaacttagtccaccaaattcaat accgggagatgaattcttgcctgaatgcagatgatctggacctaatgaattcctcaggaggccattctgagccacactgaaagga aaatgggaatttataacccagtgagttcagcctttaagataccttgatgaagacctggactattgaatggagcagaaattcacctctc tcactgactattacagttgcatttttatggagttcttcttctcctaggattcc |
| **93** | 203680 _at | ttttcattgttcattgatatgctcagtatgctgccacataagatgaatttaattatattcaaccaaagcaatatactcttacatgatttctagg ccccatgacccagtgtctagagacattaattctaaccagttgtttgcttttaaatgagtgatttcatttgggaaacaggtttcaaatgaat atatatacatgggtaaaattactctgtgctagtgtagtcttactagagaatgtttatggtcccacttgtatatgaaaatgtggttagaatgtt aattggataatgtatatataagaagttaaagtatgtaaagtataacttcagccacattttagaacactgtttaacattttgcaaaacctt cttgtaggaaaagagagctctctacatgaagatgacttgttttatatttcagatttatttaaaagccatgtctgttaaacaagaaaaaa cacaaaagaactccagattcctggttcatcattctgtattcttactcacttttcaagtt |
| **94** | 203729 _at | atcctcattcttatactgcttttcgtggccactttggacaagtcctggtggactctccctgggaaagagtccctgaatctctggtacgact gcacgtggaacaacgacaccaaaacatgggcctgcagtaatgtcagcgagaatggctggctgaaggcggtgcaggtcctcatg gtgctctccctcattctctgctgtctctccttcatcctgttcatgttccagctctacaccatgcgacgaggaggtctcttctatgccaccggc ctctgccagctttgcaccagcgtggcggtgtttactggcgccttgatctatgccattcacgccgaggagatcctggagaagcacccg cgagggggcagcttcggatactgcttcgccctggcctgggtggccttccccctcgccctggtcagcggcatcatctacatccaccta cggaagcgggagtgagcgccccgc |
| **95** | 203748 _x_at | caagtatcggggctctgctatcaaggtgcaaagtccttcgtggatgcaacctcaaccatatattctacagcaccctggtgccgtgtta actccctcaatggagcacaccatgtcactacagcccgcatcaatgatcagccctctggcccagcagatgagtcatctgtcactagg cagcaccggaacatacatgcctgcaacgtcagctatgcaaggagcctacttgccacagtatgcacatatgcagacgacagcggt tcctgttgaggaggcaagtggtcaacagcaggtggctgtcgagacgtctaatgaccattctccatatacctttcaacctaataagtaa ctgtgagatgtacagaaaggtgttcttacatgaagaagggtgtgaaggctgaacaatcatggatttttctgatcaattgtgctttagga aattattgacagttttgcacaggttcttgaaaacgttatttataatgaaatcaactaaaactatttttgctataagttctataaggtgcataa aacccttaaattcatctagtagctgttccccc |
| **96** | 203766 _s_at | caccttgggtctgtgtcacttgtaggtttctctgcctccaggttgcctcaacagcaggaggcacagcagtttcaccatctttgaggtgag ggtggggtgccccagctaggaagcaagatcgctgtgctaggtctgaccaaaaccagagggcagtctagtcctgggggtaaagc cctcagatcccagggtacactcttctccattccctccacccacttgcctgtcaccccagtcacctaagcaatcactgggcccagagg agaggagacagacacacactggctcctggacctaaagggtatgagctggagctaaggccagctagagcttccactgtcagccc tcactgtcagccccactgcaccccccctgtgcctgctgggcactgggcactagctagatgctttaggttgcttcagctgatccttcaact ctgtgaggtg |
| **97** | 203881 _s_at | tatgtgacgctggaccttttcttacccaaggatttttaaaactcagatttaaaacaaggggttactttacatcctactaagaagtttaagt aagtaagtttcattctaaaatcagaggtaaatagagtgcataaataatttgttttaatctttttgtttttcttttagacacattagctctggagt gagtctgtcataatatttgaacaaaaattgagagctttattgctgcattttaagcataattaatttggacattatttcgtgttgtgttctttataa ccaccgagtattaaactgtaaatcataatgtaactgaagcataaacatcacatggcatgttttgtcattgttttcaggtactgagttctta cttgagtatcataatatattgtgttttaacaccaacactgtaacatttacgaattattttttttaaacttcagttttactgcattttcacaacatat cagacttcaccaa |
| **98** | 203908 _at | tgtgtaatacgccaaccagtcaagttgtgttttggccagagatttagatatgtccaatttcctggctcatttcattgtgctctatgggtacgt ataaaaagcaagaattctgtttcctaggcaaacattgcaactcagggctaaagtcatccagtgaaacttttagagccagaagtaac tttgtcccagtcctacaatgtgaaaagagtgaatagttgcctctttttagccattttcatggctggtacatattcgtacgcattacttttcaga atcaatacgcactttcagatattcttatttttattctcttaagtctttattaactttggagagagaaatgatgcatctttttattttaaatgaagta gatcaacatggtggaacaaaatgataaagaacagaaaacatttcaatatattactaataactttttccaatataaatcctaaaattcct ataacatagtattttacagtttatgaagcttctattgtgacttttatg |
| **99** | 203913 _s_at | aacactcacatacaagttcatactttaaaagaggaaagctacttaacaatgacaaatatttcacaataataatttttacttatataccat ctttcaactgaacatttcagttcttccaagagcttcttagagtagtatattttgggggcagtcaaggaataaactacagtgtaaacatat cccagatgaaaactgctgtatggaaaatgacagaaagtaactgattgacactgttgattcacagttcangcctcctatctgggaa |
| | | agacatttctttcctctgctcactttaagaacttttaccgactccaaaaatctcaggaattaaacttttaacagttacagcaataaagaat agttagtactccaaaaatattatatttaagatgctcaacaagaaaaaaatgcaaatgtaatattttttcaaattacttctttattgacttgt cca |
| **100** | 203914 _x_at | gatttccaaatcagtcgttttctcaaaaaaatatcgtataagtgactcatcctgtctgctaactccagacctcccagcttgaagccaaat ctttccatgtgagattgatatggatttcctagaagtactggaatgttgtcatatcttgccctattttaattctgctatagaaaacaattgcctt cacttttaaggagtaatttgaatattaataactctggtctagatttcatataatgtattaaagacaaagtagtgaacatcaatgaacatc tgatagagataaactgtaatcaggcataagcttgtttgtatgttctggcagtgactaatcagtaaatgatgtcggtttgcccagtatcact tatcttctgtatttttcctctgtcgtgtaaatagtataacctttcatttatggacaatttttggactagtagccttcaatatacattctgctt |
| **101** | 203951 _at | acgcacacaactactacaattccgcctaggtccacaaggccttcactgttttccccccaagggaggctgctgctgctcttggctgga gccagccagggccagccgacccccctctccctgcatggcatcctccagcccctgtagaactcaacctctacagggttagagtttgg agagagcagactggcgggggggcccattgggggggaaggggaccctccgctctgtagtgctacagggtccaacatagaacaggg tgtccccaacagcgcccaaaggacgcactgagcaacgctattccagctgtcccccccactccctcacaagtgggtacccccagga ccagaagctcccccagcaaagcccccagagcccaggctcggcctgcccccaccccattcccgcagtgggagcaaactgcatg cccagagacccagcggacacacgcggtttggtttgcagcgactggcatac |
| **102** | 203963 _at | ccaaatttcatttcagccacttctgcaggatccctactgccaacctggaatggagacttttatctacttctctctctgaagatgtcaaat cgtggtttagatcaaatatatttcaagctataaaagcaggaggttatctgtgcaggggctggcatcatgtatttaggggcaagtaat aatggaatgctactaagatactccatattcttccccgaatcacacagacagtttctgacaggcgcaactcctccatttcctcccgcag gtgagaaccctgtggagatgagtcagtgccatgactgagaaggaaccgacccctagttgagagcaccttgcagttccccgagaa ctttctgattcacagtctcatttgacagcatgaaatgtcctcttgaagcatagctttttaaatatctttttccttctactcctccctctgactcta agaattctctcttctggaatcgcttg |
| **103** | 203980 _at | gtgtgatgcttttgtaggtacctggaaacttgtctccagtgaaaactttgatgattatatgaaagaagtaggagtgggctttgccacca ggaaagtggctggcatggccaaacctaacatgatcatcagtgtgaatggggatgtgatcaccattaaatctgaaagtacctttaaa aatactgagatttccttcatactgggccaggaatttgacgaagtcactgcagatgacaggaaagtcaagagcaccataaccttag atgggggtgtcctggtacatgtgcagaaatgggatggaaaatcaaccaccataaagagaaaacgagaggatgataaactggtg gtggaatgcgtcatgaaaggcgtcacttccacgagagtttatgagagagcataagccaaggacgttgacctggactgaagttcg cattgaactctacaacattctgtgggatatattgttcaaaaagatattgttttccctgatttagcaagca |
| **104** | 204018 _x_at | ggtccccacagactcagagagaacccaccatggtgctgtctcctgccgacaagaccaacgtcaaggccgcctggggtaaggtc ggcgcgcacgctggcgagtatggtgcggaggccctggagaggatgttcctgtccttccccaccaccaagacctacttcccgcactt cgacctgagccacggctctgcccaggttaagggccacggcaagaaggtggccgacgccctgaccaacgccgtggcgcacgtg gacgacatgcccaacgcgctgtccgccctgagcgacctgcacgcgcacaagcttcgggtggacccggtcaacttcaagctccta agccactgcctgctggtgaccctggccgcccacctccccgccgagttcacccctgcggtgcacgcctccctggacaagttcctggc ttctgtgagcaccgtgctgacctccaaataccgttaagctg |
| **105** | 204034 _at | gcccaggctgacttacacattgaggatggagactccatccgcttcgggcgcttcgcgttggagaccagggccagccctggccac accccaggctgtgtcaccttcgtcctgaatgaccacagcatggccttcactggagatgccctgttgatccgtgggtgtgggcggaca gacttccagcaaggctgtgccaagaccttgtaccactcggtccatgaaaagatcttcacacttccaggagactgtctgatctaccct gctcacgattaccatgggttcacagtgtccaccgtggaggaggagaggactctgaaccctcggctcaccctcagctgtgaggagtt tgtcaaaatcatgggcaacctgaacttgcctaaacctcagcagatagactttgctgttccagccaacatgcgctgtggggtgcaga cacccactgcctgatctcacttctgtcagatgctcccatccactattaatgcactaggtgggaggagagggcggcaatgacactgc acct |
| **106** | 204036 _at | aattgccacatttcttatggcattaaaaattttacaaaaacataattttaatggctatattatattccatttaatggatgcaactcangttta tttaaccattcccatngttgttaactatttaggttgtttctaattttcattattataaagttgcagaaatttggtgtacataaaactgtctccatat aattgattattaggatatattcccatgaaggattcntttttttaaaaaaatgtgaaatntcatcttgtacttacacctttcatgnaaagggat ttcctgcttttgtactgcatgggtggcagttgtgaggaaaagccagtcaaatgacctttttacaaaagaaatgcagtggtcacttcagtt gagagtgactttttaatacaacaagatcaactagaagaattcaactgtctcaagaatcaaggtaccccaatatatctcgcaattcca aactttgtttgagggactcgttatccagctcttggtagccacacctgcaatgta |
| **107** | 204069 _at | aagccttacagttatcctgcaagggacaggaaggtctgatttgcaggatttttagagcattaaaataactatcaggcagaagaatctt tcttctcgcctaggatttcagccatgcgcgcgctctctctctttctctctcttttcctctctctccctctttctagcctggggcttgaatttgcatgt ctaattcatttactcaccatatttgaattggcctgaacagatgtaaatcgggaaggatgggaaaaactgcagtcatcaacaatgatta atcagctgttgcaggcagtgtcttaaggagactggtaggaggaggcatggaaaccaaaaggccgtgtgtttagaagcctaattgtc acatcaagcatcattgtccccatgcaacaaccaccaccttatacatcacttcctgttttaagcagctctaaaacatagactgaagattt atttttaatatgttgactttatttctgagcaaagcatcggtcatgtgtgtatttttttcatagtcccaccttggagcatttatg |
| **108** | 204083 _s_at | gaaggcggctgatgagagcgagagaggaatgaaggtcatcgaaaccgggccatgaaggatgaggagaagatggaactgc aggagatgcagctgaaggaggccaagcacatcgctgaggattcagaccgcaaatatgaagaggtggccaggaagctggtgat cctggaaggagagctggagcgctcggaggagagggctgaggtggccgagagccgagccagacagctggaggaggaacttc gaaccatggaccaggccctcaagtccctgatggcctcagaggaggagtattccaccaaagaagataaatatgaagaggagatc aaactgttggaggagaagctgaaggaggctgagacccgagcagagtttgccgagaggtctgtggcaaagttggagaaaacca tcgatgac |
| **109** | 204122 _at | catgggggggacttgaaccctgcagcaggctcctgctcctgcctctcctgctggctgtaagtggtctccgtcctgtccaggcccaggc ccagagcgattgcagttgctctacggtgagcccgggcgtgctggcagggatcgtgatgggagacctggtgctgacagtgctcattg ccctggccgtgtacttcctgggccggctggtccctcgggggcgaggggctgcggaggcagcgacccggaaacagcgtatcact gagaccgagtcgccttatcaggagctccagggtcagaggtcggatgtctacagcgacctcaacacacagaggccgtattacaa atgagcccgaatcatgacagtcagcaa |
| **110** | 204130 _at | tccccagcagtggctcggtgagccatgtgcacctatggcccagccactgcagcacaggaggctccgtgagccttggttcctcccc gaaaacccccagcattacgatcccccaagtgtcctggaccctggcctaaagaatcccacccccacttcatgcccactgccgatgc ccaatccaggcccggtgaggccaaggtttcccagtgagcctctgcgcctctccactgtttcatgagcccaaacaccctcctggcac aacgctctaccctgcagcttggagaactccgctggatgggagtctcatgcaagacttcactgcagcctttcacaggactctgcagat agtgcctctgcaaactaaggagtgactaggtgggttggggacccccctcaggattgtttctcggcaccagtgcctcagtgctgcaatt gagggctaaatcccaagtgtctcttgactggctcaagaattagggcccca |
| **111** | 204135 _at | cagaccggcagtcatcatggcagtttcagcgttcaaacagcaatagctcaagtgtgataactactgaggataataaaatccacatt cacttaggaagtccttacatgcaagctgtagccagcccttcagcaccactgcaggataaccgaactcaaggcttaattaacgggg cactaaacaaaacaaccaataaagtcaccagcagtattactatcacaccaacagccacacctcttcctcgacaatcacaaattac agtaagtaatatatataactgaccacgctcaccctcatccagtccatactgatatttttgcaaggaactcaatcctttttaatcatccctc catatcccccaagactgactgaactcgtactttgggaaggtttgtgcatgaactatacaagagtatctgaaactaactgttgcctgcat agtcatatcgagtgtgcacttactgtatatcttttcat |
| **112** | 204326 _x_at | gcgtgttttcctcttgatcgggaactcctgcttctccttgcctcgaaatggaccccaactgctcctgctcgcctgttggctcctgtgcctgt gccggctcctgcaaatgcaaagagtgcaaatgcacctcctgcaagaagagctgctgctcctgctgccctgtgggctgtgcnaagt gtgcccagggctgcatctgcaaagggacgtcagacaagtgcagctgctgtgcctgatgccaggacagctgtgctctcagatgtaa atagagcaacctatataaacctggattttttttttttttttgtacaaccctgacccgtttgctacatcttttttctat |
| **113** | 204388 _s_at | ttccacatcagtaactgccctggggtttgtgctgtacaaatacaagctcctgccacggtcttgaagttctgttcttatgctctctgctcact ggttttcaataccaccaagaggaaaatattgacaagtttaaaggctgtgtcattgggccatgtttaagtgtactggatttaactacctttg gcttaattccaatcattgttaaagtaaaaacaattcaaagaatcacctaattaatttcagtaagatcaagctccatcttatttgtcagtgt agatcaactcatgttaattgata[?]aataaagccttgtgatcact |
| **114** | 204389 _at | ctttgcgggcacagagactgccacaaagtggagcggctacatggaaggggcagttgaggctggagaacgagcagctaggga ggtcttaaatggtctcgggaaggtgaccgagaaagacatctgggtacaagaacctgaatcaaaggacgttccagcggtagaaat cacccacaccttctgggaaaggaacctgccctctgtttctggcctgctgaagatcattggatttccacatcagtaactgccctggggt ttgtgctgtacaaatacaagctcctgccacggtcttgaagttctgttcttatgctctctgctcactggttttcaataccaccaagaggaaa atattgacaagtttaaaggctgtgtcattgggccatgtttaagtgtactggatttaactacctttg |
| **115** | 204438 _at | taattgcttgttttctagcctggcaagatattttcataaaagagggataacaatgctgattactaccttttaaaatattttagataaatgca cagcaccacagcaccacatctaagcattagtgatgggtagctgatgtcagcttcatgtggatttaagcactctagaaacaatgaag cttcttggcatattttaaggagctcccaaaatgtgttacctattaaattgtaactcagcaagtagaagaccattgaaaagtcaggtac aaatttcctcaagtggcataaaaatgtagtcagttttctcttttaccagtttttatttccactccaattatttagaactttatttgtacatgtgca gaagaataaggcagctgagaatcttgtttcccccaagagagttttacaggctgagtgttgcaaatgtgttctttgtcctgttatatgtat |
| **116** | 204457 _s_at | gcactgccatgtatgaaagtctctttatgatgtttgttttttgtcatttttgttctttacatcaagaaattttatgtttaaatatgcggagaatgta tattgcctctgctcctatcagggttgctaaaccctggtacatcgtatataaaatgtattaaaactggggtttgttaccagttgctgtactttg tatatagaatttttataaattgtatgcttcagaaataatttatttttaaaaagaaattaaaagttttaaactcacatccatattacacctttcc cccctgaaatgtatagaatccatttgtcatcaggaatcaaaacccacagtccattgtgaagtgtgctatatttagaacagtcttaaaat gtacagtgtattttatagaattgaagttaacattcttattttcaagagaatttatggacgttgtagaaatgtacaaatgcatttccaaactg ccttaaacgttgtatt |
| **117** | 204508 _s_at | gggactgtctgtcaaaagactctgtatatcttttgtggatgagttttgtgagagaacagagagaccattgtacctggcacaagggctct tcatgaaaagggagacttactgggaggtgcaagacagtggcatttctcctctcctcttgctgctcagcacagccctggattgcagcc ccgaggctgagaccagacaaagcccgggaggcagaaagatgctccaagaaccaacactatcaatgtctttgcaaatcctcaca ggattcctgtgggtccagctttggaactgggaaacctttcttcggatccgcactcattccactgatgccagctgcccctgaaggatgc cagtactgtggtgtgtgagtctcagcagccgcccacacgctcctaactctgctgcatggcagatgcctaggtgga |
| **118** | 204532 _x_at | attaatcagccccagagtgctttaaaaaattctcttaaataaaaataatagactcgctagtcagtaaagatatttgaatatgtatcgtgc cccctccggtgtctttgatcaggatgacatgtgccatttttcagaggacgtgcagacaggctggcattctagattacttttcttactctga aacatggcctgtttgggagtgcgggattcaaaggtggtcccaccgctgcccctactgcaaatggcagttttaatcttatcttttggcttct gcagatggttgcaattgatccttaaccaataatggtcagtcctcatctctgtcctgcttcataggtgccaccttgtgtgtttaaa |
| **119** | 204570 _at | ctacgcgtgtccttgggcggagaagggaggtgactccggcggaagaggacaaggcagaatgcaggcccttcgggtgtcccag gcgctgatccgctccttcagctccaccgcccggaaccgctttcagaaccgagtgcgcgagaaacagaagctcttccaggaggac aatgacatcccgttgtacctgaagggcggcatcgttgacaacatcctgtaccgagtgacaatgacgctgtgtctgggcggcactgt ctacagcttgtactcccttggctgggcctccttccccaggaattaagaccaagaagcctgggggcctgagagact |
| **120** | 204607 _at | tggtgacacaaacagccttttcccaggtacttggtacctggagcgagtggacgagcagcatcgccgaaagtatgcccggcgtccc gtctaaaggtgttctgcagatccatggaaagcttcctgggaaacgtatgctagcagagcttctccccgtgaatcatatttttaagatcc cactcttagctggtaaatgaatttgaatcgacatagtagccccataagcatcagccctgtagagtgaggagccatctctagcgggc |
| | | ccttcattcctctccatgctgcaatcactgtcctgggcttatggtgcctatggactaggggtcctttgtgaaagagcaagatggagcaa tggagagaagacctcttcctgaatcactggactccagaaatgtgcatgcagatcagctgttgccttca |
| **121** | 204673 _at | ttgatgccagcatttgcatcccggctccatcacattcatgcccaatggatgctgcaagacctgcacccctcgcaatgagaccagg gtgccctgctccaccgtccccgtcaccacggaggtttcgtacgccggctgcaccaagaccgtcctcatgaatcattgctccgggtcc tgcgggacatttgtcatgtactcggccaaggcccaggccctggaccacagctgctcctgctgcaaagaggagaaaaccagcca gcgtgaggtggtcctgagctgccccaatggcggctcgctgacacacacctacacccacatcgagagctgccagtgccaggaca ccgtctgcgggctccccaccggcacctcccgccgggcccggcgctcccctaggcatctggggagcgggtgagcggggtgggc acagccccccttcactgccctcgacagctttacctcccccggaccctctgagcctcctaagctcggcttcctctcttcagatatttattgtct gagtctttgttcagtccttg |
| **122** | 204688 _at | cttgcttatatcatgtgctgccgacgggaaggcgtggaaaagagaaacatgcaaacaccagacatccaactggtccatcacagt gctattcagaaatctaccaaggagcttcgagacatgtccaagaatagagagatagcatggcccctgtcaacgcttcctgtgttcca ccctgtgactggggaaatcatacctcctttacacacagacaactatgatagcacaaacatgccattgatgcaaacgcagcagaac ttgccacatcagactcagattccccaacagcagactacaggtaaatggtatccctgaagaaagaaaactgactgaagcaatgaa tttataatcagacaatatagcagttacatcacatttcttttctcttccaataatgcatgagcttttctggcatatgtt |
| **123** | 204697 _s_at | cctcccgggaggacagccttgaggcgggcctgcccctccaggtccgaggctaccccgaggagaagaaagaggaggagggc agcgcaaaccgcagaccagaggaccaggagctggagagcctgtcggccattgaagcagagctggagaaagtggcccacca gctgcaggcactacggcggggctgagacaccggctggcagggctggccccagggcaccctgtggccctggctctgctgtcccct tggcaggtcctggccagatggcccggacgctgcttccggtagggaggcagcctccagcctgcccaagcccaggccaccctatc gccccctacgcgccttgtctcctactcctgactcctacctgccctggaacatcctttgcagggcagccccacaactttaaacattgac gattccttctctgaacacaggcagctttctagaagtttcccttcctccatcctatccactgggcacaactgcaataacttctgaccttttgg tgaaagctgagaactcctgactgtaacatattctg |
| **124** | 204719 _at | gcaggccatactggttccattgttctgtataatactgaataaataaatttacttttacatgatcgtataagtttctagataagataaacaa attctgtttaaatttttttaataaaaatcttaaaacactttttttctaacctagactgagaaattcatgtttactttctaggtgtatgatactttgt aaagttgatactttcctaagaatttaacatgtcatatttttgaaatagatttaagtgtgcttcttattgctaaaaatactaaatgtcatgggtc atagtatctgatatcaatatcgttgataacatatccacaggtaacaccatgatgtaggcataaatggaaaacaaaaaccctactattt caaatatattgtactttttttatttctgtaagccaactgtgtgccatttcactggacttttaaatctagactttagtgatgtctacattgtaaatg atcttttgtggatatttgtcacttggtttcagaaagttcacaaatgtagcaacagctcacatgactgagt |
| **125** | 204745 _x_at | tcccttctcgcttgggaactctagtctcgcctcgggttgcaatggaccccaactgctcctgtgccgctggtgtctcctgcacctgcgcca gctcctgcaagtgcaaagagtgcaaatgcacctcctgcaagaagagctgctgctcctgctgccctgtgggctgtgccaagtgtgcc caaggctgcatctgcaaaggggcatcggagaagtgcagctgctgcgcctgatgtcgggacagccctgctcccaagtacaaata gagtgacccgtaaaatctaggatttttttgtttttttgctacaatcttgacccct |
| **126** | 204818 _at | aaaggctggcatcttatggctcatcaaaggcggctgtgaccatgttctcatcagttatgagactggagctttccaagtggggaattaa agttgcttccatccaacctggaggcttcctaacaaatatcgcaggcaccagtgacaagtgggaaaagctggagaaggacattctg gaccacctccccgctgaggtacaggaagactacggccaggactacatcttagcacagcggaatttcctcctattgatcaactcgtt agccagcaaggacttctctccggtgctgcgggacatccagcatgctatcttggcgaagagcccttttgcctattacacgccaggga aaggcgcttacttgtggatctgccttgctcactatttgcctattggca |
| **127** | 204834 _at | ggtgtcgaactatatgccttgtatgatcagttttatgtggctaatgagtttctcaaatatcgtttacacgttggtaactataatggcacagct ggagatgcattacgtttcaacaaacattacaaccacgatctgaagtttttcaccactccagataaagacaatgatcgatatccttctg ggaactgtggctgtactacagttcaggctggtggtttgatgcatgtctttctgcaaacttaaatggcaaatattatcaccaaaaatac agaggtgtccgtaatgggattttctggggtacctggcctggtgtaagtgaggcacaccctggtggctacaagtcctccttcaaagag gctaagatgatgatcagacccaagcactttaagccataaatcactctgttcattcctccaggtattcgttatctaatagggcaattaatt ccttcagcacttt |
| **128** | 204894 _s_at | ccaggaggggctgtccaatcacattcaggcatgcgaatgagctgggccctgggtgaggtgggggtctggcctagtggggaggg gcctggcctgggtggggcagggcctggcctggtccaggcttgggctccattcccatcactgctgtccctcctgaggtctggattggg gatggggacaaagaaatagcaagagatgagaaacaacagaaactttttctctaaaggactggttaaatcaattctgatacagcc ttacaatacaatagtatgcagctaaaaaataattgtatgtctttatatactaatatgtaataatcttcaggtgaaaaggcaagccaca gaaatgtgtatagcgcacttcccatttgtgtttcagaaaggagtagaatataaacacataattgcttatgtatgcctattcagaataaat gggtaacactgattacttttgggaggggaaccagtaggttgaggacaggagagggaagggtcttaacacttacaccctttgtacat tttg |
| **129** | 204895 _x_at | aggagcccaggaacgacgtggtcttccagcccatttccggggaagacgtgcgcgatgtgacagccctgaacgtgagcacgctg aaggcttacttcagatgcgatggctacaagggctacgacctggtctacagcccccagagcggcttcacctgcgtgtccccgtgca gtaggggctactgtgaccatggaggccagtgccagcacctgcccagtgggccccgctgcagctgtgtgtccttctccatctacacg gcctggggcgagcactgtgagcacctgagcatgaaactcgacgcgttcttcggcatcttctttggggccctgggcggcctcttgctg ctgggggtcgggacgttcgtggtcctgcgcttctggggttgctccggggccaggttctcctatttcctgaactcagctgaggccttgcct tgaaggggcagctgtggcctaggctacctcaagactcacctcatccttaccgcacatttaaggcgccattgcttttgggagact |
| **130** | 204897 _at | agcagcttattgtttctctgaaagtgtgtagttttactttcctaaggaattaccaagaatatcctttaaaatttaaaaggatggcaagtt gcatcagaaagctttattttgagatgtaaaaagattcccaaacgtggttacattagccattcatgtatgtcagaagtgcagaattggg |
| | | gcacttaatggtcaccttgtaacagttttgtgtaactcccagtgatgctgtacacatatttgaagggtctttctcaaagaaatattaagca tgttttgttgctcagtgtttttgtgaattgcttggttgtaattaaattctgagcctgatattgatatg |
| **131** | 204931 _at | gagaacgggtacattcacccggtcaacctgacgtggccctttatggtggccgggaaacccgagagtgacctgaaagaagtggtg accgcgagccgcttatgtggaaccaccgcgtcctgaccttggaggtgcgagtctgggaaaggcgcgctcccgggggggagcggg ccccgggaaggcgacccctgccctcagtgctctctgtctctgcttccccctcgcaatgctcctctctctgtcccaccccgcgagaaca ctttacaacgacgaggagattcgtttccaaaccagaggagatcaattgtacttacaaagattcccatctatttaactttattaacttctac cgtgaatgactctgcaagccttgctggtcca |
| **132** | 204938 _s_at | ttctctcgaccacttaaaacttcagacttcctgtcctgctggtatcatggagaaagtccaatacctcactcgctcagctataagaagag cctcaaccattgaaatgcctcaacaagcacgtcaaaagctacagaatctatttatcaatttctgtctcatcttaatatgtctcttgctgatc tgtatcatcgtgatgcttctctgaagttctgctacaacctctagatctgcagcttgccacatcagcttaaa |
| **133** | 204939 _s_at | tggcccatctattacatctacagctgacccttgaacatgggggttaggggagctgacaattcgtgggtccgcaaaatcttaactacct aatagcctactattgaccataaaccttactgataacataaacagtaaattaacacatattttgcgtgttatatgtattatacactatattcc tacaataaagt |
| **134** | 204940 _at | aaatctgttctaagacatatgatcaacagatgagaactggtggttaatatgtgacagtgagattagtcatatcactaatatactaaca acagaatctaatcttcatttaaggcactgtagtgaattatctgagctagagttacctagcttaccatactatatctttggaatcatgaaac cttaagacttcagaatgatttgcaggttgtcttccattccagcctaacatccaatgcaggcaaggaaaataaaagatttccagtgac agaaaaatatattatctcaagtatttttaaaaatatatgaattctctctccaaatattaactaattattagattatatttgaaatgaacttgtt ggcccatc |
| **135** | 204955 _at | ggacaaagagcgctatgtctccctggtgatgcctgtggccctgttcaacctgattgacacttttcccttgagaaagaagagatggtc ctacaagccgaaatgagccagacctgtaacacctgacatgatggttcctctcttggcaattcctcttcattgtctacatagtgacatgc acacgggaaagccttaaaaatatccttgatgtacagattttatttgtaattttaaaagtctattttattatgagctttctttgcacttaaaaatt agcatgctgctttttgtacttggaagtgtttcaaaaaattatatgaccatatttactctttctaacctttctttactccatcatggctggttgattt gtagagaaattagaacccataaccatacacaggctatcaacatgttattcaatgtgacacctaactcttttc |
| **136** | 205097 _at | tactcatgcctttttgtttaggataaataggtaagcacaaagagctcttcaaaatcagaaaaaacaataggagtccttccttgtcttttct gtgatctctgtccttgtttctgagactttctctaccattaagctctattttagctttcagttattctagtttgtttcccatggaatctgtcctaaact ggtgtttttgtcagtgacagtcttgccagtcagcaatttctaacagcattttaaatgagtttgatgtacagtaaatattgatgacaatgac agcttttaactcttcaagtcacctaaagctattatgcaggaggatttagaagtcacattcataaaacccaagngctatgggtgtattatt catgatagctggcccacaggtcatgaattgag |
| **137** | 205112 _at | agaagctcaccaagtcaactaaacagccccgaggacttacatcaccttctcagctcttgacctcagaaagtatccaaaccaagg aggagaaacctgtgggtggcttgtcctccagtgacacaatggattaccgacagtgactaagggcagcatgtttaacccaggtgaa gatct |
| **138** | 205200 _at | tttctggccttcacccagacgaagaccttccacgaggccagcgaggactgcatctcgcgcggggcaccctgagcacccctcag actggctcggagaacgacgccctgtatgagtacctgcgccagagcgtgggcaacgaggccgagatctggctgggcctcaacga catggcggccgagggcacctgggtggacatgaccggcgcccgcatcgcctacaagaactgggagactgagatcaccgcgca acccgatggcggcaagaccgagaactgcgcggtcctgtcaggcgcggccaacggcaagtggttcgacaagcgctgccgcgat cagctgccctacatctgccagttcgggatcgtgtagccggc |
| **139** | 205259 at | gcagactgggagttgctagcaaacaaatggcttacttacaaaagcagcttttagttcagacttagtttttataaaatgagaattctgact tacttaaccaggtttgggatggagatggtctgcatcagctttttgtattaacaaagttactggctctttgtgtgtctccaggtaactttgcttg attaaacagcaaagccatattctaaattcactgttgaatgcctgtcccagtccaaattgtctgtctgctcttattttgtaccatattgctctt aaaaatcttggtttggtacagttcataattcaccaaaaagttcatataatttaaagaaacactaaattagtttaaaatgaagcaatttat atctttatgcaaaaacatatgtctgtctttgcaaagga |
| **140** | 205267 _at | gcacccagccttttaccatgtgggtttctttagtgtcttaaaagcgtccataagccaccattctgtggaaccaaggccccctccacgca aacaccctccctcctggggacctctggagcctcagccagaagtaccattaggtttaattttaatttgttttgctggagaaacatcaggtt tgtaggagactgagttgttagcaggtgtgcttagctcttgatagtgaacgtgtaccttgggaactggctcacccacctgctaatagcac catcgtcactattaagcagacatttcagttggtagaatccatgtagaagtcatggacttttctgggaaatgacttttctgggaaatgaca gtttctttgacatattttctttgcccactt |
| **141** | 205382 _s_at | cgccatcaccgagcgcttgatgtgcgcggagagcaatcgccgggacagctgcaagggtgactccgggggcccgctggtgtgcg ggggcgtgctcgagggcgtggtcacctcgggctcgcgcgtttgcggcaaccgcaagaagcccggatctacacccgcgtggcg agctatgcggcctggatcgacagcgtcctggcctagggtgccggggcctgaaggtcagggtcacccaagcaacaaagtcccga gcaatgaagtcatccactcctgcatctggttggtctttattgagcacctactatatgcagaaggggag |
| **142** | 205403 _at | gggccacgccaggaatattcagaaaataatgagaactacattgaagtgccattgattttgatcctgtcacaagagaggatttgcac atggattttaaatgtgttgtccataataccctgagttttcagacactacgcaccacagtcaaggaagcctcctccacgttctcctgggg cattgtgctggccccactttcactggccttcttggttttgggggaatatggatgcacagacggtgcaaacacagaactggaaaagc agatggtctgactgtgctatggcctcatcatcaagactttcaatcctatcccaa |
| **143** | 205412 _at | gctgctctggttctcatgacggcagatgcagcgaagaggctcaatgttacaccactggcaagaatagtagcatttgctgacgctgct gtagaacctattgattttccaattgctcctgtatatgctgcatctatggttcttaaagatgtgggattgaaaaagaagatattgcaatgt gggaagtaaatgaagcctttagtctggttgtactagcaaacattaaaatgttggagattgatcccaaaaagtgaatatcaatggag |
| | | gagctgtttctctgggacatccaattgggatgtctggagccaggattgttggtcatttgactcatgccttgaagcaaggagaatacggt cttgccagtatttgcaatggaggaggaggtgcttctgccatgctaattcagaagctgtagacaacctctgctatttaaggagacaacc ctatgtgaccagaa |
| **144** | 205433 _at | ggaaagcaggattccatcgctggaacaattacatgatggactggaaaaatcaatttaacgattacactagcaagaaagaaagtt gtgtgggtctctaattaatagatttaccctttatagaacatattttcctttagatcaaggcaaaaatatcaggagctttttacacacctact aaaaaagttattatgtagctgaaacaaaaatgccagaaggataatattgattcctcacatctttaacttagtattttacctagcatttcaa aacccaaatggctagaacatgtttaattaaatttcacaatataaagttctacagttaattatgtgcatattaaaacaatggcctggttca atttctttctttccttaataaatttaagtttttcccccaaaattatcagtgctctgcttttagtcacgtgtattttcattaccactcgtaaaaag gtatctttttaaatgaattaaatattgaaacactgtacaccatagtttaca |
| **145** | 205464 _at | gagggtgatgccatctaaccctgcccctgtccaccccgggtgggtgaaactcactgagcagccaagactgttgcccgaggactc actgtatggtgccctctccaaagggtcggagggtagctctccaggccagagcttgtgtccttcaacagagaggccagcggcaac tggtccgttactggccaagggctctgaagaatcaacggtgctggtacaggatacaggaataaattgtatcttcacctggttcctaccc tcgtccctacctgtcctgatcctggtcctgaagacccctcggaacaccctctcctggtggcaggccacttccctcccagtgccagtctc catccaccccagagaggaacaggcgggtgggccatgtggttttctccttcctggccttggctggcctctggggcaggggtggtgga gagatggaagggcatcaggtgtagggaccctgccaagtggcacctgattactctag |
| **146** | 205480 _s_at | ttaatgtgccaaggagccgttttctgcctgtcaaaaccacatcagatctcttgctggtgatgtcaaacctctatagtcttaatgcaggat ctctgacaatgagtgaaaagcgggaatttcctacagtgcccttggttaaattaggcagttcttttacgaaggttcaagattatctaaga agatttgaaagtataccagatatgcttgaattggatcacctcacagtttcaggagatgtgacatttggaaaaaatgtttcattaaaggg aacggttatcatcattgcaaatcatggtgacagaattgatatcccacctggagcagtattagagaacaagatagtgtctggaaacct tcgcatcttggaccactgaaatgaaaaatactgtggacacttaaataatgggctagtttcttacaatgaaatgttctctaggatttaggc actaaaaggtactttactatgttactgtaccctgc |
| **147** | 205547 _s_at | caagaatgatgggcactaccgtggagatcccaactggtttatgaagaaagcgcaggagcataagagggaattcacagagagc cagctgcaggagggaaagcatgtcattggccttcagatgggcagcaacagaggggcctcccaggccggcatgacaggctacg gacgacctcggcagatcatcagttagagcggagagggctagccctgagcccggcgctcccccagctccttggctgcagccatcc cgcttagcctgcctcacccacacccgtgtggtaccttcagccctggccaagctttgaggctctgtcactgagcaatggtaactgcac ctgggcagctcctccctgtgcccccagcctcagcccaacttcttacccgaaagcatcactgccttggcccctccctcccggcggccc ccatcacctctactgtctcctccctgggctaagcaggggagaagcgggctgggggtagcctggatgtgggcgaagtccactgtcct ccttggcggcaaaagcccatt |
| **148** | 205554 _s_at | tcaaagacttcgtgattatccccctgcacaccaccccagagacatccgttaaggagatcgatgagttggttgaggtctacacggac gtgaaacaccgctggaaggcggagaatttcattttcatgggtgacttcaatgccggctgcagctacgtccccaagaaggcctgga agaacatccgcttgaggactgaccccaggtttgtttggctgatcggggaccaagaggacaccacggtgaagaagagcaccaac tgtgcatatgacaggattgtgcttagaggacaagaaatcgtcagttctgttgttcccaagtcaaacagtgtttttgacttccagaaagct tacaagctgactgaagaggaggccctggatgtcagcgaccactttccagttgaatttaaactacagtcttcaagggccttcaccaa cagcaaaaaatctgtcactctaaggaagaaaacaaagagcaaacgctcctagacccaagggtctcatcttattaaccatttcttgc ctctaaata |
| **149** | 205593 _s_at | aacgaggagcacatgaccctgctgaagatgattttgataaaatgctgtgatatctctaacgaggtccgtccaatggaagtcgcaga gccttgggtggactgtttattagaggaatattttatgcagagcgaccgtgagaagtcagaaggccttcctgtggcaccgttcatggac cgagacaaagtgaccaaggccacagcccagattgggttcatcaagtttgtcctgatcccaatgtttgaaacagtgaccaagctctt ccccatggttgaggagatcatgctgcagccactttgggaatcccgagatcgctacgaggagctgaagcggatagatgacgccat gaaagagttacagaagaagactgacagcttgacgtctggggccaccgagaagtccagagagagaagcagagatgtgaaaa acagtgaaggagactgtgcctgaggaaagcgggggcgtggctgcagttctggacggctggccgagctgcgcgggatccttg tgcagggaagagctgccctgggcacctggcaccacaagaccatgttttctaa |
| **150** | 205683 _x_at | tgacgcaaaataccaccttggcgcctacacgggagacgacgtccgcatcgtccgtgacgacatgctgtgtgccgggaacaccc ggagggactcatgccagggcgactccggagggcccctggtgtgcaaggtgaatggcacctggctgcaggcgggcgtggtcag ctggggcgagggctgtgcccagcccaaccggcctggcatctacacccgtgtcacctactacttggactggatccaccactatgtcc ccaaaaagccgtgagtcaggcctgggttggccacctgggtcactggaggaccaacccctgctgtccaaaacaccactgcttccta cccaggtggcgactgcccccacaccttccctgccccgtcctgagtgccccttcctgtcctaagcccctgctctcttctgagcccctt cccctgtcctgaggacccttccctatcctgagcccccttccctgtcctaagcctgacgcctgcaccgggccctccagccctcccctgc ccagatagctggtggtgggcgctaatcct |
| **151** | 205892 _s_at | gaagagggagctctattgccaccatgagtttctccggcaagtaccaactgcagagccaggaaaactttgaagccttcatgaaggc aatcggtctgccggaagagctcatccagaaggggaaggatatcaaggggtgtcggaaatcgtgcagaatggaagcacttca agttcaccatcaccgctgggtccaaagtgatccaaaacgaattcacggtggg |
| **152** | 205929 _at | ccttctctggtctccttgagatgatcgtagacacagggatgattcccacccaaacccacgtattcattcagtgagttaaacacgaattg atttaaagtgaacacacacaagggagcttgcttgcagatggtctgagttcttgtgtcctggtaattcctctccaggccagaataattgg catgtctcctcaacccacatggggttcctggttgttcctgcatcccgatacctcagccctggccctgcccagcccatttgggctctggttt tctggtggggctgtcctgctgccctcccacagcctccttctgtttgtcgagcatttcttctactcttgagagctcaggcagcgttagggct gcttaggtctcatggaccagtggctggtctcacccaactgcagtttactattgctatcttttctggatgatcagaaaataattccataaa |
| | | tctattgtctacttgcgatttttaaaaaatgtatatttttatatatattgttaaatcctttgcttcattcca |
| **153** | 205935 _at | tctcccaacctctactgtaaactttctggtccgagaacgagccgaacacagcgcgacgcagggactaggacggcccggtgacc gcgcggattcaggattgcggggacgcagaaaggttaaggcacttttaaaaactatagcaaggctcctgtttatttattctactttctttc cctaataatcaaaacaccgcgtaggctcctccgtttatcagtattaatggtgtaactttgttggcaatatttgccgtgtagaatttttttag atatccattgtaaatttgaaacaaagaccgatctgtgtaaaaacaaatttccatatgttttatataaatatatatataatatgaaggacta ccctcctttttttttttgtatttggctgctagagtgcagcattgtgacacgtatttgaaattgaaatttccttctgcactgtataaaggac catttgaggatgttttgccttttgtgtatttt |
| **154** | 205950 _s_at | gagccccattcacaaattttgacccctctactctccttccttcatccctggatttctggacctaccctggctctctgactcatcctcctcttta tgagagtgtaacttggatcatctgtaaggagagcatcagtgtcagctcagagcagctggcacaattccgcagccttctatcaaatgtt gaaggtgataacgctgtccccatgcagcacaacaaccgcccaacccaacctctgaaggcagaacagtgagagcttcatttga tgattctgagaagaaacttgtccttcctcaagaacacagccctgcttctgacataatccagttaaaataataatttttaagaaataaatt tatttcaatattagcaagacagcatgccttcaaatcaatctgtaaaactaagaaacttaaattttagttcttactgcttaattcaaataata attagtaagctagcaaatagtaatctgtaagcataagcttatcttaaattcaagtttagtttgaggaattctttaaaattacaactaagtg atttgtatgtctattttttc |
| **155** | 206000 _at | ggcagctttttatcagccttgctttggataggacctccaaggactaagcctccagccccatgtgacccttgtcatctctctgcccca cataattatgttactttgctatgtgctcctaatgtatctagtgtgtcctgtgacaacactcatcacacttcattgtaaatcacttgttttattgac tgtctttcctatagactgtaagctccatgagggcaggcacatgttgttctcattgaccgtgctggccccagtgcctagatgcatggctg gcacattgttggcactcaacaatggttgaatgaataaaacaataaatgaatgaataactaagatatagaaactctcatttatattgca gattgaatatatatgatgaaattcttatgttgaatatgttagaatcaaatactcatttttcattagatacagtagtgtcatcactcttttaagat cttgttaaagatttcaaataaaggtacttctggcgagccaggctgcacagcattgctttcct |
| **156** | 206094 _x_at | tatttgaatatgtatcgtgccccctccggagtctttgatcaggatgacatgtgccatttttcagaggacgtgcagacaggctggcattct agattacttttcttactctgaaacatggcctgtttgggagtgcgggattcaaaggtggtcccacggctgcccctactgcaaatggcagt tttaatcttatcttttggcttctgcagatggttgcattgatccttaaccaataatggtcagtcctcatctctgtcctgacttcataggt |
| **157** | 206134 _at | ctttctatattgttatcagtccaggaaacaggtaaacagatgtaattagagacattggctcttgtttaggcctaatctttctttttactttttttt tctttttctttttttttaaagatcatgaatttgtgacttagttctgccctttggagaacaaaagaaagcagtcttccatcaaatcaccttaaa atgcacggctaaactattcagagttaacactccagaattgttaaattacaagtactatgctttaatgcttctttcatcttactagtatggcct ataaaaaaaataataccacttgatgggtgaaggctttggcaatagaaagaagaatagaattcaggttttatgttattcctctgtgttca cttcgccttgctcttgaaagtgcagtatttttctacatcatgtcgagaatgattcaatgtaaatatttttcattttatcatgtatatcctatacac acatctccttcatcatcatatatgaagtttatttgagaagtctacattgcttacattt |
| **158** | 206143 _at | taacaatccatgatgctgttttgcatattttgatgaagaaagattacagtacttcaaagtttaatcccagtcaggaaaaagatggaaa aattgattttaccataaatacaaatggaggattacgtaatcgggtatatgaggtgccagttgaaacaaaattctaatcaacatataatt cagaaggatcttcatctgactatgacataaaaacaactttatacccagaaagttattgataagttcatacattgtacgaagagtattttt gacagaatatgtttcaaactttggaacaagatggttctagcatggcatatttttcacatatctagtatgaaattatataagtattctaaattt tatatcttgtagctttatcaaaggtgaaaattattttgttcatacatatttttgtagcactgacagatttccatcctagtcactaccttcatgc ataggtttagcagtatagtggcgccactgttttgaatct |
| **159** | 206149 _at | tcatcaggctccagttattctccatctcccagctcagctttttctgtctgtaagcctgattttcaggaaggctctttcctagtgatggagatg accaccatcagctccaggcttctatcctgctaacccagtaacccagtgggaagagatttacttattccaataattccaagtggagagt gtcattgacccgtttggggtctcatctctacttctaggggaatgaaacactttgagtggccaggcctgtgtcatgtgctaattcctagag ccagggaaataaggtctgaggattcaggatggggtgaaaggtggttgcttaaaggaaaatgaaatacaattagcagaataagg ggaaacgagtggtctgctctgctcggcaaaacaagagatgcccattactgtgagggacccttgaagtctggactcttaaatgggt tttgctgatttcctgggtgcatgctagg |
| **160** | 206198 _s_at | accagcaacaacttcaatccggtggagaacaaagatattgtggttttaacctgtcaacctgagactcagaacacaacctacctgtg gtgggtaaacaatcagagcctcctggtcagtcccaggctgctctccactgacaacaggaccctcgttctactcagcgccacaa agaatgacataggaccctatgaatgtgaaatacagaacccagtgggtgccagccgcagtgacccagtcaccctgaatgtccgct atgagtcagtaca |
| **161** | 206199 _at | atattagttaccctggtgtgctgtattctctaaaacctttaaatgtttgcatgcagccattcgtcaaatgtcaaatattctctctttggctgga atgacaaaaactcaaataaatgtatgattaggaggacatcataacctatgaatgatggaagtccaaaatgatggtaactgacagt agtgttaatgccttatgtttagtcaaactctcatttaggtgacagcctggtgactccagaatggagccagtcatgctaaatgccatatac tcacactgaaacatgaggaagcaggtagatcccagaacagacaaaactttcctaaaaacatgagagtccaggctgtctgagtca gcacagtaagaaagtcctttctgctttaactcttagaaaaaagtaatatgaagtattctgaaattaaccaatcagtttatttaaatcaatt tatttatattcttctgttcctggattcccattttacaaaacccactgttctactgttgtattgcccagt |
| **162** | 206208 _at | cccaggaccctgaagacgaaattgcggtgctggcctttctggtggaggctggaacccaggtgaacgagggcttccagccactgg tggaggcactgtctaatatccccaaacctgagatgagcactacgatggcagagagcagcctgttggacctgctccccaaggagg agaaactgaggcactacttccgctacctgggctcactcaccacaccgacctgcgatgagaaggtcgtctggactgtgttccggga gcccattcagcttcacagagaacagatcctggcattctctcagaagctgtactacgacaaggaacagacagtgagcatgaagga caatgtcaggcccctgca |
| **163** | 206209 _s_at | accacaccgacctgcgatgagaaggtcgtctggactgtgttccgggagcccattcagcttcacagagaacagatcctggcattctc tcagaagctgtactacgacaaggaacagacagtgagcatgaaggacaatgtcaggcccctgcagcagctgggcagcgcac ggtgataaagtccggggccccgggtcggccgctgccctgggccctgcctgccctgctgggccccatgctggcctgcctgctggcc ggcttcctgcgatgatggctcacttctgcac |
| **164** | 206262 _at | actgactggacgcacgtggaaaggagctatttttggaggctttaagagtaaagaatctgtcccgaaacttgtggctgactttatggct aagaagttttcactggatgcattaataacaaatatttttacctttgaaaaaataaatgaaggatttgacctgcttcgctctggaaagagt atccgtaccgtcctgacgttttgaaacaatacagatgccttcccttgtagcagttttcagcctcctctaccctacatgatctggagcaac agctaggaaatatcattaattctgctcttcagagatgttaaaaataaattacacgtgggagctttccaa |
| **165** | 206377 _at | gttggtcactgttatttgcctactgctggaagaaggacaaccgctggcaaggtagcgttccccaatctgaatacctgcaggctccca catgagggagagggcagactcaggtgggaagatgtgccatgcgtaaggcatcaacgtgtatctgtgggatcttcgttgccttcagt aatcagggtgtgaaaaaagcagacaagttgtgtgtgtgtgtgtgtgtctaagaaaacttgtgtgcttttcaaaaggcagtgctaagc acaagatttcaagaaagcctcttcttgttgcctagctgagtgggagagtcattttccccagacactacatttggatacaggtgccaaa gaacattattaaggaattatttagaaacaatgtgtctagtttaagaaagtggttttcagtattgtgacaatacaacgtttttacaaggttgt tttctaccaccatatttaaagatatttttatgaccgtgtatactcacactttgctt |
| **166** | 206385 _s_at | aaagcccttcatctaatatttgttgctattgccaatttttcaatgaaatgacctaaaacaacaaaaaaaataacctatacggtagtt gctttagggggtgggggggatgctatctgttagtgcttaaaagggggtaaatgcttgccgctttagaggtggatggtgctcataaaagg ccccagtcgggggtatttaaaaaggactgaacagaaatccttagctagtagaatggcagcacgctgtaaaattattactgtattgtgt actggctataagatgtagacacctttcagtaagccaatcatttgtaaccattctagcagtgtcatattaggttaataaggctgctgtttt aaagggcattttatttgggttttggtgaaattctttaatttgttgattatattcacataaaatcagcattcattgacacatagctctaatgac atatgtatgaaaaaccatacactggatgacctagtcga |
| **167** | 206422 _at | ctgatggttctttctctgatgagatgaacaccattcttgataatcttgccgccagggactttataaactggttgattcagaccaaaatcac tgacaggaaataactatatcactattcaagatcatcttcacaacatcacctgctagccacgtgggatgtttgaaatgttaagtcctgta aatttaagaggtgtattctgaggccacattgctttgcatgccaataaataaattttcttttagtgttgtgtagccaaaaattacaaatgga ataaagttttatcaaaatattgctaaaatatcagctttaaaatatgaaagtgctagattctgttattttcttcttattttggatgaagtacccc aacctgtttacatttagcgataaaattatttttctatgatataatttgtaaatgtaaattattccgatctgacatatctgcattataataatagg agaatagaagaactggtagccacagtggtgaaattggaaagagaactttcttcctgaaacctttgtcttaaaaatactcagctttcaa tgtatcaa |
| **168** | 206461 _x_at | gccggctcctgcaagtgcaaaaagtgcaaatgcacctcctgcaagaagagctgctgctcctgttgcccccctgggctgtgccaagt gtgcccagggctgcatctgcaaaggggcgtcagagaagtgcagctgctgtgcctgatgtcgggacagccctgctgtcagatgaa aacagaatgacacgtaaaatccgaggtttttttttctacaactccgactcatttgctacattcctttttttctg |
| **169** | 206561 _s_at | ttccctgctggaggatcccaagattaaggagattgctgcaaagcacaaaaaaaccgcagcccaggttctgatccgtttccatatcc agaggaatgtgattgtcatccccaagtctgtgacaccagcacgcattgttgagaacattcaggtctttgactttaaattgagtgatgag gagatggcaaccatactcagcttcaacagaaactggagggcctgtaacgtgttgcaatcctctcattggaagactatcccttcaat gcagaatattgaggttgaatctcctggtgagattatacaggagattctctttcttcgctgaagtgtgactacctccactcatgtcccatttt agccaagcttatttaagatcacagtgaacttagtcctgttatagacgagaatcgaggtgctgttttagacatttatttctgtatgttcaact aggatcagaatatcacagaaaagcatggcttgaataaggaaatgacaatttttttccacttatctgatcagaacaaatgtttattaagc atcagaaactctgccaacactga |
| **170** | 206576 _s_at | taagtccagtagtagcaaagccccaaatcaaagccagcaagaccacagtcacaggagataaggactctgtgaacctgacctg ctccacaaatgacactggaatctccatccgttggttcttcaaaaaccagagtctcccgtcctcggagaggatgaagctgtcccagg gcaacaccaccctcagcataaaccctgtcaagagggaggatgctgggacgtattggtgtgaggtcttcaacccaatcagtaaga accaaagcgaccccat |
| **171** | 206637 _at | tgagcctggggttctggtgttagaatatttttaagtaggctttactgagagaaactaaatattggcatacgttatcagcaacttcccctgtt caatagtatgggaaaaataagatgactgggaaaaagacacacccacaccgtagaacatatattaatctactggcgaatgggaa aggagaccattttcttagaaagcaaataaacttgatttttttaaatctaaaatttacattaatgagtgcaaaataacacataaaatgaa aattcacacatcacatttttctggaaaacagacggattttacttctggagacatggcatacggttactgacttatgagctaccaaaact aaattctttctctgctattaactggctagaagacattcatctatttttcaaatgttctttcaaaacatttttataagtaatgtttgtatctatttcat gctttact |
| **172** | 206641 _at | atttctttggcagttttcgtgctaatgtttttgctaaggaagataagctctgaaccattaaaggacgagtttaaaaacacaggatcaggt ctcctgggcatggctaacattgacctggaaaagagcaggactggtgatgaaattattcttccgagaggcctcgagtacacggtgg aagaatgcacctgtgaagactgcatcaagagcaaaccgaaggtcgactctgaccattgctttccactcccagctatggaggaag gcgcaaccattcttgtcaccacgaaaacgaatgactattgcaagagcctgccagctgctttgagtgctacggagatagagaaatc aatttctgctaggtaattaaccatttcgactcgagcagtgccactttaaaaatcttttgtcagaatagatgatgtgtcagatctctttagga tgactgtatttttcagttgccgatacagctttttgtcctctaactg |
| **173** | 206664 _at | gaagacactaccaacatgatattacgtattgatctgaccacacacaatgttactctagaagaaccaatagaaatcaactggtcatg aagatcaccatcaattttagttgtcaatgggaaaaaacaccaggatttaagtttcacagcacttacaatttccctcttcacttggttcttg tactctacaaaatatagctttcataacatcgaaaagttattttgtagcgtacatcaatgataatgctaattttattatagtaatgtgacttgg attcaattttaaggcatatttaacaaaattgaatagccctatttatccttgttaagtatcagctacaattgtaaactagttactaaacatgt |
| | | atgtaaatagctaagatataatttaaacgtgatttttaaattaaataaaatttttatgtaattatatatactatatttttctcaatgtttagcaga tttaagatatgtaacaacaattatttgaagatttaattacttcttagtatgtgcattta |
| **174** | 206710 _s_at | attgcaatttcttaggtaaccttatatttacaataaatgaagattaccctcaaatgctagaagctgtctaggtccgtccggtgtgtcagat ttcctcagattagatgtgccaataaccaagtttattcagtaaacaacttgtacttgtttcatctggtttattactctcacccataaacagtaa tgactctctgaccctctggaaatatgtaatgcttccaatcttg |
| **175** | 206784 _at | ttttggacctgcggtggtggccaaccactggaacttccactggatctactggctgggcccactcctggctggcctgcttgttggactgc tcattaggtgcttcattggagatggaagacccgcctcatcctgaaggctcggtgagcagagctcgtgggattcctgctgctccagg tgtcctcagctcacctgtcccagactgaggacaggggagttcctgcatttcctgccagggcagaggcccagaggagcgaccccct gcttccactgcttgggcctgctttctcagatagactgactgctgaggaggctctaggttcttggaattcctttgtgctcatcagagacccc agcctggggaacacgctgcccgcactgcccagagagcagtgcaaacaccacaacacgagcgtgtttcttgagaggaatgtccc cgagttggacaaggaggctgtttctgcacatcagctcatttcc . |
| **176** | 207003 _at | gaggggtcaccgtgcaggatggaaatttctccttttctctggagtcagtgaagaagctcaaagacctccaggagccccaggagcc cagggttgggaaactcaggaactttgcacccatccctggtgaacctgtggttcccatcctctgtagcaacccgaactttccagaaga actcaagcctctctgcaaggagcccaatgcccaggagatacttcagaggctggaggaaatcgctgaggacccgggcacatgtg aaatctgtgcctacgctgcctgtaccggatgctagggggcttgcccactgcctgcctcccctccgcagcagggaagctcttttctcc tgcagaaaggccacccatgatactccactcccagcagctcaacctaccctggtccagtcgggaggagcagcccggggagga actgggtgact |
| **177** | 207080 _s_at | tcgtctgcttcacaagctatcgctatggtgttcgtgcgcaggccgtggcccgccttgaccacagtgcttctggccctgctcgtctgccta ggggcgctggtcgacgcctaccccatcaaacccgaggctcccggcgaagacgcctcgccggaggagctgaaccgctactacg cctccctgcgccactacctcaacctggtcacccggcagcggtatgggaaaagagacggcccg |
| **178** | 207126 _x_at | tatttgaatatgtatcgtgccccctccggtgtctttgatcaggatgacatgtgccatttttcagaggacgtgcagacaggctggcattcta gattacttttcttactctgaaacatggcctgtttgggagtgcgggattcaaaggtggtcccaccgctgcccctactgcaaatggcagttt taatcttatcttttggcttctgcagatggttgcaattgatccttaaccaataatggtcagtcctcatctctgtcctgcttcataggtgccacctt gtgtgtttaaa |
| **179** | 207134 _x_at | tgacgcaaaataccaccttggcgcctacacgggagacgacgtccgcatcgtccgtgacgacatgctgtgtgccgggaacaccc ggagggactcatgccagggcgactccggagggcccctggtgtgcaaggtgaatggcacctggctgcaggcgggcgtggtcag ctggggcgagggctgtgcccagcccaaccggcctggcatctacacccgtgtcacctactacttggactggatccaccactatgtcc ccaaaaagccgtgagtcaggcctgggttggccacctgggtcactggaggaccaacccctgctgtccaaaacaccactgcttccta cccaggtggcgactgccccccacaccttccctgccccgtcctgagtgccccttcctgtcctaagcccctgctctcttctgagcccctt cccctgtcctgaggacccttccccatcctgagccccttccctgtcctaagcctgacgcctgcaccgggccctccggccctcccctg cccaggcagctggtggtgggcgct |
| **180** | 207245 _at | atgatcacaaaatgttgcctgttttgtttccgaaagcttgccaaaacaggaaagaagaagaaaagggattagttatatcaaaagcc tgaagtggaatgaccaaaagatgggactcctcctttattccagcatggagggttttaaatggaggatttcctttttcctgcgacaaaac gtcttttcacaacttaccctgttaagtcaaaatttattttccaggaatttaatatgtactttagttggaattattctatgtcaatgatttttaagct atgaaaaataataatataaaaccttatgggcttatattgaaatttattattctaatccaaaagttaccccacacaaaagttactgagctt ccttatgtttcacacattgtatttgaacacaaaacattaacaactccactcatagtatcaacattgttttgcaaatactcagaatattttgg cttcattttgagcagaat |
| **181** | 207266 _x_at | caagtatcggggctctgctatcaaggtgcaaagtccttcgtggatgcaacctcaaccatatattctacagcaccctggtgccgtgtta actccctcaatggagcacaccatgtcactacagcccgcatcaatgatcagccctctggcccagcagatgagtcatctgtcactagg cagcaccggaacatacatgcctgcaacgtcagctatgcaaggagcctacttgccacagtatgcacatatgcagacgacagcggt tcctgttgaggaggcaagtggtcaacagcaggtggctgtcgagacgtctaatgaccattctccatatacctttcaa |
| **182** | 207390 _s_at | tggactggtgtcgagccaagactcggggcctacgagcacgtcgacatccagaacttctcctccagctggagtgatgggatggcctt ctgtgccctggtgcacaacttcttccctgaggccttcgactatgggcagcttagccctcagaaccgacgccagaacttcgaggtggc cttctcatctgcggagatgctggtggactatgtgcccctggtggaggtggacgacatgatgatcatgggcaagaagcctgacccca agtgtgtcttcacctatgtgcagtcgctctacaaccacctgcgacgccacgaactggcctcgcgcggcaagaatgtctagcctgcc cgcccgcatggccagccagtggcaactgccgcccccactctccgggcaccgtctcctgcctgtgcgtccgcccaccgctgccctg tctgttgcgacaccctccccccacatacacacgcagcgttttga |
| **183** | 207392 _x_at | ggatgtgatagcattcctgctggcctgcgtggcaactgtgatatttatcatcacaaaattttgcctgttttgttccgaaagcttgccaaaa caggaaagaagaagaaaagagattagttatatcaaaagcctgaagtggaatgactgaaagatgggactcctcctttatttcagca tggagggttttaaatggaggatttcctttttcctgtgacaaaacatcttttcacaacttaccttgttaagacaaaatttattttccagggattt aatacgtactttagttggaattattctatgtcaatgatttttaagctatgaaaaatacaatgggggaaggatagcatttggagatatac ctaatgttaaatgacgagttactggatgcagcacgcaacatggcacatgtgtatacatatgtagctaacccttcgttgtgcacatgtac cctaaaacttaaagtataatttaaaaaaagcaaaaaaaaaaaaataccaactcttttttttaaaccaggaaggaaaatgtgaacatg gaaacaacttctagtattggatctg |
| **184** | 207432 _at | gccccggagtgcagctgcggggacccgctgctcgaccccggcctgccggagcccgaggccccgcccctgcgggtcccgaa ccgcttaccctcatccctgggcctgtcgagcccttcagcatcgtgaccatgcccggccccggggtccggcgccaccctggctgcc cagccctattggcgaggaggaggagaatctggcctgagatcttagagcccagcccccctaaggacagggaaccaggtccctgca |
| | | cggcacccacgcaggtgtcccggtctgcataagcctcgtatgcctttgtaaagtccacctacacttttgaccagctctcgctgcccgc atgtgtttggcgctgtgctagggcgggagttcttccagactcttggaccagcccgccctgaccaccagctctacttcccaaccccc actgcctgagaggtctctatcagtgtcctgcctgaattctttccttcaagtgaagatgtgactgactacctcctcgagttgtcatga |
| **185** | 207502 _at | gaacccagggagcgcgatgggctgcagggctgcgtcagggctcctgccaggagtggccgtggtcctcctgctgctgctgcagag cacacagtcagtctacatccagtaccaaggcttccgggtccagctggaatccatgaagaagctgagtgacctggaggcacagtg ggcacccagcccccgcctgcaggcccagagcctcctgcccgccgtgtgccaccaccctgctctgcctcaggaccttcagcctgtc tgcgcctcgcaggaggcttccagcatcttcaagaccctgaggaccatcgctaacgacgactgtgagctgtgtgtgaacgttgcgtgt accggctgcctctgagatagccctgggtaccctgagcccaccagggacacctcgcccttcagcccaccaccctggcaggcttcc atccccgtccatgctcaagatgggtc |
| **186** | 207761 _s_at | ttattcttagcgtcactggtctggctttcagaattaacatacaaggttgccacacctagttctgcccagctttatgtcttttattccagtattcc accaaagtttgttttcctgcattccagttctcaagtcttaagataaagattgtacttgacagtttagtatatccataaaactatttgaggtgg ttaaggttcttgggttcattttccttaatactttgctgaatattgtagattgtaggcaatgaaaaagtctactaaattaggaaaaccttgaat aattaggtatcctaggtaagagcccctaaacatcaagcaatctgtgagtctgtaaagaaataaatattttttggattattcttatctaattc caccccctgttggaagatgatttctttgttctttgcaactatggaagctgtgaaaatcatcacaagtgcctctgaaagcgagtgttaggtt ggttagagggt |
| **187** | 207961 _x_at | gtgacggtgctgaagaaggccctggatgaagagacgcggtcccatgaggctcaggtccaggagatgaggcagaaacacgca caggcggtggaggagctcacagagcagcttgagcagttcaagagggccaaggcgaacctagacaagaataagcagacgct ggagaaagagaacgcagacctggccggggagctgcgggtcctgggccaggccaagcaggaggtggaacataagaagaag aagctggaggcgcaggtgcagacactgagtttttagaaaaacatatccacggtaaccggtccctggcaattctgtttacatgaaat ggggagaaagtcaccgaaatgggtgccgccggcccccactcccaattcattccctaacctgcaaacctttccaacttctcacgtca ggcctttgagaattctttccccctctcctggtttccacacctcagacacgcacagttcaccaagtgccttctgtagtcacatgaattgaa aaggagacgctgctcccacggaggggagcaggaatgctgcactgtttacaccctgactgtgcttaaa |
| **188** | 207977 _s_at | tagtggccgtgaggagcatcttcagtaagaaggaaggttctgacagacaatggaactacgcctgcatgcccacgccacagagc ctcggggaacccacggagtgctggtgggaggagatcaacagggctggcatggaatggtaccagacgtgctccaacaatgggct ggtggcaggattccagagccgctacttcgagtcagtgctggatcgggagtggcagttttactgttgtcgctacagcaagaggtgccc atattcctgctggctaacaacagaatatccaggtcactatggtgaggaaatggacatgatttcctacaattatgattactatatccgag gagcaacaaccactttctctgcagtggaaagggatcgccagtggaagttcataatgtgccggatgactgaatacgactgt |
| **189** | 207980 _s_at | aatgtcatagacactgatttcatcgacgaggaagttcttatgtccttggtgatagaaatgggtttggaccgcatcaaggagctgcccg aactctggctggggcaaaacgagtttgattttatgacggacttcgtgtgcaaacagcagcccagcagagtgagctgttgactcgatc gaaacccc |
| **190** | 208131 _s_at | cccccccaattgctgccaatagtggataacatgtatcactcactgccaaaaatagaaagtgaccatgaaaaataaattcgctgggg aagggggctccatgctggtgtggccaaggctgagagctctctcttctctgttacaaaacgagataagcaagtnttagaattgcctta aggccacactggcatctccctgaccttctccagggacagaagcaggagtaagtttctcatcccatgggcgaccagggccatctcct cccaccagtggcccccactcacagggagctggcaatgccctacctgcctgttctccagatggagaaacaggctctgagatttcac aggtcttgcccaaagtcattgatttaatgattaaaaagaataaacacagtgtttcctgagtagcagtgattgttatgccttgctattta |
| **191** | 208370 _s_at | gatgtattaacctacctgtgaatcatatgttgtaggaaaagctgttcccatgtctaacaggacttgaattcaaagcatgtcaagtggat agtagatctgtggcgatatgagagggatgcagtgcctttccccattcattcctgatggaattgttatactaggttaacattgtaattttttc tagttgtaatgtgtatgtctggtaaataggtattatatttggccttacaataccgtaacaatgtttgtcattttgaaatacttaatgccaagt aacaatgcatgctttggaaatttggaagatggttttattctttgagaagcaaatatgtttgcattaaatgctttgattgttcatatcaagaaa ttgattgaacgttctcaaaccctgtttacggtacttggtaagagggagccggtttgggagagaccattgcatcgctgtccaagtgtttct tgt |
| **192** | 208383 _s_at | agggcaagtgttcccaaattgacgccacctaataatcatcaccacaccgggagcagatctgaaggcacactttgattttttaagga taagaaccacagaacactgggtagtagctaatgaaattgagaagggaaatcttagcatgcctccaaaaattcacatccaatgcat actttgttcaaatttaaggttactcaggcattgatcttttcagtgtttttcacttagctatgtggattagctagaatgcacaccaaaaagat acttgagctgtatatatatatgtgtgtgtgtgtgtgtgtgtgtgtgtgtgcatgtatgtgcacatgtgtctgtgtgatatttggtatgtgtattt gtatgtactgttattcaaaatatatttaatacctttggaaaatcttgggcaagatgacctactagttttccttgaaaaaaagttgctttgttat taatattgtgct |
| **193** | 208399 _s_at | ccgagccgagcttactgtgagtgtggagatgttatcccaccatgtaaagtcgcctgcgcaggggagggctgcccatctccccaac ccagtcacagagagatagggaaacggcatttgagtgggtgtccagggccccgtagagagacatttaagatggtgtatgacagagc attggccttgaccaaatgttaaatcctctgtgtgtatttcataagttattacaggtataaaagtgatgacctatcatgaggaaatgaaag tggctgatttgctggtaggattttgtacagtttagagaagcgattatttattgtgaaactgttctccactccaactcctttatgtggatctgttc aaagtagtcactgtatatacgtatagagaggtagataggtaggtagatttaaattgcattctgaatacaaactcatactccttagagc ttgaattacattttaaaatgcatatgtgctgtttggcaccgtggcaagatggtatcagagagaaacccatcaattgctcaaatactc |
| **194** | 208450 _at | ggagctgtcaccatgacgggggaacttgaggttaagaacatggacatgaagccggggtcaaccctgaagatcacaggcagca tcgccgatggcactgatggctttgtaattaatctgggccaggggacagacaagctgaacctgcatttcaaccctcgcttcagcgaat ccaccattgtctgcaactcattggacggcagcaactgggggcaagaacaacgggaagatcacctgtgcttcagcccagggtcag aggtcaagttcacagtgacctttgagagtgacaaattcaaggtgaagctgccagatgggcacgagctgactttcccaacaggctg |
| | | ggtcacagccacctgagctacctgagcgtaagggcgggttcaacatgtcctc |
| **195** | 208581 _x_at | gcgtgttttcctcttgatcgggaactcctgcttctccttgcctcgaaatggaccccaactgctcctgctcgcctgttggctcctgtgcctgt gccggctcctgcaaatgcaaagagtgcaaatgcacctcctgcaagaagagctgctgctcctgctgccctgtcggctgtgccaagt gtgcccagggctgcatctgcaaagggacgtcagacaagtgcagctgctgtgcctga |
| **196** | 208596 _s_at | cccgaccatcgaatcttgcgaacaacacgatacttgttaagtggctaccccaaaacgatctgcttggtcacccgatgacccgtgcct ttatcacccatgctggttcccatggtgtttatgaaagcatatgcaatggcgttcccatggtgatgatgcccttgtttggtgatcagatgga caatgcaaagcgcatggagactaagggagctggagtgaccctgaatgttctggaaatgacttctgaagatttagaaaatgctctaa aagcagtcatcaatgacaaaagttacaaggagaacatcatgcgcctctccagccttcacaaggaccgcccggtggagccgctg gacctggccgtgttctgggtggagtttgtgatgaggcacaagggcgcgccacacctgcgccccgcagcccacgacctcacctggt accagtaccattccttggacgtgattggtttcctcttggccgtcgtgctgacagtggccttcatcacctttaaatgttgtgcttatggctacc ggaaatgcttggggaaaaaagggcgagttaagaaagcccacaaatccaagacc |
| **197** | 208747 _s_at | tgcagaggcctatgttttcactcctaacatgatctgtgctggaggagagaagggcatggatagctgtaaaggggacagtggtggg gcctttgctgtacaggatcccaatgacaagaccaaattctacgcagctggcctggtgtcctggggggccccagtgtgggacctatgg gctctacacacgggtaaagaactatgttgactggataatgaagactatgcaggaaaatagcacccccgtgaggactaatccag atacatcccaccagcctctccaagggtggtgaccaatgcattaccttctgttccttatgatattctcattatttcatcatgactgaaagaa gacacgagcgaatgatttaaatagaacttgattgttgagacgccttgctagaggtagagtttgatcatagaattgtgctggtcatacat ttgtggtctgactccttggggtcctttccccggagtacctattgtagataacactatgggtggggcactccttt |
| **198** | 208763 _s_at | gggcctaaccagccttgggagtattgactggtcccttacctcttatggctaagtctttgactgtgttcatttaccaagttgacccagtttgt cttttaggttaagtaagactcgagagtaaaggcaaggagggggccagcctctgaatgcggccacggatgccttgctgctgcaac cctttccccagctgtccactgaaacgtgaagtcctgttttgaatgccaaacccaccattcactggtgctgactacatagaatggggttg agagaagatcagtttgggcttcacagtgtcatttgaaaacgtttttttgttttgttttgtaattattgtggaaaactttcaagtgaacagaagg atggtgtcctactgtggatgagggatgaacaaggggatggctttgatccaatggagcctggaggtgtgcccagaaagcttgtctgt agcgggtttgtgagagtgaacactttccacttttgacaccttatcctgatgtatggttc |
| **199** | 208788 _at | agatgtgtttagaacctcttgtttaaaaataatagactgcttatcataaaatcacatctcacacatttgaggcagtggtcaaacaggta aagcctatgatgtgtgtcattttaaagtgtcggaatttagcctctgaataccttctccattgggggaaagatattcttggaaccactcatg acatatcttagaaggtcattgacaatgtataaactaattgttggtttgatatttatgtaaatatcagtttaccatgctttaattttgcacattcg tactatagggagcctattggttctctattagtcttgtgggttttctgtttgaaaaggagtcatggcatctgtttacatttaccttatcaaaccta gaatgtgtatatttataaatgtatgtcttcattgctaggtactaatttgcagatgtctttacatatttcaatacagaaactataacattcaata gtgtgctgtcaaagtgtgcttagctcacctggatatacctacattg |
| **200** | 208789 _at | gaagggagtgttgctcccagtccagaggcctgattctgttcggactgggttctcaagacacgaccaggttctcaagacacgagtcc ccttgttcctccccattaaaggggggtttgtcagaagcaagaacagcccctctccccagtcacagcctgaaggaggccccgaga gcttcctccttccccccacctgctccttaccttctctgccctgctttttagaactgcagttcattgttttaagggattgggggagggagcctg gggacacaaaccttttatacaatacaaagctttgcttttttttttttttnnnnnnnncctttttcccttttctcggttctcttctctcctctgaatggct gaagacccctctgccgagggaggttggggattgtggacaaggtcccttggtgctgatggcctgaaggggcctgagctgtgggc agatgcagttttctgtgggcttggggaacctctcacgttgctgtgtcctggtgagcagcccgaccaataaacctgcttt |
| **201** | 208791 _at | ggctgcctgcggatgaaggaccagtgtgacaagtgccgggagatcttgtctgtggactgttccaccaacaacccctcccaggcta agctgcggcgggagctcgacgaatccctccaggtcgctgagaggttgaccaggaaatataacgagctgctaaagtcctaccagt ggaagatgctcaacacctcctccttgctggagcagctgaacgagcagtttaactgggtgtcccggctggcaaacctcacgcaagg cgaagaccagtactatctgcgggtcaccacggtggcttcccacacttctgactcggacgttccttccggtgtcactgaggtggtcgtg aagctctttgactctgatcccatcactgtgacggtccctgtagaagtctccaggaagaaccccta |
| **202** | 208792 _s_at | agcagctgaacgagcagtttaactgggtgtcccggctggcaaacctcacgcaaggcgaagaccagtactatctgcgggtcacca cggtggcttcccacacttctgactcggacgttccttccggtgtcactgaggtggtcgtgaagctctttgactctgatcccatcactgtga cggtccctgtagaagtctccaggaagaaccctaaatttatggagaccgtggcggagaaagcgctgcaggaataccgcaaaaag caccgggaggagtgagatgtggatgttgc |
| **203** | 208894 _at | cgatcaccaatgtacctccagaggtaactgtgctcacgaacagccctgtggaactgagagagcccaacgtcctcatctgtttcata gacaagttcacccca |
| **204** | 208920 _at | gatataccatgtattcacactttaaaaatacacatagcagagtatcggaaagtatgtaccaaaacattgatctggctaccactggg agtcaggatcatgagttcatcttcttcntncttnnnactgcttttccaaacattctccagtaagcaggtactacatttataatggaagga attttttaaaaaattttaagctgtacactttcaagtaagatctgaattctaatgctggcttgtgcctcttactatgtggtttggtcattatagat aatgcccagtttcagtttccccatctttgaaaaggagataatatgttatctcttgggagtagttctgaatatgaagtgtgttgatacaaga agcactgacaatgtttctgtttcttaaatttaaaactggcctggtttgcctttttttatcaagagagcttaacagataaaaaatgaaattagt ctattttctacttgccagcagagtatctgtcttattttaggatgcagtgtgaaacttaccattcaactgaacaac |
| **205** | 209047 _at | gaaggctggattctatctacataagtcctttcaattccaccagggccagagcagctccaccactgtgcacttagccatgatggcaac agaaaccaagagacacaattacgcaggtatttagaagcagaggacaaccagaaggcccttaactatcaccagtgcatcacat ctgcacactctcttctccattccctagcaggaacttctagctcatttaacagataaagaaactgaggcccacggtttcagctagacaa tgatttggccaggcctagtaaccaaggccctgtctctggctactccctggaccacgaggctgattcctctcatttccagcttctcagtttc tgcctgggcaatggccaggggccaggagtggggagagttgtgatggaggggagaggggtcacacccacccccctgcctggttnt |
| | | aggctgctgcacaccaaggccctgcatctgtctgctctgcatatatgt |
| **206** | 209074 _s_at | agacagctgtttagggtcttctcccctcacccatgctttcatcatcccctccgcacagcctccccgtccaggccttctaaccacaccta cccagggctgccgcattcctgcactcagaagtctgcagcggtgcctcacaaacttgattgtgcataaaaatcactggggatcttgtta atacagattctaactcaatagatctgggagatcctgcatttctaacaagctcccaggtaaggcggaggctgctggtgtgaggaccat gctgtgagcagcagggcgagagtgcccagggctgatatatattggaaatatcacccctgaagccatcgctggcccccacctcctg tggactgatgccccagggattcccaccccacttctgcaaccccaggtatccttcattatccaccccatcccagactcccaccccagg gattgcccgtgaagactttggcctagcaaattgtgttggttatgtgagtgttgttttaatcagagatgtacatgattgccaatctgcatttctt accagtgtgaccacac |
| **207** | 209101 _at | atcatcagatcgactcttatacgagtaatatgcctgctatttgaagtgtaattgagaaggaaaattttagcgtgctcactgacctgcctg tagccccagtgacagctaggatgtgcattctccagccatcaagagactgagtcaagttgttccttaagtcagaacagcagactcag ctctgacattctgattcgaatgacactgttcaggaatcggaatcctgtcgattagactggacagcttgtggcaagtgaatttgcctgta acaagccagatttttaaaatttatattgtaaatattgtgtgtgtgtgtgtgtgtgtatatatatatatatatgtacagttatctaagttaatttaa agttgtttgtgcctttttatttttgtttttaatgctttgatatttcaatgttagcctcaatttctgaacaccataggtagaatgtaaagcttgtctga tcgttcaaagcatgaaatggatacttatatggaaattctgctcagatagaatgacagtccgtcaaaacagattgtttgcaaagggga ggcatcagtgtcttg |
| **208** | 209114 _at | tacaccacaatggctgagcacttcctgacgttgctggtagtgcctgccatcaagaaagattatggttcccaggaagacttcactcaa gtgtggaacaccaccatgaaaggctcaagtgctgtggcttcaccaactatacggatttgaggactcaccctacttcaaagagaa cagtgcctttcccccattctgttgcaatgacaacgtcaccaacacagccaatgaaacctgcaccaagcaaaaggctcacgacca aaaagtagagggttgcttcaatcagcttttgtatgacatccgaactaatgcagtcaccgtgggtggtgtggcagctggaattgggg cctcgagctggctgccatgattgtttccatgtatctgtactgcaatctacaataagtccacttctgcctctgccactactgctgccacatg ggaaactgtgaagaggcaccctgggcaagcagcagtgattgggggaggggacaggatctaacaatgtcacttgggccagaat ggacctggcctttctgctcccagacttgggggctag |
| **209** | 209116 _x_at | gcagttatgggcaaccctaaggtgaaggctcatggcaagaaagtgctcggtgcctttagtgatggcctggctcacctggacaacct caagggcacctttgccacactgagtgagctgcactgtgacaagctncangtggatcctgagaacttcaggctnctnggcaacgtg ntngtctgngtgctggcccatcactttggcaaagaattcaccccaccag |
| **210** | 209138 _x_at | tctctgggctccaggctgaggacgaggctgattattactgctcatatgcaggtagttacactgtggttttcggcggagggaccaa actgaccgtcctaggtcagcccaaggctgccccctcggtcactctgttcccgccctcctctgaggagcttcaagccaacaaggcca cactggtgtgtctcataagtgacttctacccgggagccgtgacagtggcctggaaggcagatagcagccccgtcaaggcgggag tggagaccaccacaccctccaaacaaagcaacaacaagtacgcggccagcagctatctgagcctgacgcctgagcagtgga agtcccacagaagctacagctgccaggtcacgcatgaagggagcaccgtggagaagacagtggcccctacagaatgtt |
| **211** | 209147 _s_at | tttggtgcagctgctagtcagtccctgactgacattgccaagtattcaataggcagactgcggcctcacttcttggatgtttgtgatcca gattggtcaaaaatcaactgcagcgatggttacattgaatactacatatgtcgagggaatgcagaaagagttaaggaaggcaggt tgtccttctattcaggccactcttcgttttccatgtactgcatgctgtttgtggcactttatcttcaagccaggatgaagggagactgggca agactcttacgccccacactgcaatttggtcttgttgccgtatccatttatgtgggcctttctcgagtttctgattataaacaccactggag cgatgtgttgactggactcattcagggagctctggttgcaatattagttgctgtatatgtatcggatttcttcaaagaaagaacttcttttaa agaaagaaaagaggaggactctcatacaactctgcatgaaacaccaacaactgggaatcactatccgagcaatcaccagc |
| **212** | 209156 _s_at | gcacgccatcaatgccatcgtgcgcagcccgcgtggcggggcccggaggcacgcagagctgtccttcgtgttcctcacggacgg cgtcacgggcaacgacagtctgcacgagtcggcgcactccatgcgcaacgagaacgtggtacccaccgtcctggccttgggca gcgacgtggacatggacgtgctcaccacgctcagcctgggtgaccgcgccgccgtgttccacgagaaggactatgacagcctg gcgcaacccggcttcttcgaccgcttcatccgctggatctgctagcgccgccgcccgggccccgcagtcgagggtcgtgagccca ccccgtccatggtgctaagcgggccc |
| **213** | 209167 _at | tgtttcctcatagctagttctcaagctgcatgtaagattttaacggaagagaaaataggcctggacctgaaggtctcaaatatgttga gaagaaagtatgaactataaggaacttgagatgtagatttattttgcaggaaatacgaggaaaataggaaagaagtgtttgccgc atcaagtgtaaagaatgatactgaggatttttacattttatgaaatgaaataatggcatttacaaaatgaaaaatgtagtttcacaact aagttttgttaacagagactgcatgctttgcttatagttcttaattttggttttgacattcatttaatttttccatgttaaatatgtagtttaattattt actcaaaataaacattgttcatgcttttaggcctttgggggaattgattttatccacaggtagaaaatggtctttgcacacactacactt atttcaaatatacaatgtgctcccgaactttcgcattagtctt |
| **214** | 209170 _s_at | gaaatatcactgacactccagactaaagcagagtctaggtttctgcaattttgttacagtaatttgtaaatagctttagtaaactcacctt gcatggtagattaataagatgacttactgtacatgaattacacaataatgagatctggtggctatttccacattttgaaaaggattcagt tatttactgacagtggtgagcatcctttttaaaataatgttctgatacttaaacattagagagcagtatctttaaatgaattattaacacttt ggaatacttacattttctgttatttttgattgcctgataaccagtttcaatgatgaaaatgaaaacaagtgctgaagatgaaatggaaga gaaccgttttaatctggattttgtttttgtcacacctggaaaatactttgcaaatatgttctaaattgaaaacaatttttttatgatcacatggtt cactaccaaatgaccctcaaataagccagatq |
| **215** | 209191 _at | gatagtcggaatagagccgccccaactcagatcctacaacacgcaagttccttcttgaaccctggtgcctcctaccctatggccctg aatggtgcactggtttaattgtgttggtgtcggcccctcacaaatgcagccaagtcatgtaattagtcatctggaacaaagactaaaa acagcagagaattgcgggttctacccagtcagaagatcacaccatggagactttctactagaggacttgaaagagaactgaggg gccacaaaataaacttcaccttccattaagtgttcaagcatgtctgcaaattaggagggagttagaaacagtctttttcatcctttgtga |
| | | tgaagcctgaaattgtgccgtgttgccttatatgaatatg |
| **216** | 209209 _s_at | tgtctccccgctatctaaaaaagtataagaacaagcagataacagcgagaatcttggaggcccatcagaatgtagctcagatga gtctaattgaagccaagatgagatttattcaagcttggcagtcactacctgaatttggcatcactcacttcattgcaaggttccaaggg ggcaaaaaagaagaacttattggaattgcatacaacagactgattcggatggatgccagcactggagatgcaattaaaacatgg cgtttcagcaacatgaaacagtggaatgtcaactgggaaatcaaaatggtcaccgtagagtttgcagatgaagtacgattgtccttc atttgtactgaagtagattgcaaagtggttcatgaattcattggtggctacatatttctctcaacacgtgcaaaagaccaaaacgaga gtttagatgaagagatgttctacaaacttaccagtggttgggtgtgaatagaaatactgtttaatgaaactccacggccataa |
| **217** | 209210 _s_at | aaaatgctattagtccgtcgtgcttnatttgtttttgtccttgaataagcatgttatgtatatngtctcgtgtttttatttttacaccatattgtatta cacttttagtattcaccagcataancactgtctgcctaaaatatgcaactctttgcattacaatatgaagtaaagttctatgaagtatgc attttgtgtaactaatgtaaaaacacaaattttataaaattgtacagtttttaaaaactactcacaactagcagatggcttaaatgtagc aatctctgcgttaattaaatgcctttaagagatataattaacgtgcagttttaatatctactaaattaagaatgacttcattatgatcatgat ttgccacaatgtccttaactctaatgcctggactggccatgttctagtctgttgcgctgttacaatctgtattggtgctagtcagaaaattc ctagctcacatagcccaaaagggtgcgagggagaggtggattaccagtattgttcaataatccatggttca |
| **218** | 209283 _at | gaccagttcttcggagagcacctgttggagtctgatcttttcccgacgtctacttccctgagtcccttctaccttcggccaccctccttcct gcgggcacccagctggtttgacactggactctcagagatgcgcctggagaaggacaggttctctgtcaacctggatgtgaagcac ttctccccagaggaactcaaagttaaggtgttgggagatgtgattgaggtgcatggaaaacatgaagagcgccaggatgaacat ggtttcatctccagggagttccacaggaaataccggatcccagctgatgtagaccctctcaccattacttcatccctgtcatctgatgg ggtcctcactgtgaatggaccaaggaaacaggtctctggccctgagcgcaccattcccatcacccgtgaaaagaagcctgctgtc accgcagcccccaagaaaaagatgccctttcttgaattgcatttttaaaacaagaaagtttccccaccagtgaatgaa |
| **219** | 209301 _at | tgaatcttcgggtgtttccctttagctaagcacagatctaccttggtgatttggaccctggttgctttgtgtctagttttctagacccttcatct cttacttgatagacttactaataaaatgtgaagactagaccaattgtcatgcttgacacaactgctgtggctggttggtgctttgtttatgg tagtagtttttctgtaacacagaatataggataagaaataagaataaagtaccttgactttgttcacagcatgtagggtgatgagcact cacaattgttgactaaa |
| **220** | 209312 _x_at | ctgtgtatccttcaaagacccagcccctgcagcaccataacctcctggtctgttctgtgagtggtttctatccaggcagcattgaagtc aggtggttccggaatggccaggaagagaagactggggtggtgtccacaggcctgatccacaatggagactggaccttccagac cctggtgatgctggaaacagttcctcggagtggagaggtttacacctgccaagtggagcacccaagcgtgacaagccctctcaca gtggaatggagagcacggtctgaatctgcacagagcaagatgctgagtggagtcgggggctttgtgctgggcctgctcttccttgg ggccgggctgttcatctacttcaggaatcagaaaggacactctggacttcagcca |
| **221** | 209335 _at | agtcaaatgccaaacactagctctgtattaatccccatcattactggtaaagcctcatttgaatgtgtgaattcaatacaggctatgtaa aatttttactaatgtcattattttgaaaaaataaatttaaaaatacattcaaaattannannnnanacaagcttaattgttaatattcccta aacacaattttatgaagggagaagacattggtttgttgacaataacagtacatcttttcaagttctcagctatttcttctacctctccctatc ttacatttgagtatggtaacttatgtcatctatgttgaatgtaagcttataaagcacaaagcatacatttcctgactggtctagagaactg atgtttcaatttacccctctgctaaataaa |
| **222** | 209357 _at | ggcaaactgcttaatcttgtggattttgtagatggtttcaaatgactgaactgcattcagatttacgagtgaaggaaaaattgcattag ttggttgcatgaactttgaagggcagatattactgcacaaactgccatctcgcttcatttttttaactatgcatttgagtacagactaatttt aaaatatgctaaactggaagattaaacagatgtggcccaaactgttctggatcaggaaagtcatactgttcactttcaagttggctgt cccccccgccgcccccccccacccccatatgtacagatgataatagggtgtggaatgtcgtcagtggcaaacatttcacagattatt ttgtttctgtcttcaacatttttgacactgtgctaat |
| **223** | 209373 _at | ctcctccatgagtctgacatctcggaaactgagcagctgccggacgcctgggtcaggaatccaagaccccacctcttaaggactg gttcctcagaaagcaccctcagggaaaaaggtgaaaacattacatccgtggattctcctgccacaaccgcattggaagaaaagg ctgccgcaacatctcagcgaggagtgaaggacccatgtcccaggaaccgcgctgcgccacctgcactcaccccccctcacattct cttaagcacccggtggccctccgaggcctggcggaatggtggtgcccacggggttgggcaagggctcaccaggacctcaacgg gcaaagttgtgcacactaaaatatcaaatcaaggtgcttggttttaaagtaaatgtttttctaaagaaagctgtgttcttctgttgaccca gacgaatagggcacagccctgtaactgcacgtgccttctgtcattgggaatg |
| **224** | 209374 _s_at | caccatcacgtgcctggtgacggcttctctcccgcggacgtcttcgtgcagtggatgcagagggggcagcccttgtccccggag aagtatgtgaccagcgccccaatgcctgagccccaggccccaggccggtacttcgcccacagcatcctgaccgtgtccgaaga ggaatggaacacgggggagacctacacctgcgtggtggcccatgaggccctgcccaacagggtcaccgagaggaccgtgga caagtccacc |
| **225** | 209436 _at | ttctttttatattgtcctccacctccatcattttcaataaaagatagggcttttgctcccttgttcttggagggaccattattacatctctgaac tacctttgtatccaacatgttttaaatccttaaatgaattgctttctcccaaaaaaagcacaatataaagaaacacaagatttaattatttt tctacttgggggggaaaaaangtcctcatgtagaagcacccacttttgcaatgttgttctaagctatctatctanctctcagcccatgata aagttccttaagctggtgattcctaatcaaggacaagccaccctagtgtctcatgtttgtat |
| **226** | 209457 _at | ggaagcacaatttccaccttatttttgaactttggcagtttcaatgtctgtctctgttgcttcggggcataagctgatcaccgtctagttgg gaaagtaaccctacagggtttgtagggacatgatcagcatcctgatttgaaccctgaaatgttgtgtagacaccctcttgggtccaat gaggtagttggttgaagtagcaagatgttggcttttctggattttttttgccatgggttcttcactgaccttggactttggcatgattcttagtc atacttgaacttgtctcattccacctcttctcagagcaactcttcctttgggaaaagagttcttcagatcatagaccaaaaaagtcatac cttcgaggtggtagcagtagattccaggaggagaagggtacttgctaggtatcctgggtcagtggcggtgcaaactggtttcctcag |
| | | ctgcctgtccttctgtgtgcttatgtctcttgtgacaattgttttcctccct |
| **227** | 209458 _x_at | agagaacccaccatggtgctgtctcctgccgacaagaccaacgtcaaggccgcctggggtaaggtcggcgcgcacgctggcg agtatggtgcggaggccctggagaggatgttcctgtccttccccaccaccaagacctacttcccgcacttcgacctgagccacggc tctgcccaggttaagggccacggcaagaaggtggccgacgcgctgaccaacgccgtggcgcacgtggacgacatgcccaac gcgctgtccgccctgagcgacctgcacgcgcacaagcttcgggtggacccggtcaacttcaagctcctaagccactgcctgctgg tgaccctggccgcccacctccccgccgagttcacccctgcggtgcacgcctccctggacaagttcctggcttctgtgagcaccgtg ctgacctccaaataccgttaagctg |
| **228** | 209496 _at | tggaggaatttcacaagcacccgcccgtgcagtgggccttccaggagaccagtgtggagagcgccgtggacacgcccttccca gctggaatatttgtgaggctggaatttaagctgcagcagacaagctgccggaagagggactggaagaaacccgagtgcaaagt caggcccaatgggaggaaacggaaatgcctggcctgcatcaaactgggctctgaggacaaagttctgggccggttggtccactg ccccatagagacccaagttctgcgggaggctgaggagcaccaggagacccagtgcctcagggtgcagcgggctggtgagga cccccacagcttctacttccctggacagttcgccttctccaaggccctgccccgcagctaagccagcactgagctgcgtggtgcctc caggaccgctgccggtggtaaccagtggaagaccccagcccccagggagaggaccccgttctatccccagccatgataata |
| **229** | 209498 _at | tttgtcaggaaaccttggcctctgctaaggtgtatttggtccttgagaagtgggagcaccctacagggacactatcactcatgctggtg gcattgtttacagctagaaagctgcactggtgctaatgccccttggggaaatggggctgtgaggaggaggattataacttaggccta gcctcttttaacagcctctgaaatttatcttttcttctatggggtctataaatgtatcttataataaaaaggaaggacaggaggaagaca ggcaaatgtacttctcacccagtcttctacacagatggaatctctttggggctaagagaaaggttttattctatattgcttacctgatctca tgttaggcctaagaggctttctccaggaggattagcttggagttctctatactcaggtacctctttcagggttttctaaccctgacacgga ctgtgcatactttccc |
| **230** | 209612 _s_at | actggatgcgttaataacccatgttttaccttttgaaaaaataaatgaaggatttgacctgcttcactctgggaaaagtatccgtaccgt cctgacgttttgaggcaatagagatgccttcccctgtagcagtcttcagcctcctctaccctacaagatctggagcaacagctagga aatatcattaattcagctcttcagagatgttatcaataaattacacatgggggctttccaaagaaatggaaattgatgggaaattatttt caggaaaatttaaaattcaagtgagaagtaaataaagtgttgaacatcagctggggaattgaagccaacaaaccttccttcttaac cattctactgtgtcacctttgccattgaggaaaaatattcctgtgacttcttgcattttggtatcttcataatctttagtcatcgaatcccagt ggaggggacccttttacttgccctgaacatacacatgctggccattgtgattgaagtcttctaactctgtctcagt |
| **231** | 209613 _s_at | gcagatttcttgcttcatatgacaaagcctcaattactaattgtaaaaactgaactattcccagaatcatgttcaaaaaatctgtaatttt gctgatcgaaagtgcttcattgactaaacagtattagtttgtggctataaatgattatttagatgatgactgaaaatgtgtataaagtaat taaaagtaatatggtggctttaagtgtagagatgggatggcaaatgctgtgaatgcagaatgtaaaattggtaactaagaaatggc acaaacaccttaagcaatatatttcctagtagatatatatacacatacatatatacacatatacaaatgtatatttttgcaaaattgttt tcaatctagaacttttctattaactaccatgtcttaaaatcaagtctataatcctagcattagtttaatattttgaatatgtaaacacctgtgtt aatgctttgttaatgcttttcccactctcatttgttaatgctttcccactctcgggaaggatttgcattttgagctttatctctaaatgtgacatg ca |
| **232** | 209621 _s_at | ggatgccgctctgtgacaaatgtggcagtggcatagtcggtgctgtggtgaaggcgcgggataagtaccggcaccctgagtgcttc gtgtgtgccgactgcaacctcaacctcaagcaaaagggctacttcttcatagaaggggagctgtactgcgaaacccacgcaaga gcccgcacaaaacccccaagaggctatgacacggtcactctgtatcccaaagcttaagtctctgcaggcgtggcacacgcacgc acccacccacgcgcacttacacgagaagacattcatggctttgggcagaaggattgtgcagattgtcaactccaaatctaaagtc aaggctttagacctttatcctattgtttattgaggaaaaggaatggaggcaaatgcctgctatgtgaaaaaaacatacacttagcta tgttttgcaactctttttggggctag |
| **233** | 209651 _at | ttcctgaagctcttcggctgacagcccgctcggctcgccctctcccccggaggccgcgccctcccggaaaagccgggtcctccag accccgaggccttgctctcagagcgggaggccccacccactggagagccccgcccctaaggtactatgagttctcaggggtcaa gttcagaaacggcccagccagacctaaacccacacgcccacaaagtggattgcacacagacaagaactcccgtgcgggcctc cactctattcccacccttgagggagccccccttactgggggagggtccttgcaattccagcgaatcggaggccaggccaggacgtc cttgctccctgcaccctcactgttctgtgcactttttctacctacataaacacacgcatt |
| **234** | 209656 _s_at | gcagggctagttattccgatttcttgcacaattatttagctttttgtaagttcaacatgtaaattttaaagacataaatatagagagacttat gtgtttgaatataaatgatatatatggattagcatgtacctgtatattattaaacatgcaatgaactgactggtaagtgacgtctaattgta tggctagcaatgtaatttattcagactgtatttttgtacagagcagtgcactctaacctatgcctctgtgtcctctttaatgcctaaagctgt gcctagaaatttcatctgtcttaaaagtaaaatatacttcatgctgtttatgctattagttctgtactgctattctatatttattatttttaaatat atgacatgtttactacttaaacatgaattcatggtatcctggttattttttttaagtcatctgggggaaaacctgtttatcactccagtgatttt gagtttgcagtttcacaatcagttcttcat |
| **235** | 209667 _at | caggccttttccactttgagggaggtgcttcgaagaatgttgcccacacctaagtgttagaagcctatgtccgttcatccctgagaggt ctgaa |
| **236** | 209668 _x_at | tggcggactccatgtttgtgatccctgcactccaagtagcacattttcagtgttcccgggcccctgtgtacttctacgagttccagcatc agcccagctggctcaagaacatcaggccaccgcacatgaaggcagaccatggtgatgagcttccttttgttttcagaagtttctttgg gggcaactacattaaattcactgaggaagaggagcagctaagcaggaagatgatgaagtactgggccaactttgcgagaaatg ggaacccgaatggcgagggtctgccacactggccgctgttcgaccaggaggagcaatacctgcagctgaacctacagcctgcg gtgggccgggctctgaaggcccacaggctccagttctggaagaaggcgctgccccaaaagatccaggagctcgaggagcctg aagagagacacacagagctgtagctccctgtgccggggaggagggggtgggttcgctgacaggcgagggtcagcctgctgtg |
| **237** | 209687 _at | gagagctcgctttgagtgactgggttttgtgattgcctctgaagcctatgtatgccatggaggcactaacaaactctgaggtttccgaa atcagaagcgaaaaaatcagtgaataaaccatcatcttgccactacccccctcctgaagccacagcagggtttcaggttccaatca gaactgttggcaaggtgacatttccatgcataaatgcgatccacagaaggtcctggtggtattgtaactttttgcaaggcatttttttata tatatttttgtgcacatttttttttacgtttctttagaaaacaaatgtatttcaaaatatatttatagtcgaacaattcatatatttgaagtggagc catatgaatgtcagtagtttatacttctctattatctcaaactactggcaatttgtaaagaaatatatatgatatataaatgtgattgcagct tttcaatgttagccacagt |
| **238** | 209735 _at | tgtggtttctgtagcaacacttctcatgaccatctgttttgtgtttatgatgatttttcaggtctgttggtcaatctcacaaccattgcatcttgg ctgtcatggcttcagtacttcagcattccacgatatggatttacggctttgcagcataatgaatttttgggacaaaacttctgcccagga ctcaatgcaacaggaaacaatccttgtaactatgcaacatgtactggcgaagaatatttggtaaagcagggcatcgatctctcacc ctggggcttgtggaagaatcacgtggccttggcttgtatgattgttattttcctcacaattgcctacctgaaattgttatttcttaaaaatat tcttaaatttccccttaattcagtatgatttatcctcacataaaaaagaagcactttgattgaagtattcaatcaagtttttttgttgttttctgtt cccttgccatcacactgttgcacagca |
| **239** | 209763 _at | gaacacgcagagagtttccctagatatactcctgcctccaggtgctgggacacacctttgcaaaatgctgtgggaagcaggagct ggggagctgtgttaagtcaaagtagaaaccctccagtgtttggtgttgtgtagagaataggacatagggtaaagaggccaagctg cctgtagttagtagagaagaatggatgtggttcttcttgtgtatttatttgtatcataaacacttggaacaacaaagaccataagcatca tttagcagttgtagccattttctagttaactcatgtaaacaagtaagagtaacataacagtattaccctttcactgttctcacaggacatg tacctaattatggtacttatttatgtagtcactgtatttctggatttt |
| **240** | 209791 _at | ctccagccccagagctgaaaacaccaagtgcctatttgagggtgtctgtctggagacttagagtttgtcatgtgtgtgtgtgtnnttggt taatgtgggtttatgggtttcttcttttttttttttttttttnnagtctacattaggggaagtgagcgcctcccatgtgcagacagtgtgtcttt atagatttttctaaggctttccccaatgatgtcggtaatttctgatgtttctgaagttcccaggactcacacacccgttcccatctcacttgc ccacccagtgtgacaaccctcggtgtggatatacccccgtggactcatggctcttccccacccccactttctataaatgtaggcctag aatacgcttctctgttgcaaaactcagctaagttcctgcttccaccttgatgttgaaatatcttatgtaagagggcaggggatgtcgtga agatggc |
| **241** | 209868 _s_at | ggaatgacacttacttacgacccaactacagctgctatacagaacggattttatccttcaccatacagtattgctacaaaccgaatg atcactcaaacttctattacaccctatattgcatctcctgtatctgcctaccaggtgcaaagtccttcgtggatgcaacctcaaccatat attctacagcaccctggtgccgtgttaactccctcaatggagcacaccatgtcactacagcccgcatcaatgatcagccctctggcc cagcagatgagtcatctgtcactaggcagcaccggaacatacatgcctgcaacgtcagctatgcaaggagcctacttgccacagt atg |
| **242** | 209948 _at | ggaacgaaaccagcgtcctattccagcgcctctacggccccaggccctcctcttctccctcttctggcccaccttcctgctgaccgg tggcctcctcattatcgccatggtgaagagcaaccagtacctgtccatcctggcggcccagaagtagagccatccatccatgccat accacttgtcagggcacaggggactggctgggcccccagggctgctccccacttgcagcacaatgccttctccacctgccctccc actcttccagtccaatccacgctgtcttctgttgcaggactaacctttgagaaatccttttgtgaagtcattgcctgctcaagaatgtaca gtggctccccaatgccttggagccataaggccagccagttctagctctctattacctgtccccactcaactgactcatacctgtttccg gctgcatcactatgtgccccacagagaacgatgatcgtcacctctgtgcctgagttctccctgttgtctcaaagcggtacc |
| **243** | 210084 _x_at | ccggtcagcaggatcatcgtgcacccacagttctacatcatccagactggagcggatatcgccctgctggagctggaggagccc gtgaacatctccagccgcgtccacacggtcatgctgccccctgcctcggagaccttcccccggggatgccgtgctgggtcactgg ctggggcgatgtggacaatgatgagcccctcccaccgccatttcccctgaagcaggtgaaggtccccataatggaaaaccacatt tgtgacgcaaaataccaccttggcgcctacacgggagacgacgtccgcatcatccgtgacgacatgctgtgtgccgggaacacc cggagggactcatgccagggcgactctggagggcccctggtgtgcaaggtgaatggcacctggctacaggcgggcgtggtcag ctgggacgagggctgtgcccagcccaaccggcctggcatctacacccgtgtcacctactacttggactggatccaccactatgtcc ccaaaaagccgtgagtcaggcctggggtgt |
| **244** | 210107 _at | ggccaaatcaccgacctgaaggcggaaattcacgggggcagtctcattaatctgacttggacagctcctggggatgattatgacc atggaacagctcacaagtatatcattcgaataagtacaagtattcttgatctcagagacaagttcaatgaatctcttcaagtgaatact actgctctcatcccaaaggaagccaactctgaggaagtctttttgtttaaaccagaaaacattacttttgaaaatggcacagatcttttc attgctattcaggctgttgataaggtcgatctgaaatcagaaatatccaacattgcacgagtatctttgtttattcctccacagactccgc cagagacacctagtcctgatgaaacgtctgctccttgtcctaatattcatatcaacagcaccattcctggcattcacattttaaaaattat gtggaagtggataggagaactgcagctgtcaatagcctagggc |
| **245** | 210133 _at | acccccttcagcgactagagagctacaggagaatcaccagtggcaaatgtccccagaaagctgtgatcttcaagaccaaactggc caaggatatctgtgccgaccccaagaagaagtgggtgcaggattccatgaagtatctggaccaaaaatctccaactccaaagcc ataaataatcaccatttttgaaaccaaaccagagcctgagtgttgcctaatttgttttcccttcttacaatgcattctgaggtaacctcatt atcagtccaaagggcatgggttttattatatatatatatatttttttttaaaaaaaaacgtattgcatttaatttattgaggctttaaaacttat cctccatgaatatcagttatttttaaactgtaaagctttgtgcagattctttacccccctgggagccccaattcgatcccctgtcacgtgtgg gcaatgttccccc |
| **246** | 210139 _s_at | tgtgaagctttacgcgcacacggacaaaatgcccaaactggagcccttgcaaaacacggcttgtggcattggcatacttgccctt acaggtggagtatcttcgtcacacatctaaatgagaaatcagtgacaacaagtctttgaaatggtgctatggatttaccattccttatta tcactaatcatctaaacaactcactggaaatccaattaacaattttacaacataagatagaatggagacctgaataattctgtgtaat ataaatggtttataactgcttttgtacctagctaggctgctattattactataatgagtaaatcataaagccttcatcactcccacatttttctt |
| | | acggtcggagcatcagaacaagcgtctagactccttgggaccgtgagttcctagagcttggctgggtctaggctgttctgtgcctcc aaggactgtctggcaatgacttgtattggccaccaactgtagatgtatatatggtgcccttctgatgctaagactccagaccttttgt |
| **247** | 210298 _x_at | tctactgcgtgacttgccatgagaccaagtttgccaagcattgcgtgaagtgcaacaaggccatcacatctggaggaatcacttac caggatcagccctggcatgccgattgctttgtgtgttacctgctctaagaagctggctgggcagcgtttcaccgctgtggaggacc agtattactgcgtggattgctacaagaactttgtggccaagaagtgtgctggatgcaagaaccccatcactgggaaaaggactgtg tcaagagtgagccgcccagtctctaaagctaggaagcccccagtgtgccacgggaaacgcttgcctctcaccctgtttcccagcg ccaacctccggggcaggcatccgggtggagaggacttgtccctcgtgggtggtggttctttatagaaaaatcgaagcttagca gctcctcgtggcccgggtttggtaaaggctccagtgtggtggcctatgaaggacaatcctggcacgactactgcttccactgcaaaa aatgctccgtgaatctggccaacaagcgctttgttttccacc |
| **248** | 210299 _s_at | gcataggagataaaacccccactgagatgctctcatgcctcagctggacccaccgtgtagacacacgacatgcaagagttgcag cggctgctccaactcactgctcaccctctctgtgagcagaaagaaccctactgacatgcatggttaacttcctcatcagaactctgcc cttcccttctgttcttttgtgctttcaaataactaacacgaacttccagaaaattaacatttgaacttagctgtaattctaaactgacctttcc ccgtactaacgtttggtttccccgtgtggcatgcttttctgagccttcctactttaaagcatggaacatgcaggtgatttgggaagtgtag aaagacctgagaaaacgagcctgtttcagaggaacatcgtcacaacgaatacttctggaagcttaacaaaactaaccctgctgtc ctt |
| **249** | 210302 _s_at | atttcgttttgcttttggttgcctgaatgttgtcaccaagtgaaaaaattatttaactatatgtaaaatttctcttttaaaaaaaagttttactga tgttaaacgttctcagtgccaatgtcagactgtgctcctccctctcctgaacctctaccctcaccctgagctgtcttgttgaaaacagt |
| **250** | 210495 _x_at | agccctttgctctggaggaagttctccagcttcagctcaactcacagcttctccaagcatcaccctgggagtttcctgagggttttctca taaatgagggctgcacattgcctgttctgcttcgaagtattcaataccgctcagtatttaaatgaagtgattctaagatttggtttgggat caataggaaagcatatgcagccaaccaagatgcaaatgttttgaaatgatatgaccaaattttaagtaggaaagtcacccaaac acttctgctttcacttaagtgtctggcccgcaatactgtaggaacaagcatgatcttgttactgtgatattttaaatatccacagtactcac tttttccaaatgatcctagtaattgcctagaaatatctttctcttacctgttatttatcaatttttcccagtatttttatacggaaaaaattgtattg aaaacacttagtatgcagttgataagaggaatttggtataattatggtgggtgattatttttatactgtatgtgccaaagctttactactgt g |
| **251** | 210517 _s_at | gtgccatagtgcaggcttggggagctttaagcctcagttatataacccacgaaaaacagagcctcctagatgtaacattcctgatca aggtacaattctttaaaattcactaatgattgaggtccatatttagtggtactctgaaattggtcactttcctattacacggagtgtgctaa aactaaaaagcattttgaaacatacagaatgttctattgtcattgggaaatttttcttctaacccagtggaggttagaaagaagttatat tctggtagcaaattaactttacatcctttttcctacttgttatggttgtttggaccgataagtgtgcttaatcctgaggcaaagtagtgaatat gttttatatgttatgaagaaaagaattgttgtaagtttttgattctactcttatatgctggactgcattcacacatggcatgaaataagtcag gttctttacaaatggtatttgatagatactggattgtgtttgtgccatatttgtgccatt |
| **252** | 210524 _x_at | gtcaggactgttatggccctgttgtgctgagttagttggaacagaattcaagctccctgcactagtccacctgccccactgcttcttcgc ttctctcttggaaagtccagtctctcctcggcttgcaatggaccccaactgctcctgcgccgctggtgtctcctgcacctgcgctggttcc tgcaagtgcaaagagtgcaaatgcacctcctgcaagaagagctgctgctcctgctgccccgtgggctgtagcaagtgtgcccagg gctgtgtttgcaaaggggcgtcagagaagtgcagctgctgcgactgatgccaggacaacctttctcccagatgtaaacagagaga catgtacaaacctggattttttttttataccaccttgacccattgctacattccttttcctgtgaaa |
| **253** | 210735 _s_at | caggaaggaccttgctttggaccctacacacttcggctctctggacacttgcgacacctcaaggtgttctctgtagctcaatctgcaa acatgccaggcctcagggatcctctgctgggtgcctccttgccttgggaccatggccaccccagagccatccgatcgatggatggg atgcactctcagaccaagcagcaggaattcaaagctgcttgctgtaactgtgtgagattgtgaagtggtctgaattctggaatcaca aaccaagccatgctggtgggccattaatggttggaaaacactttcatccggggctttgccagagcgtgctttcaagtgtcctggaaa gtctgctgcttctccaagctttcagacaagaatgtgcactctctgcttaggttttgcttgggaaactcaacttctttcctctggagacggg gcatctccctctgatttccttctgctatgacaaaacctttaatctgcaccttacaactcggggacaa |
| **254** | 210764 _s_at | ttaccaatgacaaccctgagtgccgccttgtgaaagaaacccggatttgtgaggtgcggccttgtggacagccagtgtacagcag cctgaaaaagggcaagaaatgcagcaagaccaagaaatcccccgaaccagtcaggtttacttacgctggatgtttgagtgtgaa gaaataccggcccaagtactgcggttcctgcgtggacggccgatgctgcacgccccagctgaccaggacatgtgaagatgcggt tccgctgcgaagatggggagacattttccaagaacgtcatgatgatccagtcctcaaatgcaactacaactgcccgcatgccaatg aagcagcgtttcccttctacaggctgttcaatgacattcacaaatttagggactaaatgctacctgggtttccagggcacacctagac aaacaagggagaagagtgtcagaatcagaatcatggagaaaatgggcgggggtggtgtgggtgatgggactcattgtagaaa ggaagccttgctcattcttgaggagcattaaggtatttcgaaactgccaagggtgc |
| **255** | 210809 _s_at | aaattgtggagttagcctcctgtggagttagcctcctgtggtaaaggaattgaagaaaatataacaccttacaccctttttcatcttgac attaaaagttctggctaactttggaatccattagagaaaaatccttgtcaccagattcattacaattcaaatcgaagagttgtgaactgt tatcccattgaaaagaccgagccttgtatgtatgttatggatacataaaatgcacgcaagccattatctctccatgggaagctaagtt ataaaaataggtgcttggtgtacaaaactttttatatcaaaggctttgcacatttctatatgagtgggtttactggtaaattatgttatttttt acaactaattttgtactctcagaatgtttgtcatatgcttcttgcaatgc |
| **256** | 210946 _at | aaggcaagaggatgcatctttcttcctggtgtacaagcctttaaagacttctgctgctgatatgcctcttggatgcacactttgtgtgtac atagttacctttaactcagtggttatctaatagctctaaactcattaaaaaaactccaagccttccaccaaaacagtgccccacctgta tacatttttattaaaaaaatgtaatgcttatgtataaacatgtatgtaatatgctttctatgaatgatgtttg |
| **257** | 210982 _s_at | gaaggagacggtctggcggcttgaagaatttggacgatttgccagctttgaggctcaaggtgcattggccaacatagctgtggaca aagccaacttggaaatcatgacaaagcgctccaactatactccgatcaccaatgacaagttcaccccaccagtggtcaatgtcac gtggcttcgaaatggaaaacctgtcaccacaggagtgtcagagacagtcttcctgcccagggaagaccaccttttccgcaagttcc actatctccccttcctgccctcaactgaggacgtttacgactgcagggtggagcactggggcttggatgagcctcttctcaagcactg ggagtttgatgctccaagccctctcccagagactacagagaacgtggtgtgtgccctgggcctgactgtgggtctggtgggcatcat tattgggaccatc |
| **258** | 211161 _s_at | aatattggatatcaactgcttgtaaaggtgctcctcctttttcttgtcattgctggtcaagattactaatatttgggaaggctttaaagacgc atgttatggtgctaatgtactttcacttttaaactctagatcagaattgttgacttgcattcagaacataaatgcacaaaatctgtacatgt ctcccatcagaaagattcattggcatgccacaggggattctcctccttcatcctgtaaaggtcaacaataaaaaccaaattatgggg ctgcttttgtcacactagcatagagaatgtgttgaaatttaacttgtaagcttgtatgtggttgttgatcttttttttccttacagacacccata at |
| **259** | 211372 _s_at | atctcatacccgcaaattttaaccttgtcaacctctggggtattagtatgccctgacctgagtgaattcacccgtgacaaaactgacgt gaagattcaatggtacaaggattctcttcttttggataaagacaatgagaaatttctaagtgtgaggggaccactcacttactcgtac acgatgtggccctggaagatgctggctattaccgctgtgtcctgacatttgcccatgaaggccagcaatacaacatcactaggagt attgagctacgcatcaagaaaaaaaaagaagagaccattcctgtgatcatttccccctcaagaccatatcagcttctctggggtc aagactgacaatcccgtgtaaggtgtttctgggaaccggcacacccttaaccaccatgctgtggtggacggccaatgacacccac atagagagcgccta |
| **260** | 211538 _s_at | agctcgaaagagtttgcaaccccatcatcagcaaactttaccaaggtggtcctggcggcggcagcggcggcggcggttcagga gcctccgggggacccaccatcgaagaagtggactaagcttgcactcaagtcagcgtaaacctctttgcctttctctctctctcttttttttt gtttgtttctttgaaatgtccttgtgccaagtacgagatctattgttggaagtcttggtatatgcaaatgaaaggagaggtgcaacaact tagtttaattataaaagttccaaagtttgtttttaaaaacattattcgaggtttctctttaatgcattttgcgtgtttgctgacttgagcatttttg attagttcgtgcatggagatttgtttgagatgagaaaccttaagtttgcacacctgttctgtagaagcttg |
| **261** | 211548 _s_at | gctttttttgttgtcagctatcttaagaatcattaaatacacctgctttgggtaaaactctttgcaagcagtaattaacactagtaacagtg aaagcacaagatttccaaatcagtcgttttctcaaaaaaatatcgtataagtgactcatcctgtctgctaactccagacctcccagctt gaagccaaatctttccatgtgagattgatatggatttcctagaagtactggaatgttgtcatatcttgccctattttaattctgctatagaaa acaattgccttcacttttaaggagtaatttgaatattaataactctggtctagattttcatataatgtattaaagacaaagtagtgaacatc aatgaacatctgatagagataaactgtaatcaggcataagcttgtttgtatgttctggcagtgactaatcagtaaatgatgtcggtttgc ccagtatcacttatcttctg |
| **262** | 211549 _s_at | cgtgaacggcaaagtggcgctggtgaccggcgcggctcagggcataggcagagcctttgcagaggcgctgctgcttaagggcg ccaaggtagcgctggtggattggaatcttgaagcaggtgtacagtgtaaagctgccctggatgagcaatttgaacctcagaagact ctgttcatccagtgcgatgtggctgaccagcaacaactgagagacacttttagaaaagttgtagaccactttggaagactggacatt ttggtcaataatgctggagtgaataatgagaaaaactgggaaaaaactctgcaaattaatttggtttctgttatcagtggaacctatctt ggtttggattacatgagtaagcaaaatggaggtgaaggcggcatcattatcaatatgtcatctttagcagcccaccattgattgccaa tggattgataacactcattgaagatgatgctttaaatggtgctattatgaagatcacaacttctaagggaattcattttcaagactatgat acaactccatttcaag |
| **263** | 211596 _s_at | gagtattacattggccttgggggacagaaaggaggaagttctgacttttcagggctaccttatttctactaaggacccagagcaggc ctgtccatgccattccttcgcacagatgaaactgagctgggactggaaaggacagcccttgacctgggttctgggtataatttgcact tttgagactggtagctaaccatcttatgagtgccaatgtgtcatttagtaaaacttaaatagaaacaaggtccttcaaatgttcctttggc caaaagctgaagggagttactgagaaaatagttaacaattactgtcaggtgtcatcactgttcaaaaggtaagcacatttagaatttt gttcttgacagttaactgactaatcttacttccacaaaatatgtgaatttgctgcttctgagaggca |
| **264** | 211637 _x_at | ggactggtgaagccttcggagaccctgtccctcacctgcagtgtctctggtggctccatcagtagttactactggagctggatccggc agcccccagggaagggactggagtggattgggtatatcaattacagtgggagcaccaactataacccctccctcaagagtcgag tcaccatatcagtagacacgtccaagaaccagttctccctgaagctgagctctgtgaccgctgcggacacggccgtgtattactgtg cgagaacgatttcggggggtcg |
| **265** | 211643 _x_at | aatagtgatgacgcagcttccagccaccctgtctgtgtctccaggggaaagagccaccctctcctgcagggccagtcagagtgttc gcagcaacttagcctggtaccagcaaaaacctggccaggctcccaggctcctcatctacggtgcatccaccagggccaatggtat cccagccaggttcagtggcagtgggtctgggacagagttcactctcaccatcagcggcctgcagtctgacgattttgcagtttattact gtcaacactataataactggcctccgtggacgt |
| **266** | 211644 _x_at | aattgtgttgacgcagtctccaggcaccctgtctttgtctccaggggaaagagccaccctctcctgcagggccagtcagagtattag cagcagtttcttagcctggtaccagcagaaagttggccaggctcccaggctcctcatctatggtgcatccagcagggccactggca tcccagacaggttcagtggcagtgggtctgggacagacttcactctcaccatcagcagactggagcctgaagattttgcagtgtatt actgtcagcaatatggtagctcgc |
| **267** | 211645 _x_at | tcctccctgtctgcatctgtaggagacagagtcaccatcacttgccgggcaagtcagggcattagaaatgatttagcctggtatcag cagaaaccagggaaagcccctaagcgcctgatctatgctgcatccagtttgcaaagtggggtcccatcaaggttcagcggcagt ggatctgggacagaattcactctcacaatcagcagcctgcagcctgaagattttgcaacttattactgtctacagcataatagttacc cttg |
| **268** | 211671 _s_at | gcagttgagtcgtcatcacttttcagtgatgggagagtagatggtgaaatttattagttaatatatcccagaaattagaaaccttaatat gtggacgtaatctccacagtcaaagaaggatggcacctaaaccaccagtgcccaaagtctgtgtgatgaactttctcttcatactttttt ttcacagttggctggatgaaatttctagactttctgttggtgtatcccccccctgtatagttaggatagcattttgatttatgcatggaaac ctgaaaaaaagtttacaagtgtatatcagaaaagggaagttgtgccttttatagctattactgtctggttttaacaatttcctttatatttagt gaactacgcttgctcatttttcttacataatttttattcaagttattgtacagctgtttaagatgggcagctagttcgtagctttccca |
| **269** | 211696 _x_at | tgaggagaagtctgccgttactgccctgtggggcaaggtgaacgtggatgaagttggtggtgaggccctgggcaggctgctggtg gtctacccttggacccagaggttctttgagtcctttggggatctgtccactcctgatgctgttatgggcaaccctaaggtgaaggctcat ggcaagaaagtgctcggtgcctttagtgatggcctggctcacctggacaacctcaagggcacctttgccacactgagtgagctgca ctgtgacaagctgcacgtggatcctgagaacttcaggctcctgggcaacgtgctggtctgtgtgccggcccatcactttggcaaaga attcacccaaccagtgcaggctgcctatcagaaagtggtggctggtgtggctaatgccctggcccacaagtatcactaagctcgctt tcttgctgtccaatttctattaaaggttcctttgttccctaagtccaactactaaactg |
| **270** | 211699 _x_at | agagaacccaccatggtgctgtctcctgacgacaagaccaacgtcaaggccgcctggggtaaggtcggcgcgcacgctggcg agtatggtgcggaggccctggagaggatgttcctgtccttccccaccaccaagacctacttcccgcacttcgacctgagccacggc tctgcccaggttaagggccacggcaagaaggtggccgacgcgctgaccaacgccgtggcgcacgtggacgacatgcccaac gcgctgtccgccctgagcgacctgcacgcgcacaagcttcgggtggacccggtcaacttcaagctcctaagccactgcctgctgg tgaccctggccgcccacctccccgccgagttcacccctgcggtgcacgcctccctggacaagttcctggcttctgtgagcaccgtg ctgacctccaaataccgttaagctg |
| **271** | 211719 _x_at | gggagtttcctgagggttttctcataaatgagggctgcacattgcctgttctgcttcgaagtattcaataccgctcagtatttaaatgaa gtgattctaagatttggtttgggatcaataggaaagcatatgcagccaaccaagatgcaaatgttttgaaatgatatgaccaaaatttt aagtaggaaagtcacccaaacacttctgctttcacttaagtgtctggcccgcaatactgtaggaacaagcatgatcttgttactgtgat attttaaatatccacagtactcactttttccaaatgatcctagtaattgcctagaaatatctttctcttacctgttatttatcaatttttcccagta tttttatacggaaaaaattgtattgaaaacacttagtatgcagttgataagaggaatttggtataattatggtgggtgattatttttttatactg tatgtgccaaagctttactactgtg |
| **272** | 211745 _x_at | tcctgccgacaagaccaacgtcaaggccgcctggggtaaggtcggcgcgcacgctggcgagtatggtgcggaggccctggag aggatgttcctgtccttccccaccaccaagacctacttcccgcacttcgacctgagccacggctctgcccaggttaagggccacgg caagaaggtggccgacgcgctgaccaacgccgtggcgcacgtggacgacatgcccaacgcgctgtccgccctgagcgacctg cacgcgcacaagcttcgggtggacccggtcaacttcaagctcctaagccactgcctgctggtgaccctggccgcccacctccccg ccgagttcacccctgcggtgcacgcctccctggacaagttcctggcttctgtgagcaccgtgctgacctccaaataccgttaagctg gagcctcggtggc |
| **273** | 211798 _x_at | tacgcagactccgtgaagggccgattcaccatctccagagacaattccaagaacacgctgtatcttcaaatgaacagcctgagag ccgaggacacggccgtattactgtgcaagagagacttgtgtgattgggccaaggtaccctggtcaccgtgtcgagaggtggcgg tggctcgggcggtggtgggtcgggtggcggcggatcttctgagctgactcaggaccctgctgtgtctgtggccttgggacagacagt caggatcacatgccaaggagacagcctcagaagctattatgcaagctggtaccagcagaagccaggacaggcccctgtacttg tcatctatggtaaaaacaaccggccctcagggatcccagaccgattctctggctccagctcaggaaacacagcttccttgaccatc actggggctcaggcggaagatgaggctgactattactgtaactcccgggacagcagtggtaaccatgtggtattcggcggaggg accaagctgaccgtcctaggtgcggccgcagaacaaaaactcatctcagaagaggatctgaatggggccgcat |
| **274** | 211813 _x_at | ttgcacaagtttcctgggctggaccgtttcaacagagaggcttatttgactttatgctagaagatgaggcttctgggataggcccaga agttcctgatgaccgcgacttcgagccctccctaggcccagtgtgccccttccgctgtcaatgccatcttcgagtggtccagtgttctg atttgggtctggacaaagtgccaaaggatcttcccccctgacacaactctgctagacctgcaaaacaacaaaataaccgaaatca aagatggagactttaagaacctgaagaaccttcacgttgtctaccttcataacaacaatatctctgtagttggatcaagtgacttctgc ccacctggacacaacaccaaaaaggcttcttattcgggtgtgagtcttttcagcaacccggtccagtactgggagatacagccatc caccttcagatgtgtctacgtgcgctctgccattcaactcggaaacta |
| **275** | 211848 _s_at | tgtccatacagagtgtgcattccctggcaggggctcctgctcacagcctcgcttttaaccttctggaacctgccaaacagtgcccaga ccaatattgatggtgtgccgttcaatgtcgcagaagggaaggaggtccttctagtagtccataatgagtcccagaatctttatggcta caactggtacaaagggcaaagggtgcatgccaactatcgaattataggatatgtaaaaaatataagtcaagaaaatgccccagg gcccgcacacaacggtcgagagacaatataccccaatggaaccctgctgatccagaacgtcacccacaatgacgcaggaatc tataccctacacgttataaaagaaaatcttgtgaatgaagaagtaaccagacaattctacgtattctatgagtcagtacaagcaagtt cacctgacctctcagctgggaccgctgtcagcatcatgattggagtactggctgggatggctctgatatagc |
| **276** | 211889 _x_at | tgcacagtactcctggcttatcaatggaacattccagcaaagcacacaagagctctttatccctaacatcactgtgaataatagtgg atcctatacctgccacgccaataactcagtcactggctgcaacaggaccacagtcaagacgatcatagtcactgagagacagaa tctcaccatgttgcccggctggactcgaactcctgggctcaagcaatcctcccatctgtttcccaaagtgctgagattacagataat gctctaccacaagaaaatggcctctcacctggggccattgctggcattgtgattggagtagtggccctggttgctctgatagcagtag ccctggcatgttttctgcatttcgggaagaccggcagggcaagcgaccagcgtgatctcacagagcacaaaccctcagtgtccaa ccacactcaggaccactccaatgacccacctaacaagatgaatgaagttacttattctaccctgaactttgaagccca |
| **277** | 211896 _s_at | tcctccttcccttacggaattacatcttgatggcaacaaaatcagcagagttgatgcagctagcctgaaaggactgaataatttggct aagttgggattgagtttcaacagcatctctgctgttgacaatggctctctggccaacacgcctcatctgagggagcttcacttggaca acaacaagcttaccagagtacctggtgggctggcagagcataagtacatccaggttgtctaccttcat |
| **278** | 211959 _at | tcacaccgtagcccacatggatagcacagttgtcagacaagattccttcagattccgagttgcctaccggttgttttcgttgttgttgttgt tgtttttctttttcttttttttttgaagacagcaataaccacagtacatattactgtagttctctatagttttacatacattcataccataactctgt tctctcc |
| **279** | 211964 _at | cactttccaatgccacagacaactcacattgttcaactcccnttctcggggtggacagacgagacaacagcacacaggcagcc agccgtggnnccagaggctcganggggctcaggggcntcanggcacccgtccccacacgnagggccccgtngggtgggnc ctnggccctngctttctacngccnaatgnttatgnccagctccatgttctcccaaataccgttgatgtgaattattttaaaggcaaaac ngtgctctttattttanaaaacactgataatcacactgcggtaggtcattcttttgccacatccctatagaccactgggtttggcaaaact caggcagaagtggaganccnttctagacatcantgtcagccttgctacttgaaggtacaccccatagggtcggaggtgctgtccc c |
| **280** | 211985 _s_at | gaggcaaatggatctcgatatttcagatgggcttttgatgcactgttgccaaggaaggctttttctgatttttgacaaatgaatttttgcac actttcattggtgtctttcggcaacttacacacattgaaaat |
| **281** | 211990 _at | gaggacttaggagagatctgaactccagctgccctacaaactccntcncagcttttcttctcacttcatgtgaaaactactccagtgg ctgactgaattgctgacccttcaagctctgtccttatccattacctcaaagcagtcattccttagtaaagtttccaacaaatagaaatta atgacactttggtagcactaatatggagattatcctttcattgagccttttatcctctgttctcctttgaagaacccctcac |
| **282** | 211991 _s_at | gcactgggaggcccaagagccaatccagatgcctgagacaacggagactgtgctctgtgccctgggcctggtgctgggcctagt nggcatcatcgtgggcaccgtcctcatcataaagtctctgcgttctg |
| **283** | 212077 _at | tgcagaagtcaaaccctcatgacaaagtaggcacaagtctacaataagctaaatcagaatttacaaatacaagtgtcccaggta gcattgactcccgtcattggagtgaaatggatcaaagtttgaattaaggcctatggtaaggtaacattgctttgttgtactttgaacaa gagctcctcctgatcactattacatatttttctagaaaatctaaagttcagaagagaatgtatcactgctgacttttattccaatatttggat ggagtaagttttagggtagaattttgttcagtttggatttaatcttttgaaaagtaaattccttgtttactggtttgactataattctctgttatcttt acgaggtaaaactgcaagctgactagcatgttctgtgaatctgccattcctaaaaatttataaacacttgatacttttcactgataatg gatcgctcca |
| **284** | 212091 _s_at | tcacagcgggcaggacggaccccgcccacgacgtgcgggtggcggtggtgcagtacagcggcacgggccagcagcgccca gagcgggcgtcgctgcagttcctgcagaactacacggccctggccagtgccgtcgatgccatggactttatcaacgacgccaccg acgtcaacgatgccctgggctatgtgacccgcttctaccgcgaggcctcgtccggcgctgccaagaagaggctgctgctcttctca gatggcaactcgcagggcgccacgcccgctgccatcgagaaggccgtgcaggnagnccagcgggcaggcatcgagatcttc gtggtggtcgtgggccgccaggtgaatgagccccacatccgcgtcctggtcaccggcaagacggccgagtacgacgtggccta cggcgagagccacctgttccgtgtccccagctaccaggccctgctccgcggtgtcttccaccagacagtctccaggaaggt |
| **285** | 212097 _at | gaatttcacctgtaaacctgagtcgtacagaaagctgcctggtatatccaaaangcttttattcctcctgctcatattgtgattctgccnt ttggggnacttttncttnaaaccttcagttatgattttttttncatacacttattggnaactctgcttgattttgcctcttccagtcttcctgaca ctttaattaccaacctgttacctactttgacttttgcatttaaaacagacactggcatggatatagttttacttttaaactgtgtacataact gaaaatgtgctatactgcatactttttaaatgtaaagatatttttatcttatatgaagaaaatcacttaggaaatggctttgtgattcaatct gtaaactgtgtattccaagacatgtctgttctacatagatgcttagtccctcatgcaaatcaattactggtccaaaagattgctgaaattt tatatgcttactgatatattttacaatttttatcatgcatgtcctgtaaaggttacaagcctgcacaataa |
| **286** | 212136 _at | tcttccccttgagcttcagagaggagagttggcatggttaaatctgaatggttacctcactgctgaaaacccagaggggcgtggcac actcgcttgtgtggaaaagcctctaaatgcatcccttcctttctttcctgcttcctttgccttacaattgaagcagcccgtggtaccatcac agtatgcagagacttcctcacctttcatatctagggaccacccccgatgcattggtgagggtgggcacttataaatgcctgctattgtt aagccattccagcctcttcctctgaatagaccagacgccctttcacttagttcagtgccagtccttttgccttcccaaccctgctgttagg cctgctgttccctttgctcttgattaggagagatggaaggagatgagctcccataactgaattggcctttggttcatgttttctccccatat gtatatatgccatatgtgaatatgccatatatatgtgccaacaaatctatctacgttgttcttttcaaattagcacgcagataggaattttg agtttcttcttctt |
| **287** | 212158 _at | tatgtaaactttaacttccactttgtataaatttttaagtgtcagactatccattttacacttgctttatttttcattacctgtagctttgggcagat ttgcaacagcaaattaatgtgtaaaattggattattactacaaaaccgtttagtcatatctatctaatcagatcttcttttgggaggatttg atgtaagttactgacaagcctcagcaaacccaaagatgttaacagtattttaagaagttgctgcagattcctttggccactgtatttgtt aatttcttgcaatttgaaggtacgagtagaggtttaaagaaaaatcagtttttgttcttaaaaatgcatttaagttgtaaacgtcttttaag cctttgaagtgcctctgattctatgtaacttgttgcagactggtg |
| **288** | 212185 _x_at | cgccggctcctgcaaatgcaaagagtgcaaatgcacctcctgcaagaaaagctgctgctcctgctgccctgtgggctgtgccaag tgtgcccagggctgcatctgcaaaggggcgtcggacaagtgcagctgctgcgcctgatgctgggacagccccgctcccagatgt aaagaacgcgacttccacaaacctggattt |
| **289** | 212192 _at | caactgtttttgcgactttataggcaggtaaattttgctattactattgaatacaaatgacaattcatttatgaccactcaaacagcgttag taaccatttagtgacaaaggattaaaacatccatctggatgttaattttgaagatgtaaattatatgttgtttaaatttttccaggcatctga aaaccttatctgctagacaatgtaagattcacacagagttatctgggattctgatttttaaatagtacatatcattaaaccattttctctaa atgtaagaagagcagaaaaaatcttataagattatcagatttttctaatgacacagaaatgtaagaaaaaaatccctttatattgaaa aaagatgcagtcaaagtcttttcagacatgcccaaactttgagaatttcttcaaccatctaatgctataaagatttttgttcttcctgttcac aaccagttgtataacagaaatactagctactgttttccttc |
| **290** | 212195 _at | gatgggtcgtgtgatgagatgcatttaaggccgatagtgatagatgtttttttatttcttgaacacaggctttgtctgaatgatgttcttttat ctcttgaacacaagctttgaatgataactacaggttttaagtgctgttacattaataccataatgtgatgtgttagaaacaaagggatat |
| | | ttcaaaggtagatatttgaaaattctctagtctcaatatgtatgtgtattgaatatactctaaaaataaatgtgcaatttgctagtaggaca atgcagtgactgactagcattaggtatgtttcttttatatcctagctatgtcccactttcttctaagtgcaatcctttcatgttcacttgctgtttt accccatctactctaacttcatttggaaggcttgtctagagtatagcatgtatttttacctttgcagtgaattgcatgtgctaattgtaacca cagctatttttatgttgacataactccaaatgttatattaaatgttctattatatattagctctaatcccttaagtaaa |
| **291** | 212224 _at | acagtgttctctaatgttacagatgagatgcgcattgccaaagaggagatttttggaccagtgcagcaaatcatgaagtttaaatcttt agatgacgtgatcaaaagagcaaacaatactttctatggcttatcagcaggagtgtttaccaaagacattgataaagccataacaa tctcctctgctctgcaggcaggaacagtgtgggtgaattgctatggcgtggtaagtgcccagtgcccctttggntgggattcaagatgt ctggaaatggaagagaactgggagagtacggtttccatgaatatacagaggtcaaaacagtcacagtgaaaatctctcagaag aactcataaagaaaatacaagagtggagagaagctcttcaatagctaagcatctccttacagtcactaatat |
| **292** | 212230 _at | caaggtcccactctaggtgatagacagggaccccttctactgaacctttgaggaaaggaggaaggaagaaatgcgtttagatctt ggatgcagacctttcaaagggttaaatgtaaccatatggatcaaccacatgcacatccttactacagaatccgtcctttcatttcaact tatagcaagctatgattttatatataaatattatataaataatgtataaaacattaaaagttaactatgtaagatattatttctgaaacaat ttagctatatccactatgattataaactgtgtctcgacctgtgttatttacattagctgcttaaaaaagcattgagttaattttttaaatatca actaaaatatcatagttctgtggtagacattgttttataatgaaataactgcaactagagaaaactgtataaaaacattaaattgtcagt atttttgtaaggttccattttgtaaagagaataatattcaaagactttgtagcatacaaagtgaaaacttgtatctgcgaaacta |
| **293** | 212233 _at | aagcagcctagggatgagcatttctttgaaagcaattaggttattcacctggtattaaaactatttactgttaaaaatctgtgacttcat gaagttgatttttaaaggcagcatcaaaaactgaaaaggnaagggaaaaaataggcagcttctctgcacttgtttggagctcccca aaacaggagccatggagaagtggcatcaagaccgggctgccctttcgagaacaccctgtggcagttcagagacacgcttttcct acactgcatgcagcccctctttccagcactggaaagaagtggtcttgagcccagctgagaagcacttcacactcctctctcttgttctg aatggtgtttgtgtcagtctgcagctgtgtatggtattatgtcttataatcctgcatcacttctatcctatccagtcatat |
| **294** | 212265 _at | gggtagatgggagtgtcgcttgtatgttatcgtacagctgacatgtatttttgtctattctttattatcttagtttcatgctatgtatgtaccata accaacctattgcctatgagaaacatgtaagataatgtatttacagccattgttacaagtttataatgtatttttctatcttgttttatatgtat gttatataacattcaaaaagaattttttcttgattgagaaaaggatacaaaatgcaaaatccacaattttgataactgaaaattgcca attgttttgcagtactttattatattgggtgtcttgtctttttttgggcttttagttagctaatgaatgaatacattagacacttttgggttttagttgg gattttacatagcttgcatttttaattctttggttctttgctgtttctattaacccacagcattatt |
| **295** | 212288 _at | gaaggttgtcatgggctgttccttgggggtttttatcctgctcaccgtggagataagcctgcggcttgtctaaccagcgcagcgcaaa ggtctcaatgccttttggtaacatccgtcattgcagaagaaagtttacacgacgtcaaaaagtgacgttcatgctaagtgtttttccag aaatattggtttcatgtttcttattggctctgcctcctgtgcttatatcatccaaaaactttttaaaaaggtccagaattctattttaacctgat gttgagcacctttaaaacgttcgtatgtgtgttgcactaattctaaactttggaggcatttgctgtgtgaggccgatcgccactgtaaag gtcctagagttgcctgtttgtctctggagat |
| **296** | 212386 _at | gagatttaccatgtatcagtgcctggctttttgttataaagcttgtttgtctagtgctcttttgctataaaatagactgtagtacaccctagta ggaaaaaaaaaaactaaatttaaaaataaaaaatatatttggcttatttttcgcaggagcaatccttttataccatgaatatt |
| **297** | 212387 _at | gtttatacaactgtcagtccacccagtggtgcaactggttctgattcagtcttccgatnccttttattttcacttttcctatttctgaattttttt ttnatttgtgatcttgattttgatgaggggttggggagtggggagggagtcgaaccaagacttggagttaagaggattttcatcttttgc atccaacaggcagaatatgatctgtgtccaaaagtgaacttgagtcaggaatgaatcaatttcagcataaacaagcacaaaaattt agtctgctggctgactggaagcaaaaaagtcaagatggaatatgatgaattccaacacaatggggcaccaaggcctttaggcct ctctttttatttgctttggtttgttgtttttctttagagacatgctctttctcatgggacttgaagtggactcatcttgtgcagtgctggttttgcc atactcatttcaagtattatagacatatgtaatggtgaaaatatatgaactgtggccttttcattcttgttacttgtg |
| **298** | 212397 _at | gaagtgactgttgtaccatggttgtgcacatgcttcagaatcctatggaagagaatattcctacttgcagtacatcaaggaatggat ggtggaccctactattcatgtttgagacataaatgttcactttaaagcaattgcataatagataaaaacctgaactttcattggattttgt taattttcctcattttgaattatgtgcactaccatagctacatcagtttgatacagtattgaaaaattatcagttatatttgctgtttatgatcta tttgtagattaggattaaaatggatttaatccatttttaaggctgtgtgaatttttctaaacaagaaccatttgcaatatggatttcttagaga ttaaaccaattataacttattagcagtcgcgagcacatgttcatatagtcaatgtaaaaatacactaatgagtatttggtaaatcccagt aggcttttaccattagca |
| **299** | 212414 _s_at | tccctcacttctcttaagccaagaagtttgctttccctagctgcagtgtagatggctcttgtttttgtttttttgttttaatcatttggcattcacat gtggctgttaatatgtgcttgttttt |
| **300** | 212419 _at | tggtgggagggctctcaatcagcagggccccaggagggaagaagaagtggggcaaagcntggcctcgccgctcgggagcttt gccatctgagccacgcctcctccaggccatgctccttgaacttggaaatgtcaaccggagcccttacaccagccctccagcatcta atagacttgaatctactctaaacgaatatttaatccaacctcactacattgtagctcagtccaacgactaaccctgaaatgggggtgtt ccagccttcagcgagatggccaagcggtcccctgggggctgtggcagcggncttatccttctctgttgccaaccttgccgtccgacc tcctccgcccccatgcggtgaccccgtccgtgtctgtgtctgtccatacgtgtgagtccagctaaaaagacaaaacagaacccgtg ggcccagctcggaaggtgcgtggagaaggctccgacgtctccgaagtgcagcccttgggatggcattccgttgtgtgccttattcct ggagaatctgtatacggctcgcctatagaa |
| **301** | 212464 _s_at | cttctccaagcatcaccctgggagtttcctgagggttttctcataaatgagggctgcacattgcctgttctgcttcgaagtattcaatanc cgctcagtattttaaatgaagtgattctaagantttggtttgggatcaatanggaaagncatatgnncagnnccaanccaagatgc aaatgttttgaaatgatatgaccaaaatttaagtaggaaagtcancccaaacacttctgctttcacttaagtgtctggcccgcaatac tgtaggaacaagcatgatcttgttactgtgatattttaaatatccacagtactcactttttccaaatgatcctagtaattgcct |
| **302** | 212592 _at | gtatcaaaatcttccaattatcatgctcacctgaaagaggtatgctctcttaggaatacagtttctagcattaaacaaataaacaagg ggagaaaataaaactcaaggagtgaaaatcaggaggtgtaataaaatgttcctcgcattccccccgcnttttttttttttttgactttg ccttggagagccagagcttccgcatttctttactattctttttaaaaaaagtttcactgtgtagagaacatatatgcataaacataggtc aattatatgtctccattagaaaaataataattggaaaacatgttctagaactagttacaaaaataatttaaggtgaaatctctaatattta taaaagtagcaaaataaatgcataattaaaatatatttggacataacagactt |
| **303** | 212667 _at | caggaggacacagcattattctagtgctgtactgttccgtacggcagccactacccacatgtaactttttaagatttaaatttaaattagt taacattcaaaacgcagctccccaatcacactagcaacatttcaagtgcttgagagccatgcatgattagtggttaccctattgaata ggtcagaagtagaatcttttcatcatcacagaaagttctattggacagtgctcttctagatcatcataagactacagagcacttttcaa agctcatgcatgttcatcatgttagtgtcgtattttgagctggggttttgagactnccccttagagatagagaaacagacccaagaaatg tgctcaattgcaatgggccacatacctagatctccagatgtcatttcccctctcttatttaagttatg |
| **304** | 212671 _s_at | accaatgaggttcctgaggtcacagtgttttccaagtctcccgtgacactgggtcagcccaacaccctcatctgtcttgtggacaaca tctttcctcctgtggtcaacatcacntggctgagcaatgggcactcagtcacagaaggtgtttctgagaccagcttcctctccaagagt gatcattccttcttcaagatcagttacctcaccttcctcccttctgntgatgagatttatgactgcaaggtggagcactggggcctggat gagcctcttctgaaacactgggagcctg |
| **305** | 212713 _at | cacatggttctagcctggacctggctgggctccatgagaatgagttgcctccaccctgtcccaacagctgacagccaggagccact ctcccagctgcaggcctttgtggtccatcttgtcctgcttcctcactgtggacccctgtctgggccaccctagtgtgctaagctgagcag tgcagtgtgaacagggcccatggtgtattctaggccacagcccagcactcctctgggctgctctcaaaccatgtcccatcttcagcat ccctcccaccaacttactcccctgtggtgagtaccgtggaaccccagcccacctcactatcatactcagcttcccctgatggcccatc ccagcccctgaagctntatgccaagaacacagctaccgcacaccaccctgaaacagccacagccaaggtaggcatgcatatg aggtcttccccataccctctgggtgttgagaggtttagccacatgagggagcagaggacaatctctgcagggctgggagtgggta gggactgaaggtctcaat |
| **306** | 212730 _at | tcagtgtctgggctcatgacttgtaaatggaagctgatgtgaacaggtaattaatattatgacccacttctatttactttgggaaatatctt ggatcttaattatcatctgcaagtttcaagaagtattctgccaaaagtatttacaagtatggactcatgagctattgttggttgctaaatgt gaatcacgcgggagtgagtgtgcccttcacactgtgacattgtgacattgtgacaagctccatgtcctttaaaatcagtcactctgca cacaagagaaatcaacttcgtggttggatggggccggaacacaaccagtctttttgtatttattgttactgagacaaaacagtactca ctgagtgtttttcagtttcctactggtggttttgatattgtttgtttaagatgtatatttagaatgacatcatctaagaagctgattttgctaaact cctgttccc |
| **307** | 212741 _at | gaactcatgtgatttacccttttcaactttttggaaaacgatttaatttattctaattagattaaccctattaatctatggattgggtatcaaaa tgaatgccagtccagatgtgcctagacacgaaattggagctgaggactctcacgatatgcaagttcatccaacgtgaagataccat aagctttttctctgaaccagagaaatgaaagtcagtttaagaggctgatagatcttggccctgttaaggcatccacttcacagttctga aggctgagtcagccccactccacagttaggccaagaattagattttaaaacttcatctgtctgtcccagttaactgttaaataaggcct catcctccactgaagagtatggattgaaggattgtgaactatgtttagtgtgattgtgaacttggtgcctaatgttccatgtctgaagtttg ccccagtgctacacgttggagta |
| **308** | 212764 _at | gatgcaattggttctcctgcattgagatttgatttaacagtgttatgttaacatttatacttgccttggactgtagaacagaacttaaatgg gaatgtattagttttacaactacaatcaagtcattttacctttacccagtttttaatataaaacttaaattttgaaattcactgtgtgactaata gcatgatgctctgcagttttattaagaaatnagcctaaccatacaactctcatttccttagtaagccaaattaggattaacttctataaac agtgttgggaacaatgtttaacatttgtgccaatttgttcctgtattcatgtatgtaagttacagatctgactcttcatttttaagttccttgtta catcatggtcatt |
| **309** | 212814 _at | cctatcctacttgcccatatgagcacggctccccatggccacatactcctgcaaagcttttatgctgcttcgcttttctctaaacagatct gatattgctgctcctgtggttttctcaaaattaactttgccgtggtttttaaaaaggaatcaaaatgcattgttgcattaagctttttcaataa aggaaaattacggaaggaaaataggcaacaccagcaaattatatgtggacaggttctaaactctatatatacatatatatatatata tctatatatctatatacgtaatcatctagttctgtcatcttactgaaaggaataacacttctaaagatcaccatttctgagaagttcttgga aatctttatgtctaa |
| **310** | 212859 _x_at | caccgcgcagagctcagggggtggtgcgcccggcccttctgcggcgcacagcccagcccaggaacgcgggcggtgcggact cagcgggccgggtgcaggcgcggagctgggcctctgcgcccggcccganctccgtctataaanagagcagccagttgcaggg ctcnantctgctttccaactgcctgactgcttgttcgtctcactggtgtgagctccagcatcccctttgctcgaaatggaccccaactgct cttgcgccactggntggctcctgcacgtgcgccggctcctgcaagtgcaaagagtgcaaatgcacctcctgcaagaagagctgct gttcctgctnccccgtggnctgtgccaagtgtgcccagggctgcgtctgcaaaggggcatcggagaagtgcagctgctgtgcctga tgtgggaacagctcttctcccagatgtaaatagaacaacctgcacaacctggnattttttaaaaatacaacactgagccatttgctg catttc |
| **311** | 212956 _at | tactgatgcttctgatactggatgtttagcttcttactgcaaaaacataagtaaaacagtcaactttaccatttccgtattctccatagattg aagaaatttataccacatatcgcatatgaccatctttccatcaaatcaatgtagagataatgtaaactgaaaaaaatctgcaagat aatgtaactgaatgttttaaaaacagaacttgtcactttatataaaagaatagtatgctctatttcctgaatggatgtggaaatgaaagc tagcgcacctgcactttgaattcttgcttctttttttattactgttatgattttgctttttacagatgttggacgatttttttcttctgattgttgaattcat aatcatggtctcatttcctttgcttctttggaatatttctttcaacacattcctttatttattatacattgtgtcctttttttagctattgctgct |
| **312** | 213068 _at | gacaggagggtgtccacatatgttaacatcagttggatctcctatagaagtttctgctgctctctttccttctccctgagctggtaactgc aatgccaacttcctgggcctttctgactagtatcacacttctaataaaatccacaa |
| **313** | 213071 _at | gctccacatgagccatgcatgcttagcaatccaagtgcagagctctttgctccaggagtgaggagactgggaggtgaaatgggg aaatggaagggtttggaggcagagctgaaaacagggttggaaggatttcctgaattagaagacaaacgttagcatacccagtaa ggaaaannngnnnaggggccanggggaacccgtgaggatcactctcaaatgagattaaaaacaaggaagcagagaatggt cagagaatgggattcagattgggaacttgtggggatgagagtgaccaggttgaactgggaagtggaaaaaggagtttgagtcac tggcacctagaagcctgcccacgattcctaggaaggctggcagacaccctggaaccctggggagctactggcaaactctcctgg attggncctnatnnttttggtgggaaaggctgccctggggatcaactttccttctgtgtgtggctcaggagttcttctgcagagatggcg ctatctttcctcctcctgtgatgtcctgctcccaaccattgtactcttcatta |
| **314** | 213317 _at | aagtgtgcataatttcatttaacgttaaagaaatagatccaattcctttcttgcaaccaaaaataaataaaatacgttgcctcaatataa ggtttgggctattctgtgtttctatagaagcaatctgtttttggtaaaatgtactttaaggatccagtcatctgaagtattttatgtagagtta gagatttcacaatattgactatacatatatttaaaatataaattatccagctgatgtttgaatttgtcttactttcctggccacctcgttgtcct attttataagctggggagttaactagcttaacaaaagatgcttagcttttgtaaaagaacaagtgtttcatttacaaagacactccaa atgatagttacttgatttctcgagacctttaactatggtgatgaataacaggacttgctttcaagccttaataaatgtaaaatgccttttaa tgaagatacagctgagtgttttcctcatgaatctgaaccaattaccaatttgtgttccagtcttgatt |
| **315** | 213428 _s_at | gaaaccaaggtcaggaggccgttgcagacataaatctcggcgactcggccccgtctcctgagggtcctgctggtgaccggcctg gaccttggccctacagccctggaggccgctgctgaccagcactgaccccgacctcagagagtactcgcaggggcgctggctgc actcaagaccctcgagattaacggtgctaaccccgtctgctcctccctcccgcagagactggggcctggactggacatgagagcc ccttggtgccacagagggctgtgtcttactagaaacaacgcaaacctctccttcctcagaatagtgatgtgttcgacgttttatcaaag gccccctttctatgttcatgttagttttgctccttctgtgtttttttctgaaccatatccatgttgctgacttttc |
| **316** | 213451 _x_at | cacaggcaggtgactactccatgcgcgtggacctgcggctggggacgaggctgtgttcgcccagtacgactccttccacgtaga ctcggctgcggagtactaccgcctccacttggagggctaccacggcaccgcaggggactccatgagctaccacagcggcagtgt cttctctgcccgtgatcgggaccccaacagcttgctcatctcctgcgctgtctcctaccgagggggcctggtggtacaggaactgcca ctacgccaacctcaacgggctctacgggagcacagtggaccatcagggagtgagctggtaccactggaagggcttcgagttctc ggtgcccttcacggaaatgaagctgagaccaagaaactttcgctccccagcgggggaggctgagctgctgcccacctctctcg caccccagtatgactgccgagcactgaggggtcgccccgagagaagagccagggtccttcaccacccagccgctggaggaa gccttctctgccagcgatctcgcagcactgtgtttacag |
| **317** | 213509 _x_at | ccaatggcgagggtctgccacactggccgctgttcgaccaggaggagcaatacctgcagctgaacctacagcctgcggtgggc cgggctctgaaggcccacaggctccagttctggaagaaggcgctgccccaaaagatccaggagctcgaggagcctgaagag agacacacagagctgtagctccctgtgccggggaggagggggtgggttcgctgacaggcgagggtcagcctgctgtgcccaca cacacccactaaggagaaagaagttgattccttcattcacttcgccattcattcatacttccgtccatccattcagaaagcatttattaa gaatttactcaggcatgatggcccatacttgtaatcccagctattgggaaggatgagatggaggatggcttgaggccagaggtttg agaccgaccagcc |
| **318** | 213624 _at | gggagagtccatctggaagctggagtatatcctgacccagacctacgacattgaagatttgcagccggaaagtttatatggattag ctaaacaatttacaatcctagacagtaagcagtttataaaatactacaattacttctttgtgagttatgacagcagtgtaacatgtgata agacatgtaaggcctttcagatttgtgcaattatgaatcttgataatatttcctatgcagattgcctcaaacagctttatataaagcacaa ttactagtatttcacagtttttgctaatagaaaatgctgattctgattctgagatcaatttgtgggaattttacataaatctttgttaattactga gtgggcaagtagacttcctgtctttgctttcttttttttttcttttgatgccttaatgtagatatctttatcattctgaattgtattatatatttaaant gctcattaatagaatgatggatgtaaattggatgtaaatattcagtttatataattatatctaatttgtacccttgttgaaattgtcattta |
| **319** | 213629 _x_at | cttcgcttctctcttggaaagtccagtctctcctcggcttgcaatggaccccaactgctcctgcgccgctggtgtctcctgcacctgcgct ggttcctgcaagtgcaaagagtgcaaatgcacctcctgcaagaagagctgctgctcctngctnccccgtgggctgtagcaagtgt gcccagggctgtgtttgcaaaggggcgtcagagaagtgcagctgctgcgactgatgccaggacaacctttctcccagatgtaaac agagagacatgtacaaacctggattttttttttataccaccttgacccatttgctacattccttttcctgtgaaa |
| **320** | 213746 _s_at | cctggggctgagcaaggcctacgtaggccagaagagcagcttcacagtagactgcagcaaagcaggcaacaacatgctgctg gtnggggttcatggcccnangaccccctgcgaggagnnnnngnngnagcacgtgggcagccggctntacagcgtgtcctacc tgctcaaggacaagggggagtacacactggtggtcaaatggggg |
| **321** | 213891 _s_at | gaaactgtatgggtagctttttgttgtttttgttttgttttgttttgttttgttttagttgtaggtcgcagcggggaaatttttgcgactgtac acatagctgcagcattaaaaacttaaaaaaattgttaaaaaaanaaaaaaagggaaaacatttcaaaaaaaaaaaaanngat aaacagttacaccttgttttcaatgtgtggctgagtgcctcgatttttcatgtttttggtgtattctgatttgtagaagtgtccaaacaggttg tgtgctggagttccttcaagacaaaaacaaacccagcttggtcaaggccattacctgtttcccatctgtagttattcg |
| **322** | 213921 _at | gctgctgccgcggggaagcaggaactggccaagtacttcttggcagagctgctgtctgaacccaaccagacggagaatgatgc cctggaacctgaagatctgtcccaggctgctgagcaggatgaaatgaggcttgagctgcagagatctgctaactcaaacccggct atggcaccccgagaacgcaaagctggctgcaagaatttcttctggaagactttcacatcctgttagctttcttaactagtattgtccata tcagacctctgatccctcgcccccacaccccatctctcttccctaatcctccaagtcttcagcgagacccttgcattagaaactga |
| **323** | 213953 _at | acaggaaattgctacttaccgccgccttctggaaggagaagacgtaaaactacagaatatcagttaagcaccctggaagaga gagatataaagaaaaccagnaagattaagacagtcgtgcaagaagtagtggatggcaaggtcgtgtcatctgaagtcaaagag gtggaagaaaatatctaaatagctaccagaaggagatgctgctgaggttttgaaagaaatttggctataatcttatctttgctccctgc aagaaatcagccataagaaagcactattaatactctgcagtgattagaaggggtggggtggcgggaatcctatttatcagactctgt aattgaatataaatgttttactcagaggagctgcaaattgcctgcaaaaatgaaatccagtgagcactagaatatttaaaacatcatt |
| | | actgccatctttatcatgaagcacatcaattacaagctgtagaccacctaatatca |
| **324** | 214027 _x_at | gagaaagccaggcaggtagccaggggactagcccctgtggagactgggggcttgaaattgtccccgtggtctcttactttccttt ccccagcccagggtggacttagaaagcaggggctacaagagggaatccccgaaggtgctggaggtgggagcaggagattga gaaggagagaaagtgggtgagatgctggagaagagaggagaggagagaggcagagagcggtctcaggctggtgggaggg gcgcccacctccccacgccctcccctcccctgctgcaggggctctggagaga |
| **325** | 214038 _at | atgagccttcatacatggactgagagtcagagcttgaagaaaagcttatttattttccccaacctcccccaggtgcagtgtgacattat tttattataacatccacaaagagattatttttaaataatttaaagcataatatttcttaaaaagtatttaattatatttaagttgttgatgttttaa ctctatctgtcatacatcctagtgaatgtaaaatgcaaaatcctggtgatgtgtttttttgtttttgttttcctgtgagctcaactaagttcacgg caaaatgtcattgttctccctcctacctgtctgtagtgttgtggggtcctcccatggatcatcaaggtgaaacactttggtattctttggca atcagtgctcctgtaagtcaaatgtgtgctttgtactgctgttgtt |
| **326** | 214091 _s_at | atgcctacaggtatgcgtgattgtgtgtgtgtgcatgggtgnacagccacntgtctacctatgtgtctttctgggaatgtgtaccatctgt gtgcctgcagctgtgtagtgctggacagtgacaaccctttctctccagttctccactccaatgataatagttcacttacacctaaaccc aaaggaaaaaccagctntaggtccaattgttntgctctanactgatacctcaaccttggggccagcatctcccactgcctccaaata ttagtaantatgactgacgtncccanaagtttntgggtnnnaccacactccccaaccccccactcctanttcctgaagggccctccc aaggntacatccccaccccacagttctccctgagagagatcaacctccctgagatcaaccaaggcagatgtgacagcaagggc cacggacc |
| **327** | 214142 _at | gcctctggcaatgccattcaggccaggtcttcctcctatngtgnagannatnnnnntggnggtggaaagcgattctctcattctggc aaccagttggnacggccccatcaccgccctccgggtccgagtcaacacatactacatcgtaggtcttcaggtgcgctatggcaag gtgtggagcgactatgtgggtggtcgcaacggagacctggaggagatctttctgcaccctggggaatcagtgatccaggtttctgg gaagtacaagtggtacctgaagaagctggtatttgtgacagacaagggccgctatctgtcttttgggaaagacagtggcacaagttt caatgccgtccccttgcaccccaacaccgtgctccgcttcatcagtggccggtctggttctctcatcgatgccattggcctgcactggg atgtttaccccactagctgcagcagatgctgagcctcctctccttggcaggggcactgtgatgaggagtaagaactcccttatcacta ac |
| **328** | 214164 _x_at | acaaggcccaggctggggccagggccagaggggaaggccctggattctcactcatgtgagatcttgaatctctttctttgttctgtttg tttagttagtatcatctggtaaaatagttaaaaacaacaaaaaactctgtatctgtttctagcatgtgctgcattgactctattaatcaca tttcaaattcaccctacattcctctcctcttcactagcctctctgaaggtgtcctggccagccctggagaagcactggtgtctgcagcac ccctcagttcctgtgcctcagcccacaggccactgtgataatggtctgtttagcacttctgtat |
| **329** | 214414 _x_at | tcaacttcaagctcctaagccactgcctgctggtgaccctggccgcccacctccccgccgagttcaacccctgnggtgcacgcctc cctgganaagttcctggcttctgtgagcaccgtgctgacctccaaataccgttaagctggagcctcggtagccgttcnnnnnncnn gctnggcnntccaacgggccctcctcccctccttgcaccggcccttcctggtctttgaataaagtctgagtg |
| **330** | 214433 _s_at | cttcctcggaggcagcattgttaagggaggccctgtgcaagtgctggaggacgaggaactaaagtcccagccagagcccctagt ggtcaagggaaaacgggtggctggaggccctcagatgatccagctcagcctggatggaagcgcctctacatcaccacgtcgct gtacagtgcctgggacaagcagttttaccctgatctcatcagggaaggctctgtgatgctgcaggttgatgtagacacagtaaaag gagggctgaagttgaaccccaacttcctggtggacttcgggaaggagccccttggcccagcccttgcccnatgagctccgctacc ctgggggcgattgtagctctgacatctggatttgaaggctccaccctcatcacccacactccctattttgggccctcacttccttgggga cctggcttcattctgctctctcttggcacccgacccttggcagcatgtaccacacagccaagctgagactgtggcaatgtgttgagtc atatacatttactgaccactgttgct |
| **331** | 214505 _s_at | ccatcggtgcggactccaaggaggtgcactataagaaccgcttctggcatgacacctgcttccgctgtgccaagtgccttcacccct tggccaatgagacctttgtggccaaggacaacaagatcctgtgcaacaagtgcaccactcgggaggactcccccaagtgcaag gggtgcttcaaggccattgtggcaggagatcaaaacgtggagtacaaggggaccgtctggcacaaagactgcttcacctgtagt aactgcaagcaagtcatcggactggaagcttcttccctaaaggggaggacttctactgcgtgacttgccatgagaccaagtttgc caagcattgcgtgaagtgcaacaagggtttggtaaaggctccagtgtggtggcctatgaaggacaatcctggcacgactactgctt ccactgcaaaaaatgctccgtgaatctggccaacaagcgctttgttttccaccaggagcaagtgtattgtcccgactgtgc |
| **332** | 214598 _at | ttgtcaaggggctttgcattcaaactgcttttccagggctatactcagaagaaagataaaagtgtgatctaagaaaaagtgatggtttt aggaaagtgaaaatatttttgtttttgtatttgaagaagaatgatgcatttgacaagaaatcatatatgtatggatatattttaataagtat ttgagtacagactttgaggtttcatcaatataaataaaagagcagaaaaatatgtcttggttttcatttgcttaccaaaaaaacaacaa caaaaaaagttgtcctttgagaacttcacctgctcctatgtgggtacctgagtcaaaattgtcattttttgttctgtgaaaaataaatttcctt cttgtaccatttctgtttagttttactaaaatctgtaaatactgtatttttctgtttattccaaattgatgaaactgacaatccaatttgaaagttt gtgtcgacgtctgtctagcttaaatgaatg |
| **333** | 214677 _x_at | tcataagtgacttctacccgggagccgtgacagtggcctggaaggcagatagcagccccgtcaaggcgggagtggagaccac cacaccctccaaacaaagcaacaacaagtacgcggccagcagctatctgagcctgacgcctgagcagtggaagtcccacag aagctacagctgccaggtcacgcatgaagggagcaccgtggagaagacagtggcccctacagaatgttcataggttctcaacc ctcac |
| **334** | 214696 _at | gagtatacatcggtgcaggcttcctggatgacagttgggtgatatgtgtcatgtggcctaaaagcctccatgtcatttgacctacgaat tctatctttgggaatttatcctaagaaantacttanggatttanttngtgataagatgttcatcccagcattgcaatggagaaaaatggg aagcaatggtttggttgggaatttattcctttctgctgtaacgaaagtttgcaataggggattgcttaagtaaattattgtatctccatcca gatggtggagtaccgcgcagacattaaaagtcatgtaaaagaacatctgactgaaagaaaaatgctccttgaatattaaaggtt |
| | | gtaaaaatagtgcatgttatgtgatttcaattttgtttttaaaatatgggtgtatgcttgtatacgtagagcagataaaaaagacggaag gcatactaaaaaatgttgagtggttatctttgtatggtggaacaaagtcactgtaattttcatctttggtt |
| **335** | 214752 _x_at | tggggctgagcaaggcctacgtaggccagaagagcagcttcacagtagactgcagcaaagcaggcaacaacatgctgctggt gggggttcatggcccaaggacccccctgcgaggagatcctggtgaagcacgtgggcagccggctctacagcgtgtcctacctgct caaggacaagggggagtacacactggtggtcaaatggggggacgagcacatcccaggcagcccctaccgcgttgtggtgccc tgagtntggggcccgtgccagccggcagcccccaagcctgccccgctacccaagcagccccgccctnttcccctcaaccccgg cccaggccgccctggccgcccgcctgtcactgcagccgcccctgccctgtgccgtgntgcgctcacctgcctccccagccagccg ctgacctctcggctttcacttgggcagagggagccatttggtggcgctgcttgtcttctttggttctgggaggggt |
| **336** | 214768 _x_at | aatgctctgggtctctggatccagtggggnatattgtgatgactcagtctccactctccctgcccgtcacccctggagagccggcctc catctcctgcaggtctagtcagagcctcntgcatnntaatggatacaactatttggattggtacctgcagaagccagggcagtctcc acagctcctgatctatttgggttctaatcgggcctccggggtcccngacaggttcagtggcagtggatcaggcacagattttacactg aaaatcagcagagtggaggctgaggatgttggggtttattactgcatgcaagctctacaaactcctcngacnttnggccangggga ccaagntggaanatcaaacgaactgtggctgcaccatct |
| **337** | 214777 _at | gcaagatggtgttgcagacccaggtcttcatttctctgttgctctggatctctggtgcctacggggacatcgtgatgacccagtctccag actccctggctgtgtctctgggcgagagggccaccatcaactgcangtccagccagagtgttttatacagctccaacaataagaac tacttagcttggtaccagcagaaaccaggacagcctcctaagctnctcatttactgggcatctacccgggaatccggggtccctga ccgattcagtggcagcgggtctgggacagattt |
| **338** | 214916 _x_at | gaggtgcagctgttggagtctgggggaggcttggtacagcctggggggtccctgagactctcctgtgcagcctctggattcaccttta gcanctatgccatgagctgggtccgccaggctccaggnaaggggctggagtgggtctcanntattagtggtagtggtnntancac atactacgnagactccgtgaagggccggttcaccatctccagagacaattccaagaacacgctgtntctgcaaatgaacagcctg agagccgaggncacggccgtatattactgtncnnnagaaaatggtgtcagcagttggannntnccaaganannaccccaacct atgttgactactggggccaggaaccctggtcaccgtctcctcagggagtgcatccgccccaacccttttcccccctcgtctcctgtgag aattccccgtcggatacgaagcagcgtggccgttggctgctc |
| **339** | 215049 _x_at | cgtcagtcatcctttattgcagtcgggatccttggggttgttctgttggccattttcgtcgcattattcttcttgactaaaaagcgaagacag agacagcggcttgcagtttcctcaagaggagagaacttagtccaccaaattcaataccgggagatgaattcttgcctgaatgcaga tgatctggacctaatgaattcctcagaaaattcccatgagtcagctgatttcagtgctgctgaactaatttctgtgtctaaatttcttcctatt tctggaatggaaaaggaggccattctgagccacactgaaaaggaaaatgggaatttataacccagtgagttcagcctttaagata ccttgatgaagacctggactattgaatggagcagaaattcacctctctcactgactattacagttgcatttttatggagttcttcttctccta ggattcctaagactgctgctga |
| **340** | 215076 _s_at | ggggaatggagcaaaacagtctttgaatatcgaacacgcaaggctgtgagactacctattgtagatattgcaccctatgacattggt ggtcctgatcaagaatttggtgtggacgttggccct |
| **341** | 215118 _s_at | cttgctctgttgcagattggcagatgccgcctccctatgtggtgctggacttgccgcaggagaccctggaggaggagacccccggc gccaacctgtggcccaccaccatcaccttcctcaccctcttcctgctgagcctgttctatagcacagcactgaccgtgaccagcgtcc ggggcccatctggcaacagggagggcccccagtactgagcgggagccggcaaggcacagggaggaagtgtggaggaacct cttggagaagccagctatgcttgccagaactcagcc |
| **342** | 215125 _s_at | gcataaattaatcagccccagagtgctttaannnnttctcttaaataccggtgtctttgatcaggatgacatgtgccatttttcagagga cgtgcagacaggctggcattctagattactttcttactctgaaacatggcctgtttgggagtgcgggattcaaaggtggtcccaccgc tgcccctactgcaaatggcagttntaatcttatcttttggcttctgcagatggttgcaatngatccttaaccaataatggtcagtcctcatc tctgt |
| **343** | 215176 _x_at | ctactctggctccgaggtgccagatgtgacatccagatgacccagtctccatcctccctgtctgcatctgtaggagacagagtcacc atcacttgccgggcaagtcagagcattagcagctatttaaattggtatcagcagaaaccaggnaaagcccctangctcctgatcta tgctgcatccantttgcaaagtggggtcccatcaaggttcagtggcagtggatctgggacagatttcactctcaccatcagcagtctg caacctgaagattttgcaacttactactgtcaacagagttacagtacccctca |
| **344** | 215193 _x_at | gccgagtactggaacagccagaaggacntcctggaagacnagcgggccnnggtggacacctactgcagacacaactacgg ggttgnngagagcttcacngtgcagcggcgagtccatcctnaggtgactgtgtatcctncaaagacccagcccctgcagcacca caanctcctggtctgntctgtgagtggtttctatccaggcagcattgnaagtcaggtggttncggaanggcnaggaagagaagnct ggggntggtgtccacnggcctgatccagaatggagactggaccttccagaccctggtgatgctngaaacagttcctcggagtgga gaggtttacacctgccaagtggagcacccaagcntnacgagccctctcacagtggaatggagngcacggtctgaatctgcaca gagcaagatgctgagtggagtcgggggctt |
| **345** | 215299 _x_at | aagatcctggagtttgtggggcgctccctnccagaggagacngtggacntcatggttnagcacacgtcgttcaaggagatgaag aagaaccctatgaccaactacaccaccgtccnccnggagttcatggaccacagcatctccccccttcatgaggaaaggcatggct ggggacnngngnngnccacnttcaccgtggcgcagaatgagcgcttcgatgcggacntatgcggagaagatggcaggncng cagcctcangcttccgctntgagcngtgagaggggnnncntggagtcacngcagagggagtgtgcgaatcaaacctgaccaa gcggntcaagaataaaatatgaattgagggccngggacggtaggtcatgtctgtaatcccagcaatttggaggctgaggtggga ggatcatttgagcccaggagttcgagaccaacctgggcaacatagtgagattctgttaaaaaaataaaataaaataaaaccaattt ttaaaaagagaataaaatatgattgtgggccaggcagagtggctcatgc |
| **346** | 215382 _x_at | ccggtcagcaggatcatcgtgcacccacagttctacatcatccagactggagcggatatcgccctgctggagctggaggagccc gtgaacatctccagccgcgtccacacggtcatgctgccccctgcctcggagaccttcccccgggnntgccgtgctgggtcactgg ctggggcgatgtggacaatgatgagcccctcccaccgccatttcccctgaagcaggtgaaggtccccataatggaaaaccacatt tgtgacgcaaaataccaccttggcgcctacacgggagacgacgtccgcatcatccgtgacgacatgctgtgtgccgggaacacc cggagngnntcatgccagggcgactcnggagggcccctggtgtgcaaggtgaatggcacctggctncaggcgggcgtggtca gctgggncgagggctgtgcccagcccaaccggcctggcatctacacccgtgtcacctactacttggactggatcc |
| **347** | 215388 _s_at | gaaccacctcaatgcaaagattctacggaaaatgtgggccccctccacctattgacaatggggacattacttcattcccgttgtca gtatatgctccagcttcatcagttgagtaccaatgccagaacttgtatcaacttgagggtaacaagcgaataacatgtagaaatgga caatggtcagaaccaccaaaatgcttacatccgtgtgtaatatcccgagaaattatggaaaattataacatagcattaaggtggac agccaaacagaagctttatttgagaacaggtgaatcagctgaatttgtgtgtaaacggggatatcngtctttcatcacgttctcacac attgcgaacaacatgttgggatgggnnnctggagtatccaacttgtgcannnngatagaatcaatcataaaatgcacacctttattc agaactttagtattaaatcagttcttaatttcatttttaagtattgttttactcctttttattcatacgtaaaattttggatta |
| **348** | 215657 _at | tcttccaatttattatctgcaccaattattttgggcacttctcctgtgtatgtaaactttggagtttaggtctacgtcatttatataagagcctgt agacctgagtggatggacactgcctcttagaactagaacttagaactttatcttgaaaatgtacnncnnnngcngaannnccnca cagagtatgtgtcaggtaggaaccaaatttatgattgttatattcaaactggttttctgttccttgaaaatggtntttttctgatcaaagcct atttaggagttattatgttcttgagtttattccatgtgtgtgaatattttgaatgaatccactaataagatatgcatgtatgtctggannnnn nncnnnnnagaataaatatttaagtagactttaacaagtaaatggttgtaggcatcattaagtcttaaatagccttttcaaagatatta gtgttctngtttaattattcagataattcctttaattctccactcaaatctgacttta |
| **349** | 215867 _x_at | gattcctgtgggtccagctttggaactgggaaacctttcttcggatccgcactcattccactgatgccagctgcccctgaaggatgcc agtactgtggtgtgtgagtctcagcagccgcccacacgctcctaactctgctgcatggcagatgcctaggtggaaatagcaaaaa caaggcccgggctggggccagggccagaggggaaggccctggattctcactcatgtgagatcttgaatctctttctttgttctgtttgtt tagttagtatcatctggtaaaatagttaaaaaacaacaaaaaactctgtatctgtttctagcatgtgctgcattgactctattaatcacatt tcaaattcaccctacattcctctcctcttcactagcctctctgaaggtgtcctggccagccctggagaagcactggtgtctgcagcacc cctcagttcctgtgcctcagcccacaggccactgtgataatggtctgtttagcacttctgtat |
| **350** | 216207 _x_at | ctctggctcccaggtgccagatgtgccatccagttgacccagtctccatcctccctgtctgcatctgtaggagacagagtcaccatca cttgccgggcaagtcagggcattagcagtgctttagcctgntatcagcagaaaccagggaaagctcctaagctcctgatctatgat gcctccagtttgnaaantggggtcccatcaaggttcagcggcagtggatctgggacagatttcactctcaccatcagcagcctgca gcctgaagattttgc |
| **351** | 216336 _x_at | ccaactgcctgactgcttgttggcctcaccggtgggagctccagcatctcctttgctcgaaatggaccccaactgctcctgtgccact ggtggctcctgcatgtgcgccggctcctgcaagtgcaaagagtgcaaatgcacctcctgcaagaagagctgctgctcctgctgcc ccgtgggctgtgccaagtgtgcccagggctgcatctgcaaaggggcgttggagaagtgcaactgctatgcctgacgtggggaca gctctgctcccagatgtaaatagagtaacctgcacaaacctggatttttaaaaaatacaacactgagccatttgcttcatt |
| **352** | 216401 _x_at | tcctccctgtctgcatctgtaggagacagagtcaccatcacttgccgggtgagtcagggcattagcagttatttaaattggtatcggca gaaaccagggaaagttcctaagctcctgatctatagtgcatccaatttgcaatctggagtcccatctcggttcagtggcagtggatct gggacagatttcactctcactatcagcagcctgcagcctgaagatgttgcaacttattacggtcaacggacttacaatgccc |
| **353** | 216442 _x_at | tggaggaagttctccagcttcagctcaactcacagcttctccaagcatcaccctgggagtttcctgagggttttctcataaatgagggc tgcacattgcctgttctgcttcgaagtattcaataccgctcagtattttaaatgaagtgattctaagattggtttgggatcaataggaaag catatgcagccaaccaagatgcaaatgttttgaaatgatatgaccaaaattttaagtaggaaagtcacccaaacacttctgctttcac ttaagtgtctggcccgcaatactgtaggaacaagcatgatcttgttactgtgatattttaaatatccacagtactcactttttccaaatgat cctagtaattgcctagaaatatctttctcttacctgttatttatcaatttttcccagtatttttatacggaaaaaattgtattgaaaacacttagt atgcagttgataagaggaatttggtataattatggtgggtgattatttttatactgtatgtgccaaagctttactactgtg |
| **354** | 216474 _x_at | ccgccatttcctctgaagcaggtgaaggtccccataatggaaaccacatttgtgacgcaaaataccaccttggcgcctacacgg gagacgacgtccgcatcgtccgtgacgacatgctgtgtgccgggaacacccggagggactcatgccagggcgactccggagg gcccctggtgtgcaaggtgaatggcacctggctgcaggcgggcgtggtcagctggggcgagggctgtgcccagcccaaccggc ctggcatctacacccgtgtcacctactacttggactggatccaccactatgtccccaaaaagccgtgagtcaggcctgggttggcca cctgggtcactggaggaccaa |
| **355** | 216491 _x_at | agtcgggcccaggactggtgaagccttcggagaccctgtccctcacctgcactgtctctggtggctccatcagtagttactactgga gctggatccggcagcccgccgggaagggactggagtggattgggcgtatctataccagtgggagcaccaactacaacccctcc ctcaagagtcgagtcaccatgtcagtagacacgtccaagaaccagttctccctgaagctgagctctgtgaccgccgcggacacg gccgtgtattactgtgcga |
| **356** | 216510 _x_at | agtgggtctcagctattagtgggagtgcaggtaccacatactacgcagactccgtgaagggccggttcaccacctccagagaca attccaagaacacgctgtatctgcaaatgnacagnctgagagccgaggacacggccgtatattactgtgcgaaatccc |
| **357** | 216576 _x_at | tcctccctgtctgcatctgtaggagacagagtcaccatcacttgccaggcgagtcaggacattagtaatttttaacttggtatcagaa gaagccagggaaagcccctaaagtcctgatctacgatgcatccaatttggaaacaggggtcccatcaaggttcagtggaagtggt tctgggacagattttactttcaccatcgccagcctgcagcctgacgattttgcaacatattactgtcaacaatataatgatcaccccctc actttcggccctgggaccaaagtg |
| **358** | 216834 _at | atgaaactgattacaacaggctgtaagaatcaaagtcaactgacatctatgctacatattattatatagtttgtactgagctattgaagt cccattaacttaaagtatatgttttcaaattgccatgctactattgcttgtcggtgtattttattttattgtttttgactttggaagagatgaact gtgtatttaacttaagctattgctcttaaaaccagggatcagaatatatttgtaagttaaatcattggtgctaataataaatgtggattttgt attaaaatatatagaagcaatttctgtttacatgtccttgctacttttaaaaacttgcatttattcctcagatttt |
| **359** | 216984 _x_at | catctcctgcactggaaccagcagtgacgttggtgcttataactatgtctcctggtaccaacagcacccaggcaaagcccccaaac tcatgatttatgaggtcagtaatcggccctcaggggtttctaatcgcttctctggctccaagtctggcaacacggcctccctgaccatct ctgggctccaggctgacgacgagggtgattactactgcatctcatatacaagtagcaaccctctcgtggtt |
| **360** | 217022 _s_at | tcaagtgggaagagcgctgttcaaggaccacctgagcgtgacctctgtggctgctacagcgtgttccagtgtcctgccgggctgtgc cgagccatggaaccatgggggagaccttcacttgcactgctgcccaccccgagttgaagaccccactaaccgccaacatcacaa aatccggaaacacattccggcccgaggtccacctgctgccgccgccgtcggaggagctggccctgaacgagctggtgacgctg acgtgcctggcacgtggcttcagcccaaggatgtgctggttcgctggctgcaggggtcacaggagctgccccgcgagaagtacct gacttgggcatcccggcaggagcccagccagggcaccaccaccttcgctgtgaccagcatactgcgcgtggcagccgaggact ggaagaagggggacaccttctcctgcatggtgggccacgaggccctgccgctggccttcacacagaagaccatcgaccgcttg gcgggtaaacccacccatgtcaatgtgtctgttgtcatggcgga |
| **361** | 217109 _at | gctgtcaggattcttcaaggagaatgaatactgggaatcaagacaggactataccttatccataggcgcaggtgcacagggga ggccataaagatcaaacatgcatggatgggtcctcacgcagacacacccacagaaggacactagcctggcgcgcgtgcacac acacacacacacacacgagttcataatgtggtgatggccctaagttaagcaaaatgcttctgcacacaaaactctctggtttacttc aaat |
| **362** | 217110 _s_at | gcacctgagcatgaaactcgacgcgttcttcggcatcttctttggggccctgggcggcctcttgctgctgggggtcgggacgttcgtg gtcctgcgcttctggggttgctccggggccaggttctcctatttcctgaactcagctgaggccttgccttgaaggggcagctgtggcct aggctacctcaagactcacctcatccttaccgcacatttaaggcgccattgcttttgggagactggaaaagggaaggtgactgaag gctgtcaggattct |
| **363** | 217148 _x_at | catctcctgcactggaaccagcagtgacgttgntggttataacnatgtctcctggtaccaacannacccaggcaaagcccccaaa ctcatgatttatgnngtcagtaatcggccctcaggggtttctaatcgcttctctg |
| **364** | 217165 _x_at | gcgctggttcctgcaagtgcaaagagtgcaaatgcacctcctgcaagaagagctgctgctcctgctgccccgtgggctgtagcaa gtgtgcccagggctgtgtttgcaaaggg |
| **365** | 217179 _x_at | aggacagagggtcaccatctcttgctctggaagcagctccaacattgggaagaattatgtatcctggtaccagcaactcccagga acagcccccaaactcctcatctataaaaataataagcgaccctcagggattcctgaccgattctctggctccaagtctggcacgtca gccaccctgggcatcaccagactccagactggggacgaggccgattattactgcggaacatgggatagcagcctgagtggtgg ccgggggatat |
| **366** | 217232 _x_at | tgaggagaagtctgccgttactgccctgtggggcaaggtgaacgtggatgaagttggtggtgaggccctgggcaggctgctggtg gtctacccttggacccagaggttctttgagtcctttggggatctgtccactcctgatgctgttatgggcaaccctaaggtgaaggctcat ggcaagaaagtgctcggttctttgagtcctttggggatctgtccactcctgatgctgttatgggcaaccctaaggtgaaggctcatggc aagaaagtgctcggtgcctttagtgatggcctggctcacctggacaacctcaagggcacctttgccacactgagtgagctgcactgt gacaagctgcacgtggatcctgagaacttcaggctcctgggcaacgtgctggtctgtgtgctggcccatcactttggcaaaga |
| **367** | 217235 _x_at | cagtgtccggatctcctggacagtcagtcaccatctcctgcaccggaaccagcagtgatgttggtggttataactatgtctcctggtac caacagcacccaggcaaagcccccacactcatgatttatgatgtcattaagcggccctcaggggtccctgatcgcttctctggctcc aaatctggcaacacggcctccctgaccatctctgggctccaggctgaggatgaggctgattattattgcagctcaaatgcaggcag tttttatgtct |
| **368** | 217258 _x_at | tcctgcactgggagcagctcctacatcggggcaggttatgatgtacactggtaccggcaacttcccggcacagcccccaaactcct catctatggtaacaccgatcggccctcaggggtccctgaccgattctctggctccaagtctggcatctcagcctccctggccatcact gggctccaggctgaggatgaggctgattattactgcaagtcctatgacagcagcctgag |
| **369** | 217378 _x_at | gatgtgacatccaggtgacccagtctccatcttccctgtctgcgtctgtaggagacagagtcaccatcacctgccgggcaagtcag ggcattagcaatgggttatcctggtatcagcagaaaccagggcaagcccctacgctcctgatctatgctgcatccagtttgcagtcg gggtcccatctcggttcagtggcagtggatctgggacagatttcactctcaccatcagcagcctgcagcctgaagatgttgc |
| **370** | 217414 _x_at | ggtccccacagactcagagagaacccaccatggtgctgtctcctgccgacaagaccaacgtcaaggccgcctggggtaaggtc ggcgcgcacgctggcgagtatggtgcggaggccctggagaggcacttcgacctgagccacggctctgcccaagttaagggcca cggcaagaaggtggccgacgcgctgaccaacgccgtggcgcacgtggacgacatgcccaacgcgctgtccgccctgagcga cctgcacgcgcacaagcttcgggtggacccggtcaacttcaagctaagccactgcctgctggtgaccctggccgcccacctcccc gccgagttcacccctgcggtgcacgcttccctggacaagttcctggcttctgtgagcaccgtgctgacctccaaataccgttaagctg gagcctcggtagcc |
| **371** | 217480 _x_at | gctgccaggtgccagatgtgacatccagatgacccagtctccatcctccctgtctgcatctgtaggagacagagtcaccatcacttg ccgggcgagtcagggcattagcaataatttaaattggtatcagcagaaaccagggaaaactcctaagctcctgatctatgctgcac ccagtctgcaaagtgggattccctctcggttcagtgacagtggatctggggcagattacactctcaccatccgcagcctgcagcctg aagattttgc |
| **372** | 217546 _at | agtcgctccatttatcgcttgagatctccagccttaccgcggctcgaaatggaccccaactgctcctgcaccactggtgtctcctgcg cctgcaccggctcctgcaagtgcaaagagtgcaaatgcacctcctgcaagaagagctgctgctcctgctgccccgtgggctgtgc |
| | | caagtgtgcccacggctgtgtctgcaaagggacgttggagaactgcagctgctgtgcctgatgtgggaacagctcttctcccagat gttaatagaacaagctgcacaacctggatttttttcaatacgatactgagccatttgct |
| **373** | 217757 _at | cctccagacctccttgaaatacaatattctcccagaaaggaagagttcccctttgctttaggagtgcagactctgcctcaaacttgtg atgaacccaaagcccacaccagcttccaaatctccctaagtgtcagttacacagggagccgctctgcctccaacatggcgatcgtt gatgtgaagatggtctctggcttcattcccctgaagccaacagtgaaaatgcttgaaagatctaaccatgtgagccggacagaagt cagcagcaaccatgtcttgatttaccttgataaggtgtcaaatcagacactgagcttgttcttcacggttctgcaagatgtcccagtaa gagatctcaaaccagccatagtgaaagtctatgattactacgagacggatgagtttgcaatcgctgagtacaatgctccttgca |
| **374** | 217762 _s_at | ctattaccattctaactttcataaaaagttgggatcaagaagcagctgatttcctgccagggcttatattaggggntgattcttaaagg acattaggattggtgctcagaaatggttaatcatgctgtgtgctagccagggccagctggtaccttctttgccatgagcattcaaggg acngctaacctttattgacaatctatatngcaaaagtcaggaaagaggttgtgagctgattggattaaagacctggcacttcagtaa ctcagcacgcttccacttcactcaacttaagagagttcattgacagtgttaggatgtgaaggctgggaaacacttattttgcttcaaga gttccacttggctctcccaaataggtacctcaaaaactgttagcaagcggcatttggatgtcttgacaggggctttgcagggatttttag ggtttttccacattgtccacattaatggttggcatgattgtgcttgcaggccaag |
| **375** | 217764 _s_at | gaatatgacgttaccttgcagactaaaaggttgaaggcccgaaactaacttttagctaacaataagggctgtgccccaatggaa ctgagttcatttctgagaaaggtttggatgactgaaatatttcctctacagtcaaggactttggcatgcggtggctgaaactgagcttttt tgtgtgggctccagttctcactgttctgcaatgctcatggcaagttgaatggtgagctagcttataaattaaagagctctgaactgtattc agaccgactgggtatctagcttactgttttaacatcattgttgaaaccagaccctgtagtccagtggtgctgccctgttgtgcaaactgc tcctttttctcgtgtttttgtaaagagcttccatctgggctggacccagttcttgcacatacaagacaccgctgcagtcagctaggaccttt ccgccatgtattctattctgtagtaaagcatttccatcaacaatgcctaattgtatctgttattttggtttaacacacactgattcatact |
| **376** | 217767 _at | ggtctacgcctattacaacctggaggaaagctgtacccggttctaccatccggaaaaggaggatggaaagctgaacaagctctg ccgtgatgaactgtgccgctgtgctgaggagaattgcttcatacaaaagtcggatgacaaggtcaccctggaagaacggctggac aaggcctgtgagccaggagtggactatgtgtacaagacccgactggtcaaggttcagctgtccaatgactttgacgagtacatcat ggccattgagcagaccatcaagtcaggctcggatgaggtgcaggttggacagcagcgcacgttcatcagccccatcaagtgca gagaagccctgaagctggaggagaagaaacactacctcatgtggggtctctcctccgatttctggggagagaagcccaacctca gctacatcatcgggaaggacacttgggtggagcactggcctgaggaggacgaatgccaagacgaagagaaccagaaacaat gccaggacctcggcgccttcaccgagagcatggttgtc |
| **377** | 217897 _at | cccctgcatatcttctcagcaataactccatggctctgggaccctacccttccaaccttccctgcttctgagacttcaatctacagcc cagctcatccagatgcagactacagtccctgcaattgggtctctggcaggcaatagttgaaggactcctgttccgttggggccagca caccgggatggatggagggagagcagaggcctttgcttctctgcctacgtccccttagatgggcagcagaggcaactcccgcatc ctttgctctgcctgtcggtggtcagagcggtgagcgaggtgggttggagactcagcaggctccgtgcagcccttgggaacagtgag aggttgaaggtcataacgagagtggaactcaacccagatcccgcccctcctgtcctctgtgttcccgcggaaaccaaccaaacc gtgcgctgtgacccattgctgttctctgtatcgtgatctatcctcaacaaca |
| **378** | 217967 _s_at | ctcttttactactgacctttcacagagaaaaatatttcccttgaaaaaactgggcttgtcatttttttcccttgtagctttaagcagagac ataagtgccttgcattacacatagtaaactttctttaaaaaaaaaaaaaaaagatttggagactaccagggtaagattccaacttgt ccaaaagctttctggccttacatattttattataaaaattctcaagtctggtaatcttctatgtcagagctagtgatttcaaaggtttcaca attccccaagacaaaagtgattttcgttcattataataaggttaagtgatatgtgattcataacaattttgatgtgaagaagggaagga catcattgacttaataatagtatcagtcggtgcaacagttggcaacatgtgccttcacactttaccataaagagacgggtttgagggtt tgccttctaaagtctgcaacttcaagaaaaaaaatcgacactgtggattgactttcccggt |
| **379** | 218087 _s_at | aactttgtatagcccatgtacctaccttgtatagaaaataattttaaaaatttgaatggaaggggtaaaggaagtcatgaagttttttt gcatttttatttaaatgaaggaattccaaataactcacctacagatttttagcacaaaaatagccattgtaaagtgttaaaatttacgat aagtattctattggggaggaaaggtaactctgatctcagttacagtttttttttcctttttaatttcattattttgggtttttggtttttgcagtcctat ttatctgcagtcgtattaagtcctattgctagaataggttactacaaaaaaggttatattctgaaagaaaaataactgacattatatata accaattaatttaaagtattgccatttaaattacacactgagagcatgtcctatgcagacatagatttttctgttcatttatttttcttcattgc agtggattgatttgataaatagatgtgttgaattactacatttgctgtacatat |
| **380** | 218162 _at | ggagctagccttgtttttgcatctttctcactcccatacatttatattatatccccactaaatttcttgttcctcattcttcaaatgtgggccagt tgtggctcaaatcctctatatttttagccaatggcaatcaaattctttcagctcctttgtttcatacggaactccagatcctgagtaatccttt tagagcccgaagagtcaaaaccctcaatgttccctcctgctctcctgccccatgtcaacaaatttcaggctaaggatgccccagac ccagggctctaaccttgtatgcgggcaggcccagggagcaggcagcagtgttcttcccctcagagtgacttggggagggagaaa taggaggagacgtccagctctgtcctctcttcctcactcctcccttcagtgtcctgaggaacaggactttctccacattgtttgta |
| **381** | 218224 _at | gcgggtcttgctggctaaaatgcccaggtaaagggttggttggacacagcgcttagtgcacgctgtcatcatggacatcataatcag ttgtgaaaaacacgcgaacctatgacacttcttattccacactgaatgtgaaattgcatgttcagatgtttnactacgaggcctggctc acaggaagtgttcagtaaaagtatgcactgttagattactgataacgcggatagatttttgttnaccataaattgttccagatttatatta atggaaggaagtgtgcatttattagctattantcaactttacaangcaaacatcttatttctcatctttaaacatgtcgaccagtttaattg aaaagtattctgagactgcaaaatggggtgttaaaaaatactgcagttacggagctgtgtaaaccagtttctcattgcataagatac agatgtaaattgcatggagaggttgatatgcacctgtacagtaattcactcccccatttcacttctttgtcagagaatagttcttgttcata ctgagtgtt |
| **382** | 218312 _s_at | gcatccgaggggagccgccggatgtggaagaagactcggctttcctgcagccatttagtgccgccccatgctaggttatttgacatt gtgcagtgtagagttgccttaaagtgcgtgatctgccagtgctttcttcaagtcaccccttgccccgattcctcatgtttgcgctccccagg gttgctcaagtggaaattttgtcagctgtttagccttttcgtacttggcgtgatgtcaacttcacttctaatctgcaaaagcagaagctgttt cctagtttacctcgcgtgtgtttacctatatggagtagctcgcagagatcacagaaatgcttgcagcctaaggcagggttttcagacc gtgggtcccagcccatttagtaaaatgggaaatcaattagcaagtggtcaccagcattacacagca |
| **383** | 218353 _at | tatacagtttaataagcctcttgcaagttacttgttctctcacctgaggtattttttcctccccaccttgcccctgttcctcccttcctcttctcc ctttgcaagaggaaatatttaacatatttgggtccaacttcaataatgtaataattaatacattaaaagcatttaacttcctttctagaaa aatgcacaggctaaggcatagacaaaacaaagagaaatgctgagaaatttgccactggagacaagcaatctgaataaatattt gccaaaagttctttttatgtcatatagtgtcaggatttgaaggagctattttttttaatgttgcaactagcaactcatcttcggaagacaca gccaggagaatgaagtagaagtgaaaggtttataaatccatttgtaagcatttatcccatatattttaaattcaagaaaaattgtgtttat ctttagaattttgtattcaatactttatgtactatgtgactcatgcttctggataaataaagcaccaaatatgtatctgtaaccacaatcac acata |
| **384** | 218418 _s_at | ggcttgatgtagactggcttgctttgatttttagtgaagggaatgtacgtaaaacaaaatagggcttggctggtcaaaggagacaag caggatggatggatggatggatgaatagatagatggtgtttgcatgtaaattgcagagaaaacaaaaccaaagctgattggaaa caattaattgtgggtgtctgagggggaaggtcgcagctttgggcagctttgagaagcggtacaagagctctgtgcctgtgtgtccag ccctggagccagccagtgcatttattttaagctcttagaagcaactccttggcccaggaatgcgtgacccctgagatgggtccacgc atctctctacacgtccttctctccgtgggatactggactcgtgcctctgcgcccattctcttctcacgcatatccatgagctttaatttcactt tctgatcacggtacgtccataaagccagt |
| **385** | 218468 _s_at | ggagatgacttaagttggcagcagtaatcttcttttaggagcttgtaccacagtcttgcacataagtgcagatttgccccaagtaaag agaatttcctcaacactaacttcacggggataatcaccacgtaactacccttaaagcatatcactagccaaagaggggaatatctg ttcttcttactgtgcctatattaagactagtacaaatgtggtgtgtcttccaactttcattgaaaatgccatatctataccatatttattcgag tcactgatgatgtaatgatatatttttttcattattatagtagaatatttttatggcaagagatttgtggtcttgatcatacctatt |
| **386** | 218469 _at | gaagctgacacaccgtatgttgttagagtcttttatctggtcaggggaaacaaaatcttgacccagctgaacatgtcttcctgagtcag tgcctgaatctttatttttaaattgaatgttccttaaaggttaacatttctaaagcaatattaagaaagactttaaatgttattttggaagact tacgatgcatgtatacaaacgaatagcagataatgatgactagttcacacataaagtccttttaaggagaaaatctaaaatgaaaa gtggataaacagaacatttataagtgatcagttaatgcctaagagtgaaagtagttctattgacattcctcaagatatttaatatcaact gcattatgtattatgtctgcttaaatcatttaaaaacggcaaagaattatatagactatgaggtaccttgctgtgtaggaggatgaaag gggagttgatagtctcataaaactaatttggcttcaagtttcatgaatctgtaactagaatttaattttcaccccaataatgttctatatagc ctttgctaa |
| **387** | 218541 _s_at | gttcaaaatatttccaagcctgagtattgtctattggtatagatttttagaaatcaataattgattatttatttgcacttattacaatgcctgaa aaagtgcaccacatggatgttaagtagaaattcaagaaagtaagatgtcttcagcaactcagtaaaaccttacgccaccttttggttt gtaagaggtttttatacatttcaaacaggttgcacaaaagttaaaataatggggtcttttataaatccaaagtactgtgaaaacatttta catatttttaaatcttctgactaatgctaaaacgtaatctaattaaatttcatacagttactgcagtaagcattaggaagtgaatatgata tacaaaatagtttataaagactctatagtttctataatttattttactggcaaatgtcatgcaacaa |
| **388** | 218546 _at | ggcagcccacaagtttctcgtggggagatggaggcagagcccagggtaggggacagagctgctggggcctttccttgcctggga atctgtcccaggaagagcttccccactcccatcccccaaattggaaaaaccgtacattcaagcctgtttggccctgaaattcttaag aatctggttaagaattaactcactaatgtcaaaagtcaaaacctcctaggggttgtcctgggagtcaggttcacgggtacagaagat gaatctcagatgtcactcaacctgagccgtcattctctgtggcagggctgccctgggtttctcttactcaatccctggagtgtaagcattt ggattgtgtcacagattaccttttaccttttctttcttttttttcttttttcaatatcagtgcccacaccttactgagtattgagtttagagctttc gcttgatgtgcttgaccaagagacttcttttgtatccttttct |
| **389** | 218559 _s_at | tcaagtgcgttctttagaccaatgcattgcgtttctttccctgcttttgagatagtaggaagagttcttggtggtgtcccccccttcaattct tcagttgtatagtagttatagggaagatatgggtgtttttcttattattactttttttttctgcaggtcagtaaaaggatttaagttgcactgac aaaaataccaaaataaaagtgtatttttaagttcccatttgaaattgctggcgctgctggccggatgcatttttgagtttgtattagttgat aaattaacagtaataacaagattgtatgaaccgcatggtgcttgcagttttaaatattgtggatatttgtcctgcatcagaaacgagctt tggtttttacagattcaactgtgttgaaatcaaacctgccgcaacagaaattgtttttatttcatgtaaaataagggatcaatttcaaacc ctgcttatg |
| **390** | 218756 _s_at | cctcctgcctctggattttaggtgttgatttctggatcacgggataccacttcctgtccacaccccgaccaggggctagaaaatttgtttg agatttttatatcatcttgtcaaattgcttcagttgtaaatgtgaaaaatgggctggggaaaggaggtggtgtccctaattgttttacttgtt aacttgttcttgtgcccctgggcacttggcctttgtctgctctcagtgtcttccctttgacatgggaaaggagttgtggccaaaatccccat cttcttgcacctcaacgtctgtggctcaggctggggtggcagagggaggccttcaccttatatctgtgttgttatccagggctccaga cttcctcctctgcctgccccactgcaccctctccccttatctatctccttctcggctccccagcccagtcttggcttcttgtcccctcctgg ggtcatccctccactctgactctgactatggcagcag |
| **391** | 219014 _at | ggcatgtgtgactgtttcagcgactgcggagtctgtctctgtggcacattttgtttcccgtgccttgggtgtcaagttgcagctgatatgaa tgaatgctgtctgtgtggaacaagcgtcgcaatgaggactctctacaggacccgatatggcatccctggatctattgtgatgactata tggcaactctttgctgtcctcattgtactctttgccaaatcaagagagatatcaacagaaggagagccatgcgtactttctaaaaactg atggtgaaaagctcttaccgaagcaacaaaattcagcagacacctcttcagcttgagttcttcaccatcttttgcaactgaaatatgat ggatatgcttaagtacaactgatggcatgaaaaaaatcaaatttttgatttattataaatgaatgttgtccctgaacttagctaaatggtg |
| | | caacttagtttctccttgctttcatattatcg |
| **392** | 219059 _s_at | agagaatgcccttctccttattgtaaccctgtctggatcctatcctcctacctccaaagcttcccacggcctttctagcctggctatgtcct aataatatcccactgggagaaaggagttttgncaaagtgcaaggacctaaaacatctcatcagtatccagtggtaaaaggcctc ctggctgtctgaggctaggtgggttgaaagccaaggagtcactgagaccaaggctttctctactgattccgcagctcagaccctttct tcagctctgaaagagaaacacgtatcccacctgacatgtccttctgagcccggtaagagcaaaagaatggcagaaaagtttagc ccctgaaagccatggagattctcataacttgagacctaatctctgtaaagctaaaataaagaaatagaacaaggctgaggatacg acagtacactgtcagcagggactgtaaacacagacagggtcaaagtgttttctctgaacacattgagttggaatcactgtttagaac acacacacttactttttctggtctctaccactgctgatattttct |
| **393** | 219087 _at | attaccttatcatgtcttagagcccgtctttatgtttaaaactaatttcttaaaataaagccttcagtaaatgttcattaccaacttgataaat gctactcataagagctggtttggggctatagcatatgcttttttttttaattattacctgatttaaaatctctgtaaaaacgtgtagtgtttc ataaaatctgtaactcgcattttaatgatccgctattataagcttttaatagcatgaaaattgttaggctatataacattgccacttcaact ctaaggaatatttttgagatatccctttggaagaccttgcttggaagagcctggacactaacaattctacaccaaattgtctcttcaaat acgtatggactggataactctgagaaacacatctagtataactgaataagcagagcatcaaattaaacagacagaaaccgaaa gctctatataaatgctcagagttctttatgtatt |
| **394** | 219508 _at | atggggctggggacttgaattggatgcttcaaaaccatcacctgttggccaacaagtttgacccaaaggtagatgataatgctcttc agtgcttagaagaatacctacgttataaggccatctatgggactgaactttgagacacactatgagagcgttgctacctgtggggca agagcatgtacaaacatgctcagaacttgctgggacagtgtgggtgggagaccagggctttgcaattcgtggcatcctttaggata agagggctgctattagattgtgggtaagtagatcttttgccttgcaaattgctgcctgggtgaatgctgcttgttctctcacccctaaccct agtagttcctccactaactttctcactaagtgagaatgagaactgctgtgatagggagagtgaaggagggatatgtggtagagcac ttgatttcagttgaatgcctgctggtagcttttccattctgtggagctgccgttcctaataattccag |
| **395** | 219543 _at | ggaattcttctcttacttcaataaaatgggttttaacataactttaaattcagttaaatatacaatattgaatacctatagttgactttgggat ggggactttttcaagtcattaagagtgtttgtttaaggtgatctcattgatggtagttctcagccgtctcaaaaactgcaagctaatcagt cagacattctttaatgaccccaatttttcactttaattgttaccatgttttctatttttactgattttgctaaagcatgtaagagtgaatttattat agcagtaatcttgtgtttctcctgatgtgca |
| **396** | 219607 _s_at | atcaacacatttagcttggcgttttattcattccatcacccttactgtaactactatggcaactcaaataattgtcatgggactatgtccat cttaatgggtctggatggcatggtgctcctcttaagtgtgctggaattctgcattgctgtgtccctctctgcctttggatgtaaagtgctctgt tgtacccctggtggggttgtgttaattctgccatcacattctcacatggcagaaacagcatctcccacaccacttaatgaggtttgagg ccacccaaagatcaacagacaaatgctccagaaatctatgctgactgtgacacaag |
| **397** | 219669 _at | catctctcaggaggtgggctgtccaccaaaatgagcattcagggctgcgtggcccaaccttccagcttcttgttgaaccacaccag acaaatcgggatcttctctgcgcgtgagaagcgtgatgtgcagcctcctgcctctcagcatgagggaggtggggctgagggcctg gagtctctcacttggggggtggggctggcactggccccagcgctgtggtggggagtggtttgcccttcctgctaactctattaccccc acgattcttcaccgctgctgaccacccacactcaacctccctctgacctcataacctaatggccttggacaccagattctttcccattct gtccatgaatcatcttccccacacacaatcattcatatctactcacctaacagcaacactggggagagcctggagcatccggactt gccctatgggagag |
| **398** | 219796 _s_at | tccttgtccacaagcactatggcccccggctcaagtgctgctctggcaaagctccggagccccagccccaaggctttgacaacca ggcgttcctccctgaccacaaggccaactgggcgcccgtccccagccccacgcacgaccccaagcccgcggaggcaccgat gcccgcagagcccgcacccccggccctgcctccccaggcggtgcccctgagcccccgcagcggcccgagctggcggaag ccccacggcggtgaggtccatcctgaccaaggagcggcggccagagggcgggtacaaggctgtctggtttggcgaggacatc gggacggaggcagacgtggtcgttctcaacgcgcccaccctggacgtggatggcgccagtgactccggcagcggcgatgagg gcgagggcgcggggagggtgggggtccctacgatgcgcccggtggtgatgactcctacatctaagtggccc |
| **399** | 219799 _s_at | accatcgctggtggtatcccagggtccctgctcaagttttcttgaaaaggagggctggaatggtacatcacataggcaagtcctgc cctgtatttaggctttgcctgcttggtgtgatgtaagggaaattgaaagacttgcccattcaaatgatctttaccgtggcctgccccatg cttatggtccccagcatttacagtaacttgtgaatgttaagtatcatctcttatctaaatatt |
| **400** | 219948 _x_at | gaatactcataattcttatctctataatcaaaagtataatttactgtagaaaaataaagagatgcttgttctgaaagtaagatcagtgaa ctgcttttcagtctcaatctttgagaattgtaaattcatcaaataattgcttacatagtaaaaatttaaggtattagaaaacctgcataaca aatagtattatatattaaatatttgatatgtaaagctctacacaaagctaaatatagtgtaataatgtttacactaataagcaaatatgtt aatcttctcatttttttactgtcatataatcttagtgatatgcctattaatagttttaaataaataaattggctcatctggcttttgaaattttga aattcttacagatgttgattaggtatatctacaaattaatttcaattttaaaatgatgatataaaaataaatataagtatttttcttgtgtatgt ata |
| **401** | 220026 _at | agtaactttgtttatccctcaagcaaatcctgatgacattgatcctactcctactcctactcctactcctgataaaagtcataattctggag ttaatatttctacgctggtattgtctgtgattgggtctgttgtaattgttaactttattttaagtaccaccatttgaaccttaacgaagaaaaa aatcttcaagtagacctagaagagagttttaaaaaacaaaacaatgtaagtaaaggatatttctgaatcttaaaattcatcccatgtg tgatcataaactcataaaaataattttaagatgtcggaaaaggatactttgattaaataaaaacactcatggatatgtaaaaactgtc aagattaaaatttaatagtttcatttattgttattttatttgtaagaaatagtgatgaacaaagatcctttttcatactgatacctggttgtata ttatttgatgcaacagttttctgaaatgat |
| **402** | 220037 _s_at | ttccacttctattccacggagaaaaaaattgatttgtgtcacagaagtttttatggaaactagcaccatgtctacagaaactgaaccat ttgttgaaaataaagcagcattcaagaatgaagctgctgggtttggaggtgtccccacggctctgctagtgcttgctctcctcttctttgg |
| | | tgctgcagctggtcttggattttgctatgtcaaaaggtatgtgaaggccttcccttttacaaacaagaatcagcagaaggaaatgatc gaaaccaaagtagtaaaggaggagaaggccaatgatagcaaccctaatgaggaatcaaagaaaactgataaaaacccaga agagtccaagagtccaagcaaaactaccgtgcgatgcctggaagctgaagtttagatga |
| **403** | 220075 _s_at | ctcaactcggccatcacatatcgaattaccaaccactcacacttccggatggagggagaggttgtgctgaccaccaccacactgg cacaggcgggagccttctacgcagaggttgaggcc |
| **404** | 220266 _s_at | tccattaccaagagctcatgccacccggttcctgcatgccagaggagcccaagccaaagaggggaagacgatcgtggccccg gaaaaggaccgccacccacacttgtgattacgcgggctgcggcaaaacctacacaaagagttcccatctcaaggcacacctgc gaacccacacaggtgagaaaccttaccactgtgactgggacggctgtggatggaaattcgcccgctcagatgaactgaccagg cactaccgtaaacacacggggcaccgcccgttccagtgccaaaaatgcgaccgagcattttccaggtcggacc |
| **405** | 220376 _at | aaaacccattctttcagttacatctactaagagctcgtagttcaataacattccaaagaacgtagtttggaaaacactggcctatcgg ctgtaataggaagggtctaaagaaaattatttgctgagtcctcataatcaattggctataatcacaatttaataatttatcaatgcaaac agtacattaaaaactgtagcacaatctataatttttaccttcatatgcagagaaattaagaatttacatcttttacaaatatgtgtatctctg aaataatgaaatatattgatgggtgaaataatatttctggtattttcttcattataacacgtggaatgggacagtggctagaggtatgga tggagcaaggtcaaatatgagctgatggagctgggcgctaaataagggtataatatgctacttctgtgtatgttgtgtatggtatagtat gctatttctgtgtatgttcaaattgtttatgtatataataaagttttaaaaatttgtatgtacttgacaattccttagaa |
| **406** | 220468 _at | agaggagacactttggcgttcttcaagcagaactgaggctgcgaaaaatccaagtctctacagagacactgatgaagttgaaag ggtaattgtttttccatgccaaatgaggaaatcaaattaatgagttgacaaacttttcctgagatgttatttcatctacatttagttaaaca acttagaatgatatctagaaaatatttattttcagccaggaactttagcaaactgtggcaatgatcactgggaatgaaaacgtataat gttctacaatttttgttatcacattggatgactttgaatatagtagtgacacttgggaccaaataaattattttatatgactactagaacaa agttttagtaagctgtctgctaatagagtcagaattcttccagattgtttccagctgtaacaggtattgaatttccaccgtgctcctatgta acagctgggtggtgtaaaaaatgaacttcaaattatggagtggaagaagcgtaatgttaatatcttgtaaattcgtattccctat |
| **407** | 220645 _at | cattttccaggatctcagtgtgagtatcattgatgcctgggatataacaattgcatatggcacaaataatgtacacccacctcaacatg tagtcggaaatcagattaatatattattaaactatatttgttaaataacacaaaagtctgaaattcattcacttaagtaaaaaaatttattg actgtctactagcaggccagatgctgtgtttggctctgaattcccaactagcaagagcagagaatctacattatggctgatccataag ccaccaattcagctaaatgagatgtttctaatctgggcttccacttaaaaataaaactaaaaatactcatgaaaagagcctacccttc ttgatcagagccatacccctcttcaccaatgggaagt |
| **408** | 220812 _s_at | ggtattttagtctagttttatatgaacggttgtatcagggtaaccaactcgatttgggatgaatcttagggcaccaaagactaagacag tatctttaagattgctagggaaaagggccctatgtgtcaggcctctgagcccaagccaagcatcgcatcccctgtgatttgcacgtat acatccagatggcctaaagtaactgaagatccacaaaagaagtaaaaatagccttaactgatgacattccaccattgtgatttgttc ctgccccaccctaactgatcaatgtactttgtaatctcccccacccttaagaaggtactttgtaatcttccccacccttaagaaggttcttt gtaattctccccacccttgagaatgtactttgtgagatccaccctgcccacaaaacattgctcttaacttcaccgcctaacccaaaac ctataagaactaatgataatccatcacccttcgc |
| **409** | 220834 _at | gctggccaagactactgggccgtgctttctggaaaaggcatttcagccacgctgatgatcttctccctcttggagttcttcgtagcttgtg ccacagcccatttgccaaccaagcaaacaccacaaccaatatgtctgtcctggttattccaaatatgtatgaaagcaaccctgtga caccagcgtcttcttcagctcctcccagatgcaacaactactcagctaatgcccctaaatagtaaaagaaaaaggggtatcagtct aatctcatggagaaaaactacttgcaaaaacttcttaagaagatgtcttttattgtctacaatgatttctagtctttaaaaactgtgtttga gatttgtttttaggttggtcgctaatgatggctgtatctcccttcactgtctcttcctacattaccactactacatgctggcaaaggtgaagg atcagaggactgaaaaatgattctgcaactctcttaaa |
| **410** | 221004 _s_at | agcccaccagcaggagcttggagtttggggagtggggatgagtccgtcaagcacaactgttctctgagtggaaccaaagaagc aaggagctaggacccccagtcctgccccccaggagcacaagcagggtcccctcagtcaaggcagtgggatgggcggctgag gaacggggcaggcaaggtcactgctcagtcacgtccacgggggacgagccgtgggttctgctgagtaggtggagctcattgcttt ctccaagcttggaactgttttgaaagataacacagagggaaagggagagccacctggtacttgt |
| **411** | 221305 _s_at | gccactatcttgaagaaggtgcacagtgccctgctcctctttcctatgtccccagaattctcttagggttctcagatgccatgactttcaa ggagagagtacggaaccacatcatgcacttggaggaacatttatttgccagtatttttccaaaaatgccctagaaatagcctctga aattctccaaacacctgtcacagcatatgatctctacagccacacatcaatttggttgttgcgaacagactttgttttggactatcccaa acccgtgatgcccaatatgatcttcattggtggtatcaactgccatcagggaaagcc |
| **412** | 221541 _at | ctttatcatccccacaaacattttgaaactggaatatttgtcttcagaaaatggaacaagactataaatgataagccctgtccctagc accacctctcctgtgtgtggaatagaggcccctcgtgctaccaacacttaccctgtgtttaaaaagatcttgtaccaagccaacggc gttcctggctctcctgcccacaggatgaacattttcggcttccttaggagttttgccctaccgtattccaaagcgtgtgctggtttctcatat tgtctgtaggctcac |
| **413** | 221584 _s_at | gtgaggctcagtcagaacctccaccctcccccacaccaaagacaggggcagcgtagtattcaaaccagtattgtggtggggaat aattgtatacatgtaaattatcaagccctatgagtggaagaatttttcaaattattttgtctctctatatattgatttatattatgtataactat ctctttatataaactatatataattatatatatataactatataattatatatatataactatatatataactatatatatgtatcccctagtattg gatcatgaagagctcttcatgcattctttgcaaaggaggttataaagttacgccctcagaacatttataactataagaatgtgccagtt aaagtgctcaacaggaaatatgacagtttaaaagcattgtaaaactcacatagcttacttctctctctaaagtgcaacaaggatgaa tagaatgggccaaggtatgacaattaatggttctgcatgacctagccactgctgggggtttcttctataacgttgtccttgtga |
| **414** | 221667 _s_at | gggacttaacatttcacgttgtatcttacttgcagtgaatgcaagggttacttttctctggggacctcccccatcacccaggttcctactct gggctcccgattcccatggctcccaaaccatgccgcatggtttggttaatgaaacccagtagctaaccccactgtgcttccacatgc ctggcctaaaatgggtgatatacaggtcttatatccccatatggaatttatccatcaaccacataaaaacaaacagtgccttctgccc tctgcccagatgtgtccagcacgttctcaaagtttccacattagcactccctaaggacgctgggagcctgtcagtttatgatctgacct aggtccccccttttcttctgtcccctgtttttaagtccggatttttacagaaggaactgtctccagacagctcatcaaggaaccaagcaa aggccagatagcctgacagataggctagtggtaattgtgtatatgggcgggacgtgtgtgtcatta |
| **415** | 221747 _at | cctctgtcctcaaatgtccaaaatgttggaggacctctgttcatatcccacgcctgggctcttgccagcagtggagttactgtagaggg atgtcccaagcttgttttccaatcagtgttaagctgtttgaaactctcctgtgtctgtgttttgtttgtgcgtgtgtgtgagagcacatcagtgt gtgcaggctgtgtttccccatttctctcctcccttcagacccatcattgagaacaaatgtaagaaatcccttcccaccaccctccctgcc tcccaggccctctgcgggggaaacaagatcacccagcatcct |
| **416** | 221748 _s_at | atattttgtatcatcgtgcctatagccgctgccaccgtgtataaatcctggtgtntgctccttatcctggacatgaatgtattgtacactga cgcgtccccactcctgtacagctgctttgtttctttgcaatgcattgtatggcttta |
| **417** | 221841 _s_at | atccgacttgaatattcctggacttacanaatgccaaggggtgactggaagttgtggatatcagggtataaattatatccgtgagtt gggggagggaagaccagaattcccttgaattgtgtattgatgcaatataagcataaaagatcaccttgtattctctttaccttctaaaa gccattattatgatgttagaagaagaggaagaaattcaggtacagaaaacatgtttaaatagcctaaatgatggtgcttggtgagtct tggttctaaaggtaccaaacaaggaagccaaagttttcaaactgctgcatactttgacaaggaaaatctatatttgtcttccgatcaa catttatgacctaagtcaggtaatatacctggtttacttctttagcatttttatgcagacagtctgttatgcactgtggtttcagatgtgcaat aatttgtacaatggtttattcccaagtatgccttaagcagaacaaatgtgtttttctatatagttccttgccttaa |
| **418** | 221896 _s_at | ccaatcctgaaggtactccctgtttgctgcagaatgtcagatattttggatgttgcataagagtcctatttgccccagttaattcaacttttg tctgcctgttttgtggactggctggctctgttagaactctgtccaaaaagtgcatggaatataacttgtaaagcttcccacaattgacaat atatatgcatgtgtttaaaccaaatccagaaagcttaaacaatagagctgcataatagtatttattaaagaatcacaactgtaaacat gagaataacttaaggattctagtttagtttttgtaattgcaaattatattttgctgctgatatattagaataatttttaaatgtcatcttgaaat agaaatatgtattttaagcactcacgcaaaggtaaatgaacacgttttaaatgtgtgtgttgctaatttttccataagaattgtaaacatt gaactgaacaaattacctataatggatttggttaatgacttatgagcaagctggtttggccagacagtatacccaaacttttatataata tacagaaggctatcacacttgtgaa |
| **419** | 222043 _at | tcgactctgctgctcatggaagaacagaattgctcctgcatgcaactaattcaataaaactgtcttgtgagctgatcgcttggagggt cctcttttttatgttgagttgctgcttcccggcatgccttcatttgctatgggggcaggcagggggggatggaaaataagtagaaacaa aaaagcagtggctaagatggtatagggactgtcataccagtgaagaataaaagggtgaagaataaaaggatatgatgacaa ggttgatccacttcaagaattgcttgctttcaggaagagagatgtgtttcaacaagccaacta |
| **420** | 222162 _s_at | aataacgcaaatggcttcctctttccttttttggaccatctcagtctttattgtgtaattcattttgaggaaaaacaactccatgtatttatt caagtgcattaaagtctacaatggaaaaaaagcagtgaagcattagatgctggtaaaagctagaggagacacaatgagcttagt acctccaatttcctttctttcctaccatgtaaccctgctttgggaatatggatgtaaagaagtaacttgtgtctcatgaaaatcagtacaat cacacaaggaggatgaaacgccggaacaaaaatgaggtgtgtagaacagggtcccacaggtttggggacattgagatcacttg tcttgtggtggggaggctgctgaggggtagcaggtccatctccagcagctggtccaacagtcgtatcctggtgaatgtctgttcagct cttct |
| **421** | 222453 _at | tcaaatggaacctgccctctaaagcactttctttcctttacttgcgtggtttcatgtaagctgtgctgtttagaacaacatctcagacttta caaagaaatgacaaagaaggcaattgcactttttaagggatatcgacaagcagtttctgtttctaaaggacaaaatacagagtgt gtgtcatttttaattagattctttcccctgctgagttggaaattccagtgcagcactgattgaccacagttgccaatctaaaagcacaaa gacagaagtaaagctttatgctaattttatttcaatatgatagaaaatttatcttggtatgtcctttttagataactccagcaggaaactgt aactgctatgtctttaggaaaacgtagaagaaagaacattattattctttaattcctacaaggtacttgaaaaccttaagtgaaaaag atttctatctttttatcttggcgcatt |
| **422** | 222513 _s_at | tgccactaattcattcacactaaggtgtaaatgattgataataggaatgagttacctcttcccacagacatttgtttttaagtatgacaga gcagggccttaatcccaagggaaaaggttatggaactggagggggtgagctttctgggtagaaggagacttcctgaatttccttaa aacccagtaagagtaagacctgttgtttggaaggtctgctccaccatctaagagcactgtttttttttttgttgttgttgttgttttacggtct ctgagggaatatagtaaaaatgcatatgcacgtgcaatttgcacggcagcatttcaccgattgtggactgtattggctaatgtgtttcct ggtctttagatgcaaaccattaataacactatcttatctcatagtttttttcaggggtgcttcttgattagtagggaattttgaacacctcttta aatacagctagaaaataaaaccaatttgtaaagccacatttgcatatgatgccagcctcacgcatttgtatatctccagaaattcagg tatgcctcaccaatttgcccgtctttaataa |
| **423** | 222717 _at | taaactaacccttacattccatgtgatgtgatgtaggcttataagntgctaaaatctataggtntggaagtgaaagattctattttttcttt tttngtacataatgggaatttcattccagattatatttatttacatattaatttcacagaatattaatatttcttaacttcttaaagcatgctag cgttttatgtatatgtacacatatattcagacagggtaattttatctgctgcctaacattgtactaaaatattgctttatctgtttttaattacaa aatgctaatgatttcttaaattatagtttaaagacaattggcccaggaagcaaatcccctgcctttagtatgaaccactataagtaacc ttacaaatagagttaatccaagacaatattaacaaactgtgctttgtctttaataaagggataggattaacaaacatattgatggcat aacctattcagctatgtccttatttttgcaataatgtaacctcaaatattggattgttgaaccaacaatggctgtgttaaa |
| **424** | 222722 _at | tttatgatgactcagtggtgccagagtaaagtttctaaaataacattcctctcacttgtaccccactaaaagtattagnctacacattac attgaagttaaacacaaaattatcagtgttttagaaacatgagtccggactgtgtaagtaaaagtacaaacattatttccaccataaa gtatgtattgaaatcaagttgtctctgtgtacagaatacatacttattcccatttttaagcatttgcttctgttttccctacctagaatgtcaga |
| | | tgtttttcagttatctccccatttgtcaaagttgacctcaagataacatttttcattaaagcatctgagatctaagaacacaattattattcta acaatgattattagctcattcacttattttgataactaatgatcacagctattatactactttctcgttattttgtgtgcatgcctcatttccctga cttaaacctcactgagagcgcaaaatgcagctttatactttttacttt |
| **425** | 223121 _s_at | gataacctacatcaaccgagataccaaaatcatcctggagaccaagagcaagaccatttacaagctgaacggtgtgtccgaaa gggacctgaagaaatcggtgctgtggctcaaagacagcttgcagtgcacctgtgaggagatgaacgacatcaacgcgccctatc tggtcatgggacagaaacagggtggggagctggtgatcacctcggtgaagcggtggcagaagggcagagagagttcaagc gcatctcccgcagcatccgcaagctgcagtgctagtcccggcatcctgatggctccgacaggcctgctccagagcacggctgac catttctgctccgggatctcagctcccgttccccaagcacactcctagctgctccagtctcagcctgggcagcttccccctgcctttgc acgtttgcatccccagcatttcctgagttataaggccacaggagtggatagctgttttcacctaaaggaaaagcccacccgaatctt gtagaaat |
| **426** | 223122 _s_at | ggtgcaactgtgacttgggtctggttggttgttgttgttgttttgagtcagctgatttcacttcccactgaggttgtcataacatgcaaattg cttcaattttctctgtggcccaaacttgtgggtcacaaaccctgttgagataaagctggctgttatctcaacatcttcatcagctccagac tgagactcagtgtctaagtcttacaacaattcatcattttataccttcaatgggaacttaaactgttacatgtatcacattccagctacaat acttccatttattagaagcacattaaccatttctatagcatgatttcttcaagtaaaaggcaaaagatataaattttataattgacttgagt actttaagccttgtttaaaacatttcttacttaactttgcaaattaaacccattgtagcttacctgtaat |
| **427** | 223235 _s_at | agggtacgagaacttgccaatgggaaattcatccgagtggcactggcagagaaggataggagtggaatgcccacacagtgac caacagaactggtctgcgtgcataaccagctgccaccctcaggcctgggccccagagctcagggcacccagtgtcttaaggaac catttggaggacagtctgagagcaggaacttcaagctgtgattctatctcggctcagacttttggttggaaaaagatcttcatggcccc aaatcccctgagacatgccttgtagaatgattttgtgatgttgtgatgcttgtggagcatcgcgtaaggcttcttgcttatttaaactgtgc aaggtaaaaatcaagcctttggagccacag |
| **428** | 223343 _at | gagtccaaatgtcatcagtgctcatttgagataccctgctatcgatggtcgctacaaaccaggaaatactcaagttattatgtgtatac attggntttagntttatgaaacaatttaccttcatgatctcatagttaaaattgtaataaatttaggaatataaaggatcaatatgggaag caaaatttctaaaggcagtttctgttgttttaattagtatttgtgtagttcaaaccaggaaggatttgactatcattagatttgcttaactttat gaaagctaaaatattctctgttataaaggggcaactccatctggtcctatagcatctttactactgatttttttttngtttaatttgaaaatgc aaagaattgttaaatgttcttaaatgttctcactacaaaaaaagaaaaaagataactacgtgaggtgatggatatgttaattagctgg attgtggtaatcattttggaatgtatatgtatatcaaaacatgtagtacaccctaaatatat |
| **429** | 223395 _at | agatacatttttacccaccataaatgttacaatatctgaatatgctttgtcaaactatccctttatgcaatcgtcttcatattgtttttatgattct aatcaagctgtatgtagagactgaatgtgaagtcaagtctgagcacaaaaagataatgcacgatgagattgcctaccattttatag gatatttactatgtatttatacgttaagacctctatgaatgaatgtatcagagaatgtctttgtaactgtttaattcaatctgtaataaaaatc taactaactaactcatttatttctattaaaaggtattgtcctttaggcggggaatgggaatccttgctgcactgttgcagtcattctgaaa ggacctttccctgtacttacctttcaacatgcttcaatcttatcaacgctacat |
| **430** | 223484 _at | gaaaaccgatgtgatccttgatcgaaaaaaaaatccagaaccttgggaaactgtggaccctactgtacctcaaaagcttataaca atcaaccaacaatggaaacccattgaagagttgcaaaatgtccaaagggtgaccaaatgacgagccctcgcctctttcttctgaa gagtactctataaatctagtggaaacatttctgcacaaactagattctggacaccagtgtgcggaaatgcttctgctacatttttagggt ttgtctacatttttgggctctggataaggaattaaaggagtgcagcaataactgcactgtctaaaagtttgtgcttatttcttgtaaattg aatattgcatattgaaatttttgtttatgatctatgaatgtttttcttaaaatttacaaagctttgtaaattagatttctttaataaaatgccattt gtgcaagatttct |
| **431** | 223551 _at | aattgtgttgtgatgctactcactttgattgcaatgatgatgtccaaggtaagctattaaaaggcaggttacttccaaatcgcactgaag gaaaaggttaagaataatacatgatcacagaaatgcataccactgtctgtaaacccaacaaaattcactgttctcttttggattattta gcctgatgtatttttaattcaatttttatggtgatgggcaaatcattcttggtaaatgtaaatcaaacatgattgatttaaaacttcatggaat ttgtagaaaattatggacatttttggtgagaaagaacaatagtcaaaactcacatggatagagtgtgtttgtttttgccaaaaatgccc cagaccttttcccaaacctcaaaaacgtcttggaaaaattgtaaaagtttgataacagaaacatctttaggatatttttgtctgacgtatt ttgcttctagtatgtgcctactgtgattttttcatgtggaaaatgcaaaattgtaacaaaatggttatatggaacatgcctattaaacga a |
| **432** | 223597 _at | ggcatctggcacgtgcccaataagtcccccatgcagcactggagaaacagctccctgctgaggtaccgcacggacactggcttc ctccagacactgggacataatctgtttggcatctaccagaaatatccagtgaaatatggagaaggaaagtgttggactgacaacg gcccggtgatccctgtggtctatgattttggcgacgcccagaaaacagcatcttattactcaccctatggccagcgggaattcactgc gggatttgttcagttcagggtatttaataacgagagagcagccaacgccttgtgtgctggaatgagggtcaccggatgtaacactga gcaccactgcattggtggaggaggatactttccagaggccagtccccagcagtggagatttttctggttttgattggagtggatatg gaactcatgttggttacagcagcagccgtgagataactgaggcagctgtgcttctattcta |
| **433** | 223623 _at | gagaagcacctgttccaactaagactaaagtggccgttgatgagaataaagccaaagaattccttggcagcctgaagcgccaga agcggcagctgtgggaccggactcggcccgaggtgcagcagtggtaccagcagtttctctacatgggctttgacgaagcgaaatt tgaagatgacatcacctattggcttaacagagatcgaaatggacatgaatactatggcgattactaccaacgtcactatgatgaag actctgcaattggtccccggagcccctacggctttaggcatggagccagcgtcaactacgatgactactaaccatgacttgccaca cgctgtacaagaagcaaatagcgattctcttcatgtatctcctaatgccttacactacttggtttctgatttgctctatttcagcagatctttc tacctact |
| **434** | 223754 _at | agctgccgggcaacgtgttgtgtaagtgaacatctgggaggtaaacactacacgtgaagagtggtgaaagggaacattgattact gaagtgccctggagagggaaagcactggtcaacatcacatggacaaatttcattgttttctaaagatggcctggaagtagtctttgc cactgcttcctccacaaacagctcttcataacatgggctgcatgaaatcaaagcaaactttcccatttcctaccatatatgaaggtga gaagcagcatgagagtgaagaaccctttatgccagaagagagatgtctacctaggatggcttctccagttaatgtcaaagaggaa gtgaaggaacctccagggaccaatattgtgatcttggaatatgcacaccgcctgtctcaggatatcttgtgtgatgccttgcagcaat gggcatgcaataacatcaagtaccatgacattccatacattgagagtgaggggccttgaggctgtaggatgaca |
| **435** | 223952 _x_at | ggggctatactccatccaaatatgcagtggaaggtttcaatgacagcttaagacgggacatgaaagcttttggtgtgcacgtctcat gcattgaacgtctagacaaactgaaaggcaataaatcctatgtgaacatggacctctctccggtggtagagtgcatggaccacgct ctaacaagtctcttccctaagactcattatgccgctggaaaagatgccaaaattttctggatacctctgtctcacatgccagcagctttg caagactttttattgttgaaacagaaagcagagctggctaatcccaaggcagtgtgactcagctaaccacaaatgtctcctccagg ctatgaaattggccgatttcaagaacacatctccttttcaacc |
| **436** | 224009 _x_at | ggggctatactccatccaaatatgcagtggaaggtttcaatgacagcttaagacgggacctgaaagcttttggtgtgcacgtctcat gcattgaaccaggattgttcaaaacaaacttggcagatccagtaaaggtaattgaaaaaaactcgccatttgggagcagctgtct ccagacatcaaacaacaatatggagaaggttacattgaaaaaagtctagacaaactgaaaggcaataaatcctatgtgaacatg gacctctctccggtggtagagtgcatggaccacgctctaacaagtctcttccctaagactcattatgccgctggaaaagatgccaaa attttctggatacctctgtctcacatgccagcagctttgcaagactttttattgttgaaacagaaagcagagctggctaatcccaaggc agtgtgactcagctaaccacaaatgtctcctccaggctatgaaattggccgatttcaagaacacatctcctttcaacc |
| **437** | 224342 _x_at | tatctgcggccccaggacagaaggtcgccatctcctgctctggaagcagctccaacattgggaataattatgtatcctggtaccaac aactcccaggaacagcccccaaactcctcatttatgacaataataagcgaccctcagggattcctggccgattctctggctccaagt ctggcacgtcagccaccctgggcatcaccggactccagactggggacgaggccgattattactgcggaacatgggatagcagc ctgag |
| **438** | 224352 _s_at | ttttatttcaggcttccagctgtccctgtgagttatcctggacatttcgatggtttttggtaaggccaaactctgataagcaaaacagaga atactgacggtatacttaaccatatgtgtaactgatacttggcaccatggaatttttcattgagttatttcctcattcttttaaaaaataagg gactataaatcagttatttagtatcttttgttttgtagctgattccttaactttcttgtatgcctctagtaatttcagagattaaatattgctttaa actgtgatactttgatttgctagattgacaaaactgatactaatataattaagttcatctttgaaatacatctttgtgcgtagagccaaaa aaagagataaaattaataatagttcacttgttatttgagattaatttggcatttgaaatgatcatttttatttacaatcatttataatgaatca atgttccagttagctttaaaaggtatacggtgctaattagtaaaatattgaaggcaatatttttactgctagcttgca |
| **439** | 224412 _s_at | taatcctgacattagctgacttgctagtgagcttgctttaaaaatctacactcttgcattcttaggcatacaggggaaatgttgaaaagg aaggtggaaaaccaagaatttagtttgccaatgattgcctctgattcttgtaagtttgagttccacaagggctaatttattccccttttactt gggttttggggtggtggaaagcgggaaatttgggtgatttgttgattggcaatgaggataaaatgttaatactttttggggacttaaca actttatcctattctacaagtcagtaaaggaacaattggtactcacctcagtgctgcactcaactatggaaagaggcagagtttgcttg cccaattgccaaactaaa |
| **440** | 224480 _s_at | attagtgtcattagtgtgttggaagagaaatactattcagtaagcttcgccaaagaaaagtgagtcaagttaatgtgtgtgcattta tatgtaggcagctcgtagaccacatttagccagcaactggtaacaaagagcttagttttccttgtttgaatgctgtagatctgtaccta gtacccctcccatctactgatttgtttgtttttgtaaccaaacacattttcagatagaaggagccttaaaaaaaaaaaaatcacattga gtaacttcagtatgaatgaatgagagtgtgtggagctacccctcaccctccacccctttgtgctttttattcccgaattttcccagtctctta aacagaaaaatgactgatataattatcttttggaaactgagccttaattttttttagagggggaaataagtttccccaactcacacagc ataagcaatgtttgacagcaatataatgccgttgtaaactactgagagtattgtatctgttctggtaaccatgtaca |
| **441** | 224560 _at | ttgtttttgacatcagctgtaatcattcctgtgctgtgtttttattacccttggtaggtattagacttgcacntttttaaaaaaaggtttctgcat cgtggaagcatttgacccagagtggaacgcgtggcctatgcaggtggattccttcaggtctttcctttggttctttgagcatnctttgcttt cattcgtctcccgtctttggttctccagttcaaattattgcaaagtaaaggatctttgagtaggttcggtctgaaaggtgtggcctttatattt gatccacacacgttggtcttttaaccgtgctgagcagaaaacaaaacaggttaagaagagccgggtggcagctgacagaggaa gccgctcaaataccttcacaat |
| **442** | 224663 _s_at | ggtcagatggtcaactttttcagtattatttatagttggcacttgattgcagttctgtgaggcttgagcattcatacacctcacctgccttgg caagcctattttagtgatatggcagcacggatataacactatgcattaaaagcactttttgtaataagtttaatatcctaaaggaatgc caattaagttttgttaactgtgtcatcaacttatcctagtacctcagtgttcattcctgttacctgcatatcttcttaaaagaaatagctgttatt aatgcctttttgttttccattgagtgtacactactgaataagtgtaggagttttatgtttaccatgtgagtcctgcaacactaaagatattttg aatatcagtcatgatggcaatttctgtataaaagagccttaaatg gaacattgttttgagatcaaactccccaccc |
| **443** | 224694 _at | gatatctggccatgggtaacctcattgtaactatcatcagaatgggcagagatgatcttgaagtgtcacatacactaaagtccaaac actatgtcagatgggggtaaaatccattaaagaacaggaaaaaataattataagatgataagcaaatgtttcagcccaatgtcaa cccagttaaaaaaaaaattaatgctgtgtaaaatggttgaattagtttgcaaactatataaagacatatgcagtaaaaagtctgttaat gcacatcctgtgggaatggagtgttctaaccaattgccttttcttgttatctgagctctcctatattatcatactcagataaccaaattaaa agaattagaatatgatttttaatacacttaacattaaactcttctaactttcttctttctgtgataattcagaagatagttatggatcttcaatg cctctgagtcattgttataaaaaatcagttatcactataccatgctat |
| **444** | 224823 _at | gacctaatacatttcctctgtgtgtgtgtgtaacattccaaatactttttttttcttttccactgtttgtaaggtgcaacaatttaatatttttaagg gactttttaagagttccttaagaaccaatttaaaattacttcagtgcaatcctacacagtatcaacattagaattttgatattagtcttatgtt atcttccattctatttttatctgcttttgctgctagtttcaaactgccagtatttttccttttgcttttaaaatagttacaatatttttcatgatagcca |
| | | cagtattgccacagtttattataataaagggtttttatttgatttagcgcattcaaagctttttctatcacttttgtgttcagaatataacctttgt gtgcgtgtatgttgtgtgtgtgcatgtgtggcgtatatgtgtgttacaggttaatgccttcttggaattgtgttaatgttctcttggtttattatgc catcagaatggtaaatgagaacactacaactgtagtcagctcacaatttt |
| **445** | 224836 _at | gagtgagtcccagttacatcaaacagtgacttccagttattccccagtaagtctgagtggttccttcaagctgggtgtctttccagccttt gccagtctagccccagcagggcaccgtgtatgaatgcagtttggtgctgttttagagtatgcctgctccccagccccctgcctggaa ccctctgagcaacttgctctgacctataatgtcttaggtgcaacacggaccccaccagagctcttggatacccccctagatccatgtg gctttatgtgaggggactgaatgcagacacaccatagccccttctactactttccctctcgccctgccacctagttccacatggaac caacaagttgagtgcatccctgttgggtgttttgtgttgagactggctgaaatgaggagactttgaccatgtgacgtgtcaacagactc aaggagacaaccacctcaactgggtcatgtg |
| **446** | 224840 _at | ccccagtctcacctacgaaatatgaagagcaaaagctgattttgcttacttgctaaactgttgggaaagctctgtagagcatggttcc agtgaggccaagattgaaatttgatactaaaaggccacctagctttttgcagataacaaacaagaaagctattccaagactcag atgatgccagctgtctcccacgtgtgtattatggttcaccaggggaactggcaaaagtgtgtgtggggaggggaagggtgtgtga gtggttctgagcaaataactacagggtgcccattaccactcaagaagacacttcacgtattcttgtatcaaattcaataatcttaaaca atttgtgtagaagtccacagacatctttcaaccaccttttaggctgcatatggattgccaagtcag |
| **447** | 224959 _at | tcatgcgtcatccctaaaataataagatacatgggatcaaatagcccttgccttttcaacacaaatcagttggaaaattatggtttgag tcctgttgctgccatggcttctgtttctcagaaatgagtgtgtatgaacataccaatctatgtaataggctaccttttttgtcttctttggaact ttgtacacaaaccaagacaatatcagggtgacaggtgaatgaacttaaattctcagtcttgtctattcaccaaaaaagtatactgcct gtttttctttaattattcaaggttgatgacttttaggaacatgttttatactgtatttttaattaaagcaagtgccttgatgtaattccatgtaaa tcattgcttaaccctcttatgggatgaggatgagttattaatgtattgcagcctactggaaaggagggggagttggttaatagcagata ctttcttctagaagcttatgtttatgctgtttattatgtaagatcctgtatgtgtgttgaga |
| **448** | 224963 _at | ataatgatccattcgagttctgtgatccttattgttcttaattgtgtttctctacgtattgttacagatgagccatacgtttctttgtatcaatgta gacatgacttcagatacctctgaggacctacccagcagtctaggaccctgggccaagtgctgggactatggtactaaatccagta gatgggctgtgtagcaactctcccagggaacacactagggtacttagggaggtgctttgtggagcatgttgaagctttgagatctga gcaggaggcagtgatgtccctggtctattcagggaaagatttcagtgtgaaatggtaaacatccaattgacaggatttagattttgctt agtttttctgctttttaatgtttctatcccccatctcagtgtttctttatccatcccagtgatgcct |
| **449** | 224964 _s_at | ataattttacatgatcctcaatatcaactccagtttaaaagtgttatttttaaaacatttgaaaccaagtactgtttaatttcaatcagaag atgcaaatacatactttgatctatgtttgattttgctaataatatttgaaggagattgcctaccaaggacaaaacaataaatttaaaaat caaacgatttctccatacgctcatagtcacatatggaattttgagaaaataaagcatgctgtctttaggaatttttatacttctttgtctttctt ccttaatatttgcttctagctgctcttggcaatgatgaattgttatgtatgcattaatgttttgcagcccaaaagttgttcacatttttcctatat aagatctgtggagtgtgtgtttcaaagagagaactacagaaatgttaaagcaggaaaacctgaatgtgatgtgcacattttcatccc acatggaca |
| **450** | 224989 _at | catttctgtcacatgcacttagttgacattacatctacatatattagctttttcctacatgagccatctatttacttagtaaccagtgttcttaat gaagtatttagtcttgggtttcttgtaaaatttctctgcattccttagacagtgtactatacatgaaatattcttgttgacctagtaatttatatt attccatttaattcttaaacctatggcctttttattgagcacactcttaaatcattatttggcttgtaaacattcatctgaattgtggctacaatc ctctttaaataatctaggaaaaaagaaagataaagcttacattttcacagttttggctcttaaacacattccacaaatgccattaagaa tttattttgttttaggccagtcatggt |
| **451** | 224990 _at | gagcttctagaaatttcacttgcaagtttatttttgcttcctgtgttactgccattcctatttacagtatatttgagtgaatgattatatttttaaaa agttacatggggctttttggttgtcctaaacttacaaacattccactcattctgtttgtaactgtgattataattttgtgataatttctggcctg attgaaggaaatttgagaggtctgcatttatatatttaaatagatttgataggtttttaaattgctttttttcataaggtatttataaagttatttg gggttgtctgggattgtgtgaaagaaaattagaaccacgctgtatttacatttaccttggtagtttatttgtggatggcagttttctgtagttt nggggactgtggtagctcttggattgttttgcaaattacagctgaaatctgtgtcatggattaaactggcttatgtggctagaataggaa gagagaaaaaatgaaatggttgtttactaattttatactcccatt |
| **452** | 225207 _at | ttgtgtgtaatttcatggtggcctagtgttgtggtgcttctggtaatggtaatagaagctcaactattttttgtggatttcagtttttatcatcag aagtcctagacagtgacatttcttaatggtgggagtccagctcatgcatttctgattatacaaaacagtttgcagtaggttatttgtcattt cagtttttactgaaatttgagctaaacatttttacatgtaaatacttgtatttaccaaagatttaaatcagttgattaattaattaactcaaat actgtgaactatctntaaaacactagaaaaaagaaatgttagtatctcaattacaccaactgtgcaaatgaactttgataaaataga aataatctacattggcctttgtgaaatctggggaagagctttaggattctagtagatggatactgaatactcaggcccacttaanttatt aatgtatacattgtgtttttgtctttatgctatgtacag |
| **453** | 225242 _s_at | ggcgtgcaatttggtctgcgccacataaccattctgaagcttttaggcgttggagaggaagttgggggagtgttagaactgttccca attaatgggagctctgttgttgagcgagaagacgtaccagcccatttggtgaaagacattcgtaactattttcaagtgagcccggagt acttctccatgcttctagtcggaaaagacggaaatgtcaaatcctggtatccttccccaatgtggtccatggtgattgtgtacgatttaat tgattcgatgcaacttcggagacaggaaatggcgattcagcagtcactggggatgcgctgcccagaagatgagtatgcaggctat ggttaccatagttaccaccaaggataccaggatggttaccaggatgactaccgtcatcatgagagttatcaccatggatacccttac tgagcagnnannnnnnaccttagactcagccagtttcctctgcagctgctaaaactacatgtggccagctcca |
| **454** | 225269 _s_at | aactgtgtacttgtctggtcagctgtgtatgatcagttatctacctcagagtctattttcttttgtgctgggacaggttgctggccctccctgtt tccacagaccaaatcctcctagctcaggagctagggctaagcagttatttcttcaagtatttttagttcttaaatttatgcttgtatttgat gatagatgtcagtgacatttcatagtttcaaaagtccttgctgctctgagaagtgtagattctagtgaaaattacatagtcataagaga |
| | | aatgtgtttttgtttttgtttttgtttcatttttttaaagttgtggtattattggttctatgctccctggaatattactgctttgtgaaagtccagactga acgcagcaccctctgtgtacctagtacagttataaacctgggtctctcactacttgatatttttgcattagttaagacagaaatttgatag ctcggttagaggggaggggaaatctgctgctagaaatgtctgaactaagtgccatactcgtctgggtaagatttgggaaacataac ctctgtac |
| **455** | 225275 _at | gatgagagcaaaggggtcgcaccatatggaaatgttgaaaactattgtaaagtagtattatgaagtagcttttgtgtcattcatgtcga tgacatgaaagtgaagtaaatttattctatgtaaattcacactaaaaccagtacagtaccataagtagaatacatgtaagaatcacct agtcttcactatattgagtaaatataacatgctaatttacaattaatgaaactaaactttaaacatctccattatatctacatccttttgaa ggtatttatcatagttgccaattttaattttaggattgactttctctttctgaatgacttcataaagtttggtgtgaattttgaagacttgggttac taatgattgtatctttgctagtcaacaacttatgaaatatactcaatgcgtctgatgtgtcattaagtgcagaaataactaagacacaaa taacctttgcaaaccttcaagctgtgtaatattccaatgttgtttttttcttgtatatatacttatatcacgtaggatgtaaaaccagtatgac cttgtctagtctccaaacttaa |
| **456** | 225353 _s_at | gccacacgtccaaaaccaatcaggtcaactcggcggtgtgctgctgaggttgcaggtgggcgaggaggtgtggctggctgtca atgactactacgacatggtgggcatccagggctctgacagcgtcttctccggcttcctgctcttccccgactagggcggcagatgc gctcgagncccacgggccttccacctccctcagcttcctgcatggacccaccttactggccagtctgcatccttgcctagaccattctc cccaccagatggacttctcctccagggagcccaccctgacccacccccactgcacccccctccccatgggttctctccttcctntgaa cttctttaggagtcactgcttgtgtggttcctgggacacttaaccaatgccttctggtactgcc |
| **457** | 225381 _at | gtatgtttgtaagtgcttctgcacgaatgtttatacatgactgtttccatagtacttatgtttttaaaaatattcagtcatttcctactataatcct catgtatccatgtaactgactcaaaaatacttcagccacagaaagctaaaactgagcaaatctcattcttcttttccatccccttgcat gtggctggcatttagtaatgattaataatatggccagctgaataacagaggtttgagacacaattctttctcaaaggagtcagctaag ctgggtctacttatggacaaacatctaaatgtgtggaagtatctgatatttgacaatggtaaatttccacttagctagctagcattgtcag acttcaatctcctcatggctctggccgtcctgttttaagcatgataattgttggccacatctcacatagttctcattgagtgagttcataaat aaacagggtttttttttttttaaagagcagccaagcacaaagtgtgactttgttgacattttatgtgactttgtcatatgttcctaacc |
| **458** | 225442 _at | agtgtcaggtgccttgcaggaaaataatctgagtcccaagctagcctgtgctcatccacaatcacaatgaacatgtcaaggaaga atttgcagagactcaagggaagcacaatgggataaggtaatcactttcagtgaaaaactgttttcttgaaaacaggcttggacaca attgaaagctggcttcctgcaaacacaccaagagtctgtaatctagcctatccattatatgtcctttattattcatgatatcctattcttcta ccttgttgcctggtaactttttctgaggactgagtttctgcagcgatgtggtgcactcttcctgtgatgaggaaacatctgggcccct |
| **459** | 225458 _at | ctggacacccggttaccaaagtcagcaaagaagatgcggtaatcgccgcctgatctccacatggtgaacacaacactcccacc aacacctccttgactggtcggtcttcagcaccgggggtgggcaggcaggtgttctgtgttgacgagaattgcacaggctaaacaca aacacggaaccagagtgagaacacctcactcacggcagcccaggctgctccctaccaggtgacggagcgcgccggggctgt gggtgccaggggctgagtgctagggactcgtcatgagtggggatccccacgttcctgtcactgctgtcaaacagaaggtaaacag tcttatgaatgtatttccttaggaaaacttgtaaaaacttttattaggatatctatttaatactgaactttggcctactttgtgatagactataa acaaattgaggaaatcactatttctcacttctgtattttctcaaaaataatttgttacagagttcaatatactgtgtaccattgatcttctatt gtg |
| **460** | 225575 _at | gaaactgcggatcccaaactgttccctttttcatttcttgaaatgttaccactacagacattttttaaggtgaataaacagttgtgatgtg ctgtacntaaaatcatgtttaatcgtataaggaaacatttcaatacacttatacaggaagaaaactatagatgaagtacatgtgtgtg attcagtctgattcacagaattctgagagtaatatggaataaaacaactccacttagatgataactgaagcatttcctgccttgtgaaa atttggattttaaattgctgttagaatgggaaatttggacactttatatcattgtataatttcagaatttagtttctgtatcttttggaaaacatg attatagcaaaaacatagaaaataatctattactaaaacaccataaatgtaaaactagtatgcttggctgttaactctaaagatgtta cttatgtctgtttta |
| **461** | 225602 _at | tgagggtgagcttactcagggcccccagaggaagccctcagcctctgccctccccccacacagggcgggagcccaggcctgtt cctggcagctgtggctgcagctgtgctcctgctccctcctggaatgtgcgacaagcccaaatgttccngggnaggcggccggggc agggggcttagaagtgctaatatggttctgtgttttgcctgaaacgataccaggttcccctgaatagcaactttacaaggtccatgtgg gagggaccaacccagatgccctgctgagtgtccctgaaaccatggcagctccatctgtcaagatggcaggggccggagtgagg gggctgctggcttaacagcaggcatctgggcaggccagtcctcaaagcagctcctgaaggtctgtgttgcactgtcaccagtctca agctatgcctctaatttcaccagggatattg |
| **462** | 225604 _s_at | gtgtgatgcatgtgagcgtctctggcacacacacttggacatacagttctgtgtgcgctcattcttattacaggagtgagcaaaggaa gcatttaccccgatggttacctagaccacgattatttggattgggggagg |
| **463** | 225626 _at | atccctgtattcatggcttgactttgtgactgctctacactgcatgtctgacattgcagagtgagctatgttgaggtaaactggttggttgt cattattttgcaatcagcctggtctctcccatgaagatgtcgtgtgcataagcacaatcatcactgattagaagatcacagcagaata cccttggattagagagaagttcgtaccttgcatttctctgaattctagtctctcataagcactgctttgctggatgattttcactgctttgtgtt aatgactttgagcgatctctcacatgatggggttctttagtacatggtaacagccatgtcatcttacacacctagcattgtgaatgctgta gtgacatcctttataggcaccttacagctcaaaacttttgtttcatttcatgccttacttatca |
| **464** | 225688 _s_at | agtgccatttattctagtttatcatgttttgcatgtttgaaagtatgaatgtgctctttcctaaaacatggcaaatgaatagatgtagagaat aacaatattacttacaagatgaaatgattagattagaagtgtccctttattaaactttgtcagcctgactgggtacaattcttttgttaattt gcagtgtggtttgtatacacgtatacgtgttatcaataataagatttgcaactggatgacacaagattttacttgaacagtgaaggac aaaaatcatgattgtggaagatatttttaaaatctgatttgcagcgatcacttttaaaccctgtagtgatgtaagactaaaatataattg ctaagatttgttggttaatgtaaagatatgacttttctgcactgtactctcttcataggattgtaaaggtgttctaatccaattgcatgatgt |
| | | agtaagcctcttaaatatgtgtgtta |
| **465** | 225710 _at | tcctatgtcttctttcttaaatccagttgctgattttgtaaaatacagttgtgataaagcagcattacgggggggaaaaagctatattcca actggtgttaaatgtattcaacaaaatcttacatcatacagtatttatttcttaattaatagaacttcagtgatatacttggtagatatctca agccttttgtcttttacacaatggtgctctatcctattgttttcttttcaaagaagcatctgaacacttgcatttctattttcctatccaaaggca tccacatctaagtgtgtttttaaagttgattaaaattatttttctgttaaagcattctgaaagtgtttgtctttacctagaatgatttgtacacac tcgtggtcaactgaacatgaatgtcagtagtagtctaattatgggaagggtaaacgtgttagattaaggctcttaaagctctaaacca tataaactatggacttgtatcatgatttaactgttcttagatcttcttacacagtgattcattcctctatttgtacagtggcttt |
| **466** | 225720 _at | tatcaaaggtttgccagccaataaagtgcatcccaagtatacaggggagaaagctagactcctacagggtcctagagtttaagta attttttgttattaatataggtaataatttttctaatttttatttttggttccaaatgtaaagctccttgtgtttacctctgtttatgtcattcttgacat gtttatctaaattatgtgtgctctgtgacaggtgaaatgtaaatctgggatccatagtcaagatatcataaggacctacttcccagccta cctttcttcctctacctgataatgataatactcaaaataacaacattcaaaggaaacacaaagaaatcctgctttcacatctcctatttct tgggctccttaataactactgatggtttgttcatgaaaaaaaatttttaaatcaaaagattgtacttggccctgagttgaaaaaatttcaa aaatcaaaagtttgtacttggccctgagttgaa |
| **467** | 225721 _at | cagaaacatgaccctcgctggtcttgggtccacatatcattggactctgggggacacaaagatgcctgtgacactttggtgttgccg agttagtcaacaattattctgggaaaaagcagaattgaattcttctctagatgtcctaccaggttggccaagggccacaaagcag gctaataaattcccacaggatccagacaccaggcaaaattgctctaagaagccagttactgtcatccctctatggttctagaaaaa atagtacaaaaatgacaggtcatcctatgagcgtcatgccaatgaaaccccatcttctggagaagcccttgaatcagaattatcttttt tcttgatgtcgtcagatgcagccagtttcttaatttttttaaaaactgtatgtttctgtggtatgtatattgtacacctaactacctggcacttg gaaatcacagcactact |
| **468** | 225728 _at | ttatcctgcgacaacactttcagcagantagcctctccttatctcacagatcacaagcacccctagatagtgtgattctgtcagatagc atttatgcaaaaatctatgaagttaaaagatcgtagaagccaaatgaaatgtacatatctactgactgatgacaagggaatttcatta ggaagaaggtaaagaaacatcgttgagtagcctaccttgatttctgtcaagttcataaccagcttcatattttaaaggcttcaggtttga aattaagtcaactgcatgcagctttgctgataaatgaataattctctttgatgccatttatgagaaaagacttcaatatctgttgcctgtca tatttaagaaaaattactgtttctactctctgtatctgattttaaaagaaaaaactattcatacctggcttccaggtaattgactttga |
| **469** | 225782 _at | gaactgagtcaatatggcaaggtgtatgtgatctgtgggagttatgccatttaacataggaagtgcatgggactttccctctctgcact ccagctcttactgtaccattagaagatgcagaattctgttggtgtgcaaaaagtatagccttacattcaagcagaatggatctgaaga aagcagcaatatctgttactagagaacattcccatgtgtttaaactcttcacttcttagatgcatttaaattcttaatgcaaatgacgtag caatttgaaaacttctccgtattacttgtgtttaaaatgtcttgctttaaatacaaaacaaatggtaaaggggattatcttttgtttagatggt taaatattatttttgccttagatagctttgtaataatttttctccagacagttcaacacttttga |
| **470** | 225894 _at | tactgactgactacaggggctgattgtgaagcacgaggaaccccatgtgtgtggagactgtagggtgagagcacacaattattag catcatttctgagtgatctcacagattttttttcttgtgtttgttttgctttttgacaactgcttctcccacgttccttgcaattctattctctcaccttc actttactatttgtattcgatggaccaggataattcaggcaaggttaccttgtaaacttnaattggccacacaccatgttgtcacccagc tggctatgaagtgaataatggtactgaaagtaaacctgaagacctttctcagatctattttaagtctgagtctgaccaaccatggaaa atattcgacatgaattaatgtagagaactataaagcatttatgacagctccaagaaaaatcatctactctatgcaggagatatgttta gagacctctcagaaaaacttgcctggtttgagggtacacagtaccattttaatcttctgaaaatatctgtattcctgctctttttctgctgtc actgtcaatctgctatattttt |
| **471** | 225895 _at | ttcctgacgggaatgttgtgctataatgaatctgcataacgcttgggattctaggaggaaggaaggttccatggacatgtaagtaca gcatattcccctcagtcttctaggagggcagagtgaatcccagaactggtaagattgggaatctgagcattgccactttaatcttaga atatttatcattttgacacatcctgtttttagagaggaaaacaaacacagtttctgcattggtagtgtaaagcataccttgttaggaacgt gttttgtaagacacatttgggttgtcattctagagcatgtcaaactttgtacttcaaaatatatttagtatgattgttagtggtaacatatatc aaggctttgaattaactgttttatttaattttcacaagaagcacttatttagccataggaaaaccaatctgagctacaaatagttctttaa aataagcccaggttatttagctattctagaaagtgccgacttctttcaagaag |
| **472** | 226001 _at | ttaaatattcattccattacatctagactcaccaagaactacatgttatgatgttaagttgaagttgaaacatgatgttttgcattaaattta agatatgcaaatttatgtagagaaaataaatgttatataccctataatctttcacctaattagtatttaattatatggatttgttttatattata aaagatgttttgattttgtcttttgatattgacaaaattgtttggatatccttatgttctcaagtctgtatctgcctcccctgccttatttcttatgttt tgccacagttaacccattgtgcttctttgtaatcaaacagtttgtgggagaatgggcttattgaatgtctaaaaaataagtttaaagtgttt gttaccctaagtttttttacatttttaaactctaattacatatgtgaatgttattactctcagtgaattgttattgtttgcaaaaatgcactgggca gtaacattttgt |
| **473** | 226051 _at | gccggactggaaccgtctgagcggcctaacccgcgcccgggtagagacctgcgggggatgacagctgaaccgcctaaagga ggtgaaggctttcgtcacgcaggacattccattctatcacaacctggtgatgaaacacctccctgggccgaccctgagctcgtgct gctgggccgccgctacgaggaactagagcgcatcccactcagtgaaatgacccgcgaagagatcaatgcgctagtgcaggag ctcggcttctancgcaaggcngcgcccgacgcgcaggtgccccccgagtacgtgtgggcgcccgcgaagcccccagaggaa acttcggaccacgctgacctgtaggtccgggggcgcggcggagctgggacctacctgcctgagtcctggagacagaatgaagc gctcagcatccc |
| **$47 4.0** | 226084 _at | aatggcttctatgatcagaactgggaaaacagtgnatcttatggtggaagaggtnctcagcaagtgtacagtatttaccttcctttgtct tacatnggctttttaaattttccattaatttcaacataattatgggaacaagtgtacagaagaatttttttttaagatatgtgagaacttttca tagatgaactttttaacaaatgtttcatttacaggaaattgcaaagaaaattctcaagtgatagtcttttttttaagtgtttcgtaagacaa |
| **0** | | aaattgaataatgtttttgaagttctggcaagattgaagtctgatattgcagtaatgatatttattaaaaacccataactaccaggaata atgatacctcccaccccttgattcccataacataaaagtgctacttgagagtgggggagaatggcatggtaggctacttttcagggc cttgacaagtacatcacccagtggtatcctacatacttctttcaagatcttcaaccatgaggtaaaagagccaagttcaaagaaccc tagcacaaatttgctttgg |
| **475** | 226103 _at | ggaacctatcaatttgagtggactttttctttagtagtacaccatttnggttgtngtagtttcaaagtctttctgaagcagatatattgggatt ggagcggggtggggaaaactgtcactcctttcagaggaaaagggaggagcatggagaaaacaaaaattaaaggacttaa agaatggctatacagtgttgagtgttgaggatattaaacatgttatttttcaaacgtatgtaatatatattaaatttataaagcaaatttatg ttgtgatcttgcctgaacaaattatattttaatgaaaaaactttctattaatagttcacgcaagagaaaacactttcaacatagtcgaag gcttcaagatctaagtgtatcagacttagggaaaaagtggcacaaccttcgatttaaaattctagtctttaaaatgagtttgtaaataat tagctattacgttctattaagttgtt |
| **476** | 226147 _s_at | agggatctctctacaagagcccctgcccctctgttggaggcacagttttagaataaggaggaggagggagaagagaaaatgtaa aggagggagatctttcccaggccgcaccatttctgtcactcacatggacccaagataaaagaatggccaaaccctcacaacccc tgatgtttgaagagttccaagttgaagggaaacaaagaagtgtttgatggtgccagagaggggctgctctccagaaagctaaaatt taatttctttttcctctgagttctgtacttcaaccagcctacaagctggcacttgctaacaaatcag |
| **477** | 226302 _at | ggtaattatggaaactcctcaaagaggagaaagtaattttttccagacatttttctcattctgtgtctttcacacactagtttccatagttc gagaattctgtttttaccattgggctgtgaatgttcacaatatcagtcctgttgaattcctatgaggtaatcacaatgtgtatatgttcatttt ctaggtatgataaaagaatgtatggctttttattctgtggaagtaaaatcctgaacgtttacaacttttccttaacttgtaaataaaaaatt gtaagtttttttctttttttacagaaaacttagcttgtgtaattctgttagtttcagatttctctcctgtttttgcaaattgtgggaaagattgacaat gcaaatgtgtcaaagacatactgttgggtgc |
| **478** | 226303 _at | acatgggtttatacaagttcctcttgagaaggcaaaaagaccaccatgtgtgagagctctttgacttggccaataggggcctatctta atgcacttgtttggacacatttctgatcttattgtaaaggctgcaaaaggagaggatgaaatgctgtaaaagtaggaaatgaagtgg aagctggaagaaaatgtaattggtggtacagctatggccagatggtggagggagggtggggacccctgccggcaagcaga gtgtcacagctggctttcctcacttgggaaaagggtactgccggtctagcagcctcctctgtactcagccaggacacccagcgcgt gggacctgtttgtgtctgttttgcttccttgggaacggcacagtcactcaccctgccatttgcggaaatgacctggtgcactttgactgtt aagcaatgcgttattgctgtagtcaaggttagtgcaagcaaggaaacattcccagtaaggtatttgtttccattttctgtctgtgcttctgt cagaaacttgctaggac |
| **479** | 226304 _at | caaacgtcctggactgcacagacctcccactccagaccatccaggcctggttcccaagacccgatccttcccctgcaaccagac agtctacaactgccccctccagcccattttctgccgtgaaaccccagccagccacaccagactctggaacccttttcgactgcccc aactcttggacaccaggccaactagaacacccaacaccaaactgtacagactctcccaccccaacctccccagactctgcacg gatgtcctaggccccctccccaactctaaccagaccccatcccccctaagtccctttgtcttgacccccaagtcttcaaccagatatcct cggcaacccacctcccaccctcctcctcttctccttcaagacccaactgagcacccgctctgattccccacagcctttctccctgcca ccactcccttagtctttcccaggcttactctcccaataaatgtgctag |
| **480** | 226333 _at | ggtggagttggtggtcttaatttggagatgcaggggcaacctgtgaccctttgaggcaagagccctgcacccagctgtcccgtgca gccgtgggcaggggctgcacacggaggggcaggcgggccagttcagggtccgtgccaggccctcctcagtgccctgtgaagg cctcctgtcctccgtgcggctgggcaccagcaccagggagtttctatggcaaccttagtgattattaaggaacactgtcagttttatga acatatgctcaaatgaaattctactttaggaggaaaggattggaacagcatgtcacaaggctgttaattaacagagagaccttattg gatggagatcacatctgttaaatagaatacctcaactctacgttgttttcttggagataaataatagtttcaagtttttgtttgtttgttttacct aattacctgaaagcaaataccaaaggctgatgtctgtatatggggcaaaggt |
| **481** | 226430 _at | atttttccatcttattcaagtcagagcactttttttaaatagcaatacaacaggcaagagaatactcaaaaatatttaaaactgtattgat accaagagtatgttttaaatattttctaataaatacttgagcggtttttgtctggcaggcttccaaatttgccaaaattaagcgttcagtattt tcaacacatacgctttttactggtttatactgaactatctgatgagaattcctgtgttcccaaagcaactgatgtttacaggtcttgtgtttct cctcctcctttctaaggatgagggaatccacaacagactttctctagaaaacactaatgatggacaactttttggtgtcatcaatgagtt ggctactaccttgatgtaaaaatttgtaaggaaaattttcaccatttcgagtgtcaagtgtatttttaactgtctggtttgtacttttatgacttt tgtactaccaaagcgga |
| **482** | 226492 _at | agtgatctctggaagcgctaaagctaaaatttctgttcttgaaacacttcagctttgcaactaaaatattacagattaataaattaa accaaccaatgataaacactactcagtccaccaacaacaaacgtgtttgaattcaccttaccaatattaatcccagcgtgtgtaaaa cagaacagtaactctatgtgaccccagataacatttgtaacattgtgcttccttgtagtttgtaatgtgagttcaatcagtatttatgttga aatttctaacattaaatctagtctctatcctgttaatttaatttttaaatgctttatccatttgtgcaaaggtaaacgcagattgtatctttttaat ggtacggcataaaaagtaaccctcaagtgaagtgtctcta |
| **483** | 226594 _at | tgacgagacctcttcaaaaacccacagtaaaactcccctccctccagttggccaccagtctgccaccaaacatgaacaaattctg ctgctaatcggtttcccttgtgatctggttcctgaggtcttcggatctgtgcaatgaattatttattgtttattaaaccgacagtggtgtccca gagaggaaccataaataaaatggaaatctggtgctgtgataaagtaataactagcattaatgagacctggttttcctttcagaaagt ccagtatacctgtaacaaaggttaaagcaatttatatttaatttgcattctgatgttaacatttaaacagcaattctaacaaaaatgcatc gagtctaattcttacctctatcaaaaaacaactgtataaatttatgaccaacattaaaacaaaaacaaaatgtaaattttctctttagaa atgattaactggaaatgagtgagacagcaccttgtgggttt |
| **484** | 226654 _at | tgctcgcattgatcatcctaatcatcttattcagcctatcccagagaaaacggcacagggaacagtatgatgtgcctcaagagtggc gaaaggaaggcacncctggcatcttccagaagacggccatcngnnnagaccagaatctgagggagagcagattcggccttga |
| | | gaacgcctacaacaacttccggcccaccctggagactgttgactctggcacagagctccacatccagaggccggagatggtag catccactgtgtgagccaacggggcctcccaccctcatctagctctgttcaggagagctgcaaacacagagcccaccacaagc ctccggggcgggtcaagaggagaccgaagtcaggccctgaagccggtcctgctctgagctgacagacttggccagtcccctgc ctgtgctcctgctggggaaggctgggggctgtaagcctctc |
| **485** | 226682 _at | gctgattctaattgtgtgtaggtcttgaggattaagcacacaaatttcacaaacttctgtttgagtaaacaaactcagccttctgtaaata tacatgcaagtttggaaacagtaatactgtacctataaatatatgctgtctgntttgtgtacagtatgtaaaaactcctttctgccacact aaaaatgcaagccatttatgggaatcctaaaactagtattgaactaaaactttgctaatgatctttattagaggatcgtccaacttttca cttaccttgggttttcttttcaattcactcttacactagtctgcttatttccagctgtttattttattgagtcctgaatttaaaaaaaaatattttg attcattttgtaaatacaagctgtacaaaaaagagagatttaatgttgtcttttaaatactccaattttcattctaatatgaatgttgttatatt gtacttagaaactgtacctttaatattacattacctttattaaaagtgcattgaacacatcaatttagatgtgctttatgtactgttatccta |
| **486** | 226694 _at | taagcattcagtgagctgccaattttgattttgtgttgctctttacccaaattattttttcttgtttttctttttttggggggaggaggggaaaaaa gcagcaatactgtgtttggaaattatactctgtatctggttttcctgtgtatgttaaccacttaaatgttattatcctgctttggttttagagtgat tgtgaggcattcaatgca |
| **487** | 226811 _at | agtgcctaaatcttgtttacctatcactttaaaaaataattgaagtgtaagctaaataaaatgcttggagtttngcctgggctagtgag agttggtgcaaattcttgtgtgtgtttgcataggaaggtgagatgaccatctactaaagaggaagtagctaaatacagatctgtgggt gtttttaaaaaaactcaacctatctggtgttttattttaatggataaaaatgtaatttttctaaggtagcaacttatttccaaattaatatagat gaaaaatagataccaattagactaaattgaaagctttttgttctatattgcatagcctttgaaatatttcttagtgcctaggaggtctggg gattcctctttcgtggtggtcactaaccttacttgatgcaga |
| **488** | 226818 _at | aacaccaattatgcaaactactttttttccagcagaaagggagctgacatgatcaaatccatgttttcaaatgaactgaaaaaggc atccagcaccacttatancacatttatttcagttccaggctgacagccntggggnatctagcaggatnnataattgtcatctggtgag agttgggactttgctcaattttaataccaatctctcctgattgtaattacctccactactttcatgatcccccctacaatattttttaaatgatat atttattcactgaatcaaatgtcattaatgagttatcttctgtggaggatgactgttctctttgttaatgttcacaatcaagatcttgggctga gaagaggcccttcacccaaggagtttgaagtatcacagcgtgtgggaaggtgggaaccaggatacccattcatttccaaccgag acacagagaagtgagtcacagaatttgagccngctctcttgactgcccagccagagacactgatttctgtaacctcttcacttgatcc tgcctcttaagcatta |
| **489** | 226834 _at | agtgcctaaatcttgtttacctatcactttaaaaaaataattgaagtgtaagctaaataaaatgcttggagtttngcctgggctagtgag agttggtgcaaattcttgtgtgtgtttgcataggaaggtgagatgaccatctactaaagaggaagtagctaaatacagatctgtgggt gtttttaaaaaaactcaacctatctggtgttttattttaatggataaaaatgtaatttttctaaggtagcaacttatttccaaattaatatagat gaaaaatagataccaattagactaaattgaaagctttttgttctatatttgcatagcctttgaaatatttcttagtgcctaggaggtctggg gattcctctttcgtggtggtcactaaccttacttgatgcaga |
| **490** | 226841 _at | cctctgagtgttttgtgggttctgggtgttttgtacattttagccaagctaaccacttgtctgcaagtactgactttcctatgaattctttgaag attattgagtcagaaaggaaaaatatagccccaaattcccaggcttttaatgcattacattaactgcctattgaaatgagaagttcttc acaaacttgtatacccactaacaagattgcacataaacatgcattaaagtatatactaagaaaccctctgtccaacggctcatgcat atgaagtccgaacatgggagtttgccaattgcattcatcaagtcgttttgcggagtcagatccctgatggaagagctcacaggctctg ccttccaagtcctgggttcctaactggtgaccttagcctggggtctgtggggagaccaaccctggcttccaagaaaccacattcca tggactatcagaaatagacacagatttgggtgacaaagctggctctgtatttgcatttt |
| **491** | 227006 _at | gcaagcgcgtgctgagcaagctgcagtctccatcgcgggcccgcgggncagggggcagtcccgggggggctgcagaagcggc acncnngcgtcaccgtcaagtatgaccggcggganctgcagcggcggctggacgtggagaagtggatcgacgggcgcctgg aggagctgtaccgcggcatggaggcagacatgcccgatgagatcaacattgatgaattgttggagttagagagtgaagaggag agaagccggaaaatccagggactcctgaagtcatgtgggaaacctgtcgaggacttcatccaggagctgctggcaaagcttcaa ggcctccacaggcagcccngcctccgccagccaagcccctcccacgacggcagcctcagccccctccaggaccggcccgg actgctcacccctgaccctcttgcactctccctgccccccggacgccgcccagcttgcttgtgtataagttgtatttaatggttctg |
| **492** | 227052 _at | cactgttccatggtcagcaagtcatatttcataatgtggattttccaaaataattattgaatacagctattctatggctacttttagtgtttttgt ggtatgtggtgtgggagtgtttatggaattaccagtatcttaaattttcaaaggaaccttggaagtctatcactctaaatgaaagtctgtc actctacatgaattatgtgctcaaatttgaccaactcagtttaagacacaaaacagtaatttgaagaaggaaaaatgaagagagttt ctagtttaatgggttaaatttttgttgttgcaatagtaagtttagtcttcttataatatttctaaatgaaaaatcataggtatttgttaccatgtgt gaagattantttgttaaaagcaaaagtggtcgtgtgatatgctaaatgttaattactgattttatatgtttaaatcacgccaaacaaattat gtctgtgccatccagggtctgttgttaatctttttctgagtacttggattgggataaagggcttgtactatgcactt |
| **493** | 227061 _at | acacttatagtctactgcccatgtaaggatcagctccggctaagaggccaaagatgggtgacatcgttatgctctgccntttattttttct ttcttacccacttagcttcctaattggaggaaggaggcgtggtaaaggtatatgaagactatggtttaattagaccagaaaacactgt cataatctctggggtcatcagaatgtccagttttgtctttgggccaagataagggcagtgggatttatgatgtgttgtttatagtctgaaa ctactctggtgatcanccagggtcagtttctttaatgatggtttccaactggcctaatacattaagtaagactggctgataacatgacca gacagacataaagaccctgttgggaatgacattgaactctcaaagtcaagatttcttacacaaatctatcagctggagaaaatgaa ggcagtgtggtatatgtgtgcaaataaggacattatgaagcttaaatatggaatgtctcttggacccccgatgtcatctgtattctcttttt cttcttgtactatatcctttgcctgtaaata |
| **494** | 227099 _s_at | tcccagtgcgaactctgctgtgagtgtgcggggaggcgcgcccgcgctgagtcggcggcgggtagccactccatgcccttgtc cgatggtttgcaactccgattttgcacaccgctccaccgtgcccccagcgcacacccattcacactcacgccaacactctcgctg |
| | | aacacttttataattgttaggcgtggccgttgggactttgggcgcagcgcggctgctactgcgtctggaggattgatatttatttttgcatt gcgatggctgaaggcatttatttaacgatctttttacctggatatgtctgtgaggctcctgaa |
| **495** | 227235 _at | gtgaagtaccatagaccatattgtctagatgggacaaaataaattttgactaagaacttactgtttagagagaaaaaatagtaa ataatgttcaaaatgagaagacaataactgacaagtctgtgatttttgggttagatgagaccactttgatttttgaggaataaaagga gttagtttttggtcctgatgtgtaaaatgattcatggggtttcacatacagacaagtagtttcttttcagcaaatatttaacatatctctcaa tatatttttcaccagtttttggtaaatatgcattgctataggtataccaagtttttttgttatgtttagtaactaatttcatttttctgtatatgtaaa tatagctattggaccctgcattgaaaaccttcttagtactaacaacactccttttattattaccttcatagttactttaaatgacatatttatttc gagttttacttctggctgagttgcaaagtctatgagaatgttacctcctgtctcagaagcttgttagttattgtgccacc |
| **496** | 227265 _at | acatctgctagaaccttttgccttaactattcaccaatatatgctaatattcataaatatggattgactgtttacaaacattagaatcttgtc ttggttccattttgatggctaatatttgttatcttaattaagactatttctgaggtcatgattacttgaaaatattgactaaaactgggtcctta gaaattccaggtggagctgatttacctatgactgaggggaaaaaaaaatcaaattttactgataatagtaatgctccaaatgaatta atgacacatctgttcaataaataaagagcttaaatatacaaaacataagaaatctgggcaacaaaacttgtggtctttacttttgaat agctacccaagaaaaggttttaaaggtaaaagttatgagtaatgtcatcacaataagctcttgtttaaaattcttttctttatgtataatta ggtttatgtttcatgtcttt |
| **497** | 227404 _s_at | atcccatgggcaataaagcgcattcaatgtgtttataagccaaacagtcactttgtttaagcaaacacaagtacaaagtaaaatag aaccacaaaataatgaactgcatgttcataacatacaaaaatcgccgcctactcagtaggtaactacaacattccaactcctgaat atatttataaatttacattttcagttaaaaaaatagacttttgagagttcagattttgttttagatttgttttcttacattctggagaaccgaag ctcagctcagccctcttccttattttgctcccaaagcctcccccaaatcatcactccctgcccccttaaggctagaggtgagcatgtc cctcacaattgcacatgtcaagccatcagcaaggcgcatcacacaaaaggcaccaag |
| **498** | 227522 _at | cagtgagtcgaggttgcgctactgcactccagcctggacaacagaggagactctgtctcnaaaaaaaaaaaacctacagct gttcaaggaccagctgacaggtcaagtgtggccttttctggtctttgaacacatcatagaaagtgacaaatgctgcaaagccatga agaacatgaactataaacgggtagactaactgcccagcttagacacttatctatgccacaaaacagctgaatttgtcacatttatata ttgcaatatgggaagtattgagatcaaaacaggattccattgacctaattat |
| **499** | 227529 _s_at | gccatcacagttgcgattccatgagtagctgctttatgactgcttttgtactatctggatgtgcccagagttacttctgtacaagctctgta tctatgtccgttgagaacattattttaacaagaagaacaccaacagtagcatgaaatataatactgttttataattctaaagctgctgtta atttatgaagtacataataatctaatgtaaactgcagaagtcagagcaa |
| **500** | 227561 _at | gcaaccattggggaatgaccttttcatttcagaagtggatgaggaaggtggtgagcatcaggtatattnctggaccatttcaagtg ctggtgagaagaaaggaactctttgcctgaactgggcttggttttccaagtgctgctttggaaatgaagacccagagatgcagagct tatggtagttcataaatcttcatgttctattatctttcatctgccaataaagttcattttcaataatgtccaccattgctgtgcccagaataac cacaggcaaacatcaaaacaatacgcataagttagacaagattaaatcttgtctgatatctgcacaaacagatatgcaccatgttg gaaacatgtgttttcctagtcccatccaggcttcccacaagaaagccatgatgtgggtctaaacc |
| **501** | 227623 _at | attgtttcccatagcagaatgtcaatattcacagtacatttctgtaaagagcaaaccaatataatgttttgagtgttgaaaaaaattcca gatttntgaagaattagacaactcttcatctaccttatttctagttcacacagttatctcaaattccactgaaactaatggatactgtcttg tgtagatgccagttgagtttataatgtgacctagtaaagctgtctttttttgttgtgttgtatgagtgtcggatcatgcttttaggaatacttttatt aaaatggtgtgcattcatgcaaaaggccaactggcttttgtgaacaatagatcttttctcccctttatttgttctcttgacacttttgtgaaa attacctagcctgata |
| **502** | 227662 _at | aaagccttccctaaaaagagagcatgccagtcatagagacactaattggcacttttcctccttcttaggtttaaatgttataccaatgc atgtgtttgaaatatccattaagatgatttaaaatttgtcttgtattttgagtttcttaaatgcgtggattcttgttcatttatctctgaatatgtctc ttttatattttggcacaattatacacattggaaagggccaacctattagggctcaagtatgtatatgcnaaaaaaaaaagttatatcaa acaggcacagttattacaactagagagaaattccagaaatatttgtttttttaagagaaagtaaattttcacattagattctattcaaag tactaatatctacatggtccacacttttctatttaaaaaattgtgttctcttgttaaatagattaacatttccacttctgtttatcacaa |
| **503** | 227682 _at | atctttccatattgaggacagcctctgcatataaaaatgttcattgggagaaaattatttttaaaactaaatttttatagtagtcctccatt actggcaattaaggagcaacccacaaaatgtcattatgtggttctttgattaggcataaaaagtattgcaaacagcttttctattcctttg gaatatgaatctttttaatgtctaaagttaacctgcaacatttctagtaggacaagtcaatgtaaattaggtaatttttgcctgttttatacc ttctgtngcatttgttcaaatgaaaacatttttaaaacatgattgaatttttacttctcagataattggtttgagtgtggaatttctttttaaca ctttgatatgtaagttagtgtaaattaatacttttataacactactaggctttatatttttatggaatgttttaaaattgaaatttttgaantgtta atttagcagtactttatcattatgtgccaatactgacaag |
| **504** | 227705 _at | tttactccagtcattcgatgttgctgagatttacatatgactcttgtcaacatctcatcttttgacccaatcttattcatttaataagaggtctc attcatttgcatggaaaaatgctcattgtatattgcaaagtgaaaataacgagttgcaaaacagtgtatacatatatgtgtgtatatatgt acactttatttgtacatttctatgtgacataatgcaaaggaaagtgtctgatttattatacaccaaaggttaacagtgaatctctgtgtga tctcttttttttcttttgcctatctgcatcttctcacttgccaaaaaatgaatatatgtttatgtgtgtatattacttgtgtcacaaaaaacccta aagtagacagtaaaagaacttgtcaatcgcctttggaaggcaatgaaacacttaataaactctcaataa |
| **505** | 227725 _at | agccaagaactgaccggggccagggnctgccatggtctccttgcctgctccaaggcacaggatacagtgggaatcttgagactct ttggccatttcccatggctcagactaagctccaagcccttcangagttccaagggaacacttgaaccatggacaagactctctcaa gatggcaaatggctaattgaggttctgaagttcttcagtacattgctgtaggtcctgaggccagggattttaattaaatggggtgatgg gtggccaataccacaattcctgctgaaaaacactcttccagtccaaaagcttcttgatacagaaaaaagagcctggatttacagaa acatatagatctggtttgaattccagatcgagtttacagttgtgaaatcttgaaggtattacttaacttcactacagattgtctagaagac |
| | | ctttctaggagttatctgattctagaagggtctatacttgtccttgtctttaagctatttgacaactctacgtgtt |
| **506** | 227727 _at | gggctgttccagagattcgatcctcttaaggcattatcagtgagcaaatgtgaaggaaatggtgtctggaagaaagttctggttcaca tgccttgtagctaagtctttctgcaaacaacctcccttccccccgtcgagtcattggtgactttgatgggggatttctggttatgtcaag gctctggagacaggaanggcctttggccgccttgggtagttgacctgccttttctgactccgggacgagccagtcctaggctgcctc ngggagcacttgaggtatcccgcaggccatgaggacccactgggcagctcctggacagcctcttggctccagcccccacccga aagtggnacactgnntccgccctggccacctggggactggcactgtggtgcacagtggcccaatgtggccaacggaagtttt |
| **507** | 227735 _s_at | cacaggtgtagcactcccaaagcaagactccaganngnnnnnnacctcatgcctggcacctgaggtacccagcagcctcctgt ctcccctttcagccttcacagcagtgagctgcaatgttggagggcttcatctcgggctgcaaggaccctgggaaagttccagaactc cacgtccttgtctcaattgtgccatcaactttcagagctatcatgagccaacctc |
| **508** | 227736 _at | caccatgaccggtcacagctacaaatccagagaccatcaatcctgctagagtgcagggnggcaagcacccaagggtggctga ccaagactgcagagtctcctccatcttcaggtccattcagcctcctggcatttaactaccagcatccagtggtccccaaggaatccct tcctagcctcctgacatgagtctgctggaaagagcatccaaacaaacaagtaat |
| **509** | 227826 _s_at | ggttagattccgttttgactgcttggtatctcattgtaagcatttcccagggacactacacagtctttataattatgatcactacatcttttgg ataacttaggcttttgcactattttgtcacgatgcctcaaacttttctatcattgtctgtaattactcctataatcttttatt |
| **510** | 227827 _at | aatatctcaagatttctaggcattttcctatgtctaatagtaggatgttctctctgaatttatttattacaaaataacattacaacaataaa gcaaaataatatttaaaagaaagaaaaaacaaacagtcgcaataccagaacaccattgtagtcgtttgtgtatgtgtgacttctttac tgtgttcatgcagagacaagctttgtaatacctttgtaactactgtacaggtgcgtggttagattccgttttgactgcttggtatctcattgta agcatttcccagggacactacacagtctttataattatgatcactacatcttttggataacttaggcttttgcactatttgtcacgatgcct caaacttttctatcattgtctgtaattactcctataatctttattataaaatattgaaacaaagtcacatcactaaaaacactgtgagtata atttattagtaagtgtagactgtgtatcattagcaaa |
| **511** | 228133 _s_at | ggagctgcttcaagaagaaacccggcagaagctcaacgtgtctacgaagctgcgccagctggaggaggagcggaacagcct gcaagaccagctggacgaggagatggaggccaagcagaacctggagcgccacatctccactctcaacatccaggtgcctgcc ccgtgtccttgcttccttcatgggtcctctcaacttctctgcgctgagatcccccgcaggcagatcgcggtggagtgttggtgcgatggt gcttgacccccccagcttcccctgctattgggtttctccaacgaggagacatggtcttcgcttctcagagtctgtggggccagggacag gggccantcatggtcccccctctcaccctaccctggacgctgtccttgtagctctccgactcgaagaagaagctgcaggactttgcca gcaccgtggaagctctggaagaggg |
| **512** | 228195 _at | tgttacctttcagttacatgcctttgatcctaaaattctctacttttgntgccttatcagttctttgcaatctgcctgtggttatcagcacttaaa gcacaattttgaaggggaaaaaaatgataatcaccttagtcccaaagaaataatttntgtcaaactgccttattagtattnaaaaca gacacactgaatgaagtagcatgatacgcatatatcctactcagtatcattggccttttatcaaatggggaaactatactttgtattac atagttttagaaatcgaaagttagagactctttataagtaatgtcaaggaacagtaatttaaaaacaaagttctaacaaatatattgttt gcttaatcacaatgccctcaacttgtatttgaataactaaataggacatgtcttccttggagctgtgggcattagttcagaagc |
| **513** | 228202 _at | gtttacacctatactgcataatccaacaattttaatttcagttgaagacatgttactaatataactattattaaaagagtagaggatgtgt aattaaccatatcttctaaaacatggttactaaaagaatatgtaacatcaatattgaccttggtttcttacacaagtgttgctaactcaat agtgaaggagacactattaaattttctgaacccatgagagatactagagatggggagtggaaagtgtttggttcagggatatctgaa gaacagaagggcagagatttcttaagtgacgcctcatctacaagctggaaattcctaaaaacaagtagaaagcttataaacaac aggtgatacactcacctcactggtttagtaaattaccaatacagaaagtatccctagtcttaaaaacaagtggaaaatttgaactga ttagtcatattcctttgattacactgtt |
| **514** | 228232 _s_at | cagtcagagtggacaaacctctgtgggaggctctactgcactgagatgcagctcttccgagggggctcctaagccagtgtacaac tgggtgcgtcttggaacttttcctacaccttctcctggcagcatggttcnagatgaggtgtctggccagctcattctcaccaacctctccc tgacctcctcgggcacctaccgctgtgtggccaccaaccagatgggcagtgcatcctgtg |
| **515** | 228241 _at | gcctcctcagacactctcaagaggatggggagatgacatcacttgggtacaacttatgaagaaggtctcttttatgctcaaaaaa gtaagaagccattaatggttattcatcacctggaggattgtcaatactctcaagcactaaagaaagtatttgcccaaaatgaagaaa tacaagaaatggctcagaataagttcatcatgctaaaccttatgcatgaaaccactgataagaatttatcacctgatggcaatatgt gcctagaatcatgtttgtagacccttctttaacagttagagctgacatagctggaagatactctaacagattgtacacatatgagcctc gggatttacccctattgatagaaaacatgaagaaagcattaagacttattcagtcagagctataagagatgatagaaaaaagcctt cacttcaaagaagtcaaatttcatgaagaaaacctctggcacattgacaaat |
| **516** | 228469 _at | aaggttatagccacattcaacttgaatggtcccnaatgttaaatttaccatgaatactgtgtcttacaatgctgagctctcgactagact aaccagaataggaacacagtctcaaaggataccataagacaaacagtatttagacatataaaaactacagagatgataacttg aagaataaataacgtgaaaaactactatattcttgaatgggagtagaatgtaagctctttgagggcagggactttgactgtcttgttca ttgctatatgataatgcctggcccaacagcaggcattctgtagtgtctgttgaacaaacatctaaaatctggcaaagtgaacaatggt tcctgaggataagtatttaaataacaaaattaactttcactacaaaagctgccaacttacctttatcttctggtgctatcccctgagctttc ttaagatca |
| **517** | 228504 _at | gaatgcatgatctcaagccttaactactataatctttttctgcccctcagaaattgaataacctaaccaagatgcctttaggggatgcc ctaagtaaatgtaatttcagatttcagggnttttttttttttcctctctaagtgttccttccctttcttctcctgctctccatcatgttatggagacc agtgaggaaccagtgttaacttggtgacaatgtgacagctggtgctttatctaagctccgttttctatttcttgggaatgctttattgtggaa actgcttcagatacttaaattgaatcataacttgcttctgtaaattgcgtaaagacaacaaactgattttagtttgaaaagtttatcttttact tgtaaaccttgtttgccagttacct |
| **518** | 228507 _at | agccccaggtgtatttctgagtgtcaacagtgttaatttcaagcatgctaataaaatgtnnnnncggnnnatannnnnnnnnnaa nnannncnttnaaantnnnggtttccnntgntaaaancanccncntttgcttttgttagctgtaatattttttgtcatttagataagacctg gtttggctctcaataaaagatgaagacagtagctctgtacagggatatatctatattagtcttcatctgatgaatgaagaaattttctcat attatgttcaagaaagtatttacttcctaaaaatagaattcccgattctgtctattttggttgaataccagaacaaatctttccgttgcaatc ccagtaaaacgaaagaaaaggaatatcttacagactgttcatattagatgtatgtagactgttaatttgcantttccccatatttcctgc ctatcttacccagataactttctttgaaggtaaaagctgtgcaaaaggcatgagactcaggcctactctttgtttaaat |
| **519** | 228640 _at | aatcttgtgagttgtgacctaggtgaaggtntaatgataacatctggaggaagcatcatttgttcagctacctagggtttactttcagact tttcctacagtttaaagcacttgcgttcagtgtggtacgatctgtttgtaatgttaatcattgactattgttctgctacttggatgttgatagtga agcagagaacaatttatcatcgtttattatgagtcactatgcacgcaactatgcttaacatggcgaaatgtattaaacactcgtccaac tattt |
| **520** | 228706 _s_at | aaagggtggcatttgcgtcacgtggaccagggacagtgctgaaatcagcagtgctcagaaacaatttaacatgttgaaacgaca atattctaaaatactgatgaatcttgcatcaatataattattgggtttttttcttttcctgctgtataactccttgccatgcaaactctcaaga ggccaatatattcctggccatgtttgaatgagcctcttaaaataaacttagagccatgcaaatgccagcagcttaatggatttcatgg aatgaaataccgtgattaactcatagctacatatcattgcataaatgggatttatctttttctcacttatttttgcggtgaaa |
| **521** | 228707 _at | agagccatgcaaatgccagcagcttaatggatttcatggaatgaaataccgtgattaactcatagctacatatcattgcataaatgg gatttatctttttctcacttatttttgcggtgaaagtcgagggcatgcaagagtttctcttccagaagccaagaggagaacaaaggtcc taatgctgtactattccaccctttggacgcctcatccaggacgcagaggactctaggtttaacatttt |
| **522** | 228750 _at | gacccagagtcacctaagagaactcttccaacttaaatgactagggtgtgtaatcagttgcttcttattctgtgtctcagatttcctagtc catgacatttaatagtgagtatagagtaggatattctatacattttagtatttggcccttggaaacaaagttaaataccatgtcactcag aagactctcaaagtttgtgtataaatggcgaagtatgcactatgagagattctaatccattcacatcattcttgagttgtgcaaatacac ttggctttgatttcacttggtgatctaaatttgtttatttaagctgcaagaaagttacattaatttggaatgtgtcatcacttggacccagtat atcagatttttattgagtaaaatgttaaaaataataagtgtaactcagattctggatgttcgagtttacaatacattgcctgtaataaaag ctgattctgaagtgcatttttcttgacttactttggcag |
| **523** | 228766 _at | atgcacgtacaaatttcttaacctgttatcaatgtctgagctacataattatctttctagttggagtttgttttaggtgtgtaccaactgacatt tcagtttttctgtttgaagtccaatgtattagtgactctgtggctgctctcttcacctgccccttgtggcctgtctacaattctaaatggatttg aactcaatgtcgtcgcttctggtttcctgcatataccaatagcattacctatgactttttttttcctgagctattttcactgagctgagctaatg aactaaaactgagttatgtttaatatttgtatcaaatacataaaaggaatactgctttttccttttgtggctcaaaggtagctgcatt |
| **524** | 228846 _at | ggaatcttttccaataaccagctaaagatttgcactgaaatacaacttgtatgccttttgcatttttaaagcctgcttcctggatttaagca gagtgatagtgttcaaagagccagttcagcctgtaacatatttgaaaaagatatgtctgcactttgaggtccctttgaatgccattcac tagacctctcaagcattttgtttcattgctacatccaagcgcctcacaagtccacaatgcgggacagcatcaaaagctcaagactttg naaaaagcttgtgggcttgcactgggggagggaagggaacaaaattgtgtacttctttgtttaatttagaaataaggcatccaaga gatgccattattttctgtgtttcaattgttgtgcctttgagttaaactgcatttttgtcttttggttgaaatctgaaatgtactgtcccaatat |
| **525** | 228854 _at | ctccttatctgttctagttccgaagcagtttcactcgaagttgtgcagtcctggttgcagctttccgcatctgccttcgtttcgtgtagattga cgcgtttctttgtaatttcagtgtttctgacaagatttaaaaaaaaaaaaaggaaaaaaaaagaaaaaatgaatttactgctgcag gtttttttctctctccatgtgtcactaagtgaagtttgtgccttctatagcaaagagaatatttttacatcctactaacagtagattttttgtag tgaacatttttgtatttttatttataagtctcataagaaaaatagcaatgttcagttgtataccttgaatctgcagttaga |
| **526** | 228885 _at | aataggtcttgcaggtttcctttgaattttaagtatcataaatatttttaagtaaataatacggggtgtcagtaatatctgcagaatgaat gcagtctttcatgctaatgagttagtctggaaaaataaagtcttatttctatgttttattcatagaaatggagtattaatttttaatattttcac catatgtgataacaaaggatctttcatgaatgtccaagggtaagtcagtattaattaatgctgtattacaaggcaatgctaccttctttatt ccccctttgaactacctttgaagtcactatgagcacatggatagaaatttaactttttttgtaaagcaagcttaaaatgtttatgtatacat acccagcaacttttataaatgtgttaaacaatttactgattttataataaatatttggtaagattttgaataatatgaattcaggcagata tactaaactgcttttatttacttgtttagaaaattgtatatatatgtttgtgtatcctaacagctgctatgaa |
| **527** | 228961 _at | gagaacatcagatctaatagagcatagtgatactatttaatttaaccaaagtctctagtgaatatttcanctttgaatgtaaactaaca aataaacctgaccaccaaggagattgtttgcccagagtttcaaagcacattgtctacaaatggaaattgaaataatttataaaatatt gacgttactatgtttttaaaaagttcctaatttttcactaaatggaggaaactattagttttattgttaaatatggtagatattaatattcctct tagatgaccagtgattccaattgtcccagtttgaaataagtaccctgtgagtatgagataaattagtgacaatcagaacaagtttcagt atcagatgttcaagaggaagttgctattgcattgattttaatatttgtacataaacactgatttttttgagcattattttgtatttgttgtactttaa tacctggtgtacagttccagaaa |
| **528** | 229070 _at | gaaggatgctgaggacctagatgagaagttacctgcaaaaggcaaaagggttacttagtgtcagaaccaaggcaatgacttctct ctcccagatctcctagctactggtcctgggccannttttttttttaaataatcccaacntttctttaaaagacaagcatttcagtaagcta gttattttcatgggttgctcatccatttttttcagtgatctaaaaatgtagggagatggctactactgaagttgtctgtctacttgggataata gcaaattaattgaagacaatgggaaagtaagttataaaaaatactgggaaatctgtttctcttctgagcaagcattcagggcaggta taaacatcaaacatagtgacattgtcaaaacctcttccatt |
| **529** | 229254 _at | ggaaagagaaacgtggctcaagtgctggctcaccttctagctgtgtggccctgggcaggttactgaggctcttccaanctcacttttc acatgtaaaatggcatttctaaaagtacctaccctacctcaaaggaattctgaaaaagtaagtgaatttgtnannnaagcaaagc actcacaatatgcctgacacacaaactgcttaataaatgttagtaatttttaaaacctcctcataagcggcttagaaattattcatgtttg gtacatatttattgcaagtcaagcagatccaactttttgaaagctggtggtctaggagtatagtttaattatattgtaaatactcaaaa |
| | | gtattagatgccagtctaatttaaatgcagtctgactgtagcgagtaactgacctggcaatg |
| **530** | 229530 _at | gggactcagttcttagagaagtcttttggtcttagagttcatcaagcagagaaggggaaggacatggtccaggaaataaatcag cacacaaagagatggggtcttgagggtgcatagtacattctggacagccagttacctgggatgagttggagggaggagaataa ggacaaaagaccatctgggcaaaaatcacgaaggggtatgtgtgtcatgtaaaggtgtgccatgatagttattcatattgctattgta atattaatatatagtaattaactacacatgacacagctttacatgaccttaagtagttatcaacattaccataatagtaatattaataact acaataagagccattattattcacttgagg |
| **531** | 229659 _s_at | gttagcaagtgccagcttgtaggctggttgaagtacagaactcagaggaaaaaagaaattaaattttagctttctggagagcagcc cctctctggcaccatcaaacacttctttgtttcccttcaacttggaactcttcaaacatcaggggttgtgagggtttggccattcttttatctt gggtccatgtgagtgacagaaatggtgcggcctgggaaagatctccctcctttacattttctcttctccctcctcctccttattctaaaact gtgcctccaacagaggggcaggggctcttgtagagagatccctggcccaggacaggagatgccaaatctaatttatctcactgag ggc |
| **532** | 229831 _at | tttcctaccaatttcacattttgcagaaacttgttcacatttccaacaatatcagaattagaaaacagttcagataacaagaaagatta aaaattagggaaattctgatatcaccataaagcactatttacatttagagattacatttaagataaagtcatcatacacaaaaacaa taaatatttataactttctctataaggtccgcatatactgtatatattgaaacaatctgaatgactagtagatttcatatgaccattgttatttc cactttctccaatacttgtattttatgctacatgtaatgaagttggaccttttattatttagtaattcctatatgttcctatacttttcattttcaan atgattgctctattgtttcatgttgtttctagcaatatatctccatgagatatgcactttgtttcatattgaaaagtataaaatttatctttcaattc ctgtgtgtgtatcctatggttatctgtatgtat |
| **533** | 229839 _at | tcccaacccggttctggcaaagaatccagttatcccttccatgaagacgcacataactctcttacttggtctttccattagggacaaca taagtcttgttttacatcaaataaaaacaatgttaaaaagtgtgtgaaccttaaaaatggaagtctactagtttacatacctacttcaga ggacatggaaatgaccatgggcctgcatttcagggaccaaagcaaattaggcctggcctaaaatacatcagaccttttg |
| **534** | 230087 _at | gttgaccccaactttggaactcagatcataggatctcccagaggtttcaggtgttcctaagaagcttctaaggaagttttggaagaa agactaataggtgtaaacatataaacatatatattatatatagctatatatatttaaagagatctctacatatatatatatataaatacaa ctactgtactctctgcatctgtacaaagtatgagttaaactgcccaggtagtgtgagatacgtaatgcatgaatgtgaatatcctgaaa tctgccttcttagcaacgtgccgcgtgtactggagagaggacagttgctcctgggagttctctcaatgggcaaatgtacaagttgatg ccgtcttatccaccaacaagccattaaggttggtttcctgctgagactttgtaccggaagcgagaccggcagcactgggggtgtcc atatgccacttatgagg |
| **535** | 230264 _s_at | ggccaaggcacatttgactcctgagatgaatttttgtggtcataatcaaatacttagttgtttttgatgccccaaaataaagtgagaatg gtaatttgccaggaattcttcataacagtatcttacaaaaaacgtgttgctctcttcacagtattatgtgtaaagtcattgtttaaagcacg aatgttccctctggggtacttgttaaagctaaatttatttgcttccctccacttagaagtgctgcacactttacagcagcttcctttctttcca tngcactgcctagttaacagaagtcttataaaaatttaaaaagacacatttcttacaaaaaagagttgaatgaggtaaaatggcatt agatggctctatatttttaaagctatgtaattgttcagcgtcactttctaagtacttatacatatctaaacatgtcttcatggtttatattttca cttatatatgctggqctgqattaaqctttqt |
| **536** | 230595 _at | gggcagagggactatgttgtgagcctngcnaaagaagnttgtgtggggactgtgggcagtgaatgcgttgggaacaatatgga aaactgggagctgccttggaatctacagggccgnnnnnnnnnnnnnnnnnnnnnnnnnnnnnnnggctgaagaaaaga agaatggtattcatgcagttcccatttacagatgaaaacaagaggactttttctgtgaagtcaagaaagtggttacaatggtactttc agcctgtccgaattatgtattgcccctcccctttttattaataacattgaagtgtgatgggacaaccactgaagccgtctgttgaaacct gctgggactttttagccattctcttcaacataaagaatgggtgtttttggaggggggtgagaggaatggggaaatgttgtcaaagagta caatgttttagttgagacaggaggaatatatttgttgagatctacagcacagcat |
| **537** | 230788 _at | gatagcgaatgcactcagggtcagcagtggagtttaaaaattgtctcttttcaacttatttaaatgacagcacctgagaagaggaac cgttttacactggatgtttctcatgtagaacaagaaatcttctggaattgatgtttacatgtctgttgttggtcatctctcctgtgtcttaaata ctttaatgttggaagagcatagtgtttgggctagtgggtttctgacagcccatgggaatgccctgaaactactgtatctgatgtttgttttc gatgaggttccatgttttgttttc |
| **538** | 230830 _at | gcagctgtggtggtcattataagcatngtccntcctgggaagaagcatccaggcaagcagaaaagaaaagatgcagccacca gaaaaagaaactccagaagtcctgcatttggatgaggccaaggatcacaacagcctaaacaacctaagagaaactttgctctca gaaaagccaaacttggcccaggtggaacttgagttaaaagagagagatgtgctgtcagttttccttccggatgtaccagaaactga gagctagtgagggttcagagaagccccatcctaagccagacacatgatgtgggctcagctcagtggcctgaaacctctcaggtttt agagtctctcccaagaagccgcttttttc |
| **539** | 231120 _x_at | tactagttgttttacactctcttttcttattcttagggcttttgtgtatgtctnactnntntttnaannanntncncnnnnanncagaccttaa nttttatattttttaaagtngctaacatagcngnaggcacttaagcatttagtcaatgatattggtagaaatagtaaaatacatcctttaa atatatatctaagcatatattttaaaggagcnaaaataaaaccaaagtgttagtaaattttgatttattagatattttagaaaaataata gaattctgaagttttaaaaatgtcagtaattaatttattttcattttcagaaatatatgcatgcagttatgttttatttgattgttgacttaggcta tgtctgtatacagtaaccaa |
| **540** | 231579 _s_at | gagtaggttcggtctgaaaggtgtgggcctttatatttgatccacacacgttggtcttttaaccgtgctgancagaaaacaaaacngg ttaanaaananccgggtggnnagntgacagaggnaanccgctnaaataccttcacaataaatagtggcaatatatatatagtttaa gaaggctccccatttggcatcgtttaatttaaatgttatgttataagcacagctctcttctccaattttcatcctgcaagcaactcaaaan nnttnaaannaannttnnnntgtagttattttcaaatctttgcttgataagtattaagaaatantggacttgctgccgtaatttaaagctct gttgantttgtttccgnttggantttgggggaggggagcactgtgtttatgctggaatatgaagtctgagaccttccggtgctgggaac |
| | | acacaa |
| **541** | 231773 _at | ggaacctataaatgtagtttcatctgtcgtcaattactgcagaaaattatgtgtatccacaacctagttattttaaaaattatgttgactaa atacaaagtttgttttctaaaatgtaaatatttgccacaatgtaaagcaaatcttagctatattttaaatcataaataacatgttcaagata cttaacaatttatttaaaatctaagattgctctaacgtctagtgaaaaaaatatttttaaaatttcagccaaataatgcattttatttataaa aatacagacagaaaattagggagaaacctctagttttgccaatagaaaatgcttcttccattgaataaaagttatttcaaattgaann ngtncctttcacangtnnatgattaaatctganttcttaatnatatatcctatgctgatttcccaaaacatgacccatagtattaaatac a |
| **542** | 231925 _at | tctttttgagtgatagtgccattgaatgagcagtatggaaacagtgttacttgatattttgagctttctcaggtttatctaaatcagtggtag cttaacaaaacccagactaattgtgtgtaattgtatttttaataaaaggaaagtacatttcctataatagcatagtactgtttgcatgtaa gagtatgcaaaaccttgtgtgtgtgtgtgtgtgtgtgtgtgtgtgtgtgtcttagtgtgtgtaaggcatggcagccaactttgtatctgctatt tttagtacgagcagagcttcataattgtggtcactagaactgtacttaccatggacagttaaaact |
| **543** | 231975 _s_at | tctctcagtttaaacatcttaacagagaatttgttactgttattaaaagaattgcatttggaagcaagaaaacaaccggatttgcaatc atgtgaagaaacaataatgcctttattatcaagtgttaagcacaattcttataacaaactgtgataaattctttttgtttttttttcctttgcccc attattttcttatgaacaaaccaaaaattcatggtgagcagttgcagtgttggctgatatatcttttatgtacagggaatttgaaaaggac agtggattcatttagaagtgtaactggtgctgtgattatagcaatacattgttagttgtacttcatctttttcatgctagctttttaaatgtttag ttttcctcttgtcatggtcagctgctgaatttacttgaag |
| **544** | 233565 _s_at | agattctggccacggctgggaacgttgtcaccctgaccatcatccccagtgtgatctacgagcacatggtcaaaaagttgcctcca gtcctgctccaccacaccatggaccactccatcccagatgcctgaagccactgcagggcagggcaggcagggggggcttcccg ccctcctgcagcaaagggcaaccaccctcggatgatgggttgcagccggcctgctgcttaaggtgggggctgccatgaggggg cgtgtccaggagggtgaccatgggatggcttatacacacaggcctccttggagcctcagactccaagctaggctgaggctcaggc agggcccacaggcagccgattctcttgtgctgatttaaatgctggacacggaggcaggctgtttaaacgctgcttaaagtcgcaact gggcccctttcaagaaatttgctctaccaggaaaacagttacacattttaagagaacagagctacgttcttgtgagagctttttcctg ccttgacttgctctttgtcacagactgca |
| **545** | 234764 _x_at | gtcaccatctcttgttctggaagcagctccaacatcggaagtaattatgtatactggtaccagcagctcccaggaacggcccccaa actcctcatctataggaataatcagcggccctcaggggtccctgaccgattctctggctccaagtctggcacctcagcctccctggc catcagtgggctccggtccgaggatgaggctgattattactgtgcagcatgggatgacagattgagtggttttat |
| **546** | 234987 _at | gtttactctgagattctgtagtcctaatattgtatcattgtgctgtctgcaaaacaacttgaatctatttgnttgcatcttttgttacatgtaac gcagctgtactttatgttctttgcaactgtttccattatgagaacgctgtgctatttacaaggttacatttttcttggccaggcgaggtggtc atgcctgtaatcccagcactttgggaggccaaggtgggcggatcacttgaggtaaagagttgagaccagcctggctagcatggcg aaacccagtctctactaaaaatancaaaaattagccgggtgaaattagccgggcg |
| **547** | 235146 at | ggtgttttaaggttagcacatgcacaaaggaacgagttggtttaaaaagataaatcactgcaaagaatgaaattggcttattcacat caaaactagataagatgctaaaaaaaaaagatatgaaacagaactaacctatagtttcctgaaatcagtacagtttaatttataag aagctagaaagtaatgcaccttgattgttttaggaatgatttatgtgttgcaattttaatttatttaaagcatgtctactgtgtttgtcctaaga gaaatatttcaacaaaacgtgctctgtgtttaagatatgtttaggcagtagttagcaactctgaaagtagaaactggaaatgtttattgt gaggcttgttgcagaatttccattttgtgagttactacttagtttcatgtcagcctaaaattgtaaattccctgtagatcttcaccccattgtg gtgtcatcaatgaatccaaagcaggtgccattattt |
| **548** | 235766 _x_at | gcagggtcataatcacacagcagtgccttatatagttgccataagacttcagtgcagtacaacatanttttacngctacatatcaggg catattctatatggtgtatttgtgttagaataacacattaaatgtctttaaacataaaaataagaatgtttgcatgtttcagttttcaagaac caaatgagtaattagctatagattccactggccttaaacatacaattaagtgtatacatgatatagtgcacacacaaaagccaccttt aattattgaaataacctgtattctttttggaaatcatttaagtttggtattgaagtactatattttttgtgcatcaatgtatttttctatttacaagc ctatgt |
| **549** | 235849 _at | ggagatcgtgttctgctttttacatgaggagcagaactgggccatacacgtgttcaagaactaggggagctacctggtagcaagtg agtgcagacccacctcaccttgggggaatctcaaactcataggcctcagatacacgatcacctgtcatatcaggtgagcactggc ctgcttggggagagacctgggcccctccaggtgtaggaacagcaacactcctggctgacaactaagccaatatggccctaggtc attcttgcttccaatatgcttgccactccttaaatgtcctaatgatgagaaactctctttctgaccaattgctatgtttacataacacgcatg tactcatgcatcccttgccagagcccatatatgtatgcatatataaacatagcactttttactacatagctcagcacattgcaaggtttg catttaagttaaaaaaaaaaagaaaaaaaacctaagggtgaaacgatgccactttgacgc |
| **550** | 236300 _at | tgactttcatacacacccagtacatctcaaaggatgctaagggacattttctgccagtagagttctccccctttttnggtgacagcaat attattatgttcacatctaactccagagcttacttcctgtggtgccaatgtatttgttgcaatttactacatttttatatgagcctatttataggt gccattaaactcaggtctttcaaatgaaagagtttctagcccncttagggaaaaagataattgtttagaaaccataaaatcaatggt aggaaaagttggaactggttacctggatgccatggttctctgttaaataaagtaagagaccaggtgnannnnnaannnnnanna nngntattttcagcatgctaataaatgtctttccggttatatatctatctaaaattaacctttaaaatattggtttccttgataaaagcacca ctttggcttttgttagctgtaatattttgtcatttagattaagacctggttaggctctcaataaaagatgaagacagtagctctgtacagcg gataatatc |
| **551** | 236313 _at | gctagttgcattatactgggtcatgaaaaattatcccttgaaatagatatgaaacatgttacttcatttctggtttaaataacttgtggaat ctttcctaatgacaacntgatattaagggaaactaaagaaaatgttattgtggatcccacagtactatattacactgtttttttttgtttgtttt gttagtttttttttatttaaagcaaacctcaaacattattgggtatcaattaccacctggttgtattaaaatagtaacttatcaatgccatgta |
| | | aaaattaattccattttcgaagccacctggcagacaggtttagctgtttcatcagcagcctaatatatactgttaaatttgttaaggatttc actttgaaggatacatgcaaaacatatagttactattttcatgagtcctgcttctagctccattgtggaatacagaaaattaaatatacct gttaagttcgtatctaaacctaagacattaccaaggtttgtacaaattctactacctgacatttattccaaga |
| **552** | 238143 _at | ctcccctgaagaacgtgtccagcaacgcagagtgccctgcttgttatgaatctaatggaacttcctgtngtgggaagccctggaaat gctatgaagaagaacagtgtgtctttctagttgcagaacttaanaatgacattgagtctaagagtctcgtgctgaaaggctgttccaa cgtcagtaacgccacctgtcagttcctgtctggtgaaaacaagactcttggaggagtcatctttcgaaagtttgagtgtgcaaatgta aacagcttaacccccacgtntgcaccaaccacttcccacaacgtgggctccaaagcttccctctacctcttggcccttgccagcctc cttcttcggggactgctgccctgaggtcctggggctgcactttgcccagcaccccatttctncttctctgaggtccagagcacccccctg cggtgctgacaccctctttccctgctctgccccgtttaactgcccagtaagtgggagtcacaggtctccaggcaatgccgacagctg ccttgt |
| **553** | 238750 _at | tttgtccatagaggcttcaagatagataggtaagagnccagtagtgttcataagaagccaatagagagcaggagccactttatcag gtggcaggtgtcctgggcctccctgctggctagtcccaagcggtggtgttgccaggatgtcttggaggtgataatgggacacacag aggcactgagtctccataggttaaaatgccaccaaaactggcctttgcctaatatccctcattgactatttagcatttaatttatttatttc ctgacatttctgcaagctttgtatttatatttccactttatagatgaggaaatttgaggctcttagaggtaaaatgacttgcccaggtcaca caggaagtggcagagacaagctttttaaataagaaaaaattaataaaatataatatgagagtaacttaaaatattaataaaccac aattttaaattaattaaccgtgataaccaacattaataaaagttaagataccaaaacactggtgtctaattctttcaac |
| **554** | 238751 _at | gttctgttgtttatccatgaagcctatatatttctagtgcaaaangcccagtgggtctcaaaacattcttggaggggtttgacacactgct attagtagtgactcataattccacgattccctgggatacagagaaggcagggggtagtactgtttgaacaaaaatcttcccaaga ccctggcaaagagatcttccttaggaatcagtggcatatgcgtttatgttctttaacagggaaagtaaaagtgagcgcactttttgaa gctcataaatactcatgtgactgtaaatttagaaatgttaattaattgggcctaatgtttgatctggaaaagtacttagcagtcaccagg aatttgccttgaactgactcgaaccacaaatcagaaccgtatttccacaattta |
| **555** | 239272 _at | gcactgttaaaatccggctaggccaagatttcccaatgtcttgaagccaatggctaggaaaacagctgagcctgggactggacttt gtcagtaacctctcttccctagggcacgggaggagtngggtgaataggacctcttggcttgaggaccatgattgtgaatacagaca tttaattcagaatgacacctacattgatgaattttcattgtacaagtgtttttccagatcaaacacaaaacccatgtaattccttgataaat tttcaatgcagggcagataaactgttgctaac |
| **556** | 241994 _at | gtgatcttggactgtcaattcccctncctgtgatccattttactgcaaacataagggttgcagtaaaggttgtctcacgtcttctgcttta aaagcctataaatatatgacctgaaaactccagttacataaaggatctgcagctatctaaggcttggtttcttactgtcatatgatacc tgggtctaatgaactctgctgagatcacctcaagtttctgcggttggtaaagagaacaaggaagaacaaacatcccttttattgctc caaatggtgatttaatccctacatggtgctgggtggacaatgtgtcactgtcacatgccttnactgtataaatccaaccttctgccaga gagaatctgtggttctggccatggagggaggatagtggaaatgatatagttggactggtgcttgatgtcacta |
| **557** | 242317 _at | atgtgattatacttctctttgacttgtcagcttagctttagnngatacactctggtgcccaactattattgtatcagtgaacttnccactttnct tttncctttnctctcaatttttgttgtatcattcctaccttgtgaggacatataatatttacattctgttgtcatcctcacatttcttagttccacag tttaaatgtatttgaaactcaaaacattcccattaatctcttggtcagctgaaattaatgatttaatagtttccttaaaaagactcatgga acaatttccctaaatttttgccatgtcaaatatgtttatctgtagcctttacacagtaaaaacaatttggctagataatacaattctcagttc atat |
| **558** | 242447 _at | agggcacttctcaacattaattttggaattattcaccatttaaaaattgtcactgctctttttcattactgtagataactgagagttagctgtt ctaggaaaaggccatgtttcagttagtgctatgacttctttttcgattaaggttgaagcagtgttctcttagtagcctgaacacttctcaa agcgacttatcactagcggctttaatattgagcttctatatcgttgtgtccacctcacttttaaggctgttcatacctggcaatattcactta ctctgctatttttaaaaactatttaaagttttaagcacaatgttgactcttctgtaatttcctaaaattatattgttttcctacagacagctcaa ctttccttgatatgt |
| **559** | 242601 _at | ctcattattcctttacatgcagaatagaggcatttatgcaaattgaactgcagtttttcagcatatacacaatgtcttgtgcaacagaaa aacatgttggggaaatattcctcagtggagagtcgttctcatgctgacggggagaacgaaagtgacaggggtttcctcataagttttg tatgaaatatctctacaaacctcaattagttctactctacactttcactatcatcaacactgagactatcctgtctcacctacaaatgtgg aaactttacattgttcgatttttcagcagact |
| **560** | 243278 _at | ataaatgaccagactttttctaagaaaaatgttgctttaatgcatttcatgaatttttactcttatatcattgcttgctagtaatagcaaatctg cttttctgcatctgctttgcgtagctattgtaaggctttgaactaatgtatgtatttattgcttgaacttctgtgcataccttataaagcataatg tctgacaatttaaatggctcatgtattcttgcttntatcataagctgattatggggactatgatcttttgtatacagcaaattttaaactgtag cacaaacatctgtttatgtattggtggaatatacctgttttatttatctttttttgaggtaaactaatttttgatacttttcattactgtgtactatgtt catactttgaattctctgacgttagaagtcatggttgagaattgtaacagctgttattcgttctgtattcatggctttc |
| **561** | 200832 _s_at | aaaagcgaggtggccatgttatgctggtggttgccagggcctccaaccactgtgccactgacttgctgtgtgaccctgggcaagtc acttaactataaggtgcctcagttttccttctgttaaaatggggataataatactgacctacctcaaagggcagttttgaggcatgacta atgctttttagaaagcattttgggatccttcagcacaggaattctcaagacctgagtatttttataataggaatgtccaccatgaacttg atacgtccgtgtgtcccagatgctgtcattagtctatatggttctccaagaaactgaatgaatccattggagaagcggtggataacta gccagacaaaatttgagaatacataaacaacgcattgccacggaaacatacagaggatgcctttctgtgattgggtgggatttttc ccttttatgtgggatatagtagttacttgtgacaagaataatttggaataatttctattaatatcaactctgaagctaattgtactaatctg agat |
| **562** | 201147 | gagtcggagatgatgcagcacacacacaattccccagcccagtgatgcttgtgttgaccagatgttcctgagtctggagcaagca |
| | _s_at | cccaggccagaataacagagctttcttagttggtgaagacttaaacatctgcctgaggtcaggaggcaatttgcctgccttgtacaa aagctcaggtgaaagactgagatgaatgtctttcctctccctgcctcccaccagacttcctcctggaaaacgctttggtagatttggcc aggagctttcttttatgtaaattggataaatacacacaccatacactatccacagatatagccaagtagatttgggtagaggatactat ttccagaatagtgtttagctcacctagggggatatgtttgtatacacatttgcatatacccacatgggg |
| **563** | 201162 _at | ggaatgtcactggtgcccaggtgtacttgagctgtgaggtcatcggaatcccgacacctgtcctcatctggaacaaggtaaaagg ggtcactatggagttcaaaggacagaactcctgcctggtgaccggacaacctggccattcagacccggggtggcccagaaaa gcatgaagtaactggctgggtgctggtatctcctctaagtaaggaagatgctggagaatatgagtgccatgcatccaattcccaag gacaggcttcagcatcagcaaaaattacagtggttgatgccttacatg |
| **564** | 201163 _s_at | aggtgaaggtgccgagctataaacctccagaatattattagtctgcatggttaaaagtagtcatggataactacattacctgttcttgc ctaataagtttcttttaatccaatccactaacactttagttatattcactggttttacacagagaaatacaaaataaagatcacacatca agactatctacaa |
| **565** | 201185 _at | ctcaaagactctcccgtggatgacggatgaggactctgggctgctggaataggacactcaagacttttgactgccattttgtttgttca gtggagactccctggccaacagaatccttcttgatagtttgcaggcaaaacaaatgtaatgttgcagatccgcaggcagaagctct gcccttctgtatcctatgtatgcagtgtgctttttcttgccagcttgggccattcttgcttagacagtcagcatttgtctcctcctttaactgagt catcatcttagtccaactaatgcagtcgatacaatgcgtagatagaagaagccccacgggagccaggatgggactggtcgtgtttg tgcttttctccaagtcagcacccaaaggtcaatgcacagagaccccgggtgggtgagcgctggcttctcaaacggccgaagttgc ctctttt |
| **566** | 201261 _x_at | tctctctttctgtgtgtgtgtgtgtgtgtgtgtgtgtgtgtgtgtcttgtgcttcctcagacctttctcgcttctgagcttggtggcctgttccc tccatctctccgaacctggcttcgcctgtccctttcactccacaccctctggccttctgccttgagctggactgctttctgtctgtccggcc tgcacccagcccctgcccacaaaccccagggacagcggtctccccagcctgccctgctcaggccttgcccccaaacctgtact gtcccggaggaggttgggaggtggaggcccagcatcccgcgcagatgacaccatcaaccgccagagtcccagacaccggtttt cctagaagcccctcacccccactggcccactggtggctaggtctccccttatccttctggtccagcgcaaggaggggctgcttctga ggtcggtggctgtctttccattaaagaaacacc |
| **567** | 201792 _at | ccaccgctggctgggaggagtcggagactgagacctacacagaggtggtgacagagtttgggaccgaggtggagcccgagttt gggaccaaggtggagcccgagtttgagacccagttggagcctgagttcgagacccagctggaacccgagtttgaggaagagg aggaggaggagaaagaggaggagatagccactggccaggcattccccttcacaacagtagagacctacacagtgaactttgg ggacttctgagatcagcgtcctaccaagaccccagcccaactcaagctacagcagcagcacttcccaagcctgctgaccacagt cacatcacccatcagcacatggaaggcccctggtatggacactgaaaggaagggctggtcctgcccctttgaggggtgcaaac atgactgggacctaagagccagaggctgtgtagaggctcctgctccacctgccagtctcgtaagagatggggttgctgcagtgttg gagtaggggcagagggagggagccaaggtcactcca |
| **568** | 202237 _at | ctactacatgattggtgagcagaagttctccagcctcccctgggccgggaggcagtagaggctgctgtgaaagaggctggctac acaatcgaatggtttgaggtgatctcgcaaagttattcttccaccatggccaacaacgaaggacttttctccctggtggcgaggaag ctgagcag |
| **569** | 202238 _s_at | tcgtcactgactactcagaccagaacctgcaggagctggagaagtggctgaagaaagagccagaggcctttgactggtcccca gtggtgacctatgtgtgtgatcttgaagggaacagagtcaagggtccagagaaggaggagaagttgagacaggcggtcaagca ggtgctgaagtgtgatgtgactcagagccagccactgggggccgtccccttacccccggctgactgcgtgctcagcacactgtgtc tggatgccgcctgcccagacctccccacctactgcagggcgctcaggaacctcggcagcctactgaagccaggggggcttcctgg tgatcatggatgcgctcaagagcagctactac |
| **570** | 202310 _s_at | tggcctacatggaccagcagactggcaacctcaagaaggccctgctcctccagggctccaacgagatcgagatccgcgccgag ggcaacagccgcttcacctacagcgtcactgtcgatggctgcacgagtcacaccggagcctggggcaagacagtgattgaatac aaaaccaccaagacctcccgcctgcccatcatcgatgtggccccttggacgttggtgccccagaccaggaattcggcttcgacg ttggccctgtctgcttcctgtaaactccctccatcccaacctggctccctcccacccaaccaactttccccccaacccggaaacaga caagcaacccaaactgaacccccctcaaaagccaaaaaatgggagacaatttcacatggactttggaaaatattttttcctttgcatt catctctcaaacttagtttttatctttgaccaaccgaacatgacca |
| **571** | 202311 _s_at | gctccccattttataccaaaggtgctacatctatgtgatgggtggggtggggagggaatcactggtgctatagaaattgagatgccc ccccaggccagcaaatgttccttttgttcaaagtctattttattccttgatatttttnttntttttttttttttgtggatggggacttgtgaattttt ctaaaggtgctatttaacatggaggagagcgtgtgcggctccagcccagcccgctgctcactttccaccctctctccacctgcctct ggcttctcaggcctctgctctccgacctctctcctctgaaaccctcctccacagctgcagcccatcctcccggctccctcctagtctgtc ctgcgtcctctgtccccgggtttcagagacaacttcccaaagcacaaagcagtttttncccctaggggtggaggaagcaaaaga ctctgtacctattttgt |
| **572** | 202404 _s_at | actttcccatgagtgtgatccacattgttaggtgctgacctagacagagatgaactgaggtccttgtttgttttgttcataatacaaaggt gctaattaatagtatttcagatacttgaagaatgttgatggtgctagaagaatttgagaagaaatactcctgtattgagttgtatcgtgtg gtgtattttttaaaaaattgatttagcattcatatttccatcttattcccaattaaaagtatgcagattatttgcccaaagttgtcctcttcttc agattcagcatttgttctttgccagtctcattttcatcttcttccatggttccacagaagctttgtttcttgggca |
| **573** | 202450 _s_at | gaacaacgcctgtggcattgccaacctggccagcttccccaagatgtgactccagccagccaaatccatcctgctcttccatttcttc cacgatggtgcagtgtaacgatgcactttggaagggagttggtgtgctatttttgaagcagatgtggtgatactgagattgtctgttcag tttccccatttgtttgtgcttcaaatgatccttcctactttgcttctctccacccatgacctttttcactgtggccatcaggactttccctgacag |
| | | ctgtgtactcttaggctaagagatgtgactacagcctgcccctgactgtgttgtcccagggctgatgctgtacaggtacaggctggag attttcacataggttagattctcattcacgggactagttagctttaagcaccctagaggactagggtaatctgacttctcacttcctaagtt cccttctatatcctcaaggtagaaatgtctatgttttctactccaattc |
| **574** | 202859 _x_at | gtacccagttaaattttcatttcagataaacaacaaataattttttagtataagtacattattgtttatctgaaagttttaattgaactaacaa tcctagtttgatactcccagtcttgtcattgccagctgtgttggtagtgctgtgttgaattacggaataatgagttagaactattaaaacag ccaaaactccacagtcaatattagtaatttcttgctggttgaaacttgttattatgtacaaatagattcttataatattatttaaatgactgc atttttaaatacaaggctttatatttttaactttaagatgtttttatgtgctctccaaattttttttactgtttctgattgtat |
| **575** | 202878 _s_at | atccgaattctccatatattcactaatcaaagacactattttcatactagattcctgagacaaatactcactgaagggcttgtttaaaaat aaattgtgttttggtctgttcttgtagataatgcccttctattttaggtagaagctctggaatccctttattgtgctgttgctcttatctgcaaggt ggcaagcagttcttttcagcagatttgcccactattcctctgagctgaagttctttgcatagatttggcttaagcttgaattagatccctgc aaaggcttgctctgtgatgtcagatgtaattgtaaatgtcagtaatcacttcatgaatgctaaatgagaatgtaagtatttttaaatgtgtg tatttcaaatttgtttgactaattctggaattacaagatttctatgcaggatttaccttcatcctgtgcatgtttcccaaactgtgaggaggg aaggctcagagatcgagcttctcctctgagttctaacaaaatggtgctttgagggtcagcctttaggaaggtgcagctttgttgtcctttt g |
| **576** | 202917 _s_at | ggcatcatgttgaccgagctggagaaagccttgaactctatcatcgacgtctaccacaagtactccctgataaaggggaatttccat gccgtctacagggatgacctgaagaaattgctagagaccgagtgtcctcagtatatcaggaaaaagggtgcagacgtctgg |
| **577** | 202998 _s_at | gccagtcttgaccgggatgaggcccacagacaggttgtcatcagcttgtcccattcaagccaccgagctcaccacagacacagt ggagccgcgctcttctccagtgacacgtggacaaatgcgggctcatcagcccccccagagagggtcaggccgaaccccatttct cctcctcttaggtcattttcagcaaacttgaatatctagacctctcttccaatgaaaccctccagtctattatagtcacatagataatggtg ccacgtgttttctgatttggtgagctcagacttggtgcttccctctccacaacccccacccccttgtttttcaagatactattattatattttcac agacttttgaagcacaaatttattggcatttaatattggacatctgggcccttggaagtacaaatctaaggaaaaaccaacccactgt gtaagtgactcatcttcctgttgttccaattctgtgggtttttgattcaacggtgctataaccagggtcctgggtgacagggcgctcactg agcaccatgtgtcatcacagaca |
| **578** | 203083 _at | caggaaatagtcactcatcccactccacataaggggtttagtaagagaagtctgtctgtctgatgatggatagggggcaaatcttttt ccccttctgttaatagtcatcacatttctatgccaaacaggaacgatccataactttagtcttaatgtacacattgcatttgataaaatta attttgttgtttcctttgaggttgatcgttgtgttgttttgctgcactttttactttttttgcgtgtggagctgtattcccgagacaacgaagcgttgg gatacttcattaaatgtagcgactgtcaacagcgtgcaggttttctgtttctgtgttgtggggtcaaccgtacaatggtgtgggaatgac gatgatgtgaatatttagaatgtaccatattttttgtaaattatttatgtttttctaaacaaatttatcgtataggttgatgaaacgtcatgtgttt tgccaaagactgtaaatatttatttatgtgttcacatggtcaaaatttcaccactgaaaccctgcacttagctagaacct |
| **579** | 203325 _s_at | cccacctggagctgaatcacatgacctagctgcaccccagcgcctgggcccgccccacgctctgtccacacccangcgccccg ggagcggggccatgcctccagccccccagctcgcccgacccatcctgttcgtgaataggtctcaggggttgggggagggactgc cagatttggacactatattttttctaaattcaacttgaagatgtgtatttcccctgaccttcaaaaaatgttccaaggtaagcctcgtaaa ggtcatcccaccatcaccaaagcctccgtttttaacaacctccaacacgatccatttagaggccaaatgtcattctgcaggtgccttc ccgatggattaaag |
| **580** | 203570 _at | ctggaggacagcacctgtgacttcggcaacctcaagcgctatgcatgcacctctcatacccagggcctgagcccaggctgctatg acacctacaatgcggacatcgactgccagtggatcgacataaccgacgtgcagcctgggaactacatcctcaaggtgcacgtga acccaaagtatattgttttggagtctgacttcaccaacaacgtggtgagatgcaacattcactacacaggtcgctacgtttctgcaac aaactgcaaaattgtccaatcctgatctccgggagggacagatggccaatctctccccttccaaagcaggccctgctccccgggc agcctcccgccgaggggcccagccccaacccacaggcagggaggggcatccctccctgccggcctcagggagcgaacgtg gatgaaaaccacagggattccggatgccagaccccatttatacttcacttttctctacagtgttgttttgttgttgttggtttttatttttatac tttggccataccacagagctagattgccc |
| **581** | 203878 _s_at | tgccagcgactgtctcagactgggcagggaggctttggcatgacttaagaggaagggcagtcttgggacccgctatgcaggtcct ggcaaacctggctgccctgtctcatccctgtccctcagggtagcaccatggcaggactgggggaactggagtgtccttgctgtatcc ctgttgtgaggttccttccaggggctggcactgaagcaagggtgctggggccccatggccttcagccctggctgagcaactgggct gtagggcagggccacttcctgaggtcaggtcttggtaggtgcctgcatctgtctgccttctggctgacaatcctggaaatctgttctcca gaatccaggccaaaaagttcacagtcaaatggggaggggtattcttcatgcaggagaccccaggccctggaggctgcaacata cctcaatcctgtcccaggccggatcctcctgaagcccttttcgcagcactgctatcctccaaagccattgtaaatgtgtgtacagtgtgt ataaaccttcttcttctt |
| **582** | 204006 _s_at | gagtacaggtgccagacaaacctctccaccctcagtgacccggtgcagctagaagtccatatcggctggctgttgctccaggccc ctcggtgggtgttcaaggaggaagaccctattcacctgaggtgtcacagctggaagaacactgctctgcataaggtcacatatttac agaatggcaaagacaggaagtattttcatcataattctgacttccacattccaaaagccacactcaaagatagcggctcctacttct gcagggggcttgttgggagtaaaaatgtgtcttcagagactgtgaacatcaccatcactcaaggtttggcagtgtcaaccatctcatc attctctccacctgggtaccaagtctctttctgcttggtgatggtactcctttttgcagtggacacaggactatatttctctgtgaagacaa acatttgaagctcaacaagagactggaaggaccataaacttaaatggagaaaggaccctcaagacaaatgacc |
| **583** | 204051 _s_at | aaccagccagtcccaagaagaacattaaaactaggagtgcccagaagagaacaaacccgaaaagagtgtgagctaactagt ttccaaagcggagacttccgacttccttacaggatgaggctgggcattgcctgggacagcctatgtaaggccatgtgccccttgccc taacaactcactgcagtgctcttcatagacacatcttgcagcatttttcttaaggctatgcttcagtttttctttgtaagccatcacaagcca |
| | | tagtggtaggtttgccctttggtacagaag |
| **584** | 204320 _at | gaaaatgtaccttggtgccaccaacccatttgtgccacatgcaagttttgaataaggatgtatggaaacaacgctgcatatacag gtaccatttaggaaataccgatgcctttgtgggggcagaatcacagacaaaagctttgaaaatcataaagatataagttggtgtgg ctaagatggaaacagggctgattcttgattcccaattctcaactctcctttcctatttgaatttcttggtgctgtagaaaacaaaaaaa gaaaaatatatattcataaaaaatatggtgctcattctcatccatccaggatgtactaaaacagtgtgtttaataaattgtaattattttgt gtacagttctatactgttatctgtgtccatttccaaaacttgcacgtgtccctgaattccgctgactctaatttatgaggatgccgaactct gatggcaataatatatgtattatgaaaatgaagttatgatttccgatgaccctaagtcc |
| **585** | 204475 _at | gaagaactgtctattttctcagtcatttttaacctctagagtcactgatacacagaatataatcttatttatacctcagtttgcatattttttac tatttagaatgtagccctttttgtactgatataatttagttccacaaatggtgggtacaaaaagtcaagtttgtggcttatggattcatatag gccagagttgcaaagatcttttccagagtatgcaactctgacgttgatcccagagagcagcttcagtgacaaacatatcctttcaag acagaaagagacaggagacatgagtctttgccggaggaaaagcagctcaagaacacatgtgcagtcactggtgtcaccctgg ataggcaagggataactcttctaacaca |
| **586** | 204620 _s_at | tgccgtgctcccaaaacatttaaatgaaagtattggcattcaaaagacagcagacaaaatgaaagaaaatgagagcagaaa gtaagcatttccagcctatctaatttctttagttttctatttgcctccagtgcagtccatttcctaatgtataccagcctactgtactatttaaa atgctcaatttcagcaccgatggccatgtaaataagatgatttaatgttgattttaatcctgtatataaaataaaaagtcacaatgagttt gggcatatttaatgatgattatggagccttagaggtctttaatcattggttcggctgcttttatgtagtttaggctggaaatggtttcacttgc tctttgactgtcagcaagactgaagatggcttttcctggacagctagaaacacaaaatcttgtaggtcattgcacctatctcagccat aggtgcagtttgcttctacatgatgctaaaggctgcgaatgggatcctgatggaactaaggactccaatgtcgaactcttctttgctgc |
| **587** | 204811 _s_at | ggggaactgagggcaaggggatatagtgatggggctcagatggactgggaggagggggagggtgatgcattaattaatggctt cgttaattaatgtcatgttgcttgtcgctttctcagtgtgtgtgtgtggtccatgcccactgctggtgccagggtgggtgtccatgtgcacc cggcctggatgccagctgtgtccttcgggggcgtgcgtgtaactgtagtgtagtcaggtgctcaatggagaatataaacatatacag aaaaatatatattttaagtttaaaaaacagaaaaacagacaaaacaatccccatcaggtagctgtctaacccccagctgggtcta atccttctcattacccacccgacctggctgcccctcaccttggctgggggactgggggccatttcctttctctgcccttttttttgttgttc tattttgtacagacaagttggaaaaacaacagcgacaaaaaagtcaagaaactttgtaaaatatcgtgtgtgtgattccttgt |
| **588** | 205479 _s_at | cccgaccggtgggcatttgtgaggcccatggttgagaaatgaataatttcccaattaggaagtgtaagcagctgaggtctcttgagg gagcttagccaatgtgggagcagcggtttggggagcagagacactaacgacttcagggcagggctctgatattccatgaatgtat caggaaatatatatgtgtgtgtatgtttgcacacttgttgtgtgggctgtgagtgtaagtgtgagtaagagctggtgtctgattgttaagtc taaatatttccttaaactgtgtggactgtgatgccacacagagtggtctttctggagaggttataggtcactcctggggcctcttgggtcc cccacgtgacagtgcctgggaatgtacttattctgcagcatgacctgtgaccagcactgtctcagtttcactttcacatagatgtcccttt cttggccagttatcccttccttttagcctagttcatccaatcctcactgggtgggg |
| **589** | 205765 _at | caccacctacctatgatgccgtggtacagatggagtaccttgacatggtggtgaatgaaacactcagattattcccagttgctattag acttgagaggacttgcaagaaagatgttgaaatcaatggggtattcattcccaaagggtcaatggtggtgattccaacttatgctcttc accatgacccaaagtactggacagagcctgaggagttccgccctgaaaggttcagtaagaagaaggacagcatagatccttac atatacacaccctttggaactggacccagaaactgcattggcatgaggtttgctctcatgaacatgaaacttgctctaatcagagtcc ttcagaacttctccttcaaaccttgtaaagaaacacagatccccttgaaattagacacgcaaggacttcttcaaccagaaaaaccc attgttctaaaggtggattcaagagatggaaccctaagtggagaatgagttattctaaggacttctactttggtcttcaagaaagctgt gccccagaacaccagagatttcaacttagtca |
| **590** | 205828 _at | gaaaatcgatgcagccatttctgataaggaaaagaacaaaacatatttcttgtagaggacaaatactggagatttgatgagaaga gaaattccatggagccaggctttcccaagcaaatagctgaagactttccagggattgactcaaagattgatgctgtttttgaagaattt gggttcttttatttctttactggatcttcacagttggagtttgacccaaatgcaaagaaagtgacacacactttgaagagtaacagctgg cttaattgttgaaagagatatgtagaaggcacaatatgggcactttaaatgaagctaataattcttcacctaagtctctgtgaattgaa atgttcgttttctcctgcctgtgctgtgactcgagtcacactcaagggaacttgagcgtgaatctgtatcttgccggtcatttttatgttatta cagggcattcaaatggctgctgcttagcttgcaccttgtcacatagagtgatctttcccaagagaaggggaagcactcgtgtgcaa cagac |
| **591** | 205927 _s_at | tccacacacggccaggcctgtttatctacactgctgcccactcctctctccagctccacatgctgtacctggatcattctgaagcaaat tccgagcattacatcattttgtccataaatatttctaacatccttaaatatacaatcggaattcaagcatctcccattgtcccacaaatgtt tggctgtttttgtagttggattgtttgtattaggattcaagcaaggcccatatattgcatttatttgaaatgtctgtaagtctctttccatctaca gagtttagcacatttgaacgttgctggttgaaatcccgaggtgtcatttgacatggttctctgaacttatctttcctataaaatggtagttag atctggaggtctgatttgtggcaaaaatacttcctaggtggtgctgggtacttcttgttgcatcctgtcaggaggcagataatgctggtg cctctctattggtaatgttaagactgctgggtgggtttggagttcttggc |
| **592** | 207173 _x_at | gaacatccaagtctttcttctttttaagttgtcaaagaagcttccacaaaattagaaaggacaacagttctgagctgtaatttcgcctta aactctggacactctatatgtagtgcatttttaaacttgaaatatataatattcagccagcttaaacccatacaatgtatgtacaataca atgtacaattatgtctcttgagcatcaatcttgttactgctgattcttgtaaatctttttgcttctactttcatcttaaactaatacgtgccagata taactgtcttgtttcagtgagagacgccctatttctatgtcatttttaatgtatctatttgtacaattttaaagttcttattttagtatacatataaa tatcagtattctgacatgtaagaaaatgttacggcatcacacttatattta |
| **593** | 207191 _s_at | aatctcttctagagcacctgctatccccaacttctagacctgctccaaactagtgactaggatagaatttgatcccctaactcactgtct gcggtgctcattgctgctaacagcattgcctgtgctctcctctcaggggcagcatgctaacggggcgacgtcctaatccaactggga |
| | | gaagcctcagtggtggaattccaggcactgtgactgtcaagctggcaagggccaggattgggggaatggagctggggcttagct gggaggtggtctgaagcagacagggaatgggagaggaggatgggaagtagacagtggctggtatggctctgaggctccctgg ggcctgctcaagctcctcctgctccttgctgttttctgatgatttgggggcttgggagtccctttgtcctcatctgagactgaaatgtgggg atccaggatggcttccttcctcttacccttcctccctcagcctgcaacctctatcctggaacctgtcctccctttctccccaactatgcatct gttgtctgctcct |
| **594** | 208063 _s_at | aagaagctaagcctgatctcctgtaaaaacatcatttccctgatggacaccagcggcaatggaagctggagtttgatgaattcaa agtgttctgggacaagctgaagcagtggattaaccttttccttcggtttgatgctgacaagtccggcaccatgtctacctatgaactac ggactgcactgaaagctgcaggctttcagctgagcagccacctcctgcagctgattgtgctcaggtatgcggatgaggagctcca gctggacttcgatgacttcctcaactgcctggtccggctggagaatgcgagccgggtgttccaggctctcagtacaaagaacaag gagttcattcatctcaatataaatgagttcatccatttgacaatgaacatctgaggctgccttgtagagatgcagcctgcccagctgaa tcttggcttctggaccttgaccttcagaacttctcttggtgtggaaccattacgcccagggttcactc |
| **595** | 208782 _at | actcccaagagcaaatccacattcctcttgagttctgcagcttctgtgtaaatagggcagctgtcgtctatgccgtagaatcacatgat ctgaggaccattcatggaagctgctaaatagcctagtctggggagtcttccataaagttttgcatggagcaaacaaacaggattaa actaggtttggttccttcagccctctaaaagcatagggcttagcctgcaggcttccttgggctttctctgtgtgtgtagttttgtaaacacta tagcatctgttaagatccagtgtccatggaaacattcccacatgccgtgactctggactatatcagtttttggaaagcagggttcctctg cctgctaacaagcccacgtggaccagtctgaatgtctttcctttacacctatgtttttaagtagtcaaacttcaagaaacaatctaaac aagtttctgttgcatatgtgtttgtgaacttgtatttgtatttagtaggcttctatattgcatttaacttgtttttgtaactcctgattcttcct |
| **596** | 208850 _s_at | gaagcctcaagttccagtgcagagatcctacttctctgagtcagctgacccccctccccgcaatccctcaaaccttgaggagaagtg gggaccccacccctcatcaggagttccagtgctgcatgcgattatctacccacgtccacgcggccacctcaccctctccgcacacc tctggctgtctttttgtactttttgttccagagctgcttctgtctggtttatttaggttttatccttcctttctttgagagttcgtgaagagggaagc caggattggggacctgatggagagtgagagcatgtgaggggtagtggatggtggggtaccagccactggaggggtcatccttg cccatcgggaccagaaacctgggagagacttggatgaggagtggttggctgtgcctgggcctagcacggacatggtctgtcctg acagcactcctcggcaggcatggctggtgcctg |
| **597** | 208851 _s_at | aaagcagcgctagtggagggttggagaaggaggtaaggatgagggttcatcatccctccctgcctaaggaagctaaaagcatg gccctgctgcccctccctgcctccacccacagtggagagggctacaaaggaggacaagaccctctcaggctgtcccaagctccc aagagcttccagagctctgacccacagcctccaagtcaggtggggtggagtcccagagctgcacagggtttggcccaagtttcta agggaggcacttcctcccctcgcccatcagtgccagcccctgctggctggtgcctgagcccctcagacagcccccctgccccgcag gcctgccttctcagggacttctgcggggcctgaggcaagccatggagtgagacccaggagccggacacttctcaggaaatggctt ttcccaacccccagcccccacccggtggttcttcctgttctgtgactgtgtatag |
| **598** | 208937 _s_at | ttcagccagtcgccaagaatcatgaaagtcgccagtggcagcaccgccaccgccgccgcggccccacgtgcgcgctgaagg ccggcaagacagcgagcggtgcgggcgaggtggtgcgctgtctgtctgagcagagcgtggccatctcgcgctgccggggcgcc ggggcgcgcctgcctgccctgctggacgagcagcaggtaaacgtgctgctctacgacatgaacggctgttactcacgcctcaag gagctggtgcccaccctgccccagaaccgcaaggtgagcaaggtggagattctccagcacgtcatcgactacatcagggacctt cagttggagctgaactcggaatccgaagttggaacccccgggggccgagggctgccggtccgggctccgctcagcaccctcaa cggcgagatcagcgcc |
| **599** | 209218 _at | gattccctgcatcaactaagaaaagcctgttttcttatttcaaacttggtggcgaatgtgttgcgggtcctgttgggctgctttctgtattgt ctcctaaccctctagttttaattggacacttctttgctgttgcaatctatgccgtgtatttttgctttaagtcagaaccttggattacaaacct cgagcccttctcagtagtggtgctgtattgtacaaagcgtgttctgtaatatttcctctaatttactcagaaatgaagtatatggttcattaa gcttaaaggggaaccattgtgaatgaatatttggaacttaccaagtcctaagagacttttggaagaggatatatatagcatagtacc ataccacttata |
| **600** | 209395 _at | gctgtggggatagtgaggcatcgcaatgtaagactcgggattagtacacacttgttgatgattaatggaaatgtttacagatccccaa gcctggcaagggaatttcttcaactccctgccccctagccctccttatcaaaggacaccattttggcaagctctatcaccaaggagc caaacatcctacaagacacagtgaccatactaattataccccctgcaaagccagcttgaaaccttcacttaggaacgtaatcgtgt cccctatc |
| **601** | 209396 _s_at | tcaccaatgccatcaaggatgcactcgctgcaacgtagccctctgttctgcacacagcacgggggccaaggatgccccgtcccc ctctggctccagctggccgggagcctgatcacctgccctgctgagtcccaggctgagcctcagtctccctcccttggggcctatgca gaggtccacaacacacagatttgagctcagccctggtgggcagagaggtagggatggggctgtggggatagtgaggcatcgca atgtaagactcgggattagtacacacttgttgatgattaatggaaatgtttacagatccccaagcctggcaagggaatttcttcaactc cctgccccctagccctccttatcaaaggacaccattttggcaagctctatcaccaaggagccaaacatcctacaagacacagtga ccatactaattataccccctgcaaagccagcttgaaaccttcact |
| **602** | 209596 _at | tccaacctccttcaaattcagtcaccactgttatattaccttctccaggaaccctccagtggggaaggctgcgatattagatttccttgta tgcaaagtttttgttgaaagctgtgctcagaggaggtgagaggagaggaaggagaaaactgcatcataactttacagaattgaatc tagagtcttccccgaaaagcccagaaacttctctgcagtatctggcttgtccatctggtctaaggtggctgcttcttccccagccatga gtcagtttgtgcccatgaataatacacgacctgttatttccatgactgctttactgtattttt |
| **603** | 209875 _s_at | gaatggtgcatacaaggccatccccgttgcccaggacctgaacgcgccttctgattggacagccgtggaaggacagttatga aacgagtcagctggatgaccagagtgctgaaacccacagccacaagcagtccagattatataagcggaaagctaatgatgag agcaatgagcattccgatgtgattgatagtcaggaactttccaaagtcagccgtgaattccacagccatgaatttcacagccatgaa |
| | | gatatgctggttgtagaccccaaaagtaaggaagaagataaacacctgaaatttcgtatttctcatgaattagatagtgcatcttctga ggtcaattaaaggagaaaaaatacaatttctcactttgcatttagtcaaaagaaaaaatgctttatagcaaatgaaagagaaca tgaaatgcttctttctcagtttattggttgaatgtgtatctatttgagtctggaaataactgatgtgtttgataattagtttagtttgtggcttcatg gaa |
| **604** | 209955 _s_at | acagctttccaaggtgacaaactcctctatgcagtgtatcgaaagctgggtgtttatgaagttgaagaccagattacagctgtcaga aaattcatagaaatgggtttcattgatgaaaaaagaatagccatatgggctggtcctatggaggatacgtttcatcactggcccttg catctggaactggtcttttcaaatgtggtatagcagtggctccagtctccagctgggaatattacgcgtctgtctacacagagagattc atgggtctcccaacaaaggatgataatcttgagcactataagaattcaactgtgatggcaagagcagaatatttcagaaatgtaga ctatcttctcatccacggaacagcagatgataatgtgcactttcagaactcagcacagattgctaaagctctggttaatgcacaagtg gatttccaggcaatgtggtactctgaccagaaccacggcttatccggcctgtccacgaaccacttatacacccacatgacccacttc ctaaagcagtg |
| **605** | 210095 _s_at | tcttttgtcctccttagcacaatgtaaaaagaatagtaatatcagaacaggaaggaggaatggcttgctggggagcccatccagg acactgggagcacatagagattcacccatgtttgttgaacttagagtcattctcatgcttttctttataattcacacatatatgcagagaa gatatgttcttgttaacattgtatacaacatagccccaaatatagtaagatctatactagataatcctagatgaaatgttagagatgcta tatgatacaactgtggccatgactgaggaaaggagctcacgcccagagactgggctgctctcccggaggccaaacccaagaa ggtctggcaaagtcaggctcagggagactctgccctgctgcagacctcggtgtggacacacgctgcatagagctctccttgaaaa cagagg[?][?]gtctcaagacattctgcctacctattagc |
| **606** | 210511 _s_at | aaaggagcagtcgcacagacctttcctcatgctgcaggcccggcagtctgaagaccaccctcatcgccggcgtcggcggggctt ggagtgtgatggcaaggtcaacatctgctgtaagaaacagttctttgtcagtttcaaggacatcggctggaatgactggatcattgct ccctctggctatcatgccaactactgcgagggtgagtgcccgagccatatagcaggcacgtccgggtcctcactgtccttccactca acagtcatcaaccactaccgcatgcggggccatagcccctttgccaacctcaaatcgtgctgtgtgcccaccaagctgagaccca tgtccatgttgtactatgatgatggtcaaaacatcatcaaaaaggacattcagaacatgatcgtggaggagtgtgggtgctcataga gttgcccagc |
| **607** | 211571 _s_at | acaagcatcctgtctcacgaagaacaaatgtttgttaatcgtgtgggccatgattatcagtggataggcctcaatgacaagatgtttg agcatgacttccgttggactgatggcagcacactgcaatacgagaattggagacccaaccagccagacagcttcttttctgctgga gaagactgtgttgtaatcatttggcatgagaatggccagtggaatgatgttccctgcaattaccatctcacctatacgtgcaagaaag gaacagttgcttgcggccagccccctgttgtagaaaatgccaagacctttggaaagatgaaacctcgttatgaaatcaactccctg attagataccactgcaaagatggtttcattcaacgtcaccttccaactatccggtgcttaggaaatggaagatggctatacctaaaa ttacctgcatgaacccatctgcataccaaaggacttattctatgaaatactttaaaaattcctcatcagcaaaggacaattcaataaat acatccaaacatgatcatcgttggagccggaggtgg |
| **608** | 211966 _at | tggtgatgtctgctactatgccagccggaacgacaagtcctactggctctctaccactgcgccgctgcccatgatgcccgtggccga ggacgagatcaagccctacatcagccgctgttctgtgtgtgaggccccggccatcgccatcgcggtccacagtcaggatgtctcca tcccacactgcccagctgggtggcggagtttgtggatcggatattccttcctcatgcacacggcggcgggagacgaaggcggtgg ccaatcactggtgtcaccgggcagctgtctagaggacttccgcgccacaccattcatcgaatgcaatggaggccgcggcacctgc cactactacgccaacaagtacagcttctggctgaccaccattcccgagcagagcttccagggctcgccctccgccgacacgctca aggccggcctcatccgcacacacatcagccgctgccaggtgtgcatgaagaacctgtgagccggcgcgtgccaggaagggcc attttggtgcttattcttaacttattacctcaggtgccaacccaaaa |
| **609** | 211980 _at | gaaagactgtgctgtcctttaacataggtttttaaagactaggatattgaatgtgaaacatccgttttcattgttcacttctaaaccaaaa attatgtgttgccaaaaccaaacccaggttcatgaatatggtgtctattatagtgaaacatgtactttgagcttattgtttttattctgtatta aatattttcagggttttaaacactaatcacaaactgaatgacttgacttcaaaagcaacaaccttaaaggccgtcatttcattagtattc ctcattctgcatcctggcttgaaaaacagctctgttgaatcacagtatcagtattttcacacgtaagcacattcgggccatttccgtggtt tctcatgagctgtgttcacagacctcagcagggcatcgcatggaccgcaggagggcagattcggaccact |
| **610** | 211981 _at | tcggctactcttttgtgatgcacaccagcgctggtgcagaaggctctggccaagccctggcgtcccccggctcctgcctggaggagt ttagaagtgcgccattcatcgagtgtcacggccgtgggacctgcaattactacgcaaacgcttacagcttttggctcgccaccatag agaggagcgagatgttcaagaagcctacgccgtccaccttgaaggcaggggagctgcgcacgcacgtcagccgctgccaagt ctgtatgagaagaacataatgaagcctgactcagctaatgtcacaacatggtgctacttcttcttcttttgttaacagcaacgaaccct agaaatatatcctgtgtacctcactgtccaatatgaaaaccgtaaagtgccttataggaatttgcgtaactaacacaccctgc |
| **611** | 212344 _at | ggaaaacacctcatttgaccttgccagctgaccttcaaaccctgcatttgaaccgaccaacattaagtccagagagtaaacttgaat ggaataacgacattccagaagttaatcatttgaattctgaacactggagaaaaaccgaaaaatggacggggcatgaagagact aatcatctggnaaaccgatttcagtggcgatggcatgacagagctagagctcgggcccagccccaggctgcagcccattcgcag gcacccgaaagaacttccccagtatggtggtcctggaaaggacattttgaagatcaactatatcttcctgtgcattccgatggaattt cagttcatcagatgttcaccatggccaccgcagaacaccgaagtaattccagcatagcggggaagatgttgaccaaggtggaga agaatcacgaaaaggagaagtcacagcacctagaaggcagcgcctcctcttcactctcctctgattagatgaaactgttaccttac ccta |
| **612** | 212353 _at | aatatccttgttgtgtattaggtttttaaataccagctaaaggattacctcactgagtcatcagtaccctcctattcagctccccaagatg atgtgtttttgcttaccctaagagaggtttcttcttatttttagataattcaagtgcttagataaattatgttttctttaagtgtttatggtaaactc ttttaaagaaaatttaatatgttatagctgaatctttttggtaactttaaatctttatcatagactctgtacatatgttcaaattagctgcttgcc |
| | | tgatgtgtgtatcatcggtgggatgacagaacaaacatatttatgatcatgaataatgtgctttgtaaaaagatttcaagttattaggaa gcatactctgtttttttaatca |
| **613** | 212354 _at | gtgtgcacacggagactcatcgttataatttactatctgccaagagtagaagaaaggctggggatattgggttggcttggttttgatt ttttgcttgtttgtttgttttgtactaaaacagtattatcttttgaatatcgtagggacataagtatatacatgttatccaatcaagatggctag aatggtgcctttctgagtgtctaaaacttgacacccctggtaaatctttcaacacacttccactgcctgcgtaatgaagttttgattcatttt taaccactggaatttttcaatgccgtcattttcagttagatgattttgcactttgagattaaaatgccatgtctatttgattagtcttattttttatt tttacaggcttatcagtctcactgttggctgtcattgtgacaaagtcaaataaacccccaaggacgacacacagtatggatcacatat tgtttgacattaagcttttgccagaaaatgttgcatgtgttttacctcgactt |
| **614** | 212488 _at | gaaaggcgatctcttcactgtgaaaagttgcccgggtgcagcgcctttttccttctaccatggaaatgcaggctgggcccttggggt gagcctgcggggctctggtgctgtccccgacccccaccaccaccagaatgcagttccagcttaggaagccacaaacaagccac ccaggaggaacaaaacaccgccagcgtggattttccaaatttccctggaaagtaagtctcgctcttgccaaagaaaagtctggctt ggagagtctctggagcccaggatgccagcatgtgccaatgactgtcaccttcatctcttcaaaagaaaagccatagccgaggact gtcccgcgacccccgtggactgcgtctaggtcatgtgattctgttttcatttctcatcccatccaattgtccttttctcctgtcattttcttcctc tgtggtcccttcaaagttgttataatttgtactgaacttcaaaatgtgtcccgttctccccagaccactctagccacagtatatt |
| **615** | 212489 _at | attgaaaagcaggtaccagtgccccttttcagacagtttttgattcgctctagacnttttttnttttttaatagggagggaaaaaatttgat aattttcttntttctacatgcacttaagactaaaacacaggtttggattaatttattgcttcctttttccgcttttcttcccgcagagcctgatg ggagaatgtccagggcagggaaaccacatttttgtaggtgataactcaatgaaaattggtgcttatttttacacttctctcttgtggctc tcttgtggtgctatctgttttaaggtctccttgaaggcgcactggggtccctggccatgcctcgttctccctgctttctttatcctgttattgcct ccacagtctgttgccaaggactctaagatcaatgcacgtcactttcctttccactgggcaggatagccaagcacactccctcctgcg ctctcccgccccggtgcgtccactcccgagggctgttatgaggactgggttgtgcctacttgatttgaaaacacacac |
| **616** | 213106 _at | acttctcagcaaataaatctcccttaagtaggaaanctagatttcatattngcttnctttgaattaacagcaactttccacaggtaaatct gttcttgcaaagatgtgagcagaatagttaaaaataatatttttatgtttcatggttctaaatggaagccataaatgcagtaaatactatc tgttgtttaactactttaatcgtcatttttttacattttcaagtttattaggttaagaaaaacagggcagccttggaaggcagctactacaga aaactgcagttttgcgttaaagataaagtagtattttcagctccctgaaaaaccattcctgctgaaactgctgtagaaattgtgaagct gcatgagtggagagtattgaatctgtggttatagtagttttctcaggtttgtttatcttgatgtttgatgcactgtgttttatagttattaaaattg agtaatattatttctatgcagtgttatgtgtcattggccttttgtgaatgtgcatgttttaaactgcaaattttaaacattttgtcctctaattgtta t |
| **617** | 213125 _at | gaatgagtattcctatacgacccagatagactacaaccccaaggaccgcctgctctatgcctgggacaatggccaccaggtcact taccatgtcatctttgcctactgacacccttgtccccacaagcagaagcacagaggggtcactagcaccttgtgtgtatgtgtgtgcg cgcacgtgtgtgtaggtgggtatgtgttgtttaaaaatatatattattttgtataatattgcaaatgtaaaatgacaatttgggtctattttttta tatggattgtagatcaatccatacgtgtatgtgctggtctcatcctccccagtttatatttttgtgcaatgaacttctcctttgaccagtaa ccaccttccttcaagccttcagcccctccagctccaagtctcagatctcgaccattgaaaaggtttcttcatctgggtcttgcaggagg caggcaacac |
| **618** | 213524 _s_at | gtgctcggcctgatggagactgtgtgcagccccttcacggccgccagacgtctgcgggaccaggaggcagccgtggcggagct gcaggccgccctggagcgacaggctctccagaagcaagccctgcaggagaaaggcaagcagcaggacacggtcctcggcg gccgggccctgtccaaccggcagcacgcctcctaggaactgtgggagaccagcggagtgggagggagacgcagtagacag agacagaccgagaaggaagggagagacagagggggcgcgcgcacaggagcctgactccgctgggagagtgcaggagca cgtgctgtttttatttggacttaacttcagagaaaccgctgacatctagaactgacctaccacaagcatccaccaaaggagtttggg attgagtttntgctgctgtgcagcactgcattgtcatgacatttccaacactgtgtgaattatctaaatgcgtctaccattttgcactaggg ag |
| **619** | 213869 _x_at | cacagcctccaagtcaggtggggtggagtcccagagctgcacagggtttggcccaagtttctaagggaggcacttcctcccntcg cccatcagtgccagcccctgctggctggtgcctgagcccctcagacagcccccctgccccgcaggcctgccttctcaggacttctg cggggcctgaggcaagccatggagtgagacccaggagccggacacttctcaggaaatggcttttcccaacccccagcccncca cccggtggttcttcctgttctgtgactgtgtatagtgccaccacagcttatggcatctcattgaggacaaagaaaactgcacaataaa accaagcctctggaatctgtcctcgtgtccacctggccttcgctcctccancagtgcctgcctgnccccncttcgctggggtctccac gggtgaggctggggaacgccacctcttcctcttccctgacttctccccaaccacttagtagcaacgctaccccaggggctaatgact gcacactgggcttcttttcagaatgaccctaacgagacacatttgcccaaa |
| **620** | 213905 _x_at | cacaaaccccaggacagcggtctccccagcctgccctgctcangccttgcccccaaacctgtactgtcccggaggaggttgg gaggtggaggcccagcatcccgcgcagatgacaccggttttcctagaagcccctcacccccactggcccactggtggctaggtct ccccttatccttctggtccagcgcaaggaggggctgcttctgaggtcggtggctgtctttccattaaagaaacaccgtg |
| **621** | 214234 _s_at | gaatgaagaaaagtcgcctcaacgacaaacaaaagcaccgactagatttccttcagctgatgattgactcccagaattcgaaag aaactgagtcccacaaagctctgtctgatctggagctcgcagcccagtcaataatcttcatttttgctggctatgaaaccaccagcag tgttctttccttcactttatatgaactggccactcaccctgatgtccagcagaaactgcaaaaggagattgatgcagttttgcccaata |
| **622** | 214235 _at | ggtgaggggatgacccctggagatgaagggaagaggtgaagccttagcaaaaatgcctcctcaccactccccaggagaattttt ataaaaagcataatcactgattccttcactgacataatgtaggaagcctctgaggagaaaaacaaagggagaaacatagagaa cggttgctactggcagaagcataagatctttgtacaatattgctggccctggttcacctgtttactgttatcacaata |
| **623** | 214247 | acttaggtaattgtagggcgaggattataaatgaaatttgcaaaatcacttagcagcaactgaagacaattatcaaccacgtggag |
| | _s_at | aaaatcaaaccgagcagggctgtgtgaaacatggttgtaatatgcgactgcgaacactgaactctacgccactccacaaatgatg ttttcaggtgtcatggactgttgccaccatgtattcatccagagttcttaaagtttaaagttgcacatgattgtataagcatgctttctttgag ttttaaat |
| **624** | 215646 _s_at | tctcacctatacgtgcaagaaaggaacagttgcttgcggccanccccctgttgtagaaaatgccaagacctttggaaagatgaaac ctcgttatgaaatcaactccctgattagataccacntgcaaagatggtttcattcaacgtcaccttccaactatccggtgcttaggaaa tggaagatgggctatacctaaaattacctgcatgaacc |
| **625** | 217430 _x_at | agggcctaagggtgacagaggtgatgctggtcccaaaggtgctgatggctctcctggcaaagatggcgtccgtggtctgaccggc cccattggtcctcctggccctgctggtgcccctggtgacaaggggggaccccattcccgaggagctttatgag |
| **626** | 217763 _s_at | aacattgtaatggccatcgctggaaacaagtgcgacctctcagatattagggaggttcccctgaaggatgctaaggaatacgctg aatccataggtgccatcgtggttgagacaagtgcaaaaaatgctattaatatcgaagagctctttcaaggaatcagccgccagatc ccacccttggacccccatgaaaatggaaacaatggaacaatcaaagttgagaagccaaccatgcaagccagccgccggtgct gttgacccaagggcgtggtccacggtacttgaagaagccagagcccacatcctgtgcactgctgaaggaccctacgctcggtgg cctggcacctcactttgagaagagtgagcacactggctttgcatcctggaaggcctgcaggggggcggggcaggaaatgtacctg aaaaggattttagaaaaccctgggaaacccaccacaccaccacaaaatggcctttagtgt |
| **627** | 218211 _s_at | gccacaccttcgcgaaacctgtggtggcccaccagtcctaacgggacaggacagagagacagagcagccctgcactgttttccc tccaccacagccatcctgtccctcattggctctgtgctttccactatacacagtcaccgtcccaatgagaaacaagaaggagcacc ctccacatggactcccacctgcaagtggacagcgacattcagtcctgcactgctcacctgggtttactgatgactcctggctgcccc accatcctctctgatctgtgagaaacagctaagctgctgtgacttccctttaggacaatgttgtgtaaatctttgaaggacacaccgaa gacctttatactgtgatcttttacccctttcactcttggctttcttatgttgc |
| **628** | 218638 _s_at | ctgccccgagctcgaagaagaggctgagtgcgtccctgataactgcgtctaagaccagagccccgcagcccctggggcccccg gagccatggggtgtcgggggctcctgtgcaggctcatgctgcaggcggccgaggcacagggggtttcgcgctgctcctgaccgc ggtgaggccgcgccgaccatctctgcactgaagggccctctggtggccggcacgggcattgggaaacagcctcctcctttcccaa ccttgcttcttagggggcccccgtgtcccgtctgctctcagcctcctcctcctgcaggataaagtcatccccaaggctccagctactcta aattatggtctccttataagttattgctgctccaggagattgtccttcatcgtccaggggcctggctcccacgtggttgcagatacctcag acctggtgctctaggctgtgctgagcccactctcccgagggcgcatccaagcgggggccacttgagaagtgaataaatggggcg gtttcggaagcgtcagtgtttccatgttatgg |
| **629** | 219955 _at | gaagttgcaacattcgtttgataggaattccagaaaaggagagttatgagaatagggcagaggacataattaaagaaataattga tgaaaactttgcagaactaaagaaaggttcaagtcttgagattgtcagtgcttgtcgagtacctagtaaaattgatgaaaagagact gactcctagacacatcttggtgaaattttggaattctagtgataaagagaaaataataagggcttctagagagagaagagaaatta cctaccaaggaacaagaatcaggttgacagcagacttatcactggacacactggatgctagaagtaaatggagcaatgtcttca aagttctgctggaaaaaggctttaatcctagaatcctatatccagccaaaatggcatttgattttaggggcaaaacaaaggtatttctt agtattgaagaatttagagattatgttttgcatatgcccaccttgagagaattactggggaataatataccttagcacgccagggtgac taca |
| **630** | 221011 _s_at | gagtggttcatccatactctcattccctcgcctccccttgtggacgggggtcttgccttttcaattcctgtgtttggtgtcttcccttatctgct accctgaatcacctgtcctggtcttgctgtgtgatggaacatgcttgtaaactgcgtaacaaatctactttgtgtatgtgtctgtttatgg gggtggtttattatttttgctggtccctagaccactttgtatgaccgtttgcagtctgagcaggccaggggctgacagctaatgtcagga ccctcagcggtggagcctgctgggggggacccagctgctcttggacaagtggctgagctcctatctggcctcctcttttttttttttcaag taatttgtgtgtatttctaactgattgtattgaaaaaattcctagtatttcagtaaaaatgcctgttgtgagatgaacctcctgtaacttctatc tgttctttttttgaggctcaggga |
| **631** | 221729 _at | tggaattagaccatttggcctttgaactttcataggaaaaatgacccaacatttcttagcatgagctacctcatctctagaagctgggat ggacttactattcttgtttatatttagatactgaaaggtgctatgcttctgttattattccaagactggagataggcagggctaaaaggt attattatttttcctttaatgatggtgctaaaattcttcctataaaattccttaaaaataaagatggtttaatcactaccattgtgaaaacata actgttagacttcccgtttctgaaagaaagagcatcgttccaatgcttgttcactgttcctctgtcatactgtatctggaatgctttgtaata cttgcatgcttcttagaccagaacatgtaggtccccttgtgtctcaatactttttttttcttaattgcatttgttggctctatttaattt |
| **632** | 221730 _at | tagattccggtatatcgttcttcaagacacttgctctaagcggaatggaaatgtgggcaagactgtctttgaatatagaacacagaat gtggcacgcttgcccatcatagatcttgctcctgtggatgttggcggcacagaccaggaattcggcgttgaaattgggccagtttgtttt gtgtaaagtaagccaagacacatcgacaatgagcaccaccatcaatgaccaccgccattcacaagaactttgactgtttgaagtt gatcctgagactcttgaagtaatggctgatcctgcatcagcattgtatatatggtcttaagtgcctggcctccttatccttcagaatatttat tttacttacaatcctcaagttttaattgatttaaatatttttcaatacaacagtttaggtttaagatgaccaatgacaatgaccaccttt |
| **633** | 221731 _x_at | tttcagcaccgatggccatgtaaataagatgatttaatgttgattttaatcctgtatataaantaaaaagtncncaatgagtttngggca tatttaatgatgattatggagccttagaggtctttaatcattggttcnggctgcttttatgtagtttaggctggaaatggtttcacttgctctttg actgtcagcaagactgaagatggcttttcctggacagctagaaaacacaaaatcttgtaggtcattgcacctatctcagccataggt gcagtttgcttctacatgatgctaaaggctgcgaatgggatcctgatggaactaaggactccaatgtcgaactcttctttgctgcattcc tttttcttcacttacaagaaaggcctgaatggaggacttttctgtaaccaggaacatttttaggggtcaaagtgctaataattaactcaa ccaggtctactttttaatggcttcataacactaactcataaggttaccgatcaatgcatttcatacggatatagacctagggctctgga gggtgggg |
| **634** | 221874 | ctggcctacgtgttcaattttctatgaacaaaggctttagtccttgacccagggctaaagtggtctgtccaagctgttgttggtagaggg |
| | _at | agtatgataaaatgtttaaatctcatttggttaccttgagtcctggaacacgcagtaactgtcatgctatagtcntcatctgtatttggctg ggaatacaaatgaagattgtggtgtattcaagcagtagggtttttgcttttgtttttgttttagtgccaacaaactttttttttgtctgactacat taaagataagactgactatatttatacaacagaaactttgtaatagatttttttcagctttgtgaaatcgaatttttttttcatcagggctggttg gatttcctttttaccctgtaatccaagcgttaatagtttgttagaagatgggttattgcatgtcactt |
| **635** | 223969 _s_at | gaagatcaaggatgttctcaacagtctagagtacagtccctctcctataagcaagaagctctcgtgtgctagtgtcaaaagccaag gcagaccgtcctcctgccctgctgggatggctgtcactggctgtgcttgtggctatggctgtggttcgtgggatgttcagctggaaacc acctgccactgccagtgcagtgtggtggactggaccactgcccgctgctgccacctgacctgacagggaggaggctgagaactc agttttgtgaccatgacagtaatgaaaccagggtcccaaccaagaaatctaactcaaacgtcccacttcatttgttccattcctgattct tgggtaataaa |
| **636** | 223970 _at | ggagctcagagatctaagctgctttccatcttttctcccagccccaggacactgactctgtacaggatggggccgtcctcttgcctcctt ctcatcctaatccccccttctccagctgatcaacccggggagtactcagtgttccttagactccgttatggataagaagatcaaggatgt tctcaacagtctagagtacagtccctctcctataagcaagaagctctcgtgtgctagtgtcaaaagccaaggcagaccgtcctcctg ccctgctgggatggctgtcactggctgtgcttgtggctatggctgtggttcgtgggatgttcagctggaaaccacctgccactgccagt gcagtgtggtggactggaccactgcccgctgctgccacctgacctgacagggaggaggctgagaactcagttttgtgaccatgac agtaatgaaaccagggtcccaaccaagaaatctaactcaaacgtcccact |
| **637** | 224724 _at | gcctgagaaagcaagcacgcactctcagtcaacatgacagattctggaggataaccagcaggagcagagataacttcaggaa gtccatttttgcccctgcttttgctttggattatacctcaccagctgcacaaaatgcatttttcgtatcaaaagtcaccactaaccctccc ccagaagctcacaaaggaaaacggagagagcgagcgagagagatttccttggaaatttctcccaagggcgaaagtcattgga atttttaaatcataggggaaaagcagtcctgttctaaatcctcttattcttttggtttgtcacaaagaaggaactaagaagcaggacag aggcaacgtggagaggctgaaaacagtgcagagacgtttgacaatgagtcagtagcacaaaagagatgacatttacctagcac tataaaccctggttgcctctgaagaaactgccttcattgtatatatgtgactatttacatgtaatcaacatggaacttttaggggaacct aataagaaatcccaattttcaggagtggtggtgtcaataaacgctctgtggccagtg |
| **638** | 225664 _at | ggaacccagagctgctgtgtatttcgagcgggcagtttatcttttgctatacttattttcaattcaattacaccacgattcaaataattccc ctcctaaaaccaaaaaggagggaaacgtcaactccattgcaattacttatcttcctcttctatctctgttatacgccggggcatagaat gctcgtatacatctctttaacaaccacaaaccttaagccatgtagatgaagttagtgcatcaacggatacagttccatattgccttaa acctccttgttttagacacactaacatttataccaaattgcagattattctgcagagagggaattgcatgtttgtgttgta |
| **639** | 225681 _at | aattaatattcatcgcacttcttctgtggaaggactttgtgaaggaattggtgctggattagtggatgttgctatctgggttggcacttgttc agattacccaaaaggagatgcttctactggatggaattcagtttctcgcatcattattgaagaactaccaaaataaatgctttaattttc atttgctacctctttttttattatgccttggaatggttcacttaaatgacattttaaataagtttatgtatacatctgaatgaaaagcaaagct aaatatgtttacagaccaaagtgtgatttcacactgtttttaaatctagcattattcatttgcttcaatcaaaagtggtttcaatattttttta gttggttagaatactttcttcatagtcacattctctcaacctataatttggaatattgttgtggtcttttgtttttctcttagtatagcatttttaaaa aaatataaaagctaccaatctttgtacaatttg |
| **640** | 225799 _at | aaatgactggatggtcgctgctttttaagtttcaaattgacattccagacaagcggtgcctgagcccgtgcctgtcttcagatcttcaca gcacagttcctgggaaggtggagccaccagcctctccntgaataactggagatgaaacaggaagctctatgacacacttgatc gaatatgacagacacngaaaatcacgactcanccccctccagcacctctacctgttgcccgccgatcacagccggaatgcagct gaaagattccctggggcctggttccaaccgcccactgtggactctgaggcctctgcatttgcgggtggtctgcctgtgatattttggtca tgggctggtctg |
| **641** | 226237 _at | gaagaggagcaacatctatgccaaatactgtgcattctacaatggtgctaatctcagacctaaatgatactccatttaatttaaaaaa gagttttaaataattatctatgtgcctgtatttccctttttgagtgctgcacaacatgttaacatattagtgtaaaagcagatgaaacaacc acgtgttctaaagtctagggattgtgctataatccctatttagttcaaaattaaccagaattcttccatgtgaaatggaccaaactcatat tattgttatgtaaatacagagttttaatgcagtatgacatcccacaggggaaaagaatgtctgtagtgggtgactgttatcaaatatttta tagaatacaatgaacggtgaacagactggtaacttgtttgagttcccatgacagatttgagacttg |
| **642** | 226248 _s_at | tgaaagtgggcatctctgcaggcacctgtactgccatcctgctcaccgtcttgacctgctacttttggaaaaagaatcaaaanctag agtacangtactccangctggtgatgaatgctactctcaaggactgnnnnntnncngnngctgacagctgcgccatcatggnan ncnnnnangnanngnnnnnnnncancnnnnnncanntgcgccatcatggaaggcgaggatgtagaggacgacctcatcttt accagcaagaagtcactctttgggaagatcaaatcatttacctccaagaggactcctgatggatttgactcagtgccgctgaagac atcctcaggaggcccagacatggacctgtgagaggcactgcctgcctcacctgcctcctcaccttgcatagcacctttgcaagcct gcggcgatttgggtgccagcatcctgcaacacccactgctggaaatctcttcattgtggccttatcagatgtttgaatttcagatctttttt atagagtacccaaaccctcctttctgcttgcctcaaacctgccaaatatacccacac |
| **643** | 226311 _at | aaacgacgcaaatctctgagctggggaccacttggagaaccggcttagtaacagtcctgatcttcgcaagccagcttcttctgcatc tgaggggctcctggcgcccagaggaggcagacagatgtcttctagctgagtttctaaccgcatgatgagactcagaccttccgctg cactagaaaatctgcaacagtgtccctgagtcacttctccttagtgggcagactcgtgttagatttgtggaacccagctctctgatttac tccttttggaaaacccatggaatttcatgtataaggctttcattgtattttaaggtttttctgtttgttttgagtatatacatggtgctcaatagc aacatcttagcagatgaagcagtttatgattccactccctcctgtatgacaggtagccactatactgaatcaaggtgctgaactcaaa tcacaaaattctggcttaccgatacaacaaccaatac |
| **644** | 226777 _at | tataaggtaactctttagtcctccatttagcacattttaaatcctccaaagaataagtatcatgtgattattttagctttacaaaaaaaag ttgaatggcgttttatttcatggcctataagcaggtaccttagtagggcagatataggaaaaacaaattagagcaaaacaaatcctc |
| | | tacaaatccaaggcaggaaaagtggtggcagagtgactcattctcctgtccctcccatcaggtcaaatcaggaggctgcagtgaa tgcctgttctttgaatgtgtagcagttgttncctgtaactctttaaaacttggctataggctgtttagcacagtacagattaaagatacagtt acgtaaacagcaaagtaattttatagtgcttcatccatttatcatgctttggtttgctaatttttcacatacctttttctatcacagtctgttgctt ttgtacacatttctcatattggggttcgaca |
| **645** | 226930 _at | ggtgccaggaaggtcacagatggacactggccattctggtcatctcagtctggaactcagtcccacttcttggcctggacaatgaac aggattcagttttgctgttaactttgcttctctactttttttgtttgtttgtaatagcacatcccagagacatcagaaaccagcaactgattca gtgtgatttccagactttttaggcatgaaattcggacacttcagtatttccaggaatagcatatgcacgctgttcttgcttcatggaatgct acatgctttctgtttttctcattttggatttctccaaaactaactgaatttaagcttcaggtccctttgtatgcagtagaaaggaattattaaa aacaccaccaaagaaaataaatatatcctacttgaaatttactctatggacttacccactgctagaataaatgtatcaaatcttatttgt aaattctcaattttgatatatatatgtatatatgcatatacatatccacacttgtctgcaag |
| **646** | 227140 _at | ttaccctctatttaaatgctttgaaaaacagtgcattgacaatgggttgatatttttcttaaaagaaaaatataattatgaaagccaaga taatctgaagcctgttttattttaaaactttttatgttctgtggttgatgttgtttgtttgtttgtttctatttgttggtttttacttgtttttttgttttgttttgt tttgttttgcatactacatgcagttctttaaccaatgtctgtttggctaatgtaattaaagttgttaatttatatgagtgcatttcaactatgtca atggtttcttaatatttattgtgtagaagtactggtaattttttttatttacaatatgtttaaagagataacagtttgatatgttttcatgtgtttatag cagaagttatttatttctatggcattccagcggatattttggtgtttgcgaggcatgcagtcaatatttttgtacagttagtggacagtattca gcaacgcctgatagcttctttggcctt |
| **647** | 227566 _at | ccacacaccaggcactaatcacctggtgaggatttggcatatccaccaaaaaatgcatccgatttaaccaacatctccaccagcg ctacggactcctcccaattctgacatctcttgcagacaatactatgctctctacacactgtttagaaatggaaaggtgatctgcactgta tcttgggtttgttggctatgcttcctttgatgacatatattatacagtatatatatacatatatttttttgttagagttctagccatttatttctccg cagggtcctttctcagacattactgcatgctgtatatggcgttagctgtgtgttgatcttctaaaagatgatagagtttactggtaattgtgt aatcagctcctgcctttttatt |
| **648** | 227676 _at | caggaaactcttctctgacttggggagttcctacgcganacaactgggcttccgggacagctgggtcttcataggagccaaagac ctcaggggtaaaagcccctttgagcagttcttaaagaacagcccagacacaaacaaatacgagggatggccagagctgctgga gatggagggctgcatgcccccgaagccattttagggtggctgtggctcttcctcagccaggggcctgaagaagctcctgcctgactt aggagtcagagcccggcaggggctgaggaggaggagcaggggggtgctgcgtggaaggtgctgcaggtccttgcacgctgtgt cgcgcctctcctcctcggaaacagaaccctcccacagcacatcctacccggaagaccagcctcagagggtccttctggaaccag ctgtctgtggagagaatggggtgctttcgtcagggactgctgacggctggtcctgaggaaggacaaactgcccagacttgagcc |
| **649** | 227719 _at | cacttgatagactgtaagcacctgcttaactttgtgtcccaaatatttagtgtgtatatatatatatatatanncncacacacacacatat atattcaacaaataaagcaaaatataacatgcatttcacatttgtctttccctgttacgattttaatagcagaactgtatgacaagtttag gtgatcctagcatatgttaaattcaaattaatgtaaaacagattaacaacaacaaagaaactgtctatttgagtgaagtcatgctttct attataataacttggcttcggttatccatcaaatgcacacttatactgttatctgattg |
| **650** | 229218 _at | gccaaaaggcctggcttctgataggaaactggtaagaaactcttcatgaaaacacatcactaatattcgctattactctcctggtctg aagtcagcttttctgaaccattaaggtatttcatcacaagttatattttataatatcagtttaagaggcttttattcatgtgaacaccagnnc cctttcaggggcatggtctttttgaaaaaaaaaaaanaaaaaaannaacagttttagccacatatcagatatttctatatctaattatc ctttatggctaacattctgcctccattgttaaggtataattgttcctgaatttaaaggtggtttggcctctaatttaattctgattcagactctcc tgtcaggactcaagaaaatttaattaattaccaaggattaagtcttctggttaaggtttctgggaaaaaaaaatagcaaagatgttgat ttcttggaatccttttacaggttcataacagaaaaatcttcattccctgtaggcatttaattaaacctagttgagaagtgtgtgggattcct caattatgaacaaaacacgtatattggctttcttt |
| **651** | 229802 _at | gacatgattgtctataatctcgctagccttgtactgtgtgtgcatagcaattacagggaagtaatctagctcctgactattatgttgaact atgtcgctgctttttacaaacttgtcttgatccaaagcagtcacaatgataaccctgcatatctgggaatcataagtcaactatgtatctc tgtgtgtgtatatatatgtatgtatgtatctattttcaaactgtgatttaatatttaaatattcctactgccatttttgtgactgaaaaactacac atgaggaaacgtcttagaattttccaatagaggaaaaataacacttgggcaatctgtcatgtttcacaacagttctcatttttctcatgat ttgtgtagcgtggaatgtgtttgctcaatgtgaagggttttcattgctcaatttctctgtgtaa |
| **652** | 231766 _s_at | ggttccggctaacacattttctaagtcgccagtgctgcttacagtttgaatacatgaaaatcctgtttctnagatgtttgcgcacgtgctta ttaggaaatgagtctgtatggaaatctcaccacagataatggttaacgaaccgggtcgacatcacaaaggagggtggagactcttt ttactaacttgaatgagacaaaagcagtggtgtcagtttataatcctgatgcatttcagtaataatgtagaaaaacattattttaaaaaa gttccaacacacagccatgaggagccnnnnnnnnnntcagtttgaaagaggtgcataataaaactactaaccagaggagtct atgccatttt |
| **653** | 231832 _at | gagtttcaactttaaatgttcactatgtcatttagtgtccanctttacggataggttgactatctaaataggcatttttagtcattaaaaaa antctagtcaccaggaggatccctataactcaaaataacttgttgtaaaagaaaatttgtttacttacccattagtaagttcctgcatat tcattataagatggcaaatcaaacttttctaggatgaagacagcttatttttaagttgtatagtcttagttggtttagggtctcaattttaatta ataaaatacttggtttttatttgcttgtccttttgaattcctgttttaataatttaaaatgagcacaaagaangttgaagttcagattaatctct tctgaatgatgtttttttcctctgtgatgagttgtttctg |
| **654** | 231879 _at | tgctgtagtcactggtgttcctcacccaccagctgtaactcagtttgtgtgaggtacagccacagaagatgtcatgtactgtatattacc tggtgatagttgcttttcacccccgagttcagtttctaggagccaatgaaacttcccctcacctcctcatctttccaagttgttctttgaatt gaggagtttgaaggcataaacagttacttggggatttgcgaaaatcctacttagttactgcgtttacagttctttggcccagtctctgac ccttcccnagtatttgtgcnatgattgtgtttactgctggatttttgaaggtttttttttnnaagaaagtgccattcattatttgatt |
| **655** | 232176 _at | gggatcactgggagaagccatggcattatcttcaggcaatttagtctgtcccaaataaaataaatccttgcatgtaaatcattcaagg gttatagtaatatttcatatactgaaaagtgtctcataggagtcctcttgcacatctaaaaggctgaacatttaagtatcccgaattttct tgaattgctttccctatagattaattacaattggatttcatcatttaaaaccatacttgtatatgtagttataatatgtaaggaatacattgtt tataaccagtatgtacttcaaaaatgtgtattgtcaaacatacctaactttcttgcaataaatgcaaaagaaactggaacttgacaatt ataaatagtaatagtgaagaaaaaatagaaaggttgcaattatataggccatgggtggctcaaaactttgaa |
| **656** | 232458 _at | gattcctggattgatgtcttatctacaggcttctttttaaaaatnnnnnnngattggctgtgagtttaccaggattatagttgaggctaaa ggacagctcctcaggaaagcccctgttctacatctacggtcacatgctggaccttgagttgtcactcagagaaaagagtgccatcta ccgaaactccacagtttccattgtgaatggcttctttggtgcagagttccaaaaattatgtagcccagctctttaattttgtaacatctaat gatatcaccgccttgaagtgattaaagtagattgcttaaagaattaaagctttaaagatgaaagatgttattgcttttgctggacatgag gaacagttgtaaagtttccaggtctacaataactttctggancccctctcagtgaactgtttcttgta |
| **657** | 232481 _s_at | gaagtccatcctttggtccaaagcatctggaagaggaagaagagaggaatgagaaagaaggaagtgatgcaaacatctcca aagaagtcttttggaacaggaaaatcattcaccactcacagggtcaaatatgaaatacaaaaccacgaaccaatcaacagaatt tttatccttccaagatgccagctcattgtacagaaacattttagaaaaagaaagggaacttcagcaactgggaatcacagaatacc taaggaaaaacattgctcagctccagcctgatatggaggcacattatcctggagcccacgaagagctgaagttaatggaaacatt aatgtactcacgtccaaggaaggtattagtggaacagacaaaaaatgagtattttgaacttaaagctaatttacatgctgaacctga ctatttagaagtcctggagcagcaaacatagatggagagtttgagggctttcgcagaaatgctgtgattctgttttaagtccataccttg taaataagtgccttacgtgagtgtgtcatcaatcagaacctaagc |
| **658** | 233555 _s_at | gctgctcaagcgcctgcagaacaacgacacgtgcagcatgccaggcctcacgtgcttcacccacgacaaccagcactggcag acggcgcctttctggacactggggcctttctgtgcctgcaccagcgccaacaataacacgtactggtgcatgaggaccatcaatga gactcacaatttcctcttctgtgaatttgcaactggcttcctagagtactttgatctcaacacagacccctaccagctgatgaatgcagt gaacacactggacagggatgtcctcaaccagctacacgtacagctcatggagctgaggagctgcaagggttacaagcagtgta acccccggactcgaaacatggacctgg |
| **659** | 234994 _at | acaatcggctaaccttgacatttctttttaccttcatatgccactatctcggtagttcaaaaaaatttagttcttgataaattgccttgaagttt accttgtgctggagagccttatgataactccaaagactttcttacggtataatacatgttgtttaggattgtgttcttagtcactgaagata ataaatattaaaatggatgttttcatcagaaaattttcatgttttcctttaaggtaacataattgtaagaattgtttaataaaatactcagga aattctaaaggtttctccnaatacctaaacatttctgaacatcagtattgcagttgtggaagagcagaaggaggatacatttgtttgtgt tgctccccaaaattccaccttgcatttgcatcacaaacttccctcaattgaggcagttttctttg |
| **660** | 235976 _at | cagtgctgctgtgaactaaagtatgtcatttatgctcaaagtttaattcttcttgggatattttaaaaatgctactgagattctgctgtaa atatgactagagaatatattgggtttgctttatttcataggcttaattctttgtaaatctgaatgaccataatagaaatacatttcttgtggca agtaattcacagttgtaaagtaaataggaaaaattatttatttttattgatgtacattgatagatgccataaatcagtagcaaaaggca cttctaaaggtaagtggtttaagttgcctcaanagagggacaatgtagctttattttacaagaaggcatagttagatttctatgaaatatt tattctgtacagttttatatanttttggttcacaaaagtaattattcttgggtgcctttcaa |
| **661** | 236894 _at | aaagtatattgtgctagcttgtctaagaataaacttnnatactgttgggggagggctgcacctgtcaagataacctgtcaatgtagta ggaaaacaggaggggacagtaacagaaaagcacgggaaaagatggcaaggttagttaaaatagaaaagtgctcagttcctc atacctgtaatcccagcagtttagggggccaaggaaggtgggtcacttgagcccaagagttcaaggccatcctgggcaatgtggc gaaagtgtctacaaaaaaatacaaaaagaggaagaaatgatatttcacaagtttgtatcatttgtcat |
| **662** | 237521 _x_at | cagtccaaaatccatagacacagacagtagaaaggtggttatncaagggnctggtaggagagggagttagtatttagtggtatag agttttagtgtttnccgggtaaaaatgttnctagagatctgttgcaaccatgtgaatatacttaacactaccgtactgtacactgaaaaa atggttaagaggataaatttaaatggttaagaggaaaaatggttaagaggataatgttgttttgttaccacagtagtaacttttaaaaa ccctcatgtatgttcttt |
| **663** | 37892_ at | caacccattttgtgccacatgcaagttttgaataaggatggtatagaaaacaacgctgcatatacaggtaccatttaggnannanc ngatgcctttntgggggcagaatcacatggcaaaagctttgaaaatcataaagatataagttggtgtggctaagatggaaacagg gctgattcttgattcccaattctcaactctccttttcctatttgaatttctttggtgctgtagaaaacaaaaaaagaaaaatatatattcata aaaaatatggtgctcattctcatccatccaggatgtactaaaacagtgtgtttaataaattgtaattattttgtgtacagttctatactgttat ctgtgtccatttccaaaacttgcacgtgtccctgaattcc |

**TABLE 7: MSP primers and PCR conditions**

| **Gene** | **Probe set** | **Gene Name** | **Forward primer 5'- 3'** | **Reverse primer 5' - 3'** | **Re-anneal Temp** |
|---|---|---|---|---|---|
| CA4 | 206208_at | Carbonic anhydrase IV | TCGTTTTCGCGTTTAGTT GTC (SEQ ID NO:664) | GCGCACCGAAAAAACCG (SEQ ID NO:665) | 61.0°C |
| CXCL1 2 | 209687_at | Chemokine,CXC Motif, Ligand 12 | CGGTTACGGTTAGTATTCG GTTTC (SEQ ID NO:666) | AAAATACGACGATAAAAA ACGCG (SEQ ID NO:667) | 64.0°C |
| DF | 205382_s at | complement factor D | CGAGGGTTTTTAGCGATT TGTC (SEQ ID NO:668) | AAACGAACCGCTCCCCG (SEQ ID NO:669) | 64.0°C |
| MAMD C2 | 228885_at | MAM domain-containing protein 2 precursor | TTCGGCGTTTTCGTTTTTTA C (SEQ ID NO:670) | CCCCTTAACAACATAATCG CG (SEQ ID NO:671) | 60.0°C |
| MT1M | 217546_at | Metallothionein-1M | GATGGTGCGTTCGGTATTT ATGT (SEQ ID NO:672) | GCTTACACCCGCCCGACTA (SEQ ID NO:673) | 62.0°C |
| CAGE | | Control unbiased amplification assay | GTTTTGTGATTGGTTAGGT TATTAAAGT (SEQ ID NO:674) | CCRTCACCTTCTACACCAA AAAATA (SEQ ID NO:675) | 61.0°C |

**TABLE 8: COBRA Primers and PCR conditions**

| **Gene** | **Probe set** | **Gene Name** | **Forward primer 5'-3'** | **Reverse primer 5'-3'** | **Re-anneal Temp, Enzyme** |
|---|---|---|---|---|---|
| ADAM DECI | 206134_at | ADAM-like, decysin 1 | TGTGGATTTATTTTTATAA GGATATTGAAT (SEQ ID NO:676) | ATAACTCATTAAAAACT CACATCTAAACACTA (SEQ ID NO:677) | 61.0°C BstU I |
| GPM6B | 209170_s _at | Neuronal membrane glycoprotein M6-b | TTAAGAAAGAGAGTAAG GAGGAAGAGTT (SEQ ID NO:678) | AAAATCCCAATTAAAAA ACAACCA (SEQ ID NO:679) | 60.0°C Hinf I |
| P2RY14 | 206637_at | Purinergic receptor P2Y, G-protein coupled, 14 | CAAAATAATAAATCCCTC TACTACTATTATCAA (SEQ ID NO:680) | GGAGGAAAGGAATTAG TTTAGAAGTTAGTT (SEQ ID NO:681) | 60.0°C Acl I |

### BIBLIOGRAPHY

Affymetrix. GeneChip expression data analysis fundamentals. Affymetrix, Santa Clara, CA USA, 2001.
Alon et al., Proc. Natl. Acad. Sci. USA: 96, 6745-6750, June 1999
Ausubel, F. et al., "Current Protocols in Molecular Biology", John Wiley & Sons, (1998)
Bonner et al (1973) J. Mol. Biol. 81:123
Clark et al. 2006, Nature Protocols I:2353-2364
DeRisi, et al., Nature Genetics 14:457-460 (1996)
Germer et al., Genome Res. 10:258-266 (2000)
Guo et al., Nucleic Acids Res. 22:5456-5465 (1994)
Heid et al., Genome Res. 6:986-994 (1996)
Hubbell E.W., W. M. Liu, and R. Mei. Robust estimators for expression analysis. Bioinformatics, 18:1585-1592, 2002.
Irizarry R.W., B. M. Bolstad, F. Collin, L. M. Cope, B. Hobbs, and T. P. Speed. Summaries of affymetrix genechip probe level data. Nucleic Acid Research, 31, 2003.
Kraus, M. and Aaronson, S., 1991. Methods Enzymol., 200:546-556
Maskos and Southern, Nuc. Acids Res. 20:1679-84, 1992
Moore et al., BBA, 1402:239-249, 1988
Nielsen (1999) Curr. Opin. Biotechnol. 10:71-75
Nielsen et al. (1991) Science 254: 1497-1500
Pease et al., Proc. Natl. Acad Sci. USA 91(11):5022-5026 (1994)
Pevzner et al., J. Biomol. Struc. & Dyn. 9:399-410, 1991
Schena, et al. Science 270:467-470 (1995)
Smith et al., Science 258:1122-1126 (1992)
Smyth G.K. Bioinformatics and Computational Biology Solutions using R and Bioconductor. Springer, New York, 2005.
Smyth G.K. Linear models and empirical bayes methods for assessing differential expression in microarray experiments. Statistical Applications in Genetics and Molecular Biology, 3(1):Article 3, 2004.
T. Sano and C. R. Cantor, Bio/Technology 9:1378-81 (1991)
Urdea et al., Nucleic Acids Symp. Ser., 24:197-200 (1991)
Wedemeyer et al., Clinical Chemistry 48:9 1398-1405, 2002)
Weissleder et al., Nature Medicine 6:351-355, 2000

## Claims

1. A method of screening for the onset or predisposition to the onset of a large intestine neoplasm or monitoring the progress of a neoplasm in an individual, said method comprising assessing the level of expression of one or more genes or transcripts selected from:
**(i)** the gene, genes or transcripts detected by Affymetrix probeset IDs:
211964_at; and/or
**(ii)** COL4A1 or COL4A2
in a biological sample from said individual wherein a lower level of expression of the genes or transcripts of group (i) and/or group (ii) relative to control levels is indicative of a neoplastic large intestine cell or a cell predisposed to the onset of a neoplastic state.

2. The method according to claim 1, wherein said control level is a non-neoplastic level.

3. The method according to claim 1 or claim 2, wherein said cell is a colorectal cell.

4. The method according to any preceding claim, wherein said biological sample is a faecal sample, enema wash, surgical resection, tissue biopsy or blood sample.

5. The method according to any preceding claim, wherein said level of expression is assessed by screening for changes to mRNA expression, protein expression genomic DNA or the chromatin protein with which said gene is associated.

6. The method according to claim 5 wherein said changes to genomic DNA are changes to DNA methylation.

7. The method according to any preceding claim, wherein said individual is a human.

8. A molecular array, which array comprises a plurality of:
(i) nucleic acid molecules comprising a nucleotide sequence corresponding to any one or more of the neoplastic marker genes selected from:
(a) the gene, genes or transcripts detected by Affymetrix probeset IDs: 209687_at; and/or
(b) CXCL12;
or a sequence exhibiting at least 80% identity thereto or a functional derivative, fragment, variant or homologue of said nucleic acid molecule; or
(ii) nucleic acid molecules comprising a nucleotide sequence capable of hybridising to any one or more of the sequences of (i) under medium stringency conditions or a functional derivative, fragment, variant or homologue of said nucleic acid molecule; or
(iii) nucleic acid probes or oligonucleotides comprising a nucleotide sequence capable of hybridising to any one or more of the sequences of (i) under medium stringency conditions or a functional derivative, fragment, variant or homologue of said nucleic acid molecule; or
(iv) probes capable of binding to any one or more of the proteins encoded by the nucleic acid molecules of (i) or a derivative, fragment or, homologue thereof
wherein the level of expression of said marker genes of (i) or proteins of (v) is indicative of the neoplastic state of a cell or cellular subpopulation derived from the large intestine.
